# EUROPEAN PATENT SPECIFICATION

(11) **EP 3 972 651 B1**
(45) Date of publication and mention of the grant of the patent: **23.09.2026**
(21) Application number: 20733328.7
(22) Date of filing: 19.05.2020
(51) Int. Cl.: A61K 47/68

(54) **MCL-1 INHIBITOR ANTIBODY-DRUG CONJUGATES AND METHODS OF USE**
MCL-1-INHIBITOR-ANTIKÖRPER-WIRKSTOFF-KONJUGATE UND VERWENDUNGSVERFAHREN
CONJUGUÉS ANTICORPS-MÉDICAMENT INHIBITEURS DE MCL-1 ET PROCÉDÉS D'UTILISATION

(30) Priority: 20.05.2019 US 201962850098 P
(43) Date of publication of application: 30.03.2022
(73) Proprietor: Novartis AG, 4056 Basel (CH); Les Laboratoires Servier, 92284 Suresnes Cedex (FR)
(72) Inventor: BURGER, Matthew, T., Cambridge, MA 02139 (US); CHANRION, Maia, 92130 Issy Les Moulineaux (FR); COLLAND, Frédéric, 95380 Puiseux-En-France (FR); CSEKEI, Marton, 2120 Dunakeszi (HU); DELACOUR, Lea, 1116 Luxembourg (LU); DESOS, Patrice, 92270 Bois Colombes (FR); GENESTE, Olivier, 92500 Rueil-Malmaison (FR); HENLIN, Jean-Michel, 92150 Suresnes (FR); KOSTOVA, Vesela, 92210 Saint Cloud (FR); KOTSCHY, Andras, 2045 Torokbalint (HU); MARAGNO, Ana, Leticia, 78290 Croissy-Sur-Seine (FR); MCNEILL, Eric, Cambridge, MA 02139 (US); PALERMO, Mark, G., Cambridge, MA 02139 (US); ROCCHETTI, Francesca, 75005 Paris (FR); STARCK, Jérôme, 92500 Rueill-Malmaison (FR); YU, Bing, Cambridge, MA 02139 (US); ZHANG, Qiang, Cambridge, MA 02139 (US); PROSZENYÁK, Ágnes, 1037 Budapest (HU); SIPOS, Szabolcs, Budapest 1165 (HU); CHEN, Zhuoliang, Cambridge, MA 02139 (US); NAKAJIMA, Katsumasa, Cambridge, MA 02139 (US); D'ALESSIO, Joseph, Anthony, Cambridge, MA 02139 (US)
(74) Representative: Elkington and Fife LLP
(86) International application number: PCT/US2020/033615
(87) International publication number: WO 2020/236825

(56) References cited:
- EP-A1- 2 886 545
- EP-A1- 3 165 536
- WO-A1-2014/150937
- WO-A1-2016/203432
- WO-A1-2016/207216
- WO-A1-2017/125224
- WO-A1-2018/098534
- WO-A1-2018/148566
- WO-A1-2019/035911
- WO-A1-2019/035914
- WO-A1-2019/035927
- WO-A1-2019/089594

## Description

### FIELD OF THE INVENTION

The present disclosure relates to antibody-drug conjugates (ADCs) comprising an Mcl-1 inhibitor and an antibody or antigen-binding fragment thereof that binds an antigen target, e.g., an antigen expressed on a tumor or other cancer cell. The disclosure further relates to methods and compositions useful in the treatment and/or diagnosis of cancers that express a target antigen and/or are amenable to treatment by modulating Mcl-1 expression and/or activity, as well as methods of making those compositions. Linker-drug conjugates comprising an Mcl-1 inhibitor drug moiety and methods of making same are also disclosed.

### BACKGROUND OF THE INVENTION

Apoptosis, or programmed cell death, is a physiological process that is crucial for embryonic development and maintenance of tissue homeostasis. Apoptotic-type cell death generally involves morphological changes such as condensation of the nucleus and DNA fragmentation, as well as biochemical changes such as the activation of caspases that can cause damage to key structural components of the cell. Regulation of apoptosis is complex and typically involves the activation or repression of several intracellular signaling pathways (Cory et al. (2002) Nature Review Cancer 2:647-656).

Deregulation of apoptosis is associated with certain pathologies. For instance, increased apoptosis is associated with neurodegenerative diseases such as Parkinson's disease, Alzheimer's disease, and ischemia. Conversely, deficits in apoptosis can play a role in the development of cancers and chemoresistance, autoimmune diseases, inflammatory diseases, and viral infections. The absence of apoptosis is one of the phenotypic signatures of cancer (Hanahan et al. (2000) Cell 100:57-70). Anti-apoptotic proteins of the Bcl-2 family are associated with numerous types of cancer, such as colon cancer, breast cancer, small cell lung cancer, non-small cell lung cancer, bladder cancer, ovarian cancer, prostate cancer, chronic lymphoid leukemia, lymphoma, myeloma, and pancreatic cancer.

Myeloid cell leukemia 1 (Mcl-1), an anti-apoptotic Bcl-2 family member, is a regulator of cell survival. Amplification of the Mcl-1 gene and/or overexpression of the Mcl-1 protein has been observed in multiple cancer types and is commonly implicated in tumor development (Beroukhim et al. (2010) Nature 463(7283):899-905). Mcl-1 is one of the most frequently amplified genes in human cancer and is also a critical survival factor that has been shown to mediate drug resistance to a variety of anti-cancer agents.

Mcl-1 is believed to promote cell survival by binding to and neutralizing the death-inducing activities of pro-apoptotic proteins such as Bim, Noxa, Bak, and Bax. Inhibition of Mcl-1 releases these pro-apoptotic proteins, often leading to the induction of apoptosis in tumor cells dependent on Mcl-1 for survival. Therapeutically targeting Mcl-1 or proteins upstream and/or downstream of it in an apoptotic signaling pathway, therefore, may represent promising strategies to treat various malignancies and to overcome drug resistance in certain human cancers.

WO2014/150937A1 is directed to anti-cKIT antibodies, antibody fragments, antibody drug conjugates, and their uses for the treatment of cancer. In some embodiments, the antibody drug conjugate is an MCL1 inhibitor.

### SUMMARY OF THE INVENTION

In some embodiments, the present disclosure provides, in part, novel antibody-drug conjugate (ADC) compounds with biological activity against cancer cells. The compounds may slow, inhibit, and/or reverse tumor growth in mammals, and/or may be useful for treating human cancer patients. The present disclosure more specifically relates, in some embodiments, to ADC compounds that are capable of binding and killing cancer cells. In some embodiments, the ADC compounds disclosed herein comprise a linker that attaches an Mcl-1 inhibitor to a full-length antibody or an antigen-binding fragment. In some embodiments, the ADC compounds are also capable of internalizing into a target cell after binding.

In a first aspect of the present invention, there is provided ADC compounds represented by Formula (1):

Ab-(L-D)*ₚ* (1)

wherein Ab is an antibody or an antigen-binding fragment thereof;
D is an Mcl-1 inhibitor, wherein D comprises a compound of Formula (II): 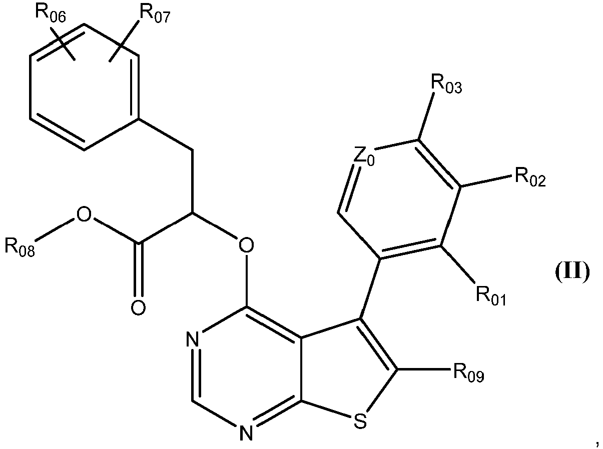
   or an enantiomer, diastereoisomer, atropisomer, deuterated derivative, and/or
   pharmaceutically acceptable salt thereof;
   wherein:
      Z₀ is a nitrogen atom or a C-R₀₄ group,
      R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a linear or branched (C₁-C₆)alkoxy group, a -S-(C₁-C₆)alkyl group, a cyano group, -Cy₀₈, -NR₀₁₁R₀₁₁',
      R₀₂, R₀₃ and R₀₄ independently of one another are a hydrogen atom,
   a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, - (C₀-C₆)alkyl-Cy₀₁, -(C₂-C₆)alkenyl-Cy₀₁, -(C₂-C₆)alkynyl-Cy₀₁, -O-(C₁-C₆)alkyl-NR₀₁₁R₀₁₁', -O-(C₁-C₆)alkyl-R₀₃₁, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or - SO₂-(C₁-C₆)alkyl,
   or the pair (R₀₂, R₀₃) or (R₀₃, R₀₄) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched (C₁-C₆)alkyl, -NR₀₁₃R_{013'}, -(C₀-C₆)alkyl-Cy₀₁ and oxo,
      R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
   or the pair (R₀₆, R₀₇), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched (C₁-C₆)alkyl group, -NR₀₁₃R₀₁₃', -(C₀-C₆)alkyl-Cy₀₁ and an oxo,
      R₀₈ is a hydrogen atom, a linear or branched (C₁-C₈)alkyl group, an aryl group, a heteroaryl group, an aryl-(C₁-C₆)alkylgroup, or a heteroaryl(C₁-C₆)alkyl group,
      R₀₉ is a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, -C(O)-NR₀₁₄R₀₁₄',
      R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched (C₁-C₆)alkyl group, and wherein one or more of the carbon atoms of the linear or branched (C₁-C₆)alkyl group is optionally deuterated,
      R₀₁₂ is -Cy₀₅, -Cy₀₅-(C₀-C₆)alkyl-Cy₀₆, -Cy₀₅-(C₀-C₆)alkyl-O-(C₀-C₆)alkyl-Cy₀₆, - Cy₀₅-(C₀-C₆)alkyl-NR₀₁₁C₀-C₆)alkyl-Cy₀₆, -Cy₀₅-Cy₀₆-O-(C₀-C₆)alkyl-Cy₀₇, -Cy₀₅-(C₀-C₆)alkyl-Cy₀₉, -NH-C(O)-NH-R₀₁₁, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, or -C(O)-OR₀₁₁,
      R₀₁₃, R₀₁₃', R₀₁₄ and R₀₁₄' independently of one another are a hydrogen atom, or an optionally substituted linear or branched (C₁-C₆)alkyl group,
      Cy₀₁, Cy₀₂, Cy₀₃, Cy₀₅, Cy₀₆, Cy₀₇, Cy₀₈ and Cy₁₀ independently of one another, are a cycloalkyl group, a heterocycloalkyl group, an aryl group or a heteroaryl group, each of which is optionally substituted,
      Cy₀₉ is 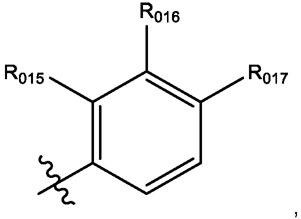
      R₀₁₅ is a hydrogen atom; a -(CH₂)ₚ₀-O-(CHR₀₁₈-CHR₀₁₉-O)_{q0}-R₀₂₀ group; a linear or branched (C₁-C₆)alkoxy(C₁-C₆)alkyl group; a -U₀-(CH₂)_{q0}-NR₀₂₁R₀₂₁' group; or a -(CH₂)ᵣ₀-U₀-(CH₂)ₛ₀-heterocycloalkyl group,
      R₀₁₆ is a hydrogen atom; a hydroxy group; a hydroxy(C₁-C₆)alkyl group; a - (CH₂)ᵣ₀-U₀-(CH₂)ₛ₀-heterocycloalkyl group; a (CH₂)ᵣ₀-U₀-V₀-O-P(O)(OR₀₂₀)₂ group; a -O-P(O)(O⁻M⁺)₂ group; a -O-S(O)₂OR₀₂₀ group; a -S(O)₂OR₀₂₀ group; a -(CH₂)ₚ₀-O-(CHR₀₁₈-CHR₀₁₉-O)_{q0}-R₀₂₀ group; a -(CH₂)ₚ₀-O-C(O)-NR₀₂₂R₀₂₃ group; or a -U₀-(CH₂)_{q0}-NR₀₂₁R₀₂₁' group,
      R₀₁₇ is a hydrogen atom; a -(CH₂)ₚ₀-O-(CHR₀₁₈-CHR₀₁₉-O)_{q0}-R₀₂₀ group; a - CH₂-P(O)(OR₀₂₀)₂ group, a -O-P(O)(OR₀₂₀)₂ group; a -O-P(O)(O⁻M⁺)₂ group; a hydroxy group; a hydroxy(C₁-C₆)alkyl group; a -(CH₂)ᵣ₀-U₀-(CH₂)ₛ₀-heterocycloalkyl group; a -U₀-(CH₂)_{q0}-NR₀₂₁R₀₂₁' group; or an aldonic acid,
      M⁺ is a pharmaceutically acceptable monovalent cation,
      U₀ is a bond or an oxygen atom,
      V₀ is a -(CH₂)ₛ₀- group or a -C(O)- group,
      R₀₁₈ is a hydrogen atom or a (C₁-C₆)alkoxy(C₁-C₆)alkyl group,
      R₀₁₉ is a hydrogen atom or a hydroxy(C₁-C₆)alkyl group,
      R₀₂₀ is a hydrogen atom or a linear or branched (C₁-C₆)alkyl group,
      R₀₂₁ and R₀₂₁' independently of one are a hydrogen atom, a linear or branched (C₁-C₆)alkyl group, or a hydroxy(C₁-C₆)alkyl group,
      or the pair (R₀₂₁, R₀₂₁') together with the nitrogen atom to which they are attached form an aromatic or non-aromatic ring containing 5 to 7 ring members,
      R₀₂₂ is a (C₁-C₆)alkoxy(C₁-C₆)alkyl group, a -(CH₂)ₚ₀-NR₀₂₄R₀₂₄' group, or a -(CH₂)ₚ₀-O-(CHR₀₁₈-CHR₀₁₉-O)_{q0}-R₀₂₀ group,
      R₀₂₃ is a hydrogen atom or a (C₁-C₆)alkoxy(C₁-C₆)alkyl group,
      or the pair (R₀₂₂, R₀₂₃) together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched (C₁-C₆)alkyl group or a heterocycloalkyl group,
      R₀₂₄ and R₀₂₄' independently of one another are a hydrogen atom or a linear or branched (C₁-C₆)alkyl group,
   or the pair (R₀₂₄, R₀₂₄') together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched (C₁-C₆)alkyl group,
      R₀₃₁ **is** 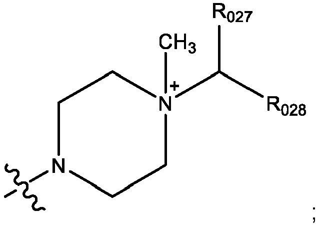
   R₀₂₇ is a hydrogen atom or a linear or branched (C₁-C₆)alkyl group,
   R₀₂₈ is a -O-P(O)(O⁻)(O⁻) group, a -O-P(O)(O⁻)(OR₀₃₀) group,
      a -O-P(O)(OR₀₃₀)(OR₀₃₀') group, a -(CH₂)ₚ₀-O-SO₂-O⁻ group, a -(CH₂)ₚ₀-SO₂-O⁻ group, a -(CH₂)ₚ₀-O-SO₂-OR₀₃₀ group, -Cy₀₁₀, a -(CH₂)ₚ₀-SO₂-OR₀₃₀ group, a -O-C(O)-R₀₂₉ group, a -O-C(O)-OR₀₂₉ group or a -O-C(O)-NR₀₂₉R₀₂₉' group;
   R₀₂₉ and R₀₂₉' independently of one another are a hydrogen atom, a linear or branched (C₁-C₆)alkyl group or a linear or branched amino(C₁-C₆)alkyl group,
   R₀₃₀ and R₀₃₀' independently of one another are a hydrogen atom, a linear or branched (C₁-C₆)alkyl group or an aryl(C₁-C₆)alkylgroup,
   p₀ is an integer equal to 0, 1, 2, or 3,
   q₀ is an integer equal to 1, 2, 3 or 4,
   r₀ and s₀ are independently an integer equal to 0 or 1;
   wherein one of the R₀₃, R₀₉, or R₀₁₂ groups is covalently attached to the linker,

   L is a linker that covalently attaches Ab to D; and
   p is an integer from 1 to 16.

In an embodiment of the first aspect, p is an integer from 1 to 6 or from 2 to 4, or p is 2 or 4; or p is determined by liquid chromatography-mass spectrometry (LC-MS).

In an embodiment of the first aspect, L comprises:
an attachment group;
at least one bridging spacer group; and
at least one cleavable group, optionally at least one cleavable group comprising a pyrophosphate group and/or a self-immolative group.

In an embodiment of the first aspect, -(L-D) is of the formula (A): 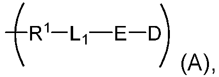 wherein:
R¹ is an attachment group;
L₁ is a bridging spacer group;
E is a cleavable group.

In an embodiment of the first aspect:
(1) the cleavable group comprises a pyrophosphate group or the cleavable group comprises 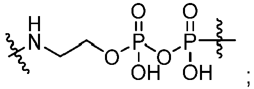
(2) the bridging spacer group comprises:
   (i) a polyoxyethylene (PEG) group;
   (ii) a PEG group selected from, PEG1, PEG2, PEG3, PEG4, PEG5, PEG6, PEG7, PEG8, PEG9, PEG10, PEG11, PEG12, PEG13, PEG14, and PEG15;
   (iii) a -CO-CH₂-CH₂-PEG12- group;
   (iv) a butanoyl, pentanoyl, hexanoyl, heptanoyl, or octanoyl group; or
   (v) a hexanoyl group;
(3)
   (i) the attachment group is formed from at least one reactive group selected from a maleimide group, thiol group, cyclooctyne group, and an azido group; optionally wherein:
      a) the maleimide group has the structure: 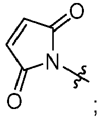
      b) the azido group has the structure: -N=N⁺=N⁻;
      c) the cyclooctyne group has the structure: 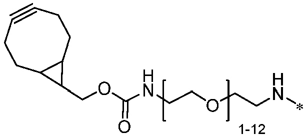 or 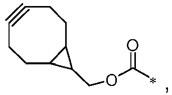 and wherein⁻* is a bond to the antibody or antigen-binding fragment; or
      d) the cyclooctyne group has the structure: 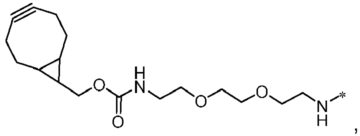 and
      wherein ⁻* is a bond to the antibody or antigen-binding fragment; or
   (ii) the attachment group has a formula comprising: 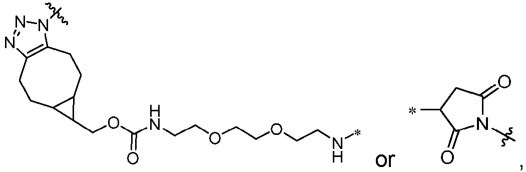 and
      wherein ⁻* is a bond to the antibody or antigen-binding fragment;
(4) the antibody or antigen-binding fragment is joined to the linker (L) by an attachment group selected from: 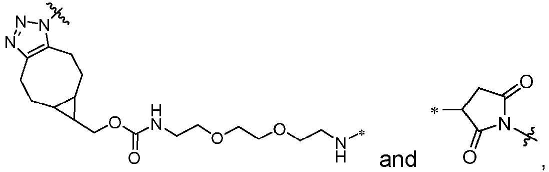 wherein ⁻* is a bond to the antibody or antigen-binding fragment, and wherein 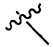 is a bond to the bridging spacer group;
(5) the bridging spacer group is -CO-CH₂-CH₂-PEG12-;
(6) the bridging spacer group is joined to a cleavable group; optionally the cleavable group is -pyrophosphate-CH₂-CH₂-NH₂-; or
(7) the cleavable group is joined to the Mcl-1 inhibitor (D), or the cleavable group is joined to the Mcl-1 inhibitor (D) through a phenyl-pyrimidinyl group.

In an embodiment of the first aspect, the linker comprises:
an attachment group,
at least one bridging spacer group,
a peptide group, and
at least one cleavable group.

In an embodiment of the first aspect, -(L-D) is of the formula (B): 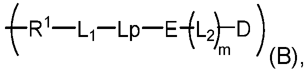 wherein:
R¹ is an attachment group;
L₁ is a bridging spacer;
Lp is a peptide group comprising 1 to 6 amino acid residues or Lp comprises a group 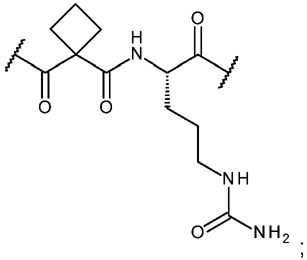
E is a cleavable group
L₂ is a bridging spacer;
m is 0 or 1; and
D is an Mcl-1 inhibitor as defined in the first aspect.

In an embodiment of the first aspect:
(1)
   (i) the attachment group is formed from at least one reactive group comprising a maleimide group, thiol group, cyclooctyne group, and/or an azido group, optionally wherein:
      a) the maleimide group has the structure: 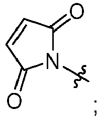
      b) the azido group has the structure: -N=N⁺=N⁻;
      c) the cyclooctyne group has the structure: 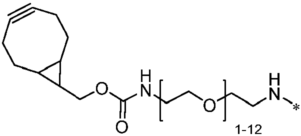 or 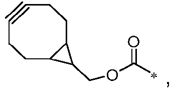 and wherein⁻* is a bond to the antibody or antigen-binding fragment; or
      d) the cyclooctyne group has the structure: 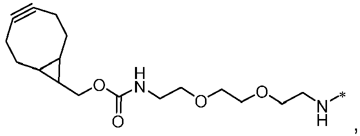 and
      wherein ⁻* is a bond to the antibody or antigen-binding fragment; or
   (ii) the attachment group has a formula comprising: 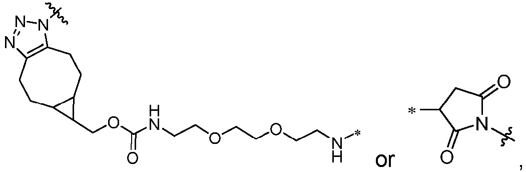 and wherein ⁻* is a bond to the antibody or antigen-binding fragment;
(2)
   (i) at least one bridging spacer comprises a PEG group, optionally the PEG group is selected from, PEG1, PEG2, PEG3, PEG4, PEG5, PEG6, PEG7, PEG8, PEG9, PEG10, PEG11, PEG12, PEG13, PEG14, and PEG15; or
   (ii) at least one bridging spacer is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, a polyhydroxyalkyl group, and *-C(O)-N(CH₃)-CH₂-CH₂-N(CH₃)-C(O)-**, wherein ** indicates the point of direct or indirect attachment of the at least one bridging spacer to the attachment group and * indicates the point of direct or indirect attachment of the at least one bridging spacer to the peptide group;
(3) L₁ is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, and a polyhydroxyalkyl group, wherein ** indicates the point of direct or indirect attachment of L₁ to R¹ and * indicates the point of direct or indirect attachment of L₁ to Lp;
(4) m is 1 and L₂ is -C(O)-N(CH₃)-CH₂-CH₂-N(CH₃)-C(O)-;
(5)
   (i) the peptide group comprises 1 to 6, 1 to 4, 1 to 3 or 1 to 2 amino acid residues, optionally the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
   (ii) the peptide group comprises Val-Cit, Val-Ala, Val-Lys, and/or sulfo-Ala-Val-Ala;
   (iii) the peptide group is selected from: 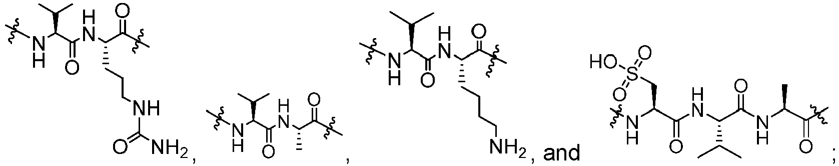
(6) (i) the cleavable group comprises a pyrophosphate and/or a self-immolative group; (ii) the cleavable group comprises a self-immolative group; or (iii) the cleavable group comprises a self-immolative group comprising para-aminobenzyl-carbamate, para-aminobenzyl-ammonium, para-amino-(sulfo)benzyl-ammonium, para-amino-(sulfo)benzyl-carbamate, para-amino-(alkoxy-PEG-alkyl)benzyl-carbamate, para-amino-(polyhydroxycarboxytetrahydropyranyl)alkyl-benzyl-carbamate, or para-amino-(polyhydroxycarboxytetrahydropyranyl)alkyl-benzyl-ammonium; or
(7) m is 0 or 1 or m is 1 and the bridging spacer comprises

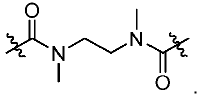

In an embodiment of the first aspect:
(1) -(L-D) is formed from a compound selected from: 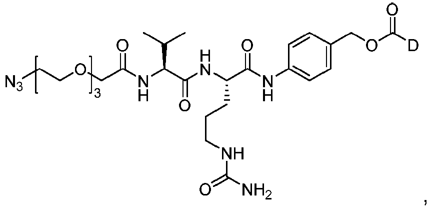 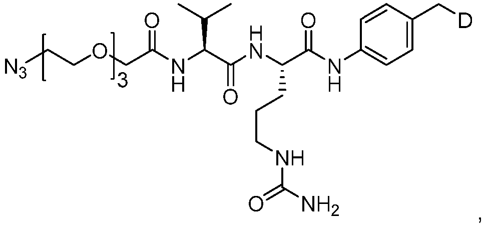 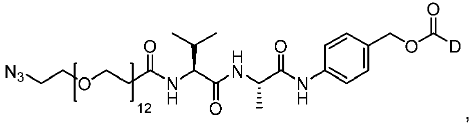 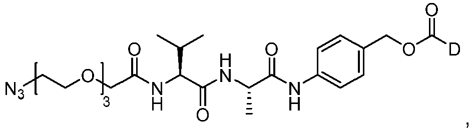 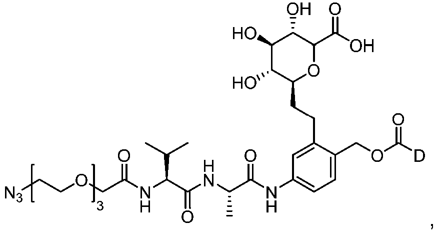 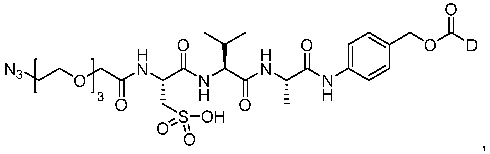 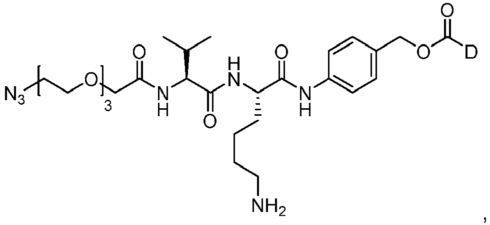 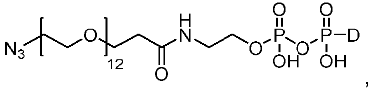 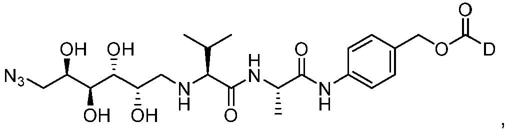 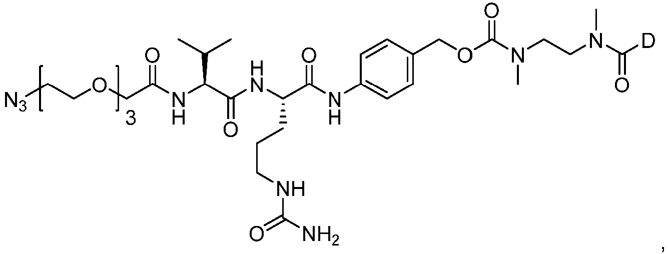 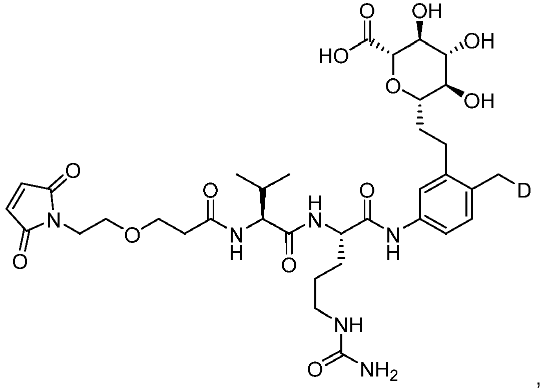 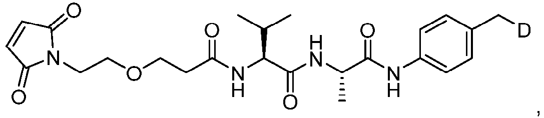 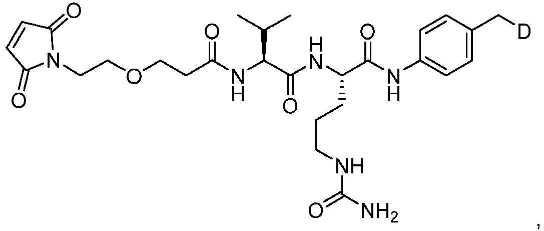 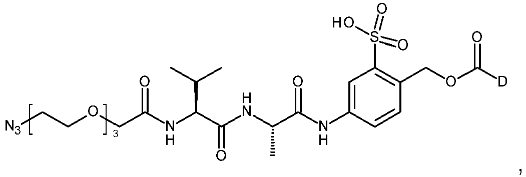 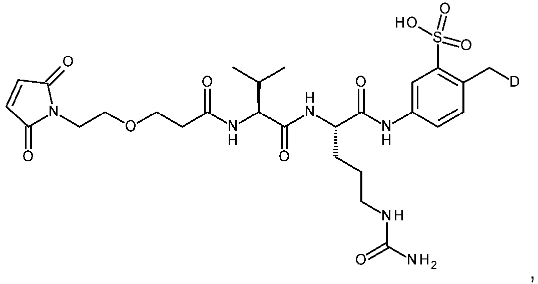 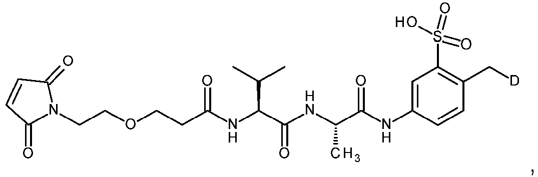 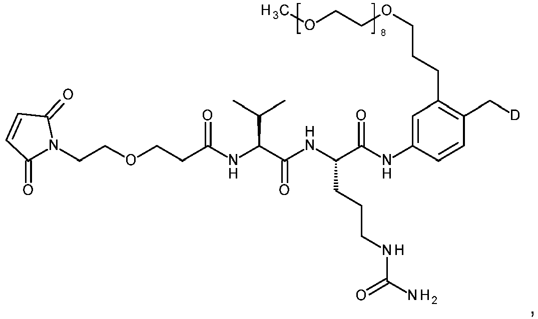 and 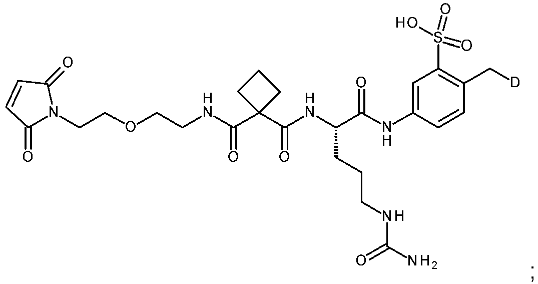 or
(2) -(L-D) comprises a formula selected from: 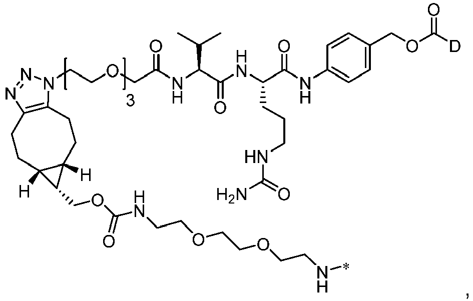 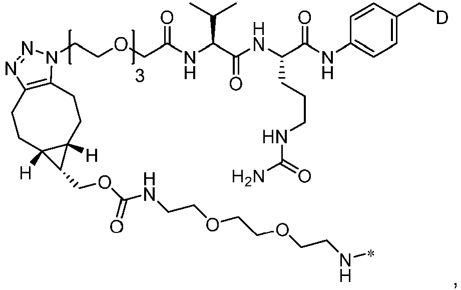 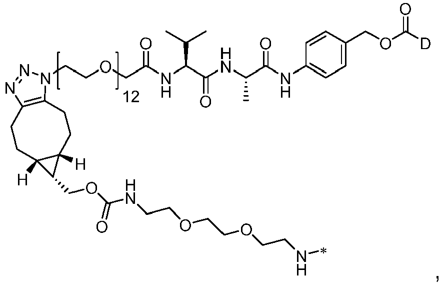 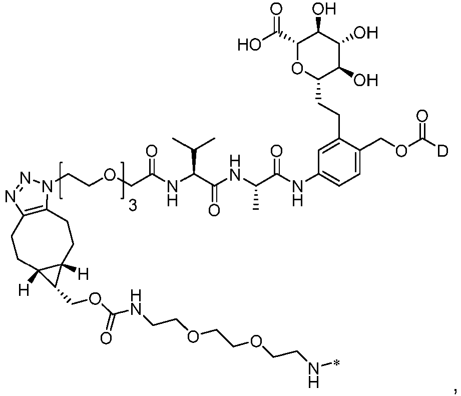 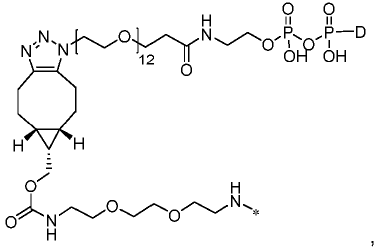 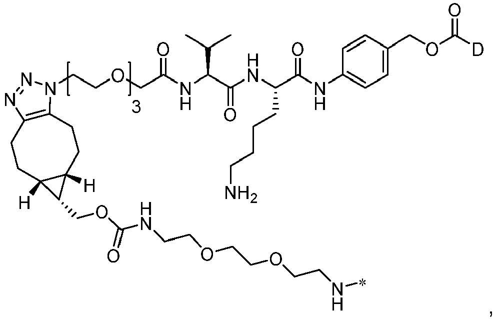 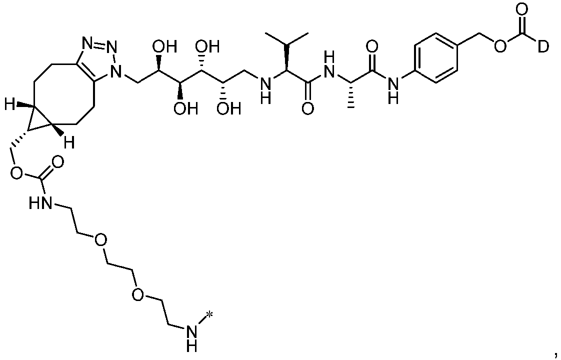 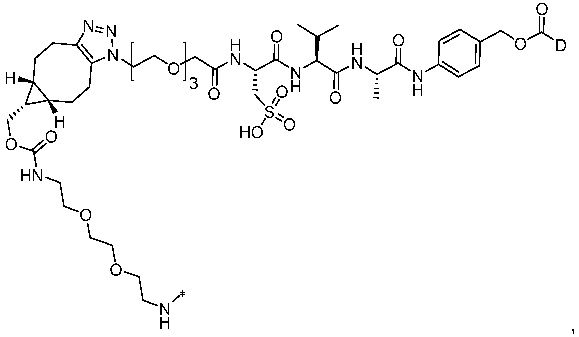 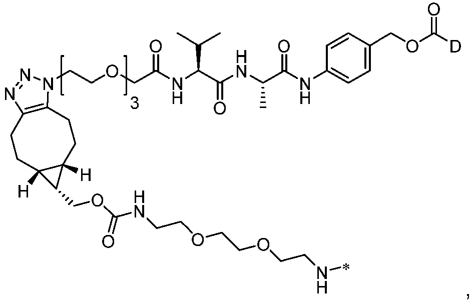 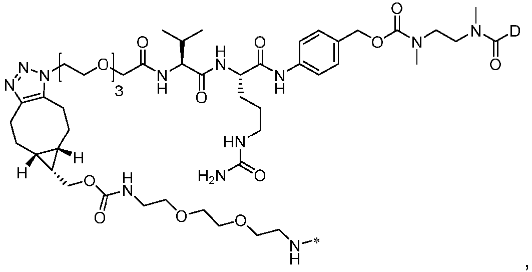 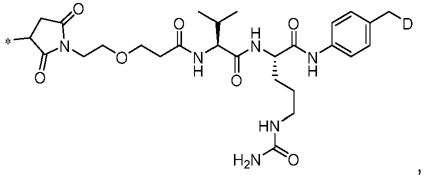 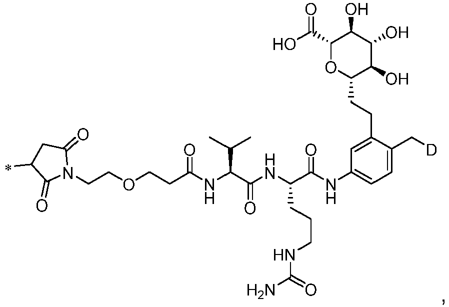 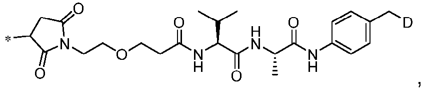 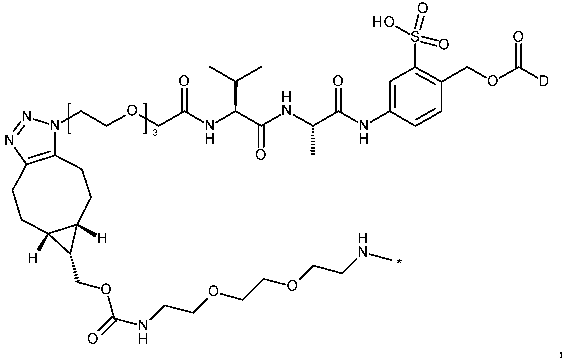 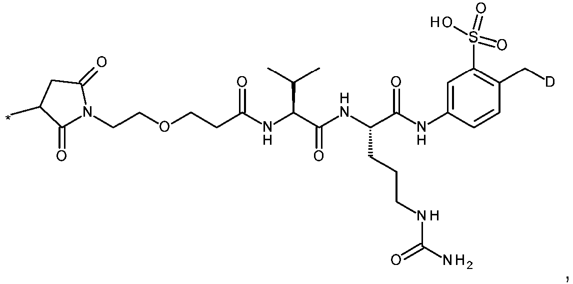 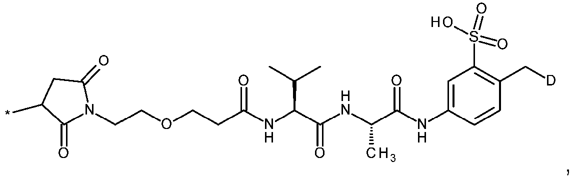 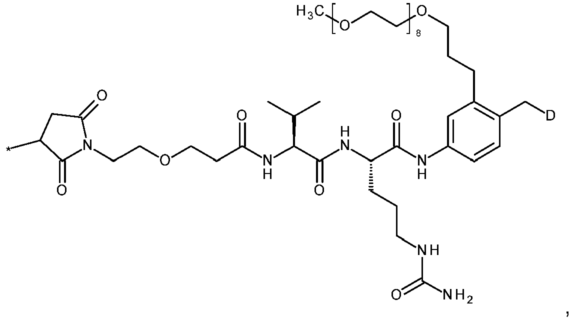 and 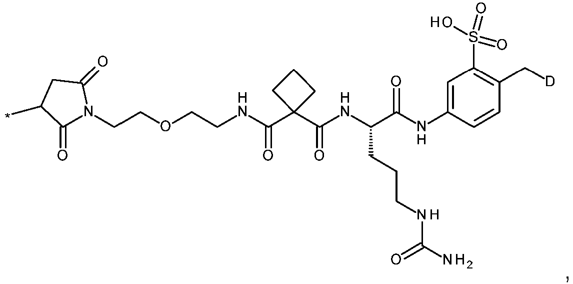 and
   wherein ⁻* is a bond to the antibody or antigen-binding fragment.

In an embodiment of the first aspect:
(1) -(L-D) is of the formula (C): 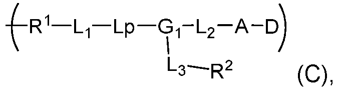 wherein:
   R¹ is an attachment group;
   L₁ is a bridging spacer;
   Lₚ is a peptide group comprising 1 to 6 amino acids;
   D is an Mcl-1 inhibitor as defined in the first aspect;
   G₁-L₂-A is a self-immolative spacer;
   L₂ is a bond, a methylene, a neopentylene or a C₂-C₃ alkenylene;
   A is a bond, -OC(=O)-*, 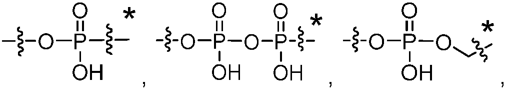 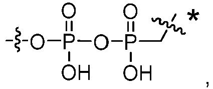 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D;
   L₃ is a spacer moiety; and
   R² is a hydrophilic moiety; or
(2) -(L-D) is of Formula (D): 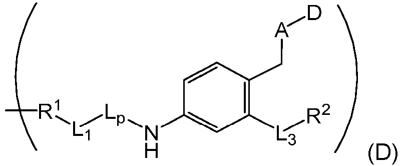 wherein:
   R¹ is an attachment group;
   L₁ is a bridging spacer;
   Lp is a peptide group comprising 1 to 6 amino acids;
   A is a bond, -OC(=O)-*, 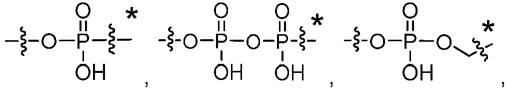 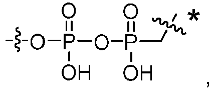 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D;
   L₃ is a spacer moiety; and
   R² is a hydrophilic moiety.

In an embodiment of the first aspect:
(1) L₁ comprises: 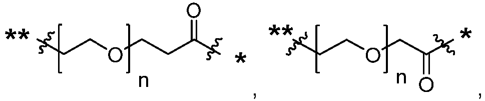 or

   *-CH(OH)CH(OH)CH(OH)CH(OH)-**,

   wherein each n is an integer from 1 to 12, wherein the * of L₁ indicates the point of direct or indirect attachment to Lp, and the ** of L₁ indicates the point of direct or indirect attachment to R¹;
(2) L₁ is 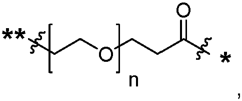 and n is an integer from 1 to 12 or n is 1 or n is 12, wherein the * of L₁ indicates the point of direct or indirect attachment to Lp, and the ** of L₁ indicates the point of direct or indirect attachment to R¹;
(3) L₁ is 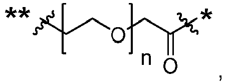 and n is an integer from 1 to 12, wherein the * of L₁ indicates the point of direct or indirect attachment to Lp, and the ** of L₁ indicates the point of direct or indirect attachment to R¹;
(4) L₁ comprises 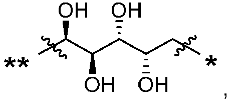 wherein the * of L₁ indicates the point of direct or indirect attachment to Lp, and the ** of L₁ indicates the point of direct or indirect attachment to R¹;
(5) L₁ is a bridging spacer comprising:
   *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH₂)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH₂)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, wherein the * of L₁ indicates the point of direct or indirect attachment to Lp, and the ** of L₁ indicates the point of direct or indirect attachment to R¹;
   X₁ is 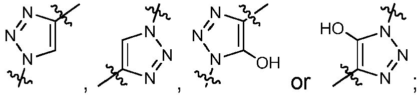 and
   each m is independently selected from 1, 2, 3, 4, 5, 6, 7, 8, 9 and 10;
   each n is independently selected from 1, 2, 3, 4, 5, 6, 7, 8, 9 and 10; and
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   and each R³ is independently selected from H and C₁-C₆alkyl.

In an embodiment of the first aspect:
(1) R² is a hydrophilic moiety comprising polyethylene glycol, polyalkylene glycol, a polyol, a polysarcosine, a sugar, an oligosaccharide, a polypeptide, or C₂-C₆ alkyl substituted with 1 to 3 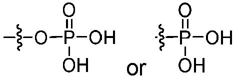 groups;
(2) R² is 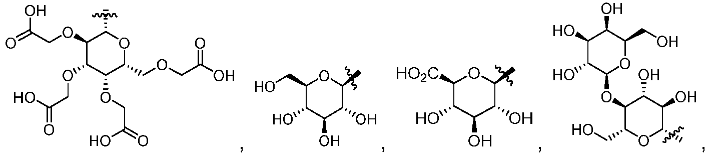 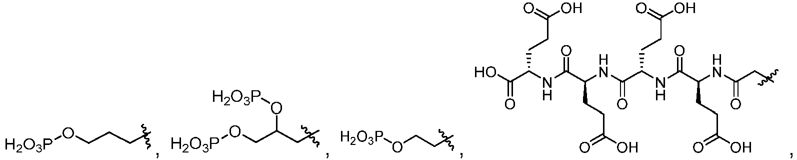 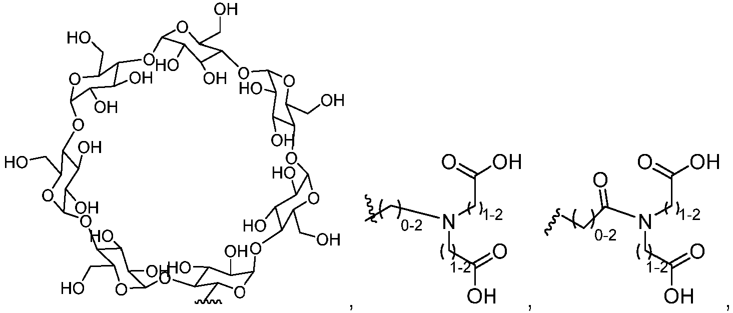 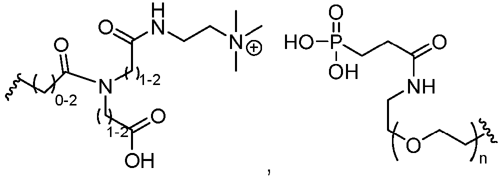 wherein n is an integer between 1 and 6, 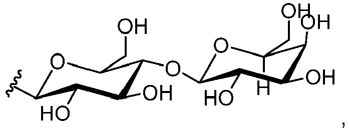 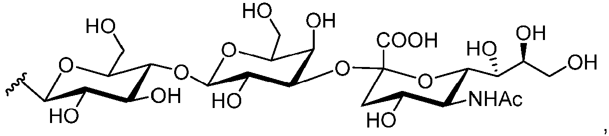 or 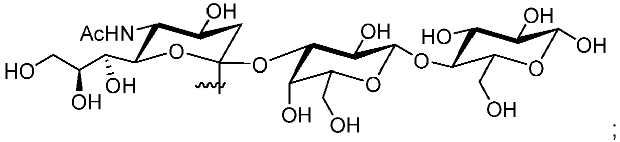
(3) the hydrophilic moiety represented by R² comprises:
   (i) a polysarcosine, e.g., with the following moiety: 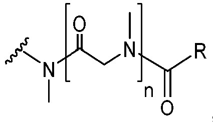 , wherein n is an integer between 3 and 25; and R is H, -CH₃ or - CH_{2C}H_{2C}(=O)OH; or
   (ii) a polyethylene glycol of formula: 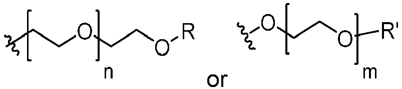 , wherein R is H, -CH₃, CH₂CH₂NHC(=O)ORₐ, -CH₂CH₂NHC(=O)Rₐ, or -CH₂CH₂C(=O)ORₐ, R' is OH, -OCH₃, -CH₂CH₂NHC(=O)ORₐ, -CH₂CH₂NHC(=O)Rₐ, or -OCH₂CH₂C(=O)ORₐ, in which R^{a} is H or C₁₋₄ alkyl optionally substituted with either OH or C₁₋₄ alkoxyl, and each of m and n is independently an integer between 2 and 25; or
(4) the hydrophilic moiety represented by R² comprises

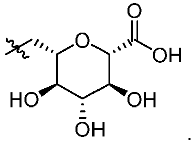

In an embodiment of the first aspect:
(i) L₃ is a spacer moiety having the structure 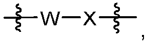 wherein:
   W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, - CH₂N(X-R²)C(=O)-, -C(=O)NR^{b}-, -C(=O)NH-, -CH₂N R^{b} C(=O)-, -CH₂N R^{b} C(=O)NH-, - CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
   X is a bond, triazolyl, or -CH₂-triazolyl-; or
(ii) L₃ is a spacer moiety having the structure 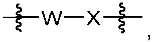 wherein:
   W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, -CH₂N(X-R²)C(=O)-, -C(=O)NR^{b}-, -C(=O)NH-, -CH₂NR^{b}C(=O)-, -CH₂NR^{b}C(=O)NH-, -CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
   X is -CH₂-triazolyl-C₁₋₄ alkylene-OC(O)NHS(O)₂NH-, -C₄₋₆ cycloalkylene-OC(O)NHS(O)₂NH-, -(CH₂CH₂O)ₙ-C(O)NHS(O)₂NH-, -(CH₂CH₂O)ₙ-C(O)NHS(O)₂NH-(CH₂CH₂O)ₙ-, or -CH₂-triazolyl-C₁₋₄ alkylene-OC(O)NHS(O)₂NH-(CH₂CH₂O)ₙ-, wherein each n independently is 1, 2, or 3.

In an embodiment of the first aspect:
(1) the attachment group is formed by a reaction comprising at least one reactive group;
(2) the attachment group is formed by reacting:
   a first reactive group that is attached to the linker, and
   a second reactive group that is attached to the antibody or antigen-binding fragment or is an amino acid residue of the antibody or antigen-binding fragment, wherein optionally,

   (i) at least one of the reactive groups comprises:
      a thiol,
      a maleimide,
      a haloacetamide,
      an azide,
      an alkyne,
      a cyclooctene,
      a triaryl phosphine,
      an oxanorbornadiene,
      a cyclooctyne,
      a diaryl tetrazine,
      a monoaryl tetrazine,
      a norbornene,
      an aldehyde,
      a hydroxylamine,
      a hydrazine,
      NH₂-NH-C(=O)-,
      a ketone,
      a vinyl sulfone,
      an aziridine,
      an amino acid residue, 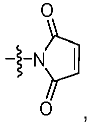 -ONH₂, -NH₂, 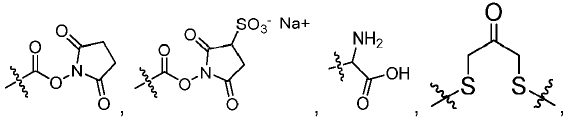 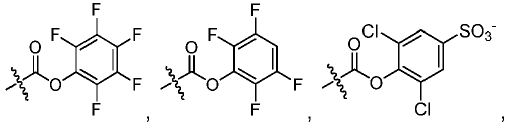 -N₃, 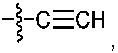 -SH, -SR³, -SSR⁴, -S(=O)₂(CH=CH₂), -(CH₂)₂S(=O)₂(CH=CH₂), -NHS(=O)₂(CH=CH₂), - NHC(=O)CH₂Br, -NHC(=O)CH₂I, 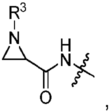 -C(O)NHNH₂, 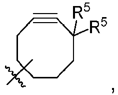 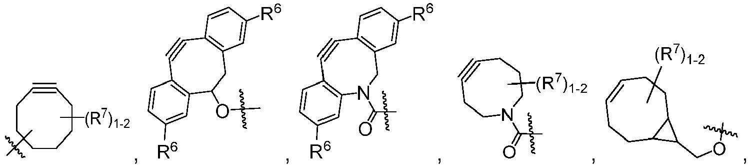 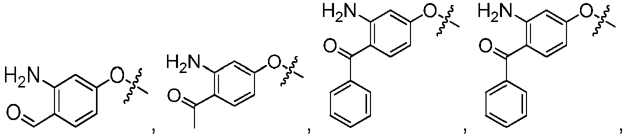 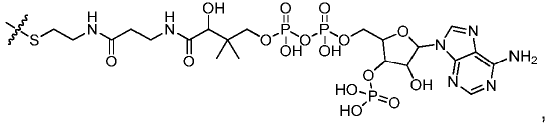 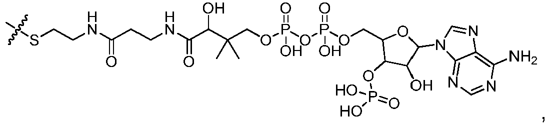 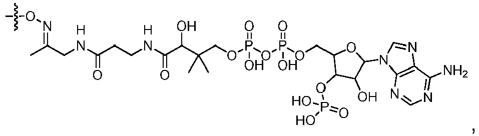 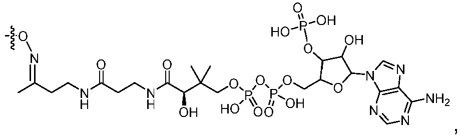 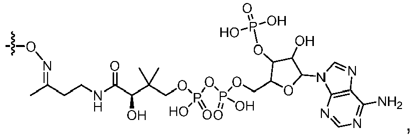 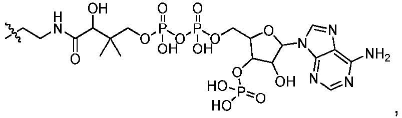 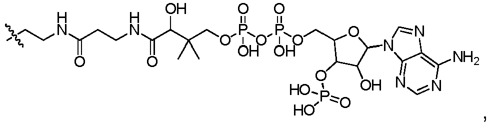 or 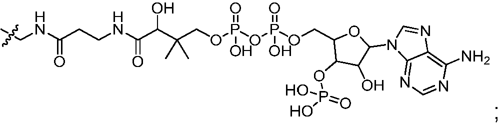 wherein:
         each R³ is independently selected from H and C₁-C₆alkyl;
         each R⁴ is 2-pyridyl or 4-pyridyl;
         each R⁵ is independently selected from H, C₁-C₆alkyl, F, Cl, and -OH;
         each R⁶ is independently selected from H, C₁-C₆alkyl, F, Cl, -NH₂, -OCH₃, - OCH₂CH₃, -N(CH₃)₂, -CN, -NO₂ and -OH;
         each R⁷ is independently selected from H, C₁₋₆alkyl, fluoro, benzyloxy substituted with -C(=O)OH, benzyl substituted with -C(=O)OH, C₁₋₄alkoxy substituted with -C(=O)OH and C₁₋₄alkyl substituted with -C(=O)OH; and/or
   (ii) the first reactive group and second reactive group comprise:
      a thiol and a maleimide,
      a thiol and a haloacetamide,
      a thiol and a vinyl sulfone,
      a thiol and an aziridine,
      an azide and an alkyne,
      an azide and a cyclooctyne,
      an azide and a cyclooctene,
      an azide and a triaryl phosphine,
      an azide and an oxanorbornadiene,
      a diaryl tetrazine and a cyclooctene,
      a monoaryl tetrazine and a norbornene,
      an aldehyde and a hydroxylamine,
      an aldehyde and a hydrazine,
      an aldehyde and NH₂-NH-C(=O)-,
      a ketone and a hydroxylamine,
      a ketone and a hydrazine,
      a ketone and NH₂-NH-C(=O)-,
      a hydroxylamine and 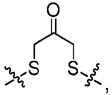
      an amine and 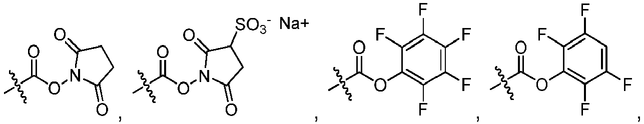 or 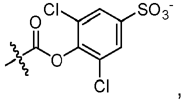 or
      a CoA or CoA analogue and a serine residue; or
(3) the attachment group comprises a group selected from: 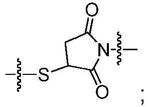 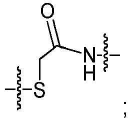 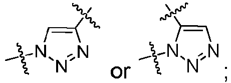 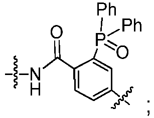 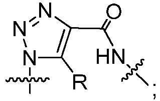 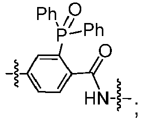 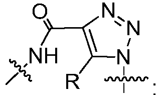 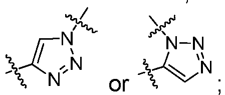 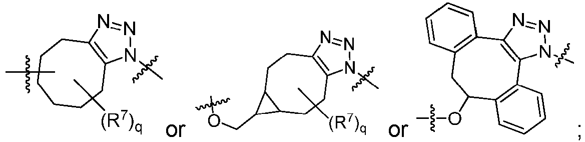 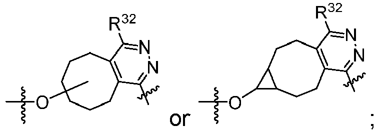 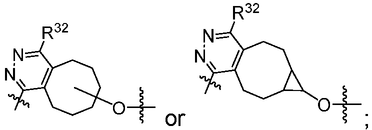 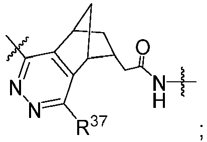 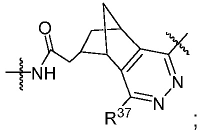 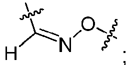 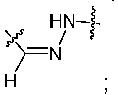 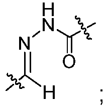 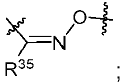 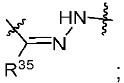 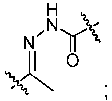 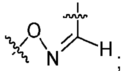 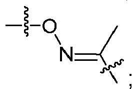 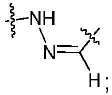 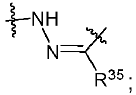 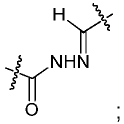 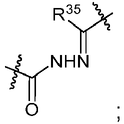 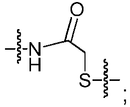 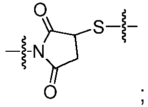 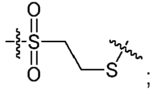 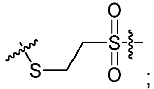 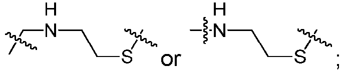 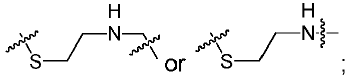 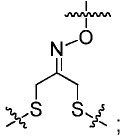 amide; 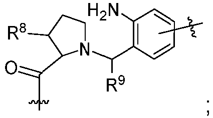 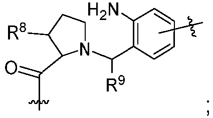 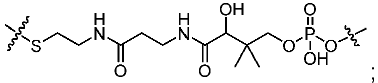 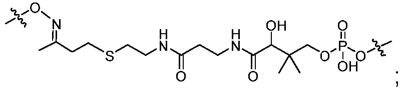 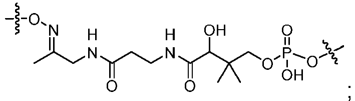 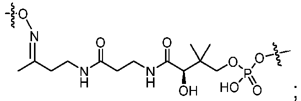 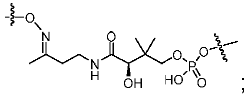 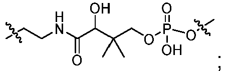 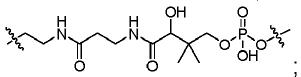 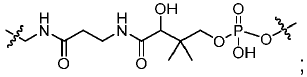 and
   disulfide, wherein:
   R³² is H, C₁₋₄ alkyl, phenyl, pyrimidine or pyridine;
   R³⁵ is H, C₁₋₆ alkyl, phenyl or C₁₋₄ alkyl substituted with 1 to 3 -OH groups;
   each R⁷ is independently selected from H, C₁₋₆ alkyl, fluoro, benzyloxy substituted with -C(=O)OH, benzyl substituted with -C(=O)OH, C₁₋₄ alkoxy substituted with - C(=O)OH and C₁₋₄ alkyl substituted with -C(=O)OH;
   R³⁷ is independently selected from H, phenyl and pyridine;
   q is 0, 1, 2 or 3;
   R⁸ is H or methyl; and
   R⁹ is H, -CH₃ or phenyl.

In an embodiment of the first aspect:
(1) the peptide group represented by Lp comprises 1 to 4 or 1 to 3 or 1 or 2 amino acid residues, optionally the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
(2) the peptide group represented by Lp comprises Val-Cit, Phe-Lys, Val-Ala, Val-Lys, Leu-Cit, sulfo-Ala-Val, and/or sulfo-Ala-Val-Ala; or
(3) Lp is selected from:

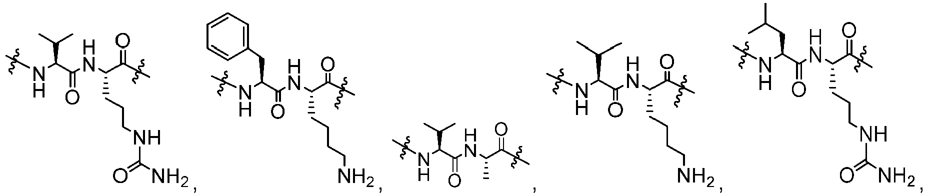

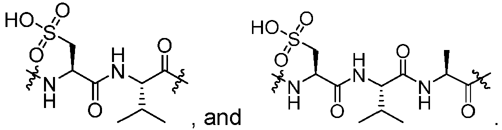

In an embodiment of the first aspect:
-(L-D) comprises or is formed from a compound of formula:
(1) 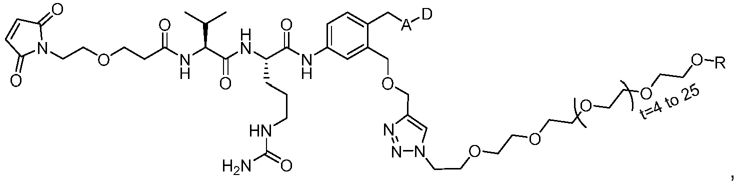 wherein:
   R is H, -CH₃ or -CH₂CH₂C(=O)OH;
   A is a bond, -OC(=O)-*, 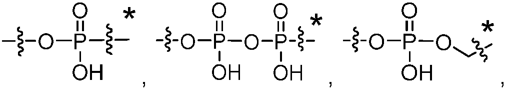 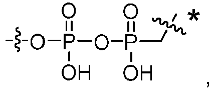 -OC(=O)N(CH₃)CH₂CH₂N(CH₃)C(=O)-* or

      -OC(=O)N(CH₃)C(R^{a})₂C(R^{a})₂N(CH₃)C(=O)-*,

      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(2) 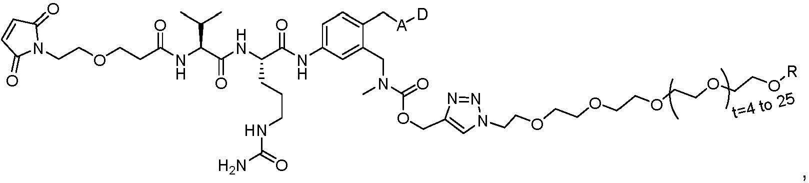 wherein:
   R is H, -CH₃ or -CH₂CH₂C(=O)OH;
   A is a bond, -OC(=O)-*, 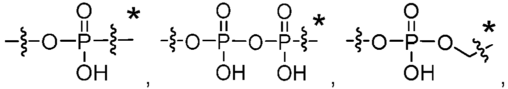 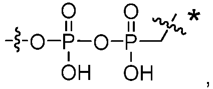 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(3) 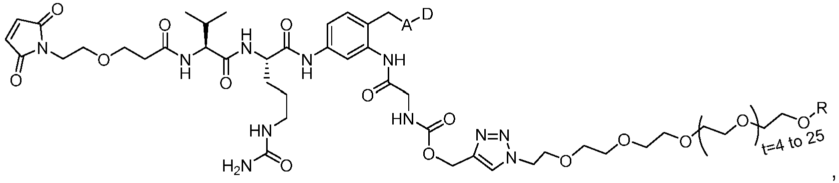 wherein:
   R is H, -CH₃ or -CH₂CH₂C(=O)OH;
   A is a bond, -OC(=O)-*, 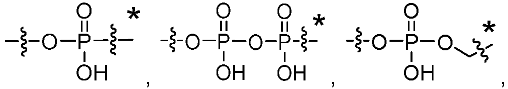 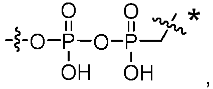 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(4) 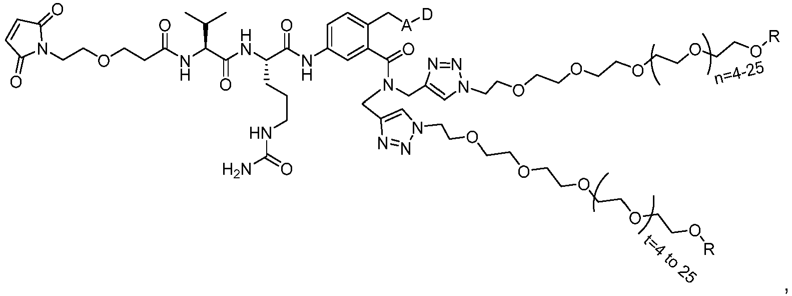 wherein:
   each R is independently selected from H, -CH₃, and -CH₂CH₂C(=O)OH;
   A is a bond, -OC(=O)-*, 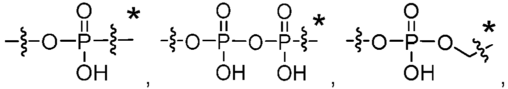 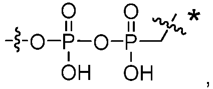 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(5) 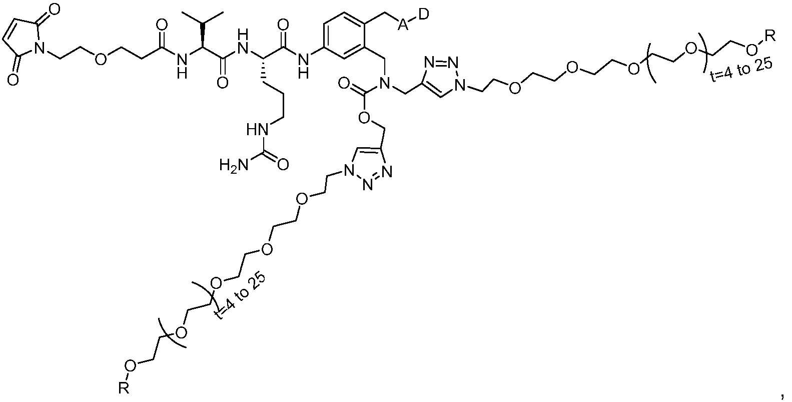 wherein:
   each R is independently selected from H, -CH₃, and -CH₂CH₂C(=O)OH;
   A is a bond, -OC(=O)-*, 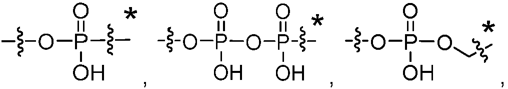 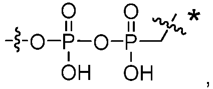 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(6) 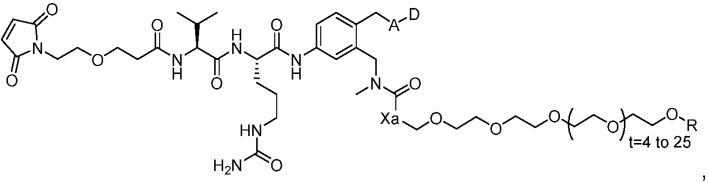 wherein:
   Xa is -CH₂-, -OCH₂-, -NHCH₂- or -NRCH₂- and each R independently is H, -CH₃ or -CH₂CH₂C(=O)OH;
   A is a bond, -OC(=O)-*, 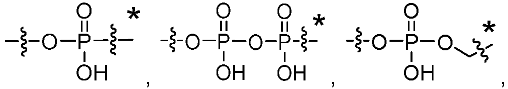 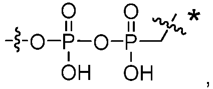 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(7) 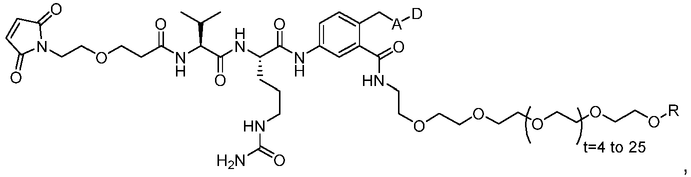 wherein:
   R is H, -CH₃ or -CH₂CH₂C(=O)OH;
   A is a bond, -OC(=O)-*, 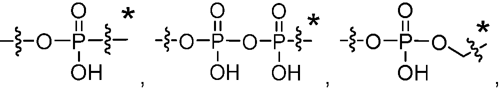 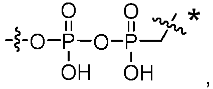 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(8) 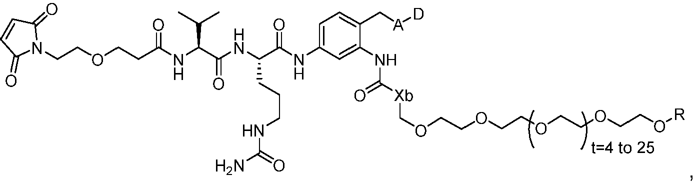 wherein:
   Xb is -CH₂-, -OCH₂-, -NHCH₂- or -NRCH₂- and each R independently is H, -CH₃ or - CH₂CH₂C(=O)OH;
   A is a bond, -OC(=O)-*, 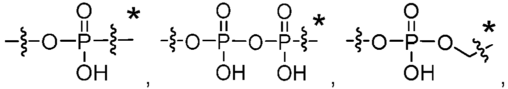 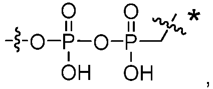 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(9) 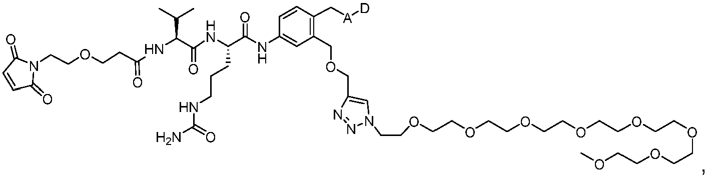 wherein:
   A is a bond, -OC(=O)-*, 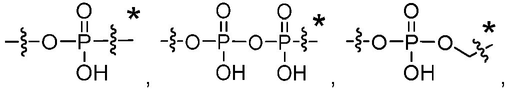 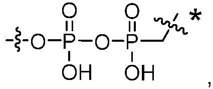 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(10) 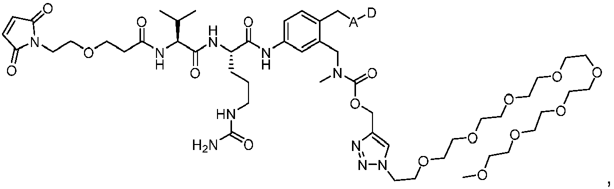 wherein:
   A is a bond, -OC(=O)-*, 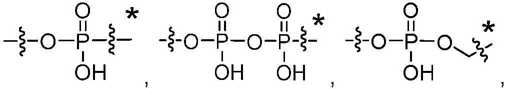 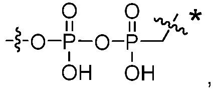 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(11) 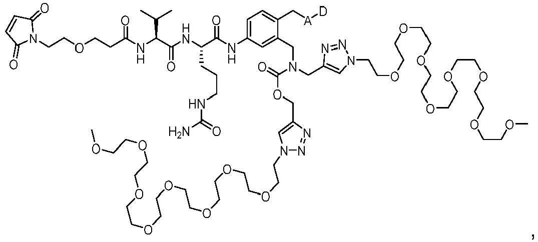 wherein:
   A is a bond, -OC(=O)-*, 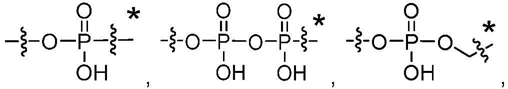 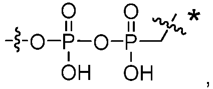 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(12) 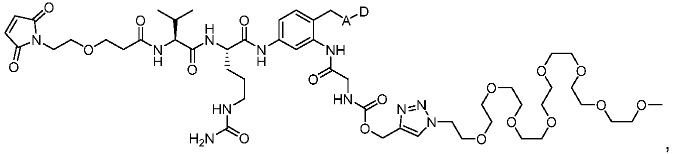 wherein:
   A is a bond, -OC(=O)-*, 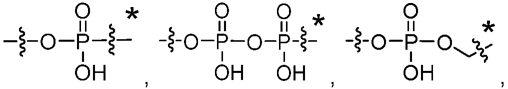 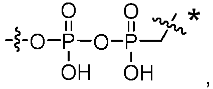 -OC(=O)N(CH₃)CH₂CH₂N(CH₃)C(=O)-* or -OC(=O)N(CH₃)C(R^{a})₂C(R^{a})₂N(CH₃)C(=O)-*,
   wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(13) 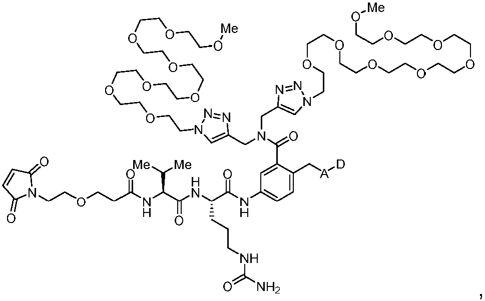 wherein:
   A is a bond, -OC(=O)-*, 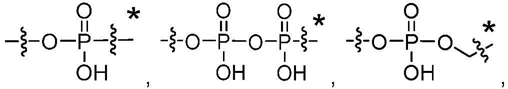 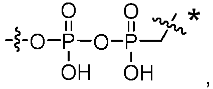 -OC(=O)N(CH₃)CH₂CH₂N(CH₃)C(=O)-* or -OC(=O)N(CH₃)C(R^{a})₂C(R^{a})₂N(CH₃)C(=O)-*,
      wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(14) 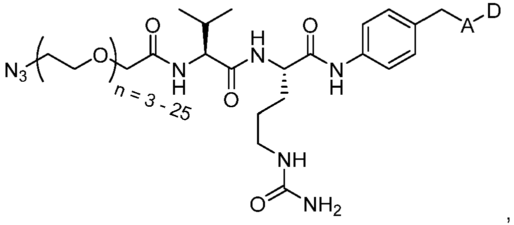 wherein:
   A is a bond, -OC(=O)-*, 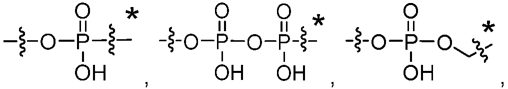 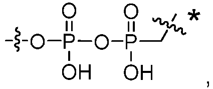 -OC(=O)N(CH₃)CH₂CH₂N(CH₃)C(=O)-* or -OC(=O)N(CH₃)C(R^{a})₂C(R^{a})₂N(CH₃)C(=O)-*,
   wherein each R^{a} is independently selected from H, C₁-C₆ alkyl, and C₃-C₈ cycloalkyl and the * of A indicates the point of attachment to D; and
   D is an Mcl-1 inhibitor as defined in claim 1;
(15) 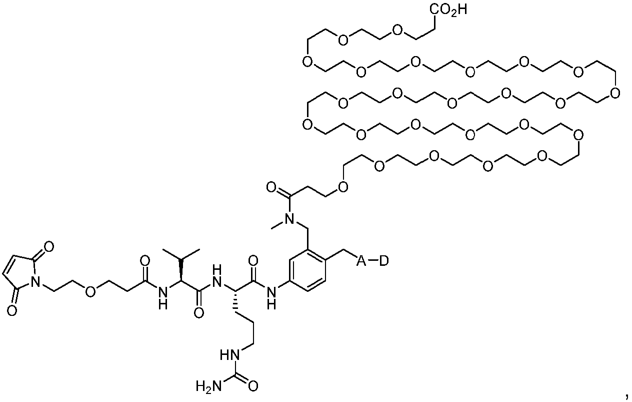 wherein:
   A is a bond, -OC(=O)-*, 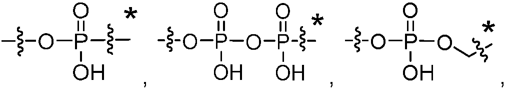 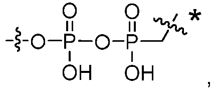 -OC(=O)N(CH₃)CH₂CH₂N(CH₃)C(=O)-* or
   -OC(=O)N(CH₃)C(R^{a})₂C(R^{a})₂N(CH₃)C(=O)-*,
   wherein each R^{a} is independently selected from H, C₁-C₆ alkyl, and C₃-C₈ cycloalkyl and the * of A indicates the point of attachment to D; and
   D is an Mcl-1 inhibitor as defined in claim 1; or
(16) 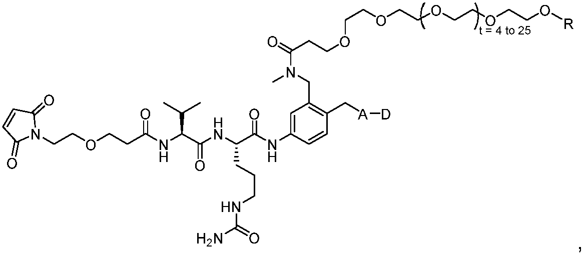 wherein:
   each R independently is H, -CH₃ or -CH₂CH₂C(=O)OH;
   A is a bond, -OC(=O)-*, 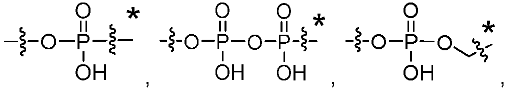 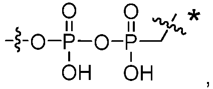 -OC(=O)N(CH₃)CH₂CH₂N(CH₃)C(=O)-* or -OC(=O)N(CH₃)C(R^{a})₂C(R^{a})₂N(CH₃)C(=O)-*,
   wherein each R^{a} is independently selected from H, C₁-C₆ alkyl, and C₃-C₈ cycloalkyl and the * of A indicates the point of attachment to D; and
D is an Mcl-1 inhibitor as defined in claim 1.

In an embodiment of the first aspect, A is a bond and/or R is -CH₃.

In an embodiment of the first aspect, D comprises a compound of Formula (III): 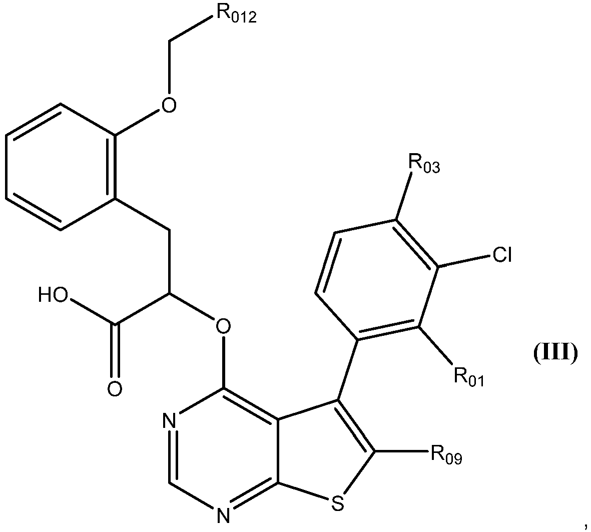
or the enantiomer, diastereoisomer, atropisomer, deuterated derivative, and/or pharmaceutically acceptable salt thereof;
wherein:
   R₀₁ is a linear or branched (C₁-C₆)alkyl group,
   R₀₃ is -O-(C₁-C₆)alkyl-NR₀₁₁R₀₁₁', or 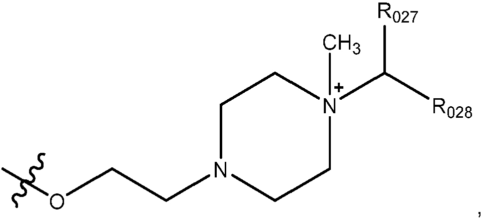
   wherein R₀₁₁ and R₀₁₁' independently of one another are a hydrogen atom, an optionally substituted linear or branched (C₁-C₆)alkyl group, or -(C₀-C₆)alkyl-Cy₀₁;
   or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom may be substituted by 1 or 2 groups selected from a hydrogen atom or a linear or branched (C₁-C₆)alkyl group,
   and wherein R₀₂₇ is a hydrogen atom and R₀₂₈ is a -(CH₂)ₚ₀-O-SO₂-O⁻ group or a -(CH₂)ₚ₀-SO₂-OR₀₃₀ group;
   R₀₉ is a linear or branched (C₂-C₆)alkynyl group or -Cy₀₂,
   R₀₁₂ is -Cy₀₅, -Cy₀₅-(C₀-C₆)alkyl-Cy₀₆, or -Cy₀₅-(C₀-C₆)alkyl-Cy₀₉,
   Cy₀₁, Cy₀₂, Cy₀₅ and Cy₀₆ independently of one another, are a cycloalkyl group, a heterocycloalkyl group, an aryl group or a heteroaryl group, each of which is optionally substituted,
   Cy₀₉ is 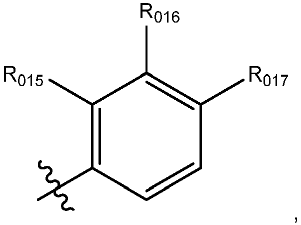
   R₀₁₅, R₀₁₆, and R₀₁₇ are as defined for formula (II),
wherein one of the R₀₃, R₀₉, or R₀₁₂ groups is covalently attached to the linker.

In an embodiment of the first aspect:
(1) R₀₁ is methyl or ethyl;
(2) R₀₃ is -O-CH₂-CH₂-NR₀₁₁R₀₁₁' in which R₀₁₁ and R₀₁₁' form, together with the nitrogen atom carrying them, a piperazinyl group which may be substituted by a group being a hydrogen atom or a linear or branched (C₁-C₆)alkyl group;
(3) R₀₃ comprises the formula: 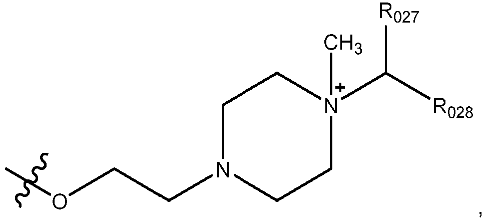 wherein R₀₂₇ is a hydrogen atom and R₀₂₈ is a -(CH₂)ₚ₀-SO₂-OR₀₃₀ group;
(4) R₀₃ comprises the formula: 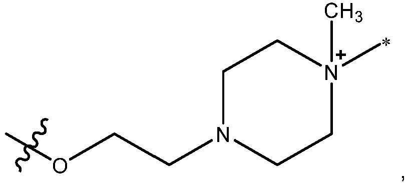 wherein ⁻* is a bond to the linker;
(5) R₀₉ is Cy₀₂;
(6) Cy₀₂ is an optionally substituted aryl group;
(7) Cy₀₅ comprises a heteroaryl group selected from a pyrazolyl group and a pyrimidinyl group;
(8) Cy₀₅ is a pyrimidinyl group; or
(9) the L is attached to D by a covalent bond from L to R₀₃ of formula (II), or (III); and/or the L is attached to D by a covalent bond from L to R₀₉ of formula (II), or (III).

In an embodiment of the first aspect:
(1) D comprises: 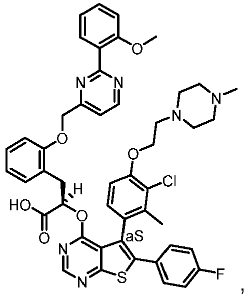 or an enantiomer, diastereoisomer, atropisomer, deuterated derivative, and/or pharmaceutically acceptable salt thereof;
(2) D comprises: 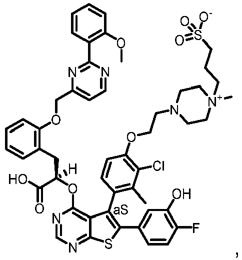 or an enantiomer, diastereoisomer, atropisomer, deuterated derivative, and/or pharmaceutically acceptable salt thereof;
(3) D comprises: 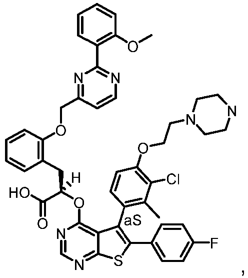 or an enantiomer, diastereoisomer, atropisomer, deuterated derivative, and/or pharmaceutically acceptable salt thereof;
(4) D comprises: 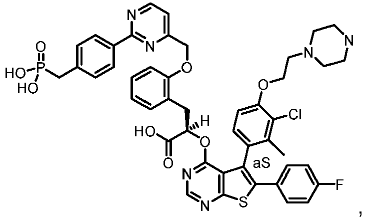 or an enantiomer, diastereoisomer, atropisomer, deuterated derivative, and/or pharmaceutically acceptable salt thereof;
(5) D comprises: 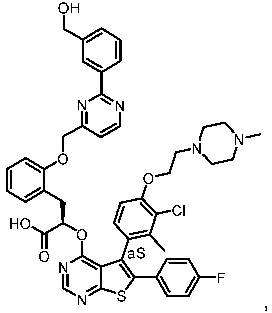 or an enantiomer, diastereoisomer, atropisomer, deuterated derivative, and/or pharmaceutically acceptable salt thereof;
(6) D comprises: 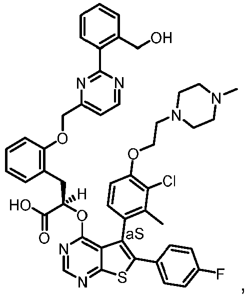 or an enantiomer, diastereoisomer, atropisomer, deuterated derivative, and/or pharmaceutically acceptable salt thereof;
(7) D comprises: 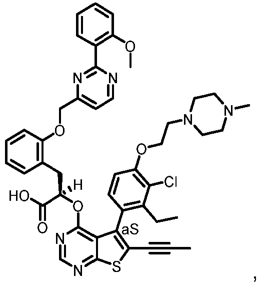 or an enantiomer, diastereoisomer, atropisomer, deuterated derivative, and/or pharmaceutically acceptable salt thereof;
(8) D comprises: 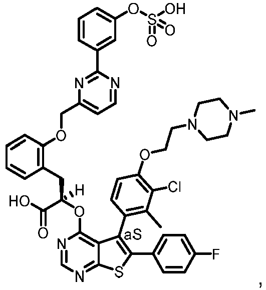 or an enantiomer, diastereoisomer, atropisomer, deuterated derivative, and/or pharmaceutically acceptable salt thereof;
(9) D comprises: 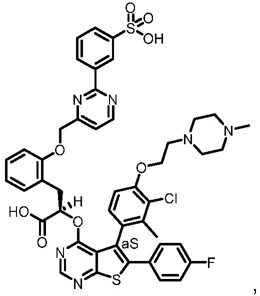 or an enantiomer, diastereoisomer, atropisomer, deuterated derivative, and/or pharmaceutically acceptable salt thereof;
(10) D comprises: 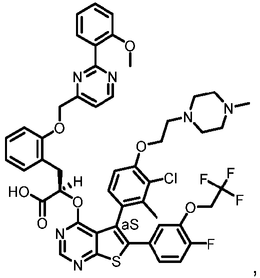 or an enantiomer, diastereoisomer, atropisomer, deuterated derivative, and/or pharmaceutically acceptable salt thereof;
(11) D comprises: 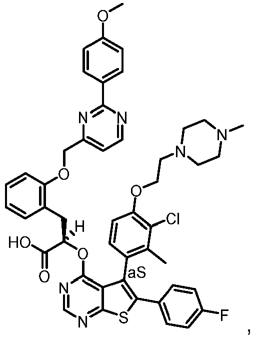 or an enantiomer, diastereoisomer, atropisomer, deuterated derivative, and/or pharmaceutically acceptable salt thereof;
(12) D comprises: 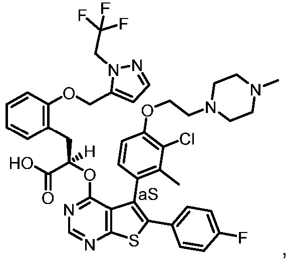 or an enantiomer, diastereoisomer, atropisomer, deuterated derivative, and/or pharmaceutically acceptable salt thereof;
(13) D comprises: 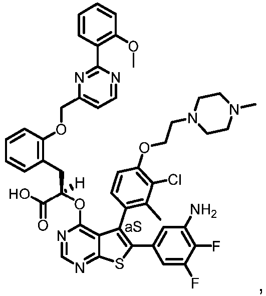 or an enantiomer, diastereoisomer, atropisomer, deuterated derivative, and/or pharmaceutically acceptable salt thereof; or
(14) D comprises: 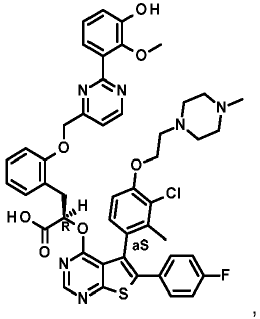 or an enantiomer, diastereoisomer, atropisomer, deuterated derivative, and/or pharmaceutically acceptable salt thereof.

In an embodiment of the first aspect, -(L-D) is formed from a compound in Table A or an enantiomer, diastereoisomer, atropisomer, deuterated derivative, and/or pharmaceutically acceptable salt thereof.

In an embodiment of the first aspect, the antibody or antigen-binding fragment binds to a target antigen on the cancer cell; optionally wherein:
(1)
   (i) the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB;
   (ii) the target antigen is BCMA, CD33, PCAD, HER2, CD38, CD46, CD48, or CD79b; or
   (iii) the target antigen is BCMA, CD33, CD48, PCAD, or HER2;
(2) the antibody or antigen-binding fragment is an anti-BCMA antibody or antigen-binding fragment;
(3) the antibody or antigen-binding fragment comprises:
   (a) three heavy chain complementarity determining regions (HCDRs) comprising amino acid sequences of SEQ ID NO:15 (HCDR1), SEQ ID NO:16 (HCDR2), and SEQ ID NO:17 (HCDR3);
      and three light chain complementarity determining regions (LCDRs) comprising amino acid sequences of SEQ ID NO:18 (LCDR1), SEQ ID NO:19 (LCDR2), and SEQ ID NO:20 (LCDR3); or
   (b) the antibody or antigen-binding fragment comprises a heavy chain variable region comprising an amino acid sequence of SEQ ID NO:1, and a light chain variable region comprising an amino acid sequence of SEQ ID NO:2;
(4)
   (a) the antibody or antigen-binding fragment comprises an IgG1 heavy chain constant domain or a modified IgG1 heavy chain constant domain, optionally the IgG1 heavy chain constant domain comprises a cysteine residue (C) at position 152 and position 375, or the IgG1 heavy chain constant domain comprises a cysteine residue (C) at position 156 and position 379; and/or
   (b) the antibody or antigen-binding fragment comprises an Ig kappa light chain constant domain;
(5) the antibody or antigen-binding fragment is an anti-CD33 antibody or antigen-binding fragment;
(6)
   (a) the antibody or antigen-binding fragment comprises three heavy chain complementarity determining regions (HCDRs) comprising amino acid sequences of SEQ ID NO:21 (HCDR1), SEQ ID NO:22 (HCDR2), and SEQ ID NO:23 (HCDR3); and three light chain complementarity determining regions (LCDRs) comprising amino acid sequences of SEQ ID NO:24 (LCDR1), SEQ ID NO:25 (LCDR2), and SEQ ID NO:26 (LCDR3); and/or
   (b) the antibody or antigen-binding fragment comprises a heavy chain variable region comprising an amino acid sequence of SEQ ID NO:3, and a light chain variable region comprising an amino acid sequence of SEQ ID NO:4;
(7)
   (a) the antibody or antigen-binding fragment comprises an IgG1 heavy chain constant domain or a modified IgG1 heavy chain constant domain, optionally the IgG1 heavy chain constant domain comprises a glutamine (Q) at position 297; and/or
   (b) the antibody or antigen-binding fragment comprises an Ig kappa light chain constant domain;
(8) the antibody or antigen-binding fragment is an anti-PCAD antibody or antigen-binding fragment;
(9)
   (a) the antibody or antigen-binding fragment comprises three heavy chain complementarity determining regions (HCDRs) comprising amino acid sequences of SEQ ID NO:33 (HCDR1), SEQ ID NO:34 (HCDR2), and SEQ ID NO:35 (HCDR3); and three light chain complementarity determining regions (LCDRs) comprising amino acid sequences of SEQ ID NO:36 (LCDR1), SEQ ID NO:37 (LCDR2), and SEQ ID NO:38 (LCDR3); and/or
   (b) the antibody or antigen-binding fragment comprises a heavy chain variable region comprising an amino acid sequence of SEQ ID NO:7, and a light chain variable region comprising an amino acid sequence of SEQ ID NO:8;
(10) the antibody or antigen-binding fragment is an anti-HER2 antibody or antigen-binding fragment;
(11)
   (a) the antibody or antigen-binding fragment comprises three heavy chain complementarity determining regions (HCDRs) comprising amino acid sequences of SEQ ID NO:39 (HCDR1), SEQ ID NO:40 (HCDR2), and SEQ ID NO:41 (HCDR3); and three light chain complementarity determining regions (LCDRs) comprising amino acid sequences of SEQ ID NO:42 (LCDR1), SEQ ID NO:43 (LCDR2), and SEQ ID NO:44 (LCDR3); and/or
   (b) the antibody or antigen-binding fragment comprises a heavy chain variable region comprising an amino acid sequence of SEQ ID NO:9, and a light chain variable region comprising an amino acid sequence of SEQ ID NO:10;
(12)
   (a) the antibody or antigen-binding fragment comprises an IgG1 heavy chain constant domain or a modified IgG1 heavy chain constant domain, optionally the IgG1 heavy chain constant domain comprises a glutamine (Q) at position 297 or the IgG1 heavy chain constant domain comprises a serine (S) at position 297; and/or
   (b) the antibody or antigen-binding fragment comprises an Ig kappa light chain constant domain; or
(13) the antibody or antigen-binding fragment is an anti-CD38 antibody or antigen-binding fragment; an anti-CD46 antibody or antigen-binding fragment; an anti-CD48 antibody or antigen-binding fragment; or an anti-CD79b antibody or antigen-binding fragment.

In some embodiments, Ab is an antibody or an antigen-binding fragment thereof that targets a cancer cell.

In some embodiments, *p is* an integer from 1 to 8. In some embodiments, p is an integer from 1 to 5. In some embodiments, p is an integer from 2 to 4. In some embodiments, *p* is 2. In some embodiments, p is 4. In some embodiments, *p* is determined by liquid chromatography-mass spectrometry (LC-MS).

In some embodiments, the linker (L) comprises an attachment group, at least one spacer group, and at least one cleavable group. In some cases, the cleavable group comprises a pyrophosphate group and/or a self-immolative group. In specific embodiments, L comprises an attachment group; at least one bridging spacer group; and at least one cleavable group comprising a pyrophosphate group and/or a self-immolative group.

In some embodiments, the antibody-drug conjugate comprises a linker-drug (or "linker-payload") moiety -(L-D) is of the formula (A): 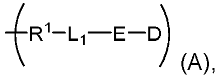 wherein R¹ is an attachment group, L₁ is a bridging spacer group, and E is a cleavable group.

In some embodiments, the cleavable group comprises a pyrophosphate group. In some embodiments, the cleavable group comprises:

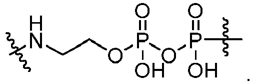

In some embodiments, the bridging spacer group comprises a polyoxyethylene (PEG) group. In some cases, the PEG group may be selected from PEG1, PEG2, PEG3, PEG4, PEG5, PEG3, PEG7, PEG3, PEG9, PEG10, PEG11, PEG12, PEG13, PEG14, and PEG15. In some embodiments, the bridging spacer group may comprise: -CO-CH₂-CH₂-PEG12-. In other embodiments, the bridging spacer group comprises a butanoyl, pentanoyl, hexanoyl, heptanoyl, or octanoyl group. In some embodiments, the bridging spacer group comprises a hexanoyl group.

In some embodiments the attachment group is formed from at least one reactive group selected from a maleimide group, thiol group, cyclooctyne group, and an azido group. For example, maleimide group may have the structure:

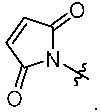

The azido group may have the structure: -N=N⁺=N⁻.

The cyclooctyne group may have the structure: 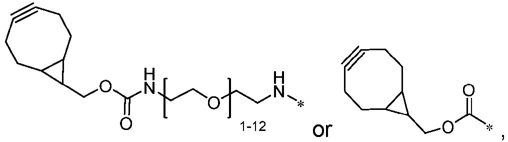 and wherein⁻* is a bond to the antibody.

In some cases, the cyclooctyne group has the structure: 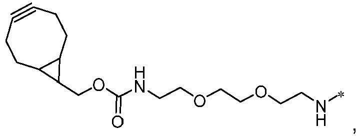 and wherein ⁻* is a bond to the antibody.

In some embodiments, the attachment group has a formula comprising 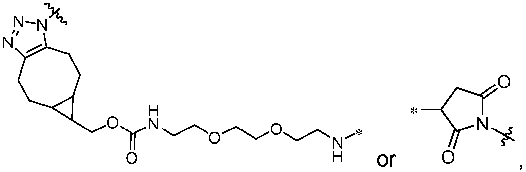 and wherein ⁻* is a bond to the antibody.

In some embodiments, the antibody is joined to the linker (L) by an attachment group selected from: 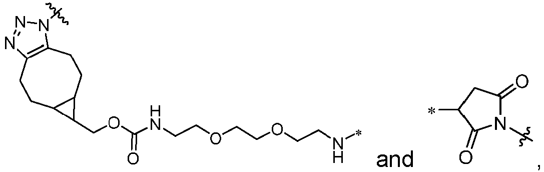 wherein ⁻* is a bond to the antibody, and wherein 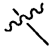 is a bond to the bridging spacer group.

In some embodiments, the bridging spacer group is joined to a cleavable group.

In some embodiments, the bridging spacer group is -CO-CH₂-CH₂-PEG12-.

In some embodiments, the cleavable group is -pyrophosphate-CH₂-CH₂-NH₂-.

In some embodiments, the cleavable group is joined to the Mcl-1 inhibitor (D).

In some embodiments, the cleavable group is joined to the Mcl-1 inhibitor (D) group through a phenyl-pyrimidinyl group.

In some embodiments, the linker comprises: an attachment group, at least one bridging spacer group, a peptide group, and at least one cleavable group.

In some embodiments, the antibody-drug conjugate comprises a linker-drug moiety, -(L-D), is of the formula (B): 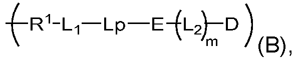 wherein R¹ is an attachment group, L₁ is a bridging spacer, Lp is a peptide group comprising 1 to 6 amino acid residues, E is a cleavable group, L₂ is a bridging spacer, m is 0 or 1; and D is an Mcl-1 inhibitor. In some cases, m is 1 and the bridging spacer comprises:

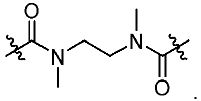

In some embodiments, the at least one bridging spacer comprises a PEG group. In some cases, the PEG group is selected from, PEG1, PEG2, PEG3, PEG4, PEG5, PEG3, PEG7, PEG8, PEG9, PEG10, PEG11, PEG12, PEG13, PEG14, and PEG15. In some cases, the at least one bridging spacer is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, a polyhydroxyalkyl group, and *-C(O)-N(CH₃)-CH₂-CH₂-N(CH₃)-C(O)-**, wherein ** indicates the point of direct or indirect attachment of the at least one bridging spacer to the attachment group and * indicates the point of direct or indirect attachment of the at least one bridging spacer to the peptide group..

In some embodiments, L₁ is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, and a polyhydroxyalkyl group, wherein ** indicates the point of direct or indirect attachment of L₁ to R¹ and * indicates the point of direct or indirect attachment of L₁ to Lp.

In some embodiments, m is 1 and L₂ is -C(O)-N(CH₃)-CH₂-CH₂-N(CH₃)-C(O)-.

In some embodiments, the peptide group comprises 1 to 12 amino acid residues. In some embodiments, the peptide group (Lp) comprises 1 to 10 amino acid residues. In some embodiments, the peptide group (Lp) comprises 1 to 8 amino acid residues. In some embodiments, the peptide group (Lp) comprises 1 to 6 amino acid residues. In some embodiments, the peptide group comprises 1 to 4 amino acid residues. In some embodiments, the peptide group comprises 1 to 3 amino acid residues. In some embodiments the peptide group comprises 1 to 2 amino acid residues. In some cases, the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr). For example, the peptide group may comprise Val-Cit, Val-Ala, Val-Lys, and/or sulfo-Ala-Val-Ala. In some embodiments, the peptide group (Lp) comprises 1 amino acid residue linked to a 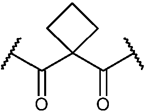 group. In some embodiments, the peptide group (Lp) comprises a group :

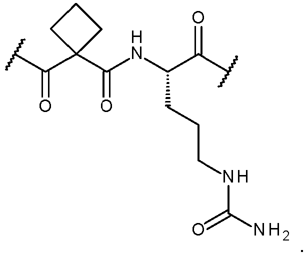

In some cases, the peptide group comprises a group selected from:

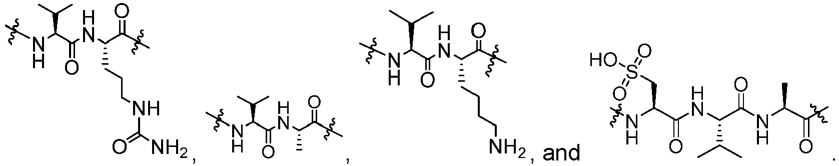

In some embodiments, the self-immolative group comprises para-aminobenzyl-carbamate, para-aminobenzyl-ammonium, para-amino-(sulfo)benzyl-ammonium, para-amino-(sulfo)benzyl-carbamate, para-amino-(alkoxy-PEG-alkyl)benzyl-carbamate, para-amino-(polyhydroxycarboxytetrahydropyranyl)alkyl-benzyl-carbamate, or para-amino-(polyhydroxycarboxytetrahydropyranyl)alkyl-benzyl-ammonium.

In some embodiments, m is 1 and the bridging spacer comprises

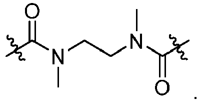

In some embodiments, the linker-drug moiety, -(L-D), is formed from a compound selected from: 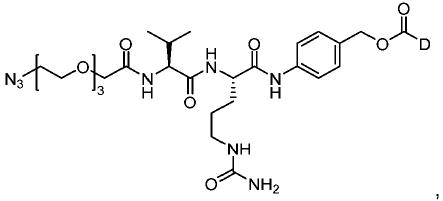 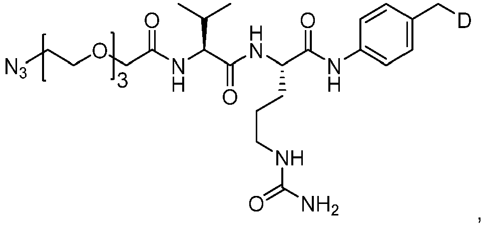 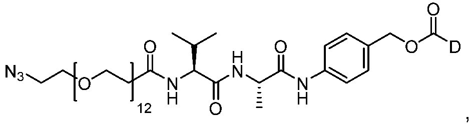 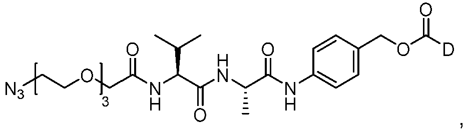 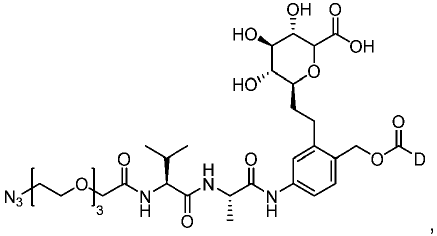 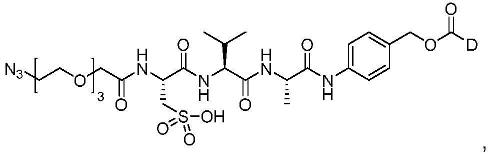 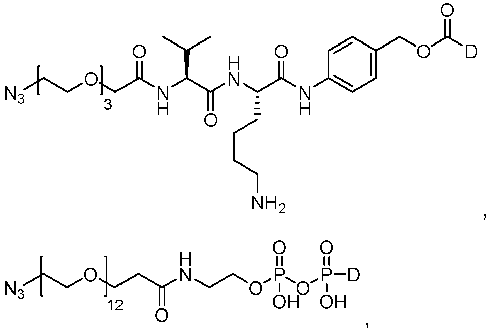 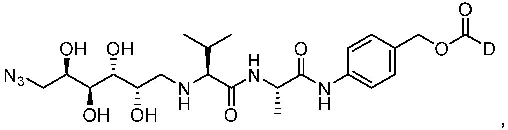 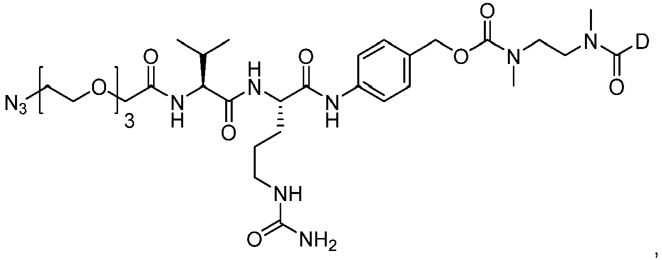 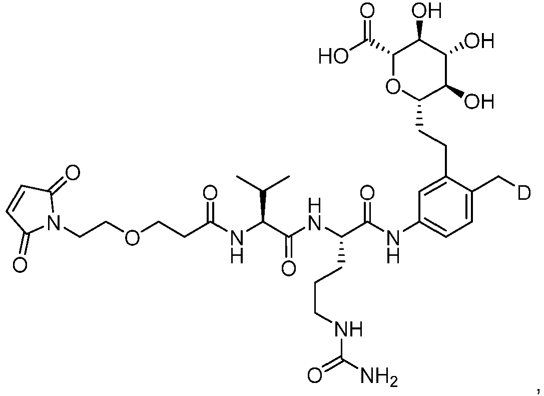 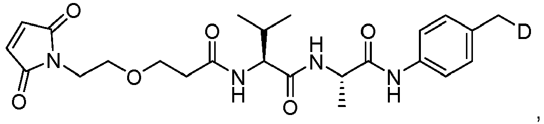 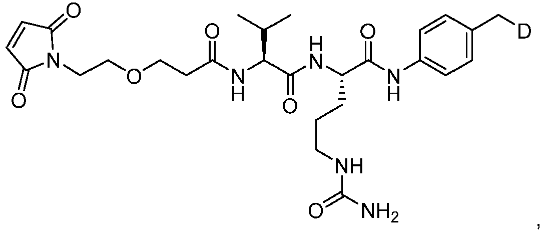 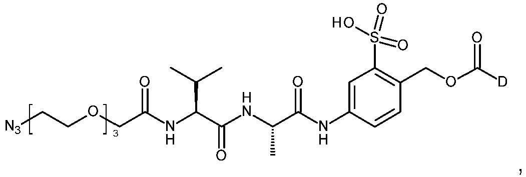 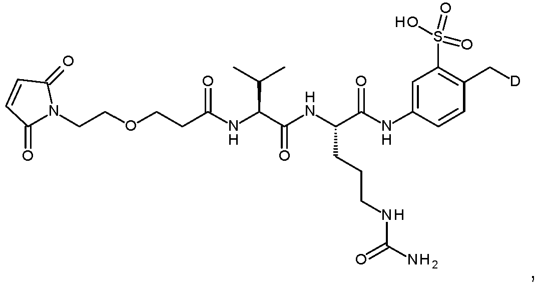 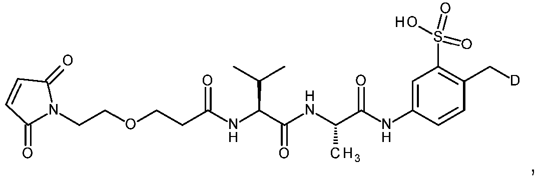 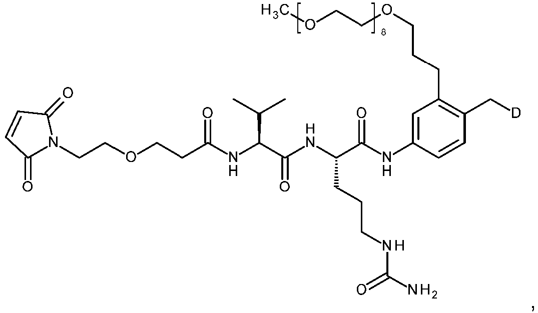 and

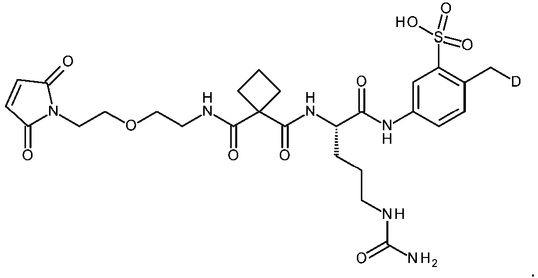

In some embodiments, the antibody-drug conjugate comprises the linker-drug group, -(L-D), which comprises a formula selected from: 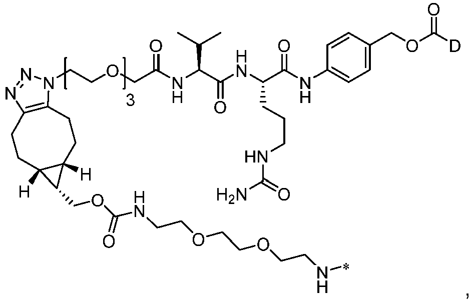 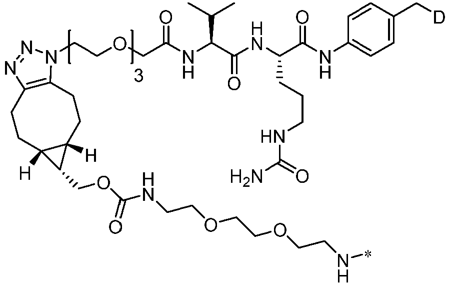 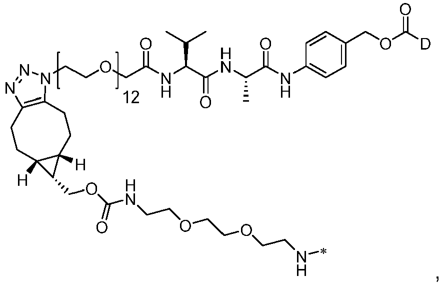 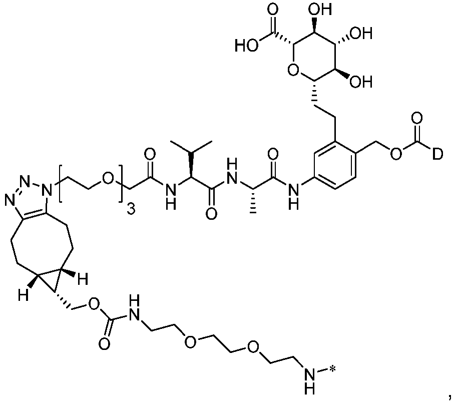 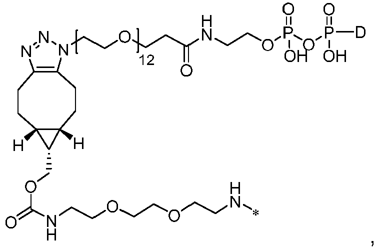 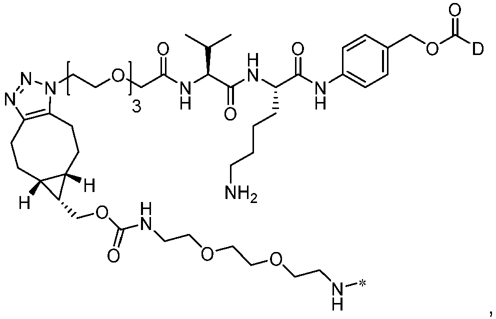 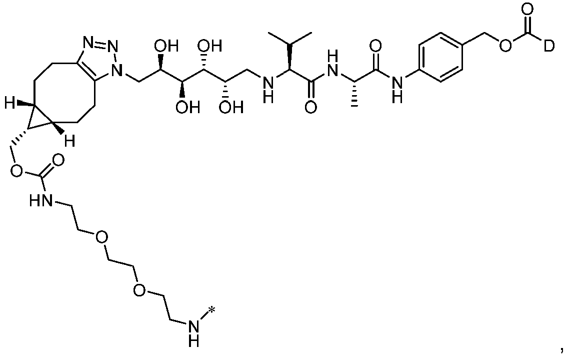 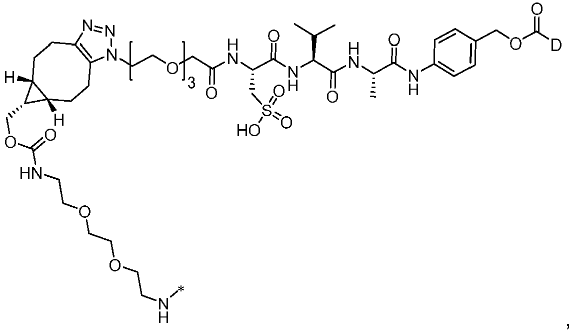 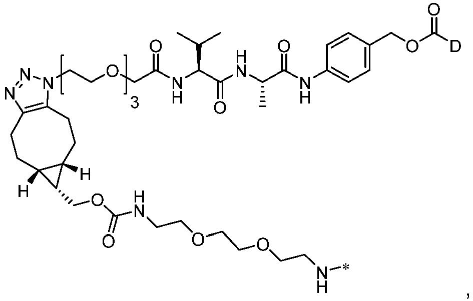 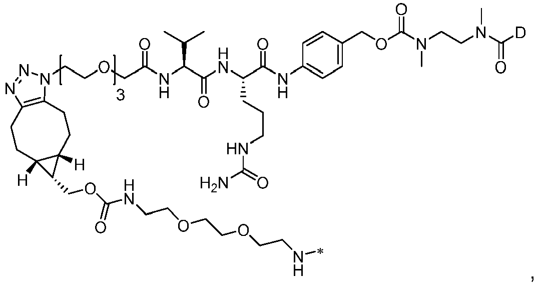 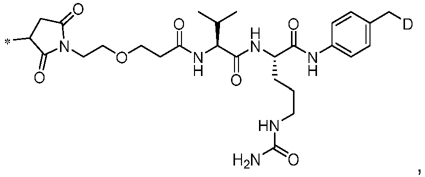 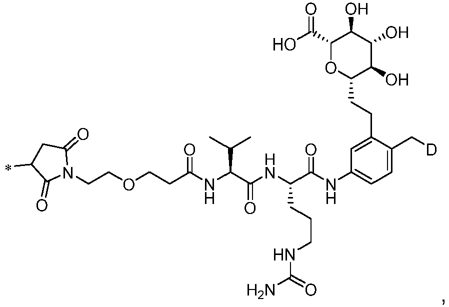 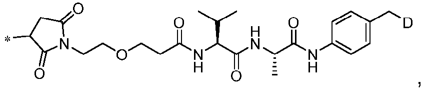 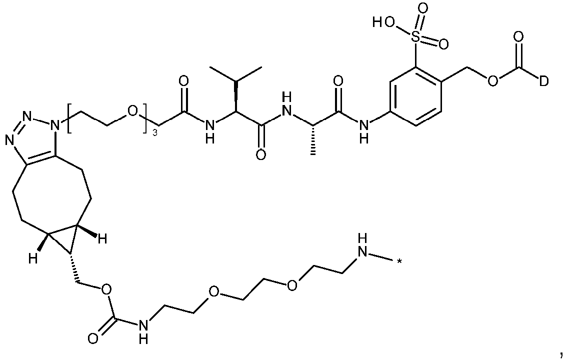 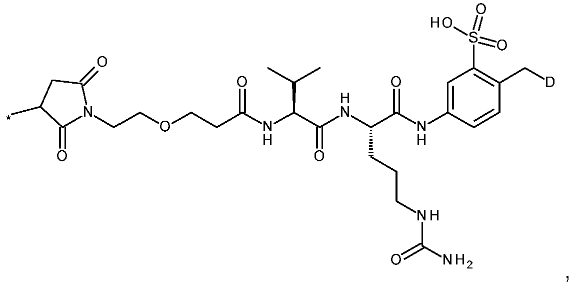 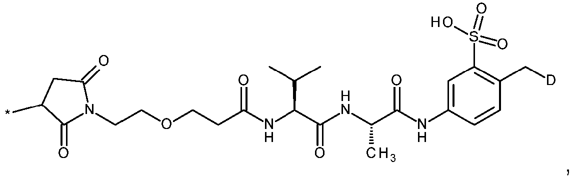 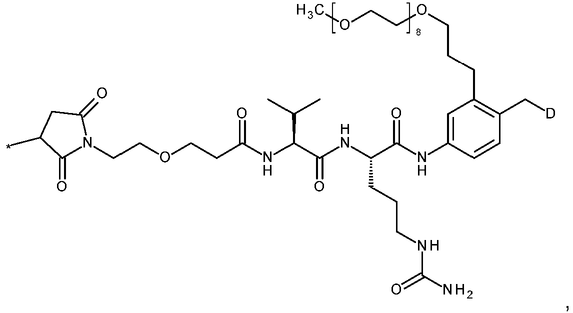 and 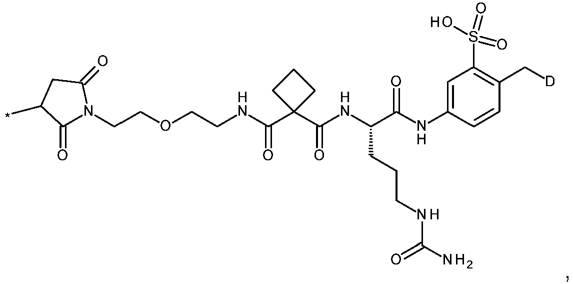 and
wherein ⁻* is a bond to the antibody.

In some embodiments, the antibody-drug conjugate comprises the linker drug group, -(L-D), which is of the formula (C): 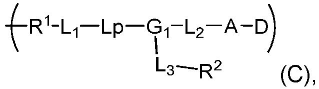 wherein: R¹ is an attachment group, L₁ is a bridging spacer; Lₚ is a peptide group comprising 1 to 6 amino acids; D is an Mcl-1 inhibitor; G₁-L₂-A is a self-immolative spacer; L₂ is a bond, a methylene, a neopentylene or a C₂-C₃ alkenylene; A is a bond, -OC(=O)-*, 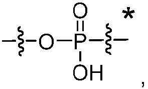 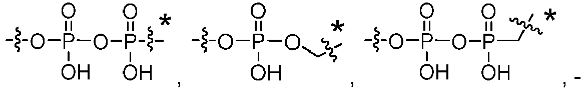 OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; L₃ is a spacer moiety; and R² is a hydrophilic moiety.

In some embodiments, the antibody-drug conjugate comprises the linker drug group, -(L-D), which is of the formula (D): 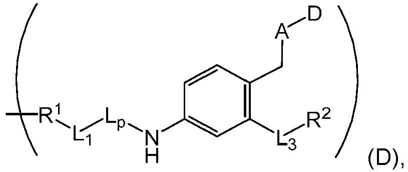 wherein: R¹ is an attachment group; L₁ is a bridging spacer; Lp is a peptide group comprising 1 to 6 amino acids; A is a bond, -OC(=O)-*, 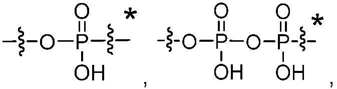 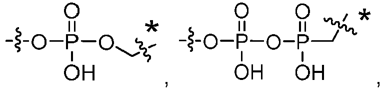 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; L₃ is a spacer moiety; and R² is a hydrophilic moiety.

In some embodiments, L₁ comprises: 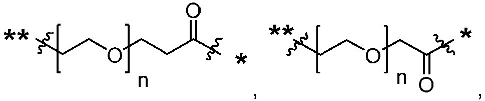 or *-CH(OH)CH(OH)CH(OH)CH(OH)-**,wherein each n is an integer from 1 to 12, wherein the * of L₁ indicates the point of direct or indirect attachment to Lp, and the ** of L₁ indicates the point of direct or indirect attachment to R¹.

In some embodiments, L₁ is 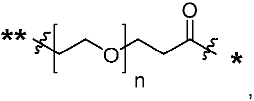 and n is an integer from 1 to 12 wherein the * of L₁ indicates the point of direct or indirect attachment to Lp, and the ** of L₁ indicates the point of direct or indirect attachment to R¹.

In some embodiments, L₁ is 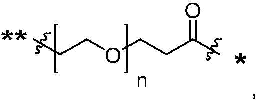 and n is 1, wherein the * of L₁ indicates the point of direct or indirect attachment to Lp, and the ** of L₁ indicates the point of direct or indirect attachment to R¹.

In some embodiments, L₁ is 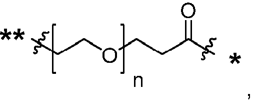 and n is 12, wherein the * of L₁ indicates the point of direct or indirect attachment to Lp, and the ** of L₁ indicates the point of direct or indirect attachment to R¹.

In some embodiments, L₁ is 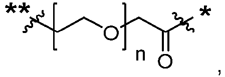 and n is an integer from 1 to 12, wherein the * of L₁ indicates the point of direct or indirect attachment to Lp, and the ** of L₁ indicates the point of direct or indirect attachment to R¹.

In some embodiments, L₁ comprises 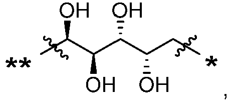 wherein the * of L₁ indicates the point of direct or indirect attachment to Lp, and the ** of L₁ indicates the point of direct or indirect attachment to R¹.

In some embodiments, L₁ is a bridging spacer comprising:
*-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH₂)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH₂)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of direct or indirect attachment to Lp, and the ** of L₁ indicates the point of direct or indirect attachment to R¹,
wherein X₁ is 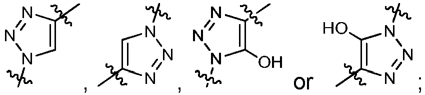 and
each m is independently selected from 1, 2, 3, 4, 5, 6, 7, 8, 9 and 10;
each n is independently selected from 1, 2, 3, 4, 5, 6, 7, 8, 9 and 10; and
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30.

In some embodiments, R² is a hydrophilic moiety comprising polyethylene glycol, polyalkylene glycol, a polyol, a polysarcosine, a sugar, an oligosaccharide, a polypeptide, or C₂-C₆ alkyl substituted with 1 to 3 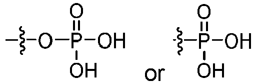 groups. In some embodiments, R² is 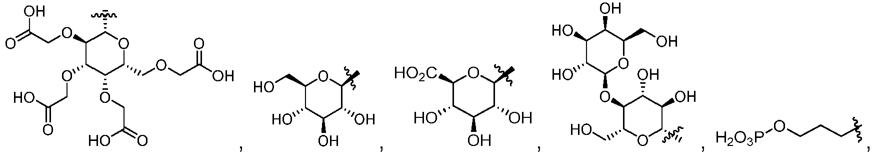 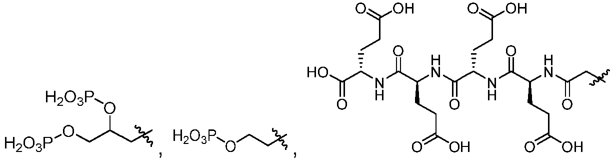 or 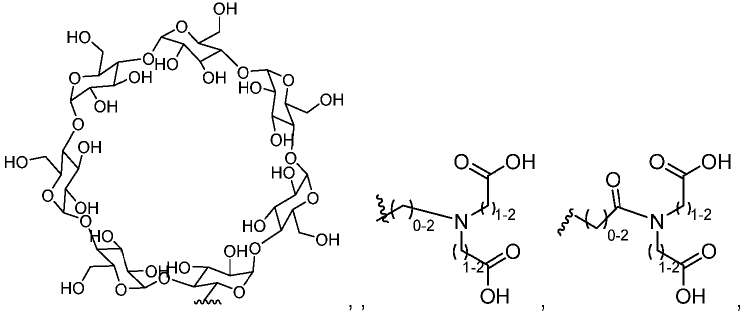 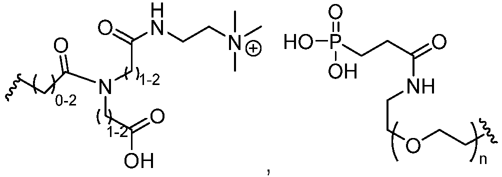 wherein n is an interger between 1 and 6, 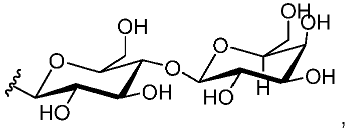 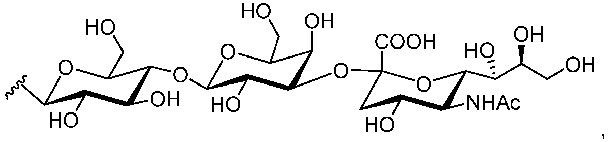 or

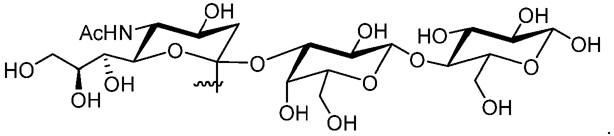

In some embodiments, the hydrophilic moiety comprises a polyethylene glycol of formula: 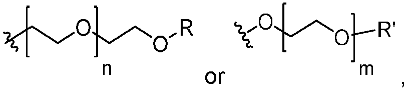 wherein R is H, -CH₃ CH₂CH₂NHC(=O)ORₐ, -CH₂CH₂NHC(=O)Rₐ, or or -CH₂CH₂C(=O)ORₐ, R' is OH, -OCH₃, CH₂CH₂NHC(=O)ORₐ, -CH₂CH₂NHC(=O)Rₐ, or -OCH₂CH₂C(=O)ORₐ, and each of m and n is an integer between 2 and 25 (*e.g*., between 3 and 25).

In some embodiments, the hydrophilic moiety comprises

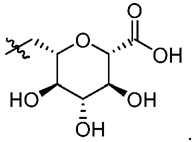

In some embodiments, the hydrophilic moiety comprises a polysarcosin, e.g., with the following moiety 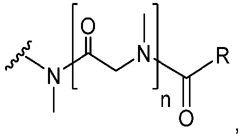 wherein n is an integer between 3 and 25; and R is H, -CH₃ or - CH₂CH₂C(=O)OH.

In some embodiments, L₃ is a spacer moiety having the structure 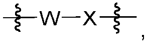 wherein:
W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, -CH₂N(X-R²)C(=O)-, -C(=O)NR^{b}-, -C(=O)NH-, -CH₂NR^{b}C(=O)-, -CH₂NR^{b}C(=O)NH-, -CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
X is a bond, triazolyl or -CH₂-triazolyl-.

In some embodiments, L₃ is a spacer moiety having the structure 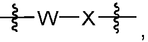 wherein:
W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, -CH₂N(X-R²)C(=O)-, -C(=O)NR^{b}-, -C(=O)NH-, -CH₂NR^{b}C(=O)-, -CH₂NR^{b}C(=O)NH-, -CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
X is -CH₂-triazolyl-C₁₋₄ alkylene-OC(O)NHS(O)₂NH-, -C₄₋₆ cycloalkylene-OC(O)NHS(O)₂NH-, -(CH₂CH₂O)ₙ-C(O)NHS(O)₂NH-, -(CH₂CH₂O)ₙ-C(O)NHS(O)₂NH-(CH₂CH₂O)ₙ-, or -CH₂-triazolyl-C₁₋₄ alkylene-OC(O)NHS(O)₂NH-(CH₂CH₂O)ₙ-, wherein each n independently is 1, 2, or 3.

In some embodiments, the attachment group is formed by a reaction comprising at least one reactive group. In some cases, the attachment group is formed by reacting: a first reactive group that is attached to the linker, and a second reactive group that is attached to the antibody or is an amino acid residue of the antibody.

In some embodiments, at least one of the reactive groups comprises:
a thiol,
a maleimide,
a haloacetamide,
an azide,
an alkyne,
a cyclcooctene,
a triaryl phosphine,
an oxanorbornadiene,
a cyclooctyne,
a diaryl tetrazine,
a monoaryl tetrazine,
a norbornene,
an aldehyde,
a hydroxylamine,
a hydrazine,
NH₂-NH-C(=O)-,
a ketone,
a vinyl sulfone,
an aziridine,
an amino acid residue, 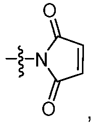 -ONH₂, -NH₂, 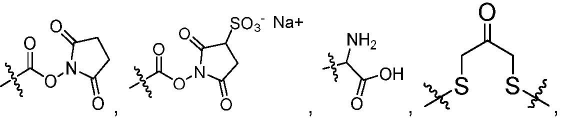 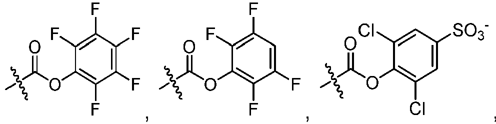 -N₃, 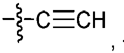 -SH, -SR³, -SSR⁴, -S(=O)₂(CH=CH₂), -(CH₂)₂S(=O)₂(CH=CH₂), -NHS(=O)₂(CH=CH₂), -NHC(=O)CH₂Br, -NHC(=O)CH₂I, 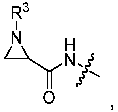 -C(O)NHNH₂, 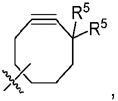 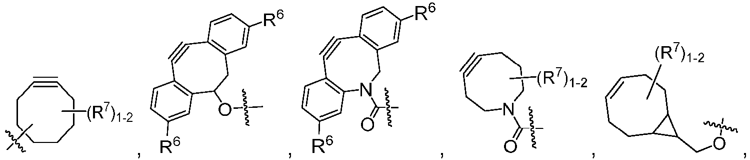 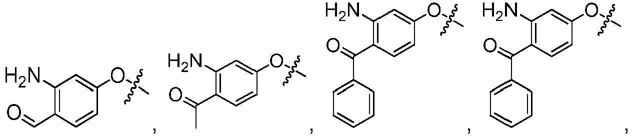 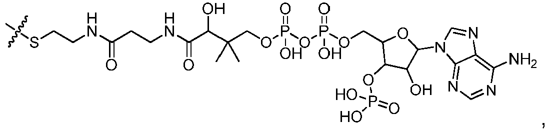 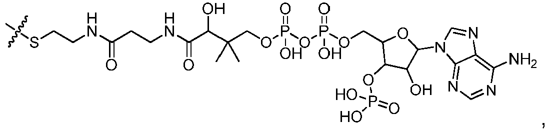 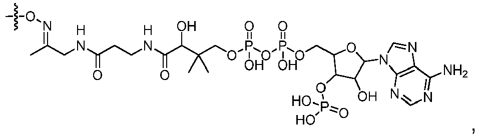 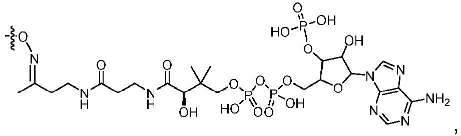 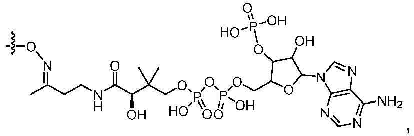 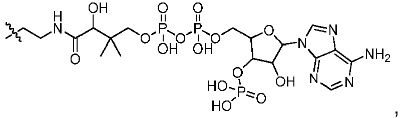 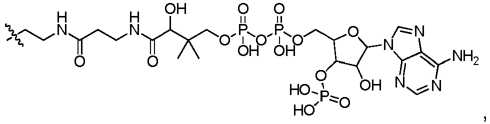 or 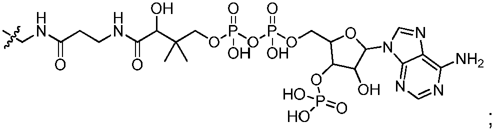
wherein:
each R³ is independently selected from H and C₁-C₆alkyl;
each R⁴ is 2-pyridyl or 4-pyridyl;
each R⁶ is independently selected from H, C₁-C₆alkyl, F, Cl, and -OH;
each R⁶ is independently selected from H, C₁-C₆alkyl, F, Cl, -NH₂, -OCH₃, -OCH₂CH₃, - N(CH₃)₂, -CN, -NO₂ and-OH;
each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH.

In some embodiments, the first reactive group and second reactive group comprise:
a thiol and a maleimide,
a thiol and a haloacetamide,
a thiol and a vinyl sulfone,
a thiol and an aziridine,
an azide and an alkyne,
an azide and a cyclooctyne,
an azide and a cyclooctene,
an azide and a triaryl phosphine,
an azide and an oxanorbornadiene,
a diaryl tetrazine and a cyclooctene,
a monoaryl tetrazine and a norbornene,
an aldehyde and a hydroxylamine,
an aldehyde and a hydrazine,
an aldehyde and NH₂-NH-C(=O)-,
a ketone and a hydroxylamine,
a ketone and a hydrazine,
a ketone and NH2-NH-C(=O)-,
a hydroxylamine and 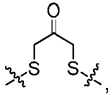
an amine and 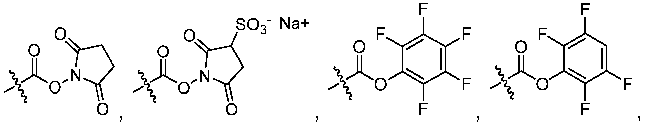 or 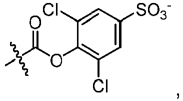 or
a CoA or CoA analogue and a serine residue.

In some embodiments, the attachment group comprises a group selected from: 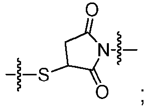 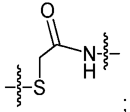 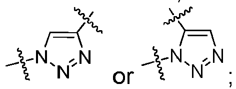 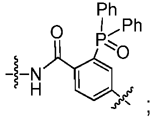 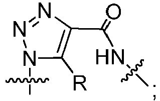 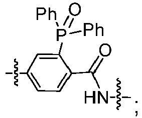 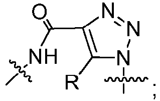 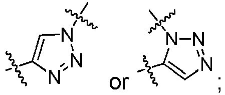 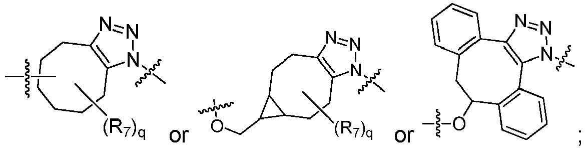 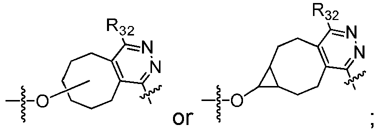 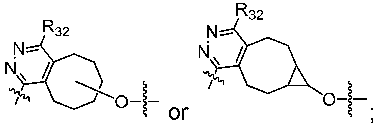 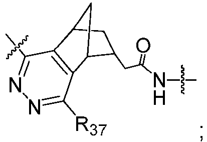 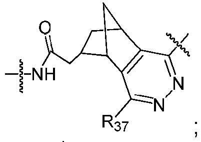 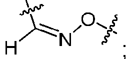 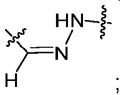 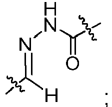 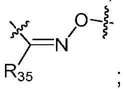 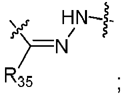 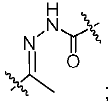 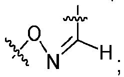 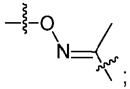 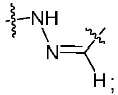 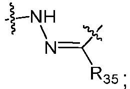 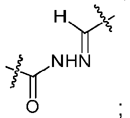 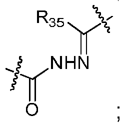 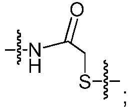 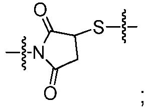 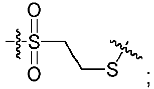 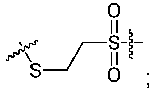 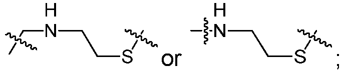 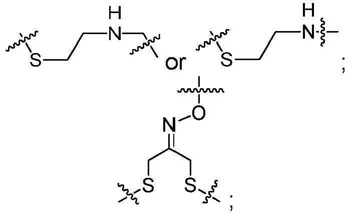 amide; 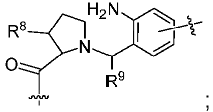 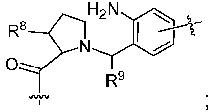 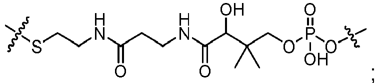 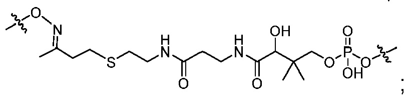 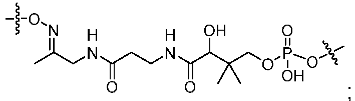 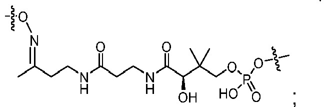 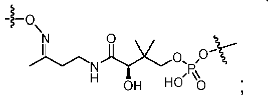 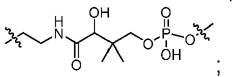 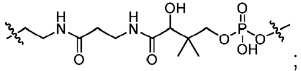 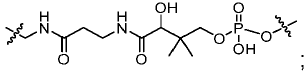 and
disulfide,
wherein:
R³² is H, C₁₋₄ alkyl, phenyl, pyrimidine or pyridine;
R³⁵ is H, C₁₋₆ alkyl, phenyl or C₁₋₄ alkyl substituted with 1 to 3 -OH groups;
each R⁷ is independently selected from H, C₁₋₆ alkyl, fluoro, benzyloxy substituted with -C(=O)OH, benzyl substituted with -C(=O)OH, C₁₋₄ alkoxy substituted with -C(=O)OH and C₁₋₄ alkyl substituted with -C(=O)OH;
R³⁷ is independently selected from H, phenyl and pyridine;
q is 0, 1, 2 or 3;
R⁸ is H or methyl; and
R⁹ is H, -CH₃ or phenyl.

In some embodiments, the peptide group (Lp) comprises 1 to 6 amino acid residues. In some embodiments, the peptide group (Lp) comprises 1 to 4 amino acid residues. In some embodiments, the peptide group comprises 1 to 3 amino acid residues. In some embodiments, the peptide group comprises 1 to 2 amino acid residues. In some embodiments, the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr). In some embodiments, the peptide group comprises Val-Cit, Phe-Lys, Val-Ala, Val-Lys, Leu-Cit, sulfo-Ala-Val, and/or sulfo-Ala-Val-Ala. In some embodiments, Lp is selected from:

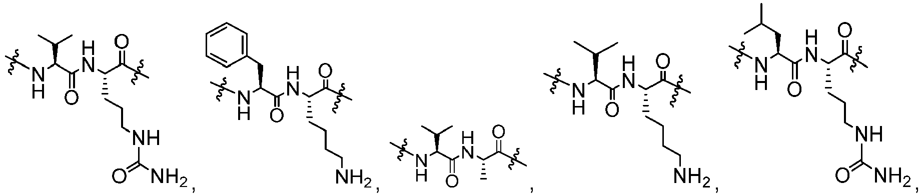

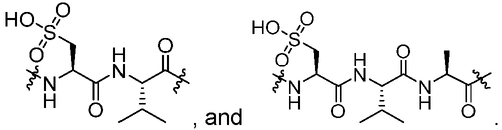

In some embodiments, the linker-drug group -(L-D) comprises or is formed from a compound of formula: 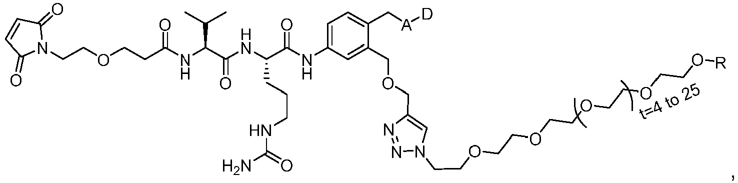 wherein:
R is H, -CH₃ or -CH₂CH₂C(=O)OH;
A is a bond, -OC(=O)-*, 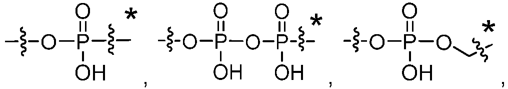 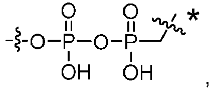 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 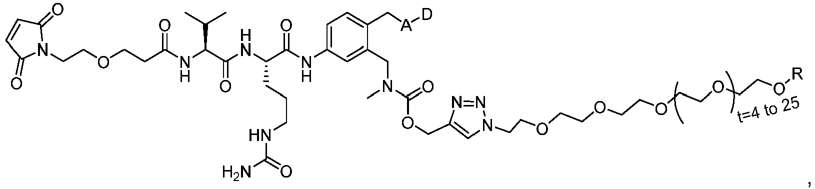 wherein:
R is H, -CH₃ or -CH₂CH₂C(=O)OH;
A is a bond, -OC(=O)-*, 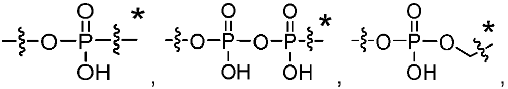 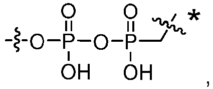 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 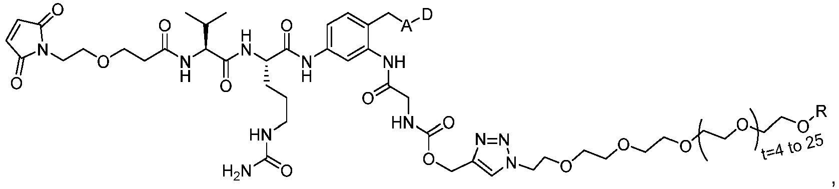 wherein:
R is H, -CH₃ or -CH₂CH₂C(=O)OH;
A is a bond, -OC(=O)-*, 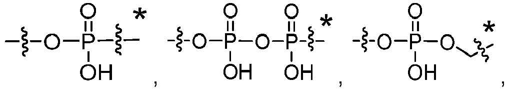 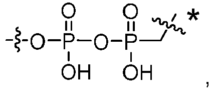 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 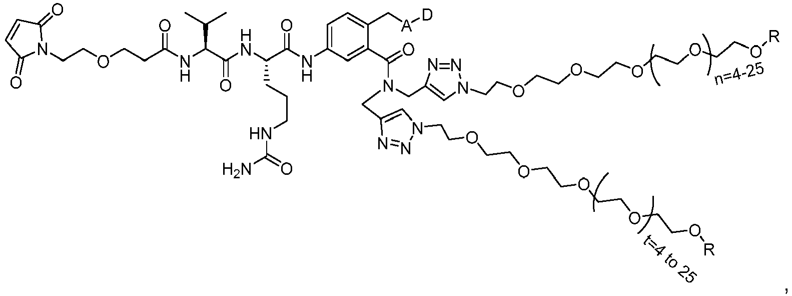 wherein:
each R is independently selected from H, -CH₃, and -CH₂CH₂C(=O)OH;
A is a bond, -OC(=O)-*, 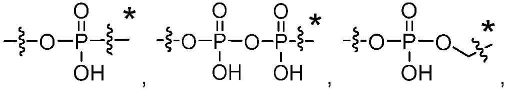 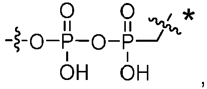 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 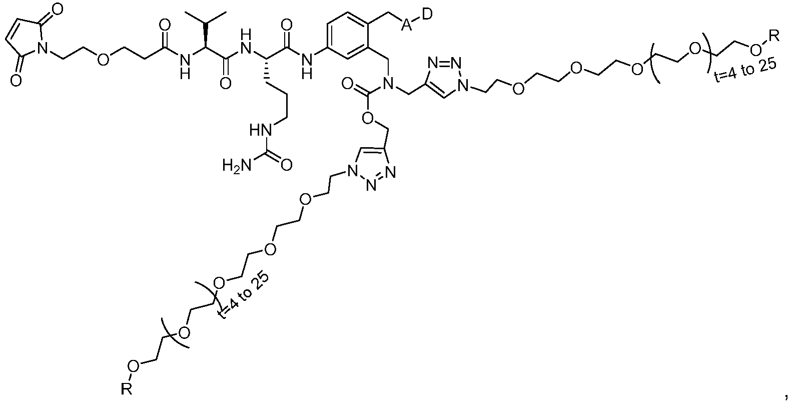 wherein:
each R is independently selected from H, -CH₃, and -CH₂CH₂C(=O)OH;
A is a bond, -OC(=O)-*, 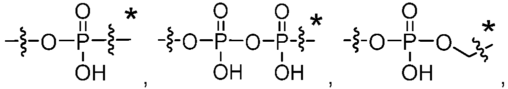 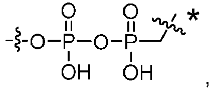 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 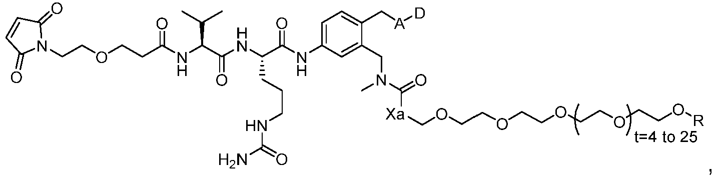 wherein:
Xa is -CH₂-, -OCH₂-, -NHCH₂- or -NRCH₂- and each R independently is H, -CH₃ or -CH₂CH₂C(=O)OH;
A is a bond, -OC(=O)-*, 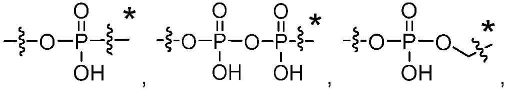 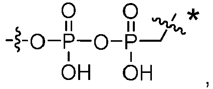 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 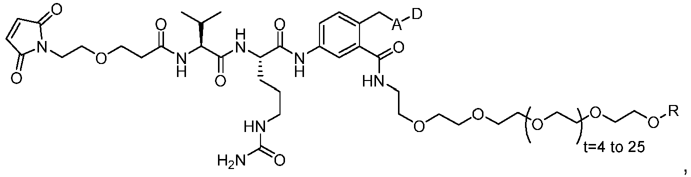 wherein:
R is H, -CH₃ or -CH₂CH₂C(=O)OH;
A is a bond, -OC(=O)-*, 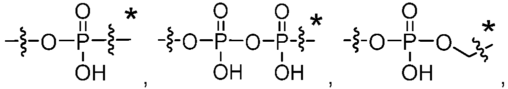 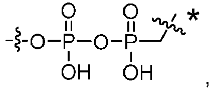 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 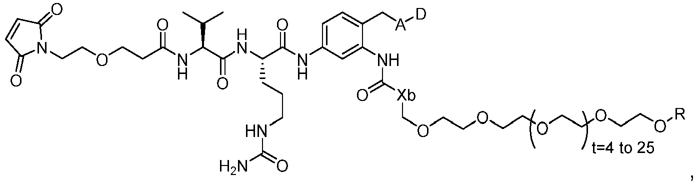 wherein:
Xb is -CH₂-, -OCH₂-, -NHCH₂- or -NRCH₂- and each R independently is H, -CH₃ or -CH₂CH₂C(=O)OH;
A is a bond, -OC(=O)-*, 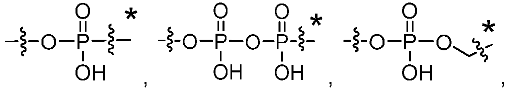 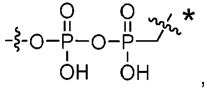 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 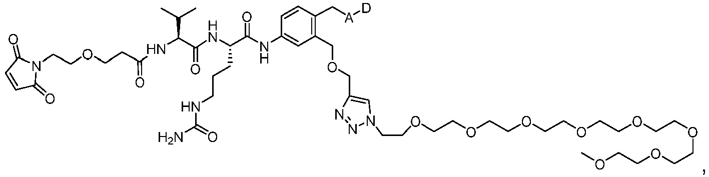 wherein:
A is a bond, -OC(=O)-*, 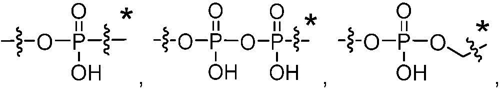 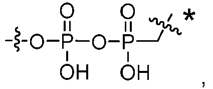 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 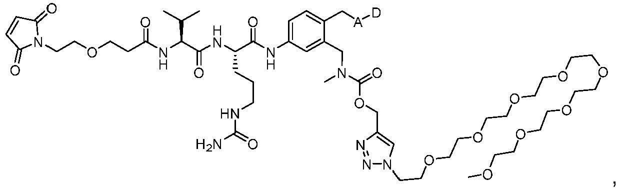 wherein:
A is a bond, -OC(=O)-*, 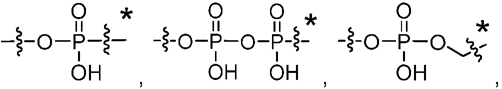 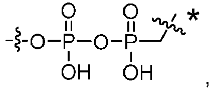 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 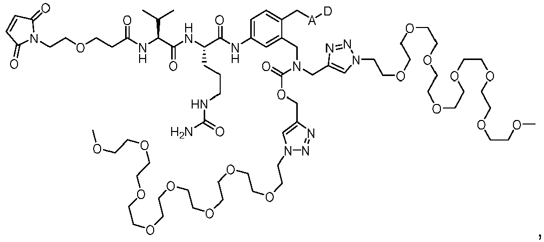 wherein:
A is a bond, -OC(=O)-*, 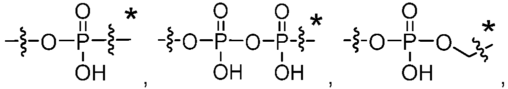 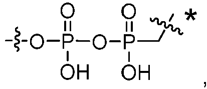 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 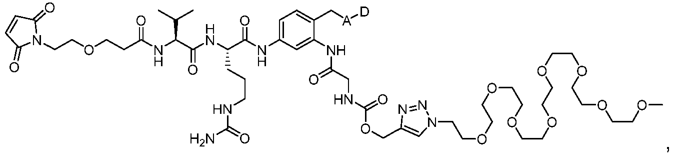 wherein:
A is a bond, -OC(=O)-*, 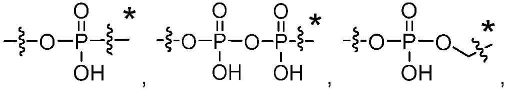 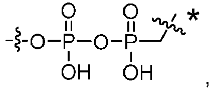 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 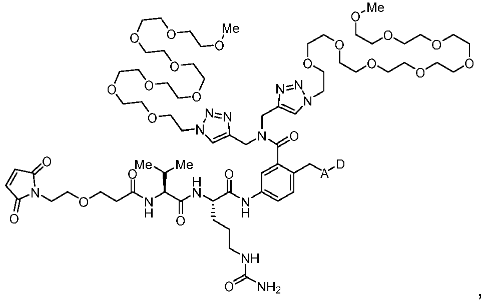 wherein:
A is a bond, -OC(=O)-*, 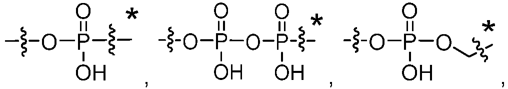 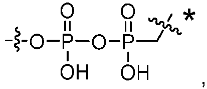 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 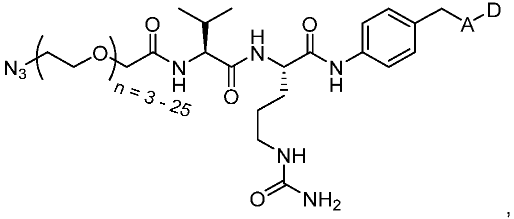 wherein:
A is a bond, -OC(=O)-*, 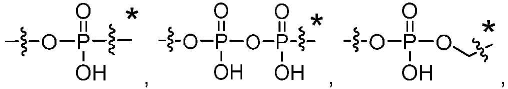 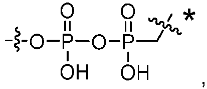 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 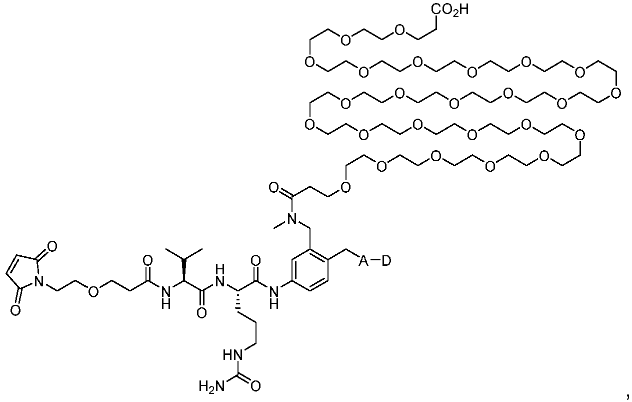 wherein:
A is a bond, -OC(=O)-*, 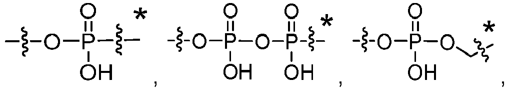 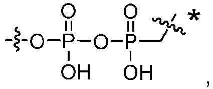 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
D is an Mcl-1 inhibitor.

In some embodiments, the linker-drug group -(L-D) comprises or is formed from a compound of formula: 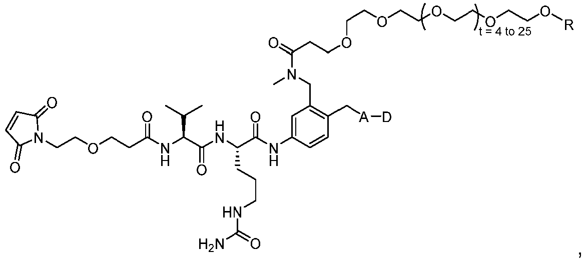 wherein:
each R independently is H, -CH₃ or -CH₂CH₂C(=O)OH;
A is a bond, -OC(=O)-*, 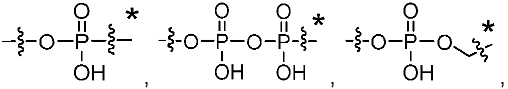 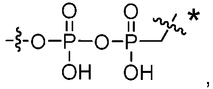 -OC(=O)N(CH₃)CH₂CH₂N(CH₃)C(=O)-* or -OC(=O)N(CH₃)C(R^{a})₂C(R^{a})₂N(CH₃)C(=O)-*,
   wherein each R^{a} is independently selected from H, C₁-C₆ alkyl, and C₃-C₈ cycloalkyl and the * of A indicates the point of attachment to D; and
D is an Mcl-1 inhibitor.

In some embodiments, A is a bond.

In some embodiments, R is -CH₃.

In some embodiments, the Mcl-1 inhibitor (D) comprises a compound of Formula (I): 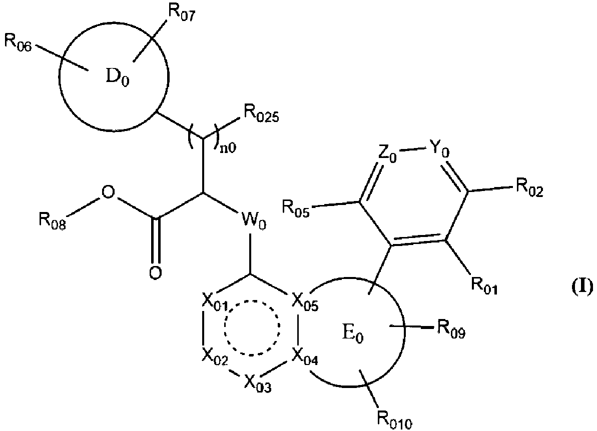 wherein:
Ring D₀ is a cycloalkyl group, a heterocycloalkyl group, an aryl group or a heteroaryl group,
Ring E₀ is a furyl, thienyl or pyrrolyl ring,
X₀₁, X₀₃, X₀₄ and X₀₅ independently of one another is a carbon atom or a nitrogen atom,
X₀₂ is a C-R₀₂₆ group or a nitrogen atom,
   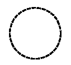 means that the ring is aromatic,
Y₀ is a nitrogen atom or a C-R₀₃ group,
Z₀ is a nitrogen atom or a C-R₀₄ group,
R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a hydroxy(C₁-C₆)alkyl group, a linear or branched (C₁-C₆)alkoxy group, -S-(C₁-C₆)alkyl group, a cyano group, a nitro group, -Cy₀₈, -(C₀-C₆)alkyl-NR₀₁₁R₀₁₁', -O-(C₁-C₆)alkyl-NR₀₁₁R₀₁₁', -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₄-C₆)alkyl-NR₀₁₁-C(O)-R₀₁₁', -SO₂-NR₀₁₁R₀₁₁', or -SO₂-(C₁-C₆)alkyl,
R₀₂, R₀₃, R₀₄ and R₀₅ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a hydroxy(C₁-C₆)alkyl group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-NR₀₁₁R₀₁₁', -O-(C₁-C₆)alkyl-R₀₃₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
or the pair (R₀₁, R₀₂), (R₀₂, R₀₃), (R₀₃, R₀₄), or (R₀₄, R₀₅) together with the carbon atoms to which they are attached form an aromatic or non-aromatic ring containing 5 to 7 ring members, which optionally contains 1 to 3 heteroatoms selected from O, S and N, wherein the resulting ring is optionally substituted by 1 or 2 groups selected from halogen, linear or branched (C₁-C₆)alkyl, (C₀-C₆)alkyl-NR₀₁₁R₀₁₁', -NR₀₁₃R₀₁₃',
   -(C₀-C₆)alkyl-Cy₀₁ or oxo,
R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-(C₁-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-R₀₁₂, -C(O)-OR₁₁, -O-C(O)-R₀₁₁, -C(O)-NR₀₁₁R₀₁₁', -NR₁₁-C(O)-R₀₁₁', -NR₀₁₁-C(O)-OR₀₁₁', -(C₄-C₆)alkyl-NR₀₁₁-C(O)-R₀₁₁', -SO₂-NR₀₁₁R₀₁₁', or -SO₂-(C₁-C₆)alkyl,
or the pair (R₀₆, R₀₇), when fused with the two adjacent carbon atoms, together with the carbon atoms to which they are attached form an aromatic or non-aromatic ring containing 5 to 7 ring members, which optionally contains 1 to 3 heteroatoms selected from O, S and N, wherein the resulting ring is optionally substituted by a linear or branched (C₁-C₆)alkyl group, -NR₀₁₃R₀₁₃', -(C₀-C₆)alkyl-Cy₀₁ or an oxo,
W₀ is a -CH₂- group, a -NH- group or an oxygen atom,
R₀₈ is a hydrogen atom, a linear or branched (C₁-C₈)alkyl group, a -CHR₀ₐR_{0b} group, an aryl group, a heteroaryl group, an aryl(C₁-C₆)alkyl group or a heteroaryl(C₁-C₆)alkyl group,
R₀₉ is a hydrogen atom, a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, or -C(O)-NR₀₁₄R₀₁₄',
R₀₁₀ is a hydrogen atom, a linear or branched (C₁-C₆)alkyl group, a linear or branched (C₂-C₆)alkenyl group, a linear or branched (C₂-C₆)alkynyl group, an aryl(C₁-C₆)alkyl group, a (C₁-C₆)cycloalkylalkyl group, a linear or branched (C₁-C₆)haloalkyl, or -(C₁-C₆)alkyl-O-Cy₀₄,
or the pair (R₀₉, R₀₁₀), when fused with the two adjacent carbon atoms, together with the carbon atoms to which they are attached form an aromatic or non-aromatic ring containing 5 to 7 ring members, which optionally contains 1 to 3 heteroatoms selected from O, S and N,
R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the nitrogen may be substituted by 1 or 2 groups selected from a hydrogen atom and a linear or branched (C₁-C₆)alkyl group, and wherein one or more of the carbon atoms of the linear or branched (C₁-C₆)alkyl group is optionally deuterated,
R₀₁₂ is -Cy₀₅, -Cy₀₅-(C₀-C₆)alkyl-O-(C₀-C₆)alkyl-Cy₀₆, -Cy₀₅-(C₀-C₆)alkyl-Cy₀₆, -Cy₀₅-(C₀-C₆)alkyl-NR₀₁₁-(C₀-C₆)alkyl-Cy₀₆, -Cy₀₅-Cy₀₆-O-(C₀-C₆)alkyl-Cy₀₇, -Cy₀₅-(C₀-C₆)alkyl-O-(C₀-C₆)alkyl-Cy₀₉, -Cy₀₅-(C₀-C₆)alkyl-Cy₀₉, -NH-C(O)-NH-R₀₁₁, -Cy₀₅-(C₀-C₆)alkyl-NR₀₁₁-(C₀-C₆)alkyl-Cy₀₉, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, and -C(O)-OR₀₁₁,
R₀₁₃, R₀₁₃', R₀₁₄ and R₀₁₄' independently of one another are a hydrogen atom or an optionally substituted linear or branched (C₁-C₆)alkyl group,
R₀ₐ is a hydrogen atom or a linear or branched (C₁-C₆)alkyl group,
R_{0b} is a -O-C(O)-O-R_{0c} group, a -O-C(O)-NR_{0c}R_{0c}' group, or a -O-P(O)(OR_{0c})₂ group,
R_{0c} and R_{0c}' independently of one another are a hydrogen atom, a linear or branched (C₁-C₈)alkyl group, a cycloalkyl group, a (C₁-C₆)alkoxy(C₁-C₆)alkyl group, or a (C₁-C₆)alkoxycarbonyl(C₁-C₆)alkyl group,
or the pair (R_{0c}, R_{0c}') together with the nitrogen atom to which they are attached form a non-aromatic ring composed of from 5 to 7 ring members, which may contain in addition to the nitrogen atom from 1 to 3 heteroatoms selected from oxygen and nitrogen, wherein the nitrogen is optionally substituted by a linear or branched
   (C₁-C₆)alkyl group,
Cy₀₁, Cy₀₂, Cy₀₃, Cy₀₄, Cy₀₅, Cy₀₆, Cy₀₇, Cy₀₈ and Cy₀₁₀ independently of one another, represent a cycloalkyl group, a heterocycloalkyl group, an aryl group or a heteroaryl group, each of which is optionally substituted,
Cy₀₉ is 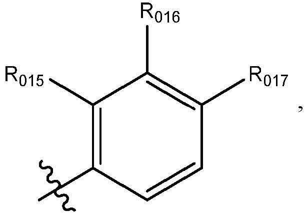 or Cy₀₉ is a heteroaryl group which is substituted by a group selected from -O-P(O)(OR₀₂₀)₂; -O-P(O)(O⁻M⁺)₂; -(CH₂)ₚ₀-O-(CHR₀₁₈-CHR₀₁₉-O)_{q0}-R₀₂₀; hydroxy; hydroxy(C₁-C₆)alkyl; -(CH₂)ᵣ₀-U₀-(CH₂)ₛ₀-heterocycloalkyl; and -U₀-(CH₂)_{q0}-NR₀₂₁R₀₂₁',
R₀₁₅ is a hydrogen atom; a -(CH₂)ₚ₀-O-(CHR₀₁₈-CHR₀₁₉-O)_{q0}-R₀₂₀ group; a linear or branched (C₁-C₆)alkoxy(C₁-C₆)alkyl group; a -U₀-(CH₂)_{q0}-NR₀₂₁R₀₂₁' group; or a -(CH₂)ᵣ₀-U₀-(CH₂)ₛ₀-heterocycloalkyl group,
R₀₁₆ is a hydrogen atom; a hydroxy group; a hydroxy(C₁-C₆)alkyl group; a -(CH₂)ᵣ₀-U₀-(CH₂)ₛ₀-heterocycloalkyl group; a (CH₂)ᵣ₀-U₀-V₀-O-P(O)(OR₀₂₀)₂ group; a -O-P(O)(O⁻M⁺)₂ group; a -O-S(O)₂OR₀₂₀ group; a -S(O)₂OR₀₂₀ group; a -(CH₂)ₚ₀-O-(CHR₀₁₈-CHR₀₁₉-O)_{q0}-R₀₂₀ group; a -(CH₂)ₚ₀-O-C(O)-NR₀₂₂R₀₂₃ group; or a -U₀-(CH₂)_{q0}-NR₀₂₁R₀₂₁' group,
R₀₁₇ is a hydrogen atom; a -(CH₂)ₚ₀-O-(CHR₀₁₈-CHR₀₁₉-O)_{q0}-R₀₂₀ group; a -CH₂-P(O)(OR₀₂₀)₂ group, a -O-P(O)(OR₀₂₀)₂ group; a -O-P(O)(O⁻M⁺)₂ group; a hydroxy group; a hydroxy(C₁-C₆)alkyl group; a -(CH₂)ᵣ₀-U₀-(CH₂)ₛ₀-heterocycloalkyl group; a -U₀-(CH₂)_{q0}-NR₀₂₁R₀₂₁' group; or an aldonic acid,
M⁺ is a pharmaceutically acceptable monovalent cation,
U₀ is a bond or an oxygen atom,
V₀ is a -(CH₂)ₛ₀- group or a -C(O)- group,
R₀₁₈ is a hydrogen atom or a (C₁-C₆)alkoxy(C₁-C₆)alkyl group,
R₀₁₉ is a hydrogen atom or a hydroxy(C₁-C₆)alkyl group,
R₀₂₀ is a hydrogen atom or a linear or branched (C₁-C₆)alkyl group,
R₀₂₁ and R₀₂₁' independently of one are a hydrogen atom, a linear or branched (C₁-C₆)alkyl group, or a hydroxy(C₁-C₆)alkyl group,
or the pair (R₀₂₁, R₀₂₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the resulting ring is optionally substituted by a hydrogen atom or a linear or branched (C₁-C₆)alkyl group,
R₀₂₂ is a (C₁-C₆)alkoxy(C₁-C₆)alkyl group, a -(CH₂)ₚ₀-NR₀₂₄R₀₂₄' group, or a -(CH₂)ₚ₀-O-(CHR₀₁₈-CHR₀₁₉-O)_{q0}-R₀₂₀ group,
R₀₂₃ is a hydrogen atom or a (C₁-C₆)alkoxy(C₁-C₆)alkyl group, or the pair (R₀₂₂, R₀₂₃) together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched (C₁-C₆)alkyl group or a heterocycloalkyl group,
R₀₂₄ and R₀₂₄' independently of one another are a hydrogen atom or a linear or branched (C₁-C₆)alkyl group,
   or the pair (R₀₂₄, R₀₂₄') together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched (C₁-C₆)alkyl group,
R₀₂₅ is a hydrogen atom, a hydroxy group, or a hydroxy(C₁-C₆)alkyl group,
R₀₂₆ is a hydrogen atom, a halogen atom, a linear or branched (C₁-C₆)alkyl group, or a cyano group,
R₀₂₇ is a hydrogen atom or a linear or branched (C₁-C₆)alkyl group,
R₀₂₈ is a -O-P(O)(O⁻)(O⁻) group, a -O-P(O)(O⁻)(OR₀₃₀) group, a -O-P(O)(OR₀₃₀)(OR₀₃₀') group, a -(CH₂)ₚ₀-O-SO₂-O⁻ group, a -(CH₂)ₚ₀-SO₂-O⁻ group, a -(CH₂)ₚ₀-O-SO₂-OR₀₃₀ group, -Cy₀₁₀, a -(CH₂)ₚO-SO₂-OR₀₃₀ group, a -O-C(O)-R₀₂₉ group, a -O-C(O)-OR₀₂₉ group or a -O-C(O)-NR₀₂₉R₀₂₉' group;
R₀₂₉ and R₀₂₉' independently of one another represent a hydrogen atom, a linear or branched (C₁-C₆)alkyl group or a linear or branched amino(C₁-C₆)alkyl group,
R₀₃₀ and R₀₃₀' independently of one another are a hydrogen atom, a linear or branched (C₁-C₆)alkyl group or an aryl(C₁-C₆)alkyl group,
R₀₃₁ is 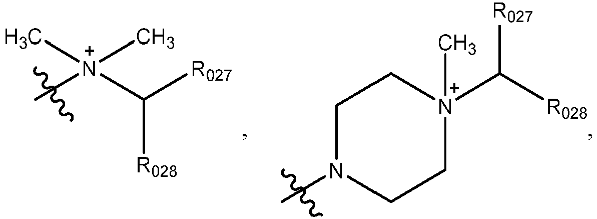 or 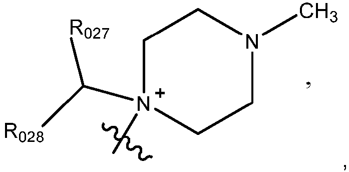 wherein the ammonium cation optionally exists as a zwitterionic form or has a monovalent anionic counterion,
n₀ is an integer equal to 0 or 1,
p₀ is an integer equal to 0, 1, 2, or 3,
q₀ is an integer equal to 1, 2, 3 or 4,
r₀ and s₀ are independently an integer equal to 0 or 1;
   wherein, at most, one of the R₀₃, R₀₉, or R₀₁₂ groups, if present, is covalently attached to the linker, and wherein the valency of an atom is not exceeded by virtue of one or more substituents bonded thereto,
or an enantiomer, diastereoisomer, atropisomer, deuterated derivative, and/or pharmaceutically acceptable salt of any of the foregoing.

In some embodiments, Cy₀₁, Cy₀₂, Cy₀₃, Cy₀₄, Cy₀₅, Cy₀₆, Cy₀₇, Cy₀₈ and Cy₀₁₀, independently of one another, is a cycloalkyl group, a heterocycloalkyl group, an aryl group or a heteroaryl group, each of which is optionally substituted by one or more groups selected from halo; -(C₁-C₆)alkoxy; -(C₁-C₆)haloalkyl; -(C₁-C₆)haloalkoxy; -(CH₂)ₚ₀-O-SO₂-OR₀₃₀; -(CH₂)ₚ₀-SO₂-OR₀₃₀; -O-P(O)(OR₀₂₀)₂; -O-P(O)(O⁻M⁺)₂; -CH₂-P(O)(OR₀₂₀)₂; -(CH₂)ₚ₀-O-(CHR₀₁₈-CHR₀₁₉-O)_{q0}-R₀₂₀; hydroxy; hydroxy(C₁-C₆)alkyl; -(CH₂)ᵣ₀-U₀-(CH₂)ₛ₀-heterocycloalkyl; or -U₀-(CH₂)_{q0}-NR₀₂₁R₀₂₁'.

In some embodiments, D comprises a compound of Formula (II): 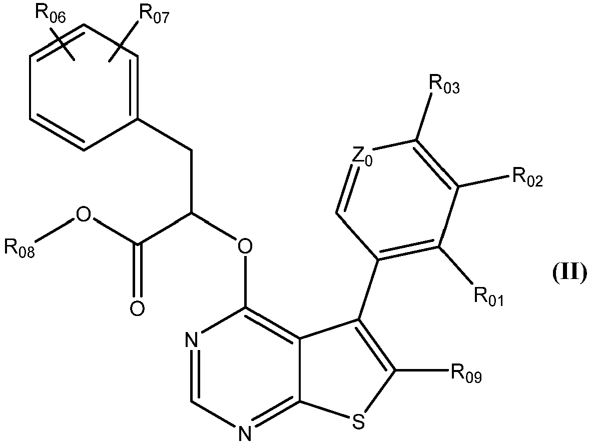 wherein:
Z₀ is a nitrogen atom or a C-R₀₄ group,
R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a linear or branched (C₁-C₆)alkoxy group, a -S-(C₁-C₆)alkyl group, a cyano group, -Cy₀₈, -NR₀₁₁R₀₁₁',
R₀₂, R₀₃ and R₀₄ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-NR₀₁₁R₀₁₁', -O-(C₁-C₆)alkyl-R₀₃₁, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl, or the pair (R₀₂, R₀₃) or (R₀₃, R₀₄) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched (C₁-C₆)alkyl, -NR₀₁₃R₀₁₃', -(C₀-C₆)alkyl-Cy₀₁ and oxo,
R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl, or the pair (R₀₆, R₀₇), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched (C₁-C₆)alkyl group, -NR₀₁₃R₀₁₃', -(C₀-C₆)alkyl-Cy₀₁ and an oxo,
R₀₈ is a hydrogen atom, a linear or branched (C₁-C₈)alkyl group, an aryl group, a heteroaryl group, an aryl-(C₁-C₆)alkylgroup, or a heteroaryl(C₁-C₆)alkyl group,
R₀₉ is a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, -C(O)-NR₀₁₄R₀₁₄',
R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched (C₁-C₆)alkyl group, and wherein one or more of the carbon atoms of the linear or branched (C₁-C₆)alkyl group is optionally deuterated,
R₀₁₂ represents -Cy₀₅, -Cy₀₅-(C₀-C₆)alkyl-Cy₀₆, -Cy₀₅-(C₀-C₆)alkyl-O-(C₀-C₆)alkyl-Cy₀₆, -Cy₀₅-(C₀-C₆)alkyl-NR₀₁₁-(C₀-C₆)alkyl-Cy₀₆, -Cy₀₅-Cy₀₆-O-(C₀-C₆)alkyl-Cy₀₇, -Cy₀₅-(C₀-C₆)alkyl-Cy₀₉, -NH-C(O)-NH-R₀₁₁, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, or -C(O)-OR₀₁₁,
R₀₁₃, R₀₁₃', R₀₁₄ and R₀₁₄' independently of one another are a hydrogen atom, or an optionally substituted linear or branched (C₁-C₆)alkyl group,
Cy₀₁, Cy₀₂, Cy₀₃, Cy₀₅, Cy₀₆, Cy₀₇ and Cy₀₈ independently of one another, are an optionally substituted cycloalkyl group, an optionally substituted heterocycloalkyl group, an optionally substituted aryl group or an optionally substituted heteroaryl group,
   Cy₀₉ is 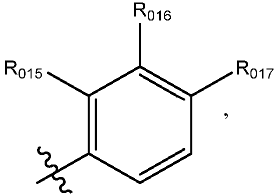 wherein R₀₁₅, R₀₁₆, and R₀₁₇ are as defined for formula (I),
   R₀₃₁ is 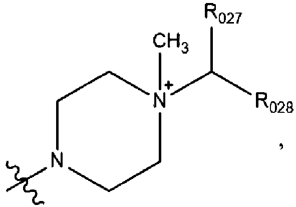 wherein R₀₂₇ and R₀₂₈ are as defined for formula (I) wherein, at most, one of the R₀₃,R₀₉, or R₀₁₂ groups, if present, is covalently attached to the linker,
or an enantiomer, diastereoisomer, atropisomer, deuterated derivative, and/or pharmaceutically acceptable salt of any of the foregoing.

In some embodiments, D comprises a compound of Formula (III): 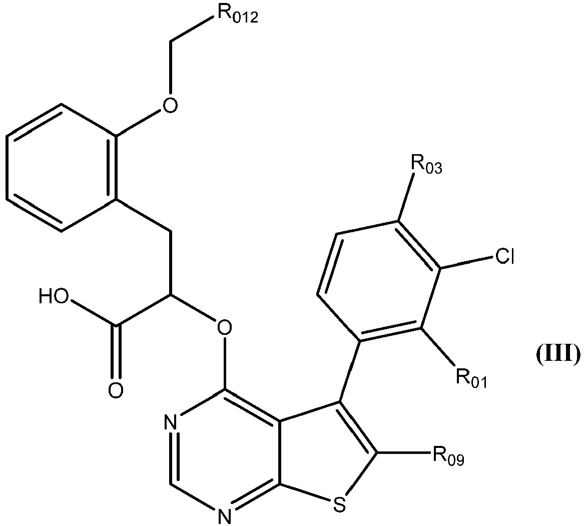 wherein:
R₀₁ is a linear or branched (C₁-C₆)alkyl group,
R₀₃ is -O-(C₁-C₆)alkyl-NR₀₁₁R₀₁₁', or 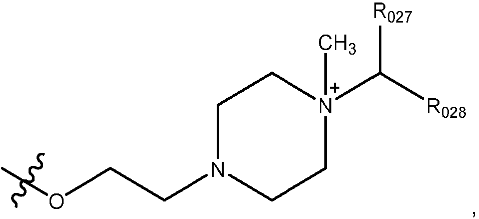
wherein R₀₁₁ and R₀₁₁' independently of one another are a hydrogen atom, an optionally substituted linear or branched (C₁-C₆)alkyl group, or -(C₀-C₆)alkyl-Cy₀₁;
or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom may be substituted by 1 or 2 groups selected from a hydrogen atom or a linear or branched (C₁-C₆)alkyl group,
and wherein R₀₂₇ is a hydrogen atom and R₀₂₈ is a -(CH₂)ₚ₀-O-SO₂-O⁻ group or a -(CH₂)ₚ₀-SO₂-OR₀₃₀ group;
R₀₉ is a linear or branched (C₂-C₆)alkynyl group or -Cy₀₂,
R₀₁₂ is -Cy₀₅, -Cy₀₅-(C₀-C₆)alkyl-Cy₀₆, or -Cy₀₅-(C₀-C₆)alkyl-Cy₀₉,
Cy₀₁, Cy₀₂, Cy₀₅ and Cy₀₆ independently of one another, are a cycloalkyl group, a heterocycloalkyl group, an aryl group or a heteroaryl group, each of which is optionally substituted,
Cy₀₉ is 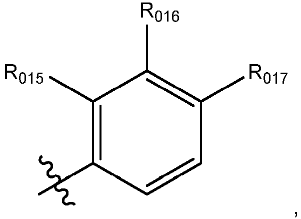
R₀₁₅, R₀₁₆, and R₀₁₇ are as defined for formula (I),
wherein, at most, one of the R₀₃, R₀₉, or R₀₁₂ groups, if present, is covalently attached to the linker,
or the enantiomer, diastereoisomer, atropisomer, deuterated derivative, and/or pharmaceutically acceptable salt of any of the foregoing.

In some embodiments, Cy₀₁, Cy₀₂, Cy₀₅, Cy₀₆, independently of one another, is a cycloalkyl group, a heterocycloalkyl group, an aryl group or a heteroaryl group, each of which is optionally substituted by one or more groups selected from halo; -(C₁-C₆)alkoxy; -(C₁-C₆)haloalkyl; -(C₁-C₆)haloalkoxy; -(CH₂)ₚ₀-O-SO₂-OR₀₃₀; -(CH₂)ₚ₀-SO₂-OR₀₃₀; -O-P(O)(OR₀₂₀)₂; -O-P(O)(O⁻M⁺)₂; -CH₂-P(O)(OR₀₂₀)₂; -(CH₂)ₚ₀-O-(CHR₀₁₈-CHR₀₁₉-O)_{q0}-R₀₂₀; hydroxy; hydroxy(C₁-C₆)alkyl; -(CH₂)ᵣ₀-U₀-(CH₂)ₛ₀-heterocycloalkyl; or -U₀-(CH₂)_{q0}-NR₀₂₁R₀₂₁'.

In some embodiments, R₀₁ is methyl or ethyl.

In some embodiments, R₀₃ is -O-CH₂-CH₂-NR₀₁₁R_{011'} in which R₀₁₁ and R₀₁₁' form, together with the nitrogen atom carrying them, a piperazinyl group which may be substituted by a substituted by a hydrogen atom or a linear or branched (C₁-C₆)alkyl group.

In some embodiments, R₀₃ comprises the formula: 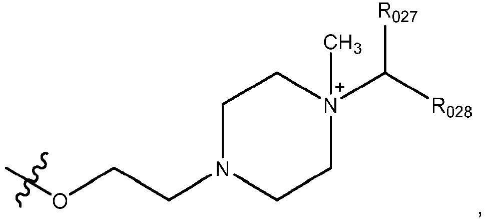 wherein R₀₂₇ is a hydrogen atom and R₀₂₈ is a -(CH₂)ₚ₀-O-SO₂-OR₀₃₀ group, p₀ is an integer equal to 0, 1, 2, or 3; and wherein R₀₃₀ represents a hydrogen atom, a linear or branched (C₁-C₆)alkyl group or an aryl(C₁-C₆)alkyl group.

In some embodiments, R₀₃ comprises the formula: 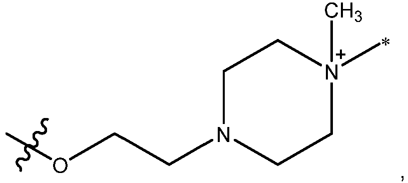 wherein -* is a bond to the linker.

In some embodiments, Cy₀₁, Cy₀₂, Cy₀₃, Cy₀₄, Cy₀₅, Cy₀₆, Cy₀₇, Cy₀₈ and Cy₀₁₀ independently of one another, are an optionally substituted cycloalkyl group, an optionally substituted heterocycloalkyl group, an optionally substituted aryl group or an optionally substituted heteroaryl group, wherein the optional substituents are selected from optionally substituted linear or branched (C₁-C₆)alkyl, optionally substituted linear or branched (C₂-C₆)alkenyl group, optionally substituted linear or branched (C₂-C₆)alkynyl group, optionally substituted linear or branched (C₁-C₆)alkoxy, optionally substituted (C₁-C₆)alkyl-S-, hydroxy, oxo (or N-oxide where appropriate), nitro, cyano, -C(O)-OR₀', -O-C(O)-R₀', -C(O)-NR₀'R₀", -NR₀'R₀", -(C=NR₀')-OR₀", linear or branched (C₁-C₆) haloalkyl, trifluoromethoxy, or halogen, wherein R₀' and R₀" are each independently a hydrogen atom or an optionally substituted linear or branched (C₁-C₆)alkyl group, and wherein one or more of the carbon atoms of linear or branched (C₁-C₆)alkyl group is optionally deuterated.

In some embodiments, R₀₉ is a Cy₀₂ group, preferably an aryl group, more preferably a phenyl group. In some embodiments, Cy₀₂ is an optionally substituted aryl group.

In some embodiments, Cy₀₅ comprises a heteroaryl group selected from a pyrazolyl group and a pyrimidinyl group.

In some embodiments, Cy₀₅ is a pyrimidinyl group.

In some embodiments, Cy₀₅ is a pyrimidinyl group and Cy₀₆ is phenyl group.

In some embodiments, the linker (L) is attached to D by a covalent bond from L to R₀₃ of formulas (I), (II), or (III). In some embodiments, the linker (L) is attached to D by a covalent bond from L to R₀₉ of formulas (I), (II), or (III).

In some embodiments, D comprises: 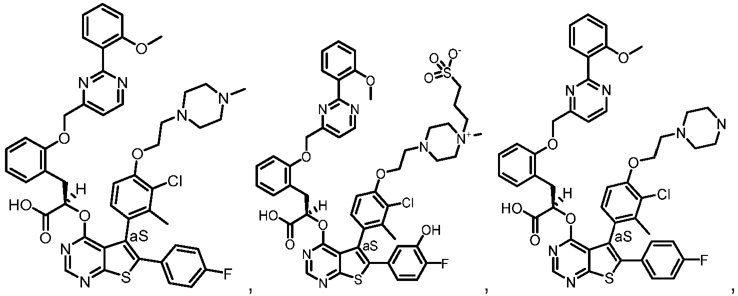 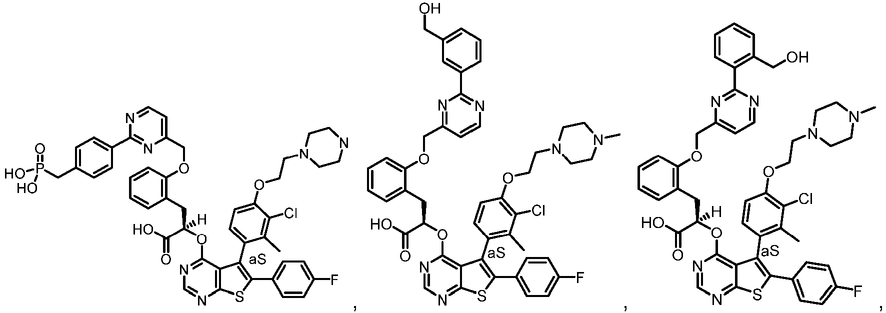 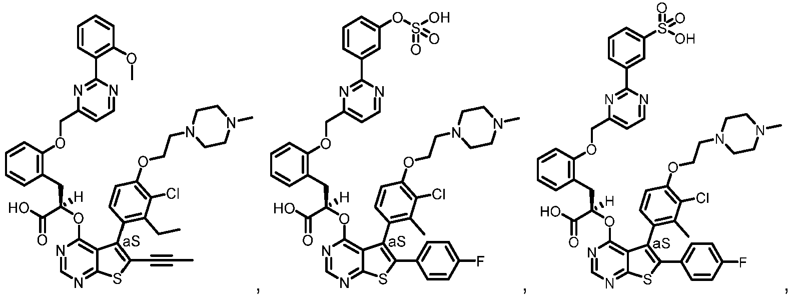 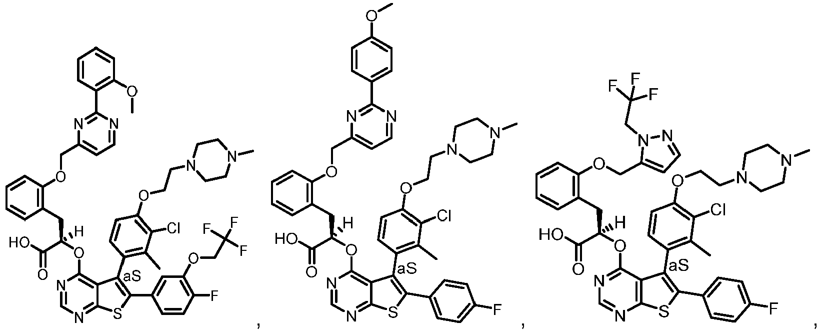 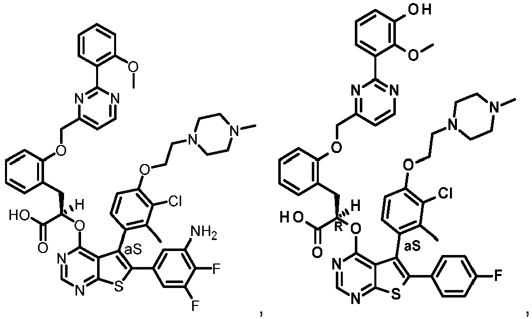 or
an enantiomer, diastereoisomer, atropisomer, deuterated derivative, and/or a pharmaceutically acceptable salt of any of the foregoing.

In some embodiments, -(L-D) is formed from a compound selected from Table A or an enantiomer, diastereoisomer, atropisomer, deuterated derivative, and/or pharmaceutically acceptable salt thereof. For compounds in Table A, depending on their electronic charge, these compounds can contain one pharmaceutically acceptable monovalent anionic counterion M₁⁻. In some embodiments, the monovalent anionic counterion M₁⁻ can be selected from bromide, chloride, iodide, acetate, trifluoroacetate, benzoate, mesylate, tosylate, triflate, formate, or the like. In some embodiments, the monovalent anionic counterion M₁⁻ is trifluoroacetate or formate.

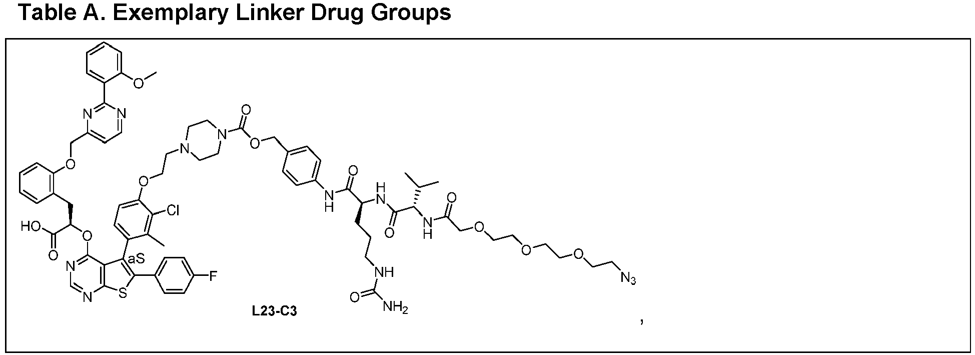

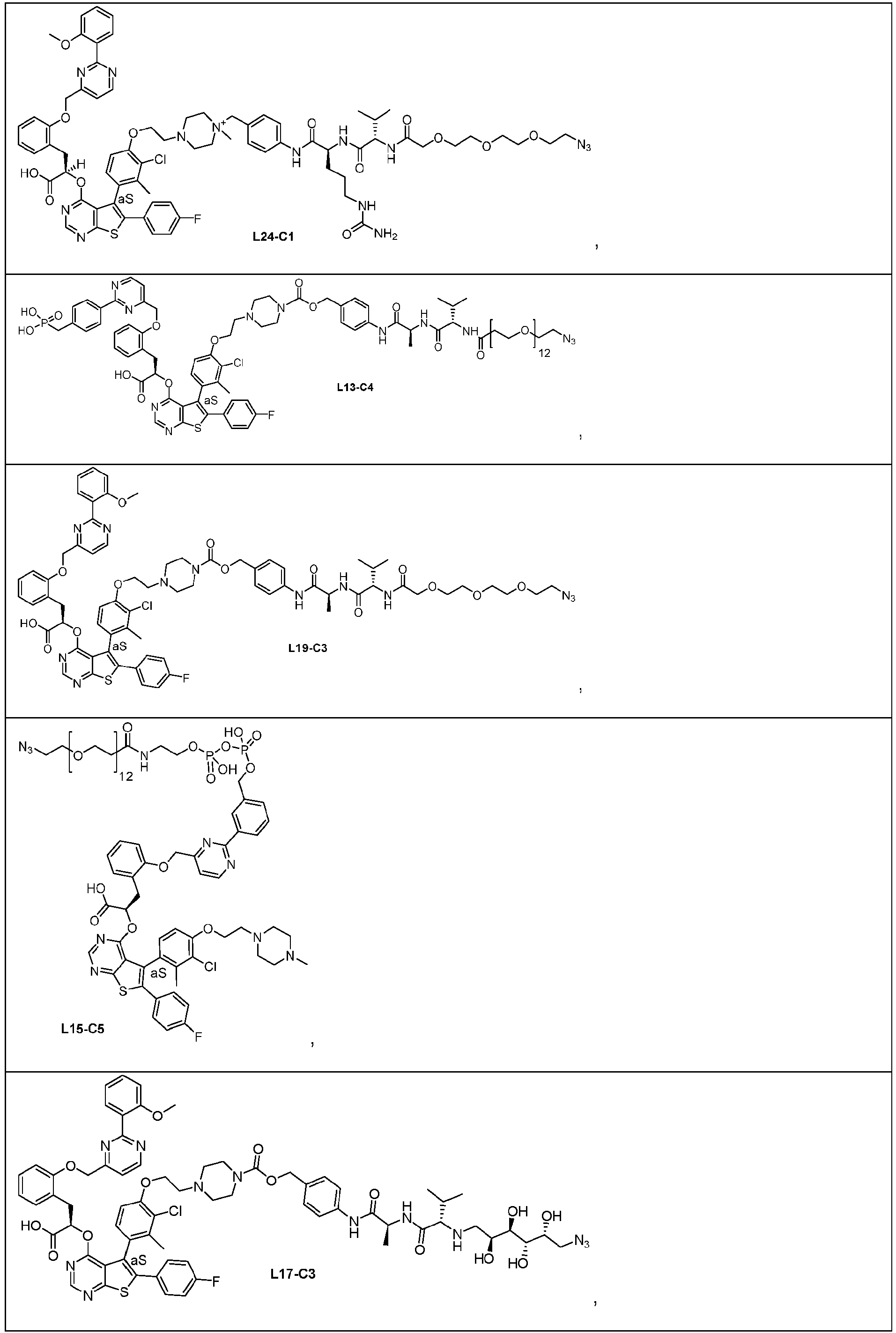

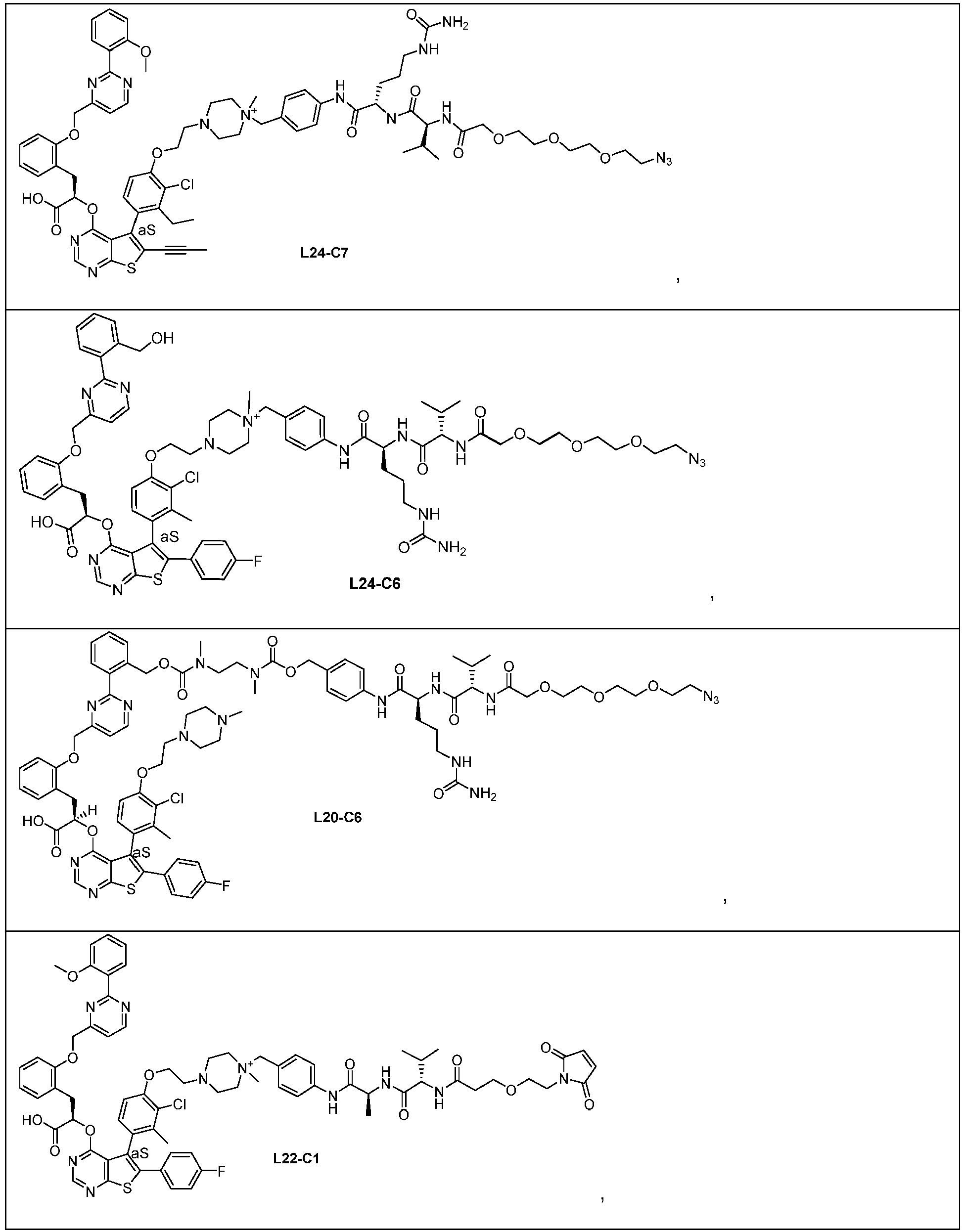

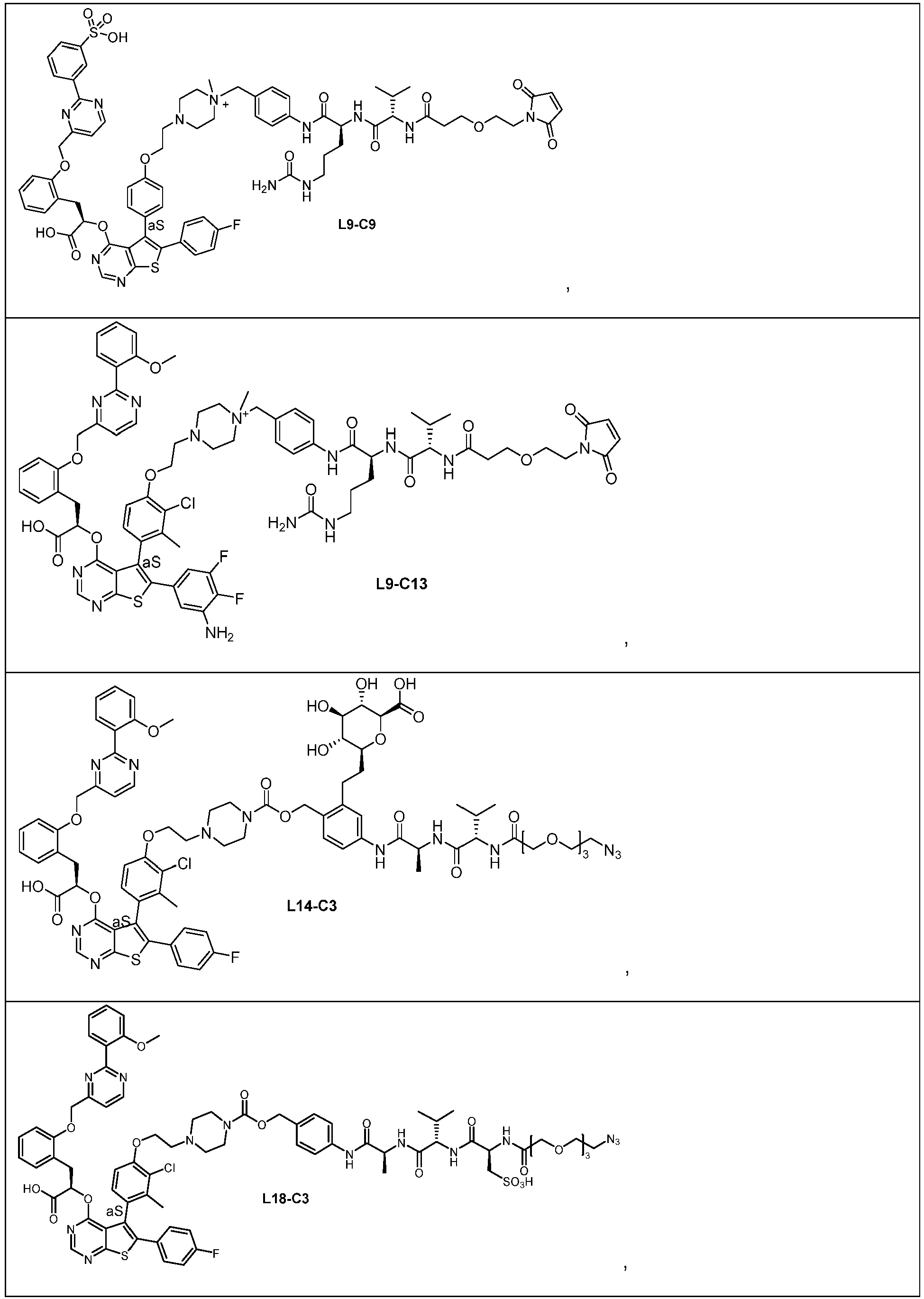

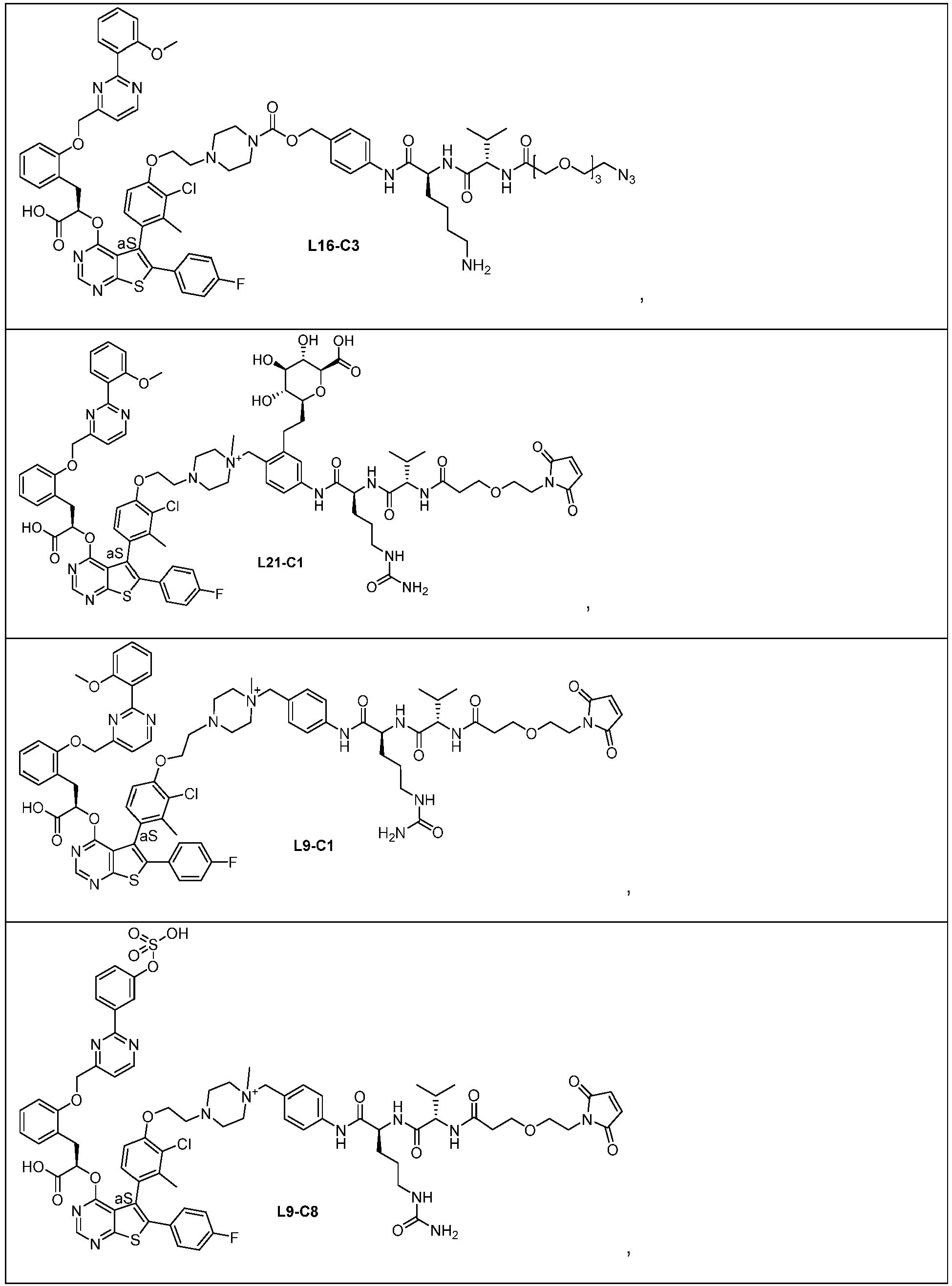

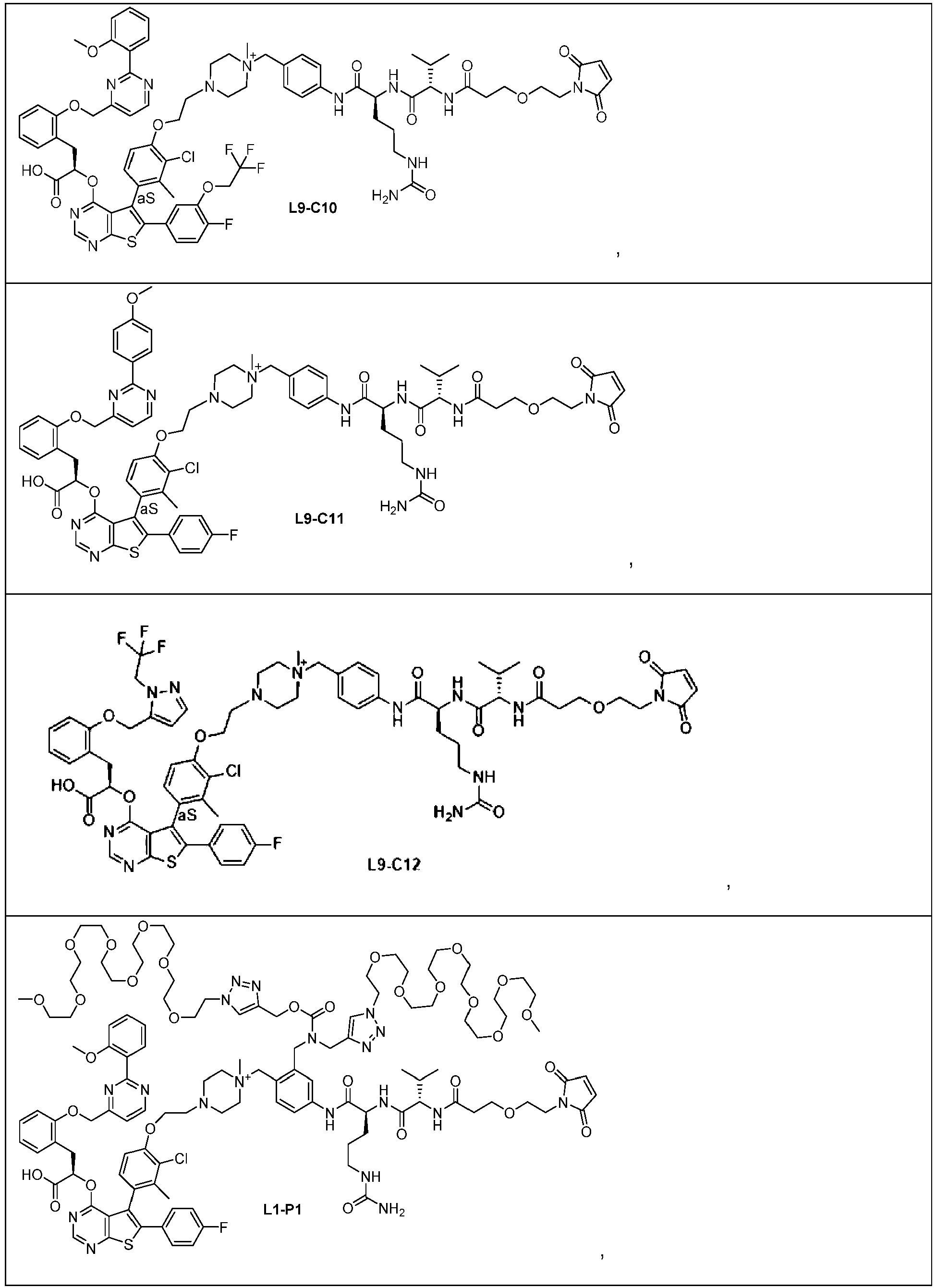

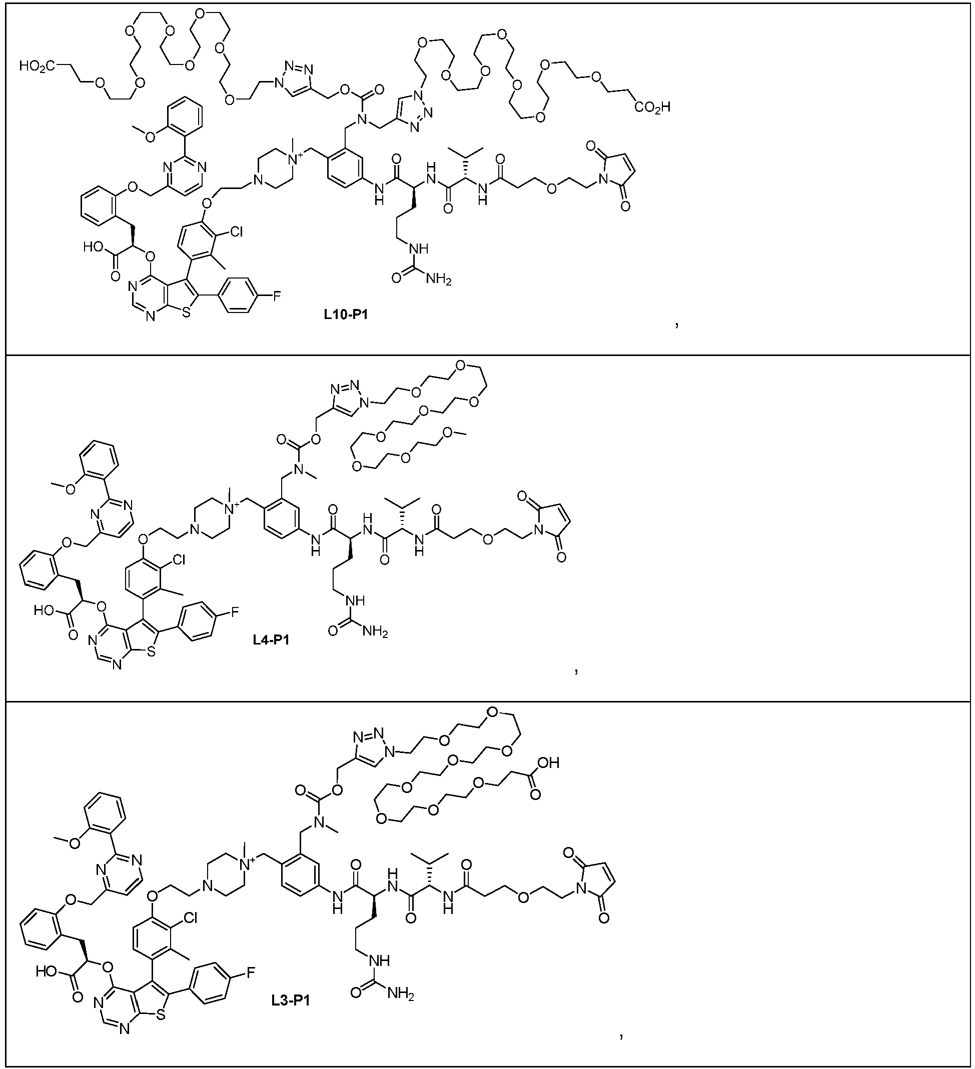

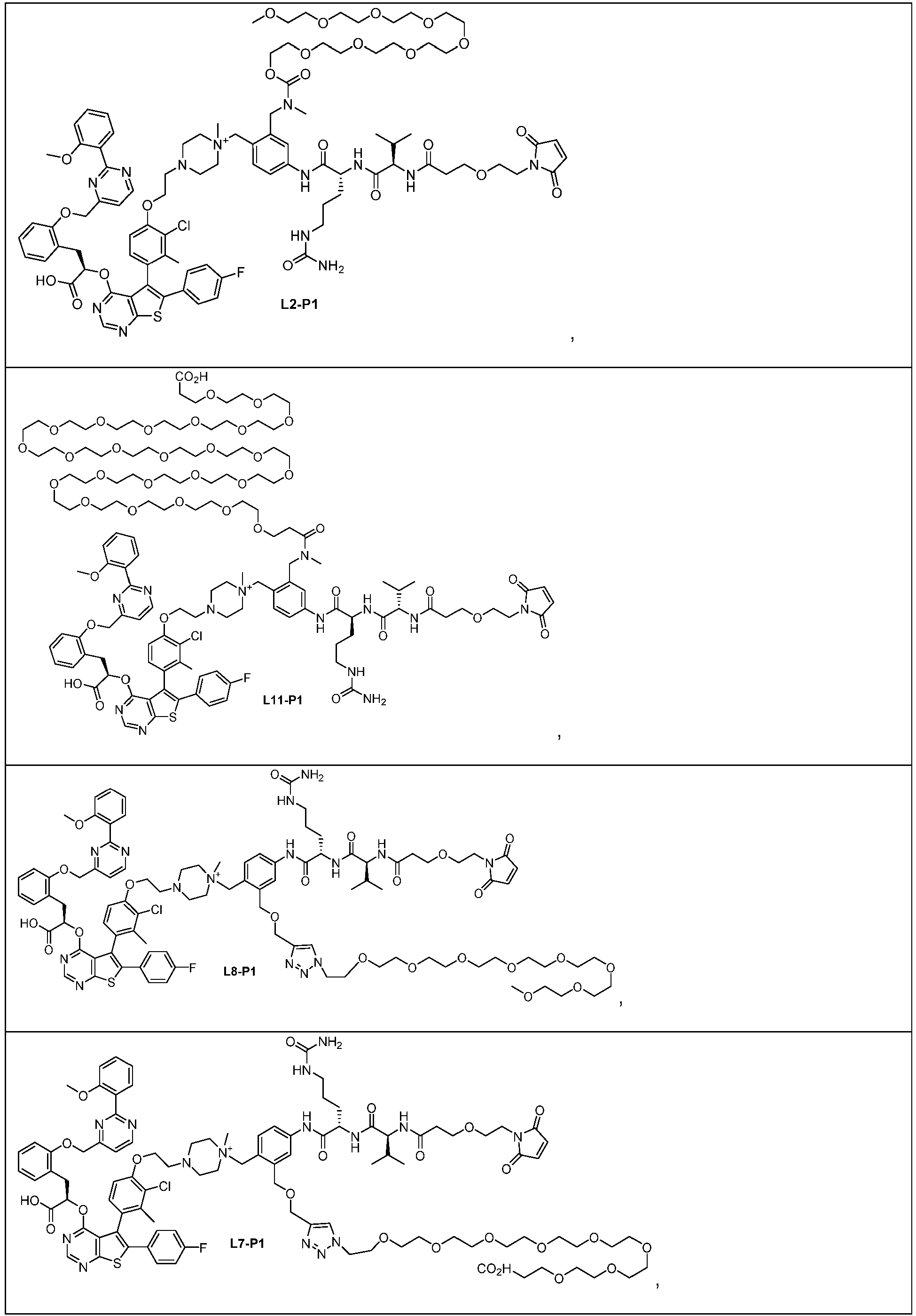

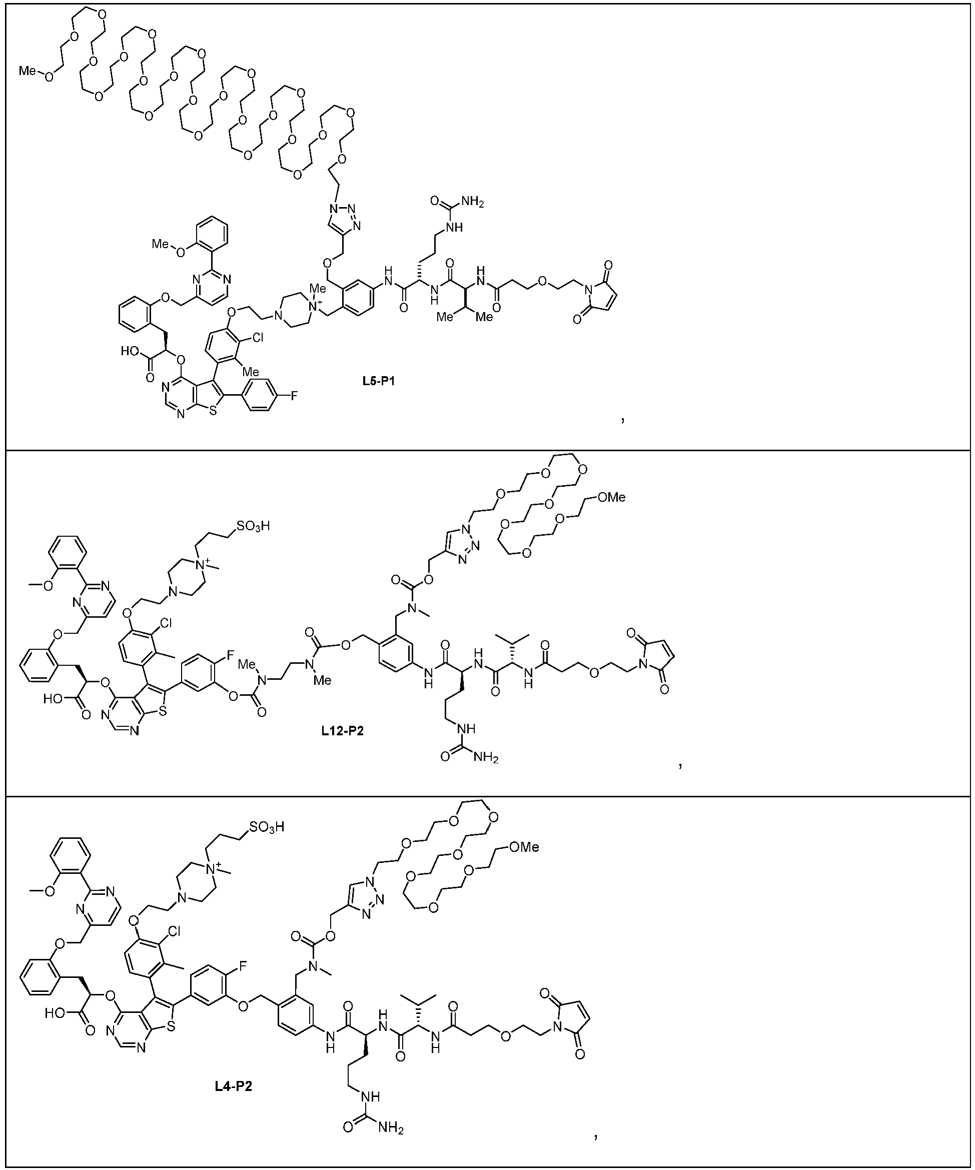

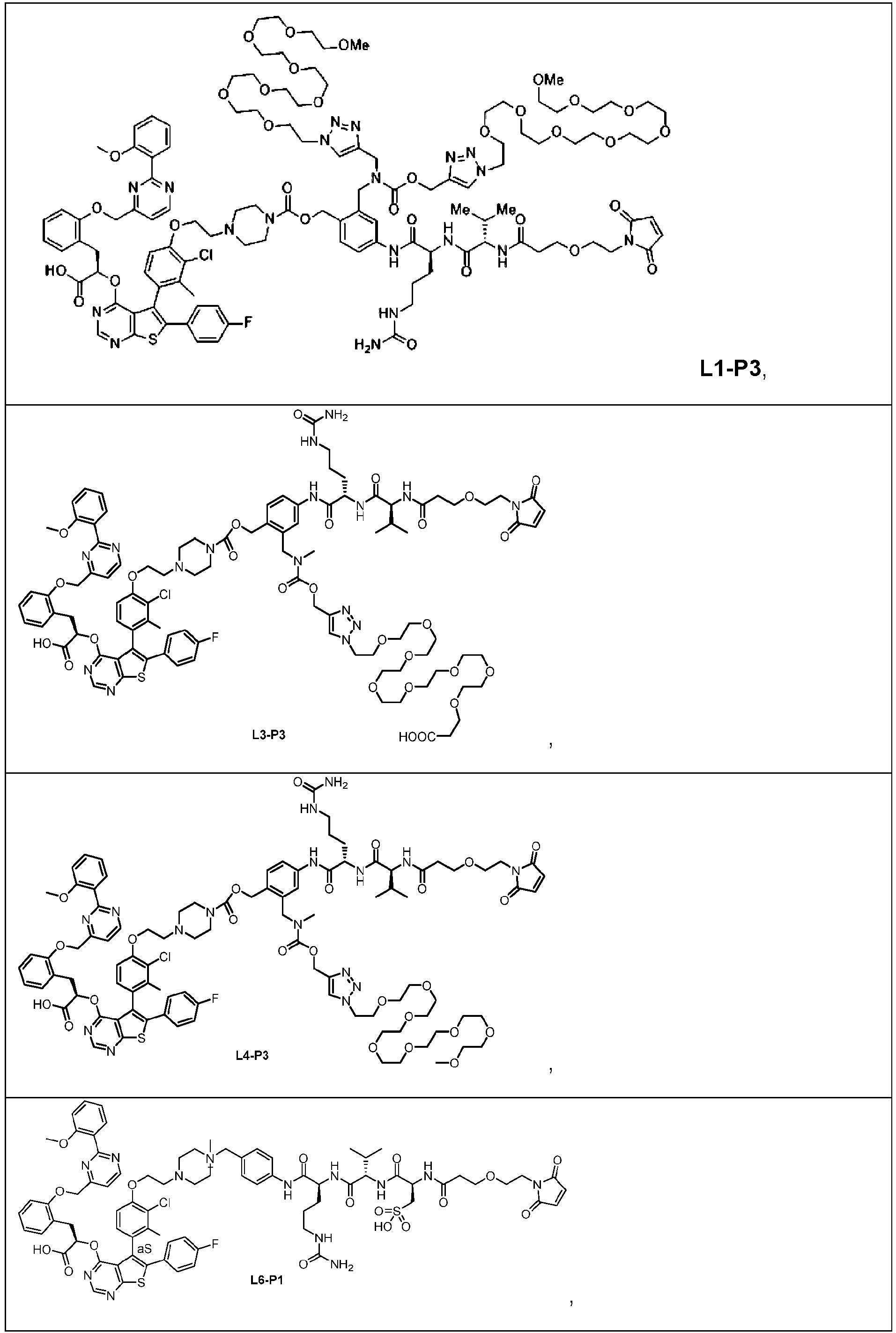

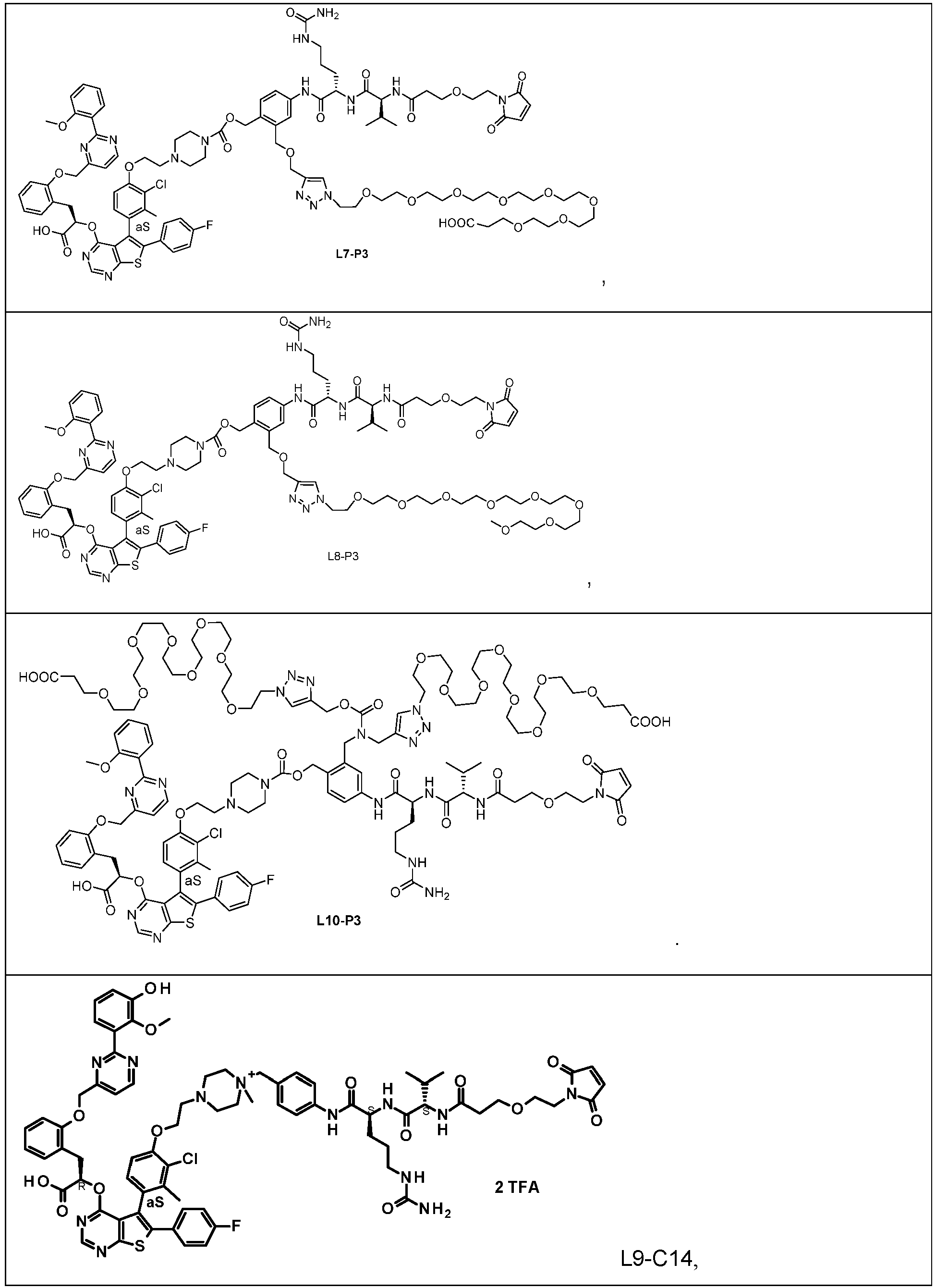

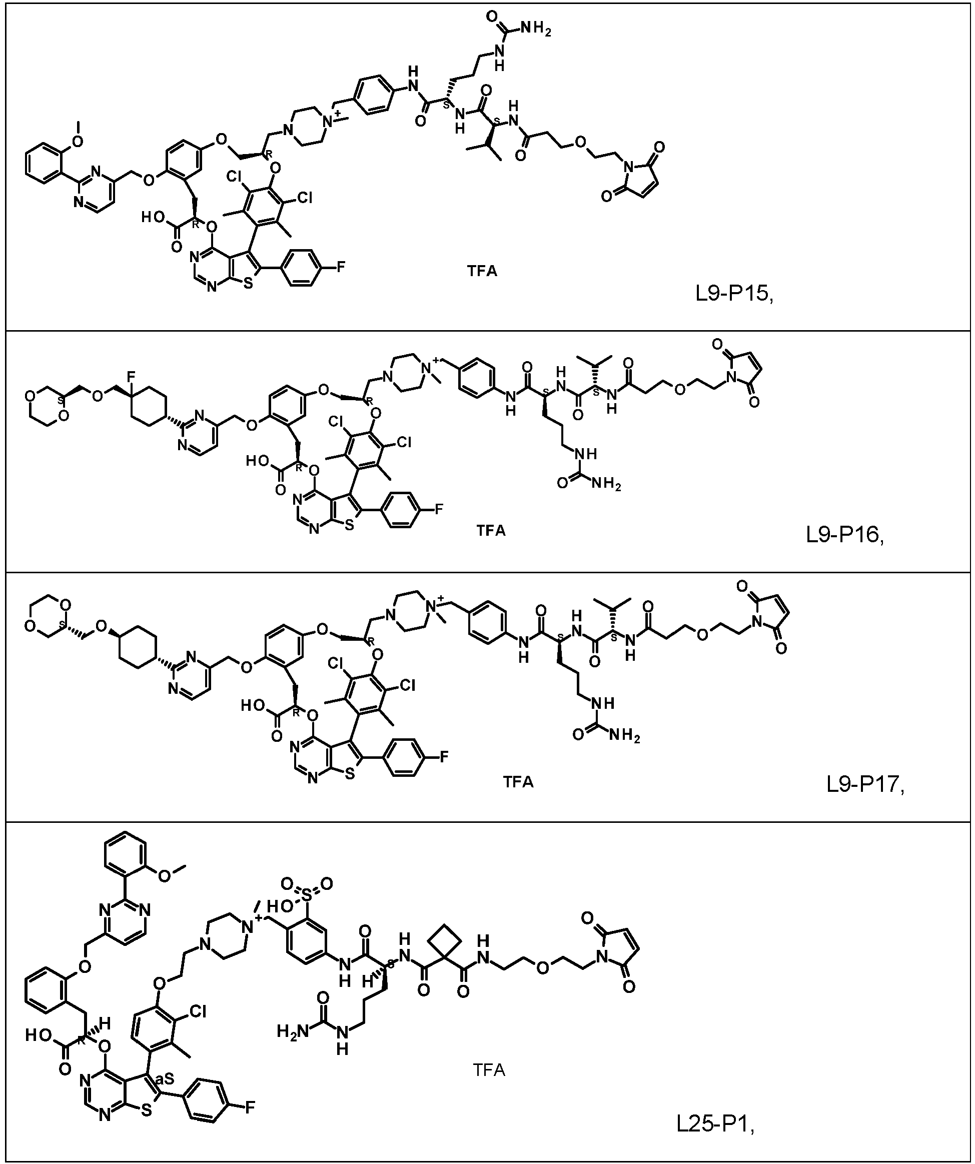

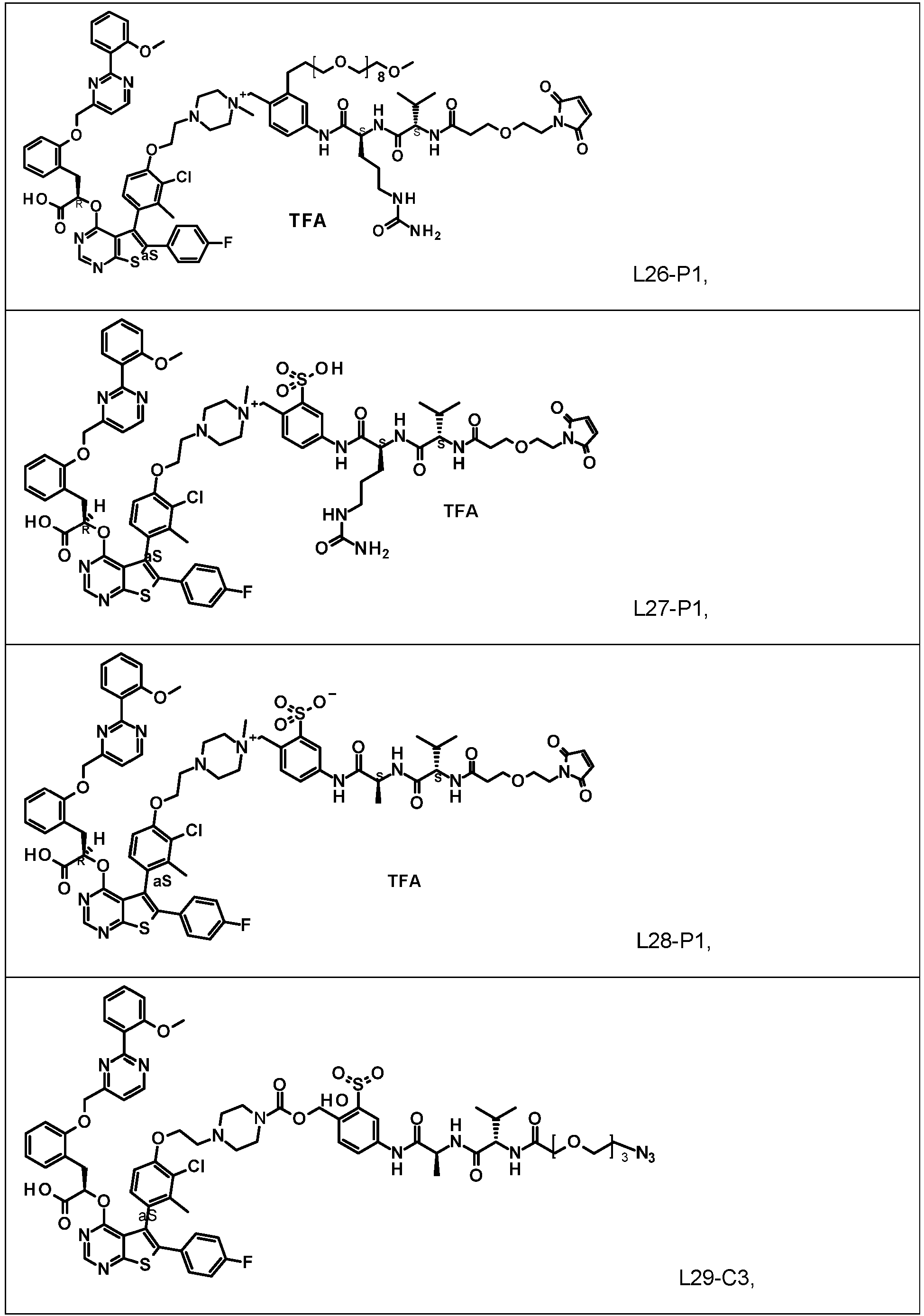

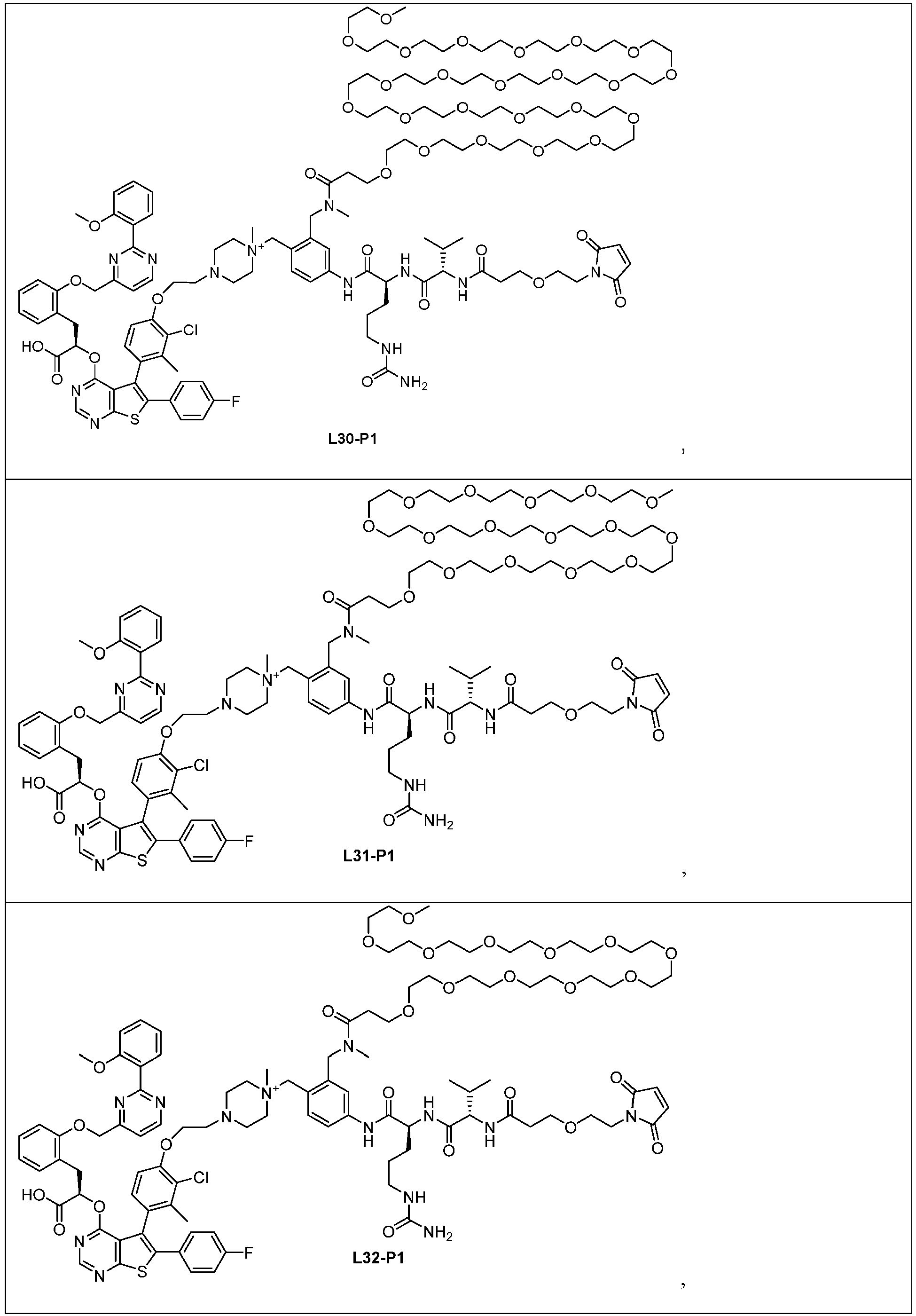

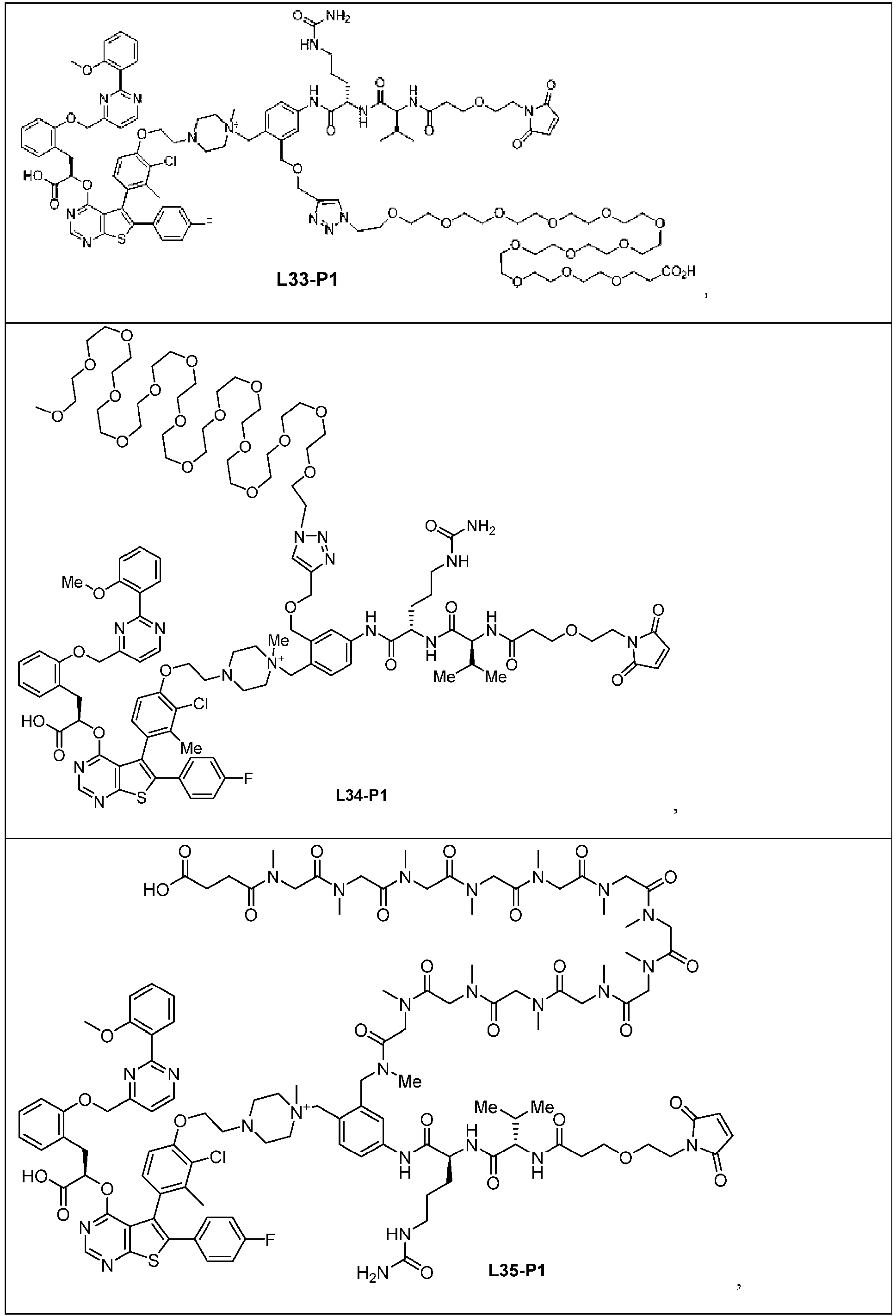

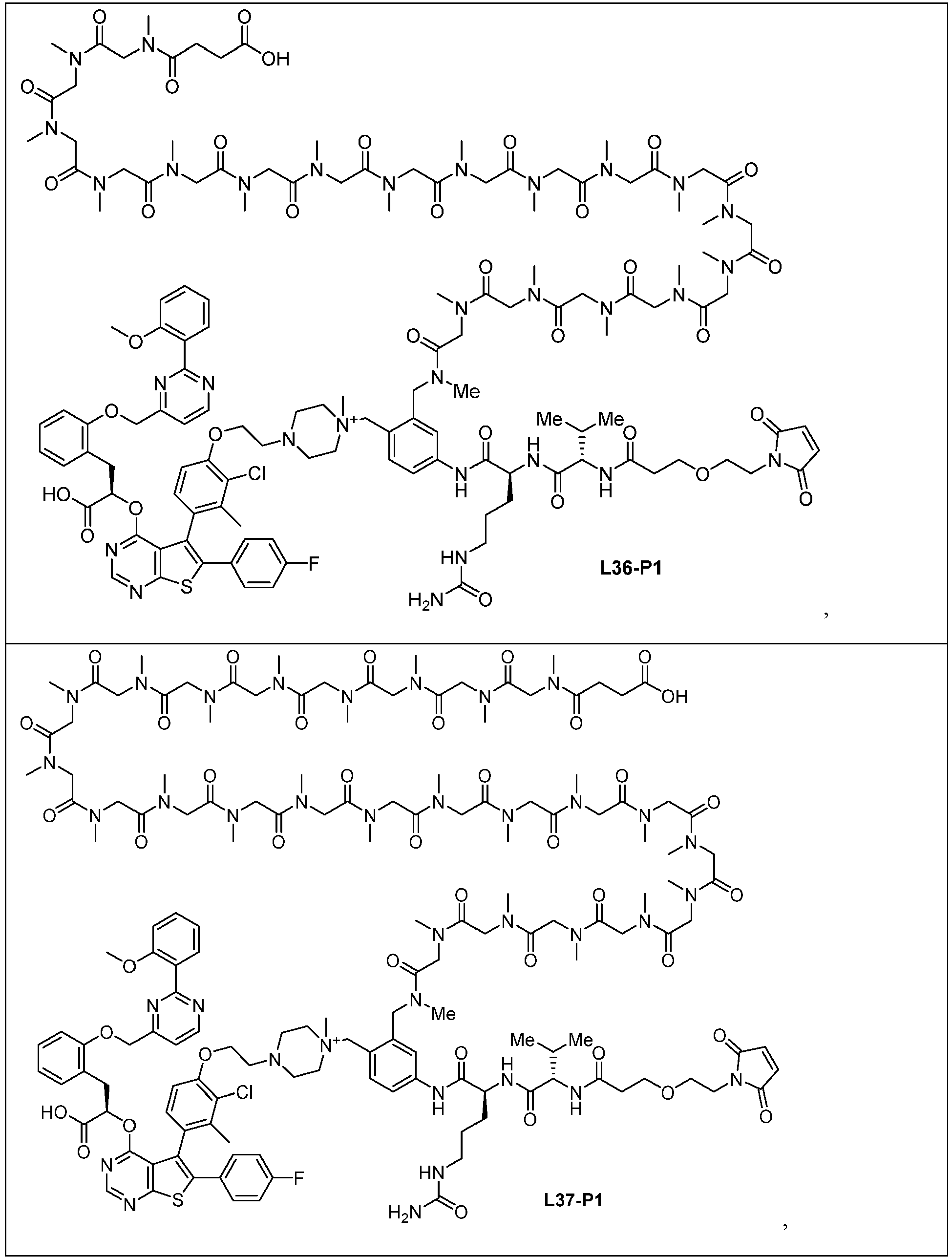

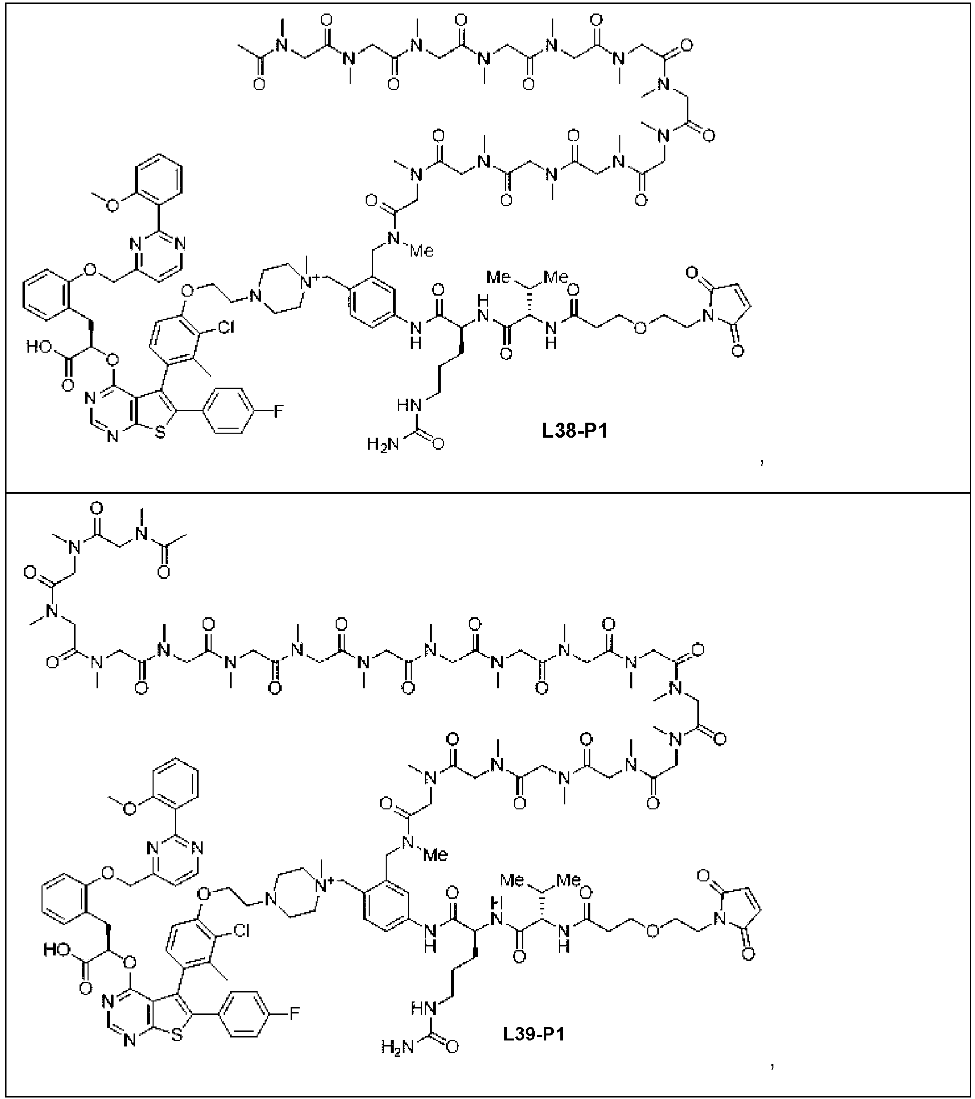

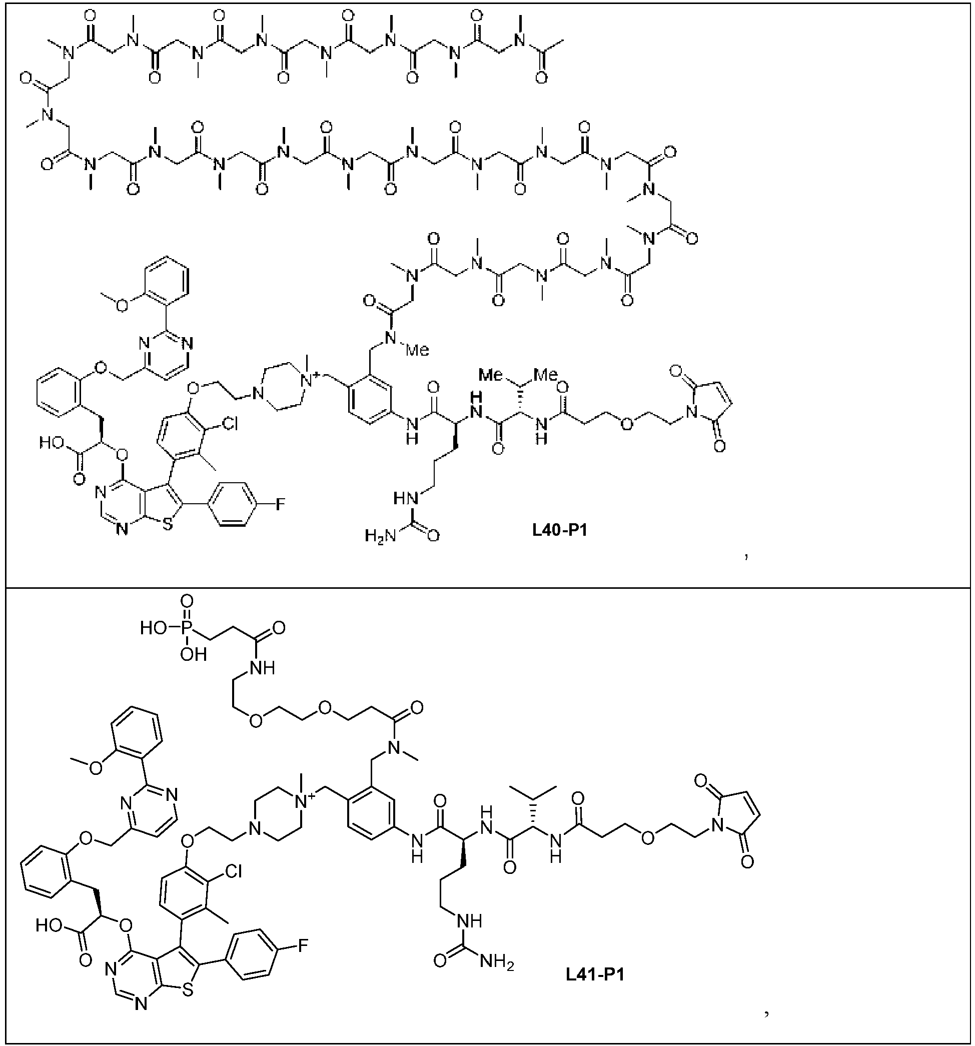

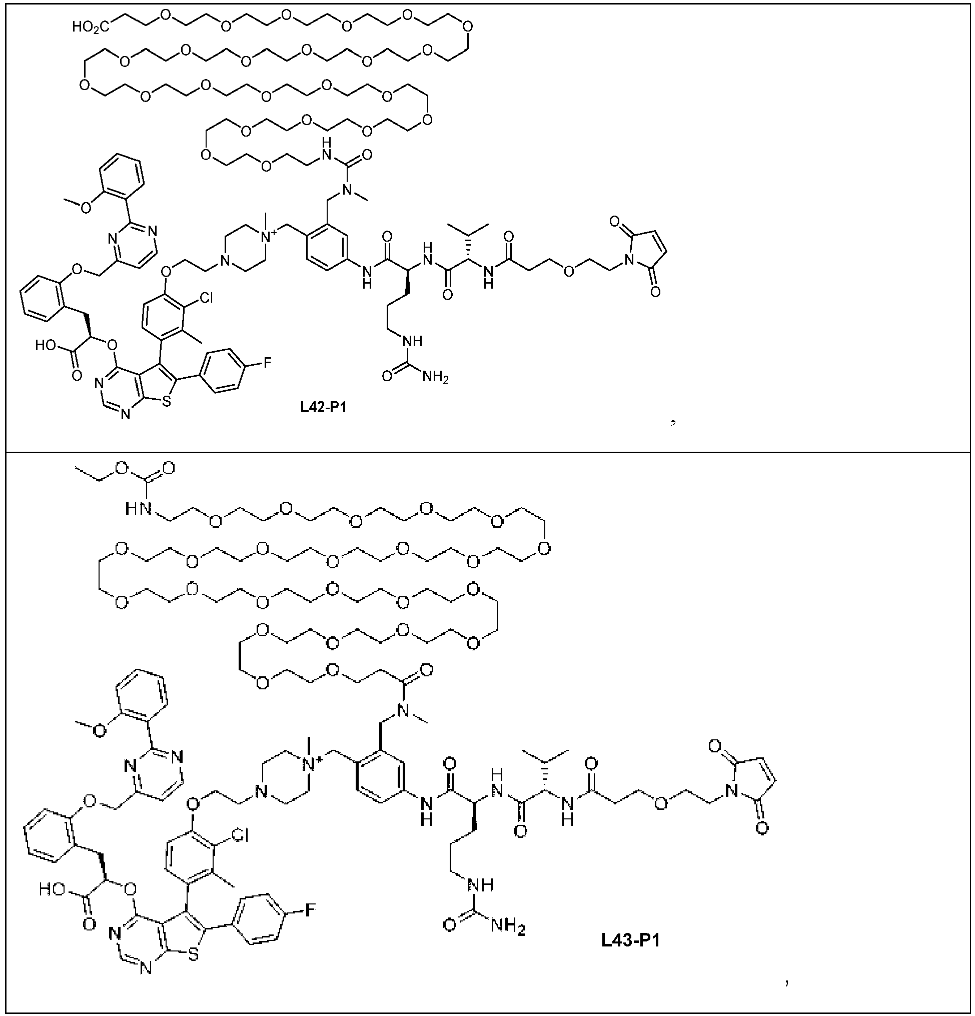

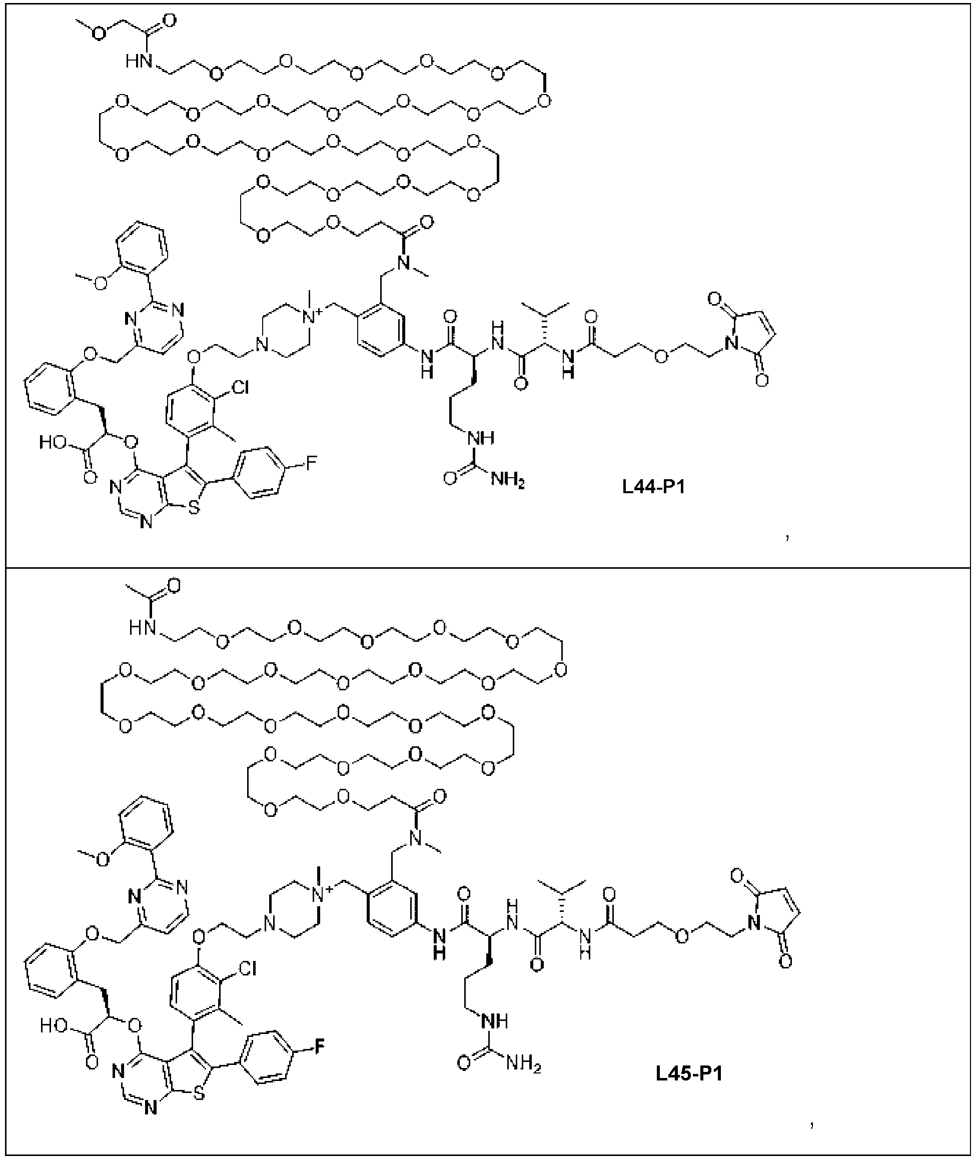

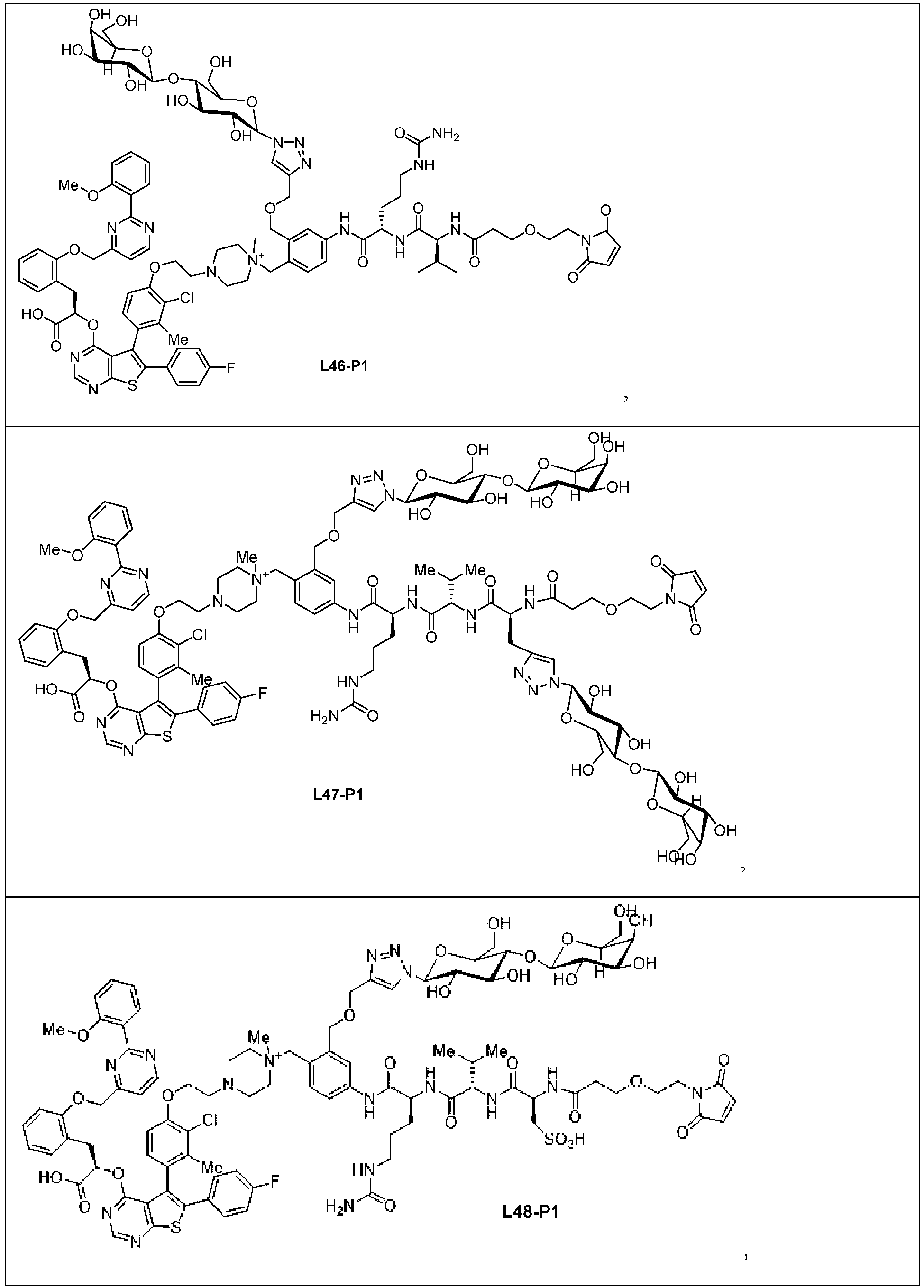

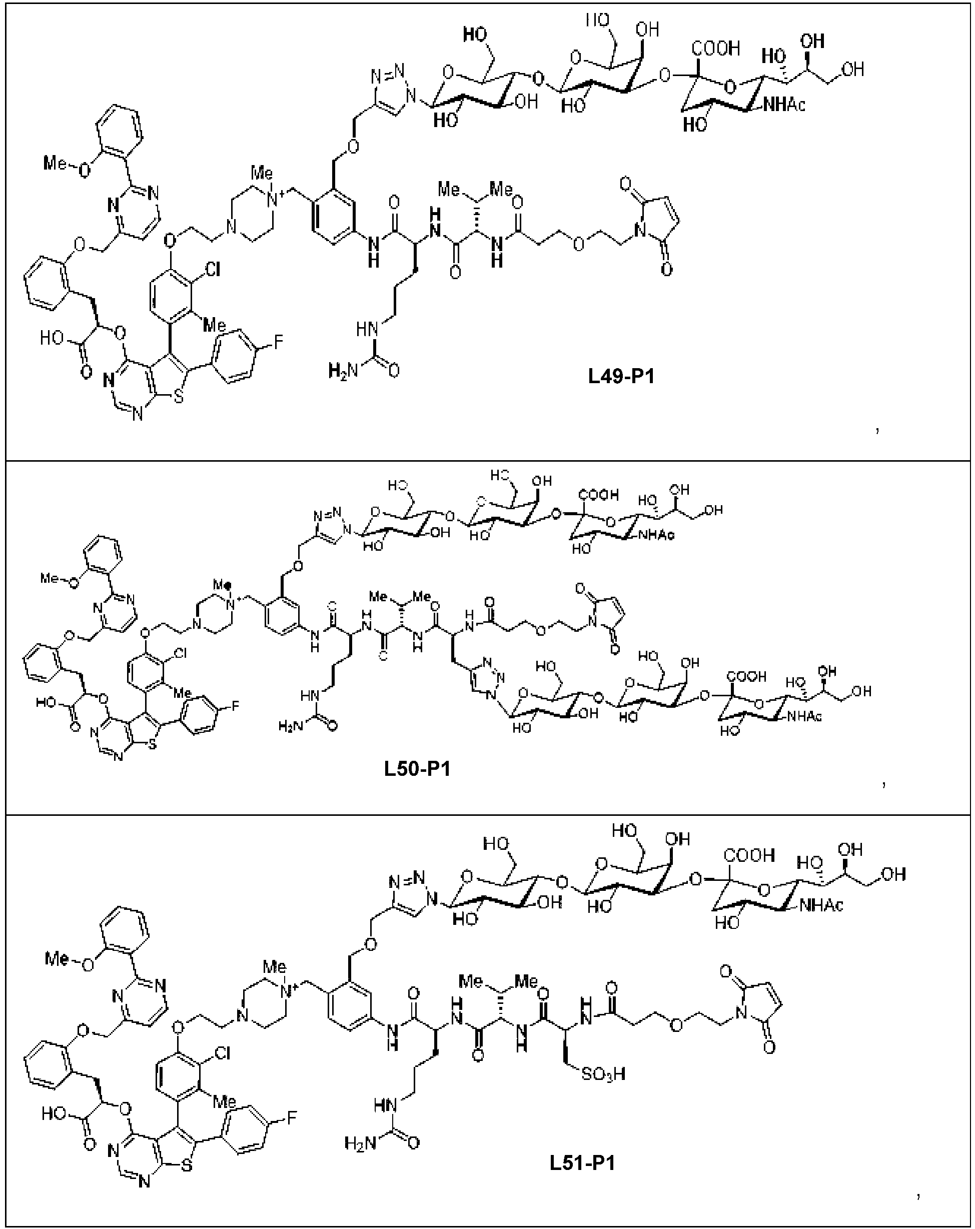

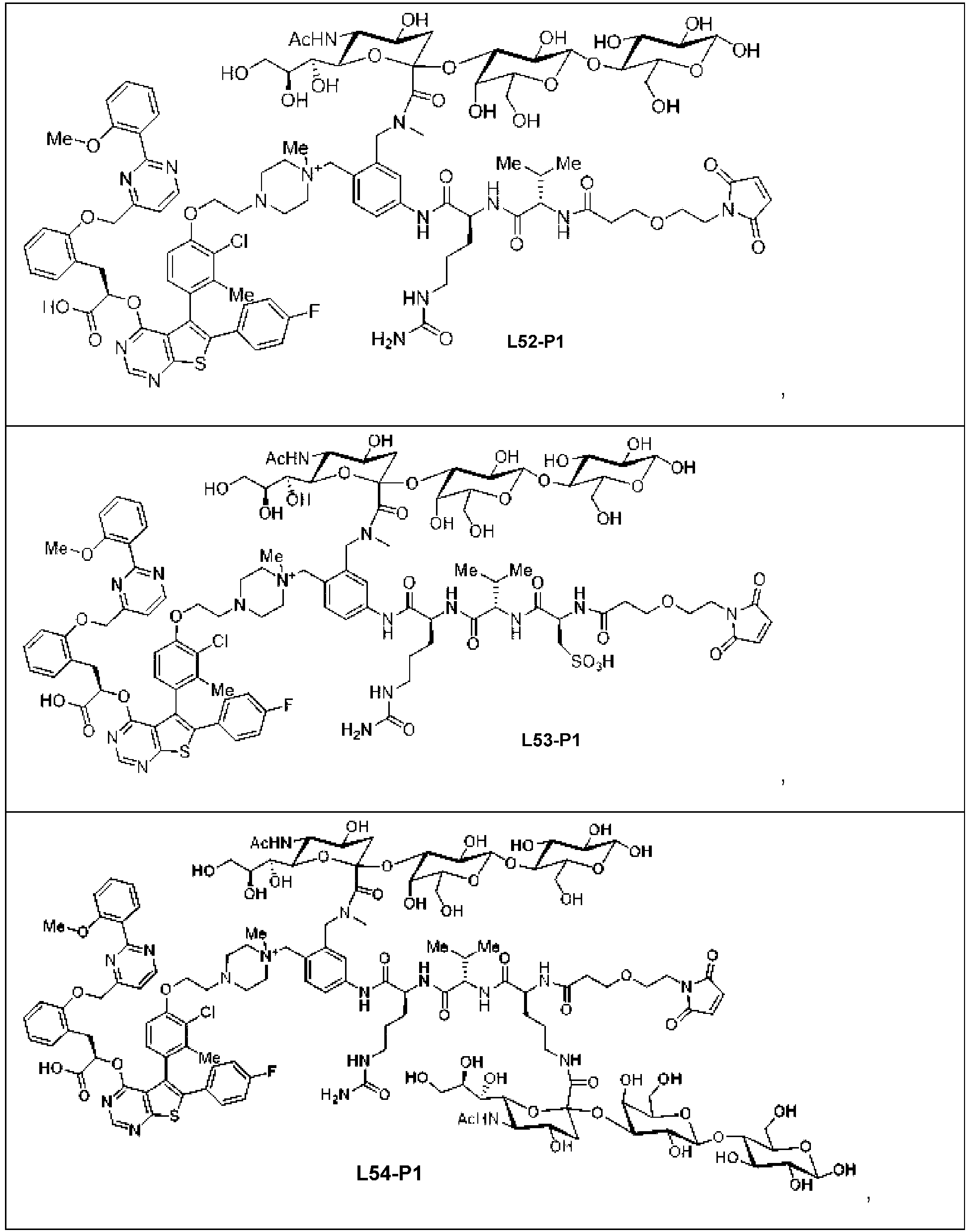

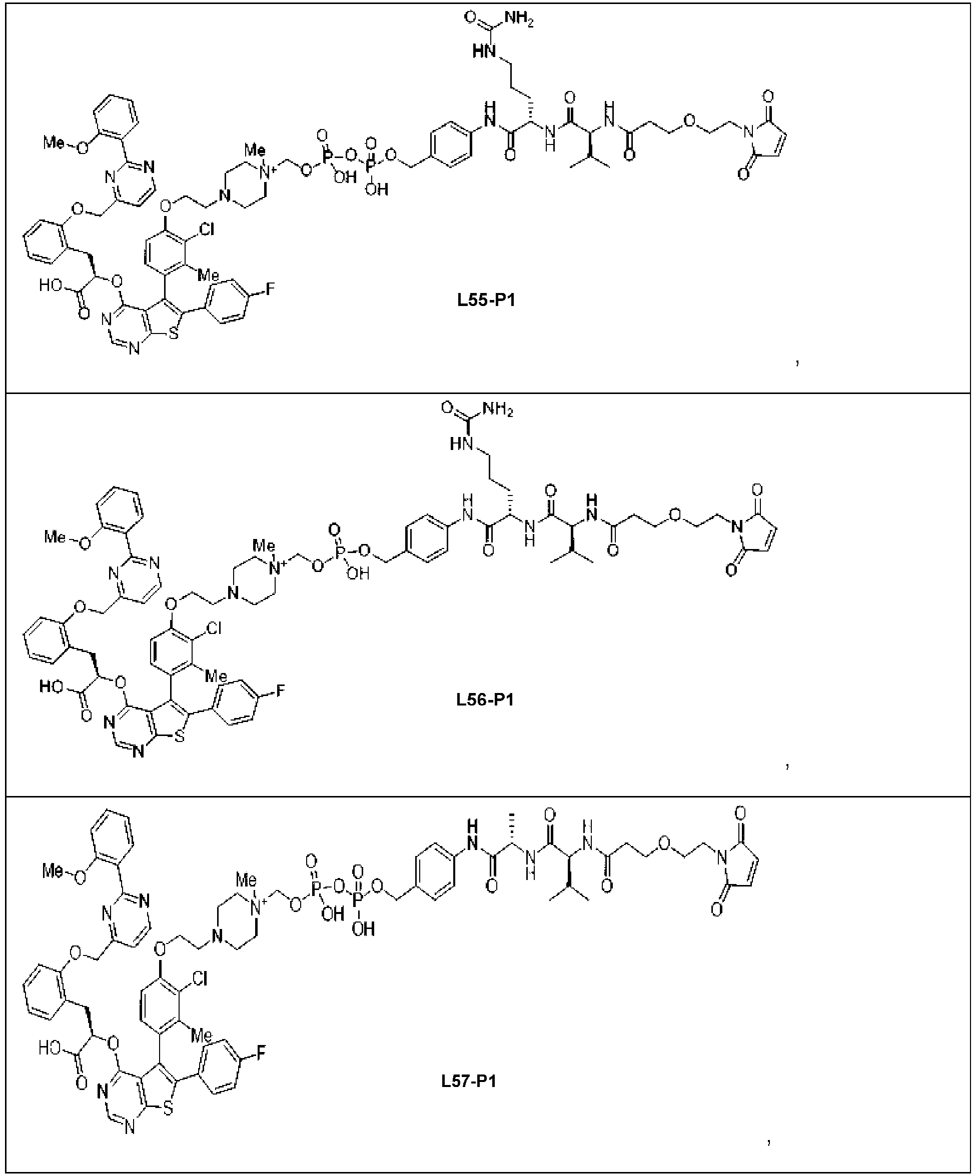

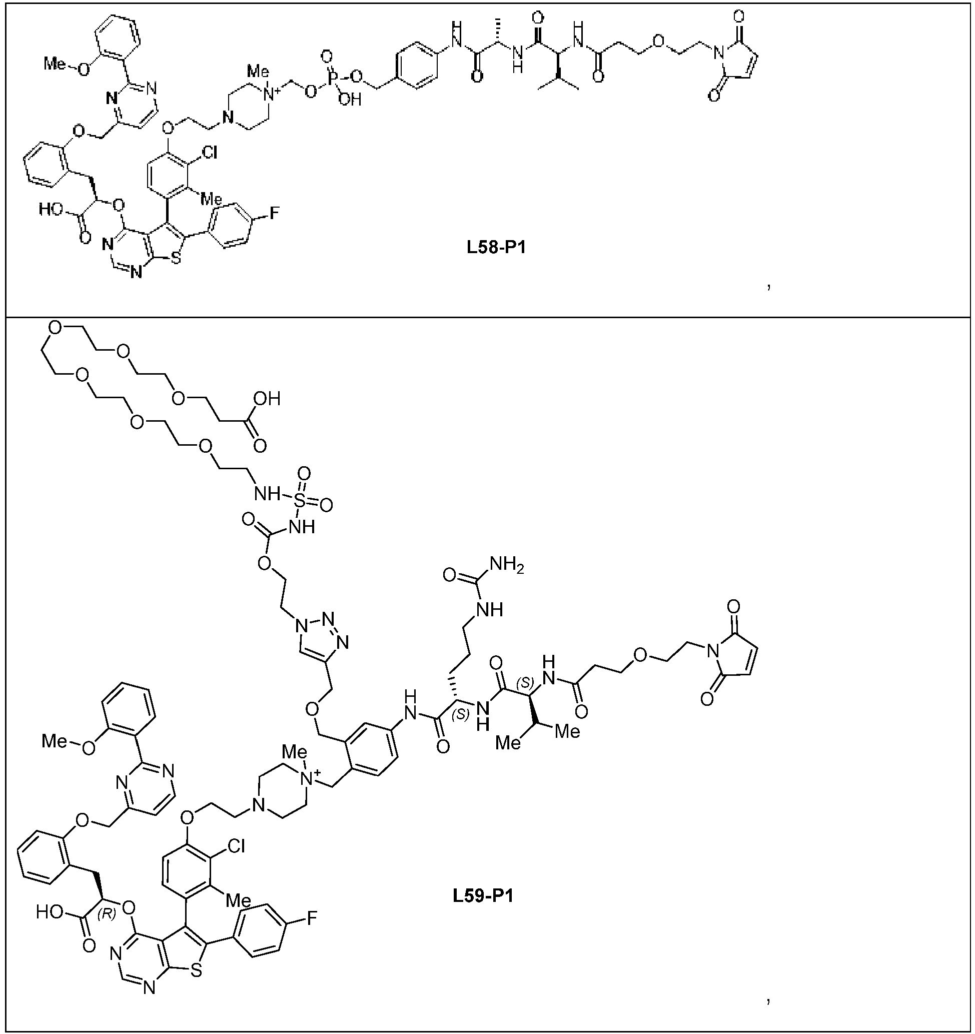

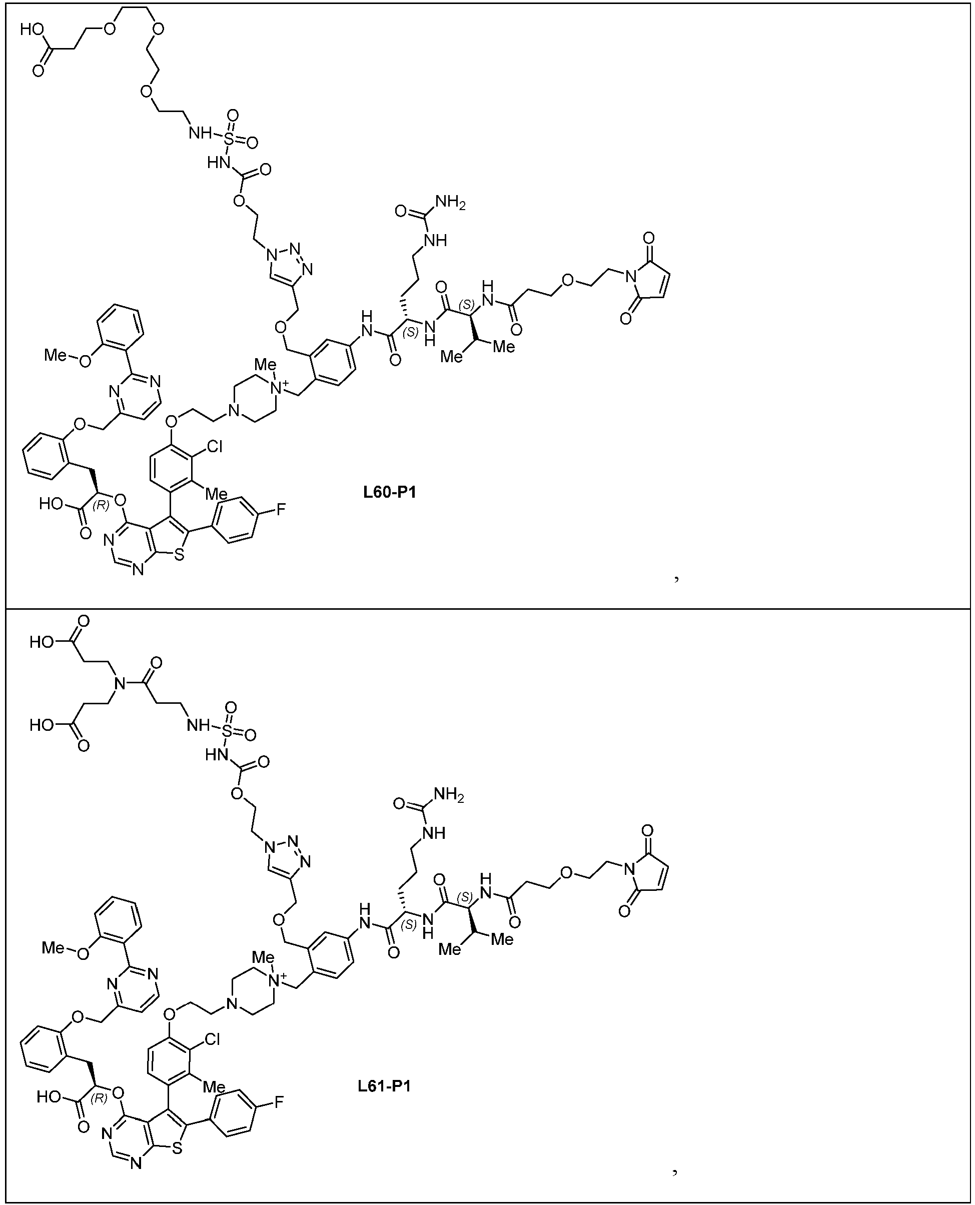

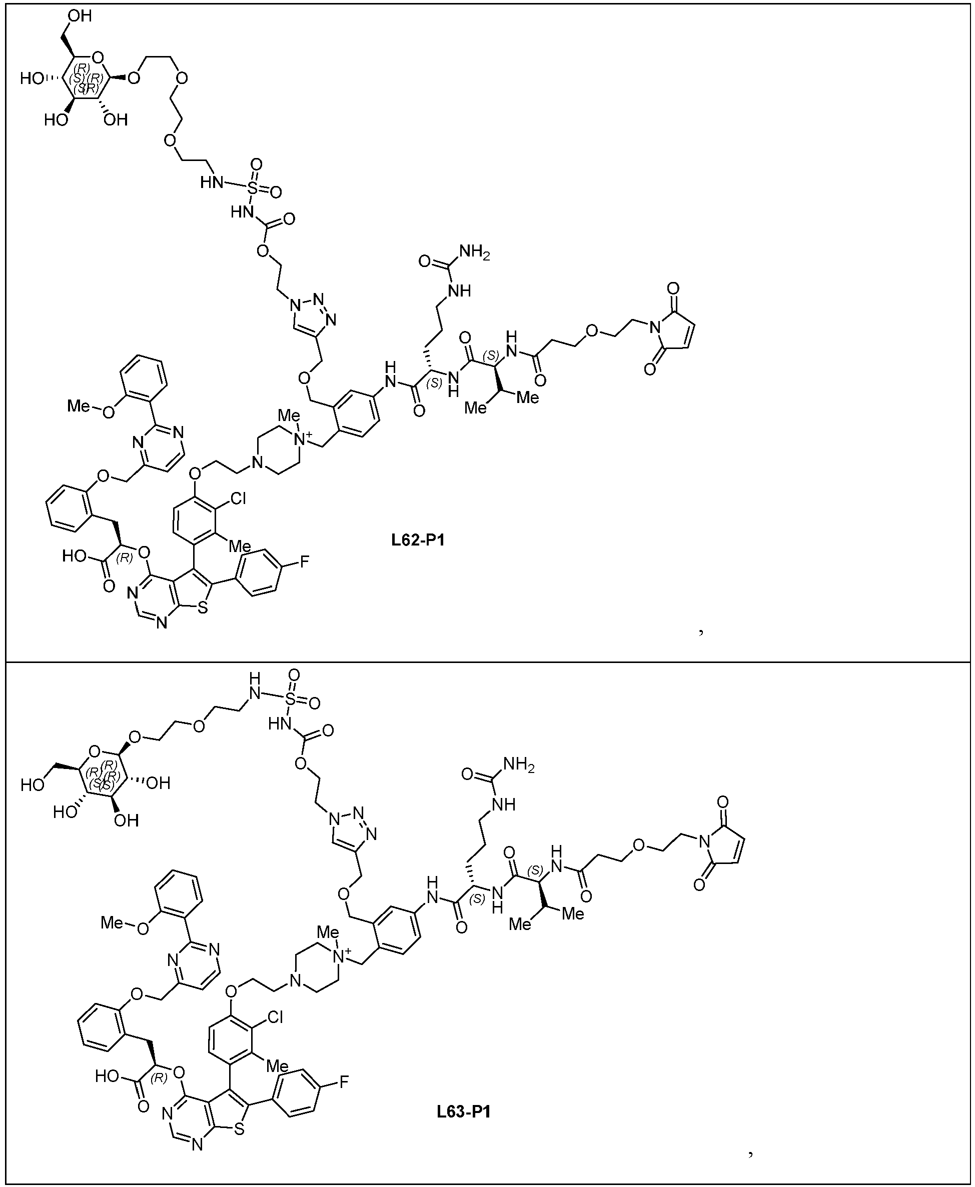

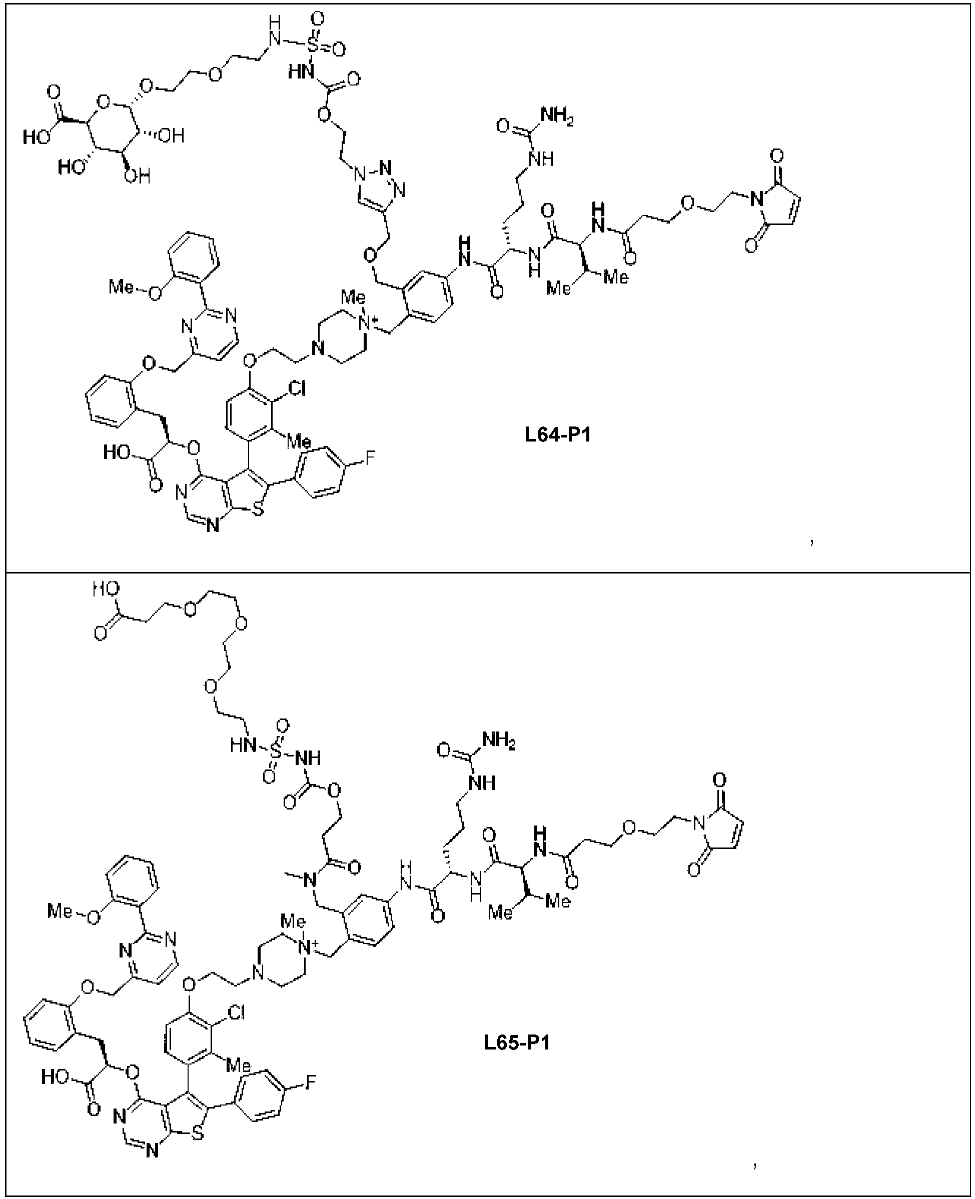

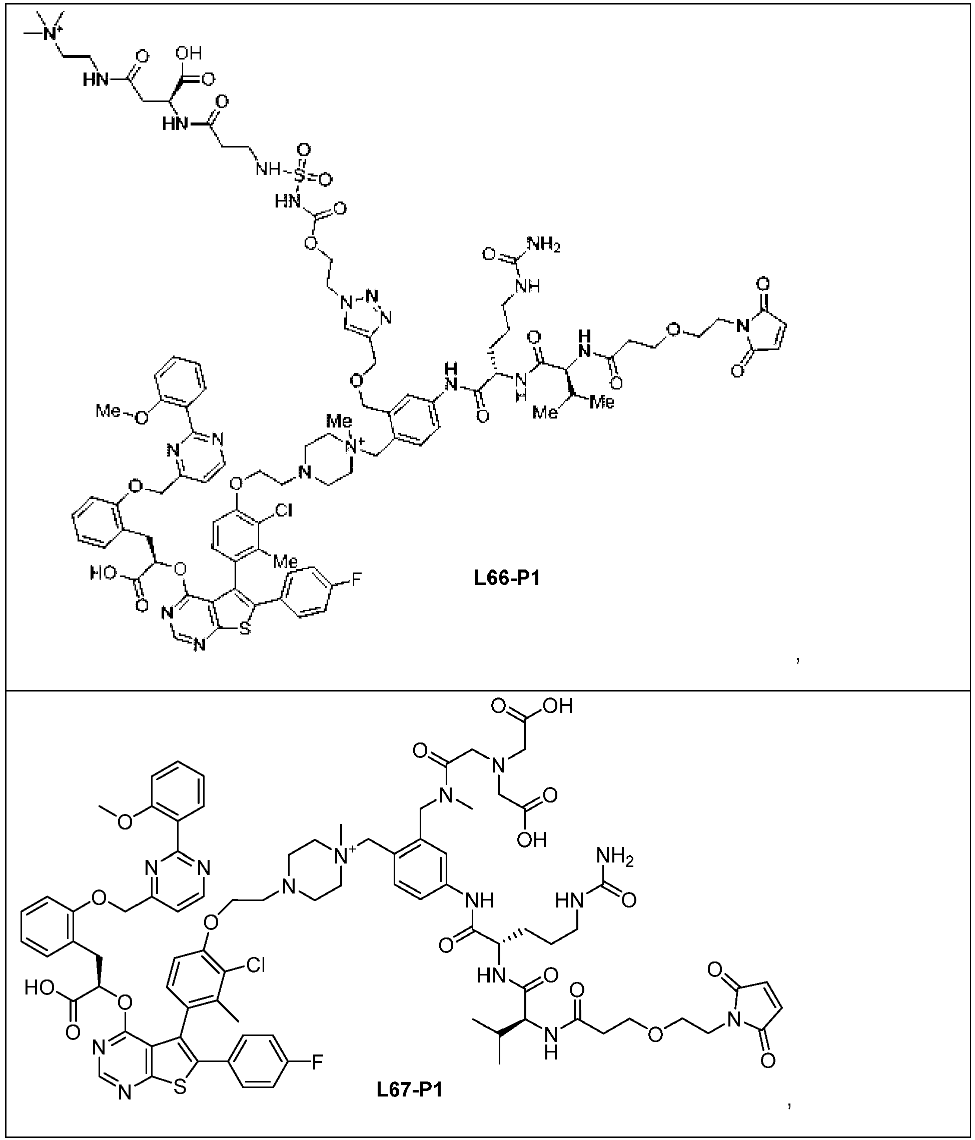

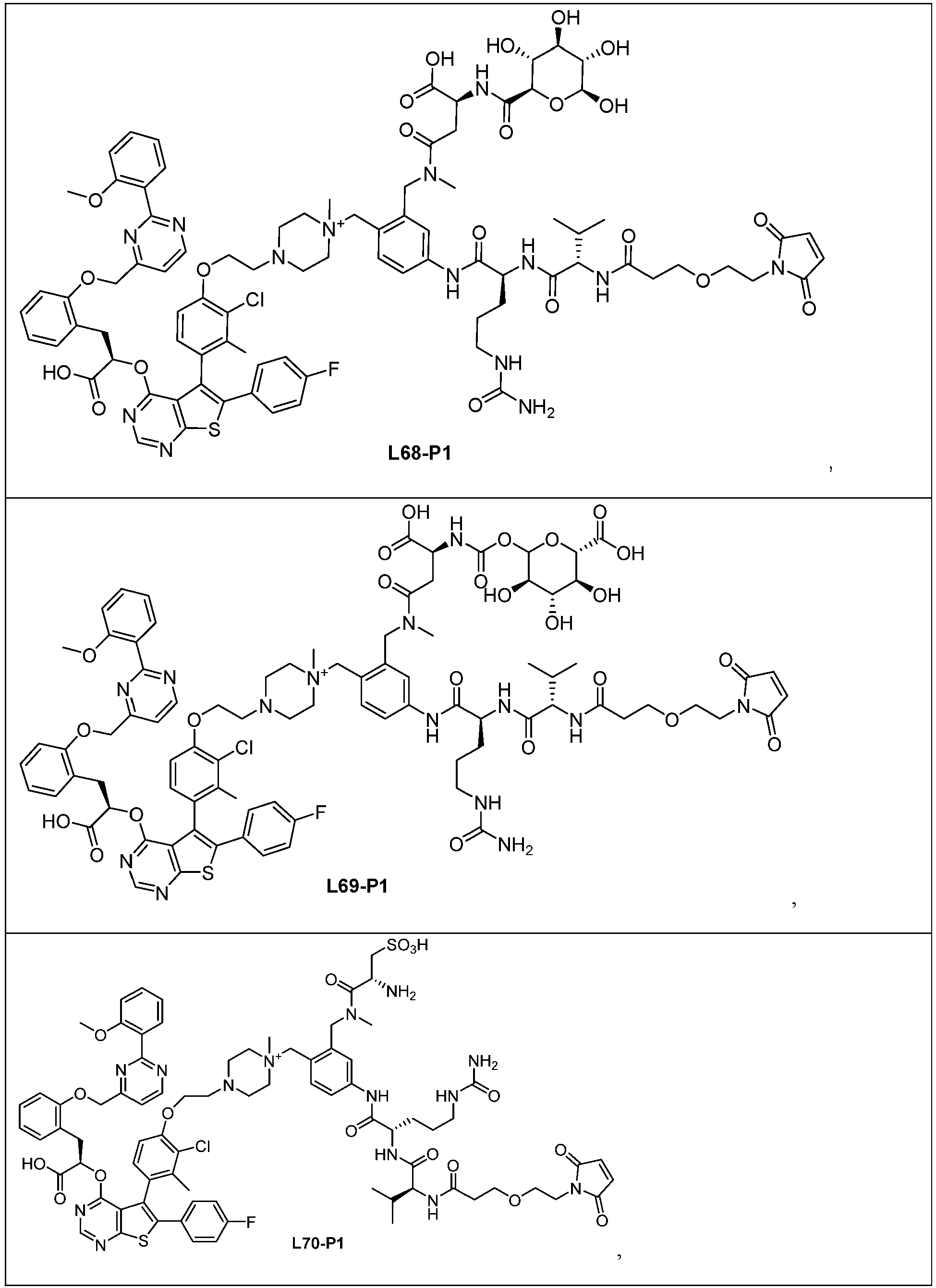

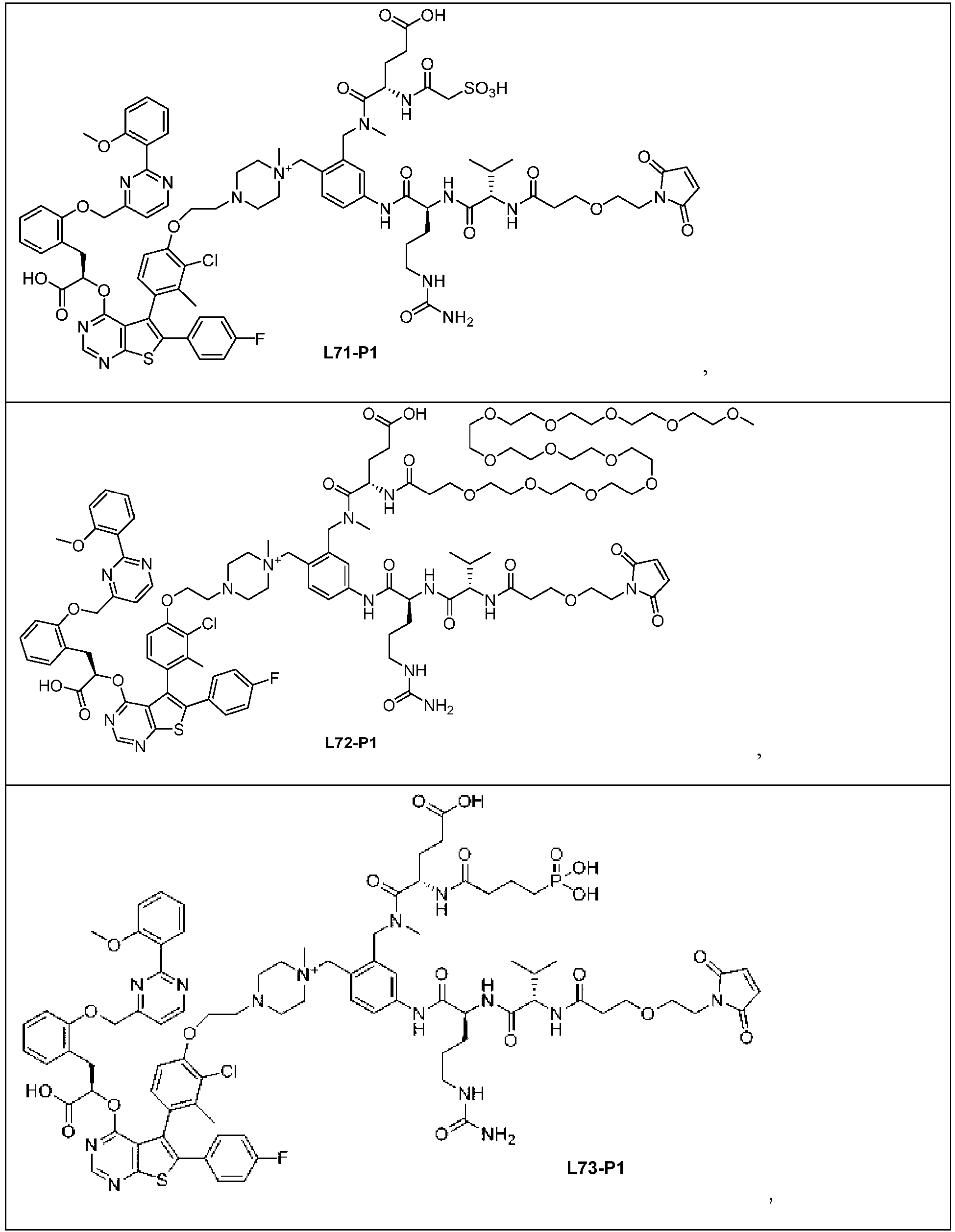

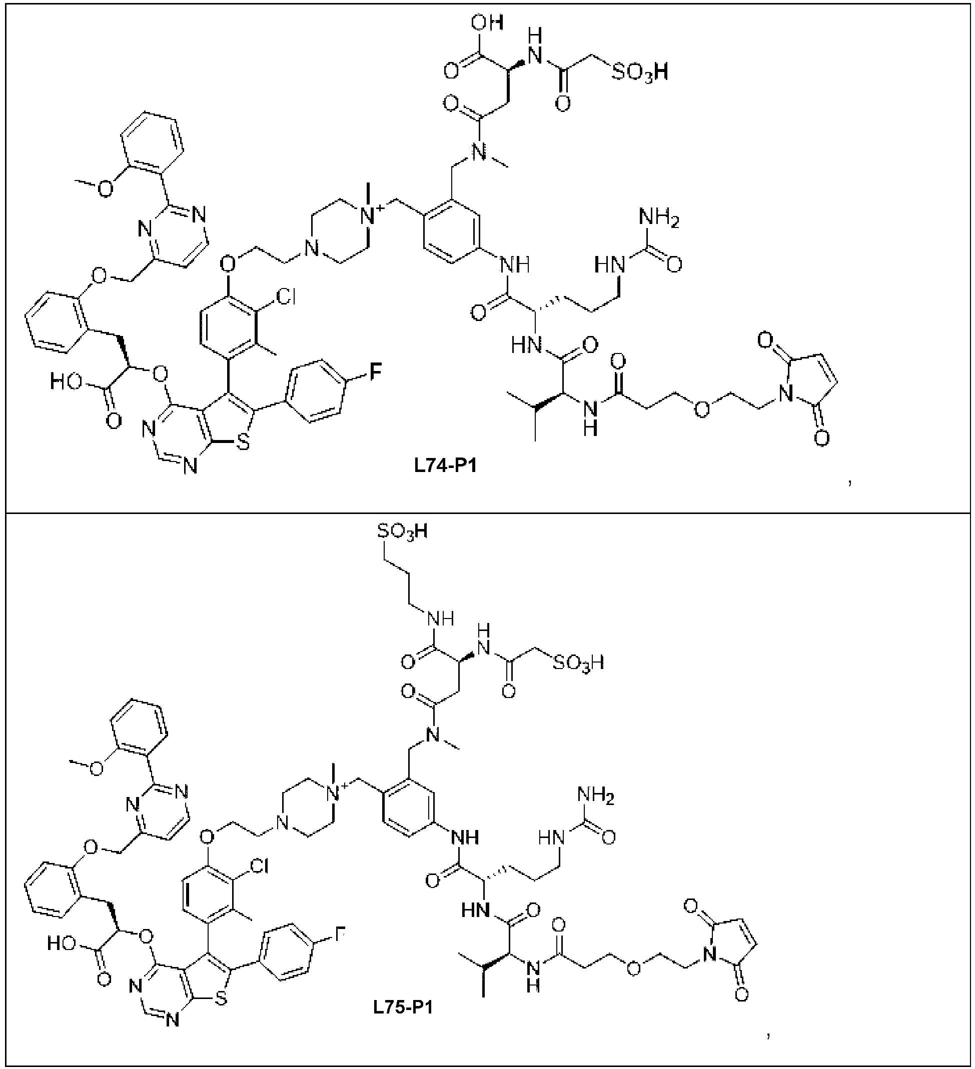

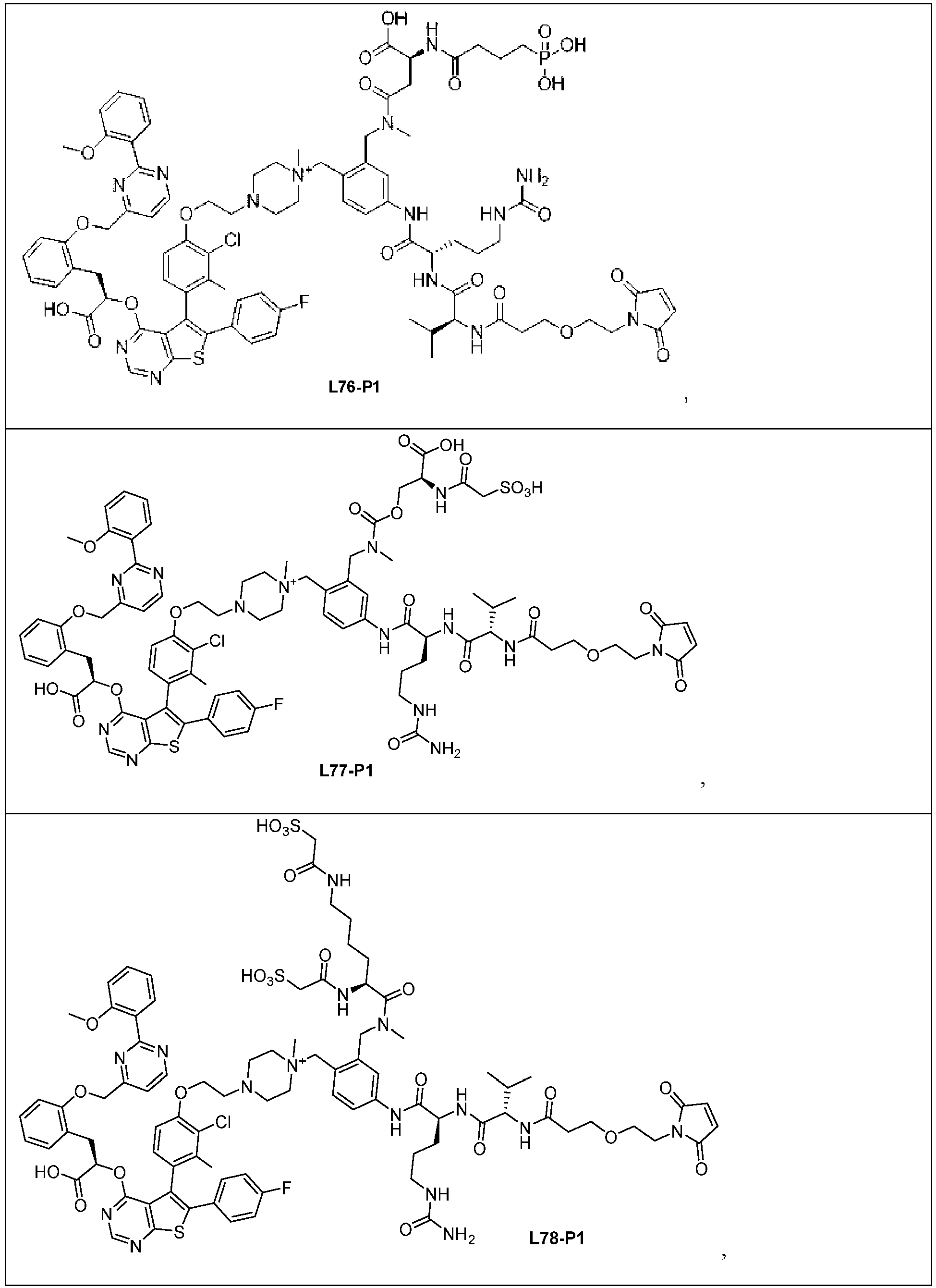

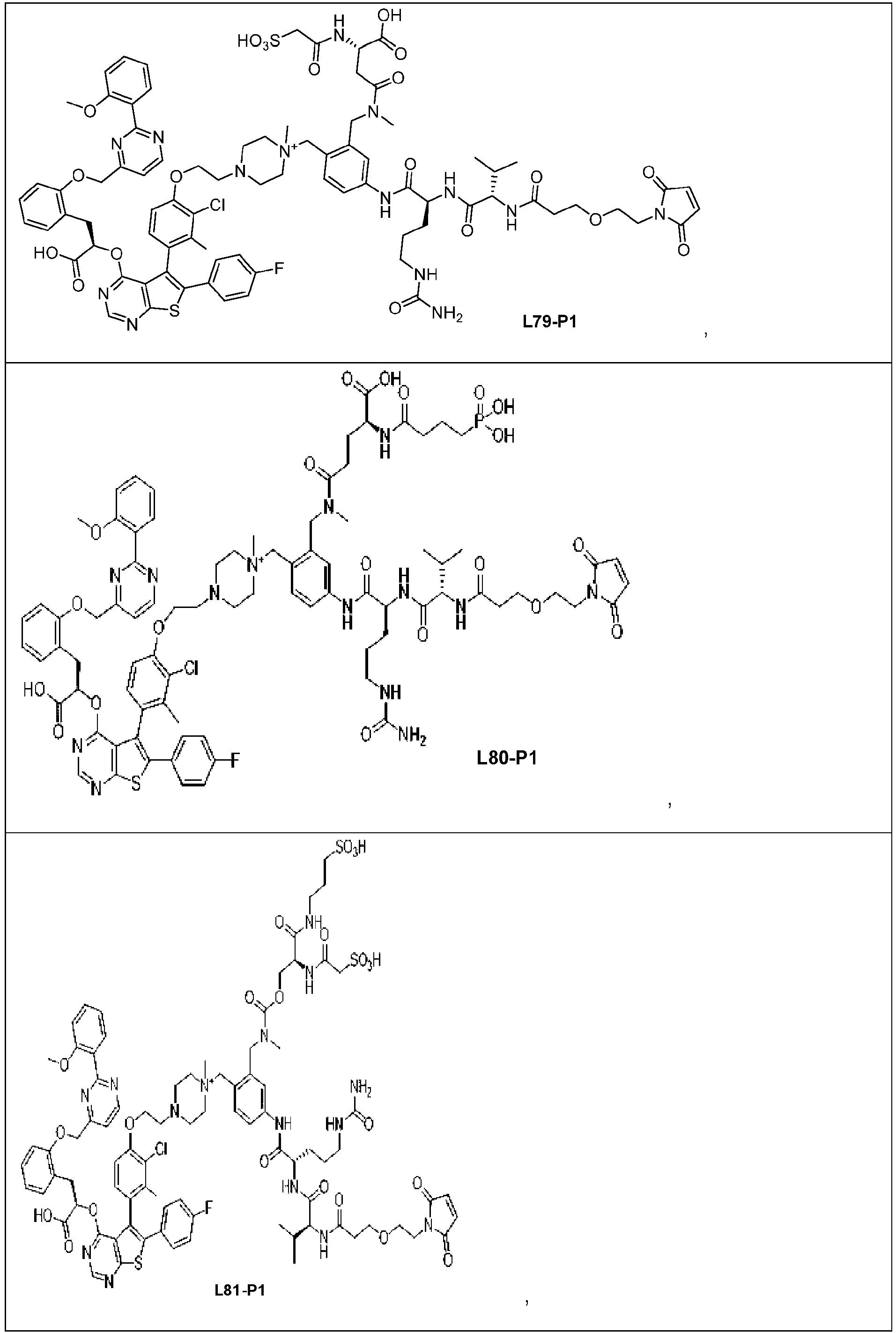

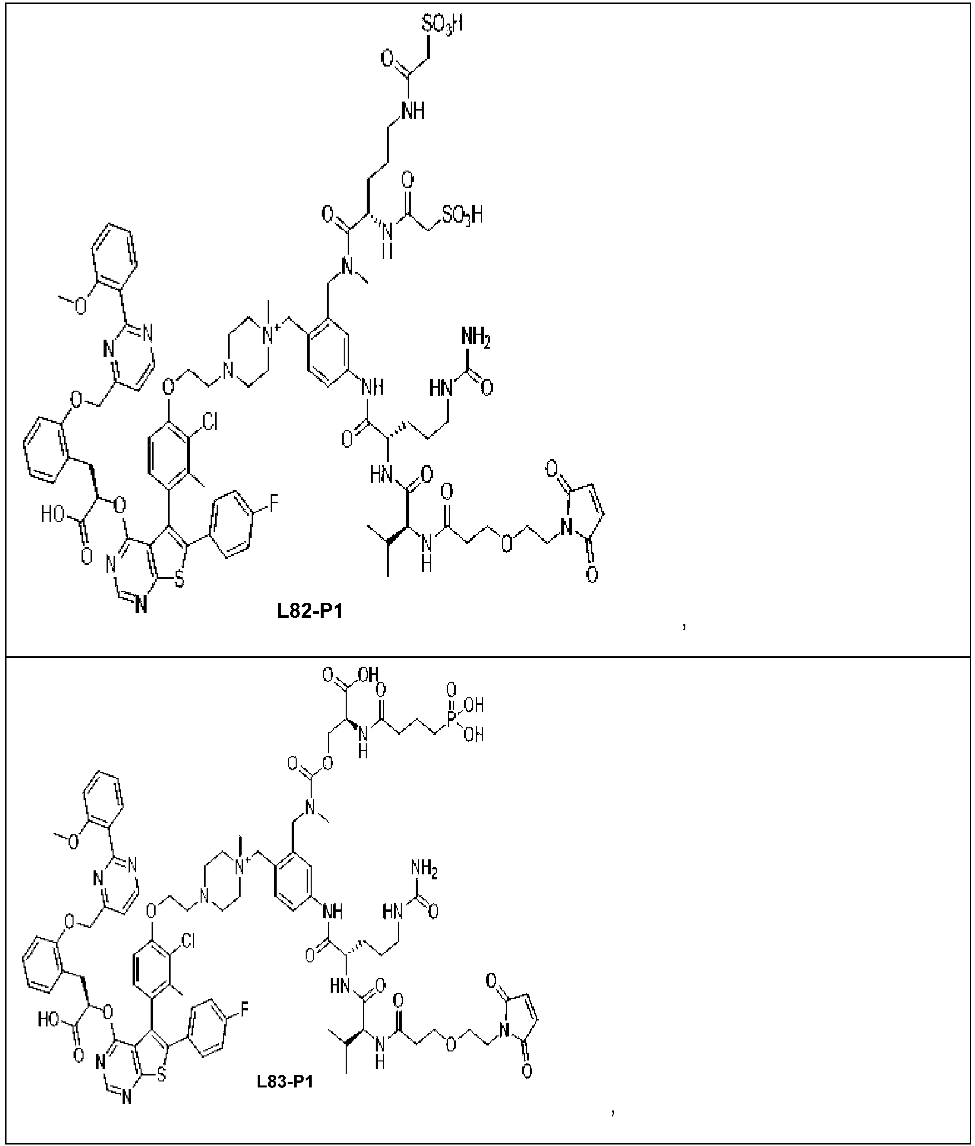

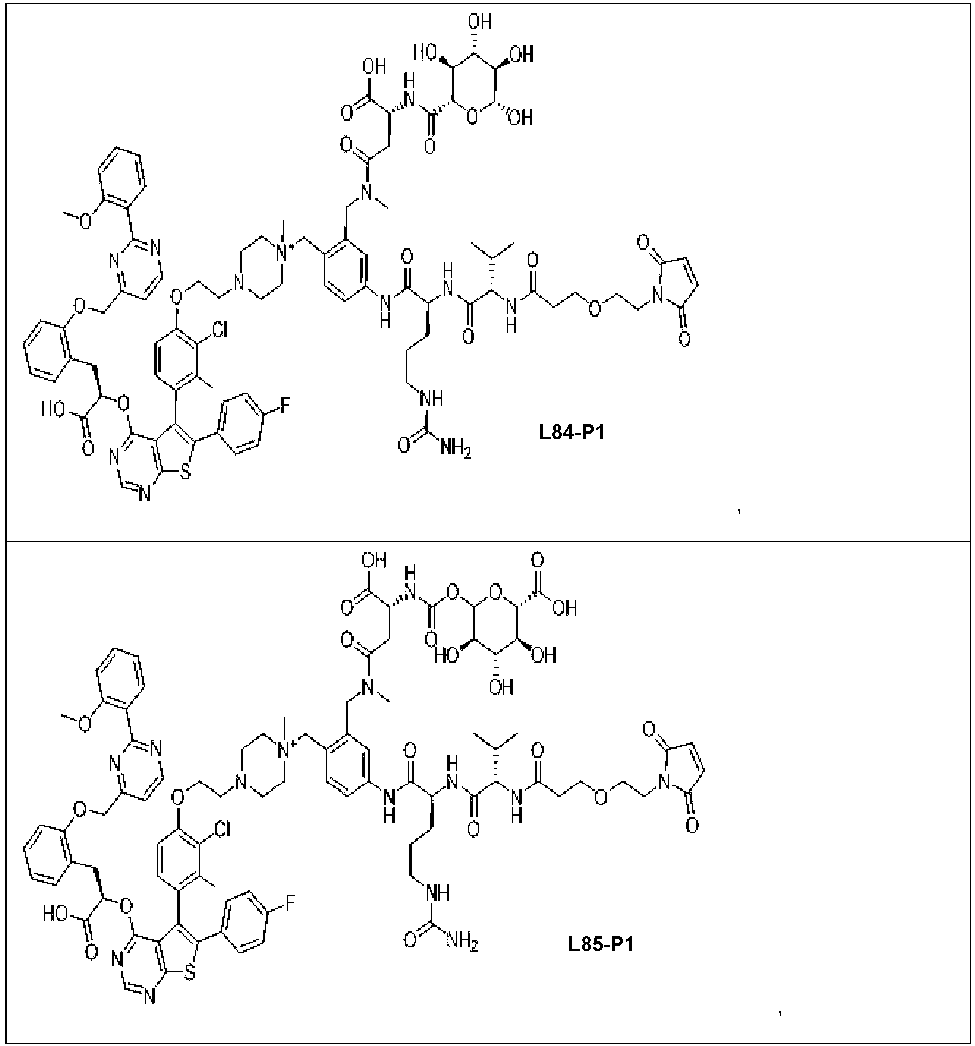

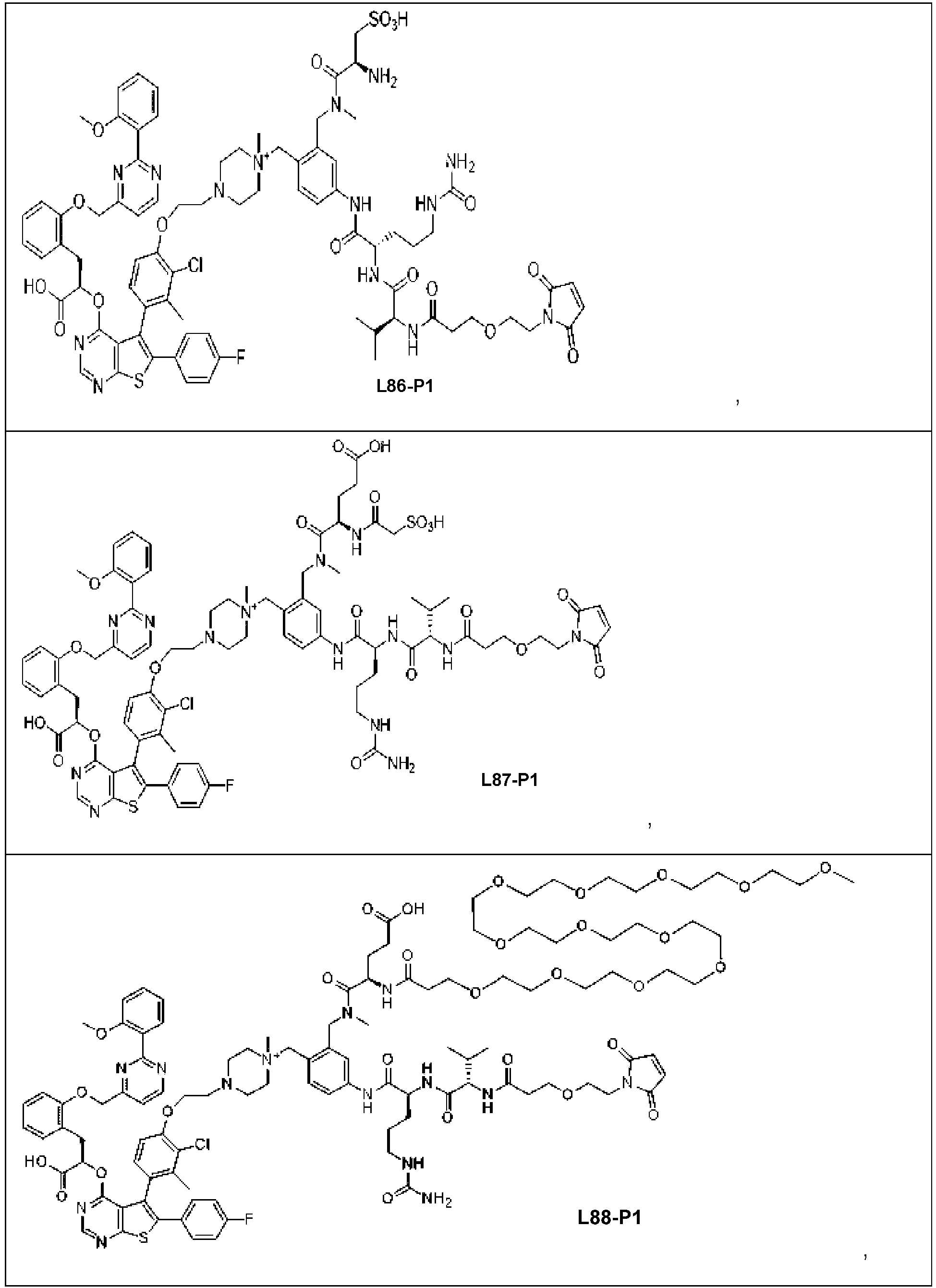

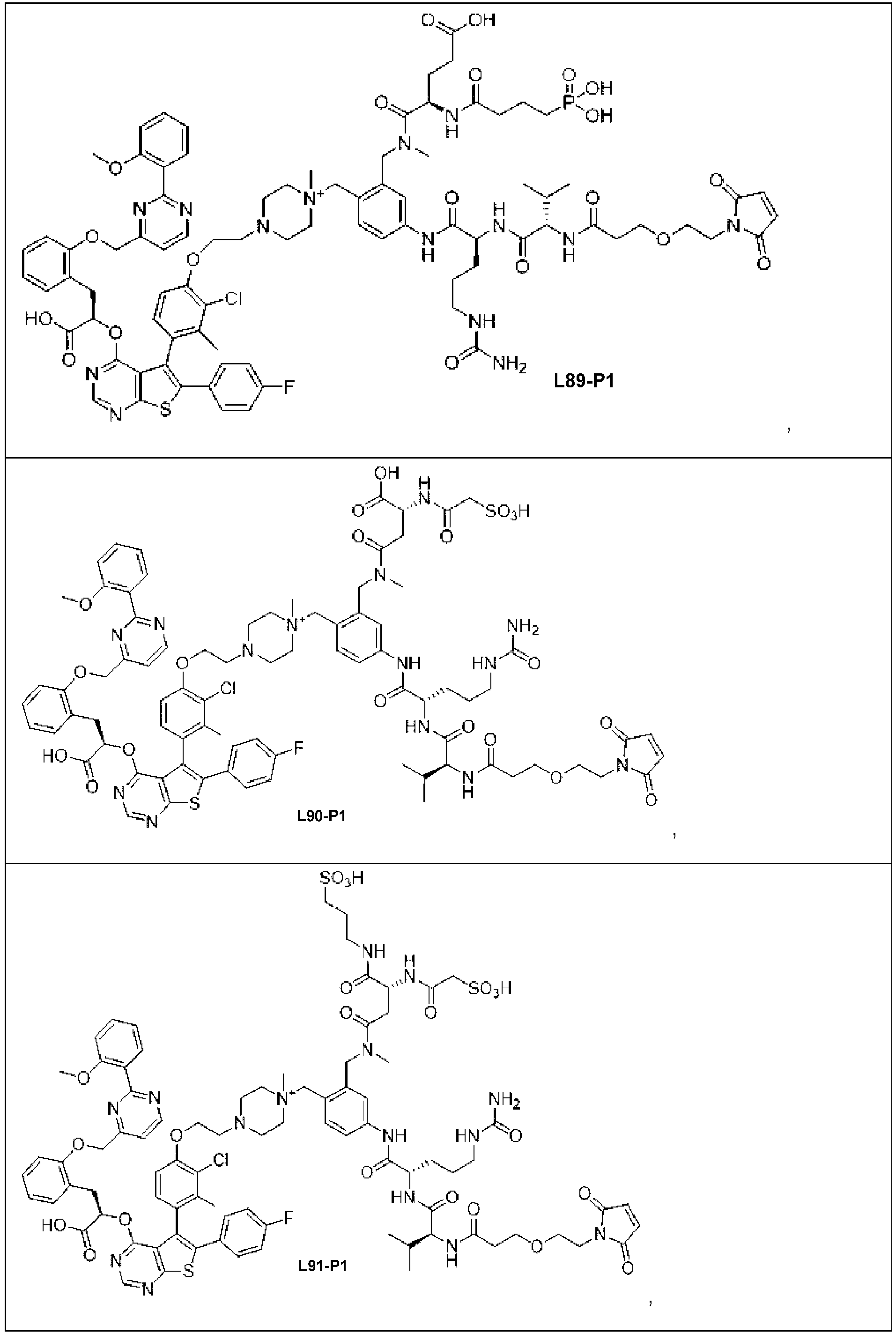

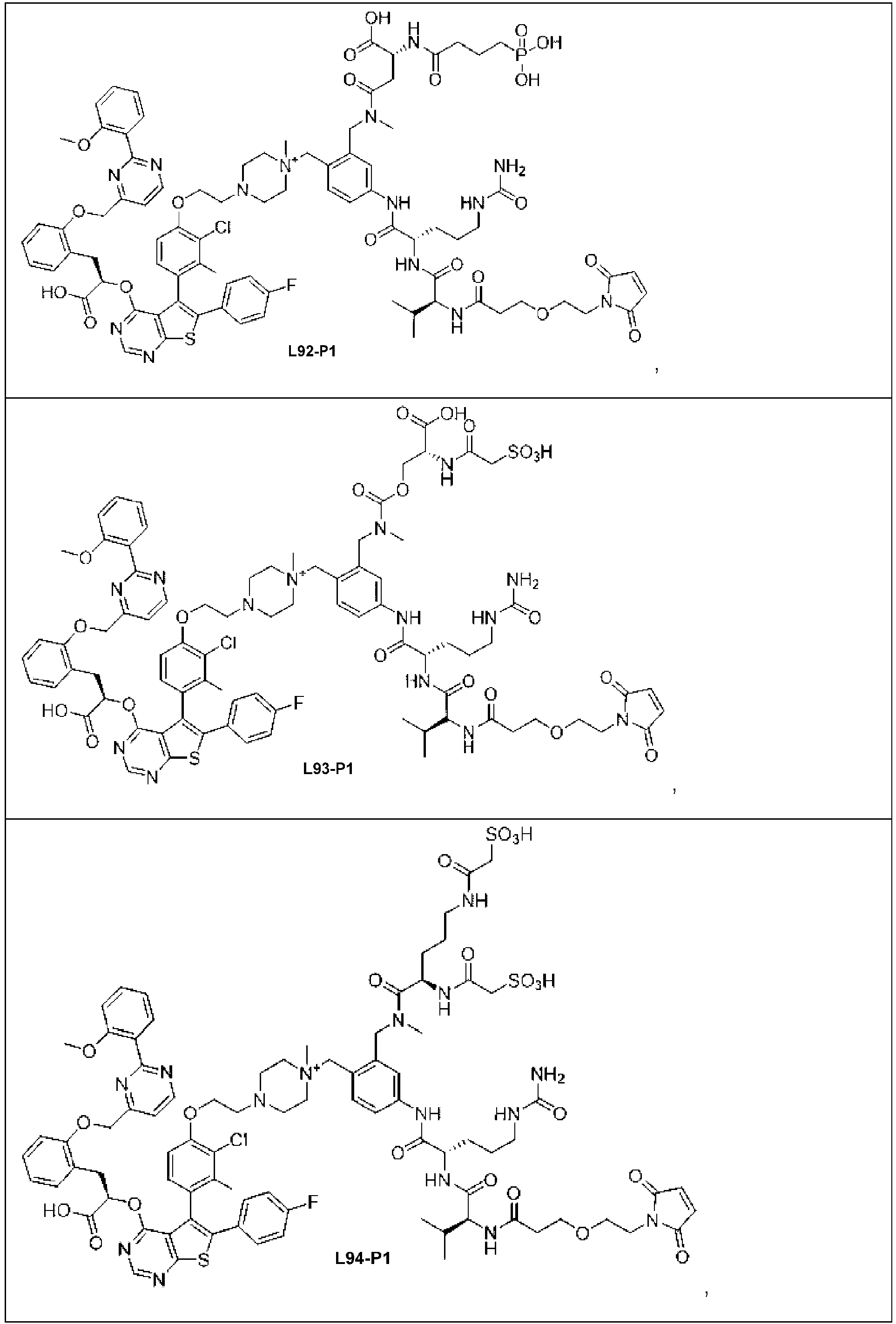

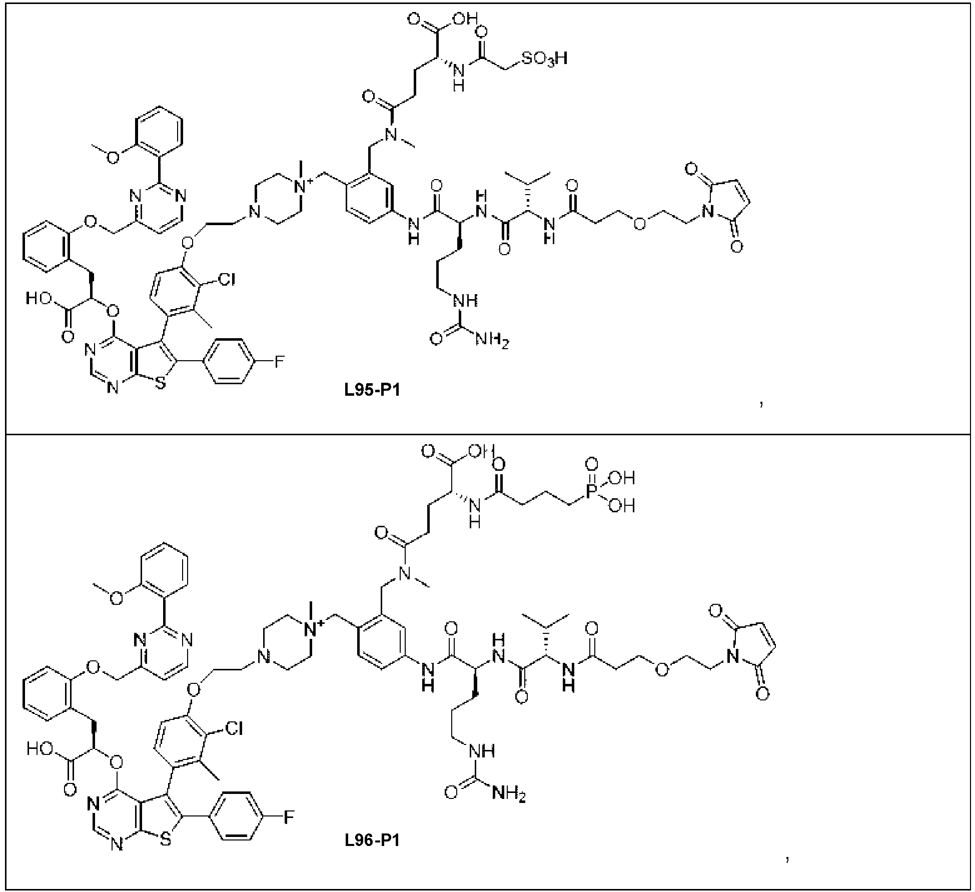

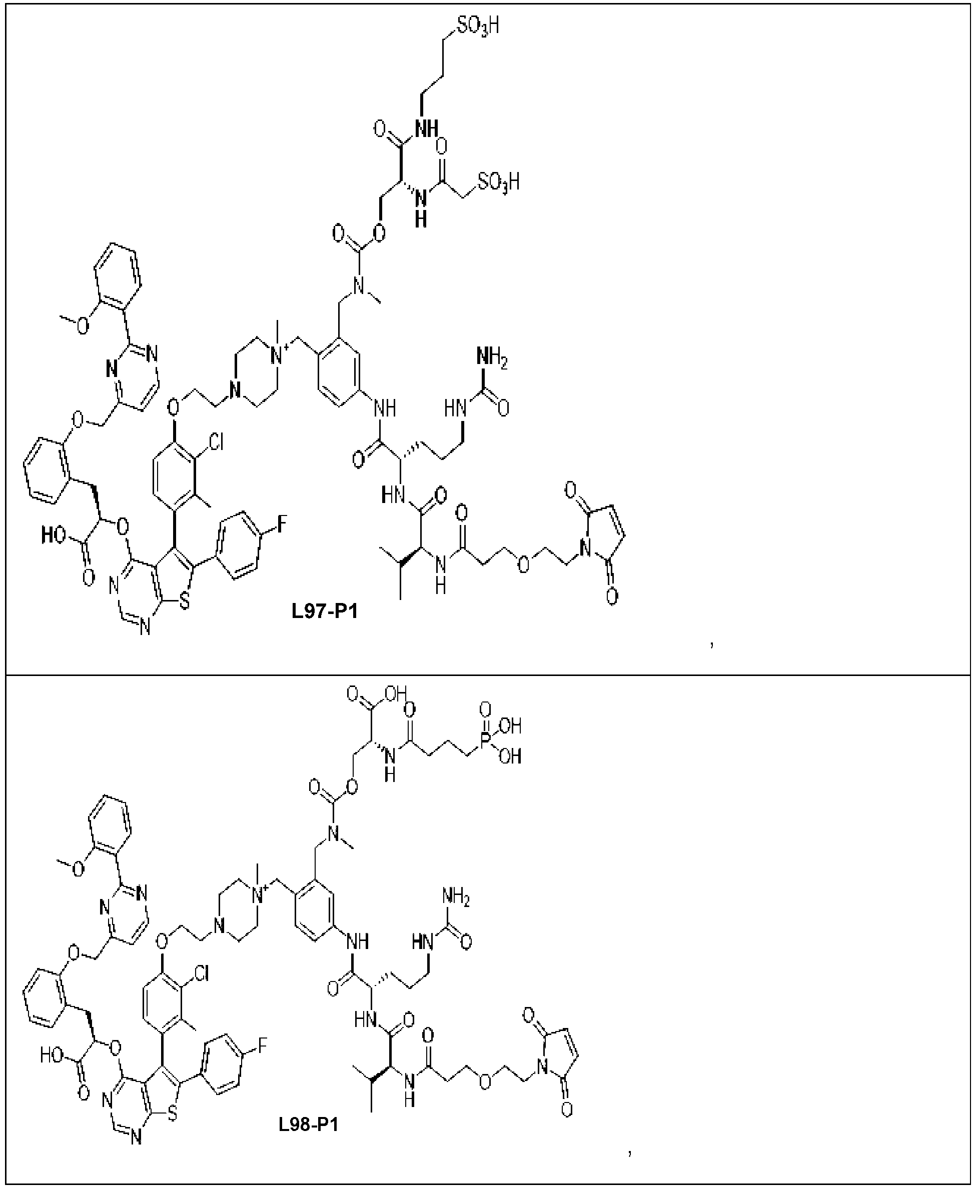

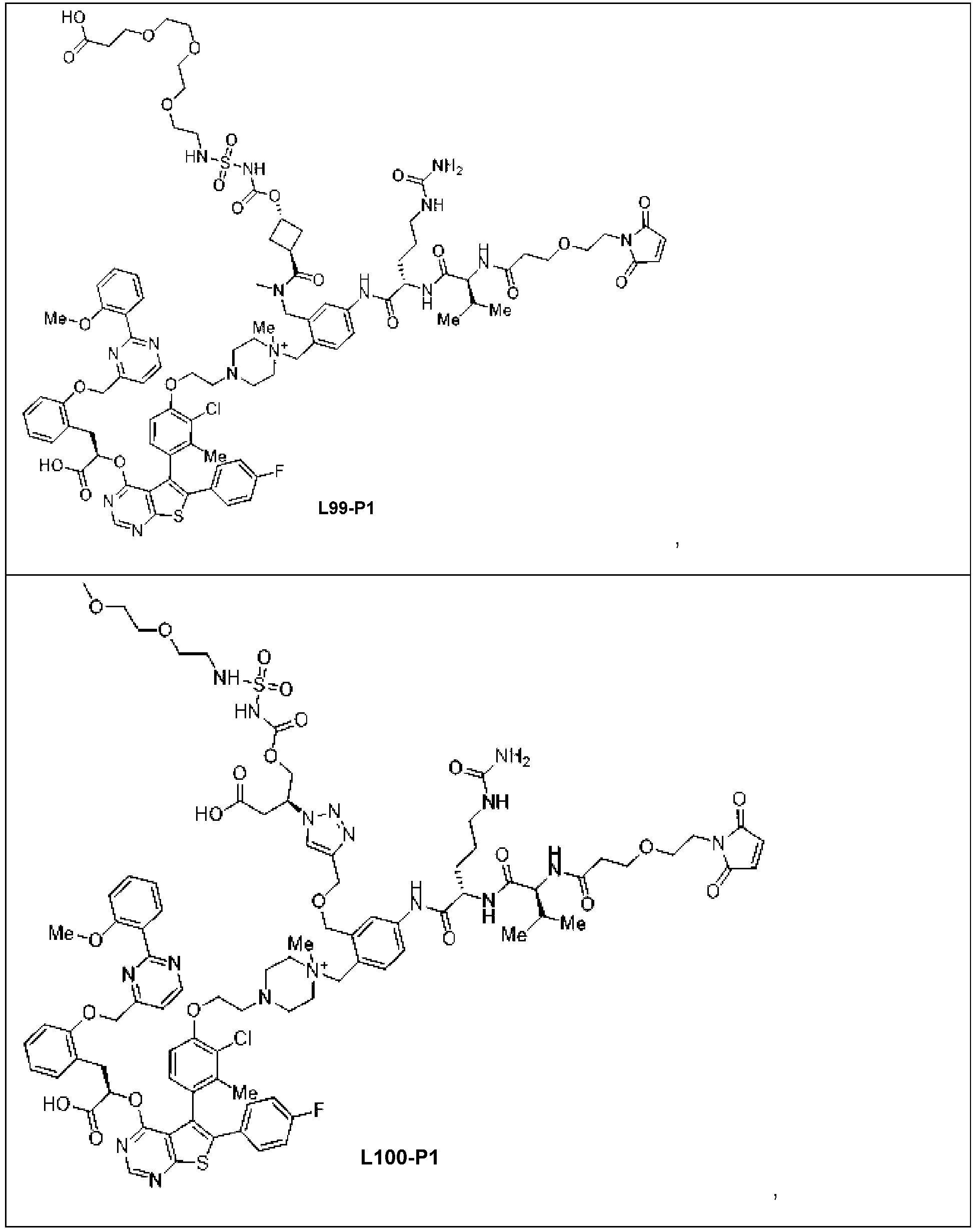

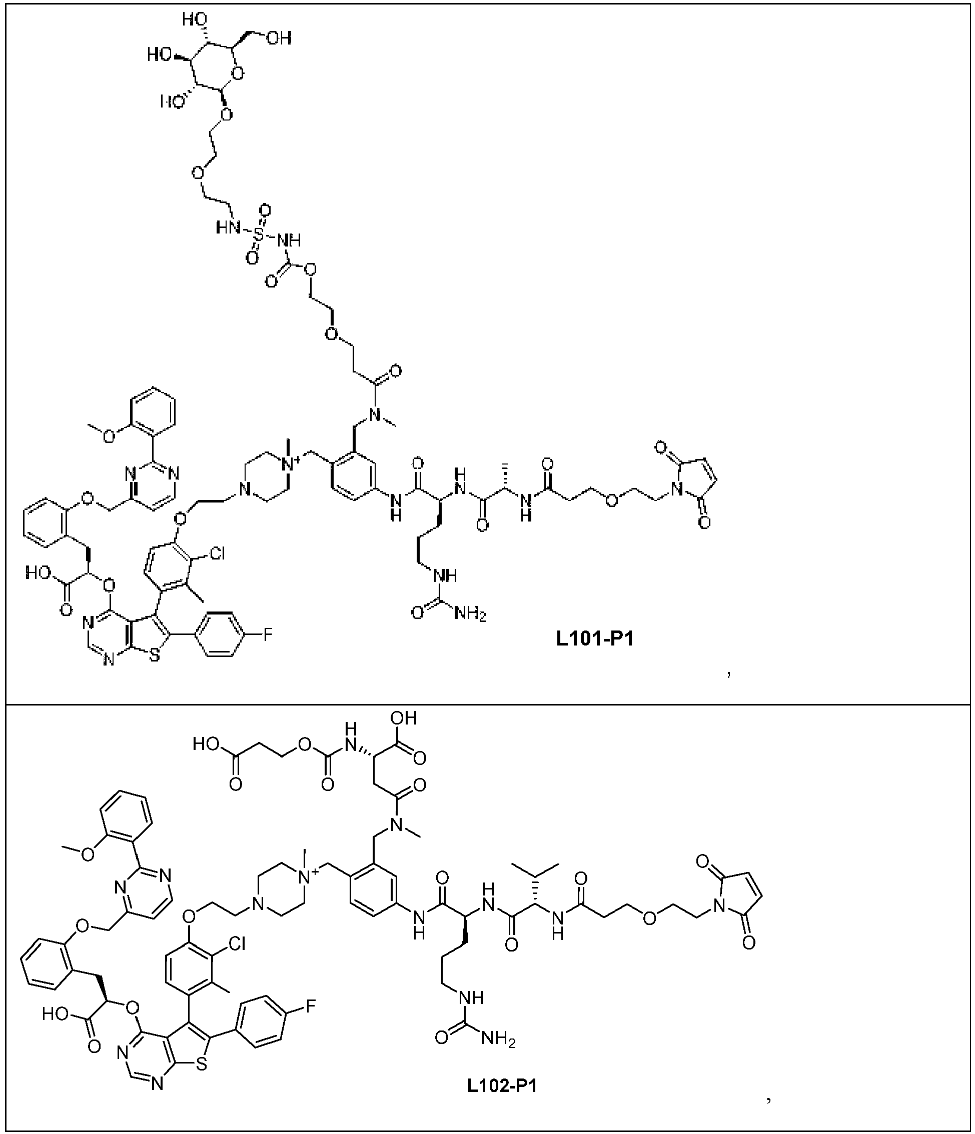

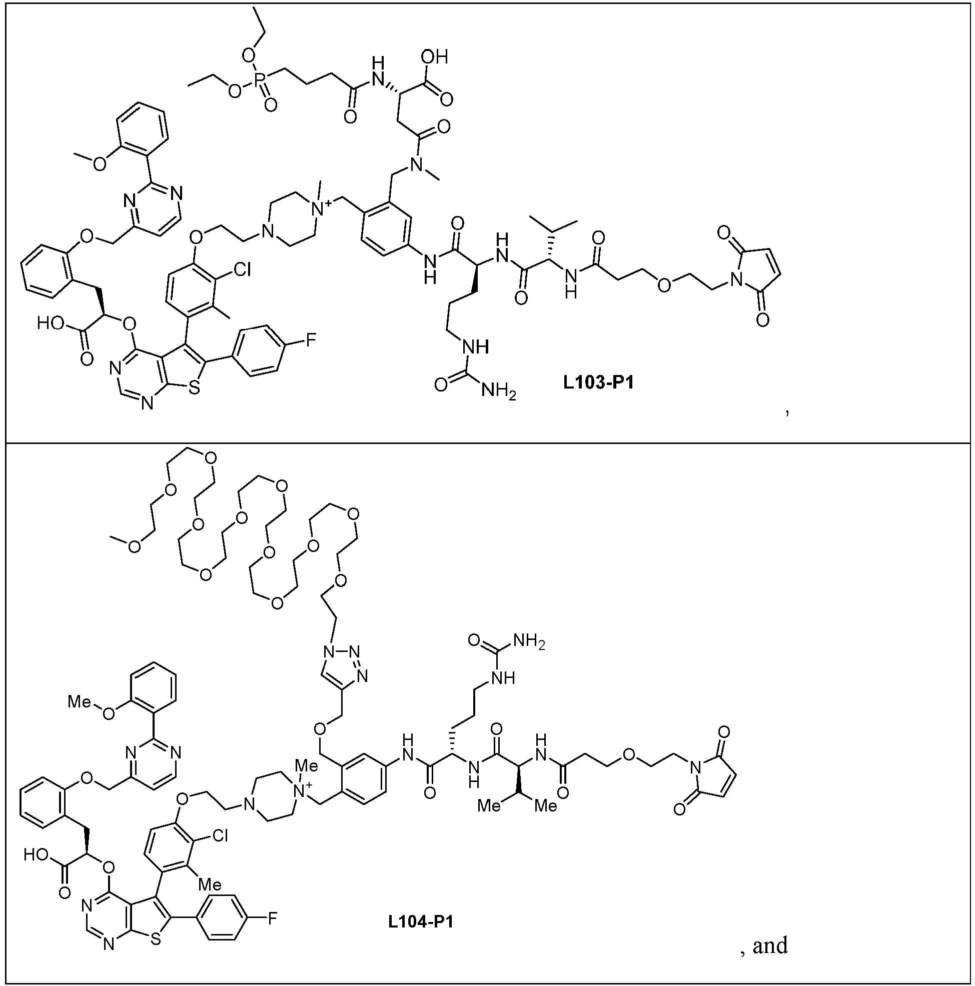

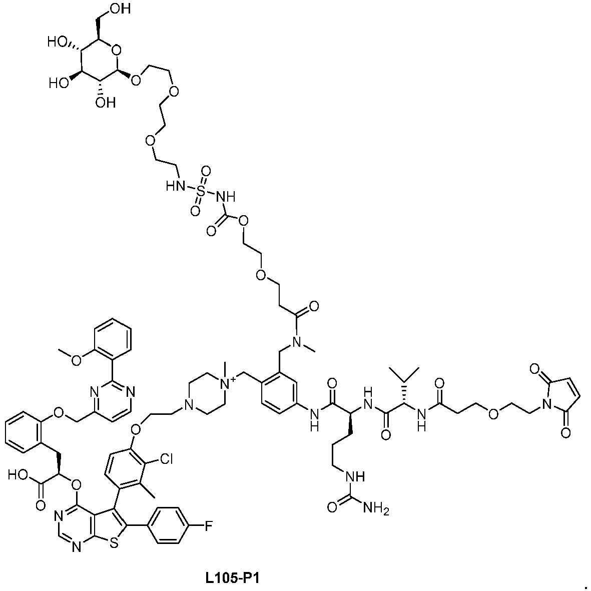

In some embodiments, the antibody-drug conjugate has a formula according to any one of the structures shown in Table 1a.

**Table 1a. ADC Structures**

| ***ADC Structure*** | ***L*/*P Name*** |
|---|---|
| 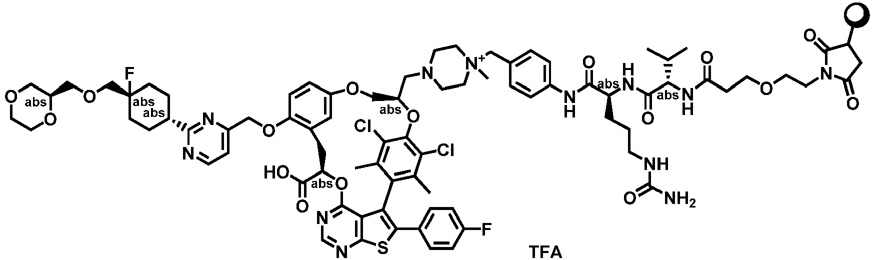 | L9-P16 |
| 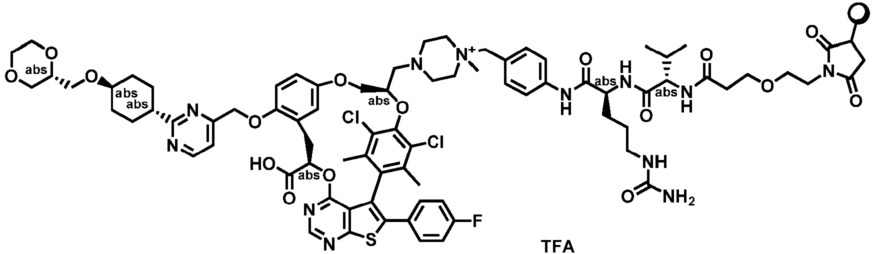 | L9-P17 |
| 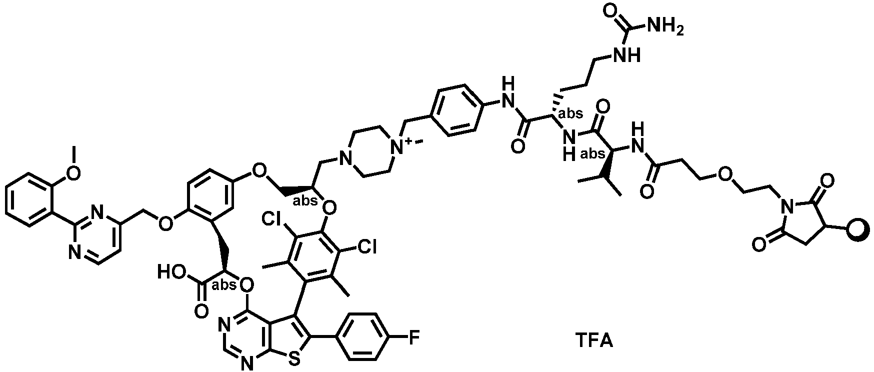 | L9-P15 |
| 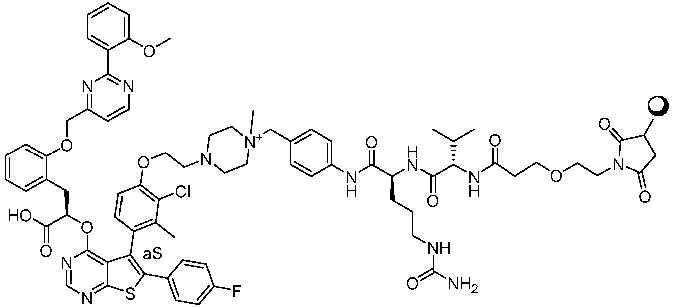 | L9-C1 |
| 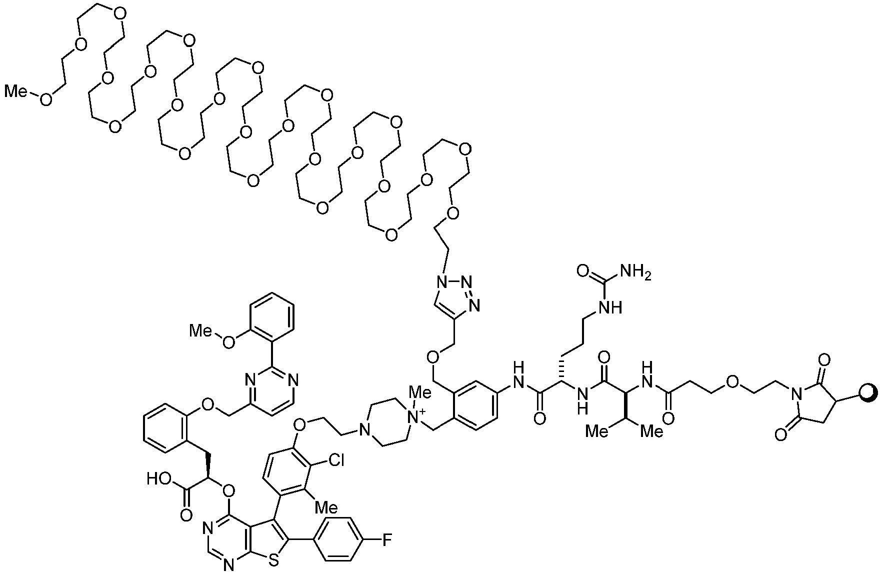 | L5-P1 |
| 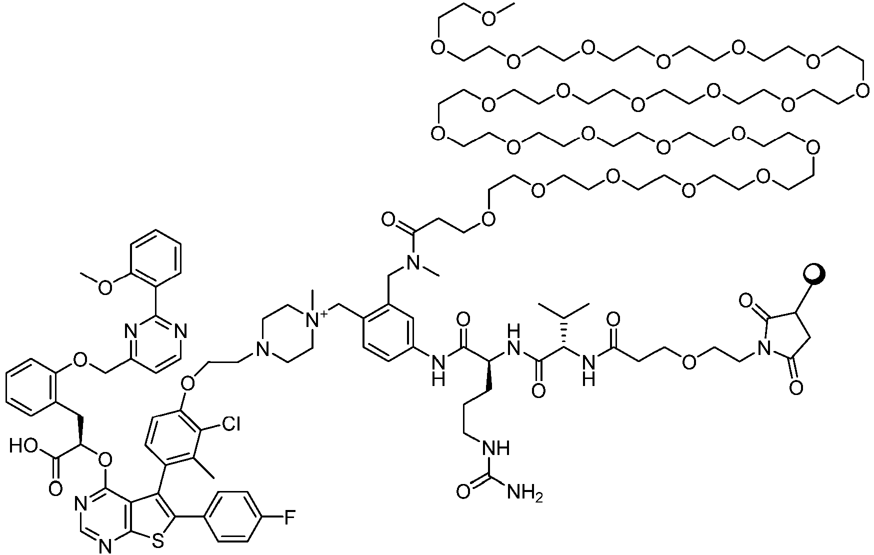 | L30-P1 |
| 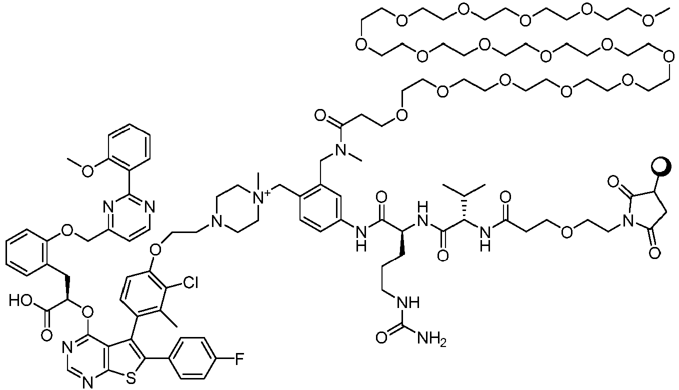 | L31-P1 |
| 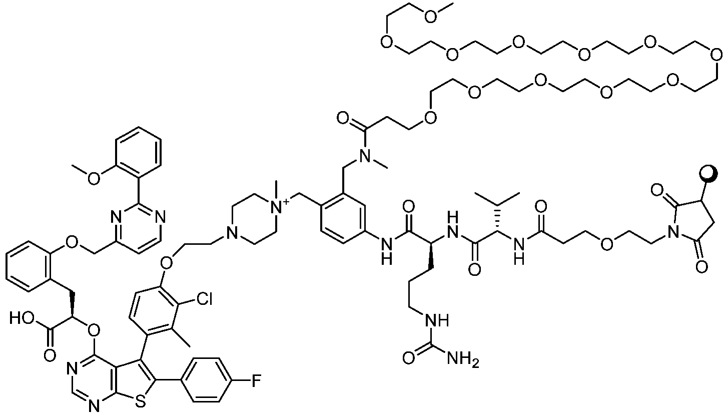 | L32-P1 |
| 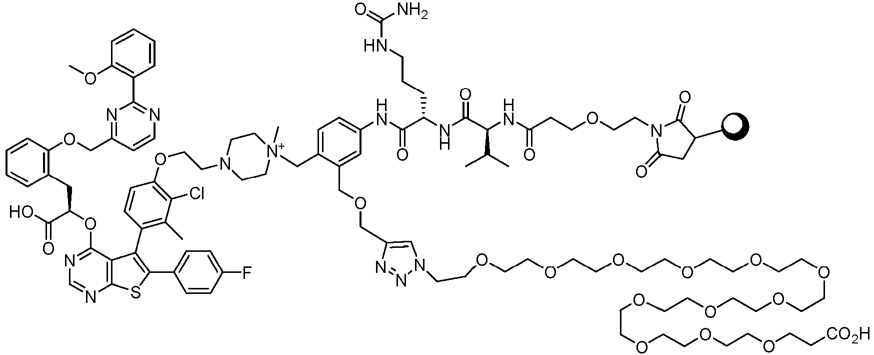 | L33-P1 |
| 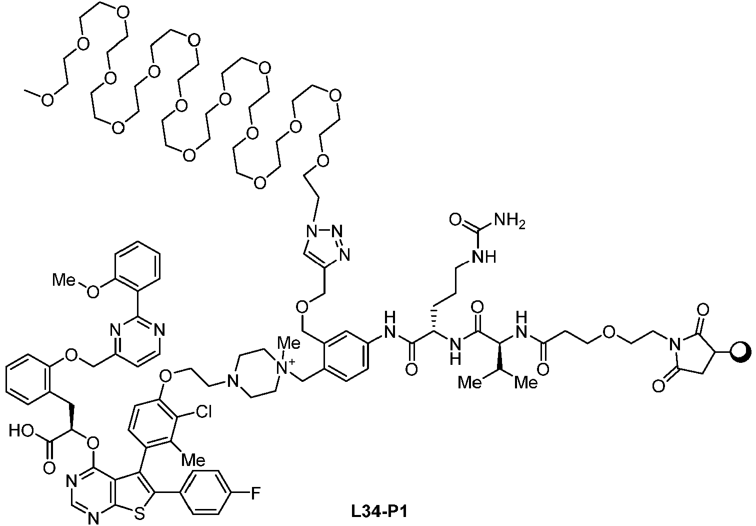 | L34-P1 |
| 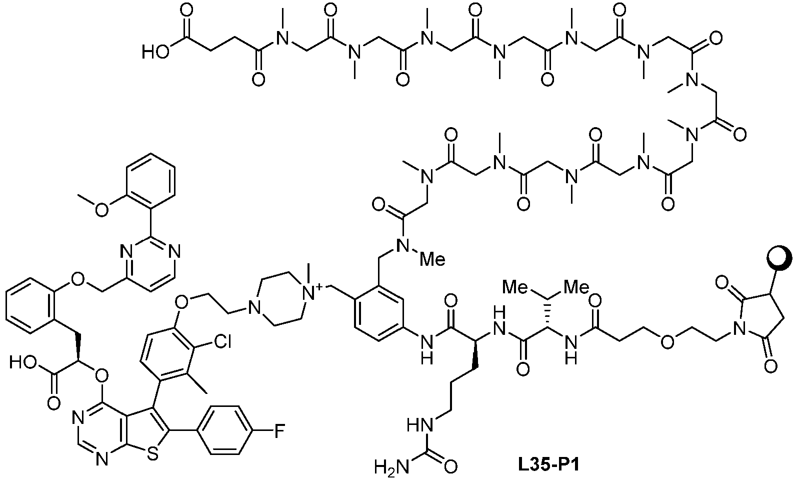 | L35-P1 |
| 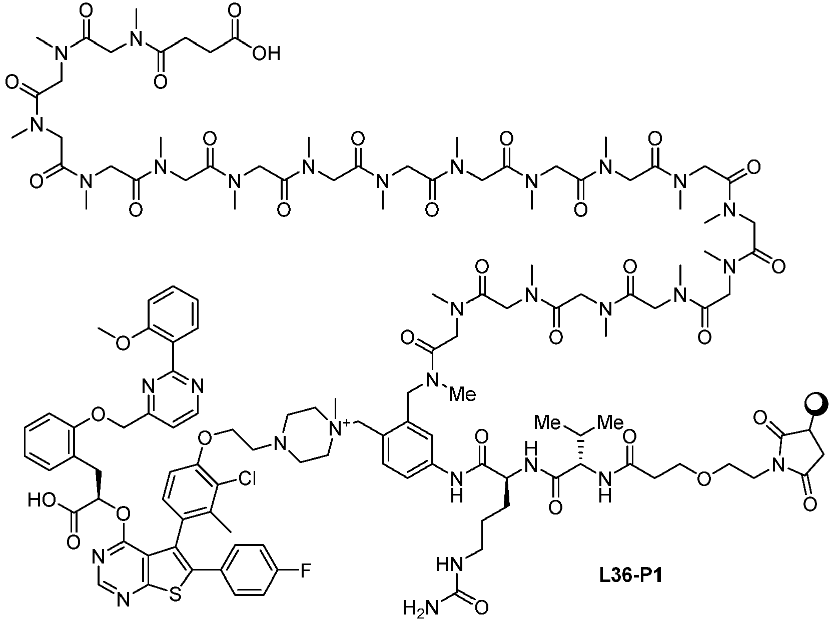 | L36-P1 |
| 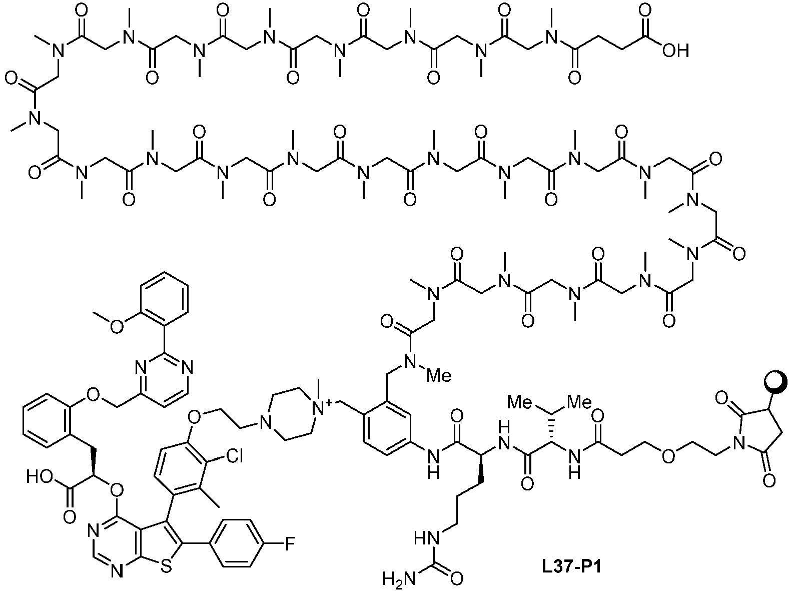 | L37-P1 |
| 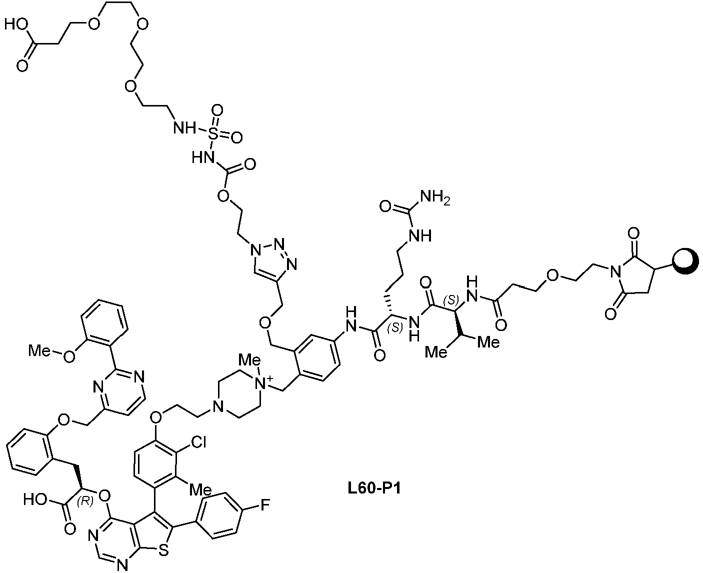 | L60-P1 |
| 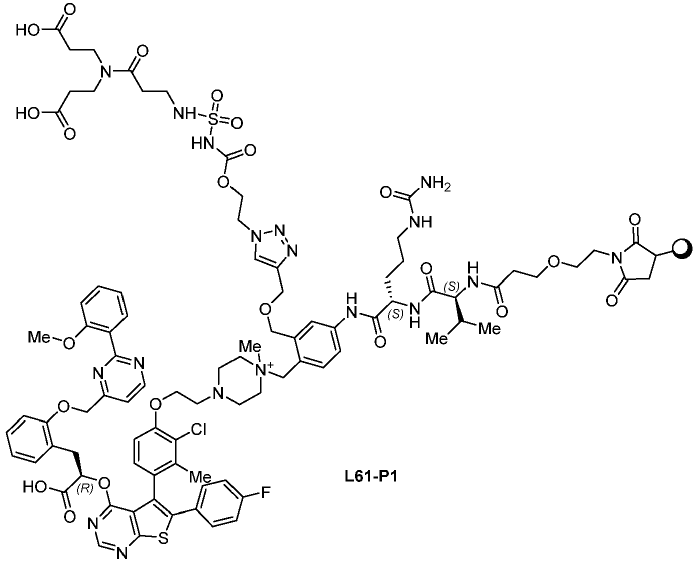 | L61-P1 |
| 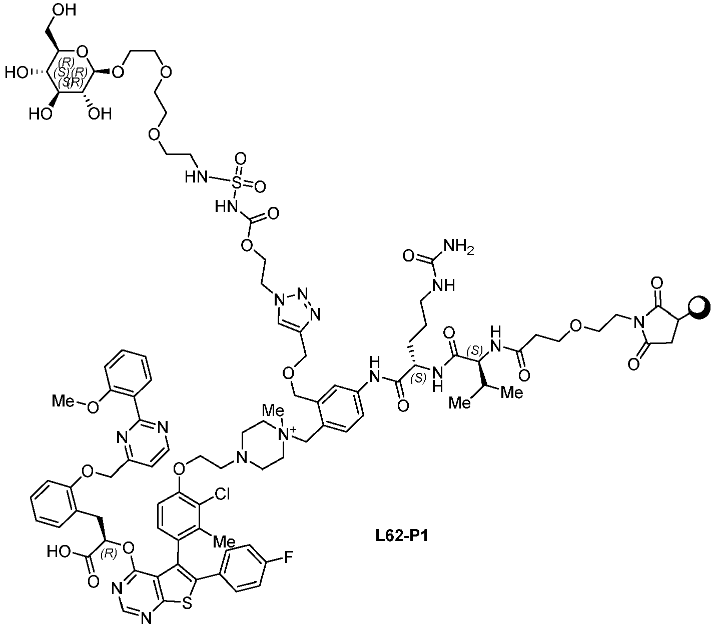 | L62-P1 |
| 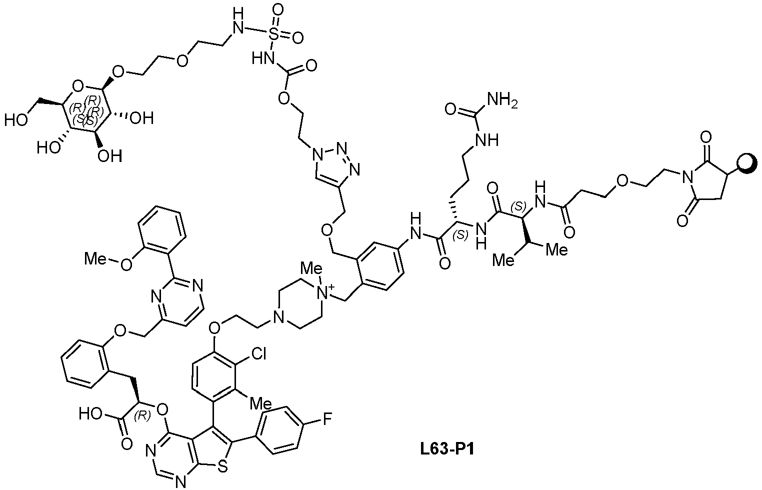 | L63-P1 |
| 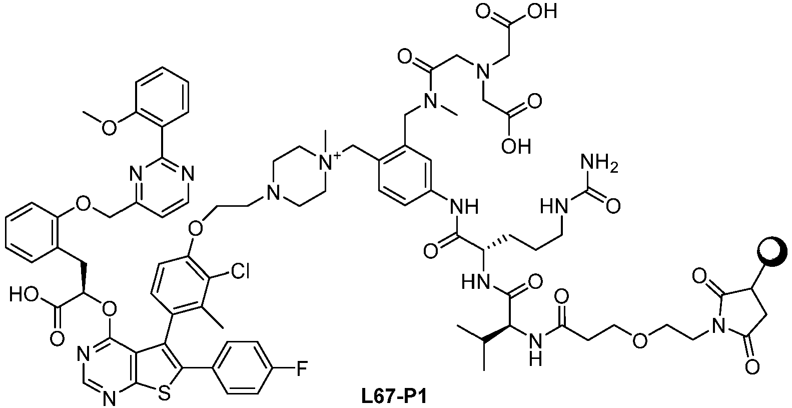 | L67-P1 |
| 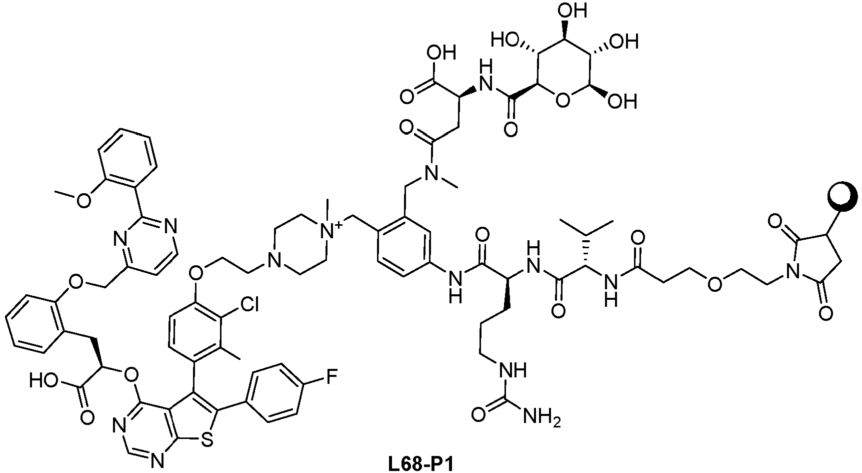 | L68-P1 |
| 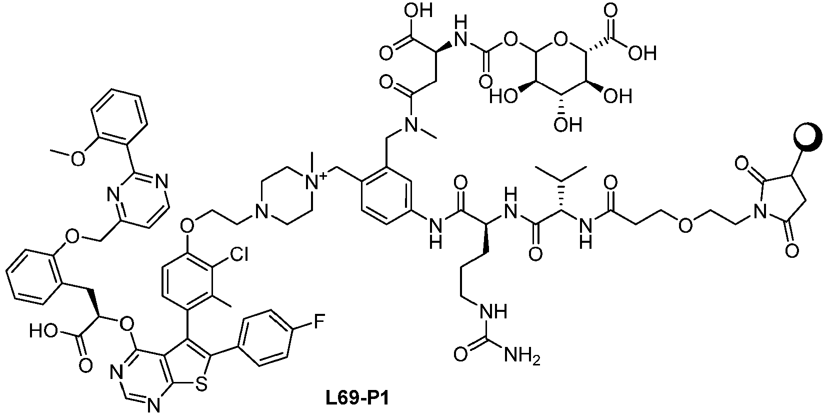 | L69-P1 |
| 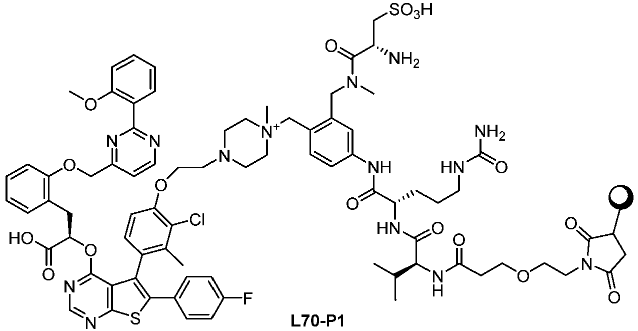 | L70-P1 |
| 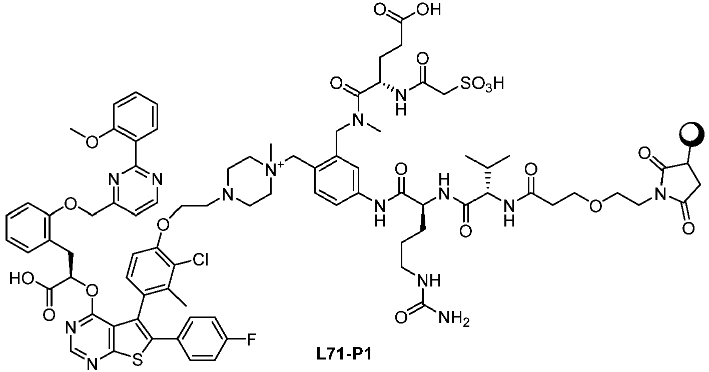 | L71-P1 |
| 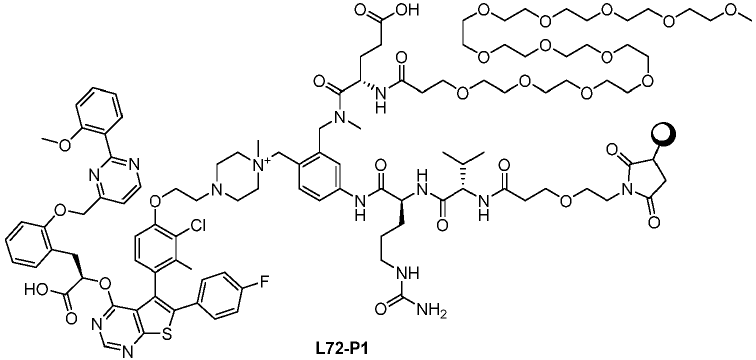 | L72-P1 |
| 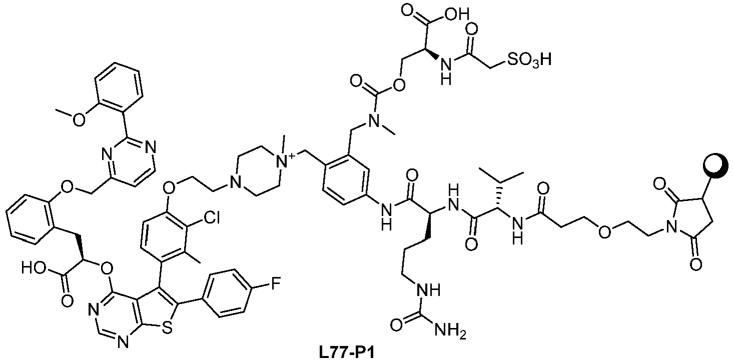 | L77-P1 |
| 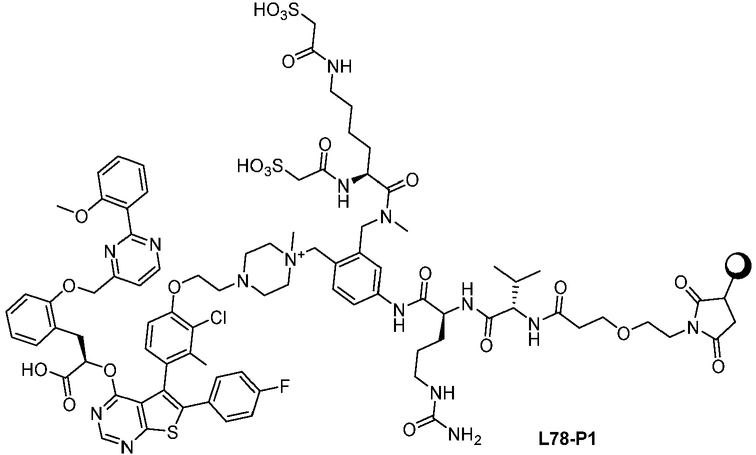 | L78-P1 |
| 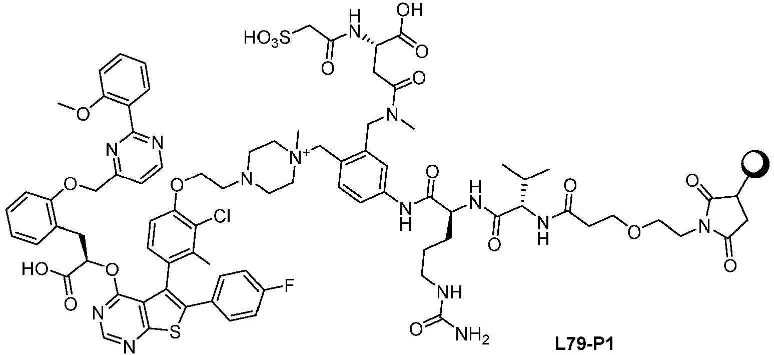 | L79-P1 |
| 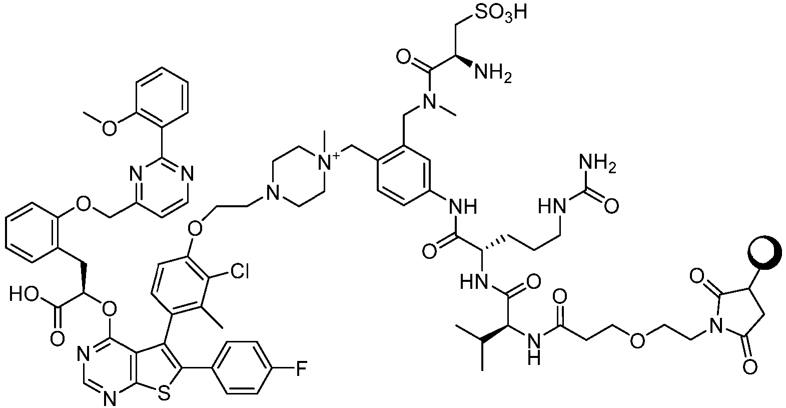 | L86-P1 |
| = **an antibody or an antigen-binding fragment thereof** | |
| The ADCs depicted above can also be represented by the following formula: | |
| Ab-(L-D)*ₚ*, | |
| 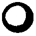 wherein 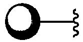 is an antibody or an antigen-binding fragment thereof covalently linked to the linker-payload (L/P) depicted above; *p* is an integer from 1 to 16. In some embodiments, *p* is an integer from 1 to 8. In some embodiments, *p is* an integer from 1 to 5. In some embodiments, *p* is an integer from 2 to 4. In some embodiments, *p is* 2. In some embodiments, *p is* 4. In some embodiments, *p is* determined by liquid chromatography-mass spectrometry (LC-MS | |

In some embodiments, the antibody-drug conjugate has a formula according to any one of the structures shown in Table 1b.

**Table 1b. ADC Structures**

| ***ADC Structure*** | ***Target*** | ***L*/*P Name*** |
|---|---|---|
| 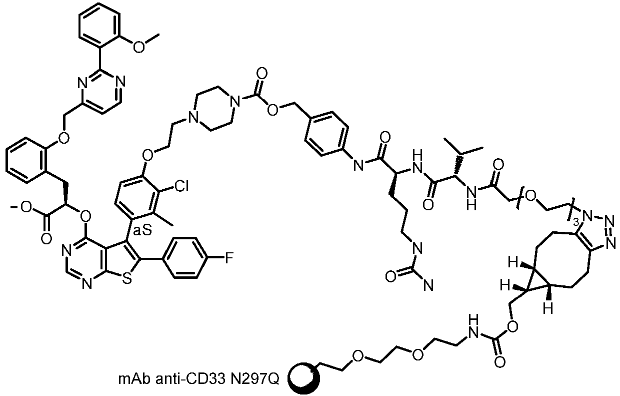 | CD33 | L23-C3 |
| 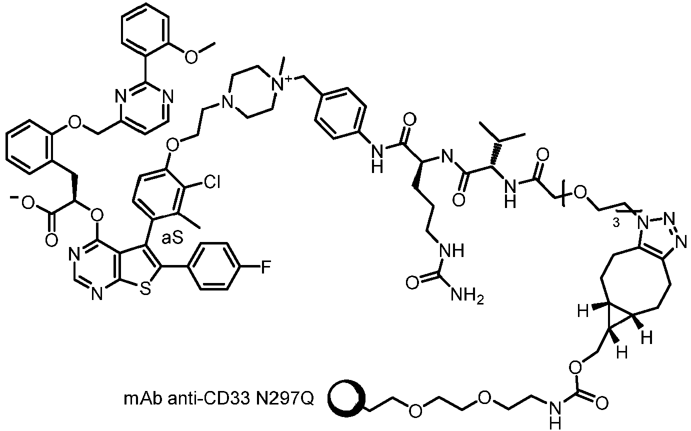 | CD33 | L24-C1 |
| 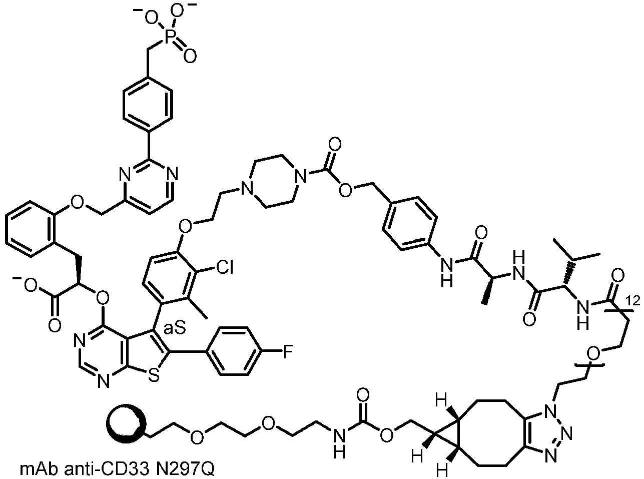 | CD33 | L13-C4 |
| 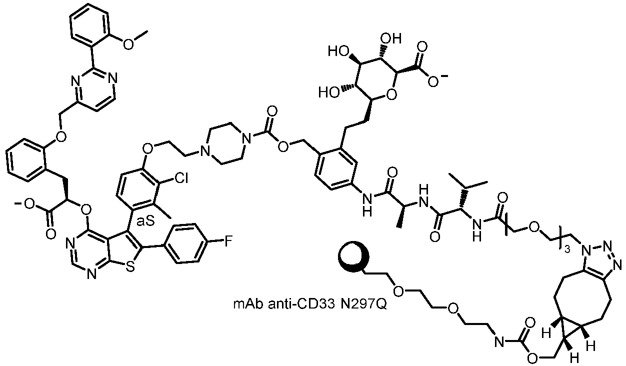 | CD33 | L14-C3 |
| 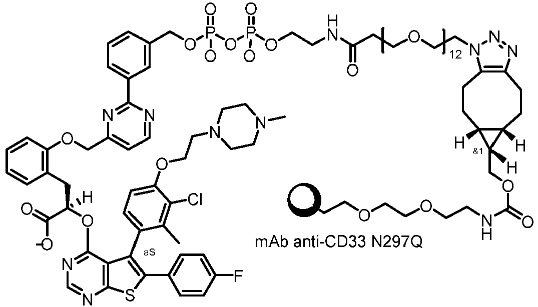 | CD33 | L15-C5 |
| 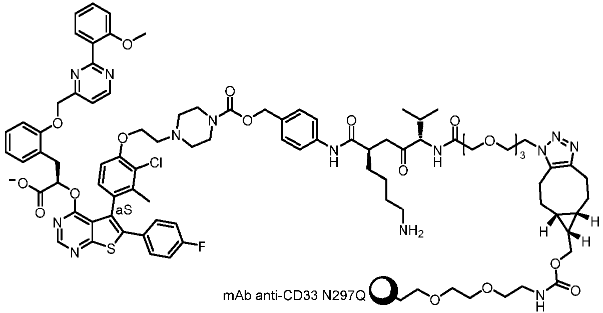 | CD33 | L16-C3 |
| 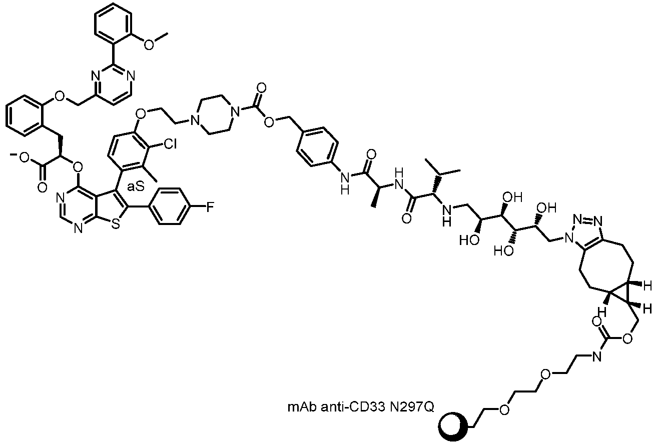 | CD33 | L17-C3 |
| 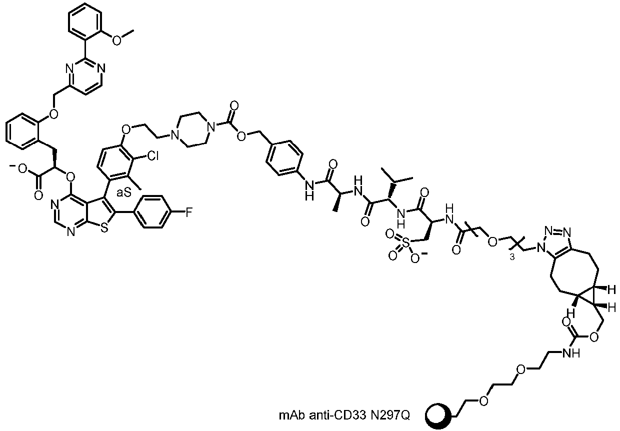 | CD33 | L18-C3 |
| 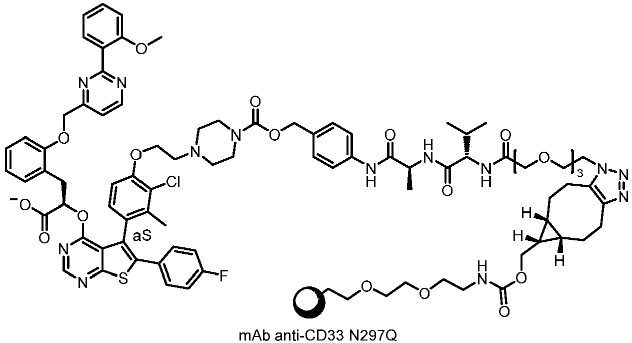 | CD33 | L19-C3 |
| 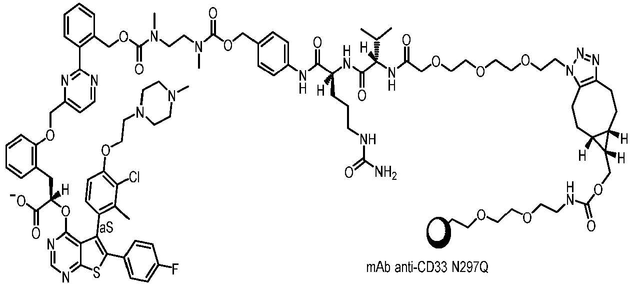 | CD33 | L20-C6 |
| 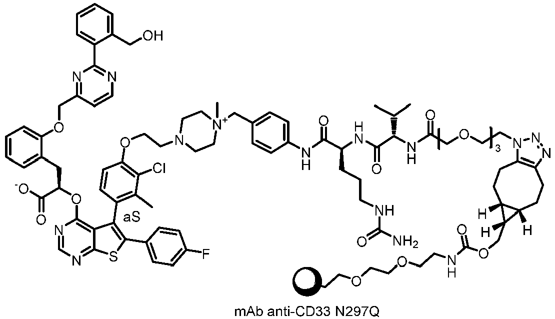 | CD33 | L24-C6 |
| 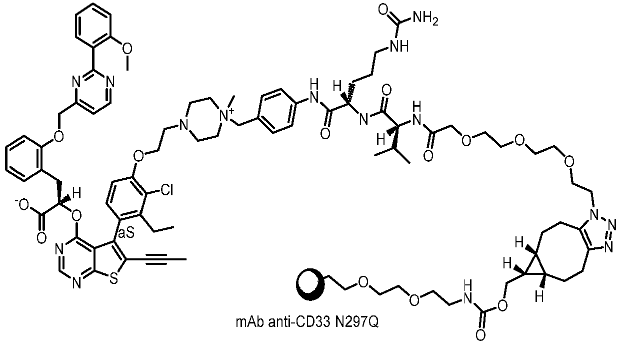 | CD33 | L24-C7 |
| 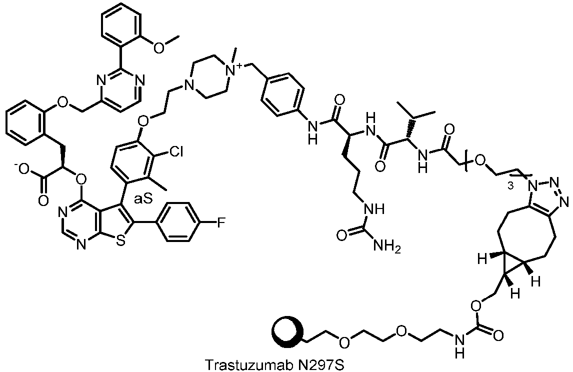 | HER2 | L24-C1 |
| 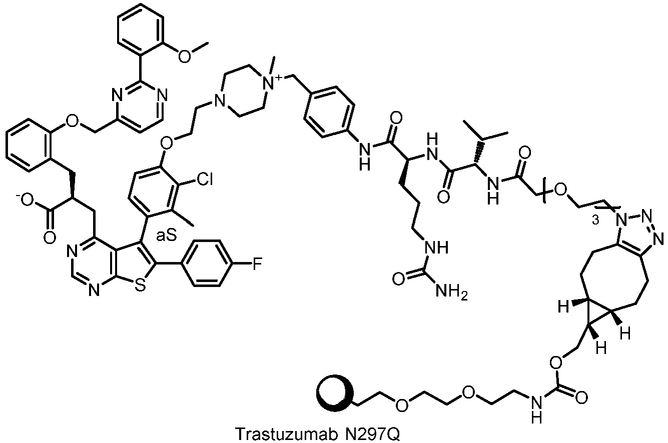 | HER2 | L24-C1 |
| 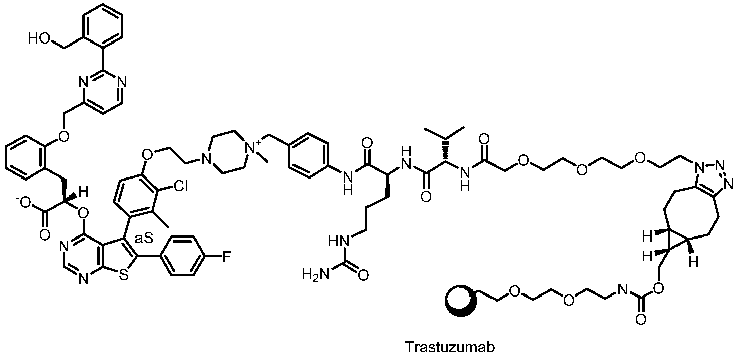 | HER2 | L24-C6 |
| 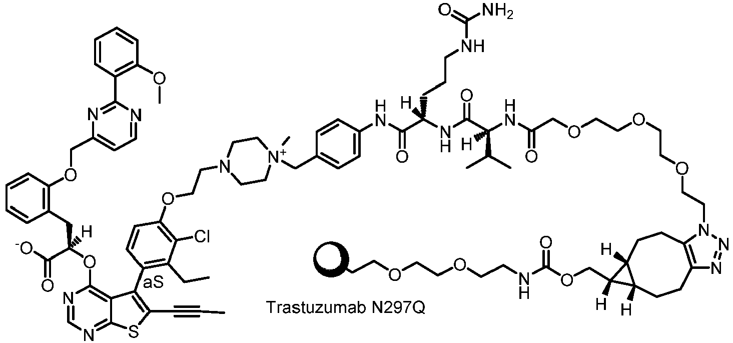 | HER2 | L24-C7 |
| 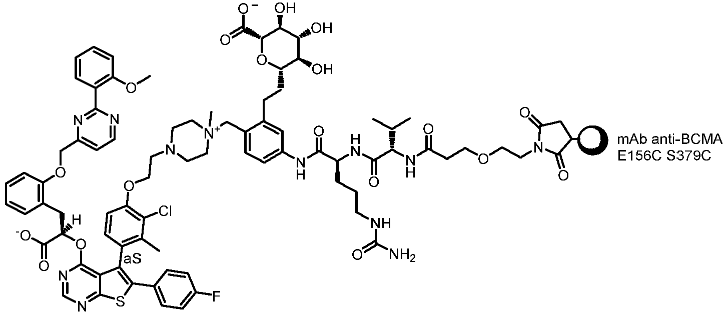 | BCMA | L21-C1 |
| 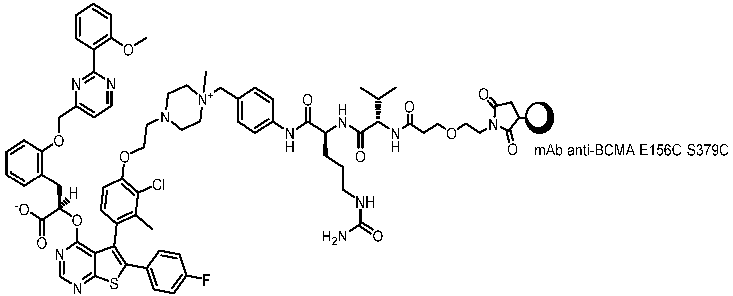 | BCMA | L9-C1 |
| 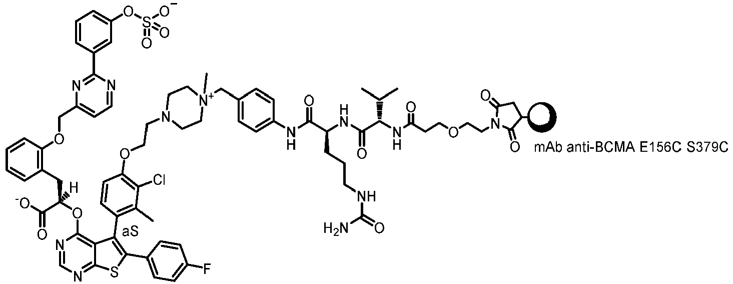 | BCMA | L9-C8 |
| 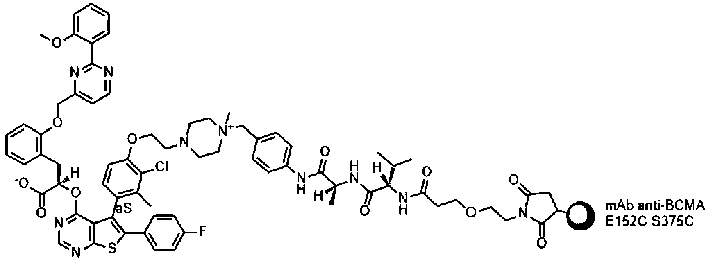 | BCMA | L22-C1 |
| 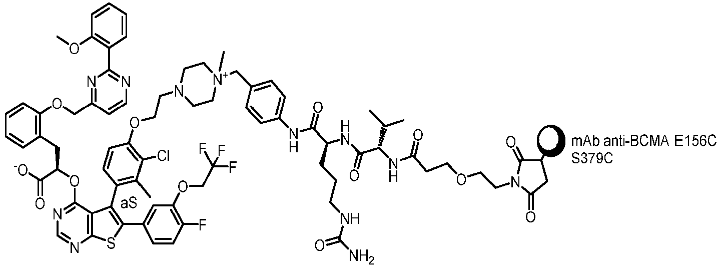 | BCMA | L9-C10 |
| 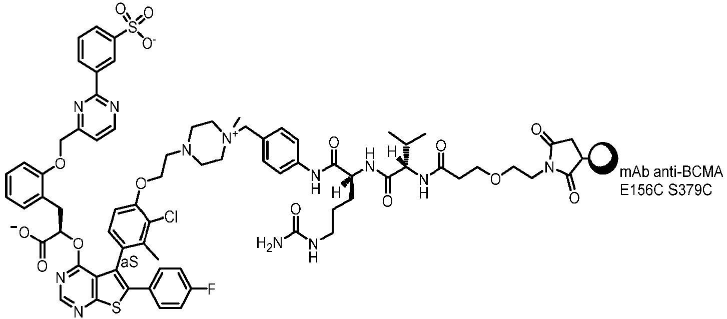 | BCMA | L9-C9 |
| 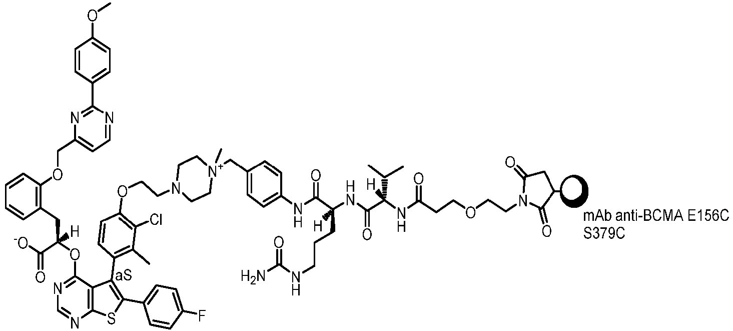 | BCMA | L9-C11 |
| 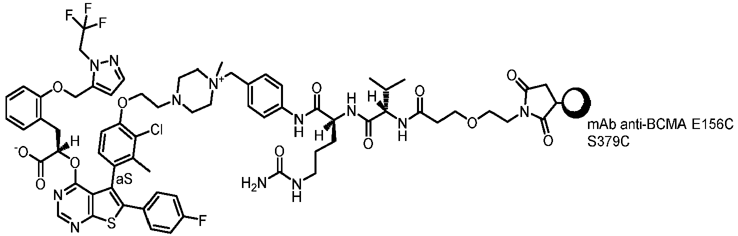 | BCMA | L9-C12 |
| 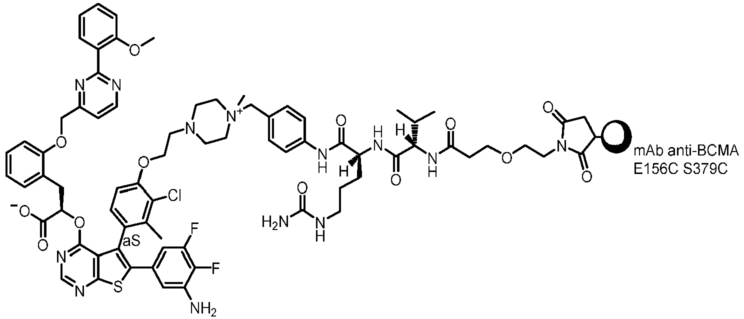 | BCMA | L9-C13 |
| 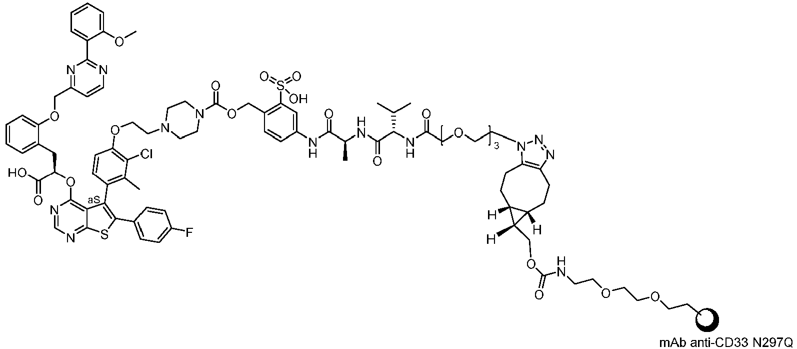 | CD33 | L29-C3 |
| 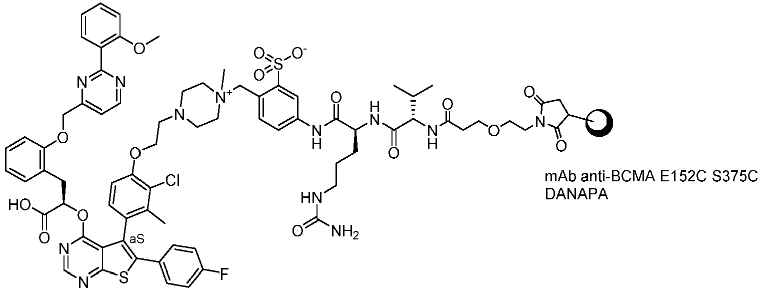 | BCMA DANAPA | L27-P1 |
| | BCMA DANAPA | L28-P1 |
| 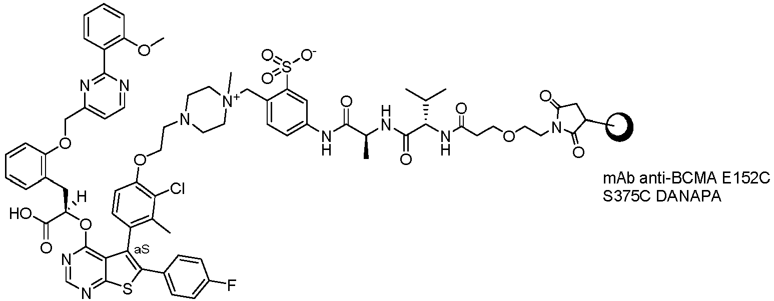 | | |
| 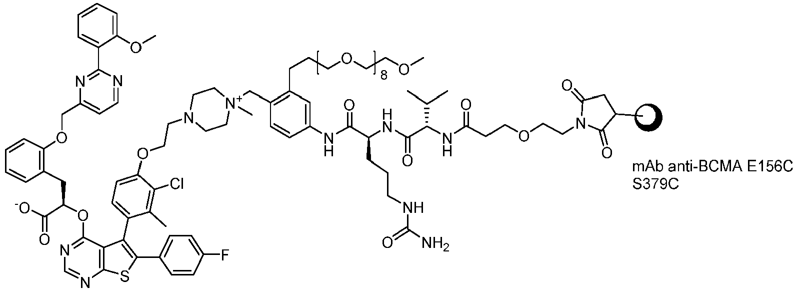 | BCMA | L26-P1 |
| 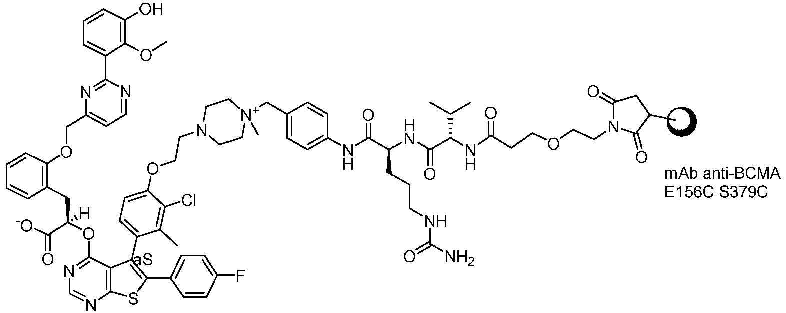 | BCMA | L9-P14 |
| 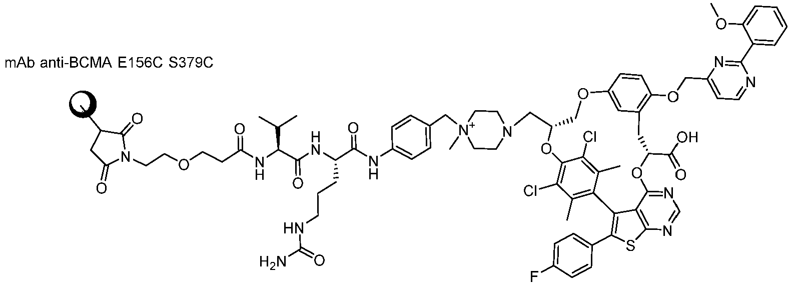 | BCMA | L9-P15 |
| 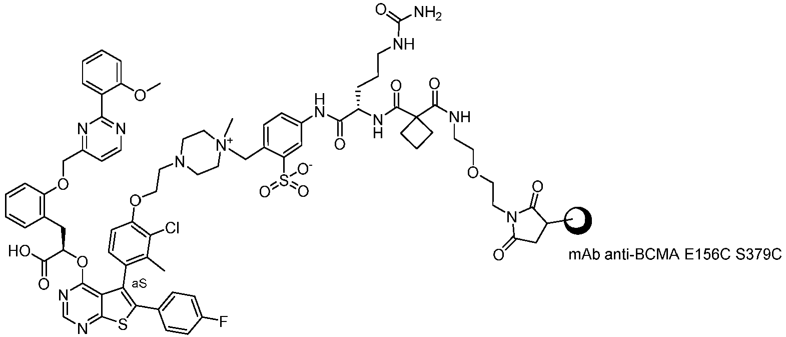 | BCMA | L25-P1 |
| 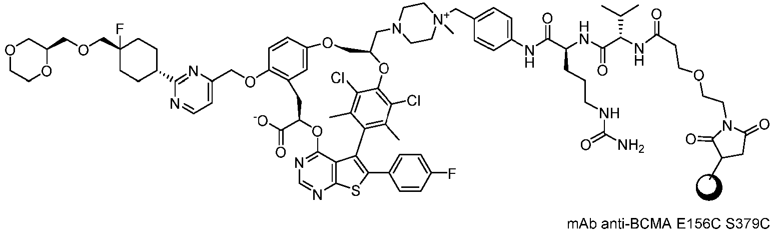 | BCMA | L9-P16 |
| 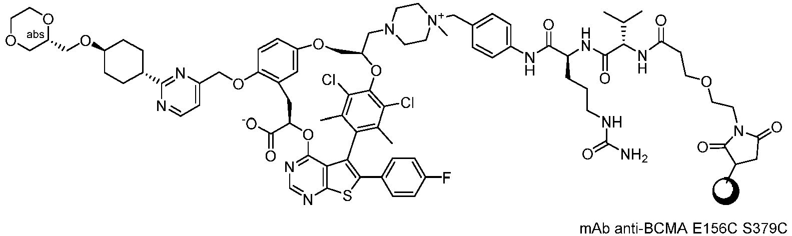 | BCMA | L9-P17 |
| 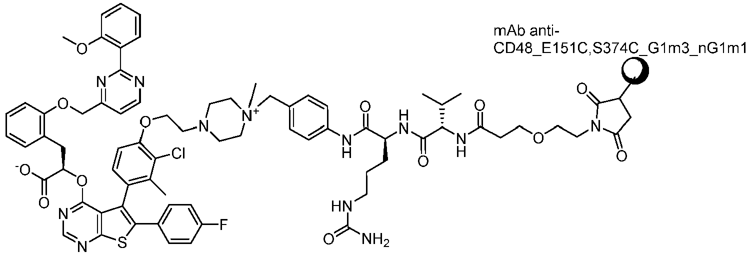 | CD46 | L9-P1 |
| The ADCs depicted above can also be represented by the following formula: Ab-(L-D)*ₚ*, | | |
| wherein 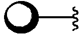 is the specific antibody described above covalently linked to the linker-payload (L/P) depicted above; *p* is an integer from 1 to 16. In some embodiments, *p* is an integer from 1 to 8. In some embodiments, *p* is an integer from 1 to 5. In some embodiments, *p* is an integer from 2 to 4. In some embodiments, *p is* 2. In some embodiments, *p* is 4. In some embodiments, *p* is determined by liquid chromatography-mass spectrometry (LC-MS) | | |

As used herein, "L/P" refers to the linker-payloads, linker-drugs, or linker-compounds disclosed herein and the terms "L#-P#" and "L#-C#" are used interchangeably to refer to a specific linker-drug disclosed herein, while the codes "P#" and "C#" are used interchangeably to refer to a specific compound unless otherwise specified. For example, both "L1-C1" and "L1-P1" refer to the same linker-payload structure disclosed herein, while both "C1" and "P1" indicate the same compound disclosed herein, including an enantiomer, diastereoisomer, atropisomer, deuterated derivative, and/or pharmaceutically acceptable salt of any of the foregoing.

In some embodiments, the antibody or antigen-binding fragment binds to a target antigen on a cancer cell. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48.

In some embodiments, the antibody or antigen-binding fragment is an anti-BCMA antibody or antigen-binding fragment. In some embodiments, the antibody or antigen-binding fragment comprises three heavy chain complementarity determining regions (HCDRs) comprising amino acid sequences of SEQ ID NO:15 (HCDR1), SEQ ID NO:16 (HCDR2), and SEQ ID NO:17 (HCDR3); and three light chain complementarity determining regions (LCDRs) comprising amino acid sequences of SEQ ID NO:18 (LCDR1), SEQ ID NO:19 (LCDR2), and SEQ ID NO:20 (LCDR3). In some embodiments, the antibody or antigen-binding fragment comprises a heavy chain variable region comprising an amino acid sequence of SEQ ID NO:1, and a light chain variable region comprising an amino acid sequence of SEQ ID NO:2. In some embodiments, the antibody or antigen-binding fragment comprises an IgG1 heavy chain constant domain or a modified IgG1 heavy chain constant domain. In some embodiments, the IgG1 heavy chain constant domain comprises a cysteine residue (C) at position 152 and position 375. In some embodiments, the IgG1 heavy chain constant domain comprises a cysteine residue (C) at position 156 and position 379. In some embodiments, the antibody or antigen-binding fragment comprises an Ig kappa light chain constant domain.

In some embodiments, the antibody or antigen-binding fragment is an anti-CD33 antibody or antigen-binding fragment. In some embodiments, the antibody or antigen-binding fragment comprises three heavy chain complementarity determining regions (HCDRs) comprising amino acid sequences of SEQ ID NO:21 (HCDR1), SEQ ID NO:22 (HCDR2), and SEQ ID NO:23 (HCDR3); and three light chain complementarity determining regions (LCDRs) comprising amino acid sequences of SEQ ID NO:24 (LCDR1), SEQ ID NO:25 (LCDR2), and SEQ ID NO:26 (LCDR3). In some embodiments, the antibody or antigen-binding fragment comprises a heavy chain variable region comprising an amino acid sequence of SEQ ID NO:3, and a light chain variable region comprising an amino acid sequence of SEQ ID NO:4. In some embodiments, the antibody or antigen-binding fragment comprises an IgG1 heavy chain constant domain or a modified IgG1 heavy chain constant domain. In some embodiments, the IgG1 heavy chain constant domain comprises a glutamine residue (Q) at position 297. In some embodiments, the antibody or antigen-binding fragment comprises an Ig kappa light chain constant domain.

In some embodiments, the antibody or antigen-binding fragment is an anti-PCAD antibody or antigen-binding fragment. In some embodiments, the antibody or antigen-binding fragment comprises three heavy chain complementarity determining regions (HCDRs) comprising amino acid sequences of SEQ ID NO:33 (HCDR1), SEQ ID NO:34 (HCDR2), and SEQ ID NO:35 (HCDR3); and three light chain complementarity determining regions (LCDRs) comprising amino acid sequences of SEQ ID NO:36 (LCDR1), SEQ ID NO:37 (LCDR2), and SEQ ID NO:38 (LCDR3). In some embodiments, the antibody or antigen-binding fragment comprises a heavy chain variable region comprising an amino acid sequence of SEQ ID NO:7, and a light chain variable region comprising an amino acid sequence of SEQ ID NO:8.

In some embodiments, the antibody or antigen-binding fragment is an anti-HER2 antibody or antigen-binding fragment. In some embodiments, the antibody or antigen-binding fragment comprises three heavy chain complementarity determining regions (HCDRs) comprising amino acid sequences of SEQ ID NO:39 (HCDR1), SEQ ID NO:40 (HCDR2), and SEQ ID NO:41 (HCDR3); and three light chain complementarity determining regions (LCDRs) comprising amino acid sequences of SEQ ID NO:42 (LCDR1), SEQ ID NO:43 (LCDR2), and SEQ ID NO:44 (LCDR3). In some embodiments, the antibody or antigen-binding fragment comprises a heavy chain variable region comprising an amino acid sequence of SEQ ID NO:9, and a light chain variable region comprising an amino acid sequence of SEQ ID NO:10. In some embodiments, the antibody or antigen-binding fragment comprises an IgG1 heavy chain constant domain or a modified IgG1 heavy chain constant domain. In some embodiments, the IgG1 heavy chain constant domain comprises a glutamine residue (Q) at position 297. In some embodiments, the IgG1 heavy chain constant domain comprises a serine residue (S) at position 297. In some embodiments, the antibody or antigen-binding fragment comprises an Ig kappa light chain constant domain.

In some embodiments, the antibody or antigen-binding fragment is an anti-CD38 antibody or antigen-binding fragment. In some embodiments, the antibody or antigen-binding fragment is an anti-CD46 antibody or antigen-binding fragment. In some embodiments, the antibody or antigen-binding fragment is an anti-CD48 antibody or antigen-binding fragment. In some embodiments, the antibody or antigen-binding fragment is an anti-CD79b antibody or antigen-binding fragment.

In a second aspect, there is provided compositions comprising multiple copies of the antibody-drug conjugate of the first aspect, wherein the average p of the antibody-drug conjugates in the composition is from about 2 to about 16, e.g., about 2 to about 8, e.g., about 2 to about 4. In some embodiments, the average p of the antibody-drug conjugates in the composition is from about 2 to about 4.

In a third aspect, there is provided pharmaceutical compositions comprising the antibody-drug conjugate of the first aspect, or the composition of the second aspect, and a pharmaceutically acceptable carrier.

Further provided herein, in some embodiments, are therapeutic uses for the described ADC compounds and compositions, e.g., in treating a cancer.
In a fourth aspect, there is provided the antibody-drug conjugate of the first aspect, the composition of the second aspect, or the pharmaceutical composition of the third aspect for use in treating a subject having or suspected of having a cancer. The cancer expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

In a fifth aspect, there is provided the antibody-drug conjugate of the first aspect, the composition of the second aspect, or the pharmaceutical composition of the third aspect for use in reducing or inhibiting the growth of a tumor in a subject. The tumor expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the antibody-drug conjugate, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%.

In a sixth aspect, there is provided the antibody-drug conjugate of the first aspect, the composition of the second aspect, or the pharmaceutical composition of the third aspect for use in reducing or slowing the expansion of a cancer cell population in a subject. The cancer cell population expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the cancer cell population is from a tumor or a hematological cancer. In some embodiments, the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer cell population is from a lymphoma or gastric cancer. In some embodiments, administration of the antibody-drug conjugate, composition, or pharmaceutical composition reduces the cancer cell population by at least about 10%, at least about 20%, at least about 30%, at least about 40%, at least about 50%, at least about 60%, at least about 70%, at least about 80%, at least about 90%, at least about 95%, or at least about 99%. In some embodiments, administration of the antibody-drug conjugate, composition, or pharmaceutical composition slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%.

In a seventh aspect, there is provided a method of determining whether a subject having or suspected of having a cancer will be responsive to treatment with the antibody-drug conjugate of the first aspect, the composition of the second aspect, or the pharmaceutical composition of the third aspect by providing a biological sample from the subject; contacting the sample with the antibody-drug conjugate; and detecting binding of the antibody-drug conjugate to cancer cells in the sample. The cancer cells in the sample express a target antigen. In some embodiments, the cancer expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer. In some embodiments, the sample is a tissue biopsy sample, a blood sample, or a bone marrow sample

In an embodiment of the fourth, fifth, sixth and seventh aspect, it further comprising administering to the subject in need thereof at least one additional therapeutic agent, preferably the one additional therapeutic agent is a Bcl-2 inhibitor, more preferably the one additional therapeutic agent is venetoclax, compound A1 or compound A2.

Methods of producing the described ADC compounds and compositions are also disclosed. In an eighth aspect, there is provided a method of producing an antibody-drug conjugate of the first aspect, by reacting an antibody or antigen-binding fragment with a cleavable linker joined to an Mcl-1 inhibitor under conditions that allow conjugation.

In a ninth aspect, there is provided a compound having one of the following structures: 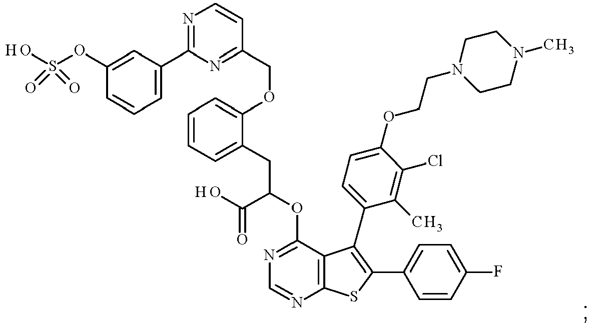 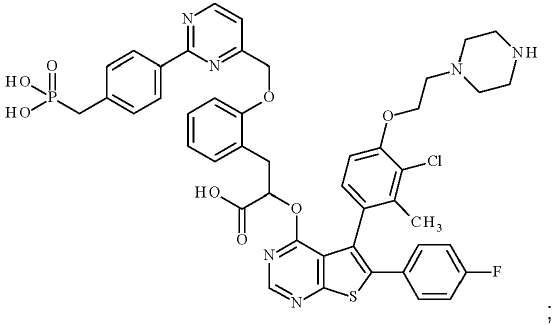 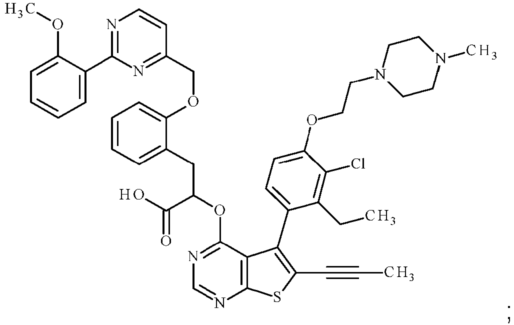 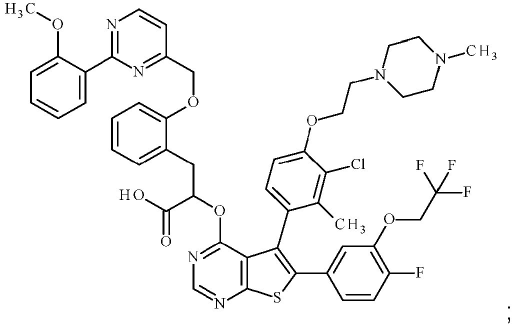 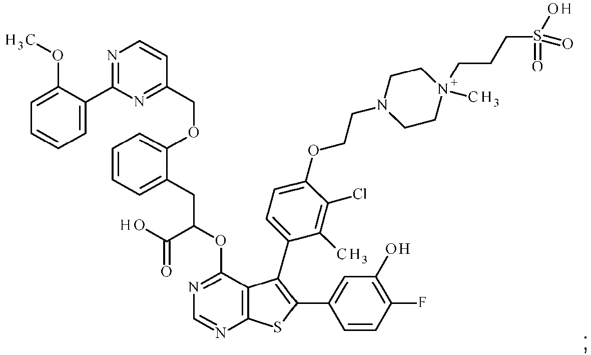 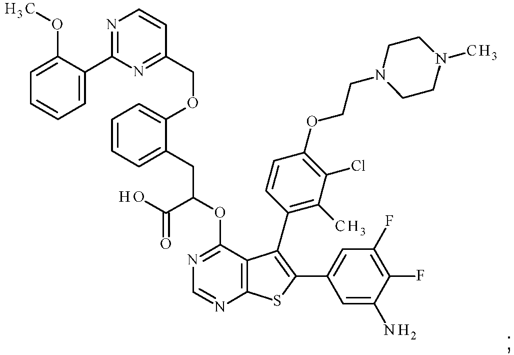 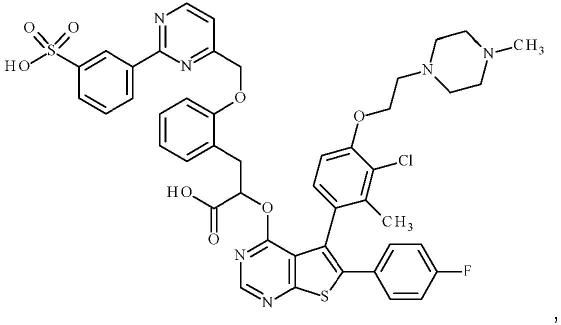 or 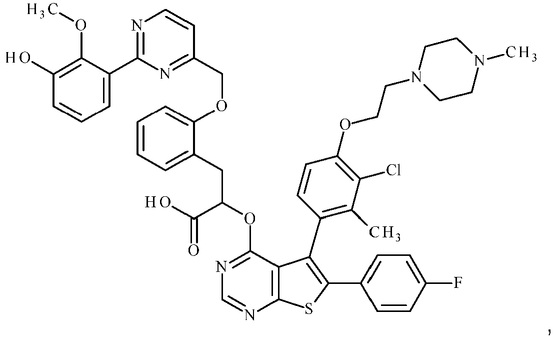 or a salt thereof (e.g., pharmaceutically acceptable salt thereof).

In an embodiment of the ninth aspect, the compound is used as a synthesis intermediate for the preparation of an antibody-drug conjugate of Formula (1) according to the first aspect.

In a tenth aspect, there is provided the compound according to ninth aspect or a salt thereof with a pharmaceutically acceptable acid or base, for use as a pro-apoptotic agent.

### BRIEF DESCRIPTION OF THE DRAWINGS

**FIG. 1** shows an exemplary site-specific antibody conjugation using bacterial transglutaminase (BTG).
**FIG. 2A** shows a dose response curve of free Mcl-1 payload (P1), a CD38-targeting Mcl-1 ADC, and a non-targeting isotype ADC on A4-Fuk cells. **FIG. 2B** shows a dose response curve of free Mcl-1 payload (P1), a CD38-targeting Mcl-1 ADC, and a non-targeting isotype ADC on KMS021 BM cells.
**FIG. 3A** shows a dose response curve of free Mcl-1 payload (P1), a CD48-targeting Mcl-1 ADC, and a non-targeting isotype ADC on NCI-H929 cells. **FIG. 3B** shows a dose response curve of free Mcl-1 payload (P1), a CD48-targeting Mcl-1 ADC, and a non-targeting isotype ADC on OPM-2 cells. **FIG. 3C** shows a dose response curve of free Mcl-1 payload (P1), a CD48-targeting Mcl-1 ADC, and a non-targeting isotype ADC on AMO1 cells.
**FIG. 4** shows a dose response curve of free Mcl-1 payload (P1), a CD79b-targeting Mcl-1 ADC, and a non-targeting isotype ADC on RS4;11 cells.
**FIG. 5A** shows a dose response curve of free Mcl-1 payload (P1) and a HER2-targeting Mcl-1 ADC on HCC1954 cells. **FIG. 5B** shows a dose response curve of free Mcl-1 payload (P1) and a HER2-targeting Mcl-1 ADC on HCC2218 cells.
**FIG. 6A** shows a dose response curve of free Mcl-1 payload (P1), CD33-targeting Mcl-1 ADCs, and a non-targeting isotype ADC on AMO1-CD33 clone D2 cells. **FIG. 6B** shows a dose response curve of free Mcl-1 payload (P1), CD33-targeting Mcl-1 ADCs, and a non-targeting isotype ADC on MOLM-13 cells.
**FIG. 7** shows a dose response curve of free Mcl-1 payload (P1), BCMA-targeting Mcl-1 ADCs, and a non-targeting isotype ADC on NCI-H929 cells.
**FIG. 8A** and **FIG. 8B** show the results of an MTT cell viability assay. All tested anti-CD33 ADCs and corresponding payloads induced a dose dependent decrease in the viability of AMO1-CD33 clone D2 cells; while no significant effect was observed with the corresponding naked antibody.
**FIGs. 9A** and **9B** show the results of a CTG cell viability assay. All tested anti-HER2 ADCs and corresponding payloads induced a dose dependent decrease in the viability of HCC1954 cells; while no significant effect was observed with the corresponding naked antibody.
**FIGs. 10A****,** **10B**,**10C**, and **10D** show the results of an MTT cell viability assay. All tested anti-BCMA ADCs and corresponding payloads induced a dose dependent decrease in the viability of NCI-H929 cells; while no significant effect was observed with the corresponding naked antibody.
**FIG. 11** shows tumor volume (mm³) of NCI-H929-grafted female SCID mice upon treatment with isotype control IgG1-Linker-Payload Fc silent, anti-BCMA_CysmAb Fc silent, or anti-BCMA_CysmAb Fc silent_L7-P1 (10 and/or 30 mg/kg, administered once IV, n=6).
**FIG. 12** shows percentage (%) of body weight loss of NCI-H929-grafted female SCID mice upon treatment with isotype control IgG1-Linker-Payload Fc silent, anti-BCMA_CysmAb Fc silent, or anti-BCMA_CysmAb Fc silent_L7-P1 (10 and/or 30 mg/kg, administered once IV, n=6).
**FIG. 13A** shows the results of a CTG cell viability assay testing anti-CD48 ADCs in NCI-H929 cells. **FIG. 13B** shows the results of a CTG cell viability assay testing anti-CD48 ADCs in KHM1B cells. **FIG. 13C** shows the results of a CTG cell viability assay testing anti-CD48 ADCs in KMS21BM cells.
**FIG. 14** shows the tumor volume (mm³) of H929-grafted female SCID mice with time after randomization (days) upon treatment with anti-BCMA_CysmAb Fc silent or different anti-BCMA_CysmAb Fc silent ADCs (20 mg/kg, administered once IV, n=6).
**FIG. 15** shows % of body weight loss of H929-grafted female SCID mice with time after randomization (days) upon treatment with anti-BCMA_CysmAb Fc silent or different anti-BCMA_CysmAb Fc silent ADCs (20 mg/kg, administered once IV, n=6).
**FIG. 16** shows the tumor volume (mm³) of H929-grafted female SCID mice with time after randomization (days) upon treatment with IgG1-Linker-Payload Fc silent, anti-CD48_CysmAb Fc silent or anti-CD48_CysmAb Fc silent_ **L5-P1** (30 mg/kg, administered once IV, n=8).
**FiG. 17** shows % of body weight loss of H929-grafted female SCID mice with time after randomization (days) upon treatment with IgG1-Linker-Payload Fc silent, anti-CD48_CysmAb Fc silent or anti-CD48_CysmAb Fc silent_ **L5-P1** (30 mg/kg, administered once IV, n=8).
**FIG. 18** shows the results of a CTG cell viability assay. The anti-CD46-Mcl-1 ADC **Ab C - L9-C1** and the corresponding payload **C1** induced a dose dependent decrease in the viability of KMS21BM cells, while no significant effect was observed with the corresponding naked antibody.
**FIGs. 19A** and **19B** show the results of a MTT cell viability assay. All the ADCs induced a dose dependent decrease in the viability of AMO1-CD33 clone D2 cells at single agent. Interestingly, the activity of the ADCs is significantly improved when in combination with the BCL2 inhibitor compound A1, while no significant effect was observed after treatment of these cells with the corresponding naked antibody at single agent or in combination with 1µM of compound A1.
**FIGs. 20A** and **20B** show the results of a MTT cell viability assay. All the anti-BCMA-Mcl-1 ADCs induced a dose dependent decrease in the viability of AMO1 cells. Interestingly, the activity of the ADCs was significantly improved when in combination with the BCL2 inhibitor compound A1, while no significant effect was observed after treatment of these cells with the corresponding naked antibody at single agent or in combination with 1µM of compound A1.
**FIGs. 21A** and **21B** showthe results of a MTT cell viability assay. All the anti-BCMA-Mcl-1 ADCs induced a dose dependent decrease in the viability of H929 cells. Interestingly, the activity of the ADCs is significantly improved when in combination with the BCL2 inhibitor compound A1, while no significant effect was observed after treatment of these cells with the corresponding naked antibody at single agent or in combination with 1µM of compound A1.
**FIG. 22** shows the inhibition activities of CD48 MCL-1 antibody drug conjugate CD48-L7-P1, isotype IgG1-L7-P1 ADC, and MCL-1 free payload P1 against KMS-21BM, KMS-20, KMS-27 and NCI-H929
**FIGs. 23A** and **23B** show the inhition activities of CD48 MCL-1 antibody drug conjugate CD48-L7-P1, isotype IgG1-L7-P1 ADC, and MCL-1 free payload P1 in combination with venetoclax against KMS-21BM, KMS-20, KMS-27 and NCI-H929.

### DETAILED DESCRIPTION OF ILLUSTRATIVE EMBODIMENTS

The disclosed compositions and methods may be understood more readily by reference to the following detailed description taken in connection with the accompanying figures, which form a part of this disclosure.

Throughout this text, the descriptions refer to compositions and methods of using the compositions. Where the disclosure describes or claims a feature or embodiment associated with a composition, such a feature or embodiment is equally applicable to the methods of using the composition. Likewise, where the disclosure describes or claims a feature or embodiment associated with a method of using a composition, such a feature or embodiment is equally applicable to the composition.

When a range of values is expressed, it includes embodiments using any particular value within the range. Further, reference to values stated in ranges includes each and every value within that range. All ranges are inclusive of their endpoints and combinable. When values are expressed as approximations, by use of the antecedent "about," it will be understood that the particular value forms another embodiment. Reference to a particular numerical value includes at least that particular value, unless the context clearly dictates otherwise. The use of "or" will mean "and/or" unless the specific context of its use dictates otherwise. Where a reference and the specification conflict, the specification will control.

Unless the context of a description indicates otherwise, e.g., in the absence of symbols indicating specific point(s) of connectivity, when a structure or fragment of a structure is drawn, it may be used on its own or attached to other components of an ADC, and it may do so with any orientation, e.g., with the antibody attached at any suitable attachment point to a chemical moiety such as a linker-drug. Where indicated, however, components of an ADC are attached in the orientation shown in a given formula. For example, if Formula (1) is described as Ab-(L-D)*ₚ* and the group "-(L-D)" is described as 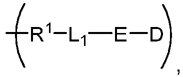 then the elaborated structure of Formula (1) is 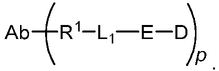 It is not

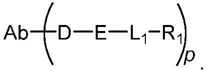

It is to be appreciated that certain features of the disclosed compositions and methods, which are, for clarity, described herein in the context of separate embodiments, may also be provided in combination in a single embodiment. Conversely, various features of the disclosed compositions and methods that are, for brevity, described in the context of a single embodiment, may also be provided separately or in any sub-combination.

As used throughout this application, antibody drug conjugates can be identified using a naming convention in the general format of "target antigen/antibody-linker-payload". For example only, if an antibody drug conjugate is referred to as "Target X-L0-P0", such a conjugate would comprise an antibody that binds Target X, a linker designated as L0, and a payload designated as P0. Alternatively, if an antibody drug conjugate is referred to as "anti-Target X-L0-P0", such a conjugate would comprise an antibody that binds Target X, a linker designated as L0, and a payload designated as P0. In another alternative, if an antibody drug conjugate is referred to as "AbX-L0-P0", such a conjugate would comprise the antibody designated as AbX, a linker designated as L0, and a payload designated as P0. An control antibody drug conjugate comprising a non-specific, isotype control antibody may be referenced as "isotype control IgG1-L0-P0" or "IgG1-L0-P0".

Any formula given herein is also intended to represent unlabeled forms as well as isotopically labeled forms of the compounds. Isotopically labeled compounds have structures depicted by the formulae given herein except that one or more atoms are replaced by an atom having a selected atomic mass or mass number. Isotopes that can be incorporated into compounds of the invention include, for example, isotopes of hydrogen, carbon, nitrogen, oxygen, fluorine, and chlorine, such as ³H, ¹¹C, ¹³C, ¹⁴C, ¹⁵N, ¹⁸F, and ³⁶Cl. Accordingly, it should be understood that the present disclosure includes compounds that incorporate one or more of any of the aforementioned isotopes, including for example, radioactive isotopes, such as ³H and ¹⁴C, or those into which non-radioactive isotopes, such as ²H and ¹³C are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F or labeled compound may be particularly desirable for PET or SPECT studies. Isotopically-labeled compounds can generally be prepared by conventional techniques known to those skilled in the art, e.g., using an appropriate isotopically-labeled reagents in place of the non-labeled reagent previously employed.

### Definitions

Various terms relating to aspects of the description are used throughout the specification and claims. Such terms are to be given their ordinary meaning in the art unless otherwise indicated. Other specifically defined terms are to be construed in a manner consistent with the definitions provided herein.

As used herein, the singular forms "a," "an," and "the" include plural forms unless the context clearly dictates otherwise. The terms "comprising", "having", "being of" as in "being of a chemical formula", "including", and "containing" are to be construed as open terms (i.e., meaning "including but not limited to") unless otherwise noted. Additionally whenever "comprising" or another open-ended term is used in an embodiment, it is to be understood that the same embodiment can be more narrowly claimed using the intermediate term "consisting essentially of" or the closed term "consisting of".

The term "about" or "approximately," when used in the context of numerical values and ranges, refers to values or ranges that approximate or are close to the recited values or ranges such that the embodiment may perform as intended, as is apparent to the skilled person from the teachings contained herein. In some embodiments, about means plus or minus 20%, 15%, 10%, 5%, 1%, 0.5%, or 0.1% of a numerical amount. In one embodiment, the term "about" refers to a range of values which are 10% more or less than the specified value. In another embodiment, the term "about" refers to a range of values which are 5% more or less than the specified value. In another embodiment, the term "about" refers to a range of values which are 1% more or less than the specified value.

The terms "antibody-drug conjugate," "antibody conjugate," "conjugate," "immunoconjugate," and "ADC" are used interchangeably, and refer to one or more therapeutic compounds (e.g., an Mcl-1 inhibitor) that is linked to one or more antibodies or antigen-binding fragments. In some embodiments, the ADC is defined by the generic formula: Ab-(L-D)*ₚ* (Formula 1), wherein Ab = an antibody or antigen-binding fragment, L = a linker moiety, D = a drug moiety (e.g., an Mcl-1 inhibitor drug moiety), and *p* = the number of drug moieties per antibody or antigen-binding fragment. In ADCs comprising an Mcl-1 inhibitor drug moiety, "*p*" refers to the number of Mcl-1 inhibitor compounds linked to the antibody or antigen-binding fragment.

The term "antibody" is used in the broadest sense to refer to an immunoglobulin molecule that recognizes and specifically binds to a target, such as a protein, polypeptide, carbohydrate, polynucleotide, lipid, or combinations of the foregoing through at least one antigen recognition site within the variable region of the immunoglobulin molecule. An antibody can be polyclonal or monoclonal, multiple or single chain, or an intact immunoglobulin, and may be derived from natural sources or from recombinant sources. An "intact" antibody is a glycoprotein that typically comprises at least two heavy (H) chains and two light (L) chains inter-connected by disulfide bonds. Each heavy chain is comprised of a heavy chain variable region (abbreviated herein as VH) and a heavy chain constant region. The heavy chain constant region comprises three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. An antibody can be a monoclonal antibody, human antibody, humanized antibody, camelised antibody, or chimeric antibody. The antibodies can be of any isotype (e.g., IgG, IgE, IgM, IgD, IgA and IgY), class (e.g., IgG1, IgG2, IgG3, IgG4, IgA1 and IgA2), or subclass. An antibody can be an intact antibody or an antigen-binding fragment thereof.

The term "antibody fragment" or "antigen-binding fragment" or "functional antibody fragment," as used herein, refers to at least one portion of an antibody that retains the ability to specifically interact with (e.g., by binding, steric hinderance, stabilizing/destabilizing, spatial distribution) an epitope of an antigen (e.g., BCMA, CD33, PCAD, or HER2). Antigen-binding fragments may also retain the ability to internalize into an antigen-expressing cell. In some embodiments, antigen-binding fragments also retain immune effector activity. The terms antibody, antibody fragment, antigen-binding fragment, and the like, are intended to embrace the use of binding domains from antibodies in the context of larger macromolecules such as ADCs. It has been shown that fragments of a full-length antibody can perform the antigen binding function of a full-length antibody. Examples of antibody fragments include, but are not limited to, Fab, Fab', F(ab')2, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen-binding fragment can also be incorporated into single domain antibodies, maxibodies, minibodies, nanobodies, intrabodies, diabodies, triabodies, tetrabodies, bispecific or multi-specific antibody constructs, ADCs, v-NAR and bis-scFv (see, e.g., Holliger and Hudson (2005) Nat Biotechnol. 23(9):1126-36). Antigen-binding fragments can also be grafted into scaffolds based on polypeptides such as a fibronectin type III (Fn3) (see US Patent No. 6,703,199, which describes fibronectin polypeptide minibodies). The term "scFv" refers to a fusion protein comprising at least one antigen-binding fragment comprising a variable region of a light chain and at least one antigen-binding fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, an scFv may have the VL and VH variable regions in either order, e.g., with respect to the N-terminal and C-terminal ends of the polypeptide, the scFv may comprise VL-linker-VH or may comprise VH-linker-VL. Antigen-binding fragments are obtained using conventional techniques known to those of skill in the art, and the binding fragments are screened for utility (e.g., binding affinity, internalization) in the same manner as are intact antibodies. Antigen-binding fragments, for example, may be prepared by cleavage of the intact protein, e.g., by protease or chemical cleavage.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991) "Sequences of Proteins of Immunological Interest," 5th Ed. Public Health Service, National Institutes of Health, Bethesda, MD ("Kabat" numbering scheme); Al-Lazikani et al. (1997) J Mol Biol. 273(4):927-48 ("Chothia" numbering scheme); ImMunoGenTics (IMGT) numbering (Lefranc (2001) Nucleic Acids Res. 29(1):207-9; Lefranc et al. (2003) Dev Comp Immunol. 27(1):55-77) ("IMGT" numbering scheme); or a combination thereof. In a combined Kabat and Chothia numbering scheme for a given CDR region (for example, HC CDR1, HC CDR2, HC CDR3, LC CDR1, LC CDR2, or LC CDR3), in some embodiments, the CDRs correspond to the amino acid residues that are defined as part of the Kabat CDR, together with the amino acid residues that are defined as part of the Chothia CDR. As used herein, the CDRs defined according to the "Chothia" number scheme are also sometimes referred to as "hypervariable loops."

In some embodiments, under Kabat, the CDR amino acid residues in the heavy chain variable domain (VH) are numbered 31-35 (HCDR1) (e.g., insertion(s) after position 35), 50-65 (HCDR2), and 95-102 (HCDR3); and the CDR amino acid residues in the light chain variable domain (VL) are numbered 24-34 (LCDR1) (e.g., insertion(s) after position 27), 50-56 (LCDR2), and 89-97 (LCDR3). In some embodiments, under Chothia, the CDR amino acids in the VH are numbered 26-32 (HCDR1) (e.g., insertion(s) after position 31), 52-56 (HCDR2), and 95-102 (HCDR3); and the amino acid residues in VL are numbered 26-32 (LCDR1) (e.g., insertion(s) after position 30), 50-52 (LCDR2), and 91-96 (LCDR3). By combining the CDR definitions of both Kabat and Chothia, in some embodiments, the CDRs comprise or consist of, e.g., amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. In some embodiments, under IMGT, the CDR amino acid residues in the VH are numbered approximately 26-35 (CDR1), 51-57 (CDR2) and 93-102 (CDR3), and the CDR amino acid residues in the VL are numbered approximately 27-32 (CDR1), 50-52 (CDR2), and 89-97 (CDR3). In some embodiments, under IMGT, the CDR regions of an antibody may be determined using the program IMGT/DomainGap Align.

The term "monoclonal antibody," as used herein, refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256:495, or may be made by recombinant DNA methods (see, e.g., US Patent No. 4,816,567). Monoclonal antibodies may also be isolated from phage antibody libraries using the techniques described in Clackson et al. (1991) Nature 352:624-8, and Marks et al. (1991) J Mol Biol. 222:581-97, for example. The term also includes preparations of antibody molecules of single molecular composition. A monoclonal antibody composition displays a single binding specificity and affinity for a particular epitope.

The monoclonal antibodies described herein can be non-human, human, or humanized. The term specifically includes "chimeric" antibodies, in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they specifically bind the target antigen and/or exhibit the desired biological activity.

The term "human antibody," as used herein, refers an antibody produced by a human or an antibody having an amino acid sequence of an antibody produced by a human. The term includes antibodies having variable regions in which both the framework and CDR regions are derived from sequences of human origin. Furthermore, if the antibody contains a constant region, the constant region is also derived from such human sequences, e.g., human germline sequences, or mutated versions of human germline sequences or antibody containing consensus framework sequences derived from human framework sequences analysis, for example, as described in Knappik et al. ((2000) J Mol Biol. 296(1):57-86). The structures and locations of immunoglobulin variable domains, e.g., CDRs, may be defined using well known numbering schemes, e.g., the Kabat numbering scheme, the Chothia numbering scheme, or a combination of Kabat and Chothia, and/or ImMunoGenTics (IMGT) numbering. The human antibodies of the invention may include amino acid residues not encoded by human sequences (e.g., mutations introduced by random or site-specific mutagenesis in vitro or by somatic mutation in vivo, or a conservative substitution to promote stability or manufacturing). However, the term "human antibody," as used herein, is not intended to include antibodies in which CDR sequences derived from the germline of another mammalian species, such as a mouse, have been grafted onto human framework sequences.

The term "recombinant human antibody," as used herein, refers to a human antibody that is prepared, expressed, created, or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the human antibody, e.g., from a transfectoma, antibodies isolated from a recombinant, combinatorial human antibody library, and antibodies prepared, expressed, created or isolated by any other means that involve splicing of all or a portion of a human immunoglobulin gene, sequences to other DNA sequences. Such recombinant human antibodies have variable regions in which the framework and CDR regions are derived from human germline immunoglobulin sequences. In some embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

The term "chimeric antibody," as used herein, refers to antibodies wherein the amino acid sequence of the immunoglobulin molecule is derived from two or more species. In some instances, the variable regions of both heavy and light chains correspond to the variable regions of antibodies derived from one species with the desired specificity, affinity, and activity while the constant regions are homologous to antibodies derived from another species (e.g., human) to minimize an immune response in the latter species.

As used herein, the term "humanized antibody" refers to forms of antibodies that contain sequences from non-human (e.g., murine) antibodies as well as human antibodies. Such antibodies are a type of chimeric antibody which contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the framework (FR) regions are those of a human immunoglobulin sequence. The humanized antibody optionally also will comprise at least a portion of an immunoglobulin constant region (Fc), typically that of a human immunoglobulin. The humanized antibody can be further modified by the substitution of residues, either in the Fv framework region and/or within the replaced non-human residues to refine and optimize antibody specificity, affinity, and/or activity.

The term "Fc region," as used herein, refers to a polypeptide comprising the CH3, CH2 and at least a portion of the hinge region of a constant domain of an antibody. Optionally, an Fc region may include a CH4 domain, present in some antibody classes. An Fc region may comprise the entire hinge region of a constant domain of an antibody. In some embodiments, an antibody or antigen-binding fragment comprises an Fc region and a CH1 region of an antibody. In some embodiments, an antibody or antigen-binding fragment comprises an Fc region CH3 region of an antibody. In some embodiments, an antibody or antigen-binding fragment comprises an Fc region, a CH1 region, and a kappa/lambda region from the constant domain of an antibody. In some embodiments, an antibody or antigen-binding fragment comprises a constant region, e.g., a heavy chain constant region and/or a light chain constant region. In some embodiments, such a constant region is modified compared to a wild-type constant region. That is, the polypeptide may comprise alterations or modifications to one or more of the three heavy chain constant domains (CH1, CH2, or CH3) and/or to the light chain constant region domain (CL). Example modifications include additions, deletions, or substitutions of one or more amino acids in one or more domains. Such changes may be included to optimize effector function, half-life, etc.

"Internalizing" as used herein in reference to an antibody or antigen-binding fragment refers to an antibody or antigen-binding fragment that is capable of being taken through the cell's lipid bilayer membrane to an internal compartment (i.e., "internalized") upon binding to the cell, preferably into a degradative compartment in the cell. For example, an internalizing anti-HER2 antibody is one that is capable of being taken into the cell after binding to HER2 on the cell membrane. In some embodiments, the antibody or antigen-binding fragment used in the ADCs disclosed herein targets a cell surface antigen (e.g., BCMA, CD33, PCAD, or HER2) and is an internalizing antibody or internalizing antigen-binding fragment (i.e., the ADC transfers through the cellular membrane after antigen binding). In some embodiments, the internalizing antibody or antigen-binding fragment binds a receptor on the cell surface. An internalizing antibody or internalizing antigen-binding fragment that targets a receptor on the cell membrane may induce receptor-mediated endocytosis. In some embodiments, the internalizing antibody or internalizing antigen-binding fragment is taken into the cell via receptor-mediated endocytosis.

"Non-internalizing" as used herein in reference to an antibody or antigen-binding fragment refers to an antibody or antigen-binding fragment that remains at the cell surface upon binding to the cell. In some embodiments, the antibody or antigen-binding fragment used in the ADCs disclosed herein targets a cell surface antigen and is a non-internalizing antibody or non-internalizing antigen-binding fragment (i.e., the ADC remains at the cell surface and does not transfer through the cellular membrane after antigen binding). In some embodiments, the non-internalizing antibody or antigen-binding fragment binds a non-internalizing receptor or other cell surface antigen. Exemplary non-internalizing cell surface antigens include but are not limited to CA125 and CEA, and antibodies that bind to non-internalizing antigen targets are also known in the art (see, e.g., Bast et al. (1981) J Clin Invest. 68(5):1331-7; Scholler and Urban (2007) Biomark Med. 1(4):513-23; and Boudousq et al. (2013) PLoS One 8(7):e69613).

The term "B-cell maturation antigen" or "BCMA," as used herein, refers to any native form of human BCMA (also known as tumor necrosis factor receptor superfamily member 17 (TNFRSF17)). The term encompasses full-length human BCMA (e.g., UniProt Reference Sequence: Q02223; SEQ ID NO:72), as well as any form of human BCMA that may result from cellular processing. The term also encompasses functional variants or fragments of human BCMA, including but not limited to splice variants, allelic variants, and isoforms that retain one or more biologic functions of human BCMA (i.e., variants and fragments are encompassed unless the context indicates that the term is used to refer to the wild-type protein only). BCMA can be isolated from human, or may be produced recombinantly or by synthetic methods.

The term "anti-BCMA antibody" or "antibody that binds to BCMA," as used herein, refers to any form of antibody or antigen-binding fragment thereof that binds, e.g., specifically binds, to BCMA. The term encompasses monoclonal antibodies (including full-length monoclonal antibodies), polyclonal antibodies, and biologically functional antigen-binding fragments so long as they bind, e.g., specifically bind, to BCMA. WO 2012/163805 provides exemplary BCMA-binding sequences, including exemplary anti-BCMA antibody sequences. In some embodiments, the anti-BCMA antibody used in the ADCs disclosed herein is an internalizing antibody or internalizing antigen-binding fragment. J6M0 (WO 2012/163805) is an exemplary anti-BCMA antibody.

The term "myeloid cell surface antigen CD33" or "CD33," as used herein, refers to any native form of human CD33 (also known as sialic acid binding Ig-like lectin 3 (SIGLEC3)). The term encompasses full-length human CD33 (e.g., UniProt Reference Sequence: P20138; SEQ ID NO:73), as well as any form of human CD33 that may result from cellular processing. The term also encompasses functional variants or fragments of human CD33, including but not limited to splice variants, allelic variants, and isoforms that retain one or more biologic functions of human CD33 (i.e., variants and fragments are encompassed unless the context indicates that the term is used to refer to the wild-type protein only). CD33 can be isolated from human, or may be produced recombinantly or by synthetic methods.

The term "anti-CD33 antibody" or "antibody that binds to CD33," as used herein, refers to any form of antibody or antigen-binding fragment thereof that binds, e.g., specifically binds, to CD33. The term encompasses monoclonal antibodies (including full-length monoclonal antibodies), polyclonal antibodies, and biologically functional antigen-binding fragments so long as they bind, e.g., specifically bind, to CD33. US 2013/0078241 provides exemplary CD33-binding sequences, including exemplary anti-CD33 antibody sequences. In some embodiments, the anti-CD33 antibody used in the ADCs disclosed herein is an internalizing antibody or internalizing antigen-binding fragment. MuMy9-6ch (US 2013/0078241) is an exemplary anti-CD33 antibody.

The term "P-cadherin" or "PCAD," as used herein, refers to any native form of human PCAD (also known as cadherin 3, type 1 or CDH3). The term encompasses full-length human PCAD (e.g., UniProt Reference Sequence: P22223; SEQ ID NO:74), as well as any form of human PCAD that may result from cellular processing. The term also encompasses functional variants or fragments of human PCAD, including but not limited to splice variants, allelic variants, and isoforms that retain one or more biologic functions of human PCAD (i.e., variants and fragments are encompassed unless the context indicates that the term is used to refer to the wild-type protein only). PCAD can be isolated from human, or may be produced recombinantly or by synthetic methods.

The term "anti-PCAD antibody" or "antibody that binds to PCAD," as used herein, refers to any form of antibody or antigen-binding fragment thereof that binds, e.g., specifically binds, to PCAD. The term encompasses monoclonal antibodies (including full-length monoclonal antibodies), polyclonal antibodies, and biologically functional antigen-binding fragments so long as they bind, e.g., specifically bind, to PCAD. WO 2016/203432 provides exemplary PCAD-binding sequences, including exemplary anti-PCAD antibody sequences. In some embodiments, the anti-PCAD antibody used in the ADCs disclosed herein is an internalizing antibody or internalizing antigen-binding fragment. NOV169N31Q (WO 2016/203432) is an exemplary anti-PCAD antibody.

The term "human epidermal growth factor receptor 2," "HER2," or "HER2/NEU," as used herein, refers to any native form of human HER2. The term encompasses full-length human HER2 (e.g., UniProt Reference Sequence: P04626; SEQ ID NO:75), as well as any form of human HER2 that may result from cellular processing. The term also encompasses functional variants or fragments of human HER2, including but not limited to splice variants, allelic variants, and isoforms that retain one or more biologic functions of human HER2 (i.e., variants and fragments are encompassed unless the context indicates that the term is used to refer to the wild-type protein only). HER2 can be isolated from human, or may be produced recombinantly or by synthetic methods.

The term "anti-HER2 antibody" or "antibody that binds to HER2," as used herein, refers to any form of antibody or antigen-binding fragment thereof that binds, e.g., specifically binds, to HER2. The term encompasses monoclonal antibodies (including full-length monoclonal antibodies), polyclonal antibodies, and biologically functional antigen-binding fragments so long as they bind, e.g., specifically bind, to HER2. US Patent Nos. 5,821,337 and 6,870,034 provide exemplary HER2-binding sequences, including exemplary anti-HER2 antibody sequences. In some embodiments, the anti-HER2 antibody used in the ADCs disclosed herein is an internalizing antibody or internalizing antigen-binding fragment. Trastuzumab (US Patent Nos. 5,821,337 and 6,870,034; see also Molina et al. (2001) Cancer Res. 61(12):4744-9) is an exemplary anti-HER2 antibody.

The term "cluster of differentiation 38" or "CD38," as used herein, refers to any native form of human CD38 (also known as ADP-ribosyl cyclase/cyclic ADP-ribose hydrolase). The term encompasses full-length human CD38 (e.g., UniProt Reference Sequence: P28907; SEQ ID NO:76), as well as any form of human CD38 that may result from cellular processing. The term also encompasses functional variants or fragments of human CD38, including but not limited to splice variants, allelic variants, and isoforms that retain one or more biologic functions of human CD38 (i.e., variants and fragments are encompassed unless the context indicates that the term is used to refer to the wild-type protein only). CD38 can be isolated from human, or may be produced recombinantly or by synthetic methods.

The term "cluster of differentiation 48" or "CD48," as used herein, refers to any native form of human CD48 (also known as B-lymphocyte activation marker (BLAST-1) or signaling lymphocytic activation molecule 2 (SLAMF2)). The term encompasses full-length human CD48 (e.g., UniProt Reference Sequence: P09326; SEQ ID NO:77), as well as any form of human CD48 that may result from cellular processing. The term also encompasses functional variants or fragments of human CD48, including but not limited to splice variants, allelic variants, and isoforms that retain one or more biologic functions of human CD48 (i.e., variants and fragments are encompassed unless the context indicates that the term is used to refer to the wild-type protein only). CD48 can be isolated from human, or may be produced recombinantly or by synthetic methods.

The term "cluster of differentiation 79b" or "CD79b," as used herein, refers to any native form of human CD79b (also known as B-cell antigen receptor complex-associated protein beta chain). The term encompasses full-length human CD79b (e.g., UniProt Reference Sequence: P40259; SEQ ID NO:78), as well as any form of human CD79b that may result from cellular processing. The term also encompasses functional variants or fragments of human CD79b, including but not limited to splice variants, allelic variants, and isoforms that retain one or more biologic functions of human CD79b (i.e., variants and fragments are encompassed unless the context indicates that the term is used to refer to the wild-type protein only). CD79b can be isolated from human, or may be produced recombinantly or by synthetic methods.

The term "binding specificity," as used herein, refers to the ability of an individual antibody or antigen binding fragment to preferentially react with one antigenic determinant over a different antigenic determinant. The degree of specificity indicates the extent to which an antibody or fragment preferentially binds to one antigenic determinant over a different antigenic determinant. Also, as used herein, the term "specific," "specifically binds," and "binds specifically" refers to a binding reaction between an antibody or antigen-binding fragment (e.g., an anti-HER2 antibody) and a target antigen (e.g., HER2) in a heterogeneous population of proteins and other biologics. Antibodies can be tested for specificity of binding by comparing binding to an appropriate antigen to binding to an irrelevant antigen or antigen mixture under a given set of conditions. If the antibody binds to the appropriate antigen with at least 2, 5, 7, 10 or more times more affinity than to the irrelevant antigen or antigen mixture, then it is considered to be specific. A "specific antibody" or a "target-specific antibody" is one that only binds the target antigen (e.g., BCMA, CD33, PCAD, or HER2), but does not bind (or exhibits minimal binding) to other antigens. In some embodiments, an antibody or antigen-binding fragment that specifically binds a target antigen (e.g., BCMA, CD33, PCAD, or HER2) has a K_{D} of less than 1x10⁻⁶ M, less than 1x10⁻⁷ M, less than 1x10⁻⁸ M, less than 1x10⁻⁹ M, less than 1x10⁻¹⁰ M, less than 1x10⁻¹¹ M, less than 1x10⁻¹² M, or less than 1x10⁻¹³ M. In some embodiments, the K_{D} is 1 pM to 500 pM. In some embodiments, the K_{D} is between 500 pM to 1 µM, 1 µM to 100 nM, or 100 mM to 10 nM.

The term "affinity," as used herein, refers to the strength of interaction between antibody and antigen at single antigenic sites. Without being bound by theory, within each antigen binding site, the variable region of the antibody "arm" interacts through weak noncovalent forces with the antigen at numerous sites; the more interactions, typically the stronger the affinity. The binding affinity of an antibody is the sum of the attractive and repulsive forces operating between the antigenic determinant and the binding site of the antibody.

The term "kₒₙ" or "kₐ" refers to the on-rate constant for association of an antibody to the antigen to form the antibody/antigen complex. The rate can be determined using standard assays, such as a surface plasmon resonance, biolayer inferometry, or ELISA assay.

The term "k_{off}" or "k_{d}" refers to the off-rate constant for dissociation of an antibody from the antibody/antigen complex. The rate can be determined using standard assays, such as a surface plasmon resonance, biolayer inferometry, or ELISA assay.

The term "K_{D}" refers to the equilibrium dissociation constant of a particular antibody-antigen interaction. K_{D} is calculated by kₐ/k_{d}. The rate can be determined using standard assays, such as a surface plasmon resonance, biolayer inferometry, or ELISA assay.

The term "epitope" refers to the portion of an antigen capable of being recognized and specifically bound by an antibody (or antigen-binding fragment). Epitope determinants generally consist of chemically active surface groupings of molecules such as amino acids or carbohydrate or sugar side chains and can have specific three-dimensional structural characteristics, as well as specific charge characteristics. When the antigen is a polypeptide, epitopes can be formed from contiguous amino acids or noncontiguous amino acids juxtaposed by tertiary folding of the polypeptide. An epitope may be "linear" or "conformational." Conformational and linear epitopes are distinguished in that the binding to the former but not the latter is lost in the presence of denaturing solvents. The epitope bound by an antibody (or antigen-binding fragment) may be identified using any epitope mapping technique known in the art, including X-ray crystallography for epitope identification by direct visualization of the antigen-antibody complex, as well as monitoring the binding of the antibody to fragments or mutated variations of the antigen, or monitoring solvent accessibility of different parts of the antibody and the antigen. Exemplary strategies used to map antibody epitopes include, but are not limited to, array-based oligo-peptide scanning, limited proteolysis, site-directed mutagenesis, high-throughput mutagenesis mapping, hydrogen-deuterium exchange, and mass spectrometry (see, e.g., Gershoni et al. (2007) BioDrugs 21:145-56; and Hager-Braun and Tomer (2005) Expert Rev Proteomics 2:745-56).

Competitive binding and epitope binning can also be used to determine antibodies sharing identical or overlapping epitopes. Competitive binding can be evaluated using a cross-blocking assay, such as the assay described in "Antibodies, A Laboratory Manual," Cold Spring Harbor Laboratory, Harlow and Lane (1st edition 1988, 2nd edition 2014). In some embodiments, competitive binding is identified when a test antibody or binding protein reduces binding of a reference antibody or binding protein to a target antigen such as BCMA, CD33, PCAD, or HER2 (e.g., a binding protein comprising CDRs and/or variable domains selected from those identified in Tables 3-5), by at least about 50% in the cross-blocking assay (e.g., 50%, 60%, 70%, 80%, 90%, 95%, 99%, 99.5%, or more, or any percentage in between), and/or vice versa. In some embodiments, competitive binding can be due to shared or similar (e.g., partially overlapping) epitopes, or due to steric hindrance where antibodies or binding proteins bind at nearby epitopes (see, e.g., Tzartos, Methods in Molecular Biology (Morris, ed. (1998) vol. 66, pp. 55-66)). In some embodiments, competitive binding can be used to sort groups of binding proteins that share similar epitopes. For example, binding proteins that compete for binding can be "binned" as a group of binding proteins that have overlapping or nearby epitopes, while those that do not compete are placed in a separate group of binding proteins that do not have overlapping or nearby epitopes.

As used herein, the terms "peptide," "polypeptide," and "protein" are used interchangeably to refer to a polymer of amino acid residues. The terms encompass amino acid polymers comprising two or more amino acids joined to each other by peptide bonds, amino acid polymers in which one or more amino acid residues is an artificial chemical mimetic of a corresponding naturally-occurring amino acid, as well as naturally-occurring amino acid polymers and non-naturally-occurring amino acid polymers. The terms include, for example, biologically active fragments, substantially homologous polypeptides, oligopeptides, homodimers, heterodimers, variants of polypeptides, modified polypeptides, derivatives, analogs, fusion proteins, among others. The terms also include natural peptides, recombinant peptides, synthetic peptides, or a combination thereof. Unless otherwise indicated, a particular polypeptide sequence also implicitly encompasses conservatively modified variants thereof.

A "recombinant" protein refers to a protein (e.g., an antibody) made using recombinant techniques, e.g., through the expression of a recombinant nucleic acid.

An "isolated" protein refers to a protein unaccompanied by at least some of the material with which it is normally associated in its natural state. For example, a naturally-occurring polynucleotide or polypeptide present in a living organism is not isolated, but the same polynucleotide or polypeptide separated from some or all of the coexisting materials in the living organism, is isolated. The definition includes the production of an antibody in a wide variety of organisms and/or host cells that are known in the art.

An "isolated antibody," as used herein, is an antibody that has been identified and separated from one or more (e.g., the majority) of the components (by weight) of its source environment, e.g., from the components of a hybridoma cell culture or a different cell culture that was used for its production. In some embodiments, the separation is performed such that it sufficiently removes components that may otherwise interfere with the suitability of the antibody for the desired applications (e.g., for therapeutic use). Methods for preparing isolated antibodies are known in the art and include, without limitation, protein A chromatography, anion exchange chromatography, cation exchange chromatography, virus retentive filtration, and ultrafiltration.

As used herein, the term "variant" refers to a nucleic acid sequence or an amino acid sequence that differs from a reference nucleic acid sequence or amino acid sequence respectively, but retains one or more biological properties of the reference sequence. A variant may contain one or more amino acid substitutions, deletions, and/or insertions (or corresponding substitution, deletion, and/or insertion of codons) with respect to a reference sequence. Changes in a nucleic acid variant may not alter the amino acid sequence of a peptide encoded by the reference nucleic acid sequence, or may result in amino acid substitutions, additions, deletions, fusions, and/or truncations. In some embodiments, a nucleic acid variant disclosed herein encodes an identical amino acid sequence to that encoded by the unmodified nucleic acid or encodes a modified amino acid sequence that retains one or more functional properties of the unmodified amino acid sequence. Changes in the sequence of peptide variants are typically limited or conservative, so that the sequences of the unmodified peptide and the variant are closely similar overall and, in many regions, identical. In some embodiments, a peptide variant retains one or more functional properties of the unmodified peptide sequence. A variant and unmodified peptide can differ in amino acid sequence by one or more substitutions, additions, deletions in any combination.

A variant of a nucleic acid or peptide can be a naturally-occurring variant or a variant that is not known to occur naturally. Variants of nucleic acids and peptides may be made by mutagenesis techniques, by direct synthesis, or by other techniques known in the art. A variant does not necessarily require physical manipulation of the reference sequence. As long as a sequence contains a different nucleic acid or amino acid as compared to a reference sequence, it is considered a "variant" regardless of how it was synthesized. In some embodiments, a variant has high sequence identity (i.e., 60% nucleic acid or amino acid sequence identity or higher) as compared to a reference sequence. In some embodiments, a peptide variant encompasses polypeptides having amino acid substitutions, deletions, and/or insertions as long as the polypeptide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% amino acid sequence identity with a reference sequence, or with a corresponding segment (e.g., a functional fragment) of a reference sequence, e.g., those variants that also retain one or more functions of the reference sequence. In some embodiments, a nucleic acid variant encompasses polynucleotides having amino acid substitutions, deletions, and/or insertions as long as the polynucleotide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% nucleic acid sequence identity with a reference sequence, or with a corresponding segment (e.g., a functional fragment) of a reference sequence.

The term "conservatively modified variant" applies to both amino acid and nucleic acid sequences. For nucleic acid sequences, conservatively modified variants refer to those nucleic acids which encode identical or essentially identical amino acid sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence. For polypeptide sequences, conservatively modified variants include individual substitutions, deletions, or additions to a polypeptide sequence which result in the substitution of an amino acid with a chemically similar amino acid. Conservative substitutions providing functionally similar amino acids are well known in the art.

The term "conservative sequence modifications," as used herein, refers to amino acid modifications that do not significantly affect or alter the binding characteristics of, e.g., an antibody or antigen-binding fragment containing the amino acid sequence. Such conservative modifications include amino acid substitutions, additions, and deletions. Modifications can be introduced into an antibody or antigen-binding fragment by standard techniques known in the art, such as, e.g., site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, in some embodiments, one or more amino acid residues within an antibody can be replaced with other amino acid residues from the same side chain family and the altered antibody can be tested using the functional assays described herein.

The term "homologous" or "identity," as used herein, refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions. For example, if half (e.g., five positions in a polymer ten subunits in length) of the positions in two sequences are matched or homologous, the two sequences are 50% homologous; if 90% of the positions (e.g., 9 of 10), are matched or homologous, the two sequences are 90% homologous.

Percentage of "sequence identity" can be determined by comparing two optimally aligned sequences over a comparison window, where the fragment of the amino acid sequence in the comparison window may comprise additions or deletions (e.g., gaps or overhangs) as compared to the reference sequence (which does not comprise additions or deletions) for optimal alignment of the two sequences. The percentage can be calculated by determining the number of positions at which the identical amino acid residue occurs in both sequences to yield the number of matched positions, dividing the number of matched positions by the total number of positions in the window of comparison, and multiplying the result by 100 to yield the percentage of sequence identity. The output is the percent identity of the subject sequence with respect to the query sequence. The percent identity between the two sequences is a function of the number of identical positions shared by the sequences, taking into account the number of gaps, and the length of each gap, which need to be introduced for optimal alignment of the two sequences. Generally, the amino acid identity or homology between proteins disclosed herein and variants thereof, including variants of target antigens (such as BCMA, CD33, PCAD, or HER2) and variants of antibody variable domains (including individual variant CDRs), is at least 80% to the sequences depicted herein, e.g., identities or homologies of at least 85%, 90%, 91%, 92%, 93%, 94%, 95%, 96%, 97%, 98%, 99%, almost 100%, or 100%.

The comparison of sequences and determination of percent identity between two sequences can be accomplished using a mathematical algorithm. In some embodiments, the percent identity between two amino acid sequences is determined using the Needleman and Wunsch ((1970) J Mol Biol. 48:444-53) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In some embodiments, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. An exemplary set of parameters is a Blossum 62 scoring matrix with a gap penalty of 12, a gap extend penalty of 4, and a frameshift gap penalty of 5. The percent identity between two amino acid or nucleotide sequences can also be determined using the algorithm of Meyers and Miller ((1989) CABIOS 4:11-17) which has been incorporated into the ALIGN program (version 2.0), using a PAM120 weight residue table, a gap length penalty of 12 and a gap penalty of 4.

The term "agent" is used herein to refer to a chemical compound, a mixture of chemical compounds, a biological macromolecule, an extract made from biological materials, or a combination of two or more thereof. The term "therapeutic agent" or "drug" refers to an agent that is capable of modulating a biological process and/or has biological activity. The Mcl-1 inhibitors and the ADCs comprising them, as described herein, are exemplary therapeutic agents.

The term "chemotherapeutic agent" or "anti-cancer agent" is used herein to refer to all agents that are effective in treating cancer (regardless of mechanism of action). Inhibition of metastasis or angiogenesis is frequently a property of a chemotherapeutic agent. Chemotherapeutic agents include antibodies, biological molecules, and small molecules, and encompass the Mcl-1 inhibitors and ADCs comprising them, as described herein. A chemotherapeutic agent may be a cytotoxic or cytostatic agent. The term "cytostatic agent" refers to an agent that inhibits or suppresses cell growth and/or multiplication of cells. The term "cytotoxic agent" refers to a substance that causes cell death primarily by interfering with a cell's expression activity and/or functioning.

The term "myeloid cell leukemia 1" or "Mcl-1," as used herein, refers to any native form of human Mcl-1, an anti-apoptotic member of the Bcl-2 protein family. The term encompasses full-length human Mcl-1 (e.g., UniProt Reference Sequence: Q07820; SEQ ID NO:71), as well as any form of human Mcl-1 that may result from cellular processing. The term also encompasses functional variants or fragments of human Mcl-1, including but not limited to splice variants, allelic variants, and isoforms that retain one or more biologic functions of human Mcl-1 (i.e., variants and fragments are encompassed unless the context indicates that the term is used to refer to the wild-type protein only). Mcl-1 can be isolated from human, or may be produced recombinantly or by synthetic methods.

The term "inhibit" or "inhibition" or "inhibiting," as used herein, means to reduce a biological activity or process by a measurable amount, and can include but does not require complete prevention or inhibition. In some embodiments, "inhibition" means to reduce the expression and/or activity of Mcl-1 and/or one or more upstream modulators or downstream targets thereof.

The term "Mcl-1 inhibitor," as used herein, refers to an agent capable of reducing the expression and/or activity of Mcl-1 and/or one or more upstream modulators or downstream targets thereof. Exemplary Mcl-1 modulators (including exemplary inhibitors of Mcl-1) are described in WO 2015/097123; WO 2016/207216; WO 2016/207217; WO 2016/207225; WO 2016/207226; WO 2017/125224; WO 2019/035899, WO 2019/035911, WO 2019/035914, WO 2019/035927, US 2019/0055264, WO 2016/033486, WO 2017/147410, WO 2018/183418, and WO 2017/182625, including exemplary Mcl-1 inhibitors, that can be included as drug moieties in the disclosed ADCs. For example, exemplary Mcl-1 inhibitors that can be included as drug moieties in the disclosed ADCs are those of formula: 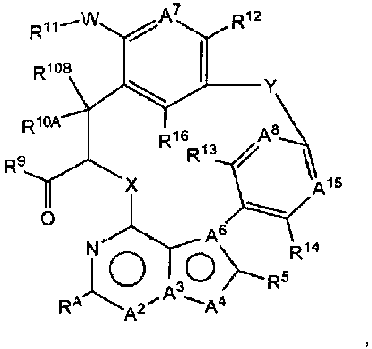 wherein each variable is defined as in WO2019/035911; WO 2019/035899; WO 2019/035914; or WO 2019/035927. Specific examples include, e.g., 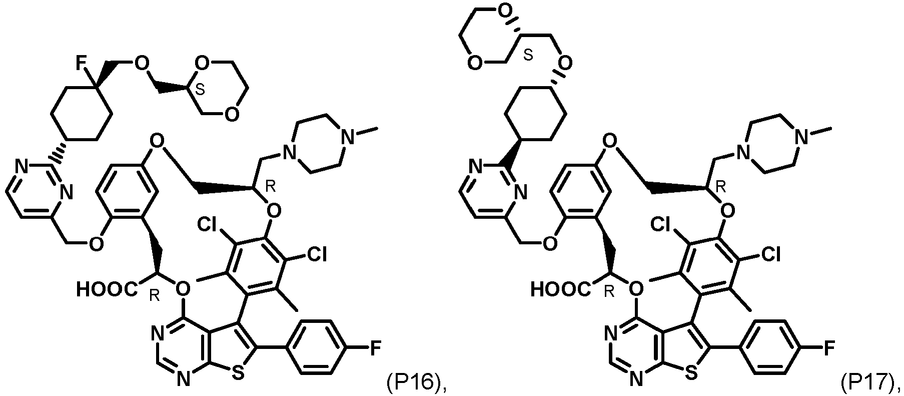 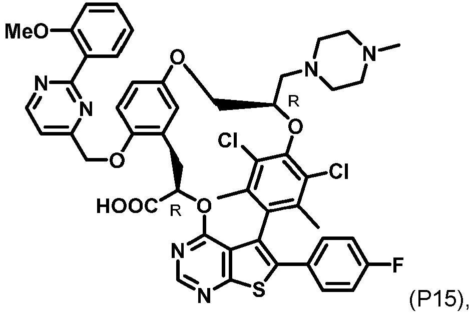 wherein each compound as a drug payload can be conjugated to an antibody or a linker via the nitrogen atom of the N-methyl in piperazinyl functional group of the compound. As used herein, the terms "derivative" and "analog" when referring to an Mcl-1 inhibitor, or the like, means any such compound that retains essentially the same, similar, or enhanced biological function or activity as compared to the original compound but has an altered chemical or biological structure.

As used herein, a "Mcl-1 inhibitor drug moiety", "Mcl-1 inhibitor", and the like refer to the component of an ADC or composition that provides the structure of an Mcl-1 inhibitor compound or a compound modified for attachment to an ADC that retains essentially the same, similar, or enhanced biological function or activity as compared to the original compound. In some embodiments, Mcl-1 inhibitor drug moiety is component (D) in an ADC of Formula (1).

The term "cancer," as used herein, refers to the presence of cells possessing characteristics typical of cancer-causing cells, such as uncontrolled proliferation, immortality, metastatic potential, rapid growth and proliferation rate, and/or certain morphological features. Often, cancer cells can be in the form of a tumor or mass, but such cells may exist alone within a subject, or may circulate in the blood stream as independent cells, such as leukemic or lymphoma cells. The term "cancer" includes all types of cancers and cancer metastases, including hematological cancers, solid tumors, sarcomas, carcinomas and other solid and non-solid tumor cancers. Hematological cancers may include B-cell malignancies, cancers of the blood (leukemias), cancers of plasma cells (myelomas, e.g., multiple myeloma), or cancers of the lymph nodes (lymphomas). Exemplary B-cell malignancies include chronic lymphocytic leukemia (CLL), follicular lymphoma, mantle cell lymphoma, and diffuse large B-cell lymphoma. Leukemias may include acute lymphoblastic leukemia (ALL), acute myeloid leukemia (AML), chronic lymphocytic leukemia (CLL), chronic myelogenous leukemia (CML), chronic myelomonocytic leukemia (CMML), acute monocytic leukemia (AMoL), etc. Lymphomas may include Hodgkin's lymphoma, non-Hodgkin's lymphoma, etc. Other hematologic cancers may include myelodysplasia syndrome (MDS). Solid tumors may include carcinomas such as adenocarcinoma, e.g., breast cancer, pancreatic cancer, prostate cancer, colon or colorectal cancer, lung cancer, gastric cancer, cervical cancer, endometrial cancer, ovarian cancer, cholangiocarcinoma, glioma, melanoma, etc. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

As used herein, the term "tumor" refers to any mass of tissue that results from excessive cell growth or proliferation, either benign or malignant, including precancerous lesions. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer.

The terms "tumor cell" and "cancer cell" may be used interchangeably herein and refer to individual cells or the total population of cells derived from a tumor or cancer, including both non-tumorigenic cells and cancer stem cells. The terms "tumor cell" and "cancer cell" will be modified by the term "non-tumorigenic" when referring solely to those cells lacking the capacity to renew and differentiate to distinguish those cells from cancer stem cells.

The term "target-negative," "target antigen-negative," or "antigen-negative," as used herein, refers to the absence of target antigen expression by a cell or tissue. The term "target-positive," "target antigen-positive," or "antigen-positive" refers to the presence of target antigen expression. For example, a cell or a cell line that does not express a target antigen may be described as target-negative, whereas a cell or cell line that expresses a target antigen may be described as target-positive.

The terms "subject" and "patient" are used interchangeably herein to refer to any human or non-human animal in need of treatment. Non-human animals include all vertebrates (e.g., mammals and non-mammals) such as any mammal. Non-limiting examples of mammals include humans, chimpanzees, apes, monkeys, cattle, horses, sheep, goats, swine, rabbits, dogs, cats, rats, mice, and guinea pigs. Non-limiting examples of non-mammals include birds and fish. In some embodiments, the subject is a human.

The term "a subject in need of treatment," as used herein, refers to a subject that would benefit biologically, medically, or in quality of life from a treatment (e.g., a treatment with any one or more of the exemplary ADC compounds described herein).

As used herein, the term "treat," "treating," or "treatment" refers to any improvement of any consequence of disease, disorder, or condition, such as prolonged survival, less morbidity, and/or a lessening of side effects which result from an alternative therapeutic modality. In some embodiments, treatment comprises delaying or ameliorating a disease, disorder, or condition (i.e., slowing or arresting or reducing the development of a disease or at least one of the clinical symptoms thereof). In some embodiments, treatment comprises delaying, alleviating, or ameliorating at least one physical parameter of a disease, disorder, or condition, including those which may not be discernible by the patient. In some embodiments, treatment comprises modulating a disease, disorder, or condition, either physically (e.g., stabilization of a discernible symptom), physiologically (e.g., stabilization of a physical parameter), or both. In some embodiments, treatment comprises administration of a described ADC compound or composition to a subject, e.g., a patient, to obtain a treatment benefit enumerated herein. The treatment can be to cure, heal, alleviate, delay, prevent, relieve, alter, remedy, ameliorate, palliate, improve, or affect a disease, disorder, or condition (e.g., a cancer), the symptoms of a disease, disorder, or condition (e.g., a cancer), or a predisposition toward a disease, disorder, or condition (e.g., a cancer). In some embodiments, in addition to treating a subject having a disease, disorder, or condition, a composition disclosed herein can also be provided prophylactically to prevent or reduce the likelihood of developing that disease, disorder, or condition.

As used herein, the term "prevent", "preventing," or "prevention" of a disease, disorder, or condition refers to the prophylactic treatment of the disease, disorder, or condition; or delaying the onset or progression of the disease, disorder, or condition.

As used herein, a "pharmaceutical composition" refers to a preparation of a composition, e.g., an ADC compound or composition, in addition to at least one other (and optionally more than one other) component suitable for administration to a subject, such as a pharmaceutically acceptable carrier, stabilizer, diluent, dispersing agent, suspending agent, thickening agent, and/or excipient. The pharmaceutical compositions provided herein are in such form as to permit administration and subsequently provide the intended biological activity of the active ingredient(s) and/or to achieve a therapeutic effect. The pharmaceutical compositions provided herein preferably contain no additional components which are unacceptably toxic to a subject to which the formulation would be administered.

As used herein, the terms "pharmaceutically acceptable carrier" and "physiologically acceptable carrier," which may be used interchangeably, refer to a carrier or a diluent that does not cause significant irritation to a subject and does not abrogate the biological activity and properties of the administered ADC compound or composition and/or any additional therapeutic agent in the composition. Pharmaceutically acceptable carriers may enhance or stabilize the composition or can be used to facilitate preparation of the composition. Pharmaceutically acceptable carriers can include solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated. The carrier may be selected to minimize adverse side effects in the subject, and/or to minimize degradation of the active ingredient(s). An adjuvant may also be included in any of these formulations.

As used herein, the term "excipient" refers to an inert substance added to a pharmaceutical composition to further facilitate administration of an active ingredient. Formulations for parenteral administration can, for example, contain excipients such as sterile water or saline, polyalkylene glycols such as polyethylene glycol, vegetable oils, or hydrogenated napthalenes. Other exemplary excipients include, but are not limited to, calcium bicarbonate, calcium phosphate, various sugars and types of starch, cellulose derivatives, gelatin, ethylene-vinyl acetate co-polymer particles, and surfactants, including, for example, polysorbate 20.

The term "pharmaceutically acceptable salt," as used herein, refers to a salt which does not abrogate the biological activity and properties of the compounds of the invention, and does not cause significant irritation to a subject to which it is administered. Examples of such salts include, but are not limited to: (a) acid addition salts formed with inorganic acids, for example, hydrochloric acid, hydrobromic acid, sulfuric acid, phosphoric acid, nitric acid and the like; and salts formed with organic acids, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (b) salts formed from elemental anions such as chlorine, bromine, and iodine. See, e.g., Haynes et al., "Commentary: Occurrence of Pharmaceutically Acceptable Anions and Cations in the Cambridge Structural Database," J. Pharmaceutical Sciences, vol. 94, no. 10 (2005), and Berge et al., "Pharmaceutical Salts," J. Pharmaceutical Sciences, vol. 66, no. 1 (1977).

In some embodiments, depending on their electronic charge, the antibody-drug conjugates (ADCs), linkers, payloads and linker-payloads described herein can contain a monovalent anionic counterion M₁⁻. Any suitable anionic counterion can be used. In certain embodiments, the monovalent anionic counterion is a pharmaceutically acceptable monovalent anionic counterion. In certain embodiments, the monovalent anionic counterion M₁⁻ can be selected from bromide, chloride, iodide, acetate, trifluoroacetate, benzoate, mesylate, tosylate, triflate, formate, or the like. In some embodiments, the monovalent anionic counterion M₁⁻ is trifluoroacetate or formate.

As used herein, the term "therapeutically effective amount" or "therapeutically effective dose," refers to an amount of a compound described herein, e.g., an ADC compound or composition described herein, to effect the desired therapeutic result (i.e., reduction or inhibition of an enzyme or a protein activity, amelioration of symptoms, alleviation of symptoms or conditions, delay of disease progression, a reduction in tumor size, inhibition of tumor growth, prevention of metastasis). In some embodiments, a therapeutically effective amount does not induce or cause undesirable side effects. In some embodiments, a therapeutically effective amount induces or causes side effects but only those that are acceptable by a treating clinician in view of a patient's condition. In some embodiments, a therapeutically effective amount is effective for detectable killing, reduction, and/or inhibition of the growth or spread of cancer cells, the size or number of tumors, and/or other measure of the level, stage, progression and/or severity of a cancer. The term also applies to a dose that will induce a particular response in target cells, e.g., a reduction, slowing, or inhibition of cell growth. A therapeutically effective amount can be determined by first administering a low dose, and then incrementally increasing that dose until the desired effect is achieved. A therapeutically effective amount can also vary depending upon the intended application (in vitro or in vivo), or the subject and disease condition being treated, e.g., the weight and age of the subject, the severity of the disease condition, the manner of administration and the like, which can readily be determined by one of ordinary skill in the art. The specific amount may vary depending on, for example, the particular pharmaceutical composition, the subject and their age and existing health conditions or risk for health conditions, the dosing regimen to be followed, the severity of the disease, whether it is administered in combination with other agents, timing of administration, the tissue to which it is administered, and the physical delivery system in which it is carried. In the case of cancer, a therapeutically effective amount of an ADC may reduce the number of cancer cells, reduce tumor size, inhibit (e.g., slow or stop) tumor metastasis, inhibit (e.g., slow or stop) tumor growth, and/or relieve one or more symptoms.

As used herein, the term "prophylactically effective amount" or "prophylactically effective dose," refers to an amount of a compound disclosed herein, e.g., an ADC compound or composition described herein, that is effective, at dosages and for periods of time necessary, to achieve the desired prophylactic result. Typically, since a prophylactic dose is used in subjects prior to or at an earlier stage of disease, the prophylactically effective amount will be less than the therapeutically effective amount. In some embodiments, a prophylactically effective amount can prevent the onset of disease symptoms, including symptoms associated with a cancer.

The term "p" or "drug loading" or "drug:antibody ratio" or "drug-to-antibody ratio" or "DAR" refers to the number of drug moieties per antibody or antigen-binding fragment, i.e., drug loading, or the number of -L-D moieties per antibody or antigen-binding fragment (Ab) in ADCs of Formula (1). In ADCs comprising an Mcl-1 inhibitor drug moiety, "p" refers to the number of Mcl-1 inhibitor compounds linked to the antibody or antigen-binding fragment. For example, if two Mcl-1 inhibitor compounds are linked to an antibody or antigen-binding fragment, p = 2. In compositions comprising multiple copies of ADCs of Formula (1), "average p" refers to the average number of -L-D moieties per antibody or antigen-binding fragment, also referred to as "average drug loading."

### Antibody-Drug Conjugates

The antibody-drug conjugate (ADC) compounds of the present disclosure include those with anti-cancer activity. In particular, the ADC compounds include an antibody or antigen-binding fragment conjugated (i.e., covalently attached by a linker) to a drug moiety (e.g., an Mcl-1 inhibitor), wherein the drug moiety when not conjugated to an antibody or antigen-binding fragment has a cytotoxic or cytostatic effect. In some embodiments, the drug moiety when not conjugated to an antibody or antigen-binding fragment is capable of reducing the expression and/or activity of Mcl-1 and/or one or more upstream modulators or downstream targets thereof. Without being bound by theory, by targeting Mcl-1 expression and/or activity, in some embodiments, the ADCs disclosed herein may provide potent anti-cancer agents. Also, without being bound by theory, by conjugating the drug moiety to an antibody that binds an antigen associated with expression in a tumor cell or cancer, the ADC may provide improved activity, better cytotoxic specificity, and/or reduced off-target killing as compared to the drug moiety when administered alone.

In some embodiments, therefore, the components of the ADC are selected to (i) retain one or more therapeutic properties exhibited by the antibody and drug moieties in isolation, (ii) maintain the specific binding properties of the antibody or antigen-binding fragment; (iii) optimize drug loading and drug-to-antibody ratios; (iv) allow delivery, e.g., intracellular delivery, of the drug moiety via stable attachment to the antibody or antigen-binding fragment; (v) retain ADC stability as an intact conjugate until transport or delivery to a target site; (vi) minimize aggregation of the ADC prior to or after administration; (vii) allow for the therapeutic effect, e.g., cytotoxic effect, of the drug moiety after cleavage or other release mechanism in the cellular environment; (viii) exhibit in vivo anti-cancer treatment efficacy comparable to or superior to that of the antibody and drug moieties in isolation; (ix) minimize off-target killing by the drug moiety; and/or (x) exhibit desirable pharmacokinetic and pharmacodynamics properties, formulatability, and toxicologic/immunologic profiles. Each of these properties may provide for an improved ADC for therapeutic use (Ab et al. (2015) Mol Cancer Ther. 14:1605-13).

The ADC compounds of the present disclosure may selectively deliver an effective dose of a cytotoxic or cytostatic agent to cancer cells or to tumor tissue. In some embodiments, the cytotoxic and/or cytostatic activity of the ADC is dependent on target antigen expression in a cell. In some embodiments, the disclosed ADCs are particularly effective at killing cancer cells expressing a target antigen while minimizing off-target killing. In some embodiments, the disclosed ADCs do not exhibit a cytotoxic and/or cytostatic effect on cancer cells that do not express a target antigen.

Exemplary BCMA-expressing cancers include but are not limited to multiple myeloma (Cho et al. (2018) Front Immunol. 9:1821).

Exemplary CD33-expressing cancers include but are not limited to colorectal cancer, pancreatic cancer, lymphoma, and leukemia (e.g., acute myeloid leukemia) (Human Protein Atlas; Walter (2014) Expert Opin Ther Targets 18(7):715-8).

Exemplary PCAD-expressing cancers include but are not limited to breast cancer, gastric cancer, endometrial cancer, ovarian cancer, pancreatic cancer, bladder cancer, prostate cancer, and melanoma (Vieira and Paredes (2015) Mol Cancer 14:178).

Exemplary HER2-expressing cancers include but are not limited to breast cancer, gastric cancer, bladder cancer, urothelial cell carcinoma, esophageal cancer, lung cancer (e.g., lung adenocarcinoma), uterine cancer (e.g., uterine serous endometrial carcinoma), salivary duct carcinoma, cervical cancer, endometrial cancer, and ovarian cancer (English et al. (2013) Mol Diagn Ther. 17:85-99).

Provided herein, in certain aspects, are ADC compounds comprising an antibody or antigen-binding fragment thereof (Ab), an Mcl-1 inhibitor drug moiety (D), and a linker moiety (L) that covalently attaches Ab to D. In some embodiments, provided herein, are ADC compounds comprising an antibody or antigen-binding fragment thereof (Ab) which targets a cancer cell, an Mcl-1 inhibitor drug moiety (D), and a linker moiety (L) that covalently attaches Ab to D. In some embodiments, the antibody or antigen-binding fragment is able to bind to a tumor-associated antigen (e.g., BCMA, CD33, PCAD, or HER2), e.g., with high specificity and high affinity. In some embodiments, the antibody or antigen-binding fragment is internalized into a target cell upon binding, e.g., into a degradative compartment in the cell. In some embodiments, the ADCs internalize upon binding to a target cell, undergo degradation, and release the Mcl-1 inhibitor drug moiety to kill cancer cells. The Mcl-1 inhibitor drug moiety may be released from the antibody and/or the linker moiety of the ADC by enzymatic action, hydrolysis, oxidation, or any other mechanism.

An exemplary ADC has Formula (1):

Ab-(L-D)*ₚ* (1)

wherein Ab = an antibody or antigen-binding fragment, L = a linker moiety, D = an Mcl-1 inhibitor drug moiety, and p = the number of Mcl-1 inhibitor drug moieties per antibody or antigen-binding fragment.

### Antibodies

The antibody or antigen-binding fragment (Ab) of Formula (1) includes within its scope any antibody or antigen-binding fragment that specifically binds to a target antigen on a cell. In some embodiment, the antibody or antigen-binding fragment (Ab) of Formula (1) includes within its scope any antibody or antigen-binding fragment that specifically binds to a target antigen on a cancer cell. The antibody or antigen-binding fragment may bind to a target antigen with a dissociation constant (K_{D}) of ≤1 mM, ≤100 nM or ≤10 nM, or any amount in between, as measured by, e.g., BIAcore^{®} analysis. In some embodiments, the K_{D} is 1 pM to 500 pM. In some embodiments, the K_{D} is between 500 pM to 1 µM, 1 µM to 100 nM, or 100 mM to 10 nM.

In some embodiments, the antibody or antigen-binding fragment is a four-chain antibody (also referred to as an immunoglobulin or a full-length or intact antibody), comprising two heavy chains and two light chains. In some embodiments, the antibody or antigen-binding fragment is an antigen-binding fragment of an immunoglobulin. In some embodiments, the antibody or antigen-binding fragment is an antigen-binding fragment of an immunoglobulin that retains the ability to bind a target cancer antigen and/or provide at least one function of the immunoglobulin.

In some embodiments, the antibody or antigen-binding fragment is an internalizing antibody or internalizing antigen-binding fragment thereof. In some embodiments, the internalizing antibody or internalizing antigen-binding fragment thereof binds to a target cancer antigen expressed on the surface of a cell and enters the cell upon binding. In some embodiments, the Mcl-1 inhibitor drug moiety of the ADC is released from the antibody or antigen-binding fragment of the ADC after the ADC enters and is present in a cell expressing the target cancer antigen (i.e., after the ADC has been internalized), e.g., by cleavage, by degradation of the antibody or antigen-binding fragment, or by any other suitable release mechanism.

In some embodiments, the antibodies comprise mutations that mediate reduced or no antibody-dependent cellular cytotoxicity (ADCC) or complement-dependent cytotoxicity (CDC). In some embodiments, these mutations are known as Fc Silencing, Fc Silent, or Fc Silenced mutations. In some embodiments, amino acid residues L234 and L235 of the IgG1 constant region are substituted to A234 and A235 (also known as "LALA"). In some embodiments, amino acid residue N297 of the IgG1 constant region is substituted to A297 (also known as "N297A"). In some embodiments, amino acid residues D265 and P329 of the IgG1 constant region are substituted to A265 and A329 (also known as "DAPA"). Other antibody Fc silencing mutations may also be used. In some embodiments, the Fc silencing mutations are used in combination, for example D265A, N297A and P329A (also known as "DANAPA").

Amino acid sequences of exemplary antibodies of the present disclosure, in addition to exemplary antigen targets, are set forth in Tables 2-6.

**Table 2. Antibodies Exemplified**

| **Antibody Target** | **Antibody Code** | **mAb Reference** |
|---|---|---|
| BCMA | BCMA or Ab B | J6M0 |
| CD33 | CD33ch or Ab G | MuMy9-6ch |
| CD33 | CD33 | gemtuzumab |
| PCAD | PCAD | NOV169N31Q |
| HER2/NEU | HER2 or Ab T | trastuzumab |
| CD38 | CD38 | daratumumab |
| CD46 | CD46 or Ab C | Anti-CD46 |
| CD48 | CD48 | SGN-CD48A |
| CD79b | CD79b | polatuzumab |

**Table 3. Amino acid sequences of mAb variable regions**

| **mAb** | **IgG chain** | **SEQ ID NO** | **Amino acid sequence** |
|---|---|---|---|
| J6M0 | VH | 1 | QVQLVQSGAEVKKPGSSVKVSCKASGG TFSNYWMHWVRQAPGQGLEWMGATYRG HSDTYYNQKFKGRVTITADKSTSTAYM ELSSLRSEDTAVYYCARGAIYNGYDVL DNWGQGTLVTVSS |
| J6M0 | VL | 2 | DIQMTQSPSSLSASVGDRVTITCSASQ DISNYLNWYQQKPGKAPKLLIYYTSNL HSGVPSRFSGSGSGTDFTLTISSLQPE DFATYYCQQYRKLPWTFGQGTKLEIK |
| MuMy9-6ch | VH | 3 | QVQLQQPGAEVVKPGASVKMSCKASGY TFTSYYIHWIKQTPGQGLEWVGVIYPG NDDISYNQKFKGKATLTADKSSTTAYM QLSSLTSEDSAVYYCAREVRLRYFDVW GAGTTVTVSS |
| MuMy9-6ch | VL | 4 | NIMLTQSPSSLAVSAGEKVTMSCKSSQ SVFFSSSQKNYLAWYQQIPGQSPKLLI YWASTRESGVPDRFTGSGSGTDFTLII SSVQSEDLAIYYCHQYLSSRTFGGGTK LEIK |
| gemtuzumab | VH | 5 | |
| gemtuzumab | VL | 6 | |
| NOV169N31Q | VH | 7 | QVQLQQSGPGLVKPSQTLSLTCAISGD SVSSQSAAWNWIRQSPSRGLEWLGRIY YRSKWYNDYALSVKSRITINPDTSKNQ FSLQLNSVTPEDTAVYYCARGEGYGRE GFAIWGQGTLVTVSS |
| NOV169N31Q | VL | 8 | DIQMTQSPSSLSASVGDRVTITCRASQ TISNTLAWYQQKPGKAPKLLIYAASNL QSGVPSRFSGSGSGTDFTLTISSLQPE DFATYYCQQYLSWFTFGQGTKVEIK |
| trastuzumab | VH | 9 | |
| trastuzumab | VL | 10 | DIQMTQSPSSLSASVGDRVTITCRASQ DVNTAVAWYQQKPGKAPKLLIYSASFL YSGVPSRFSGSRSGTDFTLTISSLQPE DFATYYCQQHYTTPPTFGQGTKVEIKR T |
| daratumumab | VH | 11 | |
| daratumumab | VL | 12 | EIVLTQSPATLSLSPGERATLSCRASQ SVSSYLAWYQQKPGQAPRLLIYDASNR ATGIPARFSGSGSGTDFTLTISSLEPE DFAVYYCQQRSNWPPTFGQGTKVEI |
| SGN-48A | VH | 13 | QVQLVQSGSELKKPGASVKVSCKASGY TFTDFGMNWVRQAPGQGLEWMGWINTF TGEPSYGNVFKGRFVFSLDTSVSTAYL QISSLKAEDTAVYYCARRHGNGNVFDS WGQGTLVTVSS |
| SGN-48A | VL | 14 | EIVLTQSPDFQSVTPKEKVTITCRASQ SIGSNIHWYQQKPDQSPKLLIKYTSES ISGVPSRFSGSGSGTDFTLTINSLEAE DAATYYCQQSNSWPLTFGGGTKVEIK |
| polatuzumab | VH | 80 | |
| polatuzumab | VL | 81 | |
| Anti-CD46 | VH | 90 | QVQLVQSGGGVVQPGRSLRLACAASGL TVNNYAMHWVRQAPGKGLEWVAVISYD GNNKYYADSVKGRFTISRDNSKNTLYL Q MNSLRAEDTAVYYCAKGGGYFDLWGRG TLVTVSS |
| Anti-CD46 | VL | 91 | QSVLTQPPSVSGAPGQRVTISCTGSSS NIGAGYDVHWYQQLPGTAPKLLIYGNN NRP SGVPDRFSGSKSGTSASLAI TGLQAEDEA DYYCSSYTSGTWLFG GGTKLTVL |

**Table 4. Amino acid sequences of mAb CDRs**

| **mAb** | **IgG chain** | **SEQ ID NO** | **Amino acid sequence** |
|---|---|---|---|
| J6M0 | HCDR1 | 15 | GGTFSNYWMH |
| J6M0 | HCDR2 | 16 | ATYRGHSDTYYNQKFKG |
| J6M0 | HCDR3 | 17 | GAIYNGYDVLDN |
| J6M0 | LCDR1 | 18 | SASQDISNYLN |
| J6M0 | LCDR2 | 19 | YTSNLHS |
| J6M0 | LCDR3 | 20 | QQYRKLPWT |
| MuMy9-6ch | HCDR1 | 21 | GYTFTSYYIH |
| MuMy9-6ch | HCDR2 | 22 | VIYPGNDDISYNQKFKG |
| MuMy9-6ch | HCDR3 | 23 | EVRLRYFDV |
| MuMy9-6ch | LCDR1 | 24 | KSSQSVFFSSSQKNYLA |
| MuMy9-6ch | LCDR2 | 25 | WASTRES |
| MuMy9-6ch | LCDR3 | 26 | HQYLSSRT |
| gemtuzumab | HCDR1 | 27 | GYTITDSNIH |
| gemtuzumab | HCDR2 | 28 | YIYPYNGGTDYNQKFKN |
| gemtuzumab | HCDR3 | 29 | GNPWLAY |
| gemtuzumab | LCDR1 | 30 | RASESLDNYGIRFLT |
| gemtuzumab | LCDR2 | 31 | AASNQGS |
| gemtuzumab | LCDR3 | 32 | QQTKEVPWS |
| NOV169N31Q | HCDR1 | 33 | TCAISGDSVSSQSAAWN |
| NOV169N31Q | HCDR2 | 34 | RIYYRSKWYNDYALSVKS |
| NOV169N31Q | HCDR3 | 35 | GEGYGREGFAI |
| NOV169N31Q | LCDR1 | 36 | RASQTISNTLA |
| NOV169N31Q | LCDR2 | 37 | AASNLQS |
| NOV169N31Q | LCDR3 | 38 | QQYLSWFT |
| trastuzumab | HCDR1 | 39 | GFNIKDTYIH |
| trastuzumab | HCDR2 | 40 | RIYPTNGYTRYADSVKG |
| trastuzumab | HCDR3 | 41 | WGGDGFYAMDV |
| trastuzumab | LCDR1 | 42 | RASQDVNTAVAW |
| trastuzumab | LCDR2 | 43 | SASFLES |
| trastuzumab | LCDR3 | 44 | QQHYTTPPT |
| daratumumab | HCDR1 | 45 | GFTFNSFAMS |
| daratumumab | HCDR2 | 46 | AISGSGGGTYYADSVKG |
| daratumumab | HCDR3 | 47 | DKILWFGEPVFDY |
| daratumumab | LCDR1 | 48 | RASQSVSSYLA |
| daratumumab | LCDR2 | 49 | DASNRAT |
| daratumumab | LCDR3 | 50 | QQRSNWPPT |
| SGN-48A | HCDR1 | 51 | GYTFTDFGMN |
| SGN-48A | HCDR2 | 52 | WINTFTGEPSYGNVFKG |
| SGN-48A | HCDR3 | 53 | RHGNGNVFDS |
| SGN-48A | LCDR1 | 54 | RASQSIGSNIH |
| SGN-48A | LCDR2 | 55 | YTSESIS |
| SGN-48A | LCDR3 | 56 | QQSNSWPLT |
| polatuzumab | HCDR1 | 82 | GYTFSSYWIE |
| polatuzumab | HCDR2 | 83 | EILPGGGDTNYNEIFKG |
| polatuzumab | HCDR3 | 84 | RVPIRLDY |
| polatuzumab | LCDR1 | 85 | ITCKASQSVDYEGDSFLN |
| polatuzumab | LCDR2 | 86 | AASNLES |
| polatuzumab | LCDR3 | 87 | QQSNEDPLT |

**Table 5. Amino acid sequences of full-length mAb Ig chains**

| **mAb** | **IgG chain** | **SEQ ID NO** | **Amino acid sequence** |
|---|---|---|---|
| J6M0 | Heavy chain | 57 | QVQLVQSGAEVKKPGSSVKVSCKA SGGTFSNYWMHWVRQAPGQGLEWM GATYRGHSDTYYNQKFKGRVTITA DKSTSTAYMELSSLRSEDTAVYYC ARGAIYNGYDVLDNWGQGTLVTVS SASTKGPSVFPLAPSSKSTSGGTA ALGCLVKDYFPCPVTVSWNSGALT SGVHTFPAVLQSSGLYSLSSVVTV PSSSLGTQTYICNVNHKPSNTKVD KRVEPKSCDKTHTCPPCPAPELLG GPSVFLFPPKPKDTLMISRTPEVT CVVVDVSHEDPEVKFNWYVDGVEV HNAKTKPREEQYNSTYRVVSVLTV LHQDWLNGKEYKCKVSNKALPAPI EKTISKAKGQPREPQVYTLPPSRE EMTKNQVSLTCLVKGFYPCDIAVE WESNGQPENNYKTTPPVLDSDGSF FLYSKLTVDKSRWQQGNVFSCSVM HEALHNHYTQKSLSLSPGK |
| J6M0 | Light chain | 58 | DIQMTQSPSSLSASVGDRVTITCS ASQDISNYLNWYQQKPGKAPKLLI YYTSNLHSGVPSRFSGSGSGTDFT LTISSLQPEDFATYYCQQYRKLPW TFGQGTKLEIKRTVAAPSVFIFPP SDEQLKSGTASVVCLLNNFYPREA KVQWKVDNALQSGNSQESVTEQDS KDSTYSLSSTLTLSKADYEKHKVY ACEVTHQGLSSPVTKSFNRGEC |
| MuMy9-6ch | Heavy chain | 59 | QVQLQQPGAEVVKPGASVKMSCKA SGYTFTSYYIHWIKQTPGQGLEWV GVIYPGNDDISYNQKFKGKATLTA DKSSTTAYMQLSSLTSEDSAVYYC AREVRLRYFDVWGAGTTVTVSSAS TKGPSVFPLAPSSKSTSGGTAALG CLVKDYFPCPVTVSWNSGALTSGV HTFPAVLQSSGLYSLSSVVTVPSS SLGTQTYICNVNHKPSNTKVDKRV EPKSCDKTHTCPPCPAPELLGGPS VFLFPPKPKDTLMISRTPEVTCVV VDVSHEDPEVKFNWYVDGVEVHNA KTKPREEQYNSTYRVVSVLTVLHQ DWLNGKEYKCKVSNKALPAPIEKT ISKAKGQPREPQVYTLPPSREEMT KNQVSLTCLVKGFYPCDIAVEWES NGQPENNYKTTPPVLDSDGSFFLY SKLTVDKSRWQQGNVFSCSVMHEA LHNHYTQKSLSLSPGK |
| MuMy9-6ch | Light chain | 60 | NIMLTQSPSSLAVSAGEKVTMSCK SSQSVFFSSSQKNYLAWYQQIPGQ SPKLLIYWASTRESGVPDRFTGSG SGTDFTLIISSVQSEDLAIYYCHQ YLSSRTFGGGTKLEIKRTVAAPSV FIFPPSDEQLKSGTASVVCLLNNF YPREAKVQWKVDNALQSGNSQESV TEQDSKDSTYSLSSTLTLSKADYE KHKVYACEVTHQGLSSPVTKSFNR GEC |
| gemtuzumab | Heavy chain | 61 | EVQLVQSGAEVKKPGSSVKVSCKA SGYTITDSNIHWVRQAPGQSLEWI GYIYPYNGGTDYNQKFKNRATLTV DNPTNTAYMELSSLRSEDTAFYYC VNGNPWLAYWGQGTLVTVSSASTK GPSVFPLAPSSKSTSGGTAALGCL VKDYFPCPVTVSWNSGALTSGVHT FPAVLQSSGLYSLSSVVTVPSSSL GTQTYICNVNHKPSNTKVDKRVEP KSCDKTHTCPPCPAPELLGGPSVF LFPPKPKDTLMISRTPEVTCVVVD VSHEDPEVKFNWYVDGVEVHNAKT KPREEQYNSTYRVVSVLTVLHQDW LNGKEYKCKVSNKALPAPIEKTIS KAKGQPREPQVYTLPPSREEMTKN QVSLTCLVKGFYPCDIAVEWESNG QPENNYKTTPPVLDSDGSFFLYSK LTVDKSRWQQGNVFSCSVMHEALH NHYTQKSLSLSPGK |
| gemtuzumab | Light chain | 62 | DIQLTQSPSTLSASVGDRVTITCR ASESLDNYGIRFLTWFQQKPGKAP KLLMYAASNQGSGVPSRFSGSGSG TEFTLTISSLQPDDFATYYCQQTK EVPWSFGQGTKVEVKRTVAAPSVF IFPPSDEQLKSGTASVVCLLNNFY PREAKVQWKVDNALQSGNSQESVT EQDSKDSTYSLSSTLTLSKADYEK HKVYACEVTHQGLSSPVTKSFNRG EC |
| NOV169N31Q | Heavy chain | 63 | QVQLQQSGPGLVKPSQTLSLTCAI SGDSVSSQSAAWNWIRQSPSRGLE WLGRIYYRSKWYNDYALSVKSRIT INPDTSKNQFSLQLNSVTPEDTAV YYCARGEGYGREGFAIWGQGTLVT VSSASTKGPSVFPLAPSSKSTSGG TAALGCLVKDYFPCPVTVSWNSGA LTSGVHTFPAVLQSSGLYSLSSVV TVPSSSLGTQTYICNVNHKPSNTK VDKRVEPKSCDKTHTCPPCPAPEL LGGPSVFLFPPKPKDTLMISRTPE VTCVVVDVSHEDPEVKFNWYVDGV EVHNAKTKPREEQYNSTYRVVSVL TVLHQDWLNGKEYKCKVSNKALPA PIEKTISKAKGQPREPQVYTLPPS REEMTKNQVSLTCLVKGFYPCDIA VEWESNGQPENNYKTTPPVLDSDG SFFLYSKLTVDKSRWQQGNVFSCS VMHEALHNHYTQKSLSLSPGK |
| NOV169N31Q | Light chain | 64 | DIQMTQSPSSLSASVGDRVTITCR ASQTISNTLAWYQQKPGKAPKLLI YAASNLQSGVPSRFSGSGSGTDFT LTISSLQPEDFATYYCQQYLSWFT FGQGTKVEIKRTVAAPSVFIFPPS DEQLKSGTASVVCLLNNFYPREAK VQWKVDNALQSGNSQESVTEQDSK DSTYSLSSTLTLSKADYEKHKVYA CEVTHQGLSSPVTKSFNRGEC |
| trastuzumab | Heavy chain | 65 | EVQLVESGGGLVQPGGSLRLSCAA SGFNIKDTYIHWVRQAPGKGLEWV ARIYPTNGYTRYADSVKGRFTISA DTSKNTAYLQMNSLRAEDTAVYYC SRWGGDGFYAMDYWGQGTLVTVSS ASTKGPSVFPLAPSSKSTSGGTAA LGCLVKDYFPEPVTVSWNSGALTS GVHTFPAVLQSSGLYSLSSVVTVP SSSLGTQTYICNVNHKPSNTKVDK KVEPPKSCDKTHTCPPCPAPELLG GPSVFLFPPKPKDTLMISRTPEVT CVVVDVSHEDPEVKFNWYVDGVEV HNAKTKPREEQYNSTYRVVSVLTV LHQDWLNGKEYKCKVSNKALPAPI EKTISKAKGQPREPQVYTLPPSRD ELTKNQVSLTCLVKGFYPSDIAVE WESNGQPENNYKTTPPVLDSDGSF FLYSKLTVDKSRWQQGNVFSCSVM HEALHNHYTQKSLSLSPGK |
| trastuzumab | Light chain | 66 | DIQMTQSPSSLSASVGDRVTITCR ASQDVNTAVAWYQQKPGKAPKLLI YSASFLYSGVPSRFSGSRSGTDFT LTISSLQPEDFATYYCQQHYTTPP TFGQGTKVEIKRTVAAPSVFIFPP SDEQLKSGTASVVCLLNNFYPREA KVQWKVDNALQSGNSQESVTEQDS KDSTYSLSSTLTLSKADYEKHKVY ACEVTHQGLSSPVTKSFNRGEC |
| daratumumab | Heavy chain | 67 | EVQLLESGGGLVQPGGSLRLSCAV SGFTFNSFAMSWVRQAPGKGLEWV SAISGSGGGTYYADSVKGRFTISR DNSKNTLYLQMNSLRAEDTAVYFC AKDKILWFGEPVFDYWGQGTLVTV SSASTKGPSVFPLAPSSKSTSGGT AALGCLVKDYFPCPVTVSWNSGAL TSGVHTFPAVLQSSGLYSLSSVVT VPSSSLGTQTYICNVNHKPSNTKV DKRVEPKSCDKTHTCPPCPAPELL GGPSVFLFPPKPKDTLMISRTPEV TCVVVD VSHEDPEVKFNWYVDGVEVHNAKT KPREEQYNSTYRVVSVLTVLHQDW LNGKEYKCKVSNKALPAPIEKTIS KAKGQPREPQVYTLPPSREEMTKN QVSLTCLVKGFYPCDIAVEWESNG QPENNYKTTPPVLDSDGSFFLYSK LTVDKSRWQQGNVFSCSVMHEALH NHYTQKSLSLSPGK |
| daratumumab | Light chain | 68 | EIVLTQSPATLSLSPGERATLSCR ASQSVSSYLAWYQQKPGQAPRLLI YDASNRATGIPARFSGSGSGTDFT LTISSLEPEDFAVYYCQQRSNWPP TFGQGTKVEIKRTVAAPSVFIFPP SDEQLKSGTASVVCLLNNFYPREA KVQWKVDNALQSGNSQESVTEQDS KDSTYSLSSTLTLSKADYEKHKVY ACEVTHQGLSSPVTKSFNRGEC |
| SGN-48A | Heavy chain | 69 | QVQLVQSGSELKKPGASVKVSCKA SGYTFTDFGMNWVRQAPGQGLEWM GWINTFTGEPSYGNVFKGRFVFSL DTSVSTAYLQISSLKAEDTAVYYC ARRHGNGNVFDSWGQGTLVTVSSA STKGPSVFPLAPSSKSTSGGTAAL GCLVKDYFPCPVTVSWNSGALTSG VHTFPAVLQSSGLYSLSSVVTVPS SSLGTQTYICNVNHKPSNTKVDKR VEPKSCDKTHTCPPCPAPELLGGP SVFLFPPKPKDTLMISRTPEVTCV VVDVSHEDPEVKFNWYVDGVEVHN AKTKPREEQYNSTYRVVSVLTVLH QDWLNGKEYKCKVSNKALPAPIEK TISKAKGQPREPQVYTLPPSREEM TKNQVSLTCLVKGFYPCDIAVEWE SNGQPENNYKTTPPVLDSDGSFFL YSKLTVDKSRWQQGNVFSCSVMHE ALHNHYTQKSLSLSPGK |
| SGN-48A | Light chain | 70 | EIVLTQSPDFQSVTPKEKVTITCR ASQSIGSNIHWYQQKPDQSPKLLI KYTSESISGVPSRFSGSGSGTDFT LTINSLEAEDAATYYCQQSNSWPL TFGGGTKVEIKRTVAAPSVFIFPP SDEQLKSGTASVVCLLNNFYPREA KVQWKVDNALQSGNSQESVTEQDS KDSTYSLSSTLTLSKADYEKHKVY ACEVTHQGLSSPVTKSFNRGEC |
| Polatuzimab | Heavy Chain | 88 | EVQLVESGGGLVQPGGSLRLSCAA SGYTFSSYWIEWVRQAPGKGLEWI GEILPGGGDTNYNEIFKGRATFSA DTSKNTAYLQMNSLRAEDTAVYYC TRRVPIRLDYWGQGTLVTVSSAST KGPSVFPLAPSSKSTSGGTAALGC LVKDYFPCPVTVSWNSGALTSGVH TFPAVLQSSGLYSLSSVVTVPSSS LGTQTYICNVNHKPSNTKVDKRVE PKSCDKTHTCPPCPAPELLGGPSV FLFPPKPKDTLMISRTPEVTCVVV DVSHEDPEVKFNWYVDGVEVHNAK TKPREEQYNSTYRVVSVLTVLHQD WLNGKEYKCKVSNKALPAPIEKTI SKAKGQPREPQVYTLPPSREEMTK NQVSLTCLVKGFYPCDIAVEWESN GQPENNYKTTPPVLDSDGSFFLYS KLTVDKSRWQQGNVFSCSVMHEAL HNHYTQKSLSLSPGK |
| Polatuzimab | Light Chain | 89 | DIQLTQSPSSLSASVGDRVTITCK ASQSVDYEGDSFLNWYQQKPGKAP KLLIYAASNLESGVPSRFSGSGSG TDFTLTISSLQPEDFATYYCQQSN EDPLTFGQGTKVEIKRTVAAPSVF IFPPSDEQLKSGTASVVCLLNNFY PREAKVQWKVDNALQSGNSQESVT EQDSKDSTYSLSSTLTLSKADYEK HKVYACEVTHQGLSSPVTKSFNRG EC |

**Table 6. Exemplary Mcl-1 and target antigen amino acid sequences**

| **Mcl-1/Antigen** | **SEQ ID NO** | **Amino acid sequence** |
|---|---|---|
| Mcl-1 | 71 | MFGLKRNAVIGLNLYCGGAGLGAGSGGATRPGGRLLATEKEASAR REIGGGEAGAVIGGSAGASPPSTLTPDSRRVARPPPIGAEVPDVT ATPARLLFFAPTRRAAPLEEMEAPAADAIMSPEEELDGYEPEPLG KRPAVLPLLELVGESGNNTSTDGSLPSTPPPAEEEEDELYRQSLE IISRYLREQATGAKDTKPMGRSGATSRKALETLRRVGDGVQRNHE TAFQGMLRKLDIKNEDDVKSLSRVMIHVFSDGVTNWGRIVTLISF GAFVAKHLKTINQESCIEPLAESITDVLVRTKRDWLVKQRGWDGF VEFFHVEDLEGGIRNVLLAFAGVAGVGAGLAYLIR |
| BCMA | 72 | MLQMAGQCSQNEYFDSLLHACIPCQLRCSSNTPPLTCQRYCNASV TNSVKGTNAILWTCLGLSLIISLAVFVLMFLLRKINSEPLKDEFK NTGSGLLGMANIDLEKSRTGDEIILPRGLEYTVEECTCEDCIKSK PKVDSDHCFPLPAMEEGATILVTTKTNDYCKSLPAALSATEIEKS ISAR |
| CD33 | 73 | MPLLLLLPLLWAGALAMDPNFWLQVQESVTVQEGLCVLVPCTFFH PIPYYDKNSPVHGYWFREGAIISRDSPVATNKLDQEVQEETQGRF RLLGDPSRNNCSLSIVDARRRDNGSYFFRMERGSTKYSYKSPQLS VHVTDLTHRPKILIPGTLEPGHSKNLTCSVSWACEQGTPPIFSWL SAAPTSLGPRTTHSSVLIITPRPQDHGTNLTCQVKFAGAGVTTER TIQLNVTYVPQNPTTGIFPGDGSGKQETRAGVVHGAIGGAGVTAL LALCLCLIFFIVKTHRRKAARTAVGRNDTHPTTGSASPKHQKKSK LHGPTETSSCSGAAPTVEMDEELHYASLNFHGMNPSKDTSTEYSE VRTQ |
| PCAD | 74 | |
| HER2/NEU | 75 | |
| CD38 | 76 | MANCEFSPVSGDKPCCRLSRRAQLCLGVSILVLILVVVLAVVVPR WRQQWSGPGTTKRFPETVLARCVKYTEIHPEMRHVDCQSVWDAFK GAFISKHPCNITEEDYQPLMKLGTQTVPCNKILLWSRIKDLAHQF TQVQRDMFTLEDTLLGYLADDLTWCGEFNTSKINYQSCPDWRKDC SNNPVSVFWKTVSRRFAEAACDVVHVMLNGSRSKIFDKNSTFGSV EVHNLQPEKVQTLEAWVIHGGREDSRDLCQDPTIKELESIISKRN IQFSCKNIYRPDKFLQCVKNPEDSSCTSEI |
| CD48 | 77 | MCSRGWDSCLALELLLLPLSLLVTSIQGHLVHMTVVSGSNVTLNI SESLPENYKQLTWFYTFDQKIVEWDSRKSKYFESKFKGRVRLDPQ SGALYISKVQKEDNSTYIMRVLKKTGNEQEWKIKLQVLDPVPKPV IKIEKIEDMDDNCYLKLSCVIPGESVNYTWYGDKRPFPKELQNSV LETTLMPHNYSRCYTCQVSNSVSSKNGTVCLSPPCTLARSFGVEW IASWLVVTVPTILGLLLT |
| CD79b | 78 | MARLALSPVPSHWMVALLLLLSAEPVPAARSEDRYRNPKGSACSR IWQSPRFIARKRGFTVKMHCYMNSASGNVSWLWKQEMDENPQQLK LEKGRMEESQNESLATLTIQGIRFEDNGIYFCQQKCNNTSEVYQG CGTELRVMGFSTLAQLKQRNTLKDGIIMIQTLLIILFIIVPIFLL LDKDDSKAGMEEDHTYEGLDIDQTATYEDIVTLRTGEVKWSVGEH PGQE |

In some embodiments, the antibody or antigen-binding fragment of an ADC disclosed herein may comprise any set of heavy and light chain variable domains listed in the tables above or a set of six CDRs from any set of heavy and light chain variable domains listed in the tables above. In some embodiments, the antibody or antigen-binding fragment of an ADC disclosed herein may comprise amino acid sequences that are conservatively modified and/or homologous to the sequences listed in the tables above, so long as the ADC retains the ability to bind to its target cancer antigen (e.g., with a K_{D} of less than 1x10⁻⁸ M) and retains one or more functional properties of the ADCs disclosed herein (e.g., ability to internalize, bind to an antigen target, e.g., an antigen expressed on a tumor or other cancer cell, etc.).

In some embodiments, the antibody or antigen-binding fragment of an ADC disclosed herein further comprises human heavy and light chain constant domains or fragments thereof. For instance, the antibody or antigen-binding fragment of the described ADCs may comprise a human IgG heavy chain constant domain (such as an IgG1) and a human kappa or lambda light chain constant domain. In some embodiments, the antibody or antigen-binding fragment of the described ADCs comprises a human immunoglobulin G subtype 1 (IgG1) heavy chain constant domain with a human Ig kappa light chain constant domain.

In some embodiments, the target cancer antigen for an ADC is BCMA.

In some embodiments, the anti-BCMA antibody or antigen-binding fragment thereof comprises three heavy chain CDRs and three light chain CDRs as follows: heavy chain CDR1 (HCDR1) consisting of SEQ ID NO:15, heavy chain CDR2 (HCDR2) consisting of SEQ ID NO:16, heavy chain CDR3 (HCDR3) consisting of SEQ ID NO:17; light chain CDR1 (LCDR1) consisting of SEQ ID NO:18, light chain CDR2 (LCDR2) consisting of SEQ ID NO:19, and light chain CDR3 (LCDR3) consisting of SEQ ID NO:20.

In some embodiments, the anti-BCMA antibody or antigen-binding fragment thereof comprises a heavy chain variable region comprising the amino acid sequence of SEQ ID NO:1, and a light chain variable region comprising the amino acid sequence of SEQ ID NO:2. In some embodiments, the anti-BCMA antibody or antigen-binding fragment thereof comprises the heavy chain variable region amino acid sequence of SEQ ID NO:1 and the light chain variable region amino acid sequence of SEQ ID NO:2, or sequences that are at least 95% identical to the disclosed sequences. In some embodiments, the anti-BCMA antibody or antigen-binding fragment thereof has a heavy chain variable region amino acid sequence that is at least 96%, at least 97%, at least 98%, or at least 99% identical to SEQ ID NO:1 and/or a light chain variable region amino acid sequence that is at least 96%, at least 97%, at least 98%, or at least 99% identical to SEQ ID NO:2.

In some embodiments, the anti-BCMA antibody or antigen-binding fragment thereof is an internalizing antibody or internalizing antigen-binding fragment. In some embodiments, the anti-BCMA antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system. In some embodiments, the IgG1 heavy chain constant domain comprises a cysteine residue (C) at the amino acid positions corresponding to 156 and 379 in a wild-type (unmodified) IgG1 heavy chain constant domain. In some embodiments, the anti-BCMA antibody comprises a human Ig kappa light chain constant domain or a modified Ig kappa light chain constant domain.

In some embodiments, the anti-BCMA antibody comprises the heavy chain amino acid sequence of SEQ ID NO:57 or a sequence that is at least 95% identical to SEQ ID NO:57, and the light chain amino acid sequence of SEQ ID NO:58 or a sequence that is at least 95% identical to SEQ ID NO:58. In some embodiments, the anti-BCMA antibody comprises the heavy chain amino acid sequence of SEQ ID NO:57 and the light chain amino acid sequence of SEQ ID NO:58, or sequences that are at least 95% identical to the disclosed sequences. In some embodiments, the anti-BCMA antibody has a heavy chain amino acid sequence that is at least 96%, at least 97%, at least 98%, or at least 99% identical to SEQ ID NO:57 and a light chain amino acid sequence that is at least 96%, at least 97%, at least 98%, or at least 99% identical to SEQ ID NO:58. In some embodiments, the anti-BCMA antibody is J6M0 (WO 2012/163805), or an antigen-binding fragment thereof.

In some embodiments, the anti-BCMA antibody or antigen-binding fragment thereof comprises the three heavy chain CDRs and three light chain CDRs of J6M0 or wherein the CDRs include no more than one, two, three, four, five, or six amino acid additions, deletions or substitutions of HCDR1 (SEQ ID NO:15), HCDR2 (SEQ ID NO:16), HCDR3 (SEQ ID NO:17); LCDR1 (SEQ ID NO:18), LCDR2 (SEQ ID NO:19), and LCDR3 (SEQ ID NO:20).

In some embodiments, the target cancer antigen for an ADC is CD33.

In some embodiments, the anti-CD33 antibody or antigen-binding fragment thereof comprises three heavy chain CDRs and three light chain CDRs as follows: heavy chain CDR1 (HCDR1) consisting of SEQ ID NO:21, heavy chain CDR2 (HCDR2) consisting of SEQ ID NO:22, heavy chain CDR3 (HCDR3) consisting of SEQ ID NO:23; light chain CDR1 (LCDR1) consisting of SEQ ID NO:24, light chain CDR2 (LCDR2) consisting of SEQ ID NO:25, and light chain CDR3 (LCDR3) consisting of SEQ ID NO:26.

In some embodiments, the anti-CD33 antibody or antigen-binding fragment thereof comprises a heavy chain variable region comprising the amino acid sequence of SEQ ID NO:3, and a light chain variable region comprising the amino acid sequence of SEQ ID NO:4. In some embodiments, the anti-CD33 antibody or antigen-binding fragment thereof comprises the heavy chain variable region amino acid sequence of SEQ ID NO:3 and the light chain variable region amino acid sequence of SEQ ID NO:4, or sequences that are at least 95% identical to the disclosed sequences. In some embodiments, the anti-CD33 antibody or antigen-binding fragment thereof has a heavy chain variable region amino acid sequence that is at least 96%, at least 97%, at least 98%, or at least 99% identical to SEQ ID NO:3 and/or a light chain variable region amino acid sequence that is at least 96%, at least 97%, at least 98%, or at least 99% identical to SEQ ID NO:4.

In some embodiments, the anti-CD33 antibody or antigen-binding fragment thereof is an internalizing antibody or internalizing antigen-binding fragment. In some embodiments, the anti-CD33 antibody comprises a human IgG1 heavy chain constant domain or a modified IgG1 heavy chain constant domain. In some embodiments, the IgG1 heavy chain constant domain comprises a glutamine residue (Q) at the amino acid position corresponding to 297 in a wild-type (unmodified) IgG1 heavy chain constant domain. In some embodiments, the anti-CD33 antibody comprises a human Ig kappa light chain constant domain or a modified Ig kappa light chain constant domain.

In some embodiments, the anti-CD33 antibody comprises the heavy chain amino acid sequence of SEQ ID NO:59 or a sequence that is at least 95% identical to SEQ ID NO:59, and the light chain amino acid sequence of SEQ ID NO:60 or a sequence that is at least 95% identical to SEQ ID NO:60. In some embodiments, the anti-CD33 antibody comprises the heavy chain amino acid sequence of SEQ ID NO:59 and the light chain amino acid sequence of SEQ ID NO:60, or sequences that are at least 95% identical to the disclosed sequences. In some embodiments, the anti-CD33 antibody has a heavy chain amino acid sequence that is at least 96%, at least 97%, at least 98%, or at least 99% identical to SEQ ID NO:59 and a light chain amino acid sequence that is at least 96%, at least 97%, at least 98%, or at least 99% identical to SEQ ID NO:60. In some embodiments, the anti-CD33 antibody is MuMy9-6ch (US 2013/0078241), or an antigen-binding fragment thereof.

In some embodiments, the anti-CD33 antibody or antigen-binding fragment thereof comprises the three heavy chain CDRs and three light chain CDRs of MuMy9-6ch or wherein the CDRs include no more than one, two, three, four, five, or six amino acid additions, deletions or substitutions of HCDR1 (SEQ ID NO:21), HCDR2 (SEQ ID NO:22), HCDR3 (SEQ ID NO:23); LCDR1 (SEQ ID NO:24), LCDR2 (SEQ ID NO:25), and LCDR3 (SEQ ID NO:26).

In some embodiments, the anti-CD33 antibody or antigen-binding fragment thereof comprises three heavy chain CDRs and three light chain CDRs as follows: heavy chain CDR1 (HCDR1) consisting of SEQ ID NO:27, heavy chain CDR2 (HCDR2) consisting of SEQ ID NO:28, heavy chain CDR3 (HCDR3) consisting of SEQ ID NO:29; light chain CDR1 (LCDR1) consisting of SEQ ID NO:30, light chain CDR2 (LCDR2) consisting of SEQ ID NO:31, and light chain CDR3 (LCDR3) consisting of SEQ ID NO:32.

In some embodiments, the anti-CD33 antibody or antigen-binding fragment thereof comprises a heavy chain variable region comprising the amino acid sequence of SEQ ID NO:5, and a light chain variable region comprising the amino acid sequence of SEQ ID NO:6. In some embodiments, the anti-CD33 antibody or antigen-binding fragment thereof comprises the heavy chain variable region amino acid sequence of SEQ ID NO:5 and the light chain variable region amino acid sequence of SEQ ID NO:6, or sequences that are at least 95% identical to the disclosed sequences. In some embodiments, the anti-CD33 antibody or antigen-binding fragment thereof has a heavy chain variable region amino acid sequence that is at least 96%, at least 97%, at least 98%, or at least 99% identical to SEQ ID NO:5 and/or a light chain variable region amino acid sequence that is at least 96%, at least 97%, at least 98%, or at least 99% identical to SEQ ID NO:6.

In some embodiments, the anti-CD33 antibody or antigen-binding fragment thereof is an internalizing antibody or internalizing antigen-binding fragment. In some embodiments, the anti-CD33 antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system.

In some embodiments, the anti-CD33 antibody comprises the heavy chain amino acid sequence of SEQ ID NO:61 or a sequence that is at least 95% identical to SEQ ID NO:61, and the light chain amino acid sequence of SEQ ID NO:62 or a sequence that is at least 95% identical to SEQ ID NO:62. In some embodiments, the anti-CD33 antibody comprises the heavy chain amino acid sequence of SEQ ID NO:61 and the light chain amino acid sequence of SEQ ID NO:62, or sequences that are at least 95% identical to the disclosed sequences. In some embodiments, the anti-CD33 antibody has a heavy chain amino acid sequence that is at least 96%, at least 97%, at least 98%, or at least 99% identical to SEQ ID NO:61 and a light chain amino acid sequence that is at least 96%, at least 97%, at least 98%, or at least 99% identical to SEQ ID NO:62. In some embodiments, the anti-CD33 antibody is gemtuzumab, or an antigen-binding fragment thereof.

In some embodiments, the anti-CD33 antibody or antigen-binding fragment thereof comprises the three heavy chain CDRs and three light chain CDRs of gemtuzumab or wherein the CDRs include no more than one, two, three, four, five, or six amino acid additions, deletions or substitutions of HCDR1 (SEQ ID NO:27), HCDR2 (SEQ ID NO:28), HCDR3 (SEQ ID NO:29); LCDR1 (SEQ ID NO:30), LCDR2 (SEQ ID NO:31), and LCDR3 (SEQ ID NO:32).

In some embodiments, the target cancer antigen for an ADC is PCAD.

In some embodiments, the anti-PCAD antibody or antigen-binding fragment thereof comprises three heavy chain CDRs and three light chain CDRs as follows: heavy chain CDR1 (HCDR1) consisting of SEQ ID NO:33, heavy chain CDR2 (HCDR2) consisting of SEQ ID NO:34, heavy chain CDR3 (HCDR3) consisting of SEQ ID NO:35; light chain CDR1 (LCDR1) consisting of SEQ ID NO:36, light chain CDR2 (LCDR2) consisting of SEQ ID NO:37, and light chain CDR3 (LCDR3) consisting of SEQ ID NO:38.

In some embodiments, the anti-PCAD antibody or antigen-binding fragment thereof comprises a heavy chain variable region comprising the amino acid sequence of SEQ ID NO:7, and a light chain variable region comprising the amino acid sequence of SEQ ID NO:8. In some embodiments, the anti-PCAD antibody or antigen-binding fragment thereof comprises the heavy chain variable region amino acid sequence of SEQ ID NO:7 and the light chain variable region amino acid sequence of SEQ ID NO:8, or sequences that are at least 95% identical to the disclosed sequences. In some embodiments, the anti-PCAD antibody or antigen-binding fragment thereof has a heavy chain variable region amino acid sequence that is at least 96%, at least 97%, at least 98%, or at least 99% identical to SEQ ID NO:7 and/or a light chain variable region amino acid sequence that is at least 96%, at least 97%, at least 98%, or at least 99% identical to SEQ ID NO:8.

In some embodiments, the anti-PCAD antibody or antigen-binding fragment thereof is an internalizing antibody or internalizing antigen-binding fragment. In some embodiments, the anti-PCAD antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system.

In some embodiments, the anti-PCAD antibody comprises the heavy chain amino acid sequence of SEQ ID NO:63 or a sequence that is at least 95% identical to SEQ ID NO:63, and the light chain amino acid sequence of SEQ ID NO:64 or a sequence that is at least 95% identical to SEQ ID NO:64. In some embodiments, the anti-PCAD antibody comprises the heavy chain amino acid sequence of SEQ ID NO:63 and the light chain amino acid sequence of SEQ ID NO:64, or sequences that are at least 95% identical to the disclosed sequences. In some embodiments, the anti-PCAD antibody has a heavy chain amino acid sequence that is at least 96%, at least 97%, at least 98%, or at least 99% identical to SEQ ID NO:63 and a light chain amino acid sequence that is at least 96%, at least 97%, at least 98%, or at least 99% identical to SEQ ID NO:64. In some embodiments, the anti-PCAD antibody is NOV169N31Q (WO 2016/203432), or an antigen-binding fragment thereof.

In some embodiments, the anti-PCAD antibody or antigen-binding fragment thereof comprises the three heavy chain CDRs and three light chain CDRs of NOV169N31Q or wherein the CDRs include no more than one, two, three, four, five, or six amino acid additions, deletions or substitutions of HCDR1 (SEQ ID NO:33), HCDR2 (SEQ ID NO:34), HCDR3 (SEQ ID NO:35); LCDR1 (SEQ ID NO:36), LCDR2 (SEQ ID NO:37), and LCDR3 (SEQ ID NO:38).

In some embodiments, the target cancer antigen for an ADC is HER2.

In some embodiments, the anti-HER2 antibody or antigen-binding fragment thereof comprises three heavy chain CDRs and three light chain CDRs as follows: heavy chain CDR1 (HCDR1) consisting of SEQ ID NO:39, heavy chain CDR2 (HCDR2) consisting of SEQ ID NO:40, heavy chain CDR3 (HCDR3) consisting of SEQ ID NO:41; light chain CDR1 (LCDR1) consisting of SEQ ID NO:42, light chain CDR2 (LCDR2) consisting of SEQ ID NO:43, and light chain CDR3 (LCDR3) consisting of SEQ ID NO:44.

In some embodiments, the anti-HER2 antibody or antigen-binding fragment thereof comprises a heavy chain variable region comprising the amino acid sequence of SEQ ID NO:9, and a light chain variable region comprising the amino acid sequence of SEQ ID NO:10. In some embodiments, the anti-HER2 antibody or antigen-binding fragment thereof comprises the heavy chain variable region amino acid sequence of SEQ ID NO:9 and the light chain variable region amino acid sequence of SEQ ID NO:10, or sequences that are at least 95% identical to the disclosed sequences. In some embodiments, the anti-HER2 antibody or antigen-binding fragment thereof has a heavy chain variable region amino acid sequence that is at least 96%, at least 97%, at least 98%, or at least 99% identical to SEQ ID NO:9 and/or a light chain variable region amino acid sequence that is at least 96%, at least 97%, at least 98%, or at least 99% identical to SEQ ID NO:10.

In some embodiments, the anti-HER2 antibody or antigen-binding fragment thereof is an internalizing antibody or internalizing antigen-binding fragment. In some embodiments, the anti-HER2 antibody comprises a human IgG1 heavy chain constant domain or a modified IgG1 heavy chain constant domain. In some embodiments, the IgG1 heavy chain constant domain comprises a glutamine residue (Q) at the amino acid position corresponding to 297 in a wild-type (unmodified) IgG1 heavy chain constant domain. In some embodiments, the IgG1 heavy chain constant domain comprises a serine residue (S) at the amino acid position corresponding to 297 in a wild-type (unmodified) IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system. In some embodiments, the anti-HER2 antibody comprises a human Ig kappa light chain constant domain or a modified Ig kappa light chain constant domain.

In some embodiments, the anti-HER2 antibody comprises the heavy chain amino acid sequence of SEQ ID NO:65 or a sequence that is at least 95% identical to SEQ ID NO:65, and the light chain amino acid sequence of SEQ ID NO:66 or a sequence that is at least 95% identical to SEQ ID NO:66. In some embodiments, the anti-HER2 antibody comprises the heavy chain amino acid sequence of SEQ ID NO:65 and the light chain amino acid sequence of SEQ ID NO:66, or sequences that are at least 95% identical to the disclosed sequences. In some embodiments, the anti-HER2 antibody has a heavy chain amino acid sequence that is at least 96%, at least 97%, at least 98%, or at least 99% identical to SEQ ID NO:65 and a light chain amino acid sequence that is at least 96%, at least 97%, at least 98%, or at least 99% identical to SEQ ID NO:66. In some embodiments, the anti-HER2 antibody is trastuzumab (US Patent Nos. 5,821,337 and 6,870,034; see also Molina et al. (2001) Cancer Res. 61(12):4744-9), or an antigen-binding fragment thereof.

In some embodiments, the anti-HER2 antibody or antigen-binding fragment thereof comprises the three heavy chain CDRs and three light chain CDRs of trastuzumab or wherein the CDRs include no more than one, two, three, four, five, or six amino acid additions, deletions or substitutions of HCDR1 (SEQ ID NO:39), HCDR2 (SEQ ID NO:40), HCDR3 (SEQ ID NO:41); LCDR1 (SEQ ID NO:42), LCDR2 (SEQ ID NO:43), and LCDR3 (SEQ ID NO:44).

In some embodiments, the target cancer antigen for an ADC is CD38.

In some embodiments, the anti-CD38 antibody or antigen-binding fragment thereof comprises three heavy chain CDRs and three light chain CDRs as follows: heavy chain CDR1 (HCDR1) consisting of SEQ ID NO:45, heavy chain CDR2 (HCDR2) consisting of SEQ ID NO:46, heavy chain CDR3 (HCDR3) consisting of SEQ ID NO:47; light chain CDR1 (LCDR1) consisting of SEQ ID NO:48, light chain CDR2 (LCDR2) consisting of SEQ ID NO:49, and light chain CDR3 (LCDR3) consisting of SEQ ID NO:50.

In some embodiments, the anti-CD38 antibody or antigen-binding fragment thereof comprises a heavy chain variable region comprising the amino acid sequence of SEQ ID NO:11, and a light chain variable region comprising the amino acid sequence of SEQ ID NO:12. In some embodiments, the anti-CD38 antibody or antigen-binding fragment thereof comprises the heavy chain variable region amino acid sequence of SEQ ID NO:11 and the light chain variable region amino acid sequence of SEQ ID NO:12, or sequences that are at least 95% identical to the disclosed sequences. In some embodiments, the anti-CD38 antibody or antigen-binding fragment thereof has a heavy chain variable region amino acid sequence that is at least 96%, at least 97%, at least 98%, or at least 99% identical to SEQ ID NO:11 and/or a light chain variable region amino acid sequence that is at least 96%, at least 97%, at least 98%, or at least 99% identical to SEQ ID NO:12.

In some embodiments, the anti-CD38 antibody or antigen-binding fragment thereof is an internalizing antibody or internalizing antigen-binding fragment. In some embodiments, the anti-CD38 antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system.

In some embodiments, the anti-CD38 antibody comprises the heavy chain amino acid sequence of SEQ ID NO:67 or a sequence that is at least 95% identical to SEQ ID NO:67, and the light chain amino acid sequence of SEQ ID NO:68 or a sequence that is at least 95% identical to SEQ ID NO:68. In some embodiments, the anti-CD33 antibody comprises the heavy chain amino acid sequence of SEQ ID NO:67 and the light chain amino acid sequence of SEQ ID NO:68, or sequences that are at least 95% identical to the disclosed sequences. In some embodiments, the anti-CD38 antibody has a heavy chain amino acid sequence that is at least 96%, at least 97%, at least 98%, or at least 99% identical to SEQ ID NO:67 and a light chain amino acid sequence that is at least 96%, at least 97%, at least 98%, or at least 99% identical to SEQ ID NO:68. In some embodiments, the anti-CD38 antibody is daratumumab, or an antigen-binding fragment thereof.

In some embodiments, the anti-CD38 antibody or antigen-binding fragment thereof comprises the three heavy chain CDRs and three light chain CDRs of gemtuzumab or wherein the CDRs include no more than one, two, three, four, five, or six amino acid additions, deletions or substitutions of HCDR1 (SEQ ID NO:45), HCDR2 (SEQ ID NO:46), HCDR3 (SEQ ID NO:47); LCDR1 (SEQ ID NO:48), LCDR2 (SEQ ID NO:49), and LCDR3 (SEQ ID NO:50).

In some embodiment, the target cancer antigen for an ADC is CD46.

In some embodiments, the anti-CD46 antibody or antigen-binding fragment are those described in WO2018/089807. In some embodiments, the anti-CD46 antibody or antigen-binding fragment thereof comprises a heavy chain variable region comprising the amino acid sequence of SEQ ID NO:90, and a light chain variable region comprising the amino acid sequence of SEQ ID NO:91. In some embodiments, the anti-CD46 antibody or antigen-binding fragment thereof comprises the heavy chain variable region amino acid sequence of SEQ ID NO:90 and the light chain variable region amino acid sequence of SEQ ID NO:91, or sequences that are at least 95% identical to the disclosed sequences. In some embodiments, the anti-CD46 antibody or antigen-binding fragment thereof has a heavy chain variable region amino acid sequence that is at least 96%, at least 97%, at least 98%, or at least 99% identical to SEQ ID NO:90 and/or a light chain variable region amino acid sequence that is at least 96%, at least 97%, at least 98%, or at least 99% identical to SEQ ID NO:91.

In some embodiments, the target cancer antigen for an ADC is CD48.

In some embodiments, the anti-CD48 antibody or antigen-binding fragment thereof comprises three heavy chain CDRs and three light chain CDRs as follows: heavy chain CDR1 (HCDR1) consisting of SEQ ID NO:51, heavy chain CDR2 (HCDR2) consisting of SEQ ID NO:52, heavy chain CDR3 (HCDR3) consisting of SEQ ID NO:53; light chain CDR1 (LCDR1) consisting of SEQ ID NO:54, light chain CDR2 (LCDR2) consisting of SEQ ID NO:55, and light chain CDR3 (LCDR3) consisting of SEQ ID NO:56.

In some embodiments, the anti-CD48 antibody or antigen-binding fragment thereof comprises a heavy chain variable region comprising the amino acid sequence of SEQ ID NO:13, and a light chain variable region comprising the amino acid sequence of SEQ ID NO:14. In some embodiments, the anti-CD48 antibody or antigen-binding fragment thereof comprises the heavy chain variable region amino acid sequence of SEQ ID NO:13 and the light chain variable region amino acid sequence of SEQ ID NO:14, or sequences that are at least 95% identical to the disclosed sequences. In some embodiments, the anti-CD48 antibody or antigen-binding fragment thereof has a heavy chain variable region amino acid sequence that is at least 96%, at least 97%, at least 98%, or at least 99% identical to SEQ ID NO:13 and/or a light chain variable region amino acid sequence that is at least 96%, at least 97%, at least 98%, or at least 99% identical to SEQ ID NO:14.

In some embodiments, the anti-CD48 antibody or antigen-binding fragment thereof is an internalizing antibody or internalizing antigen-binding fragment. In some embodiments, the anti-CD48 antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system.

In some embodiments, the anti-CD48 antibody comprises the heavy chain amino acid sequence of SEQ ID NO:69 or a sequence that is at least 95% identical to SEQ ID NO:69, and the light chain amino acid sequence of SEQ ID NO:70 or a sequence that is at least 95% identical to SEQ ID NO:70. In some embodiments, the anti-CD48 antibody comprises the heavy chain amino acid sequence of SEQ ID NO:69 and the light chain amino acid sequence of SEQ ID NO:70, or sequences that are at least 95% identical to the disclosed sequences. In some embodiments, the anti-CD48 antibody has a heavy chain amino acid sequence that is at least 96%, at least 97%, at least 98%, or at least 99% identical to SEQ ID NO:69 and a light chain amino acid sequence that is at least 96%, at least 97%, at least 98%, or at least 99% identical to SEQ ID NO:70. In some embodiments, the anti-CD48 antibody is SGN-48A, or an antigen-binding fragment thereof.

In some embodiments, the anti-CD48 antibody or antigen-binding fragment thereof comprises the three heavy chain CDRs and three light chain CDRs of gemtuzumab or wherein the CDRs include no more than one, two, three, four, five, or six amino acid additions, deletions or substitutions of HCDR1 (SEQ ID NO:51), HCDR2 (SEQ ID NO:52), HCDR3 (SEQ ID NO:53); LCDR1 (SEQ ID NO:54), LCDR2 (SEQ ID NO:55), and LCDR3 (SEQ ID NO:56).

In some embodiments, the target cancer antigen for an ADC is CD79B.

In some embodiments, the anti-CD48 antibody or antigen-binding fragment thereof comprises three heavy chain CDRs and three light chain CDRs as follows: heavy chain CDR1 (HCDR1) consisting of SEQ ID NO:82, heavy chain CDR2 (HCDR2) consisting of SEQ ID NO:83, heavy chain CDR3 (HCDR3) consisting of SEQ ID NO:84; light chain CDR1 (LCDR1) consisting of SEQ ID NO:85, light chain CDR2 (LCDR2) consisting of SEQ ID NO:86, and light chain CDR3 (LCDR3) consisting of SEQ ID NO:87.

In some embodiments, the anti-CD79B antibody or antigen-binding fragment thereof comprises a heavy chain variable region comprising the amino acid sequence of SEQ ID NO:80, and a light chain variable region comprising the amino acid sequence of SEQ ID NO:81. In some embodiments, the anti-CD79B antibody or antigen-binding fragment thereof comprises the heavy chain variable region amino acid sequence of SEQ ID NO:80 and the light chain variable region amino acid sequence of SEQ ID NO:81, or sequences that are at least 95% identical to the disclosed sequences. In some embodiments, the anti-CD79B antibody or antigen-binding fragment thereof has a heavy chain variable region amino acid sequence that is at least 96%, at least 97%, at least 98%, or at least 99% identical to SEQ ID NO:80 and/or a light chain variable region amino acid sequence that is at least 96%, at least 97%, at least 98%, or at least 99% identical to SEQ ID NO:81.

In some embodiments, the anti-CD79B antibody or antigen-binding fragment thereof is an internalizing antibody or internalizing antigen-binding fragment. In some embodiments, the anti-CD79B antibody comprises a human IgG1 heavy chain constant domain or a modified IgG1 heavy chain constant domain. In some embodiments, the IgG1 heavy chain constant domain comprises a cysteine residue (C) at the amino acid positions corresponding to 152 and 375 in a wild-type (unmodified) IgG1 heavy chain constant domain numbered according to EU numbering system.

In some embodiments, the anti-CD79B antibody comprises the heavy chain amino acid sequence of SEQ ID NO:88 or a sequence that is at least 95% identical to SEQ ID NO:88, and the light chain amino acid sequence of SEQ ID NO:89 or a sequence that is at least 95% identical to SEQ ID NO:89. In some embodiments, the anti-CD33 antibody comprises the heavy chain amino acid sequence of SEQ ID NO:88 and the light chain amino acid sequence of SEQ ID NO:89, or sequences that are at least 95% identical to the disclosed sequences. In some embodiments, the anti-CD79B antibody has a heavy chain amino acid sequence that is at least 96%, at least 97%, at least 98%, or at least 99% identical to SEQ ID NO:88 and a light chain amino acid sequence that is at least 96%, at least 97%, at least 98%, or at least 99% identical to SEQ ID NO:89. In some embodiments, the anti-CD79B antibody is polatizumab, or an antigen-binding fragment thereof.

In some embodiments, the anti-CD79B antibody or antigen-binding fragment thereof comprises the three heavy chain CDRs and three light chain CDRs of gemtuzumab or wherein the CDRs include no more than one, two, three, four, five, or six amino acid additions, deletions or substitutions of HCDR1 (SEQ ID NO:82), HCDR2 (SEQ ID NO:83), HCDR3 (SEQ ID NO:84); LCDR1 (SEQ ID NO:85), LCDR2 (SEQ ID NO:86), and LCDR3 (SEQ ID NO:87).

Residues in two or more polypeptides are said to "correspond" if the residues occupy an analogous position in the polypeptide structures. Analogous positions in two or more polypeptides can be determined by aligning the polypeptide sequences based on amino acid sequence or structural similarities. Those skilled in the art understand that it may be necessary to introduce gaps in either sequence to produce a satisfactory alignment.

In some embodiments, amino acid substitutions are of single residues. Insertions usually will be on the order of from about 1 to about 20 amino acid residues, although considerably larger insertions may be tolerated as long as biological function is retained (e.g., binding to a target antigen). Deletions usually range from about 1 to about 20 amino acid residues, although in some cases deletions may be much larger. Substitutions, deletions, insertions, or any combination thereof may be used to arrive at a final derivative or variant. Generally, these changes are done on a few amino acids to minimize the alteration of the molecule, particularly the immunogenicity and specificity of the antigen binding protein. However, larger changes may be tolerated in certain circumstances. Conservative substitutions can be made in accordance with the following chart depicted as Table 7.

**Table 7**

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

In some embodiments where variant antibody sequences are used in an ADC, the variants typically exhibit the same qualitative biological activity and will elicit the same immune response, although variants may also be selected to modify the characteristics of the antigen binding proteins as needed. Alternatively, the variant may be designed such that the biological activity of the antigen binding protein is altered. For example, glycosylation sites may be altered or removed.

Various antibodies may be used with the ADCs used herein to target cancer cells. As shown below, the linker-payloads in the ADCs disclosed herein are surprisingly effective with different tumor antigen-targeting antibodies. Suitable antigens expressed on cancer cells but not healthy cells, or expressed on cancer cells at a higher level than on healthy cells, are known in the art, as are antibodies directed against them. Further antibodies against those antigen targets may be prepared by those of skill in the art. These antibodies may be used with the linkers and Mcl-1 inhibitor payloads disclosed herein. In some embodiments, the antibody or antigen-binding fragment targets BCMA, and the BCMA-targeting antibody or antigen-binding fragment is J6M0. In some embodiments, the antibody or antigen-binding fragment targets CD33, and in some embodiments the CD33-targeting antibody or antigen-binding fragment is MuMy9-6ch. In some embodiments, the antibody or antigen-binding fragment targets PCAD, and in some embodiments the PCAD-targeting antibody or antigen-binding fragment is NOV169N31Q. In some embodiments, the antibody or antigen-binding fragment targets HER2, and in some embodiments the HER2-targeting antibody or antigen-binding fragment is trastuzumab. In some embodiments, while the disclosed linkers and Mcl-1 inhibitor payloads are surprisingly effective with several different tumor-targeting antibodies, BCMA-targeting antibodies such as J6M0, CD33-targeting antibodies such as MuMy9-6ch, PCAD-targeting antibodies such as NOV169N31Q, and HER2-targeting antibodies such as trastuzumab, provided particularly improved drug:antibody ratio, aggregation level, stability (i.e., in vitro and in vivo stability), tumor targeting (i.e., cytotoxicity, potency), minimized off-target killing, and/or treatment efficacy. Improved treatment efficacy can be measured in vitro or in vivo, and may include reduced tumor growth rate and/or reduced tumor volume.

In some embodiments, alternate antibodies to the same targets or antibodies to different antigen targets are used and provide at least some of the favorable functional properties described above (e.g., improved stability, improved tumor targeting, improved treatment efficacy, etc.). In some embodiments, some or all of these favorable functional properties are observed when the disclosed linkers and Mcl-1 inhibitor payloads are conjugated to an alternate BCMA-, CD33-, CD46, CD48, PCAD-, or HER2-targeting antibody or antigen-binding fragment. In some other embodiments, some or all of these favorable functional properties are observed when the disclosed linkers and Mcl-1 inhibitor payloads are conjugated to a BCMA-targeting antibody or antigen-binding fragment. In some embodiments, the antibody or antigen-binding fragment targets BCMA. In some embodiments, the BCMA-targeting antibody or antigen-binding fragment is J6M0. In other embodiments, some or all of these favorable functional properties are observed when the disclosed linkers and Mcl-1 inhibitor payloads are conjugated to a CD33-targeting antibody or antigen-binding fragment. In some embodiments, the antibody or antigen-binding fragment targets CD33. In some embodiments, the CD33-targeting antibody or antigen-binding fragment is MuMy9-6ch. In other embodiments, some or all of these favorable functional properties are observed when the disclosed linkers and Mcl-1 inhibitor payloads are conjugated to a PCAD-targeting antibody or antigen-binding fragment. In some embodiments, the antibody or antigen-binding fragment targets PCAD. In some embodiments, the PCAD-targeting antibody or antigen-binding fragment is NOV169N31Q. In other embodiments, some or all of these favorable functional properties are observed when the disclosed linkers and Mcl-1 inhibitor payloads are conjugated to an HER2-targeting antibody or antigen-binding fragment. In some embodiments, the antibody or antigen-binding fragment targets HER2. In some embodiments, the HER2-targeting antibody or antigen-binding fragment is trastuzumab.

### Linkers

In some embodiments, the linker in an ADC is stable extracellularly in a sufficient manner to be therapeutically effective. In some embodiments, the linker is stable outside a cell, such that the ADC remains intact when present in extracellular conditions (e.g., prior to transport or delivery into a cell). The term "intact," used in the context of an ADC, means that the antibody or antigen-binding fragment remains attached to the drug moiety (e.g., the Mcl-1 inhibitor).

As used herein, "stable," in the context of a linker or ADC comprising a linker, means that no more than 20%, no more than about 15%, no more than about 10%, no more than about 5%, no more than about 3%, or no more than about 1% of the linkers (or any percentage in between) in a sample of ADC are cleaved (or in the case of an overall ADC are otherwise not intact) when the ADC is present in extracellular conditions. In some embodiments, the linkers and/or ADCs disclosed herein are stable compared to alternate linkers and/or ADCs with alternate linkers and/or Mcl-1 inhibitor payloads. In some embodiments, the ADCs disclosed herein can remain intact for more than about 48 hours, more than 60 hours, more than about 72 hours, more than about 84 hours, or more than about 96 hours.

Whether a linker is stable extracellularly can be determined, for example, by including an ADC in plasma for a predetermined time period (e.g., 2, 4, 6, 8, 16, 24, 48, or 72 hours) and then quantifying the amount of free drug moiety present in the plasma. Stability may allow the ADC time to localize to target cancer cells and prevent the premature release of the drug moiety, which could lower the therapeutic index of the ADC by indiscriminately damaging both normal and cancer tissues. In some embodiments, the linker is stable outside of a target cell and releases the drug moiety from the ADC once inside of the cell, such that the drug can bind to its target. Thus, an effective linker will: (i) maintain the specific binding properties of the antibody or antigen-binding fragment; (ii) allow delivery, e.g., intracellular delivery, of the drug moiety via stable attachment to the antibody or antigen-binding fragment; (iii) remain stable and intact until the ADC has been transported or delivered to its target site; and (iv) allow for the therapeutic effect, e.g., cytotoxic effect, of the drug moiety after cleavage or alternate release mechanism.

Linkers may impact the physico-chemical properties of an ADC. As many cytotoxic agents are hydrophobic in nature, linking them to the antibody with an additional hydrophobic moiety may lead to aggregation. ADC aggregates are insoluble and often limit achievable drug loading onto the antibody, which can negatively affect the potency of the ADC. Protein aggregates of biologics, in general, have also been linked to increased immunogenicity. As shown below, linkers disclosed herein result in ADCs with low aggregation levels and desirable levels of drug loading.

A linker may be "cleavable" or "non-cleavable" (Ducry and Stump (2010) Bioconjugate Chem. 21:5-13). Cleavable linkers are designed to release the drug moiety (e.g., an Mcl-1 inhibitor) when subjected to certain environment factors, e.g., when internalized into the target cell, whereas non-cleavable linkers generally rely on the degradation of the antibody or antigen-binding fragment itself.

The term "alkyl", as used herein, refers to a straight or branched hydrocarbon chain radical consisting solely of carbon and hydrogen atoms, containing no unsaturation. The term "C₁-C₆alkyl", as used herein, refers to a straight or branched hydrocarbon chain radical consisting solely of carbon and hydrogen atoms, containing no unsaturation, having from one to six carbon atoms, and which is attached to the rest of the molecule by a single bond. Non-limiting examples of "C₁-C₆alkyl" groups include methyl (a C₁alkyl), ethyl (a C₂alkyl), 1-methylethyl (a C₃alkyl), n-propyl (a C₃alkyl), isopropyl (a C₃alkyl), n-butyl (a C₄alkyl), isobutyl (a C₄alkyl), sec-butyl (a C₄alkyl), tert-butyl (a C₄alkyl), n-pentyl (a C₅alkyl), isopentyl (a C₅alkyl), neopentyl (a C₅alkyl) and hexyl (a C₆alkyl).

The term "alkenyl", as used herein, refers to a straight or branched hydrocarbon chain radical group consisting solely of carbon and hydrogen atoms, containing at least one double bond. The term "C₂-C₆alkenyl", as used herein, refers to a straight or branched hydrocarbon chain radical group consisting solely of carbon and hydrogen atoms, containing at least one double bond, having from two to six carbon atoms, which is attached to the rest of the molecule by a single bond. Non-limiting examples of "C₂-C₆alkenyl" groups include ethenyl (a C₂alkenyl), prop-1-enyl (a C₃alkenyl), but-1-enyl (a C₄alkenyl), pent-1-enyl (a C₅alkenyl), pent-4-enyl (a C₅alkenyl), penta-1,4-dienyl (a C₅alkenyl), hexa-1-enyl (a C₆alkenyl), hexa-2-enyl (a C₆alkenyl), hexa-3-enyl (a C₆alkenyl), hexa-1-,4-dienyl (a C₆alkenyl), hexa-1-,5-dienyl (a C₆alkenyl) and hexa-2-,4-dienyl (a C₆alkenyl). The term "C₂-C₃alkenyl", as used herein, refers to a straight or branched hydrocarbon chain radical group consisting solely of carbon and hydrogen atoms, containing at least one double bond, having from two to three carbon atoms, which is attached to the rest of the molecule by a single bond. Non-limiting examples of "C₂-C₃alkenyl" groups include ethenyl (a C₂alkenyl) and prop-1-enyl (a C₃alkenyl).

The term "alkylene", as used herein, refers to a bivalent straight or branched hydrocarbon chain radical consisting solely of carbon and hydrogen atoms and containing no unsaturation. The term "C₁-C₆alkylene", as used herein, refers to a bivalent straight or branched hydrocarbon chain radical consisting solely of carbon and hydrogen atoms, containing no unsaturation, having from one to six carbon atoms. Non-limiting examples of "C₁-C₆alkylene" groups include methylene (a C₁alkylene), ethylene (a C₂alkylene), 1-methylethylene (a C₃alkylene), n-propylene (a C₃alkylene), isopropylene (a C₃alkylene), n-butylene (a C₄alkylene), isobutylene (a C₄alkylene), sec-butylene (a C₄alkylene), tert-butylene (a C₄alkylene), n-pentylene (a C₅alkylene), isopentylene (a C₅alkylene), neopentylene (a C₅alkylene), and hexylene (a C₆alkylene).

The term "alkenylene", as used herein, refers to a bivalent straight or branched hydrocarbon chain radical consisting solely of carbon and hydrogen atoms and containing at least one double bond. The term "C₂-C₆alkenylene", as used herein, refers to a bivalent straight or branched hydrocarbon chain radical group consisting solely of carbon and hydrogen atoms, containing at least one double bond, and having from two to six carbon atoms. Non-limiting examples of "C₂-C₆alkenylene" groups include ethenylene (a C₂alkenylene), prop-1-enylene (a C₃alkenylene), but-1-enylene (a C₄alkenylene), pent-1-enylene (a C₅alkenylene), pent-4-enylene (a C₅alkenylene), penta-1,4-dienylene (a C₅alkenylene), hexa-1-enylene (a C₆alkenylene), hexa-2-enylene (a C₆alkenylene), hexa-3-enylene (a C₆alkenylene), hexa-1-,4-dienylene (a C₆alkenylene), hexa-1-,5-dienylene (a C₆alkenylene) and hexa-2-,4-dienylene (a C₆alkenylene). The term "C₂-C₆alkenylene", as used herein, refers to a bivalent straight or branched hydrocarbon chain radical group consisting solely of carbon and hydrogen atoms, containing at least one double bond, and having from two to thee carbon atoms. Non-limiting examples of "C₂-C₃alkenylene" groups include ethenylene (a C₂alkenylene) and prop-1-enylene (a C₃alkenylene).

The term "cycloalkyl," or "C₃-C₈cycloalkyl," as used herein, refers to a saturated, monocyclic, fused bicyclic, fused tricyclic or bridged polycyclic ring system. Non-limiting examples of fused bicyclic or bridged polycyclic ring systems include bicyclo[1.1.1]pentane, bicyclo[2.1.1]hexane, bicyclo[2.2.1]heptane, bicyclo[3.1.1]heptane, bicyclo[3.2.1]octane, bicyclo[2.2.2]octane and adamantanyl. Non-limiting examples monocyclic C₃-C₈cycloalkyl groups include cyclopropyl, cyclobutyl, cyclopentyl, cyclohexyl, cycloheptyl and cyclooctyl groups.

The term "haloalkyl," as used herein, refers to a linear or branched alkyl chain substituted with one or more halogen groups in place of hydrogens along the hydrocarbon chain. Examples of halogen groups suitable for substitution in the haloalkyl group include Fluorine, Bromine, Chlorine, and Iodine. Haloalkyl groups may include substitution with multiple halogen groups in place of hydrogens in an alkyl chain, wherein said halogen groups can be attached to the same carbon or to another carbon in the alkyl chain.

As used herein, the alkyl, alkenyl, alkynyl, alkoxy, amino, aryl, heteroaryl, cycloalkyl, and heterocycloalkyl groups may be optionally substituted by 1 to 4 groups selected from optionally substituted linear or branched (C₁-C₆)alkyl, optionally substituted linear or branched (C₂-C₆)alkenyl group, optionally substituted linear or branched (C₂-C₆)alkynyl group, optionally substituted linear or branched (C₁-C₆)alkoxy, optionally substituted (C₁-C₆)alkyl-S-, hydroxy, oxo (or N-oxide where appropriate), nitro, cyano, -C(O)-OR₀', -O-C(O)-R₀', -C(O)-NR₀'R₀", -NR₀'R₀", -(C=NR₀')-OR₀", linear or branched (C₁-C₆) haloalkyl, trifluoromethoxy, or halogen, wherein R₀' and R₀" are each independently a hydrogen atom or an optionally substituted linear or branched (C₁-C₆)alkyl group, and wherein one or more of the carbon atoms of linear or branched (C₁-C₆)alkyl group is optionally deuterated.

The term "polyoxyethylene", "polyethylene glycol" or "PEG", as used herein, refers to a linear chain, a branched chain or a star shaped configuration comprised of (OCH₂CH₂) groups. In certain embodiments a polyethylene or PEG group is -(OCH₂CH₂)ₜ*-, where t is 1-40 or 4-40, and where the "-" indicates the end directed toward the self-immolative spacer and the "*-" indicates the point of attachment to a terminal end group R' where R' is OH, OCH₃ or OCH₂CH₂C(=O)OH. In other embodiments a polyethylene or PEG group is -

(CH₂CH₂O)ₜ*-, where t is 1-40 or 4-40, and where the "-" indicates the end directed toward the self-immolative spacer and the "*-" indicates the point of attachment to a terminal end group R" where R" is H, CH₃ or CH₂CH₂C(=O)OH. For example, the term "PEG12" as used herein means that t is 12.

The term "polyalkylene glycol", as used herein, refers to a linear chain, a branched chain or a star shaped configuration comprised of (O(CH₂)ₘ)ₙ groups. In certain embodiments a polyethylene or PEG group is -(O(CH₂)ₘ)ₜ*-, where m is 1-10, t is 1-40 or 4-40, and where the "-" indicates the end directed toward the self-immolative spacer and the "*-" indicates the point of attachment to a terminal end group R' where R' is OH, OCH₃ or OCH₂CH₂C(=O)OH. In other embodiments a polyethylene or PEG group is -((CH₂)ₘO)ₜ*-, where m is 1-10, t is 1-40 or 4-40, and where the "-" indicates the end directed toward the self-immolative spacer and the "*-" indicates the point of attachment to a terminal end group R" where R" is H, CH₃ or CH₂CH₂C(=O)OH.

The term "reactive group", as used herein, is a functional group capable of forming a covalent bond with a functional group of an antibody, an antibody fragment, or another reactive group attached to an antibody or antibody fragment. Non limiting examples of such functional groups include reactive groups of Table 8 provided herein.

The term "attachment group" or "coupling group", as used herein, refers to a bivalent moiety which links the bridging spacer to the antibody or fragment thereof. The attachment or coupling group is a bivalent moiety formed by the reaction between a reaction group and a functional group on the antibody or fragment thereof. Non limiting examples of such bivalent moieties include the bivalent chemical moieties given in Table 8 and Table 9 provided herein.

The term "bridging spacer", as used herein, refers to one or more linker components which are covalently attached together to form a bivalent moiety which links the bivalent peptide spacer to the reactive group, links the bivalent peptide space to the coupling group, or links the attachment group to the at least one cleavable group. In certain embodiments the "bridging spacer" comprises a carboxyl group attached to the N-terminus of the bivalent peptide spacer via an amide bond.

The term "spacer moiety", as used herein, refers to one or more linker components which are covalently attached together to form a moiety which links the self-immolative spacer to the hydrophilic moiety.

The term "bivalent peptide spacer", as used herein, refers to bivalent linker comprising one or more amino acid residues covalently attached together to form a moiety which links the bridging spacer to the self immolative spacer. The one or more amino acid residues can be an residue of amino acids selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine.

In certain embodiments a "bivalent peptide spacer" is a combination of 2 to four amino acid residues where each residue is independently selected from a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine, for example -ValCit*; -CitVal*; -AlaAla*; -AlaCit*; - CitAla*; -AsnCit*; -CitAsn*; -CitCit*; -ValGlu*; -GluVal*; -SerCit*; -CitSer*; -LysCit*; -CitLys*; - AspCit*; -CitAsp*; -AlaVal*; -ValAla*; -PheAla*; -AlaPhe*; -PheLys*; -LysPhe*; -ValLys*; - LysVal*; -AlaLys*; -LysAla*; -PheCit*; -CitPhe*; -LeuCit*; -CitLeu*; -lleCit*; -Citlle*; -PheArg*; -ArgPhe*; -CitTrp*; -TrpCit*; -PhePheLys*; -LysPhePhe*; -DPhePheLys*; -DLysPhePhe*; - GlyPheLys*; -LysPheGly*; -GlyPheLeuGly- [SEQ ID NO:160]; -GlyLeuPheGly- [SEQ ID NO:161]; -AlaLeuAlaLeu- [SEQ ID NO:162], -GlyGlyGly*; -GlyGlyGlyGly- [SEQ ID NO:163]; - GlyPheValGly- [SEQ ID NO:164]; and -GlyValPheGly- [SEQ ID NO:165], wher the "-" indicates the point of attachment to the bridging spacer and the "*" indicates the point of attachment to the self-immolative spacer.

The term "linker component", as used herein, refers to a chemical moiety that is a part of the linker. Examples of linker components include: an alkylene group: -(CH₂)ₙ- which can either be linear or branched (where in this instance n is 1-18); an alkenylene group; an alkynylene group; an alkenyl group; an alkynyl group; an ethylene glycol unit: -OCH₂CH₂- or -CH₂CH₂O-; an polyethylene glycol unit: (-CH₂CH₂O-)ₓ (where x in this instance is 2-20); -O-; -S-; a carbonyl: -C(=O); an ester: C(=O)-O or O-C(=O); a carbonate: -OC(=O)O-; an amine: - NH-; an tertiary amine; an amide: -C(=O)-NH-, -NH-C(=O)- or -C(=O)N(C₁₋₆alkyl); a carbamate: -OC(=O)NH- or -NHC(=O)O; a urea: -NHC(=O)NH; a sulfonamide: -S(O)₂NH- or -NHS(O)₂;an ether: -CH₂O- or -OCH₂-; an alkylene substituted with one or more groups independently selected from carboxy, sulfonate, hydroxyl, amine, amino acid, saccharide, phosphate and phosphonate); an alkenylene substituted with one or more groups independently selected from carboxy, sulfonate, hydroxyl, amine, amino acid, saccharide, phosphate and phosphonate); an alkynylene substituted with one or more groups independently selected from carboxy, sulfonate, hydroxyl, amine, amino acid, saccharide, phosphate and phosphonate); a C₁-C₁₀alkylene in which one or more methylene groups is replace by one or more -S-, -NH- or -O- moieties; a ring systems having two available points of attachment such as a divalent ring selected from phenyl (including 1,2- 1,3- and 1,4- disubstituted phenyls), a C₅-C₆ heteroaryl, a C₃-C₈ cycloalkyl (including 1,1-disubstituted cyclopropyl, cyclobutyl, cyclopentyl or cyclohexyl, and 1,4-disubstituted cyclohexyl), and a C₄-C₈ heterocycloalkyl; a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine; a combination of 2 or more amino acid residues where each residue is independently selected from a residue of an amino acid selected from alanine (Ala), cysteine (Cys), aspartic acid (Asp), glutamic acid (Glu), phenylalanine (Phe), glycine (Gly), histidine (His), isoleucine (Ile), lysine (Lys), leucine (Leu),methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucune (Nle), selenocysteine (Sec), pyrrolysine (Pyl), homoserine, homocysteine, and desmethyl pyrrolysine, for example Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; CitAsp; Ala-Val; Val-Ala; Phe-Lys; Lys-Phe; Val-Lys; Lys-Val; Ala-Lys; Lys-Ala; Phe-Cit; CitPhe; Leu-Cit; Cit-Leu; Ile-Cit; Cit-lle; Phe-Arg; Arg-Phe; Cit-Trp; and Trp-Cit; and a self-immolative spacer, wherein the self-immolative spacer comprises one or more protecting (triggering) groups which are susceptible to acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage.

Non-limiting examples of such self-immolative spacers include: 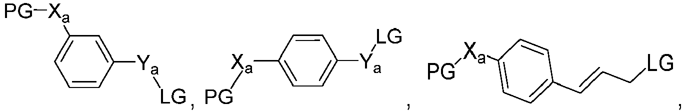 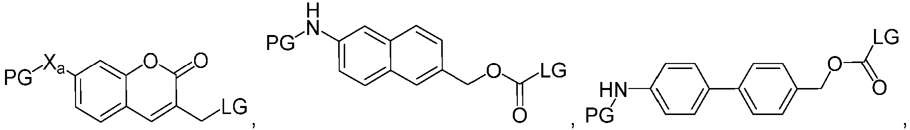 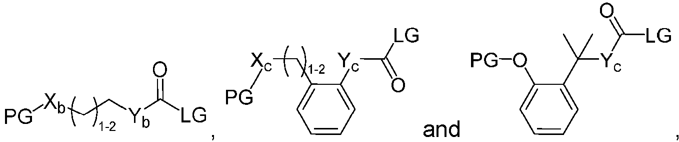 where:
PG is a protecting (triggering) group;
Xₐ is O, NH or S;
X_{b} is O, NH, NCH₃ or S;
X_{c} is O or NH;
Yₐ is CH₂, CH₂O or CH₂NH;
Y_{b} is CH₂, O or NH;
Y_{c} is a bond, CH₂, O or NH, and
LG is a leaving group such as a Drug moiety (D) of the Linker-Drug group of the invention.
Additional non-limiting examples of such self-immolative spacers are described in Angew. Chem. Int. Ed. 2015, 54, 7492 - 7509.

In addition, a linker component can be a chemical moiety which is readily formed by reaction between two reactive groups. Non-limiting examples of such chemical moieties are given in Table 8.

**Table 8**

| **Reactive Group 1 (RG1)** | **Reactive Group 2 (RG2)** | **Chemical Moiety** |
|---|---|---|
| a thiol | a thiol | -S-S- |
| a thiol | a maleimide | 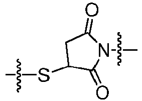 |
| a thiol | a haloacetamide | 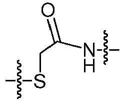 |
| an azide | an alkyne | 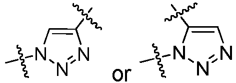 |
| an azide | a triaryl phosphine | 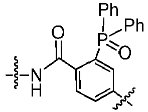 |
| an azide | a cyclooctyne | 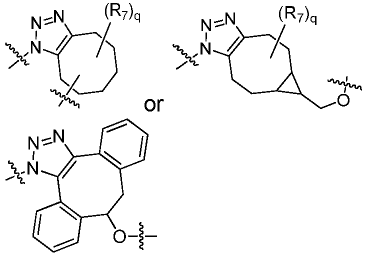 or |
| an azide | an oxanobornadiene | 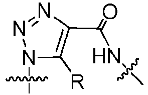 |
| a triaryl phosphine | an azide | 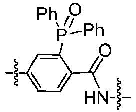 |
| an oxanobornadiene | an azide | 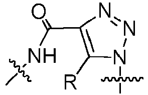 |
| an alkyne | an azide | 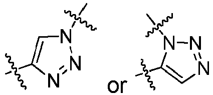 |
| a cyclooctyne | azide | 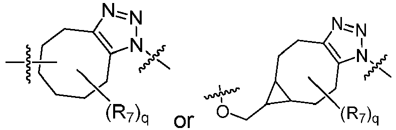 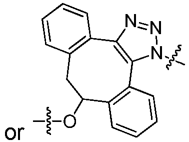 |
| a cyclooctene | a diaryl tetrazine | 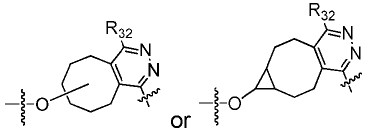 |
| a diaryl tetrazine | a cyclooctene | 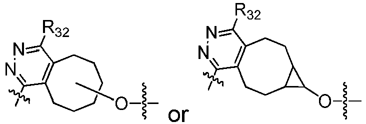 |
| a monoaryl tetrazine | a norbornene | 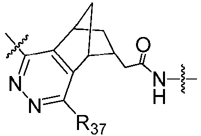 |
| a norbornene | a monoaryl tetrazine | 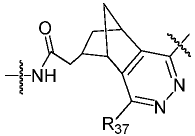 |
| an aldehyde | a hydroxylamine | 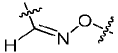 |
| an aldehyde | a hydrazine | 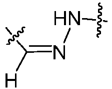 |
| an aldehyde | NH₂-NH-C(=O)- | 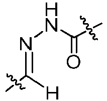 |
| a ketone | a hydroxylamine | 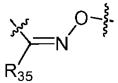 |
| a ketone | a hydrazine | 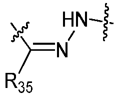 |
| a ketone | NH₂-NH-C(=O)- | 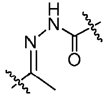 |
| a hydroxylamine | an aldehyde | 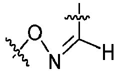 |
| a hydroxylamine | a ketone | 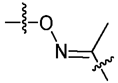 |
| a hydrazine | an aldehyde | 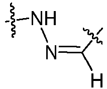 |
| a hydrazine | a ketone | 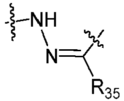 |
| NH₂-NH-C(=O)- | an aldehyde | 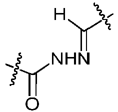 |
| NH₂-NH-C(=O)- | a ketone | 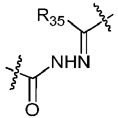 |
| a haloacetamide | a thiol | 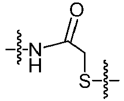 |
| a maleimide | a thiol | 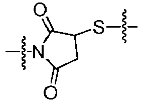 |
| a vinyl sulfone | a thiol | 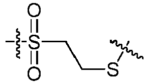 |
| a thiol | a vinyl sulfone | 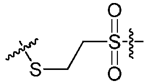 |
| an aziridine | a thiol | 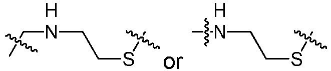 |
| a thiol | an aziridine | 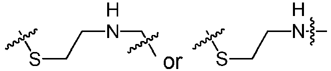 |
| 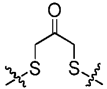 | hydroxylamine | 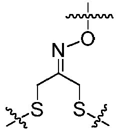 |
| 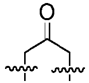 | hydroxylamine | 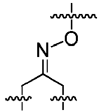 |
| 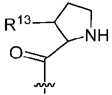 | 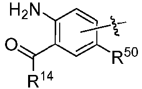 | 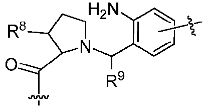 |
| 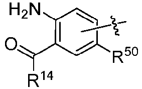 | 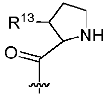 | 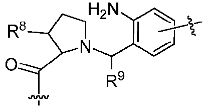 |
| 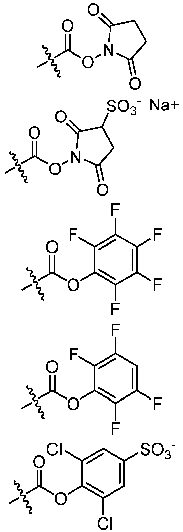 | -NH₂, | amide |
| -NH₂, | 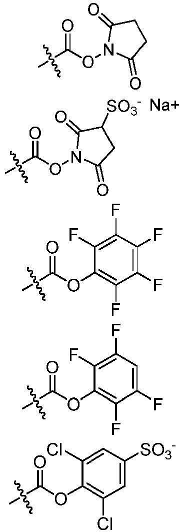 | amide |
| CoA or CoA analogue | Serine residue | 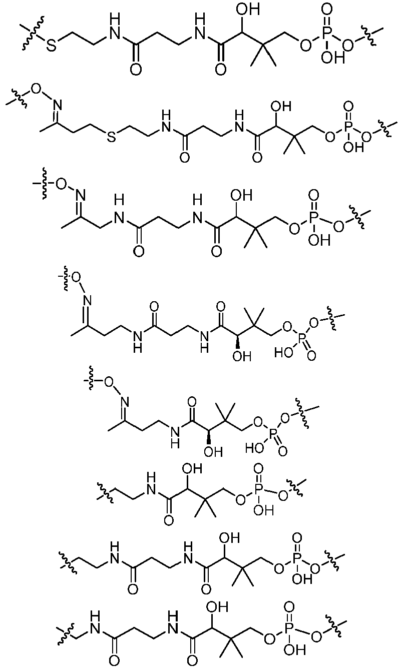 |
| pyridyldithiol | thiol | disulfide |

where: R³² in Table 8 is H, C₁₋₄ alkyl, phenyl, pyrimidine or pyridine; R³⁵ in Table 8 is H, C₁₋₆alkyl, phenyl or C₁₋₄alkyl substituted with 1 to 3 -OH groups; each R⁷ in Table 8 is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH; R³⁷ in Table 8 is independently selected from H, phenyl and pyridine; q in Table 8 is 0, 1, 2 or 3; R⁸ and R¹³ in Table 8 is H or methyl; and R⁹ and R¹⁴ in Table 8 is H, -CH₃ or phenyl; R in Table 8 is H or any suitable substituent; and R⁵⁰ in Table 8 is H.

In addition, a linker component can be a group listed in Table 9 below.

**Table 9.**

| | | | | |
|---|---|---|---|---|
| 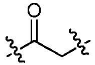 | 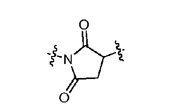 | 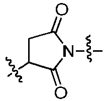 | 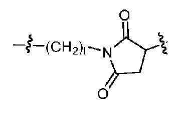 | 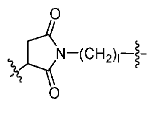 |
| 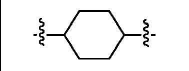 | 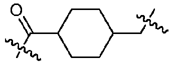 | 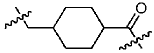 | 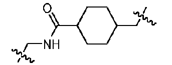 | 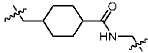 |
| | | | | |
| 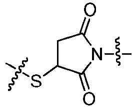 | 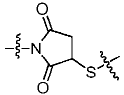 | 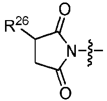 | 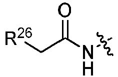 | 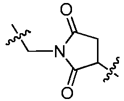 |
| 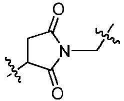 | 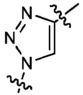 | 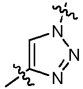 | 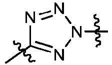 | 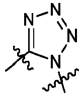 |
| 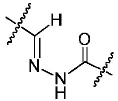 | 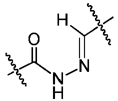 | 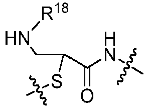 | 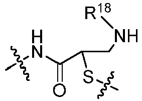 | 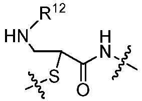 |
| 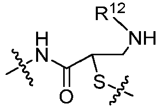 | 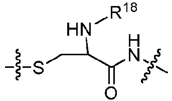 | 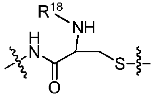 | 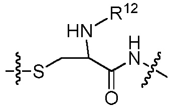 | 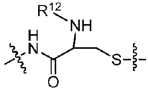 |
| 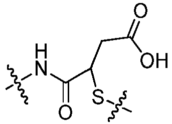 | 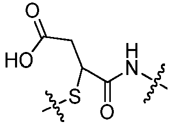 | 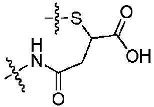 | 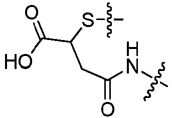 | 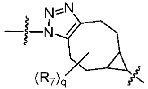 |
| 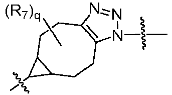 | 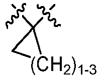 | 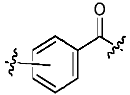 | 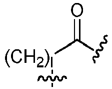 | 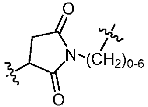 |
| 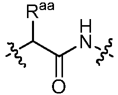 | 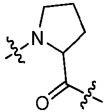 | 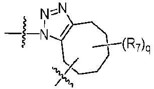 | 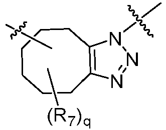 | 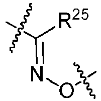 |
| 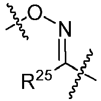 | 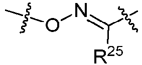 | 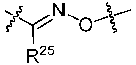 | 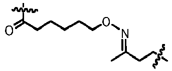 | 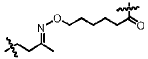 |
| 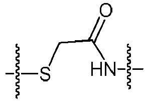 | 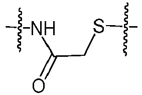 | 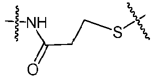 | 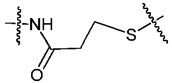 | 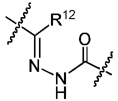 |
| 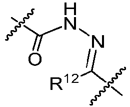 | 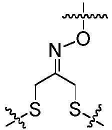 | 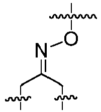 | 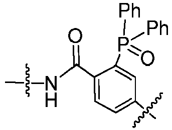 | 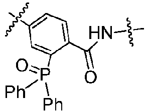 |
| 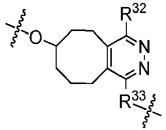 | 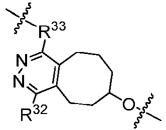 | 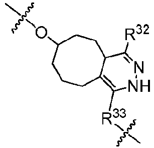 | 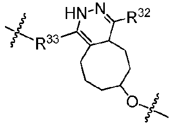 | 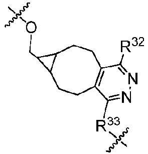 |
| 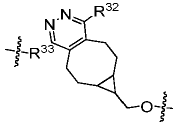 | 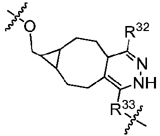 | 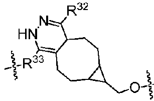 | 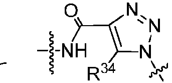 | 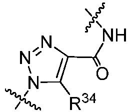 |
| 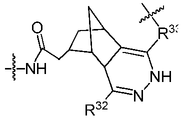 | 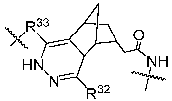 | 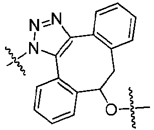 | 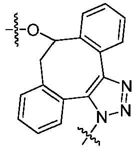 | 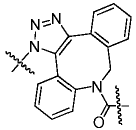 |
| 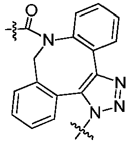 | 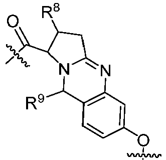 | 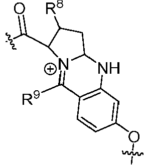 | 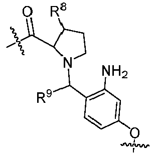 | 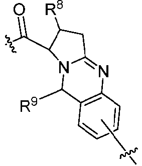 |
| 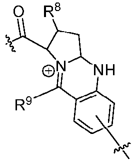 | 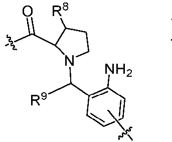 | 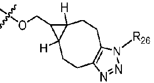 | 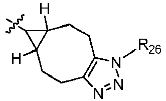 | 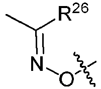 |
| each R⁷ is independently selected from H, C₁₋₆alkyl, fluoro, benzyloxy substituted with - C(=O)OH, benzyl substituted with -C(=O)OH, C₁₋₄alkoxy substituted with -C(=O)OH and C₁₋₄alkyl substituted with -C(=O)OH; | | | | |
| each R¹² is independently selected from H and C₁-C₆alkyl | | | | |
| R⁸ is H or methyl; | | | | |
| R⁹ is H, -CH₃ or phenyl; | | | | |
| each R²⁵ is independently selected from H or C₁₋₄ alkyl; | | | | |
| each R¹⁸ is independently selected from a C₁-C₆alkyl, a C₁-C₆alkyl which is substituted with azido and a C₁-C₆alkyl which is substituted with 1 to 5 hydroxyl; | | | | |
| q is 0, 1, 2 or 3; | | | | |
| l is 1, 2, 3, 4, 5 or 6; | | | | |
| R²⁶ is 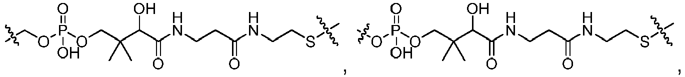 | | | | |
| 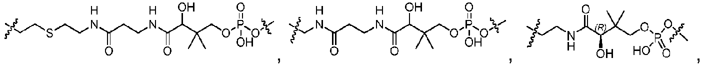 | | | | |
| 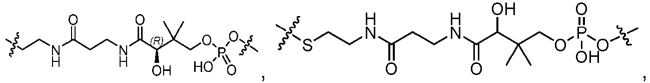 | | | | |
| 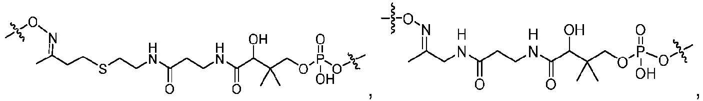 | | | | |
| 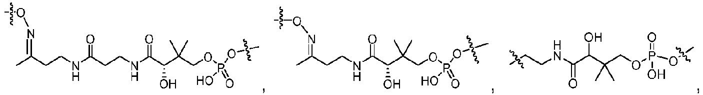 | | | | |
| 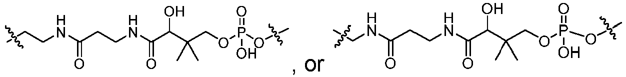 | | | | |
| R³² is independently selected from H, C₁₋₄ alkyl, phenyl, pyrimidine and pyridine; | | | | |
| R³³ is independently selected from 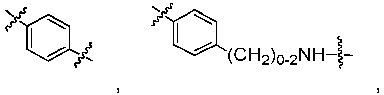 | | | | |
| 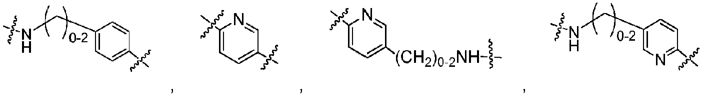 | | | | |
| 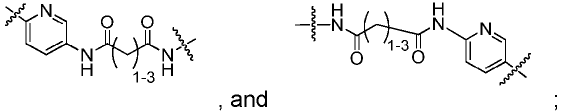 | | | | |
| R³⁴ is independently selected from H, C₁₋₄ alkyl, and C₁₋₆ haloalkyl, and | | | | |
| R^{aa} is an amino acid side chain. | | | | |

As used herein, when a partial structure of a compound is illustrated, a wavy line ( 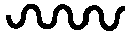 ) indicates the point of attachment of the partial structure to the rest of the molecule.

The terms "self-immolative spacer" and "self-immolative group", as used herein, refer a moiety comprising one or more triggering groups (TG) which are activated by acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage, and after activation the protecting group is removed, which generates a cascade of disassembling reactions leading to the temporally sequential release of a leaving group. Such cascade of reactions can be, but not limited to, 1,4-, 1,6- or 1,8- elimination reactions.

Non-limiting examples of self-immolative spacer or group include: 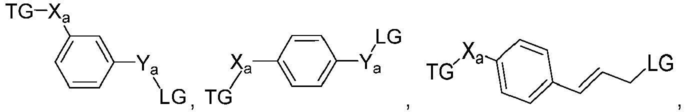 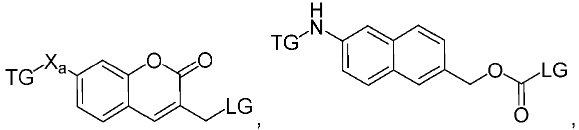 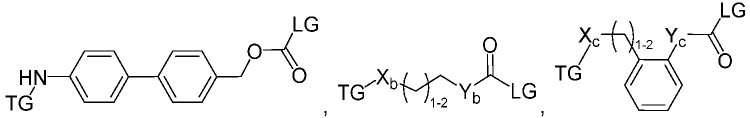 and 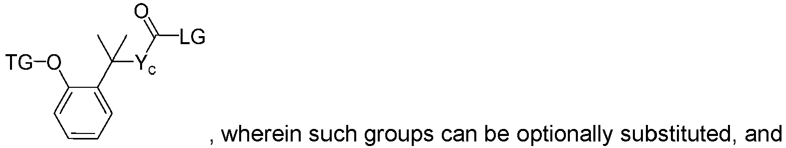 wherein:
TG is a triggering group;
Xₐ is O, NH or S;
X_{b} is O, NH, NCH₃ or S;
X_{c} is O or NH;
Yₐ is CH₂, CH₂O or CH₂NH;
Y_{b} is CH₂, O or NH;
Y_{c} is a bond, CH₂, O or NH, and
LG is a leaving group such as a Drug moiety (D) of the Linker-Drug group of the invention.
Additional non-limiting examples of self-immolative spacers are described in Angew. Chem. Int. Ed. 2015, 54, 7492 - 7509.

In certain embodiment the self-immolative spacer is moiety having the structure 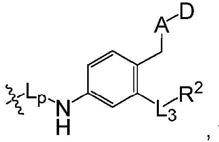 where Lp is an enzymatically cleavable bivalent peptide spacer and A, D, L₃ and R² are as defined herein.

In preferred embodiments, the self-immolative spacer is moiety having the structure 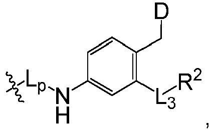 where Lp is an enzymatically cleavable bivalent peptide spacer and D, L₃ and R² are as defined herein. In some embodiments, D is a quaternized tertiary amine-containing MCI1 inhibitor.

In other preferred embodiments, the self-immolative spacer is moiety having the structure 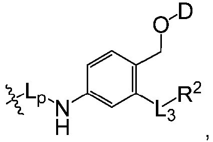 where Lp is an enzymatically cleavable bivalent peptide spacer and D, L₃ and R² are as defined herein.

The term "hydrophilic moiety", as used herein, refers to moiety that is has hydrophilic properties which increases the aqueous solubility of the Drug moiety (D) when the Drug moiety (D) is attached to the linker group of the invention. Examples of such hydrophilic groups include, but are not limited to, polyethylene glycols, polyalkylene glycols, sugars, oligosaccharides, polypeptides a C₂-C₆alkyl substituted with 1 to 3 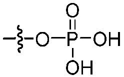 groups.

### Drug Moieties

In some embodiments, an intermediate, which is the precursor of the linker moiety, is reacted with the drug moiety (e.g., the Mcl-1 inhibitor) under appropriate conditions. In some embodiments, reactive groups are used on the drug and/or the intermediate or linker. The product of the reaction between the drug and the intermediate, or the derivatized drug (drug plus linker), is subsequently reacted with the antibody or antigen-binding fragment under conditions that facilitate conjugation of the drug and intermediate or derivatized drug and antibody or antigen-binding fragment. Alternatively, the intermediate or linker may first be reacted with the antibody or antigen-binding fragment, or a derivatized antibody or antigen-binding fragment, and then reacted with the drug or derivatized drug.

A number of different reactions are available for covalent attachment of the drug moiety and/or linker moiety to the antibody or antigen-binding fragment. This is often accomplished by reaction of one or more amino acid residues of the antibody or antigen-binding fragment, including the amine groups of lysine, the free carboxylic acid groups of glutamic acid and aspartic acid, the sulfhydryl groups of cysteine, and the various moieties of the aromatic amino acids. For instance, non-specific covalent attachment may be undertaken using a carbodiimide reaction to link a carboxy (or amino) group on a drug moiety to an amino (or carboxy) group on an antibody or antigen-binding fragment. Additionally, bifunctional agents such as dialdehydes or imidoesters may also be used to link the amino group on a drug moiety to an amino group on an antibody or antigen-binding fragment. Also available for attachment of drugs (e.g., an Mcl-1 inhibitor) to binding agents is the Schiff base reaction. This method involves the periodate oxidation of a drug that contains glycol or hydroxy groups, thus forming an aldehyde which is then reacted with the binding agent. Attachment occurs via formation of a Schiff base with amino groups of the binding agent. Isothiocyanates may also be used as coupling agents for covalently attaching drugs to binding agents. Other techniques are known to the skilled artisan and within the scope of the present disclosure. Examples of drug moieties that can be generated and linked to an antibody or antigen-binding fragment using various chemistries known to in the art include Mcl-1 inhibitors, e.g., the Mcl-1 inhibitors described and exemplified herein.

Suitable drug moieties may comprise a compound of the formulas (I), (II), (III), or an enantiomer, diastereoisomer, atropisomer, deuterated derivative, and/or addition salt thereof with a pharmaceutically acceptable acid or base. Additionally, the drug moiety may comprise any compounds of the Mcl-1 inhibitor (D) described herein.

As used herein, "atropisomers," are stereoisomers arising because of hindered rotation about a single bond, where energy differences due to steric strain or other contributors create a barrier to rotation that is high enough to allow for isolation of individual conformers (Bringmann et al. Angew. Chem. Int. Ed. 2005, 44, 5384-5427). For example, for compounds of formula (II) according to the invention, atropisomers may be as follows:

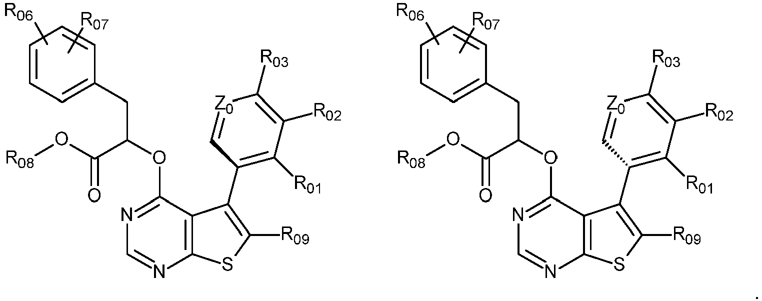

For example, a preferred atropisomer may be (5*S*ₐ), also named (5a*S*).

A drug moiety of the disclosure may be any one of the compounds disclosed in International Patent Application Publication Nos. WO 2015/097123; WO 2016/207216; WO 2016/207217; WO 2016/207225; WO 2016/207226; WO 2017/125224; WO 2019/035899; WO 2019/035911; WO 2019/035914; WO 2019/035927; WO 2016/033486; WO 2017/147410; WO 2018/183418; and WO 2017/182625, and U.S. Patent Application Publication No. 2019/0055264.

In some embodiments, a drug moiety of the disclosure may comprise a compound of Formula (I): 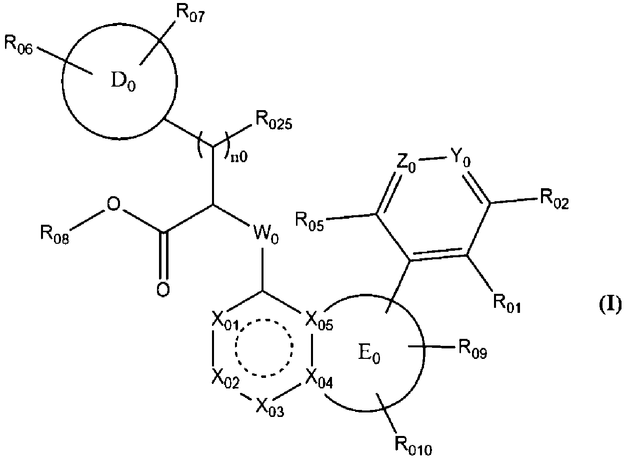 wherein:
Ring D₀ is a cycloalkyl group, a heterocycloalkyl group, an aryl group or a heteroaryl group,
Ring E₀ is a furyl, thienyl or pyrrolyl ring,
X₀₁, X₀₃, X₀₄ and X₀₅ independently of one another are a carbon atom or a nitrogen atom,
X₀₂ is a C-R₀₂₆ group or a nitrogen atom,
   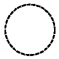 means that the ring is aromatic,
Y₀ is a nitrogen atom or a C-R₀₃ group,
Z₀ is a nitrogen atom or a C-R₀₄ group,
R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a hydroxy(C₁-C₆)alkyl group, a linear or branched (C₁-C₆)alkoxy group, -S-(C₁-C₆)alkyl group, a cyano group, a nitro group, -Cy₀₈, -(C₀-C₆)alkyl-NR₀₁₁R₀₁₁', -O-(C₁-C₆)alkyl-NR₀₁₁R₀₁₁', -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
R₀₂, R₀₃, R₀₄ and R₀₅ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a hydroxy(C₁-C₆)alkyl group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-CY₀₁, -(C₂-C₆)alkynyl-CY₀₁, -O-(C₁-C₆)alkyl-NR₀₁₁R₀₁₁', -O-(C₁-C₆)alkyl-R₀₃₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
or the pair (R₀₁, R₀₂), (R₀₂, R₀₃), (R₀₃, R₀₄), or (R₀₄, R₀₅) together with the carbon atoms to which they are attached form an aromatic or non-aromatic ring containing 5 to 7 ring members, which optionally contains 1 to 3 heteroatoms selected from O, S and N, wherein the resulting ring is optionally substituted by 1 or 2 groups selected from halogen, linear or branched (C₁-C₆)alkyl, (C₀-C₆)alkyl-NR₀₁₁R₀₁₁', -NR₀₁₃R₀₁₃', -(C₀-C₆)alkyl-Cy₀₁ or oxo,
R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-(C₁-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-CY₀₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl, or the pair (R₀₆, R₀₇), when fused with the two adjacent carbon atoms, together with the carbon atoms to which they are attached form an aromatic or non-aromatic ring containing 5 to 7 ring members, which optionally contains 1 to 3 heteroatoms selected from O, S and N, wherein the resulting ring is optionally substituted by a linear or branched (C₁-C₆)alkyl group, -NR₀₁₃R₀₁₃', -(C₀-C₆)alkyl-Cy₀₁ or an oxo,
W₀ is a -CH₂- group, a -NH- group or an oxygen atom,
R₀₈ is a hydrogen atom, a linear or branched (C₁-C₈)alkyl group, a -CHR₀ₐR_{0b} group, an aryl group, a heteroaryl group, an aryl(C₁-C₆)alkyl group, or a heteroaryl(C₁-C₆)alkyl group,
R₀₉ is a hydrogen atom, a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, or -C(O)-NR₀₁₄R₀₁₄',
R₀₁₀ is a hydrogen atom, a linear or branched (C₁-C₆)alkyl group, a linear or branched (C₂-C₆)alkenyl group, a linear or branched (C₂-C₆)alkynyl group, an aryl(C₁-C₆)alkyl group, a (C₁-C₆)cycloalkylalkyl group, a linear or branched (C₁-C₆)haloalkyl, or -(C₁-C₆)alkyl-O-Cy₀₄, or the pair (R₀₉, R₀₁₀), when fused with the two adjacent carbon atoms, together with the carbon atoms to which they are attached form an aromatic or non-aromatic ring containing 5 to 7 ring members, which optionally contains 1 to 3 heteroatoms selected from O, S and N,
R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S, and N, wherein the N atom may be substituted by 1 or 2 groups selected from a linear or branched (C₁-C₆)alkyl group, and wherein one or more of the carbon atoms of the linear or branched (C₁-C₆)alkyl group is optionally deuterated,
R₀₁₂ is -Cy₀₅, -Cy₀₅-(C₀-C₆)alkyl-O-(C₀-C₆)alkyl-Cy₀₆, -Cy₀₅-(C₀-C₆)alkyl-Cy₀₆, -Cy₀₅-(C₀-C₆)alkyl-NR₀₁₁-(C₀-C₆)alkyl-Cy₀₆, -Cy₀₅-Cy₀₆-O-(C₀-C₆)alkyl-Cy₀₇, -Cy₀₅-(C₀-C₆)alkyl-O-(C₀-C₆)alkyl-Cy₀₉, -Cy₀₅-(C₀-C₆)alkyl-Cy₀₉, -NH-C(O)-NH-R₀₁₁, -Cy₀₅-(C₀-C₆)alkyl-NR₀₁₁-(C₀-C₆)alkyl-Cy₀₉, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, -C(O)-OR₀₁₁,
R₀₁₃, R₀₁₃', R₀₁₄ and R₀₁₄' independently of one another are a hydrogen atom, or an optionally substituted linear or branched (C₁-C₆)alkyl group,
R₀ₐ is a hydrogen atom or a linear or branched (C₁-C₆)alkyl group,
R_{0b} is a -O-C(O)-O-R_{0c} group, a -O-C(O)-NR_{0c}R_{0c}' group, or a -O-P(O)(OR_{0c})₂ group,
R_{0c} and R_{0c}' independently of one another are a hydrogen atom, a linear or branched (C₁-C₈)alkyl group, a cycloalkyl group, a (C₁-C₆)alkoxy(C₁-C₆)alkyl group, or a (C₁-C₆)alkoxycarbonyl(C₁-C₆)alkyl group, or the pair (R_{0c}, R_{0c}') together with the nitrogen atom to which they are attached form a non-aromatic ring composed of from 5 to 7 ring members, which may contain in addition to the nitrogen atom from 1 to 3 heteroatoms selected from oxygen and nitrogen, wherein the nitrogen is optionally substituted by a linear or branched (C₁-C₆)alkyl group,
Cy₀₁, Cy₀₂, Cy₀₃, Cy₀₄, Cy₀₅, Cy₀₆, Cy₀₇, Cy₀₈ and Cy₀₁₀ independently of one another, are an optionally substituted cycloalkyl group, an optionally substituted heterocycloalkyl group, an optionally substituted aryl group or an optionally substituted heteroaryl group, 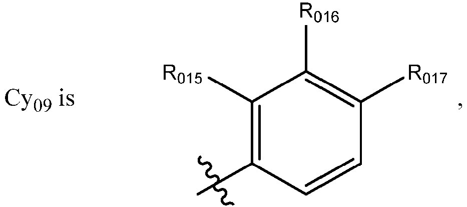 or Cy₀₉ is a heteroaryl group which is substituted by a group selected from -O-P(O)(OR₀₂₀)₂; -O-P(O)(O⁻M⁺)₂; -(CH₂)ₚ₀-O-(CHR₀₁₈-CHR₀₁₉-O)_{q0}-R₀₂₀; hydroxy; hydroxy(C₁-C₆)alkyl; -(CH₂)ᵣ₀-U₀-(CH₂)ₛ₀-heterocycloalkyl; and -U₀-(CH₂)_{q0}-NR₀₂₁R₀₂₁',
R₀₁₅ is a hydrogen atom; a -(CH₂)ₚ₀-O-(CHR₀₁₈-CHR₀₁₉-O)_{q0}-R₀₂₀ group; a linear or branched (C₁-C₆)alkoxy(C₁-C₆)alkyl group; a -U₀-(CH₂)_{q0}-NR₀₂₁R₀₂₁' group; or a -(CH₂)ᵣ₀-U₀-(CH₂)ₛ₀-heterocycloalkyl group,
R₀₁₆ is a hydrogen atom; a hydroxy group; a hydroxy(C₁-C₆)alkyl group; a -(CH₂)ᵣ₀-U₀-(CH₂)ₛ₀-heterocycloalkyl group; a (CH₂)ᵣ₀-U₀-V₀-O-P(O)(OR₀₂₀)₂ group; a -O-P(O)(O⁻M⁺)₂ group; a -O-S(O)₂OR₀₂₀ group; a -S(O)₂OR₀₂₀ group; a -(CH₂)ₚ₀-O-(CHR₀₁₈-CHR₀₁₉-O)_{q0}-R₀₂₀ group; a -(CH₂)ₚ₀-O-C(O)-NR₀₂₂R₀₂₃ group; or a -U₀-(CH₂)_{q0}-NR₀₂₁R₀₂₁' group,
R₀₁₇ is a hydrogen atom; a -(CH₂)ₚ₀-O-(CHR₀₁₈-CHR₀₁₉-O)_{q0}-R₀₂₀ group; a -CH₂-P(O)(OR₀₂₀)₂ group, a -O-P(O)(OR₀₂₀)₂ group; a -O-P(O)(O⁻M⁺)₂ group; a hydroxy group; a hydroxy(C₁-C₆)alkyl group; a -(CH₂)ᵣ₀-U₀-(CH₂)ₛ₀-heterocycloalkyl group; a -U₀-(CH₂)_{q0}-NR₀₂₁R₀₂₁' group; or an aldonic acid,
M⁺ is a pharmaceutically acceptable monovalent cation,
U₀ is a bond or an oxygen atom,
V₀ is a -(CH₂)ₛ₀- group or a -C(O)- group,
R₀₁₈ is a hydrogen atom or a (C₁-C₆)alkoxy(C₁-C₆)alkyl group,
R₀₁₉ is a hydrogen atom or a hydroxy(C₁-C₆)alkyl group,
R₀₂₀ is a hydrogen atom or a linear or branched (C₁-C₆)alkyl group,
R₀₂₁ and R₀₂₁' independently of one are a hydrogen atom, a linear or branched (C₁-C₆)alkyl group, or a hydroxy(C₁-C₆)alkyl group, or the pair (R₀₂₁, R₀₂₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the resulting ring is optionally substituted by a hydrogen atom or a linear or branched (C₁-C₆)alkyl group,
R₀₂₂ is a (C₁-C₆)alkoxy(C₁-C₆)alkyl group, a -(CH₂)ₚ₀-NR₀₂₄R₀₂₄' group, or a -(CH₂)ₚ₀-O-(CHR₀₁₈-CHR₀₁₉-O)_{q0}-R₀₂₀ group,
R₀₂₃ is a hydrogen atom or a (C₁-C₆)alkoxy(C₁-C₆)alkyl group, or the pair (R₀₂₂, R₀₂₃) together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched (C₁-C₆)alkyl group or a heterocycloalkyl group,
R₀₂₄ and R₀₂₄' independently of one another are a hydrogen atom or a linear or branched (C₁-C₆)alkyl group, or the pair (R₀₂₄, R₀₂₄') together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched (C₁-C₆)alkyl group,
R₀₂₅ is a hydrogen atom, a hydroxy group, or a hydroxy(C₁-C₆)alkyl group,
R₀₂₆ is a hydrogen atom, a halogen atom, a linear or branched (C₁-C₆)alkyl group, or a cyano group,
R₀₂₇ is a hydrogen atom or a linear or branched (C₁-C₆)alkyl group,
R₀₂₈ is a -O-P(O)(O⁻)(O⁻) group, a -O-P(O)(O⁻)(OR₀₃₀) group, a -O-P(O)(OR₀₃₀)(OR₀₃₀') group, a -(CH₂)ₚ₀-O-SO₂- group, a -(CH₂)ₚ₀-SO₂-O⁻ group, a -(CH₂)ₚ₀-O-SO₂-OR₀₃₀ group, -Cy₀₁₀, a -(CH₂)ₚ₀-SO₂-OR₀₃₀ group, a -O-C(O)-R₀₂₉ group, a -O-C(O)-OR₀₂₉ group or a -O-C(O)-NR₀₂₉R₀₂₉' group;
R₀₂₉ and R₀₂₉' independently of one another are a hydrogen atom, a linear or branched (C₁-C₆)alkyl group or a linear or branched amino(C₁-C₆)alkyl group,
R₀₃₀ and R₀₃₀' independently of one another are a hydrogen atom, a linear or branched (C₁-C₆)alkyl group or an aryl(C₁-C₆)alkyl group,
R₀₃₁ is 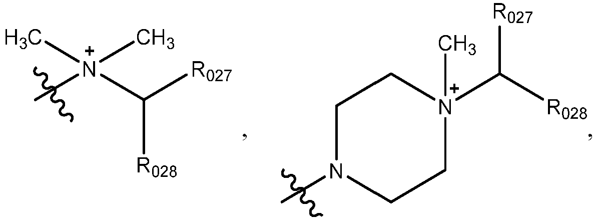 or 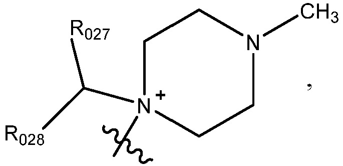 wherein the ammonium ion optionally exists as a zwitterionic form or has a monovalent anionic counterion,
n₀ is an integer equal to 0 or 1,
p₀ is an integer equal to 0, 1, 2, or 3,
q₀ is an integer equal to 1, 2, 3 or 4,
r₀ and s₀ are independently an integer equal to 0 or 1;

wherein, at most, one of the R₀₃, R₀₉, or R₀₁₂ groups, if present, is covalently attached to the linker, and
wherein the valency of an atom is not exceeded by virtue of one or more substituents bonded thereto,
or an enantiomer, diastereoisomer, atropisomer, deuterated derivative, and/or pharmaceutically acceptable salt of any of the foregoing.

In some embodiments, a drug moiety of the disclosure may comprise a compound of Formula (II): 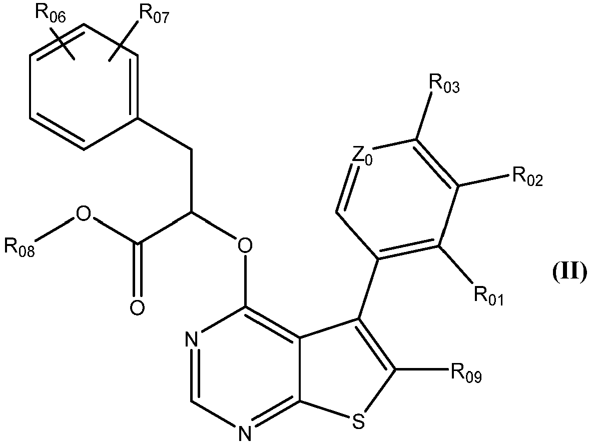 wherein:
Z₀ is a nitrogen atom or a C-R₀₄ group,
R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a linear or branched (C₁-C₆)alkoxy group, a -S-(C₁-C₆)alkyl group, a cyano group, -Cy₀₈, -NR₀₁₁R₀₁₁',
R₀₂, R₀₃ and R₀₄ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-CY₀₁, -O-(C₁-C₆)alkyl-NR₀₁₁R₀₁₁', -O-(C₁-C₆)alkyl-R₀₃₁, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
   or the pair (R₀₂, R₀₃) or (R₀₃, R₀₄) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched (C₁-C₆)alkyl,
   -NR₀₁₃R₀₁₃', -(C₀-C₆)alkyl-Cy₀₁ and oxo,
R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-CY₀₁, -(C₂-C₆)alkynyl-CY₀₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl, or the pair (R₀₆, R₀₇), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched (C₁-C₆)alkyl group, -NR₀₁₃R₀₁₃', -(C₀-C₆)alkyl-Cy₀₁ and an oxo,
R₀₈ is a hydrogen atom, a linear or branched (C₁-C₈)alkyl group, an aryl group, a heteroaryl group, an aryl-(C₁-C₆)alkylgroup, or a heteroaryl(C₁-C₆)alkyl group,
R₀₉ is a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, -C(O)-NR₀₁₄R₀₁₄',
R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched (C₁-C₆)alkyl group, and wherein one or more of the carbon atoms of the linear or branched (C₁-C₆)alkyl group is optionally deuterated,
R₀₁₂ is -Cy₀₅, -Cy₀₅-(C₀-C₆)alkyl-Cy₀₆, -Cy₀₅-(C₀-C₆)alkyl-O-(C₀-C₆)alkyl-Cy₀₆, -Cy₀₅-(C₀-C₆)alkyl-NR₀₁₁-(C₀-C₆)alkyl-Cy₀₆, -Cy₀₅-Cy₀₆-O-(C₀-C₆)alkyl-Cy₀₇, -Cy₀₅-(C₀-C₆)alkyl-Cy₀₉, -NH-C(O)-NH-R₀₁₁, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, or -C(O)-OR₀₁₁,
R₀₁₃, R₀₁₃', R₀₁₄ and R₀₁₄' independently of one another are a hydrogen atom, or an optionally substituted linear or branched (C₁-C₆)alkyl group,
Cy₀₁, Cy₀₂, Cy₀₃, Cy₀₅, Cy₀₆, Cy₀₇ and Cy₀₈ independently of one another, are an optionally substituted cycloalkyl group, an optionally substituted heterocycloalkyl group, an optionally substituted aryl group or an optionally substituted heteroaryl group,
Cy₀₉ is 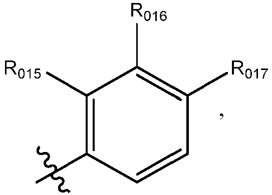 wherein R₀₁₅, R₀₁₆, and R₀₁₇ are as defined for formula (I),
   R₀₃₁ is 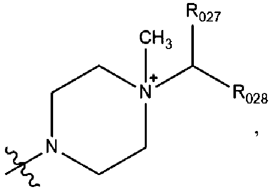 wherein R₀₂₇ and R₀₂₈ are as defined for formula (I) wherein, at most, one of the R₀₃, R₀₉, or R₀₁₂ groups, if present, is covalently attached to the linker,
or an enantiomer, diastereoisomer, atropisomer, deuterated derivative, and/or pharmaceutically acceptable salt of any of the foregoing.

In some embodiments, a drug moiety of the disclosure may comprise a compound of Formula (III): 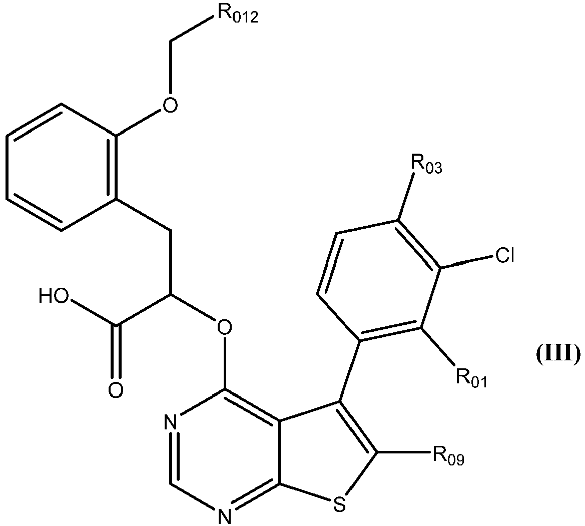 wherein:
R₀₁ is a linear or branched (C₁-C₆)alkyl group,
R₀₃ is -O-(C₁-C₆)alkyl-NR₀₁₁R₀₁₁', or 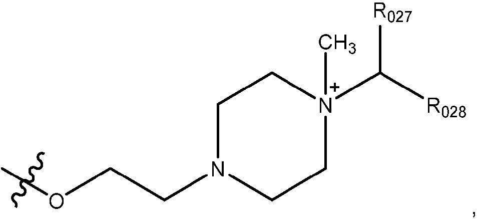
wherein R₀₁₁ and R₀₁₁' independently of one another are a hydrogen atom, an optionally substituted linear or branched (C₁-C₆)alkyl group, or -(C₀-C₆)alkyl-Cy₀₁;
or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom may be substituted by 1 or 2 groups selected from a hydrogen atom or a linear or branched (C₁-C₆)alkyl group,
and wherein R₀₂₇ is a hydrogen atom and R₀₂₈ is a -(CH₂)ₚ₀-O-SO₂-O⁻ group or a
   -(CH₂)ₚ₀-SO₂-OR₀₃₀ group;
R₀₉ is a linear or branched (C₂-C₆)alkynyl group or -Cy₀₂,
R₀₁₂ is -Cy₀₅, -Cy₀₅-(C₀-C₆)alkyl-Cy₀₆, or -Cy₀₅-(C₀-C₆)alkyl-Cy₀₉,
Cy₀₁, Cy₀₂, Cy₀₅ and Cy₀₆ independently of one another, are a cycloalkyl group, a heterocycloalkyl group, an aryl group or a heteroaryl group, each of which is optionally substituted,
Cy₀₉ is 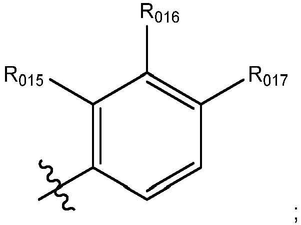
p₀, R₀₁₅, R₀₁₆, and R₀₁₇ are as defined for formula (I),
wherein, at most, one of the R₀₃, R₀₉, or R₀₁₂ groups, if present, is covalently attached to the linker,
or the enantiomer, diastereoisomer, atropisomer, deuterated derivative, and/or pharmaceutically acceptable salt of any of the foregoing.

In some embodiments, Cy₀₁, Cy₀₂, Cy₀₃, Cy₀₄, Cy₀₅, Cy₀₆, Cy₀₇, Cy₀₈ and Cy₀₁₀ independently of one another, are an optionally substituted cycloalkyl group, an optionally substituted heterocycloalkyl group, an optionally substituted aryl group or an optionally substituted heteroaryl group, wherein the optional substituents are selected from optionally substituted linear or branched (C₁-C₆)alkyl, optionally substituted linear or branched (C₂-C₆)alkenyl group, optionally substituted linear or branched (C₂-C₆)alkynyl group, optionally substituted linear or branched (C₁-C₆)alkoxy, optionally substituted (C₁-C₆)alkyl-S-, hydroxy, oxo (or N-oxide where appropriate), nitro, cyano, -C(O)-OR₀', -O-C(O)-R₀', -C(O)-NR₀'R₀", -NR₀'R₀", -(C=NR₀')-OR₀", linear or branched (C₁-C₆)haloalkyl, trifluoromethoxy, or halogen, wherein R₀' and R₀" are each independently a hydrogen atom or an optionally substituted linear or branched (C₁-C₆)alkyl group, and wherein one or more of the carbon atoms of linear or branched (C₁-C₆)alkyl group is optionally deuterated.

In some embodiments, the drug moiety (D) comprises: 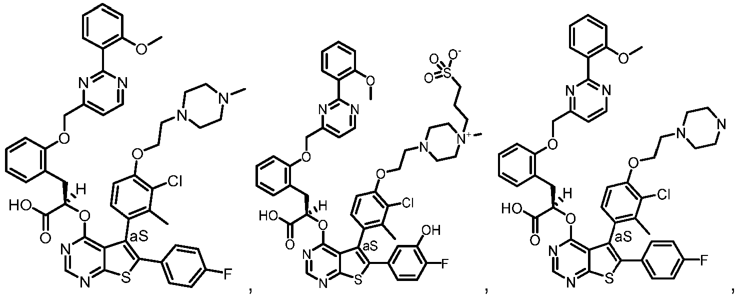 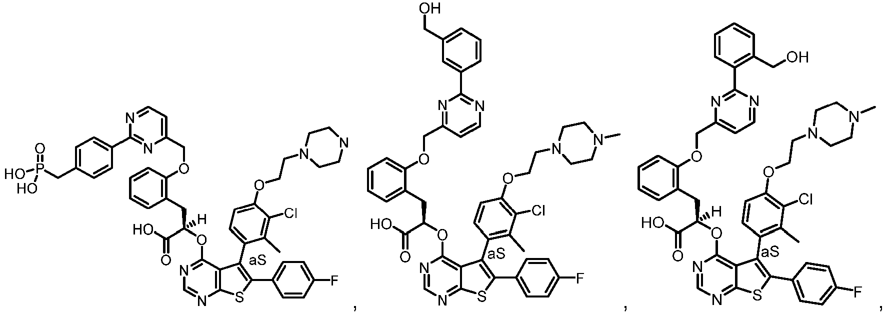 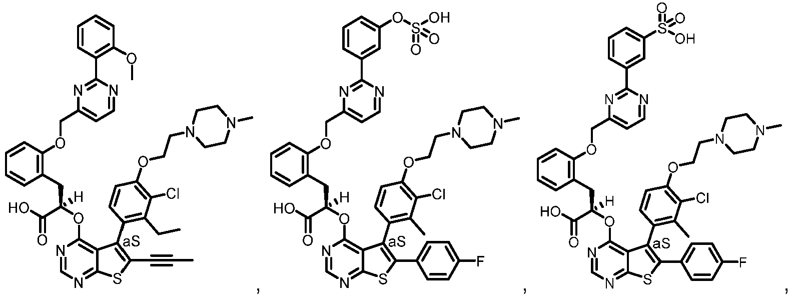 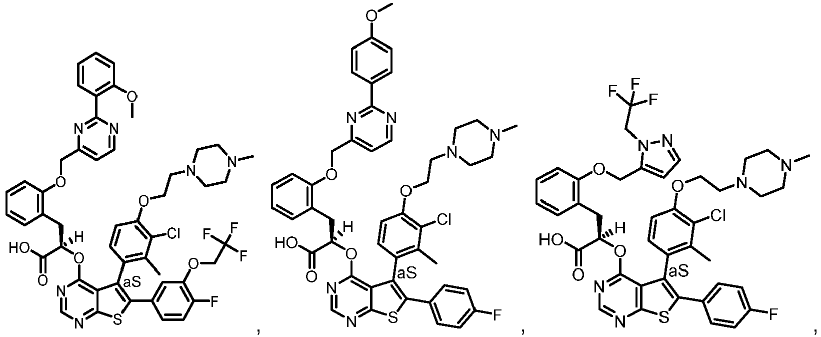 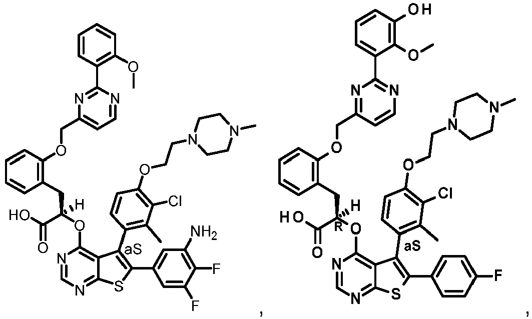 or an enantiomer, diastereoisomer, atropisomer, deuterated derivative, and/or a pharmaceutically acceptable salt of any of the foregoing.

Additionally, a drug moiety of the disclosure may comprise any one of the following: 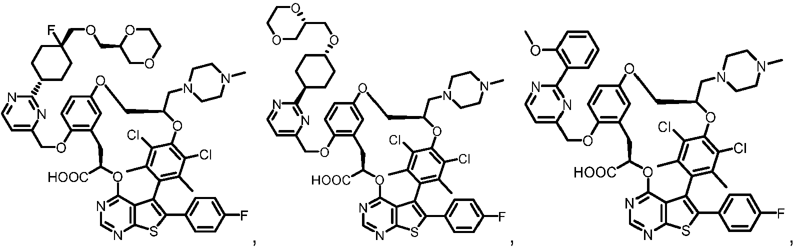 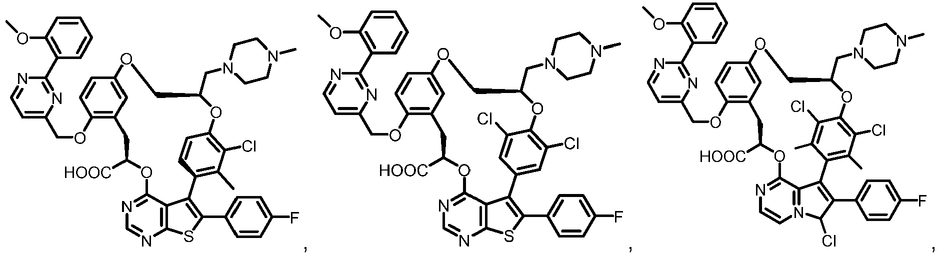 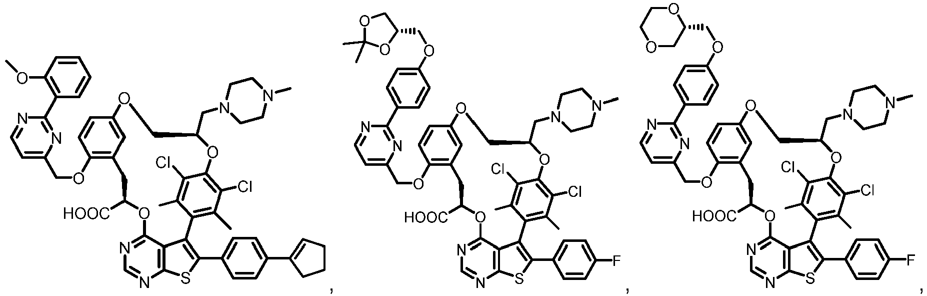 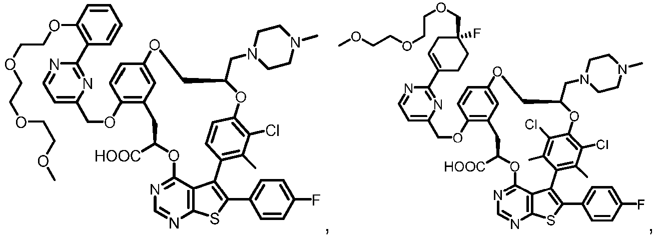 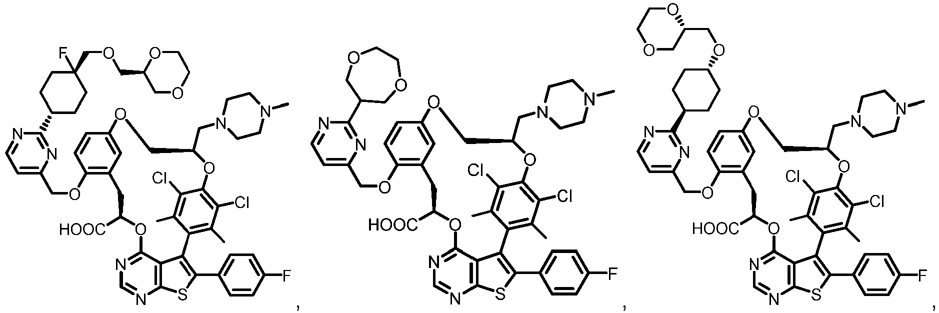 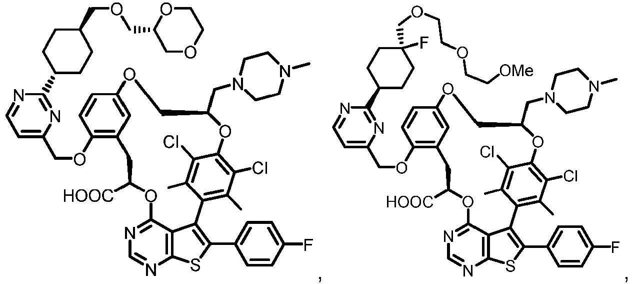 or

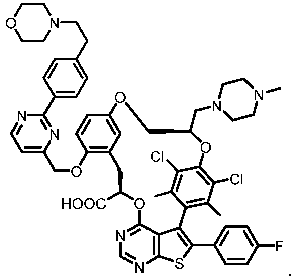

In some embodiments, the linker-drug (or "linker-payload") moiety -(L-D) may comprise a compounds in Table A or an enantiomer, diastereoisomer, atropisomer, deuterated derivative, and/or a pharmaceutically acceptable salt of any of the foregoing.

### Drug Loading

Drug loading is represented by *p,* and is also referred to herein as the drug-to-antibody ratio (DAR). Drug loading may range from 1 to 16 drug moieties per antibody or antigen-binding fragment. In some embodiments, *p* is an integer from 1 to 16. In some embodiments, *p* is an integer from 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, or 1 to 2. In some embodiments, *p* is an integer from 2 to 10, 2 to 9, 2 to 8, 2 to 7, 2 to 6, 2 to 5, 2 to 4, or 2 to 3. In some embodiments, *p* is an integer from 1 to 16. In some embodiments, *p* is an integer from 1 to 8. In some embodiments, *p* is an integer from 1 to 5. In some embodiments, *p* is an integer from 2 to 4. In some embodiments, *p* is 1, 2, 3, 4, 5, 6, 7, or 8. In some embodiments, *p* is 2. In some embodiments, *p* is 4.

Drug loading may be limited by the number of attachment sites on the antibody or antigen-binding fragment. In some embodiments, the linker moiety (L) of the ADC attaches to the antibody or antigen-binding fragment through a chemically active group on one or more amino acid residues on the antibody or antigen-binding fragment. For example, the linker may be attached to the antibody or antigen-binding fragment via a free amino, imino, hydroxyl, thiol, or carboxyl group (e.g., to the N- or C-terminus, to the epsilon amino group of one or more lysine residues, to the free carboxylic acid group of one or more glutamic acid or aspartic acid residues, or to the sulfhydryl group of one or more cysteine residues). The site to which the linker is attached can be a natural residue in the amino acid sequence of the antibody or antigen-binding fragment, or it can be introduced into the antibody or antigen-binding fragment, e.g., by DNA recombinant technology (e.g., by introducing a cysteine residue into the amino acid sequence) or by protein biochemistry (e.g., by reduction, pH adjustment, or hydrolysis).

In some embodiments, the number of drug moieties that can be conjugated to an antibody or antigen-binding fragment is limited by the number of free cysteine residues. For example, where the attachment is a cysteine thiol group, an antibody may have only one or a few cysteine thiol groups, or may have only one or a few sufficiently reactive thiol groups through which a linker may be attached. Generally, antibodies do not contain many free and reactive cysteine thiol groups that may be linked to a drug moiety. Indeed, most cysteine thiol residues in antibodies are involved in either interchain or intrachain disulfide bonds. Conjugation to cysteines can therefore, in some embodiments, require at least partial reduction of the antibody. Over-attachment of linker-toxin to an antibody may destabilize the antibody by reducing the cysteine residues available to form disulfide bonds. Therefore, an optimal drug:antibody ratio should increase potency of the ADC (by increasing the number of attached drug moieties per antibody) without destabilizing the antibody or antigen-binding fragment. In some embodiments, an optimal ratio may be 2, 4, 6, or 8. In some embodiments, an optimal ratio may be 2 or 4.

In some embodiments, an antibody or antigen-binding fragment is exposed to reducing conditions prior to conjugation in order to generate one or more free cysteine residues. An antibody, in some embodiments, may be reduced with a reducing agent such as dithiothreitol (DTT) or tris(2-carboxyethyl)phosphine (TCEP), under partial or total reducing conditions, to generate reactive cysteine thiol groups. Unpaired cysteines may be generated through partial reduction with limited molar equivalents of TCEP, which can reduce the interchain disulfide bonds which link the light chain and heavy chain (one pair per H-L pairing) and the two heavy chains in the hinge region (two pairs per H-H pairing in the case of human IgG1) while leaving the intrachain disulfide bonds intact (Stefano et al. (2013) Methods Mol Biol. 1045:145-71). In embodiments, disulfide bonds within the antibodies are reduced electrochemically, e.g., by employing a working electrode that applies an alternating reducing and oxidizing voltage. This approach can allow for on-line coupling of disulfide bond reduction to an analytical device (e.g., an electrochemical detection device, an NMR spectrometer, or a mass spectrometer) or a chemical separation device (e.g., a liquid chromatograph (e.g., an HPLC) or an electrophoresis device (see, e.g., US 2014/0069822)). In some embodiments, an antibody is subjected to denaturing conditions to reveal reactive nucleophilic groups on amino acid residues, such as cysteine.

The drug loading of an ADC may be controlled in different ways, e.g., by: (i) limiting the molar excess of drug-linker intermediate or linker reagent relative to antibody; (ii) limiting the conjugation reaction time or temperature; (iii) partial or limiting reductive conditions for cysteine thiol modification; and/or (iv) engineering by recombinant techniques the amino acid sequence of the antibody such that the number and position of cysteine residues is modified for control of the number and/or position of linker-drug attachments.

In some embodiments, free cysteine residues are introduced into the amino acid sequence of the antibody or antigen-binding fragment. For example, cysteine engineered antibodies can be prepared wherein one or more amino acids of a parent antibody are replaced with a cysteine amino acid. Any form of antibody may be so engineered, i.e. mutated. For example, a parent Fab antibody fragment may be engineered to form a cysteine engineered Fab referred to as a "ThioFab." Similarly, a parent monoclonal antibody may be engineered to form a "ThioMab." A single site mutation yields a single engineered cysteine residue in a ThioFab, whereas a single site mutation yields two engineered cysteine residues in a ThioMab, due to the dimeric nature of the IgG antibody. DNA encoding an amino acid sequence variant of the parent polypeptide can be prepared by a variety of methods known in the art (see, e.g., the methods described in WO 2006/034488). These methods include, but are not limited to, preparation by site-directed (or oligonucleotide-mediated) mutagenesis, PCR mutagenesis, and cassette mutagenesis of an earlier prepared DNA encoding the polypeptide. Variants of recombinant antibodies may also be constructed by restriction fragment manipulation or by overlap extension PCR with synthetic oligonucleotides. ADCs of Formula (1) include, but are not limited to, antibodies that have 1, 2, 3, or 4 engineered cysteine amino acids (Lyon et al. (2012) Methods Enzymol. 502:123-38). In some embodiments, one or more free cysteine residues are already present in an antibody or antigen-binding fragment, without the use of engineering, in which case the existing free cysteine residues may be used to conjugate the antibody or antigen-binding fragment to a drug moiety.

Where more than one nucleophilic group reacts with a drug-linker intermediate or a linker moiety reagent followed by drug moiety reagent, in a reaction mixture comprising multiple copies of the antibody or antigen-binding fragment and linker moiety, then the resulting product can be a mixture of ADC compounds with a distribution of one or more drug moieties attached to each copy of the antibody or antigen-binding fragment in the mixture. In some embodiments, the drug loading in a mixture of ADCs resulting from a conjugation reaction ranges from 1 to 16 drug moieties attached per antibody or antigen-binding fragment. The average number of drug moieties per antibody or antigen-binding fragment (i.e., the average drug loading, or average p) may be calculated by any conventional method known in the art, e.g., by mass spectrometry (e.g., liquid chromatography-mass spectrometry (LC-MS)) and/or high-performance liquid chromatography (e.g., HIC-HPLC). In some embodiments, the average number of drug moieties per antibody or antigen-binding fragment is determined by liquid chromatography-mass spectrometry (LC-MS). In some embodiments, the average number of drug moieties per antibody or antigen-binding fragment is from about 1.5 to about 3.5, about 2.5 to about 4.5, about 3.5 to about 5.5, about 4.5 to about 6.5, about 5.5 to about 7.5, about 6.5 to about 8.5, or about 7.5 to about 9.5. In some embodiments, the average number of drug moieties per antibody or antigen-binding fragment is from about 2 to about 4, about 3 to about 5, about 4 to about 6, about 5 to about 7, about 6 to about 8, about 7 to about 9, about 2 to about 8, or about 4 to about 8.

In some embodiments, the average number of drug moieties per antibody or antigen-binding fragment is about 2. In some embodiments, the average number of drug moieties per antibody or antigen-binding fragment is about 1.5, about 1.6, about 1.7, about 1.8, about 1.9, about 2, about 2.1, about 2.2, about 2.3, about 2.4, or about 2.5. In some embodiments, the average number of drug moieties per antibody or antigen-binding fragment is 2.

In some embodiments, the average number of drug moieties per antibody or antigen-binding fragment is about 4. In some embodiments, the average number of drug moieties per antibody or antigen-binding fragment is about 3.5, about 3.6, about 3.7, about 3.8, about 3.9, about 4, about 4.1, about 4.2, about 4.3, about 4.4, or about 4.5. In some embodiments, the average number of drug moieties per antibody or antigen-binding fragment is 4.

In some embodiments, the term "about," as used with respect to the average number of drug moieties per antibody or antigen-binding fragment, means plus or minus 20%, 15%, 10%, 5%, or 1%. In one embodiment, the term "about" refers to a range of values which are 10% more or less than the specified value. In another embodiment, the term "about" refers to a range of values which are 5% more or less than the specified value. In another embodiment, the term "about" refers to a range of values which are 1% more or less than the specified value.

Individual ADC compounds, or "species," may be identified in the mixture by mass spectroscopy and separated by, e.g., UPLC or HPLC, e.g. hydrophobic interaction chromatography (HIC-HPLC). In some embodiments, a homogeneous or nearly homogenous ADC product with a single loading value may be isolated from the conjugation mixture, e.g., by electrophoresis or chromatography.

In some embodiments, higher drug loading (e.g., p > 16) may cause aggregation, insolubility, toxicity, or loss of cellular permeability of certain antibody-drug conjugates. Higher drug loading may also negatively affect the pharmacokinetics (e.g., clearance) of certain ADCs. In some embodiments, lower drug loading (e.g., p < 2) may reduce the potency of certain ADCs against target-expressing cells. In some embodiments, the drug loading for an ADC of the present disclosure ranges from about 2 to about 16, about 2 to about 10, about 2 to about 8; from about 2 to about 6; from about 2 to about 5; from about 3 to about 5; from about 2 to about 4; or from about 4 to about 8.

In some embodiments, a drug loading and/or an average drug loading of about 2 is achieved, e.g., using partial reduction of intrachain disulfides on the antibody or antigen-binding fragment, and provides beneficial properties. In some embodiments, a drug loading and/or an average drug loading of about 4 or about 6 or about 8 is achieved, e.g., using partial reduction of intrachain disulfides on the antibody or antigen-binding fragment, and provides beneficial properties. In some embodiments, a drug loading and/or an average drug loading of less than about 2 may result in an unacceptably high level of unconjugated antibody species, which can compete with the ADC for binding to a target antigen and/or provide for reduced treatment efficacy. In some embodiments, a drug loading and/or average drug loading of more than about 16 may result in an unacceptably high level of product heterogeneity and/or ADC aggregation. A drug loading and/or an average drug loading of more than about 16 may also affect stability of the ADC, due to loss of one or more chemical bonds required to stabilize the antibody or antigen-binding fragment.

The present disclosure includes methods of producing the described ADCs. Briefly, the ADCs comprise an antibody or antigen-binding fragment as the antibody or antigen-binding fragment, a drug moiety (e.g., an Mcl-1 inhibitor), and a linker that joins the drug moiety and the antibody or antigen-binding fragment. In some embodiments, the ADCs can be prepared using a linker having reactive functionalities for covalently attaching to the drug moiety and to the antibody or antigen-binding fragment. In some embodiments, the antibody or antigen-binding fragment is functionalized to prepare a functional group that is reactive with a linker or a drug-linker intermediate. For example, in some embodiments, a cysteine thiol of an antibody or antigen-binding fragment can form a bond with a reactive functional group of a linker or a drug-linker intermediate to make an ADC. In some embodiments, an antibody or antigen-binding fragment is prepared with bacterial transglutaminase (BTG) - reactive glutamines specifically functionalized with an amine containing cyclooctyne BCN (N-[(1*R*,8*S*,9*s*)-Bicyclo[6.1.0]non-4-yn-9-ylmethyloxycarbonyl]-1,8-diamino-3,6-dioxaoctane) moiety. In some embodiments, site-specific conjugation of a linker or a drug-linker intermediate to a BCN moiety of an antibody or antigen-binding fragment is performed, e.g., as described and exemplified herein. The generation of the ADCs can be accomplished by techniques known to the skilled artisan.

In some embodiments, an ADC is produced by contacting an antibody or antigen-binding fragment with a linker and a drug moiety (e.g., an Mcl-1 inhibitor) in a sequential manner, such that the antibody or antigen-binding fragment is covalently linked to the linker first, and then the pre-formed antibody-linker intermediate reacts with the drug moiety. The antibody-linker intermediate may or may not be subjected to a purification step prior to contacting the drug moiety. In other embodiments, an ADC is produced by contacting an antibody or antigen-binding fragment with a linker-drug compound pre-formed by reacting a linker with a drug moiety. The pre-formed linker-drug compound may or may not be subjected to a purification step prior to contacting the antibody or antigen-binding fragment. In other embodiments, the antibody or antigen-binding fragment contacts the linker and the drug moiety in one reaction mixture, allowing simultaneous formation of the covalent bonds between the antibody or antigen-binding fragment and the linker, and between the linker and the drug moiety. This method of producing ADCs may include a reaction, wherein the antibody or antigen-binding fragment contacts the antibody or antigen-binding fragment prior to the addition of the linker to the reaction mixture, and vice versa. In some embodiments, an ADC is produced by reacting an antibody or antigen-binding fragment with a linker joined to a drug moiety, such as an Mcl-1 inhibitor, under conditions that allow conjugation.

The ADCs prepared according to the methods described above may be subjected to a purification step. The purification step may involve any biochemical methods known in the art for purifying proteins, or any combination of methods thereof. These include, but are not limited to, tangential flow filtration (TFF), affinity chromatography, ion exchange chromatography, any charge or isoelectric point-based chromatography, mixed mode chromatography, e.g., CHT (ceramic hydroxyapatite), hydrophobic interaction chromatography, size exclusion chromatography, dialysis, filtration, selective precipitation, or any combination thereof.

### Therapeutic Uses and Compositions

Disclosed herein are methods of using the compositions described herein, e.g., the disclosed ADC compounds and compositions, in treating a subject for a disorder, e.g., a cancer. Compositions, e.g., ADCs, may be administered alone or in combination with at least one additional inactive and/or active agent, e.g., at least one additional therapeutic agent, and may be administered in any pharmaceutically acceptable formulation, dosage, and dosing regimen. Treatment efficacy may be evaluated for toxicity as well as indicators of efficacy and adjusted accordingly. Efficacy measures include, but are not limited to, a cytostatic and/or cytotoxic effect observed in vitro or in vivo, reduced tumor volume, tumor growth inhibition, and/or prolonged survival.

Methods of determining whether an ADC exerts a cytostatic and/or cytotoxic effect on a cell are known. For example, the cytotoxic or cytostatic activity of an ADC can be measured by, e.g., exposing mammalian cells expressing a target antigen of the ADC in a cell culture medium; culturing the cells for a period from about 6 hours to about 6 days; and measuring cell viability (e.g., using a CellTiter-Glo^{®} (CTG) or MTT cell viability assay). Cell-based in vitro assays may also be used to measure viability (proliferation), cytotoxicity, and induction of apoptosis (caspase activation) of the ADC.

For determining cytotoxicity, necrosis or apoptosis (programmed cell death) may be measured. Necrosis is typically accompanied by increased permeability of the plasma membrane, swelling of the cell, and rupture of the plasma membrane. Apoptosis can be quantitated, for example, by measuring DNA fragmentation. Commercial photometric methods for the quantitative in vitro determination of DNA fragmentation are available. Examples of such assays, including TUNEL (which detects incorporation of labeled nucleotides in fragmented DNA) and ELISA-based assays, are described in Biochemica (1999) 2:34-7 (Roche Molecular Biochemicals).

Apoptosis may also be determined by measuring morphological changes in a cell. For example, as with necrosis, loss of plasma membrane integrity can be determined by measuring uptake of certain dyes (e.g., a fluorescent dye such as, for example, acridine orange or ethidium bromide). A method for measuring apoptotic cell number has been described by Duke and Cohen, Current Protocols in Immunology (Coligan et al., eds. (1992) pp. 3.17.1-3.17.16). Cells also can be labeled with a DNA dye (e.g., acridine orange, ethidium bromide, or propidium iodide) and the cells observed for chromatin condensation and margination along the inner nuclear membrane. Apoptosis may also be determined, in some embodiments, by screening for caspase activity. In some embodiments, a Caspase-Glo^{®} Assay can be used to measure activity of caspase-3 and caspase-7. In some embodiments, the assay provides a luminogenic caspase-3/7 substrate in a reagent optimized for caspase activity, luciferase activity, and cell lysis. In some embodiments, adding Caspase-Glo^{®} 3/7 Reagent in an "add-mix-measure" format may result in cell lysis, followed by caspase cleavage of the substrate and generation of a "glow-type" luminescent signal, produced by luciferase. In some embodiments, luminescence may be proportional to the amount of caspase activity present, and can serve as an indicator of apoptosis. Other morphological changes that can be measured to determine apoptosis include, e.g., cytoplasmic condensation, increased membrane blebbing, and cellular shrinkage. Determination of any of these effects on cancer cells indicates that an ADC is useful in the treatment of cancers.

Cell viability may be measured, e.g., by determining in a cell the uptake of a dye such as neutral red, trypan blue, Crystal Violet, or ALAMAR^{™} blue (see, e.g., Page et al. (1993) Intl J Oncology 3:473-6). In such an assay, the cells are incubated in media containing the dye, the cells are washed, and the remaining dye, reflecting cellular uptake of the dye, is measured spectrophotometrically.

Cell viability may also be measured, e.g., by quantifying ATP, an indicator of metabolically active cells. In some embodiments, in vitro potency and/or cell viability of prepared ADCs or Mcl-1 inhibitor compounds may be assessed using a CellTiter-Glo^{®} (CTG) cell viability assay, as described in the examples provided herein. In this assay, in some embodiments, the single reagent (CellTiter-Glo^{®} Reagent) is added directly to cells cultured in serum-supplemented medium. The addition of reagent results in cell lysis and generation of a luminescent signal proportional to the amount of ATP present. The amount of ATP is directly proportional to the number of cells present in culture

Cell viability may also be measured, e.g., by measuring the reduction of tetrazolium salts. In some embodiments, in vitro potency and/or cell viability of prepared ADCs or Mcl-1 inhibitor compounds may be assessed using an MTT cell viability assay, as described in the examples provided herein. In this assay, in some embodiments, the yellow tetrazolium MTT (3-(4, 5-dimethylthiazolyl-2)-2,5-diphenyltetrazolium bromide) is reduced by metabolically active cells, in part by the action of dehydrogenase enzymes, to generate reducing equivalents such as NADH and NADPH. The resulting intracellular purple formazan can then be solubilized and quantified by spectrophotometric means.

In certain aspects, the present disclosure features a method of killing, inhibiting or modulating the growth of a cancer cell or tissue by disrupting the expression and/or activity of Mcl-1 and/or one or more upstream modulators or downstream targets thereof. The method may be used with any subject where disruption of Mcl-1 expression and/or activity provides a therapeutic benefit. Subjects that may benefit from disrupting Mcl-1 expression and/or activity include, but are not limited to, those having or at risk of having a cancer such as a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

In some embodiments, the disclosed ADCs may be administered in any cell or tissue that expresses BCMA, such as a BCMA-expressing cancer cell or tissue. An exemplary embodiment includes a method of killing a BCMA-expressing cancer cell or tissue. The method may be used with any cell or tissue that expresses BCMA, such as a cancerous cell or a metastatic lesion. Non-limiting examples of BCMA-expressing cancers include multiple myeloma (Cho et al. (2018) Front Immunol. 9:1821). Non-limiting examples of BCMA-expressing cells include NCI-H929 multiple myeloma cells, and cells comprising a recombinant nucleic acid encoding BCMA or a portion thereof.

In some embodiments, the disclosed ADCs may be administered in any cell or tissue that expresses CD33, such as a CD33-expressing cancer cell or tissue. An exemplary embodiment includes a method of killing a CD33-expressing cancer cell or tissue. The method may be used with any cell or tissue that expresses CD33, such as a cancerous cell or a metastatic lesion. Non-limiting examples of CD33-expressing cancers include colorectal cancer, pancreatic cancer, lymphoma, and leukemia (e.g., acute myeloid leukemia) (Human Protein Atlas; Walter (2014) Expert Opin Ther Targets 18(7):715-8). Non-limiting examples of CD33-expressing cells include MOLM-13 leukemia cells, and cells comprising a recombinant nucleic acid encoding CD33 or a portion thereof.

In some embodiments, the disclosed ADCs may be administered in any cell or tissue that expresses PCAD, such as a PCAD-expressing cancer cell or tissue. An exemplary embodiment includes a method of killing a PCAD-expressing cancer cell or tissue. The method may be used with any cell or tissue that expresses PCAD, such as a cancerous cell or a metastatic lesion. Non-limiting examples of PCAD-expressing cancers include breast cancer, gastric cancer, endometrial cancer, ovarian cancer, pancreatic cancer, bladder cancer, prostate cancer, and melanoma (Vieira and Paredes (2015) Mol Cancer 14:178).

In some embodiments, the disclosed ADCs may be administered in any cell or tissue that expresses HER2, such as a HER2-expressing cancer cell or tissue. An exemplary embodiment includes a method of killing a HER2-expressing cancer cell or tissue. The method may be used with any cell or tissue that expresses HER2, such as a cancerous cell or a metastatic lesion. Non-limiting examples of HER2-expressing cancers include breast cancer, gastric cancer, bladder cancer, urothelial cell carcinoma, esophageal cancer, lung cancer (e.g., lung adenocarcinoma), uterine cancer (e.g., uterine serous endometrial carcinoma), salivary duct carcinoma, cervical cancer, endometrial cancer, and ovarian cancer (English et al. (2013) Mol Diagn Ther. 17:85-99). Non-limiting examples of HER2-expressing cells include HCC1954 and HCC2218 breast cancer cells, and cells comprising a recombinant nucleic acid encoding HER2 or a portion thereof.

Exemplary methods include the steps of contacting a cell with an ADC, as described herein, in an effective amount, i.e., an amount sufficient to kill the cell. The method can be used on cells in culture, e.g., in vitro, in vivo, ex vivo, or in situ. For example, cells that express HER2 (e.g., cells collected by biopsy of a tumor or metastatic lesion; cells from an established cancer cell line; or recombinant cells), can be cultured in vitro in culture medium and the contacting step can be affected by adding the ADC to the culture medium. The method will result in killing of cells expressing HER2, including in particular cancer cells expressing HER2. Alternatively, the ADC can be administered to a subject by any suitable administration route (e.g., intravenous, subcutaneous, or direct contact with a tumor tissue) to have an effect in vivo. This approach can be used for antibodies targeting other cell surface antigens (e.g., BCMA, CD33, PCAD).

The in vivo effect of a disclosed ADC therapeutic composition can be evaluated in a suitable animal model. For example, xenogeneic cancer models can be used, wherein cancer explants or passaged xenograft tissues are introduced into immune compromised animals, such as nude or SCID mice (Klein et al. (1997) Nature Med. 3:402-8). Efficacy may be predicted using assays that measure inhibition of tumor formation, tumor regression or metastasis, and the like.

In vivo assays that evaluate the promotion of tumor death by mechanisms such as apoptosis may also be used. In some embodiments, xenografts from tumor bearing mice treated with the therapeutic composition can be examined for the presence of apoptotic foci and compared to untreated control xenograft-bearing mice. The extent to which apoptotic foci are found in the tumors of the treated mice provides an indication of the therapeutic efficacy of the composition.

Further provided herein are methods of treating a disorder, e.g., a cancer. The compositions described herein, e.g., the ADCs disclosed herein, can be administered to a non-human mammal or human subject for therapeutic purposes. The therapeutic methods include administering to a subject having or suspected of having a cancer a therapeutically effective amount of a composition comprising an Mcl-1 inhibitor, e.g., an ADC where the inhibitor is linked to a targeting antibody that binds to an antigen (1) expressed on a cancer cell, (2) is accessible to binding, and/or (3) is localized or predominantly expressed on a cancer cell surface as compared to a non-cancer cell.

An exemplary embodiment is a method of treating a subject having or suspected of having a cancer, comprising administering to the subject a therapeutically effective amount of a composition disclosed herein, e.g., an ADC, composition, or pharmaceutical composition (e.g., any of the exemplary ADCs, compositions, or pharmaceutical compositions disclosed herein). In some embodiments, the cancer expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the cancer is a tumor or a hematological cancer. In some embodiments, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer is a lymphoma or gastric cancer.

Another exemplary embodiment is a method of delivering an Mcl-1 inhibitor to a cell expressing BCMA, comprising conjugating the Mcl-1 inhibitor to an antibody that immunospecifically binds to a BCMA epitope and exposing the cell to the ADC. Exemplary cancer cells that express BCMA for which the ADCs of the present disclosure are indicated include multiple myeloma cells.

Another exemplary embodiment is a method of delivering an Mcl-1 inhibitor to a cell expressing CD33, comprising conjugating the Mcl-1 inhibitor to an antibody that immunospecifically binds to a CD33 epitope and exposing the cell to the ADC. Exemplary cancer cells that express CD33 for which the ADCs of the present disclosure are indicated include leukemia cells.

Another exemplary embodiment is a method of delivering an Mcl-1 inhibitor to a cell expressing PCAD, comprising conjugating the Mcl-1 inhibitor to an antibody that immunospecifically binds to a PCAD epitope and exposing the cell to the ADC. Exemplary cancer cells that express PCAD for which the ADCs of the present disclosure are indicated include breast cancer and gastric cancer cells.

Another exemplary embodiment is a method of delivering an Mcl-1 inhibitor to a cell expressing HER2, comprising conjugating the Mcl-1 inhibitor to an antibody that immunospecifically binds to a HER2 epitope and exposing the cell to the ADC. Exemplary cancer cells that express HER2 for which the ADCs of the present disclosure are indicated include breast cancer cells.

In certain aspects, the present disclosure further provides methods of reducing or inhibiting growth of a tumor (e.g., a BCMA-expressing tumor, a CD33-expressing tumor, a PCAD-expressing tumor, an HER2-expressing tumor), comprising administering a therapeutically effective amount of an ADC or composition comprising an ADC. In some embodiments, the treatment is sufficient to reduce or inhibit the growth of the patient's tumor, reduce the number or size of metastatic lesions, reduce tumor load, reduce primary tumor load, reduce invasiveness, prolong survival time, and/or maintain or improve the quality of life. In some embodiments, the tumor is resistant or refractory to treatment with the antibody or antigen-binding fragment of the ADC (e.g., an anti-BCMA antibody, an anti-CD33 antibody, an anti-PCAD antibody, an anti-HER2 antibody) when administered alone, and/or the tumor is resistant or refractory to treatment with the Mcl-1 inhibitor drug moiety when administered alone.

An exemplary embodiment is a method of reducing or inhibiting the growth of a tumor in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the tumor expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the ADC, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%, as compared to growth in the absence of treatment.

Another exemplary embodiment is a method of delaying or slowing the growth of a tumor in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the tumor expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer. In some embodiments, the tumor is a gastric cancer. In some embodiments, administration of the ADC, composition, or pharmaceutical composition delays or slows the growth of the tumor by at least about 10%, at least about 20%, at least about 30%, at least about 40%, at least about 50%, at least about 60%, at least about 70%, at least about 80%, at least about 90%, at least about 95%, or at least about 99%, as compared to growth in the absence of treatment.

In certain aspects, the present disclosure further provides methods of reducing or slowing the expansion of a cancer cell population (e.g., a BCMA-expressing cancer cell population, a CD33-expressing cancer cell population, a PCAD-expressing cancer cell population, a HER2-expressing cancer cell population), comprising administering a therapeutically effective amount of an ADC or composition comprising an ADC.

An exemplary embodiment is a method of reducing or slowing the expansion of a cancer cell population in a subject, comprising administering to the subject a therapeutically effective amount of an ADC, composition, or pharmaceutical composition (e.g., any of the exemplary ADCs, compositions, or pharmaceutical compositions disclosed herein). In some embodiments, the cancer cell population expresses a target antigen. In some embodiments, the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB. In some embodiments, the target antigen is 4-1BB, 5AC, 5T4, Alpha-fetoprotein, angiopoietin 2, ASLG659, TCLI, BMPRIB, Brevican BCAN, BEHAB, C242 antigen, C5, CA-125, CA-125 (imitation), CA-IX (Carbonic anhydrase 9), CCR4, CD140a, CD152, CD19, CD20, CD200, CD21 (C3DR) I), CD22 (B-cell receptor CD22-B isoform), CD221, CD23 (gE receptor), CD28, CD30 (TNFRSF8), CD37, CD4, CD40, CD44 v6, CD51, CD52, CD70, CD72 (Lyb-2, B-cell differentiation antigen CD72), CD79a, CD80, CEA, CEA-related antigen, ch4D5, CLDN18.2, CRIPTO (CR, CRI, CRGF, TDGF1), CTLA-4, CXCR5, DLL4, DR5, E16 (LATI, SLC7A5), EGFL7, EphB2R (DRT, ERK, Hek5, EPHT3, Tyro5), Episialin, ERBB3, ETBR (Endothelin type B receptor), FCRHI (Fc receptor-like protein I), FcRH2 (IFGP4, IRTA4, SPAPI, SPAP IB, SPAP IC), Fibronectin extra domain-B, Frizzled receptor, GD2, GD3 ganglioside, GEDA, HER1, HER2/neu, HER3, HGF, HLA-DOB, HLA-DR, Human scatter factor receptor kinase, IGF-I receptor, IL-13, IL20R (ZCYTOR7), IL-6, ILGF2, ILFRIR, integrin u, IRTA2 (Immunoglobulin superfamily receptor translocation associated 2), Lewis-Y antigen, LY64 (RP105), MCP-I, MDP (DPEPI), MPF, MSLN, SMR, mesothelin, megakaryocyte, PD-I, PDCDI, PDGF-R u, Prostate specific membrane antigen, PSCA (Prostate stem cell antigen precursor), PSCA hlg, RANKL, RON, SDCI, Sema Sb, STEAP I, STEAP2, PCANAP I, STAMP I, STEAP2, STMP, prostate cancer associated gene I, TAG-72, TEMI, Tenascin C, TENB2, (TMEFF2, tomoregulin, TPEF, HPPI, TR), TGF-IJ, TRAIL-E2, TRAIL-RI, TRAIL-R2, T17M4 (BR22450, FLJ20041, TRPM4, TRPM4B, transient receptor potential cation channel subfamily M, member 4), TWEAK-R, TYRP I (glycoprotein 75), VEGF, VEGF-A, EGFR-I, VEGFR-2, or Vimentin. In some embodiments, the target antigen is BCMA, CD33, PCAD, HER2, CD38, CD46, CD48, or CD79b. In some embodiments, the target antigen is BCMA, CD33, CD48, PCAD, or HER2. In some embodiments, the target antigen is CD38 or CD48. In some embodiments, the cancer cell population is from a tumor or a hematological cancer. In some embodiments, the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer. In some embodiments, the cancer cell population is from a lymphoma or gastric cancer. In some embodiments, administration of the ADC, composition, or pharmaceutical composition reduces the cancer cell population by at least about 10%, at least about 20%, at least about 30%, at least about 40%, at least about 50%, at least about 60%, at least about 70%, at least about 80%, at least about 90%, at least about 95%, or at least about 99%, as compared to the population in the absence of treatment. In some embodiments, administration of the ADC, composition, or pharmaceutical composition slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%, as compared to expansion in the absence of treatment.

Also provided herein are methods of determining whether a subject having or suspected of having a cancer will be responsive to treatment with the disclosed ADCs and compositions. An exemplary embodiment is a method of determining whether a subject having or suspected of having a cancer will be responsive to treatment with an ADC, composition, or pharmaceutical composition (e.g., any of the exemplary ADCs, compositions, or pharmaceutical compositions disclosed herein) by providing a biological sample from the subject; contacting the sample with the ADC; and detecting binding of the ADC to cancer cells in the sample. In some embodiments, the sample is a tissue biopsy sample, a blood sample, or a bone marrow sample. In some embodiments, the method comprises providing a biological sample from the subject; contacting the sample with the ADC; and detecting one or more markers of cancer cell death in the sample (e.g., increased expression of one or more apoptotic markers, reduced expansion of a cancer cell population in culture, etc.).

Further provided herein are therapeutic uses of the disclosed ADCs and compositions. An exemplary embodiment is an ADC, composition, or pharmaceutical composition (e.g., any of the exemplary ADCs, compositions, or pharmaceutical compositions disclosed herein) for use in treating a subject having or suspected of having a cancer (e.g., a BCMA-expressing cancer, a CD33-expressing cancer, a PCAD-expressing cancer, a HER2-expressing cancer). Another exemplary embodiment is a use of an ADC, composition, or pharmaceutical composition (e.g., any of the exemplary ADCs, compositions, or pharmaceutical compositions disclosed herein) in treating a subject having or suspected of having a cancer (e.g., a BCMA-expressing cancer, a CD33-expressing cancer, a PCAD-expressing cancer, a HER2-expressing cancer). Another exemplary embodiment is a use of an ADC, composition, or pharmaceutical composition (e.g., any of the exemplary ADCs, compositions, or pharmaceutical compositions disclosed herein) in a method of manufacturing a medicament for treating a subject having or suspected of having a cancer (e.g., a BCMA-expressing cancer, a CD33-expressing cancer, a PCAD-expressing cancer, a HER2-expressing cancer). Methods for identifying subjects having cancers that express a target antigen (e.g., BCMA, CD33, PCAD, HER2) are known in the art and may be used to identify suitable patients for treatment with a disclosed ADC compound or composition.

Moreover, ADCs of the present disclosure may be administered to a non-human mammal expressing an antigen with which the ADC is capable of binding for veterinary purposes or as an animal model of human disease. Regarding the latter, such animal models may be useful for evaluating the therapeutic efficacy of the disclosed ADCs (e.g., testing of dosages and time courses of administration).

The therapeutic compositions used in the practice of the foregoing methods may be formulated into pharmaceutical compositions comprising a pharmaceutically acceptable carrier suitable for the desired delivery method. An exemplary embodiment is a pharmaceutical composition comprising an ADC of the present disclosure and a pharmaceutically acceptable carrier, e.g., one suitable for a chosen means of administration, e.g., intravenous administration. The pharmaceutical composition may also comprise one or more additional inactive and/or therapeutic agents that are suitable for treating or preventing, for example, a cancer (e.g., a standard-of-care agent, etc.). The pharmaceutical composition may also comprise one or more carrier, excipient, and/or stabilizer components, and the like. Methods of formulating such pharmaceutical compositions and suitable formulations are known in the art (see, e.g., "Remington's Pharmaceutical Sciences," Mack Publishing Co., Easton, PA).

Suitable carriers include any material that, when combined with the therapeutic composition, retains the anti-tumor function of the therapeutic composition and is generally non-reactive with the patient's immune system. Pharmaceutically acceptable carriers include any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol, mesylate salt, and the like, as well as combinations thereof. In many cases, isotonic agents are included, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the ADC.

A pharmaceutical composition of the present disclosure can be administered by a variety of methods known in the art. The route and/or mode of administration may vary depending upon the desired results. In some embodiments, the therapeutic formulation is solubilized and administered via any route capable of delivering the therapeutic composition to the cancer site. Potentially effective routes of administration include, but are not limited to, parenteral (e.g., intravenous, subcutaneous), intraperitoneal, intramuscular, intratumor, intradermal, intraorgan, orthotopic, and the like. In some embodiments, the administration is intravenous, subcutaneous, intraperitoneal, or intramuscular. The pharmaceutically acceptable carrier should be suitable for the route of administration, e.g., intravenous or subcutaneous administration (e.g., by injection or infusion). Depending on the route of administration, the active compound(s), i.e., the ADC and/or any additional therapeutic agent, may be coated in a material to protect the compound(s) from the action of acids and other natural conditions that may inactivate the compound(s). Administration can be either systemic or local.

The therapeutic compositions disclosed herein may be sterile and stable under the conditions of manufacture and storage, and may be in a variety of forms. These include, for example, liquid, semi-solid, and solid dosage forms, such as liquid solutions (e.g., injectable and infusible solutions), dispersions or suspensions, tablets, pills, powders, liposomes, and suppositories. The form depends on the intended mode of administration and therapeutic application. In some embodiments, the disclosed ADCs can be incorporated into a pharmaceutical composition suitable for parenteral administration. The injectable solution may be composed of either a liquid or lyophilized dosage form in a flint or amber vial, ampule, or pre-filled syringe, or other known delivery or storage device. In some embodiments, one or more of the ADCs or pharmaceutical compositions is supplied as a dry sterilized lyophilized powder or water free concentrate in a hermetically sealed container and can be reconstituted (e.g., with water or saline) to the appropriate concentration for administration to a subject.

Typically, a therapeutically effective amount or efficacious amount of a disclosed composition, e.g., a disclosed ADC, is employed in the pharmaceutical compositions of the present disclosure. The composition, e.g., one comprising an ADC, may be formulated into a pharmaceutically acceptable dosage form by conventional methods known in the art. Dosages and administration protocols for the treatment of cancers using the foregoing methods will vary with the method and the target cancer, and will generally depend on a number of other factors appreciated in the art.

Dosage regimens for compositions disclosed herein, e.g., those comprising ADCs alone or in combination with at least one additional inactive and/or active therapeutic agent, may be adjusted to provide the optimum desired response (e.g., a therapeutic response). For example, a single bolus of one or both agents may be administered at one time, several divided doses may be administered over a predetermined period of time, or the dose of one or both agents may be proportionally increased or decreased as indicated by the exigencies of the therapeutic situation. In some embodiments, treatment involves single bolus or repeated administration of the ADC preparation via an acceptable route of administration. In some embodiments, the ADC is administered to the patient daily, weekly, monthly, or any time period in between. For any particular subject, specific dosage regimens may be adjusted over time according to the individual's need, and the professional judgment of the treating clinician. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Dosage values for compositions comprising an ADC and/or any additional therapeutic agent(s), may be selected based on the unique characteristics of the active compound(s), and the particular therapeutic effect to be achieved. A physician or veterinarian can start doses of the ADC employed in the pharmaceutical composition at levels lower than that required to achieve the desired therapeutic effect and gradually increase the dosage until the desired effect is achieved. In general, effective doses of the compositions of the present disclosure, for the treatment of a cancer may vary depending upon many different factors, including means of administration, target site, physiological state of the patient, whether the patient is human or an animal, other medications administered, and whether treatment is prophylactic or therapeutic. The selected dosage level may also depend upon a variety of pharmacokinetic factors including the activity of the particular compositions of the present disclosure employed, or the ester, salt, or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors. Treatment dosages may be titrated to optimize safety and efficacy.

Toxicity and therapeutic efficacy of compounds provided herein can be determined by standard pharmaceutical procedures in cell culture or in animal models. For example, LD50, ED50, EC50, and IC50 may be determined, and the dose ratio between toxic and therapeutic effects (LD50/ED50) may be calculated as the therapeutic index. The data obtained from in vitro and in vivo assays can be used in estimating or formulating a range of dosage for use in humans. For example, the compositions and methods disclosed herein may initially be evaluated in xenogeneic cancer models (e.g., an NCI-H929 multiple myeloma mouse model).

In some embodiments, an ADC or composition comprising an ADC is administered on a single occasion. In other embodiments, an ADC or composition comprising an ADC is administered on multiple occasions. Intervals between single dosages can be, e.g., daily, weekly, monthly, or yearly. Intervals can also be irregular, based on measuring blood levels of the administered agent (e.g., the ADC) in the patient in order to maintain a relatively consistent plasma concentration of the agent. The dosage and frequency of administration of an ADC or composition comprising an ADC may also vary depending on whether the treatment is prophylactic or therapeutic. In prophylactic applications, a relatively low dosage may be administered at relatively infrequent intervals over a long period of time. Some patients continue to receive treatment for the rest of their lives. In therapeutic applications, a relatively higher dosage at relatively shorter intervals is sometimes required until progression of the disease is reduced or terminated, and preferably until the patient shows partial or complete amelioration of one or more symptoms of disease. Thereafter, the patient may be administered a lower, e.g., prophylactic regime.

The above therapeutic approaches can be combined with any one of a wide variety of additional surgical, chemotherapy, or radiation therapy regimens. In some embodiments, the ADCs or compositions disclosed herein are co-formulated and/or co-administered with one or more additional therapeutic agents, e.g., one or more chemotherapeutic agents, one or more standard-of-care agents for the particular condition being treated.

Kits for use in the therapeutic and/or diagnostic applications described herein are also provided. Such kits may comprise a carrier, package, or container that is compartmentalized to receive one or more containers such as vials, tubes, and the like, each of the container(s) comprising one of the separate elements to be used in a method disclosed herein. A label may be present on or with the container(s) to indicate that an ADC or composition within the kit is used for a specific therapy or non-therapeutic application, such as a prognostic, prophylactic, diagnostic, or laboratory application. A label may also indicate directions for either in vivo or in vitro use, such as those described herein. Directions and or other information may also be included on an insert(s) or label(s), which is included with or on the kit. The label may be on or associated with the container. A label may be on a container when letters, numbers, or other characters forming the label are molded or etched into the container itself. A label may be associated with a container when it is present within a receptacle or carrier that also holds the container, e.g., as a package insert. The label may indicate that an ADC or composition within the kit is used for diagnosing or treating a condition, such as a cancer a described herein.

In some embodiments, a kit comprises an ADC or composition comprising an ADC. In some embodiments, the kit further comprises one or more additional components, including but not limited to: instructions for use; other reagents, e.g., a therapeutic agent (e.g., a standard-of-care agent); devices, containers, or other materials for preparing the ADC for administration; pharmaceutically acceptable carriers; and devices, containers, or other materials for administering the ADC to a subject. Instructions for use can include guidance for therapeutic applications including suggested dosages and/or modes of administration, e.g., in a patient having or suspected of having a cancer. In some embodiments, the kit comprises an ADC and instructions for use of the ADC in treating, preventing, and/or diagnosing a cancer.

### COMBINATION THERAPIES

In some embodiments, the present disclosure provides methods of treatment wherein the antibody-drug conjugates disclosed herein are administered in combination with one or more additional therapeutic agents. Exemplary combination partners are disclosed herein.

In certain embodiments, a combination described herein comprises a PD-1 inhibitor. In some embodiments, the PD-1 inhibitor is chosen from PDR001 (Novartis), Nivolumab (Bristol-Myers Squibb), Pembrolizumab (Merck & Co), Pidilizumab (CureTech), MEDI0680 (Medimmune), REGN2810 (Regeneron), TSR-042 (Tesaro), PF-06801591 (Pfizer), BGB-A317 (Beigene), BGB-108 (Beigene), INCSHR1210 (Incyte), or AMP-224 (Amplimmune). In some embodiments, the PD-1 inhibitor is PDR001. PDR001 is also known as Spartalizumab.

In certain embodiments, a combination described herein comprises a LAG-3 inhibitor. In some embodiments, the LAG-3 inhibitor is chosen from LAG525 (Novartis), BMS-986016 (Bristol-Myers Squibb), or TSR-033 (Tesaro).

In certain embodiments, a combination described herein comprises a TIM-3 inhibitor. In some embodiments, the TIM-3 inhibitor is MBG453 (Novartis), TSR-022 (Tesaro), LY-3321367 (Eli Lily), Sym23 (Symphogen), BGB-A425 (Beigene), INCAGN-2390 (Agenus), BMS-986258 (BMS), RO-7121661 (Roche), or LY-3415244 (Eli Lilly).

In certain embodiments, a combination descdribed herein comprises a PDL1 inhibitor. In one embodiment, the PDL1 inhibitor is chosen from FAZ053 (Novartis), atezolizumab (Genentech), durvalumab (Astra Zeneca), or avelumab (Pfizer).

In certain embodiments, a combination described herein comprises a GITR agonist. In some embodiments, the GITR agonist is chosen from GWN323 (NVS), BMS-986156, MK-4166 or MK-1248 (Merck), TRX518 (Leap Therapeutics), INCAGN1876 (Incyte/Agenus), AMG 228 (Amgen) or INBRX-110 (Inhibrx).

In some embodiments, a combination described herein comprises an IAP inhibitor. In some embodiments, the IAP inhibitor comprises LCL161 or a compound disclosed in International Application Publication No. WO 2008/016893.

In an embodiment, the combination comprises an mTOR inhibitor, e.g., RAD001 (also known as everolimus).

In an embodiment, the combination comprises a HDAC inhibitor, e.g., LBH589. LBH589 is also known as panobinostat.

In an embodiment, the combination comprises an IL-17 inhibitor, e.g., CJM112.

In certain embodiments, a combination described herein comprises an estrogen receptor (ER) antagonist. In some embodiments, the estrogen receptor antagonist is used in combination with a PD-1 inhibitor, a CDK4/6 inhibitor, or both. In some embodiments, the combination is used to treat an ER positive (ER+) cancer or a breast cancer (e.g., an ER+ breast cancer).

In some embodiments, the estrogen receptor antagonist is a selective estrogen receptor degrader (SERD). SERDs are estrogen receptor antagonists which bind to the receptor and result in *e.g.,* degradation or down-regulation of the receptor (Boer K. et al., (2017) Therapeutic Advances in Medical Oncology 9(7): 465-479). ER is a hormone-activated transcription factor important for e.g., the growth, development and physiology of the human reproductive system. ER is activated by, *e.g.,* the hormone estrogen (17beta estradiol). ER expression and signaling is implicated in cancers *(e.g.,* breast cancer), *e.g.,* ER positive (ER+) breast cancer. In some embodiments, the SERD is chosen from LSZ102, fulvestrant, brilanestrant, or elacestrant.

In some embodiments, the SERD comprises a compound disclosed in International Application Publication No. WO 2014/130310.

In some embodiments, the SERD comprises LSZ102. LSZ102 has the chemical name: (E)-3-(4-((2-(2-(1,1-difluoroethyl)-4-fluorophenyl)-6-hydroxybenzo[b]thiophen-3-yl)oxy)phenyl)acrylic acid. In some embodiments, the SERD comprises fulvestrant (CAS Registry Number: 129453-61-8), or a compound disclosed in International Application Publication No. WO 2001/051056. In some embodiments, the SERD comprises elacestrant (CAS Registry Number: 722533-56-4), or a compound disclosed in U.S. Patent No. 7,612,114. Elacestrant is also known as RAD1901, ER-306323 or (6R)-6-{2-[Ethyl({4-[2-(ethylamino)ethyl]phenyl}methyl)amino]-4-methoxyphenyl}-5,6,7,8-tetrahydronaphthalen-2-ol. Elacestrant is an orally bioavailable, non-steroidal combined selective estrogens receptor modulator (SERM) and a SERD. Elacestrant is also disclosed, *e.g.,* in Garner F et al., (2015) Anticancer Drugs 26(9):948-56. In some embodiments, the SERD is brilanestrant (CAS Registry Number: 1365888-06-7), or a compound disclosed in International Application Publication No. WO 2015/136017.

In some embodiments, the SERD is chosen from RU 58668, GW7604, AZD9496, bazedoxifene, pipendoxifene, arzoxifene, OP-1074, or acolbifene, e.g., as disclosed in McDonell et al. (2015) Journal of Medicinal Chemistry 58(12) 4883-4887.

Other exemplary estrogen receptor antagonists are disclosed, e.g., in WO 2011/156518, WO 2011/159769, WO 2012/037410, WO 2012/037411, and US 2012/0071535.

In certain embodiments, a combination described herein comprises an inhibitor of Cyclin-Dependent Kinases 4 or 6 (CDK4/6). In some embodiments, the CDK4/6 inhibitor is used in combination with a PD-1 inhibitor, an estrogen receptor (ER) antagonist, or both. In some embodiments, the combination is used to treat an ER positive (ER+) cancer or a breast cancer (e.g., an ER+ breast cancer). In some embodiments, the CDK4/6 inhibitor is chosen from ribociclib, abemaciclib (Eli Lilly), or palbociclib.

In some embodiments, the CDK4/6 inhibitor comprises ribociclib (CAS Registry Number: 1211441-98-3), or a compound disclosed in U.S. Patent Nos. 8,415,355 and 8,685,980.

In some embodiments, the CDK4/6 inhibitor comprises a compound disclosed in International Application Publication No. WO 2010/020675 and U.S. Patent Nos. 8,415,355 and 8,685,980.

In some embodiments, the CDK4/6 inhibitor comprises ribociclib (CAS Registry Number: 1211441-98-3). Ribociclib is also known as LEE011, KISQALI^{®}, or 7-cyclopentyl-N,N-dimethyl-2-((5-(piperazin-1-yl)pyridin-2-yl)amino)-7H-pyrrolo[2,3-d]pyrimidine-6-carboxamide.

In some embodiments, the CDK4/6 inhibitor comprises abemaciclib (CAS Registry Number: 1231929-97-7). Abemaciclib is also known as LY835219 or N-[5-[(4-Ethyl-1-piperazinyl)methyl]-2-pyridinyl]-5-fluoro-4-[4-fluoro-2-methyl-1-(1-methylethyl)-1H-benzimidazol-6-yl]-2-pyrimidinamine. Abemaciclib is a CDK inhibitor selective for CDK4 and CDK6 and is disclosed, *e.g.,* in Torres-Guzman R *et al.* (2017) *Oncotarget* 10.18632/oncotarget. 17778.

In some embodiments, the CDK4/6 inhibitor comprises palbociclib (CAS Registry Number: 571190-30-2). Palbociclib is also known as PD-0332991, IBRANCE^{®} or 6-Acetyl-8-cyclopentyl-5-methyl-2-{[5-(1-piperazinyl)-2-pyridinyl]amino}pyrido[2,3-d]pyrimidin-7(8H)-one. Palbociclib inhibits CDK4 with an IC50 of 11nM, and inhibits CDK6 with an IC50 of 16nM, and is disclosed, *e.g.,* in Finn et al. (2009) Breast Cancer Research 11(5):R77.

In certain embodiments, a combination described herein comprises an inhibitor of chemokine (C-X-C motif) receptor 2 (CXCR2). In some embodiments, the CXCR2 inhibitor is chosen from 6-chloro-3-((3,4-dioxo-2-(pentan-3-ylamino)cyclobut-1-en-1-yl)amino)-2-hydroxy-N-methoxy-N-methylbenzenesulfonamide, danirixin, reparixin, or navarixin.

In some embodiments, the CSF-1/1R binding agent is chosen from an inhibitor of macrophage colony-stimulating factor (M-CSF), e.g., a monoclonal antibody or Fab to M-CSF *(e.g.,* MCS110), a CSF-1R tyrosine kinase inhibitor *(e.g.,* 4-((2-(((1R,2R)-2-hydroxycyclohexyl)amino)benzo[d]thiazol-6-yl)oxy)-N-methylpicolinamide or BLZ945), a receptor tyrosine kinase inhibitor (RTK) (e.g., pexidartinib), or an antibody targeting CSF-1R (*e.g.*, emactuzumab or FPA008). In some embodiments, the CSF-1/1R inhibitor is BLZ945. In some embodiments, the CSF-1/1R binding agent is MCS110. In other embodiments, the CSF-1/1R binding agent is pexidartinib.

In certain embodiments, a combination described herein comprises a c-MET inhibitor. c-MET, a receptor tyrosine kinase overexpressed or mutated in many tumor cell types, plays key roles in tumor cell proliferation, survival, invasion, metastasis, and tumor angiogenesis. Inhibition of c-MET may induce cell death in tumor cells overexpressing c-MET protein or expressing constitutively activated c-MET protein. In some embodiments, the c-MET inhibitor is chosen from capmatinib (INC280), JNJ-3887605, AMG 337, LY2801653, MSC2156119J, crizotinib, tivantinib, or golvatinib.

In certain embodiments, a combination described herein comprises a transforming growth factor beta (also known as TGF-β TGFβ, TGFb, or TGF-beta, used interchangeably herein) inhibitor. In some embodiments, the TGF-β inhibitor is chosen from fresolimumab or XOMA 089.

In certain embodiments, a combination described herein comprises an adenosine A2a receptor (A2aR) antagonist (*e.g.,* an inhibitor of A2aR pathway, *e.g.,* an adenosine inhibitor, e.g., an inhibitor of A2aR or CD-73). In some embodiments, the A2aR antagonist is used in combination with a PD-1 inhibitor, and one or more (e.g., two, three, four, five, or all) of a CXCR2 inhibitor, a CSF-1/1R binding agent, LAG-3 inhibitor, a GITR agonist, a c-MET inhibitor, or an IDO inhibitor. In some embodiments, the combination is used to treat a pancreatic cancer, a colorectal cancer, a gastric cancer, or a melanoma (e.g., a refractory melanoma). In some embodiments, the A2aR antagonist is chosen from PBF509 (NIR178) (Palobiofarma/Novartis), CPI444/V81444 (Corvus/Genentech), AZD4635/HTL-1071 (AstraZeneca/Heptares), Vipadenant (Redox/Juno), GBV-2034 (Globavir), AB928 (Arcus Biosciences), Theophylline, Istradefylline (Kyowa Hakko Kogyo), Tozadenant/SYN-115 (Acorda), KW-6356 (Kyowa Hakko Kogyo), ST-4206 (Leadiant Biosciences), or Preladenant/SCH 420814 (Merck/Schering). Without wishing to be bound by theory, it is believed that in some embodiments, inhibition of A2aR leads to upregulation of IL-1b.

In certain embodiments, a combination described herein comprises an inhibitor of indoleamine 2,3-dioxygenase (IDO) and/or tryptophan 2,3-dioxygenase (TDO). In some embodiments, the IDO inhibitor is used in combination with a PD-1 inhibitor, and one or more (e.g., two, three, four, or all) of a TGF-β inhibitor, an A2aR antagonist, a CSF-1/1R binding agent, a c-MET inhibitor, or a GITR agonist. In some embodiments, the combination is used to treat a pancreatic cancer, a colorectal cancer, a gastric cancer, or a melanoma (e.g., a refractory melanoma). In some embodiments, the IDO inhibitor is chosen from (4E)-4-[(3-chloro-4-fluoroanilino)-nitrosomethylidene]-1,2,5-oxadiazol-3-amine (also known as epacadostat or INCB24360), indoximod (NLG8189), (1-methyl-D-tryptophan), α-cyclohexyl-5H-Imidazo[5,1-a]isoindole-5-ethanol (also known as NLG919), indoximod, BMS-986205 (formerly F001287).

In certain embodiments, a combination described herein comprises a Galectin, e.g., Galectin-1 or Galectin-3, inhibitor. In some embodiments, the combination comprises a Galectin-1 inhibitor and a Galectin-3 inhibitor. In some embodiments, the combination comprises a bispecific inhibitor (e.g., a bispecific antibody molecule) targeting both Galectin-1 and Galectin-3. In some embodiments, the Galectin inhibitor is used in combination with one or more therapeutic agents described herein. In some embodiments, the Galectin inhibitor is chosen from an anti-Galectin antibody molecule, GR-MD-02 (Galectin Therapeutics), Galectin-3C (Mandal Med), Anginex, or OTX-008 (OncoEthix, Merck).

In some embodiments, a combination described herein comprises a MEK inhibitor. In some embodiments, the MEK inhibitor is chosen from Trametinib, selumetinib, AS703026, BIX 02189, BIX 02188, CI-1040, PD0325901, PD98059, U0126, XL-518, G-38963, or G02443714. In some embodiments, the MEK inhibitor is Trametinib.

In one embodiment, a combination described herein includes an interleukin-1 beta (IL-1β) inhibitor. In some embodiments, the IL-1β inhibitor is chosen from canakinumab, gevokizumab, Anakinra, or Rilonacept.

In certain embodiments, a combination described herein comprises an IL-15/IL-15Ra complex. In some embodiments, the IL-15/IL-15Ra complex is chosen from NIZ985 (Novartis), ATL-803 (Altor) or CYP0150 (Cytune).

In certain embodiments, a combination described herein comprises a mouse double minute 2 homolog (MDM2) inhibitor. The human homolog of MDM2 is also known as HDM2. In some embodiments, an MDM2 inhibitor described herein is also known as a HDM2 inhibitor. In some embodiments, the MDM2 inhibitor is chosen from HDM201 or CGM097.

In an embodiment the MDM2 inhibitor comprises (S)-1-(4-chlorophenyl)-7-isopropoxy-6-methoxy-2-(4-(methyl(((1r,4S)-4-(4-methyl-3-oxopiperazin-1-yl)cyclohexyl)methyl)amino)phenyl)-1,2-dihydroisoquinolin-3(4H)-one (also known as CGM097) or a compound disclosed in PCT Publication No. WO 2011/076786 to treat a disorder, e.g., a disorder described herein). In one embodiment, a therapeutic agent disclosed herein is used in combination with CGM097.

In some embodiments, a combination described herein comprises a hypomethylating agent (HMA). In some embodiments, the HMA is chosen from decitabine or azacitidine.

In certain embodiments, a combination described herein comprises an inhibitor acting on any pro-survival proteins of the Bcl2 family. In certain embodiments, a combination described herein comprises a Bcl-2 inhibitor. In some embodiments, the Bcl-2 inhibitor is venetoclax 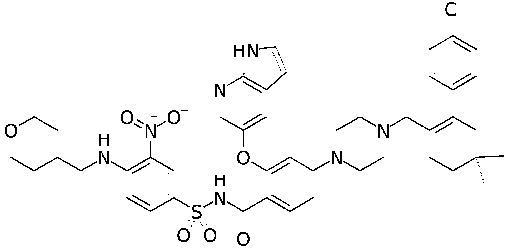 ( venetoclax).
In one embodiment, the Bcl-2 inhibitor is selected from the compounds described in WO 2013/110890 and WO 2015/011400. In some embodiments, the Bcl-2 inhibitor comprises navitoclax (ABT-263), ABT-737, BP1002, SPC2996, APG-1252, obatoclax mesylate (GX15-070MS), PNT2258, Zn-d5, BGB-11417, or oblimersen (G3139). In some embodiments, the Bcl-2 inhibitor is N-(4-hydroxyphenyl)-3-[6-[(3S)-3-(morpholinomethyl)-3,4-dihydro-1H-isoquinoline-2-carbonyl]-1,3-benzodioxol-5-yl]-N-phenyl-5,6,7,8-tetrahydroindolizine-1-carboxamide, compound A1: 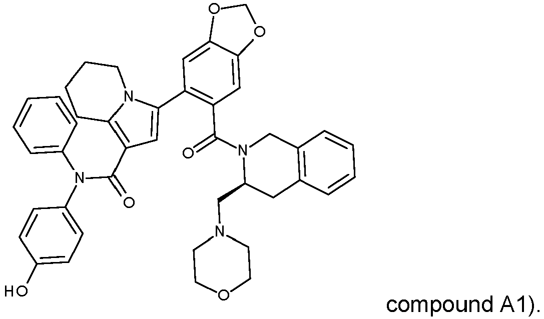 In some embodiments, the Bcl-2 inhibitor is (S)-5-(5-chloro-2-(3-(morpholinomethyl)-1,2,3,4-tetrahydroisoquinoline-2-carbonyl)phenyl)-N-(5-cyano-1,2-dimethyl-1H-pyrrol-3-yl)-N-(4-hydroxyphenyl)-1,2-dimethyl-1H-pyrrole-3-carboxamide), compound A2:

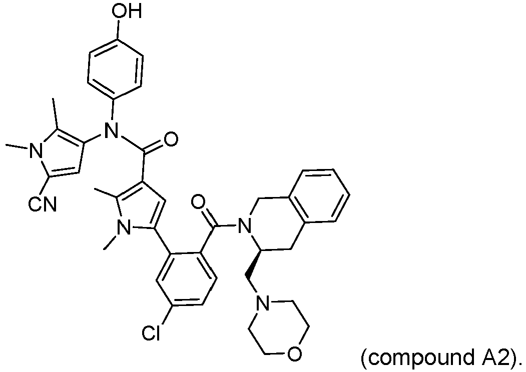

In one embodiment, the antibody-drug conjugates or combinations disclosed herein are suitable for the treatment of cancer *in vivo.* For example, the combination can be used to inhibit the growth of cancerous tumors. The combination can also be used in combination with one or more of: a standard of care treatment (e.g., for cancers or infectious disorders), a vaccine (e.g., a therapeutic cancer vaccine), a cell therapy, a radiation therapy, surgery, or any other therapeutic agent or modality, to treat a disorder herein. For example, to achieve antigen-specific enhancement of immunity, the combination can be administered together with an antigen of interest. A combination disclosed herein can be administered in either order or simultaneously.

### ADDITIONAL EMBODIMENTS

The disclosure provides the following additional embodiments for linker-drug groups, antibody-drug conjugates, linker groups, and methods of conjugation.

### Linker-Drug Group

In some embodiments, the Linker-Drug group of the invention may be a compound having the structure of Formula (A'), or a pharmaceutically acceptable salt thereof: wherein: 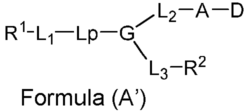
R¹ is a reactive group;
L₁ is a bridging spacer;
Lp is a bivalent peptide spacer;
G-L₂-A is a self-immolative spacer;
R² is a hydrophilic moiety;
L₂ is a bond, a methylene, a neopentylene or a C₂-C₃alkenylene;
A is a bond, -OC(=O)-*, 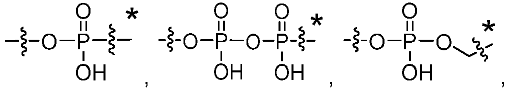 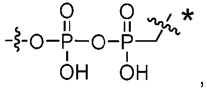 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety; and
D is a Drug moiety that is capable of inhibiting the activity of the MCI-1 protein when, e.g., released from the Antibody Drug Conjugates or immunoconjugates disclosed herein.
Certain aspects and examples of the Linker-Drug group of the invention are provided in the following listing of enumerated embodiments. It will be recognized that features specified in each embodiment may be combined with other specified features to provide further embodiments of the present invention.
**Embodiment 1.** The compound of Formula (A'), or pharmaceutically acceptable salt thereof, wherein:
   R¹ is a reactive group;
   L₁ is a bridging spacer;
   Lp is a bivalent peptide spacer comprising two to four amino acid residues;
   G-L₂-A is a self-immolative spacer;
   R² is a hydrophilic moiety;
   L₂ is a bond, a methylene, a neopentylene or a C₂-C₃alkenylene;
   A is a bond, -OC(=O)-*, 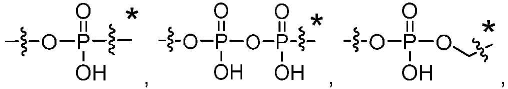 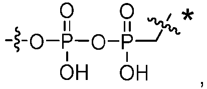 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety; and
   D is a Drug moiety as defined herein, e.g., a MCI-1 inhibitor.
**Embodiment 2.** The compound of Formula (A'), or pharmaceutically acceptable salt thereof, wherein:
   R¹ is a reactive group;
   L₁ is a bridging spacer;
   Lp is a bivalent peptide spacer comprising two to four amino acid residues;
   the 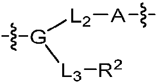 group is selected from: 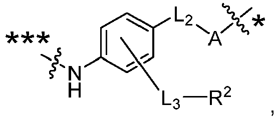 wherein the * of 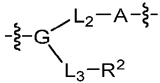 indicates the point of attachment to D (e.g., to an N or a O of the Drug moiety), the *** of 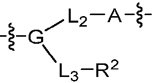 indicates the point of attachment to Lp;
   R² is a hydrophilic moiety;
   L₂ is a bond, a methylene, a neopentylene or a C₂-C₃alkenylene;
   A is a bond, -OC(=O)-*, 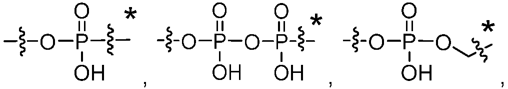 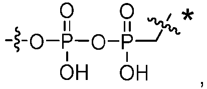 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety; and
   D is a Drug moiety as defined herein, e.g., a MCI-1 inhibitor.
**Embodiment 3.** The compound of Formula (A'), or pharmaceutically acceptable salt thereof, having the structure of Formula (B'): 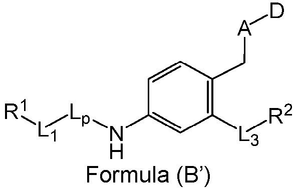 wherein:
   R¹ is a reactive group;
   L₁ is a bridging spacer;
   Lp is a bivalent peptide spacer comprising two to four amino acid residues;
   R² is a hydrophilic moiety;
   A is a bond, -OC(=O)-*, 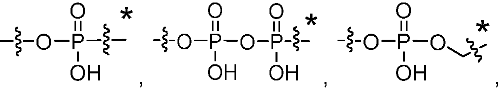 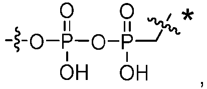 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety; and
   D is a Drug moiety as defined herein and comprising an N or an O, wherein D is connected to A via a direct bond from A to the N or the O of the Drug moiety.
**Embodiment 4.** The compound of Formula (A') or of any one of Embodiments 1 to 3, or pharmaceutically acceptable salt thereof, wherein:
   R¹ is 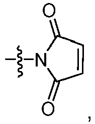 -ONH₂, -NH₂, 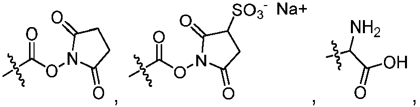 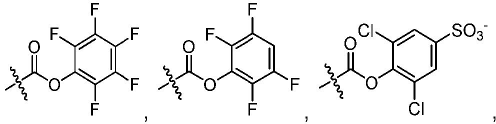 -N₃, 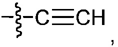 -SH, -SR³, -SSR⁴, -S(=O)₂(CH=CH₂), -(CH₂)₂S(=O)₂(CH=CH₂), -NHS(=O)₂(CH=CH₂),-NHC(=O)CH₂Br, -NHC(=O)CH₂I, 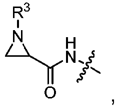 -C(O)NHNH₂, 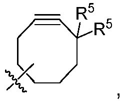 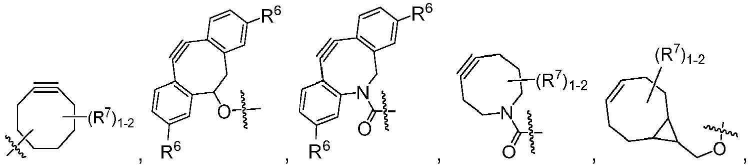 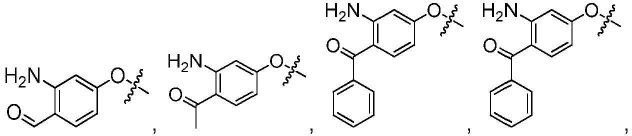 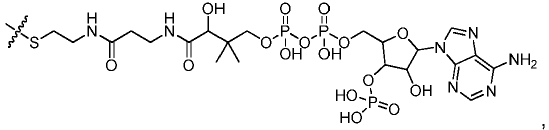 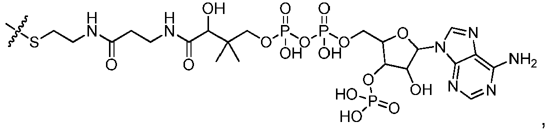 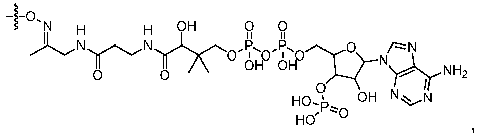 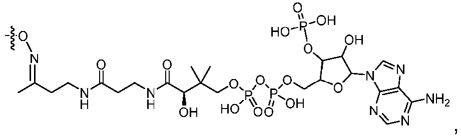 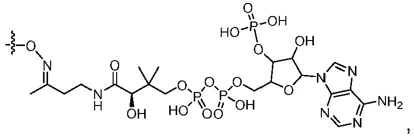 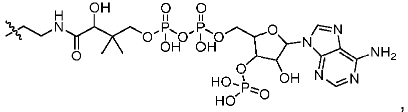 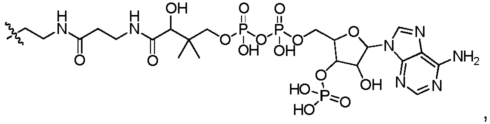 or 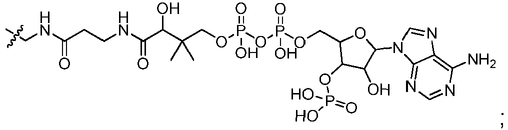
   L₁ is *-C(=O)(CH₂)ₘO(CH₂)ₘ-**;

      *-C(=O)((CH₂)ₘO)ₜ(CH₂)ₙ-**;

      *-C(=O)(CH₂)ₘ-**;

      *-C(=O)NH((CH₂)ₘO)ₜ(CH₂)ₙ-**;

      *-C(=O)O(CH₂)ₘSSC(R³)₂(CH₂)ₘC(=O)NR³(CH₂)ₘNR³C(=O)(CH₂)ₘ-**;

      *-C(=O)O(CH₂)ₘC(=O)NH(CH₂)ₘ-**;

      *-C(=O)(CH₂)ₘNH(CH₂)ₘ-**; *-C(=O)(CH₂)ₘNH(CH₂)ₙC(=O)-**;

      *-C(=O)(CH₂)ₘX₁(CH₂)ₘ-**; *-C(=O)((CH₂)ₘO)ₜ(CH₂)ₙX₁(CH₂)ₙ-**;

      *-C(=O)(CH₂)ₘNHC(=O)(CH₂)ₙ-**;

      *-C(=O)((CH₂)ₘO)ₜ(CH₂)ₙNHC(=O)(CH₂)ₙ-**;

      *-C(=O)(CH₂)ₘNHC(=O)(CH₂)ₙX₁(CH₂)ₙ-**;

      *-C(=O)((CH₂)ₘO)ₜ(CH₂)ₙNHC(=O)(CH₂)ₙX₁(CH₂)ₙ-**;

      *-C(=O)((CH₂)ₘO)ₜ(CH₂)ₙC(=O)NH(CH₂)ₘ-**;

      *-C(=O)(CH₂)ₘC(R³)₂-**;

      or

      *-C(=O)(CH₂)ₘC(=O)NH(CH₂)ₘ-**,
   where the * of L₁ indicates the point of attachment to Lp, and the ** of L₁ indicates the point of attachment to R¹;
   R² is a hydrophilic moiety selected from polyethylene glycol, polyalkylene glycol, a sugar, an oligosaccharide, a polypeptide or C₂-C₆alkyl substituted with 1 to 3 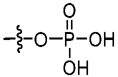 groups;
   each R³ is independently selected from H and C₁-C₆alkyl;
   R⁴ is 2-pyridyl or 4-pyridyl;
   each R⁵ is independently selected from H, C₁-C₆alkyl, F, Cl, and -OH;
   each R⁶ is independently selected from H, C₁-C₆alkyl, F, Cl, -NH₂, -OCH₃,-OCH₂CH₃, -N(CH₃)₂, -CN, -NO₂ and -OH;
   each R⁷ is independently selected from H, C₁₋₆alkyl, fluoro, benzyloxy substituted with -C(=O)OH, benzyl substituted with -C(=O)OH, C₁₋₄alkoxy substituted with
      -C(=O)OH and C₁₋₄alkyl substituted with -C(=O)OH;
   X₁ is 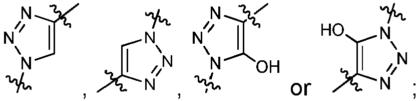
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;
   A is a bond, -OC(=O)-*, 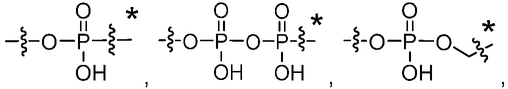 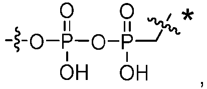 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety having the structure 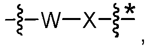 where
      W is -CH₂O-**, -CH₂N(R^{b})C(=O)O-**, -NHC(=O)C(R^{b})₂NHC(=O)O-**, -NHC(=O)C(R^{b})₂NH-**, -NHC(=O)C(R^{b})₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**,-NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²; and
      the * of L₃ indicates the point of attachment to R²;
      and
   D is a Drug moiety as defined herein and comprising an N or an O, wherein D is connected to A via a direct bond from A to the N or the O of the Drug moiety.
**Embodiment 5.** The compound of Formula (A') or of any one of Embodiments 1 to 4, or pharmaceutically acceptable salt thereof, wherein:
   R¹ is 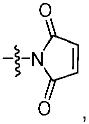 -ONH₂, 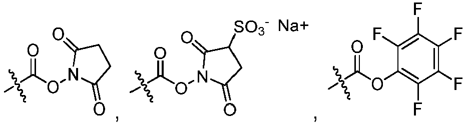 or 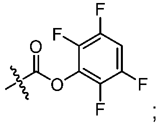
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp, and the ** of L₁ indicates the point of attachment to R¹;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 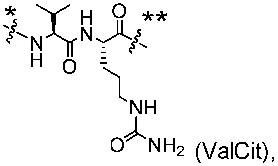 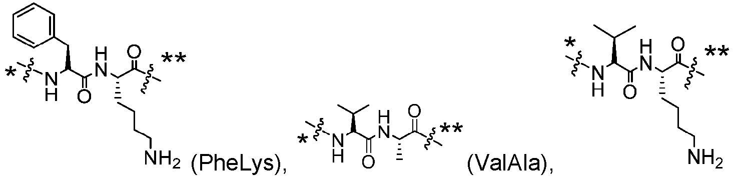 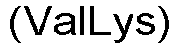 and 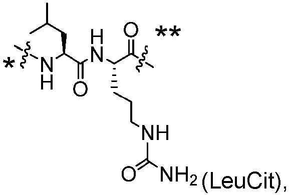 where the * of Lp indicates the attachment point to L₁ and the ** of Lp indicates the attachment point to the - NH- group of G;
   L₃ is a spacer moiety having the structure 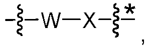 where
      W is -CH₂O-**, -CH₂N(R^{b})C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**,-CH₂NR^{b}C(=O)NH-**, -CH₂NR^{b}C(=O)NR^{b}-**, -NHC(=O)-**,-NHC(=O)O-**, -NHC(=O)NH-**, -OC(=O)NH-**, -S(O)₂NH-**,-NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH2-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 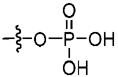 groups;
   A is a bond, -OC(=O)-*, 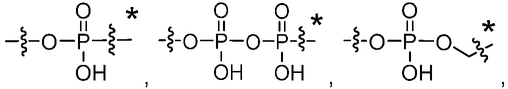 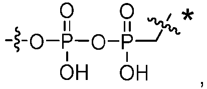 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D; and
   D is a Drug moiety as defined herein and comprising an N or an O, wherein D is connected to A via a direct bond from A to the N or the O of the Drug moiety.
**Embodiment 6.** The compound of Formula (A') or of any one of Embodiments 1 to 5, or pharmaceutically acceptable salt thereof, wherein:
   R¹ is 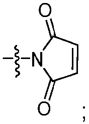
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp, and the ** of L₁ indicates the point of attachment to R¹;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 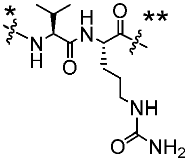 (ValCit), where the * of Lp indicates the attachment point to L₁ and the ** of Lp indicates the attachment point to the -NH- group of G;
   L₃ is a spacer moiety having the structure 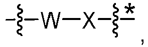 where
      W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**,-CH₂NR^{b}C(=O)NH-**, -CH₂NR^{b}C(=O)NR^{b}-**, -NHC(=O)-**,-NHC(=O)O-**, -NHC(=O)NH-**, -OC(=O)NH-**, -S(O)₂NH-**,-NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 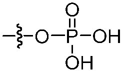 groups;
   A is a bond, -OC(=O)-*, 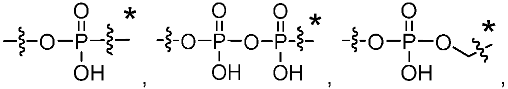 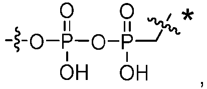 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
      and
   D is a Drug moiety as defined herein and comprising an N or an O, wherein D is connected to A via a direct bond from A to the N or the O of the Drug moiety.
**Embodiment 7.** The compound of Formula (A') or of any one of Embodiments 1 to 6, or pharmaceutically acceptable salt thereof, wherein:
   R¹ is 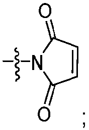
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp and the ** of L₁ indicates the point of attachment to R¹;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 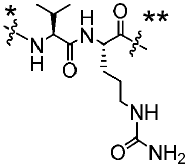 (ValCit), where the * of Lp indicates the attachment point to L₁ and the ** of Lp indicates the attachment point to the -NH- group of G;
   L₃ is a spacer moiety having the structure 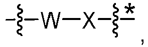 where
      W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, -C(=O)NR^{b}-**,-C(=O)NH-**, -CH₂NR^{b}C(=O)-**, -CH₂NR^{b}C(=O)NH-**,-CH₂NR^{b}C(=O)NR^{b}-**, -NHC(=O)-**, -NHC(=O)O-**, or -NHC(=O)NH-**, wherein each R^{b} is independently selected from H, C₁-C₆alkyl or C₃-C₈cycloalkyl and wherein the ** of W indicates the point of attachment to X;
      X is a bond, triazolyl or ***-CH2-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 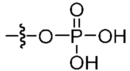 groups;
   A is a bond or -OC(=O)*, in which * indicates the attachment point to D;
      and
   D is a Drug moiety as defined herein and comprising an N or an O, wherein D is connected to A via a direct bond from A to the N or the O of the Drug moiety.
**Embodiment** 8. The compound of Formula (A') or of any one of Embodiments 1 to 7, or pharmaceutically acceptable salt thereof, wherein:
   R¹ is 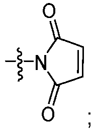
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp and the ** of L₁ indicates the point of attachment to R¹;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 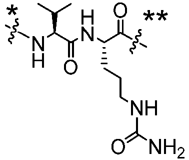 (ValCit), where the * of Lp indicates the attachment point to L₁ and the ** of Lp indicates the attachment point to the -NH- group of G;
   L₃ is a spacer moiety having the structure 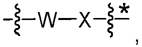 where
      W is -CH₂O-**, -CH₂N(R^{b})C(=O)O-**, -NHC(=O)CH₂NHC(=O)O-**, -CH₂N(X-R²)C(=O)O-**, or -C(=O)N(X-R²)-**, wherein each R^{b} is independently selected from H, C₁-C₆alkyl or C₃-C₈cycloalkyl and wherein the ** of W indicates the point of attachment to X;
      X is ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 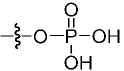 groups;
   A is a bond or -OC(=O)* in which * indicates the attachment point to D;
      and
   D is a Drug moiety as defined herein and comprising an N or an O, wherein D is connected to A via a direct bond from A to the N or the O of the Drug moiety.
**Embodiment** 9. The compound of Formula (A') or of any one of Embodiments 1 to 8, or pharmaceutically acceptable salt thereof, wherein R¹ is a reactive group selected from Table 8.
**Embodiment** 10. The compound of Formula (A') or of any one of Embodiments 1 to 9, or pharmaceutically acceptable salt thereof, wherein:
   R¹ is 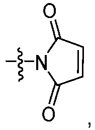 -ONH₂, -NH₂, 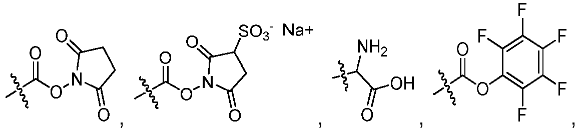 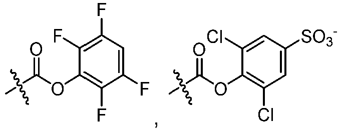 -N₃, 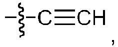 -SH, -SR³, -SSR⁴, -S(=O)₂(CH=CH₂), -(CH₂)₂S(=O)₂(CH=CH₂), -NHS(=O)₂(CH=CH₂), -NHC(=O)CH₂Br, -NHC(=O)CH₂I, 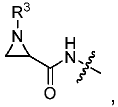 -C(O)NHNH₂, 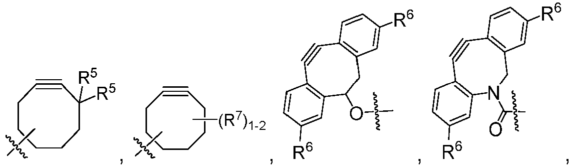 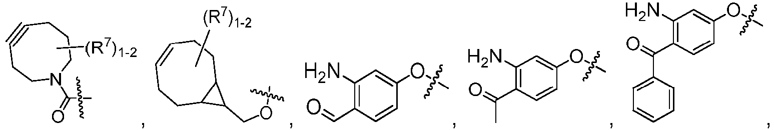 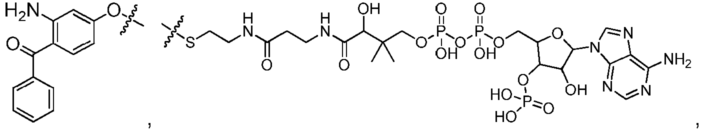 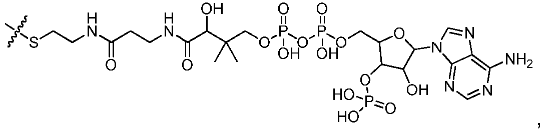 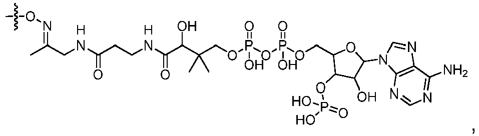 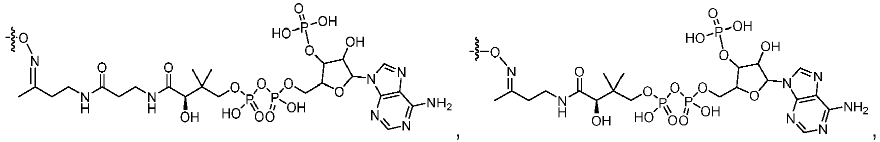 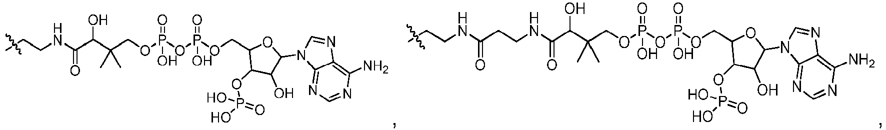 or 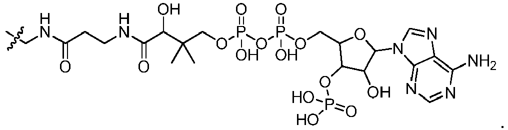
**Embodiment** 11. The compound of Formula (A') or of any one of Embodiments 1 to 9, or pharmaceutically acceptable salt thereof, wherein:
   R¹ is 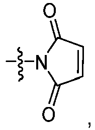 -ONH₂, -NH₂, 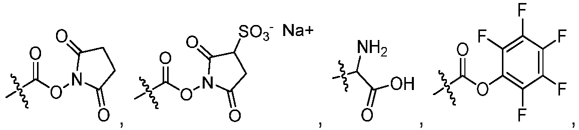 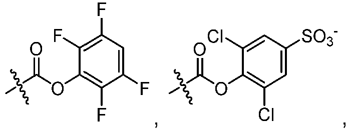 -N₃, 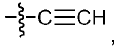 -SH, -SR³, -SSR⁴, -S(=O)₂(CH=CH₂), -(CH₂)₂S(=O)₂(CH=CH₂), -NHS(=O)₂(CH=CH₂), -NHC(=O)CH₂Br, -NHC(=O)CH₂I, 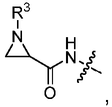 -C(O)NHNH₂, 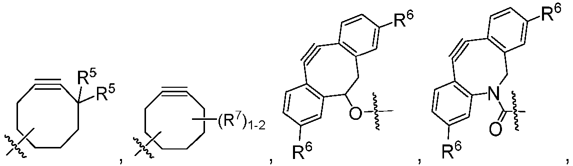 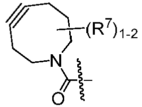 or 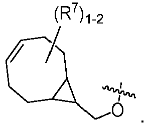
**Embodiment** 12. The compound of Formula (A') or of any one of Embodiments 1 to 9, or pharmaceutically acceptable salt thereof, wherein:
   R¹ is 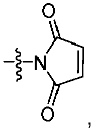 -ONH₂, 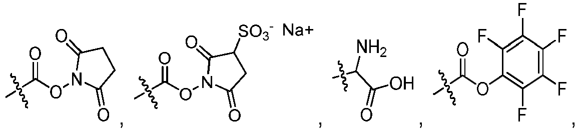 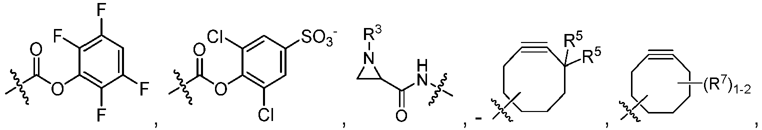 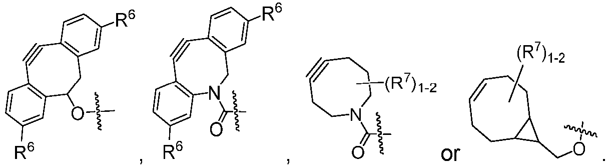
**Embodiment** 13. The compound of Formula (A') or of any one of Embodiments 1 to 9, or pharmaceutically acceptable salt thereof, wherein:
   R¹ is 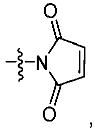 -ONH₂, 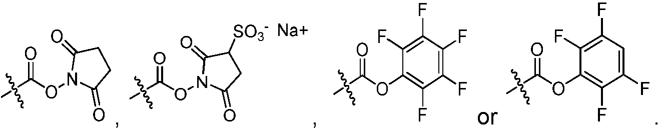
**Embodiment 14.** The compound of Formula (A') or of any one of Embodiments 1 to 9, or pharmaceutically acceptable salt thereof, wherein R¹ is 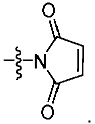
**Embodiment 15.** The compound of Formula (A') or of any one of Embodiments 1 to 9, or pharmaceutically acceptable salt thereof, wherein R¹ is -ONH₂.
**Embodiment 16.** The compound of Formula (A') or of any one of Embodiments 1 to 9, or pharmaceutically acceptable salt thereof, wherein: R¹ is 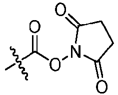 or 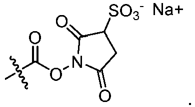
**Embodiment 17.** The compound of Formula (A') or of any one of Embodiments 1 to 9, or pharmaceutically acceptable salt thereof, wherein:
   R¹ is 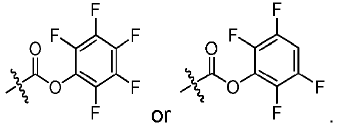
**Embodiment 18.** The compound of Formula (A') or of any one of Embodiments 1 to 9, or pharmaceutically acceptable salt thereof, having the structure: 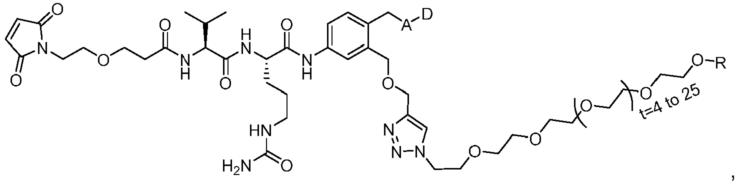 where
   R is H, -CH₃ or -CH₂CH₂C(=O)OH.
**Embodiment 19.** The compound of Formula (A') or of any one of Embodiments 1 to 9, or pharmaceutically acceptable salt thereof, having the structure: 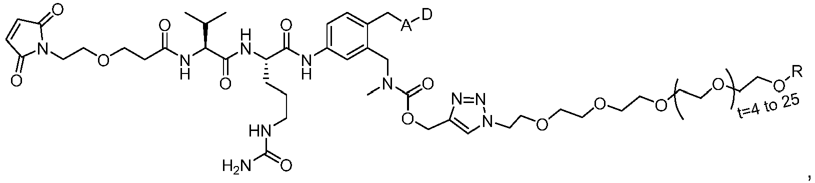 where
   R is H, -CH₃ or -CH₂CH₂C(=O)OH.
**Embodiment 20.** The compound of Formula (A') or of any one of Embodiments 1 to 9, or pharmaceutically acceptable salt thereof, having the structure: 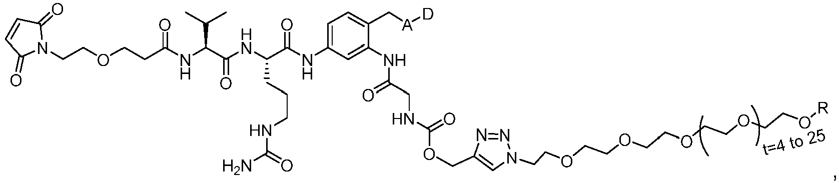 where
   R is H, -CH₃ or -CH₂CH₂C(=O)OH.
**Embodiment 21.** The compound of Formula (A') or of any one of Embodiments 1 to 9, or pharmaceutically acceptable salt thereof, having the structure: 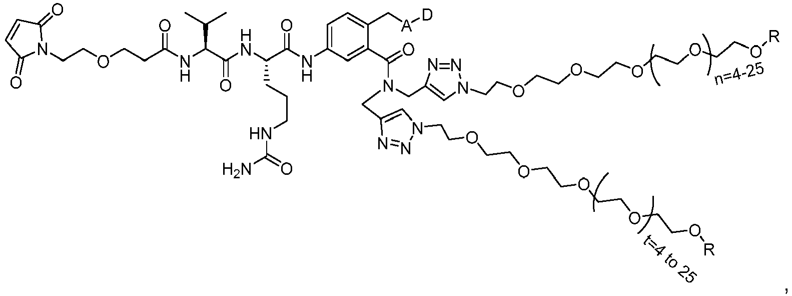 where
   each R is independently selected from H, -CH₃ or -CH₂CH₂C(=O)OH.
**Embodiment 22.** The compound of Formula (A') or of any one of Embodiments 1 to 9, or pharmaceutically acceptable salt thereof, having the structure: 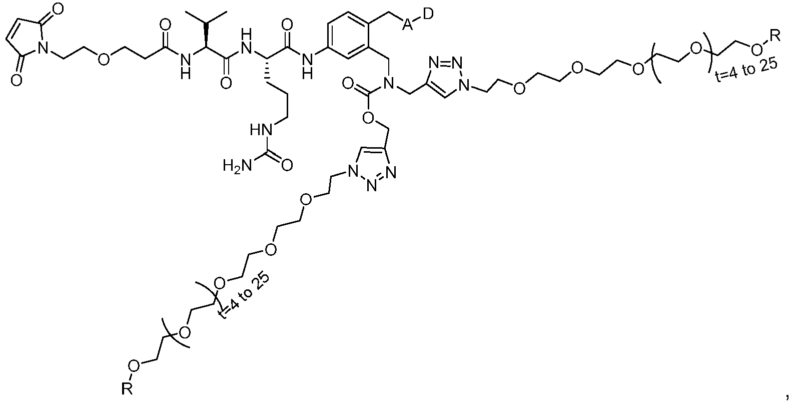 where
   each R is independently selected from H, -CH₃ or -CH₂CH₂C(=O)OH.
**Embodiment 23.** The compound of Formula (A') or of any one of Embodiments 1 to 9 or pharmaceutically acceptable salt thereof, having the structure: 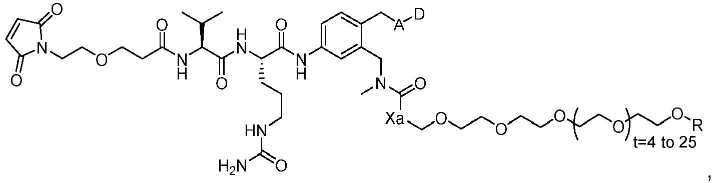 where
   Xa is -CH₂-, -OCH₂-, -NHCH₂- or -NRCH₂- and each R independently is H, -CH₃ or-CH₂CH₂C(=O)OH.
**Embodiment 24.** The compound of Formula (A') or of any one of Embodiments 1 to 9, or pharmaceutically acceptable salt thereof, having the structure: 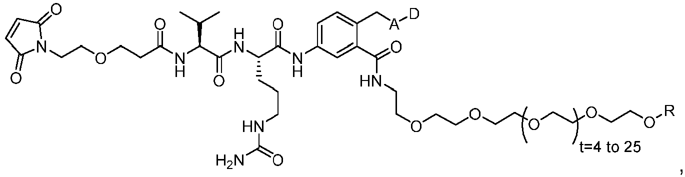 where
   R is H, -CH₃ or -CH₂CH₂C(=O)OH.
**Embodiment 25.** The compound of Formula (A') or of any one of Embodiments 1 to 9, or pharmaceutically acceptable salt thereof, having the structure: 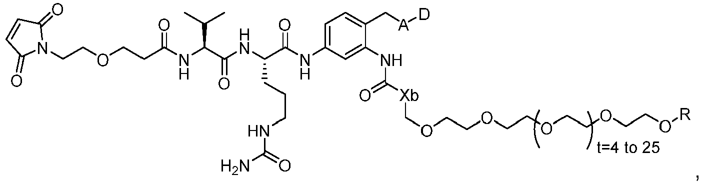 where
   Xb is -CH₂-, -OCH₂-, -NHCH₂- or -NRCH₂- and each R independently is H, -CH₃ or -CH₂CH₂C(=O)OH.
**Embodiment 26.** The compound of Formula (A') or of any one of Embodiments 1 to 9, or pharmaceutically acceptable salt thereof, having the structure: 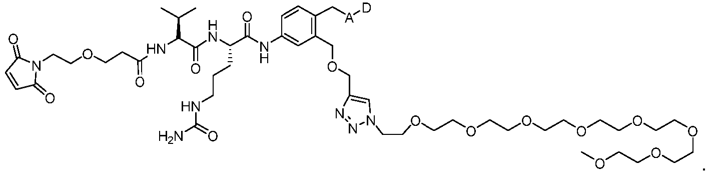
**Embodiment 27.** The compound of Formula (A') or of any one of Embodiments 1 to 9, or pharmaceutically acceptable salt thereof, having the structure: 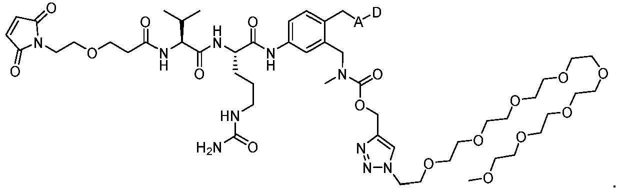
**Embodiment 28.** The compound of Formula (A') or of any one of Embodiments 1 to 9, or pharmaceutically acceptable salt thereof, having the structure: 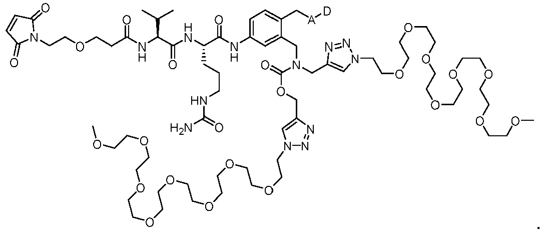
**Embodiment 29.** The compound of Formula (A') or of any one of Embodiments 1 to 9, or pharmaceutically acceptable salt thereof, having the structure: 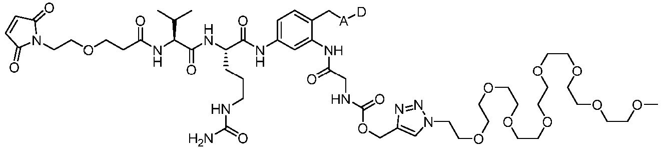
**Embodiment 30.** The compound of Formula (A') or of any one of Embodiments 1 to 9, or pharmaceutically acceptable salt thereof, having the structure: 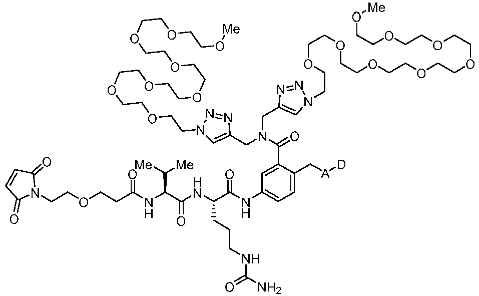
**Embodiment 31.** The compound of Formula (A') or of any one of Embodiments 1 to 9, or pharmaceutically acceptable salt thereof, having the structure of a compound in Table A.
**Embodiment 32.** A linker of the Linker-Drug group of Formula (A') having the structure of Formula (C'), 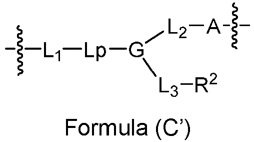 wherein
   L₁ is a bridging spacer;
   Lp is a bivalent peptide spacer;
   G-L₂-A is a self-immolative spacer;
   R² is a hydrophilic moiety;
   L₂ is a bond, a methylene, a neopentylene or a C₂-C₃alkenylene;
   A is a bond, -OC(=O)-*, 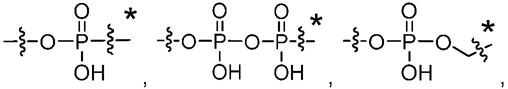 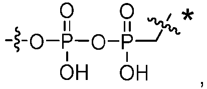 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D,
      and
   L₃ is a spacer moiety.
**Embodiment 33.** The linker of Embodiment 32, wherein:
   L₁ is a bridging spacer;
   Lp is a bivalent peptide spacer comprising two to four amino acid residues;
   G-L₂-A is a self-immolative spacer;
   R² is a hydrophilic moiety;
   L₂ is a bond, a methylene, a neopentylene or a C₂-C₃alkenylene;
   A is a bond, -OC(=O)-*, 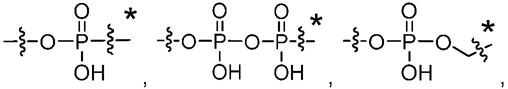 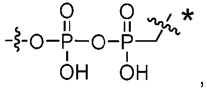 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D,
      and
   L₃ is a spacer moiety.
**Embodiment 34.** The linker of Embodiment 32 or 33, wherein:
   L₁ is a bridging spacer;
   Lp is a bivalent peptide spacer comprising two to four amino acid residues;
   the 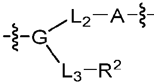 group is selected from: 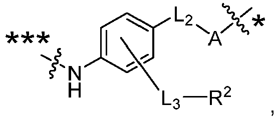 wherein the * of 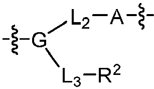 indicates the point of attachment to D (e.g., to an N or a O of the Drug moiety), the *** of 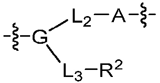 indicates the point of attachment to Lp;
   R² is a hydrophilic moiety;
   L₂ is a bond, a methylene, a neopentylene or a C₂-C₃alkenylene;
   A is a bond, -OC(=O)-*, 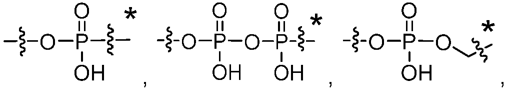 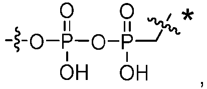 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D,
      and
   L₃ is a spacer moiety.
**Embodiment 35.** The linker of any one of Embodiments 32 to 34, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH²)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH2)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of attachment to Lp;
   R² is a hydrophilic moiety selected from polyethylene glycol, polyalkylene glycol, a sugar, an oligosaccharide, a polypeptide or C₂-C₆alkyl substituted with 1 to 3 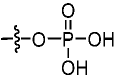 groups;
   each R³ is independently selected from H and C₁-C₆alkyl;
   X₁ is 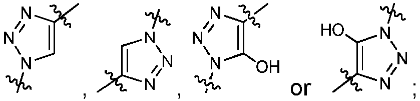
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;
   A is a bond, -OC(=O)-*, 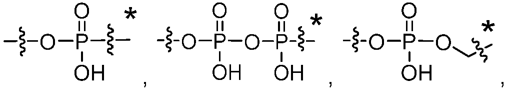 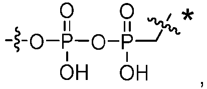 -OC(=O)N(CH₃)CH₂CH₂N(CH₃)C(=O)-* or-OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety having the structure 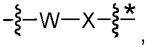 where
      W is -CH₂O-**, -CH₂N(R^{b})C(=O)O-**, -NHC(=O)C(R^{b})₂NHC(=O)O-**, -NHC(=O)C(R^{b})₂NH-**, -NHC(=O)C(R^{b})₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**,-CH₂NR^{b}C(=O)NH-**, -CH₂NR^{b}C(=O)NR^{b}-**, -NHC(=O)-**,-NHC(=O)O-**, -NHC(=O)NH-**, -OC(=O)NH-**, -S(O)₂NH-**,-NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R².
**Embodiment 36.** The linker of any one of Embodiments 32 to 35, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 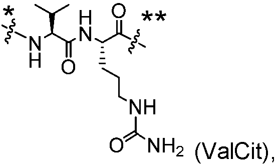 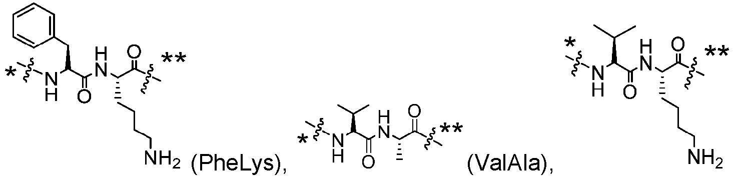 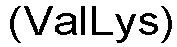 and 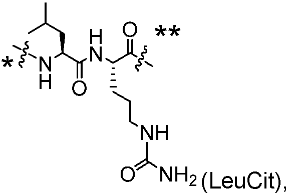 where the * of Lp indicates the attachment point to L₁;
   L₃ is a spacer moiety having the structure 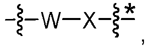 where
      W is -CH₂O-**, -CH₂N(R^{b})C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**,-CH₂NR^{b}C(=O)NH-**, -CH₂NR^{b}C(=O)NR^{b}-**, -NHC(=O)-**,-NHC(=O)O-**, -NHC(=O)NH-**, -OC(=O)NH-**, -S(O)₂NH-**,-NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 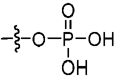 groups;
   A is a bond, -OC(=O)-*, 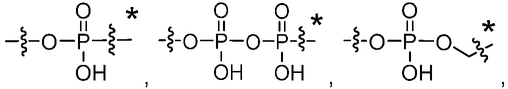 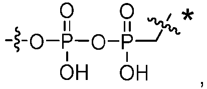 -OC(=O)N(CH₃)CH₂CH₂N(CH₃)C(=O)-* or - OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D.
**Embodiment 37.** The linker of any one of Embodiments 32 to 36, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 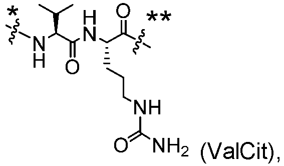 where the * of Lp indicates the attachment point to L₁ and the ** of Lp indicates the attachment point to the -NH- group of G;
   L₃ is a spacer moiety having the structure 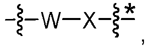 where
      W is -CH₂O-**, -CH2N(Rb)C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**,-CH₂NR^{b}C(=O)NH-**, -CH₂NR^{b}C(=O)NR^{b}-**, -NHC(=O)-**,-NHC(=O)O-**, -NHC(=O)NH-**, -OC(=O)NH-**, -S(O)₂NH-**,-NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 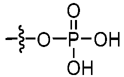 groups;
      and
   A is a bond, -OC(=O)-*, 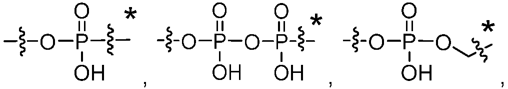 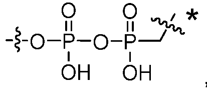 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D.
**Embodiment 38.** The linker of any one of Embodiments 32 to 37, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 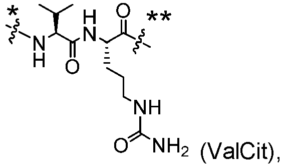 where the * of Lp indicates the attachment point to L₁;
   L₃ is a spacer moiety having the structure 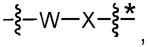 where
      W is -CH₂O-**, -CH2N(Rb)C(=O)O-**, -NHC(=O)CH₂NHC(=O)O-**, -CH₂N(X-R²)C(=O)O-**, -C(=O)N(X-R²)-**, -C(=O)NR^{b}-**, - C(=O)NH-**, -CH₂NR^{b}C(=O)-**, -CH₂NR^{b}C(=O)NH-**, - CH₂NR^{b}C(=O)NR^{b}-**, -NHC(=O)-**, -NHC(=O)O-**, or -NHC(=O)NH-**, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 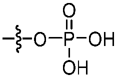 groups;
      and
   A is a bond or -OC(=O)* in which * indicates the attachment point to D.
**Embodiment 39.** The linker of any one of Embodiments 32 to 38, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 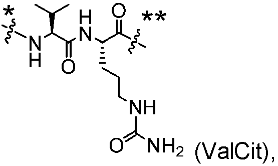 where the * of Lp indicates the attachment point to L₁;
   L₃ is a spacer moiety having the structure 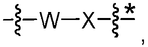 where
      W is -CH₂O-**, -CH2N(Rb)C(=O)O-**, -NHC(=O)CH₂NHC(=O)O-**, -CH₂N(X-R²)C(=O)O-**, or -C(=O)N(X-R²)-**, wherein each R^{b} is independently selected from H, C₁-C₆alkyl or C₃-C₈cycloalkyl and wherein the ** of W indicates the point of attachment to X;
      X is ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 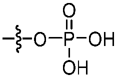 groups;
      and
   A is a bond or -OC(=O)* in which * indicates the attachment point to D.
**Embodiment 40.** The linker of Formula (C') having the structure having the structure of Formula (D'), 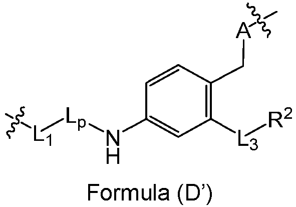 wherein
   L₁ is a bridging spacer;
   Lp is a bivalent peptide spacer;
   R² is a hydrophilic moiety;
   A is a bond, -OC(=O)-*, 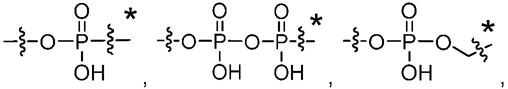 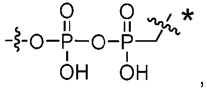 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D,
      and
   L₃ is a spacer moiety.
**Embodiment 41.** The linker of Embodiments 40, wherein:
   L₁ is a bridging spacer;
   Lp is a bivalent peptide spacer comprising two to four amino acid residues;
   R² is a hydrophilic moiety;
   A is a bond, -OC(=O)-*, 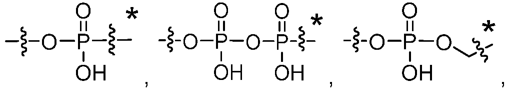 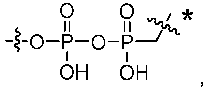 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D,
      and
   L₃ is a spacer moiety.
**Embodiment 42.** The linker of Embodiment 40 or 41, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH₂)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH₂)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of attachment to Lp;
   R² is a hydrophilic moiety selected from polyethylene glycol, polyalkylene glycol, a sugar, an oligosaccharide, a polypeptide or C₂-C₆alkyl substituted with 1 to 3 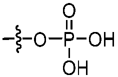 groups;
   each R³ is independently selected from H and C₁-C₆alkyl;
   X₁ is 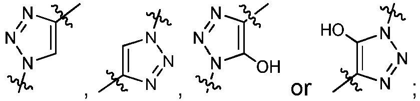
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer comprising an amino acid residue selected from glycine, valine, citrulline, lysine, isoleucine, phenylalanine, methionine, asparagine, proline, alanine, leucine, tryptophan, and tyrosine;
   A is a bond, -OC(=O)-*, 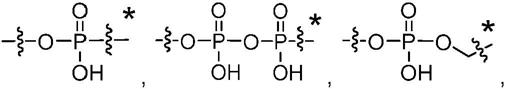 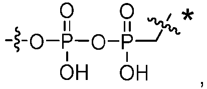 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
   L₃ is a spacer moiety having the structure 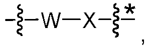 where
      W is -CH₂O-**, -CH2N(Rb)C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, - CH₂NR^{b}C(=O)NH-**, -CH₂NR^{b}C(=O)NR^{b}-**, -NHC(=O)-**, - NHC(=O)O-**, -NHC(=O)NH-**, -OC(=O)NH-**, -S(O)₂NH-**, - NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R².
**Embodiment 43.** The linker of any one of Embodiments 40 to 42, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 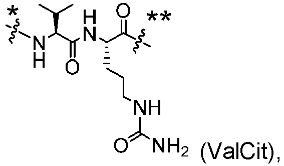 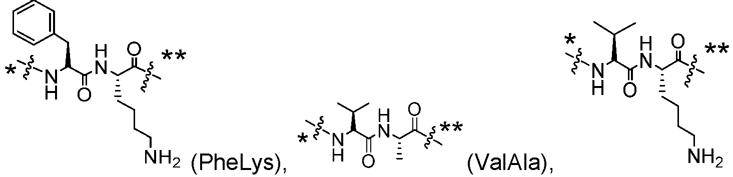 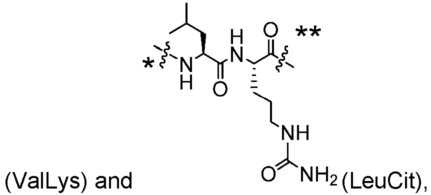 where the * of Lp indicates the attachment point to L₁ and the ** of Lp indicates the attachment point to the - NH- group of G;
   L₃ is a spacer moiety having the structure 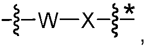 where
      W is -CH₂O-**, -CH2N(Rb)C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, - CH₂NR^{b}C(=O)NH-**, -CH₂NR^{b}C(=O)NR^{b}-**, -NHC(=O)-**, - NHC(=O)O-**, -NHC(=O)NH-**, -OC(=O)NH-**, -S(O)₂NH-**, - NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 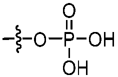 groups;
      and
   A is a bond, -OC(=O)-*, 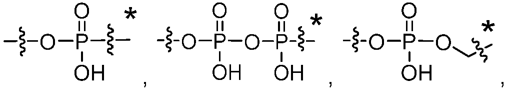 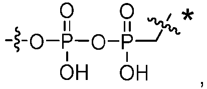 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D.
**Embodiment 44.** The linker of any one of Embodiments 40 to 43, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 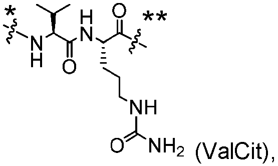 where the * of Lp indicates the attachment point to L₁ and the ** of Lp indicates the attachment point to the -NH- group of G;
   L₃ is a spacer moiety having the structure 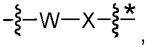 where
      W is -CH₂O-**, -CH₂N(Rb)C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, - CH₂NR^{b}C(=O)NH-**, -CH₂NR^{b}C(=O)NR^{b}-**, -NHC(=O)-**, - NHC(=O)O-**, -NHC(=O)NH-**, -OC(=O)NH-**, -S(O)₂NH-**, - NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
      X is a bond, triazolyl or ***-CH2-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 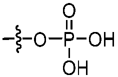 groups;
      and
   A is a bond, -OC(=O)-*, 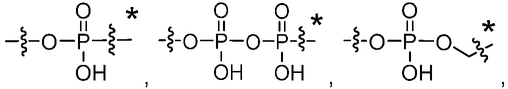 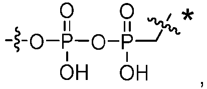 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D.
**Embodiment 45.** The linker of any one of Embodiments 40 to 44, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 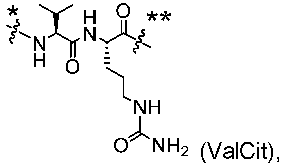 where the * of Lp indicates the attachment point to L₁ and the ** of Lp indicates the attachment point to the -NH- group of G;
   L₃ is a spacer moiety having the structure 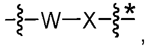 where
      W is -CH₂O-**, -CH₂N(Rb)C(=O)O-**, -NHC(=O)CH₂NHC(=O)O-**, -CH₂N(X-R²)C(=O)O-**, -C(=O)N(X-R²)-**, -C(=O)NR^{b}-**, - C(=O)NH-**, -CH₂NR^{b}C(=O)-**, -CH₂NR^{b}C(=O)NH-**, - CH₂NR^{b}C(=O)NR^{b}-**, -NHC(=O)-**, -NHC(=O)O-**, or -NHC(=O)NH-**, wherein each R^{b} is independently selected from H, C₁-C₆alkyl or C₃-C₈cycloalkyl and wherein the ** of W indicates the point of attachment to X;
      X is a bond, triazolyl or ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 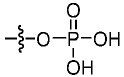 groups;
      and
   A is a bond or -OC(=O)* in which * indicates the attachment point to D.
**Embodiment 46.** The linker of any one of Embodiments 40 to 45, wherein:
   L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp;
   each m is independently selected from 1, 2, 3, 4, 5, 6, 7, 8, 9 and 10;
   each n is independently selected from 1, 2, 3, 4, 5, 6, 7, 8, 9 and 10;
   each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
   Lp is a bivalent peptide spacer selected from 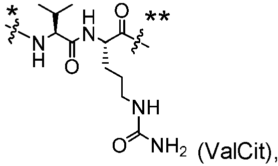 where the * of Lp indicates the attachment point to L₁ and the ** of Lp indicates the attachment point to the -NH- group of G;
   L₃ is a spacer moiety having the structure 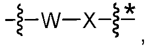 where
      W is -CH₂O-**, -CH₂N(Rb)C(=O)O-**, -NHC(=O)CH₂NHC(=O)O-**, -CH₂N(X-R²)C(=O)O-**, or -C(=O)N(X-R²)-**, wherein each R^{b} is independently selected from H, C₁-C₆alkyl or C₃-C₈cycloalkyl and wherein the ** of W indicates the point of attachment to X;
      X is ***-CH₂-triazolyl-*, wherein the *** of X indicates the point of attachment to W and the * of X indicates the point of attachment to R²;
         and
      the * of L₃ indicates the point of attachment to R²;
   R² is a hydrophilic moiety selected from polyethylene glycol, polyalkylene glycol, a sugar, an oligosaccharide, a polypeptide or C₂-C₆alkyl substituted with 1 to 3 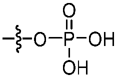 groups;
      and
   A is a bond or -OC(=O)* in which * indicates the attachment point to D.
**Embodiment 47.** The linker of any one of Embodiments 32 to 46, having the structure: 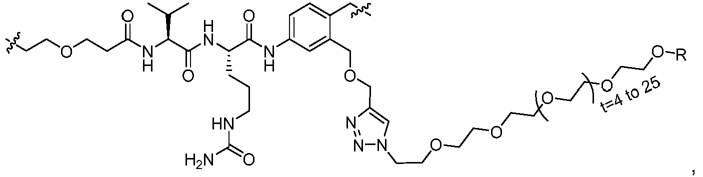 where
   R is H, -CH₃ or -CH₂CH₂C(=O)OH.
**Embodiment 48.** The linker of any one of Embodiments 32 to 46, having the structure: 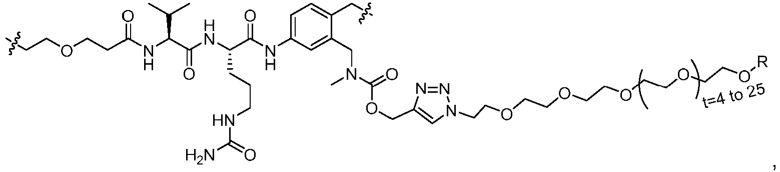 where
   R is H, -CH₃ or -CH₂CH₂C(=O)OH.
**Embodiment 49.** The linker of any one of Embodiments 32 to 46, having the structure: 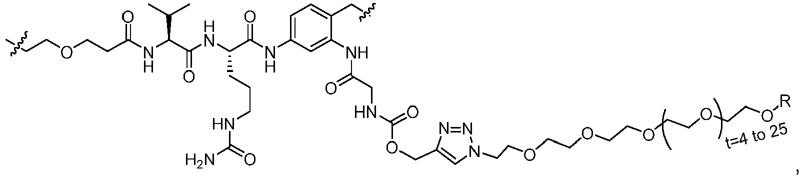 where
   R is H, -CH₃ or -CH₂CH₂C(=O)OH.
**Embodiment 50.** The linker of any one of Embodiments 32 to 46, having the structure: 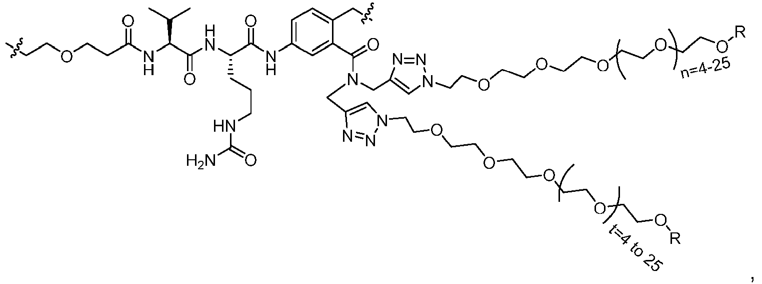 where
   each R is independently selected from H, -CH₃ or -CH₂CH₂C(=O)OH.
**Embodiment 51.** The linker of any one of Embodiments 32 to 46, having the structure: 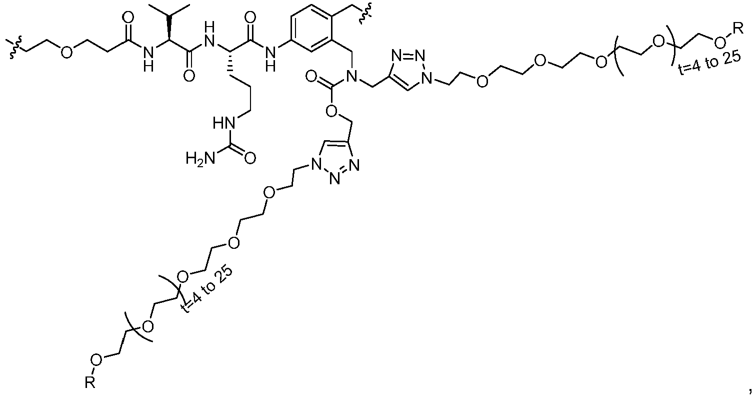 where
   each R is independently selected from H, -CH₃ or -CH₂CH₂C(=O)OH.
**Embodiment 52.** The linker of any one of Embodiments 32 to 46, having the structure: 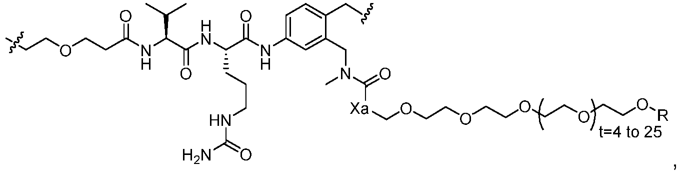 where
   Xa is -CH₂-, -OCH₂-, -NHCH₂- or -NRCH₂- and each R independently is H, -CH₃ or - CH₂CH₂C(=O)OH.
**Embodiment 53.** The linker of any one of Embodiments 32 to 46, having the structure: 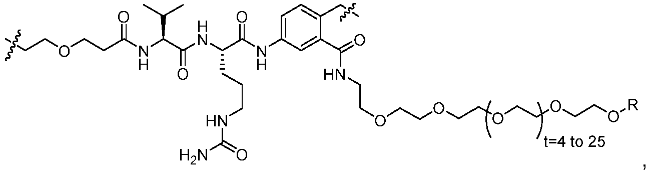 where
   R is H, -CH₃ or -CH₂CH₂C(=O)OH.
**Embodiment 54.** The linker of any one of Embodiments 32 to 46, having the structure: 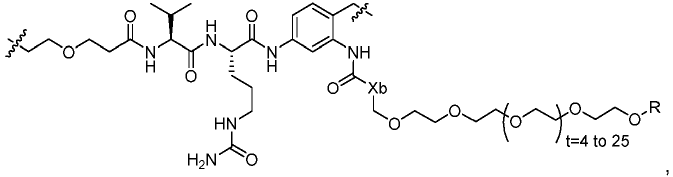 where
   Xb is -CH₂-, -OCH₂-, -NHCH₂- or -NRCH₂- and each R independently is H, -CH₃ or - CH₂CH₂C(=O)OH.
**Embodiment 55.** The linker of any one of Embodiments 32 to 46, having the structure: 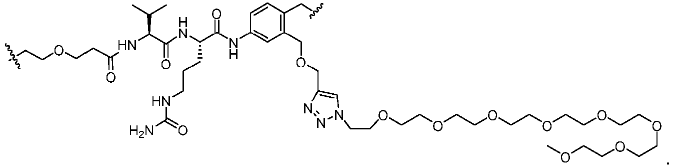
**Embodiment 56.** The linker of any one of Embodiments 32 to 46, having the structure: 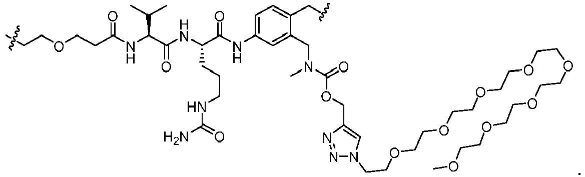
**Embodiment 57.** The linker of any one of Embodiments 32 to 46, having the structure: 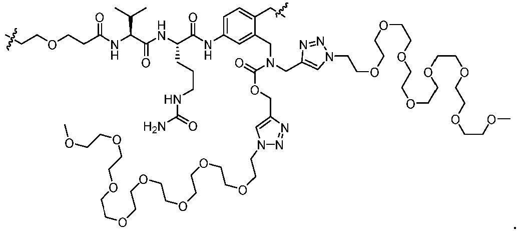
**Embodiment 58.** The linker of any one of Embodiments 32 to 46, having the structure: 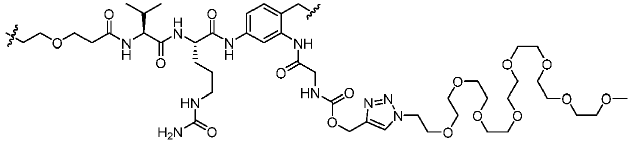
**Embodiment 59.** The linker of any one of Embodiments 32 to 46, having the structure:

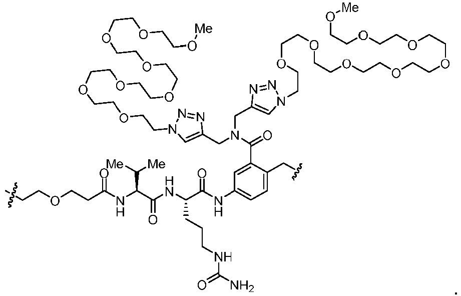

For illustrative purposes, the general reaction schemes depicted herein provide potential routes for synthesizing the compounds of the present invention as well as key intermediates. For a more detailed description of the individual reaction steps, see the Examples section below. Although specific starting materials and reagents are depicted in the schemes and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

By way of example, a general synthesis for compounds of Formula (B') is shown below in Scheme 1.

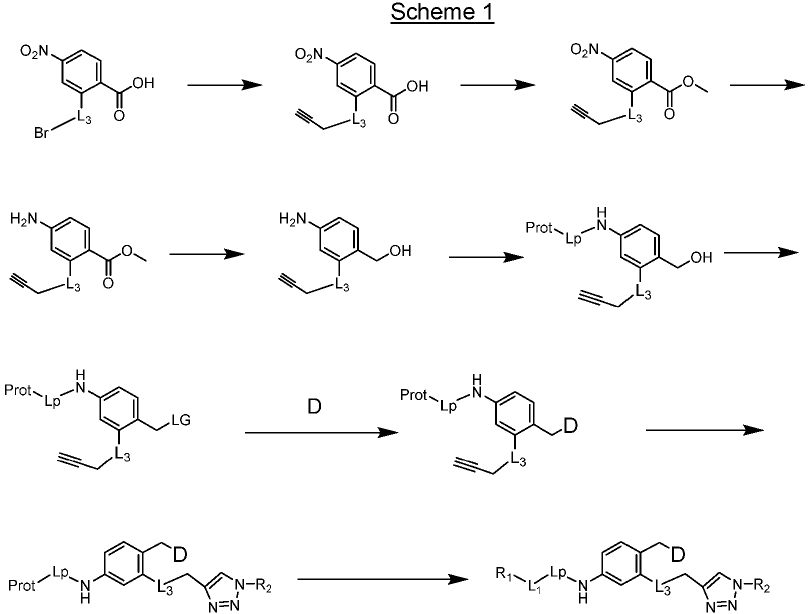

### Antibody Drug Conjugates of the Invention

The present invention provides Antibody Drug Conjugates, also referred to herein as immunoconjugates, which comprise linkers which comprise one or more hydrophilic moieties.

The Antibody Drug Conjugates of the invention have the structure of Formula (E'): 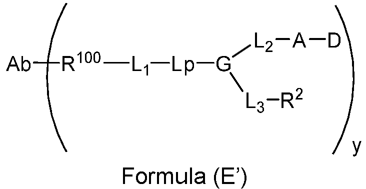 wherein:
Ab is an antibody or fragment thereof;
R¹⁰⁰ is a coupling group;
L₁ is a bridging spacer;
Lp is a bivalent peptide spacer;
G-L₂-A is a self-immolative spacer;
R² is a hydrophilic moiety;
L₂ is a bond, a methylene, a neopentylene or a C₂-C₃alkenylene;
A is a bond, -OC(=O)-*, 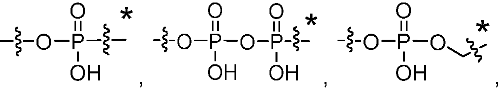 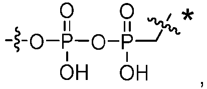 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety;
D is a Drug moiety as defined herein, e.g., a MCI-1 inhibitor, and may comprise an N or an O, wherein D can be connected to A via a direct bond from A to the N or the O of the Drug moiety,
   and
y is 1, 2, 3, 4, 5, 6, 7, 8, 9, 10, 11, 12, 13, 14, 15 or 16.

Certain aspects and examples of the Antibody Drug Conjugates of the invention are provided in the following listing of enumerated embodiments. It will be recognized that features specified in each embodiment may be combined with other specified features to provide further embodiments of the present invention.

**Embodiment 60.** The immunoconjugate of Formula (E') wherein:
Ab is an antibody or fragment thereof;
R¹⁰⁰ is a coupling group;
L₁ is a bridging spacer;
Lp is a bivalent peptide spacer comprising two to four amino acid residues;
G-L₂-A is a self-immolative spacer;
R² is a hydrophilic moiety;
L₂ is a bond, a methylene, a neopentylene or a C₂-C₃alkenylene;
A is a bond, -OC(=O)-*, 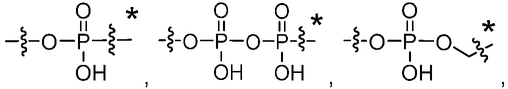 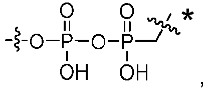 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety;
D is a Drug moiety as defined herein wherein D is connected to A via a direct bond from A to D (e.g., an N or O of the Drug moiety),
   and
y is 1, 2, 3, 4, 5, 6, 7, 8, 9, 10, 11, 12, 13, 14, 15 or 16.

**Embodiment 61.** The immunoconjugate of Formula (E') or Embodiment 60, wherein:
Ab is an antibody or fragment thereof;
R¹⁰⁰ is a coupling group;
L₁ is a bridging spacer;
Lp is a bivalent peptide spacer comprising two to four amino acid residues;
the 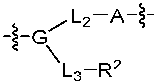 group is selected from: 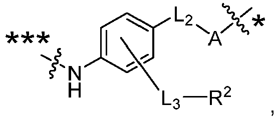 wherein the * of 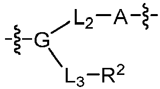 indicates the point of attachment to D (e.g., to an N or a O of the Drug moiety), the *** of 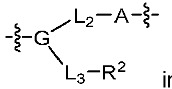 idicates the point of attachment to Lp;
R² is a hydrophilic moiety;
L₂ is a bond, a methylene, a neopentylene or a C₂-C₃alkenylene;
A is a bond, -OC(=O)-*, 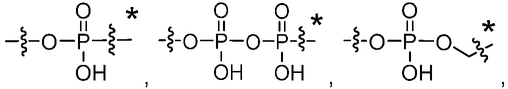 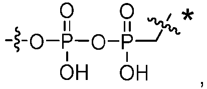 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 62.** The immunoconjugate of Formula (E') or any one of Embodiments 60 to 61 having the structure of Formula (F'), 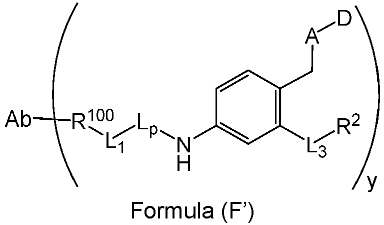 wherein:
Ab is an antibody or fragment thereof;
R¹⁰⁰ is a coupling group;
L₁ is a bridging spacer;
Lp is a bivalent peptide spacer comprising two to four amino acid residues;
R² is a hydrophilic moiety;
A is a bond, -OC(=O)-*, 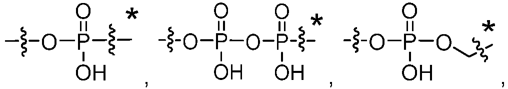 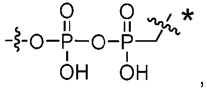 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 63.** The immunoconjugate of Formula (D') or any one of Embodiments 60 to 62, wherein:
Ab is an antibody or fragment thereof;
R¹⁰⁰ is 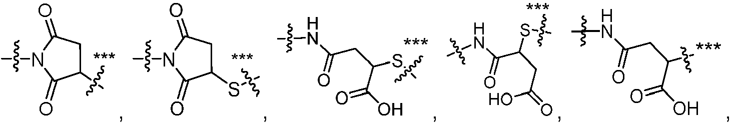 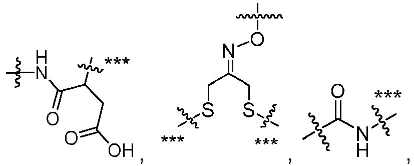 -S-, -C(=O)-, -ON=***, - NHC(=O)CH₂-***, -S(=O)₂CH₂CH₂-***, -(CH₂)₂S(=O)₂CH₂CH₂-***, - NHS(=O)₂CH₂CH₂₋***, -NHC(=O)CH₂CH₂-***, -CH₂NHCH₂CH₂-***, -NHCH₂CH₂-***, 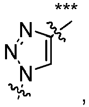 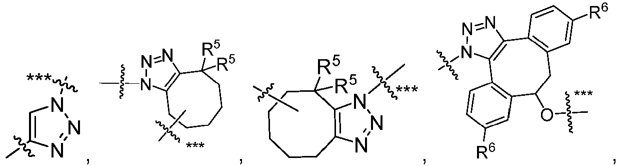 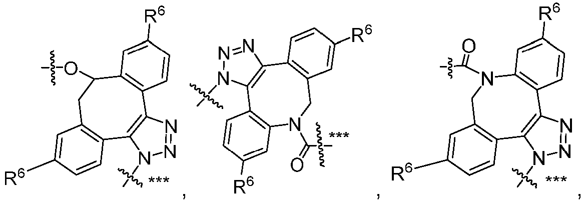 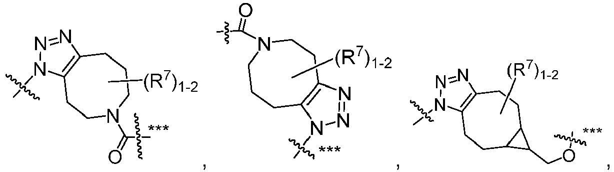 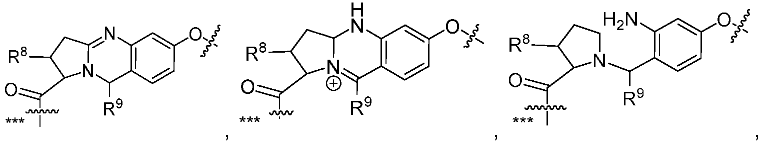 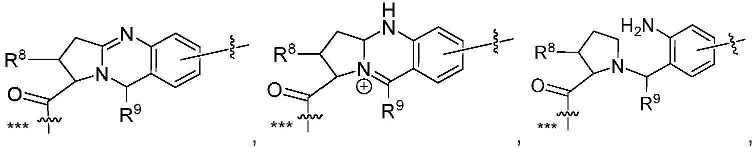 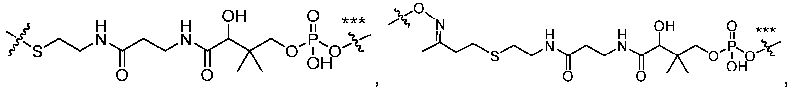 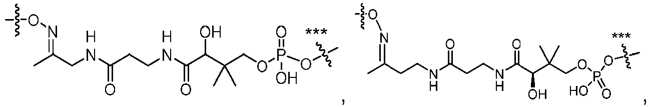 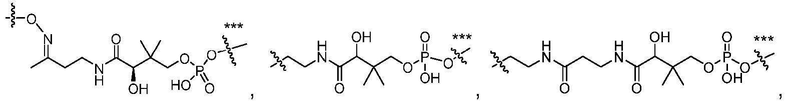 or 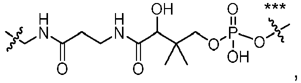 where the *** of R¹⁰⁰ indicates the point of attachment to Ab;
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH₂)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH₂)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of attachment to Lp, and the ** of L₁ indicates the point of attachment to R¹⁰⁰;
R² is a hydrophilic moiety selected from polyethylene glycol, polyalkylene glycol, a sugar, an oligosaccharide, a polypeptide or C₂-C₆alkyl substituted with 1 to 3 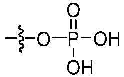 groups;
each R³ is independently selected from H and C₁-C₆alkyl;
R⁴ is 2-pyridyl or 4-pyridyl;
each R⁵ is independently selected from H, C₁-C₆alkyl, F, Cl, and -OH;
each R⁶ is independently selected from H, C₁-C₆alkyl, F, Cl, -NH₂, -OCH₃, -OCH₂CH₃, -N(CH₃)₂, -CN, -NO₂ and -OH;
each R⁷ is independently selected from H, C₁₋₆alkyl, fluoro, benzyloxy substituted with -C(=O)OH, benzyl substituted with -C(=O)OH, C₁₋₄alkoxy substituted with -C(=O)OH and C₁₋₄alkyl substituted with -C(=O)OH;
X₁ is 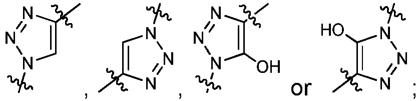
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer comprising an amino acid residue selected from valine, citrulline, lysine, isoleucine, phenylalanine, methionine, asparagine, proline, alanine, leucine, tryptophan, and tyrosine;
A is a bond, -OC(=O)-*, 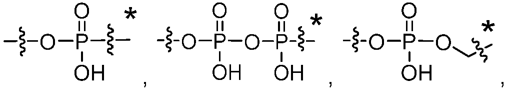 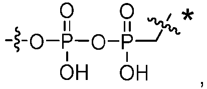 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
L₃ is a spacer moiety having the structure 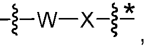 where
   W is -CH₂O-**, -CH₂N(Rb)C(=O)O-**, -NHC(=O)C(R^{b})₂NHC(=O)O-**, -NHC(=O)C(R^{b})₂NH-**, -NHC(=O)C(R^{b})₂NHC(=O)-**, -CH₂N(X-R²)C(=O)O-**, -C(=O)N(X-R²)-**, -CH₂N(X-R²)C(=O)-**, - C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**, - NHC(=O)NH-**, -OC(=O)NH-**, -S(O)₂NH-**, -NHS(O)₂-**, -C(=O)- , -C(=O)O-** or -NH-, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
      and
   the * of L₃ indicates the point of attachment to R²;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 64.** The immunoconjugate of Formula (D') or any one of Embodiments 60 to 63, wherein:
Ab is an antibody or fragment thereof;
R¹⁰⁰ is 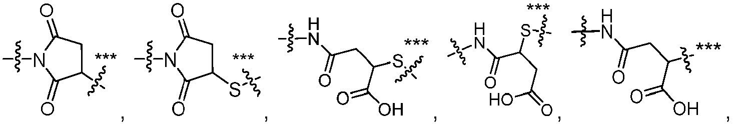 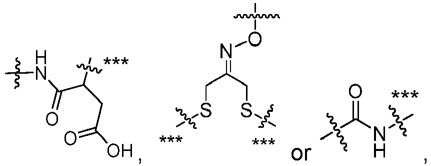 where the *** of R¹⁰⁰ indicates the point of attachment to Ab;
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp, and the ** of L₁ indicates the point of attachment to R¹⁰⁰;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from 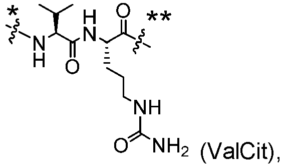 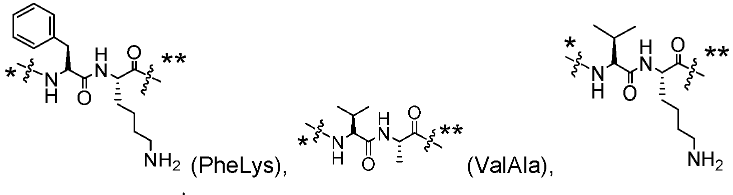 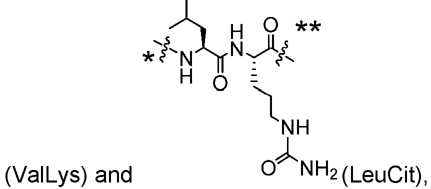 where the * of Lp indicates the attachment point to L₁ and the ** of Lp indicates the attachment point to the - NH- group of G;
L₃ is a spacer moiety having the structure 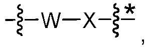 where
   W is -CH₂O-**, -CH₂N(Rb)C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, - C(=O)NR^{b}-**, -C(=O)NH-**, -CH₂NR^{b}C(=O)-**, -CH₂NR^{b}C(=O)NH-**, - CH₂NR^{b}C(=O)NR^{b}-**, -NHC(=O)-**, -NHC(=O)O-**, -NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
      and
   the * of L₃ indicates the point of attachment to R²;
R² is a hydrophilic moiety selected from polyethylene glycol, polyalkylene glycol, a sugar, an oligosaccharide, a polypeptide or C₂-C₆alkyl substituted with 1 to 3 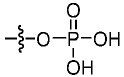 groups;
A is a bond, -OC(=O)-*, 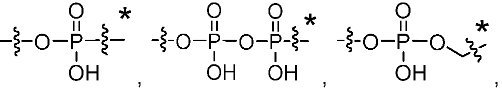 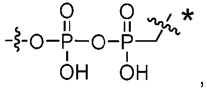 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 65.** The immunoconjugate of Formula (E') or any one of Embodiments 60 to 64, wherein:
Ab is an antibody or fragment thereof;
R¹⁰⁰ is 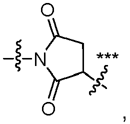 where the *** of R¹⁰⁰ indicates the point of attachment to Ab;
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp, and the ** of L₁ indicates the point of attachment to R¹⁰⁰;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from 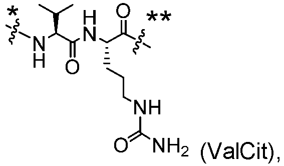 where the * of Lp indicates the attachment point to L₁ and the ** of Lp indicates the attachment point to the -NH- group of G;
L₃ is a spacer moiety having the structure 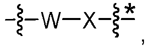 where
   W is -CH₂O-**, -CH₂N(Rb)C(=O)O-**, -NHC(=O)CH₂NHC(=O)O-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, - CH₂NR^{b}C(=O)NH-**, -CH₂NR^{b}C(=O)NR^{b}-**, -NHC(=O)-**, - NHC(=O)O-**, -NHC(=O)NH-**, -OC(=O)NH-**, -S(O)₂NH-**, - NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
      and
   the * of L₃ indicates the point of attachment to R²;
R² is a hydrophilic moiety selected from polyethylene glycol, polyalkylene glycol, a sugar, an oligosaccharide, a polypeptide or C₂-C₆alkyl substituted with 1 to 3 A is a bond, -OC(=O)-*, 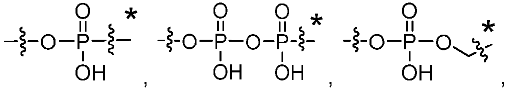 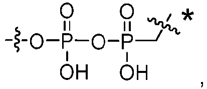 -OC(=O)N(CH₃)CH₂CH₂N(CH₃)C(=O)-* or -OC(=O)N(CH₃)C(R^{a})₂C(R^{a})₂N(CH₃)C(=O)-*, wherein each R^{a} is independently selected from H, C₁-C₆ alkyl, and C₃-C₈ cycloalkyl and the * of A indicates the point of attachment to D;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 66.** The immunoconjugate of Formula (E') or any one of Embodiments 60 to 65, wherein:
Ab is an antibody or fragment thereof;
R¹⁰⁰ is 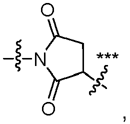 where the *** of R¹⁰⁰ indicates the point of attachment to Ab;
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp and the ** of L₁ indicates the point of attachment to R¹⁰⁰;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from 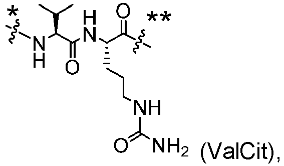 where the * of Lp indicates the attachment point to L₁ and the ** of Lp indicates the attachment point to the -NH- group of G;
L₃ is a spacer moiety having the structure 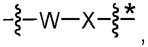 where
   W is -CH₂O-**, -CH₂N(Rb)C(=O)O-**, -NHC(=O)CH₂NHC(=O)O-**, -CH₂N(X-R²)C(=O)O-**, -C(=O)N(X-R²)-**, -C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**, or - NHC(=O)NH-**, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
      and
   the * of L₃ indicates the point of attachment to R²;
R² is a hydrophilic moiety selected from polyethylene glycol, polyalkylene glycol, a sugar, an oligosaccharide, a polypeptide or C₂-C₆alkyl substituted with 1 to 3 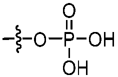 groups;
A is a bond or -OC(=O)* in which * indicates the attachment point to D;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 67.** The immunoconjugate of Formula (E') or any one of Embodiments 60 to 66, wherein:
Ab is an antibody or fragment thereof;
R¹⁰⁰ is 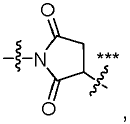 where the *** of R¹⁰⁰ indicates the point of attachment to Ab;
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; or *-C(=O)NH((CH₂)ₘO)ₜ(CH₂)ₙ-, where the * of L₁ indicates the point of attachment to Lp and the ** of L₁ indicates the point of attachment to R¹⁰⁰;
each m is independently selected from 1, 2, 3, 4, 5, 6, 7, 8, 9 and 10;
each n is independently selected from 1, 2, 3, 4, 5, 6, 7, 8, 9 and 10;
each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30;
Lp is a bivalent peptide spacer selected from 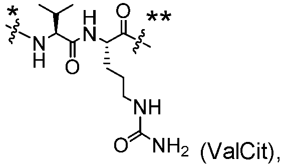 where the * of Lp indicates the attachment point to L₁ and the ** of Lp indicates the attachment point to the -NH- group of G;
L₃ is a spacer moiety having the structure 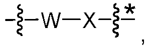 where
   W is -CH₂O-**, -CH₂N(Rb)C(=O)O-**, -NHC(=O)CH₂NHC(=O)O-**, -CH₂N(X-R²)C(=O)O-**, or -C(=O)N(X-R²)-**, wherein each R^{b} is independently selected from H, C₁-C₆alkyl or C₃-C₈cycloalkyl and wherein the ** of W indicates the point of attachment to X;
   X is ***-CH₂-triazolyl-*, wherein the *** of X indicates the point of attachment to W and the * of X indicates the point of attachment to R²;
      and
   the * of L₃ indicates the point of attachment to R²;
R² is a hydrophilic moiety selected from polyethylene glycol, polyalkylene glycol, a sugar, an oligosaccharide, a polypeptide or C₂-C₆alkyl substituted with 1 to 3 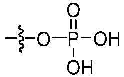 groups;
A is a bond or -OC(=O)* in which * indicates the attachment point to D;
D is a Drug moiety as defined herein and comprising an N or an O, wherein D is connected to A via a direct bond from A to the N or the O of the Drug moiety,
   and
y is 1, 2, 3, 4, 5, 6, 7, 8, 9, 10, 11, 12, 13, 14, 15 or 16.

**Embodiment 68.** The immunoconjugate of Formula (E') or any one of Embodiments 60 to 63, wherein
R¹⁰⁰ is 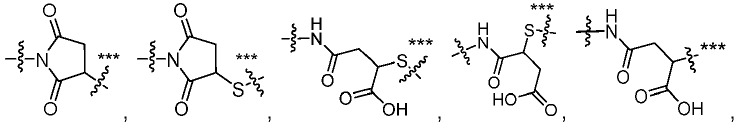 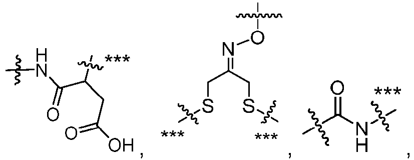 -S-, -C(=O)-, -ON=***, -NHC(=O)CH₂-***, -S(=O)₂CH₂CH₂-***, -(CH₂)₂S(=O)₂CH₂CH₂-***, -NHS(=O)₂CH₂CH₂₋***, -NHC(=O)CH₂CH₂-***, -CH₂NHCH₂CH₂-***, -NHCH₂CH₂-***, 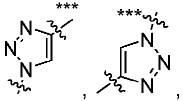 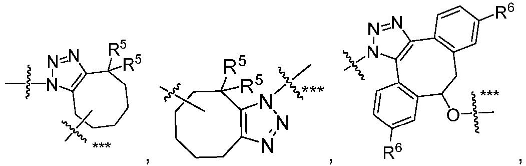 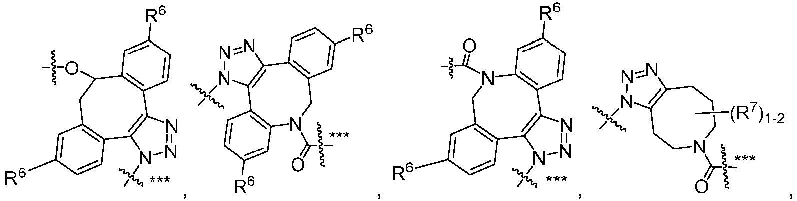 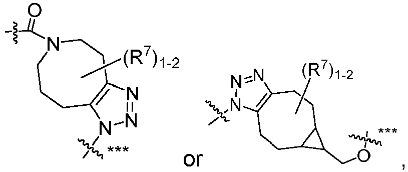 where the *** of R¹⁰⁰ indicates the point of attachment to Ab.

**Embodiment 69.** The immunoconjugate of Formula (E') or any one of Embodiments 60 to 63, wherein
R¹⁰⁰ is 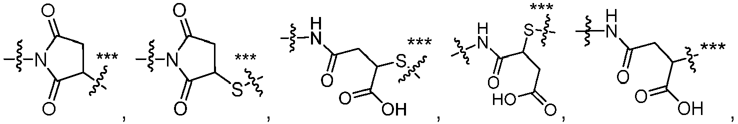 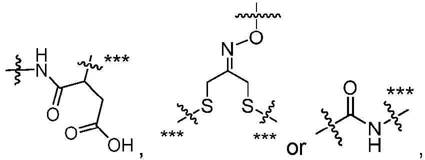 where the *** of R¹⁰⁰ indicates the point of attachment to Ab.

**Embodiment 70.** The immunoconjugate of Formula (E') or any one of Embodiments 60 to 63, wherein
R¹⁰⁰ is 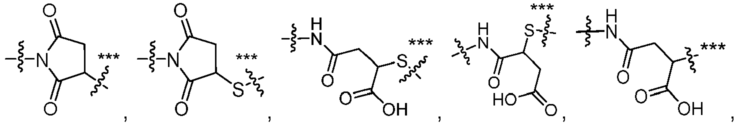 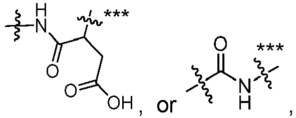 where the *** of R¹⁰⁰ indicates the point of attachment to Ab.

**Embodiment 71.** The immunoconjugate of Formula (E') or any one of Embodiments 60 to 70 having the structure: 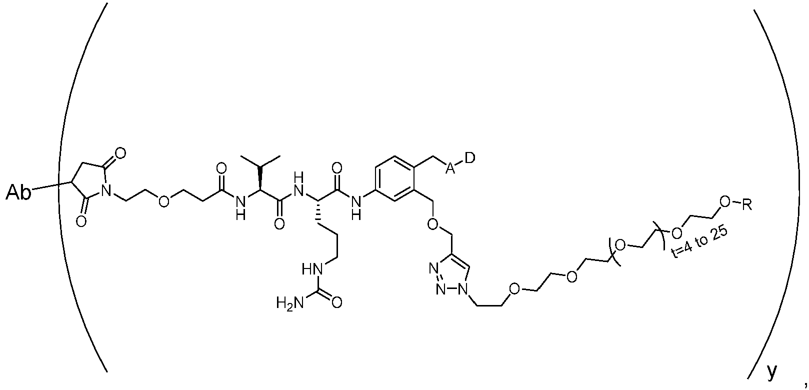 where
R is H, -CH₃ or -CH₂CH₂C(=O)OH and y is 1, 2, 3, 4, 5, 6, 7, 8, 9, 10, 11, 12, 13, 14, 15 or 16.

**Embodiment 72.** The immunoconjugate of Formula (E') or any one of Embodiments 60 to 70 having the structure: 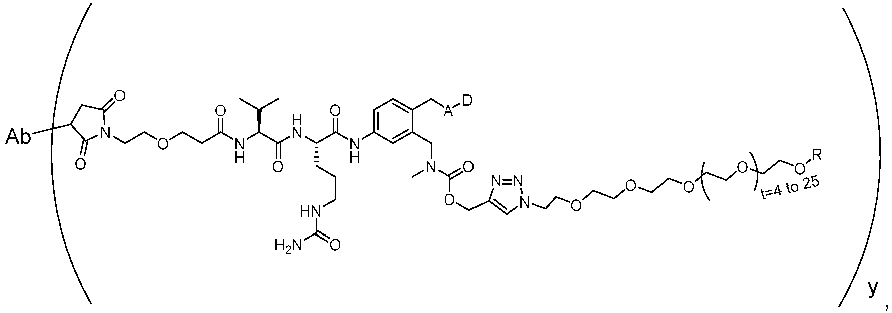 where
R is H, -CH₃ or -CH₂CH₂C(=O)OH and y is 1, 2, 3, 4, 5, 6, 7, 8, 9, 10, 11, 12, 13, 14, 15 or 16.

**Embodiment 73.** The immunoconjugate of Formula (E') or any one of Embodiments 60 to 70 having the structure: 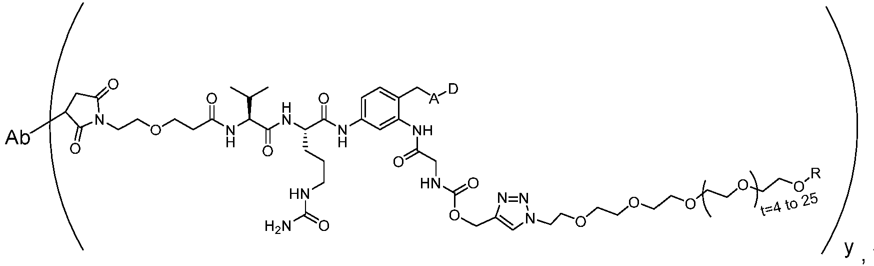 where
R is H, -CH₃ or -CH₂CH₂C(=O)OH and y is 1, 2, 3, 4, 5, 6, 7, 8, 9, 10, 11, 12, 13, 14, 15 or 16.

**Embodiment 74.** The immunoconjugate of Formula (E') or any one of Embodiments 60 to 70 having the structure: 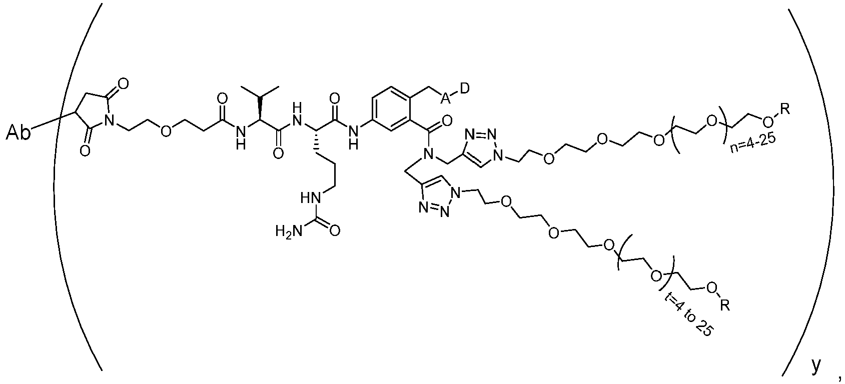 where
each R is independently selected from H, -CH₃ or -CH₂CH₂C(=O)OH and y is 1, 2, 3, 4, 5, 6, 7, 8, 9, 10, 11, 12, 13, 14, 15 or 16.

**Embodiment 75.** The immunoconjugate of Formula (E') or any one of Embodiments 60 to 70 having the structure: 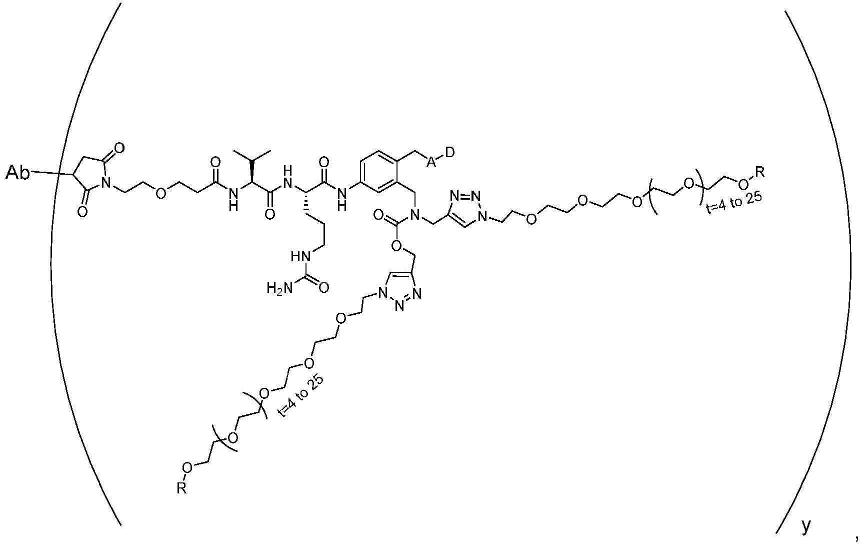 where
each R is independently selected from H, -CH₃ or -CH₂CH₂C(=O)OH and y is 1, 2, 3, 4, 5, 6, 7, 8, 9, 10, 11, 12, 13, 14, 15 or 16.

**Embodiment 76.** The immunoconjugate of Formula (E') or any one of Embodiments 60 to 70 having the structure: 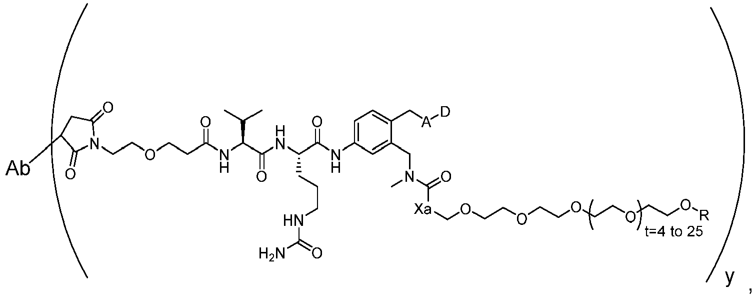 where
Xa is -CH₂-, -OCH₂-, -NHCH₂- or -NRCH₂- and each R is independently H, -CH₃ or -CH₂CH₂C(=O)OH and y is 1, 2, 3, 4, 5, 6, 7, 8, 9, 10, 11, 12, 13, 14, 15 or 16.

**Embodiment 77.** The immunoconjugate of Formula (E') or any one of Embodiments 60 to 70 having the structure: 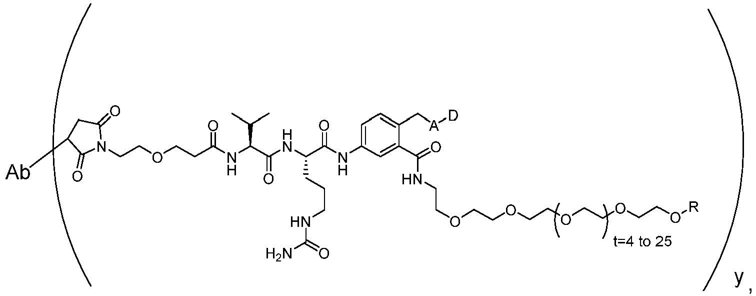 where
R is H, -CH₃ or -CH₂CH₂C(=O)OH and y is 1, 2, 3, 4, 5, 6, 7, 8, 9, 10, 11, 12, 13, 14, 15 or 16.

**Embodiment 78.** The immunoconjugate of Formula (E') or any one of Embodiments 60 to 70 having the structure: 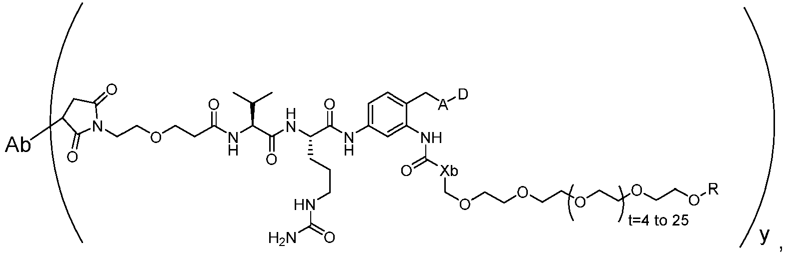 where
Xb is -CH₂-, -OCH₂-, -NHCH₂- or -NRCH₂- and each R independently is H, -CH₃ or - CH₂CH₂C(=O)OH and y is 1, 2, 3, 4, 5, 6, 7, 8, 9, 10, 11, 12, 13, 14, 15 or 16.

**Embodiment 79.** The immunoconjugate of Formula (E') or any one of Embodiments 60 to 70 having the structure: 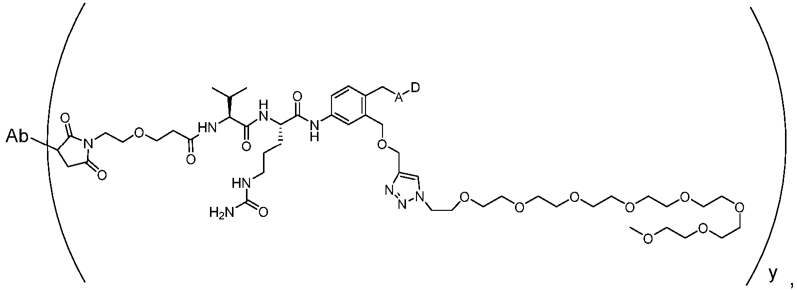 where y is 1, 2, 3, 4, 5, 6, 7, 8, 9, 10, 11, 12, 13, 14, 15 or 16.

**Embodiment 80.** The immunoconjugate of Formula (E') or any one of Embodiments 60 to 70 having the structure: 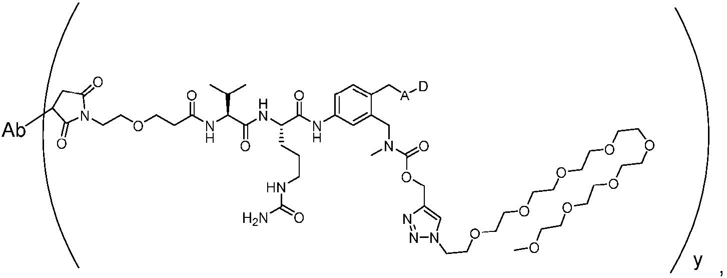 where y is 1, 2, 3, 4, 5, 6, 7, 8, 9, 10, 11, 12, 13, 14, 15 or 16.

**Embodiment 81.** The immunoconjugate of Formula (E') or any one of Embodiments 60 to 70 having the structure: 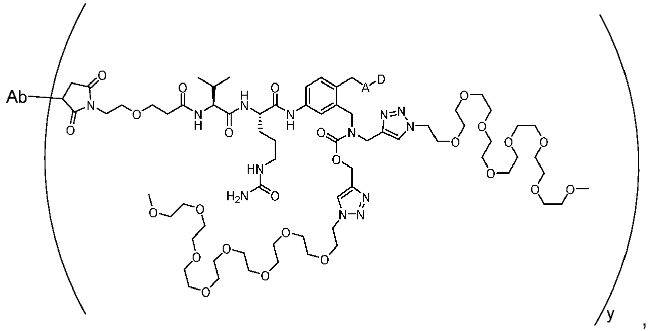 where y is 1, 2, 3, 4, 5, 6, 7, 8, 9, 10, 11, 12, 13, 14, 15 or 16.

**Embodiment 82.** The immunoconjugate of Formula (E') or any one of Embodiments 60 to 70 having the structure: 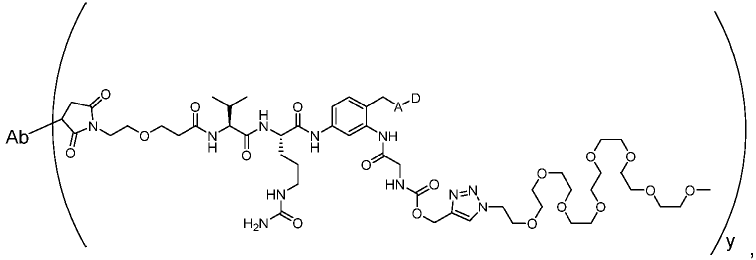 where y is 1, 2, 3, 4, 5, 6, 7, 8, 9, 10, 11, 12, 13, 14, 15 or 16.

**Embodiment 83.** The immunoconjugate of Formula (E') or any one of Embodiments 60 to 70 having the structure: 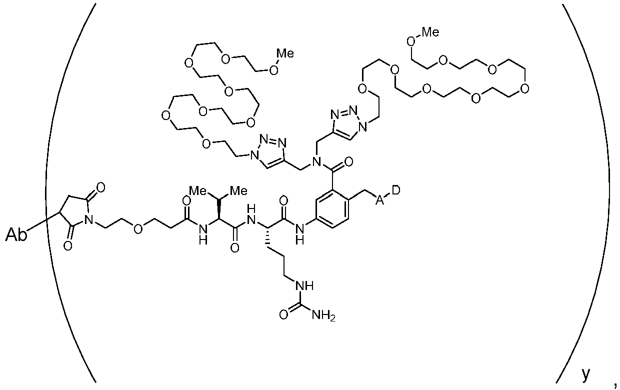 where y is 1, 2, 3, 4, 5, 6, 7, 8, 9, 10, 11, 12, 13, 14, 15 or 16.

Certain aspects and examples of the Linker-Drug groups, the Linkers and the Antibody Drug Conjugates of the invention are provided in the following listing of additional enumerated embodiments. It will be recognized that features specified in each embodiment may be combined with other specified features to provide further embodiments of the present invention.

**Embodiment 84.** The compound of Formula (A') or any one of Embodiments 1 to 2, or pharmaceutically acceptable salt thereof, the linker of Formula (C') or any one of Embodiments 32 to 39, and the immunoconjugate of Formula (E') or any one of Embodiments 60 to 61, wherein:
G is 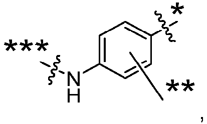 where the * of G indicates the point of attachment to L₂, and the ** of G indicates the point of attachment to L₃ and the *** of G indicates the point of attachment to Lp.

**Embodiment 85.** The compound of Formula (A') or any one of Embodiments 1 to 2, or pharmaceutically acceptable salt thereof, the linker of Formula (C') or any one of Embodiments 32 to 39, and the immunoconjugate of Formula (E') or any one of Embodiments 60 to 61, wherein:
G is 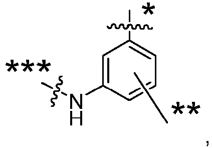 where the * of G indicates the point of attachment to L₂, and the ** of G indicates the point of attachment to L₃ and the *** of G indicates the point of attachment to Lp.

**Embodiment 86.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)O(CH₂)ₘSSC(R³)₂(CH₂)ₘC(=O)NR³(CH₂)ₘNR³C(=O)(CH₂)ₘ-**; *-C(=O)O(CH₂)ₘC(=O)NH(CH₂)ₘ-**; *-C(=O)(CH₂)ₘNH(CH₂)ₘ-**; *-C(=O)(CH₂)ₘNH(CH₂)ₙC(=O)-**; *-C(=O)(CH₂)ₘX₁(CH₂)ₘ-**; *-C(=O)((CH₂)ₘO)ₜ(CH₂)ₙX₁(CH₂)ₙ-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)(CH₂)ₘNHC(=O)(CH₂)ₙX₁(CH₂)ₙ-**; *-C(=O)((CH₂)ₘO)ₜ(CH₂)ₙNHC(=O)(CH₂)ₙX₁(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 87.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)(CH₂)ₘNH(CH₂)ₘ-**; *-C(=O)(CH₂)ₘNH(CH₂)ₙC(=O)-**; *-C(=O)(CH₂)ₘNHC(=O)(CH₂)ₙ-**; *-C(=O)((CH₂)ₘO)ₜ(CH₂)ₙNHC(=O)(CH₂)ₙ-**; *-C(=O)((CH₂)ₘO)ₜ(CH₂)ₙC(=O)NH(CH₂)ₘ-**; *-C(=O)(CH₂)ₘC(R³)₂-** or *-C(=O)(CH₂)ₘC(=O)NH(CH₂)ₘ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 88.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:
L₁ is *-C(=O)(CH₂)ₘO(CH₂)ₘ-**; *-C(=O)((CH₂)ₘO)ₜ(CH₂)ₙ-**; *-C(=O)(CH₂)ₘ-**; *-C(=O)NH((CH₂)ₘO)ₜ(CH₂)ₙ-**; *-C(=O)(CH₂)ₘNH(CH₂)ₘ-**; *-C(=O)(CH₂)ₘNH(CH₂)ₙC(=O)-**; or *-C(=O)(CH₂)ₘNHC(=O)(CH₂)ₙ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 89.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein:
L₁ is *-C(=O)(CH₂)ₘO(CH₂)ₘ-**; *-C(=O)((CH₂)ₘO)ₜ(CH₂)ₙ-**; *-C(=O)(CH₂)ₘ-** or *-C(=O)NH((CH₂)ₘO)ₜ(CH₂)ₙ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 90.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein L₁ is *-C(=O)(CH₂)ₘO(CH₂)ₘ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 91.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein L₁ is *-C(=O)((CH₂)ₘO)ₜ(CH₂)ₙ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 92.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein L₁ is *-C(=O)(CH₂)ₘ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 93.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, wherein L₁ is *-C(=O)NH((CH₂)ₘO)ₜ(CH₂)ₙ-**, where the * of L₁ indicates the point of attachment to Lp, and the ** of L₁ indicates the point of attachment to R¹ if present or the ** of L₁ indicates the point of attachment to R¹⁰⁰ if present.

**Embodiment 94.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 93, wherein Lp is an enzymatically cleavable bivalent peptide spacer.

**Embodiment 95.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 94, wherein Lp is a bivalent peptide spacer comprising an amino acid residue selected from glycine, valine, citrulline, lysine, isoleucine, phenylalanine, methionine, asparagine, proline, alanine, leucine, tryptophan, and tyrosine.

**Embodiment 96.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 95, wherein Lp is a bivalent peptide spacer comprising two to four amino acid residues.

**Embodiment 97.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 96, wherein Lp is a bivalent peptide spacer comprising two to four amino acid residues each independently selected from glycine, valine, citrulline, lysine, isoleucine, phenylalanine, methionine, asparagine, proline, alanine, leucine, tryptophan, and tyrosine.

**Embodiment 98.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 97, wherein:
Lp is a bivalent peptide spacer selected from 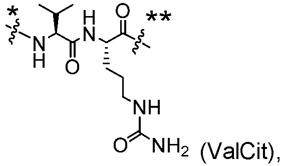 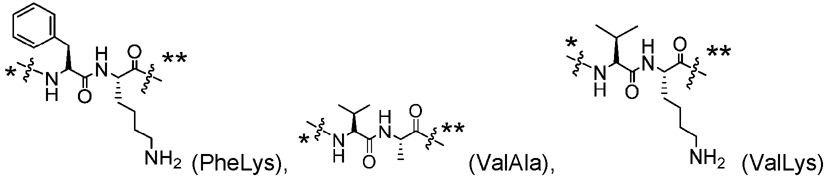 and 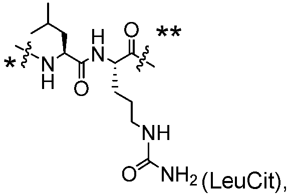 where the * of Lp indicates the attachment point to L₁ and the ** of Lp indicates the attachment point to the -NH- group of Formula (B') or the ** of Lp indicates the attachment point to the G of Formula (A').

**Embodiment 99.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is 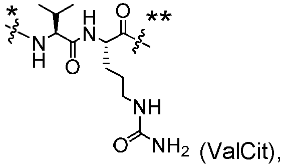 where the * of Lp indicates the attachment point to L₁ and the ** of Lp indicates the attachment point to the -NH- group of Formula (B') or the ** of Lp indicates the attachment point to the G of Formula (A').

**Embodiment 100.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is 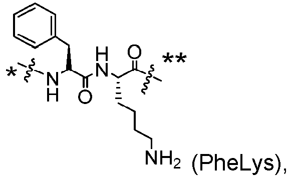 where the * of Lp indicates the attachment point to L₁ and the ** of Lp indicates the attachment point to the -NH- group of Formula (B') or the ** of Lp indicates the attachment point to the G of Formula (A').

**Embodiment 101.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is 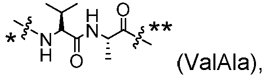 where the * of Lp indicates the attachment point to L₁ and the ** of Lp indicates the attachment point to the -NH- group of Formula (B') or the ** of Lp indicates the attachment point to the G of Formula (A').

**Embodiment 102.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is 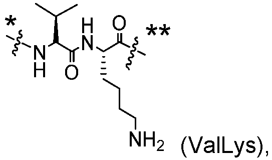 where the * of Lp indicates the attachment point to L₁ and the ** of Lp indicates the attachment point to the -NH- group of Formula (B') or the ** of Lp indicates the attachment point to the G of Formula (A').

**Embodiment 103.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 98, wherein:
Lp is 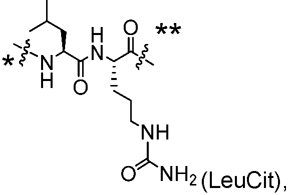 where the * of Lp indicates the attachment point to L₁ and the ** of Lp indicates the attachment point to -NH- group of Formula (B') or the ** of Lp indicates the attachment point to the G of Formula (A').

**Embodiment 104.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 103, wherein L₂ is a bond, a methylene, or a C₂-C₃alkenylene.

**Embodiment 105.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 104, wherein L₂ is a bond or a methylene.

**Embodiment 106.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 105, wherein L₂ is a bond.

**Embodiment 107.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 105, wherein L₂ is a methylene.

**Embodiment 108.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:
A is a bond, -OC(=O)-, -OC(=O)N(CH₃)CH₂CH₂N(CH₃)C(=O)- or -OC(=O)N(CH₃)C(R^{a})₂C(R^{a})₂N(CH₃)C(=O)-, wherein each R^{a} is independently selected from H, C₁-C₆alkyl or a C₃-C₈cycloalkyl.

**Embodiment 109.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein A is a bond or - OC(=O).

**Embodiment 110.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 109, wherein A is a bond.

**Embodiment 111.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 109, wherein A is -OC(=O).

**Embodiment 112.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:
A is

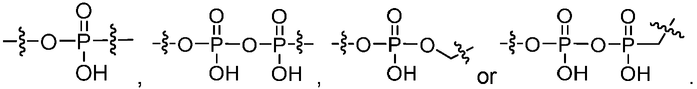

**Embodiment 113.** The compound of Formula (A') or any one of Embodiments 1 to 30, or pharmaceutically acceptable salt thereof, the linker of Formula (C') or any one of Embodiments 32 to 46, and the immunoconjugate of Formula (E') or any one of Embodiments 60 to 83, or any one of Embodiments 84 to 107, wherein:
A is -OC(=O)N(CH₃)CH₂CH₂N(CH₃)C(=O)- or -OC(=O)N(CH₃)C(R^{a})₂C(R^{a})₂N(CH₃)C(=O)- , wherein each R^{a} is independently selected from H, C₁-C₆alkyl or a C₃-C₈cycloalkyl.

**Embodiment 114.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 113, wherein:
L₃ is a spacer moiety having the structure 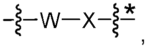
where
   W is -CH₂O-**, -CH₂N(R^{b})C(=O)O-**, -NHC(=O)C(R^{b})₂NHC(=O)O-**, -NHC(=O)C(R^{b})₂NH-**, -NHC(=O)C(R^{b})₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**, -NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
   and
the * of L₃ indicates the point of attachment to R².

**Embodiment 115.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 114, wherein:
L₃ is a spacer moiety having the structure 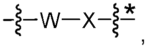
where
   W is -CH₂O-**, -CH₂N(R^{b})C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**, -NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is a bond;
   and
the * of L₃ indicates the point of attachment to R².

**Embodiment 116.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 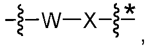
where
   W is -CH₂O-**, -CH₂N(R^{b})C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, - CH₂NR^{b}C(=O)-**, -CH₂NR^{b}C(=O)NH-**, -CH₂NR^{b}C(=O)NR^{b}-**, -NHC(=O)-**, -NHC(=O)O-**, -NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is a triazolyl, wherein the *** of X indicates the point of attachment to W and the * of X indicates the point of attachment to R²;
   and
the * of L₃ indicates the point of attachment to R².

**Embodiment 117.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 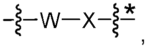
where
   W is -CH₂O-**, -CH₂N(R^{b})C(=O)O-**, -NHC(=O)CH₂NHC(=O)O-**, -NHC(=O)CH₂NH-**, -NHC(=O)CH₂NHC(=O)-**, -CH₂N(X-R²)C(=O)O-**, -C(=O)N(X-R²)-**, -CH₂N(X-R²)C(=O)-**, -C(=O)NR^{b}-**, -C(=O)NH-**, -CH₂NR^{b}C(=O)-**, -CH₂NR^{b}C(=O)NH-**, -CH₂NR^{b}C(=O)NR^{b}-**, -NHC(=O)-**, -NHC(=O)O-**, -NHC(=O)NH-**, -OC(=O)NH-**, -S(O)₂NH-**, -NHS(O)₂-**, -C(=O)-, -C(=O)O-** or -NH-, wherein each R^{b} is independently selected from H, C₁-C₆alkyl or C₃-C₈cycloalkyl and wherein the ** of W indicates the point of attachment to X;
   X is ***-CH₂-triazolyl-*, wherein the *** of X indicates the point of attachment to W and the * of X indicates the point of attachment to R²;
   and
the * of L₃ indicates the point of attachment to R².

**Embodiment 118.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 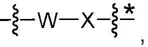
where
   W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, wherein each R^{b} is independently selected from H, C₁-C₆alkyl or C₃-C₈cycloalkyl and wherein the ** of W indicates the point of attachment to X;
   X is a bond, triazolyl or ***-CH₂-triazolyl-*, wherein the *** of X indicates the point of attachment to W and the * of X indicates the point of attachment to R²;
   and
the * of L₃ indicates the point of attachment to R².

**Embodiment 119.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 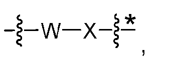
where
   W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, wherein each R^{b} is independently selected from H, C₁-C₆alkyl or C₃-C₈cycloalkyl and wherein the ** of W indicates the point of attachment to X;
   X is a bond;
   and
the * of L₃ indicates the point of attachment to R².

**Embodiment 120.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 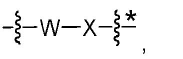
where
   W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, wherein each R^{b} is independently selected from H, C₁-C₆alkyl or C₃-C₈cycloalkyl and wherein the ** of W indicates the point of attachment to X;
   X is a triazolyl, wherein the *** of X indicates the point of attachment to W and the * of X indicates the point of attachment to R²;
   and
the * of L₃ indicates the point of attachment to R².

**Embodiment 121.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 115, wherein:
L₃ is a spacer moiety having the structure 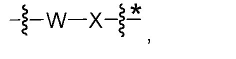
where
   W is -CH₂O-**, -CH₂N(R^{b})C(=O)O-**, -NHC(=O)CH₂NHC(=O)O-**, -CH₂N(X-R²)C(=O)O-**, -C(=O)N(X-R²)-**, wherein each R^{b} is independently selected from H, C₁-C₆alkyl or C₃-C₈cycloalkyl and wherein the ** of W indicates the point of attachment to X;
   X is ***-CH₂-triazolyl-*, wherein the *** of X indicates the point of attachment to W and the * of X indicates the point of attachment to R²;
   and
the * of L₃ indicates the point of attachment to R².

**Embodiment 122.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 121, wherein R² is a hydrophilic moiety selected from polyethylene glycol, polyalkylene glycol, a sugar, an oligosaccharide, a polypeptide or C₂-C₆alkyl substituted with 1 to 3 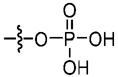 groups..

**Embodiment 123.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a sugar.

**Embodiment 124.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is an oligosaccharide.

**Embodiment 125.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polypeptide.

**Embodiment 126.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyalkylene glycol.

**Embodiment 127.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyalkylene glycol having the structure -(O(CH₂)ₘ)ₜR', where R' is OH, OCH₃ or OCH₂CH₂C(=O)OH, m is 1-10 and t is 4-40.

**Embodiment 128.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyalkylene glycol having the structure -((CH₂)ₘO)ₜR"-, where R" is H, CH₃ or CH₂CH₂C(=O)OH, m is 1-10 and t is 4-40.

**Embodiment 129.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyethylene glycol.

**Embodiment 130.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyethylene glycol having the structure -(OCH₂CH₂)ₜR', where R' is OH, OCH₃ or OCH₂CH₂C(=O)OH and t is 4-40,

**Embodiment 131.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein R² is a polyethylene glycol having the structure -(CH₂CH₂O)ₜR"-, where R" is H, CH₃ or CH₂CH₂C(=O)OH and t is 4-40.

**Embodiment 132.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
R² is 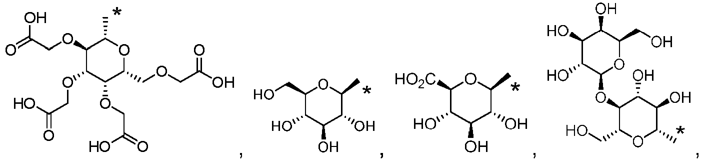 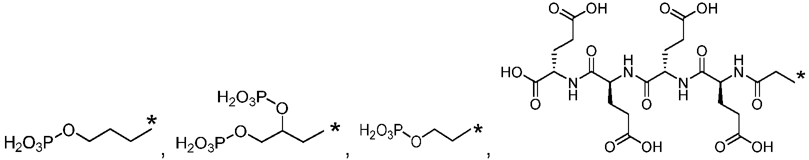 or 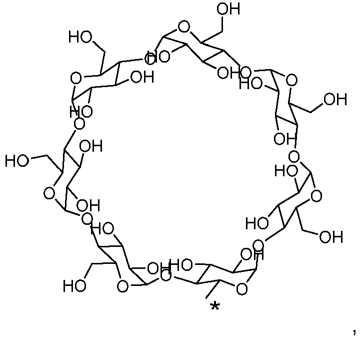 where the * of R² indicates the point of attachment to X or L₃.

**Embodiment 133.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
R² is 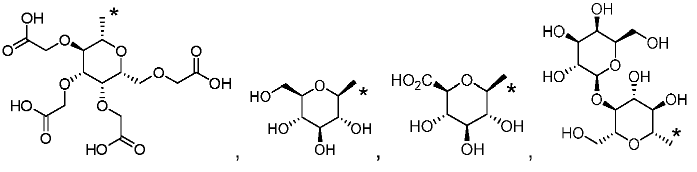 or 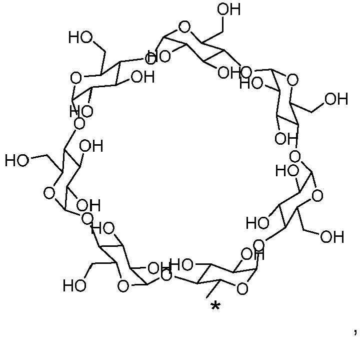 where the * of R² indicates the point of attachment to X or L₃.

**Embodiment 134.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
R² is 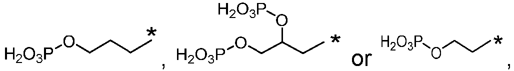 where the * of R² indicates the point of attachment to X or L₃.

**Embodiment 135.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 122, wherein:
R² is 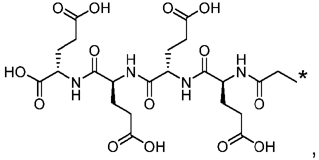 where the * of R² indicates the point of attachment to X or L₃.

**Embodiment 136.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 135, wherein:
X₁ is

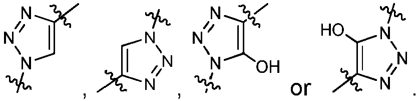

**Embodiment 137.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 135, wherein:
X₁ is

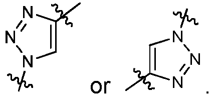

**Embodiment 138.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 137, wherein:
each m is independently selected from 1, 2, 3, 4, and 5.

**Embodiment 139.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 137, wherein:
each m is independently selected from 1, 2 and 3.

**Embodiment 140.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 139, wherein:
each n is independently selected from 1, 2, 3, 4 and 5.

**Embodiment 141.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 139, wherein:
each n is independently selected from 1, 2 and 3.

**Embodiment 142.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 2, 3, 4, 5, 6, 7, 8, 9, 10, 11, 12, 13, 14, 15, 16, 17, 18, 19, 20, 21, 22, 23, 24, 25, 26, 27, 28, 29 and 30.

**Embodiment 143.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 4, 5, 6, 7, 8, 9, 10, 11, 12, 13, 14, 15, 16, 17, 18, 19, 20, 21, 22, 23, 24 or 25.

**Embodiment 144.** The compound of Formula (A') or any one of Embodiments 1 to 17, or pharmaceutically acceptable salt thereof, the linker of Formula (C') or any one of Embodiments 32 to 46, and the immunoconjugate of Formula (E') or any one of Embodiments 60 to 70, or any one of Embodiments 84 to 141, wherein:
each t is independently selected from 1, 2, 3, 4, 5, 6, 7, 8, 9, 10, 11, 12, 13, 14, 15, 16, 17 and 18.

**Embodiment 145.** The immunoconjugate of Formula (E') or any one of Embodiments 60 to 70, or any one of Embodiments 84 to 144, wherein y is 1, 2, 3, 4, 5, 6, 7, 8, 9, 10, 11, 12, 13, or 14.

**Embodiment 146.** The immunoconjugate of Formula (E') or any one of Embodiments 60 to 70, or any one of Embodiments 84 to 144, wherein y is 1, 2, 3, 4, 5, 6, 7, 8, 9, 10, 11 or 12.

**Embodiment 147.** The immunoconjugate of Formula (E') or any one of Embodiments 60 to 70, or any one of Embodiments 84 to 144, wherein y is 1, 2, 3, 4, 5, 6, 7, 8, 9 or 10.

**Embodiment 148.** The immunoconjugate of Formula (E') or any one of Embodiments 60 to 70, or any one of Embodiments 84 to 144, wherein y is 1, 2, 3, 4, 5, 6, 7 or 8.

**Embodiment 149.** The immunoconjugate of Formula (E') or any one of Embodiments 60 to 70, or any one of Embodiments 84 to 144, wherein y is 1, 2, 3, 4, 5 or 6.

**Embodiment 150.** The immunoconjugate of Formula (E') or any one of Embodiments 60 to 70, or any one of Embodiments 84 to 144, wherein y is 1, 2, 3 or 4.

**Embodiment 151.** The immunoconjugate of Formula (E') or any one of Embodiments 60 to 70, or any one of Embodiments 84 to 144, wherein y is 1 or 2.

**Embodiment 152.** The immunoconjugate of Formula (E') or any one of Embodiments 60 to 70, or any one of Embodiments 84 to 144, wherein y is 2.

**Embodiment 153.** The immunoconjugate of Formula (E') or any one of Embodiments 60 to 70, or any one of Embodiments 84 to 144, wherein y is 4.

**Embodiment 154.** The immunoconjugate of Formula (E') or any one of Embodiments 60 to 70, or any one of Embodiments 84 to 144, wherein y is 6.

**Embodiment 155.** The immunoconjugate of Formula (E') or any one of Embodiments 60 to 70, or any one of Embodiments 84 to 144, wherein y is 8.

**Embodiment 156.** The compound of Formula (A') or any one of Embodiments 1 to 30, or pharmaceutically acceptable salt thereof, the immunoconjugate of Formula (E') or any one of Embodiments 60 to 70, or any one of Embodiments 84 to 155, wherein D is a MCI-1 inhibitor when released from the immunoconjugates.

### Other Linker Groups

Other examples of linker groups that are suitable for making ADCs or immunoconjugates of a MCI-1 inhibitor disclosed herein includes those disclosed in international application publications such as WO2018200812, WO2017214456, WO2017214458, WO2017214462, WO2017214233, WO2017214282, WO2017214301, WO2017214322, WO2017214335, WO2017214339, WO2016094509, WO2016094517, and WO2016094505.

For example, the immunoconjugates of MCI-1 inhibitors disclosed herein can have a linker-payload ("-L-D") structure selected from: 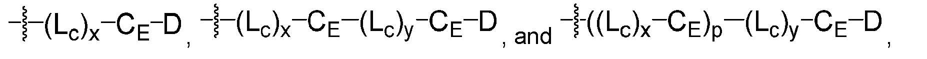 wherein:
Lc is a linker component and each Lc is independently selected from a linker component as disclosed herein;
x is an integer selected from 1, 2, 3, 4, 5, 6, 7, 8, 9, 10, 11, 12, 13, 14, 15, 16, 17, 18, 19 and 20;
y is an integer selected from 1, 2, 3, 4, 5, 6, 7, 8, 9, 10, 11, 12, 13, 14, 15, 16, 17, 18, 19 and 20;
p is an integer selected from 1, 2, 3, 4, 5, 6, 7, 8, 9 and 10;
D is a MCI-1 inhibitor disclosed herein;
and each cleavage element (C_{E}) is independently selected from a self-immolative spacer and a group that is susceptible to cleavage selected from acid-induced cleavage, peptidase-induced cleavage, esterase-induced cleavage, glycosidase induced cleavage, phosphodiesterase induced cleavage, phosphatase induced cleavage, protease induced cleavage, lipase induced cleavage or disulfide bond cleavage.

In some embodiments, L has a structure selected from the following, or L comprises a structural component selected from the following:

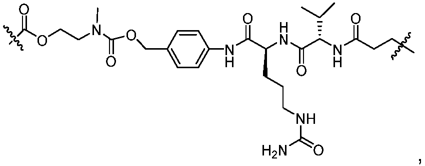

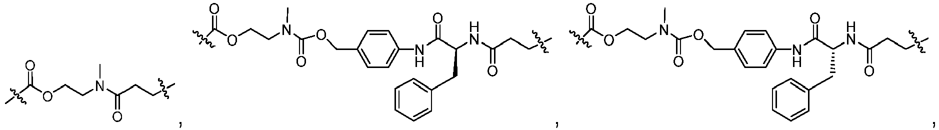

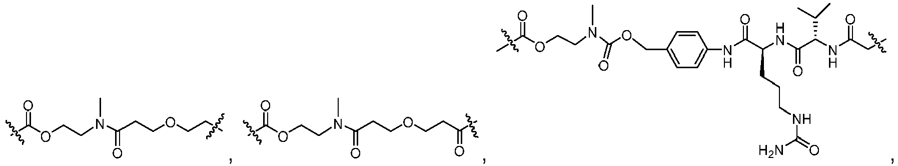

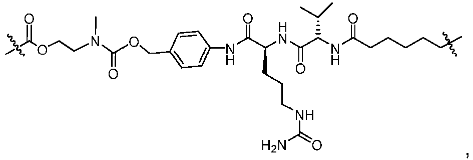

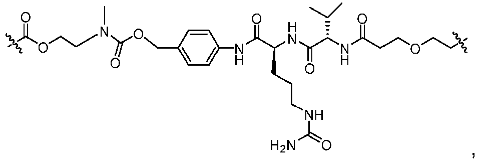

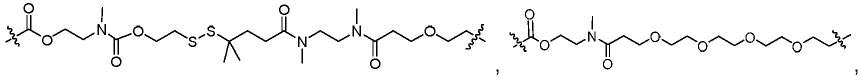

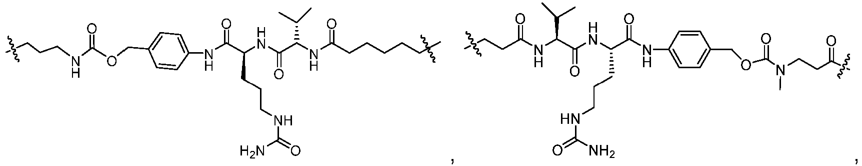

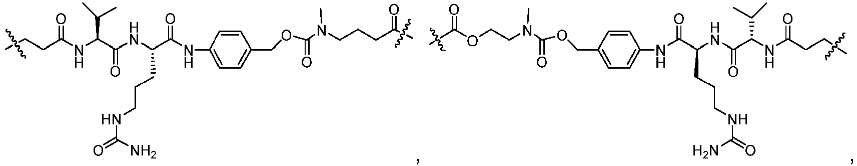

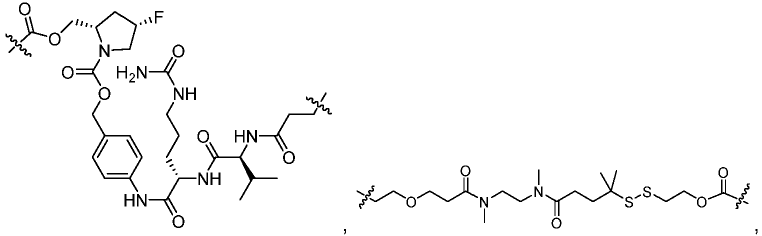

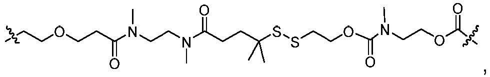

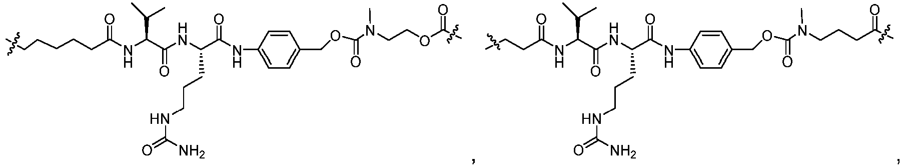

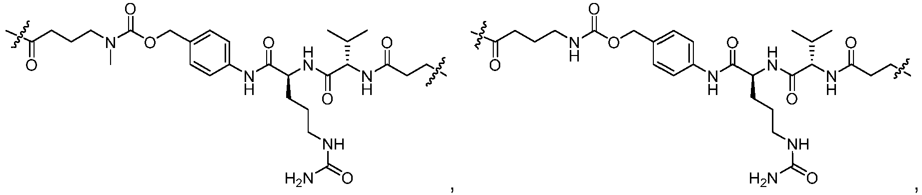

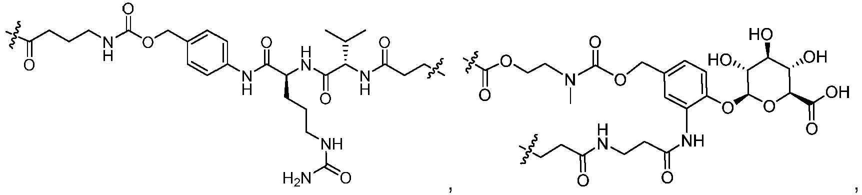

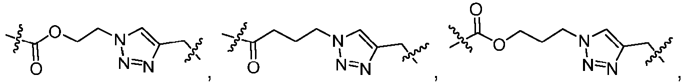

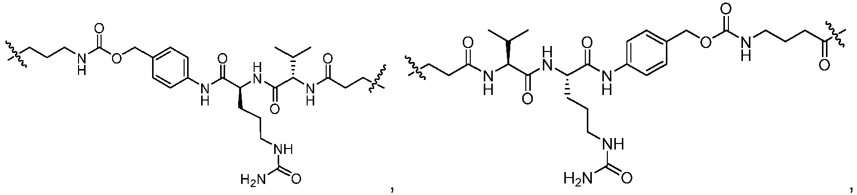

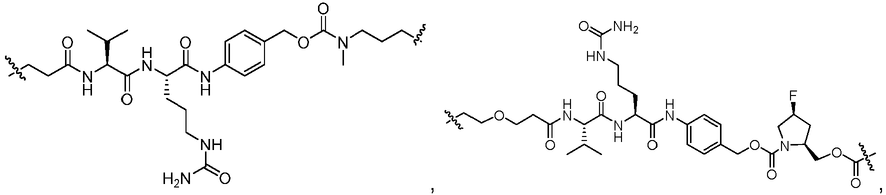

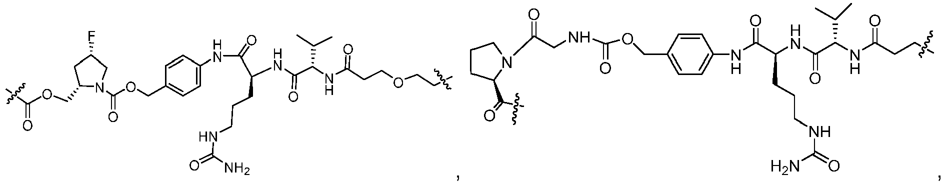

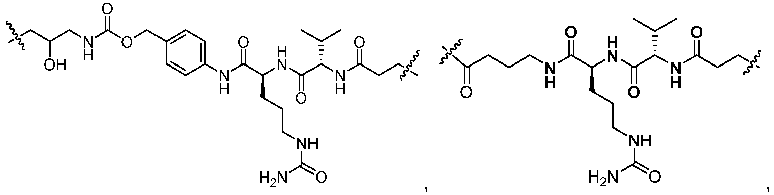

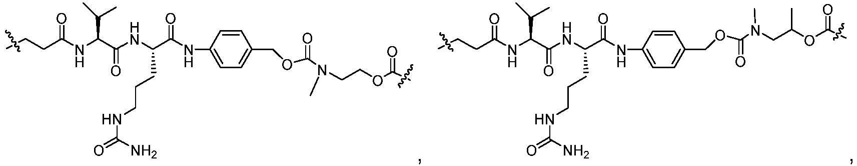

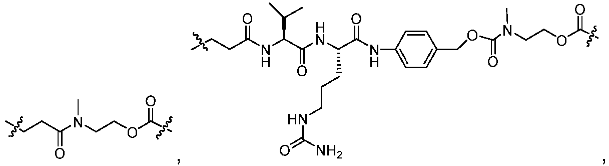

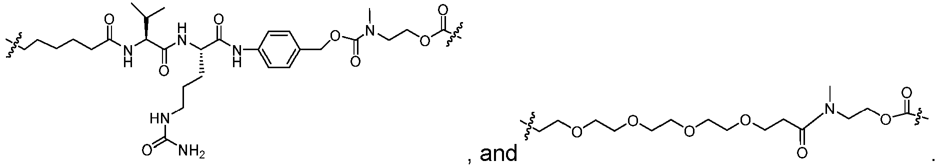

In some embodiments, Lc is a linker component and each Lc is independently selected from 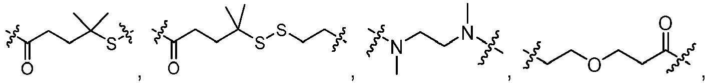 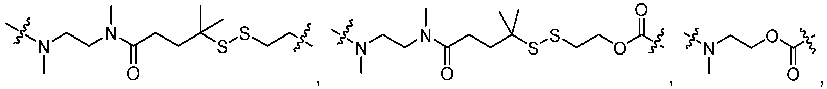 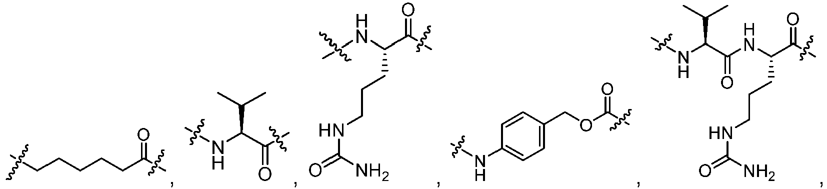 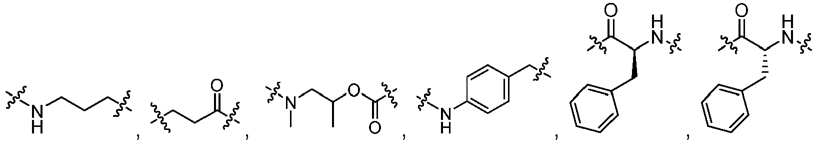 and

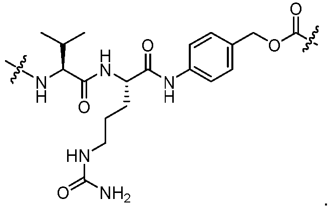

In some embodiments, the linker L comprises a linker component that is selected from:

-**C(=O)O(CH₂)ₘNR¹¹C(=O)(CH₂)ₘ-; -**C(=O)O(CH₂)ₘNR¹¹C(=O)(CH₂)ₘO(CH₂)ₘ-;

-**C(=O)O(CH₂)ₘNR¹¹C(=O)X₁ₐX₂ₐC(=O)(CH₂)ₘ-;

-**C(=O)OC(R¹²)₂(CH₂)ₘNR¹¹C(=O)X₁ₐX₂ₐC(=O)(CH₂)ₘ-;

-**C(=O)O(CH₂)ₘNR¹¹C(=O)X₁ₐX₂ₐC(=O)(CH₂)ₘO(CH₂)ₘ-;

-**C(=O)O(CH₂)ₘNR¹¹C(O)X₁ₐX₂ₐC(=O)(CH₂)ₘO(CH₂)ₘC(=O)-;

-**C(=O)O(CH₂)ₘNR¹¹C(=O)X₄C(=O)NR¹¹(CH₂)ₘNR¹¹C(=O)(CH₂)ₘO(CH₂)ₘ-;

-**C(=O)O(CH₂)ₘNR¹¹C(=O)X₅C(=O)(CH₂)ₘNR¹¹C(=O)(CH₂)ₘ₋;

-**C(=O)X₄C(=O)NR¹¹(CH₂)ₘNR¹¹C(=O)(CH₂)ₘO(CH₂)ₘ-;

-**C(=O)(CH₂)ₘNR¹¹C(=O)X₁ₐX₂ₐC(=O)(CH₂)ₘ-;

-**C(=O)O(CH₂)ₘX₆C(=O)X₁ₐX₂ₐC(=O)(CH₂)ₘ-;

-**C(=O)(CH₂)ₘNR¹¹C(=O)((CH₂)ₘO)ₙ(CH₂)ₘ-

-**C(=O)O(CH₂)ₘX₆C(=O)(CH₂)ₘ-; -**C(=O)O(CH₂)ₘX₆C(=O)(CH₂)ₘO(CH₂)ₘ-;

-**C(=O)O(CH₂)ₘX₆C(=O)X₁ₐX₂ₐC(=O)(CH₂)ₘ-;

-**C(=O)O(CH₂)ₘX₆C(=O)X₁ₐX₂ₐC(=O)(CH₂)ₘO(CH₂)ₘ-;

-**C(=O)O(CH₂)ₘX₆C(=O)X₁ₐX₂ₐC(=O)(CH₂)ₘO(CH₂)ₘC(=O)-;

-**C(=O)O(CH₂)ₘX₆C(=O)X₄C(=O)NR¹¹(CH₂)ₘNR¹¹C(=O)(CH₂)ₘO(CH₂)ₘ-;

-**C(=O)X₄C(=O)X₆(CH₂)ₘNR¹¹C(=O)(CH₂)ₘO(CH₂)ₘ-;

-**C(=O)(CH₂)ₘX₆C(=O)X₁ₐX₂ₐC(=O)(CH₂)ₘ-;

-**C(=O)O((CH₂)ₘO)ₙ(CH₂)ₘNR¹¹C(=O)X₅C(=O)(CH₂)ₘ-;

-**C(=O)O((CH₂)ₘO)ₙ(CH₂)ₘNR¹¹C(=O)X₅C(=O)(CH₂)ₘNR¹¹C(=O)(CH₂)ₘ-;

-**C(=O)O((CH₂)ₘO)ₙ(CH₂)ₘNR¹¹C(=O)X₅C(=O)(CH₂)ₘX₃(CH₂)ₘ-;

-**C(=O)O((CH₂)ₘO)ₙ(CH₂)ₘNR¹¹C(=O)X₅C(=O)((CH₂)ₘO)ₙ(CH₂)ₘ-;

-**C(=O)O((CH₂)ₘO)ₙ(CH₂)ₘNR¹¹C(=O)XsC(=O)((CH₂)ₘO)ₙ(CH₂)ₘNR¹¹C(=O)(CH₂)ₘ-;

**C(=O)O((CH₂)ₘO)ₙ(CH₂)ₘNR¹¹C(=O)X₅C(=O)((CH₂)ₘO)ₙ(CH₂)ₘNR¹¹C(=O)(CH₂)ₘX₃(CH₂) ₘ-;

-**C(=O)O((CH₂)ₘO)ₙ(CH₂)ₘNR¹¹C(=O))X₅C(=O)((CH₂)ₘO)ₙ(CH₂)ₘX₃(CH₂)ₘ-;

-**C(=O)O((CH₂)ₘO)ₙ(CH₂)ₘNR¹¹C(=O)X₅C(=O)(CH₂)ₘNR¹¹C(=O)((CH₂)mO)ₙ(CH₂)ₘ-;

**C(=O)O((CH₂)ₘO)ₙ(CH₂)ₘNR¹¹C(=O)X₅C(=O)(CH₂)ₘNR¹¹C(=O)((CH₂)ₘO)ₙ(CH₂)ₘX₃(CH₂) ₘ-;

-**C(=O)O((CH₂)ₘO)ₙ(CH₂)ₘNR¹¹C(=O)X₅(CH₂)ₘX₃(CH₂)ₘ-;

-**C(=O)O((CH₂)ₘO)ₙ(CH₂)ₘNR¹¹C(=O)X₅((CH₂)ₘO)ₙ(CH₂)ₘ-;

-**C(=O)O((CH₂)ₘO)ₙ(CH₂)ₘNR¹¹C(=O)X₅((CH₂)ₘO)ₙ(CH₂)ₘNR¹¹C(=O)(CH₂)ₘ-;

-**C(=O)O((CH₂)ₘO)ₙ(CH₂)ₘNR¹¹C(=O)X₅((CH₂)ₘO)ₙ(CH₂)ₘNR¹¹C(=O)(CH₂)ₘX₃(CH₂)ₘ-;

-**C(=O)O((CH₂)ₘO)ₙ(CH₂)ₘNR¹¹C(=O)X₅((CH₂)ₘO)ₙ(CH₂)ₘX₃(CH₂)ₘ-;

-**C(=O)O((CH₂)ₘO)ₙ(CH₂)ₘNR¹¹C(=O)X₅(CH₂)ₘNR¹¹((CH₂)ₘO)ₙ(CH₂)ₘ-;

-**C(=O)O((CH₂)ₘO)ₙ(CH₂)ₘNR¹¹C(=O)X₅C(=O)(CH₂)ₘNR¹¹((CH₂)ₘO)ₙ(CH₂)ₘX₃(CH₂)ₘ-;

-**C(=O)O((CH₂)ₘO)ₙ(CH₂)ₘNR¹¹C(=O)X₅(CH₂)ₘ-;

-**C(=O)O((CH₂)ₘO)ₙ(CH₂)ₘNR¹¹C(=O)X₅C(=O)((CH₂)ₘO)ₙ(CH₂)ₘ-;

-**C(=O)O((CH₂)ₘO)ₙ(CH₂)ₘNR¹¹C(=O)X₅(CH₂)ₘX₃(CH₂)ₘ-; -**C(=O)O(CH₂)ₘ-;

-**C(=O)O((CH₂)ₘO)ₙ(CH₂)ₘ-; -**C(=O)O(CH₂)ₘNR¹¹(CH₂)ₘ-;

-**C(=O)O(CH₂)ₘNR¹¹(CH₂)ₘC(=O)X₂ₐX₁ₐC(=O)-;

-**C(=O)O(CH₂)ₘX₃(CH₂)ₘ-; -**C(=O)O((CH₂)ₘO)ₙ(CH₂)ₘX₃(CH₂)ₘ-;

-**C(=O)O((CH₂)ₘO)ₙ(CH₂)ₘNR¹¹C(=O)(CH₂)ₘ-; -

**C(=O)O(CH₂)ₘNR¹¹C(=O(CH₂)ₘX₃(CH₂)ₘ-;

-**C(=O)O((CH₂)ₘO)ₙ(CH₂)ₘNR¹¹C(=O)(CH₂)ₘX₃(CH₂)ₘ-;

-**C(=O)O((CH₂)ₘO)ₙX₃(CH₂)ₘ-; -**C(=O)O((CH₂)ₘO)ₙ(CH₂)ₘX₃(CH₂)ₘ-;

-**C(=O)O((CH₂)ₘO)ₙ(CH₂)ₘC(=O)NR¹¹(CH₂)ₘ-; -**C(=O)O(CH₂)ₘC(R¹²)₂-;

-**C(=O)OCH₂)ₘC(R¹²)₂SS(CH₂)ₘNR¹¹C(=O)(CH₂)ₘ-,

and

-**C(=O)O(CH₂)ₘC(=O)NR¹¹(CH₂)ₘ-,

where: ** indicates point of attachment to the drug moiety (D) and the other end can be connected to R¹⁰⁰, i.e., the coupling group as described herein;
wherein:
X₁ₐ is 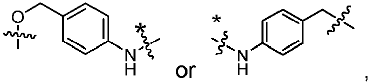 where the * indicates the point of attachment to X2a;
X₂ₐ is selected from 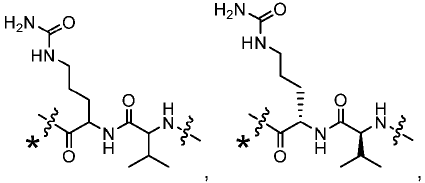 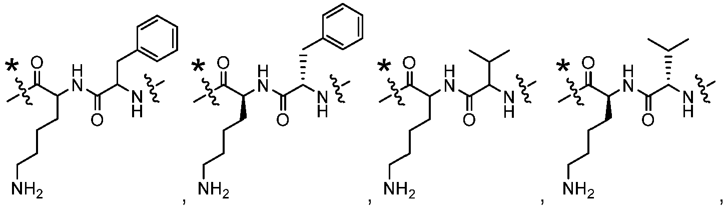 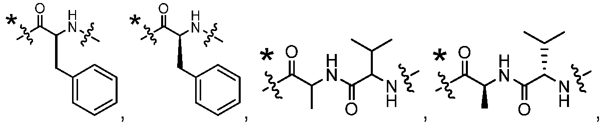 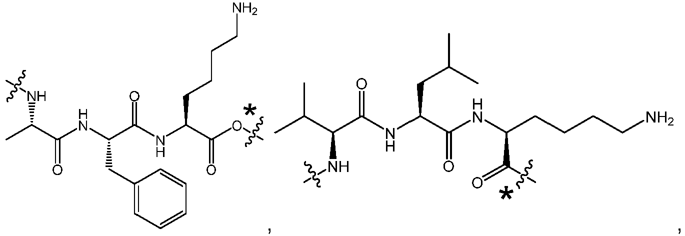 and 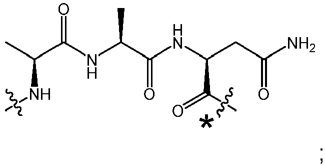 where the * indicates the point of attachment to X₁ₐ;
X₃ is 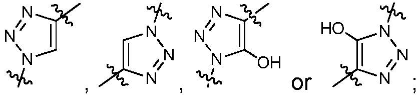
X₄ is -O(CH₂)ₙSSC(R¹²)₂(CH₂)ₙ- or -(CH₂)ₙC(R¹²)₂SS(CH₂)ₙO-;
X₅ is 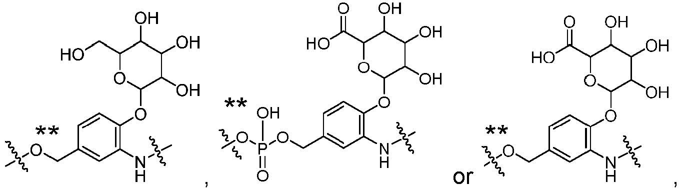 where the ** indicates orientation toward the Drug moiety;
X₆ is 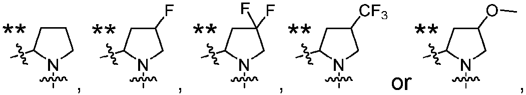 where the ** indicates orientation toward the Drug moiety;
each R¹¹ is independently selected from H and C₁-C₆alkyl;
each R¹² is independently selected from H and C₁-C₆alkyl;
each m is independently selected from 1, 2, 3, 4, 5, 6, 7, 8, 9 and 10, and
each n is independently selected from 1, 2, 3, 4, 5, 6, 7, 8, 9, 10, 11, 12, 13, 14, 15, 16,17 and 18.

### Methods of Conjugation

The present invention provides various methods of conjugating Linker-Drug groups of the invention to antibodies or antibody fragments to produce Antibody Drug Conjugates which comprise a linker having one or more hydrophilic moieties.

A general reaction scheme for the formation of Antibody Drug Conjugates of Formula (E') is shown in Scheme 2 below: 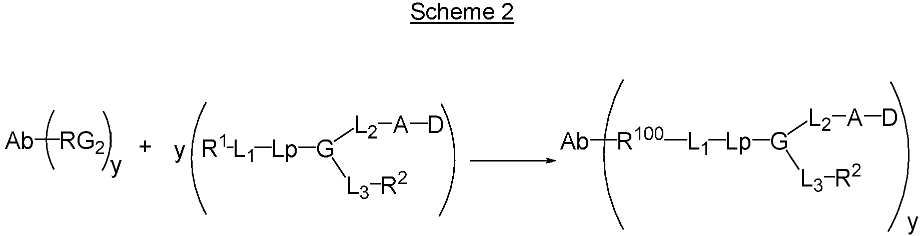 where: RG₂ is a reactive group which reacts with a compatible R¹ group to form a corresponding R¹⁰⁰ group (such groups are illustrated in Table 8 and Table 9). D, R¹, L₁, Lp, Ab, y and R¹⁰⁰ are as defined herein.

Scheme 3 further illustrates this general approach for the formation of Antibody Drug Conjugates of Formula (E'), wherein the antibody comprises reactive groups (RG₂) which react with an R¹ group (as defined herein) to covalently attach the Linker-Drug group to the antibody via an R¹⁰⁰ group (as defined herein). For illustrative purposes only Scheme 3 shows the antibody having four RG₂ groups.

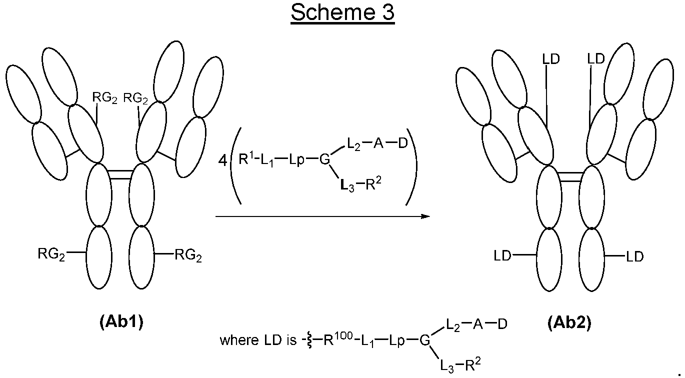

In one aspect, Linker-Drug groups are conjugated to antibodies via modified cysteine residues in the antibodies (see for example WO2014/124316). Scheme 4 illustrates this approach for the formation of Antibody Drug Conjugates of Formula (E') wherein a free thiol group generated from the engineered cysteine residues in the antibody react with an R¹ group (where R' is a maleimide) to covalently attach the Linker-Drug group to the antibody via an R¹⁰⁰ group (where R¹⁰⁰ is a succinimide ring). For illustrative purposes only Scheme 4 shows the antibody having four free thiol groups.

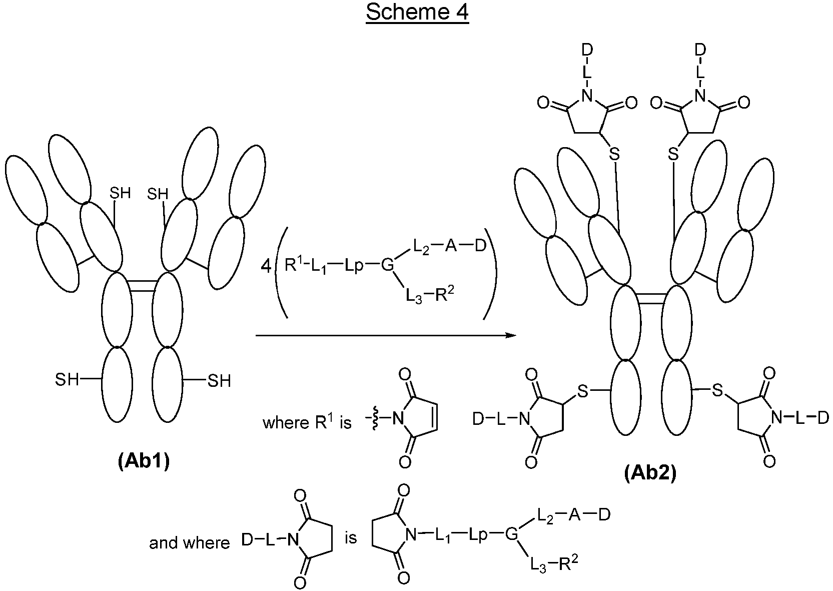

In another aspect, Linker-Drug groups are conjugated to antibodies via lysine residues in the antibodies. Scheme 5 illustrates this approach for the formation of Antibody Drug Conjugates of Formula (E') wherein a free amine group from the lysine residues in the antibody react with an R¹ group (where R¹ is an NHS ester, a pentafluorophenyl or a tetrafluorophenyl) to covalently attach the Linker-Drug group to the antibody via an R¹⁰⁰ group (where R¹⁰⁰ is an amide). For illustrative purposes only Scheme 5 shows the antibody having four amine groups.

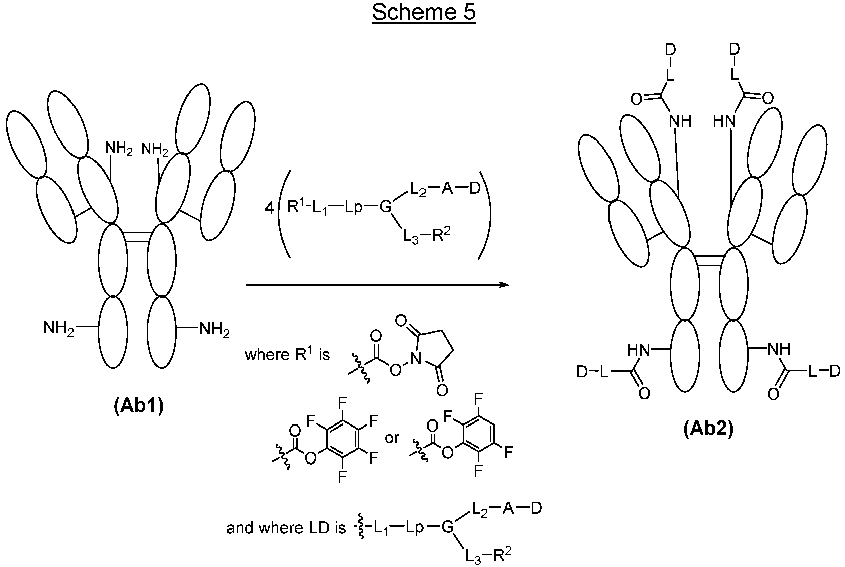

In another aspect, Linker-Drug groups are conjugated to antibodies via formation of an oxime bridge at the naturally occurring disulfide bridges of an antibody. The oxime bridge is formed by initially creating a ketone bridge by reduction of an interchain disulfide bridge of the antibody and re-bridging using a 1,3-dihaloacetone (e.g. 1,3-dichloroacetone). Subsequent reaction with a Linker-Drug group comprising a hydroxyl amine thereby form an oxime linkage (oxime bridge) which attaches the Linker-Drug group to the antibody (see for example WO2014/083505). Scheme 6 illustrates this approach for the formation of Antibody Drug Conjugates of Formula (E').

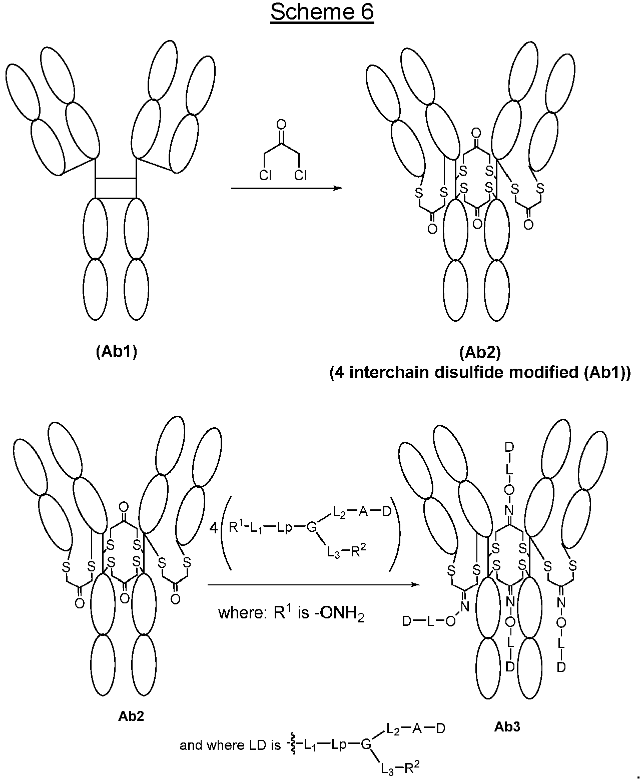

A general reaction scheme for the formation of Antibody Drug Conjugates of Formula (F') is shown in Scheme 7 below: 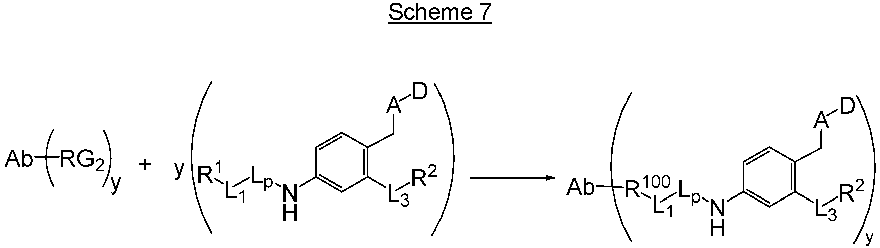 where: RG₂ is a reactive group which reacts with a compatible R¹ group to form a corresponding R¹⁰⁰ group (such groups are illustrated in Table 8 and Table 9). D, R¹, L₁, Lp, Ab, y and R¹⁰⁰ are as defined herein.

Scheme 8 further illustrates this general approach for the formation of Antibody Drug Conjugates of Formula (F'), wherein the antibody comprises reactive groups (RG₂) which react with an R¹ group (as defined herein) to covalently attach the Linker-Drug group to the antibody via an R¹⁰⁰ group (as defined herein). For illustrative purposes only Scheme 8 shows the antibody having four RG₂ groups.

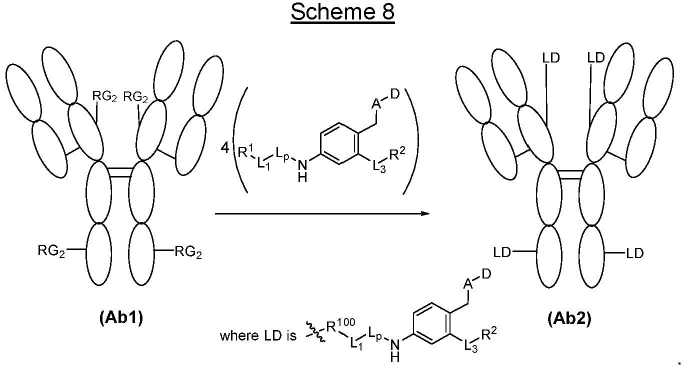

In one aspect, Linker-Drug groups are conjugated to antibodies via modified cysteine residues in the antibodies (see for example WO2014/124316). Scheme 9 illustrates this approach for the formation of Antibody Drug Conjugates of Formula (F') wherein a free thiol group generated from the engineered cysteine residues in the antibody react with an R¹ group (where R¹ is a maleimide) to covalently attach the Linker-Drug group to the antibody via an R¹⁰⁰ group (where R¹⁰⁰ is a succinimide ring). For illustrative purposes only Scheme 9 shows the antibody having four free thiol groups.

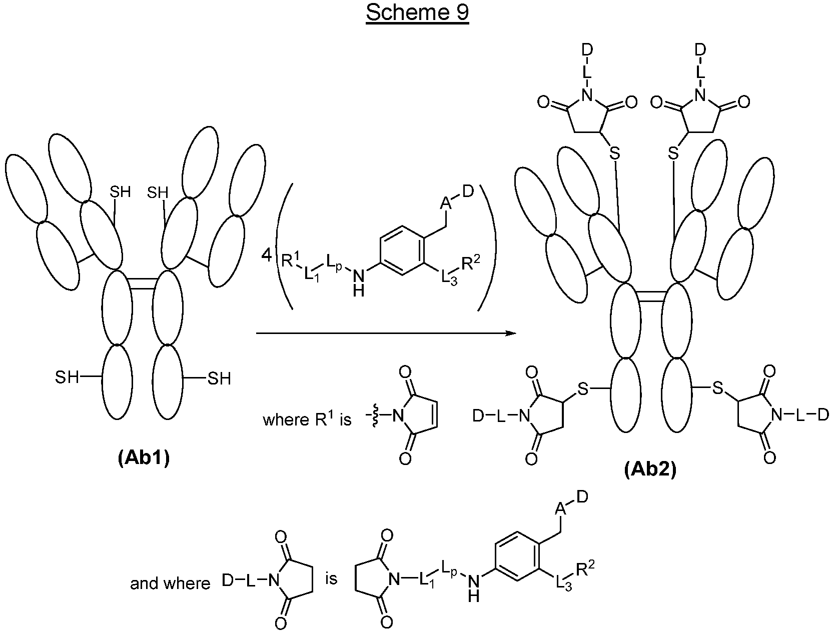

In another aspect, Linker-Drug groups are conjugated to antibodies via lysine residues in the antibodies. Scheme 10 illustrates this approach for the formation of Antibody Drug Conjugates of Formula (F') wherein a free amine group from the lysine residues in the antibody react with an R¹ group (where R¹ is an NHS ester, a pentafluorophenyl or a tetrafluorophenyl) to covalently attach the Linker-Drug group to the antibody via an R¹⁰⁰ group (where R¹⁰⁰ is an amide). For illustrative purposes only Scheme 10 shows the antibody having four amine groups.

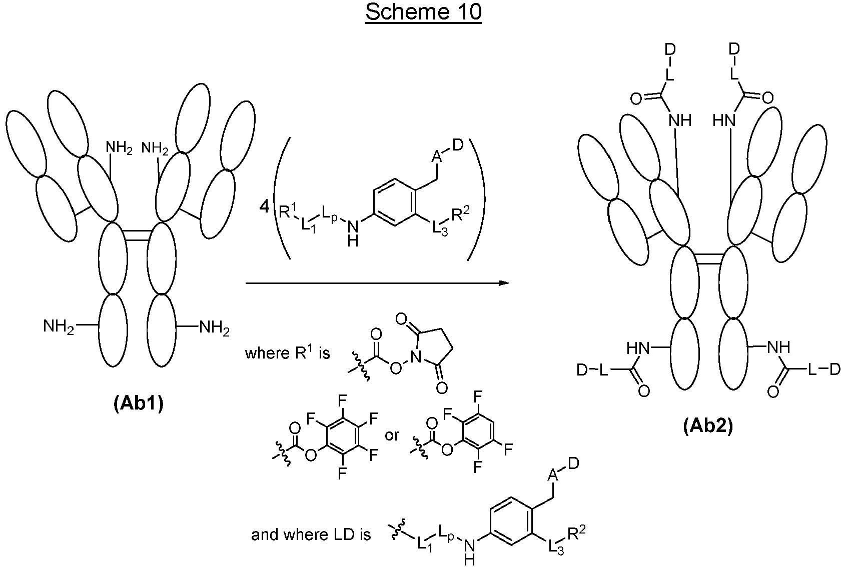

In another aspect, Linker-Drug groups are conjugated to antibodies via formation of an oxime bridge at the naturally occurring disulfide bridges of an antibody. The oxime bridge is formed by initially creating a ketone bridge by reduction of an interchain disulfide bridge of the antibody and re-bridging using a 1,3-dihaloacetone (e.g. 1,3-dichloroacetone).
Subsequent reaction with a Linker-Drug group comprising a hydroxyl amine thereby form an oxime linkage (oxime bridge) which attaches the Linker-Drug group to the antibody (see for example WO2014/083505). Scheme 11 illustrates this approach for the formation of Antibody Drug Conjugates of Formula (F').

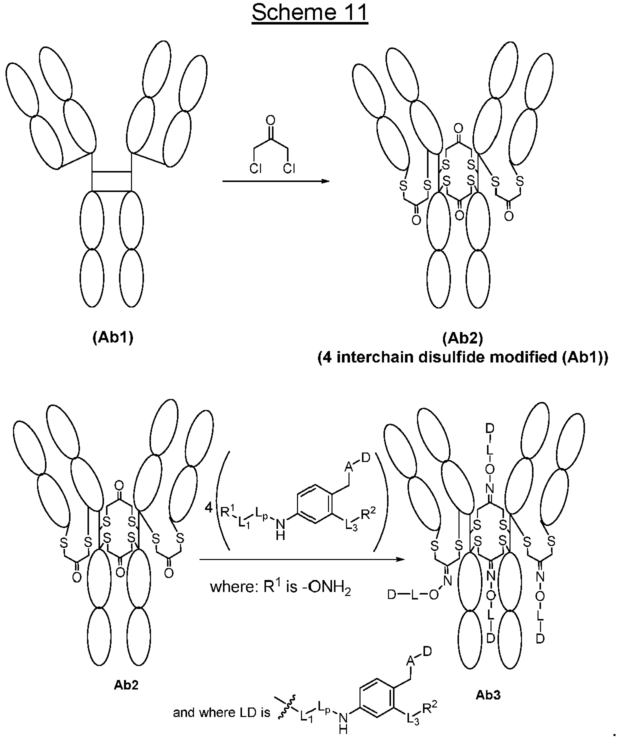

Provided are also protocols for some aspects of analytical methodology for evaluating antibody conjugates of the invention. Such analytical methodology and results can demonstrate that the conjugates have favorable properties, for example properties that would make them easier to manufacture, easier to administer to patients, more efficacious, and/or potentially safer for patients. One example is the determination of molecular size by size exclusion chromatography (SEC) wherein the amount of desired antibody species in a sample is determined relative to the amount of high molecular weight contaminants (e.g., dimer, multimer, or aggregated antibody) or low molecular weight contaminants (e.g., antibody fragments, degradation products, or individual antibody chains) present in the sample. In general, it is desirable to have higher amounts of monomer and lower amounts of, for example, aggregated antibody due to the impact of, for example, aggregates on other properties of the antibody sample such as but not limited to clearance rate, immunogenicity, and toxicity. A further example is the determination of the hydrophobicity by hydrophobic interaction chromatography (HIC) wherein the hydrophobicity of a sample is assessed relative to a set of standard antibodies of known properties. In general, it is desirable to have low hydrophobicity due to the impact of hydrophobicity on other properties of the antibody sample such as but not limited to aggregation, aggregation over time, adherence to surfaces, hepatotoxicity, clearance rates, and pharmacokinetic exposure. See Damle, N.K., Nat Biotechnol. 2008; 26(8):884-885; Singh, S.K., Pharm Res. 2015; 32(11):3541-71. When measured by hydrophobic interaction chromatography, higher hydrophobicity index scores (i.e. elution from HIC column faster) reflect lower hydrophobicity of the conjugates. As shown in Examples below, a majority of the tested antibody conjugates showed a hydrophobicity index of greater than 0.8. In some embodiments, provided are antibody conjugates having a hydrophobicity index of 0.8 or greater, as determined by hydrophobic interaction chromatography.

### EXAMPLES

The following examples provide illustrative embodiments of the disclosure. One of ordinary skill in the art will recognize the numerous modifications and variations that may be performed without altering the spirit or scope of the disclosure. Such modifications and variations are encompassed within the scope of the disclosure. The examples provided do not in any way limit the disclosure.

### Example 1. Synthesis and Characterization of Linkers, Linker-Payloads, and Precursors thereof.

Exemplary linkers, linker-payloads, and precursors thereof were synthesized using exemplary methods described in this example.

### Abbreviations:

- Cul: cupper (I) iodide
- DCC: dicyclohexyl carbodiimide
- DCM: dichloromethane
- DEA: N-ethylethanamine
- DIPEA:: N,N-Diisopropylethylamine
- DMF:: dimethylformamide
- DMSO:: dimethylsulfoxyde
- EEDQ: ethyl 2-ethoxy-2H-quinoline-1-carboxylate
- Fmoc-Cit-OH: (2S)-2-(9H-fluoren-9-ylmethoxycarbonylamino)-5-ureido-pentanoic acid
- HBTU:: (2-(1*H*-benzotriazol-1-yl)-1,1,3,3-tetramethyluronium hexafluorophosphate
- HOAt:: 1-Hydroxy-7-azabenzotriazole
- MgSO₄: magnesium sulfate
- NH₄Cl: ammonium chloride
- NMP: N-methylpyrrolidone
- Pd(PPh₃)₂Cl₂: dichloro-tri(triphenylphosphine)palladium
- PBr₃: tribromophosphane
- Pt/C 10%: platinum over carbon 10%
- SOCl₂: thionyl chloride
- THF: tetrahydrofuran
- TBAI: tetrabutylammonium, iodide
- TFA: trifluoroacetic acid

### Materials, Methods & General Procedures:

All reagents obtained from commercial sources were used without further purification. Anhydrous solvents were obtained from commercial sources and used without further drying. Flash chromatography was performed on CombiFlash Rf (Teledyne ISCO) with pre-packed silica-gel cartridges (Macherey-Nagel Chromabond Flash). Thin layer chromatography was conducted with 5 x 10 cm plates coated with Merck Type 60 F254 silica-gel. Microwave heating was performed in CEM Discover^{®} instrument.

¹H-NMR measurements were performed on 400 MHz Bruker Avance or 500 MHz Avance Neo spectrometer, using DMSO-*d₆* or CDCl₃ as solvent. ¹H NMR data is in the form of chemical shift values, given in part per million (ppm), using the residual peak of the solvent (2.50 ppm for DMSO-*d₆* and 7.26 ppm for CDCl₃) as internal standard. Splitting patterns are designated as: s (singlet), d (doublet), t (triplet), q (quartet), quint (quintet), m (multiplet), br s (broad singlet), br t (broad triplet) dd (doublet of doublets), td (triplet of doublets), dt (doublet of triplets), ddd (doublet of doublet of doublets). IR measurements were performed on a Bruker Tensor 27 equipped with ATR Golden Gate device (SPECAC). HRMS measurements were performed on a LTQ OrbiTrap Velos Pro mass spectrometer (ThermoFisher Scientific). Samples were dissolved in CH₃CN/H2O (2/1:v/v) at a concentration range from 0.01 to 0.05 mg/mL approximately and introduced in the source by an injection of 2 µL in a flow of 0.1 mL/min. ESI ionization parameters were as follow: 3.5 kV and 350°C transfer ion capillary. All the spectra were acquired in positive ion mode with a resolving power of 30,000 or 60,000 using a lock mass.

**HRMS** measurements were performed on an LTQ OrbiTrap Velos Pro mass spectrometer (ThermoFisher Scientific GmbH, Bremen, Germany). Samples were dissolved in CH₃CN/H₂O (2/1:v/v) at a concentration range from 0.01 to 0.05 mg/mL approximately and introduced in the source by an injection of 2 µL in a flow of 0.1 mL/min. ESI ionization parameters were as follows: 3.5 kV and 350°C transfer ion capillary. All the spectra were acquired in positive ion mode with a resolving power of 30 000 or 60 000 using a lock mass.

### UPLC^{®}-MS:

UPLC^{®}-MS data were acquired using an instrument with the following parameters (Table 10):

**Table 10. UPLC^{®}-MS Parameters**

| | |
|---|---|
| Instrument(s) | Waters Aquity A-class with diode array UV detector "PDA" and "ZQ detector 2" mass device and MassLinks software. |
| ZQ detector 2 | MS scan from 0.15 to 6 min and from 100 to 2372 Da |
| PDA detector | from 190 to 400 nm |
| | Aquity UPLC ^{®}BEH column C18, 1.7 µm, 130 Å, |
| Columns | 2.1x50 mm |
| | Column used at 40°C with a flowrate of 0.6mL/min |
| Solvent A | water + 0.02% TFA |
| Solvent B | acetonitrile + 0.02% TFA |
| gradient | from 2% B to 100% B in 5 min, then 0.3 min washing with 100% B and 0.5 min equilibration at 2% B for the next injection (total gradient of 6 min). |

### Preparative-HPLC:

Preparative-HPLC ("Prep-HPLC") data were acquired using an instrument with the following parameters (Table 11):

**Table 11. Prep-HPLC Parameters**

| | |
|---|---|
| Instrument(s) | Columns Waters X-Bridge 5 or 10 µm with sizes (flowrate) of: 19x50 mm (12 ml/min), 19x100 mm (12 ml/min), 30x100 mm (30-50 ml/min), 30x250 mm (30-50 ml/min), 50x250 mm (80-150 ml/min); Interchim Puriflash 4100 with a maximum of 100 bars and a maximum flowrate of 250 ml/min, or Interchim Puriflash 4250 with a maximum of 250 bars and a maximum flowrate of 250 ml/min; Quaternary solvent pump with the possibility to use 4 solvents at the same time in a gradient |
| UV | 2 wavelengths for the collection between 200 and 400 nm |
| Columns | Waters XBridge 10µm |
| Collection | 8 ml or 32 ml tubes |

Three Prep-HPLC methods were used:
a. TFA method: solvent: A water + 0.05 % TFA, B acetonitrile + 0.05 % TFA, gradient from 5 to 100% B in 15 to 30 CV
b. NH₄HCO₃ method: solvent: A water + 0.02 M NH₄HCO₃, B acetonitrile/water 80/20 + 0.02 M NH₄HCO₃, gradient from 5 to 100 % B in 15 to 30 CV
c. Neutral method: solvent: A water, B acetonitrile, gradient from 5 to 100% B in 15 to 30 CV

All the fractions containing the pure compound were combined and directly freeze-dried to afford the compound as an amorphous powder.

### Preparative SFC purification:

Preparative chiral SFC was performed on a PIC solution Prep200 system. The sample was dissolved in ethanol at a concentration of 150 mg/mL. The mobile phase was held isocratically at 40% ethanol/CO₂. The instrument was fitted with a Chiralpak IA column and a loop of 3 mL. The ABPR (automatic back-pressure regulator) was set at 100 bars.

### Preparation of L23-P3:

### (2R)-2-[(5Sa)-5-[4-[2-[4-[[4-[[(2S)-2-[[(2S)-2-[[2-(2-azidoethoxy)acetyl]amino]-3-methyl-butanoyl]amino]-5-ureido-pentanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

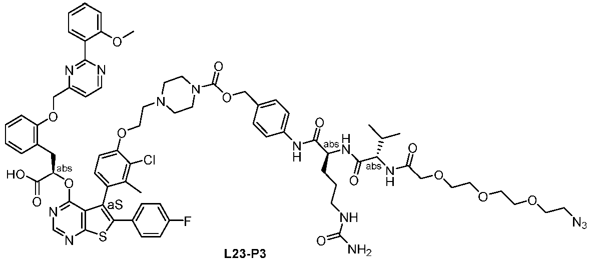

### Step 1: (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide

To a solution of 2-[2-[2-(2-azidoethoxy)ethoxy]ethoxy]acetic acid (purchased from Broadpharm, 1.4 g, 6 mmol) in THF (20 mL) was added 1-hydroxypyrrolidine-2,5-dione (690 mg, 6 mmol) and N,N'-Dicyclohexylcarbodiimide (1.2 g, 6 mmol). The reaction mixture was stirred at room temperature overnight. The precipitate was filtered off and the filtrate was concentrated to afford (2,5-dioxopyrrolidin-1-yl) 2-[2-[2-(2-azidoethoxy)ethoxy]ethoxy]acetate (1.9g, 6 mmol), used immediately without further purification.

To a solution of (2,5-dioxopyrrolidin-1-yl) 2-[2-[2-(2-azidoethoxy)ethoxy]ethoxy]acetate (1.6 g; 4.85 mmol) in DMF (15 mL) was added (2S)-2-[[(2S)-2-amino-3-methyl-butanoyl]amino]-N-[4-(hydroxymethyl)phenyl]-5-ureido-pentanamide (1.96 g; 5.17 mmol). The mixture was stirred at room temperature for 2 h and concentrated. The residue was diluted in water (20 mL) and acetonitrile (5 mL) and stirred at room temperature overnight. The mixture was purified by reverse phase C18 chromatography using the neutral method to afford (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (1.07g, 1.8 mmol). ¹H NMR (400 MHz, dmso-d6): δ 9.95 (s, 1H), 8.3 (d, 1H), 7.55 (d, 2H), 7.46 (d, 1H), 7.22 (d, 2H), 5.98 (t, 1H), 5.4 (s, 1H), 5.08 (t, 1H), 4.43 (d, 2H), 4.4 (q, 1H), 4.33 (dd, 1H), 3.95 (s, 2H), 3.6 (m, 10H), 3.38 (t, 2H), 3 (m, 2H), 2 (m, 1H), 1.7/1.6 (2m, 2H), 1.5-1.3 (m, 2H), 0.89/0.82 (2d, 6H).

### Step 2: [4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl) carbonate

To a solution of (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (100 mg, 0.168 mmol) in DMF (30 mL) was added DIPEA (32 µL, 0.179 mmol) and bis(4-nitrophenyl) carbonate (100 mg, 0.329 mmol). The mixture was stirred at room temperature for 4 h and concentrated to dryness. The residue was purified by silica gel chromatography (gradient of methanol in dichloromethane) to afford 4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl)carbonate (65 mg, 0.088 mmol).¹H NMR (400 MHz, dmso-d6): δ 9.95 (s, 1H), 8.3 (d, 1H), 7.55 (d, 2H), 7.46 (d, 1H), 7.22 (d, 2H), 5.98 (t, 1H), 5.4 (s, 1H), 5.08 (t, 1H), 4.43 (d, 2H), 4.4 (q, 1H), 4.33 (dd, 1H), 3.95 (s, 2H), 3.6 (m, 10H), 3.38 (t, 2H), 3 (m, 2H), 3.02-2.95 (m, 2H), 2 (m, 1H), 1.7 (m, 1H), 1.6 (m, 1H), 0.89 (d, 3H), 0.82 (d, 3H).

### Step 3: (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methoxycarbonyl]piperazin-1-yl]ethoxy]-3-chloro-2-methyl-phenyl]-6-(4-fluorophenyl)thieno[2,3-djpyrimidin-4-yl]oxy-3-[2-[[2-(2-methoxyphenyl)pyrimidin-4-yl]methoxy]phenyl]propanoic acid L23-P3

To a solution of ((2R)-2-[(5*Sₐ*)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid P3 (147 mg, 0.17 mmol) in DMF (16 mL) were successively added DIPEA (85 µL, 0.51 mmol), 4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl)carbonate (136 mg, 0.179 mmol), 2,6-lutidine (99 µL, 0.85 mmol) and HOAt (7 mg, 0.05 mmol). The mixture was stirred at room temperature overnight and purified by C18 reverse phase prep-HPLC by direct deposit of the reaction mixture on the Xbridge column and using the NH₄HCO₃ method to afford **L23-P3** (110 mg, 0.074 mmol). ¹H NMR (400 MHz, dmso-d6): δ 10.05 (s, 1H), 8.87 (d, 1H), 8.59 (s, 1H), 8.32 (d, 1H), 7.67 (br s, 1H), 7.59 (d, 2H), 7.52 (dd, 1H), 7.45 (td, 1H), 7.44 (d, 1H), 7.36 (dl, 1H), 7.29 (m, 2H), 7.27 (d, 2H), 7.2 (t, 2H), 7.19 (d, 1H), 7.14 (d, 1H), 7.13 (t, 1H), 7.03 (t, 1H), 6.99 (d, 1H), 6.71 (t, 1H), 6.24 (dl, 1H), 5.99 (t, 1H), 5.48 (dd, 1H), 5.41 (br s, 1H), 5.23 (m, 2H), 4.97 (s, 2H), 4.39 (m, 1H), 4.32 (dd, 1H), 4.21 (m, 2H), 3.95 (m, 2H), 3.75 (s, 3H), 3.65-3.50 (m, 10H), 3.34 (m, 2H), 3.02/2.95 (m, 2H), 2.73 (t, 2H), 2.49/2.3 (m,2H), 2.45 (m, 4H), 2.3 (m, 4H), 2 (m, 1H), 1.82 (s, 3H), 1.7/1.59 (m, 2H), 1.44/1.37 (m, 2H), 0.87 (d, 3H), 0.82 (d, 3H). ¹³C NMR (100 MHz, dmso-d6): δ 158.3, 152.9, 131.6, 131.6, 131.3, 131.3, 131, 129, 128.8, 121, 120.8, 119.5, 116.4, 116.1, 112.8, 112.4, 111.2, 74.5, 70.1, 69.3, 67.7, 66.4, 57, 56.7, 56.2, 53.7, 53.2, 50.4, 43.6, 39, 32.8, 31.6, 29.6, 27.3, 19.3, 17.7. IR Wavelength (cm⁻¹): 3500-2500, 2106, 1656. HR-ESI+: m/z [M+H]+ = 1479.5422 / 1479.5405 (measured/theoretical).

### Preparation of L24-P1:

### (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid

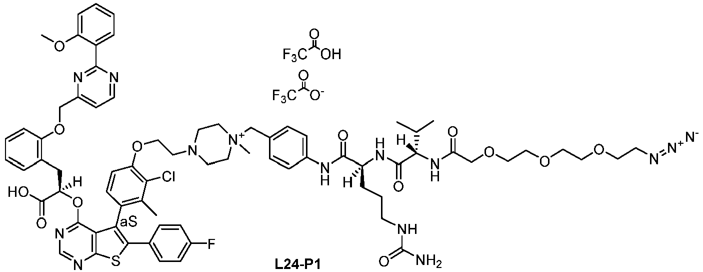

### Step 1: (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide

To a solution of (2S)-2-[[(2S)-2-[[2-[2-[2-(2-azidoethoxy)ethoxy]ethoxy]acetyl]amino]-3-methyl-butanoyl]amino]-N-[4-(hydroxymethyl)phenyl]-5-ureido-pentanamide (330 mg, 0.55 mmol; obtained according to Step 1 of the synthesis of L23-P3) in THF (10 mL) was added dropwise at 0°C a solution of phosphorus tribromide 1 M in dichloromethane (1 mL, 1 mmol). The mixture was stirred at 0°C for 1 h and finely grounded NaHCO₃ (100 mg) was added. After 10 min of stirring, the reaction was diluted with ethyl acetate and filtered. The organic layer was dried over Magnesium sulfate and concentrated. The residue (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide (283 mg, 0.43 mmol) was used without further purification. HR-ESI+: m/z [M+H]+ = = 595.3200 / 595.3198 (measured/theoretical).

### Step 2: ((2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid L24-P1

To a solution of ethyl (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate dichlorhydrate (P1) (345 mg, 0.355 mmol) in DMF (1 mL) were successively added (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide (233 mg, 0.355 mmol) and DIPEA (50 µL, 0.304 mmol). The mixture was stirred at room temperature overnight. A solution of lithium hydroxide monohydrate (15 mg, 3.55 mmol) in water (0.5 mL) was added and the reaction was stirred at room temperature for 24 h. The reaction mixture was purified by C18 reverse phase prep-HPLC by direct deposit of the reaction mixture on the XBridge column and using the TFA method to afford **L24-P1** (80 mg, 0.054 mmol). ¹H NMR (400 MHz, dmso-d6): δ 13.2 (m, 1H), 10.25 (m, 1H), 8.88 (d, 1H), 8.6 (s, 1H), 8.36 (d, 1H), 7.72 (d, 2H), 7.63 (d, 1H), 7.52 (dd, 1H), 7.46 (t, 1H), 7.44 (m, 1H), 7.43 (m, 2H), 7.37 (d, 1H), 7.3 (dd, 2H), 7.21 (t, 2H), 7.2 (d, 1H), 7.15 (d, 1H), 7.15 (t, 1H), 7.03 (t, 1H), 7 (t, 1H), 6.72 (t, 1H), 6.22 (d, 1H), 6 (t, 1H), 5.52 (m, 2H), 5.49 (dd, 1 H), 5.25 (dd, 2H), 4.5 (br s, 2H), 4.39 (m, 1H), 4.32 (m, 1H), 4.25 (m, 2H), 3.95 (br s, 2H), 3.76 (s, 3H), 3.4/3.24 (m, 4H), 3.35 (m, 2H), 3.28/2.51 (m, 2H), 3.04/2.83 (m, 4H), 3.02/2.96 (m, 2H), 2.92 (m, 2H), 2.87 (s, 3H), 1.99 (m,1 H), 1.83 (s, 3H), 1.69/1.61 (m, 2H), 1.46/1.38 (m, 2H), 0.88/0.82 (m, 6H). ¹³C NMR (125 MHz, dmso-d6): δ 134.2, 131.4, 131.3, 131.3, 131.2, 130.7, 128.7, 120.9, 120.5, 119.2, 116.3, 115.8, 112.7, 112.3, 111, 74, 70.2, 69.6, 67.8, 58.9, 56.9, 56.1, 55.4, 54, 50.5, 46.6, 44.9, 39, 32.7, 31.6, 29.8, 27.5, 19.7/18.4, 18. IR Wavelength (cm⁻¹): 3700-2200, 3000-2000, 2109, 1662, 1250-1050. HR-ESI+: m/z [M+Na]+ = 1473.5656 / 1473.5628 (measured/theoretical).

### Preparation of L13-C4:

### (2R)-2-[(5Sₐ)-5-[4-[2-[4-[[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethox y]ethoxy]propanoylamino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid

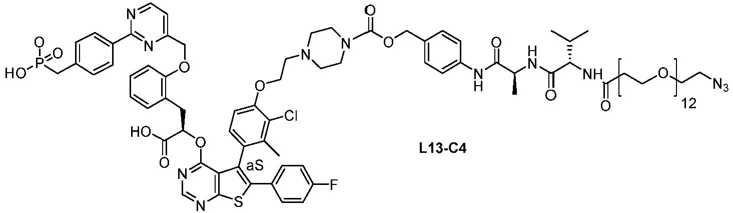

### Step 1: Synthesis of (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-N-[(1S)-2-[4-(hydroxymethyl)anilino]-1-methyl-2-oxoethyl]-3-methyl-butanamide

To a solution of (2S)-2-amino-N-[(1S)-2-[4-(hydroxymethyl)anilino]-1-methyl-2-oxoethyl]-3-methyl-butanamide (0.9 g, 3.07 mmol; obtained according to Step 3 of the synthesis of L18-C3) and 3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoic acid (purchased from Broadpharm, 2 g, 3.07 mmol) in DMF (20 mL) were successively added DIPEA (1 mL, 6.13 mmol), 3-(ethyliminomethyleneamino)propyl-dimethyl-ammonium; chloride (EDC) (0.65 g, 3.37 mmol) and [dimethylamino(triazolo[4,5-b]pyridin-3-yloxy)methylene]-dimethyl-ammonium; hexafluorophosphate (HATU) (1.28 g, 3.37 mmol). The mixture was stirred at room temperature overnight and purified by C18 reverse phase prep-HPLC by direct deposit of the reaction mixture on the Xbridge column and using the NH₄HCO₃ method to afford (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-N-[(1S)-2-[4-(hydroxymethyl)anilino]-1-methyl-2-oxo-ethyl]-3-methylbutanamide (1.64 g, 1.81 mmol). ¹H NMR (400 MHz, dmso-d6): δ 9.82 (m, 1H), 8.14 (d, 1H), 7.87 (d, 1H), 7.54 (d, 2H), 7.23 (d, 2H), 5.08 (t, 1H), 4.43 (d, 2H), 4.39 (m, 1H), 4.2 (m, 1H), 3.65-3.44 (m, 48H), 3.39 (t, 2H), 2.50-2.30 (m, 2H), 1.97 (m, 1H), 1.31 (d, 3H), 0.87/0.84 (m, 6 H). IR Wavelength (cm⁻¹): 3600-3200, 3287, 2106, 1668, 1630, 1100. HR-ESI+: m/z [M+H]+ = 919.5265 / 919.5234 (measured/theoretical).

### Step 2:[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-3-methyl-butanoyl]amino]propanoyl]amino]phenyl]methyl (4-nitrophenyl) carbonate

To a solution of (2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-N-[(1S)-2-[4-(hydroxymethyl)anilino]-1-methyl-2-oxo-ethyl]-3-methylbutanamide (210 mg, 0.228 mmol) in a mixture of THF and dichloromethane (respectively 5 and 2.5 mL) were successively added pyridine (30 µL, 0.479 mmol) and 4-Nitrophenyl chloroformate (97 mg, 0.479 mmol). The reaction was stirred at room temperature for 3h and other portions of 4-Nitrophenyl chloroformate (40 mg, 0.197 mmol) and pyridine (30 µL, 0.479 mmol) were added. The reaction mixture was stirred at 0°C for 55h and evaporated to dryness. The residue was purified by silica-gel chromatography (gradient of MeOH in dichloromethane) to afford [4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-3-methyl-butanoyl]amino]propanoyl]amino]phenyl]methyl (4-nitrophenyl) carbonate (118 mg, 0.110 mmol). ¹H NMR (400 MHz, dmso-d6): δ 10.00 (s, 1H), 8.31 (d, 2H), 8.19 (d, 1H), 7.88 (d, 1H), 7.64 (d, 2H), 7.58 (d, 2H), 7.41 (d, 2H), 5.25 (s, 2H), 4.39 (m, 1H), 4.21 (m, 1H), 3.63-3.47 (m, 48H), 3.39 (t, 2H), 2.50-2.35 (m, 2H), 1.98 (m, 1H), 1.31 (d, 3H), 0.89/0.85 (m, 6H). IR Wavelength (cm⁻¹): 3278, 2108, 1763, 1633, 1526, 1525, 1350, 1215, 1110.

### Step 3: (2R)- 2-[(5Sₐ)-5-[4-[2-[4-[[4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-djpyrimidin-4-yl]oxy-3-[2-[[2-[4-(phosphonomethyl)phenyl]pyrimidin-4-yl]methoxy]phenyl]propanoic acid (L13-C4)

To a solution of [4-[[(2S)-2-[[(2S)-2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]-3-methyl-butanoyl]amino]propanoyl]amino]phenyl]methyl (4-nitrophenyl) carbonate (52 mg, 47.6 µmol) in DMF (5 mL) were successively added (2R)-2-[(5Sₐ)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid **C4** (36.7 mg, 39.7µmol) and DIPEA (26µL, 108 µmol). The reaction was stirred at room temperature for 1 h and purified by C18 reverse phase prep-HPLC by direct deposit of the reaction mixture on the Xbridge column and using the NH₄HCO₃ method to afford **L13-C4** (36 mg, 19 µmol). ¹H NMR (400 MHz, dmso-d6): δ 10.1 (br s, 1H), 8.81 (br s, 1 H), 8.55 (m, 1 H), 8.32 (br s, 1H), 8.19 (d, 2H), 8.02 (br s, 1H), 7.66 (m, 1H), 7.58 (d, 2H), 7.37 (d, 1H), 7.29 (dd, 2H), 7.28 (d, 2H), 7.25 (d, 2H), 7.19 (t, 2H), 7.17 (d, 1H), 7.08 (t, 1H), 6.96 (d, 1H), 6.68 (t, 1H), 6.21 (d, 1H), 5.5 (m, 1H), 5.22 (m, 2H), 4.96 (s, 2H), 4.4 (m, 1H), 4.2 (dd, 1H), 4.18 (m, 2H), 3.62/3.41 (m, 24H), 3.5 (m, 4H), 3.38 (m, 2H), 3.28 (m, 4H), 2.87 (m, 2H), 2.7 (m, 2H), 2.48/2.36 (m, 2H), 2.41 (m, 4H), 1.99 (m, 1H), 1.79 (s, 3H), 1.3 (d, 3H), 0.87/0.83 (m, 6H). ¹³C NMR (100 MHz, dmso-d6): δ 130.7, 130.7, 130.6, 130.3, 129, 128.4, 127.4, 121, 119.6, 116.3, 116.1, 112.1, 70.2/67.3, 69.5, 67.5, 66.4, 58.2, 56.4, 53.2, 50.3, 49.6, 43.8, 36.3, 31, 19, 18.5, 17.8. ¹⁹F NMR (376 MHz, dmso-d6): δ -112.4. ³¹P NMR (200 MHz, dmso-d6): δ 17.8. IR Wavelength (cm⁻¹): 3290, 2102, 1698, 1651, 1237, 1094, 833, 756. HR-ESI+: m/z [M+H]+ = 1867.7129 / 1867.7154 (measured/theoretical).

### Preparation of L19-C3:

### (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

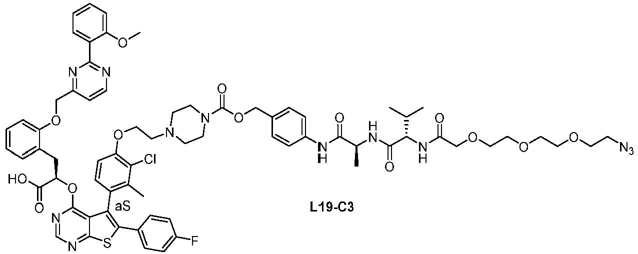

### Step 1: [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate

To a suspension of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (1 g, 1.66 mmol) in a THF/Dichloromethane mixture (respectively 100 and 30 mL), were successively added pyridine (269 µL, 3.32 mmol) and 4-Nitrophenyl chloroformate (670 mg, 3.30 mmol). The reaction was stirred at room temperature overnight and another portion of 4-Nitrophenyl chloroformate was added (335 mg, 1.66 mmol). The reaction was stirred at room temperature for 3h, concentrated and the residue was purified by silica gel chromatography (gradient of ethyl acetate in heptane) to afford [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate (658 mg, 0.97 mmol). ¹H NMR (400 MHz, dmso-d6): δ 10.07 (m, 1H), 8.31 (d, 2H), 8.19 (d, 1H), 7.89 (d, 2H), 7.74 (t, 2H), 7.64 (d, 2H), 7.57 (d, 2H), 7.41 (m, 2H), 7.41 (d, 2H), 7.4 (m, 1H), 7.32 (t, 2H), 5.24 (s, 2H), 4.43 (m, 1H), 4.36-4.19 (m, 3H), 3.92 (dd, 1H), 2 (m, 1H), 1.32 (d, 3H), 0.9/0.87 (m, 6 H). IR Wavelength (cm⁻¹): 3350-3200, 1760, 1690, 1670, 1630, 1523, 1290.

### Step 2: (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To a solution of (2R)-2-[**(5Sₐ)**-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid C3 (100 mg, 0.116 mmol) in DMF (1 mL) were successively added [4-[[(2S)-2-[[(2S)-2-(9H-fluoren-9-ylmethoxycarbonylamino)-3-methyl-butanoyl]amino]propanoyl]amino]phenyl]methyl (4-nitrophenyl) carbonate (87 mg, 0.128 mmol) and DIPEA (38 µL, 0.232 mmol). The reaction mixture was stirred at room temperature overnight and concentrated. The residue was taken up in water, filtered affording (2R)-2-[5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (110 mg, 0.078 mmol) used without further purification in the next step. ¹H NMR (400 MHz, dmso-d6): δ 10.05 (br s, 1 H), 8.88 (d, 1H), 8.57 (s, 1H), 8.23 (d, 1H), 7.88 (d, 2H), 7.75 (m, 1H), 7.74 (2d, 2H), 7.58 (d, 2H), 7.53 (dd, 1H), 7.45 (m, 1H), 7.45 (d, 1H), 7.41 (m, 1H), 7.4 (m, 2H), 7.31 (m, 2H), 7.29 (m, 2H), 7.26 (d, 2H), 7.2 (t, 2H), 7.18 (m, 1H), 7.14 (d, 1H), 7.11 (t, 1H), 7.03 (t, 1H), 6.98 (d, 1H), 6.69 (t, 1H), 6.2 (d, 1H), 5.46 (d, 1H), 5.22 (m, 2H), 4.97 (s, 2H), 4.42 (t, 1H), 4.26 (m, 2H), 4.21 (m, 1H), 4.2 (m, 2H), 3.91 (m, 1H), 3.75 (s, 3H), 3.35/2.45 (m, 2H), 3.29 (m, 4H), 2.73 (t, 2H), 2.44 (m, 4H), 1.99 (m, 1H), 1.8 (s, 3H), 1.29 (d, 3H), 0.88/0.85 (m, 6H). ¹³C NMR (100 MHz, dmso-d6): δ 158.3, 152.7, 131.6, 131.4, 131.3, 131.1, 131.1, 128.9, 128.5, 128, 127.6, 125.8, 120.9, 120.5, 120.5, 119.4, 116.4, 116, 112.7, 112.2, 111.1, 69.4, 67.8, 66.5, 66.1, 60.7, 56.8, 56.1, 53.2, 49.6, 47.1, 43.8, 33.3, 30.9, 19.7, 18.9, 18.1.

### Step 3: (2R)-2-[(5Sₐ)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2, 3-d]pyrimidin-4-yl]oxy-3-[2-[[2-(2-methoxyphenyl)pyrimidin-4-yl]methoxy]phenyl]propanoic acid

To a solution of (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (176 mg, 0.125 mmol) in DMF (3 mL) was added dropwise at 0°C piperidine (300 µL, 1.25 mmol). The reaction mixture was stirred at room temperature for 1 h and concentrated. The residue was purified by C18 reverse phase prep-HPLC by direct deposit of the reaction mixture on the Xbridge column and using the NH₄HCO₃ method to afford (2R)-2-[(5Sₐ)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (130 mg, 0.11 mmol). ¹H NMR (400 MHz, dmso-d6): δ 10.2 (s, 1H), 8.9 (d, 1H), 8.6 (dl, 1H), 8.55 (s, 1H), 7.85 (d, 1H), 7.6 (d, 2H), 7.55 (dd, 1H), 7.45 (m, 2H), 7.25 (d, 2H), 7.25 (m, 4H), 7.2 (m, 3H), 7.15 (d, 1H), 7.1 (t, 1H), 7.05 (t, 1H), 6.95 (d, 1H), 6.65 (t, 1H), 6.15 (d, 1H), 5.4 (dd, 1H), 5.2 (m, 2H), 4.95 (s, 2H), 4.45 (m, 1H), 4.2 (m, 2H), 3.75 (s, 3H), 3.4/2.35 (m, 2H), 3.3 (m, 5H), 2.6 (t, 2H), 2.4 (m, 4H), 2 (m, 3H), 1.8 (s, 3H), 1.3 (d, 3H), 0.9/0.85 (m, 6H). IR Wavelength (cm⁻¹): 3600-2500, 1678.

### Step 4: (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2, 3-d]pyrimidin-4-yl]oxy-3-[2-[[2-(2-methoxyphenyl)pyrimidin-4-yl]methoxy]phenyl]propanoic acid L19-C3

To a solution of 2R)-2-[(5Sₐ)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (50 mg, 0.042 mmol) in DMF (0.3 mL) were successively added DIPEA (14µL, 0.085 mmol) , [dimethylamino-(2,5-dioxopyrrolidin-1-yl)oxy-methylene]-dimethyl-ammonium; tetrafluoroborate (14 mg, 0.046 mmol) and a solution of 2-[2-[2-(2-azidoethoxy)ethoxy]ethoxy]acetic acid (28 mg, 0.12 mmol) in DMF (0.5 mL). The reaction mixture was stirred at room temperature for 2h and purified by C18 reverse phase prep-HPLC by direct deposit of the reaction mixture on the Xbridge column and using the NH₄HCO₃ method to afford **L19-C3** (22 mg, 0.016 mmol). ¹H NMR (400 MHz, dmso-d6): δ 10.02 (s, 1H), 8.88 (d, 1H), 8.4 (d, 1H), 7.72 (br s, 1H), 7.58 (s, 1H), 7.58 (d, 2H), 7.53 (d, 1H), 7.45 (d, 1H), 7.45 (t, 1H), 7.38 (d, 1H), 7.29 (dd, 2H), 7.27 (d, 2H), 7.2 (t, 2H), 7.18 (d, 1H), 7.14 (d, 1H), 7.11 (t, 1H), 7.03 (t, 1H), 6.98 (d, 1H), 6.7 (t, 1H), 6.21 (d, 1H), 5.46 (dd, 1H), 5.23 (m, 2H), 4.97 (s, 2H), 4.4 (m, 1H), 4.29 (dd, 1H), 4.22 (m, 2H), 3.94 (s, 2H), 3.75 (s, 3H), 3.65-3.53 (m, 10H), 3.35 (m, 2H), 3.3 (m, 4H), 3.3/2.5 (m, 2H), 2.73 (t, 2H), 2.44 (m, 4H), 2 (m, 1H), 1.81 (s, 3H), 1.3 (d, 3H), 0.88/0.82 (m, 6H). ¹³C NMR (100 MHz, dmso-d6): δ 158, 152.7, 131.4, 131.4, 131.3, 131.1, 131.1, 128.9, 128.6, 120.9, 120.7, 119.5, 116.2, 112.5, 112.1, 111.1, 70.4, 70.4, 69.7, 67.5, 66.2, 56.8, 56.7, 56.1, 53.3, 50.4, 49.5, 43.8, 31.7, 19.5, 0.82, 18.3, 18.2. ¹⁹F NMR (376 MHz, dmso-d6): δ -112.3. IR Wavelength (cm⁻¹): 3294, 2104, 1697, 1663, 1288, 1238, 1120, 1076, 1051, 1020, 833,755. HR-ESI+: m/z [M+H]+ = 1395.5083 / 1395.5070 (measured/theoretical).

### Preparation of L15-C5:

### (2R)-3-[2-[[2-[3-[[[2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethox y]ethoxy]propanoylamino]ethoxy-hydroxy-phosphoryl]oxy-hydroxy-phosphoryl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid

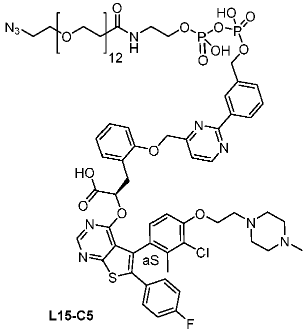

### Step 1: (2R)-2-[(56Sₐₛ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(phosphonooxymethyl]phenyl]pyrimidin-4-yl]methoxy]phenyl]propanoic acid; bis 2,2,2-trifluoroacetic acid

To a solution of ethyl (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(hydroxymethyl)phenyl]pyrimidin-4-yl]methoxy]phenyl]propanoate (110 mg, 0.123 mmol; prepared according to WO 2016/207216) in THF (0.5 mL) was added dropwise at -40°C under argon diphosphoryl chloride (51 µL, 0.368 mmol). The reaction mixture was stirred at - 40°C for 30 min. Another portion of diphosphoryl chloride (10 µL, 0.074 mmol) was added at -40°C and the reaction was stirred at -40°C for 20 min, quenched by addition of an aqueous saturated solution of potassium carbonate (0.1 mL) and allowed to warm to room temperature. The pH was adjusted to 10 by addition of potassium carbonate (powder) and the reaction was stirred for 20 min at room temperature. The reaction mixture was acidified to pH 2 by slow addition of aqueous 2 M HCl solution at 0°C, extracted with dichloromethane (4 times). The combined organic layers were concentrated, diluted with dioxane (3 mL) and a solution of lithium hydroxide monohydrate (17 mg, 0.403 mmol) in water (0.3 mL) was added. The reaction mixture was stirred at room temperature for 4 days, neutralized by an aqueous 4 M HCl solution (0.4 mL, 0.4 mmol), and evaporated. The residue was purified by C18 reverse phase prep-HPLC by direct deposit of the reaction mixture on the Xbridge column and using the TFA method to afford (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(phosphonooxymethyl)phenyl]pyrimidin-4-yl]methoxy]phenyl]propanoic acid;2,2,2-trifluoroacetic acid as a 2TFA salt (41 mg, 43 µmol). MS (ESI) m/z [M + 2H]/2+ = 487.5.

### Step 2: 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]ethyldihydrogen phosphate

To a solution of 3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoic acid (200mg, 0.311 mmol) in dichloromethane (2 mL) were added 1-hydroxypyrrolidine-2,5-dione (79 mg, 0.684 mmol), 3-(ethyliminomethyleneamino)propyl-dimethyl-ammonium; chloride (107 mg, 0.56 mmol). The reaction mixture was stirred at room temperature overnight, diluted with dichloromethane, partitioned with a saturated aqueous solution of NaHCO₃ and extracted with dichloromethane. The combined organic layers were washed with brine, dried over Magnesium sulfate and concentrated to approximately 1 mL. The residue was diluted with DMF (1 mL), 2-aminoethyl dihydrogen phosphate (30 mg, 0.214 mmol) was added and the reaction mixture was stirred at 80°C overnight, diluted with dichloromethane, washed with water. The aqueous layer was separated and freeze-dried to afford 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]ethyl dihydrogen phosphate (165 mg, 0.2 mmol). ¹H NMR (400 MHz, dmso-d6): δ 3.45-3.65 (m, 53H), 3.26-3.39 (m, 2H), 3.12 (m, 2H), 2.27 (t, 2H). HR-ESI+: m/z [M+H]+ = 767.3697 / 767.3686 (measured/theoretical).

### Step 3: (2R)-3-[2-[[2-[3-[[[2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]eth oxy]ethoxy]propanoylamino]ethoxy-hydroxy-phosphoryl]oxy-hydroxy-phosphoryl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid (L15-C5)

To a solution of 2-[3-[2-[2-[2-[2-[2-[2-[2-[2-[2-[2-[2-(2-azidoethoxy)ethoxy]ethoxy]ethoxy]ethoxy]ethoxy]ethoxy]ethoxy]ethoxy]ethoxy]ethoxy]ethoxy ]propanoylamino]ethyl dihydrogen phosphate (49 mg, 0.064 mmol) in DMF (0.2 mL) were successively added di(imidazol-1-yl)methanone (11 mg, 0.066 mmol), triethylamine (17µL, 0.066 mmol) and 4Å molecular sieves (50 mg). The reaction was stirred at room temperature for 2 h. The solid was removed by filtration and the filtrate was treated with Zinc chloride (23 mg, 0.172 mmol) and (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[3-(phosphonooxymethyl)phenyl]pyrimidin-4-yl]methoxy]phenyl]propanoic acid; bis 2,2,2-trifluoroacetic acid (41 mg, 0.043 mmol). The mixture was heated to 50°C overnight. The reaction mixture was purified by C18 reverse phase prep-HPLC by direct deposit of the reaction mixture on the Xbridge column and using the NH₄HCO₃ method to afford L15-C5 (11 mg, 6µmol). HR-ESI+: m/z [M+H]+ = 1703.5962 / 1703.5959 (measured/theoretical).

### Preparation of L17-C3:

### (2R)-2-[(5Sₐ)-5-[4-[2-[4-[[4-[[(2S)-2-[[(2S)-2-[[(2S,3R,4S,5R)-6-azido-2,3,4,5-tetrahydroxy-hexyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetic acid

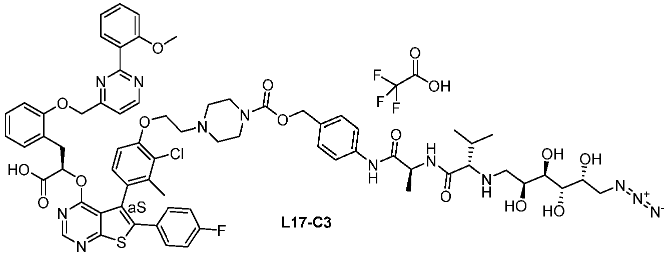

To a solution of (2R)-2-[(5Sₐ)-5-[4-[2-[4-[[4-[[(2S)-2-[[(2S)-2-amino-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (230 mg, 0.194 mmol; obtained according to Step 5 of the preparation of L19-C3) and 6-deoxy-6-azido-D-galactose (120 mg, 0.584 mmol; obtained according to Ekholm et al., ChemMedChem 2016, 11, 2501-2505) in a mixture of DMSO/water 80/20 containing 1 % of DIPEA (20 mL) was added at room temperature sodium cyanoborohydride (24 mg, 0.389 mmol). The reaction mixture was heated at 65°C for 48h. Another portion of sodium cyanoborohydride (24 mg, 0.389 mmol) and 6-deoxy-6-azido-D-galactose (120 mg, 0.584 mmol) were then added at room temperature. The reaction mixture was heated at 65°C for an additional 48h and was purified by C18 reverse phase prep-HPLC by direct deposit of the reaction mixture on the Xbridge column and using the TFA method to afford **L17-C3** (38 mg, 28 µmol). ¹H NMR (400 MHz, dmso-d6): δ 13.2 (br s, 1H), 10.2 (s, 1H), 8.88 (d, 1H), 8.85 (d, 1H), 8.62 (s, 1H), 8.53 (br s, 1H), 7.63 (d, 1H), 7.59 (d, 2H), 7.52 (d, 1H), 7.45 (t, 1H), 7.42 (d, 1H), 7.33 (dd, 2H), 7.33 (d, 2H), 7.27 (d, 1H), 7.27 (d, 1H), 7.21 (t, 2H), 7.15 (t, 1H), 7.04 (t, 1H), 7.01 (d, 1H), 6.73 (t, 1H), 6.21 (d, 1H), 5.51 (d, 1H), 5.28/5.22 (m, 2H), 5.04 (br s, 2H), 4.52 (m, 1H), 4.49 (m, 2H), 4.12 (m, 1H), 3.89 (m, 1H), 3.78 (m, 1H), 3.76 (s, 3H), 3.63 (m, 6H), 3.42/3.21 (m, 2H), 3.38 (m, 1H), 3.37 (m, 1H), 3.28/2.52 (m, 2H), 3.22 (m, 4H), 2.96 (m, 2H), 2.21 (m, 1H), 1.86 (s, 3H), 1.36 (d, 3H), 1.03/0.94 (m, 6H). ¹³C NMR (125 MHz, dmso-d6): δ 157.8, 152.5, 131.4, 131.3, 131.3, 130.6, 129.1, 129, 128.8, 120.8, 120.6, 119.4, 116.2, 116.1, 112.3, 111.3, 111.3, 74.2, 71.3, 70.4, 69.5, 69.2, 67.1, 65.6, 64.5, 64.5, 56.2, 54.8, 54.2, 51.9, 50.3, 49.9, 32.7, 29.4, 19.3, 18.9, 18. ¹⁹F NMR (470 MHz, dmso-d6): δ -74.4, -112.1. IR Wavelength (cm⁻¹): 2200-3500, 2104, 1669, 1181, 1132, 798, 758, 720. HR-ESI+: m/z [M+H]+ = 1369.4918 / 1369.4913 (measured/theoretical).

### Preparation of L24-P7:

### (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-ethyl-phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid

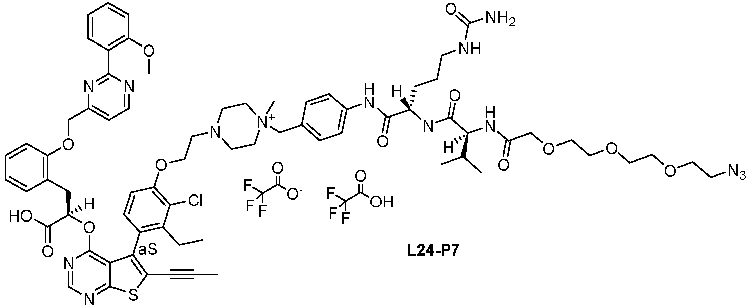

To a solution of (2S)-2-[[(2S)-2-[[2-(2-azidoethoxy)acetyl]amino]-3-methyl-butanoyl]amino]-N-[4-(bromomethyl)phenyl]-5-ureido-pentanamide (72 mg, 0.109 mmol) in THF (5 mL) were successively added (2R)-2-[(5Sₐ)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid **P7** (30 mg, 0.036 mmol) and DIPEA (19 µL, 0.108 mmol). The reaction mixture was stirred overnight at room temperature and was purified by C18 reverse phase prep-HPLC by direct deposit of the reaction mixture on the Xbridge column and using the TFA method to afford **L24-P7** (25 mg, 18µmol). ¹H NMR (400 MHz, dmso-d6): δ 10.25 (s, 1H), 8.85 (d, 1H), 8.62 (s, 1H), 8.35 (d, 1H), 7.72 (d, 2H), 7.6 (d, 1H), 7.5 (d, 1H), 7.45 (t, 1H), 7.43 (d, 2H), 7.4 (d, 1H), 7.22 (d, 1H), 7.17 (m, 1H), 7.15 (m, 1H), 7.13 (d, 1H), 7.02 (t, 1H), 7 (d, 1H), 6.78 (t, 1H), 6.3 (d, 1H), 5.98 (br s, 1H), 5.5 (dd, 1H), 5.4 (br s, 1H), 5.28/5.2 (m, 2H), 4.5 (br s, 2H), 4.38 (m, 1H), 4.3 (dd, 1H), 4.25 (m, 2H), 3.94 (br s, 2H), 3.74 (s, 3H), 3.70/3.50 (m, 10H), 3.50 (m, 8 H), 3.35 (t, 2H), 3.22/2.5 (m, 2 H), 3.0 (m, 2H), 2.95 (t, 2H), 2.9 (br s, 3H), 2.55/2.4 (m, 2H), 2.0 (s, 3H), 1.98 (m, 1H), 1.70/1.30 (m, 4H), 0.88/0.82 (m, 6H), 0.72 (t, 3H). IR Wavelength (cm⁻¹): 3321, 2111, 1660, 1188, 1124, 798,756,719. HR-ESI+: m/z [M+H-CF3COOH]+ = 1409.59077 / 1409.5903 (measured/theoretical).

### Preparation of L24-P6:

### (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoic acid; 2,2,2-trifluoroacetate; 2,2,2-trifluoroacetic acid

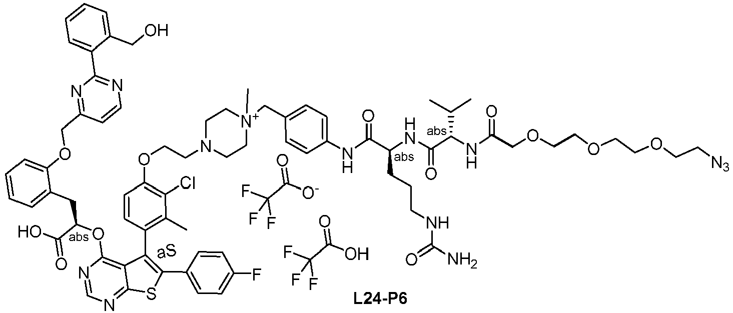

To a solution of (2S)-2-[[(2S)-2-[[2-[2-[2-(2-azidoethoxy)ethoxy]ethoxy]acetyl]amino]-3-methyl-butanoyl]amino]-N-[4-(bromomethyl)phenyl]-5-ureido-pentanamide (55.3 mg, 84 µmol) in DMF (1 mL) were successively added ethyl (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoate (53.2 mg, 59 µmol; synthesized according to EP 2 886 545) and DIPEA (44 µL, 0.252 mmol). The reaction mixture was stirred at room temperature for 1 h and concentrated under reduced pressure. The residue was diluted with dioxane (1 mL) and a solution of lithium hydroxide monohydrate (14 mg, 0.0334 mmol) in water (0.3 mL) was added. The reaction mixture was stirred at room temperature overnight, neutralized by addition of an aqueous 1 M HCl solution (0.33mL, 0.33 mmol), concentrated under reduced pressure. The crude product was purified by C18 reverse phase prep-HPLC by direct deposit of the reaction mixture on the Xbridge column and using the TFA method to afford **L24-P6** (47 mg, 32 µmol). ¹H NMR (400 MHz, dmso-d6): δ 10.27 (s, 1H), 8.94 (d, 1H), 8.61 (s, 1H), 8.38 (d, 1H), 7.93 (d, 1H), 7.73 (d, 2H), 7.68 (t, 1H), 7.66 (d, 1H), 7.5 (t, 1H), 7.45 (d, 1H), 7.43 (d, 2H), 7.38 (d, 1H), 7.37 (m, 1H), 7.3 (dd, 2H), 7.21 (d, 1H), 7.2 (t, 2H), 7.16 (t, 1H), 7.02 (d, 1H), 6.72 (t, 1H), 6.21 (d, 1H), 6.01 (m, 1H), 5.5 (d, 1H), 5.4 (m, 1H), 5.3 (m, 2H), 4.8 (s, 2H), 4.39 (m, 1H), 4.32 (dd, 1H), 4.25 (m, 2H), 3.95 (s, 2H), 3.57 (m, 16H), 3.42/3.26 (m, 2 H), 3.36 (m, 2H), 3.29/2.51 (m, 2H), 3.11/2.92 (m, 8H), 2.98 (m, 2H), 2.97 (m, 2H), 1.99 (m, 1H), 1.83 (s, 3H), 1.68/1.62 (m, 2H), 1.45/1.39 (m, 2H), 0.88/0.82 (m, 6H). ¹³C NMR (100 MHz, dmso-d6): δ 158.2, 152.1, 134.2, 131.4, 131.3, 130.9, 130.8, 130.2, 128.7, 128.1, 127, 120.8, 119.3, 116.3, 115.7, 112.2, 111, 74, 70.5, 70.1, 69.5, 67.7, 62.3, 58.8, 57.2, 55.5, 54.1, 50.5, 46.6, 38.9, 32.5, 31.5, 29.6, 27.6, 19.6, 18.6, 18.3. ¹⁹F NMR (376 MHz, dmso-d6): δ -74.6, -112.5. IR Wavelength (cm⁻¹): 3303, 2104, 1730, 1662, 1182, 1124, 833,796,761. HR-ESI+: m/z [M+2H]/2+ = 726.2957 / 726.2941 (measured/theoretical).

### Preparation of L20-C6:

### (2R)-3-[2-[[2-[2-[[2-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methoxycarbonyl-methyl-amino]ethyl-methylcarbamoyl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid

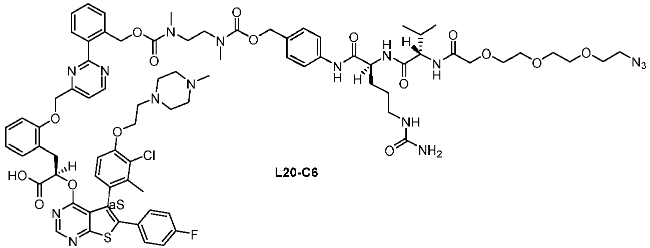

### Step 1: ethyl (2R)-3-[2-[[2-[2-[[2-[tert-butoxycarbonyl(methyl)amino]ethyl-methylcarbamoyl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoate

To a solution of (ethyl (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-[2-(hydroxymethyl)phenyl]pyrimidin-4-yl]methoxy]phenyl]propanoate (50 mg, 55 µmol; synthesized according to EP 2 886 545) in dichloromethane (0.5 mL) were successively added 4-Nitrophenyl chloroformate (19 mg, 94 µmol) and DIPEA (69 µL, 0.5 mmol). The reaction mixture was stirred at room temperature for 1 h and tert-butyl N-methyl-N-[2-(methylamino)ethyl]carbamate (54 mg, 0.287 mmol) was added. The mixture was stirred at room temperature overnight, concentrated under reduced pressure. The residue was purified by silica gel chromatography (gradient of methanol in dichloromethane) to afford ethyl (2R)-3-[2-[[2-[2-[[2-[tert-butoxycarbonyl(methyl)amino]ethyl-methylcarbamoyl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoate (30 mg, 27 µmol). ¹H NMR (500 MHz, dmso-d6): δ 9.00 (d, 1H), 8.58 (s, 1H), 7.98 (m, 1H), 7.61 (d, 1H), 7.51 (t, 1H), 7.48 (d, 1H), 7.45 (t, 1H), 7.31 (dd, 2H), 7.31 (d, 1H), 7.22 (t, 2H), 7.18 (t, 1H), 7.17 (d, 1H), 7.02 (d, 1H), 6.76 (t, 1H), 6.32 (d, 1H), 5.52 (dd, 1H), 5.47 (br s, 2H), 5.26 (m, 2H), 4.2 (m, 2H), 4.07 (m, 2H), 3.24/3.17 (2m, 4H), 3.17/2.6 (2m, 2H), 2.77/2.64 (m, 6H), 2.7 (m, 2H), 2.49/2.28 (m, 8H), 2.12 (br s, 3H), 1.87 (s, 3H), 1.3 (3s, 9H), 1.07 (t, 3H). ¹³C NMR (125 MHz, dmso-d6): δ 158.2, 152.4, 131, 130.1, 130.1, 129, 128.3, 128.2, 121.5, 121.4, 120.9, 116.3, 115.8, 112, 111.1, 74.1, 69.2, 68.1, 65.6, 61.2, 56.8, 55.2, 53.1, 46.5, 45.9, 34.5, 32.4, 28.3, 17.4, 14.9.¹⁹F NMR (470 MHz, dmso-d6): δ - 112.2. IR Wavelength (cm⁻¹): 1750, 1693, 1221/1160/1120, 834/756.

### Step 2: 2R)-3-[2-[[2-[2-[[2-[[4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methoxycarbonyl-methyl-amino]ethyl-methylcarbamoyl]oxymethyl]phenyl]pyrimidin-4-yl]methoxy]phenyl]-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoic acid L20-C6

To a solution of ethyl (2R)-3-[2-[[2-[2-[[2-[tert-butoxycarbonyl(methyl)amino]ethylmethyl-carbamoyl]oxymethyl]phenyl]pyrimidin-4-yl]methoxy]phenyl]-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-propanoate (25 mg, 22 µmol) in dichloromethane (0.5 mL) was added at 0°C trifluoroacetic acid (35 µL, 447 mmol). The reaction mixture was stirred at room temperature for 6h and concentrated under reduced pressure. The residue was diluted with DMF (0.5 mL) and [4-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]phenyl]methyl (4-nitrophenyl)carbonate (20 mg, 22 µmol; obtained according to Step 3 of the preparation of L23-P3) and DIPEA (78 µL, 0.447 mmol) were successively added. The reaction mixture was stirred at room temperature overnight, concentrated under reduced pressure, diluted with dioxane (0.5 mL) and a solution of lithium hydroxide monohydrate (3.7 mg, 89 µmol) in water (0.3 mL) was added. The reaction was stirred at room temperature overnight, neutralized at 0°C by a dropwise addition of an aqueous 1M HCl solution until pH7 and concentrated under reduced pressure.

The crude product was purified by C18 reverse phase prep-HPLC by direct deposit of the reaction mixture on the Xbridge column and using the NH₄HCO₃ method to afford L20-C6 (13 mg, 8 µmol). ¹H NMR (500 MHz, dmso-d6): δ 8.88 (m, 1H), 8.54 (s, 1 H), 7.97 (d, 1H), 7.77 (m, 1H), 7.6 (d, 2H), 7.5 (m, 1H), 7.47 (m, 1H), 7.46 (m, 1H), 7.41 (d, 1H), 7.29 (dd, 2H), 7.21 (t, 2H), 7.19 (d, 1H), 7.18 (m, 2H), 7.12 (t, 1H), 6.97 (d, 1H), 6.7 (t, 1H), 6.19 (d, 1H), 5.49 (d, 1H), 5.45 (m, 4H), 5.23 (m, 2H), 4.89 (m, 2H), 4.4 (m, 1H), 4.32 (dd, 1H), 4.22 (m, 2H), 3.94 (s, 2H), 3.56 (m, 10H), 3.39/2.44 (m, 2H), 3.34 (t, 2H), 3.28 (m, 4H), 2.99 (m, 2H), 2.75/2.7 (m, 6H), 2.73 (m, 2H), 2.5/2.37 (m, 8H), 2.18 (s, 3H), 2.04 (m, 1H), 1.81 (s, 3H), 1.74/1.62 (m, 2H), 1.46/1.38 (m, 2H), 0.86/0.8 (m, 6H). ¹³C NMR (125 MHz, dmso-d6): δ 158.3, 152.9, 131.5, 131.4, 131.3, 131, 130, 128.3, 128.3, 128, 127.7, 120.8, 119.3, 116.2, 115.6, 112.1, 111.1, 75.3, 70.5, 70.2, 69.2, 67.6, 66.6, 65.4, 57.2, 56.7, 55.1/52.9, 54, 50.5, 46.5, 45.1, 39.1, 34.4, 31.5, 29.6, 27.4, 19.9, 18.2, 18. ¹⁹F NMR (470 MHz, dmso-d6): δ - 112.5. IR Wavelength (cm⁻¹): 3323, 2106, 1691, 1660, 1220, 1120, 1051, 759. HR-ESI+: m/z [M+H]+ = 1609.6517 / 1609.6500 (measured/theoretical).

### Preparation of L22-C1:

### (2R)-2-[(5Sa)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate;2,2,2-trifluoroacetic acid

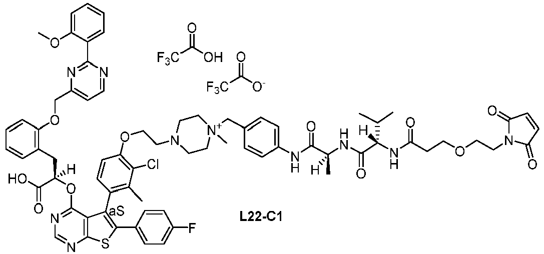

### Step 1: (2R)-2-[(5Sₐ)-5-[4-[2-[4-[[4-[[(2S)-2-[[(2S)-2-amino-3-methyl-butanoyl]amino]propanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid

To a solution of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (200 mg, 0.388 mmol) in DMF (20 mL) were successively added triphenylphosphine (152 mg, 0.581 mmol) and N-Bromosuccinimide (103 mg, 0.581 mmol). The reaction mixture was stirred at room temperature overnight and (2R)-2-[(**5Sₐ**)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid C1 (302 mg, 345 mmol) and DIPEA (120 µL, 0.691 mmol) were added. The reaction was stirred at room temperature for 2h and diethylamine (49µL, 486 mmol) was added. The reaction was stirred at room temperature for 24h , concentrated under reduced pressure and purified by C18 reverse phase prep-HPLC by direct deposit of the reaction mixture on the Xbridge column and using the TFA method to afford (2R)-2-[(5Sₐ)-5-[4-[2-[4-[[4-[[(2S)-2-[[(2S)-2-amino-3-methyl-butanoyl]amino]propanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid (253 mg, 0.220 mmol). ¹H NMR (400 MHz, dmso-d6): δ 10.4 (s, 1H), 8.89 (d, 1H), 8.75 (d, 1H), 8.61 (s, 1H), 8.08 (large, 3H), 7.72 (d, 2H), 7.63 (d, 1H), 7.52 (d, 1H), 7.46 (t, 1H), 7.45 (d, 2H), 7.39 (d, 1H), 7.31 (dd, 2H), 7.21 (d, 1H), 7.21 (t, 2H), 7.15 (d, 1H), 7.15 (t, 1H), 7.04 (t, 1H), 7.01 (d, 1H), 6.72 (t, 1H), 6.22 (d, 1H), 5.5 (dd, 1H), 5.25 (m, 2H), 4.53 (m, 2H), 4.52 (m, 1H), 4.28 (m, 2H), 3.76 (s, 3H), 3.62 (m, 1H), 3.43/3.29 (m, 4H), 3.28/2.5 (m, 2H), 3.13/2.94 (m, 4H), 3.01 (m, 2H), 2.9 (br s, 3H), 2.07 (m, 1H), 1.84 (d, 3H), 1.36 (d, 3H), 0.95 (d, 6H). ¹³C NMR (125 MHz, dmso-d6): δ 253, 158.2, 134.3, 131.5, 131.4, 131.4, 131.3, 131, 128.9, 121.1, 120.6, 119.5, 116.3, 115.9, 113, 112.3, 111.1, 74.1, 69.8, 67.5, 58.7, 57.9, 56.5, 55.4, 49.8, 46.5, 45.2, 32.9, 30.4, 18.6, 18.4, 18.3. ¹⁹F NMR (470 MHz, dmso-d6): δ -74, -112.6.

### Step 2: (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid L22-C1

To a solution of (2R)-2-[(5Sₐ)-5-[4-[2-[4-[[4-[[(2S)-2-[[(2S)-2-amino-3-methyl-butanoyl]amino]propanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis-2,2,2-trifluoroacetic acid (150 mg, 0.130 mmol) in DMF (0.4 mL) was added (2,5-dioxopyrrolidin-1-yl) 3-[2-(2,5-dioxopyrrol-1-yl)ethoxy]propanoate (60 mg, 194 mmol). The reaction mixture was stirred at room temperature for 3h, concentrated under reduced pressure and purified by C18 reverse phase prep-HPLC by direct deposit of the reaction mixture on the Xbridge column and using the TFA method to afford **L22-C1** (67 mg, 37 µmol). ¹H NMR (400 MHz, dmso-d6): δ 10.14 (s, 1H), 8.88 (d, 1H), 8.61 (s, 1H), 8.22 (d, 1H), 7.84 (d, 1H), 7.73 (d, 2H), 7.63 (d, 1H), 7.52 (dd, 1H), 7.45 (td, 1H), 7.44 (d, 2H), 7.38 (d, 1H), 7.31 (dd, 2H), 7.21 (d, 1H), 7.21 (t, 2H), 7.15 (t, 1H), 7.14 (d, 1H), 7.02 (t, 1H), 7.01 (d, 1H), 7 (s, 2H), 6.71 (t, 1H), 6.21 (d, 1H), 5.5 (dd, 1H), 5.25 (m, 2H), 4.53 (br s, 2H), 4.38 (m, 1H), 4.25 (m, 2H), 4.19 (dd, 1H), 3.76 (s, 3H), 3.58 (m, 2H), 3.54 (t, 2H), 3.48 (m, 2H), 3.43/3.3 (m, 4H), 3.28/2.51 (m, 2H), 3.16/2.98 (m, 4H), 3.04 (m, 2H), 2.91 (br s, 3H), 2.43/2.33 (m, 2H), 1.93 (m, 1H), 1.84 (s, 3H), 1.31 (d, 3H), 0.87/0.82 (m, 6H). ¹³C NMR (100 MHz, dmso-d6): δ 158, 152.8, 135.2, 134, 131.4, 131.3, 131.3, 131.2, 131, 128.9, 120.8, 120.6, 119.3, 116.3, 115.8, 112.4, 112.3, 111.1, 74.2, 69.6, 67.4, 67.4, 67.1, 67, 58.4, 57.9, 56.2, 55.2, 49.7, 46.5, 45.1, 37.1, 36.3, 32.7, 30.9, 19.6, 18.5, 18.2, 18.2. ¹⁹F NMR (376 MHz, dmso-d6): δ -74.6, -112.2. IR Wavelength (cm⁻¹): 2000-3500, 1760/1705, 1733, 1668, 1180/1128, 829/798/758/720/696. HR-ESI+: m/z [M+H]+ = 1345.4944 / 1345.4954 (measured/theoretical)

### Preparation of L9-C9:

### 3-[4-[[2-[(2R)-2-carboxy-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxyethyl]phenoxy]methyl]pyrimidin-2-yl]benzenesulfonate; 2,2,2-trifluoroacetate; 2,2,2-trifluoroacetic acid

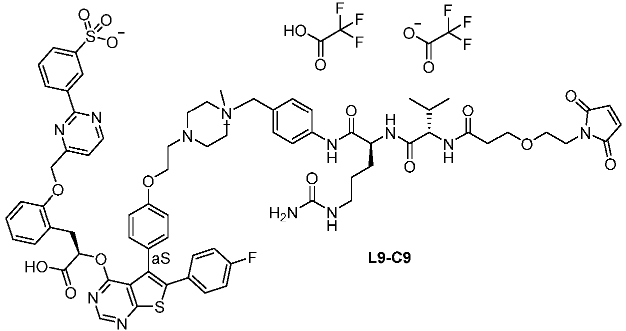

### Step 1: (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide

To a solution of 3-[2-(2,5-dioxopyrrol-1-yl)ethoxy]propanoic acid (855 mg, 4.01 mmol) in THF (42 mL) were added N,N'-dicyclohexylmethanediimine (1.05 g, 5.08 mmol) and 1-hydroxypyrrolidine-2,5-dione (510 mg, 4.43 mmol). The reaction mixture was stirred at room temperature for 20 h. The precipitate was removed by filtration and the filtrate added to a solution of (2S)-2-[[(2S)-2-amino-3-methyl-butanoyl]amino]-N-[4-(hydroxymethyl)phenyl]-5-ureido-pentanamide (1.27 g, 3.35 mmol) in DMF (42 mL). The reaction mixture was stirred at room temperature for 20 h, diluted with diethyl ether (250 mL). The solid was recovered by filtration to afford (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide (1.81 g ; 3.15 mmol) as a white solid. ¹H NMR (400 MHz, dmso-d6): δ 9.87 (s, 1H), 8.05 (d, 1H), 7.82 (d, 1H), 7.53 (d, 2H), 7.21 (d, 2H), 7.00 (s, 2H), 5.95 (t, 1H), 5.39 (s, 2H), 5.07 (t, 1H), 4.41 (d, 2H), 4.34-4.40 (m, 1H), 4.18-4.22 (m, 1H), 3.42-3.65 (m, 4H), 2.88-3.02 (m, 2H), 2.73 (s, 2H), 2.28-2.45 (m, 2H), 1.91-1.99 (m, 1H), 1.53-1.75 (m, 2H), 1.30-1.147 (m, 2H), 0.85 (d, 3H), 0.81 (d, 3H). ¹³C NMR (125 MHz, dmso-d6): δ 171.05, 170.83, 170.32, 170.09, 158.82, 137.49, 137.37, 134.50, 126.88, 118.81, 66.66, 66.53, 62.57, 57.49, 53.06, 36.74, 35.76, 30.51, 29.31, 26.79, 25.20, 19.16, 18.07. MS (ESI) m/z [M + H]+ = 575.2.

### Step 2: (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide

To a solution of (2S)-2-[[(2S)-2-[3-[2-(2,5-dioxopyrrol-1-yl)ethoxy]propanoylamino]-3-methyl-butanoyl]amino]-N-[4-(hydroxymethyl)phenyl]-5-ureido-pentanamide (37.2 mg, 65 µmol) in THF (1 mL) was added dropwise at 0°C under argon phosphorus tribromide (45 µL, 97 mmol). The reaction was stirred at 0°C for 1 h and at room temperature for 2h. The progress of the reaction was followed by UPLC-MS: an aliquot was treated by a large excess of morpholine in acetonitrile, following the formation of the corresponding morpholine adduct. The reaction was diluted with THF (3 mL), quenched by addition of 2 drops of a saturated solution of NaHCO₃, stirred for 5 min at room temperature, dried over Magnesium sulfate and filtered. The residue, containing the crude (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (45 mg, 65µmol theoretical) was used immediately in the next step. MS (ESI) m/z [M + H]+ =662.62 (morpholine adduct)

### Step 3: 3-[4-[[2-[(2R)-2-carboxy-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-ethyl]phenoxy]methyl]pyrimidin-2-yl]benzenesulfonate L9-C9

To a solution of (2R)-2-{[(*5Sₐ*)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(3-sulfophenyl)pyrimidin-4-yl]methoxy}phenyl)propanoic acid C9 (15 mg, 16 mmol) in DMF (0.8 mL) was added a solution of (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (45 mg crude, 65 µmol theoretical from step 2) in THF (1 mL) and DIPEA (14µL, 81 µmol). The reaction was stirred at room temperature heated at 50°C for 2h. The crude product was purified by C18 reverse phase prep-HPLC by direct deposit of the reaction mixture on the Xbridge column and using the TFA method to afford L9-C9 (5.2 mg, 3.5 µmol). HR-ESI+: m/z [M+H]+ = 1481.4917 / 1481.4896 (measured/theoretical).

### Preparation of L9-C13:

### (2R)-2-[6-(3-amino-4,5-difluoro-phenyl)-(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate;2,2,2-trifluoroacetic acid

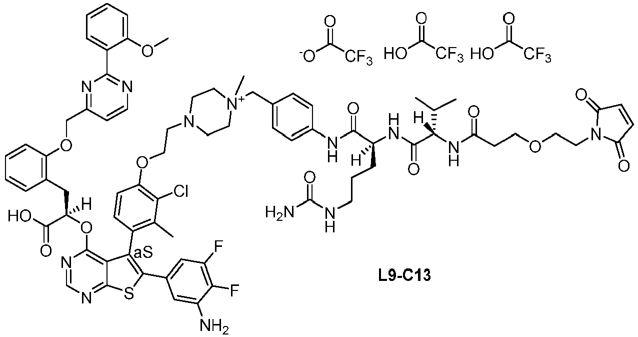

The procedure is as in the process of synthesis of L9-C9, replacing C9 used in Step 3 by (2R)-2-{[(*5Sₐ*)-6-(3-amino-4,5-difluorophenyl)-5-{3-chloro-2-methyl-4-[2-(4-methylpiperazin-1-yl)ethoxy]phenyl}thieno[2,3-d]pyrimidin-4-yl]oxy}-3-(2-{[2-(2-methoxyphenyl)pyrimidin-4-yl]methoxy}phenyl)propanoic acid C13 and using TFA method for purification. ¹H NMR (400 MHz, dmso-d6): δ 10.2 (s), 8.9 (d, 1H), 8.6 (s, 1H), 8.12 (d), 7.8 (d), 7.7 (d, 2H), 7.6 (d, 1H), 7.5 (d, 1H), 7.45 (d, 2H), 7.42 (m, 1H), 7.32 (d, 1H), 7.2 (d, 1H), 7.18 (m, 1H), 7.18 (m, 1H), 7.02 (d, 1H), 7 (s, 2H), 7 (m, 1H), 6.75 (t, 1H), 6.65 (d, 1H), 6.25 (d, 1H), 6.15 (dd, 1H), 5.98 (m, 1H), 5.48 (dd, 1H), 5.4 (br s, 1H), 5.24 (dd, 2H), 4.51 (br s, 2H), 4.38 (m, 1H), 4.28 (m, 2H), 4.22 (m, 1H), 3.80-3.40 (m, 8H), 3.75 (s, 3H), 3.26 (m, 4H), 3.1 (m, 2H), 2.98 (m, 4H), 2.9 (br s, 3H), 2.9/2.5 (2m, 2H), 2.43/2.3 (2m, 2H), 1.92 (m, 1H), 1.88 (s, 3H), 1.70-1.30 (m, 4H), 0.82 (2d, 6H). ¹⁹F NMR (470 MHz, dmso-d6): δ -74.3, - 139.3, -160.4. HR-ESI+: m/z [M+H]+ = 1464.5482 / 1464.5449 (measured/theoretical).

### Preparation of L14-C3:

### (2S,3S,4R,5R,6S)-6-[2-[(5Sₐ)-5-[[(2S)-2-[[(2S)-2-[[2-(2-azidoethoxy)acetyl]amino]-3-methyl-butanoyl]amino]propanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic acid

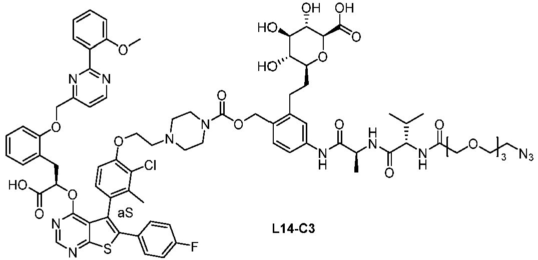

### Step 1: 2-iodo-4-nitro-benzoic acid

To a solution of 2-amino-4-nitro-benzoic acid (10.0 g, 54.90 mmol) in acetonitrile (280 mL) was added p-toluenesulfonic acid monohydrate (32.0 g, 168.2 mmol). The mixture was stirred at room temperature for 15 min, then a solution of sodium nitrite (8.00 g, 115.9 mmol) and potassium iodide (24.0 g, 144.6 mmol) in solution in water (140 mL) were added dropwise in 15 min. The reaction mixture was stirred for 19 h. After completion of the reaction, the mixture was quenched with sodium thiosulfate (13.02 g, 82.36 mmol) and acidified with an aqueous solution of hydrogen chloride 3 M (25 mL). The aqueous layer was extracted with ethyl acetate (2 x 250 mL) and the combined organic layers were washed with an aqueous solution of hydrogen chloride 1 M (100 mL), dried over sodium sulfate, filtered and concentrated to dryness. The resulting residue was taken up in dichloromethane (1 L) and was washed with an aqueous solution of HCl 1 M (100 mL). The organic layer was dried over sodium sulfate, filtered and concentrated to dryness to afford 2-iodo-4-nitro-benzoic acid (15.0 g, 51.2 mmol) as an orange powder. ¹H NMR (400 MHz, dmso-d6): δ 13.8 (br s, 1H), 8.64 (s, 1H), 8.27 (d, 1H), 7.86 (d, 1H).

### Step 2: (2-iodo-4-nitro-phenyl)methanol

To a solution of 2-iodo-4-nitro-benzoic acid (5.0 g, 17.06 mmol) in THF (70 mL) was added a solution of borane 1 M in THF (85 mL, 85.0 mmol). The reaction mixture was stirred at 65°C for 4 h. After the completion of the reaction, the reaction mixture was cooled to room temperature and was quenched with the addition of methanol (200 mL). The mixture was stirred at room temperature for 30 min, then was concentrated to dryness. The crude product was purified by silica gel chromatography (gradient of ethyl acetate in cyclohexane) to afford (2-iodo-4-nitro-phenyl)methanol (3.38 g, 12.11 mmol) as a yellow solid. ¹H NMR (400 MHz, dmso-d6): δ 8.54 (d, 1H), 8.29 (dd, 1H), 7.70 (d, 1H), 5.82 (t, 1H), 4.47 (d, 2H).

### Step 3: (4-amino-2-iodo-phenyl)methanol

To a solution of (2-iodo-4-nitro-phenyl)methanol (3.70 g, 13.26 mmol) in ethanol (100 mL) and water (25 mL) were successively added iron (3.70 g, 66.25 mmol) and ammonium chloride (800 mg, 14.96 mmol). The reaction mixture was stirred for 3 hours at 80°C. After completion of the reaction, the reaction mixture was filtered over Celite^{®}, washed with ethanol and concentrated to dryness. The resulting residue was taken up in ethyl acetate (100 mL) and washed with a saturated solution of sodium hydrogen carbonate (100 mL). The organic layer was dried over sodium sulfate, filtered and concentrated to dryness to afford (4-amino-2-iodo-phenyl)methanol (2.48 g, 9.95 mmol) as a yellow oil. ¹H NMR (400 MHz, dmso-d6): δ 7.02-7.10 (m, 2H), 6.57 (d, 1H), 5.16 (s, 2H), 4.97 (t, 1H), 4.28 (d, 2H).

### Step 4: 4-[[tert-butyl(dimethyl)silyl]oxymethyl]-3-iodo-aniline

To a solution of (4-amino-2-iodo-phenyl)methanol (3.51 g, 13.37 mmol) in dichloromethane (150 mL) was added imidazole (0.95 g, 13.95 mmol). The mixture was cooled to 0°C, then a solution of tert-butyl-chloro-dimethyl-silane (2.40 mL, 13.85 mmol) in dichloromethane (150 mL) was added dropwise over 15 minutes. The ice bath was removed, and the reaction mixture was stirred at room temperature for 16 h. After completion of the reaction, the reaction mixture was quenched with methanol (20 mL) and concentrated to dryness. The crude product was purified by silica gel chromatography (gradient of ethyl acetate in cyclohexane) to afford 4-[[tert-butyl(dimethyl)silyl]oxymethyl]-3-iodo-aniline (3.64 g ; 10.03 mmol ; 75%) as a yellow oil. ¹H NMR (400 MHz, dmso-d6): δ 7.05 (s, 1H), 7.03 (d, 1H), 6.55 (d, 1H), 5.24 (s, 2H), 4.46 (s, 2H), 0.88 (s, 9H), 0.06 (s, 6H).

### Step 5: (2S)-2-[[(2S)-2-(9H-fluoren-9-ylmethoxycarbonylamino)-3-methyl-butanoyl]amino]propanoic acid

To a solution of (2S)-2-aminopropanoic acid (3.22 g, 36.09 mmol) in water (90 mL) were successively added sodium carbonate (7.29 g, 68.74 mmol) and a solution of (2,5-dioxopyrrolidin-1-yl) (2S)-2-(9H-fluoren-9-ylmethoxycarbonylamino)-3-methyl-butanoate (15.0 g, 34.37 mmol) in dimethoxyethane (90 mL). The reaction mixture was stirred for 16 h at room temperature. After completion of the reaction, the mixture was acidified until pH=1 with an aqueous solution of hydrogen chloride 1 M, then the aqueous layer was extracted with ethyl acetate (3 x 500 mL). The combined organic layers were dried over sodium sulfate, filtered and concentrated to dryness to afford the crude mixture which was triturated with diethyl ether (50 mL) to afford (2S)-2-[[(2S)-2-(9H-fluoren-9-ylmethoxycarbonylamino)-3-methyl-butanoyl]amino]propanoic acid (11.25 g, 27.41 mmol) as a white solid. ¹H NMR (400 MHz, dmso-d6) δ 12.48 (s, 1H), 8.21 (d, 1H), 7.89 (d, 2H), 7.72-7.79 (m, 2H), 7.28-7.46 (m, 5H), 4.15-4.32 (m, 4H), 3.90 (t, 1H), 1.90-2.02 (m, 1H), 1.28 (d, 3H), 0.86-0.90 (m, 6H).

### Step 6: 9H-fluoren-9-ylmethyl N-[(1S)-1-[[(1S)-2-[4-[[tert-butyl(dimethyl)silyl]oxymethyl]-3-iodo-anilino]-1-methyl-2-oxo-ethyl]carbamoyl]-2-methyl-propyl]carbamate

To a solution of (2S)-2-[[(2S)-2-(9H-fluoren-9-ylmethoxycarbonylamino)-3-methyl-butanoyl]amino]propanoic acid (1.50 g, 3.65 mmol) in dichloromethane (18 mL) and methanol (18 mL) were successively added 4-[[tert-butyl(dimethyl)silyl]oxymethyl]-3-iodoaniline (1.33 g, 3.65 mmol) and ethyl 2-ethoxy-2H-quinoline-1-carboxylate (1.36 g, 5.48 mmol). The colorless suspension was stirred for 16 h at room temperature. After concentration to dryness, the crude product was purified by silica gel chromatography (gradient of ethyl acetate in cyclohexane) and then by C18 chromatography (gradient of methanol in water) to afford 9H-fluoren-9-ylmethyl N-[(1S)-1-[[(1S)-2-[4-[[tert-butyl(dimethyl)silyl]oxymethyl]-3-iodo-anilino]-1-methyl-2-oxo-ethyl]carbamoyl]-2-methylpropyl]carbamate (1.18 g, 1.56 mmol) as a white solid. ¹H NMR (400 MHz, dmso-d6): δ 10.05 (s, 1H). 8.16-8.24 (m, 2H), 7.88 (d, 2H), 7.71-7.77 (m, 2H), 7.55 (d, 1H), 7.37-7.48 (m, 3H), 7.27-7.37 (m, 3H), 4.56 (s, 2H), 4.38 (t, 1H), 4.18-4.33 (m, 3H), 3.91 (t, 1H), 2.08-2.20 (m, 1H), 1.30 (d, 3H), 0.83-0.95 (m, 15H), 0.06 (s, 6H).

### Step 7: (3R,4S,5R,6R)-3,4,5-tribenzyloxy-6-(benzyloxymethyl)tetrahydropyran-2-one

A suspension of (3R,4S,5R,6R)-3,4,5-tribenzyloxy-6-(benzyloxymethyl)tetrahydropyran-2-ol (30.0 g, 55.49 mmol) in DMSO (120 mL) was stirred for 30 min at room temperature (until full solubilisation) then acetic anhydride (90 mL) was added dropwise at room temperature over 15 min. The beige solution was stirred for 16 h then was cooled to 0°C and an aqueous solution of hydrogen chloride 1 M (100 mL) was slowly added. The reaction mixture was stirred for 20 min at room temperature then acetic acid was evaporated. The resulting residue was diluted with water (200 mL) and ethyl acetate (200 mL). The aqueous layer was extracted with ethyl acetate (2 x 200 mL) and the combined organic layers were washed with water (2 x 500 mL), with a saturated solution of sodium hydrogen carbonate (2 x 500 mL), then dried over sodium sulfate, filtered and concentrated to dryness to afford the crude mixture. The crude product was purified by silica gel chromatography (gradient of ethyl acetate in cyclohexane) to afford (3R,4S,5R,6R)-3,4,5-tribenzyloxy-6-(benzyloxymethyl)tetrahydropyran-2-one (25.05 g, 46.51 mmol) as a colorless oil. ¹H NMR (400 MHz, dmso-d6): δ 7.19-7.39 (m, 20H), 4.85 (d, 1H), 4.57-4.72 (m, 5H), 4.46-4.56 (m, 3H), 4.36 (d, 1H), 3.98-4.05 (m, 1H), 3.84-3.92 (m, 1H), 3.65-3.76 (m, 2H).

### Step 8: (3R,4S,5R,6R)-3,4,5-tribenzyloxy-6-(benzyloxymethyl)-2-(2-trimethylsilylethynyl)tetrahydropyran-2-ol

To a solution of trimethylsilylacetylene (24 mL, 168.6 mmol) in THF (325 mL) was added in 20 min at -78°C a solution of butyllithium 2.5 M in hexane (59.41 mL, 148.5 mmol). The colorless solution was stirred for 45 min at -78°C and then 45 min at 0°C. The reaction mixture was cooled to -78°C and a solution of (3R,4S,5R,6R)-3,4,5-tribenzyloxy-6-(benzyloxymethyl)tetrahydropyran-2-one (25.0 g, 46.41 mmol) in THF (325 mL) was added dropwise over 45 min. The reaction mixture was stirred for 4 h at this temperature then was quenched with water (200 mL). The aqueous layer was extracted with ethyl acetate (2 x 200 mL). The combined organic layers were dried over sodium sulfate, filtered and concentrated to dryness to afford (3R,4S,5R,6R)-3,4,5-tribenzyloxy-6-(benzyloxymethyl)-2-(2-trimethylsilylethynyl)tetrahydropyran-2-ol (29.56 g, 46.41 mmol) as a beige oil containing the two diastereoisomers in a ratio 4/6. ¹H NMR (400 MHz, dmso-d6): δ 7.13-7.43 (m, 20H), 4.87-4.99 (m, 1H), 4.65-4.83 (m, 4H), 3.43-3.57 (m, 3H), 3.70-3.85 (m, 2H), 3.55-3.68 (m, 3H), 3.43-3.53 (m, 2H), 0.11-0.22 (m, 9H).

### Step 9: trimethyl-[2-[(2S,3S,4R,5R,6R)-3,4,5-tribenzyloxy-6-(benzyloxymethyl)tetrahydropyran-2-yl]ethynyl]silane

To a solution of (3R,4S,5R,6R)-3,4,5-tribenzyloxy-6-(benzyloxymethyl)-2-(2-trimethylsilylethynyl)tetrahydropyran-2-ol (29.56 g, 46.42 mmol) in acetonitrile (83 mL) and dichloromethane (193 mL) were added in 20 min at -15°C a solution of triethylsilane (44.98 mL, 278.5 mmol) in a mixture of acetonitrile/dichloromethane (37 mL/18 mL) followed by a solution of boron trifluoride diethyl etherate (23.53 mL, 185.7 mmol) in acetonitrile (37 mL) over 30 min at -15°C. The colorless solution was stirred for 5 h at the same temperature, then was diluted with water (500 mL). The aqueous layer was extracted with ethyl acetate (2 x 500 mL). The combined organic layers were dried over sodium sulfate, filtered and concentrated to dryness to afford trimethyl-[2-[(2S,3S,4R,5R,6R)-3,4,5-tribenzyloxy-6-(benzyloxymethyl)tetrahydropyran-2-yl]ethynyl]silane (28.82 g, 46.41 mmol) as a brown oil. ¹H NMR (400 MHz, dmso-d6): δ 7.10-7.44 (m, 20H), 4.93 (d, 1H), 4.67-4.86 (m, 4H), 4.43-4.57 (m, 3H), 4.16-4.28 (m, 1H), 3.42-3.68 (m, 6H), 0.15 (s, 9H).

### Step 10: (2R,3R,4R,5S,6S)-3,4,5-tribenzyloxy-2-(benzyloxymethyl)-6-ethynyl-tetrahydropyran

To a solution of trimethyl-[2-[(2S,3S,4R,5R,6R)-3,4,5-tribenzyloxy-6-(benzyloxymethyl)tetrahydropyran-2-yl]ethynyl]silane (28.80 g, 46.39 mmol) in methanol (1.12 L) and dichloromethane (240 mL) was added an aqueous solution of sodium hydroxide 1 M (80 mL). The beige solution was stirred for 1 h at room temperature then was acidified until pH = 1 with an aqueous solution of hydrogen chloride 1 M and diluted with water (500 mL). The methanol was evaporated and then the aqueous layer was extracted with ethyl acetate (2 x 1 L). The combined organic layers were dried over sodium sulfate, filtered and concentrated to dryness. The crude product was purified by silica gel chromatography (gradient of ethyl acetate in cyclohexane) to afford (2R,3R,4R,5S,6S)-3,4,5-tribenzyloxy-2-(benzyloxymethyl)-6-ethynyl-tetrahydropyran (20.00 g, 36.45 mmol) as a colorless oil. ¹H NMR (400 MHz, dmso-d6): δ 3.42-3.67 (m, 7H), 4.17 (d, 1H), 4.44-4.56 (m, 3H), 4.67-4.86 (m, 4H), 4.90 (d, 1H), 7.15-7.40 (m, 20H).

### Step 11: (2S,3R,4R,5S,6R)-2-ethynyl-6-(hydroxymethyl)tetrahydropyran-3,4,5-triol

To a solution of (2R,3R,4R,5S,6S)-3,4,5-tribenzyloxy-2-(benzyloxymethyl)-6-ethynyl-tetrahydropyran (20.00 g, 36.45 mmol) in ethanthiol (400 mL) was added dropwise at room temperature over 5 min, boron trifluoride diethyl etherate (147.8 mL, 1166 mmol). The beige solution was stirred for 16 h at room temperature, then was cooled to 0°C and equipped with a gas trap containing an aqueous saturated solution of sodium hypochlorite. A saturated aqueous solution of sodium hydrogen carbonate (500 mL) was added dropwise at 0°C in 1 h (formation of carbon dioxide). After concentration to dryness, the crude product was purified by silica gel chromatography (gradient of methanol in dichloromethane) to afford (2S,3R,4R,5S,6R)-2-ethynyl-6-(hydroxymethyl)tetrahydropyran-3,4,5-triol (4.05 g, 21.52 mmol) as a white solid. ¹H NMR (400 MHz, dmso-d6): δ 5.28 (d, 1H), 4.99 (d, 1H), 4.91 (d, 1H), 4.52 (t, 1H), 3.77 (d, 1H), 3.60-3.69 (m, 1H), 3.35-3.43 (m, 1H), 3.32 (s, 1H), 2.97-3.13 (m, 4H).

### Step 12: methyl (2S,3S,4R,5R,6S)-6-ethynyl-3,4,5-trihydroxy-tetrahydropyran-2-carboxylate

To a solution of (2S,3R,4R,5S,6R)-2-ethynyl-6-(hydroxymethyl)tetrahydropyran-3,4,5-triol (4.05 g, 21.52 mmol) in a saturated aqueous solution of sodium hydrogen carbonate (81 mL) and THF (81 mL) was added (2,2,6,6-tétraméthylpipéridin-1-yl)oxyl (168 mg, 1.08 mmol). The yellow suspension was cooled to 0°C and 1,3-dibromo-5,5-dimethyl-imidazolidine-2,4-dione (12.31 g, 43.04 mmol) was added portionwise in 30 min. The reaction mixture was stirred for 4 h at 0°C then was quenched with the addition of methanol (40 mL). After 30 min stirring at this temperature, a saturated aqueous solution of potassium carbonate (10 mL) and dichloromethane (100 mL) were added. The organic layer was extracted with water (2 x 200 mL) then the combined aqueous layers were acidified until pH = 1 with an aqueous solution of hydrogen chloride 3M and concentrated to dryness. The resulting residue was taken up in methanol (100 mL) and in an aqueous solution of hydrogen chloride 3M (20 mL). The mixture was concentrated to dryness and co-evaporated several times with methanol (4 x 100 mL). The crude product was purified by silica gel chromatography (gradient of methanol in dichloromethane Cerium developer) to afford methyl (2S,3S,4R,5R,6S)-6-ethynyl-3,4,5-trihydroxy-tetrahydropyran-2-carboxylate (3.00 g, 13.88 mmol) as a beige solid. ¹H NMR (400 MHz, dmso-d6): δ 5.46 (d, 1H), 5.32 (d, 1H), 5.18 (d, 1H), 3.93-4.00 (m, 1H), 3.75 (dd, 1H), 3.65 (s, 3H), 3.40-3.44 (m, 1H), 3.31 (s, 1H), 3.09-3.19 (m, 2H).

### Step 13: methyl (2S,3S,4R,5S,6S)-3,4,5-triacetoxy-6-ethynyl-tetrahydropyran-2-carboxylate

To a solution of methyl (2S,3S,4R,5R,6S)-6-ethynyl-3,4,5-trihydroxy-tetrahydropyran-2-carboxylate (3.00 g, 13.88 mmol) in DMF (37.5 mL) and pyridine (12.5 mL) was added N,N-dimethylpyridin-4-amine (84.8 mg, 0.693 mmol). The reaction mixture was cooled to 0°C then acetic anhydride (20.0 mL, 213 mmol) was added dropwise in 5 min. The colorless solution was stirred for 3 h at room temperature then was diluted with an aqueous solution of hydrogen chloride 1 M (200 mL). The aqueous layer was extracted with ethyl acetate (2 x 200 mL). The combined organic layers were washed with an aqueous solution of hydrogen chloride 1 M (2 x 200 mL), followed with a saturated aqueous solution of potassium carbonate (200 mL), then dried over sodium sulfate, filtered and concentrated to dryness to afford the crude mixture. The crude product was purified by silica gel chromatography (gradient of ethyl acetate in cyclohexane cerium developer) to afford methyl (2S,3S,4R,5S,6S)-3,4,5-triacetoxy-6-ethynyl-tetrahydropyran-2-carboxylate (4.60 g, 13.44 mmol) as a white solid. ¹H NMR (400 MHz, dmso-d6): δ 5.33 (t, 1H), 4.93-5.01 (m, 2H), 4.70 (d, 1H), 4.44 (d, 1H), 3.67 (s, 1H), 3.64 (s, 3H), 2.02 (s, 3H), 1.94-2.01 (m, 6H).

### Step 14: methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate

To a solution of methyl (2S,3S,4R,5S,6S)-3,4,5-triacetoxy-6-ethynyl-tetrahydropyran-2-carboxylate (496 mg, 1.45 mmol) in DMF (7.3 mL) were successively added 9H-fluoren-9-ylmethyl N-[(1S)-1-[[(1S)-2-[4-[[tert-butyl(dimethyl)silyl]oxymethyl]-3-iodo-anilino]-1-methyl-2-oxo-ethyl]carbamoyl]-2-methyl-propyl]carbamate (730 mg, 0.966 mmol), DIPEA (738 µL, 4.47 mmol), copper iodide (18.4 mg, 96.6 mmol) and dichloro-bis-(triphenylphosphine)palladium(II) (67.8 mg, 96.6 mmol). The yellow solution was flushed with Argon then was stirred for 16 h at room temperature. After dilution with water (100 mL), the aqueous layer was extracted with ethyl acetate (2 x 100 mL). The combined organic layers were washed with water (2 x 200 mL), and with a saturated aqueous solution of ammonium chloride (2 x 200 mL), then dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate (782 mg, 0.806 mmol) as a yellow solid. ¹H NMR (400 MHz, dmso-d6): δ 10.09 (s, 1H). 8.20 (d, 1H), 7.89 (d, 2H), 7.70-7.78 (m, 3H), 7.55 (d, 1H), 7.32-7.46 (m, 4H), 7.27-7.32 (m, 2H), 5.41 (t, 1H), 4.96-5.14 (m, 3H), 4.67 (s, 2H), 4.51 (d, 1H), 4.36-4.44 (m, 1H), 4.16-4.32 (m, 3H), 3.88-3.95 (m, 1H), 3.64 (s, 3H), 1.94-2.07 (m, 10H), 1.30 (d, 3H), 0.84-0.93 (m, 15H), 0.08 (s, 6H).

### Step 15: methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate

A solution of methyl (2S,3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethynyl]tetrahydropyran-2-carboxylate (750 mg, 0.773 mmol) in THF (15 mL) was flushed with Argon. Dry Platinum 5% on carbon (75 mg, 50% $\frac{w}{w}$) was added. The reaction mixture was successively flushed with argon, with H₂ and was stirred for 16 h at room temperature under H₂ atmosphere (P atm). The reaction mixture was filtered through a Celite^{®} pad, washed with THF then concentrated to dryness. The complete sequence, (addition of dry platinum 5% on carbon (75 mg, 50% $\frac{w}{w}$), stirring for 16 h at room temperature under H₂ atmosphere (1 bar) and filtration through a Celite^{®} pad), was performed 4 more times. The crude product was purified by silica gel chromatography (gradient of ethyl acetate in cyclohexane) to afford methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate (470 mg, 0.483 mmol) as a white solid. ¹H NMR (400 MHz, dmso-d6): δ 9.90 (s, 1H), 8.16 (d, 1H), 7.89 (d, 2H), 7.70-7.78 (m, 2H), 7.37-7.49 (m, 4H), 7.27-7.32 (m, 3H), 7.23 (d, 1H), 5.29 (t, 1H), 4.95 (t, 1H), 4.78 (t, 1H), 4.60 (s, 2H), 4.34-4.44 (m, 2H), 4.16-4.32 (m, 3H), 3.88-3.95 (m, 1H), 3.72-3.79 (m, 1H), 3.64 (s, 3H), 2.69-2.78 (m, 1H), 2.50-2.60 (m, 1H), 1.92-2.03 (m, 10H), 1.55-1.75 (m, 2H), 1.30 (d, 3H), 0.84-0.93 (m, 15H), 0.05 (s, 6H).

### Step 16: methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate

To a solution of methyl (3S,4R,5S,6S)-3,4,5-triacetoxy-6-[2-[2-[[tert-butyl(dimethyl)silyl]oxymethyl]-5-[[(2S)-2-[[(2S)-2-(9H-fluoren-9-ylmethoxycarbonylamino)-3-methyl-butanoyl]amino]propanoyl]amino]phenyl]ethyl]tetrahydropyran-2-carboxylate (470 mg, 0.483 mmol) in THF (540 µL) and water (540 µL) was added acetic acid (1.6 mL, 28.28 mmol). The colorless solution was stirred for 16 h at room temperature then diluted with water (100 mL). The aqueous layer was extracted with ethyl acetate (2 x 100 mL). The combined organic layers were washed with water (2 x 200 mL), and with a saturated aqueous solution of sodium hydrogen carbonate (200 mL), then were dried over sodium sulfate, filtered and concentrated to dryness. The crude product was purified by silica gel chromatography (gradient of ethyl acetate in cyclohexane) to afford methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate (354 mg, 0.412 mmol) as a white solid. ¹H NMR (400 MHz, dmso-d6): δ 9.87 (s, 1H), 8.16 (d, 1H), 7.89 (d, 2H), 7.70-7.78 (m, 2H), 7.37-7.50 (m, 4H), 7.27-7.37 (m, 3H), 7.25 (d, 1H), 5.29 (t, 1H), 4.91-4.98 (m, 2H), 4.78 (t, 1H), 4.34-4.44 (m, 4H), 4.16-4.32 (m, 3H), 3.88-3.95 (m, 1H), 3.72-3.79 (m, 1H), 3.64 (s, 3H), 2.64-2.73 (m, 1H), 2.50-2.60 (m, 1H), 1.92-2.03 (m, 10H), 1.69-1.79 (m, 1H), 1.52-1.65 (m, 1H), 1.30 (d, 3H), 0.84-0.93 (m, 6H).

### Step 17: methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate

To a solution of methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-(hydroxymethyl)phenyl]ethyl]tetrahydropyran-2-carboxylate (310 mg, 0.361 mmol) in THF (7.75 mL) were successively added pyridine (146 µL, 1.80 mmol) and 4-Nitrophenyl chloroformate (182 mg, 0.901 mmol). The white suspension was stirred for 16 h at room temperature then was concentrated to dryness to afford the crude mixture. The crude product was purified by silica gel chromatography (gradient of ethyl acetate in dichloromethane) to afford methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate (257 mg, 0.251 mmol) as a white solid. ¹H NMR (400 MHz, dmso-d6): δ 10.04 (s, 1H), 8.31 (d, 2H), 8.20 (d, 1H), 7.89 (d, 2H), 7.66-7.78 (m, 2H), 7.56 (d, 2H), 7.28-7.52 (m, 8H), 5.31 (t, 1H), 5.25 (s, 2H), 4.96 (t, 1H), 4.79 (t, 1H), 4.40 (d, 2H), 4.16-4.32 (m, 3H), 3.88-3.95 (m, 1H), 3.74-3.83 (m, 1H), 3.61 (s, 3H), 2.74-2.84 (m, 1H), 2.60-2.71 (m, 1H), 1.90-2.03 (m, 10H), 1.72-1.83 (m, 1H), 1.58-1.71 (m, 1H), 1.30 (d, 3H), 0.82-0.94 (m, 6H). LC-MS: MS (ESI) m/z [M+Na]+ = 1047.6.

### Step 18: (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2- (2-methoxyphenyl)pyrimidin-4-yl]methoxy]phenyl]propanoic acid

To a solution of (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **(C3)** (118 mg, 0.121 mmol) in dimethylformamide (3.0 mL) were successively added a solution of methyl (3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]phenyl]ethyl]tetrahydropyran-2-carboxylate (130 mg, 0.127 mmol) in dimethylformamide (3.0 mL) and DIPEA (60 µL, 0.363 mmol). The reaction mixture was stirred at room temperature for 2 h. (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid was obtained as a solution in dimethylformamide and was used like this in the next step. UPLC-MS: MS (ESI) m/z [M+H]+ = 1745.6+1747.6.

### Step 19: (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To the solution of 2SR,3SR,4RS,5RS,6SR)-6-[2-[(5Sₐ)-5-[[(2S)-2-[[(2S)-2-amino-3-methyl-butanoyl]amino]propanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic acid (0.121 mmol) in DMF (3.0 mL) from step 18 were successively added methanol (2 mL) and lithium hydroxide monohydrate (64.0 mg, 1.52 mmol) in solution in water (2 ml). The reaction mixture was stirred at room temperature for 1 h. The crude product was purified by C18 reverse phase prep-HPLC by direct deposit of the reaction mixture on the Xbridge column and using the NH₄HCO₃ method to afford (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (124 mg, 0.0895 mmol) as a white powder. UPLC-MS: MS (ESI) m/z [M+H]+ = 1384.3+1386.3.

### Step 20: (2,3,4,5,6-pentafluorophenyl) 2-[2-[2-(2-azidoethoxy)ethoxy]ethoxy]acetate 2-[2-[2-(2-azidoethoxy)ethoxy]ethoxy]acetic acid

To a solution of 2-[2-[2-(2-azidoethoxy)ethoxy]ethoxy]acetic acid (75 mg, 0.342 mmol) in solution in THF (500 µL) were added a solution of 2,3,4,5,6-pentafluorophenol (75.5 mg, 0.410 mmol) in THF (500 µL) and a solution of N,N'-dicyclohexylmethanediimine (84.7 mg, 0.410 mmol) in THF (500 µL). The reaction mixture was stirred for 15 h at room temperature and the progress of the reaction was followed by UPLC-MS. The (2,3,4,5,6-pentafluorophenyl) 2-[2-[2-(2-azidoethoxy)ethoxy]ethoxy]acetate was obtained as a THF solution by simple filtration of the suspension on a small disposable frit. This solution was used without further purification in the next step. UPLC-MS: MS (ESI) m/z [M-N2+H]+ = 372.3.

### Step 21: (2SR,3SR,4RS,5RS,6SR)-6-[2-[(5Sₐ)-5-[[(2S)-2-[[(2S)-2-[[2-[2-[2-(2-azidoethoxy)ethoxy]ethoxy]acetyl]amino]-3-methyl-butanoyl]amino]propanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]piperazine-1-carbonyl]oxymethyl]phenyl]ethyl]-3,4,5-trihydroxy-tetrahydropyran-2-carboxylic acid L14-C3

To the solution of (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-[2-[(2SR,3SR,4RS,5SR,6SR)-3,4,5-triacetoxy-6-methoxycarbonyl-tetrahydropyran-2-yl]ethyl]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (118 mg, 0.085 mmol) in DMF (500 µL) were successively added the solution of (2,3,4,5,6-pentafluorophenyl) 2-[2-[2-(2-azidoethoxy)ethoxy]ethoxy]acetate (0.342 mmol) in THF from step 20 and DIPEA (42.2µL, 0.256mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the NH₄HCO₃ method to afford **L14-C3** as a white powder. UPLC-MS: MS (ESI) m/z [M+H]+ = 1599.0+1601.2. IR Wavelength (cm⁻¹): 3263, 2105, 1652, 1600, 1284/1240/1089, 756. ¹H NMR(400 MHz, dmso-d6): δ 9.98 (s), 8.85 (d, 1H), 8.52 (s, 1H), 8.38 (d, 1H), 7.93 (d, 1H), 7.56 (d, 1H), 7.5 (t, 1H), 7.49 (d, 1H), 7.47 (d, 1H), 7.44 (d, 1H), 7.42 (s, 1H), 7.3 (dd, 2H), 7.22 (d, 1H), 7.2 (t, 2H), 7.19 (t, 1H), 7.13 (d, 1H), 7.08 (t, 1H), 7.02 (t, 1H), 6.95 (d, 1H), 6.65 (t, 1H), 6.11 (d, 1H), 5.43 (d, 1H), 5.27/5.2 (m, 2H), 4.93 (br s, 2H), 4.38 (m, 1H), 4.35/4.2 (2m, 2H), 4.3 (m, 1H), 3.94 (s, 2H), 3.75 (s, 3H), 3.58 (m, 10H), 3.57 (m, 1H), 3.51/2.29 (2dd, 2H), 3.35 (m, 2H), 3.25 (m, 4H), 3.2 (m, 1H), 3.2 (m, 1H), 3.06 (m, 1H), 2.96 (m, 1H), 2.75 (m, 2H), 2.72/2.5 (m, 2H), 2.41 (m, 4 H), 2 (m, 1H), 1.99/1.6 (m, 2H), 1.8 (s, 3H), 1.3 (d, 3H), 0.88/0.82 (2d, 6H). ¹³C NMR (100 MHz, dmso-d6): δ 158.2, 152.7, 131.9, 131.4, 131.4, 131.3, 131.1, 131, 127.8, 120.6, 120.5, 120.1, 116.8, 116.3, 116, 112.6, 112, 111.6, 79.6, 79.6, 78.5, 76.8, 74.2, 73.1, 70.3, 70.3, 69.3, 66.3, 65.1, 56.8, 56.3, 56.1, 52.6, 50.3, 49.4, 43.8, 34.2, 33.5, 31.7, 28, 19.6/18.4, 18.2, 18. ¹⁹F NMR (376 MHz, dmso-d6): δ - 112.5. HR-ESI+: m/z [M+H]+ = 1599.5724 (1599.5704) (measured/theoretical)

### Preparation of L18-C3:

### (2R)-2-[(5Sₐ)-5-[4-[2-[4-[[4-[[(2S)-2-[[(2S)-2-[[(2R)-2-[[2-(2-azidoethoxy)acetyl]amino]-3-sulfo-propanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

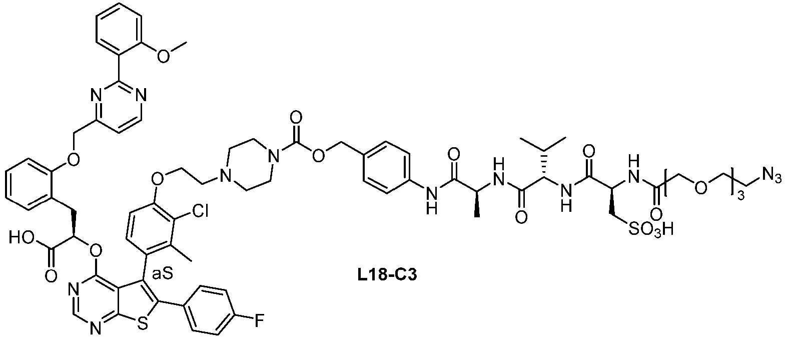

### Step 1: 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate

To a solution of (2S)-2-[[(2S)-2-(9H-fluoren-9-ylmethoxycarbonylamino)-3-methyl-butanoyl]amino]propanoic acid (6.0 g, 14.6 mmol; obtained according to Step 5 of the preparation of **L14-C3**) in dichloromethane (70 mL) and methanol (30 mL) were successively added (4-aminophenyl)methanol (2.16 g, 17.5 mmol) and ethyl 2-ethoxy-2H-quinoline-1-carboxylate (5.42 g, 21.93 mmol). The red solution was stirred at room temperature for 16 h (precipitation after few minutes). After completion of the reaction, the reaction mixture was diluted with diethyl ether (70 mL). The resulting precipitate was filtered off and dried to afford 9H-fluoren-9-ylmethyl N-[(1S)-1-[[(1S)-2-[4-(hydroxymethyl)anilino]-1-methyl-2-oxoethyl]carbamoyl]-2-methyl-propyl]carbamate (5.16 g, 10.01 mmol) as a beige solid. ¹H NMR (400 MHz, dmso-d6): δ 9.91 (s, 1H), 8.15 (d, 1H), 7.89 (d, 2H), 7.70-7.78 (m, 2H), 7.53 (d, 2H), 7.38-7.46 (m, 3H), 7.29-7.35 (m, 2H), 7.23 (d, 2H), 5.08 (t, 1H), 4.37-4.50 (m, 3H), 4.16-4.34 (m, 3H), 3.91 (t, 1H), 1.92-2.02 (m, 1H), 1.30 (d, 3H), 0.83-0.91 (m, 6H).

### Step 2: (2S)-2-amino-N-[(1S)-2-[4-(hydroxymethyl)anilino]-1-methyl-2-oxo-ethyl]-3-methylbutanamide

To a solution of 9H-fluoren-9-ylmethyl N-[(1S)-1-[[(1S)-2-[4-(hydroxymethyl)anilino]-1-methyl-2-oxo-ethyl]carbamoyl]-2-methyl-propyl]carbamate (5.16 g, 10.01 mmol) in DMF (120 mL) was added piperidine (52 mL, 525mmol). The reaction mixture was stirred for 2 h at room temperature then the piperidine was evaporated and the resulting solution was diluted with water (500 mL). The resulting solid was filtered off and the filtrate was washed twice with diethyl ether (2 x 500 mL). The aqueous layer was concentrated to dryness to afford the crude reaction mixture. The crude product was purified by silica gel chromatography (gradient of methanol (containing 7M ammonia) in dichloromethane) to afford (2S)-2-amino-N-[(1S)-2-[4-(hydroxymethyl)anilino]-1-methyl-2-oxo-ethyl]-3-methyl-butanamide (2.02 g, 6.89 mmol) as a beige solid. ¹H NMR (400 MHz, dmso-d6): δ 10.0 (s, 1H), 8.17 (s, 1H), 7.53 (d, 2H), 7.23 (d, 2H), 5.12 (t, 1H), 4.39-4.52 (m, 3H), 2.96-3.02 (m, 1H), 1.86-1.97 (m, 1H), 1.70 (br s, 2H), 1.29 (d, 3H), 0.88 (d, 3H), 0.78 (d, 3H).

### Step 3: [(2R)-2-(9H-fluoren-9-ylmethoxycarbonylamino)-3-oxo-3-sodiooxy-propyl]sulfonyloxysodium

To a solution of [(2R)-2-amino-3-oxo-3-sodiooxy-propyl]sulfonyloxysodium monohydrate (3.00 g, 12.98 mmol) in water (127 mL) was added sodium carbonate (4.13 g, 38.94 mmol). A solution of 9H-fluoren-9-ylmethyl carbonochloridate (3.69 g, 14.28 mmol) in dioxane (127 mL) was added dropwise in 15 min at room temperature. The mixture was stirred at this temperature for 4 h. After completion of the reaction, the mixture was neutralized to pH = 7 with an aqueous solution of HCl 1 M, diluted with a saturated aqueous solution of sodium hydrogenocarbonate (50 mL) and concentration to dryness. The crude product was purified by C18 reverse phase chromatography using the neutral method to afford [(2R)-2-(9H-fluoren-9-ylmethoxycarbonylamino)-3-oxo-3-sodiooxy-propyl]sulfonyloxysodium (4.4 g, 10.11 mmol) as a white solid. ¹H NMR (400 MHz, dmso-d6): δ 7.88 (d, 2H). 7.70 (d, 2H), 7.39-7.44 (m, 2H), 7.29-7.36 (m, 2H), 6.71 (s, 1H), 3.84-4.25 (m, 4H), 2.73-2.91 (m, 2H).

### Step 4:[(2R)-2-(9H-fluoren-9-ylmethoxycarbonylamino)-3-[[(1S)-1-[[(1S)-2-[4-(hydroxymethyl)anilino]-1-methyl-2-oxo-ethyl]carbamoyl]-2-methyl-propyl]amino]-3-oxo-propyl]sulfonyloxysodium

To a solution of (2S)-2-amino-N-[(1S)-2-[4-(hydroxymethyl)anilino]-1-methyl-2-oxoethyl]-3-methyl-butanamide (1.19 g, 4.04 mmol) in DMF (395 mL) were successively added [(2R)-2-(9H-fluoren-9-ylmethoxycarbonylamino)-3-oxo-3-sodiooxy-propyl]sulfonyloxysodium (4.40 g, 10.11 mmol), DIPEA (6.01 mL, 36.38 mmol) and HBTU (3.83 g, 10.11 mmol). The white suspension was stirred for 22 h at room temperature and then cooled to 0°C. Dilution with water (1.5 L), with a saturated solution of sodium carbonate (20 mL) and with solid sodium chloride, gave a white emulsion that was filtrated and the filtrate concentrated to dryness to afford the crude mixture. The crude product was purified by reverse phase C18 chromatography (gradient of methanol in water) to afford [(2R)-2-(9H-fluoren-9-ylmethoxycarbonylamino)-3-[[(1S)-1-[[(1S)-2-[4-(hydroxymethyl)anilino]-1-methyl-2-oxoethyl]carbamoyl]-2-methyl-propyl]amino]-3-oxo-propyl]sulfonyloxysodium (936 mg, 1.36 mmol) as a beige solid. ¹H NMR (400 MHz, dmso-d6): δ 9.39 (s, 1H). 8.25-8.31 (m, 1H), 8.11-8.17 (m, 1H), 7.89 (d, 2H), 7.70 (d, 2H), 7.64 (d, 2H), 7.50-7.55 (m, 1H), 7.38-7.46 (m, 2H), 7.29-7.35 (m, 2H), 7.20 (d, 2H), 5.07 (s, 1H), 4.51 (s, 1H), 4.42 (s, 2H), 4.19-4.33 (m, 4H), 4.01 (s, 1H), 2.90-3.10 (m, 2H), 2.08-2.20 (m, 1H), 1.31 (d, 3H), 0.8-0.93 (m, 6H).

### Step 5: (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxoethyl]carbamoyl]propyl]amino]-3-oxo-propane-1-sulfonate

To a suspension of [(2R)-2-(9H-fluoren-9-ylmethoxycarbonylamino)-3-[[(1S)-1-[[(1S)-2-[4-(hydroxymethyl)anilino]-1-methyl-2-oxo-ethyl]carbamoyl]-2-methyl-propyl]amino]-3-oxopropyl]sulfonyloxysodium (600 mg, 0.87 mmol) in THF (24 mL) were added DIPEA (432 µL, 2.61 mmol), followed by 4-Nitrophenyl chloroformate (439 mg, 2.17 mmol). The mixture was stirred at room temperature for 4 h. Additional 4-Nitrophenyl chloroformate (439 mg, 2.17 mmol) was added and the reaction mixture was stirred at room temperature for 16 h more. Additional 4-Nitrophenyl chloroformate (439 mg, 2.17 mmol) was added. After 5 h stirring at room temperature the mixture was concentrated to dryness and purified by silica gel chromatography (gradient of ethyl acetate in cyclohexane) and then by reverse phase C18 chromatography using the neutral method to afford (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxo-ethyl]carbamoyl]propyl]amino]-3-oxo-propane-1-sulfonate (303 mg, 0.32 mmol) as a white solid. ¹H NMR (400 MHz, dmso-d6): δ 9.52 (s, 1H), 8.25-8.37 (m, 3H), 8.06-8.24 (m, 4H), 7.89 (d, 2H), 7.76 (d, 2H), 7.70 (d, 2H), 7.49-7.61 (m, 3H), 7.35-7.45 (m, 4H), 7.26-7.35 (m, 2H), 5.23 (s, 2H), 4.48 (s, 1H), 4.20-4.33 (m, 4H), 4.01 (s, 1H), 3.57-3.66 (m, 2H), 3.10-3.18 (m, 2H), 2.90-3.10 (m, 2H), 2.08-2.20 (m, 1H), 1.33 (d, 3H), 1.21-1.26 (m, 15H), 0.86-0.92 (m, 6H). UPLC-MS: MS (ESI) m/z [M-H]-: 830.5.

### Step 6: (2R)-2-[(5Sₐ)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To a solution of (2R)-2-[(5Sₐ)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetic acid (C3) (128 mg, 0.149 mmol) in DMF (1.5mL) were successively added a solution of (2R)-2-(9H-fluoren-9-ylmethoxycarbonylamino)-3-[[(1S)-2-methyl-1-[[(1S)-1-methyl-2-[4-[(4-nitrophenoxy)carbonyloxymethyl]anilino]-2-oxo-ethyl]carbamoyl]propyl]amino]-3-oxo-propane-1-sulfonate (150 mg, 0.156 mmol) in DMF (1.5mL) and DIPEA (77 µl, 0.468 mmol). The reaction mixture was stirred for 2 h at room temperature and the progress of the reaction was followed by UPLC-MS. (2R)-2-[(5Sₐ)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid was obtained as a solution in dimethylformamide that was used like this in the next step. UPLC-MS: MS (ESI) m/z [M+H]+ = 1553.2+1555.3.

### Step 7: (2R)-2-[(5Sₐ)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To the solution of (2R)-2-[(5Sₐ)-5-[3-chloro-4-[2-[4-[[4-[[(2S)-2-[[(2S)-2-[[(2S)-2-(9H-fluoren-9-ylmethoxycarbonylamino)butanoyl]amino]-3-methyl-butanoyl]amino]propanoyl]amino]phenyl]methoxycarbonyl]piperazin-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (0.156 mmol) in dimethylformamide (3 mL) obtained in the previous step was added piperidine (30.6 µL, 0.312 mmol). The reaction mixture was stirred at room temperature for 15 h and the progress of the reaction was followed by UPLC-MS. The crude product was purified by C18 reverse phase prep-HPLC by direct deposit of the reaction mixture on the Xbridge column and using the NH₄HCO₃ method to afford (2R)-2-[(5Sₐ)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (148 mg = 0.111 mmol) as a white powder. UPLC-MS: MS (ESI) m/z [M+H]+ = 1331.4+1333.5.

### Step 8: (2R)-2-[(5Sₐ)-5-[4-[2-[4-[[4-[[(2S)-2-[[(2S)-2-[[(2S)-2-[[2-[2-[2-(2-azidoethoxy)ethoxy]ethoxy]acetyl]amino]butanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetic acid L18-C3

To the solution of (2R)-2-[(5Sₐ)-5-[4-[2-[4-[[4-[[(2S)-2-[[(2S)-2-[[(2S)-2-aminobutanoyl]amino]-3-methyl-butanoyl]amino]propanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]proanoic acid (148 mg, 0.111 mmol) in DMF (1.5 mL) were successively added the solution of (2,3,4,5,6-pentafluorophenyl) 2-[2-[2-(2-azidoethoxy)ethoxy]ethoxy]acetate (0.596 mmol; obtained according to Step 20 of the preparation of **L14-C3**) in THF (1 mL) and DIPEA (74 µL, 0.447 mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the NH₄HCO₃ method to afford **L18-C3** (60 mg, 0.0389mmol) as a white powder. IR Wavelength (cm⁻¹): 3288, 2101, 1659, 1237,1039, 833,755. ¹H NMR(400 MHz, dmso-d6): δ (m, 10H), 9.42 (s, 1H), 8.88 (d, 1H), 8.58 (s, 1H), 8.32 (d, 1H), 8.18 (d, 1H), 8.12 (d, 1H), 7.71 (m, 1H), 7.7 (d, 2H), 7.54 (dd, 1H), 7.46 (td, 1H), 7.39 (d, 1H), 7.29 (dd, 2H), 7.25 (d, 2H), 7.21 (t, 2H), 7.18 (d, 1H), 7.15 (d, 1H), 7.13 (t, 1H), 7.04 (t, 1H), 6.99 (d, 1H), 6.71 (t, 1H), 6.22 (d, 1H), 5.47 (m, 1H), 5.23 (AB, 2H), 4.98 (s, 2H), 4.71 (q, 1H), 4.3 (m, 1H), 4.24/4.19 (2m, 2H), 3.97 (dd, 1H), 3.92 (m, 2H), 3.76 (s, 3 H), 3.37 (t, 2H), 3.31 (m, 4H), 3.12/2.97 (2dd, 2H), 2.74 (t, 2H), 2.45 (m, 4H), 2.15 (m, 1H), 1.81 (s, 3H), 1.33 (d, 3H), 0.91 (2d, 6H). ¹³C NMR (100 MHz, dmso-d6): δ 157.9, 152.3, 131.3, 131.2, 131.1, 131, 130.7, 128.9, 128.6, 120.7, 120.4, 119.6, 116.4, 112.7, 112, 111.3, 70.6, 70.3, 69.4, 67.5, 66.3, 59.7, 56.7, 56.1, 53.4, 52.5, 50.8, 50.4, 49.9, 44, 29.9, 19.6, 17.8, 17.6. ¹⁹F NMR (376 MHz, dmso-d6): δ ppm 112.3. HR-ESI+: m/z [M+H]+ = 1546.503 (1546.5009) (measured/theoretical).

### Preparation of L16-C3:

### (2R)-2-[(5Sₐ)-5-[4-[2-[4-[[4-[[(2S)-6-amino-2-[[(2S)-2-[[2-(2-azidoethoxy)acetyl]amino]-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

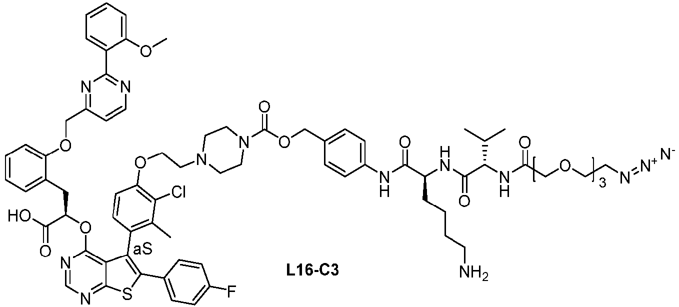

### Step 1: (2S)-6-(tert-butoxycarbonylamino)-2-[[2-(9H-fluoren-9-ylmethoxycarbonylamino)-3-methyl-butanoyl]amino]hexanoic acid

To a solution of (2S)-2-amino-6-(tert-butoxycarbonylamino)hexanoic acid (2.96 g, 12 mmol) and sodium hydrogene carbonate (1.01 g, 12 mmol) in water (30 mL) was added a solution of (2,5-dioxopyrrolidin-1-yl) 2-(9H-fluoren-9-ylmethoxycarbonylamino)-3-methylbutanoate (5.0 g, 11.5 mmol) in dimethoxyethane (30 mL), THF (15 mL) was added to improve the solubility. The reaction mixture was stirred at room temperature for 16 h. An aqueous solution of hydrochloric acid 1 M (15 mL) was added and the aqueous layer and was extracted with ethyl acetate (3 x 75 mL). The combined organic layers were dried over sodium sulfate, filtered and concentrated to dryness to afford the crude compound. Trituration in dichloromethane/pentane with sonication led to (2S)-6-(tert-butoxycarbonylamino)-2-[[2-(9H-fluoren-9-ylmethoxycarbonylamino)-3-methyl-butanoyl]amino]hexanoic acid (4.9 g, 8.63 mmol) as a white solid. ¹H NMR (400 MHz, dmso-d6): δ 12.48 (s, 1H), 7.89 (d, 2H), 7.74 (t, 2H), 7.28-7.44 (m, 6H), 6.73 (s, 1H), 4.10-4.33 (m, 5H), 3.9 (t, 1H), 2.82-2.90 (m, 2H), 1.52-1.73 (m, 2H), 1.34 (s, 9H), 1.22-1.31 (m, 4H), 0.83-0.91 (m, 6H).

### Step 2: 9H-fluoren-9-ylmethyl N-[1-[[(1S)-5-(tert-butoxycarbonylamino)-1-[[4-(hydroxymethyl)phenyl]carbamoyl]pentyl]carbamoyl]-2-methyl-propyl]carbamate

To a solution of (2S)-6-(tert-butoxycarbonylamino)-2-[[2-(9H-fluoren-9-ylmethoxycarbonylamino)-3-methyl-butanoyl]amino]hexanoic acid (1.5 g, 2.64 mmol) in dichloromethane (19 mL) and methanol (9.5 mL) was added (4-aminophenyl)methanol (651.0 mg, 5.28 mmol) in methanol (1.5 mL). Ethyl 2-ethoxy-2H-quinoline-1-carboxylate (1.31 g, 5.28 mmol) was then added. The reaction mixture was stirred at room temperature for 16 h then concentrated to dryness. The crude product was purified by silica gel chromatography (gradient of methanol in dichloromethane) to afford 9H-fluoren-9-ylmethyl N-[1-[[(1S)-5-(tert-butoxycarbonylamino)-1-[[4-(hydroxymethyl)phenyl]carbamoyl]pentyl]carbamoyl]-2-methyl-propyl]carbamate (544 mg, 0.80 mmol) as a pale red solid. ¹H NMR (400 MHz, dmso-d6): δ 9.93 (s, 1H). 8.01 (d, 1H), 7.89 (d, 2H), 7.74 (t, 2H), 7.52 (d, 2H), 7.37-7.45 (m, 3H), 7.32 (t, 2H), 7.22 (d, 2H), 6.71 (s, 1H), 5.08 (br s, 1H), 4.43 (d, 2H), 4.21-4.40 (m, 4H), 3.92 (t, 1H), 2.83-2.91 (m, 2H), 1.94-2.01 (m, 1H), 1.55-1.74 (m, 2H), 1.21-1.42 (m, 4H), 1.33 (s, 9H), 0.87 (t, 6H).

### Step 3: [4-[[(2S)-6-(tert-butoxycarbonylamino)-2-[[2-(9H-fluoren-9-ylmethoxycarbonylamino)-3-methyl-butanoyl]amino]hexanoyl]amino]phenyl]methyl (4-nitrophenyl) carbonate

To a solution of 9H-fluoren-9-ylmethyl N-[1-[[(1S)-5-(tert-butoxycarbonylamino)-1-[[4-(hydroxymethyl)phenyl]carbamoyl]pentyl]carbamoyl]-2-methyl-propyl]carbamate (600.0 mg, 0.892 mmol) in THF (19 mL), were added pyridine (361 µL, 4.46 mmol) then 4-Nitrophenyl chloroformate (448 mg, 2.22 mmol). The mixture was stirred at room temperature for 16 h then concentrated to dryness. The crude product was purified by silica gel chromatography (gradient of ethyl acetate in cyclohexane) to afford [4-[[(2S)-6-(tert-butoxycarbonylamino)-2-[[2-(9H-fluoren-9-ylmethoxycarbonylamino)-3-methyl-butanoyl]amino]hexanoyl]amino]phenyl]methyl (4-nitrophenyl) carbonate (524 mg; 0.62 mmol; 70%) as a pale pink solid. ¹H NMR (400 MHz, dmso-d6): δ 10.13 (s, 1H), 8.31 (d, 2H), 8.1 (d, 1H), 7.89 (d, 2H), 7.74 (t, 2H), 7.63 (d, 2H), 7.57 (d, 2H), 7.28-7.45 (m, 7H), 6.72 (s, 1H), 5.24 (s, 2H), 4.35-4.42 (m, 1H), 4.27-4.33 (m, 1H), 4.22 (s, 2H), 3.92 (t, 1H), 2.83-2.91 (m, 2H), 1.96-2.00 (m, 1H), 1.58-1.73 (m, 2H), 1.20-1.30 (m, 4H), 1.33 (s, 9H), 0.86 (t, 6H). ¹³C NMR (100 MHz, dmso-d6): δ 171.22, 170.67, 156.1, 155.5, 155.27, 151.92, 145.15, 143.87, 143.75, 140.68, 139.34, 129.43, 129.31, 127.6, 127.03, 125.38, 125.32, 122.58, 120.07, 119.11, 77.28, 70.23, 65.67, 60.11, 54.89, 53.43, 46.67, 31.69, 30.39, 29.22, 28.23, 22.74, 19.19, 18.26. LC-MS: MS (ESI) m/z [M+Na]+ = 837.4.

### Step 4: (2R)-2-[(5Sₐ)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To a solution of (2R)-2-[(5Sₐ)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid C3 (166.3 mg, 0.170 mmol) in DMF (1.5 mL) were successively added a solution of [4-[[(2S)-6-(tert-butoxycarbonylamino)-2-[[2-(9H-fluoren-9-ylmethoxycarbonylamino)-3-methyl-butanoyl]amino]hexanoyl]amino]phenyl]methyl (4-nitrophenyl) carbonate (150 mg, 0.179 mmol) in DMF (1.5 mL) and DIPEA (85 µl, 0.510 mmol). The reaction mixture was stirred for 1 h at room temperature and the progress of the reaction was followed by UPLC-MS. The crude product was purified by C18 reverse phase prep-HPLC by direct deposit of the reaction mixture on the Xbridge column and using the NH₄HCO₃ method to afford (2R)-2-[(5Sₐ)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (134mg, 0.0859mmol) as a white powder. UPLC-MS: MS (ESI) m/z [M+H]+ = 1559.1+1561.3, [M+Na]+: 1581.0+1583.2. IR Wavelength (cm⁻¹): 3309, 1698, 1238, 1162, 757, 744. ¹H NMR (400 MHz, dmso-d6): δ 10.05 (s, 1H), 8.87 (d, 1H), 8.6 (m, 1H), 8.06 (d, 1H), 7.88 (d, 2H), 7.74 (2d, 2H), 7.64 (m, 1H), 7.57 (d, 2H), 7.52 (dd, 1H), 7.44 (t, 1H), 7.43 (d, 1H), 7.4 (t, 2H), 7.35 (d, 1H), 7.3 (t, 2H), 7.3 (dd, 2H), 7.26 (d, 2H), 7.2 (t, 2H), 7.18 (d, 1H), 7.14 (d, 1H), 7.12 (t, 1H), 7.03 (t, 1H), 6.99 (d, 1H), 6.72 (t, 1H), 6.71 (m, 1H), 6.24 (d, 1H), 5.49 (dd, 1H), 5.23 (m, 2H), 4.97 (s, 2H), 4.38 (m, 1H), 4.29/4.23 (m, 2H), 4.22 (m, 1H), 4.2 (m, 2H), 3.92 (dd, 1H), 3.74 (s, 3H), 3.29 (m, 4H), 3.29/2.5 (2dd, 2H), 2.87 (m, 2H), 2.74 (t, 2H), 2.45 (m, 4H), 1.99 (m, 1H), 1.82 (s, 3 H), 1.68/1.6 (2m, 2H), 1.36/1.28 (2m, 4H), 1.32 (s, 9H), 0.86 (2d, 6H). ¹³C NMR (100 MHz, dmso-d6): δ 158, 131.4, 131.2, 131.2, 131, 130.8, 128.9, 128.5, 127.9, 127.5, 125.6, 120.8, 120.5, 120.4, 119.3, 116, 115.9, 112.4, 112.2, 111.2, 74.1, 69.2, 67.9, 66.7, 66.2, 60.6, 56.6, 56.2, 53.8, 53, 47.1, 43.7, 40, 32.6, 32.2, 30.6, 29.8/23.2, 28.5, 18.8, 18.1. ¹⁹F NMR (376 MHz, dmso-d6): δ -112.

### Step 5: (2R)-2-[(5Sₐ)-5-[4-[2-[4-[[4-[[(2S)-2-[[(2S)-2-amino-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To the solution of (2R)-2-[(5Sₐ)-5-[4-[2-[4-[[4-[[(2S)-6-(tert-butoxycarbonylamino)-2-[[(2S)-2-(9H-fluoren-9-ylmethoxycarbonylamino)-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (134 mg, 0.0859 mmol) in dimethylformamide (3 mL) was added piperidine (17 µL, 0.172 mmol). The reaction mixture was stirred at room temperature for 18 h and the progress of the reaction was followed by UPLC-MS. The crude product was purified by C18 reverse phase prep-HPLC by direct deposit of the reaction mixture on the Xbridge column and using the NH₄HCO₃ method to afford (2R)-2-[(5Sₐ)-5-[4-[2-[4-[[4-[[(2S)-2-[[(2S)-2-amino-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (88 mg, 0.0658 mmol) as a white powder. UPLC-MS: MS (ESI) m/z [M+H]+ = 1337.4+1339.4, [M+Na]+ = 1359.4+1361.4. IR Wavelength (cm⁻¹): 3307, 1683, 1290, 1238, 1162, 835, 754. ¹H NMR (400 MHz, dmso-d6) δ ppm 10.23 (s, 1H), 8.88 (d, 1H), 8.53 (m, 1H), 8.47 (br, 1H), 7.86 (d, 1H), 7.58 (d, 2H), 7.54 (d, 1H), 7.48 (d, 1H), 7.45 (t, 1H), 7.27 (dd, 2H), 7.25 (d, 2H), 7.19 (t, 2H), 7.18 (d, 1H), 7.14 (d, 1H), 7.08 (t, 1H), 7.03 (t, 1H), 6.96 (t, 1H), 6.72 (t, 1H), 6.67 (t, 1H), 6.14 (d, 1H), 5.42 (d, 1H), 5.21 (m, 2H), 4.97 (s, 2H), 4.4 (m, 1H), 4.21 (m, 2H), 3.75 (s, 3H), 3.42/2.35 (m, 2H), 3.29 (m, 4H), 3.24 (m, 1H), 2.87 (q, 2H), 2.72 (t, 2H), 2.43 (m, 4H), 1.99 (m, 1H), 1.78 (s, 3H), 1.7/1.61 (2m, 2H), 1.36 (m, 2H), 1.34 (s, 9H), 1.26 (m, 2H), 0.89/0.82 (2d, 6H). ¹³C NMR (100 MHz, dmso-d6): δ ppm 158.4, 131.3, 131.2, 131.1, 131, 128.4, 128, 120.8, 120.6, 120.4, 119.7, 116.1, 115.9, 112.7, 111.7, 111.2, 76.2, 69.2, 67.4, 66.3, 59.2, 56.6, 56.3, 53.6, 53.1, 43.8, 40.1, 33.2, 32.4, 31.3, 29.3, 28.8, 22.9, 19.7/17.5, 17.9. ¹⁹F NMR (376 MHz, dmso-d6): δ ppm -112.4.

### Step 6: (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To a solution of (2R)-2-[(5Sₐ)-5-[4-[2-[4-[[4-[[(2S)-2-[[(2S)-2-amino-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (82 mg, 0.0613 mmol) in DMF (500 µL) were successively added the solution of (2,3,4,5,6-pentafluorophenyl) 2-[2-[2-(2-azidoethoxy)ethoxy]ethoxy]acetate (0.245 mmol; obtained according to Step 20 of the preparation of **L14-C3)** in THF and DIPEA (30.4 µL, 0.184 mmol). The reaction mixture was stirred for 1h at room temperature and the progress of the reaction was followed by UPLC-MS. The crude product was purified by C18 reverse phase prep-HPLC by direct deposit of the reaction mixture on the Xbridge column and using the NH₄HCO₃ method to afford (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (60 mg, 0.0386 mmol) as a white powder. UPLC-MS: MS (ESI) m/z [M+H]+ = 1552.2+1554.2, [M+Na]+ = 1574.1+1576.3.

### Step 7: (2R)-2-[(5Sₐ)-5-[4-[2-[4-[[4-[[(2S)-6-amino-2-[[(2S)-2-[[2-[2-[2-(2-azidoethoxy)ethoxy]ethoxy]acetyl]amino]-3-methyl-butanoyl]amino]hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid L16-C3

To a solution of (2R)-2-[(5Sₐ)-5-[4-[2-[4-[[4-[[(2S)-2-[[(2S)-2-[[2-[2-[2-(2-azidoethoxy)ethoxy]ethoxy]acetyl]amino]-3-methyl-butanoyl]amino]-6-(tert-butoxycarbonylamino)hexanoyl]amino]phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (23 mg, 0.0148 mmol) in dichloromethane (3 mL) was added 2,2,2-trifluoroacetic acid (400 µl, 4.57 mmol). The reaction mixture was stirred for 2h at room temperature and the progress of the reaction was followed by UPLC-MS. The crude product was purified by C18 reverse phase prep-HPLC by direct deposit of the reaction mixture on the Xbridge column and using the NH₄HCO₃ method to afford L16-C3 (5 mg, 0.00344 mmol) as a white powder. UPLC-MS: MS (ESI) m/z [M+H]+ = 1552.4+1554.5, [M+Na]+ = 1574.4+1576.4. IR Wavelength (cm⁻¹): 3250, 2250-3500, 2102, 1660, 1288, 1238, 1121, 833, 755. ¹H NMR(400 MHz, dmso-d6): δ ppm 10.24 (s, 1H), 8.85 (d, 1H), 8.49 (s, 1H), 8.49 (d, 1H), 7.98 (d, 1H), 7.6 (d, 2H), 7.55 (d, 1H), 7.51 (d, 1H), 7.5 (d, 1H), 7.46 (t, 1H), 7.26 (d, 2H), 7.25 (dd, 2H), 7.18 (t, 2H), 7.17 (d, 1H), 7.14 (d, 1H), 7.05 (t, 1H), 7.02 (t, 1H), 6.89 (d, 1H), 6.6 (t, 1H), 6.05 (d, 1H), 5.32 (d, 1H), 5.21/5.15 (m, 2H), 5.02/4.96 (m, 2H), 4.36 (q, 1H), 4.31 (dd, 1H), 4.2 (m, 2H), 3.94 (s, 2H), 3.77 (s, 3 H), 3.58 (m, 10 H), 3.46/2.28 (d+t, 2H), 3.34 (t, 2H), 3.29 (m, 4 H), 2.8 (m, 2H), 2.67 (t, 2H), 2.43 (m, 4 H), 2.01 (m, 1H), 1.75 (s, 3 H), 1.69/1.6 (2m, 2H), 1.51 (m, 2H), 1.31 (m, 2H), 0.86/0.8 (2d, 6 H). ¹³C NMR (100 MHz, dmso-d6): δ ppm 157.9, 153.7, 131.4, 131.4, 131.3, 131.1, 130.9, 129.3, 127.6, 120.9, 120.4, 119.8, 116.2, 116.1, 112.6, 111.8, 111.8, 78.1, 70.5, 70.4, 69.3, 66.6, 66.6, 56.9, 56.5, 56.3, 54, 52.3, 50.4, 44, 39, 33.8, 32.2, 31.8, 28.2, 23.1, 19.7/18.1, 18.3. ¹⁹F NMR (376 MHz, dmso-d6): δ ppm -112.6. HR-ESI+: m/z [M+H]+ = 1452.5661 (1452.5648) (measured/theoretical).

### Preparation of L21-C1:

### (2S,3S,4R,5R,6S)-6-[2-[2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]-5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]ethyl]-3,4,5-trihydroxy-tetrahydropyran-2-carboxylic acid;2,2,2-trifluoroacetate

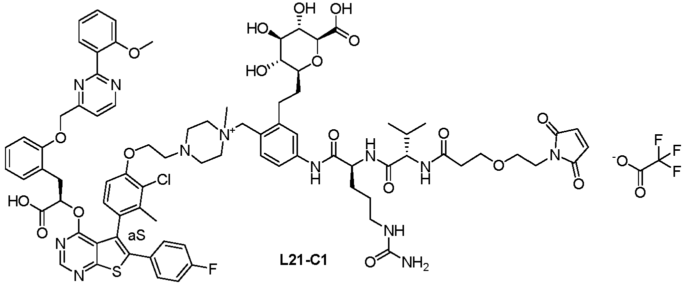

### Step 1: tert-butyl-[(2-iodo-4-nitro-phenyl)methoxy]-dimethyl-silane

To a solution of (2-iodo-4-nitro-phenyl)methanol (172 g, 61.64 mmol; obtained according to Step 2 of the preparation of **L14-C3)** in dichloromethane (300 mL) was added imidazole (5.04 g, 73.97 mmol). The mixture was cooled to 0°C, then a solution of tert-butyl-chloro-dimethyl-silane (11.15 g, 73.97 mmol) in dichloromethane (300 mL) was added dropwise in 15 min. The ice bath was removed and the reaction mixture was stirred at room temperature for 16 h. After completion of the reaction, the reaction mixture was quenched with methanol (20 mL) and concentrated to dryness. The crude product was purified by silica gel chromatography (gradient of ethyl acetate in cyclohexane) to afford tert-butyl-[(2-iodo-4-nitro-phenyl)methoxy]-dimethyl-silane (19.65 g, 49.96 mmol) as a white solid. ¹H NMR (400 MHz, dmso-d6): δ 8.57 (s, 1H), 8.31 (d, 1H), 7.66 (d, 1H), 4.67 (s, 2H), 0.92 (s, 9H), 0.14 (s, 6H).

### Step 2: methyl (2S,3S,4R,5S, 6S)-3,4,5-triacetoxy-6-[2-[2-[[tert-butyl(dimethyl)silyl]oxymethyl]-5-nitro-phenyl]ethynyl]tetrahydropyran-2-carboxylate

To a solution of tert-butyl-[(2-iodo-4-nitro-phenyl)methoxy]-dimethyl-silane compound (3.0 g, 7.63 mmol) in DMF (55 mL) were successively added methyl (2S,3S,4R,5S,6S)-3,4,5-triacetoxy-6-ethynyl-tetrahydropyran-2-carboxylate (3.39 g, 9.92 mmol; obtained according to Step 13 of the preparation of **L14-C3),** DIPEA (5.80 mL, 35.09 mmol), copper iodide (145 mg, 0.763 mmol) and dichloro-bis-(triphenylphosphine)palladium(ll) (535 mg, 0.763 mmol). The yellow solution was flushed with Argon and stirred for 16 h at room temperature. After dilution with water (300 mL), the aqueous layer was extracted with ethyl acetate (2 x 300 mL). The combined organic layers were washed with water (2 x 300 mL) then were dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-[[tert-butyl(dimethyl)silyl]oxymethyl]-5-nitrophenyl]ethynyl]tetrahydropyran-2-carboxylate (4.01 g, 6.60 mmol) as a beige solid. ¹H NMR (400 MHz, dmso-d6): δ 8.32 (dd, 1H), 8.19 (d, 1H), 7.75 (d, 1H), 5.45 (t, 1H), 5.16 (t, 1H), 5.02-5.07 (m, 2H), 4.82 (s, 2H), 4.55 (d, 1H), 3.65 (s, 3H), 1.98-2.07 (m, 9H), 0.92 (m,9H), 0.14 (s, 6H).

### Step 3: methyl (2S,3S,4R,5S,6S)-3,4,5-triacetoxy-6-[2-[2-(hydroxymethyl)-5-nitrophenyl]ethynyl]tetrahydropyran-2-carboxylate

To a solution of methyl (2S,3S,4R,5S,6S)-3,4,5-triacetoxy-6-[2-[2-[[tert-butyl(dimethyl)silyl]oxymethyl]-5-nitro-phenyl]ethynyl]tetrahydropyran-2-carboxylate (4.01 g, 6.60 mmol) in THF (48 mL) and water (48 mL) was added acetic acid (193 mL, 3.36 mol). The colorless solution was stirred for 2 days at room temperature then diluted with water (300 mL). The aqueous layer was extracted with dichloromethane (2 x 300 mL). The combined organic layers were washed with water (2 x 300 mL), and with a saturated aqueous solution of sodium hydrogen carbonate (400 mL), then dried over sodium sulfate, filtered and concentrated to dryness. The crude product was purified by silica gel chromatography (gradient of ethyl acetate in cyclohexane) to afford methyl (2S,3S,4R,5S,6S)-3,4,5-triacetoxy-6-[2-[2-(hydroxymethyl)-5-nitrophenyl]ethynyl]tetrahydropyran-2-carboxylate (2.67 g, 5.41 mmol) as a white solid. ¹H NMR (400 MHz, dmso-d6): δ 8.29 (dd, 1H), 8.15 (d, 1H), 7.79 (d, 1H), 5.68 (t, 1H), 5.45 (t, 1H), 5.16 (t, 1H), 5.02-5.07 (m, 2H), 4.62 (d, 2H), 4.55 (d, 1H), 3.65 (s, 3H), 1.98-2.07 (m, 9H).

### Step 4: methyl (2S,3S,4R,5S,6S)-3,4,5-triacetoxy-6-[2-[5-amino-2-(hydroxymethyl)phenyl]ethyl]tetrahydropyran-2-carboxylate

A solution of methyl (2S,3S,4R,5S,6S)-3,4,5-triacetoxy-6-[2-[2-(hydroxymethyl)-5-nitro-phenyl]ethynyl]tetrahydropyran-2-carboxylate (2.67 g, 5.41 mmol) in THF (59 mL) was flushed with Argon. Platinum on carbon 5% dry (1.34 g, 50% w/w) was added. The reaction mixture was successively flushed with argon, with H₂ and was stirred for 2 days at room temperature under H₂ atmosphere (P atm). The reaction mixture was filtered through a Celite^{®} pad, washed with a solution of ethyl acetate/methanol 9/1 (500 mL), then concentrated to dryness. All the sequence, (addition of platinum on carbon 5% dry (1.34 g, 50% w/w), stirring for 16 h at room temperature under H₂ (P atm) and filtration through a Celite^{®} pad), was repeated to allow a complete conversion. The crude product was purified by silica gel chromatography (gradient of ethyl acetate in cyclohexane) to afford methyl (2S,3S,4R,5S,6S)-3,4,5-triacetoxy-6-[2-[5-amino-2-(hydroxymethyl)phenyl]ethyl]tetrahydropyran-2-carboxylate (1.12 g, 2.40 mmol) as a white solid. ¹H NMR (400 MHz, dmso-d6): δ 6.93 (d, 1H). 6.67-6.33 (m, 2H), 5.30 (t, 1H), 4.96 (t, 1H), 4.88 (s, 2H), 4.81 (t, 1H), 4.61 (t, 1H), 4.39 (d, 1H), 4.29-4.24 (m, 2H), 3.78-3.72 (m, 1H), 3.65 (s, 3H), 2.65-2.54 (m, 2H), 2.07-1.98 (m, 9H), 1.79-1.68 (m, 1H), 1.63-1.52 (m, 1H).

### Step 5: methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate

To a solution of methyl (2S,3S,4R,5S,6S)-3,4,5-triacetoxy-6-[2-[5-amino-2-(hydroxymethyl)phenyl]ethyl]tetrahydropyran-2-carboxylate (1.00 g, 2.14 mmol) in DMF (21 mL) were successively added (2S)-2-(tert-butoxycarbonylamino)-5-ureido-pentanoic acid (589 mg, 2.14 mmol), DIPEA (707 µl, 4.28 mmol) and HBTU (1.22 g, 3.21 mmol). The reaction mixture was stirred for 72 hours at room temperature. After completion of the reaction, the mixture was diluted with water (100 mL) and was concentrated to dryness. The crude product was purified by silica gel chromatography (gradient of methanol in dichloromethane) to afford methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate (1.05 g, 1.45 mmol) as a beige solid. ¹H NMR (400 MHz, dmso-d6): δ 9.82 (s, 1H), 7.35-7.42 (m, 2H), 7.24 (d, 1H), 6.95 (d, 1H), 5.94 (t, 1H), 5.37 (s, 2H), 5.30 (t, 1H), 4.91-4.99 (m, 2H), 4.79 (t, 1H), 4.36-4.42 (m, 3H), 4.01-4.08 (m, 1H), 3.76 (t, 1H), 3.65 (s, 3H), 2.95-3.04 (m, 2H), 2.54-2.65 (m, 2H), 1.98-2.07 (m, 9H), 1.68-1.79 (m, 1H), 1.49-1.63 (m, 3H), 1.30-1.42 (m, 11H).

### Step 6: methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate

To a solution of compound methyl (2S,3S,4R,5S,6S)-3,4,5-triacetoxy-6-[2-[5-[[(2S)-2-(tert-butoxycarbonylamino)-5-ureido-pentanoyl]amino]-2-(hydroxymethyl)phenyl]ethyl]tetrahydropyran-2-carboxylate (950 mg, 1.31 mmol) in dichloromethane (7.5 mL) was added, at 0°C, trifluoroacetic acid (1.9 mL, 25.6 mmol). The reaction mixture was stirred at room temperature for 3 h. After completion of the reaction, the reaction mixture was concentrated to dryness and was coevaporated with toluene (2 x 50 mL) to afford the crude compound.

To this crude in solution in DMF (13 mL) were successively added (2S)-2-(9H-fluoren-9-ylmethoxycarbonylamino)-3-methyl-butanoic acid (467 mg, 1.38 mmol), DIPEA (867 µl, 5.24 mmol) and HBTU (845 mg, 2.23 mmol). The reaction mixture was stirred for 16 h at room temperature. After completion of the reaction, a saturated aqueous solution of hydrogenocarbonate (20 mL) was added, the mixture was stirred at room temperature for 1 h, was diluted with water (100 mL) and was concentrated to dryness. The crude product was purified by silica gel chromatography (gradient of methanol in dichloromethane) and then by reverse phase C18 chromatography using the neutral method to afford methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate (680 mg, 0.720 mmol) as a white solid. LC-MS: MS (ESI) m/z [M+H]+ = 946.3. ¹H NMR (400 MHz, dmso-d6): δ 9.90 (s, 1H). 8.07 (d, 2H), 7.89 (d, 2H), 7.74 (t, 2H), 7.44-7.38 (m, 3H), 7.36-7.28 (m, 3H), 7.24 (d, 1H), 5.94 (t, 1H), 5.37 (s, 2H), 5.30 (t, 1H), 4.99-4.92 (m, 2H), 4.79 (t, 1H), 4.42-4.36 (m, 4H), 4.32-4.19 (m, 3H), 3.94-3.90 (m, 1H), 3.76 (t, 1H), 3.65 (s, 3H), 2.99-2.94 (m, 2H), 2.65-2.54 (m, 2H), 2.07-1.98 (m, 10H), 1.70-1.55 (m, 4H), 1.46-1.36 (m, 2H), 0.89-0.84 (m, 6H). ¹³C NMR (100 MHz, dmso-d6): δ 171.19, 170.33, 169.58, 169.45, 169.27, 167.77, 158.81, 156.12, 143.89, 143.76, 140.69, 139.48, 137.54, 134.88, 128.44, 127.62, 127.06, 125.35, 120.08, 119.42, 116.65, 75.78, 74.61, 72.65, 71.20, 69.49, 65.68, 60.49, 60.10, 53.14, 52.40, 46.68, 32.32, 30.43, 29.54, 27.19, 26.77, 20.39, 20.34, 20.24, 19.22, 18.25.

### Step 7: methyl (2S,3S,4R,5S,6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate

To a solution of compound methyl (2S,3S,4R,5S,6S)-3,4,5-triacetoxy-6-[2-[5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]ethyl]tetrahydropyran-2-carboxylate (154 mg, 0.163 mmol) in THF (8.2 mL) was successively added triphenylphosphine (85.4 mg, 0.326 mmol) and 1-bromopyrrolidine-2,5-dione (58.0 mg, 0.326 mmol). The reaction mixture was stirred for 2h at room temperature. The progress of the reaction was followed by UPLC-MS: an aliquot was treated by a large exces of MeOH, following the formation of the corresponding methyl ether. The expected bomide derivative was stable in UPLC-MS conditions. After 5h were added triphenylphosphine (85.4 mg, 0.326 mmol) and 1-bromopyrrolidine-2,5-dione (58.0 mg, 0.326 mmol) and the reaction mixture was stirred for 15h at room temperature. The obtained crude methyl (2S,3S,4R,5S,6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate was used like this in the next step. UPLC-MS: MS (ESI) m/z [M+OMe-Br+H]+ = 960.7.

### Step 8: (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To the solution of methyl (2S,3S,4R,5S,6S)-3,4,5-triacetoxy-6-[2-[2-(bromomethyl)-5-[[(2S)-2-[[(2S)-2-(9H-fluoren-9-ylmethoxycarbonylamino)-3-methyl-butanoyl]amino]-5-ureido-pentanoyl]amino]phenyl]ethyl]tetrahydropyran-2-carboxylate (0.167mmol) in DMF from the previous step (step 7) was successively added (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (C1) (143 mg, 0.163 mmol) and DIPEA (114 µL, 0.652 mmol) The reaction mixture was stirred for 15 h at room temperature and the progress of the reaction was followed by UPLC-MS (aliquot was treated by a large exces of MeOH). The crude product was purified by C18 reverse phase prep-HPLC by direct deposit of the reaction mixture on the Xbridge column and using the TFA method to afford (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (21.3 mg, 0.0111 mmol) as a white powder. UPLC-MS: MS (ESI) m/z [M+H]+ = 1802.9+1804.9. IR Wavelength (cm⁻¹): 1755, 1672, 1226,1201,1130. ¹H NMR (400 MHz, dmso-d6) δ ppm 13.3 (br s, 1H), 10.2 (s, 1H), 8.88 (d, 1H), 8.61 (s, 1H), 8.14 (d, 1H), 7.88 (d, 2H), 7.73 (dd, 2H), 7.65 (d, 1H), 7.63 (d, 1H), 7.62 (m, 1H), 7.54 (br s, 1H), 7.51 (dd, 1H), 7.45 (t, 1H), 7.4 (t, 2H), 7.38 (m, 1H), 7.32 (t, 2H), 7.3 (dd, 2H), 7.2 (d, 1H), 7.2 (t, 2H), 7.15 (t, 1H), 7.15 (d, 1H), 7.03 (t, 1H), 7.01 (dd, 1H), 6.72 (t, 1H), 6.22 (d, 1H), 6 (br s, 1H), 5.51 (dd, 1H), 5.34 (t, 1H), 5.3 (br s, 2H), 5.27/5.21 (m, 2H), 4.98 (t, 1H), 4.85 (t, 1H), 4.57/4.49 (m, 2H), 4.39 (m, 1H), 4.35 (d, 1H), 4.27 (m, 2H), 4.26 (m, 2H), 4.23 (m, 1H), 3.93 (t, 1H), 3.76 (s, 3H), 3.71 (m, 1H), 3.64 (s, 3H), 3.4 (m, 4H), 3.29/2.51 (2dd, 2H), 3.13/2.94 (2m, 4H), 3 (m, 2H), 2.98 (m, 2H), 2.93 (br s, 3 H), 2.81 (m, 2H), 1.99/1.95 (3s, 9H), 1.98 (m, 1H), 1.84 (s, 3H), 1.77/1.59 (2m, 2H), 1.64 (2m, 2H), 1.41 (2m, 2H), 0.88/0.85 (2d, 6H). ¹³C NMR (100 MHz, dmso-d6): δ ppm 158.1, 152.9, 135.6, 131.5, 131.4, 131.3, 131.2, 131, 128.9, 128.1, 127.5, 125.7, 120.9, 120.6, 120.4, 120.3, 117, 116, 116, 112.8, 112.2, 111.2, 75.6, 74.9, 73.8, 72.9, 71.4, 69.6, 69.4, 67.5, 66.1, 60.4, 58.3, 56, 55.4, 53.9, 52.8, 47.2, 46.2, 44.3, 39, 32.8, 32.8, 31, 29.6, 27.4, 27.2, 21.1, 19.5/18.7, 18.1. ¹⁹F NMR (376 MHz, dmso-d6) δ ppm -74, -112.

### Step 9: (2S,3S,4R,5R,6S)-6-[2-[(5Sₐ)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]phenyl]ethyl]-3,4,5-trihydroxy-tetrahydropyran-2-carboxylic acid

To a solution of (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[2-[(2S,3S,4R,5S,6S)-3,4,5-triacetoxy-6-methoxycarbonyl-tetrahydropyran-2-yl]ethyl]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (21.3 mg, 0.0111 mmol) in methanol (6.0 mL) was added a solution of Lithium hydroxide monohydrate (4.66 mg µL, 0.111 mmol) in water (4 ml). The réaction mixture was stirred at room temperature for 60 h and the progress of the reaction was followed by UPLC-MS. The crude product was purified by C18 reverse phase prep-HPLC by direct deposit of the reaction mixture on the Xbridge column and using the TFA method to afford (2S,3S,4R,5R,6S)-6-[2-[(5Sₐ)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]phenyl]ethyl]-3,4,5-trihydroxy-tetrahydropyran-2-carboxylic acid (47.8 mg, 0.029 mmol) as a white powder. UPLC-MS: MS (ESI) m/z [M+H]+ = 1440.6+1442.6.

### Step 10: (2S,3S,4R,5R,6S)-6-[2-[2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]-5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]ethyl]-3,4,5-trihydroxy-tetrahydropyran-2-carboxylic acid;2,2,2-trifluoroacetate L21-C1

To a solution of (2S,3S,4R,5R,6S)-6-[2-[(5Sₐ)-5-[[(2S)-2-[[(2S)-2-amino-3-methyl-butanoyl]amino]-5-ureido-pentanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]phenyl]ethyl]-3,4,5-trihydroxy-tetrahydropyran-2-carboxylic acid (47.1 mg, 0.0282 mmol) in DMF (1.5 mL) were successively added the solution of (2,5-dioxopyrrolidin-1-yl) 3-[2-(2,5-dioxopyrrol-1-yl)ethoxy]propanoate (Purshased from Broadpharm, 13.1 mg, 0.0423 mmol) in DMF (500 µL) and DIPEA (17.2 µL, 0.0988 mmol).

The reaction mixture was stirred for 1h at room temperature and the progress of the reaction was followed by UPLC-MS. The crude product was purified by C18 reverse phase prep-HPLC by direct deposit of the reaction mixture on the Xbridge column and using the TFA method to afford L21-C1 (65 mg, 0.0310 mmol) as a white powder. HR-ESI+: m/z [M+H]+ = 1635.6093 (1635.6068) (measured/theoretical).

### Preparation of L9-C1:

### (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate;2,2,2-trifluoroacetic acid

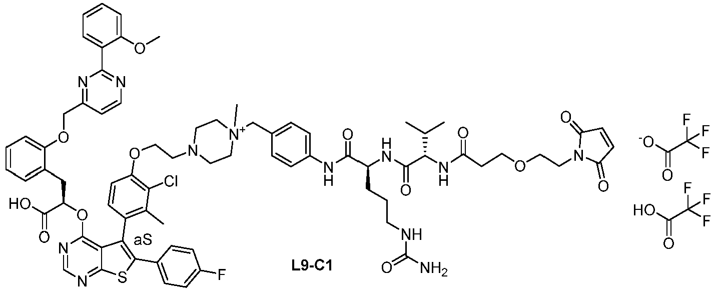

### Step 1: 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate

A solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (150 mg, 0.249 mmol) in THF (3.8 ml) was cooled to 0°C. At 0°C was added dropwise tribromophosphane (1 M in dichloromethane) (374 µL, 0.249 mmol). The reaction was stirred 5 min at 0°C and 1h at room temperature. The progress of the reaction was followed by UPLC-MS (aliquot was treated by a large excess of MeOH). The reaction mixture was diluted with ethyl acetate (3 ml) and washed with an aqueous saturated solution of sodium hydrogen carbonate (1x 6ml). The organic layer was dried over magnesium sulfate, filtered. Add DMF (10 ml) and evaporate the ethyl acetate and the THF. The obtained solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate is used like that in the next step. UPLC-MS: MS (ESI) m/z [M+Na]+ = 686.5+688.6.

### Step 2: (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To the solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (0.249 mmol) in DMF from the previous step (step 1) was successively added DMF (10 ml), (2R)-2-[(5Sa)-5-[3-chloro-2-methyl-4-(2-piperazin-1-ylethoxy)phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (C1) (218 mg, 0.249 mmol) and DIPEA (130 µL, 0.748 mmol). The reaction mixture was stirred for 15 h at room temperature and the progress of the reaction was followed by UPLC-MS (aliquot was treated by a large excess of MeOH). The obtained solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid in DMF was used like that in the next step. UPLC-MS: MS (ESI) m/z [M+Na]+ = 1458.7+1460.7.

### Step 3: (2R)-2-[(5Sₐ)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis 2,2,2-trifluoroacetic acid

To the solution of (2R)-2-[(5Sₐ)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (0.249 mmol) in dimethylformamide (3 mL) obtained in the previous step (step 2) was added piperidine (49.3 µL, 0.499 mmol). The reaction mixture was stirred at room temperature for 5 h and the progress of the reaction was followed by UPLC-MS. The crude product was purified by C18 reverse phase prep-HPLC by direct deposit of the reaction mixture on the Xbridge column and using the TFA method to afford (2R)-2-[(5Sₐ)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; bis 2,2,2-trifluoroacetic acid (31.2 mg = 0.0213 mmol) as a white powder. UPLC-MS: MS (ESI) m/z [M+Na]+ = 1236.7+1238.7.

### Step 4: (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid L9-C1

To a solution of (2R)-2-[(5Sa)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (31.2 mg, 0.0213 mmol) in DMF (1.5 mL) were successively added the solution of (2,5-dioxopyrrolidin-1-yl) 3-[2-(2,5-dioxopyrrol-1-yl)ethoxy]propanoate (23.8 mg, 0.0768 mmol) in DMF (500 µL) and DIPEA (31.2 µL, 0.179 mmol). The reaction mixture was stirred for 15h at room temperature and the progress of the reaction was followed by UPLC-MS. The crude product was purified by C18 reverse phase prep-HPLC by direct deposit of the reaction mixture on the Xbridge column and using the TFA method to afford L9-C1 (6 mg, 0.00303 mmol) as a white powder. HR-ESI+: m/z [M]+ = 1431.5437 (1431.5433) (measured/theoretical).

### Preparation of L9-C8:

### (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid;2,2,2-trifluoroacetate;2,2,2-trifluoroacetic acid

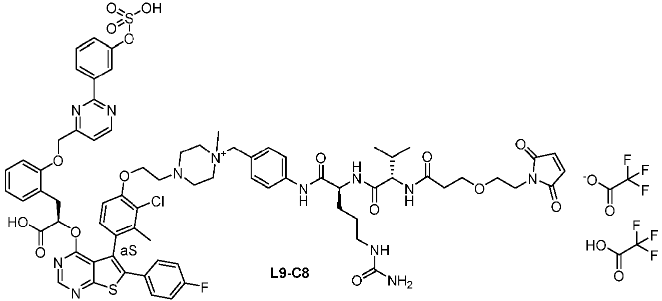

### Step 1: (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid

To the solution of 9H-fluoren-9-ylmethyl N-[(1S)-1-[[(1S)-1-[[4-(bromomethyl)phenyl]carbamoyl]-4-ureido-butyl]carbamoyl]-2-methyl-propyl]carbamate (0.0230 mmol; obtained according to Step 1 of the preparation of L9-C1) in DMF (3 mL) was successively added DMF (5 ml), ammonium; [3-[4-[[2-[(2R)-2-carboxy-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-ethyl]phenoxy]methyl]pyrimidin-2-yl]phenyl] sulfate (C8) (22 mg, 0.0230 mmol) and DIPEA (12 µL, 0.069 mmol). The reaction mixture was stirred for 15 h at room temperature and the progress of the reaction was followed by UPLC-MS (aliquot was treated by a large exces of MeOH). The obtained solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid in DMF was used in the next step. UPLC-MS: MS (ESI) m/z [M-SO₃H]+ = 1444.8+1446.7.

### Step 2: (2R)-2-[(5Sₐ)-5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid; bis 2,2,2-trifluoroacetic acid

To the solution of (2R)-2-[(5Sa)-5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid (0.0230 mmol) in dimethylformamide (3 mL) obtained in the previous step (step 1) was added piperidine (9 µL, 0.0920 mmol). The reaction mixture was stirred at room temperature for 5 h and the progress of the reaction was followed by UPLC-MS. The crude product was purified by C18 reverse phase prep-HPLC by direct deposit of the reaction mixture on the Xbridge column and using the TFA method to afford [3-[4-[[2-[(2R)-2-[5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-2-carboxyethyl]phenoxy]methyl]pyrimidin-2-yl]phenyl] sulfate; bis 2,2,2-trifluoroacetic acid (10.0 mg = 0.00633 mmol) as a white powder. UPLC-MS: MS (ESI) m/z [M-SO₃]+ = 1224.12.

### Step 3: (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid;2,2,2-trifluoroacetate;2,2,2-trifluoroacetic acid

To a solution of (2R)-2-[(5Sₐ)₋5-[4-[2-[4-[[4-[[(2S)-2-[[(2S)-2-amino-3-methyl-butanoyl]amino]-5-ureido-pentanoyl]amino]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid (10 mg, 0.00653 mmol) in DMF (1 mL) were successively added the solution of (2,5-dioxopyrrolidin-1-yl) 3-[2-(2,5-dioxopyrrol-1-yl)ethoxy]propanoate (3.1 mg, 0.00980 mmol) in DMF (500 µL) and DIPEA (4 µL, 0.0229 mmol). The reaction mixture was stirred for 15h at room temperature and the progress of the reaction was followed by UPLC-MS. The crude product was purified by C18 reverse phase prep-HPLC by direct deposit of the reaction mixture on the Xbridge column and using the TFA method to afford **L9-C8** (6 mg, 0.00401 mmol) as a white powder. UPLC-MS: MS (ESI) m/z [M+Na]+ = 1519.5+1521.2, [M+H-SO3]+ 1417.7+1419.6. HR-ESI+: m/z [M+H]+ = 1497.486 (1497.4845) (measured/theoretical).

### Preparation of L9-C10:

### (2R)-2-[(5Sₐ)5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-[4-fluoro-3-(2,2,2-trifluoroethoxy)phenyl]thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate; 2,2,2-trifluoroacetic acid

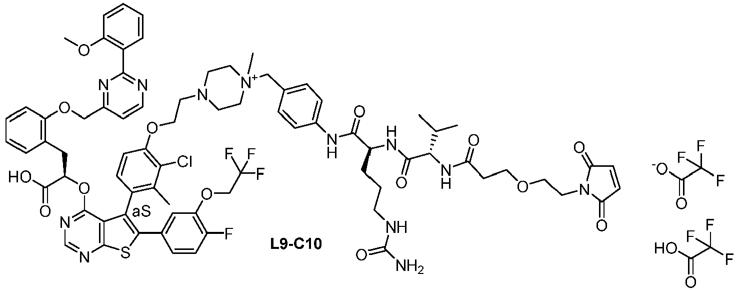

The procedure is as in the process of synthesis of L9-C9, replacing C9 used in Step 3 by (2R)-2-[((5Sa)-5-{3-chloro-2-methyl-4-[2-(4-methylpiperazin-1-yl)ethoxy]phenyl}-6-[4-fluoro-3-(2,2,2-trifluoroethoxy)phenyl]thieno[2,3-d]pyrimidin-4-yl)oxy]-3-(2-{[2-(2-methoxyphenyl)pyrimidin-4-yl]methoxy}phenyl)propanoic acid C10 and using TFA method for purification. HR-ESI+: m/z [M+H]+ = 1529.543 / 1529.5413 (measured/theoretical).

### Preparation of L9-C11:

### (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(4-methoxyphenyl)pyrimidin-4-yl]methoxy]phenyl]propanoic acid; 2,2,2-trifluoroacetate; 2,2,2-trifluoroacetic acid.

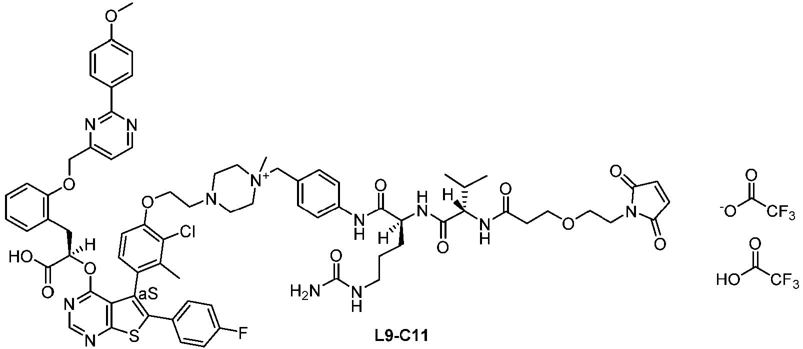

The procedure is as in the process of synthesis of **L9-C9,** replacing **C9** used in Step 3 by (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(4-methoxyphenyl)pyrimidin-4-yl]methoxy}phenyl)propanoic acid **C11** and using TFA method for purification. HR-ESI+: m/z [M+H]+ = 1431.5442 / 14.31.5433 (measured/theoretical).

### Preparation of L9-C12:

### (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2,2,2-trifluoroethyl)pyrazol-3-yl]methoxy]phenyl]propanoic acid;2,2,2-trifluoroacetate;2,2,2-trifluoroacetic acid

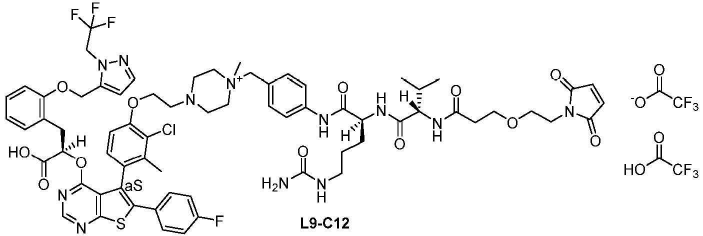

The procedure is as in the process of synthesis of **L9-C9,** replacing C9 used in Step 3 by (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[1-(2,2,2-trifluoroethyl)-1H-pyrazol-5-yl]methoxy}phenyl)propanoic acid C12 using TFA method for purification. HR-ESI+: m/z [M+H]+ = 1395.5048 / 1395;5045 (measured/theoretical).

### Preparation of L9-C14:

### (2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d] pyrimidin-4-yl]oxy-3-[2-[[2-(3-hydroxy-2-methoxy-phenyl)pyrimidin-4-yl]methoxy]phenyl]propanoic acid;2,2,2-trifluoroacetate

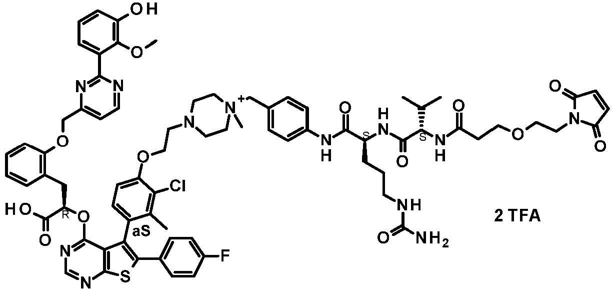

### Step 1: (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxy-3-sulfooxy-phenyl)pyrimidin-4-yl]methoxy]phenyl]propanoic acid

500 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-chloropyrimidin-4-yl)methoxy]phenyl]propanoate (0.60 mmol, WO2016/207216 **Preparation** 1) and 202 mg (3-hydroxy-2-methoxy-phenyl)boronic acid (1.20 mmol) were dissolved in 9 mL 1,4-dioxane, then 42 mg Pd(PPh₃)₂Cl₂ (0.06 mmol), 588 mg Cs₂CO₃ (1.80 mmol) and 9 mL water were added and the mixture was stirred under N₂ atmosphere at 70°C until complete conversion. Then it was diluted with water, neutralized with 2 M aqueous HCl solution, and extracted with DCM. The combined organic phase was dried over Na₂SO₄, filtered and the filtrate was concentrated under reduced pressure. The crude ester was purified via flash chromatography using heptane, EtOAc and 0.7 M NH₃ solution in MeOH as eluents to obtain a mixture of diastereoisomers. Then it was dissolved in 23.6 mL pyridine, 0.97 mL SO₃×pyrimidine (5.98 mmol) was added and the mixture was stirred at 70°C until complete conversion. Then it was concentrated under reduced pressure, and dissolved in 2 mL dioxane, then 200 mg KOH (3.57 mmol) and 1 mL water were added. The mixture was stirred at rt until complete hydrolysis. Then it was neutralized with 2 M aqueous HCl solution, and directly injected on prep-RP-HPLC, using 25 mM aqueous NH₄HCO₃ solution and MeCN as eluents. The diastereoisomer eluting later was collected as product of the title. ¹H NMR (500 MHz, DMSO-d₆) δ: 8.92 (d, 1H), 8.63 (s, 1H), 7.68 (dd, 1H), 7.63 (d, 1H), 7.34 (d, 1H), 7.30 (dd, 2H), 7.29 (d, 1H), 7.20 (t, 2H), 7.16 (t, 1H), 7.15 (d, 1H), 7.10 (t, 1H), 7.02 (d, 1H), 6.73 (t, 1H), 6.38 (d, 1H), 5.50 (dd, 1H), 5.29/5.23 (d+d, 2H), 4.21/4.16 (m+m, 2H), 3.84 (s, 3H), 3.25/2.55 (dd+dd, 2H), 3.18-2.75 (m, 10H), 2.65 (brs, 3H), 1.82 (s, 3H).HRMS calculated for C₄₇H₄₄N₆O₁₀S₂CIF: 970.2233; found 971.2297 (M+H).

### Step 2: (2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]ethoxy]-2-methyl-phenyl]-6-(4-fluorophenyl)thieno[2,3-d] pyrimidin-4-yl]oxy-3-[2-[[2-(3-hydroxy-2-methoxy-phenyl)pyrimidin-4-yl]methoxy]phenyl]propanoic acid;2,2,2-trifluoroacetate L9-C14

To a solution of (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxy-3-sulfooxy-phenyl)pyrimidin-4-yl]methoxy]phenyl]propanoic acid (20.0 mg; 0.0206 mmol) in DMF (309 µL), were successively added (2S)-N-[4-(chloromethyl)phenyl]-2-[[(2S)-2-[3-[2-(2,5-dioxopyrrol-1-yl)ethoxy]propanoylamino]-3-methyl-butanoyl]amino]-5-ureido-pentanamide (17.5 mgL; 0.0206 mmol), DIPEA (10.8 µL; 0.0618 mmol) and TBAI (1 mg; 0.0027 mmol). The reaction mixture was stirred at 70°C for 18 hours. The crude product was purified by C₁₈ reverse phase prep-HPLC by direct deposit of the reaction mixture on the X-Bridge column and using the TFA method to afford L9-C14 (10.5 mg; 0.00725 mmol) as a white powder. HR-ESI+: m/z [M+H]+ = 1448.5437 / 1448.5466 [measured/theoretical].

### Preparation of L9-P15:

### (11R,20R)-23,26-dichloro-20-[[4-[[4-[[(2S)-2-[[(2S)-2-[3-[2-(2,5-dioxopyrrol-1-yl)ethoxy propanoylamino]-3-methyl-butanoyl]amino]-5-ureido-pentanoyl]amino]phenyl]methyl]-4-methyl-piperazin-4-ium-1-yl]methyl]-3-(4-fluorophenyl)-14-[[2-(2-methoxyphenyl) pyrimidin-4-yl]methoxy]-24,25-dimethyl-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid;2,2,2-trifluoroacetate

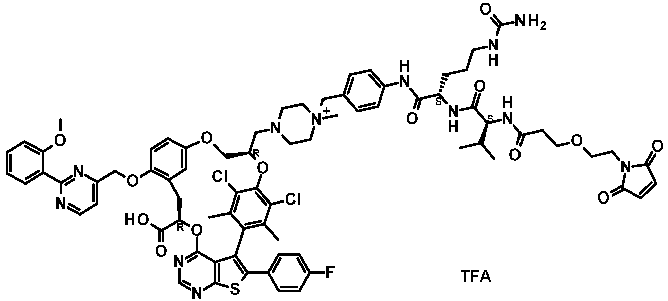

To a solution of (11R,20R)-23,26-dichloro-3-(4-fluorophenyl)-14-[[2-(2-methoxyphenyl)pyrimidin-4-yl]methoxy]-24,25-dimethyl-20-[(4-methylpiperazin-1-yl)methyl]-10,18,21-trioxa-4-thia-6,8-diazapentacyclo[20.2.2.12,5.113,17.09,28]octacosa-1(25),2,5(28),6,8,13,15,17(27),22(26),23-decaene-11-carboxylic acid **P15** (obtained according to WO 2019/035914; 10.0 mg; 0.0105 mmol) in DMF (630 µL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (10.0 mg; 0.0158 mmol), DIPEA (5.5 µL; 0.0315 mmol) and TBAI (0.5mg, 0.0010mmol). The reaction mixture was stirred at room temperature for 0.5 hour. The crude product was purified by C₁₈ reverse phase prep-HPLC by direct deposit of the reaction mixture on the X-Bridge column and using the TFA method to afford **L9-P15** (11.9 mg, 0.00733 mmol) as a white powder. HR-ESI+: m/z [M-CF₃CO₂]+ = 1507.5183 / 1507.5155

### Preparation of L9-P16:

### (11R,20R)-23,26-dichloro-14-[[2-[4-[[(2S)-1,4-dioxan-2-yl]methoxymethyl]-4-fluoro-cyclohexyl]pyrimidin-4-yl]methoxy]-20-[[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino] phenyl]methyl]-4-methyl-piperazin-4-ium-1-yl]methyl]-3-(4-fluorophenyl)-24,25-dimethyl-10,18,21-trioxa-4-thia-6,8-diazapentacyclo[20.2.2.12,5.113,17.09,28]octacosa-1(25),2,5(28),6,8,13,15,17(27),22(26),23-decaene-11-carboxylic acid;2,2,2-trifluoroacetate

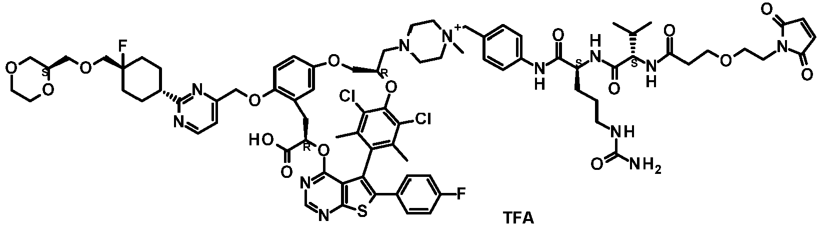

To a solution of (11R,20R)-23,26-dichloro-14-[[2-[4-[[(2S)-1,4-dioxan-2-yl]methoxymethyl]-4-fluoro-cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid **P16** (obtained according to WO 2019/035911; 14.7 mg; 0.0137 mmol) in DMF (1 mL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (13.1 mg; 0.0205 mmol) and DIPEA (7.1 µL; 0.0410 mmol). The reaction mixture was stirred at room temperature for 2 hours. The crude product was purified by direct deposit of the reaction mixture on the X-Bridge column in using the TFA method to afford **L9-P16** (7.9 mg; 0.00420 mmol) as a white powder. IR (cm⁻¹): 3327, 1768/1706, 1666, 1199/1118, 831/798. HR-ESI+: m/z [M-CF₃CO₂]+ = 1631.6071/1631.6054 [measured/theoretical]

### Preparation of L9-P17:

### (11R,20R)-23,26-dichloro-14-[[2-[4-[[(2S)-1,4-dioxan-2-yl]methoxy]cyclohexyl]pyrimidin-4-yl]methoxy]-20-[[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]phenyl]methyl]-4-methyl-piperazin-4-ium-1-yl]methyl]-3-(4-fluorophenyl)-24,25-dimethyl-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid;2,2,2-trifluoroacetate_

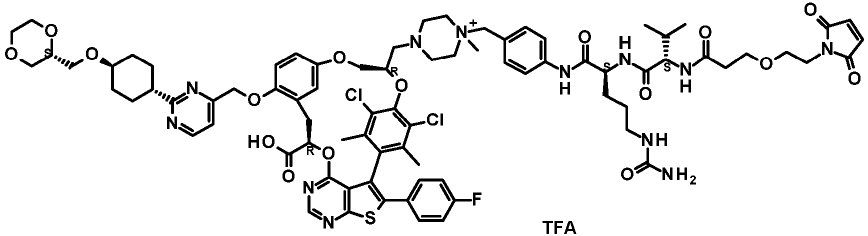

To a solution of (11R,20R)-23,26-dichloro-14-[[2-[4-[[(2S)-1,4-dioxan-2-yl]methoxy]cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid **P17** (obtained according to WO 2019/035911; 14.5 mg; 0.0139 mmol) in DMF (1 mL), were successively added (2S)-N-[4-(bromomethyl)phenyl]-2-[[(2S)-2-[3-[2-(2,5-dioxopyrrol-1-yl)ethoxy]propanoylamino]-3-methyl-butanoyl]amino]-5-ureido-pentanamide (13.3 mg; 0.0208 mmol) and DIPEA (7.3 µL; 0.0417 mmol). The reaction mixture was stirred at room temperature for 8 hours. The crude product was purified by direct deposit of the reaction mixture on the X-Bridge column and using the TFA method to afford **L9-P17** (7.0 mg, 0.00437 mmol) as a white powder. IR (cm⁻¹): 3700-2400, 1771/1738/1705, 1665, 1194/1128. HR-ESI+: m/z [M-CF₃CO₂]+ = 1599.6013 / 1599.5992. HR-ESI+: m/z [M-CF₃CO₂+H]2+ = 800.3049 / 800.3035 [measured/theoretical]

### Preparation of L25-P1:

### 2-[[4-[2-[4-[4-[(1R)-1-carboxy-2-[2-[[2-(2-methoxyphenyl)pyrimidin-4-yl]methoxy]phenyl] ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]-5-[[(25)-2-[[1-[2-[2-(2,5-dioxopyrrol-1-yl)ethoxy]ethylcarbamoyl]cyclobutanecarbonyl]amino]-5-ureido-pentanoyl]amino]benzenesulfonate;2,2,2-trifluoroacetic acid

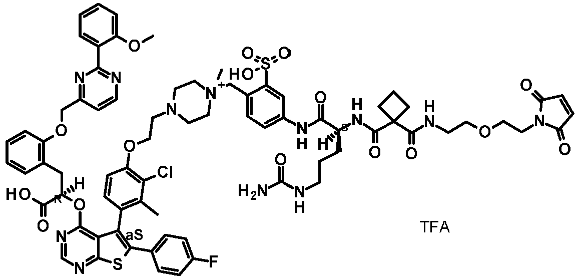

### Step 1: tert-butyl 1-[2-[2-(2,5-dioxopyrrol-1-yl)ethoxy]ethylcarbamoyl]cyclobutanecarboxylate

To a solution of 1-tert-butoxycarbonylcyclobutanecarboxylic acid (58.6 mg; 0.293 mmol) in DCM (5.85 ml), were successively added 1-[2-(2-aminoethoxy)ethyl]pyrrole-2,5-dione (53.9 mg; 0.293 mmol), EDC (84.2 mg; 0.439 mmol), HOBt (59.3 mg; 0.439 mmol), and DIPEA (204 µL; 1.17 mmol). The reaction mixture was stirred at room temperature for 18 hours. The progress of the reaction was monitored by UPLC-MS. The reaction mixture was concentrated to dryness and solubilized in DMF (1 ml) and the solution was purified by X-Bridge column C₁₈ by direct deposit of the reaction mixture on the column and in using the TFA method to afford the title compound (57.3 mg; 0.156 mmol). IR (cm⁻¹): 3390, 1697/1666. ¹H NMR (400 MHz, dmso-d6) δ ppm 7.5 (t, 1H), 7.02 (s, 2H), 3.55/3.5 (2t, 4H), 3.38 (t, 2H), 3.17 (q, 2H), 2.33 (m, 4H), 1.77 (m, 2H), 1.38 (s, 9H). UPLC-MS: MS(ESI): m/z [M+Na]+ = 389.26 [M+H-tBu]+ = 311.22

### Step 2: 1-[2-[2-(2,5-dioxopyrrol-1-yl)ethoxy]ethylcarbamoyl]cyclobutanecarboxylic acid

To a solution of tert-butyl 1-[2-[2-(2,5-dioxopyrrol-1-yl)ethoxy]ethylcarbamoyl] cyclobutanecarboxylate (7 mg; 0.0191 mmol) in DCM (0.175 mL), was added TFA (51.2 µL; 0.668 mmol). The reaction mixture was stirred at room temperature for 3.5 hours, then was concentrated to dryness to obtain the title compound (5.8 mg; 0.0187 mmol) as a colorless oil. The crude product was used in the next step. UPLC-MS: MS(ESI): m/z [M+H]+ = 311.35, [M+Na]+ = 333.37

### Step 3: (2,3,4,5,6-pentafluorophenyl) 1-[2-[2-(2,5-dioxopyrrol-1-yl)ethoxy]ethylcarbamoyl]cyclobutanecarboxylate

To a solution of 1-[2-[2-(2,5-dioxopyrrol-1-yl)ethoxy]ethylcarbamoyl]cyclobutanecarboxylic acid (18.2 mg; 0.0587 mmol) in THF (3 mL), were successively added 2,3,4,5,6-pentafluorophenol (13.0 mg; 0.0704 mmol) and DCC (14.5 mg; 0.0704 mmol). The reaction mixture was stirred at room temperature for 15 hours and the progress of the reaction was monitored by UPLC-MS. The reaction mixture was a suspension, the precipitate is filtered off and washed with THF (1 ml) to afford a solution of (2,5-dioxopyrrolidin-1-yl) 1-[2-[2-(2,5-dioxopyrrol-1-yl)ethoxy]ethylcarbamoyl]cyclobutanecarboxylate in THF. The crude product solution was used in step 9. UPLC-MS: MS(ESI): m/z [M+H]+ = 477.28, [M+Na]+ = 499.23

### Step 4: methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate

To a solution of (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid **P1** (5.0 g; 5.712 mmol) in DCM (25 mL) and methanol (25 mL), was added dropwise a solution of diazomethyl(trimethyl)silane (2 M in Et₂O) (5.712 mL; 11.42 mmol). The reaction mixture was stirred at room temperature for 2 hours and the progress of the reaction was monitored by UPLC-MS. After completion the reaction was quenched by a slow addition of acetic acid until the yellow color turn to red and was concentrated to dryness to afford the crude mixture. The crude product was purified by silica gel chromatography (gradient of methanol in DCM) to afford the title compound (4.52 g; 5.082 mmol). UPLC-MS: MS(ESI): m/z [M+H]+ = 889.27+891.6, [M+Na]+ = 911.31, [M+2H]2+= 445.59. IR (cm⁻¹): 1753, 1238/1053. ¹H NMR (400 MHz, dmso-d6) δ ppm 8.6 (s, 1H), 8.45 (d, 1H), 7.6 (d, 1H), 7.52 (dd, 1H), 7.45 (td, 1H), 7.3 (m, 3H), 7.25-7.1 (m, 5H), 7.02 (t+d, 2H), 6.78 (t, 1H), 6.31 (dd, 1H), 5.52 (dd, 1H), 5.25 (AB, 2H), 4.2 (m, 2H), 3.78/3.65 (2s, 6H), 3.2/2.58 (2dd, 2H), 2.71 (t, 2H), 2.5/2.3 (2ml, 8H), 2.12 (s, 3H), 1.88 (s, 3H).

### Step 5: 5-[[(2S)-2-(9H-fluoren-9-ylmethoxycarbonylamino)-5-ureido-pentanoyl]amino]-2-(hydroxymethyl)benzenesulfonic acid

To a solution of Fmoc-Cit-OH (2.224 g; 5.596 mmol) in DCM (22.2 mL) and methanol (22.2 mL), were successively added sodium 5-amino-2-(hydroxymethyl) benzenesulfonate (1.89 mg; 8.395 mmol) and EEDQ (2.768 g; 11.19 ml). The reaction mixture was stirred at room temperature for 25 hours, then was concentrated to dryness. The crude product was purified by silica gel chromatography (gradient of methanol in DCM) to afford the title compound (2.81 g; 4.823 mmol) as white powder. IR (cm⁻¹): 3700-3000, 1660(large), 1180. ¹H NMR (400 MHz, dmso-d6) δ ppm 10.02 (s, 1H), 7.88 (m, 3H), 7.76 (2t, 2H), 7.7 (dd, 1H), 7.61 (d, 1H), 5.99 (t, 1H), 5.38 (m, 2H), 5.03 (t, 1H), 4.72 (d, 2H), 4.3-4.2 (m, 3H), 4.15 (m, 1H), 3.06-2.90 (m, 2H), 1.75-1.30 (m, 4H). UPLC-MS: MS(ESI): m/z [M+H]+ = 583.42, [M+Na]+ = 565.31.

### Step 6: 2-(chloromethyl)-5-[[(2S)-2-(9H-fluoren-9-ylmethoxycarbonylamino)-5-ureido-pentanoyl]amino]benzenesulfonic acid

To a solution of 5-[[(2S)-2-(9H-fluoren-9-ylmethoxycarbonylamino)-5-ureido-pentanoyl]amino]-2-(hydroxymethyl)benzenesulfonic acid (543.6 mg; 0.933 mmol) in NMP (5 mL) were added at room temperature a solution of SOCl₂ (68.1 µL; 0.933 mmol) in NMP (200 µL). The reaction mixture was stirred at room temperature for 15 min and the progress of the reaction was monitored by UPLC-MS. To achieve a complete conversion, the SOCl₂ addition (68 µL) has to be done 7 more times. The excess SOCl₂ was evaporated under vaccum, and the residue was purified by direct deposit of the reaction mixture on an Oasis column in using the TFA method to afford the title compound (362 mg; 0.512 mmol) as a white solid. UPLC-MS: MS(ESI): m/z [M+H]+ = 601.19+603.23 [M+Na]+ = 622.93

### Step 7: 2-[[4-[2-[2-chloro-4-[6-(4-fluorophenyl)-4-[(1R)-2-methoxy-1-[[2-[[2-(2-methoxyphenyl) pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]-5-[[(2S)-2-(9H-fluoren-9-ylmethoxycarbonylamino)-5-ureido-pentanoyl]amino]benzenesulfonate

To a solution of 2-(chloromethyl)-5-[[(2S)-2-(9H-fluoren-9-ylmethoxycarbonylamino)-5-ureido-pentanoyl]amino]benzenesulfonic acid (195.6 mg; 0.277 mmol) from step 6 in solution in NMP (10 mL), were successively added methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate (123 mg; 0.138 mmol) from step 4, DIPEA (385 µL, 2.213 mmol) and TBAI (10 mg, 0.027 mmol). The reaction mixture was stirred at 70°C for 12 hours and the progress of the reaction was monitored by UPLC-MS. The crude product in solution in NMP was directly used in the next step. UPLC-MS: MS(ESI): m/z [M+H]+ = 1231.12+1233.45, [M+2H]2+ = 616.34+617.37

### Step 8: 5-[[(2S)-2-amino-5-ureido-pentanoyl]amino]-2-[[4-[2-[4-[4-[(1R)-1-carboxy-2-[2-[[2-(2-methoxyphenyl) pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]benzenesulfonate ;2,2,2-trifluoroacetic acid

To the previous solution of 2-[[4-[2-[2-chloro-4-[6-(4-fluorophenyl)-4-[(1R)-2-methoxy-1-[[2-[[2-(2-methoxyphenyl)pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]-5-[[(2S)-2-(9H-fluoren-9-ylmethoxycarbonylamino)-5-ureido-pentanoyl]amino]benzenesulfonate in NMP, was added a solution of lithium hydroxyde mono hydrate (82.2 mg; 1.106 mmol) in water (4 mL). The reaction mixture was stirred at room temperature for 1.5 hours and the progress of the reaction was monitored by UPLC-MS. The crude product solution was purified by direct deposit of the reaction mixture on a X-Bridge column in using the TFA method to afford the title compound (45.6 mg; 0.0374 mmol) as a white powder. UPLC-MS: MS(ESI): m/z [M+H]+ = 1217.46, [M+Na]+ = 1241.16, [M+2H]2+ = 609.61

### Step 9: 2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]-5-[[(2S)-2-[[1-[2-[2-(2,5-dioxopyrrol-1-yl)ethoxy]ethylcarbamoyl]cyclobutanecarbonyl]amino]-5-ureido-pentanoyl]amino]benzenesulfonate L25-P1

To a solution of 5-[[(2S)-2-amino-5-ureido-pentanoyl]amino]-2-[[4-[2-[4-[4-[(1R)-1-carboxy-2-[2-[[2-(2-methoxyphenyl)pyrimidin-4-yl]methoxy]phenyl]ethoxy]-6-(4-fluorophenyl)thieno[2,3-d]pyrimidin-5-yl]-2-chloro-3-methyl-phenoxy]ethyl]-1-methyl-piperazin-1-ium-1-yl]methyl]benzenesulfonate (22.6 mg; 0.0186 mmol) in DMF (1.4 mL), were successively added a THF solution of (2,3,4,5,6-pentafluorophenyl) 1-[2-[2-(2,5-dioxopyrrol-1-yl)ethoxy]ethylcarbamoyl]cyclobutanecarboxylate (from step 3) (26.8 mg; 0.0562 mmol) and DIPEA (12.9 µL; 0.0742 mmol). The reaction mixture was stirred at room temperature for 2 hours. The crude product solution was purified by direct deposit of the reaction mixture on a X-Bridge column and in using the TFA method to afford **L25-P1** (7.5 mg; 0.0050 mmol) as a white powder. IR (cm⁻¹): 3321, 1705/1624, 1666, 1581, 1180/1124, 833/798/756/719/696. ¹H NMR (400/500 MHz, dmso-d6) δ ppm 10.4 (s), 8.88 (d, 1H), 8.61 (s, 1H), 8.13 (df, 1H), 7.92 (dd, 1H), 7.78 (d), 7.74 (t), 7.63 (d, 1H), 7.52 (dd, 1H), 7.47 (d, 1H), 7.46 (t, 1H), 7.38 (d, 1H), 7.3 (dd, 2H), 7.23 (d, 1H), 7.21 (t, 2H), 7.16 (t, 1H), 7.14 (d, 1H), 7.03 (t, 1H), 7.01 (d, 1H), 7 (s, 2H), 6.73 (t, 1H), 6.22 (d, 1H), 5.99 (m), 5.55 (sl), 5.5 (dd, 1H), 5.25 (AB, 2H), 5.1 (sl, 2H), 4.37 (m, 1H), 4.33 (m, 2H), 3.76 (s, 3H), 3.7 (m, 10H), 3.55 (m, 2H), 3.5 (m, 2H), 3.42 (m, 2H), 3.28/2.52 (2dd, 2H), 3.21 (m, 2H), 3.04 (sl, 3H), 2.97 (m, 2H), 2.4 (m, 4H), 1.85 (w, 3H), 1.74/1.62 (2m, 2H), 1.73 (m, 2H), 1.43/1.35 (2m, 2H). ¹³C NMR (400/500 MHz, dmso-d6) δ ppm 157.5, 152.8, 135.4, 134.9, 131.5, 131.4, 131.4, 131.2, 131.1, 128.7, 121, 120.6, 119.2, 119.2, 116.3, 116, 112.8, 112.2, 111, 74, 69.5, 68.9, 67.4, 66.6, 56.2, 55.3/46.5, 54.1, 45.7, 39.4, 39.2, 37.2, 32.9, 29.7, 29.7, 27.3, 18, 16. ¹⁹F NMR (400/500 MHz, dmso-d6) δ ppm -74.6, -112.2. HR-ESI+: m/z [M+H]+ = 1509.4867 / 1509.4851 [measured/theoretical]

### Preparation of L26-P1:

### (2R)-2-[5-[3-chloro-4-[2-[4-[[4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy]ethoxy]ethoxy]ethoxy]propyl] phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate;2,2,2-trifluoroacetate_

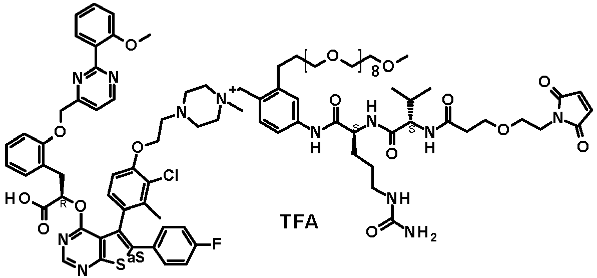

### Step 1: 3-[2-[2-[2-[2-[2-[2-[2-(2-methoxyethoxy)ethoxy]ethoxy]ethoxy]ethoxy]ethoxy]ethoxy] ethoxy]prop-1-yne

To a solution of 2-[2-[2-[2-[2-[2-[2-(2-methoxyethoxy)ethoxy]ethoxy]ethoxy]ethoxy]ethoxy] ethoxy]ethanol (1.95 g; 6.50 mmol) in THF (25.0 mL), was added at 0°C sodium hydride (260.0 mg; 6.57 mmol). After 5 minutes, a solution of 3-bromoprop-1-yne in toluene (1.42 mL; 13.14 mmol) was added. The reaction mixture was stirred at 0°C for 1 hour and 2 days at room temperature and the progress of the reaction was monitored by UPLC-MS. Then, the reaction mixture was filtered and the filtrate was concentrated to dryness, and purified by silica gel chromatography (gradient DCM in methanol) to afford the title compound (1.74 g; 4.12 mmol) as a colorless oil. ¹H NMR (CDCl₃): 2.43 (t, 1H, *J* = 2.4 Hz), 3.37 (s, 3H), 3.53-3.55 *(m,* 2H), 3.64-3.70 *(m,* 30H), 4.20 (d, 2H, *J* = 2.4 Hz).

### Step 2: 9H-fluoren-9-ylmethyl N-[(1S)-1-[[4-[[tert-butyl(dimethyl)silyl]oxymethyl]-3-iodophenyl]carbamoyl]-4-ureido-butyl]carbamate

To a solution of [[tert-butyl(dimethyl)silyl]oxymethyl]-3-iodo-aniline (10.0 g; 27.52 mmol) in methanol (70 mL) and DCM (140 mL), were successively added Fmoc-Cit-OH (12.0 g; 30.28 mmol) and EEDQ (8.17 g; 33.03 mmol). The reaction mixture was stirred for 14 hours at room temperature. After the completion of the reaction, the resulting residue was purified by column chromatography on silica gel using DCM / methanol (100/0 to 88/12) as eluent to afford the title compound (17.09 g; 21.97 mmol) as a white solid. ¹H NMR (DMSO): δ 0.09 (s, 6H), 0.91 (s, 9H), 1.38-1.48 *(m,* 2H), 1.59-1.68 *(m,* 2H), 2.93-3.05 *(m,* 2H), 4.06-*4.15 (m,* 2H), 4.20-4.29 *(m,* 3H), 4.56 (s, 2H), 5.41 (s, 2H), 5.98 (t, 1H, *J* = 5.5 Hz), 7.30-7.43 *(m,* 5H), 7.55 *(dd,* 1H, *J* = 8.8, 2.1 Hz), 7.69 (d, 1H, *J* = 7.8 Hz), 7.74 *(dd,* 1H, *J* = 7.2, 3.4 Hz), 7.89 (d, 1H, *J* = 7.5 Hz), 8.25 (d, 1H, *J* = 1.5 Hz), 10.12 *(s,* 1H).

### Step 3: (2S)-2-amino-N-[4-[[tert-butyl(dimethyl)silyl]oxymethyl]-3-iodo-phenyl]-5-ureido-pentanamide

To a solution of 9H-fluoren-9-ylmethyl N-[(1S)-1-[[4-[[tert-butyl(dimethyl)silyl]oxymethyl]-3-iodo-phenyl]carbamoyl]-4-ureido-butyl]carbamate (17.08 g; 23.00 mmol) in THF (120 mL), was added dimethylamine 2M in THF (44.5 mL; 89.00 mmol). The reaction mixture was stirred for 15 hours at room temperature. After concentration to dryness, the resulting residue was purified by column chromatography on C₁₈ using water / acetonitrile (98/02 to 0/100) as eluent to afford compound the title compound (5.47 g; 10.50 mmol) as a white solid. ¹H NMR (DMSO): δ 0.0 (s, 6H), 0.81 (s, 9H), 1.27-1.38 *(m,* 3H), 1.47-1.53 *(m,* 1H), 2.83-2.89 *(m,* 2H), 3.16-3.19 *(m,* 1H), 4.46 (s, 2H), 5.26 (s, 2H), 5.82 (t, 1H, *J* = 5.6 Hz), 7.24 (d, 1H, *J* = 8.5 Hz), 7.50 (dd, 1H, *J* = 8.3, 2.0 Hz), 8.17 (d, 1H, *J* = 2.0 Hz).

### Step 4: 9H-fluoren-9-ylmethyl N-[(1S)-1-[[(1S)-1-[[4-[[tert-butyl(dimethyl)silyl]oxymethyl]-3-iodo-phenyl]carbamoyl]-4-ureido-butyl]carbamoyl]-2-methyl-propyl]carbamate

To a solution of (2S)-2-amino-N-[4-[[tert-butyl(dimethyl)silyl]oxymethyl]-3-iodophenyl]-5-ureido-pentanamide (3.00 g; 5.76 mmol) in 2-methyltetrahydrofuran (240 mL), were successively added Fmoc-Val-OSu (8.65 g; 8.65 mmol) and DIPEA (1.90 mL; 11.53 mmol). The reaction mixture was stirred for 15 hours at room temperature. The reaction mixture was filtered through a sintered funnel and the recovered solid was washed with 2-methyltetrahydrofuran (2 x 250 mL), then dried under high vacuum to afford the title compound (3.57 g; 4.24 mmol) as a white solid. ¹H NMR (DMSO): δ 0.10 (s, 6H), 0.83-0.95 *(m,* 15H), 1.27-1.52 *(m,* 2H), 1.52-1.75 *(m,* 2H), 1.93-2.07 *(m,* 1H), 2.88-3.09 *(m,* 2H), 3.93 *(t,* 1H, *J* = 8.0 Hz), 4.17-4.49 *(m,* 4H), 4.56 (s, 2H), 5.40 (s, 2H), 5.96 *(t,* 1H, *J* = 5.6 Hz), 7.27-7.37 *(m,* 3H), 7.37-7.48 *(m,* 3H), 7.54 (d, 1H, *J* = 8.0 Hz), 7.74 *(t,* 2H, *J* = 7.2 Hz), 7.88 (d, 2H, *J* = 7.6 Hz), 8.13 (d, 1H, *J* = 7.6 Hz), 8.22 (s, 1H), 10.11 *(s,* 1H).

### Step 5: 9H-fluoren-9-ylmethyl N-[(1S)-1-[[(1S)-1-[[4-[[tert-butyl(dimethyl)silyl]oxymethyl]-3-[3-[2-[2-[2-[2-[2-[2-[2-(2-methoxyethoxy)ethoxy]ethoxy]ethoxy]ethoxy]ethoxy]ethoxy]ethoxy] prop-1-ynyl]phenyl]carbamoyl]-4-ureido-butyl]carbamoyl]-2-methyl-propyl]carbamate

To a solution of 9H-fluoren-9-ylmethyl N-[(1S)-1-[[(1S)-1-[[4-[[tert-butyl(dimethyl)silyl] oxymethyl]-3-iodo-phenyl]carbamoyl]-4-ureido-butyl]carbamoyl]-2-methyl-propyl]carbamate (1.23 g; 1.46 mmol) in dimethylformamide (15.40 mL), were added successively 3-[2-[2-[2-[2-[2-[2-[2-(2-methoxyethoxy)ethoxy]ethoxy]ethoxy]ethoxy]ethoxy]ethoxy]ethoxy]prop-1-yne (930.0 mg; 2.20 mmol) and DIPEA (2.47 mL; 14.92 mmol). After 3 purges with argon, Pd(PPh₃)₂Cl₂ (220 mg; 0.307 mmol) and Cul (68.0 mg; 0.36 mmol) were added and the reaction mixture was purged with argon 3 times. The reaction mixture was stirred for 3 hours at room temperature and the progress of the reaction was monitored by UPLC-MS. The mixture was diluted with isopropyl acetate (200 mL) and washed with brine (3 x 150 mL). The combined organic layers were dried over sodium sulfate, filtered and concentrated to dryness. The crude product was purified by C18 reverse phase prep-HPLC by direct deposit of the reaction mixture on the X-Bridge column ad using neutral method to afford the title compound (790.0 mg; 0.70 mmol) as a pale yellow gum. ¹H NMR (DMSO): δ 0.08 (s, 6H), 0.85-0.90 *(m,* 15H), 1.36-1.45 *(m,* 2H), 1.58-1.71 *(m,* 2H), 1.97-2.00 *(m,* 1H), 2.93-3.03 *(m,* 2H), 3.23 (s, 3H), 3.40-3.43 *(m,* 2H), 3.49-3.52 *(m,* 25H), 3.56-3.58 *(m,* 2H), 3.63-3.66 *(m,* 2H), 3.93 (dd, 1H, *J* = 8.9, 6.9 Hz), 4.23-4.32 *(m,* 3H), 4.37-4.43 *(m,* 3H), 4.75 (s, 2H), 5.39 (s, 2H), 5.97 (t, 1H, *J* = 6.1 Hz), 7.30-7.43 *(m,* 6H), 7.51-7.54 *(m,* 1H), 7.72-7.78 *(m,* 3H), 7.88 (d, 2H *J* = 7.5 Hz), 8.12 (d, 2H, *J* = 7.4 Hz), 10.10 *(s,* 1H).

### Step 6: 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]prop-1-ynyl]phenyl]carbamoyl]-4-ureido-butyl]carbamoyl]-2-methyl-propyl]carbamate

To a solution of 9H-fluoren-9-ylmethyl N-[(1S)-1-[[(1S)-1-[[4-[[tert-butyl(dimethyl)silyl] oxymethyl]-3-[3-[2-[2-[2-[2-[2-[2-[2-(2-methoxyethoxy)ethoxy]ethoxy]ethoxy]ethoxy]ethoxy] ethoxy]ethoxy]prop-1-ynyl]phenyl]carbamoyl]-4-ureido-butyl]carbamoyl]-2-methylpropyl]carbamate (452 mg; 0.40 mmol) in tetrahydrofuran (0.60 mL) and water (0.90 mL), was added acetic acid (4.17 mL; 72.78 mmol). The reaction mixture was stirred for 22 hours at room temperature and the progress of the reaction was monitored by UPLC-MS. After concentration to dryness, the crude product was purified by C18 reverse phase prep-HPLC by direct deposit of the reaction mixture on the X-Bridge column ad using neutral method to afford the title compound (327 mg, 0.32 mmol) as a white gum. ¹H NMR (DMSO): δ 0.87 (dd, 6H, *J* = 11.7, 6.8 Hz), 1.36-1.45 *(m,* 2H), 1.58-1.71 *(m,* 2H), 1.97-2.00 *(m,* 1H), 2.93-3.02 *(m,* 2H), 3.23 (s, 3H), 3.31 (s, 5H), 3.40-3.43 *(m,* 2H), 3.48-3.53 *(m,* 21H), 3.54-3.64 *(m,* 6H), 3.91-3.95 *(m,* 1H), 4.23-4.42 *(m,* 4H), 4.56 (d, 2H, *J* = 5.5 Hz), 5.19 (t, 1H, *J* = 5.6 Hz), 5.39 (s, 2H), 5.96 (t, 1H, *J =* 5.8 Hz), 7.30-7.34 *(m,* 2H), 7.39-7.43 *(m,* 4H), 7.50-7.52 *(m,* 1H), 7.72-7.76 (s, 3H), 7.88 (d, 1H *J* = 7.5 Hz), 8.12 (d, 2H, *J* = 7.4 Hz), 10.06 *(s,* 1H).

### Step 7: 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]propyl]phenyl] carbamoyl]-4-ureido-butyl]carbamoyl]-2-methyl-propyl]carbamate

To a solution of 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]prop-1-ynyl]phenyl]carbamoyl]-4-ureido-butyl]carbamoyl]-2-methyl-propyl]carbamate (327.0 mg; 0.32 mmol) in THF (3.7 mL), was added acetic acid (0.37 mL). After 3 purges with argon, Pt/C 5% (195 mg) was added and after 3 more purges with argon, the reaction mixture was placed under hydrogen atmosphere and stirred for 18 hours at room temperature and the progress of the reaction was monitored by UPLC-MS. The mixture was filtered through PTFE and the filtrate was concentrated to dryness, then triturated in dichloromethane/pentane (1/4 mixture, 50 mL). The precipitate was recovered by filtration to afford, after drying, the title compound (130 mg; 0.13 mmol) as a white solid. ¹H NMR (DMSO): δ 0.85-0.89 *(m,* 6H), 1.23-1.46 *(m,* 2H), 1.56-1.76 *(m,* 4H), 1.97-2.02 *(m,* 1H), 2.56-*2.60 (m,* 2H), 2.91-3.04 *(m,* 2H), 3.23 (s, 3H), 3.38-3.43 *(m,* 4H), 3.48-3.54 *(m,* 30H), 3.93 *(dd,* 1H, *J =* 8.9, 6.9 Hz), 4.21-4.31 *(m,* 3H), 4.38-4.41 *(m,* 1H), 4.45 (d, 2H, *J* = 5.3 Hz), 4.94 *(t,* 1H, *J = 5.3* Hz), 5.37 (s, 2H), 5.95 *(t,* 1H, *J* = 5.8 Hz), 7.25 (d, 1H, *J =* 8.3 Hz), 7.30-7.34 *(m,* 2H), 7.39-7.43 (s, 5H), 7.72-7.76 *(m,* 2H), 7.88 (d, 1H *J =* 7.5 Hz), 8.06 (d, 2H, *J* = 7.6 Hz), 9.88 (s, 1H). UPLC-MS: MS (ESI) m/z [M+H]+ = 1026.52

### Step 8: 9H-fluoren-9-ylmethyl N-[(1S)-1-[[(1S)-1-[[4-(bromomethyl)-3-[3-[2-[2-[2-[2-[2-[2-[2-(2-methoxyethoxy)ethoxy]ethoxy]ethoxy]ethoxy]ethoxy]ethoxy]ethoxy]propyl]phenyl] carbamoyl]-4-ureido-butyl]carbamoyl]-2-methyl-propyl]carbamate

To a solution of 9H-fluoren-9-ylmethyl N-[(1S)-1-[[(1S)-1-[[4-(hydroxymethyl)-3-[3-[2-[2-[2-[2-[2-[2-[2-(2-methoxyethoxy)ethoxy]ethoxy]ethoxy]ethoxy]ethoxy]ethoxy]ethoxy]propyl] phenyl]carbamoyl]-4-ureido-butyl]carbamoyl]-2-methyl-propyl]carbamate (60 mg; 0.0584 mmol) in THF (6.6 mL), was added dropwise at 0°C PBr₃ (1M solution in THF) (0.0877 mL; 0.0877 mmol). The solution was then stirred 3 hours at room temperature. The progress of the reaction was monitored by UPLC-MS after addition in an aliquot morpholine to react the bromo expected compound. The reaction was worked up with an aqueous saturated NH₄Cl solution (50 µL). After 5 minutes the mixture was dryed over MgSO₄, filtered and washed with THF (2 ml) to afford the bromo title compound as a THF solution used crude in the next step.

### Step 9: (2R)-2-[5-[3-chloro-4-[2-[4-[[4-[[(2S)-2-[[(2S)-2-(9H-fluoren-9-ylmethoxycarbonylamino)-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy]ethoxy]ethoxy]ethoxy]propyl] phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate

To the THF solution of 9H-fluoren-9-ylmethyl N-[(1S)-1-[[(1S)-1-[[4-(bromomethyl)-3-[3-[2-[2-[2-[2-[2-[2-[2-(2-methoxyethoxy)ethoxy]ethoxy]ethoxy]ethoxy]ethoxy]ethoxy]ethoxy]propyl] phenyl]carbamoyl]-4-ureido-butyl]carbamoyl]-2-methyl-propyl]carbamate from the previous step (0.0584 mmol), were successively added DMF (1.5 mL), (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid P1 (46.1 mg; 0.0527 mmol) and DIPEA (0.173 mL; 0.995 mmol). The reaction mixture was stirred 20 hours at room temperature and the progress of the reaction was monitored by UPLC-MS. The crude mixture containing the expected title compound and the Fmoc-deprotected one (expected in step 10) is used in the deprotective next step. UPLC-MS: MS (ESI) m/z [M-Fmoc+H+H]+ = 1660.99

### Step 10: (2R)-2-[5-[4-[2-[4-[[4-[[(2S)-2-[[(2S)-2-amino-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy] ethoxy]ethoxy]ethoxy]propyl]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl) pyrimidin-4-yl]methoxy]phenyl]propanoic acid; 2,2,2-trifluoroacetate;2,2,2-trifluoroacetic acid

To the crude mixture obtained in the previous step in DMF was added piperidine (11.6 µL; 0.117 mmol). The reaction mixture was stirred at room temperature for 15 hours and the progress of the reaction was monitored by UPLC-MS. After completion of the reaction, the crude product was purified by C₁₈ reverse phase prep-HPLC by direct deposit of the reaction mixture on the X-Bridge column in using the TFA method to give the title compound (29.2 mg; 0.0155 mmol) as a white powder. IR: 3600-2300, 1672, 1602, 1541+1516. HR-ESI+: m/z [M-CF₃COO]+ = 1660.7574 (1660.7575 theoretical)

### Step 11: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[3-[2-[2-[2-[2-[2-[2-[2-(2-methoxyethoxy)ethoxy]ethoxy]ethoxy]ethoxy]ethoxy]ethoxy]ethoxy]propyl] phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate;2,2,2-trifluoroacetate L26-P1

To a solution of (2R)-2-[5-[4-[2-[4-[[4-[[(2S)-2-[[(2S)-2-amino-3-methyl-butanoyl]amino]-5-ureido-pentanoyl]amino]-2-[3-[2-[2-[2-[2-[2-[2-[2-(2-methoxyethoxy)ethoxy]ethoxy]ethoxy] ethoxy]ethoxy]ethoxy]ethoxy]propyl]phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid; 2,2,2-trifluoroacetate;2,2,2-trifluoroacetic acid (42.5 mg; 0.0225 mmol) in DMF (1.28 mL), were successively added a solution of (2,5-dioxopyrrolidin-1-yl) 3-[2-(2,5-dioxopyrrol-1-yl)ethoxy]propanoate (Brodpharm 21854) (7.71 mg; 0.0247 mmol) and DIPEA (19.6 µL; 0.112 mmol). The reaction mixture was stirred at room temperature for 15 hours and the progress of the reaction was monitored by UPLC-MS. The crude product was purified by C₁₈ reverse phase prep-HPLC by direct deposit of the reaction mixture on the X-Bridge column and using the TFA method to afford **L26-P1** (28 mg; 0.0151 mmol) as a white powder. HR-ESI+: m/z [M-CF₃COO]+ = 1855.8105 (1855.8106 theoretical)

### Preparation of L27-P1:

### (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-3-methyl-5-ureido-pentanoyl]amino]-2-sulfo-phenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

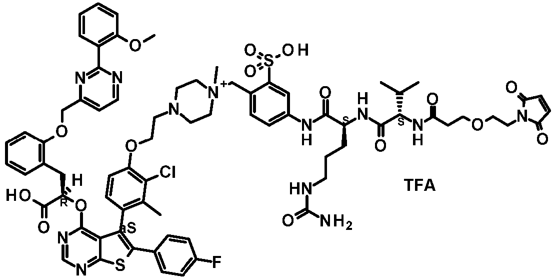

### Step 1: 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate

5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzenesulfonic acid (300 mg; 0.4263 mmol) was dissolved in anhydrous NMP (6 mL) at room temperature. In parallel, a solution of SOCl₂ (206 µL) in NMP (6 mL) was prepared. To the reaction, were added 6 times over a 75minutes period, a solution 900 µL of the SOCl₂ solution. After the last addition, the reaction mixture was stirred at room temperature for 15minutes. The crude product was purified by direct deposit of the reaction mixture on an Oasis column in using the TFA method to afford the title compound (138 mg; 0.1971 mmol) as a white powder. ¹H NMR (400 MHz, dmso-d6) δ ppm 10.15+8.1+7.42+6.0 (s+2d+m, 4H), 7.9 (m,HH), 7.75 (m, 3H), 7.42+7.31 (2m, 5H), 5.23 (s, 2H), 4.4 (m, 1H), 4.3-4.2 (m, 3H), 3.95 (dd, 1H), 3.0 (m, 2H), 2.0 (m, 1H), 1.7 + 1.6 (2m, 2H), 1.48 + 1.37 (2m, 2H), 0.88 (2d, 6H). HR-ESI+ : m/z [M+H]+ = 700.2199 / 700.2202 [measured/theoretical]

### Step 2: 5-[[(2S)-2-[[(2S)-2-amino-3-methyl-butanoyl]amino]-5-ureido-pentanoyl]amino]-2-[[4-[2-[2-chloro-4-[6-(4-fluorophenyl)-4-[(1R)-2-methoxy-1-[[2-[[2-(2-methoxyphenyl) pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]benzenesulfonic acid

To a solution of 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate (82.4 mg; 0.1177 mmol) in anhydrous NMP (2.5 mL), was added at room temperature DIEA (94 µL; 0.540 mmol) followed by methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate (60 mg; 0.067 mmol) and TBAI (12.4 mg; 0.034 mmol). The reaction was stirred at 80°C for 4 hours and overnight at room temperature. Then, 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonate was again added (14 mg; 0,017 mmol) followed by TBAI (17 µL; 0.0337 mmol) and the reaction was stirred at 80°C for 4 hours and then overnight at room temperature. The Fmoc deprotection step was realized in adding DEA (53 µL; 0.515 mmol) to the reaction and stirring at room temperature overnight. Purification was realized by direct injection of the mixture on Oasis eluted with a gradient of a solution A :H₂O/CH₃CN/NH₄HCO₃ (1960 ml/40/3.16 g) to a solution B: CH₃CN/H₂O/NH₄HCO₃ (1600 ml/400 ml/3.16 g) to afford the title compound (17 mg; 0.009 mmol). UPLC-MS: MS (ESI) m/z [M]+ = 1329

### Step 3 : (2R)-2-[5-[4-[2-[4-[[4-[[(2S)-2-[[(2S)-2-amino-3-methyl-butanoyl]amino]-5-ureido-pentanoyl]amino]-2-sulfo-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

To a mixture of 5-[[(2S)-2-[[(2S)-2-amino-3-methyl-butanoyl]amino]-5-ureido-pentanoyl] amino]-2-[[4-[2-[2-chloro-4-[6-(4-fluorophenyl)-4-[(1R)-2-methoxy-1-[[2-[[2-(2-methoxyphenyl)pyrimidin-4-yl]methoxy]phenyl]methyl]-2-oxo-ethoxy]thieno[2,3-d]pyrimidin-5-yl]-3-methyl-phenoxy]ethyl]-1-methyl-piperazin-1-ium-1-yl]methyl]benzenesulfonic acid (18 mg; 0.014 mmol) in dioxane/water (1 mL/1 mL) was added LiOH·H₂O (2.3 mg; 0.054 mmol) and the reaction was stirred at room temperature for 4 hours. The solution was adjusted to pH 6-7 by addition of HCl 1N and dioxane was evaporated under reduced pressure. Purification was realized by direct injection of the mixture on Oasis eluted with a gradient of a solution A: H₂O/CH₃CN/NH₄HCO₃ (1960 ml/40/3.16 g) to a solution B: CH₃CN/H₂O/NH₄HCO₃ (1600 ml/400 ml/3.16 g) to afford the title compound compound (11mg; 0.008 mmol).

### Step 4: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-3-methyl-5-ureido-pentanoyl]amino]-2-sulfo-phenyl]methyl]-4-methyl-piperazin-4-ium- 1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid L27-P1

To a solution of (2R)-2-[5-[4-[2-[4-[[4-[[(2S)-2-[[(2S)-2-amino-3-methyl-butanoyl]amino]-5-ureido-pentanoyl]amino]-2-sulfo-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (10.5 mg; 0.007 mmol) in DMF (0.4 mL), was added (2,5-dioxopyrrolidin-1-yl) 3-[2-(2,5-dioxopyrrol-1-yl)ethoxy]propanoate (5.7 mg; 0.018 mmol) and the solution was stirred at room temperature for 4 hours.The solution was purified by X-Bridge column C₁₈ by direct deposit of the reaction mixture on the column and in using the TFA method to afford L27-P1 (10 mg; 0.006 mmol). HR-ESI+: [M+H]+ 1511.5018 / 1511.5002 [measured/theoretical]

### Preparation of L28-P1:

### (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]-2-sulfophenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate

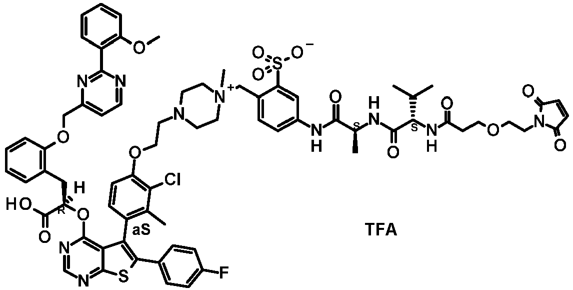

### Step 1: 9H-fluoren-9-ylmethyl N-[(1S)-1-[[(1S)-2-[4-(chloromethyl)-3-methyl-anilino]-1-methyl-2-oxo-ethyl]carbamoyl]-2-methyl-propyl]carbamate

To a solution of 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate (504.1 mg; 0.816 mmol) in NMP (5 mL), were added 6 times over a 75 minutes period, a solution of SOCl₂ (60 µL; 0.816 mmol) in NMP (500µL). The reaction mixture was stirred at room temperature for 15 minutes. The crude product was purified by direct deposit of the reaction mixture on an Oasis column in using the TFA method to afford (337 mg) as a a mixture of 70% the title compound (384 mmol) and 30% of the starting material (170 mmol) as a white powder. IR (cm⁻¹): 3600 to 2400, 1688+1648, 1599, 1518, 1022. UPLC-MS: MS (ESI) m/z [M+H]+ = 614.17+616.18 (Cl)

### Step 2: (2R)-2-[5-[4-[2-[4-[[4-[[(2S)-2-[[(2S)-2-amino-3-methyl-butanoyl]amino]propanoyl]amino]-2-methyl-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate

To a solution of methyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoate (152 mg; 0.171 mmol) in NMP (4.5 ml), were successively added 9H-fluoren-9-ylmethyl N-[(1S)-1-[[(1S)-2-[4-(chloromethyl)-3-methyl-anilino]-1-methyl-2-oxo-ethyl]carbamoyl]-2-methyl-propyl]carbamate (150 mg; 0.171 mmol), DIPEA (238 µL; 1.37 mmol) and TBAI (76 mg; 0.205mmol). The reaction mixture was stirred at 80°C for 28 hours. The reaction mixture is cooled down to room temperature. A solution of LiOH.H₂O (13.7 mg, 0.342 mmol) in water (500 µL) is then added. The reaction mixture was stirred at room temperature for 48 hours. The crude product was purified by C₁₈ reverse phase prep-HPLC by direct deposit of the reaction mixture on the X-Bridge column and using the TFA method to afford the title compound (40 mg; 0.0325 mmol) as a white powder. UPLC-MS: MS (ESI) m/z [M]+ = 1230.61+1232.61 (Cl)

### Step 3: (2R)-2-[5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]propanoyl]amino]-2-sulfophenyl]methyl]-4-methyl-piperazin-4-ium-1-yl]ethoxy]-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate L28-P1

To a solution of (2R)-2-[5-[4-[2-[4-[[4-[[(2S)-2-[[(2S)-2-amino-3-methyl-butanoyl]amino]propanoyl]amino]-2-methyl-phenyl]methyl]-4-methyl-piperazin-4-ium-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid;2,2,2-trifluoroacetate (6.0 mg; 0.0049 mmol) in solution in DMF (180 µL), were successively added (2,5-dioxopyrrolidin-1-yl) 3-[2-[2-(2,5-dioxopyrrol-1-yl)ethoxy]ethylcarbamoyl]oxetane-3-carboxylate (2.3 mg; 0.0073 mmol) and DIPEA (3.0 µL; 0.017 mmol). The reaction mixture was stirred at room temperature for 1.5 hours and was monitored by UPLC-MS. The crude product was purified by direct deposit of the reaction mixture on the X-Bridge column in using the TFA method to afford L28-P1 (2.9 mg; 0.0020 mmol) as a white powder. HR-ESI+: m/z [M+H]+ = 1425.4534/ 1425.4527 [measured/theoretical]

### Preparation of L29-C3:

### (2R)-2-[5-[4-[2-[4-[[4-[[(2S)-2-[[(2S)-2-[[2-(2-azidoethoxy)acetyl]amino]-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid

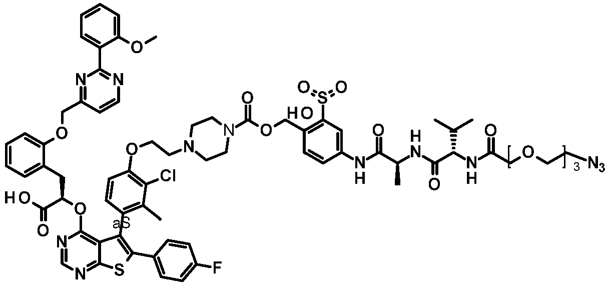

### Step 1: sodium; 5-[[(2S)-2-(tert-butoxycarbonylamino)propanoyl]amino]-2-(hydroxymethyl)benzenesulfonate

To a solution of Boc-L-Ala-OH (588 mg; 3.11 mmol) in DMF (38.6 mL), were successively added HATU (1.77 g; 4.67 mmol), sodium 5-amino-2-(hydroxymethyl)benzenesulfonate (771 mg; 3.42 mmol) and DIPEA (1.29 mL; 7.78 mmol). The reaction mixture was stirred for 16 hours at room temperature then concentrated to dryness and co-evaporated with water to afford the crude reaction mixture. The resulting residue was purified by column chromatography on silica gel using ethyl acetate/methanol 95:5 to 80:20 as eluent to afford the title compound (1.17 g; 2.95 mmol) as a white solid. ¹H NMR (DMSO): δ 1.24 (s, 9H), 1.38 *(m,* 3H), 4.05-1.44 *(m,* 1H), 4.73 (d, 2H, *J* = 4.8 Hz), 5.04 *(t,* 1H, *J* = 5.6 Hz); 6.97-7.02 *(m,* 1H), 7.33 (d, 1H, *J* = 8 Hz), 7.65-7.70 *(m,* 1H), 7.83 (s, 1H), 9.91 (s, 1H).

### Step 2: 5-[[(2S)-2-aminopropanoyl]amino]-2-(hydroxymethyl)benzenesulfonate, hydrochloride

Sodium 5-[[(2S)-2-(tert-butoxycarbonylamino)propanoyl]amino]-2-(hydroxymethyl) benzenesulfonate (1.17 g; 2.95 mmol; 1 eq.) was suspended in a solution of HCl 4N in dioxane (10 mL). The mixture was stirred at room temperature for 2 hours then concentrated to dryness to afford the crude mixture (982 mg; 2.95 mmol) as a white solid. ¹H NMR (DMSO): δ 1.45 (d, 3H, *J* = 5.6 Hz), 3.91-4.0 *(m,* 1H), 4.76 (s, 2H), 7.41 (d, 1H, *J* = 7.6 Hz), 7.66 (d, 1H, *J* = 7.6 Hz), 7.85 (s, 1H), 8.17 *(s,* 2H), 10.44 *(s,* 1H).

### Step 3: 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate

To a solution of 5-[[(2S)-2-aminopropanoyl]amino]-2-(hydroxymethyl)benzenesulfonate, hydrochloride (981 mg; 2.95 mmol) in DMF (34.5 mL) were added Fmoc-L-Val-OSu (1.29 g; 2.95 mmol; 1 eq.) and DIPEA (975 µL; 5.9 mmol). The mixture was stirred overnight at room temperature then concentrated to dryness and co evaporated with water to afford the crude mixture. The resulting residue was purified by column chromatography on silica gel using ethyl acetate /methanol 95:5 to 80:20 as eluent to afford the title compound (1.28 g; 2.072 mmol) as a colorless oil. ¹H NMR (DMSO): δ 0.80-0.92 *(m,* 6H), 1.30 (d, 3H, *J* = 6.4 Hz), 2.02-2.10 *(m,* 1H), 4.17-4.31 *(m,* 3H), 4.37-4.44 *(m,* 1H), 4.73 (d, 2H, *J* = 5.6 Hz), 5.04 *(t,* 1H, *J* = 6.4 Hz), 7.28-7.36 *(m,* 3H), 7.37-7.47 *(m,* 3H), 7.66 (d, 1H, *J* = 8.4 Hz), 7.71-7.77 *(m,* 2H), 7.83-7.85 *(m,* 1H), 7.88 (d, 2H, *J* = 7.6 Hz), 8.14 (d, 1H, *J* = 6.4 Hz), 9.99 (s, 1H).

### Step 4: 5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]benzenesulfonic acid

To a suspension of 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate (1.28 g; 2.07 mmol) in THF (65 mL), were added pyridine (875 µL; 10.8 mmol), followed by 4-nitrophenyl chloroformate (1.09 g; 5.41 mmol). The mixture was stirred overnight at room temperature. Then additional 4-nitrophenyl chloroformate (1.09 g; 5.41 mmol; 2.5 eq.) was added. After 5 hours stirring at room temperature, the mixture was concentrated to dryness then purified by column chromatography on C₁₈ using water/acetonitrile 90/10 to 0/100 as eluent in 30 minutes. Acetonitrile of the combined tubes was removed, and the rest was lyophilized to afford the title compound (650 mg; 0.83 mmol) as a white solid. ¹H NMR (DMSO): δ 0.88 *(m,* 6H), 1.31 (d, 3H, *J* = 4.8 Hz), 1.97-2.03 *(m,* 1H), 3.92 (t, 1H, *J* = 6.8 Hz), 4.23 (s, 2H), 4.24-4.34 *(m,* 1H), 4.42 *(t,* 1H *J* = 5.6 Hz), 5.69 (s, 2H), 7.30-7.48 *(m,* 6H), 7.62 (d, 2H, *J* = 8 Hz), 7.72-7.76 *(m,* 3H), 7.89 (d, 2H, *J= 6.4* Hz), 7.94 (s, 1H), 8.18 (d, 1H, *J* = 5.6 Hz), 8.33 (d, 2H, *J* = 7.6 Hz), 10.11 (s, 1H). ¹³C NMR (DMSO): δ 18.01, 18.26, 19.21, 30.4, 46.66, 49.05, 59.91, 65.67, 67.82, 117.7, 119.1, 120.06, 122.66, 125.37, 126.33, 127.05, 127.62, 128.0, 138.06, 140.67, 143.77, 143.86, 145.1, 146.23, 151.96, 155.47, 156.12, 171.0, 171.15. LCMS (2-100 ACN/H₂O+0.05% TFA): 90.41 % Tr = 12.7 min. Positiv mode 578.41 detected. Negativ mode 759.17 detected

### Step 5: (2S)-2-[[5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-sulfophenyl]methoxycarbonyl]piperazin-1-yl]ethoxy]-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid

To a suspension of (2S)-2-[[5-[3-chloro-2-methyl-4-(2-piperazin-1-ylethoxy)phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid;2,2,2-trifluoroacetic acid (178.4 mg; 0.183 mmol) in DMF (1.5 mL), were successively added 5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl]benzenesulfonic acid (150 mg; 0.192 mmol) and DIPEA (91 µL; 0.549 mmol), The mixture was stirred overnight at room temperature for 15 minutes. The crude product was purified by direct deposit of the reaction mixture on the X-Bridge column in using the NH₄HCO₃ method to afford the title compound (176 mg, 0.118 mmol) as a white solid. UPLC-MS: MS (ESI) m/z [M+H]+ = 1482.15+1484.56(CI)

### Step 6: (2S)-2-[[5-[4-[2-[4-[[4-[[(2S)-2-[[(2S)-2-amino-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid

To a solution of (2S)-2-[[5-[3-chloro-4-[2-[4-[[4-[[(2S)-2-[[(2S)-2-(9H-fluoren-9-ylmethoxycarbonylamino)-3-methyl-butanoyl]amino]propanoyl]amino]-2-sulfophenyl]methoxycarbonyl]piperazin-1-yl]ethoxy]-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid (176 mg; 0.118 mmol) in DMF (1.0 mL), was added piperidine (24.17 µL; 0.237 mmol). The reaction mixture was stirred at room temperature for 18 hours. The crude product was purified by direct deposit of the reaction mixture on a X-Bridge column in using the NH₄HCO₃ method to afford the title compound (102 mg; 0.0809 mmol) as a white powder. IR (cm⁻¹): 3620-2680, 1683, 1235. UPLC-MS: MS (ESI) m/z [M+H]+ = 1260.37+1262.37(CI). ¹H NMR (400 MHz, dmso-d6) δ ppm 10.20 (m, NH), 8.90 (m, 2H), 8.90 (m, 1H), 8.60 (m, NH), 8.60 (s, 1H), 7.90 (m, 1H), 7.78 (m, 1H), 7.70 (d, 1H), 7.55 (d, 1H), 7.48 (t, 1H), 7.45 (d, 1H), 7.3/7.2 (m, 4H), 7.20 (m, 1H), 7.20 (d, 1H), 7.15 (t, 1H), 7.15 (d, 1H), 7.05 (t, 1H), 7.00 (d, 1H), 6.7 (t, 1H), 6.2 (d, 1H), 5.48 (s, 2H), 5.50 (m, 1H), 5.23 (s, 2H), 4.50 (m, 1H), 4.25 (m, 2H), 3.75 (s, 3H), 3.4 (m, 4H), 3.40 (m, 1H), 3.35 (m, 1H) 2.80 (m, 2H), 2.5 (m, 4H), 2.50 (m, 1H), 2.05 (m, 1H), 1.80 (s, 3H), 1.30 (d, 3H), 0.90 (dd, 6H)

### Step 7: (2,3,4,5,6-pentafluorophenyl) 2-[2-[2-(2-azidoethoxy)ethoxy]ethoxy]acetate

To a solution of 2-[2-[2-(2-azidoethoxy)ethoxy]ethoxy]acetic acid (74 mg; 0.317 mmol) in THF (500 µL) were succesively added 2,3,4,5,6-pentafluorophenol (70 mg; 0.380 mmol) in solution in THF (500 µL) and DCC (78.5 mg; 0.380 mmol) in solution in THF (500 µL). The reaction mixture was stirred at room temperature for 18 hours. The crude suspension is filtred through a coton pad in a pasteur pipette and the solution is used without further work-up in step 8.

### Step 8: (2R)-2-[5-[4-[2-[4-[[4-[[(2S)-2-[[(2S)-2-[[2-(2-azidoethoxy)acetyl]amino]-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid L29-C3

To a solution of (2S)-2-[[5-[4-[2-[4-[[4-[[(2S)-2-[[(2S)-2-amino-3-methyl-butanoyl]amino]propanoyl]amino]-2-sulfo-phenyl]methoxycarbonyl]piperazin-1-yl]ethoxy]-3-chloro-2-methyl-phenyl]-6-(4-fluorophenyl)thieno[2,3-d]pyrimidin-4-yl]methyl]-3-[2-[[2-(2-methoxyphenyl)pyrimidin-4-yl]methoxy]phenyl]propanoic acid (100 mg; 0.0793 mmol) in DMF (500 µL), were succesively added the solution of (2,3,4,5,6-pentafluorophenyl) 2-[2-[2-(2-azidoethoxy)ethoxy]ethoxy]acetate in THF from step 7 (0.317 mmol) and DIPEA (39.3 µL; 0.238 mmol). The reaction mixture was stirred at room temperature for 15 minutes. The crude product was purified by direct deposit of the reaction mixture on a X-Bridge column in using the NH₄HCO₃ method to afford **L29-C3** (43 mg, 0.0291 mmol) as a white powder. IR (cm⁻¹): 3257, 2102, 1663, 1236/1082, 834/756. ¹H NMR (400 MHz, dmso-d6) δ ppm 10.03 (s, NH), 8.87 (d, 1H), 8.59 (sNC, 1H), 8.35 (d, NH), 7.89 (df, 1H), 7.69 (dd, 1H), 7.66 (m, 1H), 7.53 (d, 1H), 7.45 (t, 1H), 7.36 (d, 1H), 7.29 (dd, 2H), 7.21 (d, 1H), 7.20 (t, 2H), 7.19 (d, 1H), 7.14 (d, 1H), 7.13 (m, 1H), 7.09 (m, NH), 7.03 (t, 1H), 7.00 (d, 1H), 6.72 (t, 1H), 6.24 (d, 1H), 5.48 (dNC, 1H), 5.44 (s, 2H), 5.23(AB, 2H), 4.40 (t, 1H), 4.30 (dd, 1H), 4.24 (m, 2H), 3.94 (s, 2H), 3.75 (s, 3H), 3.58 (m, 10H), 3.38 (m, 4H), 3.36 (t, 2H), 3.30 (NC, 1H), 2.75 (t, 2H), 2.50 (m, 4H), 2.50 (m, 1H), 2.00 (m, 1H), 1.51 (s, 3H), 1.30 (d, 3H), 0.88/0.82 (2d, 6H). ¹³C NMR (100/126 MHz, dmso-d6) δ ppm 158.2, 131.6, 131.1, 130.9, 130.9, 130.9, 130.9, 128.3, 126.7, 120.7, 120.4, 119.3, 117.9, 116.2, 112.5, 112.1, 110.8, 70.2, 70.2; 69.6, 67.7, 64.0, 56.7, 56.5, 55.8, 53.2, 50.4, 49.2, 43.8, 31.7, 19.7, 18.7, 18.4, 17.8. ¹⁹F NMR (376/470 MHz, dmso-d6) δ ppm -112.0. HR-ESI+: m/z [M+H]+ = 1475.4643/1475.4638; [2M+H+Ca]3+ = 996.6289/996.6273; [M+2H]2+ = 738.2378/738.2355; [M+H+Na]2+ = 749.2255/749.2265.

### Example 2. Synthesis and Characterization of Additional Linkers, Linker-Payloads, and Precursors thereof.

Exemplary linkers, linker-payloads, and precursors thereof were synthesized using exemplary methods described in this example.

### Synthesis of 2-(bromomethyl)-4-nitrobenzoic acid

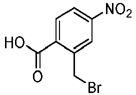

To a stirred solution of 2-methyl-4-nitrobenzoic acid (300 g, 1.5371 mol) in CCl₄ (3000 mL) was added NBS (300.93 g, 1.6908 mol) and AIBN (37.86 g, 0.2305 mol) at room temperature. The reaction mixture was stirred at 80°C for 16 h. Reaction mixture was monitored by TLC analysis. The reaction mixture was diluted with sat. NaHCO₃ solution (2 L) and extracted with ethyl acetate (2 x 2 L). The combined organic layer was dried over anhydrous sodium sulphate and concentrated under reduced pressure. The crude compound was purified by column chromatography on silica gel using 2-3% of ethyl acetate in petroleum-ether as an eluent and 2-(bromomethyl)-4-nitrobenzoic acid was obtained. ¹H NMR (400 MHz, CDCl₃): δ 8.35 (d, J=2.0 Hz, 1H), 8.20 (q, J=8.8, 2.4 Hz, 1H), 8.12 (d, J=8.8 Hz, 1H), 4.97 (s, 2H), 4.00 (s, 3H).

### Synthesis of 4-nitro-2-((prop-2-yn-1-yloxy)methyl)benzoic acid

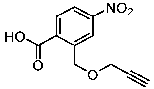

To the mixture of 2-(bromomethyl)-4-nitrobenzoic acid (250 g, 0.9122 mol) in ACN (5000 mL) was added prop-2-yn-1-ol (255.68 g, 265.50 mL, 4.5609 mol, d=0.963 g/mL) and Cs₂CO₃ (743.03 g, 2.2805 mol) at room temperature. The resulting mixture was heated to 80°C for 16 h. The reaction mixture was filtered through celite pad washed with ethyl acetate (2 L). The filtrate was concentrated under reduced pressure. The obtained crude compound was added sat. NaHCO₃ solution (1 L) and the aq layer was acidified to PH 2 by using 2N HCl (2 L). After filteration vacuum drying 4-nitro-2-((prop-2-yn-1-yloxy)methyl)benzoic acid was obtained. ¹H NMR (400 MHz, DMSO): δ 13.61 (brs, 1H), 8.37 (d, J=2.4 Hz, 1H), 8.23 (dd, J=2.4, 8.4 Hz, 1H), 8.10 (d, J=8.8 Hz, 1H), 4.95 (s, 2H), 4.37 (d, J=2.4 Hz, 2H), 3.52 (t, J=2.4 Hz, 1H)

### Synthesis of methyl 4-nitro-2-((prop-2-yn-1-yloxy)methyl)benzoate

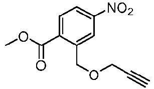

To a stirred solution of 4-nitro-2-((prop-2-yn-1-yloxy)methyl)benzoic acid (130 g, 0.5527 mol) in MeOH (1300 mL) was added SOCl₂ (526.08 g, 320.78 mL, 4.4219 mol, d=1.64 g/mL) slowly at 0°C. The reaction stirred at 70°C for 4 h. The reaction solvent was evaporated under reduced pressure. The obtained residue was dissolved in ethyl acetate (1000 mL) and washed with sat. NaHCO₃ (600 mL), water (500 mL) and brine solution (500 mL). The separated organic layer was dried over sodium sulphate, filtered and evaporated under reduced pressure to yield methyl 4-nitro-2-((prop-2-yn-1-yloxy)methyl)benzoate. ¹H NMR (400 MHz, CDCl₃): δ 8.56 (t, J=0.8 Hz, 1H), 8.18 - 8.09 (m, 2H), 5.03 (s, 2H), 4.35 (d, J=2.4 Hz, 2H), 3.96 (s, 3H), 2.49 (t, J=2.4 Hz, 1H).

### Synthesis of methyl 4-amino-2-((prop-2-yn-1-yloxy)methyl)benzoate

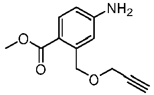

To a solution of methyl 4-nitro-2-((prop-2-yn-1-yloxy)methyl)benzoate (110 g, 0.4414 mol) in a mixture of EtOH (1100 mL) and H₂O (550 mL) was added Fe Powder (197.21 g, 3.5310 mol) and NH₄Cl (188.88 g, 3.5310 mol) at room temperature. The resulting mixture was heated at 80°C for 16 h. The reaction mixture was cooled to room temperature and filtered through celite^{®} and washed with ethyl acetate (2 L). The filtrate was concentrated under reduced pressure up to half of the volume. To the residue, ethyl acetate (1.5 L) was added and separated the two layers and the aqueous layer was extracted with ethyl acetate (2 L). The combined organic layer was dried over anhydrous sodium sulphate and concentrated under reduced pressure to obtain crude product. Purification by SiO₂ column chromatography (15-20% of ethyl acetate in petroleum-ether) yielded methyl 4-amino-2-((prop-2-yn-1-yloxy)methyl)benzoate. ¹H NMR (400 MHz, CDCl₃): δ 7.67 (d, J=8.8 Hz, 1H), 6.78 (t, J=1.6 Hz, 1H), 6.48 (q, J=8.4, 2.4 Hz, 1H), 4.79 (s, 2H), 4.25 (d, J=2.4 Hz, 2H), 3.70 (d, J=4.0 Hz, 3H), 3.42 (t, J=2.4 Hz, 1H).

### Synthesis of (4-amino-2-((prop-2-yn-1-yloxy)methyl)phenyl)methanol

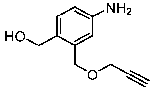

To a stirred solution of THF (1000 mL) was added LiAlH4 (1 M in THF) (21.23 g, 798.2 mmol, 798.2 mL) slowly at 0°C. A solution of methyl 4-amino-2-((prop-2-yn-1-yloxy)methyl)benzoate (70 g, 319.3 mmol) in THF (800 mL) was added slowly at 0°C. The reaction was stirred at room temperature for 4 h. The reaction mixture was cooled to 0°C, then was added water (22 mL) very slowly and followed by the addition of 20% NaOH (22 mL) and water (66 mL). The reaction mixture was stirred at 0°C for 30 min. Anhydrous sodium sulphate was added to absorb excess of water. The mixture was filtered through celite^{®}. The filter cake was washed with ethylacetate (1000 mL) and 10% MeOH/DCM (500 mL). The filtrate was concentrated under reduced pressure. The resulting crude compound was purified by SiO₂ column chromatography (35-40% of ethylacetate in petroleum-ether as an eluent) to give yield (4-amino-2-((prop-2-yn-1-yloxy)methyl)phenyl)methanol. ¹H NMR (400 MHz, CDCl₃): δ 6.98 (d, J=8.0 Hz, 1H), 6.56 (d, J=2.4 Hz, 1H), 6.43 (dd, J=2.4, 8.0 Hz, 1H), 4.98 (s, 2H), 4.64 (t, J=5.2 Hz, 1H), 4.47 (s, 2H), 4.34 (d, J=5.6 Hz, 2H), 4.15 (d, J=2.4 Hz, 2H), 3.46 (t, J=2.4 Hz, 1H).

### Synthesis of (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate

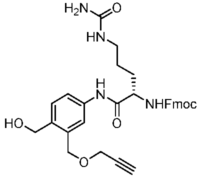

To a solution of (4-amino-2-((prop-2-yn-1-yloxy)methyl)phenyl)methanol (1.92 g, 10.04 mmoles, 1.0 equiv.), (9H-fluoren-9-yl)methyl (S)-(1-amino-1-oxo-5-ureidopentan-2-yl)carbamate (3.99 g, 10.04 mmoles, 1.0 equiv.), and (1-[bis(dimethylamino)methylene]-1H-1,2,3-triazolo[4,5-b]pyridinium 3-oxid hexafluorophosphate (4.20 g, 11.04 mmoles, 1.1 equiv.) in DMF (10 mL) was added N,N-diisopropylethylamine (2.62 mL, 15.06 mmoles, 1.5 equiv.). After stirring at ambient temperature for 1 h, the mixture was poured into water (200 mL). The resulting solids were filtered, rinsed with water, and dried under vacuum, and (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate was obtained . LCMS: MH+=571.5; Rt=0.93 min (2 min acidic method).

### Synthesis of (S)-2-amino-N-(4-(hydroxymethyl)-3-((prop-2-yn-1-yloxy)methyl)phenyl)-5-ureidopentanamide

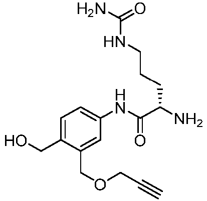

To (9H-fluoren-9-yl)methyl (S)-(1-((4-(hydroxymethyl)-3-((prop-2-yn-1-yloxy)methyl)phenyl)amino)-1-oxo-5-ureidopentan-2-yl)carbamate (6.08 g, 10.65 mmoles, 1.0 equiv.) was added dimethylamine (2 M in THF, 21.31 mL, 42.62 mmoles, 4 equiv.). After stirring at ambient temperature for 1.5 hours, the supernatant solution was decanted from the gum-like residue that had formed. The residue was triturated with ether (3 x 50 mL) and the resulting solids were filtered, washed with ether, and dried under vacuum. (S)-2-amino-N-(4-(hydroxymethyl)-3-((prop-2-yn-1-yloxy)methyl)phenyl)-5-ureidopentanamide was obtained. LCMS: MH+ 349.3; Rt=0.42 min (2 min acidic method).

### Synthesis of tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl)carbamate

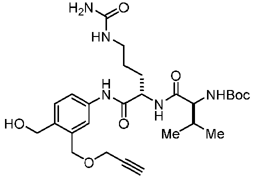

To a solution of (S)-2-amino-N-(4-(hydroxymethyl)-3-((prop-2-yn-1-yloxy)methyl)phenyl)-5-ureidopentanamide (3.50 g, 10.04 mmoles, 1.0 equiv.), (tert-butoxycarbonyl)-L-valine (2.62 g, 12.05 mmol, 1.2 equiv.), and (1-[bis(dimethylamino)methylene]-1H-1,2,3-triazolo[4,5-b]pyridinium 3-oxid hexafluorophosphate (4.58 g, 12.05 mmoles, 1.2 equiv.) in DMF (10 mL) was added N,N-diisopropylethylamine (3.50 mL, 20.08 mmoles, 2.0 equiv). After stirring at ambient temperature for 2 h, the mixture was poured into water (200 mL) and the resulting suspension was extracted with EtOAc (3x100 mL). The combined organic layers were dried over sodium sulfate and concentrated under vacuum. After purification by ISCO SiO₂ chromatography (0-20% methanol / dichloromethane), tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl)carbamate was obtained. ¹H NMR (400 MHz, DMSO-d6) δ 10.00 (s, 1H), 7.96 (d, J = 7.7 Hz, 1H), 7.55 (dq, J = 4.9, 2.2 Hz, 2H, aryl), 7.32 (d, J = 8.9 Hz, 1H, aryl), 6.76 (d, J = 8.9 Hz, 1H), 5.95 (t, J = 5.8 Hz, 1H), 5.38 (s, 2H), 5.01 (t, J = 5.5 Hz, 1H), 4.54 (s, 2H), 4.45 (dd, J = 25.2, 5.3 Hz, 3H), 4.20 (d, J = 2.4 Hz, 2H), 3.83 (dd, J = 8.9, 6.7 Hz, 1H), 3.49 (t, J = 2.4 Hz, 1H), 2.97 (dh, J = 26.0, 6.5 Hz, 2H), 1.96 (h, J = 6.6 Hz, 1H), 1.74 - 1.50 (m, 2H), 1.39 (m, 11H), 0.84 (dd, J = 16.2, 6.7 Hz, 6H). LCMS: MNa+ 570.5; Rt=0.79 min (2 min acidic method).

### Synthesis of tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate

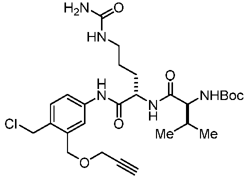

To a solution of tert-butyl ((S)-1-(((S)-1-((4-(hydroxymethyl)-3-((prop-2-yn-1-yloxy)methyl)phenyl)amino)-1-oxo-5-ureidopentan-2-yl)amino)-3-methyl-1-oxobutan-2-yl)carbamate (2.00 grams, 3.65 mmol, 1.0 equiv.) in acetonitrile (13.3 mL) at 0 °C was added thionyl chloride (0.53 mL, 7.30 mmol, 2.0 equiv). After stirring in the ice bath for one hour the solution was diluted with water (40 mL) and the resulting white precipitate was collected by filtration, air drying and drying under high vacuum to yield tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate. LCMS: MNa+ 588.5; Rt=2.17 min (5 min acidic method).

### Synthesis of 2-(((tert-butyldiphenylsilyl)oxy)methyl)-5-nitrobenzoic acid

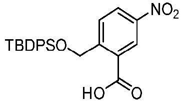

To a solution of 6-nitroisobenzofuran-1(3H)-one (90 g, 502.43 mmol, 1.00 eq) in MeOH (1000 mL) and KOH (28.19 g, 502.43 mmol, 1.00 eq) in H₂O (150 mL) was added. The brown mixture was stirred at 25°C for 1.5 h. The brown mixture was concentrated under reduced pressure to give a residue and dissolved in DCM (2000 mL). The mixture was added TBDPSCI (296.91 g, 1.08 mol, 277.49 mL, 2.15 eq) and IMIDAZOLE (171.03 g, 2.51 mol, 5.00 eq) stirred at 25°C for 12 h. The mixture was concentrated under reduced pressure to give a residue. The residue was purified by silica gel chromatography (Petroleum ether/Ethyl acetate=1/0, 1/1) and 2-(((tert-butyldiphenylsilyl)oxy)methyl)-5-nitrobenzoic acid was obtained as a white solid. ¹H NMR (400 MHz, METHANOL-d4) δ ppm 1.13 (s, 9 H) 5.26 (s, 2 H) 7.34 - 7.48 (m, 6 H) 7.68 (br d, J=8 Hz, 4 H) 8.24 (br d, J=8 Hz, 1 H) 8.46 (br d, J=8 Hz, 1 H) 8.74 (s, 1 H).

### Synthesis of (2-(((tert-butyldiphenylsilyl)oxy)methyl)-5-nitrophenyl)methanol

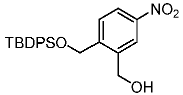

To a mixture of 2-(((tert-butyldiphenylsilyl)oxy)methyl)-5-nitrobenzoic acid (41 g, 94.14 mmol, 1 eq) in THF (205 mL) was added BH₃. THF (1 M, 470.68 mL, 5 eq). The yellow mixture was stirred at 60°C for 2h. The mixture was added MeOH (400mL), and concentrated under reduced pressure to give a residue. then addition of H₂O (200mL) and DCM(300mL), extracted with DCM (3 ×200 mL), washed with brine (300mL), dried over anhydrous MgSO₄, filtered, and concentrated under reduced pressure to give a residue. The residue was purified by silica gel chromatography (Petroleum ether/Ethyl acetate=1/0, 1/1). (2-(((tert-butyldiphenylsilyl)oxy)methyl)-5-nitrophenyl)methanol was obtained as a white solid. ¹H NMR (400 MHz, METHANOL-d4) δ ppm 1.10 (s, 9 H) 4.58 (s, 2 H) 4.89 (s, 2 H) 7.32 - 7.51 (m, 6 H) 7.68 (dd, J=8, 1.38 Hz, 4 H) 7.76 (d, J=8 Hz, 1 H) 8.15 (dd, J=8 2.26 Hz, 1 H) 8.30 (d, J=2 Hz, 1 H).

### Synthesis of 2-(((tert-butyldiphenylsilyl)oxy)methyl)-5-nitrobenzaldehyde

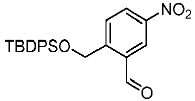

To a solution of (2-(((tert-butyldiphenylsilyl)oxy)methyl)-5-nitrophenyl)methanol (34 g, 80.65 mmol, 1 eq) in DCM (450 mL) was added MnO₂ (56.09 g, 645.22 mmol, 8 eq). The black mixture was stirred at 25°C for 36 h. The mixture was added MeOH (400mL), and concentrated under reduced pressure to give a residue. then addition of H₂O (200mL) and DCM (300mL), extracted with DCM (3 ×200 mL), washed with brine (300mL), dried over anhydrous MgSO₄, filtered, and concentrated under reduced pressure to give a residue. The residue was purified by silica gel chromatography (CH₂Cl₂=100%). 2-(((tert-butyldiphenylsilyl)oxy)methyl)-5-nitrobenzaldehyde was obtained as a white solid. ¹H NMR (400 MHz, CHLOROFORM-d) δ ppm 1.14 (s, 9 H) 5.26 (s, 2 H) 7.34 - 7.53 (m, 6 H) 7.60 - 7.73 (m, 4 H) 8.13 (d, J=8Hz, 1 H) 8.48 (dd, J=8, 2.51 Hz, 1 H) 8.67 (d, J=2 Hz, 1 H) 10.16 (s, 1 H).

### Synthesis of N-(2-(((tert-butyldiphenylsilyl)oxy)methyl)-5-nitrobenzyl)prop-2-yn-1-amine

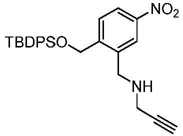

To a solution of 2-(((tert-butyldiphenylsilyl)oxy)methyl)-5-nitrobenzaldehyde (12.6 g, 30.03 mmol, 1 eq) in DCM (130 mL) was added prop-2-yn-1-amine (4.14 g, 75.08 mmol, 4.81 mL, 2.5 eq) and MgSO₄ (36.15 g, 300.33 mmol, 10 eq) then the suspension mixture was stirred at 25°C for 24hr. Taking a little reaction solution and treating with NaBH₄, the TLC showed one new point was formed. The reaction mixture was filtered and concentrated under reduced pressure to give a residue. (E)-N-[[2-[[tert-butyl(diphenyl)silyl]oxymethyl]-5-nitro-phenyl]methyl]prop-2-yn-1-imine was obtained as a yellow solid. ¹H NMR (400 MHz, CHLOROFORM-d) δ ppm 1.11 (s, 9 H) 2.48 (t, J=2.38 Hz, 1 H) 4.52 (t, J=2.13 Hz, 2 H) 5.09 (s, 2 H) 7.35 - 7.49 (m, 6 H) 7.63 - 7.72 (m, 4 H) 7.79 (d, J=8.53 Hz, 1 H) 8.25 (dd, J=8.53, 2.51 Hz, 1 H) 8.68 (d, J=2.26 Hz, 1 H) 8.84 (t, J=1.88 Hz, 1 H).

(E)-N-[[2-[[tert-butyl(diphenyl)silyl]oxymethyl]-5-nitro-phenyl]methyl]prop-2-yn-1-imine (12 g, 26.28 mmol, 1 eq) was dissolved in MeOH (100 mL) and THF (50 mL) , then NaBH₄ (1.49 g, 39.42 mmol, 1.5 eq) was added and the yellow mixture was stirred at -20°C for 2hr. LCMS showed desired compound was detected. The reaction mixture was quenched by addition MeOH (200 mL) at -20 °C, and then concentrated under reduced pressure to give a residue. The residue was dissolved with EtOAc (500 mL) washed with brine (150 mL), dried over anhydrous Na₂SO₄, filtered and concentrated under reduced pressure to give a residue. The residue was purified by flash silica gel chromatography ( Eluent of 0-10% Ethyl acetate/Petroleum ether gradient). N-(2-(((tert-butyldiphenylsilyl)oxy)methyl)-5-nitrobenzyl)prop-2-yn-1-amine was obtained as a pale yellow oil. ¹H NMR (400 MHz, CHLOROFORM-d) δ ppm 1.12 (s, 9 H) 2.13 (t, J=2.38 Hz, 1 H) 3.33 (d, J=2.51 Hz, 2 H) 3.80 (s, 2 H) 4.93 (s, 2 H) 7.36 - 7.49 (m, 6 H) 7.69 (dd, J=7.91, 1.38 Hz, 4 H) 7.77 (d, J=8.53 Hz, 1 H) 8.16 (dd, J=8.41, 2.38 Hz, 1 H) 8.24 (d, J=2.26 Hz, 1 H).

### Synthesis of (9H-fluoren-9-yl)methyl (2-(((tert-butyldiphenylsilyl)oxy)methyl)-5-nitrobenzyl)(prop-2-yn-1-yl)carbamate

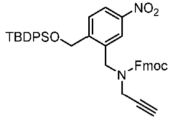

To a solution of N-(2-(((tert-butyldiphenylsilyl)oxy)methyl)-5-nitrobenzyl)prop-2-yn-1-amine (9 g, 19.62 mmol, 1 eq) and FMOC-OSU (7.28 g, 21.59 mmol, 1.1 eq) in dioxane (90 mL) was added sat. NaHCO₃ (90 mL) and the white suspension was stirred at 20°C for 12 h. The reaction mixture was diluted with H₂O (150 mL) and extracted with EtOAc (150 mL x 2). The combined organic layers were washed with brine (200 mL), dried over anhydrous Na₂SO₄, filtered and concentrated under reduced pressure to give a residue. The residue was purified by flash silica gel chromatography (Eluent of 0-30% Ethyl acetate/Petroleum ether). (9H-fluoren-9-yl)methyl (2-(((tert-butyldiphenylsilyl)oxy)methyl)-5-nitrobenzyl)(prop-2-yn-1-yl)carbamate (7.7 g, 11.08 mmol, 56.48% yield, 98% purity) was obtained as a white solid. ¹H NMR (400 MHz, CHLOROFORM-d) δ ppm 1.12 (s, 9 H) 2.17 (br d, J=14.31 Hz, 1 H) 3.87 - 4.97 (m, 9 H) 6.98 - 8.28 (m, 21 H).

### Synthesis of (9H-fluoren-9-yl)methyl (5-amino-2-(((tert-butyldiphenylsilyl)oxy)methyl)benzyl)(prop-2-yn-1-yl)carbamate

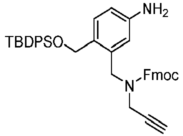

To an ice bath cooled solution of (9H-fluoren-9-yl)methyl (2-(((tert-butyldiphenylsilyl)oxy)methyl)-5-nitrobenzyl)(prop-2-yn-1-yl)carbamate (5.0 g, 7.34 mmoles, 1.0 equiv.) in 10% AcOH/CH₂Cl₂ (100 mL) was added Zn (7.20 g, 110 mmoles, 15 equiv.). The ice bath was removed, and the resulting mixture stirred for 2 hours at which time it was filtered through a pad of celite^{®}. The volatiles were removed in vacuo and the residue was dissolved in EtOAc, was washed with NaHCO₃(sat.), NaCl(sat.), dried over MgSO₄, filtered, concentrated and after ISCO SiO₂ chromatography (0-75% EtOAc/Heptanes) (9H-fluoren-9-yl)methyl (5-amino-2-(((tert-butyldiphenylsilyl)oxy)methyl)benzyl)(prop-2-yn-1-yl)carbamate was obtained. LCMS: MH+=651.6; Rt=3.77 min (5 min acidic method).

### Synthesis of (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate

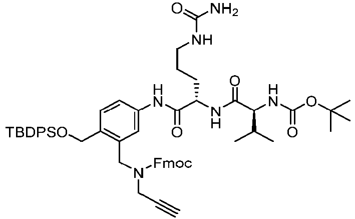

To (9H-fluoren-9-yl)methyl (5-amino-2-(((tert-butyldiphenylsilyl)oxy)methyl)benzyl)(prop-2-yn-1-yl)carbamate (2.99 g, 4.59 mmoles, 1.0 equiv) and (S)-2-((S)-2-((tert-butoxycarbonyl)amino)-3-methylbutanamido)-5-ureidopentanoic acid (1.72 g, 4.59 mmoles, 1.0 equiv.) in CH₂Cl₂ (40 mL) was added ethyl 2-ethoxyquinoline-1(2H)-carboxylate (2.27 g, 9.18 mmoles, 2.0 equiv.). After stirring for 10 min, MeOH (1 mL) was added and the solution became homogeneous. The reaction was stirred for 16 h, the volatiles were removed in vacuo and after purification by ISCO SiO₂ chromatography (0-15% MeOH/CH2Cl2) (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate was obtained. LCMS: MH+=1008.8; Rt=3.77 min (5 min acidic method).

### Synthesis of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate

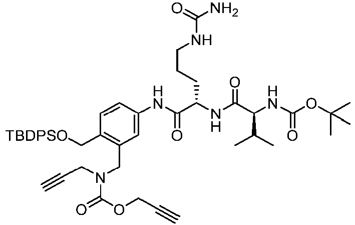

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate (1.60 g, 1.588 mmoles, 1.0 equiv.) was added 2M dimethylamine in MeOH (30 mL, 60 mmol, 37 equiv.) and THF (10 mL). After standing for 3 h, the volatiles were removed in vacuo and the residue was triturated with Et₂O to remove FMOC deprotection byproducts. To the resulting solid was added CH₂Cl₂ (16 mL) and pyridine (4 mL) and to the heterogeneous solution was added propargyl chloroformate (155 uL, 1.588 mmole, 1.0 equiv.). After stirring for 30 minutes additional propargyl chloroformate (155 uL, 1.588 mmole, 1.0 equiv.) was added. After stirring for an additional 20 min, MeOH (1 mL) was added to quench remaining chloroformate and the volatiles were removed in vacuo. Upon purification by ISCO SiO₂ chromatography (0-15% MeOH/CH₂Cl₂) prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate was obtained. LCMS: MH+=867.8; Rt=3.40 min (5 min acidic method).

### Synthesis of prop-2-yn-1-yl (5-((S)-2-((S)-2-((tert-butoxycarbonyl)amino)-3-methylbutanamido)-5-ureidopentanamido)-2-(hydroxymethyl)benzyl)(prop-2-yn-1-yl)carbamate

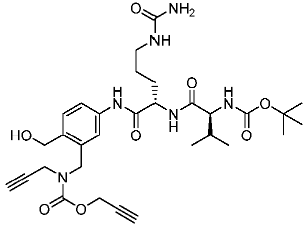

To a solution of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(prop-2-yn-1-yl)carbamate (984 mg, 1.135 mmoles, 1.0 equiv.) in THF (7.5 mL) was added 1.0 M tetrabutylammoniumn fluoride in THF (2.27 mL, 2.27 mmoles, 2.0 equiv.). After standing for 6 h, the volatiles were removed in vacuo, the residue was purified by ISCO SiO₂ chromatography (0-40% MeOH/CH2Cl2) and prop-2-yn-1-yl (5-((S)-2-((S)-2-((tert-butoxycarbonyl)amino)-3-methylbutanamido)-5-ureidopentanamido)-2-(hydroxymethyl)benzyl) (prop-2-yn-1-yl)carbamate was obtained. LCMS: MH+=629.6; Rt=1.74min (5 min acidic method).

### Synthesis of prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate

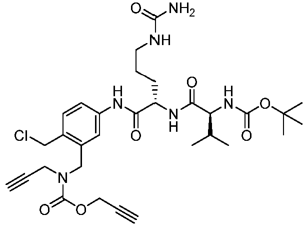

To prop-2-yn-1-yl (5-((S)-2-((S)-2-((tert-butoxycarbonyl)amino)-3-methylbutanamido)-5-ureidopentanamido)-2-(hydroxymethyl)benzyl)(prop-2-yn-1-yl)carbamate (205 mg, 0.326 mmoles, 1.0 equiv.) in CH₂Cl₂ (10 mL) was added pyridine (158 uL, 1.96 mmoles, 5 equiv.). The heterogeneous mixture was cooled in a 0 °C ice bath and thionyl chloride (71 uL, 0.98 mmoles, 3 equiv.). After stirring in the ice bath for 3 hours the reaction was directly purified by ISCO SiO₂ chromatography (0-30% MeOH/CH₂Cl₂) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate was obtained LCMS: MH+=647.6; Rt=2.54 min (5 min acidic method).

### Synthesis of 2-(hydroxymethyl)-N-methyl-5-nitrobenzamide

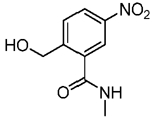

To a stirred suspension of 6-nitroisobenzofuran-1(3H)-one (500 g, 2.79 mol) in MeOH (1500 mL) was added MeNH₂ (3.00 kg, 29.94 mol, 600 mL, 31.0% purity) at 25 °C and stirred for 1 h. The solid was filtered and washed with water twice (600 mL) and dried under high vacuum to get a residue. The product 2-(hydroxymethyl)-N-methyl-5-nitrobenzamide was obtained as white solid. LCMS: Rt = 0.537 min, MS m/z = 193.2. 1H NMR: 400 MHz DMSO δ 8.57 (br d, J = 4.4 Hz, 1H), 8.31 (dd, J = 2.4, 8.6 Hz, 1H), 8.21 (d, J = 2.4 Hz, 1H), 7.86 (d, J = 8.8 Hz, 1H), 5.54 (t, J = 5.6 Hz, 1H), 4.72 (d, J = 5.5 Hz, 2H), 2.78 (d, J = 4.4 Hz, 3H).

### Synthesis of (2-((methylamino)methyl)-4-nitrophenyl)methanol

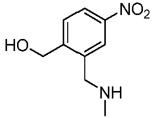

To a solution of 2-(hydroxymethyl)-N-methyl-5-nitrobenzamide (560 g, 2.66 mol) in THF (5000 mL) was cooled to 0 °C, then added BH3-Me2S (506 g, 6.66 mol) (2.0 M in THF) drop wise for 60 min and heated to 70 °C for 5 h. LCMS showed the starting material was consumed. After completion, 4M HCl (1200 mL) in Methanol was added to reaction mixture at 0 °C and heated at 65 °C for 8 h. The reaction mixture was cooled to 0 °C, the solid was filtered and concentrated in reduce pressure. The product (2-((methylamino)methyl)-4-nitrophenyl)methanol (520 g) was obtained as a white solid. LCMS: Rt = 0.742 min, MS m/z = 197.1 [M+H]+. ¹H NMR: 400 MHz DMSO δ 9.25 (br s, 2H), 8.37 (d, J = 2.4 Hz, 1H), 8.14 (dd, J = 2.4, 8.5 Hz, 1H), 7.63 (d, J = 8.4 Hz, 1H), 5.72 (br s, 1H), 4.65 (s, 2H), 4.15 (br s, 2H), 2.55 - 2.45 (m, 3H)

### Synthesis of 1-(2-(((tert-butyldiphenylsilyl)oxy)methyl)-5-nitrophenyl)-N-methylmethanamine

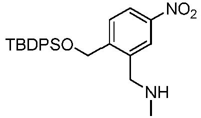

To a solution of (2-((methylamino)methyl)-4-nitrophenyl)methanol (520 g, 2.65 mol) and imidazole (721 g, 10.6 mol) in DCM (2600 mL) was cooled to 0°C was added TBDPS-CL (1.09 kg, 3.98 mol, 1.02 L) drop wise and stirred for 2 h. The mixture was poured in ice cold water (1000 mL) and extracted with ethyl acetate. The combined organic layers were washed with brine, dried over Na₂SO₄, filtered and evaporated under vacuum to give crude product. The crude product was purified by chromatography on a silica gel eluted with Ethyl acetate: Petroleum ether (from 10/1 to 1) to give a residue. The product 1-(2-(((tert-butyldiphenylsilyl)oxy)methyl)-5-nitrophenyl)-N-methylmethanamine was obtained as yellow liquid. LCMS: product: Rt = 0.910 min, MS m/z = 435.2 [M+H]+. ¹H NMR: 400 MHz CDCl3 δ 8.23 (d, J=2.4 Hz, 1H), 8.15 (dd, J=2.4, 8.4 Hz, 1H), 7.76 (d, J=8.4 Hz, 1H), 7.71 - 7.66 (m, 4H), 7.50 - 7.37 (m, 6H), 4.88 (s, 2H), 3.65 (s, 2H), 2.39 (s, 3H), 1.12 (s, 9H)

### Synthesis of (9H-fluoren-9-yl)methyl (2-(((tert-butyldiphenylsilyl)oxy)methyl)-5-nitrobenzyl)(methyl)carbamate

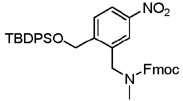

To a solution of 1-(2-(((tert-butyldiphenylsilyl)oxy)methyl)-5-nitrophenyl)-N-methylmethanamine (400 g, 920.3 mmol) in THF (4000 mL) was added FMOC-OSU (341.5 g, 1.01 mol) and Et₃N (186.2 g, 1.84 mol, 256.2 mL), the mixture was stirred at 25 °C for 1 h. The mixture was poured into water (1600 mL) and extracted with ethyl acetate (1000 mL x 2). The combined organic layers were washed with brine, dried over Na₂SO₄, filtered and evaporated under vacuum to give crude product. The crude product was purified by chromatography on a silica gel eluted with petroleum ether: ethyl acetate (from 1/0 to 1/1) to give (9H-fluoren-9-yl)methyl (2-(((tert-butyldiphenylsilyl)oxy)methyl)-5-nitrobenzyl)(methyl)carbamate as white solid. LCMS: Rt = 0.931 min, MS m/z = 657.2 [M+H]+. ¹H NMR: EW16000-26-P1A, 400 MHz CDCl3 δ 8.21 - 7.96 (m, 1H), 7.87 - 7.68 (m, 3H), 7.68 - 7.62 (m, 4H), 7.62 - 7.47 (m, 2H), 7.47 - 7.28 (m, 9H), 7.26 - 7.05 (m, 2H), 4.81 (br s, 1H), 4.62 - 4.37 (m, 4H), 4.31 - 4.19 (m, 1H), 4.08 - 3.95 (m, 1H), 2.87 (br d, J = 5.2 Hz, 3H), 1.12 (s, 9H).

### Synthesis of (9H-fluoren-9-yl)methyl (5-amino-2-(((tert-butyldiphenylsilyl)oxy)methyl)benzyl)(methyl)carbamate

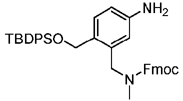

A solution of (9H-fluoren-9-yl)methyl (2-(((tert-butyldiphenylsilyl)oxy)methyl)-5-nitrobenzyl)(methyl)carbamate (3.0 g, 4.57 mmole, 1.0 equiv.) in MeOH (90 mL) and EtOAc (30 mL) was degassed and purged to a balloon of N₂ via three way stopcock. After repeating degas/N₂ purge 2x, 10% Pd/C deGussa type (0.486 g, 0.457 mmoles, 0.1 equiv.) was added. The resulting mixture was degassed and purged to a balloon of 2 H₂ via three-way stopcock. After repeating degas/H₂ purge 2x, the reaction stirred under the balloon pressure of H₂ for 4 hours. The reaction was degassed and purged to N₂, filtered through a pad of celite eluting further with MeOH. After removal of the volatiles in vacuo and pumping on high vac (9H-fluoren-9-yl)methyl (5-amino-2-(((tert-butyldiphenylsilyl)oxy)methyl)benzyl)(methyl)carbamate was obtained. LCMS: MH+=627.7; Rt=1.59 min (2 min acidic method).

### Synthesis of (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate

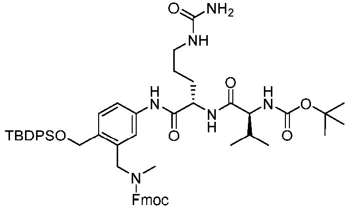

To (9H-fluoren-9-yl)methyl (5-amino-2-(((tert-butyldiphenylsilyl)oxy)methyl)benzyl)(methyl)carbamate (2.86 g, 4.56 mmoles, 1.0 equiv) and (S)-2-((S)-2-((tert-butoxycarbonyl)amino)-3-methylbutanamido)-5-ureidopentanoic acid (1.71 g, 4.56 mmoles, 1.0 equiv.) in 2:1 CH₂Cl₂/MeOH (60 mL) was added ethyl 2-ethoxyquinoline-1(2H)-carboxylate (2.256 g, 9.12 mmoles, 2.0 equiv.). The homogeneous solution was stirred for 16 hours at which time additional (S)-2-((S)-2-((tert-butoxycarbonyl)amino)-3-methylbutanamido)-5-ureidopentanoic acid (0.340 g, 0.2 equiv.) and ethyl 2-ethoxyquinoline-1(2H)-carboxylate (0.452 g, 0.4 equiv.) were addd to drive the reaction to completion. After stirring for an adiditonal 5 hours the volatiles were removed in vacuo and after purification by ISCO SiO₂ chromatography (0-5% MeOH/CH₂Cl₂) (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate was obtained. LCMS: MH+=984.1; Rt=1.54 min (2 min acidic method).

### Synthesis of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate

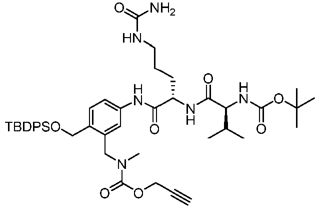

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (2.05 g, 2.085 mmol, 1.0 equiv) in THF (10 mL) was added 2.0 M dimethyl amine in MeOH (10.42 mL, 20.85 mmol, 10 equiv.). After stirring for 16 hours the volatiles were removed in vacuo. The residue was dissolved in CH₂Cl₂ (20 mL) and DIEA (0.533 mL, 4.17 mmol, 2 equiv.) and propargyl chloroformate (0.264 mL, 2.71 mmol, 1.3 equiv.) were added. After stirring at rt for 16 hours the reaction was diluted with CH₂Cl₂ (20 mL), was washed with NaHCO₃ (sat.), NaCl(sat.), dried over MgSO₄, filtered, concentrated and purified by ISCO SiO₂ chromatography (0-15% MeOH/CH₂Cl₂) to yield prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate . LCMS: MH+=843.8; Rt=1.35 min (2 min acidic method).

### Synthesis of prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate

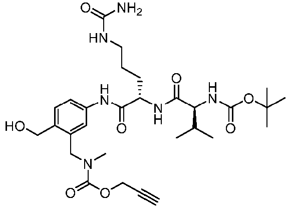

To a 0 °C solution of prop-2-yn-1-yl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (1.6 g, 1.90 mmoles, 1.0 equiv.) in THF (10.0 mL) was added 1.0 M tetrabutylammoniumn fluoride in THF (3.80 mL, 3.80 mmoles, 2.0 equiv.). After warming to room temperature and stirring for 16 h the volatiles were removed in vacuo, the residue was dissolved in EtOAc, was washed with NaHCO₃(sat.), with NaCl(sat.), dried over MgSO₄, filtered, concentrated and the residue was purified by ISCO SiO₂ chromatography (0-30% MeOH/CH2Cl2) to yield prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate. LCMS: MH+=605.7; Rt=0.81 min (2 min acidic method).

### Synthesis of prop-2-yn-1-yl (5-((S)-2-((S)-2-((tert-butoxycarbonyl)amino)-3-methylbutanamido)-5-ureidopentanamido)-2-(chloromethyl)benzyl)(methyl)carbamate

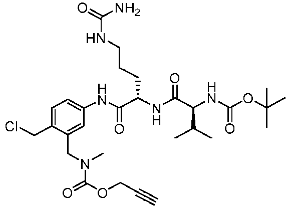

To prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate (350 mg, 0.579 mmoles, 1.0 equiv.) in CH₂Cl₂ (10 mL) was added pyridine (0.278 mL, 3.47 mmoles, 6 equiv.). The heterogeneous mixture was cooled in a 0 °C ice bath and thionyl chloride (0.126 mL, 1.73 mmoles, 3 equiv.). After stirring in the ice bath for 3 h, the reaction was purified by ISCO SiO₂ chromatography (0-30% MeOH/CH₂Cl₂) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate was obtained. LCMS: MH+=623.7; Rt=2.19 min (5 min acidic method).

### Synthesis of (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate

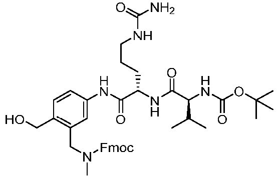

To (9H-fluoren-9-yl)methyl (5-((S)-2-((S)-2-((tert-butoxycarbonyl)amino)-3-methylbutanamido)-5-ureidopentanamido)-2-(((tert-butyldiphenylsilyl)oxy)methyl)benzyl)(methyl)carbamate (2.6 g, 2.64 mmol, 1.0 equiv.) dissolved in THF (20 mL) was added acetic acid (0.757 mL, 13.22 mmol, 5.0 equiv.) and 1.0 M TBAF in THF (2.91 mL, 2.91 mmol, 1.1 equiv.). The solution was stirred for 72 hours at which time the volatiles were removed in vacuo. After purification by ISCO SiO₂ chromatography (0-30% MeOH/CH₂Cl₂) (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate was obtained. LCMS: MH+=745.5; Rt=1.07 min (2 min acidic method).

### Synthesis of (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate

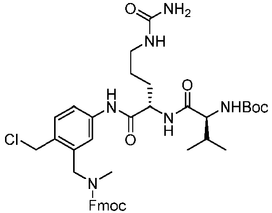

To (9H-fluoren-9-yl)methyl (5-((S)-2-((S)-2-((tert-butoxycarbonyl)amino)-3-methylbutanamido)-5-ureidopentanamido)-2-(hydroxymethyl)benzyl)(methyl)carbamate (200 mg, 0.269 mmoles, 1.0 equiv.) in CH₂Cl₂ (10 mL) was added pyridine (0.130 mL, 1.61 mmoles, 6 equiv.). The heterogeneous mixture was cooled in a 0 °C ice bath and thionyl chloride (0.059 mL, 0.806 mmoles, 3 equiv.). After stirring in the ice bath briefly the reaction was stirred as it warmed up to room temperature for 2 hours. The reaction was purified by ISCO SiO₂ chromatography (0-30% MeOH/CH₂Cl₂) and (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate was obtained. LCMS: MH+=763.2; Rt=1.18 min (2 min acidic method).

### GENERAL PROCEDURE 1

### Synthesis of 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

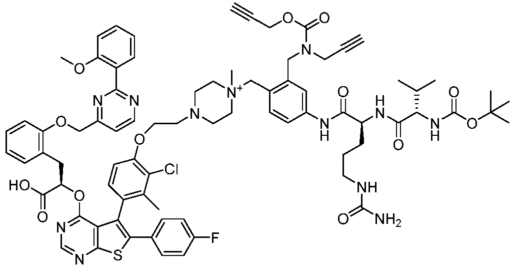

To (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid hydrochloride (73.8 mg, 0.81 mmoles, 1.0 equiv.) and prop-2-yn-1-yl (5-((S)-2-((S)-2-((tert-butoxycarbonyl)amino)-3-methylbutanamido)-5-ureidopentanamido)-2-(chloromethyl)benzyl)(prop-2-yn-1-yl)carbamate (78 mg, 0.122 mmoles, 1.5 equiv.) dissolved in DMF (0.5 mL) was added DIEA (70 uL, 0.405 mmoles, 5.0 equiv.) followed by tetrabutylammonium iodide (25.4 mg, 0.069 mmoles, 0.85 equiv.). After stirring for 5 h, the reaction was diluted with DMSO (3 mL) and was purified by RP-HPLC ISCO gold chromatography (10-100% MeCN/H2O, 0.1% NH4OH modifier). Upon lyophilization, 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. LCMS: M+=1486.3; Rt=2.70 min (5 min basic method).

### GENERAL PROCEDURE 2

### Synthesis of 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

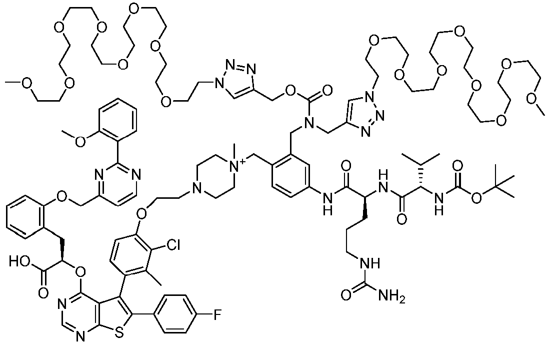

To 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (24 mg, 0.016 mmoles, 1.0 equiv) and 25-azido-2,5,8,11,14,17,20,23-octaoxapentacosane (26.5 mg, 0.065 mmoles, 4 equiv.) was added t-BuOH (1 mL). The mixture was degassed via house vacuum and purged to a balloon of N₂ via a 3-way stopcock. Degas/purge was repeated 3 times. A 16 mg/mL aqueous solution of sodium ascorbate (297 uL, 0.024 mmoles, 1.5 equiv.) was added and the solution was degassed and purged to N₂ three times. A 4 mg/mL aqueous solution of copper sulfate (298 uL, 0.0048 mmoles, 0.3 equiv.) was added and the solution was degassed and purged to N₂ three times. After stirring under N₂ for 3 h the reaction was diluted with DMSO (3 mL) and was purified by RP-HPLC ISCO gold chromatography (10-100% MeCN/H2O, 0.1% TFA modifier). Upon lyophilization 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS M+=2307.0730, Rt=2.69 min (5 min acidic method).

### GENERAL PROCEDURE 3

### Synthesis of 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L1-P1)

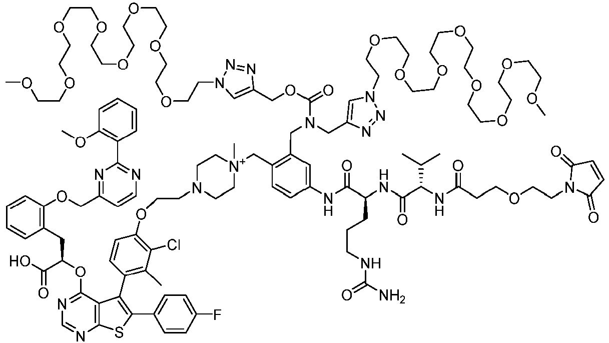

To 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (19 mg, 0.0082 mmoles, 1.0 equiv.) was added 25% TFA/CH₂Cl₂ (2 mL). After standing for 45 min, the volatiles were removed in vacuo, CH₂Cl₂ was added and the volatiles were removed in vacuo and pumped on. The residue was dissolved in DMF (1 mL) and DIEA (22 uL, 0.124 mmoles, 15 equiv.) and 2,5-dioxopyrrolidin-1-yl 3-(2-(2,5-dioxo-2,5-dihydro-1H-pyrrol-1-yl)ethoxy)propanoate (5.1 mg, 0.016 mmoles, 2 equiv. ) was added. After standing for 18 h, the solution was diluted with DMSO (3 mL) and was purified by RP-ISCO gold chromatography. Upon lyophilization, 1-(2-(((((1-(2,5,8,11,14,17,20,23-octaoxapentacosan-25-yl)-1H-1,2,3-triazol-4-yl)methoxy)carbonyl)((1-(2,5,8,11,14,17,20,23-octaoxapentacosan-25-yl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium **(L1-P1)** was obtained. HRMS: M+=2399.0797, Rt=2.43 min (5 min acidic run). ¹H NMR (400 MHz, DMSO-*d*₆) δ ppm 0.83 (dd, J=13.82, 6.72 Hz, 6 H) 1.30 - 1.52 (m, 2 H) 1.55 - 1.76 (m, 2 H) 1.83 (s, 3 H) 1.88 - 2.08 (m, 1 H) 2.28 - 2.46 (m, 7 H) 2.73 - 2.84 (m, 4 H) 2.84 - 3.08 (m, 8 H) 3.15 - 3.27 (m, 3 H) 3.43 - 3.66 (m, 68 H) 3.73 - 3.83 (m, 7 H) 4.16 - 4.30 (m, 3 H) 4.32 - 4.43 (m, 2 H) 4.47 (br s, 6 H) 4.60 (br s, 3 H) 5.16 - 5.30 (m, 3 H) 5.40 (br s, 2 H) 5.44 - 5.52 (m, 1 H) 5.99 (br t, J=5.07 Hz, 1 H) 6.21 (d, J=6.48 Hz, 1 H) 6.71 (t, *J*=7.40 Hz, 1 H) 6.97 - 7.04 (m, 2 H), 7.00 (s, 2H) 7.12 - 7.23 (m, 5 H) 7.27 - 7.54 (m, 8 H) 7.63 (d, *J*=5.14 Hz, 1 H) 7.78 - 7.94 (m, 3 H) 7.99 (br s, 1 H) 8.05 - 8.23 (m, 2 H) 8.60 (s, 1 H) 8.88 (d, *J*=5.13 Hz, 1 H) 10.24 (br s, 1 H).

### Synthesis of 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

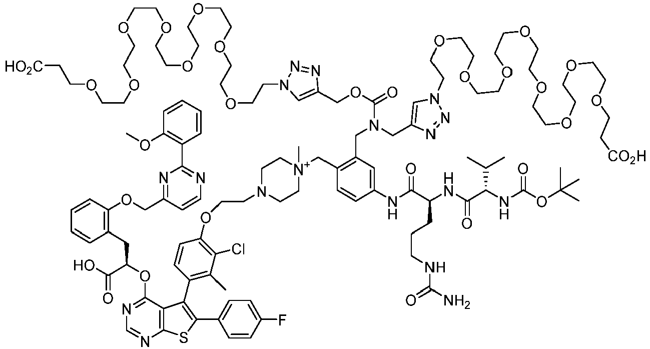

Following **GENERAL PROCEDURE 2** with 1-(4-((S)-2-((S)-2-((tert-butoxycarbonyl)amino)-3-methylbutanamido)-5-ureidopentanamido)-2-((prop-2-yn-1-yl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (30 mg, 0.020 mmoles, 1.0 equiv) and 1-azido-3,6,9,12,15,18,21,24-octaoxaheptacosan-27-oic acid (28.3 mg, 0.061 mmoles, 3 equiv.), 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=2420.0867, Rt=2.57 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L10-P1)

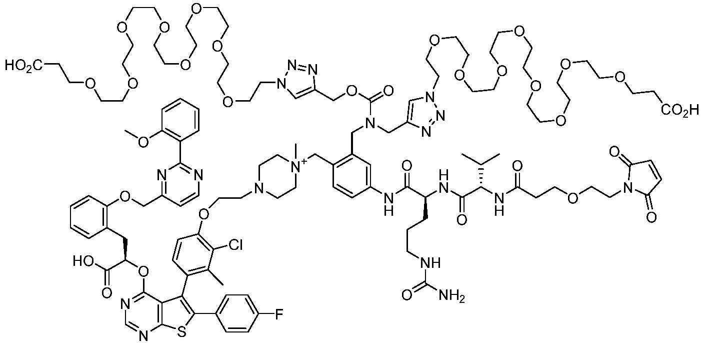

Following **GENERAL PROCEDURE 3** with 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (30 mg, 0.012 mmoles, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)((1-(26-carboxy-3,6,9,12,15,18,21,24-octaoxahexacosyl)-1H-1,2,3-triazol-4-yl)methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L10-P1)** was obtained. HRMS: M+=2515.0879, Rt=2.43 min (5 min acidic method). ¹H NMR (400 MHz, DMSO-*d*₆) δ ppm 0.83 (dd, *J*=13.82, 6.72 Hz, 6 H) 1.30 - 1.52 (m, 2 H) 1.55 - 1.76 (m, 2 H) 1.83 (s, 3 H) 1.88 - 2.08 (m, 1 H) 2.28 - 2.46 (m, 7 H) 2.73 - 2.84 (m, 4 H) 2.84 - 3.08 (m, 8 H) 3.15 - 3.27 (m, 3 H) 3.43 - 3.66 (m, 68 H) 3.73 - 3.83 (m, 7 H) 4.16 - 4.30 (m, 3 H) 4.32 - 4.43 (m, 2 H) 4.47 (br s, 6 H) 4.60 (br s, 3 H) 5.16 - 5.30 (m, 3 H) 5.40 (br s, 2 H) 5.44 - 5.52 (m, 1 H) 5.99 (br t, *J*=5.07 Hz, 1 H) 6.21 (d, *J*=6.48 Hz, 1 H) 6.71 (t, *J*=7.40 Hz, 1 H) 6.97 - 7.04 (m, 2 H), 7.00 (s, 2H) 7.12 - 7.23 (m, 5 H) 7.27 - 7.54 (m, 8 H) 7.63 (d, *J*=5.14 Hz, 1 H) 7.78 - 7.94 (m, 3 H) 7.99 (br s, 1 H) 8.05 - 8.23 (m, 2 H) 8.60 (s, 1 H) 8.88 (d, *J*=5.13 Hz, 1 H) 10.24 (br s, 1 H).

### Synthesis of 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

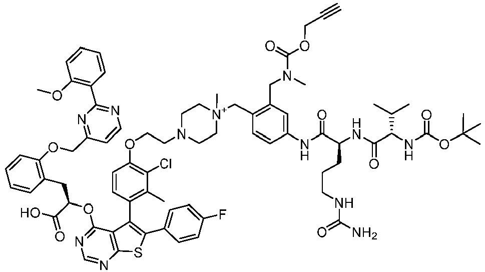

Following GENERAL PROCEDURE 1 with (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid hydrochloride (300 mg, 0.329 mmol, 1.0 equiv.) and prop-2-yn-1-yl (5-((S)-2-((S)-2-((tert-butoxycarbonyl)amino)-3-methylbutanamido)-5-ureidopentanamido)-2-(chloromethyl)benzyl)(methyl)carbamate (246 mg, 0.395 mmol, 1.2 equiv.), 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained . HRMS: M+=1461.5800, Rt=2.53 min (5 min acidic method).

### Synthesis 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

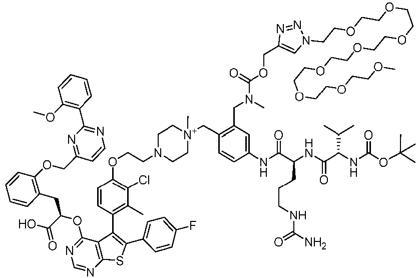

Following **GENERAL PROCEDURE 2** with 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (20 mg, 0.14 mmol, 1.0 equiv.) and 25-azido-2,5,8,11,14,17,20,23-octaoxapentacosane (16.8 mg, 0.041 mmol, 3.0 equiv.), 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1872.8359, Rt=2.56 min (5 min acidic method).

### Synthesis of 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L4-P1)

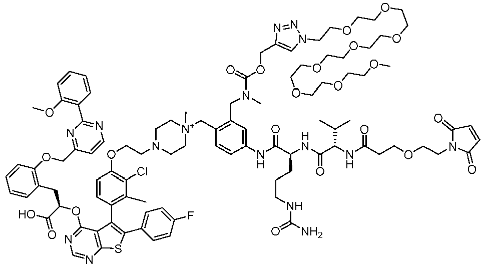

Following **GENERAL PROCEDURE 3** with 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (16.9 mg, 0.009 mmol, 1.0 equiv.), 1-(2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium **(L4-P1)** was obtained. HRMS: M+= 1967.8375, Rt=2.46 min (5 min acidic method).

### Synthesis of 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

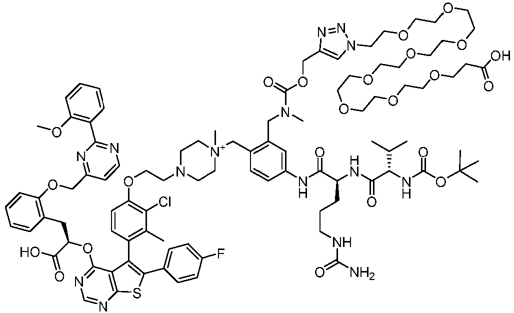

Following **GENERAL PROCEDURE 2** with 1-(4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (12 mg, 0.0082 mmol, 1.0 equiv) and 1-azido-3,6,9,12,15,18,21,24-octaoxaheptacosan-27-oic acid (11.5 mg, 0.025 mmol, 3.0 equiv.), 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1928.8459, Rt=2.52 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L3-P1)

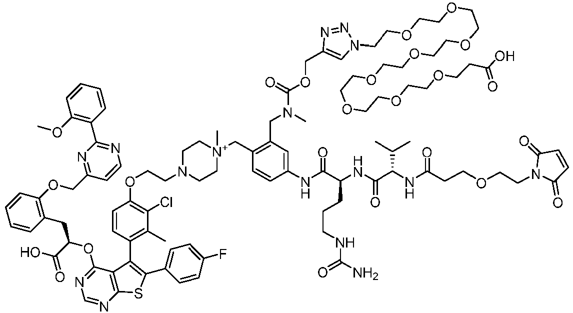

Following **general procedure 3** with 1-(4-((S)-2-((S)-2-((tert-butoxycarbonyl)amino)-3-methylbutanamido)-5-ureidopentanamido)-2-(((((1-(26-carboxy-3,6,9,12,15,18,21,24-octaoxahexacosyl)-1H-1,2,3-triazol-4-yl)methoxy)carbonyl)(methyl)amino)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (12 mg, 0.006 mmol,1.00 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((1-(26-carboxy-3,6,9,12,15,18,21,24-octaoxahexacosyl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L3-P1)** was obtained . HRMS: M+=2024.8516, Rt=2.42 min (5 min acidic method).

### Synthesis of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

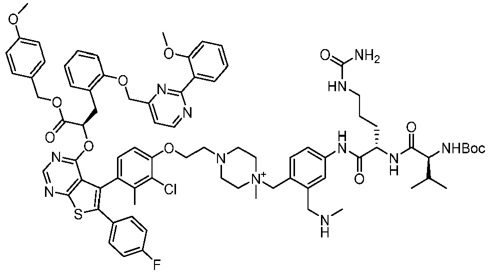

To 4-methoxybenzyl (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoate (160 mg, 0.161 mmol, 1.0 equiv.) and (9H-fluoren-9-yl)methyl (5-((S)-2-((S)-2-((tert-butoxycarbonyl)amino)-3-methylbutanamido)-5-ureidopentanamido)-2-(chloromethyl)benzyl)(methyl)carbamate (153 mg, 0.201 mmoles, 1.25 equiv.) dissolved in DMF (2 mL) was added DIEA (0.056 mL, 0.321 mmoles, 2.0 equiv.) followed by tetrabutylammonium iodide (65.3 mg, 0.177 mmoles, 1.1 equiv.). After standing for 16 h, 2.0 dimethylamine in THF (0.804 mL, 1.67 mmol, 10 equiv.) was added. After standing for 2 h, the volatiles were removed in vacuo, DMSO (6 mL) was added and the solution was purified by RP-HPLC ISCO gold chromatography (10-100% MeCN/H2O, 0.1% TFA modifier). Upon lyophilization, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1499.3700; Rt=2.59 min (5 min acidic method).

### Synthesis of 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

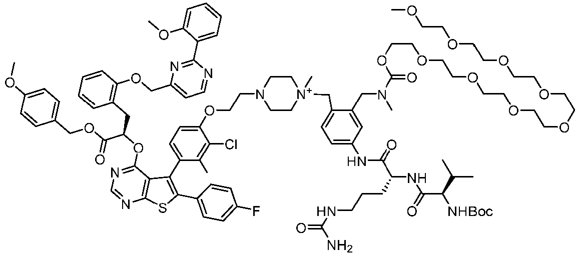

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (40 mg, 0.027 mmol, 1.0 equiv.) and 2,5-dioxopyrrolidin-1-yl (2,5,8,11,14,17,20,23-octaoxapentacosan-25-yl) carbonate (30.8 mg, 0.059 mmoles, 2.2 equiv.) dissolved in DMF (1.5 mL) was added DIEA (0.009 mL, 0.053 mmoles, 2.0 equiv.). After standing for 1 hour, DMSO (3 mL) was added and the solution was purified by RP-HPLC ISCO gold chromatography (10-100% MeCN/H2O, 0.1% TFA modifier). Upon lyophilization 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1909.3800; Rt=2.92 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((R)-2-((R)-2-(3-(2-(2,5-dioxo-2,5-dihydro-1H-pyrrol-1-yl)ethoxy)propanamido)-3-methylbutanamido)-5-ureidopentanamido)-2-(2-methyl-3-oxo-4,7,10,13,16,19,22,25,28-nonaoxa-2-azanonacosyl)benzyl)-1-methylpiperazin-1-ium (L2-P1)

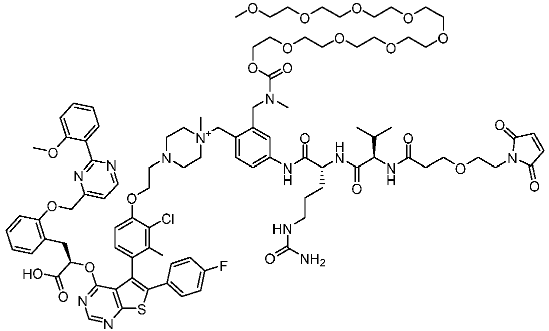

Following **GENERAL PROCEDURE 3** with 1-(4-((R)-2-((R)-2-((tert-butoxycarbonyl)amino)-3-methylbutanamido)-5-ureidopentanamido)-2-(2-methyl-3-oxo-4,7,10,13,16,19,22,25,28-nonaoxa-2-azanonacosyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (25.3 mg, 0.013 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((R)-2-((R)-2-(3-(2-(2,5-dioxo-2,5-dihydro-1H-pyrrol-1-yl)ethoxy)propanamido)-3-methylbutanamido)-5-ureidopentanamido)-2-(2-methyl-3-oxo-4,7,10,13,16,19,22,25,28-nonaoxa-2-azanonacosyl)benzyl)-1-methylpiperazin-1-ium **(L2-P1)** was obtained . HRMS: M+= 1884.7900, Rt=2.50 min (5 min acidic method).

### Synthesis of 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

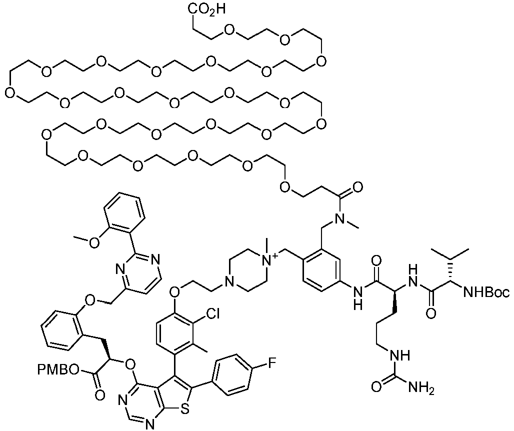

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (150 mg, 0.093 mmol, 1.0 equiv.) and 79-((2,5-dioxopyrrolidin-1-yl)oxy)-79-oxo-4,7,10,13,16,19,22,25,28,31,34,37,40,43,46,49,52,55,58,61,64,67,70,73,76-pentacosaoxanonaheptacontanoic acid (134 mg, 0.102 mmoles, 1.1 equiv.) dissolved in DMF (2 mL) was added DIEA (0.081 mL, 0.464 mmoles, 5.0 equiv.). After standing for 18 h, DMSO (6 mL) was added and the solution was purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2700.8701; Rt=2.83 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L11-P1)

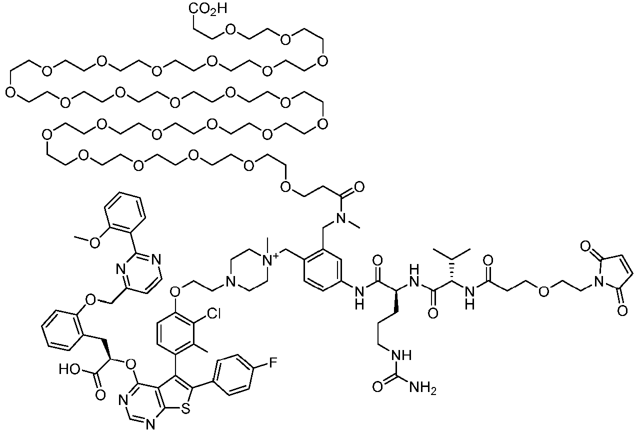

Following **GENERAL PROCEDURE 3** with 1-(4-((S)-2-((S)-2-((tert-butoxycarbonyl)amino)-3-methylbutanamido)-5-ureidopentanamido)-2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(80-carboxy-2-methyl-3-oxo-6,9,12,15,18,21,24,27,30,33,36,39,42,45,48,51,54,57,60,63,66,69,72,75,78-pentacosaoxa-2-azaoctacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L11-P1) was obtained . HRMS: M+= 2674.8201, Rt=2.44 min (5 min acidic method).

### Synthesis of 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

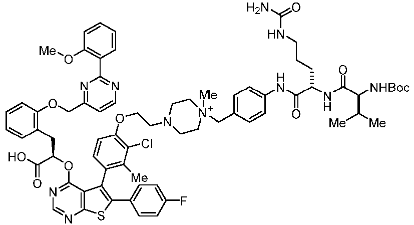

Following GENERAL PROCEDURE 1 with (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoic acid (50 mg, 0.057 mmol, 1.0 equiv.) and tert-butyl ((S)-1-(((S)-1-((4-(chloromethyl)phenyl)amino)-1-oxo-5-ureidopentan-2-yl)amino)-3-methyl-1-oxobutan-2-yl)carbamate (34.1 mg, 0.069 mmol, 1.2 equiv.), 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. LCMS: M+=1337.2, Rt=1.11 min (2 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium or (2R)-2-[(5Sₐ)-5-[3-chloro-4-[2-[4-[[4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]phenyl]methyl]-4-methyl-piperazin-4-ium-1-yl]ethoxy]-2-methylphenyl]-6-(4-fluorophenyl)thieno[2,3-d]pyrimidin-4-yl]oxy-3-[2-[[2-(2-methoxyphenyl)pyrimidin-4-yl]methoxy]phenyl]propanoic acid (L9-P1)

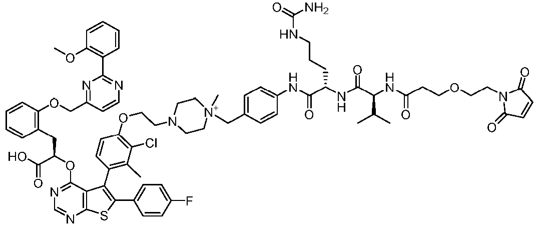

Following **GENERAL PROCEDURE 3** with 1-(4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (55 mg, 0.041 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium **(L9-P1)** was obtained. HRMS: M+=1431.5400, Rt=2.50 min (5 min acidic method).

### Synthesis of 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

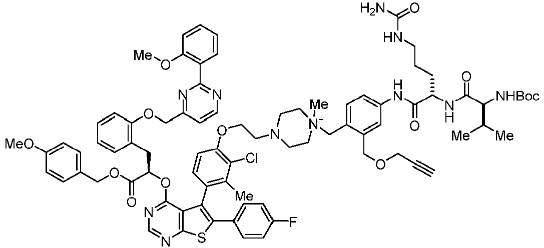

Following GENERAL PROCEDURE 1 with 4-methoxybenzyl (R)-2-((5-(3-chloro-2-methyl-4-(2-(4-methylpiperazin-1-yl)ethoxy)phenyl)-6-(4-fluorophenyl)thieno[2,3-d]pyrimidin-4-yl)oxy)-3-(2-((2-(2-methoxyphenyl)pyrimidin-4-yl)methoxy)phenyl)propanoate (85 mg, 0.085 mmol, 1.0 equiv.) and tert-butyl ((S)-1-(((S)-1-((4-(chloromethyl)-3-((prop-2-yn-1-yloxy)methyl)phenyl)amino)-1-oxo-5-ureidopentan-2-yl)amino)-3-methyl-1-oxobutan-2-yl)carbamate (58 mg, 0.102 mmol, 1.2 equiv.), 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1524.6200, Rt=2.95 min (5 min acidic method).

### Synthesis of 1-(2-(((1-(2,5,8,11,14,17,20,23-octaoxapentacosan-25-yl)-1H-1,2,3-triazol-4-yl)methoxy)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

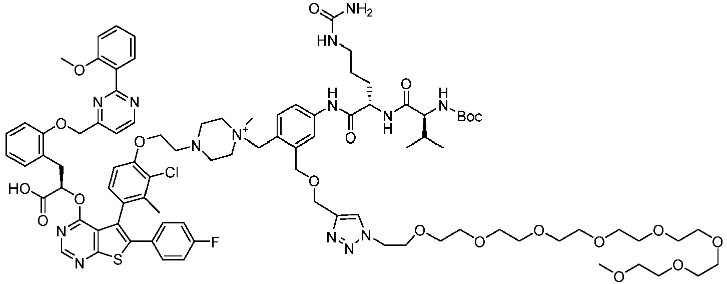

Following GENERAL PROCEDURE 2 with 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (20 mg, 0.014 mmoles, 1.0 equiv) and 25-azido-2,5,8,11,14,17,20,23-octaoxapentacosane (5.8 mg, 0.014 mmoles, 1 equiv.), 1-(2-(((1-(2,5,8,11,14,17,20,23-octaoxapentacosan-25-yl)-1H-1,2,3-triazol-4-yl)methoxy)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. LCMS: [(M+)+H+]+2/2=908.5, Rt=1.15 min (2 min acidic method).

### Synthesis of 1-(2-(((1-(2,5,8,11,14,17,20,23-octaoxapentacosan-25-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L8-P1)

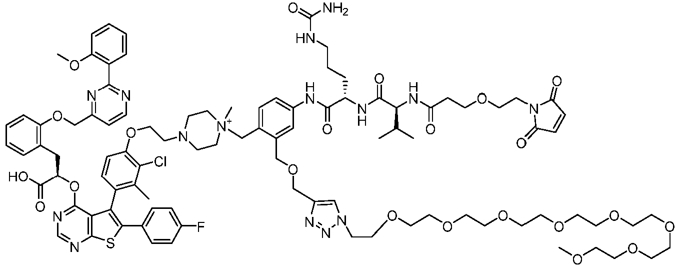

Following **GENERAL PROCEDURE 3** with 1-(2-(((1-(2,5,8,11,14,17,20,23-octaoxapentacosan-25-yl)-1H-1,2,3-triazol-4-yl)methoxy)methyl)-4-((S)-2-((S)-2-((tert-butoxycarbonyl)amino)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (20 mg, 0.010 mmol, 1.0 equiv.), 1-(2-(((1-(2,5,8,11,14,17,20,23-octaoxapentacosan-25-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium **(L8-P1)** was obtained . HRMS: M+= 1908.8097, Rt=2.37 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium

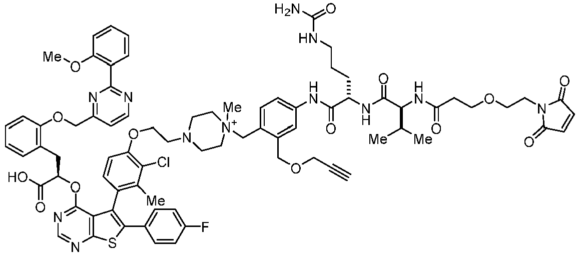

Following **GENERAL PROCEDURE 3** with 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (123.1 mg, 0.075 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium was obtained. HRMS: M+= 1499.5601, Rt=2.50 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(26-carboxy-3,6,9,12,15,18,21,24-octaoxahexacosyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L7-P1)

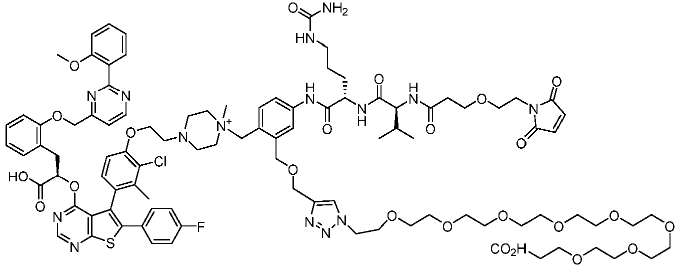

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (40 mg, 0.027 mmoles, 1.0 equiv) and 1-azido-3,6,9,12,15,18,21,24-octaoxaheptacosan-27-oic acid (37.4 mg, 0.080 mmoles, 3.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(26-carboxy-3,6,9,12,15,18,21,24-octaoxahexacosyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L7-P1) was obtained. HRMS: M+=1965.5601, Rt=2.35 min (5 min acidic method).

### Synthesis of 1-(2-(((1-(2,5,8,11,14,17,20,23,26,29,32,35,38,41,44,47,50,53,56,59,62,65,68,71-tetracosaoxatriheptacontan-73-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L5-P1)

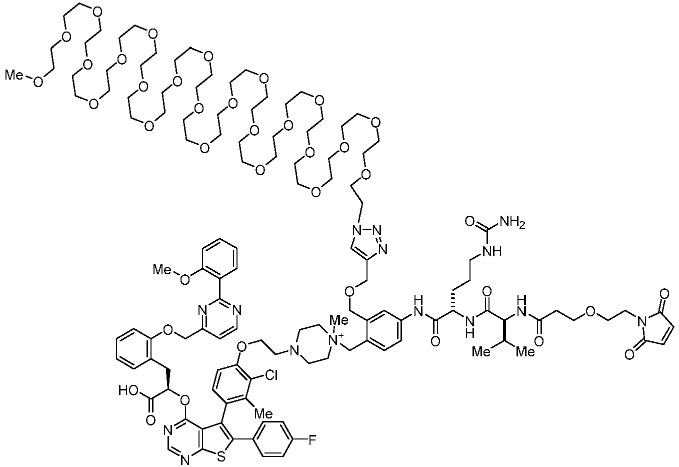

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (20.4 mg, 0.014 mmoles, 1.0 equiv) and 73-azido-2,5,8,11,14,17,20,23,26,29,32,35,38,41,44,47,50,53,56,59,62,65,68,71-tetracosaoxatriheptacontane (28.1 mg, 0.025 mmoles, 1.5 equiv.), 1-(2-(((1-(2,5,8,11,14,17,20,23,26,29,32,35,38,41,44,47,50,53,56,59,62,65,68,71-tetracosaoxatriheptacontan-73-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium **(L5-P1)** was obtained. HRMS: M+=2613.2100, Rt=2.44 min (5 min acidic method).

### Synthesis of prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate

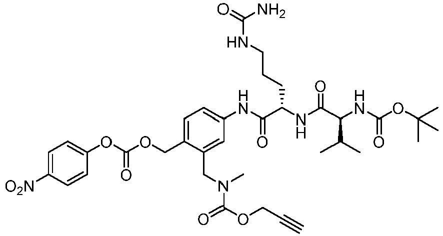

A solution of prop-2-yn-1-yl (5-((S)-2-((S)-2-((tert-butoxycarbonyl)amino)-3-methylbutanamido)-5-ureidopentanamido)-2-(hydroxymethyl)benzyl)(methyl)carbamate (249 mg, 0.412 mmoles) and 4-nitrophenyl (4-nitrosophenyl) carbonate (356 mg, 1.24 mmoles, 3.0 equiv.) in DMF (2 mL) was swirled until homogeneous and sat for 16 hours. The solution was diluted with DMSO (6 mL) and was purified by RP-HPLC ISCO gold chromatography (10-100% MeCN/H2O, no modifier). Upon lyophilization, prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate was obtained. LC/MS MH+=770.7, Rt=2.45 min (5 min acidic method).

### Synthesis of prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate

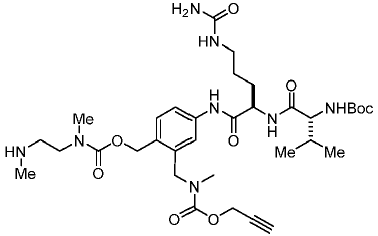

To a solution of prop-2-yn-1-yl (5-((S)-2-((S)-2-((tert-butoxycarbonyl)amino)-3-methylbutanamido)-5-ureidopentanamido)-2-((((4-nitrophenoxy)carbonyl)oxy)methyl)benzyl)(methyl)carbamate (100mg, 0.130 mmol) in DMF (1 ml) was added N,N'-Dimethyl-ethylenediamine (22.90 mg, 0.260 mmol), followed by the addition of DIPEA (0.113 ml, 0.650 mmol) at room temperature. The resulting solution was stirred at room temperature overnight. The reaction was diluted with DMSO was purified by RP-HPLC ISCO gold chromatography (10-100% MeCN/H2O, 0.1% NH4OH modifier). Upon lyophilization prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate was obtained. LCMS: MH+=719.9, Rt=0.73 min (2 min acidic method).

### Synthesis of (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium

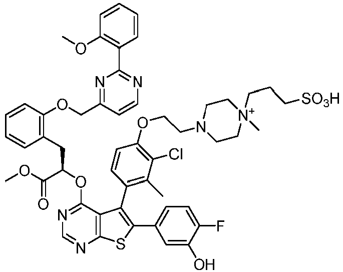

To a solution of (R)-4-(2-(4-(4-(1-carboxy-2-(2-((2-(2-methoxyphenyl)pyrimidin-4-yl)methoxy)phenyl)ethoxy)-6-(4-fluoro-3-hydroxyphenyl)thieno[2,3-d]pyrimidin-5-yl)-2-chloro-3-methylphenoxy)ethyl)-1-methyl-1-(3-sulfopropyl)piperazin-1-ium or (2*R*)-2-[(5*Sₐ*)-5-[3-chloro-2-methyl-4-[2-[4-methyl-4-(3-sulfopropyl)piperazin-4-ium-1-yl]ethoxy]phenyl]-6-(4-fluoro-3-hydroxy-phenyl)thieno[2,3-*d*]pyrimidin-4-yl]oxy-3-[2-[[2-(2-methoxyphenyl)pyrimidin-4-yl]methoxy]phenyl]propanoic acid (100 mg, 0.099 mmoles) in MeOH (1.5 mL) was added a few drops of H₂SO₄(conc.). After stirring overnight, the MeOH was removed in vacuo, the residue was dissolved in DMSO was purified by RP-HPLC ISCO gold chromatography (10-100% MeCN/H2O, 0.1% TFA modifier). Upon lyophilization, (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium was obtained. HRMS M+=1027.2900, Rt=2.31 min (5 min acidic method).

### Synthesis of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carba moyl)oxy)-4-fluorophenyl)-4-(((R)-1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium

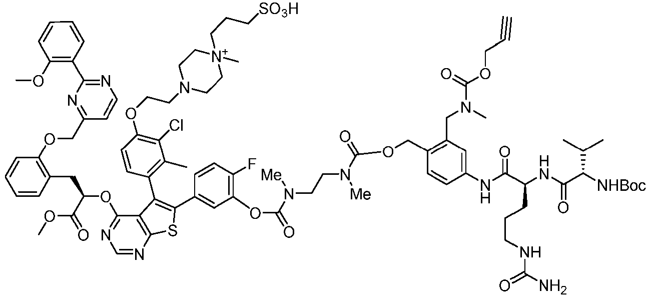

To a solution of (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium (50 mg, 0.049 mmoles, 1.0 equiv) in CH₂Cl₂ (1 mL) at 0 °C was added TEA (34 uL, 0.243 mmoles, 5.0 equiv.) followed by 4-Nitrophenyl chloroformate (10.8 mg, 0.054 mmoles, 1.1 equiv.). After stirring for 15 min, a solution of prop-2-yn-1-yl (5-((R)-2-((R)-2-((tert-butoxycarbonyl)amino)-3-methylbutanamido)-5-ureidopentanamido)-2-(((methyl(2-(methylamino)ethyl)carbamoyl)oxy)methyl)benzyl)(methyl)carbamate (66.3 mg, 0.092 mmoles, 2.0 equiv) in DMF (1 mL) was added followed by DIEA (40 uL, 0.231 mmoles, 5.0 equiv.). After stirring for 2 h, the volatiles were removed in vacuo, the solution was diluted with DMSO (3 ml) and was purified by RP-HPLC ISCO gold chromatography (10-100% MeCN/H2O, 0.1% TFA modifier). Upon lyophilization 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-(((R)-1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium was obtained. HRMS M+=1771.6700, Rt=2.57 min (5 min acidic method).

### Synthesis of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carba moyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium

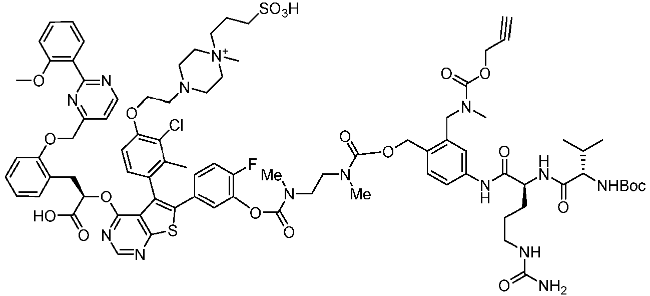

To a solution of 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-(((R)-1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-2-chloro-3-methylphenoxy)ethyl)-1-methyl-1-(3-sulfopropyl)piperazin-1-ium (23 mg, 0.013 mmoles, 1.0 equiv) in THF (1 mL) was added 2N LiOH (0.032 mL, 0.065 mmoles, 5 equiv). After stirring for 2 h, the solution was neutralized with AcOH and purified by RP-HPLC ISCO gold chromatography (10-100% MeCN/H2O, 0.1% TFA modifier). Upon lyophilization, 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium was obtained. HRMS M+=1757.6200, Rt=2.46 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carba moyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium

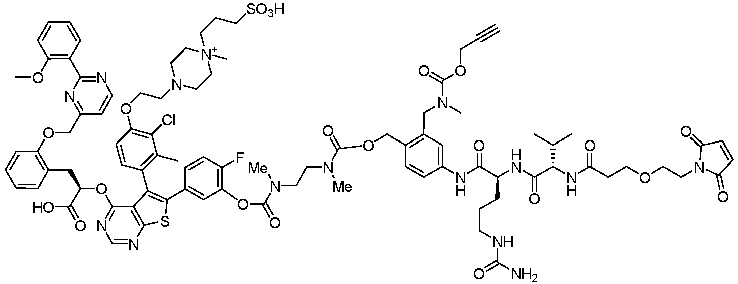

Following **GENERAL PROCEDURE 3** with 4-(2-(4-(6-(3-(((2-((((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (17 mg, 0.0097 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)thieno[2,3-d]pyrimidin-5-yl)-2-chloro-3-methylphenoxy)ethyl)-1-methyl-1-(3-sulfopropyl)piperazin-1-ium was obtained. HRMS: M+= 1852.5200, Rt=2.29 min (5 min acidic method).

### Synthesis of 4-(2-(4-(6-(3-(((2-((((2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)carbonyl)(methyl)amino)ethyl)(methyl)carbamoyl)oxy) -4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (L12-P2)

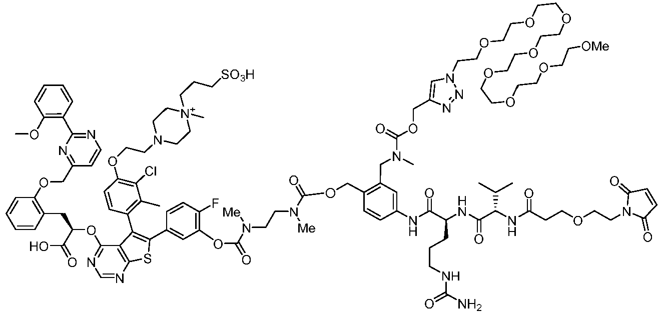

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(3-(((2-((((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)carbonyl)(methyl)amino)ethyl)(methyl)carbamoyl)ox y)-4-fluorophenyl)thieno[2,3-d]pyrimidin-5-yl)-2-chloro-3-methylphenoxy)ethyl)-1-methyl-1-(3-sulfopropyl)piperazin-1-ium (10 mg, 0.0054 mmoles, 1.0 equiv.) and 25-azido-2,5,8,11,14,17,20,23-octaoxapentacosane (4.4 mg, 0.011 mmoles, 2 equiv.), 4-(2-(4-(6-(3-(((2-((((2-(((((1-(2,5,8,11, 14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)carbonyl)(methyl)amino)ethyl)(methyl)carbamoyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium **(L12-P2** ) was obtained . HRMS: M+=2261.8601, Rt=2.24 min (5 min acidic method).

### Synthesis of 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium

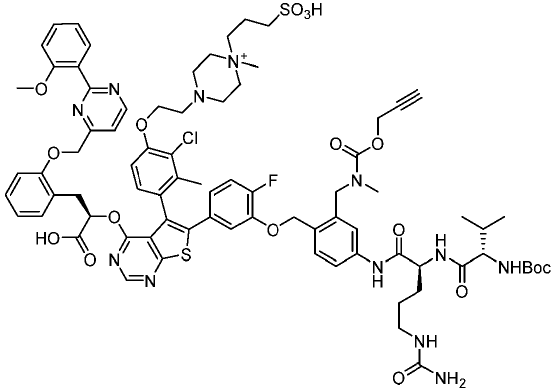

GENERAL PROCEDURE 1 was followed using (R)-4-(2-(2-chloro-4-(6-(4-fluoro-3-hydroxyphenyl)-4-((1-methoxy-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methyl-1-(3-sulfopropyl)piperazin-1-ium (40 mg, 0.039 mmol, 1.0 equiv.) and prop-2-yn-1-yl (5-((R)-2-((R)-2-((tert-butoxycarbonyl)amino)-3-methylbutanamido)-5-ureidopentanamido)-2-(chloromethyl)benzyl)(methyl)carbamate (36.4 mg, 0.058 mmol, 1.5 equiv.), with the modification of after the alkylation was complete adding 2N LiOH (0.097 mL, 0.195 mmoles, 5.0 equiv.) and stirring for 2 h prior to neutralizing and purifying by RP-HPLC. Upon lyophilization, 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium was obtained. HRMS: M+=1599.5856, Rt=1.34 min (2 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium

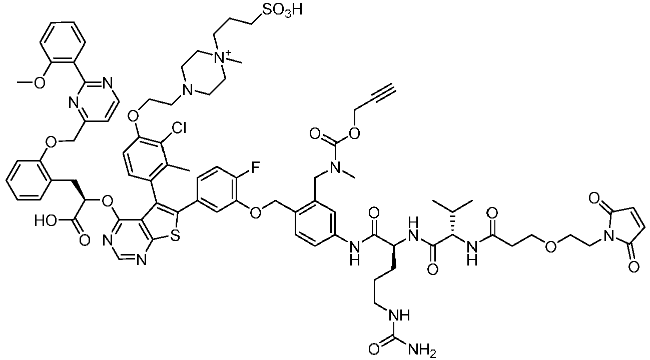

Following **GENERAL PROCEDURE 3** with 4-(2-(4-(6-(3-((4-((S)-2-((S)-2-((tert-butoxycarbonyl)amino)-3-methylbutanamido)-5-ureidopentanamido)-2-((methyl((prop-2-yn-1-yloxy)carbonyl)amino)methyl)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (46 mg, 0.029 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium was obtained. HRMS: M+= 1694.5699, Rt=2.55 min (5 min acidic method).

### Synthesis of 4-(2-(4-(6-(3-((2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium (L4-P2)

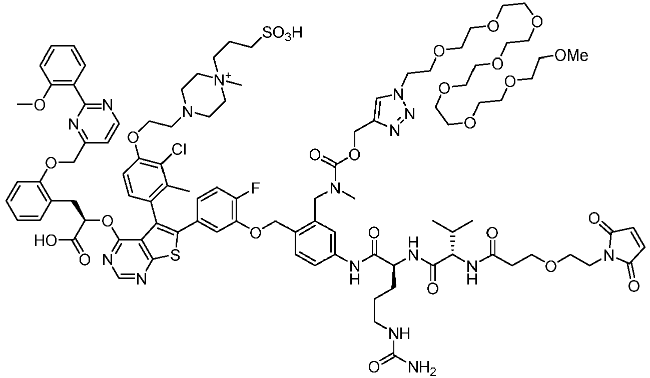

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(3-((4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((methyl((prop-2-yn-1-yloxy)carbonyl)amino)methyl)benzyl)oxy)-4-fluorophenyl)thieno[2,3-d]pyrimidin-5-yl)-2-chloro-3-methylphenoxy)ethyl)-1-methyl-1-(3-sulfopropyl)piperazin-1-ium (44 mg, 0.026 mmoles, 1.0 equiv.) and 25-azido-2,5,8,11,14,17,20,23-octaoxapentacosane (21.2 mg, 0.052 mmoles, 2 equiv.), 4-(2-(4-(6-(3-((2-(((((1-(2,5,8,11,14,17,20,23-octaoxapentacosan-25-yl)-1H-1,2,3-triazol-4-yl)methoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)oxy)-4-fluorophenyl)-4-((R)-1-carboxy-2-(2-((2-(2-methoxyphenyl)pyrimidin-4-yl)methoxy)phenyl)ethoxy)thieno[2,3-d]pyrimidin-5-yl)-2-chloro-3-methylphenoxy)ethyl)-1-methyl-1-(3-sulfopropyl)piperazin-1-ium **(L4-P2)** was obtained. HRMS: M+=2103.8000, Rt=2.47 min (5 min acidic method).

### GENERAL PROCEDURE 4:

### Synthesis of 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

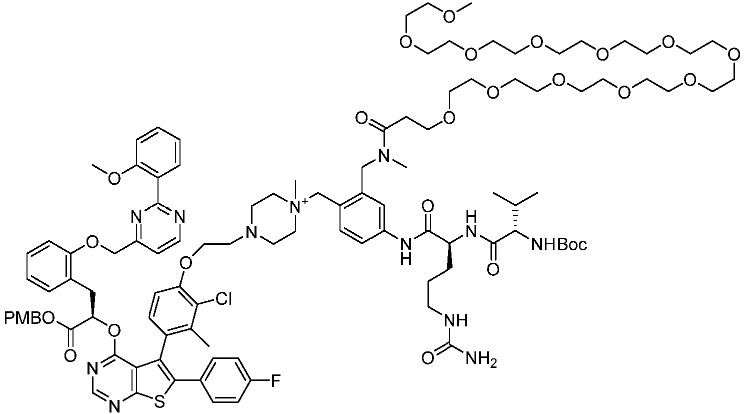

To 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (60 mg, 0.040 mmol, 1.0 equiv.) and 2,5-dioxopyrrolidin-1-yl 2,5,8,11,14,17,20,23,26,29,32,35-dodecaoxaoctatriacontan-38-oate (35.7 mg, 0.052 mmoles, 1.3 equiv.) dissolved in DMF (1 mL) was added DIPEA (0.035 mL, 0.200 mmoles, 5.0 equiv.). After standing for 18 h, DMSO (2 mL) was added and the solution was purified by RP-HPLC ISCO gold chromatography (10-70% MeCN/H2O, 0.1% TFA modifier). Upon lyophilization, 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: [M+Na]+=2092.9399; Rt=2.88 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-1-methylpiperazin-1-ium (L32-P1)

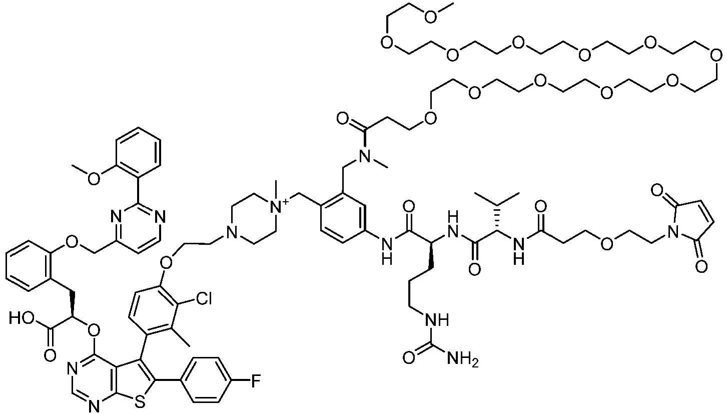

Following **GENERAL PROCEDURE 3** with 1-(4-((S)-2-((S)-2-((tert-butoxycarbonyl)amino)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (43.4 mg, 0.021 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(39-methyl-38-oxo-2,5,8,11,14,17,20,23,26,29,32,35-dodecaoxa-39-azatetracontan-40-yl)benzyl)-1-methylpiperazin-1-ium was obtained. HRMS: [M+Na]+= 2066.8799; Rt=2.44 min (5 min acidic method).

### Synthesis of 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

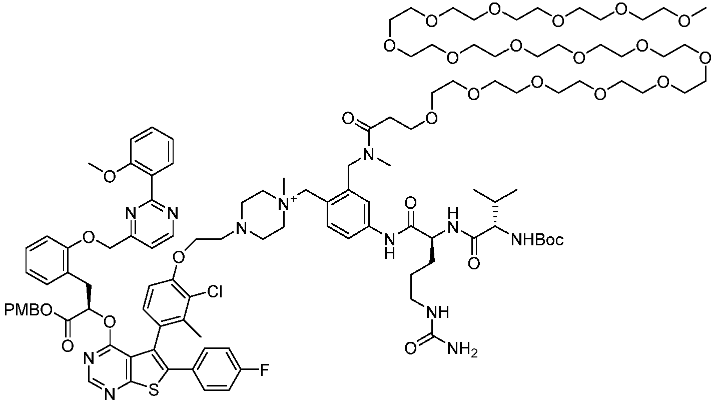

Following **GENERAL PROCEDURE 4** with 2,5-dioxopyrrolidin-1-yl 2,5,8,11,14,17,20,23,26,29,32,35,38,41,44,47-hexadecaoxapentacontan-50-oate (44.8 mg, 0.021 mmol, 1 eq), 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2246.0400; Rt=2.88 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-1-methylpiperazin-1-ium (L31-P1)

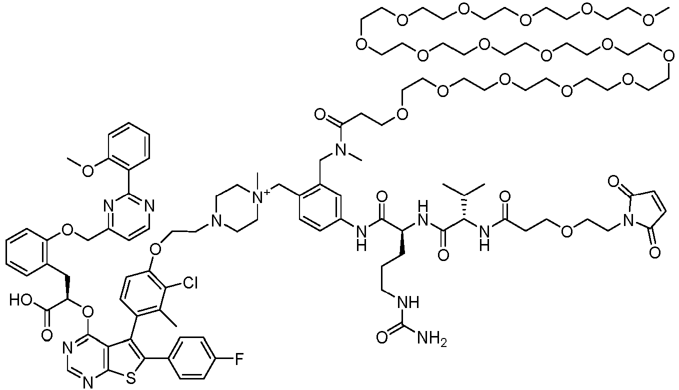

Following **GENERAL PROCEDURE 3** with 1-(4-((S)-2-((S)-2-((tert-butoxycarbonyl)amino)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (47.5 mg, 0.021 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(51-methyl-50-oxo-2,5,8,11,14,17,20,23,26,29,32,35,38,41,44,47-hexadecaoxa-51-azadopentacontan-52-yl)benzyl)-1-methylpiperazin-1-ium was obtained. HRMS: M+= 2221.0000; Rt=2.45 min (5 min acidic method).

### Syntheis of 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

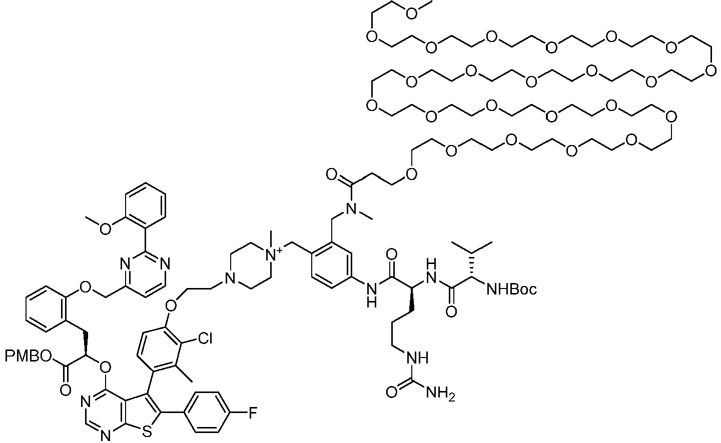

Following **GENERAL PROCEDURE 4** with 2,5-dioxopyrrolidin-1-yl 2,5,8,11,14,17,20,23,26,29,32,35,38,41,44,47,50,53,56,59,62,65,68,71-tetracosaoxatetraheptacontan-74-oate (52.6 mg, 0.043 mmol, 1.3 eq), 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2598.2500; Rt=2.88 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-1-methylpiperazin-1-ium (L30-P1)

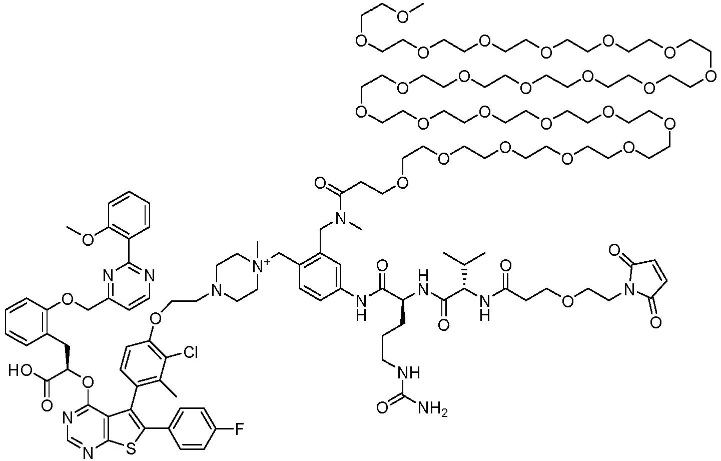

Following **GENERAL PROCEDURE 3** with 1-(4-((S)-2-((S)-2-((tert-butoxycarbonyl)amino)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (38.8 mg, 0.014 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(75-methyl-74-oxo-2,5,8,11,14,17,20,23,26,29,32,35,38,41,44,47,50,53,56,59,62,65,68,71-tetracosaoxa-75-azahexaheptacontan-76-yl)benzyl)-1-methylpiperazin-1-ium was obtained. HRMS: M+= 2573.2000; Rt=2.47 min (5 min acidic method).

### Synthesis of 1-(2-(((S)-2-((((9H-fluoren-9-yl)methoxy)carbonyl)amino)-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

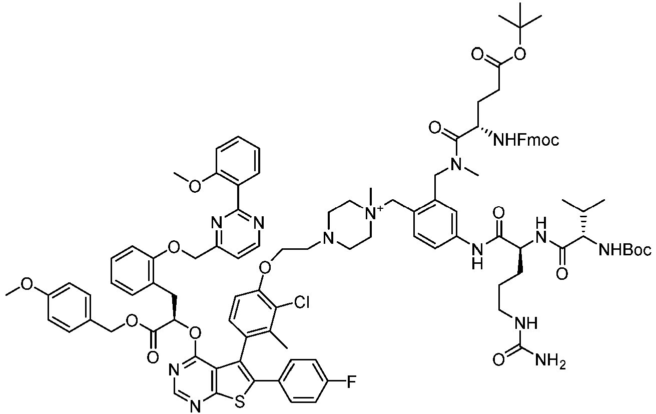

Following **GENERAL PROCEDURE 4** with 5-(tert-butyl) 1-(2,5-dioxopyrrolidin-1-yl) (((9H-fluoren-9-yl)methoxy)carbonyl)-L-glutamate (39.0 mg, 0.075 mmol, 1.1 equiv.), 1-(2-(((S)-2-((((9H-fluoren-9-yl)methoxy)carbonyl)amino)-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1906.8101; Rt=3.03 min (5 min acidic method).

### Synthesis of 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

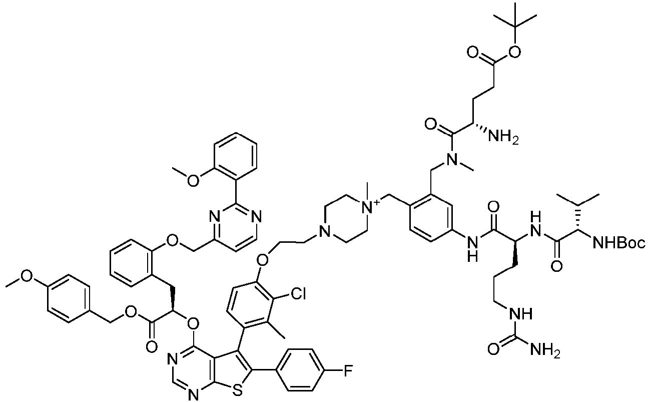

To 11-(2-(((S)-2-((((9H-fluoren-9-yl)methoxy)carbonyl)amino)-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (38.8 mg, 0.026 mmoles, 1.0 equiv.) dissolved in DMSO (2 mL) was added dimethylamine (0.192 mL, 0.384 mmoles, 20 equiv.). After standing for 4 hr, DMSO (2 mL) was added and the solution was purified by RP-HPLC ISCO gold chromatography (10-70% MeCN/H2O, 0.1% TFA modifier). Upon lyophilization, 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1684.4000; Rt=2.64 min (5 min acidic method).

### GENERAL PROCEDURE 5

### Synthesis of 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

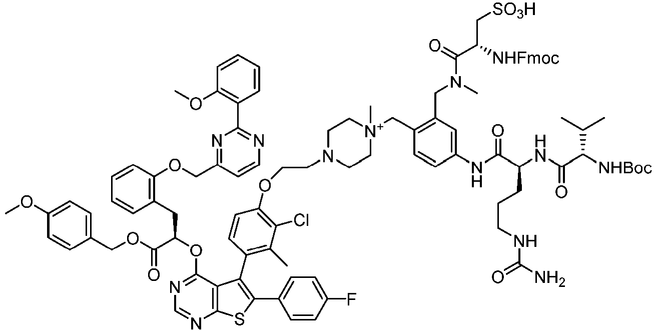

To (((9H-fluoren-9-yl)methoxy)carbonyl)(sulfo)-D-alanine (55.2 mg, 0.141 mmol, 1.3 eq) and 2-(3H-[1,2,3]triazolo[4,5-b]pyridin-3-yl)-1,1,3,3-tetramethylisouronium hexafluorophosphate(V) (41.3 mg, 0.109 mmoles, 1.0 equiv.) dissolved in DMF (2 mL) was added DIPEA (0.024 mL, 0.138 mmoles, 8.0 equiv.). After standing for 10 min, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (200 mg, 0.109 mmol, 1.0 eq) was added. After standing for 2.5 hr, DMSO (4 mL) was added and the solution was purified by RP-HPLC ISCO gold chromatography (10-70% MeCN/H2O, 0.1% TFA modifier). Upon lyophilization, 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1872.7000; Rt=3.09 min (5 min acidic method).

### Synthesis of 1-(2-(((R)-2-amino-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

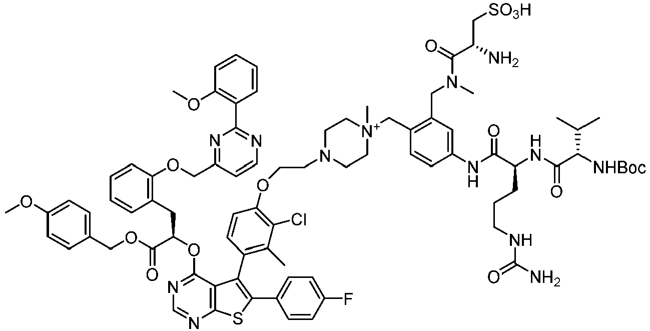

To 1-(2-(((R)-2-((((9H-fluoren-9-yl)methoxy)carbonyl)amino)-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (173 mg, 0.087 mmoles, 1.0 equiv.) dissolved in THF (2 mL) was added dimethylamine (0.870 mL, 1.740 mmoles, 20 equiv.). After standing for 5 hr, all volatiles were removed *in-vacuo.* The solid was triturated with diethyl ether. DMSO (2 mL) was added and the solution was purified by RP-HPLC ISCO gold chromatography (10-100% MeCN/H2O, 0.1% TFA modifier). Upon lyophilization, 1-(2-(((R)-2-amino-N-methyl-3-sulfopropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1650.5800; Rt=2.71 min (5 min acidic method).

### Synthesis of 1-(2-(((R)-2-amino-N-methyl-3-sulfopropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L70-P1)

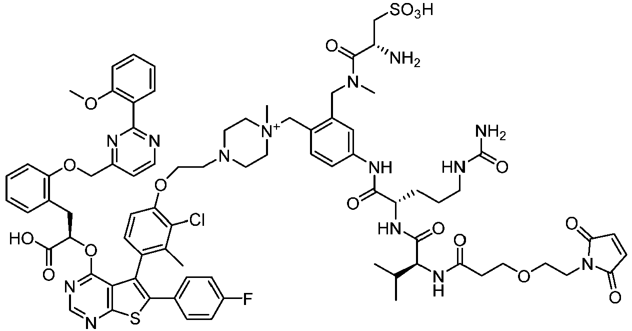

Following **GENERAL PROCEDURE 3** with 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (145 mg, 0.088 mmol, 1 eq), 1-(2-(((R)-2-amino-N-methyl-3-sulfopropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1625.5601; Rt=2.32 min (5 min acidic method).

### GENERAL PROCEDURE 6

### Synthesis of 1-(2-(((S)-5-(tert-butoxy)-N-methyl-5-oxo-2-(2-sulfoacetamido)pentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

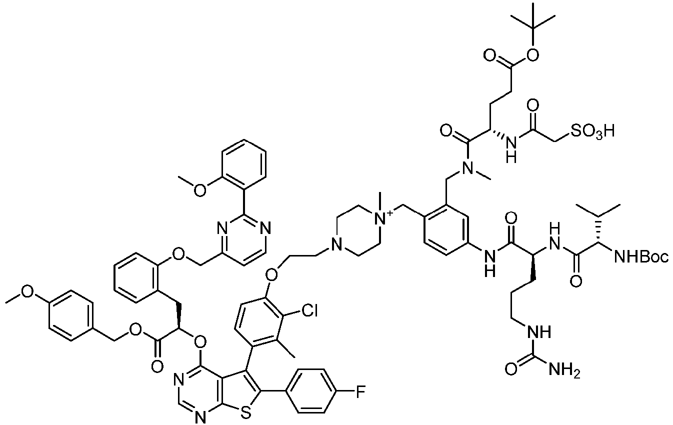

To 2-sulfoacetic acid (4.83 mg, 0.035 mmol, 2.0 equiv.) and 2-(3H-[1,2,3]triazolo[4,5-b]pyridin-3-yl)-1,1,3,3-tetramethylisouronium hexafluorophosphate(V) (9.85 mg, 0.026 mmoles, 1.5 equiv.) dissolved in DMF (1 mL) was added DIPEA (0.024 mL, 0.138 mmoles, 8.0 equiv.). After standing for 10 min, 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (29.1 mg, 0.017 mmoles, 1.0) was added. After standing for 45 min, DMSO (2 mL) was added and the solution was purified by RP-HPLC ISCO gold chromatography (10-70% MeCN/H2O, 0.1% TFA modifier). Upon lyophilization, 1-(2-(((S)-5-(tert-butoxy)-N-methyl-5-oxo-2-(2-sulfoacetamido)pentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1806.7000; Rt=3.10 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-4-carboxy-N-methyl-2-(2-sulfoacetamido)butanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L71-P1)

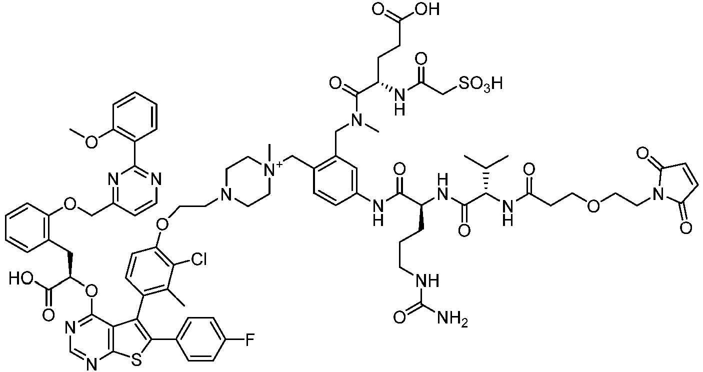

Following **GENERAL PROCEDURE 3** with 1-(2-(((S)-5-(tert-butoxy)-N-methyl-5-oxo-2-(2-sulfoacetamido)pentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (23.7 mg, 0.011 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-4-carboxy-N-methyl-2-(2-sulfoacetamido)butanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1725.5900; Rt=2.39 min (5 min acidic method).

### Synthesis of 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

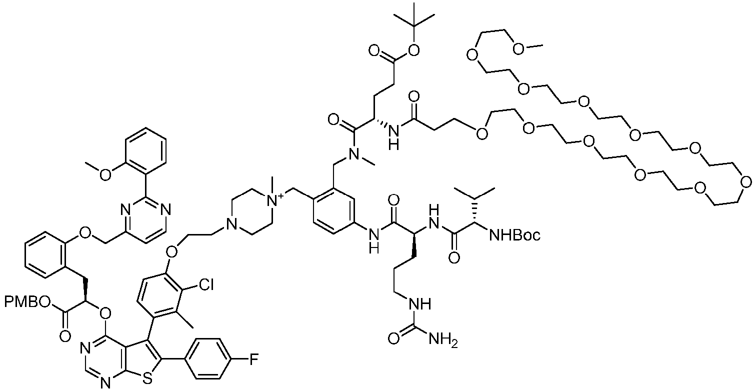

Following **GENERAL PROCEDURE 4** with 1-(2-(((S)-2-amino-5-(tert-butoxy)-N-methyl-5-oxopentanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.018 mmol, 1.0 eq) and Mal-PEG12-NHS Ester (18.31 mg, 0.027 mmol, 1.5 eq), 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2255.0400; Rt=2.97 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-((S)-40-(2-carboxyethyl)-42-methyl-38,41-dioxo-2,5,8,11,14,17,20,23,26,29,32,35-dodecaoxa-39,42-diazatritetracontan-43-yl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L72-P1)

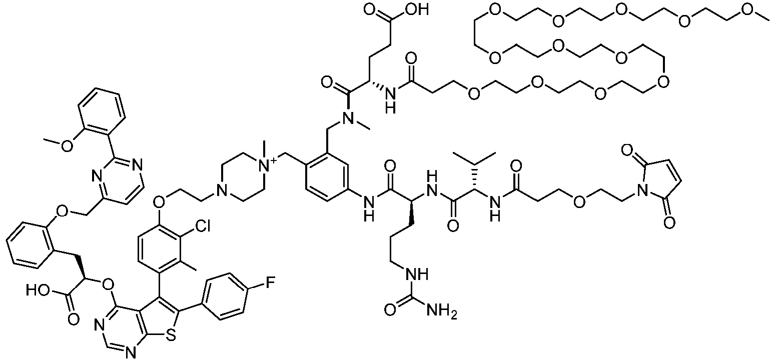

Following **GENERAL PROCEDURE 3** with 1-(2-((S)-40-(3-(tert-butoxy)-3-oxopropyl)-42-methyl-38,41-dioxo-2,5,8,11,14,17,20,23,26,29,32,35-dodecaoxa-39,42-diazatritetracontan-43-yl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20.1 mg, 8.91 µmmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-((S)-40-(2-carboxyethyl)-42-methyl-38,41-dioxo-2,5,8,11,14,17,20,23,26,29,32,35-dodecaoxa-39,42-diazatritetracontan-43-yl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: [M]+= 2173.9199; Rt=2.40 min (5 min acidic method).

### Synthesis of 1-(2-((2-(bis(2-(tert-butoxy)-2-oxoethyl)amino)-N-methylacetamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

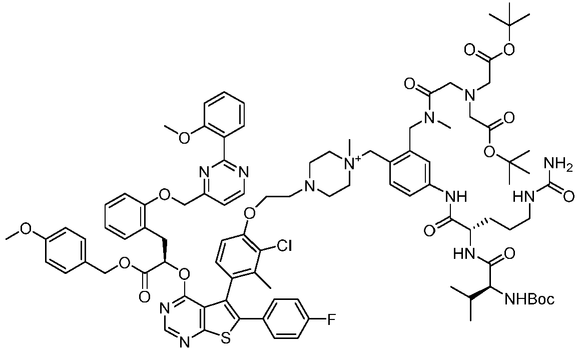

Following **GENERAL PROCEDURE 5** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (17.0 mg, 0.011 mmol, 1.0 eq) and bis(2-(tert-butoxy)-2-oxoethyl)glycine (10.97 mg, 0.017 mmol, 1.5 eq), 1-(2-((2-(bis(2-(tert-butoxy)-2-oxoethyl)amino)-N-methylacetamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1784.8000; Rt=3.31 min (5 min acidic method).

### Synthesis of 1-(2-((2-(bis(carboxymethyl)amino)-N-methylacetamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L67-P1)

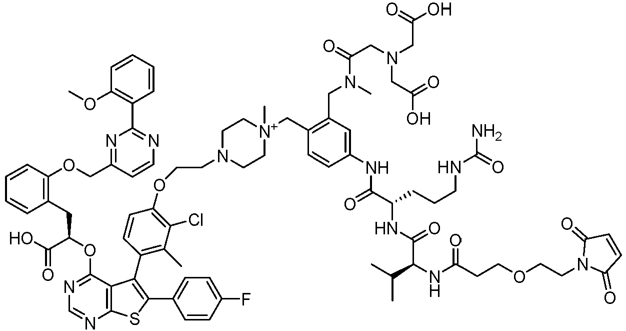

Following **GENERAL PROCEDURE 3** with 1-(2-((2-(bis(2-(tert-butoxy)-2-oxoethyl)amino)-N-methylacetamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (9.2 mg, 5.12 µmmol, 1 eq), 1-(2-((2-(bis(carboxymethyl)amino)-N-methylacetamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1647.6100; Rt=2.28 min (5 min acidic method).

### General Procedure 7

### Synthesis of 1-(2-(((S)-3-amino-4-(tert-butoxy)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

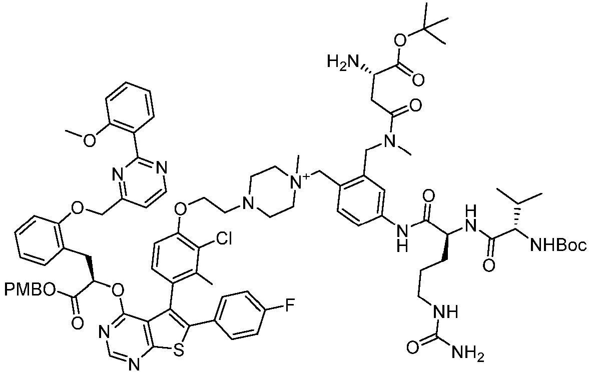

Reagents were used as DMF stock solution. To (S)-3-((((9H-fluoren-9-yl)methoxy)carbonyl)amino)-4-(tert-butoxy)-4-oxobutanoic acid (8.04 mg, 161 µL, 0.020 mmol, 1.2 equiv.) and 2-(3H-[1,2,3]triazolo[4,5-b]pyridin-3-yl)-1,1,3,3-tetramethylisouronium hexafluorophosphate(V) (6.81 mg, 681 µL, 0.018 mmoles, 1.1 equiv.) was added DIPEA (22.68 µL, 0.130 mmoles, 8.0 equiv.). After standing for 10 min, 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 353 µL, 0.016 mmoles, 1.0 equiv.) was added. After standing for 45 min, dimethyl amine (163 µl, 0.326 mmol) was added. After standing for 16 hours, DMSO (2 mL) was added and the solution was purified by RP-HPLC ISCO gold chromatography (10-100% MeCN/H2O, 0.1% TFA modifier). Upon lyophilization, 1-(2-(((S)-3-amino-4-(tert-butoxy)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=1670.2300; Rt=2.69 min (5 min acidic method).

### Synthesis of 1-(2-(((S)-4-(tert-butoxy)-N-methyl-4-oxo-3-(2-sulfoacetamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

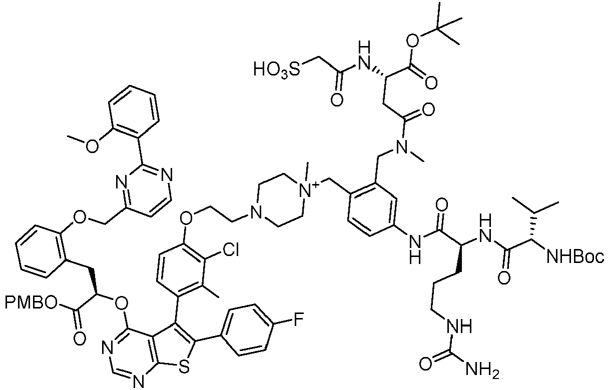

Following **GENERAL PROCEDURE 6** with 1-(2-(((S)-3-amino-4-(tert-butoxy)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20.6 mg, 10.23 µmol, 1 eq), 1-(2-(((S)-4-(tert-butoxy)-N-methyl-4-oxo-3-(2-sulfoacetamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1792.6899; Rt=3.17 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-(2-sulfoacetamido)propanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L79-P1)

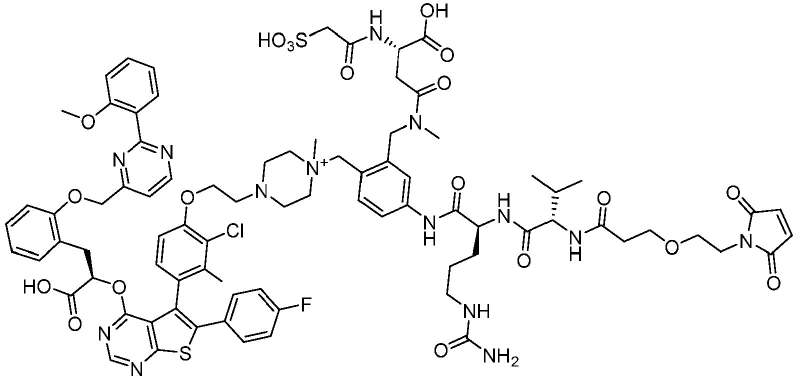

Following **GENERAL PROCEDURE 3** with 1-(2-(((S)-4-(tert-butoxy)-N-methyl-4-oxo-3-(2-sulfoacetamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (25.3 mg, 0.012 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-(2-sulfoacetamido)propanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1711.5699; Rt=2.48 min (5 min acidic method).

### Synthesis of 4-benzyl 1-(tert-butyl) ((3-(tert-butoxy)-3-oxopropoxy)carbonyl)-L-aspartate

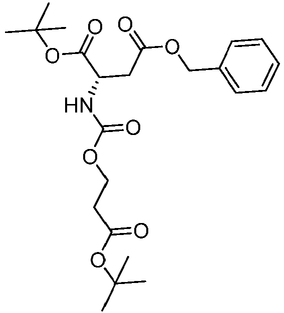

To tert-butyl 3-hydroxypropanoate (111 mg, 0.760 mmol, 1 equiv.) and bis(4-nitrophenyl) carbonate (289 mg, 0.950 mmol, 1.2 equiv.) dissolved in DMF (2 mL) was added DIPEA (0.221 mL, 1.267 mmoles, 2.0 equiv.). After standing for 1 hr, 4-benzyl 1-(tert-butyl) L-aspartate (200 mg, 0.633 mmol) was added. After standing for 16 hr, DMSO (4 mL) was added and the solution was purified by RP-HPLC ISCO gold chromatography (10-100% MeCN/H2O, 0.1% TFA modifier). Upon lyophilization, 4-benzyl 1-(tert-butyl) ((3-(tert-butoxy)-3-oxopropoxy)carbonyl)-L-aspartate was obtained. HRMS: [M+H]+=452.4; Rt=2.68 min (5 min acidic method).

### Synthesis of ((S)-4-(tert-butoxy)-3-(((3-(tert-butoxy)-3-oxopropoxy)carbonyl)amino)-4-oxobutanoic acid

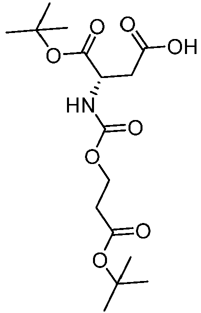

To 4-benzyl 1-(tert-butyl) ((3-(tert-butoxy)-3-oxopropoxy)carbonyl)-L-aspartate (52.7 mg, 0.117 mmol) dissolved in MeOH (2 mL) was added Palladium hydroxide (8.20 mg, 0.012 mmol, 0.1 equiv.). The reaction atmosphere was swtiched to hygrogen. After stirring for 16 hr, the reaction mixture was filtered through a celite pad. The filtrate was removed *in-vacuo* to obtain, 4-benzyl 1-(tert-butyl) ((3-(tert-butoxy)-3-oxopropoxy)carbonyl)-L-aspartate.

### Synthesis of 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

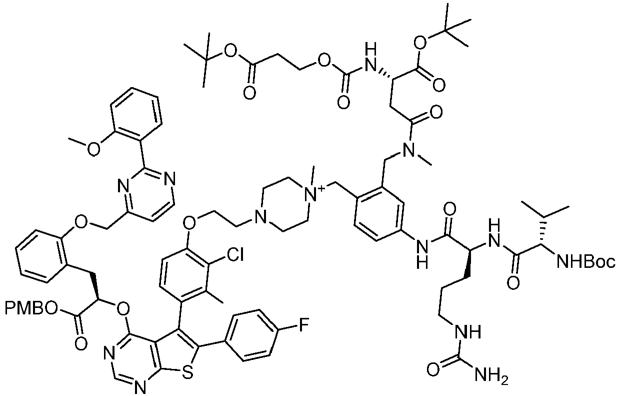

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 235 µL, 0.011 mmol, 1 eq) and 4-benzyl 1-(tert-butyl) ((3-(tert-butoxy)-3-oxopropoxy)carbonyl)-L-aspartate (7.84 mg, 204 µL, 0.022 mmol, 2 equiv.), 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1842.8000; Rt=3.23 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(((2-carboxyethoxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L102-P1)

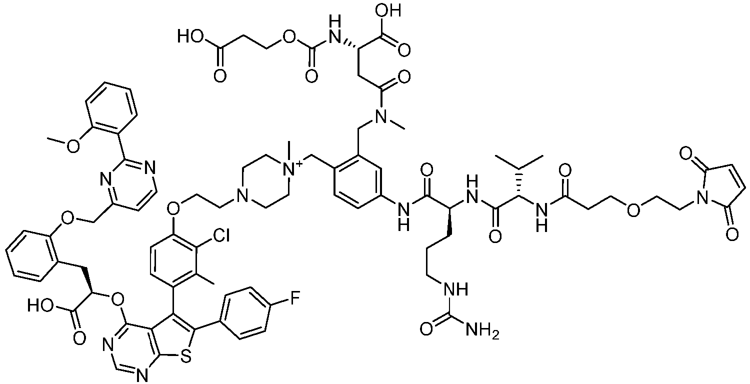

Following **GENERAL PROCEDURE 3** with 1-(2-((S)-5-(tert-butoxycarbonyl)-2,13,13-trimethyl-3,7,11-trioxo-8,12-dioxa-2,6-diazatetradecyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (17.6 mg, 0.008 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(((2-carboxyethoxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1705.6200; Rt=2.41 min (5 min acidic method).

### Synthesis of tert-butyl N-(((9H-fluoren-9-yl)methoxy)carbonyl)-O-((4-nitrophenoxy)carbonyl)-L-serinate

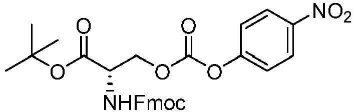

To tert-butyl (((9H-fluoren-9-yl)methoxy)carbonyl)-L-serinate (300 mg, 0.782 mmol, 1 equiv.) and bis(4-nitrophenyl) carbonate (357 mg, 1.174 mmol, 1.5 equiv.) dissolved in DMF (2 mL) was added DIPEA (0.136 mL, 0.782 mmoles, 1.0 equiv.). After standing for 16 hr, DMSO (4 mL) was added and the solution was purified by RP-HPLC ISCO gold chromatography (10-100% MeCN/H2O, 0.1% TFA modifier). Upon lyophilization, tert-butyl N-(((9H-fluoren-9-yl)methoxy)carbonyl)-O-((4-nitrophenoxy)carbonyl)-L-serinate was obtained. HRMS: M+= 566.4; Rt=3.01 min (5 min acidic method).

### Synthesis of 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

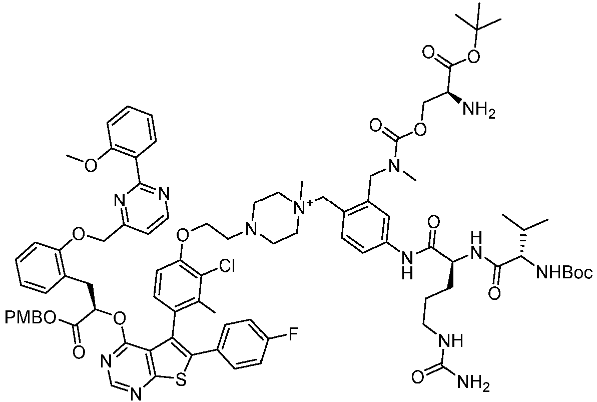

To tert-butyl N-(((9H-fluoren-9-yl)methoxy)carbonyl)-O-((4-nitrophenoxy)carbonyl)-L-serinate (7.14 mg, 143 µL, 0.013mmol, 1.2 equiv.) and 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 235 µL, 0.011 mmol, 1.0 equiv.) was added DIPEA (15.1 µL, 0.087 mmoles, 8.0 equiv.). After standing for 16 hr, Dimethyl amine (109 µl, 0.217 mmol, 20 equiv.) was added. The reaction was stirred at RT for 1 hr. DMSO (4 mL) was added and the solution was purified by RP-HPLC ISCO gold chromatography (10-100% MeCN/H2O, 0.1% TFA modifier). Upon lyophilization, 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1686.7200; Rt=2.71 min (5 min acidic method).

### Synthesis 1-(2-(((((S)-3-(tert-butoxy)-3-oxo-2-(2-sulfoacetamido)propoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

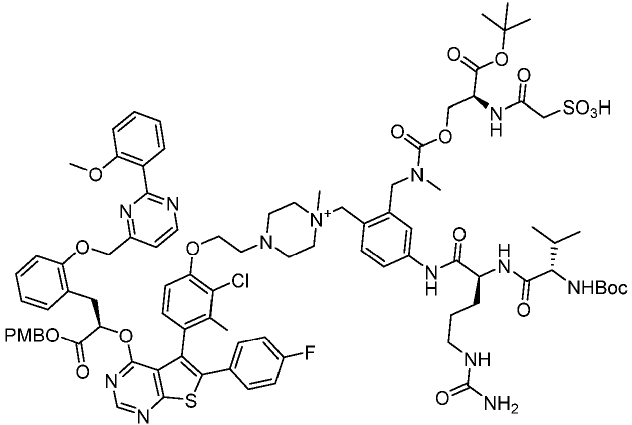

Following **GENERAL PROCEDURE 6** with 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (22.9 mg, 0.011 mmol, 1 eq), 1-(2-(((((S)-3-(tert-butoxy)-3-oxo-2-(2-sulfoacetamido)propoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1808.6899; Rt=3.18 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((S)-2-carboxy-2-(2-sulfoacetamido)ethoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L77-P1)

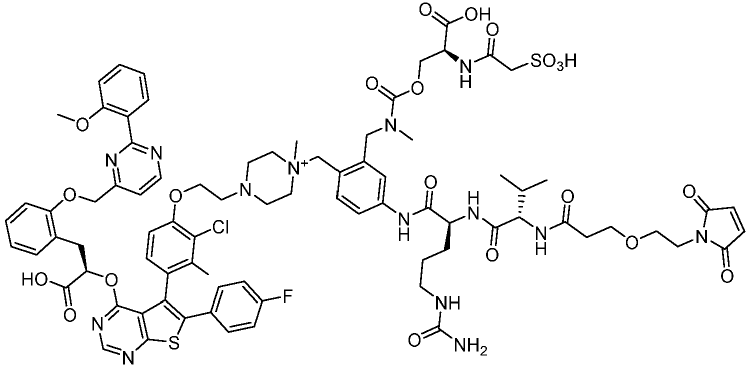

Following **GENERAL PROCEDURE 3** with 1-(2-(((((S)-2-amino-3-(tert-butoxy)-3-oxopropoxy)carbonyl)(methyl)amino)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (15.3 mg, 7.11 µmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((((S)-2-carboxy-2-(2-sulfoacetamido)ethoxy)carbonyl)(methyl)amino)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1727.5800; Rt=2.47 min (5 min acidic method).

### Synthesis of 4-benzyl 1-(tert-butyl) (4-(diethoxyphosphoryl)butanoyl)-L-aspartate

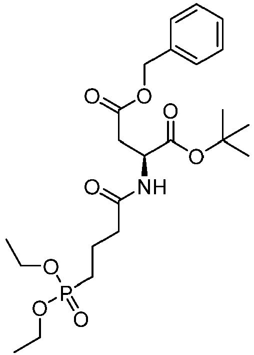

To 4-benzyl 1-(tert-butyl) L-aspartate (200 mg, 0.633 mmol, 1.0 equiv.) and 4-(diethoxyphosphoryl)butanoic acid (213 mg, 0.950 mmol, 1.5 equiv.) dissolved in DMF (2 mL) was added dicyclohexylmethanediimine (157 mg, 0.760 mmol, 1.2 equiv.), 1H-[1,2,3]triazolo[4,5-b]pyridin-1-ol hydrate (146 mg, 0.950 mmol, 1.5 equiv.) and DIPEA (0.110 mL, 0.633 mmol, 1.0 equiv.). After standing for 16 hr, DMSO (4 mL) was added and the solution was purified by RP-HPLC ISCO gold chromatography (10-100% MeCN/H2O, 0.1% TFA modifier). Upon lyophilization, 4-benzyl 1-(tert-butyl) (4-(diethoxyphosphoryl)butanoyl)-L-aspartate was obtained. HRMS: [M+H]+=486.4; Rt=2.15 min (5 min acidic method).

### Synthesis of (S)-4-(tert-butoxy)-3-(4-(diethoxyphosphoryl)butanamido)-4-oxobutanoic acid

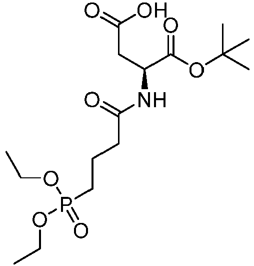

To 4-benzyl 1-(tert-butyl) (4-(diethoxyphosphoryl)butanoyl)-L-aspartate (YUBI5-040-EXP082-001 (100 mg, 0.206 mmol, 1.0 equiv.) dissolved in MeOH (2 mL) was added Palladium hydroxide (14.46 mg, 0.021 mmol, 0.1 equiv.). The reaction atmosphere was switched to hydrogen. After stirring for 16 hr, the reaction mixture was filtered through a celite pad. The filtrate was removed *in-vacuo* to obtain (S)-4-(tert-butoxy)-3-(4-(diethoxyphosphoryl)butanamido)-4-oxobutanoic acid. HRMS: [M+H]+= 396.3; Rt=1.35 min (5 min acidic method).

### Synthesis of 1-(2-(((S)-4-(tert-butoxy)-3-(4-(diethoxyphosphoryl)butanamido)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

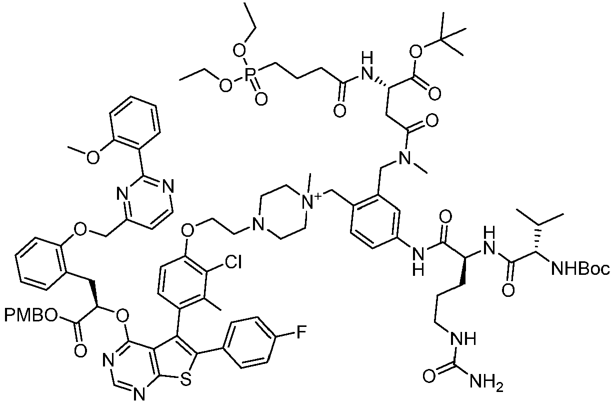

Following **GENERAL PROCEDURE** 7 with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 353 µL, 0.016 mmol, 1 eq) and (S)-4-(tert-butoxy)-3-(4-(diethoxyphosphoryl)butanamido)-4-oxobutanoic acid (12.87 mg, 161 µl, 0.033 mmol, 2 equiv.), 1-(2-(((S)-4-(tert-butoxy)-3-(4-(diethoxyphosphoryl)butanamido)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. MS: M/2+= 940.3; Rt=2.60 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(4-(diethoxyphosphoryl)butanamido)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L103-P1)

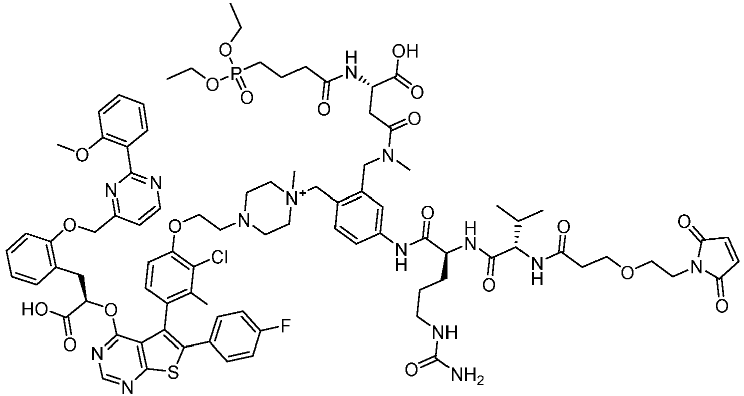

Following **GENERAL PROCEDURE 3** with 1-(2-(((S)-4-(tert-butoxy)-3-(4-(diethoxyphosphoryl)butanamido)-N-methyl-4-oxobutanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 0.009 mmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-3-(4-(diethoxyphosphoryl)butanamido)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1795.6700; Rt=2.44 min (5 min acidic method).

### Synthesis of 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

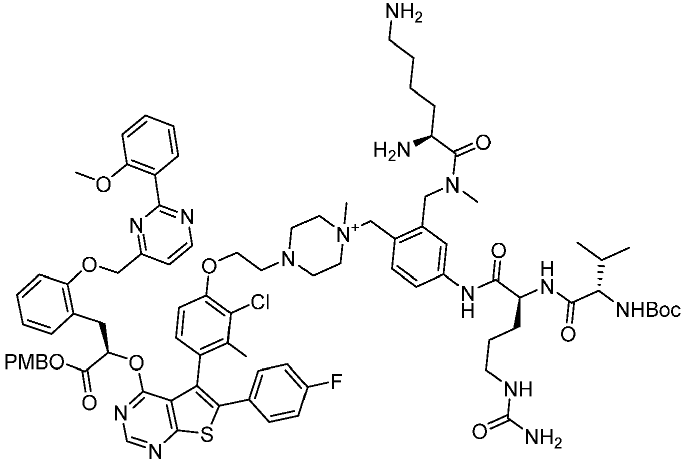

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (20 mg, 0.011 mmol, 1 eq) and (S)-2,5-bis((((9H-fluoren-9-yl)methoxy)carbonyl)amino)pentanoic acid (7.51 mg, 0.013 mmol, 1.2 eq), 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1627.4000; Rt=2.48 min (5 min acidic method).

### Synthesis of 1-(4-((S)-2-((S)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

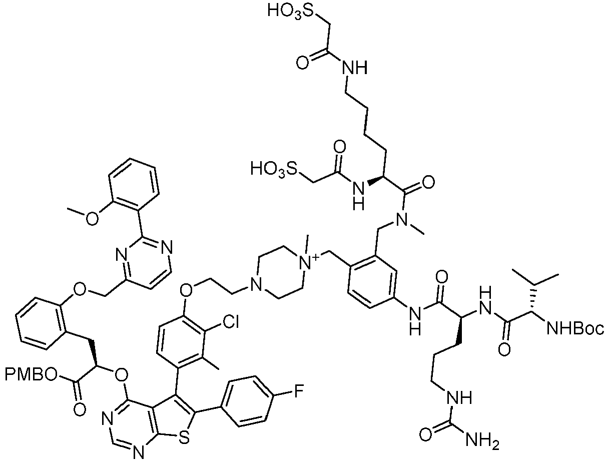

Following **GENERAL PROCEDURE 6** with 1-(4-((S)-2-((S)-2-((tert-butoxycarbonyl)amino)-3-methylbutanamido)-5-ureidopentanamido)-2-(((S)-2,6-diamino-N-methylhexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (9.7 mg, 0.0049 mmol, 1 eq), 1-(4-((S)-2-((S)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1871.6700; Rt=3.**02 min (5 min acidic method).**

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((S)-N-methyl-2,6-bis(2-sulfoacetamido)hexanamido)methyl)benzyl)-1-methylpiperazin-1-ium (L78-P1)

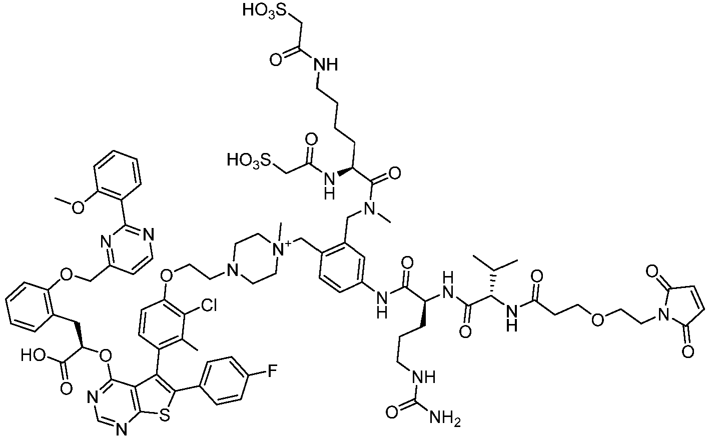

Following **GENERAL PROCEDURE 3** with -(4-((S)-2-((S)-2-((tert-butoxycarbonyl)amino)-3-methylbutanamido)-5-ureidopentanamido)-2-(((S)-N-methyl-2,6-bis(2-sulfoacetamido)hexanamido)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (15 mg, 7.55 µmol, 1 eq), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((S)-N-methyl-2,6-bis(2-sulfoacetamido)hexanamido)methyl)benzyl)-1-methylpiperazin-1-ium was obtained. HRMS: M+= 1846.6000; Rt=2.49 min (5 min acidic method).

### Synthesis of 4-benzyl 1-(tert-butyl) ((2S,3S,4S,5R,6S)-3,4,5,6-tetraacetoxytetrahydro-2H-pyran-2-carbonyl)-L-aspartate

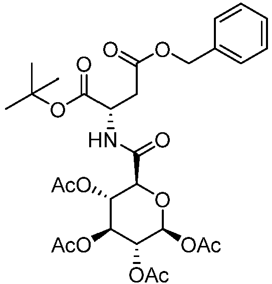

To (2S,3S,4S,5R,6S)-3,4,5,6-tetraacetoxytetrahydro-2H-pyran-2-carboxylic acid (344 mg, 0.950 mmol) and 4-benzyl 1-(tert-butyl) L-aspartate (300 mg, 0.950 mmol) dissolved in DMF (3.2 mL) was added DIPEA (0.165 mL, 0.950 mmol, 1.0 equiv.), 1H-[1,2,3]triazolo[4,5-b]pyridin-1-ol hydrate (154 mg, 0.997 mmol, 1.05 equiv.) and 3-(((ethylimino)methylene)amino)-N,N-dimethylpropan-1-amine hydrochloride (191 mg, 0.997 mmol, 1.05 equiv.) were added. After standing for 16 hr, DMSO (4 mL) was added and the solution was purified by RP-HPLC ISCO gold chromatography (10-70% MeCN/H2O, 0.1% TFA modifier). Upon lyophilization, 4-benzyl 1-(tert-butyl) ((2S,3S,4S,5R,6S)-3,4,5,6-tetraacetoxytetrahydro-2H-pyran-2-carbonyl)-L-aspartate was obtained. HRMS: M+=641.5; Rt=2.55 min (5 min acidic method).

### Synthesis of (S)-4-(tert-butoxy)-4-oxo-3-((2S,3S,4S,5R,6S)-3,4,5,6-tetraacetoxytetrahydro-2H-pyran-2-carboxamido)butanoic acid

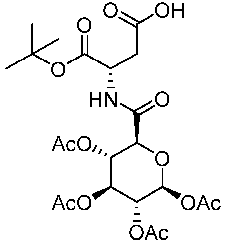

To 4-benzyl 1-(tert-butyl) ((2S,3S,4S,5R,6S)-3,4,5,6-tetraacetoxytetrahydro-2H-pyran-2-carbonyl)-L-aspartate (100 mg, 0.160 mmol, 1.0 eq) dissolved in MeOH (2 mL) was added Palladium hydroxide (11.26 mg, 0.016 mmol, 0.1 equiv.). The reaction atmosphere was swtiched to hygrogen. After stirring for 16 hr, the reaction mixture was filtered through a celite pad. The filtrate was removed in-vacuo to obtain (S)-4-(tert-butoxy)-4-oxo-3-((2S,3S,4S,5R,6S)-3,4,5,6-tetraacetoxytetrahydro-2H-pyran-2-carboxamido)butanoic acid. HRMS: [M-H]-= 532.3, Rt=1.71 min (5 min acidic method).

### Synthesis of 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

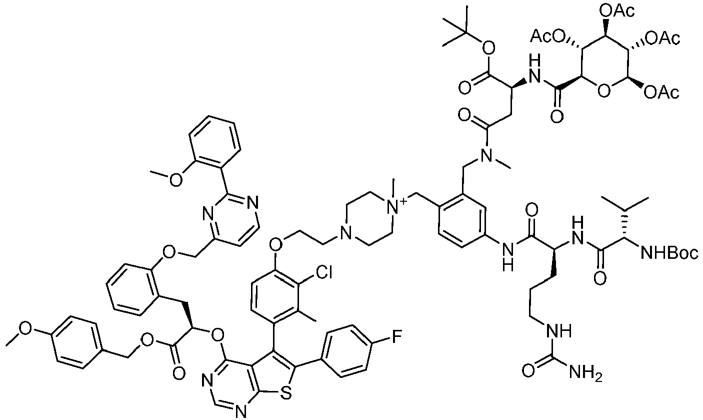

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.016 mmol, 1.0 eq) and (S)-4-(tert-butoxy)-4-oxo-3-((2S,3S,4S,5R,6S)-3,4,5,6-tetraacetoxytetrahydro-2H-pyran-2-carboxamido)butanoic acid (12.2 mg, 968 µL, 0.023 mmol, 1.4 eq), 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2014.7800; Rt=3.21 min (5 min acidic method).

### Synthesis of 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

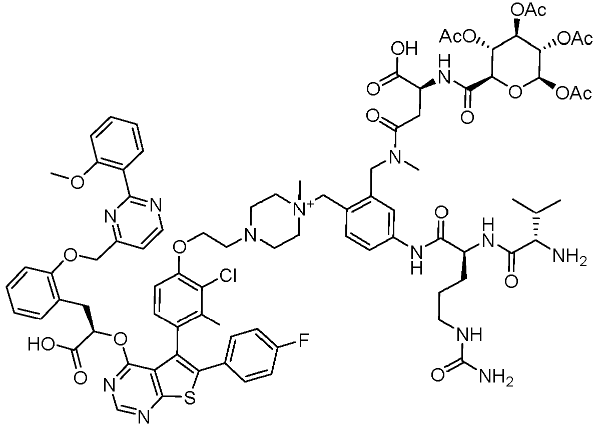

To 1-(2-(((S)-4-(tert-butoxy)-N-methyl-4-oxo-3-((2R,3R,4R,5S,6R)-3,4,5,6-tetraacetoxytetrahydro-2H-pyran-2-carboxamido)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (22.7 mg, 0.009 mmoles, 1.0 equiv.) dissolved in DCM (32mL) was added TFA (0.67 mL). After stirring for 45 min, the solvent was removed *in-vacuo* to obtain 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+=1738.6200; Rt=2.30 min (5 min acidic method).

### Synthesis of 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

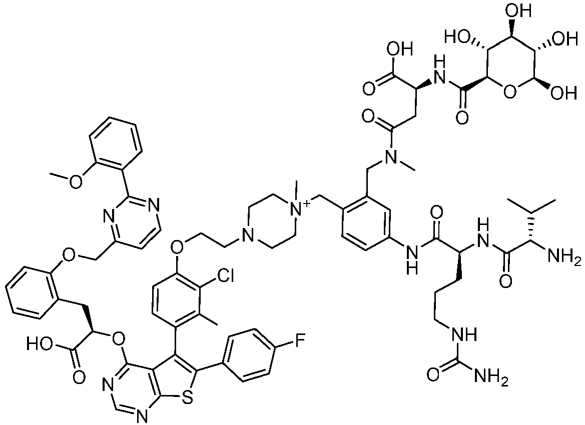

To 1-(4-((S)-2-((S)-2-amino-3-methylbutanamido)-5-ureidopentanamido)-2-(((S)-3-carboxy-N-methyl-3-((2R,3R,4R,5S,6R)-3,4,5,6-tetraacetoxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (22.7 mg, 0.009 mmoles, 1.0 equiv.) dissolved in THF (1 mL) and MeOH (1 mL) was added lithium hydroxide (5.04 mg, 0.120 mmol, 10 equiv.). After stirring for 2 hour, the solvent was removed *in-vacuo.* Water (1 mL), TFA (0.2 mL), MeCN (1 mL) and DMSO (4 mL) were added and the solution was purified by RP-HPLC ISCO gold chromatography (10-70% MeCN/H2O, 0.1% TFA modifier). Upon lyophilization, 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methylbenzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1570.5900; Rt=2.02 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-((2R,3R,4R,5S,6S)-3,4,5,6-tetrahydroxytetrahydro-2H-pyran-2-carboxamido)propanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L68-P1)

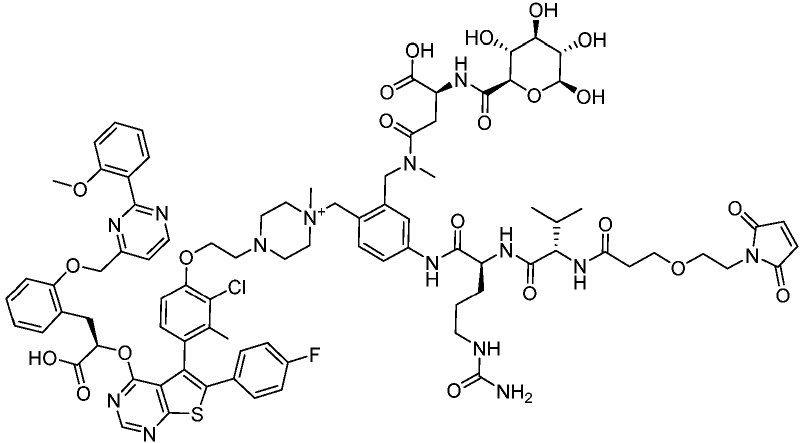

To 1-(4-((S)-2-((S)-2-amino-3-methylbutanamido)-5-ureidopentanamido)-2-(((S)-3-carboxy-N-methyl-3-((2R,3R,4R,5S,6S)-3,4,5,6-tetrahydroxytetrahydro-2H-pyran-2-carboxamido)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (10.8 mg, 0.0056 mmoles, 1.0 equiv.) and 2,5-dioxopyrrolidin-1-yl 3-(2-(2,5-dioxo-2,5-dihydro-1H-pyrrol-1-yl)ethoxy)propanoate (5.25 mg, 0.017 mmol, 3.5 equiv.) dissolved in DMF (1 mL) was added DIPEA (7.86 µL, 0.045 mmol, 8 equiv.). After standing for 1.5 hour, DMSO (2mL) were added and the solution was purified by RP-HPLC ISCO gold chromatography (10-70% MeCN/H2O, 0.1% TFA modifier). Upon lyophilization, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((S)-3-carboxy-N-methyl-3-((2R,3R,4R,5S,6S)-3,4,5,6-tetrahydroxytetrahydro-2H-pyran-2-carboxamido)propanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+=1765.6500; Rt=2.31 min (5 min acidic method).

### Synthesis of 4-benzyl 1-(tert-butyl) ((((3R,4S,5S,6S)-3,4,5-triacetoxy-6-(methoxycarbonyl)tetrahydro-2H-pyran-2-yl)oxy)carbonyl)-L-aspartate

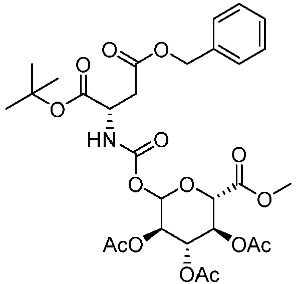

To ((3R,4S,5S,6S)-2-hydroxy-6-(methoxycarbonyl)tetrahydro-2H-pyran-3,4,5-triyl triacetate (254 mg, 0.760 mmol, 1.2 equiv.) and bis(4-nitrophenyl) carbonate (289 mg, 0.950 mmol, 1.5 equiv.) dissolved in DMF (2 mL) was added DIPEA (0.221 mL, 1.267 mmol, 2.0 equiv.). After standing for 1 hr, 4-benzyl 1-(tert-butyl) L-aspartate (200 mg, 0.633 mmol, 1.0 equiv.) was added. After standing for 16 hr, DMSO (6 mL) was added and the solution was purified by RP-HPLC ISCO gold chromatography (10-70% MeCN/H2O, 0.1% TFA modifier). Upon lyophilization, 4-benzyl 1-(tert-butyl) ((((3R,4S,5S,6S)-3,4,5-triacetoxy-6-(methoxycarbonyl)tetrahydro-2H-pyran-2-yl)oxy)carbonyl)-L-aspartate was obtained. HRMS: [M-H]-= 638.4; Rt=2.61 min (5 min acidic method).

### Synthesis of (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid

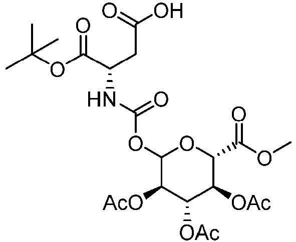

To 4-benzyl 1-(tert-butyl) ((((3R,4S,5S,6S)-3,4,5-triacetoxy-6-(methoxycarbonyl)tetrahydro-2H-pyran-2-yl)oxy)carbonyl)-L-aspartate (50 mg, 0.078 mmol, 1.0 eq) dissolved in MeOH (2 mL) was added Palladium hydroxide (5.49 mg, 7.82 µmol, 0.1 equiv.). The reaction atmosphere was swtiched to hygrogen. After stirring for 16 hr, the reaction mixture was filtered through a celite pad. The filtrate was removed in-vacuo to obtain (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid. HRMS: [M-H]-: 548.4, Rt=1.79 min (5 min acidic method).

### Synthesis of 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

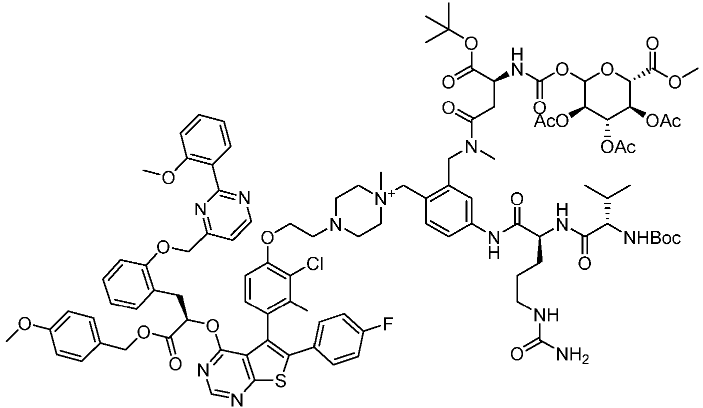

Following **GENERAL PROCEDURE 7** with 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (30 mg, 0.016 mmol, 1.0 eq) and (3S)-4-(tert-butoxy)-4-oxo-3-(((((3R,4S,5S,6S)-3,4,5-triacetoxy-6-(methoxycarbonyl)tetrahydro-2H-pyran-2-yl)oxy)carbonyl)amino)butanoic acid (12.5 mg, 0.250 mL, 0.023 mmol ,1.4 eq), 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+=2030.7900; Rt=3.19 min (5 min acidic method).

### Synthesis of 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

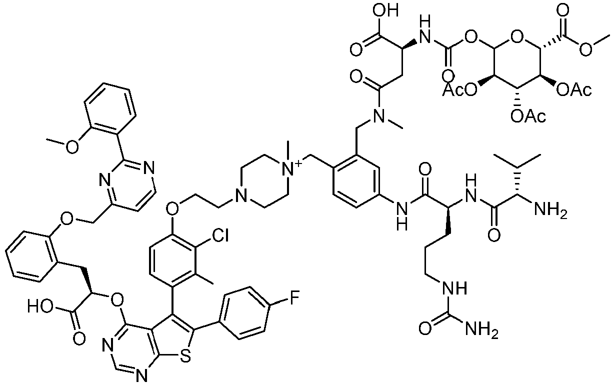

To 1-(2-(((3S)-4-(tert-butoxy)-N-methyl-4-oxo-3-(((((3R,4S,5S,6S)-3,4,5-triacetoxy-6-(methoxycarbonyl)tetrahydro-2H-pyran-2-yl)oxy)carbonyl)amino)butanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (25.8 mg, 10.86 µmol, 1.0 equiv.) dissolved in DCM (2 mL) was added TFA (0.67 mL). After stirring for 2 hrs, the solvent was removed *in-vacuo* to obtain 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+=1754.6200; Rt=2.31 min (5 min acidic method).

### Synthesis of 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

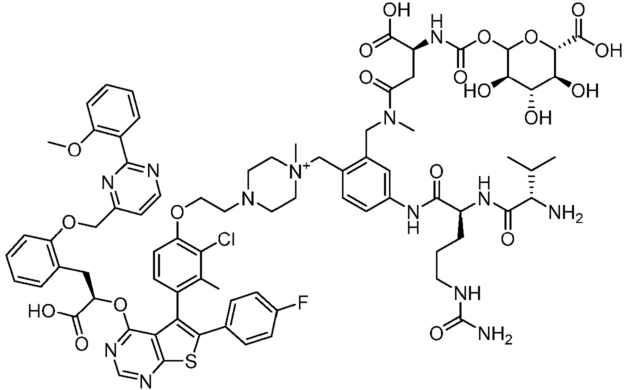

To 1-(4-((S)-2-((S)-2-amino-3-methylbutanamido)-5-ureidopentanamido)-2-(((3S)-3-carboxy-N-methyl-3-(((((3R,4S,5S,6S)-3,4,5-triacetoxy-6-(methoxycarbonyl)tetrahydro-2H-pyran-2-yl)oxy)carbonyl)amino)propanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (26 mg, 0.012 mmol, 1.0 equiv.) dissolved in THF (1 mL) and MeOH (1 mL) was added lithium hydroxide (5.20 mg, 0.124 mmol, 10 equiv.). After stirring for 2 hour, the solvent was removed *in-vacuo.* Water (1 mL), TFA (0.2 mL), MeCN (1 mL) and DMSO (4 mL) were added and the solution was purified by RP-HPLC ISCO gold chromatography (10-70% MeCN/H2O, 0.1% TFA modifier). Upon lyophilization, 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=1614.5800; Rt=2.04 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L69-P1)

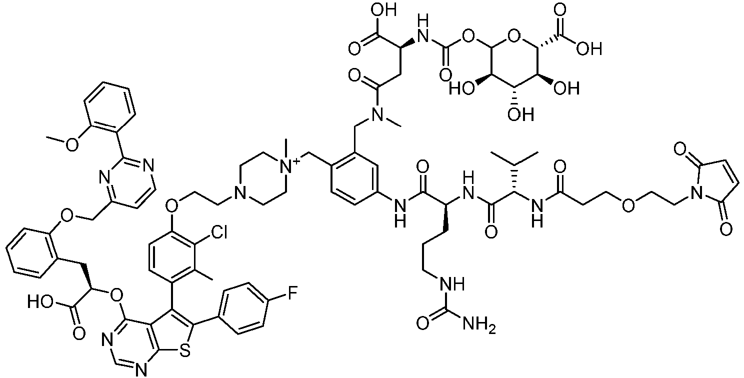

To 1-(4-((S)-2-((S)-2-amino-3-methylbutanamido)-5-ureidopentanamido)-2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (10 mg, 5.11 µmol, 1.0 equiv.) and 2,5-dioxopyrrolidin-1-yl 3-(2-(2,5-dioxo-2,5-dihydro-1H-pyrrol-1-yl)ethoxy)propanoate (4.75 mg, 0.015 mmol, 3.5 equiv.) dissolved in DMF (1 mL) was added DIPEA (7.12 µl, 0.041 mmol, 8 equiv.). After standing for 1.5 hour, DMSO (2mL) were added and the solution was purified by RP-HPLC ISCO gold chromatography (10-70% MeCN/H2O, 0.1% TFA modifier). Upon lyophilization, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((3S)-3-carboxy-3-(((((3R,4S,5S,6S)-6-carboxy-3,4,5-trihydroxytetrahydro-2H-pyran-2-yl)oxy)carbonyl)amino)-N-methylpropanamido)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+=1809.6300; Rt=2.32 min (5 min acidic method).

### Synthesis of 1-(2-((3-(2-(2-aminoethoxy)ethoxy)-N-methylpropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

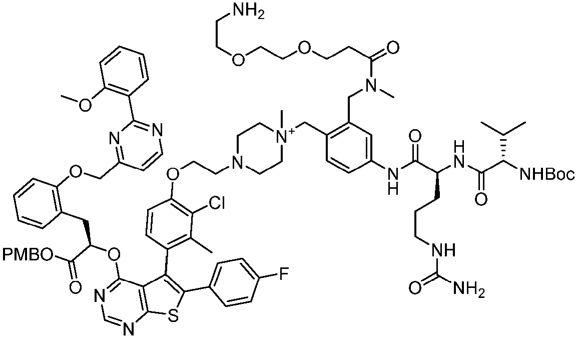

A mixture of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (45 mg, 0.026 mmol), 1-(9H-fluoren-9-yl)-3-oxo-2,7,10-trioxa-4-azatridecan-13-oic acid (12 mg, 0.030 mmol), HBTU (12 mg, 0.032 mmol), and DIPEA (0.023 mL, 0.13 mmol) in DMF (1 mL) was stirred at RT for 30 min. Me₂NH (2M in THF, 0.065 mL, 0.13 mmol) was added, and the mixture was stirred at RT for 1 h. Additional amount of Me₂NH (2M in THF, 0.1 mL, 0.2 mmol) was added. The mixture was continued to be stirred at RT for 1 h, diluted with DMSO (3 mL), and the solution was purified by RP-HPLC ISCO gold chromatography (MeCN/H₂O, 0.1% TFA modifier). Upon lyophilization, 1-(2-((3-(2-(2-aminoethoxy)ethoxy)-N-methylpropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1658.7200, Rt=2.57 min (5 min acidic method).

### Synthesis of 1-(4-((S)-2-((S)-2-((tert-butoxycarbonyl)amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

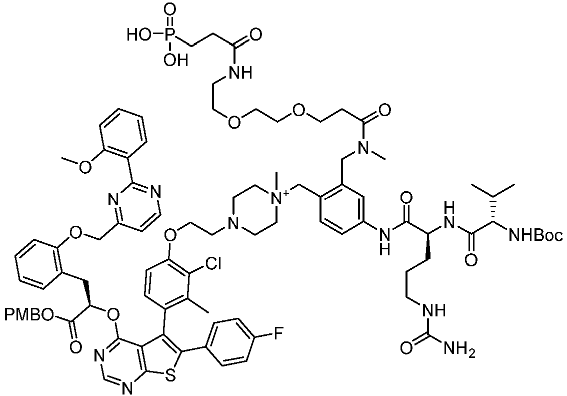

A mixture of 3-phosphopropionic acid (11 mg, 0.071 mmol), HBTU (27 mg, 0.071 mmol), and DIPEA (0.060 mL, 0.34 mmol) in DMF (0.5 mL) was stirred at RT for 10 min. This mixture was added to a solution of 1-(2-((3-(2-(2-aminoethoxy)ethoxy)-N-methylpropanamido)methyl)-4-((S)-2-((S)-2-((tert-butoxycarbonyl)amino)-3-methylbutanamido)-5-ureidopentanamido)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (40 mg, 0.021 mmol) and DIPEA (0.010 mL, 0.057 mmol) in DMF (0.5 mL). The mixture was stirred at RT for 2 days. The mixture was diluted with DMSO (3 mL), and the solution was purified by RP-HPLC ISCO gold chromatography (MeCN/H₂O, 0.1% TFA modifier). Upon lyophilization, 1-(4-((S)-2-((S)-2-((tert-butoxycarbonyl)amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 1794.7100, Rt=2.78 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(2-methyl-3,13-dioxo-15-phosphono-6,9-dioxa-2,12-diazapentadecyl)benzyl)-1-methylpiperazin-1-ium (L41-P1)

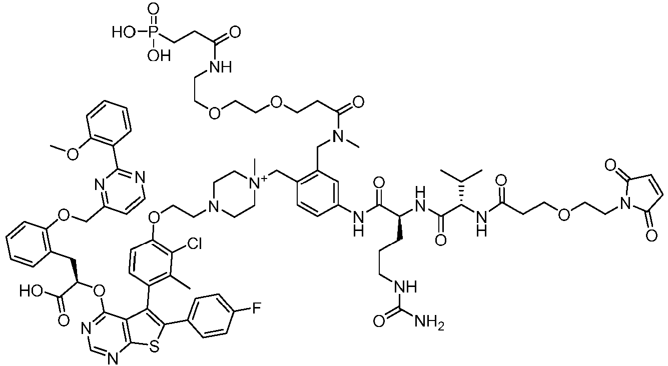

Following **GENERAL PROCEDURE 3** (except that the product after the first step with TFA/CH₂Cl₂ was purified by RP-HPLC ISCO gold chromatography [MeCN/H₂O, 0.1% NH₄OH modifier]) with 1-(4-((S)-2-((S)-2-((tert-butoxycarbonyl)amino)-3-methylbutanamido)-5-ureidopentanamido)-2-(2-methyl-3,13-dioxo-15-phosphono-6,9-dioxa-2,12-diazapentadecyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(2-methyl-3,13-dioxo-15-phosphono-6,9-dioxa-2,12-diazapentadecyl)benzyl)-1-methylpiperazin-1-ium was obtained. HRMS: M+= 1769.4500, Rt=2.33 min (5 min acidic method).

### Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11,14,17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

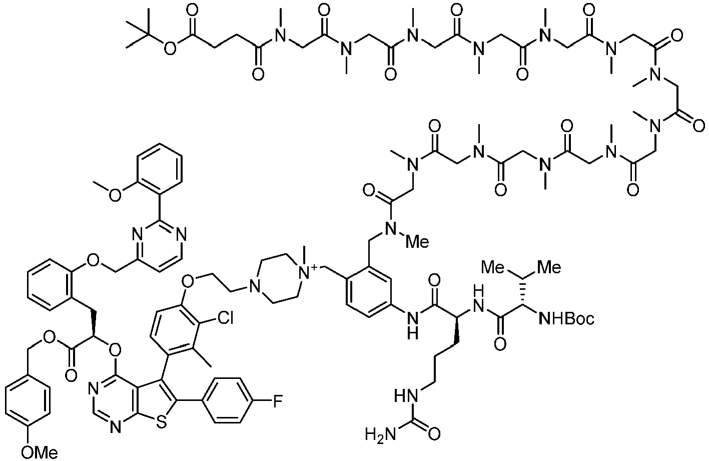

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (43.6 mg, 0.025 mmol, 1.0 equiv.), 3,6,9,12,15, 18,21,24,27,30,33,36,42,42-tetradecamethyl-4,7,10,13,16,19,22,25,28,31,34,37,40-tridecaoxo-41-oxa-3,6,9,12,15,18,21,24,27,30,33,36-dodecaazatritetracontanoic acid (25.9 mg, 0.025 mmol, 1.0 equiv.), and HATU (10.5 mg, 0.028 mmol, 1.1 equiv.) in DMF (0.25 mL) was added DIPEA (22 µL, 0.126 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11,14,17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 2508.1499 Rt=2.78 min (5 min acidic method).

### Synthesis of 1-(2-(41-carboxy-2,5,8,11,14,17,20,23,26,29,32,35,38-tridecamethyl-3,6,9,12,15,18,21,24,27,30,33,36,39-tridecaoxo-2,5,8,11,14,17,20,23,26,29,32,35,38-tridecaazahentetracontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L35-P1)

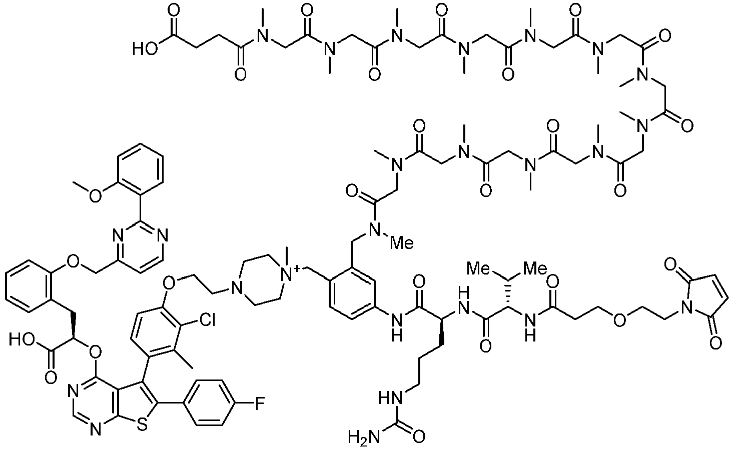

Following **GENERAL PROCEDURE 3** with 1-(4-((S)-2-((S)-2-((tert-butoxycarbonyl)amino)-3-methylbutanamido)-5-ureidopentanamido)-2-(2,5,8,11,14,17,20,23,26,29,32,35,38,44,44-pentadecamethyl-3,6,9,12,15,18,21,24,27,30,33,36,39,42-tetradecaoxo-43-oxa-2,5,8,11,14,17,20,23,26,29,32,35,38-tridecaazapentatetracontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (50.4 mg, 0.019 mmol, 1.0 equiv.), 1-(2-(41-carboxy-2,5,8,11,14,17,20,23,26,29,32,35,38-tridecamethyl-3,6,9,12,15,18,21,24,27,30,33,36,39-tridecaoxo-2,5,8,11,14,17,20,23,26,29,32,35,38-tridecaazahentetracontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 2427.0400, rt = 2.30 min. (5 min acidic method).

### Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

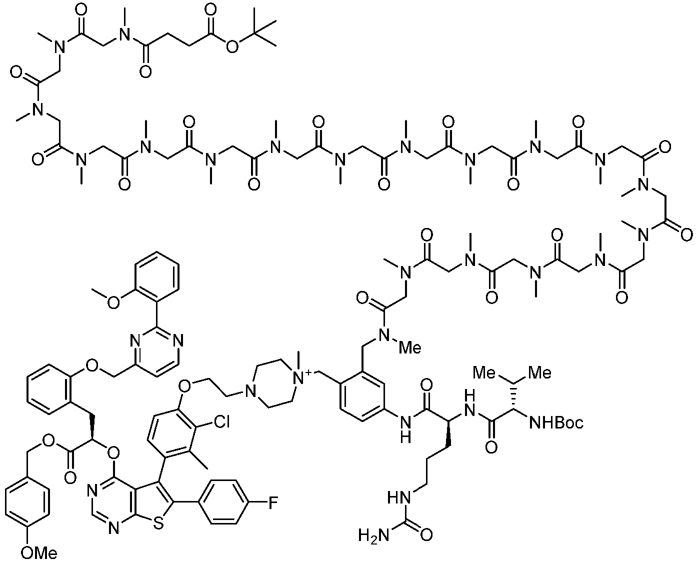

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (31.9 mg, 0.018 mmol, 1.0 equiv.), 3,6,9,12,15,18,21,24,27,30,33,36,39,42,45,48,51,54,60,60-icosamethyl-4,7,10,13,16,19,22,25,28,31,34,37,40,43,46,49,52,55,58-nonadecaoxo-59-oxa-3,6,9,12,15,18,21,24,27,30,33,36,39,42,45,48,51,54-octadecaazahenhexacontanoic acid (26.8 mg, 0.018 mmol, 1.0 equiv.), and HATU (7.7 mg, 0.020 mmol, 1.1 equiv.) in DMF (0.25 mL) was added DIPEA (16 µL, 0.092 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 2934.3601 Rt=2.73 min (5 min acidic method).

### Synthesis of 1-(2-(59-carboxy-2,5,8,11,14,17,20,23,26,29,32,35,38,41,44,47,50,53,56-nonadecamethyl-3,6,9,12,15,18,21,24,27,30,33,36,39,42,45,48,51,54,57-nonadecaoxo-2,5,8,11,14,17,20,23,26,29,32,35,38,41,44,47,50,53,56-nonadecaazanonapentacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L36-P1)

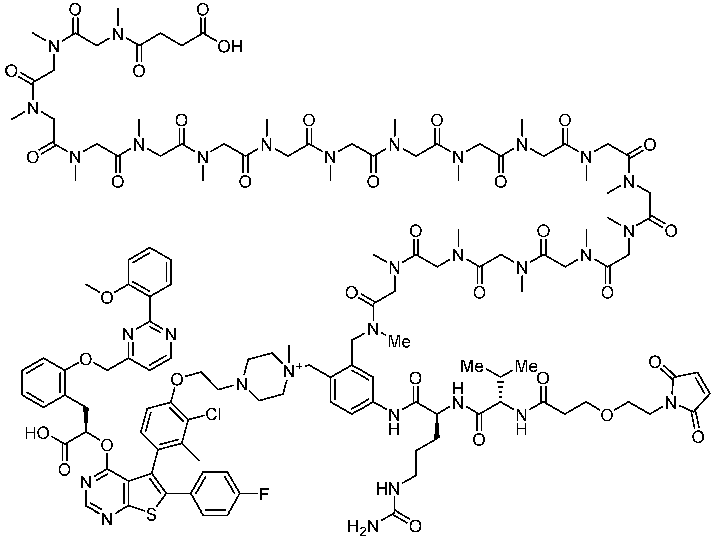

Following **GENERAL PROCEDURE 3** with 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,62,62-henicosamethyl-3,6,9,12,15,18,21,24,27,30,33,36,39,42,45,48,51,54,57,60-icosaoxo-61-oxa-2,5,8,11,14,17,20,23,26,29,32,35,38,41,44,47,50,53,56-nonadecaazatrihexacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (45.5 mg, 0.015 mmol, 1.0 equiv.), 1-(2-(59-carboxy-2,5,8,11,14,17,20,23,26,29,32,35,38,41,44,47,50,53,56-nonadecamethyl-3,6,9,12,15,18,21,24,27,30,33,36,39,42,45,48,51,54,57-nonadecaoxo-2,5,8,11,14,17,20,23,26,29,32,35,38,41,44,47,50,53,56-nonadecaazanonapentacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 2853.2766, rt = 2.20 min. (5 min acidic method).

### Synthesis of 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

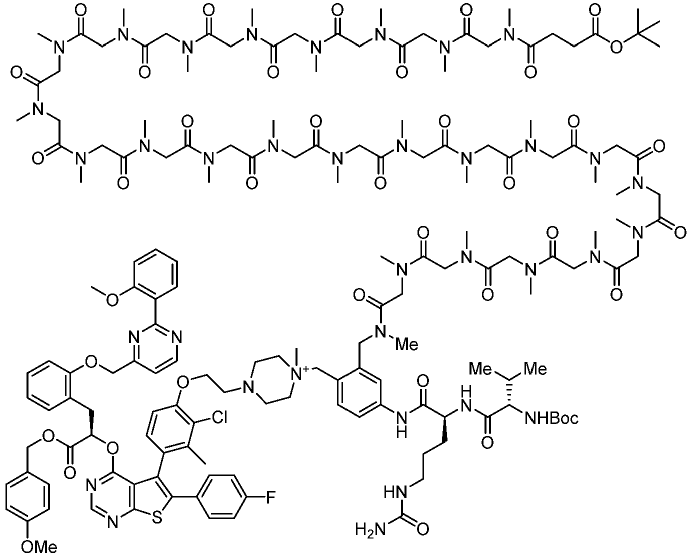

To a stirred solution of 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (27.8 mg, 0.016 mmol, 1.0 equiv.), 3,6,9,12,15,18,21,24,27,30,33,36,39,42,45,48,51,54,57,60,63,66,69,72,78,78-hexacosamethyl-4,7,10,13,16,19,22,25,28,31,34,37,40,43,46,49,52,55,58,61,64,67,70,73,76-pentacosaoxo-77-oxa-3,6,9,12,15,18,21,24,27,30,33,36,39,42,45,48,51,54,57,60,63,66,69,72-tetracosaazanonaheptacontanoic acid (30.2 mg, 0.016 mmol, 1.0 equiv.), and HATU (6.7 mg, 0.018 mmol, 1.1 equiv.) in DMF (0.25 mL) was added DIPEA (14 µL, 0.080 mmol, 5.0 equiv.). The resulting solution was stirred at ambient temperature for 1 hour. The reaction was diluted with 1 mL DMSO and purified by RP-HPLC ISCO gold chromatography (10-100% MeCN/H2O, 0.1% TFA modifier). Upon lyophilization 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS M+ 3360.5798 Rt=2.68 min (5 min acidic method).

### Synthesis of 1-(2-(77-carboxy-2,5,8,11,14,17,20,23,26,29,32,35,38,41,44,47,50,53,56,59,62,65,68,71,74-pentacosamethyl-3,6,9,12,15,18,21,24,27,30,33,36,39,42,45,48,51,54,57,60,63,66,69,72,75-pentacosaoxo-2,5,8,11,14,17,20,23,26,29,32,35,38,41,44,47,50,53,56,59,62,65,68,71,74-pentacosaazaheptaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L37-P1)

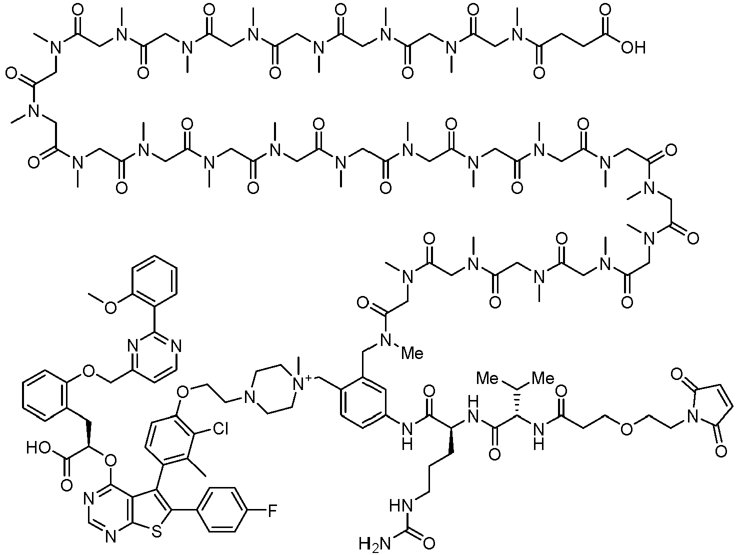

Following **GENERAL PROCEDURE 3** with 1-(4-((S)-2-((S)-2-((tert-butoxycarbonyl)amino)-3-methylbutanamido)-5-ureidopentanamido)-2-(2,5,8,11,14,17,20,23,26,29,32,35,38,41,44,47,50,53,56,59,62,65,68,71,74,80,80-heptacosamethyl-3,6,9,12,15,18,21,24,27,30,33,36,39,42,45,48,51,54,57,60,63,66,69,72,75,78-hexacosaoxo-79-oxa-2,5,8,11,14,17,20,23,26,29,32,35,38,41,44,47,50,53,56,59,62,65,68,71,74-pentacosaazahenoctacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (41.3 mg, 0.012 mmol, 1.0 equiv.), 1-(2-(77-carboxy-2,5,8,11,14,17,20,23,26,29,32,35,38,41,44,47,50,53,56,59,62,65,68,71,74-pentacosamethyl-3,6,9,12,15,18,21,24,27,30,33,36,39,42,45,48,51,54,57,60,63,66,69,72,75-pentacosaoxo-2,5,8,11,14,17,20,23,26,29,32,35,38,41,44,47,50,53,56,59,62,65,68,71,74-pentacosaazaheptaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+ 3279.4678, rt = 2.21 min. (5 min acidic method).

### Synthesis of Tert-butyl 1-((N-((2-azidoethoxy)carbonyl)sulfamoyl)amino)-3,6,9,12,15,18-hexaoxahenicosan-21-oate

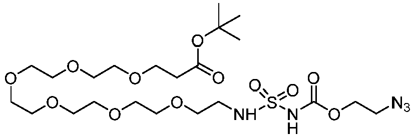

To 2-azidoethan-1-ol (105 mg, 1.21 mmol) in CH₂Cl₂ (15 ml) was added sulfurisocyanatidic chloride (0.105 ml, 1.21 mmol) at 0°C. The mixture was stirred at 0°C for 30 min. then TEA (0.336 ml, 2.41 mmol) and tert-butyl 1-amino-3,6,9,12,15,18-hexaoxahenicosan-21-oate (518 mg, 1.27 mmol) in CH₂Cl₂ (1 mL) were added. The mixture was stirred at 0°C for 1h and rt for 2 h, then was quenched with Satd NH4Cl, and 1 N HCl (2.4 mL). The aqueous was extracted with CH2Cl2 (5X). The organic layers were dried over anh, Na₂SO₄, filtered and concentrated via rotary evaporation to give a clear oil. Purification via flash chromatography (0-15% MeOH in CH₂Cl₂, ELSD detection) provided tert-butyl 1-((N-((2-azidoethoxy)carbonyl)sulfamoyl)amino)-3,6,9,12,15,18-hexaoxahenicosan-21-oate as a clear oil (474 mg, 0.788 mmol): LCMS: M+NH4+=619.5, Rt=0.94 min (acidic, 2 min). 1H NMR (400 MHz, DMSO-d6) δ 11.33 (s, 1H), 7.76 (s, 1H), 4.28 - 4.20 (m, 2H), 3.60 (td, J = 5.6, 5.0, 2.9 Hz, 4H), 3.55 - 3.45 (m, 22H), 3.16 - 3.04 (m, 2H), 2.46 - 2.38 (m, 2H), 1.41 (s, 9H).

### Synthesis of 1-((N-((2-azidoethoxy)carbonyl)sulfamoyl)amino)-3,6,9,12,15,18-hexaoxahenicosan-21-oic acid

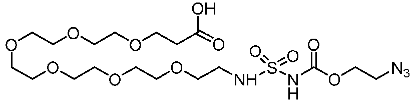

To tert-butyl 1-((N-((2-azidoethoxy)carbonyl)sulfamoyl)amino)-3,6,9,12,15,18-hexaoxahenicosan-21-oate (145 mg, 0.241 mmol) in CH₂Cl₂ (1 mL) at 0°C was added TFA (1 mL, 12.98 mmol). The mixture was stirred at rt for 1.45 h, then concentrated via rotary evaporation at 25°C water bath and dried in high vac for 30 min. The residue was azeotropically dried with anh toluene (3X 2 mL), and dried in vacuum overnight to provide 1-((N-((2-azidoethoxy)carbonyl)sulfamoyl)amino)-3,6,9,12,15,18-hexaoxahenicosan-21-oic acid as a clear oil (147 mg, 89% by weight based on theoretical yield): LCMS: M+=546.3, 0.65 min (acidic, 2 min, ELSD); 1H NMR (400 MHz, DMSO-d6) δ 11.30 (s, 1H), 7.77 - 7.71 (m, 1H), 4.25 - 4.19 (m, 2H), 3.63 - 3.55 (m, 4H), 3.54 - 3.45 (m, 22H), 3.07 (q, J = 6.0 Hz, 2H), 2.47 - 2.40 (m, 2H).

### Synthesis of 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate

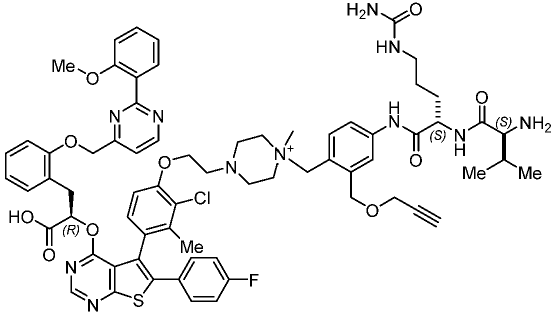

To 1-(4-((S)-2-((S)-2-((tert-butoxycarbonyl)amino)-3-methylbutanamido)-5-ureidopentanamido)-2-((prop-2-yn-1-yloxy)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium trifluoroacetate (321 mg, 0.210 mmol) at 0°C was added 25% TFA in CH₂Cl₂ (12.3 mL, 40.0 mmol). The reaction mixture was raised to Rt, stirred for 1h, then concentrated under high vacuum at RT water bath. The crude was diluted with DMSO (3 mL) and was purified by RP-HPLC ISCO gold chromatography (150 g, 10-80% MeCN/H2O, 0.1% TFA modifier). Upon lyophilization 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate was obtained as a white powder (224 mg, 0.158 mmol): HRMS: M+=1304.5100, Rt=2.15 min (5 min acidic)

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate

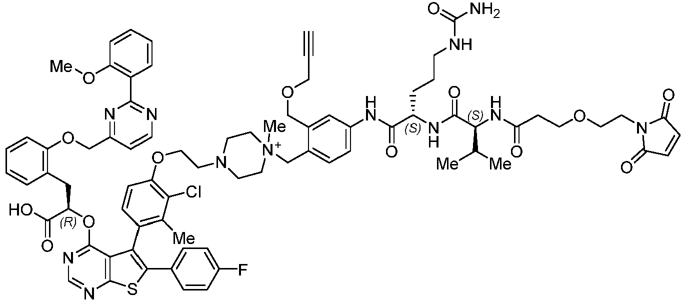

Following **GENERAL PROCEDURE 3** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate (164 mg, 0.115 mmol) and Mal-Peg1-NHS ester (72 mg, 0.23 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetatewas obtained as a white powder (170 mg, 0.105 mmol), HRMS: M+ =1499.5699, Rt=2.45 min (5 min acidic); 1H NMR (400 MHz, DMSO-d6) δ 10.16 (s, 1H), 8.81 (d, J = 5.1 Hz, 1H), 8.54 (s, 1H), 8.07 (d, J = 7.2 Hz, 1H), 7.75 (d, J = 8.5 Hz, 1H), 7.69 (d, J = 6.9 Hz, 2H), 7.56 (d, J = 5.1 Hz, 1H), 7.45 (dd, J = 7.5, 1.8 Hz, 1H), 7.42 - 7.29 (m, 3H), 7.24 (dd, J = 8.9, 5.4 Hz, 2H), 7.18 - 7.05 (m, 5H), 6.99 - 6.91 (m, 4H), 6.65 (t, J = 7.4 Hz, 1H), 6.15 (d, J = 7.0 Hz, 1H), 5.91 (s, 1H), 5.43 (dd, J = 9.8, 3.5 Hz, 1H), 5.23 - 5.12 (m, 2H), 4.54 (d, J = 11.2 Hz, 4H), 4.33 - 4.09 (m, 7H), 3.69 (s, 3H), 3.42 - 2.78 (m, 32H, overlapping with DMSO), 2.39 - 2.20 (m, 2H), 1.91 - 1.81 (m, 1H), 1.77 (s, 3H), 1.46 (dd, J = 93.8, 31.3 Hz, 3H), 0.76 (dd, J = 13.8, 6.7 Hz, 6H); 19F NMR (376 MHz, DMSO-d6): -112.18 ppm

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(20-carboxy-3,6,9,12,15,18-hexaoxaicosyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate (L59-P1)

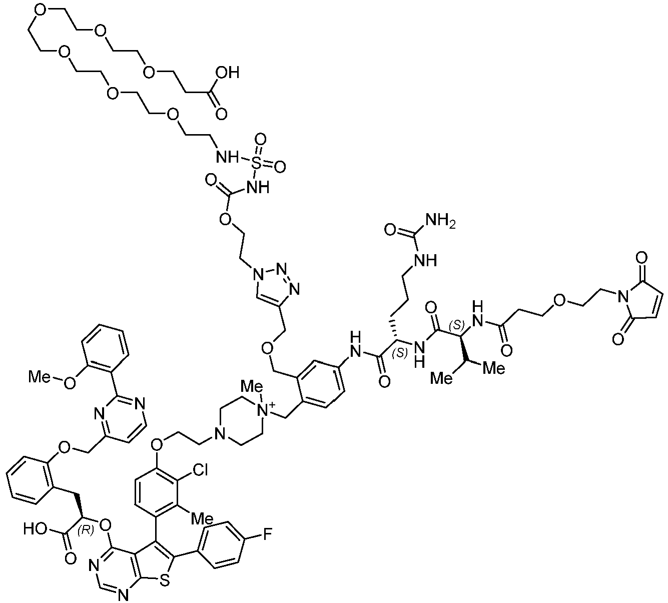

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (32 mg, 0.021 mmol) and 1-((N-((2-azidoethoxy)carbonyl)sulfamoyl)amino)-3,6,9,12,15,18-hexaoxahenicosan-21-oic acid (26.2 mg, 0.043 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(20-carboxy-3,6,9,12,15,18-hexaoxaicosyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate was obtained as a white powder: HRMS: M+ =2044.7700, Rt=2.36 min (5 min acidic).

### Synthesis of 3-(2-(2-(2-((N-((2-azidoethoxy)carbonyl)sulfamoyl)amino)ethoxy)ethoxy)ethoxy)propanoic acid

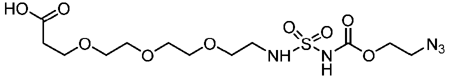

To 2-azidoethan-1-ol (105 mg, 1.21 mmol) in CH₂Cl₂ (15 ml) was added sulfurisocyanatidic chloride (0.105 ml, 1.21 mmol) at 0°C. The mixture was stirred at 0°C for 30 min. At 0°C TEA (0.336 ml, 2.41 mmol), and tert-butyl 3-(2-(2-(2-aminoethoxy)ethoxy)ethoxy) propanoate (401 mg, >80% technical purity, 1.447 mmol) in CH₂Cl₂ (1 mL) were added. After being stirred at 0°C for 1 h, then rt for 1 h, the mixture was quenched with satd. NH₄Cl, and 1 N HCl (2.4 mL). The aqueous was extracted with CH₂Cl₂ (5X). The organic layers were dried over anh, Na₂SO₄, filtered and concentrated via rotary evaporation to provide a clear oil. Following purification by flash chromatography (0-15% MeOH in CH₂Cl₂, ELSD detection) tert-butyl 3-(2-(2-(2-((N-((2-azidoethoxy)carbonyl)sulfamoyl)amino)ethoxy)ethoxy)ethoxy)propanoate was obtained as thick clear oil (356 mg, 0.910 mmol): LCMS: MS+NH4+=487.4, Rt=0.90 min (acidic, 2 min, ELSD); 1H NMR (400 MHz, DMSO-d6) δ 11.31 (s, 1H), 7.78 - 7.70 (m, 1H), 4.26 - 4.19 (m, 2H), 3.58 (td, J = 5.6, 5.0, 3.7 Hz, 4H), 3.53 - 3.39 (m, 10H), 3.10 - 3.03 (m, 2H), 2.44 - 2.39 (m, 2H), 1.40 (s, 9H).

To tert-butyl 3-(2-(2-(2-((N-((2-azidoethoxy)carbonyl)sulfamoyl)amino)ethoxy)ethoxy)ethoxy)propanoate (162 mg, 0.345 mmol) in CH₂Cl₂ (1 mL) at 0°C was added TFA (1 mL, 13.0 mmol). After being stirred at rt for 2 h, the mixture was concentrated via rotary evaporation with a water bath at 25 °C. The residue was dried in high vac for 30 min, by azeotropic distillation with anh. toluene (3X 2 mL), and in vacuo overnight to provide 3-(2-(2-(2-((N-((2-azidoethoxy)carbonyl)sulfamoyl)amino)ethoxy)ethoxy)ethoxy)propanoic acid as thick oil (139 mg, 0.335 mmol): LCMS: MS+NH4+=431.4, Rt=0.62 min (acidic, 2 min, ELSD): 1H NMR (400 MHz, DMSO-d6) δ 11.32 (d, J = 15.4 Hz, 1H), 7.78 - 7.69 (m, 1H), 4.27 - 4.15 (m, 2H), 3.67 - 3.54 (m, 8H), 3.49 - 3.42 (m, 4H), 3.07 (q, J = 6.0 Hz, 2H), 2.47 - 2.39 (m, 4H).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(2-(2-(2-(2-carboxyethoxy)ethoxy)ethoxy)ethyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate (L60-P1)

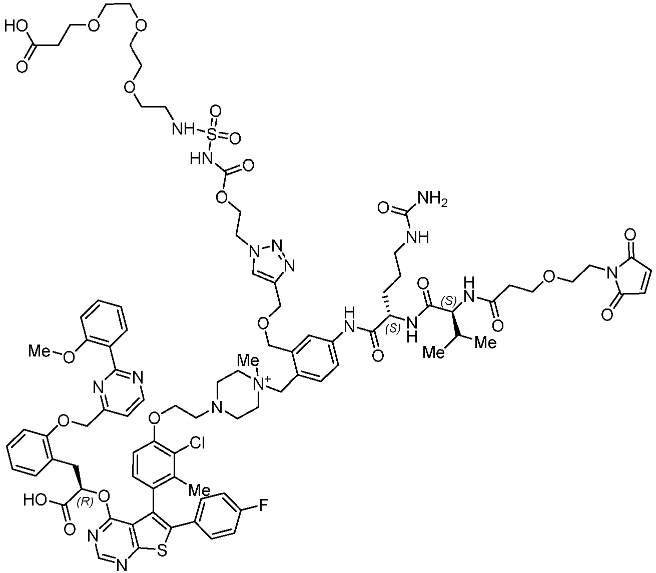

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (15 mg, 10 µmol) and 3-(2-(2-(2-((N-((2-azidoethoxy)carbonyl)sulfamoyl)amino)ethoxy)ethoxy)ethoxy)propanoic acid (12 mg, 0.029 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-(2-(((N-(2-(2-(2-(2-carboxyethoxy)ethoxy)ethoxy)ethyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium trifluoroacetate was obtained as a white powder: HRMS: MS+=1912.7000, Rt=2.38 min (5 min acidic).

### Synthesis of (2R,3R,4S,5R,6R)-2-(acetoxymethyl)-6-(2-(2-((tert-butoxycarbonyl)amino)ethoxy)ethoxy)tetrahydro-2H-pyran-3,4,5-triyl triacetate

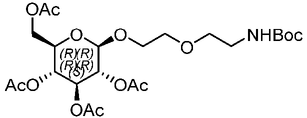

A mixture of tert-butyl (2-(2-hydroxyethoxy)ethyl)carbamate (1535 mg, 7.48 mmol), Ag2CO3 (8248 mg, 14.96 mmol) and a piece of crystalline iodine in CH₂Cl₂ (6 mL) was stirred with powdered 4A molecule sieve (1400 mg) for 15 min. To the mixture was added (*2R,3R,4S,5R,6R*)-2-(acetoxymethyl)-6-bromotetrahydro-2H-pyran-3,4,5-triyl triacetate (2050 mg, 4.99 mmol) in CH₂Cl₂ (6.00 ml), also stirred with powdered 4A molecule sieve (1400 mg) for 15 min prior to addition. The resulting mixture was covered with aluminum foil and stirred at rt for 60 h, and then filtered through celite with EtOAc washing. The filtrate was concentrated to give a clear oil that was purified by flash chromatography (0-10% MeOH in CH₂Cl₂, ELSD detection) to provide, after concentration of appropriate fractions, a thick oil (640 mg) as a 47/53% mixture of the desired (*2R,3R, 4S, 5R, 6R*)-2-(acetoxymethyl)-6-(2-(2-((tert-butoxycarbonyl)amino)ethoxy)ethoxy)tetrahydro-2H-pyran-3,4,5-triyl triacetate and (*2S,3aR,5R,6R,7S,7aR*)-5-(acetoxymethyl)-2-(2-(2-((tert-butoxycarbonyl)amino)ethoxy)ethoxy)-2-methyltetrahydro-5H-[1,3]dioxolo[4,5-b]pyran-6,7-diyl diacetate: LCMS: MS+=536.4, Rt=0.96 min (2 min, acid, ELSD); 1H NMR (400 MHz, DMSO-d6) δ 6.77 (s, 2H), 5.78 (s, 2H), 5.76 (s, 1H), 5.28 (t, J = 9.5 Hz, 1H), 5.04 (t, J = 3.1 Hz, 1H), 4.91 (t, J = 9.7 Hz, 1H), 4.87 - 4.74 (m, 3H), 4.38 (ddd, J = 5.2, 3.1, 0.9 Hz, 1H), 4.24 - 4.10 (m, 3H), 4.07 - 3.96 (m, 2H), 3.92 (dt, J = 8.8, 4.1 Hz, 1H), 3.80 (dt, J = 11.2, 4.4 Hz, 1H), 3.68 - 3.60 (m, 1H), 3.57 - 3.45 (m, 7H), 3.38 (td, J = 6.2, 1.5 Hz, 7H), 3.07 (dt, J = 6.1, 3.0 Hz, 4H), 2.08 (s, 3H), 2.06 (s, 3H), 2.04 (s, 6H), 2.01 (s, 3H), 2.00 (s, 3H), 1.95 (s, 3H), 1.65 (s, 3H), 1.39 (s, 19H).

### Synthesis of (2R,3R,4S,5R,6S)-2-(acetoxymethyl)-6-(2-(2-aminoethoxy)ethoxy)tetrahydro-2H-pyran-3,4,5-triyl triacetate

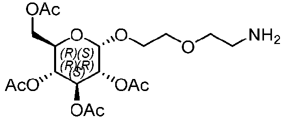

To a 47/53% mixture of (2R,3R,4S,5R,6R)-2-(acetoxymethyl)-6-(2-(2-((tert-butoxycarbonyl)amino)ethoxy)ethoxy)tetrahydro-2H-pyran-3,4,5-triyl triacetate and (2S,3aR,5R,6R,7S,7aR)-5-(acetoxymethyl)-2-(2-(2-((tert-butoxycarbonyl)amino)ethoxy)ethoxy)-2-methyltetrahydro-5H-[1,3]dioxolo[4,5-b]pyran-6,7-diyl diacetate (622 mg, 1.161 mmol) in CH₂Cl₂ (16 mL) at 0°C was added TFA (4.0 mL, 52 mmol). The reaction mixture was raised to Rt and stirred for 1 h. The mixture was concentrated and the residue was dried under vacuo for 60 min to give a light yellow oil. The crude product was diluted with DMSO (4 mL) and was purified by RP-HPLC ISCO gold chromatography (5-60% MeCN/H2O, 0.1% TFA modifier, ELSD detection). Upon lyophilization of appropriate fractions, (2R,3R,4S,5R,6S)-2-(acetoxymethyl)-6-(2-(2-aminoethoxy)ethoxy)tetrahydro-2H-pyran-3,4,5-triyl triacetate, as a TFA salt, was obtained as a white powder (195 mg, 0.355 mmol): LCMS: MS+=436.4, Rt=0.58 min (acidic, 2 min); 1H NMR (400 MHz, DMSO-d6) δ 7.72 (s, 3H), 5.27 (t, J = 9.4 Hz, 1H), 4.92 (t, J = 9.6 Hz, 1H), 4.86 - 4.75 (m, 2H), 4.22 - 4.15 (m, 1H), 4.07 - 3.94 (m, 3H), 3.89 - 3.53 (m, 14H, mixed with DMSO), 3.03 - 2.91 (m, 2H), 2.03 (s, 3H), 2.01 (s, 3H), 1.99 (s, 3H), 1.94 (s, 3H).

### Synthesis of 2-azidoethyl (N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamate

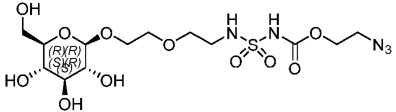

To 2-azidoethan-1-ol (16 mg, 0.18 mmol) in CH₂Cl₂ (2.5 ml) was added sulfurisocyanatidic chloride (0.016 ml, 0.184 mmol) at 0°C. The mixture was stirred at 0 C for 1 h, then TEA (0.128 ml, 0.919 mmol) and (*2R,3R,4S,5R, 6R*)-2-(acetoxymethyl)-6-(2-(2-aminoethoxy)ethoxy)tetrahydro-2H-pyran-3,4,5-triyl triacetate, as a TFA salt (116 mg, 0.211 mmol) in CH₂Cl₂ (1.5 mL) were added. After being stirred at 0°C for 1 h, then rt for 1 h, the mixture was quenched with satd NH₄Cl, and 1 N HCl (0.919 mL, 0.919 mmol). The aqueous was extracted with CH₂Cl₂ (5X). The organic layer was over anh, Na₂SO₄, filtered and concentrated via rotary evaporation to afford (*2R,3R,4S,5R, 6R*)-2-(acetoxymethyl)-6-(2-(2-((N-((2-azidoethoxy)carbonyl)sulfamoyl)amino)ethoxy)ethoxy)tetrahydro-2H-pyran-3,4,5-triyl triacetate as a clear oil (121 mg): LCMS: MS+=628, Rt=0.85 min (acidic, 2min, ELSD); 1H NMR (400 MHz, DMSO-d6) δ 11.32 (s, 1H), 7.79 - 7.71 (m, 1H), 5.26 (t, J = 9.5 Hz, 1H), 4.90 (t, J = 9.7 Hz, 1H), 4.85 - 4.73 (m, 2H), 4.25 - 4.14 (m, 3H), 4.06 - 3.94 (m, 2H), 3.79 (ddd, J = 11.3, 5.3, 3.8 Hz, 1H), 3.68 - 3.40 (m, 8H), 3.16 - 3.00 (m, 3H), 2.02 (s, 3H), 2.00 (s, 3H), 1.98 (s, 3H), 1.94 (s, 3H).

The product above was dissolved in dioxane (3 ml) and cooled at 0°C. LiOH.H₂O (0.5 M in water, 2.94 ml, 1.47 mmol) was added. The resulting clear solution was stirred at rt for 1 h and then quenched at 0°C with HCl (5N, 0.147 mL, 0.735 mmol). The mixture was concentrated via rotary evaporation at 20 °C water bath to remove most of dioxane. The residual solution (ca. 3 mL) was purified by prep HPLC (Sunfire 5µm 30x50 mm column, 2-12% of Acetonitrile with 0.1% FA in Water. Flow Rate: 75 mL/min., MS 459.3, 476.3 detection) to provide, after removal of solvent of appropriate fractions via lyophilization, 2-azidoethyl (N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamate as a semi-solid (64 mg, 0.14 mmol): LCMS: M+NH4+=477.3, MS-=458 (acidic, 2min, ELSD); 1H NMR (400 MHz, DMSO-d6) δ 11.32 (s, 1H), 7.71 (s, 1H), 4.96 (d, J = 4.9 Hz, 1H), 4.89 (dd, J = 13.7, 4.8 Hz, 2H), 4.47 (t, J = 5.9 Hz, 1H), 4.22 (t, J = 5.0 Hz, 2H), 4.15 (d, J = 7.8 Hz, 1H), 3.90 - 3.81 (m, 1H), 3.67 (ddd, J = 12.0, 5.7, 2.0 Hz, 1H), 3.62 - 3.39 (m, 8H), 3.19 - 2.90 (m, 6H).

### Syntheis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate (L63-P1)

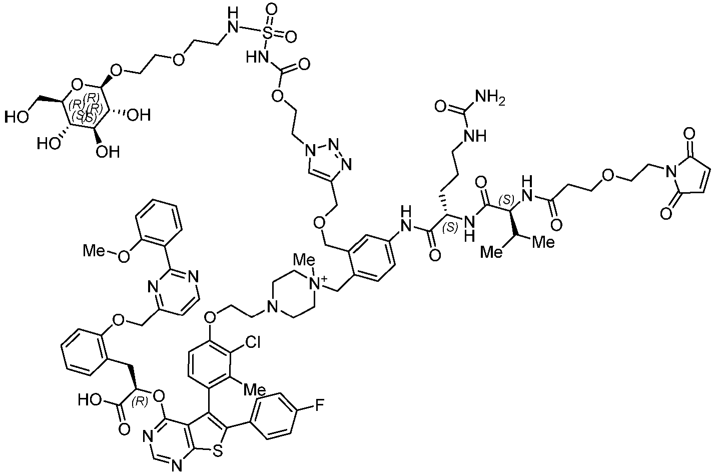

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (36 mg, 0.022 mmol) and 2-azidoethyl (N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamate (22 mg, 0.048 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(((2R,3R,4S,5S,6R)-3,4,5-trihydroxy-6-(hydroxymethyl)tetrahydro-2H-pyran-2-yl)oxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate was obtained as a white powder: HRMS: MS+=1958.6899, Rt=2.31 min (5 min acidic); 1H NMR (400 MHz, DMSO-d6) δ 11.17 (s, 1H), 10.16 (s, 1H), 8.81 (d, J = 5.2 Hz, 1H), 8.54 (s, 1H), 8.07 (d, J = 13.5 Hz, 2H), 7.74 (d, J = 8.6 Hz, 1H), 7.68 (q, J = 4.3, 2.7 Hz, 3H), 7.56 (d, J = 5.1 Hz, 1H), 7.45 (dd, J = 7.6, 1.8 Hz, 1H), 7.42 - 7.28 (m, 3H), 7.23 (ddd, J = 8.5, 5.4, 2.6 Hz, 2H), 7.17 - 7.04 (m, 5H), 6.99 - 6.91 (m, 4H), 6.65 (t, J = 7.4 Hz, 1H), 6.14 (dd, J = 7.6, 1.8 Hz, 1H), 5.93 (s, 1H), 5.43 (dd, J = 9.8, 3.6 Hz, 1H), 5.23 - 5.11 (m, 2H), 4.59 (dd, J = 11.3, 7.4 Hz, 8H), 4.42 - 4.06 (m, 12H), 3.53 - 3.17 (m, 27H, overlapping with DMSO), 3.09 - 2.80 (m, 17H, overlapping with DMSO), 2.39 - 2.20 (m, 3H), 1.86 (h, J = 6.9 Hz, 1H), 1.77 (s, 3H), 1.68 - 1.21 (m, 5H), 0.76 (dd, J = 13.8, 6.8 Hz, 6H); 19F NMR (376 MHz, DMSO-d6): -112.18 ppm.

### Synthesis of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate

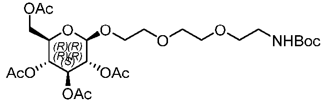

A mixture of tert-butyl (2-(2-(2-hydroxyethoxy)ethoxy)ethyl)carbamate (1046 mg, 4.20 mmol), Ag₂CO₃ (4627 mg, 8.39 mmol) and a piece of crystalline iodine in CH₂Cl₂ (3 mL) was stirred with powdered 4A molecule sieve (700 mg) for 15 min. To the mixture was added (2R,3R,4S,5R,6R)-2-(acetoxymethyl)-6-bromotetrahydro-2H-pyran-3,4,5-triyl triacetate (1150 mg, 2.80 mmol) in CH₂Cl₂ (3.00 mL) (also stirred with powdered 4A molecule sieve (700 mg) for 15 min). The resulting mixture was covered with aluminum foil and stirred at rt for 60 h, then filtered through celite with EtOAc washing. The filtrate was concentrated to give a clear oil. Purification via flash chromatography (20-100% EtOAc in heptane, ELSD detection) provided a 28/72% mixture of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate and (*2S,3aR,5R,6R*,*7S*,*7aR*)-5-(acetoxymethyl)-2-((2,2-dimethyl-4-oxo-3,8,11-trioxa-5-azatridecan-13-yl)oxy)-2-methyltetrahydro-5H-[1,3]dioxolo[4,5-b]pyran-6,7-diyl diacetate as a thick clear oil (307 mg, 0.529 mmol): LCMS: MS+=580.4, Rt=0.96 min (acid, 2 min, ELSD only); 1H NMR (400 MHz, DMSO-d6) δ 6.74 (s, 3H), 5.76 (s, 8H), 5.26 (t, J = 9.5 Hz, 1H), 5.02 (t, J = 3.1 Hz, 3H), 4.90 (t, J = 9.7 Hz, 1H), 4.84 - 4.72 (m, 5H), 4.37 (ddd, J = 5.2, 3.1, 0.9 Hz, 3H), 4.18 (dd, J = 12.2, 5.0 Hz, 1H), 4.11 (d, J = 4.5 Hz, 5H), 4.08 - 3.94 (m, 4H), 3.91 (dt, J = 8.6, 4.1 Hz, 3H), 3.85 - 3.75 (m, 1H), 3.66 - 3.58 (m, 1H), 3.58 - 3.51 (m, 7H), 3.42 - 3.31 (m, 14H), 3.06 (q, J = 5.9 Hz, 8H), 2.07 (s, 7H), 2.05 (s, 7H), 2.02 (s, 10H), 1.99 (d, J = 1.0 Hz, 6H), 1.99 (s, 3H), 1.94 (s, 3H), 1.63 (s, 7H), 1.38 (s, 36H), 1.18 (t, J = 7.1 Hz, 4H).

### Synthesis of (2R,3R,4S,5R,6R)-2-(acetoxymethyl)-6-(2-(2-(2-aminoethoxy)ethoxy)ethoxy)tetrahydro-2H-pyran-3,4,5-triyl triacetate

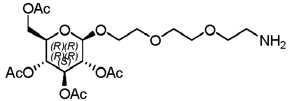

To a mixture of (2R,3R,4S,5R,6R)-2-(acetoxymethyl)-6-((2,2-dimethyl-4-oxo-3,8,11-trioxa-5-azatridecan-13-yl)oxy)tetrahydro-2H-pyran-3,4,5-triyl triacetate and (*2S,3aR,5R,6R,7S,7aR*)-5-(acetoxymethyl)-2-((2,2-dimethyl-4-oxo-3,8,11-trioxa-5-azatridecan-13-yl)oxy)-2-methyltetrahydro-5H-[1,3]dioxolo[4,5-b]pyran-6,7-diyl diacetate (323 mg, 0.557 mmol) in CH₂Cl₂ (8 mL) at 0°C was added TFA (1.9 mL, 25 mmol). After being stirred at rt for for 45 min, the mixture was concentrated and the residue was dried under vacuum for 60 min to give a light yellow oil. Purification via flash chromatography (0-20% MeOH in CH₂Cl₂, with 0.2% NH₄OH modifier in MeOH, ELSD detection) afforded (2R,3R,4S,5R,6R)-2-(acetoxymethyl)-6-(2-(2-(2-aminoethoxy)ethoxy)ethoxy)tetrahydro-2H-pyran-3,4,5-triyl triacetate as a clear oil (82 mg, 0.171 mmol). LCMS: MS+=480.4, Rt=0.61 min (acidic, 2 min, ELSD); 1H NMR (400 MHz, DMSO-d6) δ 7.76 (s, 2H), 5.28 (t, J = 9.4 Hz, 1H), 4.93 (t, J = 9.7 Hz, 1H), 4.86 - 4.75 (m, 2H), 4.20 (dd, J = 12.2, 5.0 Hz, 1H), 4.08 - 3.95 (m, 2H), 3.88 - 3.79 (m, 1H), 3.67 - 3.53 (m, 11H, overlapping with DMSO), 3.00 (q, J = 5.5 Hz, 2H), 2.04 (s, 3H), 2.02 (s, 3H), 2.00 (s, 3H), 1.96 (s, 3H).

### Synthesis of 2-azidoethyl (N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamate

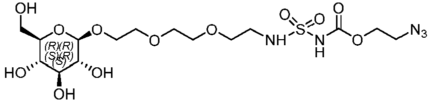

To 2-azidoethan-1-ol (12.5 mg, 0.144 mmol) in CH₂Cl₂ (2 mL) was added sulfurisocyanatidic chloride (0.012 ml, 0.14 mmol) at 0°C. The mixture was stirred at 0°C for 45 min, then TEA (0.040 ml, 0.29 mmol) and (2R,3R,4S,5R,6R)-2-(acetoxymethyl)-6-(2-(2-(2-aminoethoxy)ethoxy)ethoxy)tetrahydro-2H-pyran-3,4,5-triyl triacetate (77 mg, 0.16 mmol) in CH₂Cl₂ (1 mL) were added. After being stirred at 0°C for 1h, then at rt for 1 h, the mixture was quenched with satd. NH₄Cl, and 1 N HCl (0.29 mL). The aqueous was extracted with CH₂Cl₂ (5X). The organic layers were dried over anh. Na₂SO₄, filtered and concentrated via rotary evaporation to give (2R,3R,4S,5R,6R)-2-(acetoxymethyl)-6-(2-(2-(2-((N-((2-azidoethoxy)carbonyl)sulfamoyl)amino)ethoxy)ethoxy)ethoxy)tetrahydro-2H-pyran-3,4,5-triyl triacetate as a clear oil (75 mg): LCMS: 0.97 min, MS+=672.4, 96, Rt-0.87 min (acidic, 2min, ELSD).

To the product above in dioxane (4 mL) at 0°C was added LiOH.H2O (0.5 M in water, 3.45 ml, 1.72 mmol). The resulting clear solution was stirred at rt for 1 h and then was concentrated via rotary evaporation with 20°C water bath. The residue was purified by prep HPLC (Sunfire 5µm 30x50 mm column, 2-12% of Acetonitrile with 0.1% FA in Water. Flow Rate: 75 mL/min., MS 503.5, 520.3 detection) to provide, after lyophilization, 2-azidoethyl (N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamate as a clear thin film (22 mg, 0.044 mmol): LCMS: MS+=504.3, Rt=0.52 min (acidic, 2min, ELSD); 1H NMR (400 MHz, DMSO-d6) δ 11.31 (s, 1H), 7.74 (s, 1H), 4.96 (d, J = 4.9 Hz, 1H), 4.89 (dd, J = 12.5, 4.8 Hz, 2H), 4.47 (t, J = 5.9 Hz, 1H), 4.22 (t, J = 5.0 Hz, 2H), 4.15 (d, J = 7.8 Hz, 1H), 3.93 - 3.82 (m, 1H), 3.67 (dd, J = 11.2, 5.8 Hz, 1H), 3.62 - 3.40 (m, 12H), 3.17 - 2.90 (m, 6H).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate (L62-P1)

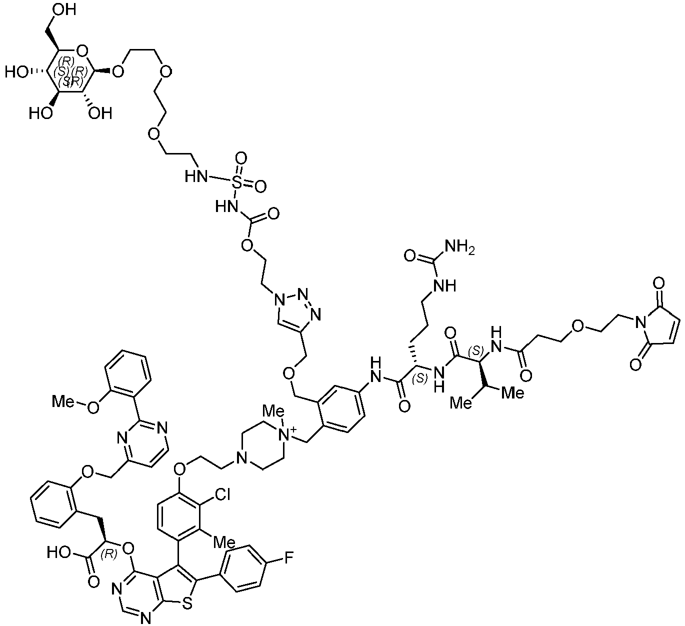

Following GENERAL PROCEDURE 2 with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (32 mg, 0.020 mmol) and 2-azidoethyl (N-(2-(2-(2-(((2S,3R,4S,5S,6R)-3,4,5-trihydroxy-6-(hydroxymethyl)tetrahydro-2H-pyran-2=yl)oxy)ethoxy)ethoxy)ethyl)sulfamoyl)carbamate (21 mg, 0.042 mmol), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-(((1-(2-(((N-(2-(2-(2-(((2R,3R,4S,5S,6R)-3,4,5-trihydroxy-6-(hydroxymethyl)tetrahydro-2H-pyran-2-yl)oxy)ethoxy)ethoxy)ethyl)sulfamoyl)carbamoyl)oxy)ethyl)-1H-1,2,3-triazol-4-yl)methoxy)methyl)benzyl)-1-methylpiperazin-1-ium trifluoroacetate was obtained as a white powder: HRMS: MS+=2002.7100, Rt=2.37 min (5 min acidic).

### Synthesis of Di-tert-butyl 3,3'-((3-((((9H-fluoren-9-yl)methoxy)carbonyl)amino)propanoyl)azanediyl)dipropionate

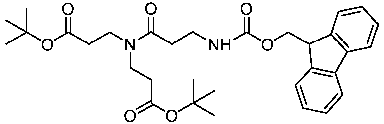

To a mixture of di-tert-butyl 3,3'-azanediyldipropionate (403 mg, 1.47 mmol), 3-((((9H-fluoren-9-yl)methoxy)carbonyl)amino)propanoic acid (505 mg, 1.62 mmol) and DIPEA (0.309 mL, 1.77 mmol) in CH₂Cl₂ (3 mL) was added *N*-(Dimethylaminopropyl)-*N'*-ethylcarbodiimide hydrochloride.HCl (367 mg, 1.92 mmol). After being stirred at rt for 2h, the mixture was quenched with satd. NH₄Cl, and extracted with CH₂Cl₂ (3X). The combined organic phase was washed with brine, dried over anh. Na₂SO₄, filtered and concentrated. The resulting crude product was purified by flash chromatography (0-50% EtOAc in heptane) to provide di-tert-butyl 3,3'-((3-((((9H-fluoren-9-yl)methoxy)carbonyl)amino)propanoyl)azanediyl)dipropionate as a white foam (290 mg, 0.511 mmol): LCMS: MS+=567.5, Rt=1.32 min (acid, 2 min); PMR: 1H NMR (400 MHz, DMSO-d6) δ 7.94 - 7.88 (m, 2H), 7.70 (d, J = 7.5 Hz, 2H), 7.47 - 7.40 (m, 2H), 7.40 - 7.31 (m, 2H), 7.22 (t, J = 5.7 Hz, 1H), 4.31 (d, J = 6.8 Hz, 2H), 4.26 - 4.18 (m, 1H), 3.56 - 3.03 (m, 8H), 2.50 - 2.38 (m, 4H), 1.41 (s, 9H), 1.40 (s, 9H).

### Synthesis of Di-tert-butyl 3,3'-((3-aminopropanoyl)azanediyl)dipropionate, 1-(9H-fluoren-9-yl)-N,N-dimethylmethanamine

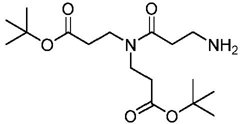

To di-tert-butyl 3,3'-((3-((((9H-fluoren-9-yl)methoxy)carbonyl)amino)propanoyl)azanediyl)dipropionate (288 mg, 0.508 mmol) in CH₂Cl₂ (3 mL) was added dimethylamine (2 N in THF, 1 mL, 2 mmol). The mixture was stirred at rt for 1 h. More dimethylamine (2 N in THF, 1 mL, 2 mmol) was added. After being stirred for additional 4 h, the mixture was concentrated and the residue was purified by flash chromatography (0-25% MeOH in CH₂Cl₂, 37 min, 0.2% NH₄OH modifier was in MeOH, ELSD detection) to provide di-tert-butyl 3,3'-((3-aminopropanoyl)azanediyl)dipropionate, 1-(9H-fluoren-9-yl)-N,N-dimethylmethanamine as a light brown oil (62 mg, 0.472 mmol): LCMS: MS+=345.4, Rt=0.76 min (acidc, 2 min, ELSD); 1H NMR (400 MHz, DMSO-d6) δ 4.05 (s, 2H), 3.51 (t, *J =* 7.2 Hz, 2H), 3.43 (t, *J =* 7.3 Hz, 2H), 3.31 (s, 2H), 2.79 (t, *J =* 6.4 Hz, 2H), 2.55 - 2.45 (m,17H, overlapping with DMSO), 2.41 (t, *J* = 7.3 Hz, 2H), 1.41 (s, 9H), 1.40 (s, 9H).

### Synthesis of 3,3'-((3-((N-((2-azidoethoxy)carbonyl)sulfamoyl)amino)propanoyl)azanediyl)dipropionic acid

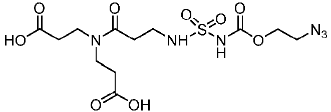

To 2-azidoethan-1-ol (16 mg, 0.18 mmol) in CH₂Cl₂ (2.5 ml) was added sulfurisocyanatidic chloride (0.016 ml, 0.18 mmol) at 0°C. The mixture was stirring at 0°C for 30 min, then TEA (0.051 ml, 0.37 mmol) and di-tert-butyl 3,3'-((3-aminopropanoyl)azanediyl)dipropionate (73 mg, 0.21 mmol) in CH₂Cl₂ (1 mL). After being stirred at 0°C for 1h and then rt for 1 h, the mixture was quenched with Satd NH₄Cl, and 1 N HCl (0.37 mL). The aqueous was extracted with CH₂Cl₂ (5X). The organic layers were dried over anh. Na₂SO₄, filtered and concentrated via rotary evaporation. The resulting residue was purified by flash chromatography (0-10% MeOH in CH₂Cl₂ , ELSD and UV214 detection) to provide di-tert-butyl 3,3'-((3-((N-((2-azidoethoxy)carbonyl)sulfamoyl)amino)propanoyl)azanediyl)dipropionate, as a thick clear oil (70 mg, 0.13 mmol): LCMS MS+= 537.4 , Rt=1.08 min (acidic, 2 min, ELSD); 1H NMR (400 MHz, DMSO-d6) δ 11.39 (s, 1H), 7.65 - 7.59 (m, 1H), 4.28 - 4.23 (m, 2H), 3.63 - 3.59 (m, 2H), 3.54 - 3.47 (m, 2H), 3.47 - 3.38 (m, 2H), 3.18 - 3.09 (m, 2H), 2.61 - 2.54 (m, 4H), 2.43 (dd, J = 8.5, 6.0 Hz, 2H), 1.43 (s, 9H), 1.42 (s, 9H).

To the product above in CH₂Cl₂ (2 ml) at 0°C was added TFA (2 ml). After being stirred at rt for 1.5 h, the mixture was concentrated via rotary evaporation at 25 °C water bath. The residue was dried in high vac for 30 min, then by azeotropic distillation with anh. toluene (3X 3 mL), and further dried in high vac overnight to provide 3,3'-((3-((N-((2-azidoethoxy)carbonyl)sulfamoyl)amino)propanoyl)azanediyl)dipropionic acid as a white solid (72 mg, 77% by weight based on theoretical yield. It was used directly in the next step): LCMS MS+=425.3 , Rt=0.52 min (acidic, 2 min, ELSD); 1H NMR (400 MHz, DMSO-d6) δ 12.28 (s, 1H), 11.35 (s, 1H), 7.58 (t, J = 5.8 Hz, 1H), 4.26 - 4.20 (m, 2H), 3.61 - 3.55 (m, 2H), 3.50 (t, J = 7.4 Hz, 2H), 3.42 (t, J = 7.4 Hz, 2H), 3.12 (q, J = 6.8 Hz, 2H), 2.56 (dd, J = 15.1, 7.4 Hz, 4H), 2.43 (t, J = 7.4 Hz, 2H), 2.08 (s, 1H).

### Synthesis of 1-(2-(((1-(2-(((N-(3-(bis(2-carboxyethyl)amino)-3-oxopropyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate (L61-P1)

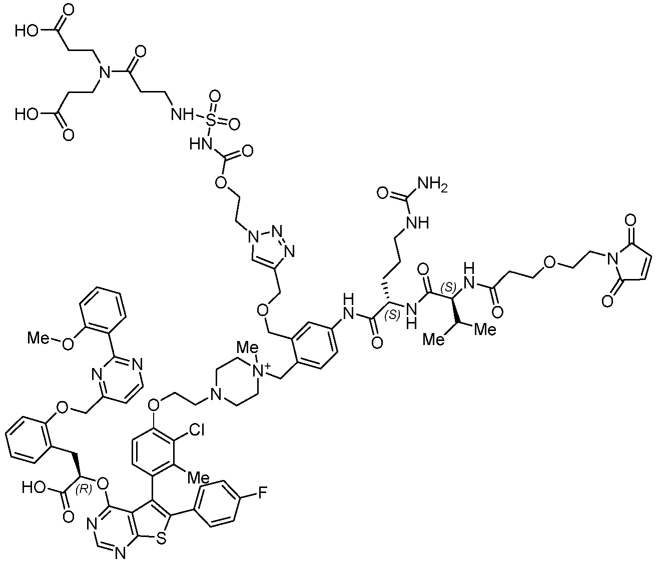

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium trifluoroacetate (20 mg, 0.012 mmol) and 3,3'-((3-((N-((2-azidoethoxy)carbonyl)sulfamoyl)amino)propanoyl)azanediyl)dipropionic acid (15 mg, 0.027 mmol), 1-(2-(((1-(2-(((N-(3-(bis(2-carboxyethyl)amino)-3-oxopropyl)sulfamoyl)carbamoyl)oxy)ethyl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium trifluoroacetate was obtained as a white powder: HRMS: MS+=1923.6500 Rt=2.34 min (5 min acidic); 1H NMR (400 MHz, DMSO-d6) δ 11.21 (s, 1H), 10.16 (s, 1H), 8.81 (d, J = 5.1 Hz, 1H), 8.54 (s, 1H), 8.08 (d, J = 13.7 Hz, 2H), 7.77 - 7.50 (m, 5H), 7.47 - 7.20 (m, 6H), 7.18 - 7.04 (m, 5H), 6.99 - 6.90 (m, 4H), 6.65 (t, J = 7.4 Hz, 1H), 6.14 (dd, J = 7.6, 1.7 Hz, 1H), 5.92 (s, 1H), 5.43 (dd, J = 9.8, 3.5 Hz, 1H), 5.24 - 5.11 (m, 3H), 4.65 - 4.51 (m, 9H), 4.45 - 3.81 (m, 52H, overlapping with DMSO), 3.68 (s, 3H), 3.54 - 2.77 (m, 31H), 2.39 - 2.20 (m, 5H), 1.86 (q, J = 6.8 Hz, 1H), 1.77 (s, 3H), 1.69 - 1.00 (m, 6H), 0.76 (dd, J = 13.9, 6.8 Hz, 6H); 19F NMR (376 MHz, DMSO-d6): -112.16 ppm.

### Synthesis of 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

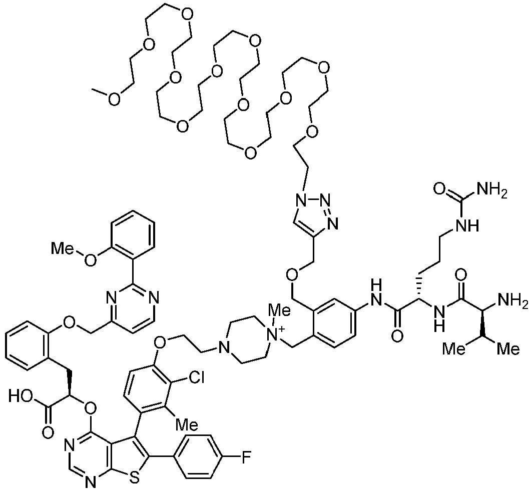

Following **GENERAL PROCEDURE 2** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (87.5 mg, 0.063 mmoles, 1.0 equiv) and mPEG12-Azide (73.3 mg, 0.125 mmol, 2 equiv ), 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1889.8544, Rt=2.19 min (5 min acidic method).

### Synthesis of 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L104-P1)

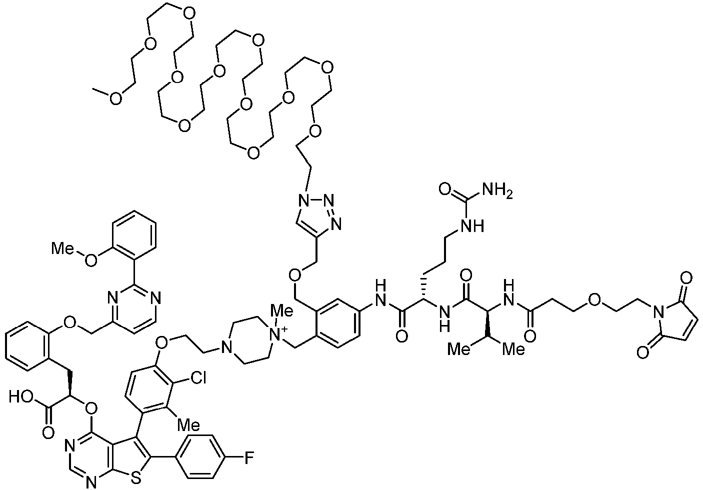

Following GENERAL PROCEDURE 3 with 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-amino-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (22 mg, 0.011 mmol, 1.0 equiv.), 1-(2-(((1-(2,5,8,11,14,17,20,23,26,29,32,35-dodecaoxaheptatriacontan-37-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 2084.9099, Rt=2.45 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium

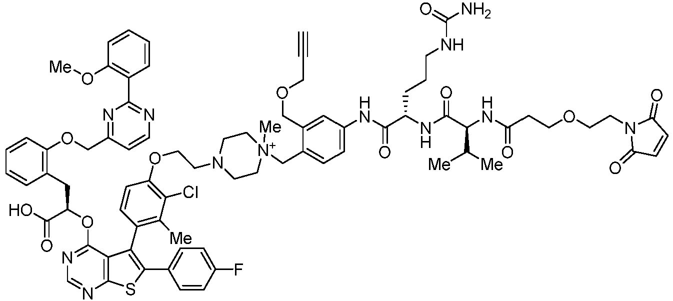

Following **GENERAL PROCEDURE 3** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (155 mg, 0.119 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium was obtained. HRMS: M+= 1499.5699, Rt=2.39 min (5 min acidic method).

### Synthesis of 1-(2-(((1-(2,5,8,11,14,17,20,23,26,29,32,35,38,41,44,47-hexadecaoxanonatetracontan-49-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (L34-P1)

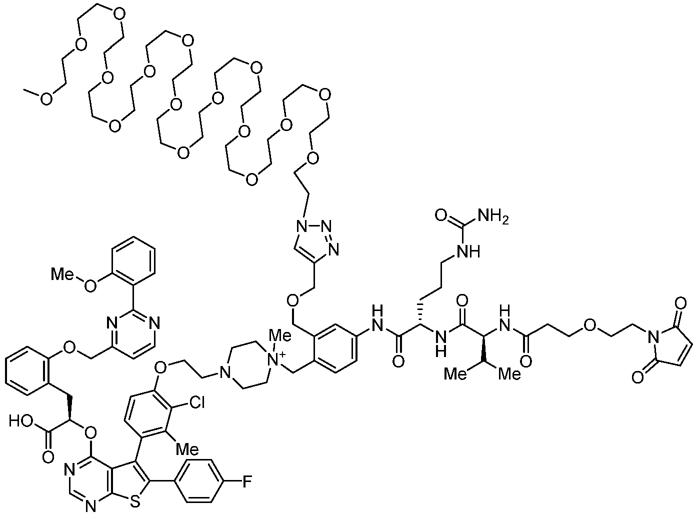

Following **GENERAL PROCEDURE 2** with 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((S)-2-((S)-2-(3-(2-(2,5-dioxo-2,5-dihydro-1H-pyrrol-1-yl)ethoxy)propanamido)-3-methylbutanamido)-5-ureidopentanamido)-2-((prop-2-yn-1-yloxy)methyl)benzyl)-1-methylpiperazin-1-ium (50 mg, 0.033 mmoles, 1.0 equiv) and m-PEG16-azide (from Broadpharm BP-23558) (50.8 mg, 0.067 mmol, 2 equiv), 1-(2-(((1-(2,5,8,11,14,17,20,23,26,29,32,35,38,41,44,47-hexadecaoxanonatetracontan-49-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+=2261.0196, Rt=2.28 min (5 min acidic method).

### Synthesis of 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

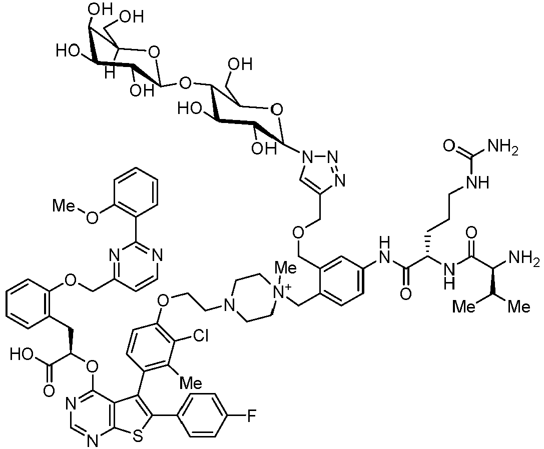

Following **GENERAL PROCEDURE 2** with 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (87.5 mg, 0.063 mmoles, 1.0 equiv) and 1-azido-1-deoxy-beta-D-lactopyranoside (22.99 mg, 0.063 mmol, 1 equiv), 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 1671.6400, Rt=1.95 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L46-P1)

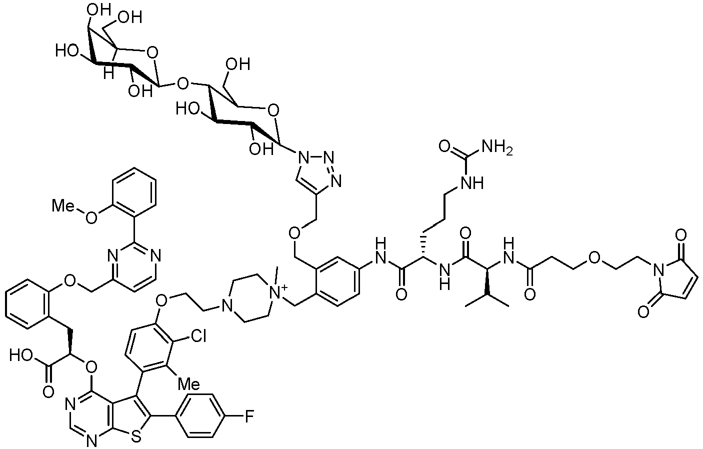

Following **GENERAL PROCEDURE** 3 with 1-(4-((S)-2-((S)-2-amino-3-methylbutanamido)-5-ureidopentanamido)-2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (35 mg, 0.020 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium was obtained. HRMS: M+= 1866.6899, Rt=2.28 min (5 min acidic method).

### Synthesis 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium

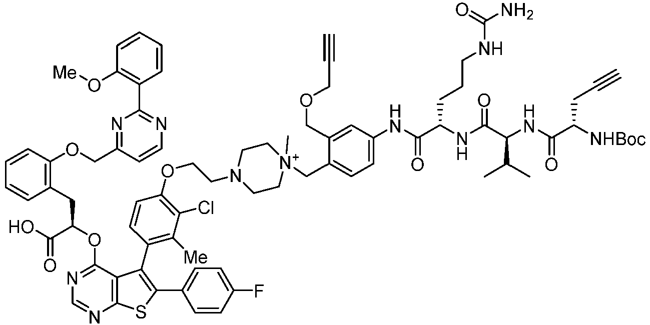

To the mixture of Boc-Propargyl-Gly-OH (40 mg, 0.188 mmol, 1 equiv ) and HATU (71.3 mg, 0.188 mmol, 1 equiv) in DMF (0.5 ml) was added DIPEA (65.5 µl, 0.375 mmol, 2 equiv). The mixture was stirred at RT for 30 min. Then a solution of 1-(4-((S)-2-((S)-2-amino-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (245 mg, 0.188 mmol, 1 equiv) in DMF (1 ml) was added into the reaction mixture. The reaction mixture was stirred at RT for 30min. The crude mixture was separated with C18 column ( 100 cartridge, MeCN/Water with 0.1% Formic Acid, 0-100% over 15CV) to obtain 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium. HRMS: M+= 1499.6000 , Rt= 2.91 min (5 min acidic method).

### Synthesis 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

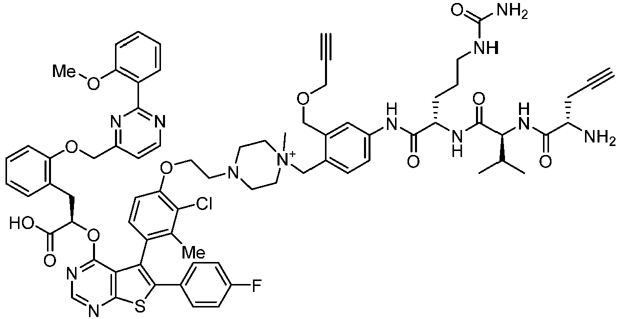

At 0°C ice-water bath, to 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(4-((6S,9S,12S)-9-isopropyl-2,2-dimethyl-4,7,10-trioxo-6-(prop-2-yn-1-yl)-12-(3-ureidopropyl)-3-oxa-5,8,11-triazatridecan-13-amido)-2-((prop-2-yn-1-yloxy)methyl)benzyl)-1-methylpiperazin-1-ium (56 mg, 0.037 mmol) was added TFA (25% in DCM) 2 mL, Then the reaction mixture was raised to RT and stirred for 1h. The crude mixture was concentrated under high vacuum . Then the mixture was disolved in MeOH, and was purified by C-18 column(50 g cartridge, MeCN/WAter with 0.1% Formic Acid 0-100% over 16 CV) to obtain 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium. HRMS: M+= 1399.5400 , Rt= 2.17 min (5 min acidic method).

### Synthesis of 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium

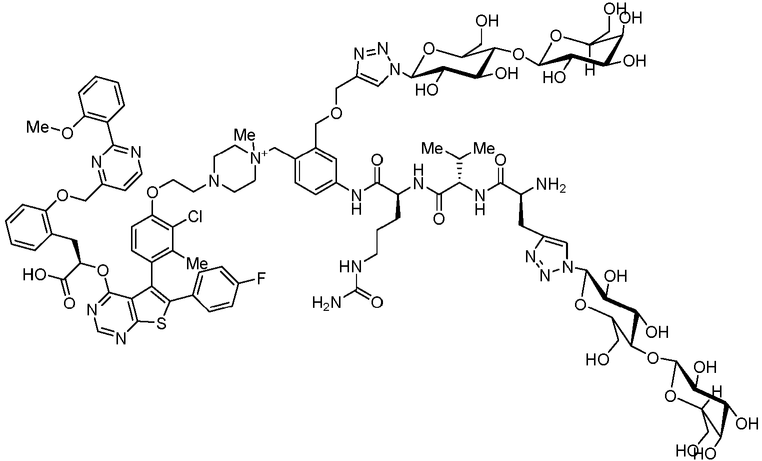

Following **GENERAL PROCEDURE** 2 with 1-(4-((S)-2-((S)-2-((S)-2-aminopent-4-ynamido)-3-methylbutanamido)-5-ureidopentanamido)-2-((prop-2-yn-1-yloxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (44 mg, 0.031 mmol, 1.0 equiv) and 1-azido-1-deoxy-beta-D-lactopyranoside (69.2 mg, 0.188 mmol, 6 eq) , 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium was obtained. HRMS: M+= 2133.7800 , Rt= 1.95 min (5 min acidic method).

### Synthesis of 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((2S,5S,8S)-8-((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methyl)-15-(2,5-dioxo-2,5-dihydro-1H-pyrrol-1-yl)-5-isopropyl-4,7,10-trioxo-2-(3-ureidopropyl)-13-oxa-3,6,9-triazapentadecanamido)benzyl)-1-methylpiperazin-1-ium (L47-P1)

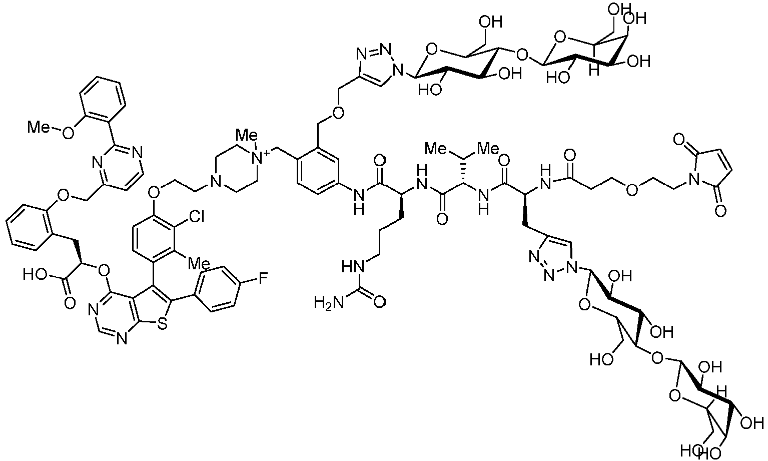

Following **GENERAL PROCEDURE 3** with 1-(4-((S)-2-((S)-2-((S)-2-amino-3-(1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)propanamido)-3-methylbutanamido)-5-ureidopentanamido)-2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)benzyl)-4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-methylpiperazin-1-ium (19 mg, 0.009 mmol, 1.0 equiv.), 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(((1-((2S,3S,4S,5R,6S)-3,4-dihydroxy-6-(hydroxymethyl)-5-(((2R,3S,4R,5S,6S)-3,4,5-trihydroxy-6-(hydroxymethyl)tetrahydro-2H-pyran-2-yl)oxy)tetrahydro-2H-pyran-2-yl)-1H-1,2,3-triazol-4-yl)methoxy)methyl)-4-((2S,5S,8S)-8-((1-((2R,3R,4R,5S,6R)-3,4-dihydroxy-6-(hydroxymethyl)-5-(((2S,3R,4S,5R,6R)-3,4,5-trihydroxy-6-(hydroxymethyl)tetrahydro-2H-pyran-2-yl)oxy)tetrahydro-2H-pyran-2-yl)-1H-1,2,3-triazol-4-yl)methyl)-15-(2,5-dioxo-2,5-dihydro-1H-pyrrol-1-yl)-5-isopropyl-4,7,10-trioxo-2-(3-ureidopropyl)-13-oxa-3,6,9-triazapentadecanamido)benzyl)-1-methylpiperazin-1-ium was obtained. HRMS: M+= 2328.8301, Rt=2.15 min (5 min acidic method).

### 1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium

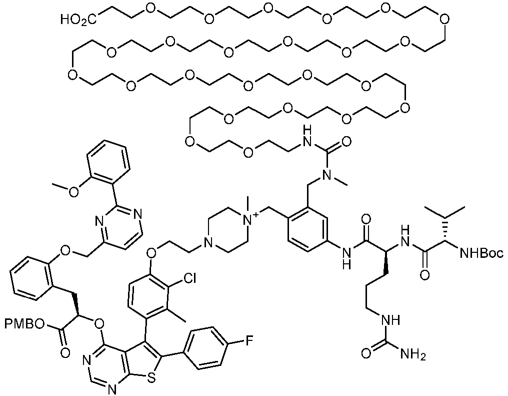

A mixture of 1-amino-3,6,9,12,15,18,21,24,27,30,33,36,39,42,45,48,51,54,57,60,63,66,69,72-tetracosaoxapentaheptacontan-75-oic acid (50 mg, 0.044 mmol), bis(4-nitrophenyl) carbonate (13 mg, 0.043 mmol), and DIPEA (20 µL, 0.12 mmol) in THF (2 mL) was stirred at RT for 2 h. The mixture was concentrated by blowing nitrogen gas to it. The resulting solid residue was taken up in DMF (1 mL). 1-(4-((S)-2-((S)-2-((tert-butoxycarbonyl)amino)-3-methylbutanamido)-5-ureidopentanamido)-2-((methylamino)methyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium (50 mg, 0.029 mmol) and DIPEA (100 µL, 0.573 mmol) were added. The mixture was stirred at RT for 5 min. The mixture was diluted with DMSO (2 mL), and the solution was purified by RP-HPLC ISCO gold chromatography (MeCN/H₂O, 0.1% TFA modifier). Upon lyophilization, 1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium was obtained. HRMS: M+= 2671.2700, Rt=2.88 min (5 min acidic method).

### 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L42-P1)

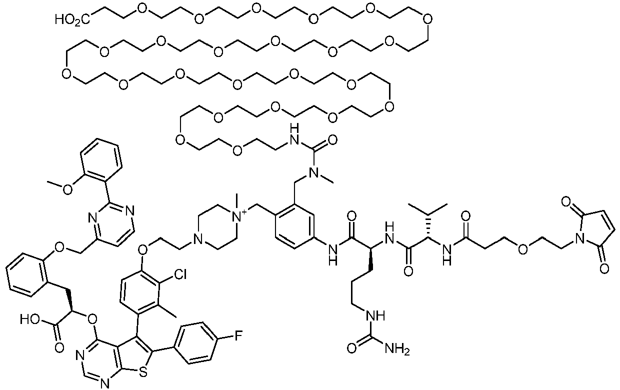

Following **GENERAL PROCEDURE 3** with1-(4-((S)-2-((S)-2-((tert-butoxycarbonyl)amino)-3-methylbutanamido)-5-ureidopentanamido)-2-(78-carboxy-2-methyl-3-oxo-7,10,13,16,19,22,25,28,31,34,37,40,43,46,49,52,55,58,61,64,67,70,73,76-tetracosaoxa-2,4-diazaoctaheptacontyl)benzyl)-4-(2-(2-chloro-4-(6-(4-fluorophenyl)-4-(((R)-1-((4-methoxybenzyl)oxy)-3-(2-((2-(2-methoxyphenyl)pyrimidin-4-yl)methoxy)phenyl)-1-oxopropan-2-yl)oxy)thieno[2,3-d]pyrimidin-5-yl)-3-methylphenoxy)ethyl)-1-methylpiperazin-1-ium, 4-(2-(4-(4-((R)-1-carboxy-2-(2-((2-(2-methoxyphenyl)pyrimidin-4-yl)methoxy)phenyl)ethoxy)-6-(4-fluorophenyl)thieno[2,3-d]pyrimidin-5-yl)-2-chloro-3-methylphenoxy)ethyl)-1-(2-(78-carboxy-2- methyl-3-oxo-7,10,13,16,19,22,25,28,31,34,37,40,43,46,49,52,55,58,61,64,67,70,73,76-tetracosaoxa-2,4-diazaoctaheptacontyl)-4-((S)-2-((S)-2-(3-(2-(2,5-dioxo-2,5-dihydro-1H-pyrrol-1-yl)ethoxy)propanamido)-3-methylbutanamido)-5-ureidopentanamido)benzyl)-1-methylpiperazin-1-ium (L42-P1) was obtained. HRMS: M+= 2646.7700, Rt=2.38 min (5 min acidic method).

The following compounds were prepared using procedures similar to those described above.

| **Code** | **Linker Payload Structure** | **Synthetic Methods** | **Characterizations** |
|---|---|---|---|
| L33-P1 | 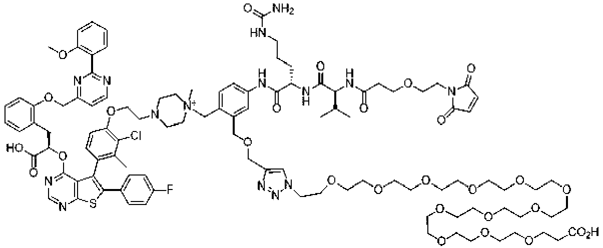 | General Procedure 2 | HRMS: M+ 2142.9099, rt = 2.39 min. (5 min acidic method). |
| L38-P1 | 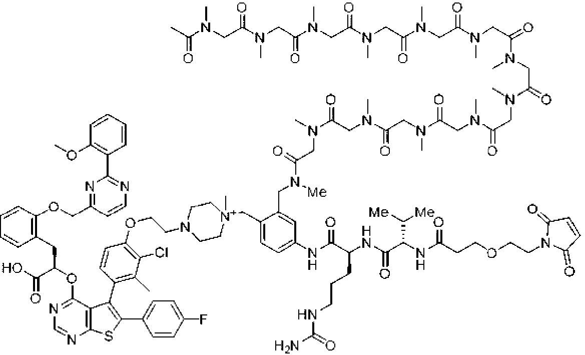 | Similar to synthesis of L35-P1 and using General Procedure 3 | HRMS: M+ 2369.0400, rt = 2.11 min. (5 min acidic method). |
| L39-P1 | 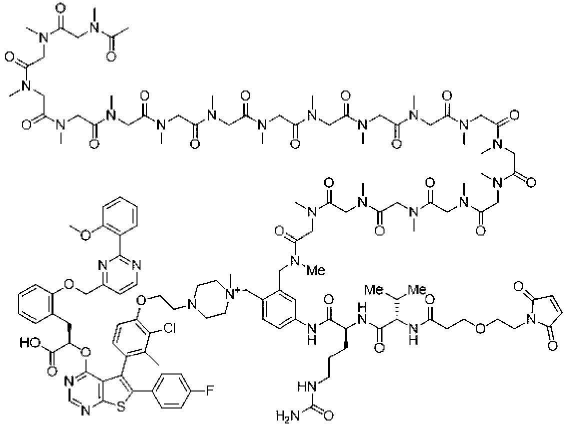 | Similar to synthesis of L35-P1 and using General Procedure 3 | HRMS: M+ 2795.2537, rt = 2.07 min. (5 min acidic method). |
| L40-P1 | 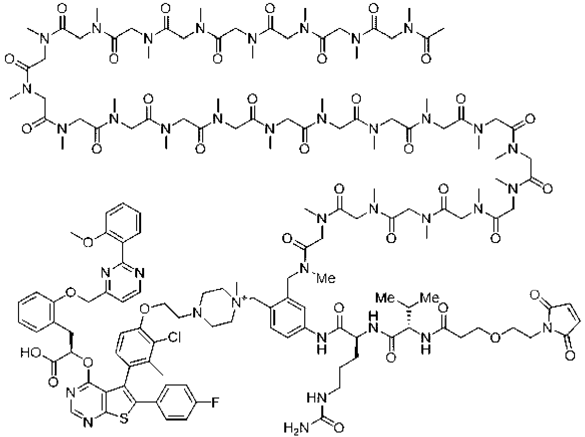 | Similar to synthesis of L35-P1 and using General Procedure 3 | HRMS: M+ 3221.4653, rt = 2.05 min. (5 min acidic method). |
| L43-P1 | 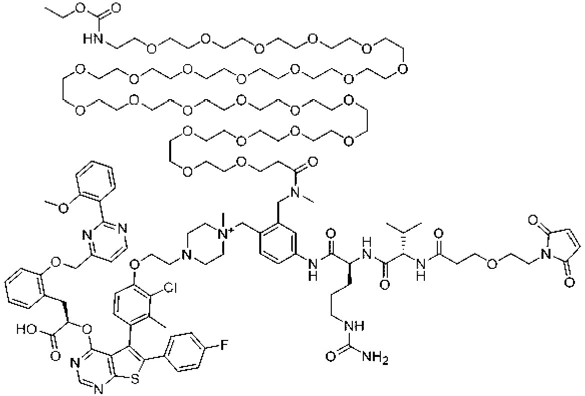 | Similar to synthesis of L35-P1 and using General Procedure 3 | HRMS: M+ 2673.7827, rt = 2.46 min. (5 min acidic method). |
| L44-P1 | 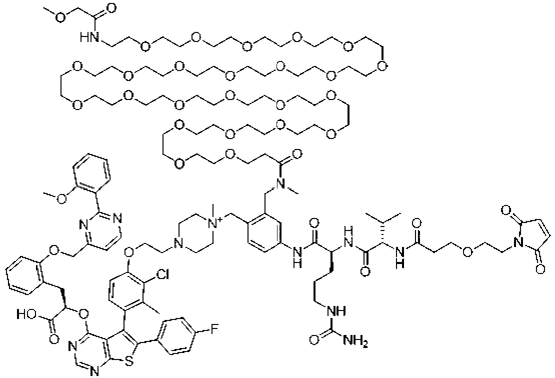 | Similar to synthesis of L35-P1 and using General Procedure 3 | HRMS: M+ 2673.7832, rt = 2.39 min. (5 min acidic method). |
| L45-P1 | 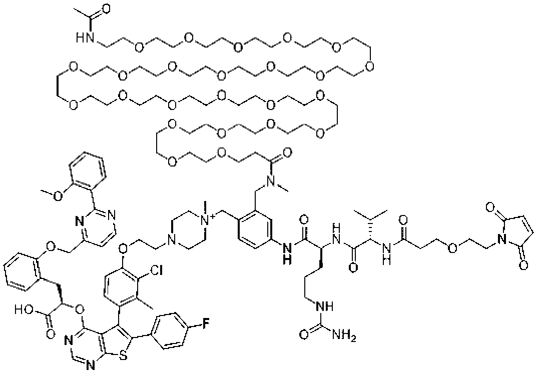 | Similar to synthesis of L35-P1 and using General Procedure 3 | HRMS: M+ 2644.2253, rt = 2.50 min. (5 min acidic method). |
| L95-P1 | 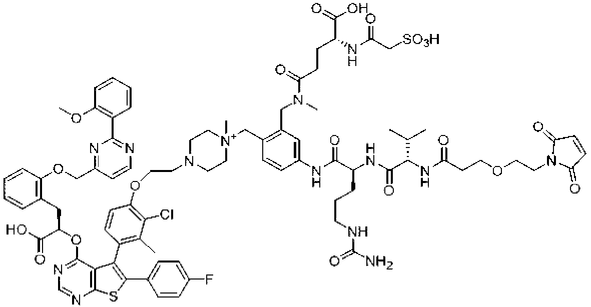 | Similar to synthesis of L71-P1 | HRMS: M+ 1725.6000 , rt = 2.32 min. (5 min acidic method) |
| L101-P1 | 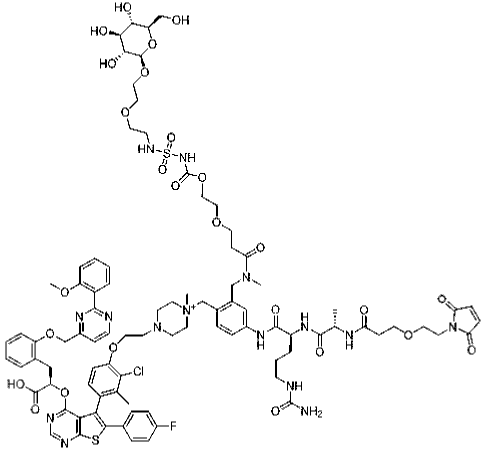 | Similar to synthesis of L62-P1 | HRMS: M+ 1962.7100, rt = 2.50 min. (5 min acidic method). |
| L105-P1 | 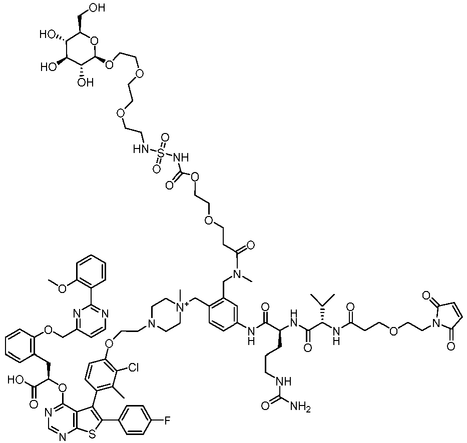 | Similar to synthesis of L62-P1 | HRMS: M+ 1962.7100, rt = 2.50 min. (5 min acidic method). |

The synthetic methods for preparing the polyethylene glycols in L43-P1, L44-P1 and L45-P1 are described below.

### Synthesis of 2-oxo-6,9,12,15,18,21,24,27,30,33,36,39,42,45,48,51,54,57,60,63,66,69,72,75-tetracosaoxa-3-azaoctaheptacontan-78-oic acid

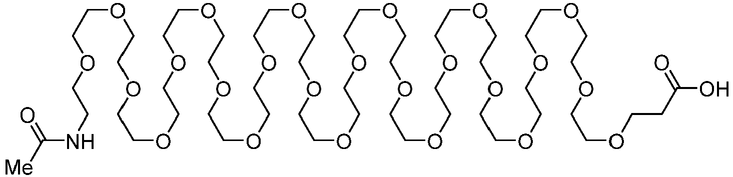

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 µL, 0.192 mmol, 2.2 equiv.) in dichloromethane (0.5 mL) was added acetic anhydride (8.9 mg, 8.25 µL, 1.0 equiv.). The resulting mixture was stirred at ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 2-oxo-6,9,12,15,18,21,24,27,30,33,36,39,42,45,48,51,54,57,60,63,66,69,72,75-tetracosaoxa-3-azaoctaheptacontan-78-oic acid (62.3 mg, 0.052 mmol, 60% yield) was obtained. LC/MS [M-H]- 1187.3 Rt=0.75 min. (2 min acidic method). 1H NMR (400 MHz, DMSO-d6) δ 7.86 (s, 1H), 3.60 (t, J = 6.4 Hz, 3H), 3.50 (d, J = 4.9 Hz, 91H), 3.40 (t, J = 5.9 Hz, 2H), 3.18 (q, J = 5.8 Hz, 2H), 2.44 (t, J = 6.4 Hz, 2H), 1.80 (s, 3H).

### Synthesis of 4-oxo-3,8,11,14,17,20,23,26,29,32,35,38,41,44,47,50,53,56,59,62,65,68,71,74,77-pentacosaoxa-5-azaoctacontan-80-oic acid

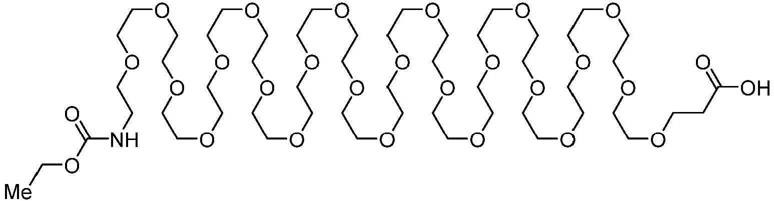

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 µL, 0.192 mmol, 2.2 equiv.) in dichloromethane (0.5 mL) was added ethyl chloroformate (9.5 mg, 8.34 µL, 1.0 equiv.). The resulting mixture was stirred at ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 4-oxo-3,8,11,14,17,20,23,26,29,32,35,38,41,44,47,50,53,56,59,62,65,68,71,74,77-pentacosaoxa-5-azaoctacontan-80-oic acid (75 mg, 0.062 mmol, 71% yield) was obtained. LC/MS [M-H]-1217.3 Rt=0.81 min. (2 min acidic method). 1H NMR (400 MHz, DMSO-d6) δ 7.03 (s, 1H), 3.97 (q, J = 7.1 Hz, 2H), 3.60 (t, J = 6.4 Hz, 2H), 3.50 (d, J = 5.0 Hz, 92H), 3.40 (t, J = 6.1 Hz, 2H), 3.11 (q, J = 5.9 Hz, 2H), 2.45 (q, J = 6.5 Hz, 2H), 1.15 (t, J = 7.1 Hz, 3H,).

### Synthesis of 4-oxo-2,8,11,14,17,20,23,26,29,32,35,38,41,44,47,50,53,56,59,62,65,68,71,74,77-pentacosaoxa-5-azaoctacontan-80-oic acid

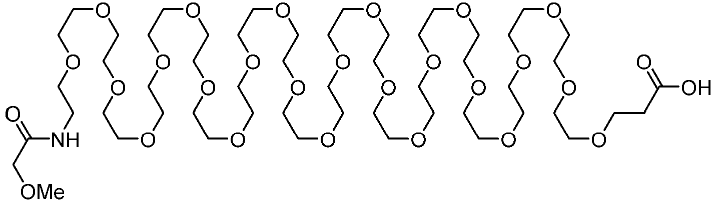

To a stirred solution of 1-amino-3,6,9,12,15,18,21,24,27,30,33,36,39,42,45,48,51,54,57,60,63,66,69,72-tetracosaoxapentaheptacontan-75-oic acid (100 mg, 0.087 mmol, 1.0 equiv.) and DIPEA (24.8 mg, 34 µL, 0.192 mmol, 2.2 equiv.) in dichloromethane (0.5 mL) was added methoxyacetyl chloride (11.36 mg, 9.57 µL, 1.2 equiv.). The resulting mixture was stirred at ambient temperature for 1.5 hours. The solvent was removed under reduced pressure. The resulting residue was taken up in DMSO (1 mL) and purified by RP-HPLC ISCO gold chromatography (10-100% MeCN/H2O, 0.1% TFA modifier). Upon lyophilization 4-oxo-2,8,11,14,17,20,23,26,29,32,35,38,41,44,47,50,53,56,59,62,65,68,71,74,77-pentacosaoxa-5-azaoctacontan-80-oic acid (69 mg, 0.057 mmol, 65% yield) was obtained. LC/MS [M-H]-1217.4 Rt=0.75 min. (2 min acidic method). 1H NMR (400 MHz, DMSO-d6) δ 7.68 (s, 1H), 3.79 (s, 2H), 3.60 (t, J = 6.4 Hz, 2H), 3.51 (s, 92H), 3.43 (t, J = 6.0 Hz, 2H), 3.30 (s, 3H), 3.26 (q, J = 6.0 Hz, 2H), 2.44 (t, J = 6.4 Hz, 2H).

### Example 3. Synthesis and Characterization of Mcl-1 Payloads

Exemplary payloads were synthesized using exemplary methods described in this example.

### Preparation of C1:

### (2R)-2-{[(5Sₐ)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid

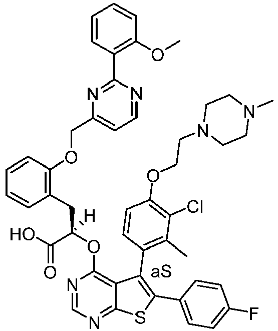

C1 was prepared according to Example 30 in WO 2015/097123.

### Preparation of C2:

### (2R)-2-[(5Sₐ)-5-[3-chloro-2-methyl-4-[2-[4-methyl-4-(3-sulfopropyl) piperazin-4-ium-1-yl]ethoxy]phenyl]-6-(4-fluoro-3-hydroxy-phenyl)thieno[2,3-d] pyrimidin-4-yl]oxy-3-[2-[[2-(2-methoxyphenyl)pyrimidin-4-yl]methoxy]phenyl]propanoic acid

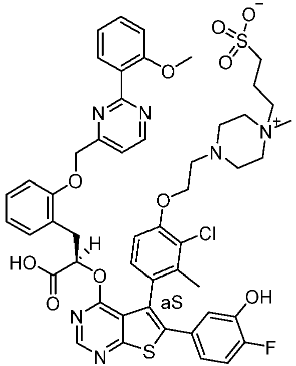

### Step A: 5-bromo-4-chloro-6-(4-fluoro-3-tetrahydropyran-2-yloxy-phenyl)thieno[2,3-d]pyrimidine

4.49 g 5-bromo-4-chloro-6-iodo-thieno[2,3-d]pyrimidine (11.96 mmol; obtained according to WO 2015/097123, Preparation 1a) and 4.31 g (4-fluoro-3-tetrahydropyran-2-yloxy-phenyl)boronic acid (17.94 mmol) were dissolved in 60 mL THF, then 134 mg Pd(OAc)2 (0.60 mmol), 508 mg tBuXPhos (1.20 mmol), 11.69 g Cs2CO3 (35.88 mmol) and 60 mL water were added and the mixture was stirred at 70°C under N2 atmosphere until no further conversion was observed. Then it was diluted with water, neutralized with 2 M aqueous HCl solution, and extracted with DCM. The combined organic layer was dried over Na₂SO₄, filtered and the filtrate was concentrated under reduced pressure. The crude product product was purified via flash chromatography using heptane and EtOAc as eluents to give 5-bromo-4-chloro-6-(4-fluoro-3-tetrahydropyran-2-yloxy-phenyl)thieno[2,3-d]pyrimidine. 1H NMR (500 MHz, DMSO-d₆) δ: 9.02 (s, 1H), 7.64 (dd, *J* = 7.9, 2.1 Hz, 1H), 7.47 (dd, *J* = 11.0, 8.5 Hz, 1H), 7.36 (m, 1H), 5.63 (m, 1H), 3.81 (m, 1H), 3.61 (m, 1H), 1.94-1.78 (m, 3H), 1.69-1.50 (m, 3H). ¹³C NMR (125 MHz, DMSO-d₆) δ: 166.6, 153.9, 153.1, 152.7, 144.3, 139.2, 127.7, 126.6, 124.2, 119.9, 117.1, 100.7, 97.2, 61.6, 29.5, 24.5, 18.2. HRMS calculated for C₁₇H₁₃N₂₀O₂SBrCIF: 441.9554; found 442.9624 (M+H).

### Step B: ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate

3.09 g 5-bromo-4-chloro-6-(4-fluoro-3-tetrahydropyran-2-yloxy-phenyl)thieno[2,3-d]pyrimidine (6.97 mmol), 3.28 g ethyl (2R)-2-hydroxy-3-[2-[[2-(2-methoxyphenyl)pyrimidin-4-yl]methoxy]phenyl]propanoate (8.02 mmol; obtained according to WO 2015/097123, Preparation 3bs) were dissolved in 70 mL tert-butanol, then 6.82 g Cs₂CO₃ (20.9 mmol) was added and the mixture was stirred under N₂ atmosphere at 70°C until no further conversion was observed. Then it was diluted with water, neutralized with 2 M aqueous HCl solution, and extracted with DCM. The combined organic layer was dried over Na₂SO₄, filtered and the filtrate was concentrated under reduced pressure. The crude product was purified via flash chromatography using heptane and EtOAc as eluents to give ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of diastereoisomers. HRMS calculated for C₄₀H₃₆N₄O₇SBrF: 814.1472; found 815.1539 (M+H).

### Step C: ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate

3.65 g ethyl (2R)-2-[5-bromo-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (4.47 mmol) and 2.12 g 1-[2-[2-chloro-3-methyl-4-(4,4,5,5-tetramethyl-1,3,2-dioxaborolan-2-yl)phenoxy]ethyl]-4-methyl-piperazine (5.36 mmol; obtained according to WO 2015/097123, Preparation 5b) were dissolved in 22 mL dioxane, then 315 mg PdCl₂×AtaPhos (0.45 mmol), 4.37 g Cs₂CO₃ (13.41 mmol) and 22 mL water were added and the mixture was stirred at 70°C under N₂ atmosphere until complete conversion. Then it was diluted with water, neutralized with 2 M aqueous HCl solution, and extracted with EtOAc. The combined organic layer was dried over Na₂SO₄, filtered and the filtrate was concentrated under reduced pressure. The crude product product was purified via flash chromatography using EtOAc and MeOH, then DCM and MeOH as eluents to give ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of two diastereoisomer pairs. HRMS calculated for C₅₄H₅₆N₆O₈SCIF: 1002.3553; found 1003.3614 and 1003.3622 (M+H).

### Step D: (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl) pyrimidin-4-yl]methoxy]phenyl]propanoic acid

3.47 g ethyl (2R)-2-[5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (3.46 mmol) was dissolved in 35 mL dioxane, then 1.45 g LiOH×H₂O (34.6 mmol) and 35 mL water were added. The mixture was stirred at room temperature until complete hydrolysis. Then it was diluted with water, acidified to pH 4 with 2 M aqueous HCl solution, and extracted with DCM. The combined organic phase was dried over Na₂SO₄, filtered and the filtrate was concentrated under reduced pressure. The atropisomers were purified and separated via preparative reverse phase chromatography using 25 mM aqueous NH₄HCO₃ solution and MeCN as eluents. The atropisomer pair eluting later was isolated as (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoic acid HRMS calculated for C₅₂H₅₂N₆O₈SCIF: 974.3240; found 975.3303 (M+H).

### Step E: (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate

2.39 g (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoic acid (2.45 mmol), 1.13 g DTBAD (4.91 mmol) and 1.29 g PPh₃ (4.91 mmol) were dissolved in 49 mL toluene, then 0.61 mL PMB-OH (4.91 mmol) was added and the reaction mixture was stirred at 50°C until complete conversion. Then the mixture was diluted with DCM and then concentrated under reduced pressure and then purified via flash chromatography, using heptane and EtOAc as eluents to give (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate as a mixture of diastereoisomers. HRMS calculated for C₆₀H₆₀N₆O₉SCIF: 1094.3815; found 1095.3880 (M+H).

### Step F: (2R)-2-[(5Sₐ)-5-[3-chloro-2-methyl-4-[2-[4-methyl-4-(3-sulfopropyl) piperazin-4-ium-1-yllethoxy]phenyl]-6-(4-fluoro-3-hydroxy-phenyl)thieno[2,3-d] pyrimidin-4-yl]oxy-3-[2-[[2-(2-methoxyphenyl)pyrimidin-4-yl]methoxy]phenyl]propanoic acid (C2)

600 mg (4-methoxyphenyl)methyl (2R)-2-[(5Sa)-5-[3-chloro-2-methyl-4-[2-(4-methylpiperazin-1-yl)ethoxy]phenyl]-6-(4-fluoro-3-tetrahydropyran-2-yloxy-phenyl)thieno[2,3-d]pyrimidin-4-yl]oxy-3-[2-[[2-(2-methoxyphenyl)pyrimidin-4-yl]methoxy]phenyl]propanoate (0.548 mmol) was dissolved in 11 mL MeCN, then 0.48 mL oxathiolane 2,2-dioxide (5.48 mmol) was added, and the mixture was stirred under N₂ atmosphere at 60°C until complete conversion. Then it was concentrated under reduced pressure, dissolved in 8 mL DCM, then 2.2 mL TFA was added and mixture was stirred at room temperature until complete cleavage of THP and PMB. Then it was concentrated (heating bath removed). It was dissolved in 10 mL THF, and concentrated again under reduced pressure in 30°C bath. The crude product was purified via preparative reverse phase chromatography using 5 mM aqueous NH₄HCO₃ solution and MeCN as eluents to obtain give C2. 1H NMR (400 MHz, DMSO-d₆) δ: 13.19 (br s, 1H), 10.16 (br s, 1H), 8.89 (d, *J* = 5.2 Hz, 1H), 8.58 (s, 1H), 7.68 (br s, 1H), 7.52 (dd, *J* = 7.5, 1.8 Hz, 1H), 7.46 (m, 1H), 7.33 (d, *J* = 8.3 Hz, 1H), 7.22-7.09 (m, 4H), 7.06-7.00 (m, 2H), 6.86 (dd, *J* = 8.3, 2.0 Hz, 1H), 6.74 (t, *J* = 7.4 Hz, 1H), 6.66 (m, 1H), 6.23 (d, *J* = 6.7 Hz, 1H), 5.46 (dd, *J* = 9.8, 3.3 Hz, 1H), 5.27 (d, *J =* 15.2 Hz, 1H), 5.22 (d, *J* = 15.2 Hz, 1H), 4.23 (m, 2H), 3.76 (s, 3H), 3.46 (m, 2H), 3.41-3.23 (m, 5H), 2.97 (s, 3H), 2.94-2.77 (m, 6H), 2.48 (m, 1H), 2.45 (t, *J* = 7.0 Hz, 2H), 2.00-1.90 (m, 2H), 1.86 (s, 3H). ¹³C NMR (100 MHz, DMSO-d₆) δ: 170.8, 166.2, 165.9, 164.7, 157.7, 157.2, 155.4, 153.6, 152.7, 152.3, 149.9, 145.1, 137.0, 135.9, 131.0, 130.8, 130.3, 129.2, 128.34, 128.32, 128.2, 128.0, 122.0, 120.5, 120.1, 118.8, 118.2, 116.6, 115.6, 112.2, 111.9, 110.6, 73.3, 69.0, 67.3, 59.2, 59.1, 55.71, 55.68, 47.6, 46.1, 31.8, 18.1, 17.6. HRMS calculated for C₅₀H₅₀N₆O₁₀S₂CIF: 1012.2703; found 1013.2775 (M+H).

### Preparation of C3:

### (2R)-2-{[(5Sa)-5-{3-chloro-2-methyl-4-[2-(piperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid

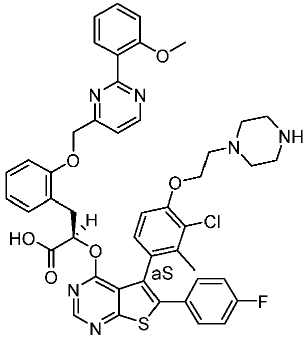

C3 was prepared according to Example 744 in WO 2015/097123.

### Preparation of C4:

### (2R)-2-{[(5Sa)-5-{3-chloro-2-methyl-4-[2-(piperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid

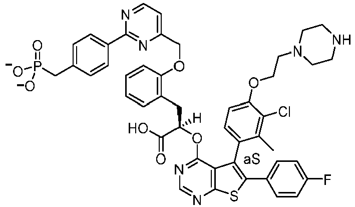

### Step 1: ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate

To a solution of ethyl (2R)-3-[2-[(2-chloropyrimidin-4-yl)methoxy]phenyl]-2-hydroxy-propanoate (25 g, 74.2 mmol) in THF (38 mL) were successively added 5-bromo-6-(4-fluorophenyl)-4-iodo-thieno[2,3-d]pyrimidine (23 g, 67.5mmol) and cesium carbonate (67 g, 203 mmol). The reaction was heated at reflux overnight, and the volatiles were evaporated. The residue was diluted with ethyl acetate and water (respectively 500 and 400 mL) and the solution is filtered. The organic layer was separated, washed with brine, dried over magnesium sulfate and concentrated under vacuum. The residue was purified by silica gel chromatography (gradient of ethyl acetate in petroleum ether to afford ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate as a slightly orange solid. ¹H NMR (400 MHz, dmso-d6): δ 8.83 (d, 1H), 8.65 (s, 1H), 7.75 (m, 2H), 7.71 (d, 1H), 7.48 (d, 1H), 7.45 (m, 2H), 7.25 (t, 1H), 7.06 (d, 1H), 6.95 (t, 1H), 5.75 (dd, 1H), 5.28 (2*d, 2H), 4.18 (q, 2H), 3.6/3.3 (2*dd, 2H), 1.12 (t, 3H). IR Wavelenght (cm⁻¹): 1749.

### Step 2: (4-bromo-2-chloro-3-methyl-phenoxy)-triisopropyl-silane

To a solution of 4-bromo-2-chloro-3-methyl-phenol (100 g, 482 mmol) in dichloromethane (1.5 L) were added imidazole (82 g, 1.2 mol) and dropwise over 1 h chloro(triisopropyl)silane (102 mL, 482 mmol). The reaction was stirred at room temperature for 1 h and water was added (500 mL). The organic layers were washed with brine (200 mL), dried over Magnesium sulfate and concentrated. The residue was used without further purification. ¹H NMR (400 MHz, CDCl₃): δ 7.48 (s, 1H), 7.2 (dd, 1H), 6.7 (d, 1H), 1.3 (m, 3H), 1.1 (2s, 18H).

### Step 3: tert-butyl-[2-chloro-3-methyl-4-(4,4,5,5-tetramethyl-1,3,2-dioxaborolan-2-yl)phenoxy]-dimethyl-silane

To a solution of (4-bromo-2-chloro-3-methyl-phenoxy)-triisopropyl-silane (27.2 g, 71.9 mmol) in THF (350 mL) at -78°C under Argon was added dropwise over 30 min a solution of n-butyl lithium 1.6 M in THF (49.5 mL, 79.9 mmol). The reaction was stirred at -78 °C for 2 h and a solution of 2-Isopropoxy-4,4,5,5-tetramethyl-1,3,2-dioxaborolane (16.1 g, 86.4 mmol) in THF (50 mL) was added dropwise over 30 min. After 2h stirring at -78°C, the reaction mixture was quenched by a slow addition of water (20 mL) and warmed to room temperature, diluted with water (200 mL) and extracted with ethyl acetate. The combined organic layers were washed with brine, dried over Magnesium sulfate and concentrated under vacuum. The residue was used without further purification. ¹H NMR (400 MHz, dmso-d6): δ 7.5 (d, 1H), 6.82 (d, 1H), 2.52 (s, 3H), 1.32 (m, 3H), 1.3 (s, 12H), 1.08 (s, 18H).

### Step 4: 2-chloro-3-methyl-4-(4,4,5,5-tetramethyl-1,3,2-dioxaborolan-2-yl)phenol

To a solution of tert-butyl-[2-chloro-3-methyl-4-(4,4,5,5-tetramethyl-1,3,2-dioxaborolan-2-yl)phenoxy]-dimethyl-silane (25.4 g, 59.8 mmol) in THF (750 mL) was added dropwise at room temperature a solution of Tetrabutylammonium Fluoride 1 M in THF (90 mL, 90 mmol). The reaction mixture was stirred for 2 h, concentrated, diluted with ethyl acetate, partitioned with water and extracted with ethyl acetate. The combined organic layers were washed with brine, dried over Magnesium sulfate and concentrated under vacuum. The residue was purified by silica gel chromatography (gradient of ethanol in dichloromethane) to afford 2-chloro-3-methyl-4-(4,4,5,5-tetramethyl-1,3,2-dioxaborolan-2-yl)phenol. 1H NMR (400 MHz, dmso-d6): δ 10.4 (m, 1H), 7.4 (d, 1H), 6.8 (d, 1H), 2.5 (s, 3H), 1.3 (s, 12H).IR Wavelenght (cm⁻¹): 3580-3185, 1591, 857, 827.

### Step 5: ethyl (2R)-2-{[(5Sₐ)-5-(3-chloro-4-hydroxy-2-methylphenyl)-6-(4-fluorophenyl)thieno[2,3-d]pyrimidin-4-yl]oxy}-3-{2-[(2-chloropyrimidin-4-yl)methoxy]phenyl}propanoate

To a solution of ethyl (2R)-2-[5-bromo-6-(4-fluorophenyl)thieno[2,3-d]pyrimidin-4-yl]oxy-3-[2-[(2-chloropyrimidin-4-yl)methoxy]phenyl]propanoate (43.8 g, 61.2 mmol) and 2-chloro-3-methyl-4-(4,4,5,5-tetramethyl-1,3,2-dioxaborolan-2-yl)phenol (19.7 g, 73.5 mmol) in a mixture of THF/H₂O 1/1 (800 mL) was added cesium carbonate (40 g, 122 mmol). The reaction was degased by bubbling argon through the solution for 20 min and Bis(di-tert-butyl(4-dimethylaminophenyl)phosphine)dichloropalladium(II) (4.35g, 6.1 mmol) was added . The reaction mixture was heated at 80°C under argon overnight. The reaction was diluted with water, partitioned with ethyl acetate and extracted with ethyl acetate. The combined organic layers were washed with brine, dried over Magnesium sulfate and concentrated under vacuum. The residue was purified by silica gel chromatography (gradient of methanol in dichloromethane) to afford ethyl (2R)-2-[5-(3-chloro-4-hydroxy-2-methyl-phenyl)-6-(4-fluorophenyl)thieno[2,3-d]pyrimidin-4-yl]oxy-3-[2-[(2-chloropyrimidin-4-yl)methoxy]phenyl]propanoate as a mixture of diastereoisomers 85/15 (aS/aR or Sₐ/Rₐ).Optically pure *aS* (or Sₐ) was obtained by Preparative SFC purification.

### Step 6: tert-butyl 4-(2-{2-chloro-4-[4-{[(2R)-3-{2-[(2-chloropyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate

To a solution of triphenylphosphine (2.66 g, 10 mmol) in THF was added at room temperature under argon Diisopropyl azodicarboxylate (2.33 g, 10 mmol). After 15 min of stirring, was added a solution of tert-butyl 4-(2-hydroxyethyl)piperazine-1-carboxylate (2.33g, 10 mmol) in THF (8 mL). The reaction was stirred at room temperature for 1 h, then was added dropwise a solution of and (2R)-2-[(5Sₐ)-5-(3-chloro-4-hydroxy-2-methyl-phenyl)-6-(4-fluorophenyl)thieno[2,3-d]pyrimidin-4-yl]oxy-3-[2-[(2-chloropyrimidin-4-yl)methoxy]phenyl]propanoic acid (3.57 g, 5 mmol) in THF (8 mL). The reaction was stirred at room temperature for 96 h and concentrated. The residue was purified by silica gel chromatography (gradient of methanol (containing 7M ammonia) in dichloromethane) to afford tert-butyl 4-(2-{2-chloro-4-[4-{[(2R)-3-{2-[(2-chloropyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate.¹H NMR (400 MHz, CDCl₃): δ 8.95 (d, 1H), 8.58 (s, 1H), 8.32 (d, 2H), 7.58 (d, 1H), 7.41 (dd, 2H), 7.32 (d, 1H), 7.29 (dd, 2H), 7.22 (t, 2H), 7.21 (d, 1H), 7.19 (t, 1H), 7.05 (d, 1H), 6.75 (t, 1H), 6.31 (dd, 1H), 5.53 (dd, 1H), 5.29 (dd, 2H), 4.2 (m, 2H), 4.05 (q, 2H), 3.97 (m, 4H), 3.3 (m, 2H), 3.2 (t, 4H), 3.19/2.59 (m, 2H), 2.72 (t, 2H), 2.4 (t, 4H), 1.87 (s, 3H), 1.37 (s, 9H), 1.18 (t, 6H), 1.05 (t, 3H). ¹³C NMR (125 MHz, CDCl₃): δ 158, 152, 131, 131, 130, 130, 128, 127, 120.5, 116, 116, 112, 110, 73, 68.5, 67, 62, 61, 56, 52, 43, 32, 32, 28, 17, 16, 14.

### Step 7: tert-butyl 4-(2-{2-chloro-4-[4-{[(2R)-3-{2-[(2-{4-[(diethoxyphosphoryl)methyl]phenyl}pyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate

To a solution of tert-butyl 4-(2-{2-chloro-4-[4-{[(2R)-3-{2-[(2-chloropyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate (337 mg, 0.367 mmol) and [4-(diethoxyphosphorylmethyl)phenyl]boronic acid (200mg, 0.735 mmol) in dioxane (2.5 mL), were added water (2.5 mL) and cesium carbonate (241 mg, 0.735 mmol). The reaction mixture was degased by bubbling argon through the solution for 30 min, Bis(triphenylphosphine)palladium(II) dichloride (2.5 mg, 3.6 µmol) was added and the reaction mixture was heated by microwave irradiation in a sealed vessel at 90 °C for 3 h. The reaction was diluted with ethyl acetate and water. The aqueous layer was extracted with ethyl acetate and the combined organic layers were washed with brine, dried over Magnesium sulfate and concentrated under vacuum. The residue was purified by silica gel chromatography (gradient of methanol in dichloromethane) to afford tert-butyl 4-(2-{2-chloro-4-[4-{[(2R)-3-{2-[(2-{4-[(diethoxyphosphoryl)methyl]phenyl}pyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate.¹H NMR (500 MHz, dmso-d6): δ 8.95 (d, 1H), 8.58 (s, 1H), 8.32 (d, 2H), 7.58 (d, 1H), 7.41 (dd, 2H), 7.32 (d, 1H), 7.29 (dd, 2H), 7.22 (t, 2H), 7.21 (d, 1H), 7.19 (t, 1H), 7.05 (d, 1H), 6.75 (t, 1H), 6.31 (dd, 1H), 5.53 (dd, 1H), 5.29 (2*d, 2H), 4.2 (m, 2H), 4.05 (q, 2H), 3.97 (m, 4H), 3.3 (m, 2H), 3.2 (t, 4H), 3.19/2.59 (2*dd, 2H), 2.72 (t, 2H), 2.4 (t, 4H), 1.87 (s, 3H), 1.37 (s, 9H), 1.18 (t, 6H), 1.05 (t, 3H). ¹³C NMR (125 MHz, dmso-d6) δ 158, 152, 131, 131, 130, 130, 128, 127, 120.5, 116, 116, 112, 110, 73, 68.5, 67, 62, 61, 56, 52, 43, 32, 32, 28, 17, 16, 14

### Step 8: Synthesis of (2R)-2-[(5Sₐ)-S-[3-chloro-2-methyl-4-(2-piperazin-1-ylethoxy)phenyl]-6-(4-fluorophenyl)thieno[2,3-d]pyrimidin-4-yl]oxy-3-[2-[[2-[4-(phosphonomethyl)phenyl]pyrimidin-4-yl]methoxy]phenyl]propanoic acid

To a solution of tert-butyl 4-(2-{2-chloro-4-[4-{[(2R)-3-{2-[(2-{4-[(diethoxyphosphoryl)methyl]phenyl}pyrimidin-4-yl)methoxy]phenyl}-1-ethoxy-1-oxopropan-2-yl]oxy}-6-(4-fluorophenyl)thieno[2,3-d]pyrimidin-5-yl]-3-methylphenoxy}ethyl)piperazine-1-carboxylate (540 mg, 0.486 mmol) in dichloromethane (5 mL) was added bromotrimethylsilane (186 µL, 1.46 mmol). The reaction mixture was heated to reflux overnight. Another portion of bromotrimethylsilane was added at room temperature (186 µL, 1.46mmol) and the reaction was heated at reflux for 20h and concentrated to dryness. The residue was taken up in methanol, stirred at room temperature for 3h and concentrated to afford a brown viscous oil which was diluted with dioxane (4 mL) and water (4 mL). Lithium hydroxide monohydrate (100 mg, 24 mmol) was added by portions and the reaction mixture was stirred at room temperature for 1 h, heated at 45°C for 3 h and concentrated. The residue was diluted with water (5 mL), acidified to pH2 by dropwise addition of an aqueous 2 M HCl solution. The precipitate was filtered, washed with THF and purified by C18 reverse phase prep-HPLC by direct deposit of the reaction mixture on the Xbridge column and using the NH₄HCO₃ method to afford **C4.** ¹H NMR (500 MHz, dmso-d6): δ 8.88 (br d, 1 H), 8.25 (d, 2 H), 7.75 (t, 1 H), 7.59 (s, 1 H), 7.52 (d, 1 H), 7.35 (d, 2 H), 7.23 (dd, 2 H), 7.18 (d, 1 H), 7.15 (t, 2 H), 7.11 (t, 1 H), 7.02 (d, 1 H), 6.82 (d, 1 H), 6.64 (m, 1 H), 5.51 (d, 1 H), 5.28/5.07 (m, 2 H), 3.82/3.55 (2m, 2 H), 3.35/2.55 (br s, 2 H), 2.81 (d, 2 H), 2.55 (m, 4 H), 2.4/2.27 (2m, 2 H), 2.21 (m, 4 H), 1.65 (br s, 3 H). ¹³C NMR (125 MHz, dmso-d6): δ 131.5, 130.2, 129.7, 127.4, 127.2, 120.3, 115.9, 115.3, 111.9, 110.3, 75.1, 69.2, 67.3, 56.4, 49.9, 42.4, 40, 38.9, 18.1. ³¹P NMR (200 MHz, dmso-d6): δ 15 HR-ESI+ : m/z [M+H]+ = 925.2356 / 925.2346 (measured/theoretical)

### Preparation of C5:

### (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(4-hydroxyphenyl)pyrimidin-4-yl]methoxy}phenyl)propanoic acid

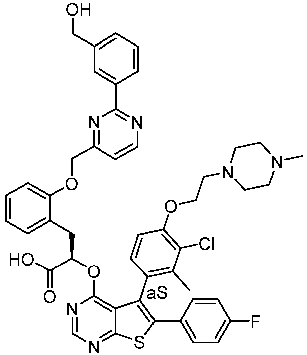

C5 was prepared according to Example 3 in WO 2016/207216.

### Preparation of C6:

### (2R)-2-{[(5Sₐ)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-[2-({2-[2-(hydroxymethyl)phenyl]pyrimidin-4-yl}methoxy)phenyl]propanoic acid

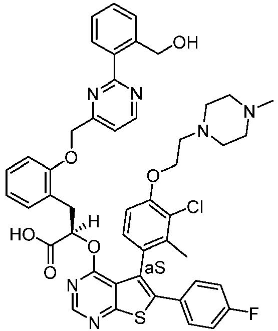

C6 was prepared according to Example 728 in WO 2015/097123.

### Preparation of C7:

### (2R)-2-[(5Sₐ)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid

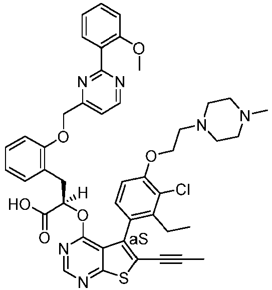

### Step A: ethyl (2R)-2-(5-iodo-6-prop-1-ynyl-thieno[2,3-d]pyrimidin-4-yl)oxy-3-[2-[[2-(2-methoxyphenyl)pyrimidin-4-yl]methoxy]phenyl]propanoate

5.0 g 4-chloro-5-iodo-6-prop-1-ynyl-thieno[2,3-d]pyrimidine (15.0 mmol; obtained according to WO 2015/097123, Preparation 2f) and 6.10 g ethyl (2R)-2-hydroxy-3-[2-[[2-(2-methoxyphenyl)pyrimidin-4-yl]methoxy]phenyl]propanoate (15.0 mmol; obtained according to WO 2015/097123, Preparation 3bs) were dissolved in 150 mL tert-butanol, then 14.7 g Cs₂CO₃ (45.0 mmol) was added and the mixture was stirred under N₂ atmosphere at 50°C until no further conversion was observed. Then water and brine was added and the mixture was extracted with EtOAc. The combined organic layer was dried over Na₂SO₄, filtered and the filtrate was concentrated under reduced pressure. The crude product was purified via flash chromatography using heptane and EtOAc as eluents to give ethyl (2R)-2-(5-iodo-6-prop-1-ynyl-thieno[2,3-d]pyrimidin-4-yl)oxy-3-[2-[[2-(2-methoxyphenyl)pyrimidin-4-yl]methoxy]phenyl]propanoate. 1H NMR (400 MHz, DMSO-d₆) δ: 8.89 (d, *J* = 5.1 Hz, 1H), 8.59 (s, 1H), 7.62 (d, *J* = 5.2 Hz, 1H), 7.55 (dd, *J* = 7.5, 1.6 Hz, 1H), 7.51 (dd, *J* = 7.5, 1.6 Hz, 1H), 7.43 (m, 1H), 7.26 (m, 1H), 7.11 (m, 2H), 7.02 (td, *J* = 7.5, 0.9 Hz, 1H), 6.94 (td, *J* = 7.4, 0.8 Hz, 1H), 5.79 (dd, *J* = 9.1, 4.6 Hz, 1H), 5.31 (d, *J* = 14.9 Hz, 1H), 5.26 (d, *J* = 14.9 Hz, 1H), 4.13 (m, 2H), 3.76 (s, 3H), 3.60 (dd, *J* = 13.8, 4.5 Hz, 1H), 3.33 (m, 1H), 2.21 (s, 3H), 1.10 (t, *J* = 7.1 Hz, 3H). ¹³C NMR (100 MHz, DMSO-d₆) δ: 169.4, 166.5, 165.7, 164.8, 161.3, 157.7, 155.8, 153.6, 132.2, 131.0, 130.8, 128.3, 124.0, 120.9, 120.1, 115.5, 112.2, 112.0, 110.5, 98.9, 79.5, 74.4, 74.3, 69.1, 61.1, 55.7, 13.9, 4.6.

### Step B: 2-chloro-3-ethyl-4-(4,4,5,5-tetramethyl-1,3,2-dioxaborolan-2-yl)phenol

33.7 g [2-chloro-3-ethyl-4-(4,4,5,5-tetramethyl-1,3,2-dioxaborolan-2-yl)phenoxy]-triisopropyl-silane (76.9 mmol; obtained according to WO 2015/097123, Preparation 5e) was dissolved in 600 mL THF and was cooled to 0°C, then 92.3 mL TBAF (92.3 mmol, 1M solution in THF) was added dropwise and the mixture was stirred until complete conversion. Then it was diluted with brine, acidified with citric acid then extracted with DCM. The combined organic layer was dried over Na₂SO₄, filtered and the filtrate was concentrated under reduced pressure. The crude product was purified via flash chromatography using heptane and EtOAc as eluents to give 2-chloro-3-ethyl-4-(4,4,5,5-tetramethyl-1,3,2-dioxaborolan-2-yl)phenol. 1H NMR (400 MHz, CDCl₃) δ: 7.63 (d, *J* = 8.2 Hz, 1H), 6.86 (d, *J* = 8.2 Hz, 1H), 5.87 (s, 1H), 3.09 (q, *J =* 7.44 Hz, 2H), 1.33(s, 12H), 1.15 (t, *J* = 7.44 Hz, 3H).

### Step C: ethyl (2R)-2-[5-(3-chloro-2-ethyl-4-hydroxy-phenyl)-6-prop-1-ynyl-thieno[2,3-d]pyrimidin-4-yl]oxy-3-[2-[[2-(2-methoxyphenyl)pyrimidin-4-yl]methoxy]phenyl]propanoate

353 mg ethyl (2R)-2-(5-iodo-6-prop-1-ynyl-thieno[2,3-d]pyrimidin-4-yl)oxy-3-[2-[[2-(2-methoxyphenyl)pyrimidin-4-yl]methoxy]phenyl]propanoate (0.50 mmol) and 282 mg 2-chloro-3-ethyl-4-(4,4,5,5-tetramethyl-1,3,2-dioxaborolan-2-yl)phenol (0.55 mmol) were dissolved in 2 mL dioxane, then 35 mg PdCl₂×AtaPhos (0.05 mmol), 326 mg Cs₂CO₃ (1.00 mmol) and 1 mL water were added and the mixture was stirred in a microwave reactor at 100°C under N₂ atmosphere for 20 minutes. Then it was diluted with brine, acidified to pH 5 with 1 M aqueous HCl solution and extracted with DCM. The combined organic layer was dried over Na₂SO₄, filtered and the filtrate was concentrated under reduced pressure. The crude product was purified via flash chromatography using heptane and EtOAc as eluents. Then it was further purified via preparative reverse phase chromatography using 5 mM aqueous NH₄HCO₃ solution and MeCN as eluents to give ethyl (2R)-2-[5-(3-chloro-2-ethyl-4-hydroxyphenyl)-6-prop-1-ynyl-thieno[2,3-d]pyrimidin-4-yl]oxy-3-[2-[[2-(2-methoxyphenyl)pyrimidin-4-yl]methoxy]phenyl]propanoate as a 2:1 mixture of atropisomers. ¹H NMR (500 MHz, DMSO-d₆) δ: 10.35/10.29 (s, 1H), 8.93 (d, *J* = 5.1 Hz, 1H), 8.59/8.57 (s, 1H), 7.63/7.60 (d, *J* = 5.1 Hz, 1H), 7.54-6.93 (m, 8 H), 6.84/6.74 (t, *J =* 7.5 Hz, 1H), 6.43/6.18 (dd, *J* = 7.5, 1.4 Hz, 1H), 5.51/5.40 (m, 1H), 5.30-5.16 (m, 2H), 4.17-3.99 (m, 2H), 3.76/3.75 (s, 3H), 3.34-3.14 (m, 1H), 2.93-2.35 (m, 3H), 2.02/1.98 (s, 3H), 1.08/1.04 (t, *J* = 7.0 Hz, 3H), 0.94/0.76 (t, *J* = 7.5 Hz, 3H).

### Step D: (2R)-2-[(5Sa)-5-[3-chloro-2-ethyl-4-[2-(4-methylpiperazin-1-yl)ethoxy]phenyl]-6-prop-1-ynyl-thieno[2,3-d]pyrimidin-4-yl]oxy-3-[2-[[2-(2-methoxyphenyl)pyrimidin-4-yl]methoxy]phenyl]propanoic acid, C7

322 mg ethyl (2R)-2-[5-(3-chloro-2-ethyl-4-hydroxy-phenyl)-6-prop-1-ynyl-thieno[2,3-d]pyrimidin-4-yl]oxy-3-[2-[[2-(2-methoxyphenyl)pyrimidin-4-yl]methoxy]phenyl]propanoate (0.44 mmol), 190 mg 2-(4-methylpiperazin-1-yl)ethanol (1.32 mmol) and 346 mg PPh₃ (1.32 mmol) were dissolved in 10 mL dry toluene, then 304 mg DTBAD (1.32 mmol) was added and the mixture was stirred at 50°C under N₂ atmosphere until no further conversion was observed. Then the mixture was concentrated under reduced pressure and the residue was purified via flash chromatography using heptane, EtOAc and MeOH as eluents, then further purified via preparative reverse phase chromatography using 5 mM aqueous NH₄HCO₃ solution and MeCN as eluents to obtain the ester intermediate. It was dissolved in 2 mL dioxane, then 84 mg LiOH×H₂O (2.00 mmol) and 1 mL water were added. The mixture was stirred at 50°C until complete hydrolysis. Then it was diluted with brine, neutralized with 2 M aqueous HCl solution, and extracted with DCM. The combined organic phase was dried over Na₂SO₄, filtered and the filtrate was concentrated under reduced pressure. The atropoisomers were purified and separated via preparative reverse phase chromatography using 5 mM aqueous NH₄HCO₃ solution and MeCN as eluents. The atropoisomer eluting later was isolated as C7. 1H NMR (400 MHz, DMSO-d₆) δ: 8.88 (d, *J* = 5.2 Hz, 1H), 8.60 (s, 1H), 7.76 (d, *J* = 5.0 Hz, 1H), 7.54 (dd, *J* = 7.6, 1.8 Hz, 1H), 7.46 (m, 1H), 7.26 (d, *J* = 8.5 Hz, 1H), 7.20-7.13 (m, 3H), 7.04 (td, *J* = 7.5, 0.9 Hz, 1H), 7.00 (d, *J* = 8.0 Hz, 1H), 6.78 (t, *J* = 7.5 Hz, 1H), 6.32 (dd, *J* = 7.4, 1.5 Hz, 1H), 5.48 (dd, *J* = 9.7, 2.9 Hz, 1H), 5.27 (d, *J* = 15.0 Hz, 1H), 5.19 (d, *J* = 15.0 Hz, 1H), 4.23 (m, 2H), 3.76 (s, 3H), 3.31 (m, 1H), 2.75 (m, 2H), 2.47 (m, 1H), 2.64-2.36 (m, 10H), 2.22 (s, 3H), 2.01 (s, 3H), 0.74 (t, *J* = 7.5 Hz, 3H). ¹³C NMR (100 MHz, DMSO-d₆) δ: 165.95, 165.89, 164.7, 157.8, 157.2, 155.3, 154.0, 141.6, 135.6, 131.0, 130.8, 130.1, 128.4, 128.0, 127.2, 121.4, 120.1, 117.8, 112.2, 111.7, 97.2, 74.9, 68.9, 67.1, 56.1, 55.7, 54.0, 32.7, 24.4, 13.1, 4.4. HRMS calculated for C₄₅H₄₅N₆O₆SCl: 832.2810; found 833.2878 (M+H).

### Preparation of C8:

### (2R)-2-[(5Sₐ)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(3-sulfooxyphenyl)pyrimidin-4-yl]methoxy]phenyl]propanoic acid

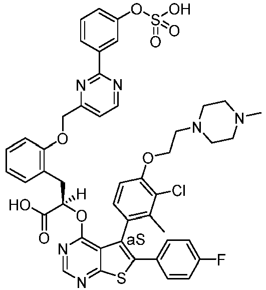

210 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[[2-(3-hydroxyphenyl)pyrimidin-4-yl]methoxy]phenyl]propanoate (0.24 mmol; obtained according to WO 2016/207216, Preparation 2) was dissolved in 9.3 mL pyridine, than 0.38 mL SO₃×pyridine (2.36 mmol) was added and the mixture was stirred at 70°C until complete conversion. Then the mixture was concentrated under reduced pressure and the residue was dissolved in 2 mL dioxane, then 200 mg KOH (3.56 mmol) and 1 mL water were added. The mixture was stirred at 70°C until complete hydrolysis of the Et ester. Then it was neutralized with 5 M aqueous HCl solution, and purified via preparative reverse phase chromatography (direct injection) using 25 mM aqueous NH₄HCO₃ solution and MeCN as eluents to give **C8.** ¹H NMR (500 MHz, DMSO-d₆) δ: 8.93 (d, *J* = 5.1 Hz, 1H), 8.63 (s, 1H), 8.27 (m, 1H), 8.13 (m, 1H), 7.61 (d, *J* = 5.1 Hz, 1H), 7.43 (t, *J* = 7.8 Hz, 1H), 7.33 (d, *J* = 7.8 Hz, 1H), 7.32-7.27 (m, 3H), 7.23-7.13 (m, 4H), 7.05 (d, *J = 7.8* Hz, 1H), 6.73 (t, *J* = 7.5 Hz, 1H), 6.31 (dd, *J* = 7.5, 1.3 Hz, 1H), 5.51 (dd, *J* = 9.8, 3.5 Hz, 1H), 5.34 (d, *J* = 15.2 Hz, 1H), 5.27 (d, *J* = 15.2 Hz, 1H), 4.24-4.08 (m, 2H), 3.26 (dd, *J* = 14.3, 3.4 Hz, 1H), 3.10-2.52 (m, 14H), 1.82 (s, 3H). ¹³C NMR (125 MHz, DMSO-d₆) δ: 170.8, 166.5, 166.4, 162.8, 162.7, 158.3, 155.3, 154.0, 153.6, 152.9, 137.8, 136.8, 135.9, 131.12, 131.05, 130.4, 130.3, 129.1, 128.5, 128.2, 127.8, 124.4, 123.5, 122.7, 121.9, 120.5, 120.2, 118.8, 116.0, 115.9, 112.0, 110.5, 73.5, 69.1, 67.2, 55.4, 43.1, 31.8, 17.5. HRMS calculated for C₄₆H₄₂N₆O₉S₂ClF: 940.2127; found 941.2191 (M+H).

### Preparation of C9:

### 2R)-2-{[5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(3-sulfophenyl)pyrimidin-4-yl]methoxy}phenyl)propanoic acid

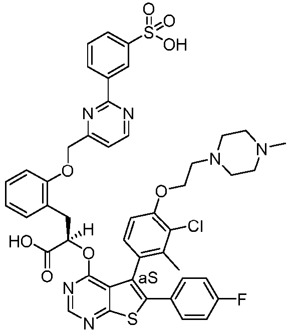

750 mg ethyl (2R)-2-[(5Sa)-5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-methylsulfanylpyrimidin-4-yl)methoxy]phenyl]propanoate (0.89 mmol; obtained according to WO 2015/097123, Preparation 10a) was dissolved in 9 mL THF, then 726 mg [3-(2,2-dimethylpropoxysulfonyl)phenyl]boronic acid (2.67 mmol), 62 mg Pd(PPh₃)₄ (0.05 mmol ) and 509 mg thiophene-2-carbonyloxycopper (2.67 mmol) were added and the mixture was stirred at 75°C until complete conversion. Then it was concentrated under reduced pressure and was purified via flash chromatography using heptane, EtOAc and 0.7 M NH₃ solution in MeOH as eluents. Then it was dissolved in 20 mL 1,1,1,3,3,3-hexafluoropropan-2-ol, 4.5 mL TFA was added and the mixture was stirred at 80°C for until complete hydrolysis of the sulfonic ester. The mixture was concentrated under reduced pressure and then dissolved in 5 mL dioxane, then 210 mg LiOH×H₂O (5.00 mmol) and 2 mL water were added. The mixture was stirred at room temperature until complete hydrolysis. Then it was diluted with brine, neutralized with 2 M aqueous HCl solution, and extracted with DCM. The combined organic phase was dried over Na₂SO₄, filtered and the filtrate was concentrated under reduced pressure. The formed atropisomers were purified and separated via preparative reverse phase chromatography using 25 mM aqueous NH₄HCO₃ solution and MeCN as eluents, then further purified using 0.1% aqueous TFA solution and MeCN as eluents to give C9. ¹H NMR (500 MHz, DMSO-d₆) δ: 13.19 (br s, 1H), 9.48 (br s, 1H), 8.94 (d, *J* = 5.1 Hz, 1H), 8.74 (t, *J* = 1.6 Hz, 1H), 8.65 (s, 1H), 8.37 (dt, *J* = 7.8, 2.9 Hz, 1H), 7.78 (dt, *J* = 7.6, 1.5 Hz, 1H), 7.60 (d, *J* = 5.1 Hz, 1H), 7.51 (t, *J* = 7.7 Hz, 1H), 7.29 (m, 3H), 7.22-7.14 (m, 3H), 7.13-7.05 (m, 2H), 6.74 (t, *J* = 7.5 Hz, 1H), 6.38 (d, *J* = 7.6 Hz, 1H), 5.53 (dd, *J* = 9.6, 3.6 Hz, 1H), 5.37 (d, *J* = 15.3 Hz, 1H), 5.31 (d, *J* = 15.3 Hz, 1H), 4.2 (m, 2H), 3.50-2.88 (m, 11H), 2.76 (s, 3H), 2.61 (dd, *J* = 14.2, 9.7 Hz, 1H), 1.79 (s, 3H). ¹³C NMR (125 MHz, DMSO-d₆) δ: 170.8, 166.5, 163.0, 162.7, 161.1, 158.4, 155.4, 153.3, 152.9, 148.6, 136.6, 136.0, 131.1, 130.1, 129.0, 128.5, 128.3, 128.1, 127.9, 125.3, 124.4, 121.9, 120.5, 118.8, 116.2, 116.0, 115.9, 112.1, 110.5, 73.2, 69.1, 66.5, 55.0, 51.7, 49.7, 42.1, 31.5, 17.5. HRMS calculated for C₄₆H₄₂N₆O₈S₂CIF: 924.2178; found 925.2274 (M+H).

### Preparation of C10:

### (2R)-2-[(5Sₐ)-5-[3-chloro-2-methyl-4-[2-(4-methylpiperazin-1-yl)ethoxy]phenyl]-6-[4-fluoro-3-(2,2,2-trifluoroethoxy)phenyl]thieno[2,3-d]pyrimidin-4-yl]oxy-3-[2-[[2-(2-methoxyphenyl)pyrimidin-4-yl]methoxy]phenyl]propanoic acid

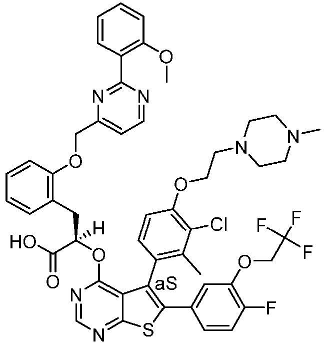

250 mg ethyl (2R)-2-[5-[3-chloro-2-methyl-4-[2-(4-methylpiperazin-1-yl)ethoxy]phenyl]-6-iodo-thieno[2,3-d]pyrimidin-4-yl]oxy-3-[2-[[2-(2-methoxyphenyl)pyrimidin-4-yl]methoxy]phenyl]propanoate (0.27 mmol; obtained according to WO 2015/097123, Preparation 30) and 79 mg [4-fluoro-3-(2,2,2-trifluoroethoxy)phenyl]boronic acid (0.40 mmol) were dissolved in 1 mL THF, then 3.0 mg PdOAc₂ (0.013 mmol), 5.7 mg tBuXPhos (0.013 mmol), 174 mg Cs₂CO₃ (0.53 mmol) and 0.27 mL water were added and the mixture was stirred at 70°C under N₂ atmosphere for 2 hours. Then it was diluted with brine, and extracted with 2-MeTHF. The combined organic layer was dried over Na₂SO₄, filtered and the filtrate was concentrated under reduced pressure. The crude ester product was purified via flash chromatography using heptane, EtOAc and 0.7 M NH₃ solution in MeOH as eluents. Then it was dissolved in 5.3 mL dioxane, then 64 mg LiOH×H₂O (1.52 mmol) and 1.3 mL water were added. The mixture was stirred at room temperature until complete hydrolysis. Then it was diluted with brine, neutralized with 2 M aqueous HCl solution, and extracted with DCM. The combined organic phase was dried over Na₂SO₄, filtered and the filtrate was concentrated under reduced pressure. The atropisomers were purified and separated via preparative reverse phase chromatography using 25 mM aqueous NH₄HCO₃ solution and MeCN as eluents. The atropisomer eluting later was isolated as C10. ¹H NMR (500 MHz, DMSO-d₆) δ: 8.91 (d, *J* = 5.2 Hz, 1H), 8.57 (s, 1H), 7.82 (d, *J* = 5.1 Hz, 1H), 7.53 (dd, *J* = 7.5, 1.7 Hz, 1H), 7.45 (m, 2H), 7.27 (dd, *J* = 11.0, 8.6 Hz, 1H), 7.22 (d, *J* = 8.6 Hz, 1H), 7.16-7.09 (m, 3H), 7.03 (t, *J* = 7.5 Hz, 1H), 6.98 (d, *J* = 8.3 Hz, 1H), 6.92 (m, 1H), 6.69 (t, *J* = 7.4 Hz, 1H), 6.16 (d, *J* = 7.2 Hz, 1H), 5.47 (dd, *J* = 10.3, 2.6 Hz, 1H), 5.25 (d, *J* = 15.1 Hz, 1H), 5.19 (d, *J* = 15.1 Hz, 1H), 4.75-4.53 (m, 2H), 4.21 (t, *J* = 5.5 Hz, 2H), 3.75 (s, 3H), 3.41 (d, *J* = 12.0 Hz, 1H), 2.77-2.30 (m, 12H), 2.24 (s, 3H), 1.81 (s, 3H). ¹³C NMR (125 MHz, DMSO-d₆) δ: 171.3, 166.2, 166.0, 164.6, 163.5, 157.9, 157.2, 155.3, 153.7, 153.1, 152.1, 150.5, 144.6, 135.8, 131.0, .130.8, 130.7, 129.6, 129.1, 128.4, 128.1, 127.8, 125.4, 123.7, 122.0, 120.4, 120.1, 118.8, 117.0, 116.7, 115.6, 112.2, 111.7, 110.5, 74.8, 68.9, 67.2, 65.6, 56.0, 55.7, 53.8, 52.0, 44.6, 32.7, 17.5. HRMS calculated for C₄₉H₄₅N₆O₇SCIF₄: 972.2695; found 973.2761 (M+H).

### Preparation of C11:

### (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(4-methoxyphenyl)pyrimidin-4-yl]methoxy}phenyl)propanoic acid

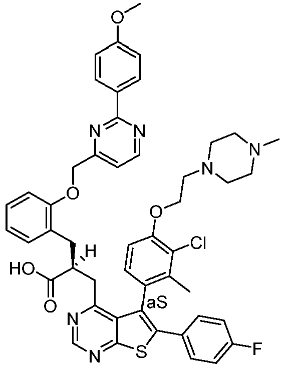

C11 was prepared according to Example 107 in WO 2015/097123.

### Preparation of C12

### (2R)-2-{[(5Sₐ)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[1-(2,2,2-trifluoroethyl)-1H-pyrazol-5-yl]methoxy}phenyl)propanoic acid

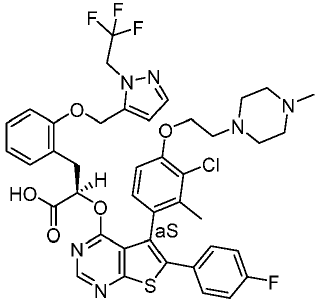

C12 was prepared according to Example 77 in WO 2015/097123.

### Preparation of C13

### (2R)-2-[(5Sₐ)-6-(3-amino-4,5-difluoro-phenyl)-5-[3-chloro-2-methyl-4-[2-(4-methylpiperazin-1-yl)ethoxy]phenyl]thieno[2,3-d]pyrimidin-4-yl]oxy-3-[2-[[2-(2-methoxyphenyl)pyrimidin-4-yl]methoxy]phenyl]propanoic acid

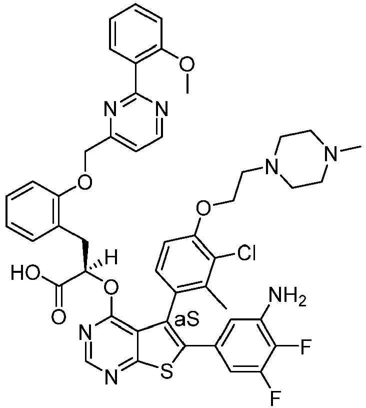

250 mg ethyl (2R)-2-[5-[3-chloro-2-methyl-4-[2-(4-methylpiperazin-1-yl)ethoxy]phenyl]-6-iodo-thieno[2,3-d]pyrimidin-4-yl]oxy-3-[2-[[2-(2-methoxyphenyl)pyrimidin-4-yl]methoxy]phenyl]propanoate (0.27 mmol; obtained according to WO 2015/097123, Preparation 30) and 102 mg 2,3-difluoro-5-(4,4,5,5-tetramethyl-1,3,2-dioxaborolan-2-yl)aniline (0.41 mmol) were dissolved in 1 mL THF, then 3.0 mg PdOAc₂ (0.013 mmol), 5.7 mg tBuXPhos (0.013 mmol), 174 mg Cs₂CO₃ (0.53 mmol) and 0.27 mL water were added and the mixture was stirred at 70°C under N₂ atmosphere for 2 hours. Then it was diluted with brine, and extracted with 2-MeTHF. The combined organic layer was dried over Na₂SO₄, filtered and the filtrate was concentrated under reduced pressure. The crude ester product was purified via flash chromatography using heptane, EtOAc and 0.7 M NH₃ solution in MeOH as eluents. Then it was dissolved in 0.7 mL dioxane, then 60 mg LiOH×H₂O (1.43 mmol) and 0.18 mL water were added. The mixture was stirred at room temperature until complete hydrolysis. Then it was diluted with brine, neutralized with 2 M aqueous HCl solution, and extracted with DCM. The combined organic phase was dried over Na₂SO₄, filtered and the filtrate was concentrated under reduced pressure. The atropoisomers were purified and separated via preparative reverse phase chromatography using 25 mM aqueous NH₄HCO₃ solution and MeCN as eluents. The atropisomer eluting later was isolated as C13. ¹H NMR (500 MHz, DMSO-d₆) δ: 8.89 (d, *J* = 5.2 Hz, 1H), 8.56 (s, 1H), 7.76 (d, *J* = 5.0 Hz, 1H), 7.53 (dd, *J* = 7.6, 1.8 Hz, 1H), 7.45 (m, 1H), 7.36 (d, *J = 8.6* Hz, 1H), 7.20 (d, *J* = 8.7 Hz, 1H), 7.13 (m, 2H), 7.03 (td, *J* = 7.5, 1.0 Hz, 1H), 6.99 (d, *J =* 8.1 Hz, 1H), 6.71 (t, *J* = 7.3 Hz, 1H), 6.62 (m, 1H), 6.21 (d, *J* = 7.5, 1.3 Hz, 1H), 6.12 (m, 1H), 5.73 (s, 2H), 5.46 (dd, *J =* 10.1, 3.1 Hz, 1H), 5.25 (d, *J* = 15.1 Hz, 1H), 5.19 (d, *J* = 15.2 Hz, 1H), 4.22 (m, 2H), 3.75 (s, 3H), 3.35 (m, 1H), 2.73 (m, 2H), 2.65-2.35 (m, 9H), 2.22 (s, 3H), 1.85 (s, 3H). ¹³C NMR (125 MHz, DMSO-d₆) δ: 171.1, 166.0, 165.3, 163.3, 157.8, 157.2, 155.3, 153.7, 152.9, 149.0, 138.9, 137.1, 135.8, 131.0, 130.8, 130.4, 128.7, 128.4, 128.1, 127.8, 125.2, 122.0, 120.4, 120.1, 118.9, 115.6, 112.2, 111.9, 110.6, 103.2, 74.5, 68.9, 67.1, 56.0, 55.7, 53.9, 52.1, 44.7, 32.6, 17.6. HRMS calculated for C₄₇H₄₄N₇O₆SClF₂: 907.2730; found 908.2803 (M+H).

### Preparation of C14

### (2R)-2-{[(5Sa)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(3-hydroxy-2-methoxyphenyl)pyrimidin-4-yl]methoxy}phenyl)propanoic acid

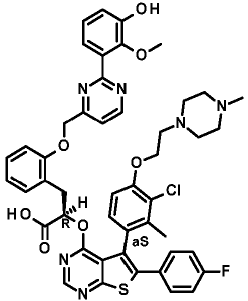

210 mg ethyl (2R)-2-[5-[3-chloro-2-methyl-4-[2-(4-methylpiperazin-1-yl)ethoxy]phenyl]-6-(4-fluorophenyl)thieno[2,3-d]pyrimidin-4-yl]oxy-3-[2-[(2-chloropyrimidin-4-yl)methoxy]phenyl]propanoate (0.25 mmol, WO2016/207216 **Preparation 1**) and 84 mg (3-hydroxy-2-methoxy-phenyl)boronic acid (0.50 mmol) were dissolved in 3.8 mL 1,4-dioxane, then 18 mg Pd(PPh₃)₂Cl₂ (0.025 mmol), 240 mg Cs₂CO₃ (0.75 mmol) and 3.8 mL water were added and the mixture was stirred under N₂ atmosphere at 70°C until complete conversion. Then it was diluted with water, neutralized with 2 M aqueous HCl solution, and extracted with DCM. The combined organic phase was dried over Na₂SO₄, filtered and the filtrate was concentrated under reduced pressure. The crude ester was purified via flash chromatography using heptane, EtOAc and 0.7 M NH₃ solution in MeOH as eluents to obtain a mixture of diastereoisomers. It was in dissolved in 2 mL dioxane, then 245 mg LiOH×H₂O (5.85 mmol) and 1 mL water were added. The mixture was stirred at rt until complete hydrolysis. Then it was neutralized with 2 M aqueous HCl solution, and directly injected on prep-RP-HPLC, using 0.1% aqueous TFA solution and MeCN as eluents. The diastereoisomer eluting later was collected as **C14.** ¹H NMR (500 MHz, DMSO-d₆) δ: 9.53 (brs, 1H), 8.91 (d, 1H), 8.56 (s, 1H), 7.79 (d, 1H), 7.42 (d, 1H), 7.26 (m, 2H), 7.19 (d, 1H), 7.18 (m, 2H), 7.12 (t, 1H), 7.06 (dd, 1H), 6.98 (m, 1H), 6.98 (m, 1H), 6.97 (d, 1H), 6.68 (t, 1H), 6.16 (d, 1H), 5.47 (m, 1H), 5.27/5.20 (d+d, 2H), 4.26/4.19 (m+m, 2H), 3.76 (s, 3H), 3.38/2.42 (dd+dd, 2H), 2.74 (m, 2H), 2.55 (br., 4H), 2.47 (br., 4H), 2.25 (s, 3H), 1.80 (s,3 H). HRMS calculated for C₄₇H₄₄N₆O₇SCIF: 890.2665; found 891.2721 (M+H).

### Preparation of P15

### (11R,20R)-23,26-dichloro-3-(4-fluorophenyl)-14-[[2-(2-methoxyphenyl)pyrimidin-4-yl]methoxy]-24,25-dimethyl-20-[(4-methylpiperazin-1-yl)methyl]-10,18,21-trioxa-4-thia-6,8-diazapentacyclo[20.2.2.12,5.113,17.09,28]octacosa-1(25),2,5(28),6,8,13,15,17(27),22(26),23-decaene-11-carboxylic acid

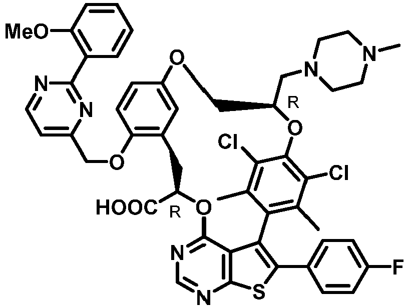

P15 was prepared according to Example 116 in WO 2019/035914.

### Preparation of P16

### (11R,20R)-23,26-dichloro-14-[[2-[4-[[(2S)-1,4-dioxan-2-yl]methoxymethyl]-4-fluoro-cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid

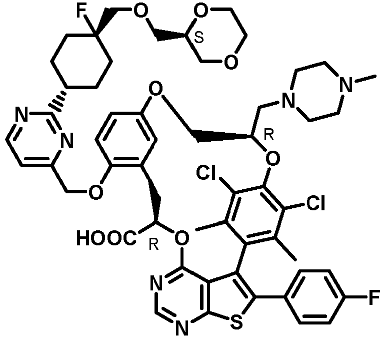

P16 was prepared according to Example 28 in WO 2019/035911.

### Preparation of P17

### (11R,20R)-23,26-dichloro-14-[[2-[4-[[(2S)-1,4-dioxan-2-yl]methoxy]cyclohexyl]pyrimidin-4-yl]methoxy]-3-(4-fluorophenyl)-24,25-dimethyl-20-[(4-methylpiperazin-1-yl)methyl]-10,18,21-trioxa-4-thia-6,8-diazapentacyclo[20.2.2.12,5.113,17.09,28]octacosa-1(24),2,5(28),6,8,13,15,17(27),22,25-decaene-11-carboxylic acid

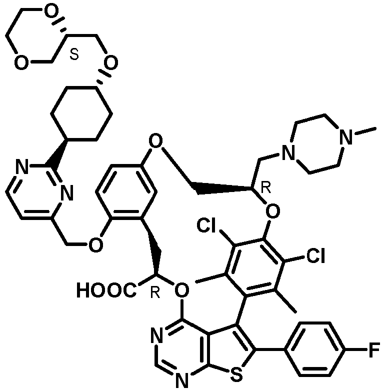

P17 was prepared according to Example 44 in WO 2019/035911.

### Example 4. Synthesis and Characterization of Mcl-1 Inhibitor ADCs

Exemplary antibody-drug conjugates (ADCs) were synthesized using the exemplary methods described in this example. Antibodies used for the preparation of the exemplary ADCs are described in Table 12. The term "CysmAb" refers to cysteine mutations in the heavy chain of the antibody that are used to conjugate linker-payloads to the antibody via maleimide group. In some embodiments, the CysmAb mutations comprising E152C and S375C (numbered according to the EU system). The mutations may also be numbered as E156C and S379C, which correspond to the E152C and S375C EU mutations.

**Table 12. Antibodies used for the synthesis of the exemplified ADC**

| **Antibody Abbreviation** | **Antibody** | **Mutation** | **SEQ ID NO (VL,VH)** | **Allotype** |
|---|---|---|---|---|
| Ab G | MuMy9-6 ch (anti-CD33) | N297Q | SEQ ID NOs 4,3 | G1m3 |
| Ab T | trastuzumab (anti-HER2) | N297S | SEQ ID NOs 10,9 | G1m3 |
| Ab T | trastuzumab (anti-HER2) | N297Q | SEQ ID NOs 10,9 | G1m3 |
| BCMA or Ab B | J6M0 (anti-BCMA) | E156C, S379C (or E152C S375C (EU) (CysmAb)) | SEQ ID NOs 2,1 | G1m3 |
| BCMA Fc silent or Ab B Fc silent | J6M0 (anti-BCMA) | E156C, S379C (or E152C S375C(EU) (CysmAb)) Fc Silent | SEQ ID NOs 2,1 | G1m3 |
| PCAD | NOV169N31Q | E152C S375C EU (CysmAb) | SEQ ID NOs 8, 7 | G1m3 |
| Ab C | Anti-CD46 | E156C S379C (or E152C S375C (EU)) | SEQ ID Nos 91, 90 | G1m3 |

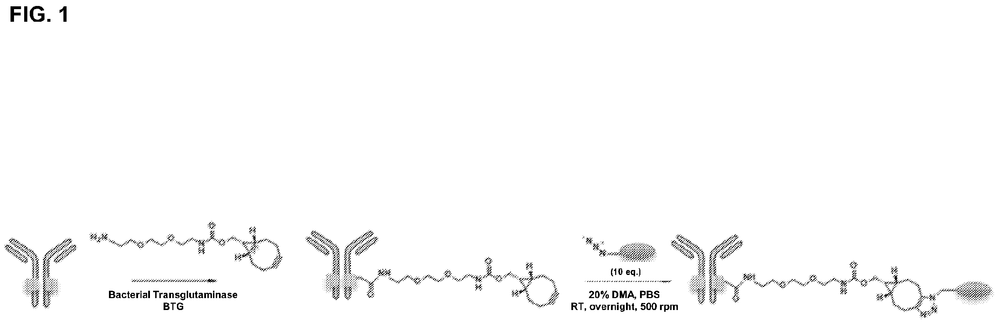

BTG: Antibody (Ab G and Ab T) was endowed with bacterial transglutaminase (BTG) - reactive glutamines specifically functionalized with amine containing cyclooctyne BCN (N-[(1*R*,8*S*,9*s*)-Bicyclo[6.1.0]non-4-yn-9-ylmethyloxycarbonyl]-1,8-diamino-3,6-dioxaoctane, also known as BCN-POE3-NH2, CAS Number: 1263166-93-3). Site-specific antibody conjugation to BCN moiety using bacterial transglutaminase was performed using methods described in Innate Pharma 2013 (presentation at ADC Summit, San Francisco, California, Oct. 15, 2013), WO 2017/059160, and WO 2016/144608,. These modifications allowed for conjugation of the described azide-containing precursors using Method A and Method B, as described below. See also **FIG. 1****.**

Redox: Antibody (anti-CD33, anti-BCMA, anti-PCAD) was incubated with RMP Protein A resin (GE) at a ratio of 10 mg Ab to 1 ml resin in PBS for 15 minutes with mixing in an appropriately sized disposable column. Cysteine HCl was added to a final concentration of 20 mM and incubated with agitation for 30 minutes at room temperature to allow the reactive cysteines to be deblocked. The resin was quickly washed with 50 column volumes PBS on a vacuum manifold. The resin was then resuspended in an equal volume PBS containing 250 nM CuCl₂. Reformation of antibody interchain disulfides was monitored by taking time points. At each time point, 25 µl of resin slurry was removed, 1 µl of 20 mM MC-valcit-MMAE was added, and the tube agitated several times. The resin was centrifuged, supernatant removed, and then eluted with 50 µl Antibody elution buffer (Thermo Fisher Scientific). The resin was pelleted and the supernatant analyzed by reverse phase chromatography using an Agilent PLRP-S 4000A 5 um, 4.6 x 50 mm column (Buffer A is water, 0.1% TFA, Buffer B Acetonitrile, 0.1% TFA, column held at 80°C, Flowrate 1.5 ml/min).
Once determined that the antibody had reformed its interchain disulfide bonds, the resin was washed with 10 column volumes PBS and the resin was resuspended in an equal volume PBS and allowed to drain to settle resin loosely. The re-oxidized antibody was eluted from the protein A resin with Antibody elution buffer and neutralized with 1/10 volume 1 M Tris pH 9.0. The antibody was then buffer exchanged into PBS using PD-10 columns. Protein concentration was adjusted to 5 mg/ml and aliquoted for single use conjugations (see Method C1-C18 below).

**Method A (DAR2):** A propylene glycol (PG, 1.5 ml, 20% of coupling volume) was added to the antibody (Ab) solution (3 mg/ml, 3.3 ml). The mixture was stirred for 30 seconds in a vortex followed by the addition of 4-fold molar excess of linker-warhead payload (5 mM, 533.8 µl in PG/DMA). The reaction was stirred at room temperature overnight at 64 rpm. The solution was centrifuged (14000 G at 4°C) for 20 minutes and then loaded on a HiLoad 26/600 Superdex 200 pg (GE Healthcare, 28989336) SEC chromatography column. The ADC was eluted with 20% PG in PBS (Sigma, P3813, 10PAK) followed by 2 cycles of dialysis (16 and 4 hours) in PBS 1X pH 7.4 (Sigma, P3813, 10PAK). The ADC was concentrated using Vivaspin 20, 50KD, PES (Sartorius Stedim, VS2031), filtered sterilely through a 0.2 µm sterile PES Filter, 25 mm (Whatmann, G896-2502), and stored at 4°C.
Alternatively, a propylene glycol (PG, 673 µl, 20% of coupling volume) was added to the antibody (Ab) solution (1.8 mg/ml, 2.8 ml, 5 mg). The mixture was stirred for 30 seconds in a vortex followed by the addition of 4-fold molar excess of linker-warhead payload (5 mM, 27 µl in PG/DMA). The reaction was stirred at room temperature overnight at 64 rpm. The solution was centrifuged (14000 G at 4°C) for 20 minutes and then loaded on a HiLoad 26/600 Superdex 200 pg (GE Healthcare, 28989336) SEC chromatography column. The ADC was eluted with 20% PG in PBS (Sigma, P3813, 10PAK) followed by 2 cycles of dialysis (16 and 4 hours) in PBS 1X pH 7.4 (Sigma, P3813, 10PAK). The ADC was concentrated using Vivaspin 20, 50KD, PES (Sartorius Stedim, VS2031), filtered sterilely through a 0.2 µm sterile PES Filter, 25 mm (Whatmann, G896-2502), and stored at 4°C.

**Method B (DAR4):** A propylene glycol (PG, 1.5 ml, 20% of coupling volume) was added to the Ab solution (3 mg/ml, 3.3 ml). The mixture was stirred for 30 seconds in a vortex followed by the addition of 10-fold molar excess of linker-warhead payload (5 mM, 533.8 µl in PG/DMA). The reaction was stirred at room temperature overnight at 64 rpm. The solution was then incubated with 10-fold molar excess of DBCO-containing Tentagel resin (0.1-0.2 mmol/g, Iris Biotech, CS-0477.0500) for 6 hours. Next, the solution was centrifuged (14000G at 4°C) for 20 minutes and then loaded on a HiLoad 26/600 Superdex 200 pg (GE Healthcare, 28989336) SEC chromatography column. The ADC was eluted with 20% PG in PBS (Sigma, P3813, 10PAK) followed by 2 cycles of dialysis (16 and 4 hours) in PBS 1X pH 7.4 (Sigma, P3813, 10PAK). The ADC was concentrated using Vivaspin 20, 50KD, PES (Sartorius Stedim, VS2031), filtered sterilely through a 0.2 µm sterile PES Filter, 25 mm (Whatmann, G896-2502), and stored at 4°C.

Alternatively, a propylene glycol (PG, 266 µl, 20% of the coupling volume) was added to the Ab solution (4.5 mg/ml, 1.4 ml, 6.3 mg). The mixture was stirred for 30 seconds in vortex followed by the addition of 10-fold molar excess of linker-warhead payload (5 mM, 84 µl in PG/DMA). The reaction was stirred at room temperature overnight at 64 rpm. This solution was incubated with 10-fold molar excess of DBCO-containing Tentagel resin (0.1-0.2 mmol/g, Iris Biotech, CS-0477.0500) for 6 hours. Then the solution was centrifuged (14000G at 4°C) for 20 minutes and it was loaded on HiLoad 26/600 Superdex 200 pg (GE Healthcare, 28989336) SEC chromatography column. The ADC was eluted with 20% PG in PBS (Sigma Life Science, P3813, 10PAK) followed by 2 cycles of dialysis (16 and 4 hours) in PBS 1X pH 7.4 (Sigma Life Science, P3813, 10PAK). The conjugate was concentrated using Vivaspin 20, 50KD, PES (Sartorius Stedim, VS2031), filtered sterilely through 0.2 µm sterile PES Filter, 25 mm (Whatmann, G896-2502) and stored at 4°C.

**Method C:** The antibody was bound on rmp Protein A resin (GE Healthcare) at a ratio of 10 mg Ab to 1 ml resin in PBS for 30 minutes by mixing in Biorad sized disposable column. To deblock the reactive cysteines, cysteine hydrochloride monohydrate was added to a final concentration of 20 mM. The mixture was agitated for 30 minutes at room temperature followed by washing 5 times the resin with 50 column volumes PBS on a vacuum manifold. The resin was then resuspended in an equal volume PBS containing 250 nM CuCl₂ and incubated for 1 hour and 30 min. The re-oxidized antibody was washed 5 times with 50 column volumes PBS on a vacuum manifold and resuspended in an equal volume PBS. To the mixture were added 10 fold-molar excess of 20mM linker-payload solution and equal volume of DMSO. The reaction was incubated at room temperature for 2 hours. To monitor the conjugation 20µl of resin slurry were removed, centrifuged, supernatant removed, and then eluted with 40 µl Antibody elution buffer (Thermo Fisher Scientific). The supernatant was analyzed by reverse phase chromatography using an Agilent PLRP-S 4000A 5 um, 4.6 x 50 mm column (Buffer A is water, 0.1% TFA, Buffer B Acetonitrile, 0.1% TFA, column held at 80°C, Flowrate 1.5 ml/min). After elimination the excess of linker-payload by washing the resin 5 times with 50 column volumes PBS on a vacuum manifold, the ADC was eluted from protein A with antibody elution buffer and neutralized with 1/10 volume 1 M Tris pH 9.0. All exemplified ADCs (except Ab C - L9-C1, Ab B - L9-P15, Ab B - L26-P1, Ab B - L9-P16, Ab B - L9-P17) were purified on HiLoad 26/600 Superdex 200 pg (GE Healthcare, 28989336) SEC chromatography column, eluted with 20% PG in PBS (Sigma Life Science, P3813, 10PAK). The ADCs were buffer exchange by dialysis in PBS 1X pH 7.4 (Sigma Life Science, P3813, 10PAK), concentrated using Vivaspin 20, 50KD, PES (Sartorius Stedim, VS2031), filtered sterilely through 0.2µm sterile PES Filter, 25mm (Whatmann, G896-2502) and stored at 4°C. For ADCs Ab C - L9-C1, Ab B - L9-P15, Ab B - L26-P1, Ab B - L9-P16 and Ab B - L9-P17, they were only buffer exchanged by dialysis in PBS 1X pH 7.4 (Sigma Life Science, P3813, 10PAK), concentrated using Vivaspin 20, 50KD, PES (Sartorius Stedim, VS2031), filtered sterilely through 0.2µm sterile PES Filter, 25mm (Whatmann, G896-2502) and stored at 4°C.

Alternatively, Antibody was incubated with RMP Protein A resin (GE) at a ratio of 10 mg Ab to 1 ml resin in PBS for 15 minutes with mixing in an appropriately sized disposable column. Cysteine HCl was added to a final concentration of 20 mM and incubated with agitation for 30 min at room temperature to allow the reactive cysteines to be deblocked. The resin was quickly washed with 50 column volumes PBS on a vacuum manifold. The resin was then resuspeneded in an equal volume PBS containing 250 nM CuCl₂. Reformation of antibody interchain disulfides was monitored by taking time points. At each time point, 25 µL of resin slurry was removed, 1 µL of 20 mM MC-valcit-MMAE was added, and the tube flicked several times. The resin was spun down, supernatant removed, and then eluted with 50 µL Antibody elution buffer (Thermo). The resin was pelleted and the supernatant analyzed by reverse phase chromatography using an Agilent PLRP-S 4000A 5um, 4.6x50mm column (Buffer A is water, 0.1% TFA, Buffer B Acetonitrile, 0.1% TFA, column held at 80 C, Flowrate 1.5 ml/min). Once it was determined that the antibody has reformed its interchain disulfide bonds, the resin was washed with 10 column volumes PBS and the resin was resuspended in an equal volume PBS and 8-12 equivalents of linker-payload in DMSO was added, with a final concentration of 10% DMSO in reaction and then incubated at room temperature for 3 hours. The resin was then washed with 50 column volumes PBS. The ADC was eluted from the protein A resin with Antibody elution buffer. The ADC was then buffer exchanged into PBS or other suitable buffer and preparative size exclusion chromatography to remove aggregates was performed (S200 Increase; GE), if required to remove aggregates. The following analyses were performed - analytical SEC to determine percent monomer, mass spectroscopy to determine DAR, LAL test to determine endotoxin load and protein concentration was determined by A280 utilizing extinction coefficient and molecular weight of antibody.

**Method C1 - (CD33-L1-P1):** To a solution of CD33 antibody (1.0 mg, 154 µl of a 6.5 mg/ml solution in 1X PBS buffer solution, 0.0067 µmoles, 1.0 equiv.) was added L1-P1 (2.53 µl of a 20 mM solution in DMSO, 0.051 µmoles, 7.5 equiv.). The resulting mixture was shaken at 400 rpm at ambient temperature for 1 hour, at which time the mixture was purified by ultracentrifugation (4 ml Amicon 10 kD cutoff membrane filter, diluting sample to 4 ml total volume with PBS buffer followed by centrifugation for 10 minutes at 7500 x g, repeated 6 times). After dilution to 5.0 mg/ml, **CD33-L1-P1** was obtained (0.842 mg, 0.0053 µmoles, 79%). The following analyses were performed: analytical size-exclusion chromatography (SEC) to determine percent monomer, mass spectroscopy (MS) to determine DAR, and protein concentration determined by A280 utilizing extinction coefficient and molecular weight of antibody. HRMS data (protein method) indicated a mass of 157884, with a DAR of 3.9 as calculated by comparing MS intensities of peaks for DAR3 and DAR4 species. SEC indicated </= 1% aggregation, as determined by comparison of the area of the high-molecular-weight peak absorbance at 210 and 280 nm with the area of the peak absorbance for monomeric ADC.

**Method C2 - (PCAD-L1-P1):** Following **Method C1** with PCAD antibody (2.0 mg, 357 µl of a 5.6 mg/ml solution, 0.013 µmoles, 1.0 equiv.) and **L1-P1** (5.04 µl of a 20 mM solution in DMSO, 0.101 µmoles, 7.5 equiv.), **PCAD-L1-P1** was obtained (1.73 mg, 0.0109 µmoles, 81%). HRMS data (protein method) indicated a mass of 158268, with a DAR of 3.9. SEC indicated </=1% aggregation.

**Method C3 - (PCAD-L10-P1):** Following **Method C1** with PCAD antibody (2.0 mg, 357 µl of a 5.6 mg/ml solution, 0.013 µmoles, 1.0 equiv.) and **L10-P1** (5.04 µl of a 20 mM solution in DMSO, 0.101 µmoles, 7.5 equiv.), **PCAD-L10-P1** was obtained (1.97 mg, 0.012 µmoles, 92%). HRMS data (protein method) indicated a mass of 158731, with a DAR of 3.9. SEC indicated </=1% aggregation.

**Method C4 - (CD33-L10-P1):** Following **Method C1** with CD33 antibody (1.1 mg, 169 µl of a 6.5 mg/ml solution, 0.0074 µmoles, 1.0 equiv.) and **L10-P1** (2.53 µl of a 20 mM solution in DMSO, 0.051 µmoles, 6.8 equiv.), **CD33-L10-P1** was obtained (1.08 mg, 0.0068 µmoles, 92%). HRMS data (protein method) indicated a mass of 158346, with a DAR of 3.9. SEC indicated </=1% aggregation.

**Method C5 - (PCAD-L4-P1):** Following **Method C1** with PCAD antibody (2.0 mg, 357 µl of a 5.6 mg/ml solution, 0.013 µmoles, 1.0 equiv.) and **L4-P1** (5.38 µl of a 20 mM solution in DMSO, 0.108 µmoles, 8.0 equiv.), **PCAD-L4-P1** was obtained (2.1 mg, 0.013 µmoles, 99%). HRMS data (protein method) indicated a mass of 156533, with a DAR of 4.2. SEC indicated 1% aggregation.

**Method C6 - (PCAD-L3-P1):** Following **Method C1** with PCAD antibody (1.0 mg, 179 µl of a 5.6 mg/ml solution, 0.0067 µmoles, 1.0 equiv.) and **L3-P1** (2.69 µl of a 20 mM solution in DMSO, 0.054 µmoles, 8.0 equiv.), **PCAD-L3-P1** was obtained (1.05 mg, 0.0067 µmoles, 99%). HRMS data (protein method) indicated a mass of 156763, with a DAR of 3.9. SEC indicated 1% aggregation.

**Method C7 - (CD33-L2-P1):** Following **Method C1** with CD33 antibody (2.0 mg, 351 µl of a 5.7 mg/ml solution, 0.013 µmoles, 1.0 equiv.) and **L2-P1** (5.4 µl of a 20 mM solution in DMSO, 0.108 µmoles, 8.0 equiv.), **CD33-L2-P1** was obtained (2.1 mg, 0.013 µmoles, 99%). HRMS data (protein method) indicated a mass of 155821, with a DAR of 3.9. SEC indicated </=1% aggregation.

**Method C8 - (PCAD-L9-P1):** Following **Method C1** with PCAD antibody (2.0 mg, 357 µl of a 5.6 mg/ml solution, 0.013 µmoles, 1.0 equiv.) and **L9-P1** (6.73 µl of a 20 mM solution in DMSO, 0.13 µmoles, 10.0 equiv.), **PCAD-L9-P1** was obtained (1.90 mg, 0.012 µmoles, 91%). HRMS data (protein method) indicated a mass of 154392, with a DAR of 3.9. SEC indicated </= 1% aggregation.

**Method C9 - (CD33-L9-P1):** Following Method C1 with CD33 antibody (1.0 mg, 154 µl of a 6.5 mg/ml solution, 0.0067 µmoles, 1.0 equiv.) and **L9-P1** (3.37 µl of a 20 mM solution in DMSO, 0.067 µmoles, 10.0 equiv.), **CD33-L9-P1** was obtained (0.84 mg, 0.0055 µmoles, 81%). HRMS data (protein method) indicated a mass of 154009, with a DAR of 3.9. SEC indicated 1% aggregation.

**Method C10 - (CD33-L8-P1):** Following **Method C1** with CD33 antibody (1.0 mg, 154 µl of a 6.5 mg/ml solution, 0.0067 µmoles, 1.0 equiv.) and **L8-P1** (3.43 µl of a 30 mg/ml solution in DMSO, 0.054 µmoles, 8.0 equiv.), **CD33-L8-P1** was obtained (0.91 mg, 0.0058 µmoles, 87%). HRMS data (protein method) indicated a mass of 1555921, with a DAR of 3.9. SEC indicated 1% aggregation.

**Method C11 - (PCAD-L8-P1):** Following **Method C1** with PCAD antibody (2.5 mg, 472 µl of a 5.6 mg/ml solution, 0.017 µmoles, 1.0 equiv.) and **L8-P1** (10.7 µl of a 30 mg/ml solution in DMSO, 0.17 µmoles, 10.0 equiv.), **PCAD-L8-P1** was obtained (1.57 mg, 0.010 µmoles, 60%). HRMS data (protein method) indicated a mass of 156307, with a DAR of 3.9. SEC indicated </= 1% aggregation.

**Method C12 - (CD33-L7-P1):** Following **Method C1** with CD33 antibody (1.0 mg, 154 µl of a 6.5 mg/ml solution, 0.0067 µmoles, 1.0 equiv.) and **L7-P1** (3.54 µl of a 30 mg/ml solution in DMSO, 0.054 µmoles, 8.0 equiv.), **CD33-L7-P1** was obtained (0.56 mg, 0.0036 µmoles, 53%). HRMS data (protein method) indicated a mass of 156151, with a DAR of 3.9. SEC indicated 1% aggregation.

**Method C13 - (PCAD-L7-P1):** Following **Method C1** with PCAD antibody (2.5 mg, 472 µl of a 5.6 mg/ml solution, 0.017 µmoles, 1.0 equiv.) and **L7-P1** (11.03 µl of a 30 mg/ml solution in DMSO, 0.17 µmoles, 10.0 equiv.), **PCAD-L7-P1** was obtained (2.60 mg, 0.017 µmoles, 99%). HRMS data (protein method) indicated a mass of 156539, with a DAR of 3.9. SEC indicated </= 1% aggregation.

**Method C14 - (BCMA-L5-P1):** Following **Method C1** with BCMA antibody (2.0 mg, 250 µl of an 8.0 mg/ml solution, 0.013 µmoles, 1.0 equiv.) and **L5-P1** (5.38 µl of a 20 mM solution in DMSO, 0.108 µmoles, 8 equiv.), **BCMA-L5-P1** was obtained (2.1 mg, 0.013 µmoles, 99%). HRMS data (protein method) indicated a mass of 159197 with a DAR of 3.9. SEC indicated 1% aggregation.

**Method C15 - (CD33-L12-P2):** Following **Method C1** with CD33 antibody (2.0 mg, 345 µl of a 5.8 mg/ml solution, 0.013 µmoles, 1.0 equiv.) and **L12-P2** (4.05 µl of a 20 mM solution in DMSO, 0.081 µmoles, 6.0 equiv.), **CD33-L12-P2** was obtained (2.1 mg, 0.013 µmoles, 99%). HRMS data (protein method) indicated a mass of 157331, with a DAR of 3.9. SEC indicated 1% aggregation.

**Method C16** - **(BCMA-L12-P2):** Following **Method C1** with BCMA antibody (2.0 mg, 250 µl of an 8.0 mg/ml solution, 0.013 µmoles, 1.0 equiv.) and **L12-P2** (3.03 µl of a 20 mM solution in DMSO, 0.061 µmoles, 4.5 equiv.), **BCMA-L12-P2** was obtained (2.1 mg, 0.013 µmoles, 99%). HRMS data (protein method) indicated a mass of 157792, with a DAR of 3.8. SEC indicated 1% aggregation.

**Method C17** - **(CD33-L4-P2):** Following **Method C1** with CD33 antibody (2.0 mg, 345 µl of a 5.8 mg/ml solution, 0.013 µmoles, 1.0 equiv.) and **L4-P2** (3.03 µl of a 20 mM solution in DMSO, 0.061 µmoles, 4.5 equiv.), **CD33-L4-P2** was obtained (2.1 mg, 0.013 µmoles, 99%). HRMS data (protein method) indicated a mass of 156700, with a DAR of 3.9. SEC indicated 1% aggregation.

**Method C18** - **(BCMA-L4-P2):** Following **Method C1** with BCMA antibody (2.0 mg, 250 µl of an 8.0 mg/ml solution, 0.013 µmoles, 1.0 equiv.) and **L4-P2** (3.03 µl of a 20 mM solution in DMSO, 0.061 µmoles, 4.5 equiv.), **BCMA-L4-P2** was obtained (2.1 mg, 0.013 µmoles, 99%). HRMS data (protein method) indicated a mass of 157164, with a DAR of 3.9. SEC indicated 1% aggregation.

Other ADCs described in the Examples, such as CD38 Mcl1-inhibitor ADCs, CD48 Mcl1-inhibitor ADCs, and CD79b Mcl1-inhibitor ADCs (e.g., those described in Tables 24-26), were synthesized via methods similar to those described above.

### Analytical Methods

### Liquid Chromatography-Mass Spectrometry (LC/MS) I

**General Methodology:** Drug-to-antibody ratio (DAR) of exemplary ADCs was determined by liquid chromatography-mass spectrometry (LC/MS) according to one of the following methods (i.e., LC-I, LCII and LC-III). For all LC methods, mobile phase A was purified MS grade water (Biosolve, Dieuze, France, 00232141B1BS), mobile phase B was MS grade acetonitrile (Biosolve, Dieuze, France, 0001204101BS), and mobile phase D was purified MS grade water supplemented with 1 % of formic acid (FA) (Honeywell/Fluka, Bucharest, Romania, 56302). Mobile phase D was fixed at 10 % in order to maintain a 0.1% FA mobile phase composition and column temperature was set at 80°C. A general MS method was optimized for all ADCs synthesized, except for one ADC where native MS was used in order to determine average DAR (see MS-I and Table 22 below).

**LC-I:** ADC was loaded onto a MassPREP Micro desalting column (2.1 x 5.0 mm, Waters, Saint- Quentin-en-Yvelines, France, 186004032). For intact mass analysis, a desalting step was performed for 0.5 min at 5% mobile phase B with a flow rate of 0.5 ml/min. Elution step was performed with a gradient from 0.51 min at 5% B to 2.0 min at 90 % B with a flow rate of 0.2 ml/min. Two wash steps were set from 2.1 min to 2.7 min and from 2.8 min to 3.4 min at 5% B to 90% B with a flow rate of 0.5 ml/min. Finally, a conditioning step was used at 3.5 min for 0.5 min at 5 % B (0.5 ml/min).

For ADC analysis in reduced conditions, a desalting step was performed for 0.5 min at 5% B with a flow rate of 0.2 ml/min. Then, the elution step started with a gradient from 0.51 min at 10% B to 7.61 min at 80 % B with a flow rate of 0.2 ml/min. Two washing steps were set from 8.1 min to 8.6 min and from 8.7 min to 9.2 min from 5% B to 90% B (0.5 ml/min). Finally, a conditioning step was performed at 9.3 min for 0.5 min at 5 % B with a flow rate of 0.5 ml/min.

**LC-II:** ADC was loaded onto a MabPac RP column (2.1 x 100 mm, 4 µm, Thermo Fisher Scientific, Rockford, IL, 088647). For analysis in both intact and reduced conditions, a desalting step was performed for 1.4 min at 20% of B with a flow rate of 0.4 mL/min. Then, the elution step was performed with a gradient from 1.5 min at 20% B to 11.5 min at 70 % B with a flow rate of 0.3 ml/min. A wash step was set from 11.75 min to 13.75 min at 90% B with a flow rate of 0.5 ml/min. Finally, a conditioning step was used at 14.0 min for 1.0 min at 20 % B with a flow rate of 0.4 ml/min.

**LC-III:** ADC was loaded onto a Bioresolve RP mAb Polyphenyl, 450A, 2.7 µm, 2.1 x 150 mm (Waters, Saint- Quentin-en-Yvelines, France, 186008946). For analysis in both intact and reduced conditions, a desalting step was performed for 1.4 min at 20% of B with a flow rate of 0.4 ml/min. Elution step was performed with a gradient from 1.5 min at 20% B to 11.5 min at 70 % B with a flow rate of 0.3 ml/min. A wash step was set from 11.75 min to 13.75 min at 90% B with a flow rate of 0.5 ml/min. Finally, a conditioning step was used at 14.0 min for 1.0 min at 20 % B with a flow rate of 0.4 ml/min.

**MS-I:** ADC was buffer exchanged with a MS compatible buffer and infused directly into a hybrid Q-TOF MS instrument (Synapt G2-S HDMS, Waters, Manchester, UK) equipped with an automated chip-based nanoESI source (Triversa Nanomate, Advion Biosciences, Ithaca, NY, USA) operating in positive ion mode. Instrumental MS parameters were tuned to preserve native 3D structure in the gas phase and ensure efficient ion desolvation and transmission.

LC-MS analysis was performed using a Waters UPLC H-Class Bio chromatography system hyphenated with a Xevo G2 XS Q-TOF ESI mass spectrometer (Waters, Manchester, UK). The ADC was either analyzed in intact condition (no preliminary treatment) or following reduction with 5 mM (final concentration) of dithiothreitol DTT (Thermo Scientific, Rockford, IL, 20291). Subsequently, treated ADC was analyzed using either the aforementioned LC-I, LC-II, or LC-III (Table 22). Electrospray-ionization time-of-flight mass spectra of the analytes were acquired using MassLynx^{™} acquisition software (Waters, Manchester, UK). Concerning the single native MS experiment performed, ADC was analyzed using MS-I methodology in order to determine average DAR. Then, the extracted intensity vs. m/z spectrum was deconvoluted using Maximum Entropy (MaxEnt) method of MassLynx^{™} software in order to determine the mass of each intact antibody species or each reduced antibody fragment depending on the treatment used. Finally, DAR was determined from the deconvoluted spectra or UV chromatogram by summing the integrated MS (total ion current) or UV (280 nm) peak area of unconjugated and conjugated given species (mAb or associated fragment), weighted by multiplying each area by the number of drug attached. The summed, weighted areas were divided by the sum of total area and the results produced a final average DAR value for the full ADC.

### Liquid Chromatography-Mass Spectrometry (LC/MS) II

LC/MS data were acquired using an instrument with the following parameters (Table 13):

**Table 13. Parameters**

| | |
|---|---|
| Pump | Waters AcQuity UPLC Binary Solvent Manager |
| Sample Manager | Waters AcQuity UPLC Sample Manager |
| Column Compartment | Waters AcQuity UPLC Column Manager |
| Detector | Waters AcQuity UPLC PDA |
| ELSD | Shimadzu ELSD-LTII |
| Mass Spec | Waters SQD |
| Columns | AcQuity UPLC BEH C18 1.7µm 2.1x50mm |
| Eluent A1 | 0.1% Formic Acid in Water |
| Eluent B1 | 0.1% Formic Acid in Acetonitrile |
| Eluent A2 | 5mM Ammonium Hydroxide in Water |
| Eluent B2 | 5mM Ammonium Hydroxide in Acetonitrile |

The methods used to generate LC/MS data were as follows (Tables 14-16):

**Table 14. 2 minute acidic method**

| 2 min acidic method | | | |
|---|---|---|---|
| Eluent A1 | 0.1% Formic Acid in Water | | |
| Eluent B1 | 0.1% Formic Acid in Acetonitrile | | |
| Flow | 1.0 mL/min | | |
| Stop Time | 3.00 min | | |
| pH | 2.6 | | |

| Gradient | Time | %A (Eluent A1) | %B (Eluent B1) |
|---|---|---|---|
| | 0.00 | 95 | 5 |
| | 0.20 | 95 | 5 |
| | 2.00 | 5 | 95 |
| | 2.50 | 5 | 95 |
| | 2.60 | 95 | 5 |
| | 3.00 | 95 | 5 |
| Column | AcQuity UPLC BEH C18 1.7µm 2.1x50mm | | |
| Column Temperature | 50°C | | |
| TAC | 210-400 nm | | |
| Mass Range | 120-1500 Da | | |
| Scan Time | 0.3 sec | | |

**Table 15. 2 minute basic method**

| 2 min basic method | | | |
|---|---|---|---|
| Eluent A2 | 5mM Ammonium Hydroxide in Water | | |
| Eluent B2 | 5mM Ammonium Hydroxide in Acetonitrile | | |
| Flow | 1.0 mL/min | | |
| Stop Time | 3.00 min | | |
| pH | 10.2 | | |

| Gradient | Time | %A (Eluent A2) | %B (Eluent B2) |
|---|---|---|---|
| | 0.00 | 95 | 5 |
| | 0.20 | 95 | 5 |
| | 2.00 | 5 | 95 |
| | 2.50 | 5 | 95 |
| | 2.60 | 95 | 5 |
| | 3.00 | 95 | 5 |
| Column | AcQuity UPLC BEH C18 1.7µm 2.1x50mm | | |
| Column Temperature | 50°C | | |
| TAC | 210-400 nm | | |
| Mass Range | 120-1500 Da | | |
| Scan Time | 0.3 sec | | |

**Table 16. 5 minute acidic method**

| 5 min acidic method | | | |
|---|---|---|---|
| Flow | 1.0 mL/min | | |
| Stop Time | 5.20 min | | |
| pH | 2.6 | | |

| Gradient | Time | %A (Eluent A1) | %B (Eluent B1) |
|---|---|---|---|
| | 0.00 | 98 | 2 |
| | 4.40 | 2 | 98 |
| | 5.15 | 2 | 98 |
| | 5.19 | 98 | 2 |
| Column | AcQuity UPLC BEH C18 1.7µm 2.1x50mm | | |
| Column Temperature | 50°C | | |
| TAC | 210-400 nm | | |
| Mass Range | 120-1500 Da | | |
| Scan Time | 0.3 sec | | |

### High Resolution Mass Spectrometry (HRMS)

HRMS data were acquired using an instrument with the following parameters (Table 17):

**Table 17. Parameters**

| | |
|---|---|
| Pump | Waters AcQuity UPLC Binary Solvent Manager |
| Sample Manager | Waters AcQuity UPLC Sample Manager |
| Column Compartment | Waters AcQuity UPLC Column Manager |
| Detector | Waters AcQuity UPLC PDA |
| ELSD | n/a |
| Mass Spec | Waters Xevo G2 Qtof |
| | AcQuity UPLC PrST C4 300Å 1.7µm 2.1x100mm |
| Columns | AcQuity UPLC CSH C18 1.7 um 2.1x50mm |
| | ProSwift RP-3U 4.6x50mm SS |
| Eluent A1 | 0.1% Formic Acid in Water |
| Eluent B1 | 0.1% Formic Acid in Acetonitrile |
| Eluent A2 | 0.05% Trifluoroacetic Acid in Water |
| Eluent B2 | 0.05% Trifluoroacetic Acid in Acetonitrile |

The methods used to generate HRMS data for linker/payloads and synthetic intermediates were as follows (Tables 18 and 19):

**Table 18. 5 minute acidic method**

| 5 min acidic method: | | | |
|---|---|---|---|
| Flow | 1.0 mL/min | | |
| Stop Time | 5.2 min | | |
| pH | 2.6 | | |

| Gradient | Time | %A (Eluent A2) | %B (Eluent B2) |
|---|---|---|---|
| | 0.00 | 98 | 2 |
| | 4.40 | 2 | 98 |
| | 5.15 | 2 | 98 |
| | 5.19 | 98 | 2 |
| Column | AcQuity UPLC BEH C18 1.7µm 2.1x50mm | | |
| Column | 50°C | | |
| TAC | 210-400 nm | | |
| Mass Range | 300-4000 Da | | |
| Processing Range | n/a | | |
| Scan Time | 0.5 sec | | |

**Table 19. 2 minute acidic method**

| | | | |
|---|---|---|---|
| 2 min acidic method: | 1.0 mL/min | | |
| Flow | | | |
| Stop Time | 2.20 min | | |
| pH | 2.6 | | |

| Gradient | Time | %A (Eluent A2) | %B (Eluent B2) |
|---|---|---|---|
| | 0.00 | 98 | 2 |
| | 0.06 | 98 | 2 |
| | 1.76 | 2 | 98 |
| | 2.00 | 2 | 98 |
| | 2.16 | 98 | 2 |
| Column | AcQuity UPLC CSH C18 1.7µm 2.1x50mm | | |
| Column Temperature | 50°C | | |
| UV | 210-400 nm | | |
| Mass Range | 100-2050 Da | | |
| Scan Time | 0.2 sec | | |

The method used to generate HRMS data for the ADCs was as follows (Table 20):

**Table 20. Protein method**

| Protein method: | | | |
|---|---|---|---|
| Flow | 1.0 mL/min | | |
| Stop Time | 3.30 min | | |
| pH | 2.6 | | |

| Gradient | Time | %A (Eluent) | %B (Eluent) |
|---|---|---|---|
| | 0.00 | 98 | 2 |
| | 0.70 | 98 | 2 |
| | 2.00 | 2 | 98 |
| | 2.10 | 2 | 98 |
| | 2.30 | 98 | 2 |
| | 3.30 | 98 | 2 |
| Column | ProSwift RP-3U 4.6x50mm SS | | |
| Column | 50°C | | |
| TAC | 210-400 nm | | |
| Mass Range | 600-3900 Da | | |
| Processing Range | 14000-170000 Da | | |
| Scan Time | 1.5 sec | | |

### Size Exclusion Chromatography (SEC) I

Size exclusion chromatography (SEC) was performed to determine the quality of the ADCs and aggregation percentage (%) after purification. The analysis was performed on analytical column Superdex 200 Increase 5/150 GL (GE Healthcare, 28990945) in isocratic conditions 100% PBS pH 7.4 (Sigma, P3813, 10PAK), flow 0.45 ml/min for 12 minutes. The % aggregate fraction of the ADC sample was quantified based on the peak area absorbance at 280 nm. Calculation was based on the ratio between the high molecular weight eluent at 280 nm divided by the sum of peak area absorbance at the same wavelength of the high molecular weight and monomeric eluents multiplied by 100%.

### Size Exclusion Chromatography (SEC) II

SEC data were acquired using an instrument with the following parameters (Table 21) and a run length of 12 minutes:

**Table 21. Parameters**

| | |
|---|---|
| Pump | Waters bioAcQuity UPLC Quaternary Solvent Manager |
| Sample Manager | Waters bioAcQuity UPLC Sample Manager FTN |
| Column Compartment | Waters AcQuity UPLC 30cm Column Heater |
| Detector | Waters AcQuity UPLC PDA |
| ELSD | n/a |
| Mass Spec | n/a |
| Columns | Superdex 200 Increase 5/150 GL |
| Eluent A1 | 1X PBS (Phosphate Buffered Saline) + 0.1M NaCl + 5% Isopropanol |
| Eluent B1 | n/a |

### Results

Some of the conjugations were performed on antibody range between 2-20 mg. All exemplified ADC were characterized by analytical size exclusion chromatography Superdex 200 Increase 5/150 GL (GE Healthcare, 28990945) to determine monomer percentage and LC-MS for DAR determination. Characterization of exemplary ADCs is summarized in Table 22 (coupling and LC/MS method, aggregation status, and DAR). The average DAR values were determined using the above LC/MS methods (LC/MS I) and percentage aggregation was determined using the above SEC methods (SEC I).

**Table 22. ADC analytical characterization and conjugation methodology**

| **ADC** | **Conjugation Method** | **LC-MS Method I** | **DAR (by LC-MS I)** | **% Agg. (by SEC I)** |
|---|---|---|---|---|
| Ab G - L23-C3 | B | LC-I | 4 | 1.3 |
| Ab G - L24-C1 | B | LC-I | 3.6 | 1 |
| Ab G - L13-C4 | B | MS-I | 3.8 | 1 |
| Ab G - L14-C3 | B | LC-II | 4 | 0.9 |
| Ab G - L15-C5 | B | LC-I | 3.7 | 0.8 |
| Ab G - L16-C3 | B | LC-I | 4 | 0.5 |
| Ab G - L17-C3 | B | LC-I | 4 | 0.6 |
| Ab G - L18-C3 | B | LC-II | 4 | 1 |
| Ab G - L19-C3 | B | LC-II | 4 | 0.9 |
| Ab G - L20-C6 | B | LC-III | 4 | 1.7 |
| Ab G - L24-C6 | B | LC-III | 4 | 0.5 |
| Ab G - L24-C7 | B | LC-III | 4 | 1 |
| Ab T - L24-C1 | A | LC-I | 2 | 0.5 |
| Ab T - L24-C1 | B | LC-I | 4 | 0.5 |
| Ab T - L24-C6 | B | LC-III | 4 | 1.8 |
| Ab T - L24-C7 | B | LC-III | 4 | 0.9 |
| Ab B - L21-C1 | C | LC-III | 3.7 | 1.7 |
| Ab B - L9-C1 | C | LC-III | 3.4 | 0.9 |
| Ab B - L9-C8 | C | LC-III | 3.6 | 2 |
| Ab B Fc silent - L22-C1 | C | LC-III | 4.1 | 0.5 |
| Ab B - L9-C10 | C | LC-III | 3.4 | 0.2 |
| Ab B - L9-C9 | C | LC-III | 4.2 | 0.9 |
| Ab B - L9-C11 | C | LC-III | 3.3 | 0.2 |
| Ab B - L9-C12 | C | LC-III | 3.3 | 0.2 |
| Ab B - L9-C13 | C | LC-III | 3.3 | <1 |
| Ab G - L29-C3 | B | LC-II | 4 | 0.2 |
| Ab B Fc silent - L27-P1 | C | LC-III | 4.1 | 0.2 |
| Ab B Fc silent - L28-P1 | C | LC-III | 4.0 | 1.1 |
| Ab B - L26-P1 | C | LC-III | 3.5 | 0.4 |
| Ab B - L9-C14 | C | LC-III | 5.3 | 0.2 |
| Ab C - L9-C1 | C | LC-III | 3.1 | 4.4 |
| Ab B - L9-P15 | C | LC-III | 3.4 | 1 |
| Ab B - L25-P1 | C | LC-III | 3.7 | 3.1 |
| Ab B - L9-P16 | C | LC-III | 3.4 | 0.2 |
| Ab B - L9-P17 | C | LC-III | 3.5 | 0.2 |

### Example 5: In Vitro Assessment I

### Inhibition of Mcl-1 by Fluorescence Polarization (FP)

The relative binding potency of each compound was determined via Fluorescence Polarization (FP). The method utilized a fluorescein-labelled ligand (Fluorescein-βAla-Ahx-A-REIGAQLRRMADDLNAQY-OH; MW 2,765) which binds to Mcl-1 protein (UniProt Reference Sequence: Q07820; SEQ ID NO:71), leading to an increased anisotropy measured in milli-polarization (mP) units using a reader. The addition of a compound which binds competitively to the same site as the ligand results in a greater proportion of unbound ligand in the system, as indicated by a decrease in mP units.

An 11-point serial dilution of each compound was prepared in DMSO and 2 µl transferred into flat bottomed, low binding, 384-well plate (final DMSO concentration 5 %). 38 µl of buffer (10 mM 4-(2-hydroxyethyl)-1-piperazineethanesulfonic acid [HEPES], 150 mM NaCl, 0.05 % Tween 20, pH 7.4), containing the fluorescein-labelled ligand (final concentration 1 nM) and Mcl-1 protein (final concentration 5 nM) was then added.

Assay plates were incubated ~2 hours at room temperature before FP was measured on a Biomek Synergy2 reader (Ex. 528 nm, Em. 640 nm, Cut off 510 nm) and mP units calculated. The binding of increasing doses of test compound was expressed as a percentage reduction in mP compared to a window established between '5 % DMSO only' and '100 % inhibition' controls. 11-point dose response curves were plotted with XL-Fit software using a 4-Parameter Logistic Model (Sigmoidal Dose-Response Model) and the inhibitory concentrations that gave a 50 % reduction in mP (IC₅₀) were determined. The results are presented in Table 23 below. The results suggest that the tested compounds inhibit interaction between the Mcl-1 protein and the fluorescein-labelled peptide.

### In Vitro Cytotoxicity

Cytotoxicity studies were carried out on the NCI-H929 multiple myeloma cell line. The cells were distributed onto microplates and exposed to the test compounds for 48 hours. The cell viability was then quantified by a colorimetric assay, the Microculture Tetrazolium Assay (Carmichael et al. (1987) Cancer Res. 47:936-42).

The results are expressed in IC₅₀ (the concentration of compound that inhibits cell viability by 50 %) and are presented in Table 23 below. The results show that the tested compounds are cytotoxic in NCI-H929 cells.

**Table 23. IC₅₀ of Mcl-1 inhibition and cytotoxicity in NCI-H929 cells**

| Payload | IC₅₀ (M) Mcl-1 FP | IC₅₀ (M) MTT H929 | Payload | IC₅₀ (M) Mcl-1 FP | IC₅₀ (M) MTT H929 |
|---|---|---|---|---|---|
| **C1** | 2.8E-09 | 3.0E-09 | **C8** | 3.0E-09 | 5.74E-07 |
| **C2** | 2.6E-09 | 8.51E-06 | **C9** | 2.5E-09 | 2.27E-06 |
| **C3** | 9.48E-10 | 4.2E-08 | **C10** | 3.4E-09 | 1.35E-08 |
| **C4** | 2.6E-09 | 3.21E-06 | **C11** | 5.3E-09 | 7.0E-09 |
| **C5** | 4.05E-09 | 4.08E-09 | **C12** | 5.9E-09 | 4.2E-08 |
| **C6** | 1.18E-09 | 3.0E-09 | **C13** | 2.2E-09 | 3.76E-09 |
| **C7** | 2.6E-09 | 1.88E-09 | **C14** | 8.4E-10 | 2.61E-09 |

### Example 6: In Vitro Assessment II

Mcl-1 ADCs were tested on nine endogenous cancer cell lines and one isogenic cell line engineered to overexpress a target of interest. Six targets were assessed: HER2, BCMA, CD38, CD48, CD79b, and CD33.

### Cell lines

- **HER2:** HCC1954 (ATCC No. CRL-2338 cultured in RPMI-1640 + 10% FBS) and HCC2218 (ATCC No. CRL-2343 cultured in RPMI-1640 + 10% FBS).
- **BCMA:** NCI-H929 (ATCC No. CRL-9068 cultured in RPMI-1640 + 10% FBS + 0.05 mM 2-mercaptoethanol).
- **CD38:** A4-Fuk (JCRB No. JCRB0097 cultured in RPMI-1640 + 10% FBS) and KMS-21BM (JCRB No. JCRB1185 cultured in RPMI-1640 + 10% FBS).
- **CD48:** NCI-H929 (ATCC No. CRL-9068 cultured in RPMI-1640 + 10% FBS + 0.05 mM 2-mercaptoethanol), OPM-2 (DSMZ No. ACC-50 cultured in RPMI-1640 + 10% FBS) and AMO1 (DSMZ No. ACC-538 cultured in RPMI-1640 + 20% FBS).
- **CD79b:** RS4;11 (ATCC No. CRL-1873 cultured in RPMI-1640 + 10% FBS).
- **CD33:** MOLM-13 (DSMZ No. ACC-554 cultured in RPMI-1640 + 10% FBS) and an isogenic cell line, AMO1-CD33 clone D2. AMO1 cell line was transfected to generate a stable AMO1 cell line expressing the exogenous protein of interest, CD33, AMO1-CD33 clone D2 (cultured in RPMI-1640 + 20% FBS).

### Inhibition of Cell Proliferation and Survival

The ability of Mcl-1 ADCs to inhibit cell proliferation and survival was assessed using a Promega CellTiter-Glo^{®} proliferation assay. Cell lines were cultured in media optimal for their growth at 5% CO₂, 37°C in a tissue culture incubator. Prior to seeding for the proliferation assay, the cells were split at least 2 days before the assay to ensure optimal growth density. On the day of seeding, adherent cells were lifted off tissue culture flasks using 0.25% trypsin. Cell viability and cell density were determined using a cell counter (Vi-Cell XR Cell Viability Analyzer, Beckman Coulter). Cells with greater than 85% viability were seeded in white clear bottom 384-well TC treated plates (Corning, Cat. No. 3765). Cells were seeded at a density of 1,000 cells per well in 45 µl of standard growth media. Plates were incubated at 5% CO₂, 37°C overnight in a tissue culture incubator. The next day, free Mcl-1 payload (P1), targeting Mcl-1 ADCs, and non-targeting isotype ADCs were prepared at 10X in standard growth media. The prepared drug treatments were then added to the cells resulting in final concentrations of 0.0005 - 500 nM and a final volume of 50 µl per well. Each drug concentration was tested in quadruplets. Plates were incubated at 5% CO₂, 37°C for 5 days in a tissue culture incubator, after which cell viability was assessed through the addition of 25 µl of CellTiter Glo^{®} (Promega, Cat. No. G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC50 and Amax values were extrapolated from the resultant curves.

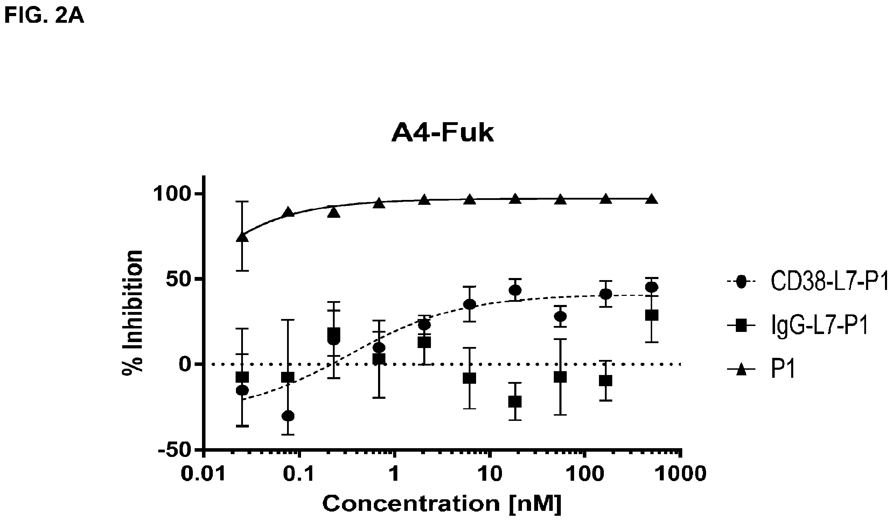

The dose response curves of representative cancer cell lines expressing six targets of interest are shown in **FIG. 2A** to **FIG. 7****.** The concentrations of treatment required to inhibit 50% of cell growth or survival (IC50) were calculated with representative IC50 values of the cell lines tested and are summarized in Tables 24-29.

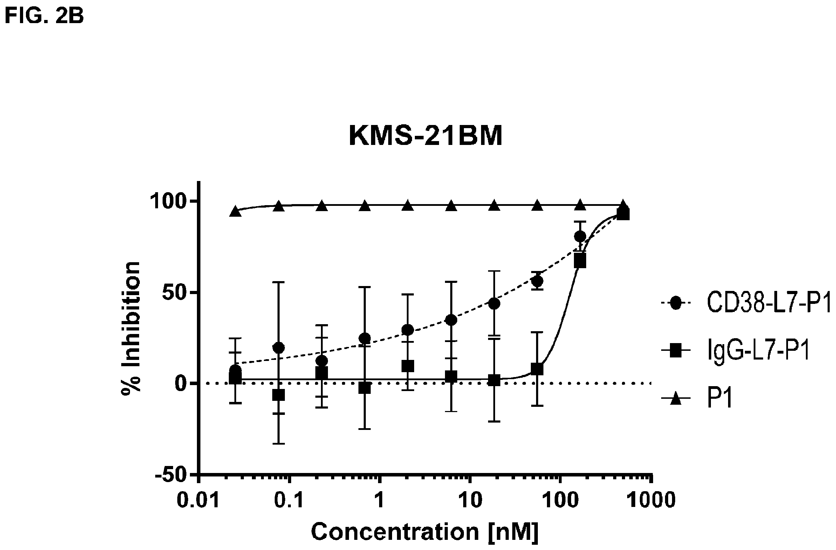

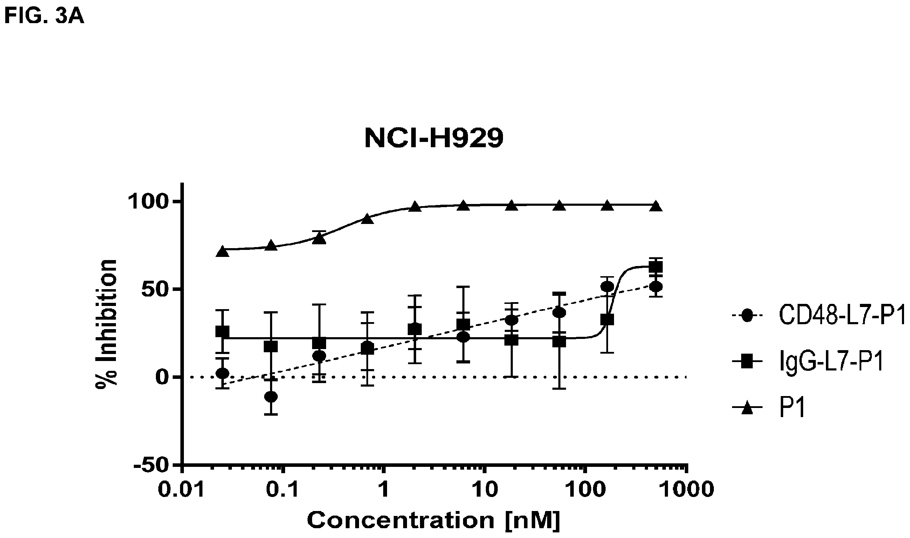

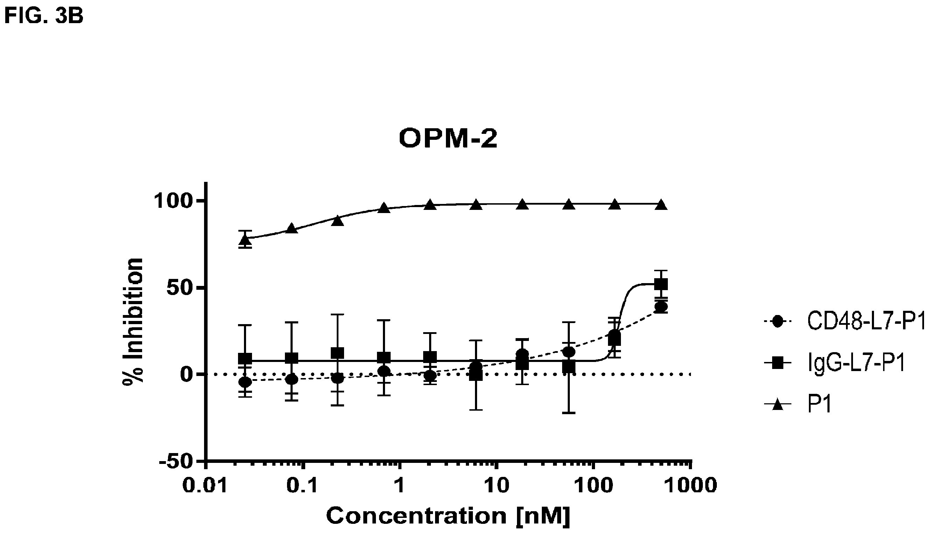

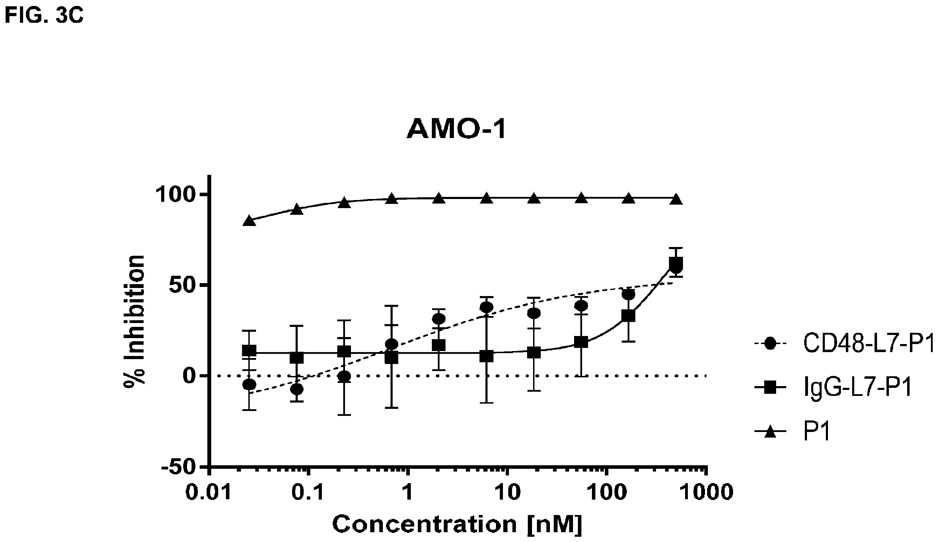

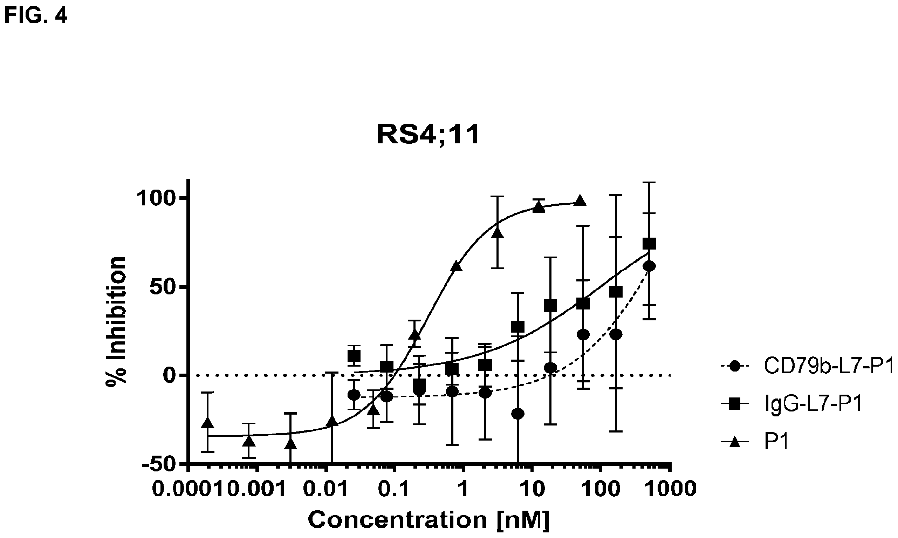

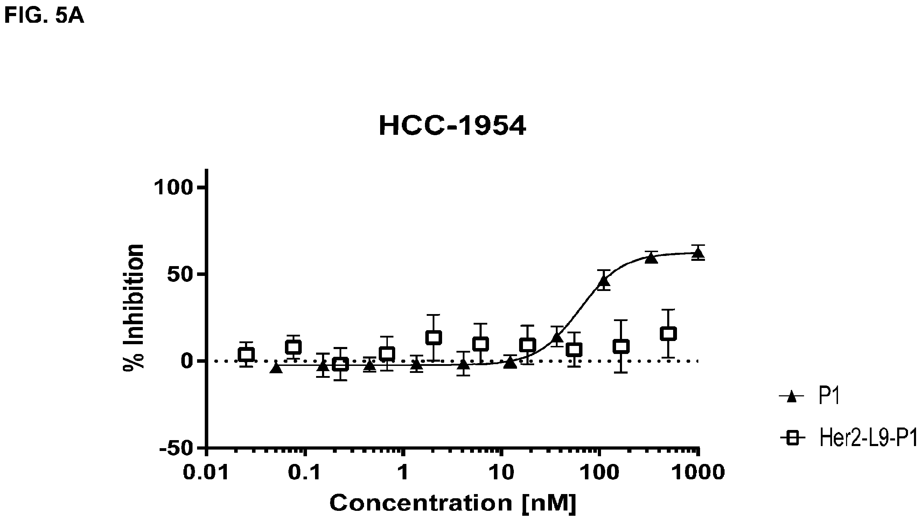

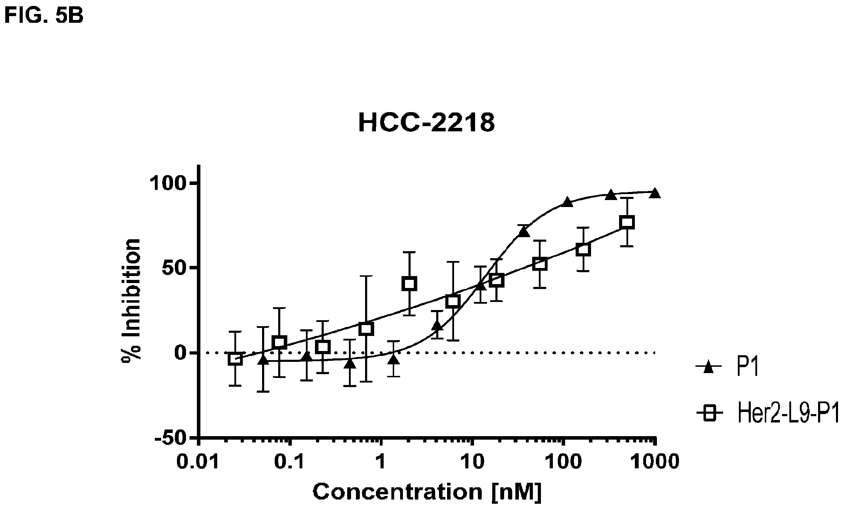

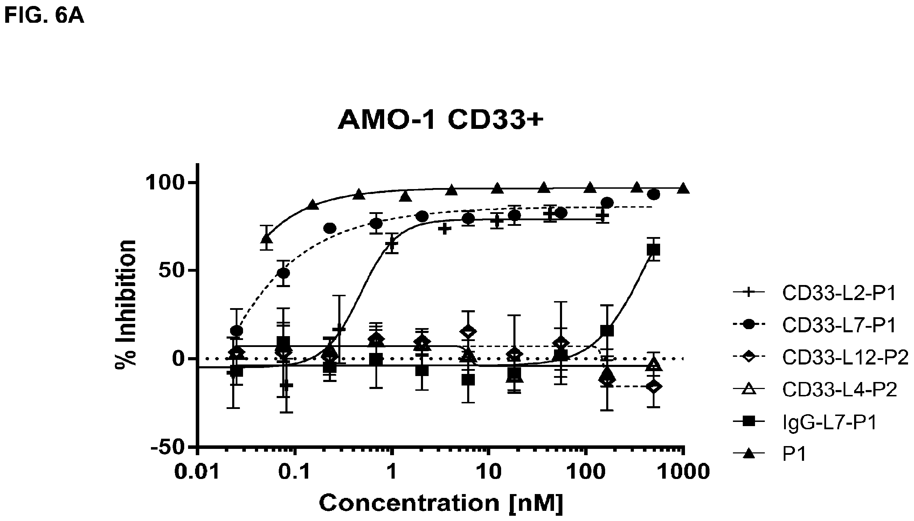

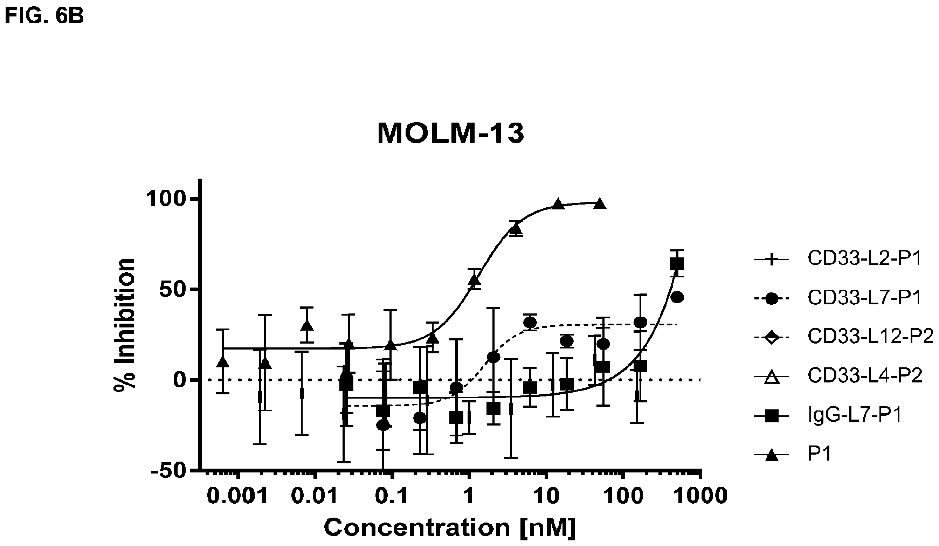

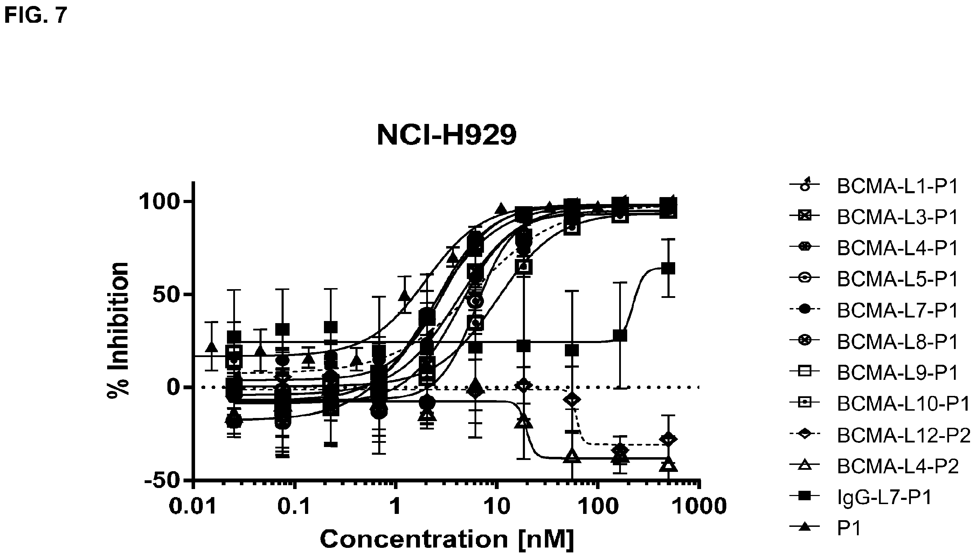

The representative cancer cell lines were shown to be sensitive to the Mcl-1 payload (P1) with IC50 values ranging from 0.038 - 5.6 nM activity, with the exception of the HER2-positive cancer cell lines in which the IC50 values ranged from 10-60 nM. The CD38-targeting Mcl-1 ADC CD38-L7-P1 tested on A4-Fuk and KMS-21BM cells demonstrated increased in vitro efficacy relative to the isotype matched non-targeting control ADC, IgG-L7-P1. However, maximum inhibition of cell growth reached 50% **(****FIG. 2A** and **FIG. 2B****).** Despite sensitivity to the Mcl-1 payload, P1, the CD48-targeting Mcl-1 ADC, CD48-L7-P1, did not induce inhibition of cell proliferation and was similar to the isotype matched non-targeting control ADC, IgG-L7-P1 in this experiment **(****FIG. 3A** to **FIG. 3C****).** A similar trend was observed for the CD79b-targeting Mcl-1 ADC, CD79b-L7-P1 **(****FIG. 4****).** A HER2-targeting Mcl-1 ADC, Her2-L9-P1, demonstrated cytotoxic activity on HCC2218 cells similar to that of the Mcl-1 payload (P1), but was inactive on HCC1954 cells **(****FIG. 5A** and **FIG. 5B****).** Cell proliferation of the isogenic cell line AMO1-CD33 clone D2 was inhibited by CD33-L2-P1, CD33-L3-P1 and CD33-L7-P1, with IC50s ranging from 0.089 - 0.476 nM. Little activity was observed with the matched non-targeting Mcl-1 ADC, IgG-L7-P1. CD33-L12-P2 and CD33-L4-P2 were inactive on the isogenic cell line AMO1-CD33 clone D2. Only the CD33-L7-P1 ADC was tested on MOLM-13 cells and its activity was modest despite having little background activity by the matched non-targeting Mcl-1 ADC, IgG-L7-P1 **(****FIG. 6A** and **FIG. 6B****).** BCMA-targeting ADCs using various linkers (L1, L3-L5, L7-L10) demonstrated similar cytotoxic activity inhibiting cell proliferation of NCI-H929 cells. The isotype matched non-targeting control, IgG-L7-P1, demonstrated some activity on the cell line at the highest testing concentration, 500 nM. The BCMA-L12-P2 and BCMA-L4-P2 ADCs were inactive on the NCI-H929 cell line **(****FIG. 7****).**

These studies indicate that Mcl-1 ADCs are capable of inhibiting cell proliferation on various cancer cell lines expressing BCMA, CD33 and, to a lesser extent, CD38. Mcl-1 ADCs demonstrated little cytotoxic activity relative to isotype matched non-targeting controls on cancer cell lines expressing CD38, CD79b, and HER2.

**Table 24. CD38 Mcl-1 ADCs**

| | **CD38** | | | |
|---|---|---|---|---|
| | A4FUK | | KMS21BM | |
| L/P or compound# | IC50 (nM) | Amax | IC50 (nM) | Amax |
| **L7-P1** | 0.494 | 81.08 | ** | ** |
| **P1** | ** | ** | ** | ** |

| | | | | |
|---|---|---|---|---|
| ** tested but curve did not converge | | | | |

**Table 25. CD48 Mcl-1 ADCs**

| | **CD48** | | | | | |
|---|---|---|---|---|---|---|
| | H929 | | OPM2 | | AMO1 | |
| L/P or compound# | IC50 (nM) | Amax | IC50 (nM) | Amax | IC50 (nM) | Amax |
| **L7-P1** | 3.708 | ~ 186.6 | ** | ** | 0.696 | 79.31 |
| **P1** | 0.394 | 25.79 | 0.136 | 22.97 | 0.038 | 19.97 |

| | | | | | | |
|---|---|---|---|---|---|---|
| ** tested but curve did not converge | | | | | | |

**Table 26. CD79b Mcl-1 ADCs**

| | **CD79b** | |
|---|---|---|
| | RS;411 | |
| L/P or compound# | IC50 (nM) | Amax |
| **L7-P1** | 1114.000 | 194.40 |
| **L9-P1** | 0.150 | 88.91 |
| **L10-P1** | 0.150 | 87.70 |
| **P1** | 0.299 | 132.40 |

| | | |
|---|---|---|
| ** tested but curve did not converge | | |

**Table 27. HER2 Mcl-1 ADCs**

| | **HER2** | | | |
|---|---|---|---|---|
| | HCC1954 | | HCC2218 | |
| L/P or compound# | IC50 (nM) | Amax | IC50 (nM) | Amax |
| **L9-P1** | ** | ** | ** | ** |
| **P1** | 63.52 | 64.80 | 14.08 | 100.40 |

| | | | | |
|---|---|---|---|---|
| ** tested but curve did not converge | | | | |

**Table 28. CD33 Mcl-1 ADCs**

| | **CD33** | | | |
|---|---|---|---|---|
| | MOLM13 | | AMO1-CD33 | |
| L/P or compound# | IC50 (nM) | Amax | IC50 (nM) | Amax |
| **L1-P1** | >0.701 | 100.61 | | |
| **L2-P1** | >150 | - | 0.476 | 84.11 |
| **L3-P1** | | | 0.089 | 108.90 |
| **L6-P1** | >0.644 | 103.75 | | |
| **L7-P1** | >150 | - | 0.368 | 69.23 |
| **L8-P1** | >0.733 | 101.22 | | |
| **L10-P1** | >0.757 | 99.65 | | |
| **L12-P2** | | | ** | ** |
| **L4-P2** | | | ** | ** |
| **P1** | 2.440 | 128.80 | 1.530 | 103.00 |

| | | | | |
|---|---|---|---|---|
| ** tested but curve did not converge | | | | |

**Table 29. BCMA Mcl-1 ADCs**

| | **BCMA** | |
|---|---|---|
| | H929 | |
| L/P or compound# | IC50 (nM) | Amax |
| **L1-P1** | 4.103 | 101.90 |
| **L3-P1** | 4.264 | 103.70 |
| **L4-P1** | 2.456 | 105.20 |
| **L5-P1** | 6.270 | 100.00 |
| **L7-P1** | 3.054 | 107.60 |
| **L8-P1** | 2.134 | 116.10 |
| **L9-P1** | 2.989 | 93.79 |
| **L10-P1** | 10.100 | 93.65 |
| **L12-P2** | ** | ** |
| **L4-P2** | ** | ** |
| **P1** | 5.608 | 89.16 |

| | | |
|---|---|---|
| ** tested but curve did not converge | | |

### Example 7. In Vitro Assessment - Anti-CD33 ADCs (Gln site-specific conjugation) AMO1-CD33 Engineered Cell Line Generation (Clone D2)

AMO1 is a multiple myeloma human cell line which does not express CD33. To generate an AMO1 clone expressing hCD33, AMO1 cells were transduced with lentiviral particles (GE Healthcare - Dharmacon) encoding hCD33 (clone ID: PLOHS 100004356) at a multiplicity of infection (MOI) of 8, in the presence of 8 µg/ml of polybrene. Cells were sorted based on GFP reporter gene expression and hCD33 presence at the cell surface was evaluated by flow cytometry. AMO1-CD33 cells were subsequently sorted as single cells in order to isolate clones with different levels of expression of hCD33. AMO1-CD33 clone D2 was selected based on its high expression of hCD33, as measured by flow cytometry.

### Cell Viability Using MTT Assay

An MTT colorimetric assay is based on the mitochondrial reduction of tetrazolium salt by living cells. The viable cell number is proportional to the production of formazan salts, which can be read spectrophotometrically at 540 nm.

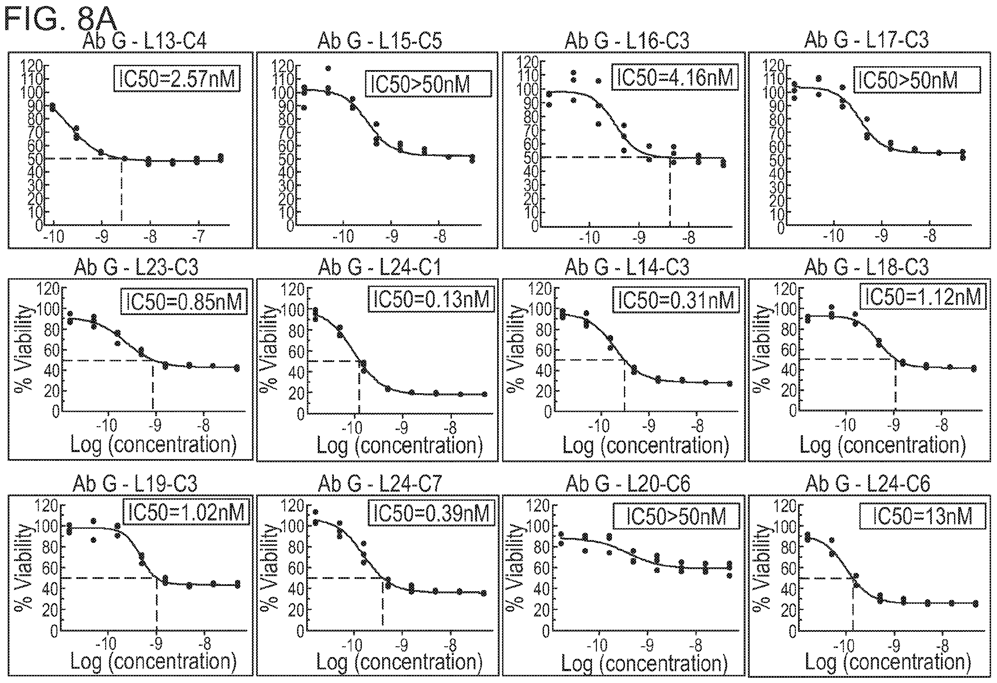

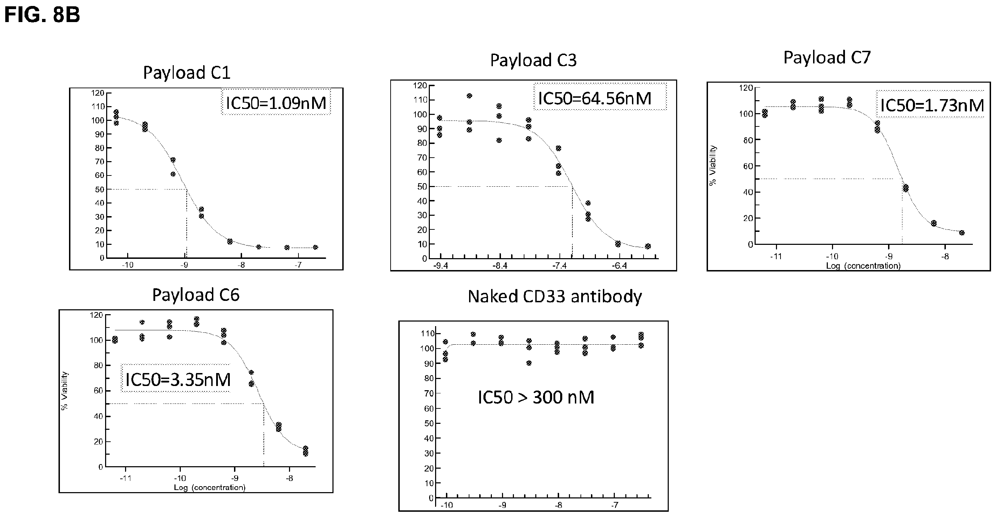

AMO1-CD33 clone D2 cells were cultivated in RPMI 1640 supplemented with 20% heat inactivated fetal bovine serum, penicillin (100 IU/ml), streptomycin (100 µg/ml), and L-glutamine (2 mM). Cells were cultured at 37°C in a humidified atmosphere containing 5% CO_{2.} Cells were seeded in 96 microwell plates (150 µl per well) and exposed to the ADCs for 48 hours (3,16 fold serially diluted; 8 concentrations each, triplicates). At the end of incubation time, 15 µl of MTT solution (5 mg/ml) were added per well and the cells were incubated for another 4 hours. Then, 100 µl of 10% sodium dodecyl sulfate (SDS)/HCl 10 mM were added per well and the plate was incubated overnight, before measurement of optical density at 540 nm. IC50 values were calculated using standard four-parametric curve fitting. For this assay, IC50 was defined as the compound concentration at which the MTT signal is reduced to 50% of that measured for the control. Each experiment was performed at least twice, with results being reproducible.
**Results:** As shown in **FIGs. 8A** and **8B****,** all tested anti-CD33 ADCs and corresponding payloads induced a dose dependent decrease in the viability of AMO1-CD33 clone D2 cells in the MTT assay, while no significant effect was observed with the corresponding naked antibody.

### Example 8. In Vitro Assessment - Anti-HER2 ADCs (Gln site-specific conjugation) Cell Viability Using CTG Assay

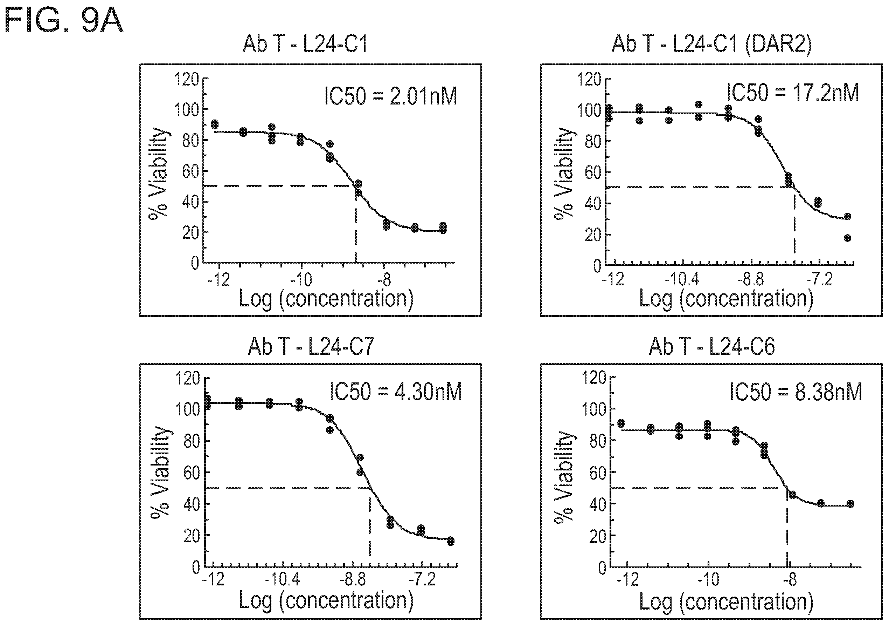

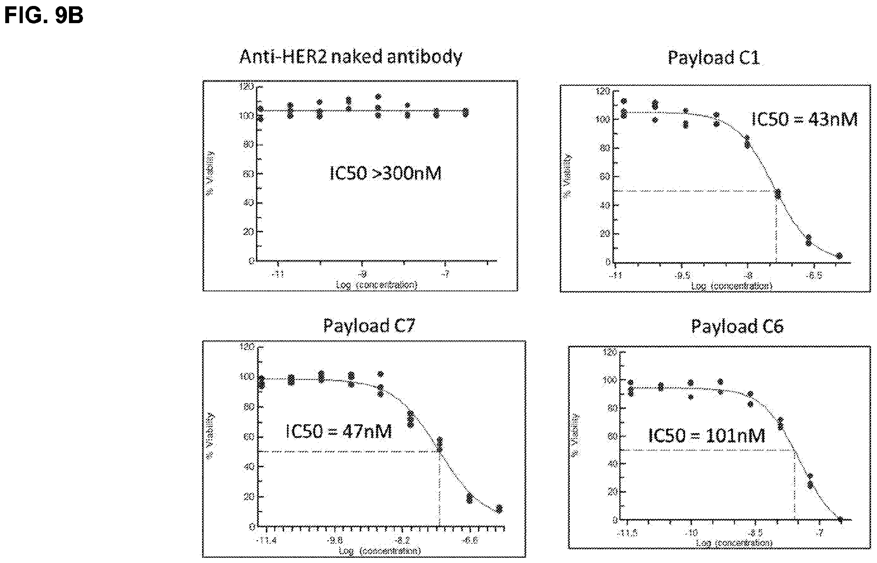

HCC1954 cells were cultivated in RPMI supplemented with 10% heat inactivated fetal bovine serum, penicillin (100 IU/ml), streptomycin (100 µg/ml), and L-glutamine (2 mM). Cells were cultured at 37°C in a humidified atmosphere containing 5% CO₂. Cells were seeded in 96 microwell plates and exposed to the ADCs for 120 hours (5 fold serially diluted; 9 concentrations each, triplicates). Effects of ADCs on cell viability were assessed after 5 days of incubation at 37°C/5% CO₂ by quantification of cellular ATP levels using CellTiterGlo^{®} reagent at 75 µl reagent/well. All conditions were tested in triplicate. Luminescence was quantified on a multipurpose plate reader. IC50 values were calculated using standard four-parametric curve fitting. For this assay, IC50 was defined as the compound concentration at which the CTG signal is reduced to 50% of that measured for the control. Each experiment was performed at least twice, with results being reproducible. **Results:** As shown in **FIGs. 9A** and **9B****,** all tested anti-HER2 ADCs and corresponding payloadsinduced a dose dependent decrease in the viability of HCC1954 cells in the CTG assay; while no significant effect was observed with the corresponding naked antibody.

### Example 9. In Vitro Assessment Effect of Anti-BCMA ADCs (Cys site-specific conjugation) on NCI-H929 (Multiple Myeloma) cell viability using MTT assay

MTT colorimetric assay is based on the mitochondrial reduction of tetrazolium salt by living cells. The viable cell number is proportional to the production of formazan salts, which can be read spectrophotometrically at 540 nm.

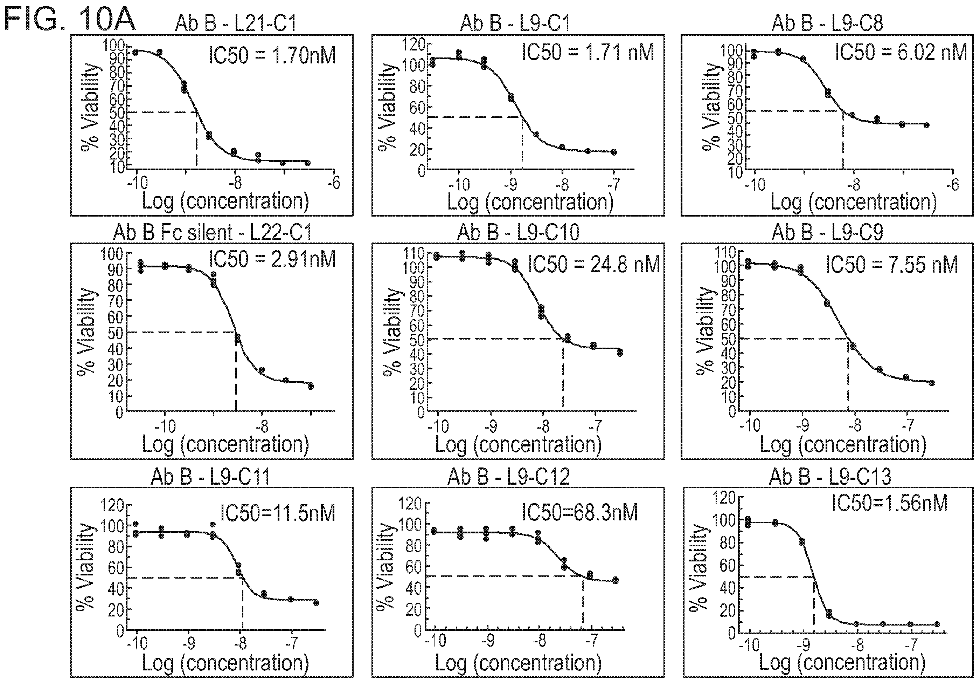

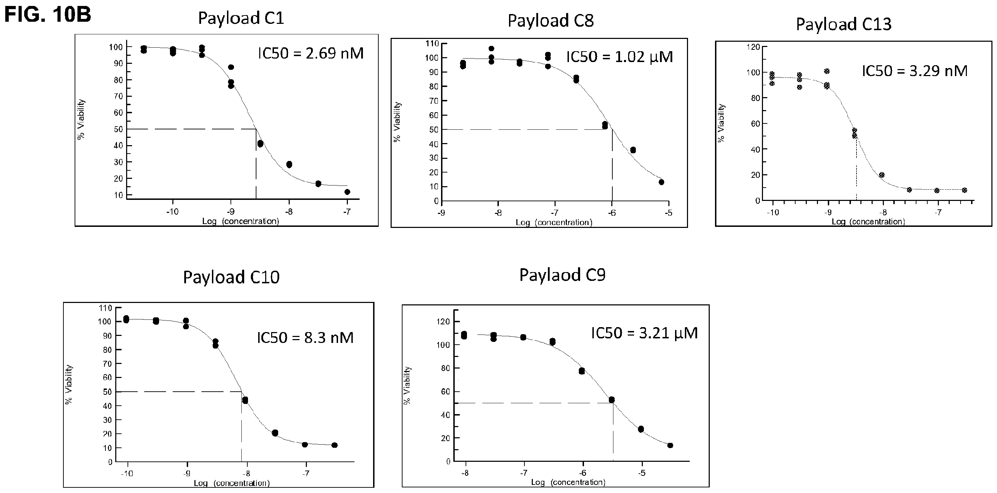

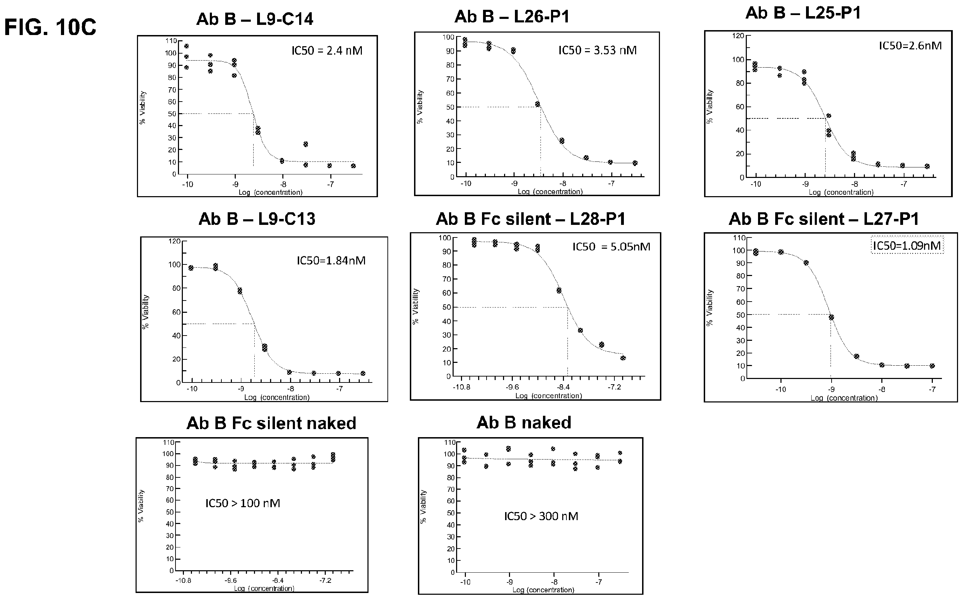

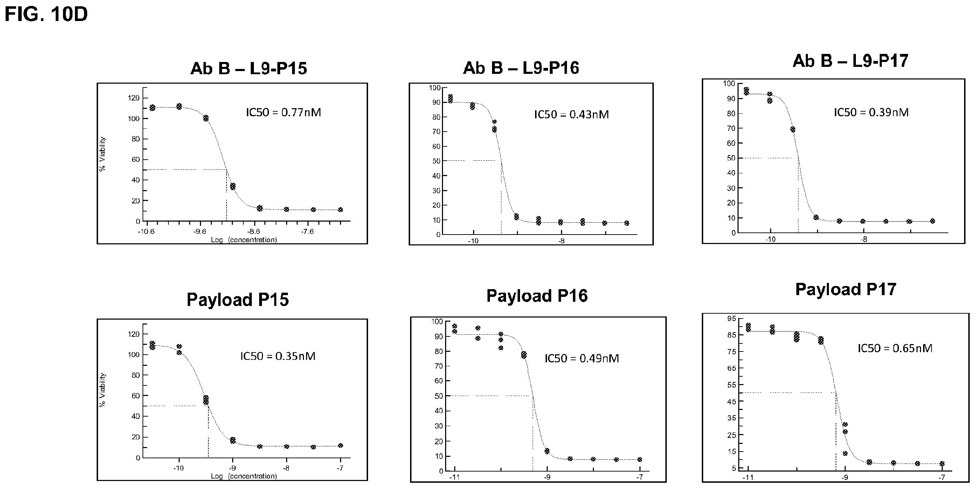

NCI-H929 cells were cultivated in RPMI 1640 supplemented with 10% heat inactivated fetal bovine serum, penicillin (100 IU/ml), streptomycin (100 µg/ml), and L-glutamine (2 mM). Cells were cultured at 37°C in a humidified atmosphere containing 5% CO_{2.} Cells were seeded in 96 microwell plates (150 µl per well) and exposed to the ADCs for 48 hours (3,16 fold serially diluted; 8 concentrations each, triplicates). At the end of incubation time, 15 µl of MTT solution (5 mg/ml) were added per well and the cells were incubated for another 4 hours. Then, 100 µl of 10% sodium dodecyl sulfate (SDS)/HCl 10 mM were added per well and the plate was incubated overnight, before measurement of optical density at 540 nm. IC50 values were calculated using standard four-parametric curve fitting. For this assay, IC50 was defined as the compound concentration at which the MTT signal is reduced to 50% of that measured for the control. Each experiment was performed at least twice, with results being reproducible.
**Results:** As shown in **FIGs. 10A****,** **10B****,****10C****, and** **10D****,** all tested anti-BCMA-Mcl1i ADCs and corresponding payloads induced a dose dependent decrease in the viability of NCI-H929 cells in the MTT assay, while no significant effect was observed with the corresponding naked antibody. **Example 10. In Vitro Assessment - Anti-CD48 ADCs**

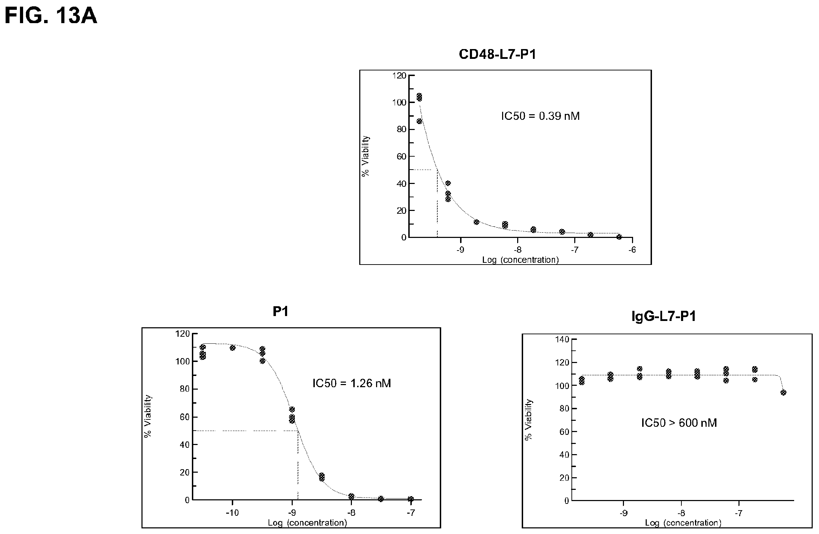

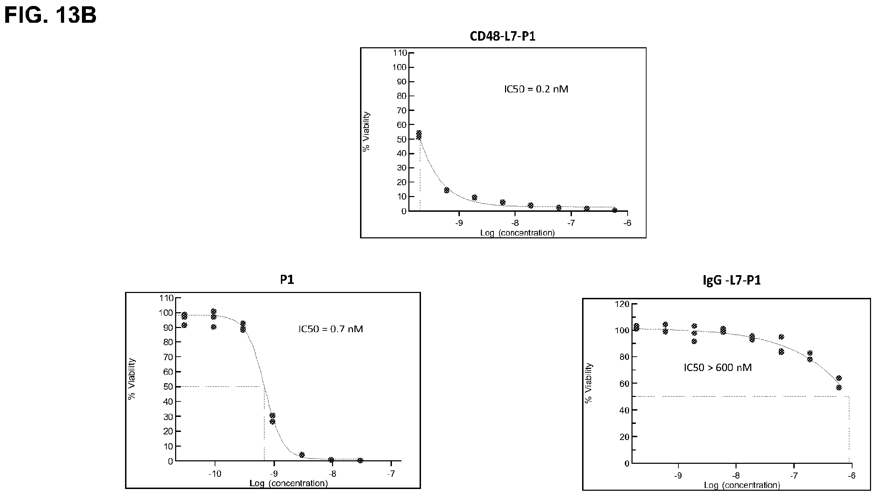

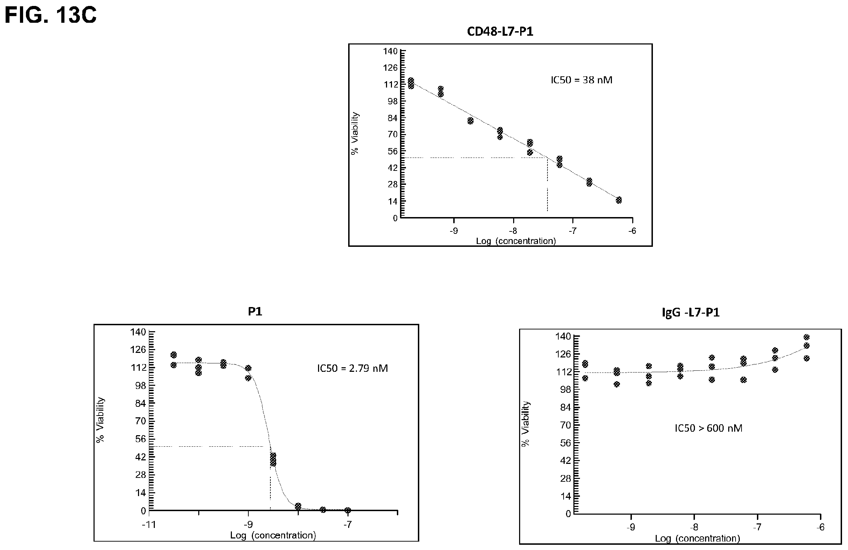

NCI-H929, KHM1B, and KMS21BM cells were cultivated in RPMI supplemented with 10% heat inactivated fetal bovine serum, penicillin (100 IU/ml), streptomycin (100 µg/ml), and L-glutamine (2 mM). Cells were cultured at 37°C in a humidified atmosphere containing 5% CO_{2.} Cells were seeded in 96 microwell plates and exposed to the ADCs for 72 hours (3,16 fold serially diluted; 8 concentrations each, triplicates). Effects of ADCs on cell viability were assessed after 3 days of incubation at 37°C/5% CO₂ by quantification of cellular ATP levels using CellTiterGlo^{®} reagent at 75 µl reagent/well. All conditions were tested in triplicate. Luminescence was quantified on a multipurpose plate reader. IC50 values were calculated using standard four-parametric curve fitting. For this assay, IC50 was defined as the compound concentration at which the CTG signal is reduced to 50% of that measured for the control. Each experiment was performed at least twice, with results being reproducible. **Results:** Effects of tested anti-CD48 ADCs in the viability of cells in the CTG assay are shown in **FIG. 13A** (NCI-H929), **FIG. 13B** (KHM1B), and **FIG. 13C** (KMS21BM). It was shown that tested anti-CD48 ADC induced a clear decrease in the viability of NCI-H929, KHM1B and KMS21BM cells in the CTG assay.

### Example 11. In Vivo Assessment I

Exemplary BCMA-targeting ADCs formulated in phosphate-buffered saline (PBS) were evaluated for in vivo therapeutic efficacy in an NCI-H929 multiple myeloma mouse model after intravenous (IV) administration.

### Materials and Methods

The following compounds were used in this example (ADCs were synthesized via methods as described in Example 4): IgG1-Linker-Payload Fc silent, anti-BCMA_CysmAb Fc silent Ab, anti-BCMA_CysmAb Fc silent_L7-P1.

NCI-H929 cells (ATCC No. CRL-9068) were cultured in RPMI supplemented with 10% FBS. Cells were resuspended in 100% matrigel (BD Biosciences) and 0.1 ml Matrigel containing 5 x 10⁶ cells was subcutaneously inoculated into the right flank of female SCID mice (Charles River). When tumors reached the appropriate volume, mice were randomized, 6 animals per group, using Easy stat software. IgG1-Linker-Payload Fc silent, anti-BCMA_CysmAb Fc silent, and anti-BCMA_CysmAb Fc silent_L7-P1 (10 mg/kg or 30 mg/kg) were injected once IV in PBS. Body weights were monitored three times a week and tumor size was measured using electronic calipers. Tumor volume was estimated by measuring the minimum and maximum tumor diameters using the formula: (minimum diameter)²(maximum diameter)/2. The last day with at least half of control animals still present in the study (d17), tumor growth inhibition was calculated using the formula: $\left(1-\frac{Median \left(DTV at Dx in treated group\right)}{Median \left(DTV at Dx in Control group\right)}\right) \times 100$ DTV (Delta Tumor Volume) at Dx was calculated as: TV at Dx - TV at Randomization. Response was evaluated as follows: CR (Complete Response) if tumor size was ≤ 25 mm³ for at least three measurements, PR (Partial Response) if tumor size was between 25 mm³ and 114 mm³ (half of the starting size) for at least three measurements. Mice were sacrificed at the first measurement for which tumor volume exceeded 2000 mm³ or at the first signs of animal health deterioration. All experiments were conducted in accordance with the French regulations in force in 2018. SCID mice were maintained according to institutional guidelines.

### Results

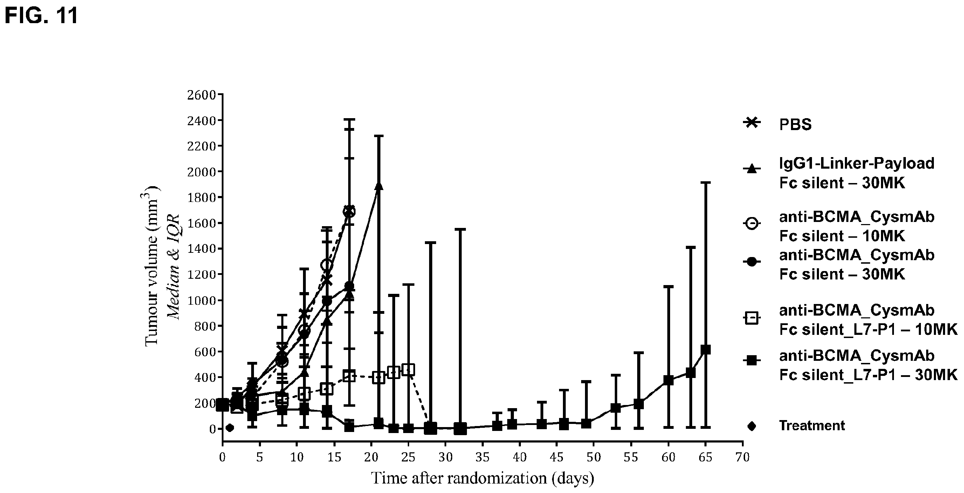

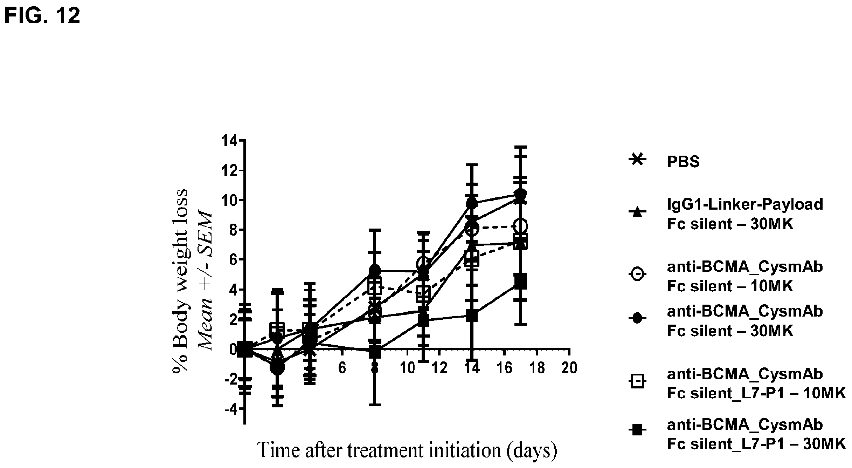

Efficacy of anti-BCMA_CysmAb Fc silent_L7-P1 on NCI-H929 xenografts is illustrated in **FIG. 11****.** Treatment was started 11 days post tumor cell inoculation (average size: 229.2 mm³). IgG1-Linker-Payload Fc silent (non-targeting ADC FS), anti-BCMA_CysmAb Fc silent (naked antibody FS), and anti-BCMA_CysmAb Fc silent_L7-P1 (BCMA-targeting ADC FS) were administered once IV at 10 and/or 30 mg/kg. On day 17, the Tumor Growth Inhibition (%TGI) induced by the BCMA-targeting ADC FS at 30 mg/kg was greater than the naked antibody FS (TGI= 99.2% *vs* 34.7% respectively, p<0.0001) and the corresponding non-targeting ADC FS (TGI= 37.7%, p<0.0001), as depicted in FIG. 11 and Table 30. At 10 mg/kg, the naked antibody FS did not display anti-tumor activity, whereas the BCMA-targeting ADC FS induced a TGI of 75.6% (p=0.0038). Complete regression (CR) was exclusively achieved on animals treated with the BCMA-targeting ADC FS. No clinically relevant body weight loss or other clinical signs due to the treatment were observed **(****FIG. 12****).**

**Table 30. Tumor growth inhibition**

| **Treatment** | **Dose/Schedule** | **%TGI (d17)** | **%CR** | **%PR** |
|---|---|---|---|---|
| IgG1-Linker-Payload Fc silent | 30MK QD, IV | 37.7 | 0 | 0 |
| anti-BCMA_CysmAb Fc silent | 10MK QD, IV | 0.5 | 0 | 0 |
| anti-BCMA_CysmAb Fc silent | 30MK QD, IV | 34.7 | 0 | 0 |
| anti-BCMA_CysmAb Fc silent_L7-P1 | 10MK QD, IV | 75.6* | 50 | 0 |
| anti-BCMA_CysmAb Fc silent_L7-P1 | 30MK QD, IV | 99.2* | 100 | 0 |

| | | | | |
|---|---|---|---|---|
| *p value <0.05 compared to control group. | | | | |

### Example 12. In Vivo Assessment II

The *in vivo* therapeutic effect of three BCMA-targeting ADCs was determined in the H929 multiple myeloma model after intravenous (IV) administration.

### Materials and Methods

The following compounds were used in this example (ADCs were synthesized via methods as described in Example 4): anti-BCMA_CysmAb Fc silent, anti-BCMA_CysmAb Fc silent_L11-P1, Ab B Fc silent - L27-P1, and anti-BCMA_CysmAb Fc silent_L5-P1.

H929 cells, obtained from ATCC, were cultured in RPMI supplemented with 10% FBS. Cells were resuspended in 100% matrigel (BD Biosciences) and 0.1ml containing 5x10⁶ cells were subcutaneously inoculated into the right flank of female SCID mice, provided by Charles River. When tumors reached the appropriate volume, mice were randomized, 6 animals per group, using Easy stat software. Anti-BCMA_CysmAb Fc silent, anti-BCMA_CysmAb Fc silent_**L11-P1/L5-P1,** Ab B Fc silent - **L27-P1** (20 mg/kg) were injected once IV in 20mM Histidine/NaCl. Mice body weight was monitored three times a week and tumor size measuredusing electronic calipers. Tumor volume was estimated by measuring the minimum and maximum tumor diameters using the formula: (minimumdiameter)²(maximum diameter)/2. The last day with at least half of control animals still present in the study (d14), tumor growth inhibition was calculated using the formula: $\left(1-\frac{Median \left(DTV at Dx in treated group\right)}{Median \left(DTV at Dx in Control group\right)}\right) \times 100$ with DTV (Delta Tumor Volume) at Dx, calculated being TV at Dx - TV at Randomization.

Response was evaluated as it follows: CR (Complete Response) if tumor size was ≤25mm3 for at least three measurements, PR (Partial Response) if tumor size was comprised between 25mm3 and 90.6 mm³ (half of the starting size) for at least three measurements.

Mice were sacrificed at the first measurement for which tumor volume exceeded 2000 mm³ or at the first signs of animal health deterioration. All experiments were conducted in accordance with the French regulations in force in 2018 after approval by Servier Research Institute (IdRS) Ethical Committee. SCID mice were maintained according to institutional guidelines.

### Results

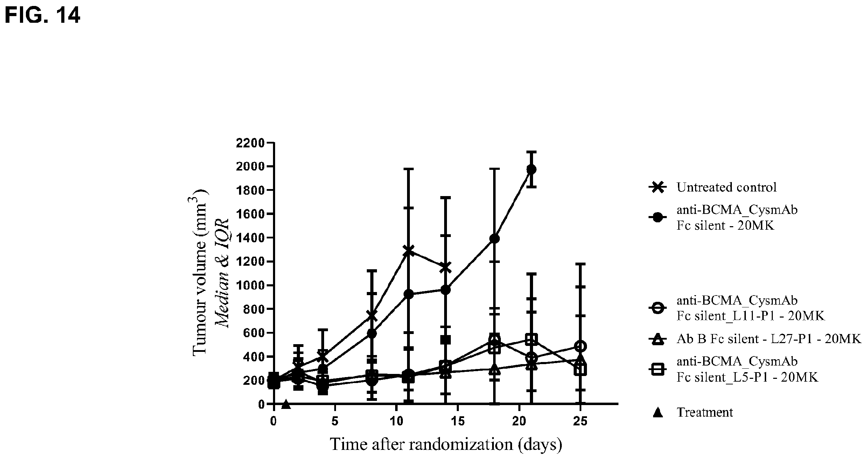

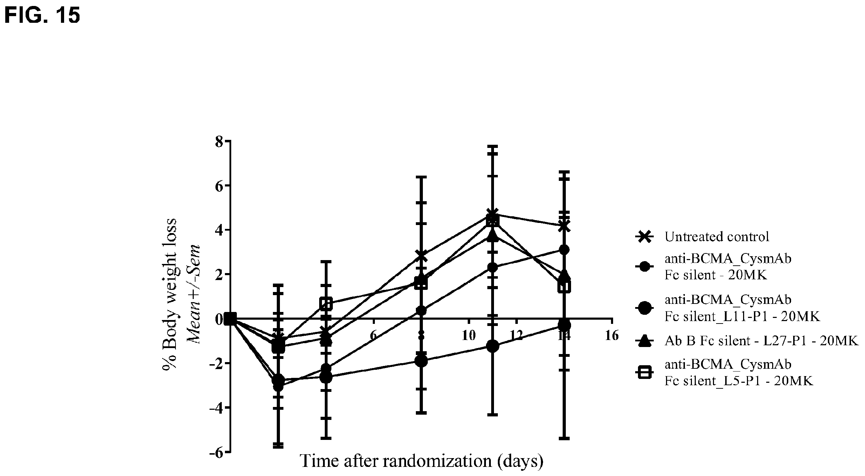

The efficacy of anti-BCMA_CysmAb Fc silent ADCs containing different linkers on H929 xenografts is illustrated in **FIG. 14****.** Treatment was started 11 days post tumor cells inoculation (median size: 181.3 mm³). Anti-BCMA_CysmAb Fc silent (naked antibody FS) and the different anti-BCMA_CysmAb Fc silent ADCs (BCMA-targeting ADCs FS) were administered once IV at 20 mg/kg. On day 14, the 3 BCMA-targeting ADCs FS **(L5-P1, L27-P1** and **L11-P1)** were found to induce a strong Tumor Growth Inhibition (%TGI) as compared to the naked antibody FS (TGI= 84.3%, 96.5%, 108.6% *vs* -1.7% respectively, p<0.01), as depicted in **FIG. 14** and Table 31. Complete regression (CR) was exclusively achieved on animals treated with BCMA targeting ADCs FS (Table 31). No clinically relevant body weight loss or other clinical signs due to the treatment were observed **(****FIG. 15****).**

**Table 31. H929 tumour growth inhibition upon treatment with anti-BCMA_CysmAb Fc silent or different anti-BCMA_CysmAb Fc silent ADCs (20 mg/kg, administered once IV)**

| **Treatment** | **Dose/Schedule** | **%TGI (d14)** | **%CR** | **%PR** |
|---|---|---|---|---|
| anti-BCMA_CysmAb Fc silent | 20MK QD, IV | -1.7 | 0 | 0 |
| anti-BCMA_CysmAb Fc silent_L11-P1 | 20MK QD, IV | 108.6** | 33.3 | 0 |
| Ab B Fc silent - L27-P1 | 20MK QD, IV | 96.5*** | 33.3 | 0 |
| anti-BCMA_CysmAb Fc silent_L5-P1 | 20MK QD, IV | 84.3** | 16.7 | 0 |

| | | | | |
|---|---|---|---|---|
| * p <= 0.05, ** p <= 0.01, *** p <= 0.001 compared to control group. | | | | |

### Example 13. In Vivo Assessment III

The *in vivo* therapeutic effect of a CD48-targeting ADC formulated in Phosphate-Buffered Saline (PBS) was determined in H929 multiple myeloma model after intravenous (IV) administration.

### Materials and Methods

The following compounds were used in this example: IgG1-Linker-Payload Fc silent, anti-CD48_CysmAb Fc silent, anti-CD48_CysmAb Fc silent_L5-P1. The anti-CD48 antibody used in the examples herein is humanized MEM102 (hMEM102), as described in US Patent Publication No. 20180092984, which was engineered with CysmAb mutations to facilitate conjugation to the linker-payload. ADCs were synthesized via methods as described in Example 4.

H929 cells, obtained from ATCC, were cultured in RPMI supplemented with 10% FBS. Cells were resuspended in 100% matrigel (BD Biosciences) and 0.1ml containing 5x10⁶ cells were subcutaneously inoculated into the right flank of female SCID mice, provided by Charles River. When tumors reached the appropriate volume, mice were randomized, 8 animals per group, using Easy stat software. IgG1-Linker-Payload Fc silent, anti-CD48_CysmAb Fc silent and anti-CD48_CysmAb Fc silent**_L5-P1** (30 mg/kg) were injected once IV in PBS. Mice body weight was monitored three times a week and tumor size measured using electronic calipers. Tumor volume was estimated by measuring the minimum and maximum tumor diameters using the formula: (minimum diameter)²(maximum diameter)/2. The last day with at least half of control animals still present in the study (d16), tumor growth inhibition was calculated using the formula: $\left(1-\frac{Median \left(DTV at Dx in treated group\right)}{Median \left(DTV at Dx in Control group\right)}\right) \times 100$ with DTV (Delta Tumor Volume) at Dx, calculated being TV at Dx - TV at Randomization.

Mice were sacrificed at the first measurement for which tumor volume exceeded 2000 mm³ or at the first signs of animal health deterioration. All experiments were conducted in accordance with the French regulations in force in 2018 after approval by Servier Research Institute (IdRS) Ethical Committee. SCID mice were maintained according to institutional guidelines.

### Results

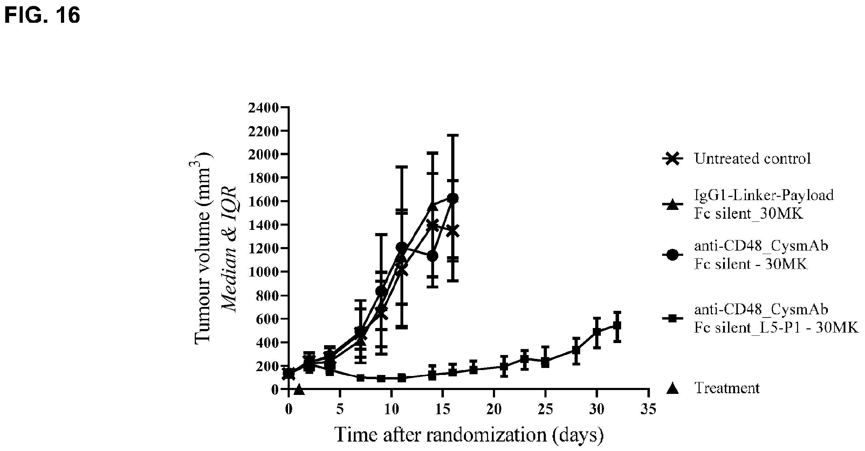

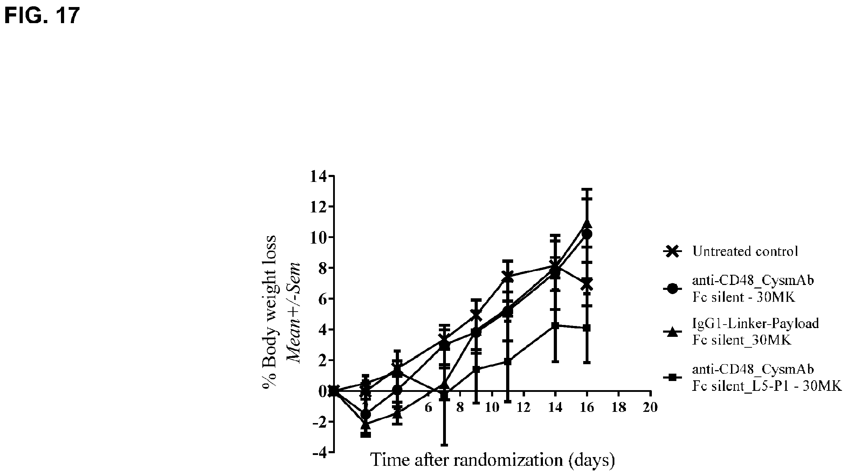

The efficacy of anti-CD48_CysmAb Fc silent_**L5-P1** on H929 xenografts is illustrated in **FIG. 16****.** Treatment was started 10 days post tumor cells inoculation (median size: 133.1 mm³). IgG1-Linker-Payload Fc silent (non-targeting ADC FS), anti-CD48_CysmAb Fc silent (naked antibody FS) and anti-CD48_CysmAb Fc silent_**L5-P1** (CD48-targeting ADC FS) were administered once IV at 30mg/kg. On day 16, the Tumor Growth Inhibition (%TGI) induced by the CD48-targeting ADC FS at 30mg/kg was greater than the naked antibody FS (TGI= 98.8% *vs* -22.9% respectively, p<0.001) and the correspondent non-targeting ADC FS (TGI= -50.4%), as depicted in **FIG. 16** and Table 32. No clinically relevant body weight loss or other clinical signs due to the treatment were observed **(****FIG. 17****).**

**Table 32. H929 tumor growth inhibition upon treatment with IgG1-Linker-Payload Fc silent, anti-CD48_CysmAb Fc silent or anti-CD48_CysmAb Fc silent_L5-P1 (30 mg/kg, administered once IV)**

| **Treatment** | **Dose/Schedule** | **%TGI (d16)** |
|---|---|---|
| IgG1-Linker-Payload Fc silent | 30MK QD, IV | -50.4 |
| anti-CD48_CysmAb Fc silent | 30MK QD, IV | -22.9 |
| anti-CD48_CysmAb Fc silent_ **L5-P1** | 30MK QD, IV | 98.2*** |

| | | |
|---|---|---|
| *** p <= 0.001 compared to control group. | | |

### Example 14. In Vitro Assessment - Effect of Anti-CD46-Mcl-1 ADC (Ab C-L9-C1) and the corresponding payload on KMS21BM (Multiple Myeloma) Cell Viability Using CTG Assay

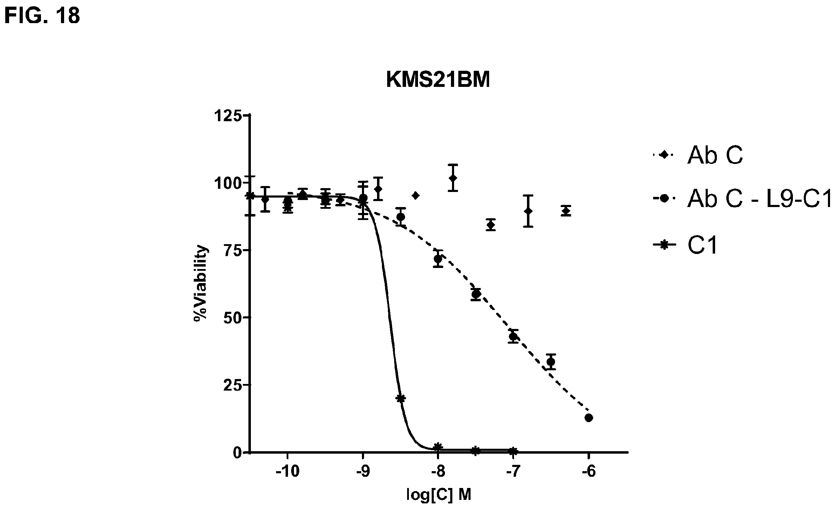

KMS21BM cells were cultivated in RPMI1640 supplemented with 10% heat inactivated fetal bovine serum, penicillin (100 IU/ml), streptomycin (100 µg/ml) and L-glutamine (2 mM), at 37°C in a humidified atmosphere containing 5% CO₂. KMS21BM cells were seeded in 96 microwell plates and exposed to the ADCs for 72h (3.16 fold serially diluted; 9 concentrations each, triplicates). Effects of ADC, naked antibody or the corresponding payload C1 on cell viability were assessed after 3 days of incubation at 37°C/5% CO₂ by quantification of cellular ATP levels using CellTiterGlo at 75 µL reagent/well. All the conditions were tested in triplicates. Luminescence was quantified on a multipurpose plate reader. IC₅₀s (Table 33) were calculated using standard four-parametric curve fitting. IC₅₀ is defined as the compound concentration at which the CTG signal is reduced to 50% of that measured for the control. Each experiment was performed at least twice, with results being reproducible. As shown in **FIG. 18****,** the anti-CD46-Mcl-1 ADC **(Ab C** - **L9-C1)** and the corresponding payload **C1** induced a dose dependent decrease in the viability of KMS21BM cells in CTG assay, while no significant effect was observed with the corresponding naked antibody.

**Table 33. IC₅₀ Results**

| **KMS21BM cell line** | **IC₅₀ (nM)** |
|---|---|
| Ab C naked | >300 |
| Ab C - L9-C1 | 87.82 |
| Payload C1 | 2.31 |

### Example 15. In Vitro Assessment - Effect of Anti-CD33-Mcl-1 ADCs in combination with the BCL2 inhibitor compound A1 in AMO-1-CD33 clone D2 cell viability using MTT assay

AMO1-CD33 clone D2 cells were cultivated in RPMI 1640 supplemented with 20% heat inactivated fetal bovine serum, penicillin (100 IU/ml), streptomycin (100 µg/ml) and L-glutamine (2 mM). Cells were cultured at 37°C in a humidified atmosphere containing 5% CO₂. Cells were seeded in 96 microwell plates (150µL per well) and exposed to the ADCs, naked antibody or the corresponding payloads for 48h (3,16 fold serially diluted; 8 concentrations each, triplicates) in the absence or in the presence of 1µM of the BCL2 inhibitor compound A1. At the end of incubation time, 15µL of MTT solution (5mg/ml) were added per well and the cells were incubated for another 4h. Then, 100µL of 10% Sodium Dodecyl Sulfate (SDS)/HCl 10mM were added per well and the plate was incubated overnight, before measurement of optical density at 540 nm. IC₅₀s were calculated using standard four-parametric curve fitting. IC₅₀ is defined as the compound concentration at which the MTT signal is reduced to 50% of that measured for the control. Each experiment was performed at least twice, with results being reproducible.

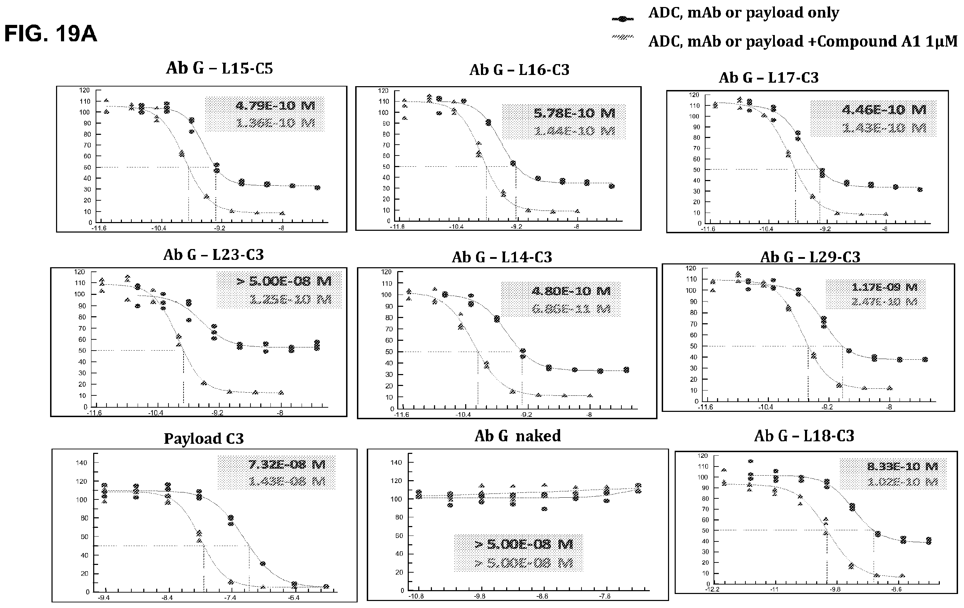

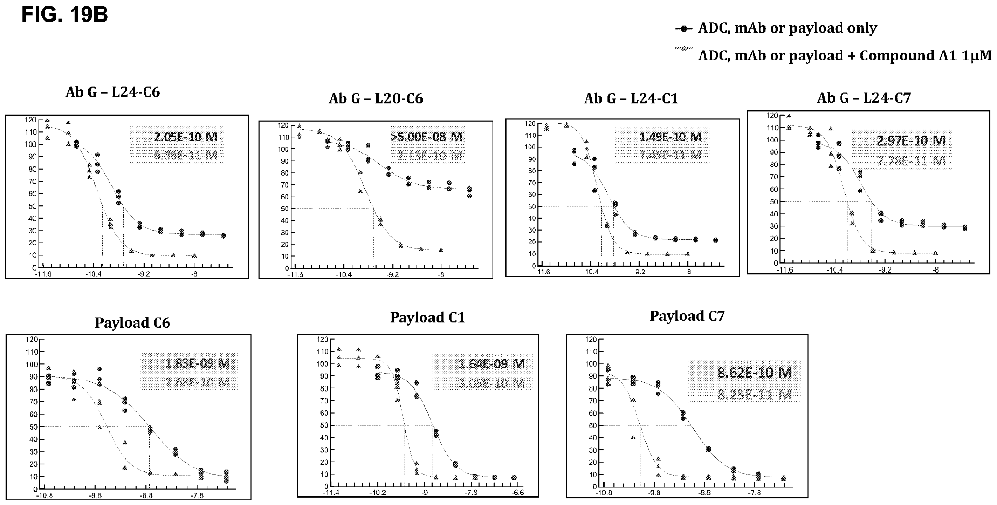

As shown in **FIGs. 19A** **and** **19B****,** all the ADCs induced a dose dependent decrease in the viability of AMO1-CD33 clone D2 cells at single agent. Interestingly, the activity of the ADCs is significantly improved when in combination with the BCL2 inhibitor compound A1, while no significant effect was observed after treatment of these cells with the corresponding naked antibody at single agent or in combination with 1µM compound A1.

### Example 16. In Vitro Assessment - Effect of Anti-BCMA-Mcl-1 ADCs as single agent and in combination with the BCL2 inhibitor compound A1 on AMO1 cell viability using MTT assay

MTT colorimetric assay is based on the mitochondrial reduction of tetrazolium salt by living cells. The viable cell number is proportional to the production of formazan salts, which can be read spectrophotometrically at 540 nm.

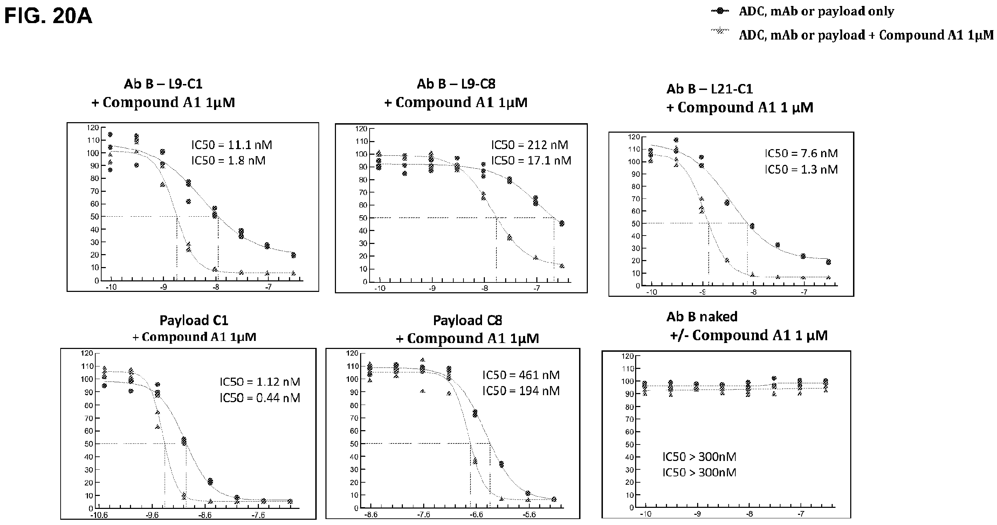

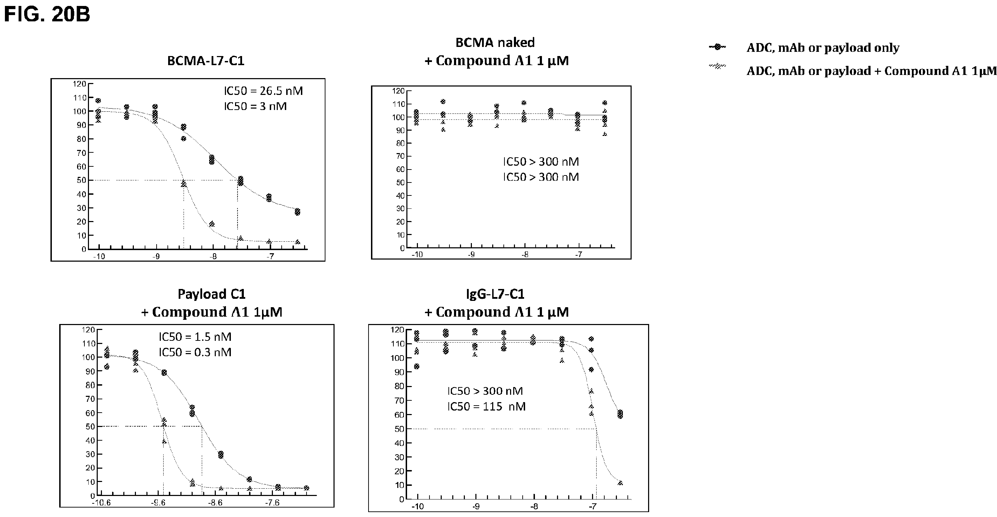

AMO1 cells were cultivated in RPMI 1640 supplemented with 20% heat inactivated fetal bovine serum, penicillin (100 IU/ml), streptomycin (100 µg/ml) and L-glutamine (2 mM). Cells were cultured at 37°C in a humidified atmosphere containing 5% CO₂. Cells were seeded in 96 microwell plates (150µL per well) and exposed to the ADCs or the corresponding payloads for 48h (3,16 fold serially diluted; 8 concentrations each, triplicates) in the absence or in the presence of 1µM of compound A1 compound. At the end of incubation time, 15µL of MTT solution (5mg/ml) were added per well and the cells were incubated for another 4h. Then, 100µL of 10% Sodium Dodecyl Sulfate (SDS)/HCl 10mM were added per well and the plate was incubated overnight, before measurement of optical density at 540 nm. C₅₀s were calculated using standard four-parametric curve fitting. IC₅₀ is defined as the compound concentration at which the MTT signal is reduced to 50% of that measured for the control. Each experiment was performed at least twice, with results being reproducible. As shown in **FIGs. 20A** and **20B****,** all the anti-BCMA-Mcl-1 ADCs induced a dose dependent decrease in the viability of AMO1 cells in the MTT assay. Interestingly, the activity of the ADCs was significantly improved when in combination with the BCL2 inhibitor compound A1, while no significant effect was observed after treatment of these cells with the corresponding naked antibody at single agent or in combination with 1µM of compound A1.

### Example 17. In Vitro Assessment - Effect of Anti-BCMA-Mcl-1 ADCs in combination with the BCL2 inhibitor compound A1 on NCI-H929 cell viability using MTT assay

MTT colorimetric assay is based on the mitochondrial reduction of tetrazolium salt by living cells. The viable cell number is proportional to the production of formazan salts, which can be read spectrophotometrically at 540 nm.

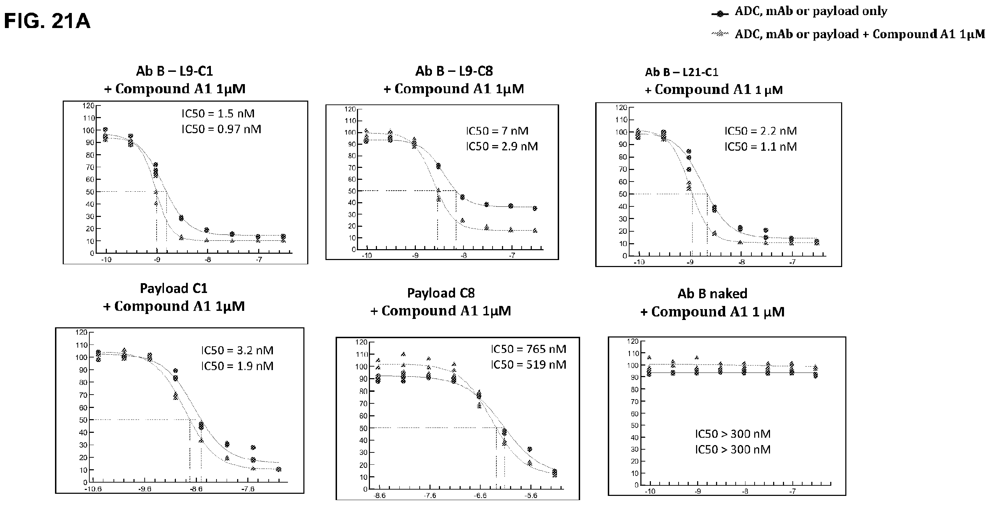

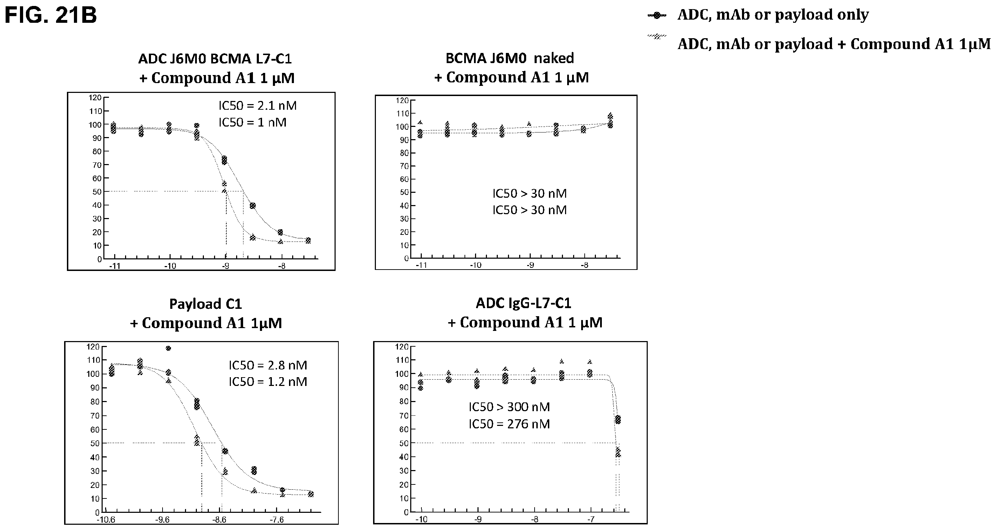

NCI-H929 cells were cultivated in RPMI supplemented with 10% heat inactivated fetal bovine serum, penicillin (100 IU/ml), streptomycin (100 µg/ml) and L-glutamine (2 mM). Cells were cultured at 37°C in a humidified atmosphere containing 5% CO_{2.}. Cells were seeded in 96 microwell plates (150 µL per well) and exposed to the ADCs or the corresponding payloads for 48h (3,16 fold serially diluted; 8 concentrations each, triplicates) in the absence or in the presence of 1µM of compound A1. At the end of incubation time, 15µL of MTT solution (5mg/ml) were added per well and the cells were incubated for another 4h. Then, 100µL of 10% Sodium Dodecyl Sulfate (SDS)/HCl 10mM were added per well and the plate was incubated overnight, before measurement of optical density at 540 nm. IC₅₀s were calculated using standard four-parametric curve fitting. IC₅₀ is defined as the compound concentration at which the MTT signal is reduced to 50% of that measured for the control. Each experiment was performed at least twice, with results being reproducible. As shown in **FIGs. 21A** and **21B****,** all the anti-BCMA-Mcl-1 ADCs induced a dose dependent decrease in the viability of H929 cells in the MTT assay. Interestingly, the activity of the ADCs is significantly improved when in combination with the BCL2 inhibitor compound A1, while no significant effect was observed after treatment of these cells with the corresponding naked antibody at single agent or in combination with 1µM of compound A1.

### Example 18. In Vitro Assessment - Anti-CD48 ADCs

### Cell Lines

The CD48 MCL-1 antibody drug conjugate CD48-L7-P1, isotype IgG1-L7-P1 ADC, and MCL-1 free payload P1 were tested against four endogenous cancer cell lines. KMS-21BM (JCRB No. JCRB1185 cultured in RPMI-1640 + 10% FBS), KMS-20 (JCRB No. JCRB1196 cultured in RPMI-1640 + 10% FBS), KMS-27 (JCRB No. JCRB1188 cultured in RPMI-1640 + 10% FBS), and NCI-H929 (ATCC No. CRL-9068 cultured in RPMI-1640 + 10% FBS + 0.05 mM 2-mercaptoethanol).

### Inhibition of cell proliferation and survival

The ability of the CD48 MCL-1 antibody drug conjugate CD48-L7-P1, isotype IgG1-L7-P1 ADC, and MCL-1 free payload P1 to inhibit cell proliferation and survival was assessed using the Promega CellTiter-Glo^{®} proliferation assay.

Cell lines were cultured in media that is optimal for their growth at 5% CO₂, 37°C in a tissue culture incubator. Prior to seeding for the proliferation assay, the cells were split at least 2 days before the assay to ensure optimal growth density. On the day of seeding, suspension cells were harvested. Cell viability and cell density were determined using a cell counter (Vi-Cell XR Cell Viability Analyzer, Beckman Coulter). Cells with higher than 85% viability were seeded in white clear bottom 384-well TC treated plates (Corning cat. # 3765). Cells were seeded at a density of 1,000 cells per well in 45 µL of standard growth media. Plates were incubated at 5% CO₂, 37°C overnight in a tissue culture incubator. The next day, free MCL-1 payload (P1), targeting MCL-1 ADC, and non-targeting isotype ADCs were prepared at 10X in standard growth media. The prepared drug treatments were then added to the cells resulting in final concentrations of 0.0005 - 500 nM and a final volume of 50 µL per well. Each drug concentration was tested in quadruplets. Plates were incubated at 5% CO₂, 37°C for 5 days in a tissue culture incubator, after which cell viability was assessed through the addition of 25 µL of CellTiter Glo^{®} (Promega, cat# G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC₅₀ and Amax values were extrapolated from the resultant curves.

### Result

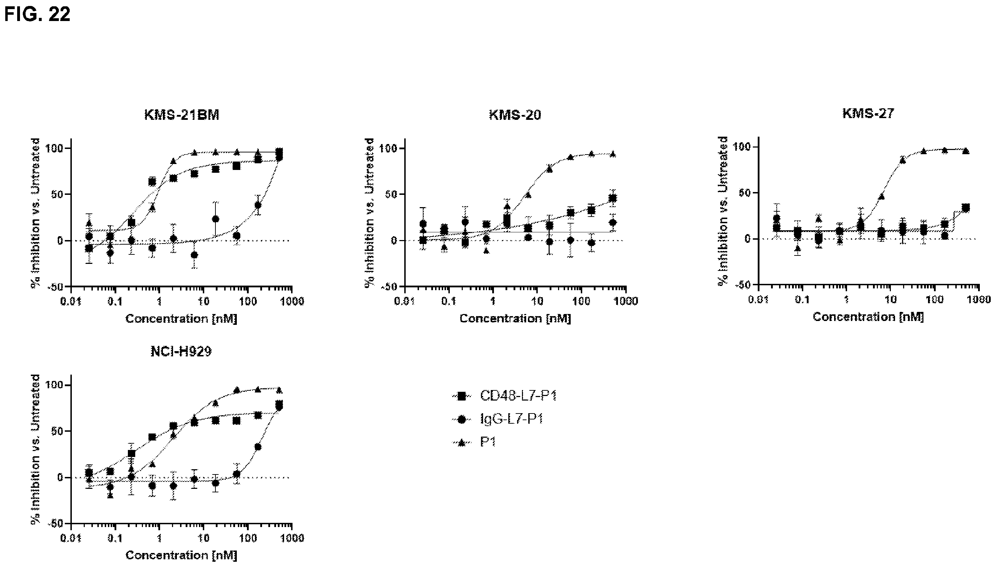

The dose response curves of representative cancer cell lines expressing the CD48 target of interest are shown in **FIG. 22** (square = CD48-L7-P1, circle IgG-L7-P1, triangle = free MCL1 payload P1). The concentrations of treatment required to inhibit 50% of cell growth or survival (IC₅₀) were calculated with representative IC50 values of the cell lines tested summarized in Table 34.

**Table 34: CD48 MCL1 ADCs**

| | CD48-L7-P1 | | IgG-L7-P1 | | P1 | |
|---|---|---|---|---|---|---|
| | IC50 (nM) | Amax | IC50 (nM) | Amax | IC50 (nM) | Amax |
| KMS-21BM | 0.257 | 112.2 | >200 | -- | 0.941 | 85.27 |
| KMS-20 | >200 | -- | >200 | -- | 4.946 | 94.34 |
| KMS-27 | >200 | -- | >200 | -- | 6.976 | 88.98 |
| NCI-H929 | 0.298 | 81.17 | >200 | -- | 2.039 | 109.1 |

### Example 19. In Vitro Assessment -Effects of Anti-CD48 ADCs as single agent and in combination with venetoclax on cell viability of various cancer cell lines

### Cell Lines

The CD48 MCL-1 antibody drug conjugates were tested against four endogenous cancer cell lines. KMS-21BM (JCRB No. JCRB1185 cultured in RPMI-1640 + 10% FBS), KMS-20 (JCRB No. JCRB1196 cultured in RPMI-1640 + 10% FBS), KMS-27 (JCRB No. JCRB1188 cultured in RPMI-1640 + 10% FBS), and NCI-H929 (ATCC No. CRL-9068 cultured in RPMI-1640 + 10% FBS + 0.05 mM 2-mercaptoethanol).

### Inhibition of cell proliferation and survival

The ability of the CD48 MCL-1 antibody drug conjugates to inhibit cell proliferation and survival was assessed using the Promega CellTiter-Glo^{®} proliferation assay. Cell lines were cultured in media that is optimal for their growth at 5% CO₂, 37°C in a tissue culture incubator. Prior to seeding for the proliferation assay, the cells were split at least 2 days before the assay to ensure optimal growth density. On the day of seeding, suspension cells were harvested. Cell viability and cell density were determined using a cell counter (Vi-Cell XR Cell Viability Analyzer, Beckman Coulter). Cells with higher than 85% viability were seeded in white clear bottom 384-well TC treated plates (Corning cat. # 3765). Cells were seeded at a density of 1,000 cells per well in 45 µL of standard growth media. Plates were incubated at 5% CO₂, 37°C overnight in a tissue culture incubator. The next day, free MCL-1 payload (P1), targeting MCL-1 ADCs and non-targeting isotype ADCs were prepared at 10X in standard growth media. The prepared drug treatments were then added to the cells resulting in final concentrations of 0.0005 - 300 nM and a final volume of 50 µL per well. Each drug concentration was tested in quadruplets. Two conditions were evaluated for each compound. The compounds were evaluated as single agents and in combination with venetoclax. The venetoclax was dosed at either 5 nM or 50 nM final concentration. Plates were incubated at 5% CO₂, 37°C for 5 days in a tissue culture incubator, after which cell viability was assessed through the addition of 25 µL of CellTiter Glo^{®} (Promega, cat# G7573), a reagent which lyses cells and measures total adenosine triphosphate (ATP) content. Plates were incubated at room temperature for 10 minutes to stabilize luminescent signals prior to reading using a luminescence reader (EnVision Multilabel Plate Reader, PerkinElmer). To evaluate the effect of the drug treatments, luminescent counts from wells containing untreated cells (100% viability) were used to normalize treated samples. A variable slope model was applied to fit a nonlinear regression curve to the data in GraphPad PRISM version 7.02 software. IC50 and Amax values were extrapolated from the resultant curves.

### Result

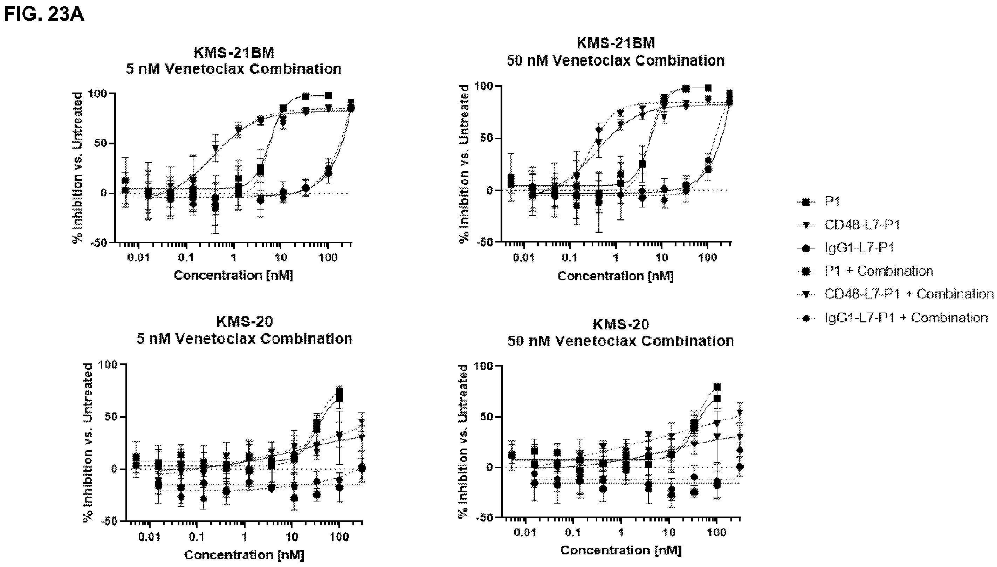

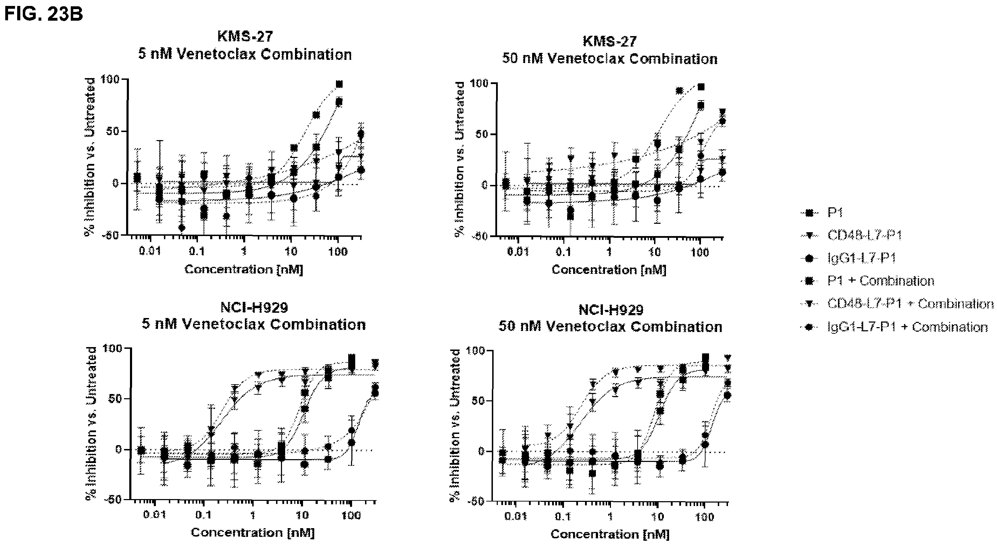

The dose response curves of representative cancer cell lines expressing the CD48 targets of interest are shown in **FIGs. 23A** and **23B****.** The concentrations of treatment required to inhibit 50% of cell growth or survival (IC50) were calculated with representative IC50 values of the cell lines tested summarized in Tables 35-37.

**Table 35: P1 + Venetoclax**

| | P1 | | P1 + 5 nM Venetoclax | | P1 + 50 nM Venetoclax | |
|---|---|---|---|---|---|---|
| | IC50 (nM) | Amax | IC50 (nM) | Amax | IC50 (nM) | Amax |
| KMS-21 BM | 5.732 | 94.25 | 5.573 | 100.8 | 5.229 | 99.41 |
| KMS-27 | 153.1 | - | 20.05 | 110.5 | 11.99 | 114.7 |
| KMS-20 | 34.71 | 65.65 | 30.55 | 75.53 | 36.16 | 82.77 |
| NCI-H929 | 10.73 | 90.09 | 8.686 | 91.53 | 8.29 | 103.4 |

**Table 36: CD48-L7-P1+ Venetoclax**

| | CD48-L7-P1 | | CD48-L7-P1+ 5 nM Venetoclax | | CD48-L7-P1 + 50 nM Venetoclax | |
|---|---|---|---|---|---|---|
| | IC50 (nM) | Amax | IC50 (nM) | Amax | IC50 (nM) | Amax |
| KMS-21BM | **0.3649** | **90.67** | **0.4164** | **90.69** | **0.301** | **84.86** |
| KMS-20 | **10.6** | **39.56** | >25 | -- | >25 | -- |
| KMS-27 | >25 | -- | >25 | -- | >25 | -- |
| NCI-H929 | **0.2259** | **92.5** | **0.2279** | **82.63** | **0.2317** | **81.49** |

**Table 37: IgG1- L7-P1+ Venetoclax**

| | IgG1-L7-P1 | | IgG1-L7-P1 + 5 nM Venetoclax | | IgG1-L7-P1 + 50 nM Venetoclax | |
|---|---|---|---|---|---|---|
| | IC50 (nM) | Amax | IC50 (nM) | Amax | IC50 (nM) | Amax |
| KMS-21BM | >200 | -- | >100 | -- | **149.9** | **116.1** |
| KMS-20 | >200 | -- | >200 | -- | >200 | -- |
| KMS-27 | >200 | -- | >200 | -- | >100 | -- |
| NCI-H929 | **138.4** | **70.94** | >100 | -- | **135.3** | **81.87** |

### Example 20. Evaluation of in vitro ADC activity in a panel of cancer cell lines

The following compounds were used in this example: CD33_CysmAb-L7-P1, CLL1_CysmAb-L7-P1, FLT3_CysmAb-L7-P1, cKIT_CysmAb-L7-P1, CD123_CysmAb-L7-P1, BCMA_CysmAb-L7-P1, CD138_CysmAb-L7-P1, CD38_CysmAb-L7-P1, CD48_CysmAb-L7-P1, SLAMF7_CysmAb-L7-P1, isotype IgG1_CysmAb-L7-P1, and free payload P1. The anti-CD33 antibody used in this example is gemtuzumab. The anti-CLL1 antibody used in this example is m6E7, as described in WO2016/205200. The anti-cKIT antibody used in this example is described in WO2014/150937. The CD123 antibody used in this example is CSL362, also known as talacotuzumab. The anti-BCMA antibody used in this example is J6M0, also known as belantamab. The anti-CD138 antibody used in this example is indatuximab. The anti-CD38 antibody used in this example is daratumumab. The anti-CD48 antibody used in this examples is humanized MEM102 (hMEM102), as described in US Patent Publication No. 20180092984. The anti-SLAMF7 antibody used in this example is azintuxizumab. Each of these antibodies was engineered with CysmAb mutations to facilitate conjugation to the linker-payload as described above. ADCs were synthesized via methods as described in Example 4.

The antibody drug conjugates were tested against cancer cell lines obtained from ATCC (American Type Culture Collection) or from cell lines derived from patient xenograft models. The cells were cultured in media that is optimal for their growth at 5% CO₂, 37°C in a tissue culture incubator. Prior to seeding for the proliferation assay, the cells were split at least 2 days before the assay to ensure optimal growth density. On the day of seeding, cells were lifted off tissue culture flasks using 0.25% trypsin. Cell viability and cell density were determined using a cell counter (Vi-Cell XR Cell Viability Analyzer, Beckman Coulter). Cells with higher than 85% viability were seeded in white clear bottom 384-well plates (Greiner cat # 781098) at a density of 1000 cells per well in 50 µL of standard growth media. Plates were incubated at 37°C overnight in a tissue culture incubator.

The ADCs were prepared in standard phosphate buffered solution to desired concentrations. A series of 10 dilutions were made for each ADC. The prepared drug treatments were then added to the cells resulting in final concentrations of 0.000005 - 300 nM. An acoustic transfer device (Echo555, Beckman Coulter) was used to add the ADCs to the cells. Each treatment was tested in triplicate assay plates. Plates were incubated at 37°C overnight or for 5 days in a tissue culture incubator. The ability of the ADCs to inhibit cell proliferation and survival was assessed using the Promega CellTiter-Glo^{®} proliferation assay. Plates were incubated at room temperature for 20 minutes to stabilize luminescent signals prior to reading using a multimode plate reader (Pherastar, BMG). Luminescent counts of untreated cells were taken the day after seeding (Day 0 readings), and after 5 days of treatment (Day 5 readings). The Day 5 readings of the untreated cells were compared to the Day 0 readings. Assays with at least one cell doubling during the incubation period were considered valid. To evaluate the effect of the drug treatments, luminescent counts from wells containing untreated cells (100% viability) were used to normalize treated samples. The concentrations of treatment required to inhibit 50% of cell growth or survival (GI50) were calculated using a four parameter logistic regression equation (Tables 38 and 39).

**Table 38. in vitro activity of ADCs against various AML cell lines**

| | **CD33** | | **CLL-1** | | **FLT3** | | **CKIT** | | **CD123** | | **Isotype IgG** | | **P1** | |
|---|---|---|---|---|---|---|---|---|---|---|---|---|---|---|
| **Cell Line** | **GI50 (µM)** | **Amax** | **GI50 (µM)** | **Amax** | **GI50 (µM)** | **Amax** | **GI50 (µM)** | **Amax** | **GI50 (µM)** | **Amax** | **GI50 (µM)** | **Amax** | **GI50 (µM)** | **Amax** |
| 21435hx | >0.3 | 80 | 0.154 | 45 | 0.001 | 34 | >0.3 | 63 | >0.3 | 90 | >0.3 | 91 | 0.012 | -91 |
| 5339hx | >0.3 | 116 | >0.3 | 103 | >0.3 | 69 | >0.3 | 53 | >0.3 | 99 | >0.3 | 96 | 0.005 | -83 |
| 5340hx | 0.063 | 12 | 0.162 | 15 | 0.019 | -24 | 0.0004 | -54 | 0.140 | -6 | >0.3 | 73 | 0.001 | -92 |
| 5979hx | 0.141 | 11 | 0.042 | 0 | 0.014 | -12 | 0.015 | 0 | 0.041 | 31 | 0.2 | 35 | 0.001 | -83 |
| aml193 | >0.3 | 106 | >0.3 | 107 | >0.3 | 110 | >0.3 | 111 | >0.3 | 108 | >0.3 | 99 | 0.006 | -87 |
| cmk | >0.3 | 107 | >0.3 | 101 | >0.3 | 104 | >0.3 | 100 | >0.3 | 100 | >0.3 | 95 | >10 | 100 |
| eol1 | 0.129 | -3 | >0.3 | 137 | 0.197 | -2 | 0.203 | 9 | >0.3 | 115 | 0.2 | 31 | 0.001 | -91 |
| f36p | >0.3 | 107 | >0.3 | 99 | >0.3 | 107 | >0.3 | 100 | >0.3 | 110 | >0.3 | 97 | >10 | 51 |
| hel | >0.3 | 119 | >0.3 | 108 | >0.3 | 111 | >0.3 | 99 | >0.3 | 108 | >0.3 | 84 | 6.086 | 4 |
| hel9217 | >0.3 | 104 | >0.3 | 99 | >0.3 | 102 | >0.3 | 97 | >0.3 | 103 | >0.3 | 87 | 5.283 | -1 |
| hl60 | >0.3 | 110 | >0.3 | 106 | >0.3 | 115 | >0.3 | 112 | >0.3 | 103 | >0.3 | 113 | 0.087 | -71 |
| kasumi1 | >0.3 | 107 | >0.3 | 115 | >0.3 | 94 | >0.3 | 107 | >0.3 | 140 | >0.3 | 108 | 0.037 | -87 |
| kasumi6 | >0.3 | 82 | >0.3 | 128 | >0.3 | 73 | >0.3 | 112 | >0.3 | 110 | >0.3 | 93 | 0.004 | -96 |
| kg1 | >0.3 | 120 | >0.3 | 100 | >0.3 | 101 | >0.3 | 98 | >0.3 | 104 | >0.3 | 97 | 0.861 | -69 |
| ko52 | >0.3 | 114 | >0.3 | 114 | >0.3 | 115 | >0.3 | 118 | >0.3 | 101 | >0.3 | 110 | 0.500 | -75 |
| m07e | >0.3 | 112 | >0.3 | 101 | >0.3 | 100 | >0.3 | 104 | >0.3 | 100 | >0.3 | 93 | 0.094 | -74 |
| molm13 | >0.3 | 75 | >0.3 | 51 | >0.3 | 65 | >0.3 | 62 | >0.3 | 95 | >0.3 | 73 | 0.002 | -54 |
| molm16 | >0.3 | 107 | >0.3 | 102 | >0.3 | 103 | >0.3 | 99 | >0.3 | 106 | >0.3 | 86 | 0.045 | -76 |
| monomac1 | >0.3 | 51 | 0.007 | 18 | 0.006 | 22 | 0.042 | 30 | >0.3 | 51 | >0.3 | 55 | 0.001 | -66 |
| monomac6 | >0.3 | 69 | 0.008 | 12 | 0.002 | 28 | 0.237 | 47 | >0.3 | 61 | >0.3 | 93 | 0.001 | -43 |
| mv411 | >0.3 | 59 | 0.097 | 30 | 0.046 | 30 | >0.3 | 95 | >0.3 | 80 | >0.3 | 54 | 0.001 | -72 |
| nb4 | >0.3 | 110 | >0.3 | 106 | >0.3 | 106 | >0.3 | 105 | >0.3 | 102 | >0.3 | 89 | 0.003 | -62 |
| nomo1 | >0.3 | 114 | >0.3 | 102 | 0.292 | 49 | >0.3 | 98 | >0.3 | 86 | >0.3 | 75 | 0.001 | -80 |
| ociaml2 | >0.3 | 109 | >0.3 | 103 | >0.3 | 100 | >0.3 | 103 | >0.3 | 100 | >0.3 | 87 | 0.127 | -50 |
| ociaml3 | >0.3 | 110 | >0.3 | 112 | >0.3 | 112 | >0.3 | 111 | >0.3 | 106 | >0.3 | 108 | 0.138 | 1 |
| ociaml5 | >0.3 | 115 | >0.3 | 105 | >0.3 | 107 | >0.3 | 103 | >0.3 | 100 | >0.3 | 98 | 0.265 | 4 |
| ocim1 | >0.3 | 112 | >0.3 | 110 | >0.3 | 113 | >0.3 | 106 | >0.3 | 103 | >0.3 | 105 | >10 | 86 |
| p31fuj | >0.3 | 120 | >0.3 | 107 | >0.3 | 105 | >0.3 | 106 | >0.3 | 93 | >0.3 | 95 | 0.116 | -73 |
| pl21 | >0.3 | 109 | >0.3 | 109 | >0.3 | 115 | >0.3 | 107 | >0.3 | 103 | >0.3 | 100 | 0.350 | -55 |
| siqm5 | >0.3 | 109 | >0.3 | 104 | >0.3 | 105 | >0.3 | 106 | >0.3 | 100 | >0.3 | 100 | 0.118 | -21 |
| skm1 | >0.3 | 107 | >0.3 | 107 | >0.3 | 107 | >0.3 | 106 | >0.3 | 105 | >0.3 | 100 | 0.119 | -49 |
| tf1 | >0.3 | 97 | >0.3 | 100 | >0.3 | 99 | >0.3 | 99 | >0.3 | 113 | >0.3 | 66 | 0.239 | 10 |
| thp1 | >0.3 | 110 | >0.3 | 111 | >0.3 | 71 | >0.3 | 103 | >0.3 | 110 | >0.3 | 102 | 0.028 | -72 |

**Table 39: in vitro activity of ADCs against various MM cell lines**

| | **BCMA** | | **CD138** | | **CD38** | | **CD48** | | **SLAMF7** | | **Isotype IgG** | | **P1** | |
|---|---|---|---|---|---|---|---|---|---|---|---|---|---|---|
| **Cell Line** | **GI50 (µM)** | **Amax** | **GI50 (µM)** | **Amax** | **GI50 (µM)** | **Amax** | **GI50 (µM)** | **Amax** | **GI50 (µM)** | **Amax** | **GI50 (µM)** | **Amax** | **GI50 (µM)** | **Amax** |
| amo1 | >0.3 | 52 | 0.001 | -1 | >0.3 | 72 | >0.3 | 55 | >0.3 | 88 | >0.3 | 112 | 0.005 | -20 |
| ejm | >0.3 | 117 | >0.3 | 121 | >0.3 | 113 | >0.3 | 115 | >0.3 | 124 | >0.3 | 116 | 0.061 | -93 |
| h929 | 0.003 | -71 | 0.002 | 29 | >0.3 | 87 | 0.0014 | 17 | >0.3 | 83 | >0.3 | 118 | 0.001 | -74 |
| karpas620 | >0.3 | 104 | >0.3 | 110 | >0.3 | 106 | >0.3 | 108 | >0.3 | 115 | >0.3 | 112 | 0.205 | -43 |
| ke97 | >0.3 | 129 | >0.3 | 119 | >0.3 | 123 | >0.3 | 66 | >0.3 | 126 | >0.3 | 117 | 7.913 | 21 |
| khm1b | >0.3 | 98 | 0.000389 | -16 | 0.000015 | 38 | 0.000566 | 11 | 0.008368 | 25 | >0.3 | 117 | 0.002 | -84 |
| kmm1 | >0.3 | 96 | >0.3 | 101 | >0.3 | 96 | >0.3 | 96 | >0.3 | 107 | >0.3 | 107 | 0.154 | -67 |
| kms11 | >0.3 | 94 | >0.3 | 109 | >0.3 | 94 | >0.3 | 97 | >0.3 | 104 | >0.3 | 102 | 0.098 | -62 |
| kms12bm | >0.3 | 102 | >0.3 | 144 | >0.3 | 127 | >0.3 | 130 | >0.3 | 142 | >0.3 | 125 | 0.011 | -79 |
| kms20 | >0.3 | 112 | >0.3 | 75 | >0.3 | 111 | >0.3 | 105 | >0.3 | 117 | >0.3 | 112 | 0.008 | -88 |
| kms21bm | 0.008547 | 5 | 0.000632 | -42 | 0.012261 | -28 | 0.000544 | 7 | 0.028214 | 4 | >0.3 | 126 | 0.001 | -76 |
| kms26 | >0.3 | 118 | >0.3 | 111 | >0.3 | 116 | >0.3 | 109 | >0.3 | 126 | >0.3 | 126 | 0.060 | -49 |
| kms27 | >0.3 | 104 | >0.3 | 111 | >0.3 | 104 | >0.3 | 103 | >0.3 | 107 | >0.3 | 103 | 0.001 | -89 |
| kms28bm | >0.3 | 105 | >0.3 | 123 | >0.3 | 115 | >0.3 | 112 | >0.3 | 115 | >0.3 | 121 | 0.006 | -42 |
| kms34 | >0.3 | 117 | >0.3 | 137 | >0.3 | 122 | >0.3 | 133 | >0.3 | 134 | >0.3 | 130 | 0.498 | -64 |
| L363 | >0.3 | 71 | >0.3 | 94 | >0.3 | 88 | >0.3 | 83 | >0.3 | 86 | >0.3 | 124 | 0.003 | -47 |
| lp1 | >0.3 | 106 | >0.3 | 131 | >0.3 | 115 | >0.3 | 122 | >0.3 | 128 | >0.3 | 126 | 0.007 | -58 |
| molp8 | 0.253701 | 46 | 0.005143 | 20 | 0.029991 | 11 | >0.3 | 61 | >0.3 | 67 | >0.3 | 67 | 0.001 | -74 |
| opm2 | >0.3 | 110 | >0.3 | 58 | >0.3 | 117 | >0.3 | 107 | >0.3 | 118 | >0.3 | 119 | 0.001 | -77 |
| rpmi8226 | >0.3 | 122 | >0.3 | 84 | >0.3 | 153 | >0.3 | 147 | >0.3 | 141 | >0.3 | 127 | 0.002 | -91 |
| skmm2 | >0.3 | 112 | >0.3 | 117 | >0.3 | 115 | >0.3 | 118 | >0.3 | 113 | >0.3 | 104 | >10 | 112 |
| u266b1 | >0.3 | 107 | >0.3 | 121 | >0.3 | 114 | >0.3 | 115 | >0.3 | 120 | >0.3 | 106 | 0.283 | -24 |

## Claims

1. An antibody-drug conjugate of Formula (1):
Ab-(L-D)*ₚ* (1)
wherein Ab is an antibody or an antigen-binding fragment thereof;
D is an Mcl-1 inhibitor, wherein D comprises a compound of Formula (II): 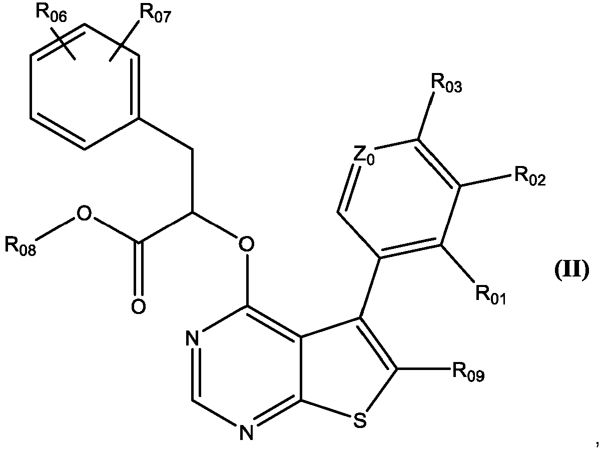
or an enantiomer, diastereoisomer, atropisomer, deuterated derivative, and/or pharmaceutically acceptable salt thereof;
wherein:
Z₀ is a nitrogen atom or a C-R₀₄ group,
R₀₁ is a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl group, a hydroxy group, a linear or branched (C₁-C₆)alkoxy group, a -S-(C₁-C₆)alkyl group, a cyano group, -Cy_{08,} -NR₀₁₁R₀₁₁',
R₀₂, R₀₃ and R₀₄ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-NR₀₁₁R₀₁₁', -O-(C₁-C₆)alkyl-R₀₃₁, -C(O)-OR₀₁₁, -O-C(O)-R₀₁₁, -C(O)-NR₀₁₁R₀₁₁', -NR₀₁₁-C(O)-R₀₁₁', - NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl, or the pair (R₀₂, R₀₃) or (R₀₃, R₀₄) together with the carbon atoms to which they are attached form an aromatic or non-aromatic ring containing 5 to 7 ring members, which optionally contains 1 to 3 heteroatoms selected from O, S and N, wherein the ring is optionally substituted by a group selected from a linear or branched (C₁-C₆)alkyl, - NR₀₁₃R₀₁₃', -(C₀-C₆)alkyl-Cy₀₁ and oxo,
R₀₆ and R₀₇ independently of one another are a hydrogen atom, a halogen atom, a linear or branched (C₁-C₆)alkyl group, a linear or branched (C₂-C₆)alkenyl group, a linear or branched (C₂-C₆)alkynyl group, a linear or branched (C₁-C₆)haloalkyl, a hydroxy group, a linear or branched (C₁-C₆)alkoxy group, a -S-(C₁-C₆)alkyl group, a cyano group, a nitro group, -(C₀-C₆)alkyl-NR₀₁₁R₀₁₁', -O-Cy₀₁, -(C₀-C₆)alkyl-Cy₀₁, -(C₂-C₆)alkenyl-Cy₀₁, -(C₂-C₆)alkynyl-Cy₀₁, -O-(C₁-C₆)alkyl-R₀₁₂, -C(O)-OR₀₁₁, -O-C(O)-R₀₁₁, -C(O)-NR₀₁₁R₀₁₁', -NR₀₁₁-C(O)-R₀₁₁', - NR₀₁₁-C(O)-OR₀₁₁', -(C₁-C₆)alkyl-NR₀₁₁-C(O)-R₀₁₁', -SO₂-NR₀₁₁R₀₁₁', or -SO₂-(C₁-C₆)alkyl,
or the pair (R₀₆, R₀₇), when fused with two adjacent carbon atoms, together with the carbon atoms to which they are attached form an aromatic or non-aromatic ring containing 5 to 7 ring members, which optionally contains 1 to 3 heteroatoms selected from O, S and N, and wherein the resulting ring is optionally substituted by a group selected from a linear or branched (C₁-C₆)alkyl group, -NR₀₁₃R₀₁₃', -(C₀-C₆)alkyl-Cy₀₁ and an oxo,
R₀₈ is a hydrogen atom, a linear or branched (C₁-C₈)alkyl group, an aryl group, a heteroaryl group, an aryl-(C₁-C₆)alkylgroup, or a heteroaryl(C₁-C₆)alkyl group,
R₀₉ is a linear or branched (C₁-C₆)alkyl group, a linear or branched (C₂-C₆)alkenyl group, a linear or branched (C₂-C₆)alkynyl group, -Cy₀₂, -(C₁-C₆)alkyl-Cy₀₂, -(C₂-C₆)alkenyl-Cy₀₂, -(C₂-C₆)alkynyl-Cy₀₂, -Cy₀₂-Cy₀₃, -(C₂-C₆)alkynyl-O-Cy₀₂, -Cy₀₂-(C₀-C₆)alkyl-O-(C₀-C₆)alkyl-Cy₀₃, a halogen atom, a cyano group, -C(O)-R₀₁₄, -C(O)-NR₀₁₄R₀₁₄',
R₀₁₁ and R₀₁₁' independently of one another are a hydrogen atom, an optionally substituted linear or branched (C₁-C₆)alkyl group, or -(C₀-C₆)alkyl-Cy₀₁, or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom is optionally substituted by a linear or branched (C₁-C₆)alkyl group, and wherein one or more of the carbon atoms of the linear or branched (C₁-C₆)alkyl group is optionally deuterated,
R₀₁₂ is -Cy₀₅, -Cy₀₅-(C₀-C₆)alkyl-Cy₀₆, -Cy₀₅-(C₀-C₆)alkyl-O-(C₀-C₆)alkyl-Cy₀₆, - Cy₀₅-(C₀-C₆)alkyl-NR₀₁₁-(C₀-C₆)alkyl-Cy₀₆, -Cy₀₅-Cy₀₆-O-(C₀-C₆)alkyl-Cy₀₇, -Cy₀₅-(C₀-C₆)alkyl-Cy₀₉, -NH-C(O)-NH-R₀₁₁, -C(O)-NR₀₁₁R₀₁₁', -NR₀₁₁R₀₁₁', -OR₀₁₁, -NR₀₁₁-C(O)-R₀₁₁', -O-(C₁-C₆)alkyl-OR₀₁₁, -SO₂-R₀₁₁, or -C(O)-OR₀₁₁,
R₀₁₃, R₀₁₃', R₀₁₄ and R₀₁₄' independently of one another are a hydrogen atom, or an optionally substituted linear or branched (C₁-C₆)alkyl group,
Cy₀₁, Cy₀₂, Cy₀₃, Cy₀₅, Cy₀₆, Cy₀₇, Cy₀₈, and Cy₀₁₀ independently of one another, are a cycloalkyl group, a heterocycloalkyl group, an aryl group or a heteroaryl group, each of which is optionally substituted,
Cy₀₉ is 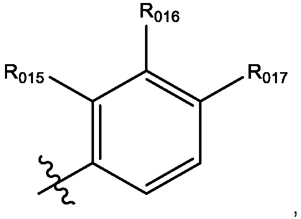
R₀₁₅ is a hydrogen atom; a -(CH₂)ₚ₀-O-(CHR₀₁₈-CHR₀₁₉-O)_{q0}-R₀₂₀ group; a linear or branched (C₁-C₆)alkoxy(C₁-C₆)alkyl group; a -U₀-(CH₂)_{q0}-NR₀₂₁R₀₂₁' group; or a -(CH₂)ᵣ₀-U₀-(CH₂)ₛ₀-heterocycloalkyl group,
R₀₁₆ is a hydrogen atom; a hydroxy group; a hydroxy(C₁-C₆)alkyl group; a - (CH₂)ᵣ₀-U₀-(CH₂)ₛ₀-heterocycloalkyl group; a (CH₂)ᵣ₀-U₀-V₀-O-P(O)(OR₀₂₀)₂ group; a -O-P(O)(O⁻M⁺)₂ group; a -O-S(O)₂OR₀₂₀ group; a -S(O)₂OR₀₂₀ group; a -(CH₂)ₚ₀-O-(CHR₀₁₈-CHR₀₁₉-O)_{q0}-R₀₂₀ group; a -(CH₂)ₚ₀-O-C(O)-NR₀₂₂R₀₂₃ group; or a -U₀-(CH₂)_{q0}-NR₀₂₁R₀₂₁' group,
R₀₁₇ is a hydrogen atom; a -(CH₂)ₚ₀-O-(CHR₀₁₈-CHR₀₁₉-O)_{q0}-R₀₂₀ group; a -CH₂-P(O)(OR₀₂₀)₂ group, a -O-P(O)(OR₀₂₀)₂ group; a -O-P(O)(O⁻M⁺)₂ group; a hydroxy group; a hydroxy(C₁-C₆)alkyl group; a -(CH₂)ᵣ₀-U₀-(CH₂)ₛ₀-heterocycloalkyl group;
a -U₀-(CH₂)_{q0}-NR₀₂₁R₀₂₁' group; or an aldonic acid,
M⁺ is a pharmaceutically acceptable monovalent cation,
U₀ is a bond or an oxygen atom,
V₀ is a -(CH₂)ₛ₀- group or a -C(O)- group,
R₀₁₈ is a hydrogen atom or a (C₁-C₆)alkoxy(C₁-C₆)alkyl group,
R₀₁₉ is a hydrogen atom or a hydroxy(C₁-C₆)alkyl group,
R₀₂₀ is a hydrogen atom or a linear or branched (C₁-C₆)alkyl group,
R₀₂₁ and R₀₂₁' independently of one are a hydrogen atom, a linear or branched (C₁-C₆)alkyl group, or a hydroxy(C₁-C₆)alkyl group,
or the pair (R₀₂₁, R₀₂₁') together with the nitrogen atom to which they are attached form an aromatic or non-aromatic ring containing 5 to 7 ring members,
R₀₂₂ is a (C₁-C₆)alkoxy(C₁-C₆)alkyl group, a -(CH₂)ₚ₀-NR₀₂₄R₀₂₄' group, or a -(CH₂)ₚ₀-O-(CHR₀₁₈-CHR₀₁₉-O)_{q0}-R₀₂₀ group,
R₀₂₃ is a hydrogen atom or a (C₁-C₆)alkoxy(C₁-C₆)alkyl group,
or the pair (R₀₂₂, R₀₂₃) together with the nitrogen atom to which they are attached form an aromatic or non-aromatic ring containing 5 to 18 ring members, which optionally contains, in addition to the nitrogen atom, 1 to 5 heteroatoms selected from O, S and N, wherein the resulting ring is optionally substituted by a hydrogen atom, a linear or branched (C₁-C₆)alkyl group or a heterocycloalkyl group,
R₀₂₄ and R₀₂₄' independently of one another are a hydrogen atom or a linear or branched (C₁-C₆)alkyl group,
or the pair (R₀₂₄, R₀₂₄') together with the nitrogen atom to which they are attached form an aromatic or non-aromatic ring composed of from 5 to 7 ring members, which may contain in addition to the nitrogen atom from 1 to 3 heteroatoms selected from O, S and N, and wherein the resulting ring is optionally substituted by a hydrogen atom or a linear or branched (C₁-C₆)alkyl group,
R₀₃₁ is 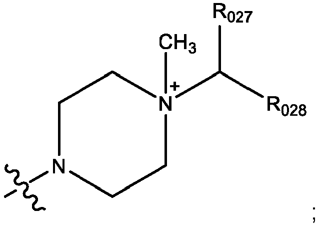
R₀₂₇ is a hydrogen atom or a linear or branched (C₁-C₆)alkyl group,
R₀₂₈ is a -O-P(O)(O⁻)(O⁻) group, a -O-P(O)(O⁻)(OR₀₃₀) group, a -O-P(O)(OR₀₃₀)(OR₀₃₀') group, a -(CH₂)ₚ₀-O-SO₂-O⁻ group, a -(CH₂)ₚ₀-SO₂-O⁻ group, a - (CH₂)ₚ₀-O-SO₂-OR₀₃₀ group, -Cy₀₁₀, a -(CH₂)ₚ₀-SO₂-OR₀₃₀ group, a -O-C(O)-R₀₂₉ group, a -O-C(O)-OR₀₂₉ group or a -O-C(O)-NR₀₂₉R₀₂₉' group;
R₀₂₉ and R₀₂₉' independently of one another are a hydrogen atom, a linear or branched (C₁-C₆)alkyl group or a linear or branched amino(C₁-C₆)alkyl group,
R₀₃₀ and R₀₃₀' independently of one another are a hydrogen atom, a linear or branched (C₁-C₆)alkyl group or an aryl(C₁-C₆)alkylgroup,
p₀ is an integer equal to 0, 1, 2, or 3,
q₀ is an integer equal to 1, 2, 3 or 4,
r₀ and s₀ are independently an integer equal to 0 or 1;
wherein one of the R₀₃, R₀₉, or R₀₁₂ groups is covalently attached to the linker,
L is a linker that covalently attaches Ab to D; and
p is an integer from 1 to 16.

2. The antibody-drug conjugate of claim 1, wherein p is an integer from 1 to 6 or from 2 to 4, or p is 2 or 4; or p is determined by liquid chromatography-mass spectrometry (LC-MS).

3. The antibody-drug conjugate of claim 1 or 2, wherein L comprises:
an attachment group;
at least one bridging spacer group; and
at least one cleavable group, optionally at least one cleavable group comprising a pyrophosphate group and/or a self-immolative group.

4. The antibody-drug conjugate of claim 3, wherein -(L-D) is of the formula (A): 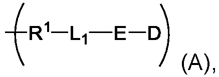 wherein:
R¹ is an attachment group;
L₁ is a bridging spacer group;
E is a cleavable group.

5. The antibody-drug conjugate of claim 3 or 4, wherein
(1) the cleavable group comprises a pyrophosphate group or the cleavable group comprises 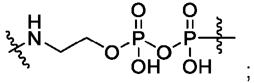
(2) the bridging spacer group comprises:
(i) a polyoxyethylene (PEG) group;
(ii) a PEG group selected from, PEG1, PEG2, PEG3, PEG4, PEG5, PEG6, PEG7, PEG8, PEG9, PEG10, PEG11, PEG12, PEG13, PEG14, and PEG15;
(iii) a -CO-CH₂-CH₂-PEG12- group;
(iv) a butanoyl, pentanoyl, hexanoyl, heptanoyl, or octanoyl group; or
(v) a hexanoyl group;
(3)
(i) the attachment group is formed from at least one reactive group selected from a maleimide group, thiol group, cyclooctyne group, and an azido group; optionally wherein:
a) the maleimide group has the structure: 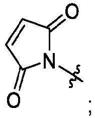
b) the azido group has the structure: -N=N⁺=N⁻;
c) the cyclooctyne group has the structure: 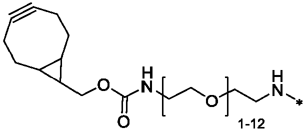 or 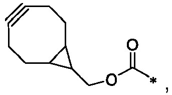 and wherein -* is a bond to the antibody or antigen-binding fragment; or
d) the cyclooctyne group has the structure: 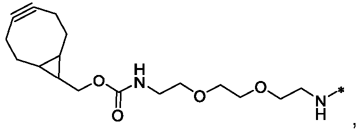 and wherein -* is a bond to the antibody or antigen-binding fragment; or
(ii) the attachment group has a formula comprising: 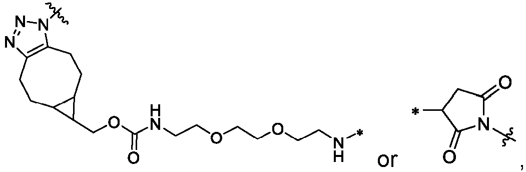 and
wherein -* is a bond to the antibody or antigen-binding fragment;
(4) the antibody or antigen-binding fragment is joined to the linker (L) by an attachment group selected from: 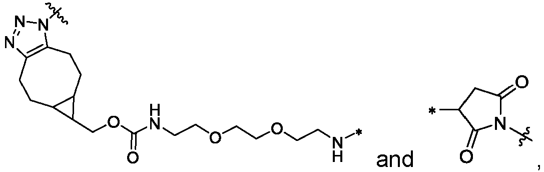 wherein -* is a bond to the antibody or antigen-binding fragment, and wherein 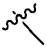 is a bond to the bridging spacer group;
(5) the bridging spacer group is -CO-CH₂-CH₂-PEG12-;
(6) the bridging spacer group is joined to a cleavable group; optionally the cleavable group is - pyrophosphate-CH₂-CH₂-NH₂-; or
(7) the cleavable group is joined to the Mcl-1 inhibitor (D), or the cleavable group is joined to the Mcl-1 inhibitor (D) through a phenyl-pyrimidinyl group.

6. The antibody-drug conjugate of any one of claims 1 to 3, wherein the linker comprises:
an attachment group,
at least one bridging spacer group,
a peptide group, and
at least one cleavable group.

7. The antibody-drug conjugate of claim 6, wherein -(L-D) is of the formula (B): 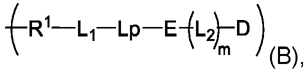 wherein:
R¹ is an attachment group;
L₁ is a bridging spacer;
Lp is a peptide group comprising 1 to 6 amino acid residues or Lp comprises a group 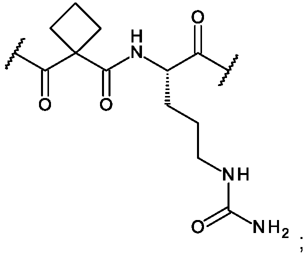
E is a cleavable group
L₂ is a bridging spacer;
m is 0 or 1; and
D is an Mcl-1 inhibitor as defined in claim 1.

8. The antibody-drug conjugate of claim 6 or 7, wherein
(1)
(i) the attachment group is formed from at least one reactive group comprising a maleimide group, thiol group, cyclooctyne group, and/or an azido group, optionally wherein:
a) the maleimide group has the structure: 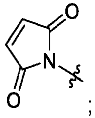
b) the azido group has the structure: -N=N⁺=N⁻;
c) the cyclooctyne group has the structure: 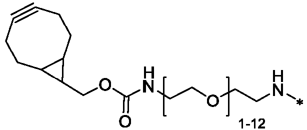 or 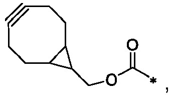 and wherein -* is a bond to the antibody or antigen-binding fragment; or
d) the cyclooctyne group has the structure: 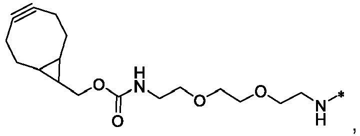 and wherein -* is a bond to the antibody or antigen-binding fragment; or
(ii) the attachment group has a formula comprising: 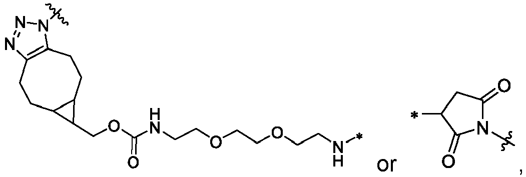 and
wherein -* is a bond to the antibody or antigen-binding fragment;
(2)
(i) at least one bridging spacer comprises a PEG group, optionally the PEG group is selected from, PEG1, PEG2, PEG3, PEG4, PEG5, PEG6, PEG7, PEG8, PEG9, PEG10, PEG11, PEG12, PEG13, PEG14, and PEG15; or
(ii) at least one bridging spacer is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, a polyhydroxyalkyl group, and *-C(O)-N(CH₃)-CH₂-CH₂-N(CH₃)-C(O)-**, wherein ** indicates the point of direct or indirect attachment of the at least one bridging spacer to the attachment group and * indicates the point of direct or indirect attachment of the at least one bridging spacer to the peptide group;
(3) L₁ is selected from *-C(O)-CH₂-CH₂-PEG1-**, *-C(O)-CH₂-PEG3-**, *-C(O)-CH₂-CH₂-PEG12**, *-NH-CH₂-CH₂-PEG1-**, and a polyhydroxyalkyl group, wherein ** indicates the point of direct or indirect attachment of L₁ to R¹ and * indicates the point of direct or indirect attachment of L₁ to Lp;
(4) m is 1 and L₂ is -C(O)-N(CH₃)-CH₂-CH₂-N(CH₃)-C(O)-;
(5)
(i) the peptide group comprises 1 to 6, 1 to 4, 1 to 3 or 1 to 2 amino acid residues, optionally the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
(ii) the peptide group comprises Val-Cit, Val-Ala, Val-Lys, and/or sulfo-Ala-Val-Ala;
(iii) the peptide group is selected from: 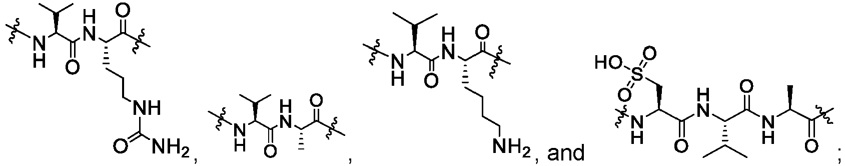
(6) (i) the cleavable group comprises a pyrophosphate and/or a self-immolative group; (ii) the cleavable group comprises a self-immolative group; or (iii) the cleavable group comprises a self-immolative group comprising para-aminobenzyl-carbamate, para-aminobenzyl-ammonium, para-amino-(sulfo)benzyl-ammonium, para-amino-(sulfo)benzyl-carbamate, para-amino-(alkoxy-PEG-alkyl)benzyl-carbamate, para-amino-(polyhydroxycarboxytetrahydropyranyl)alkyl-benzyl-carbamate, or para-amino-(polyhydroxycarboxytetrahydropyranyl)alkyl-benzyl-ammonium; or
(7) m is 0 or 1 or m is 1 and the bridging spacer comprises

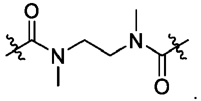

9. The antibody-drug conjugate of claim 7 or 8, wherein
(1) -(L-D) is formed from a compound selected from: 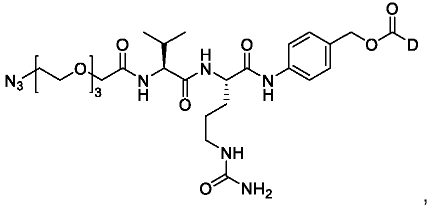 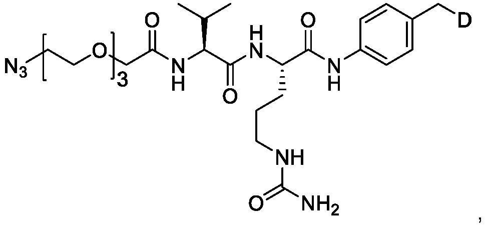 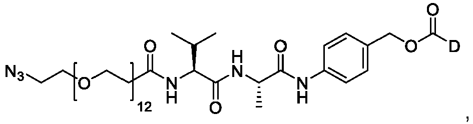 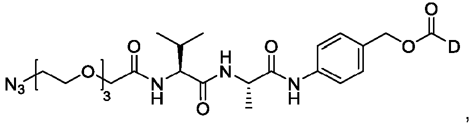 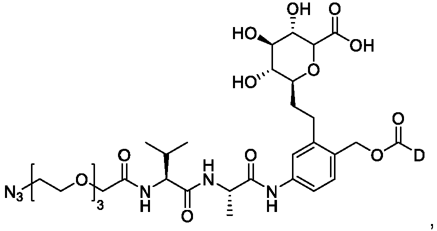 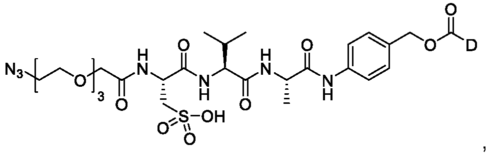 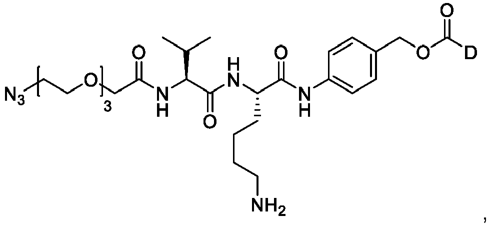 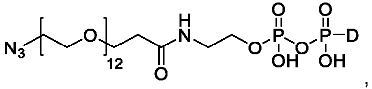 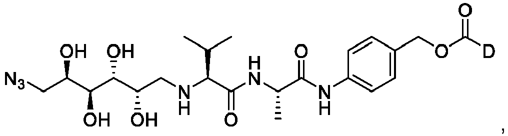 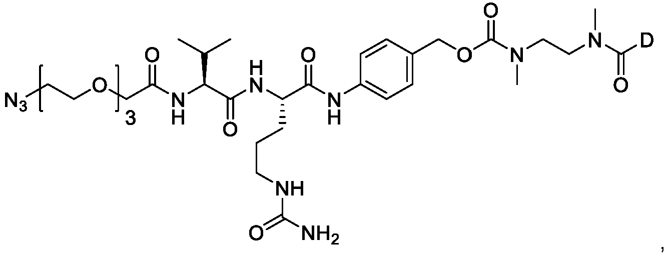 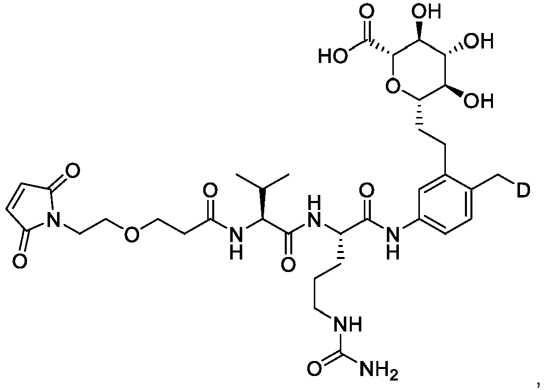 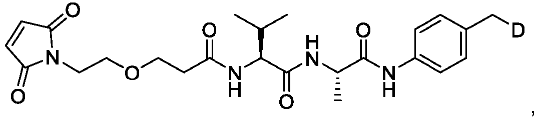 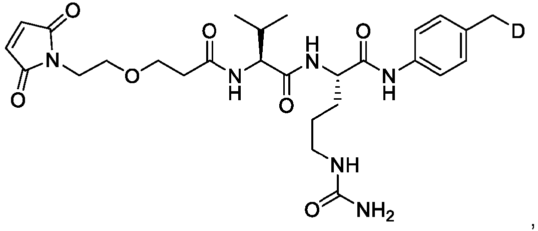 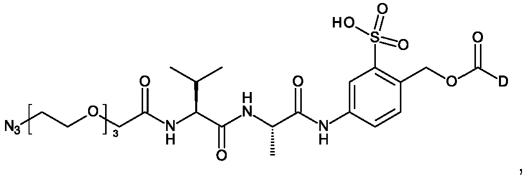 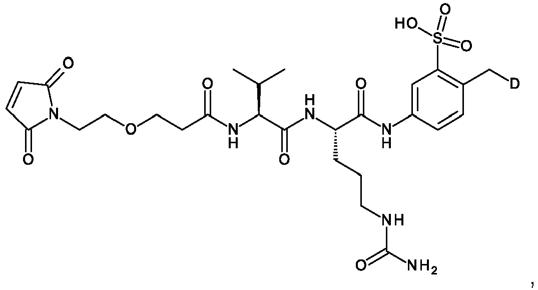 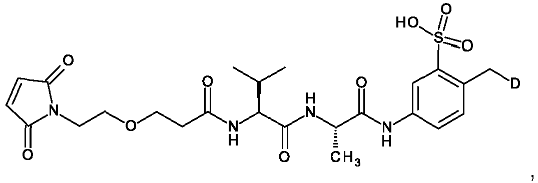 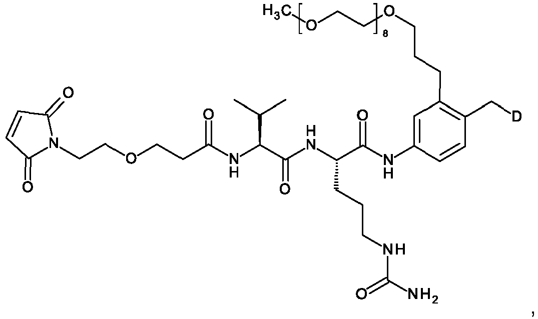 and 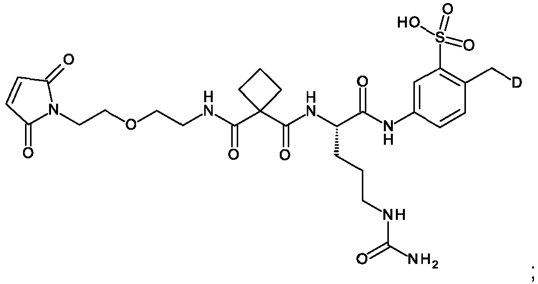 or
(2) -(L-D) comprises a formula selected from: 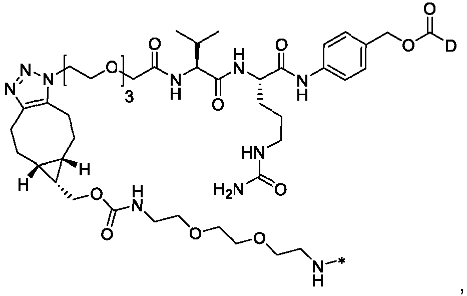 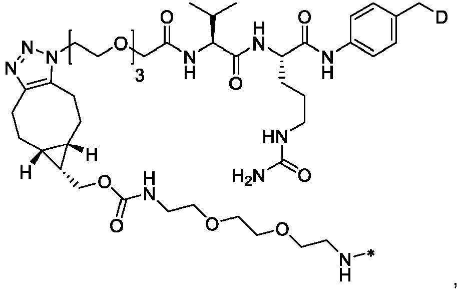 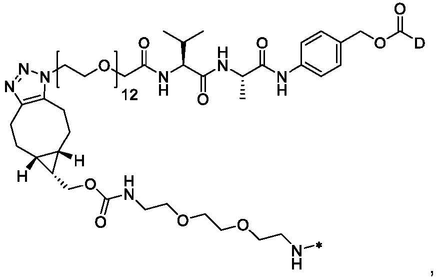 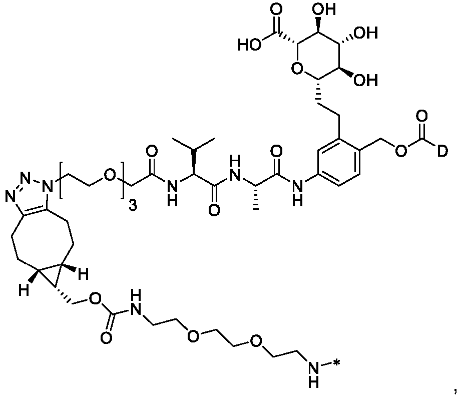 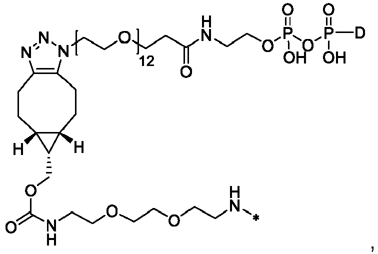 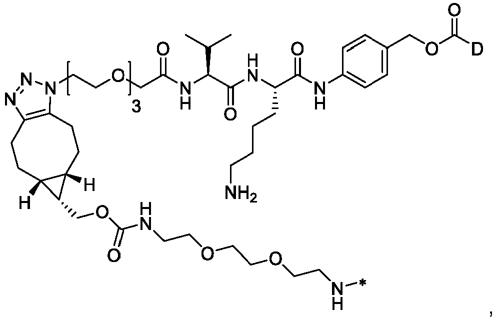 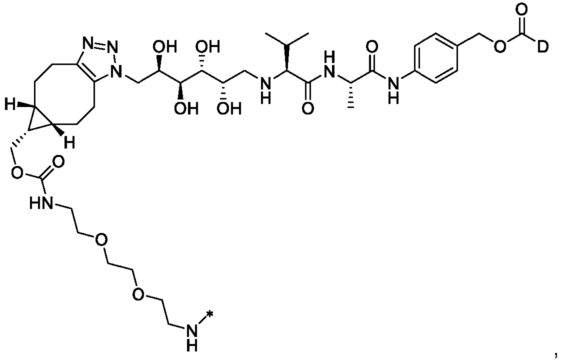 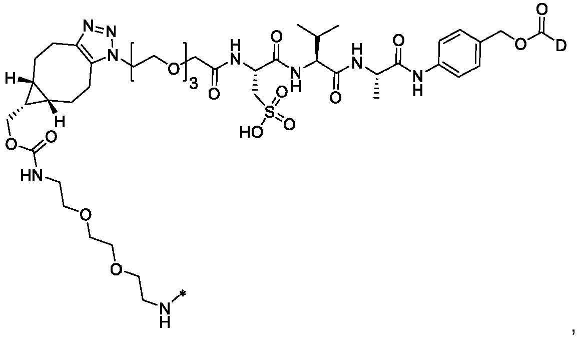 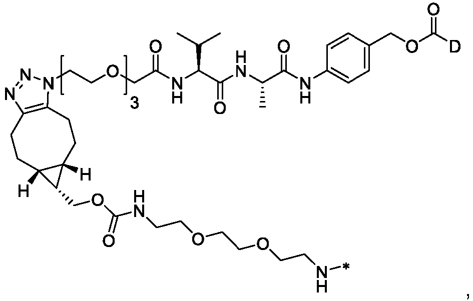 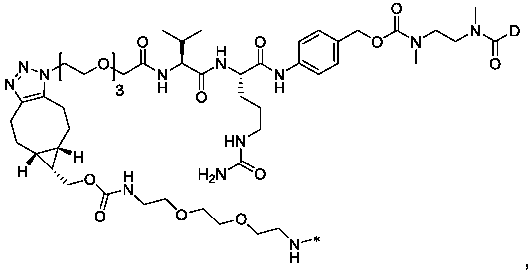 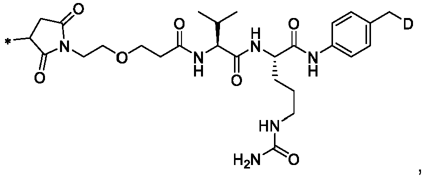 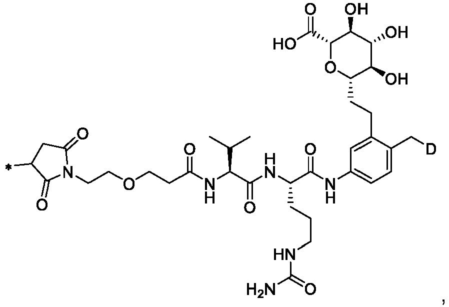 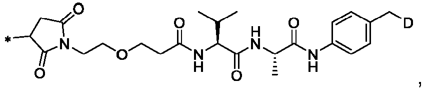 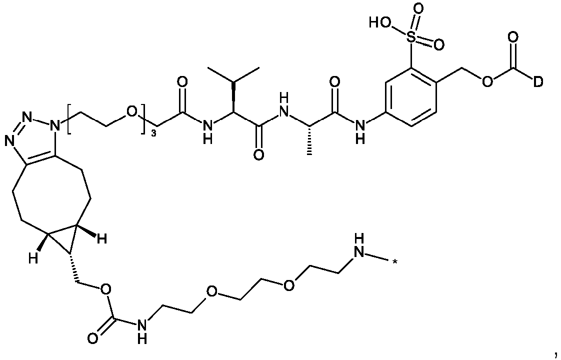 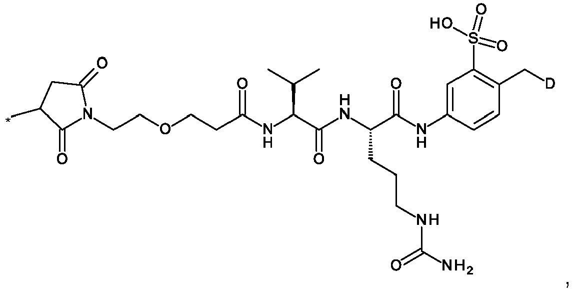 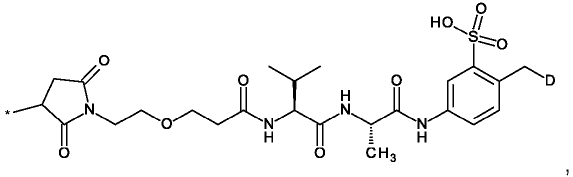 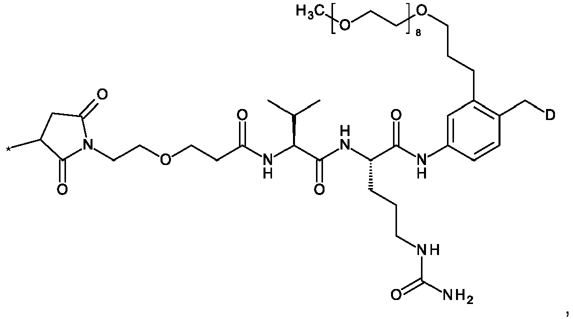 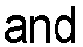 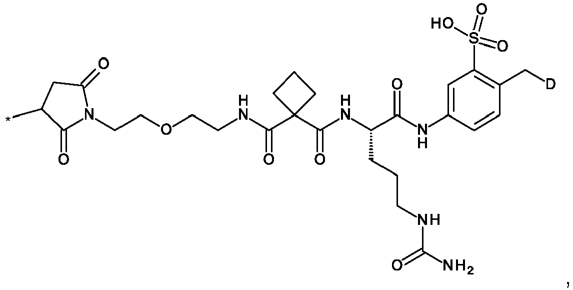 and
wherein -* is a bond to the antibody or antigen-binding fragment.

10. The antibody-drug conjugate of claim 1 or 2, wherein
(1) -(L-D) is of the formula (C): 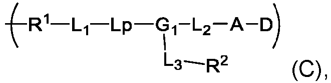 wherein:
R¹ is an attachment group;
L₁ is a bridging spacer;
Lₚ is a peptide group comprising 1 to 6 amino acids;
D is an Mcl-1 inhibitor as defined in claim 1;
G₁-L₂-A is a self-immolative spacer;
L₂ is a bond, a methylene, a neopentylene or a C₂-C₃ alkenylene;
A is a bond, -OC(=O)-*, 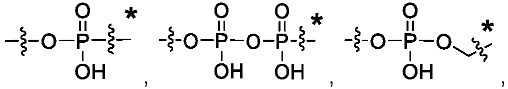 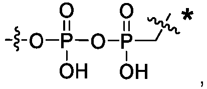 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D;
L₃ is a spacer moiety; and
R² is a hydrophilic moiety; or
(2) -(L-D) is of Formula (D): 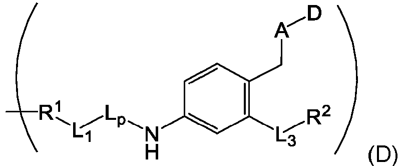 wherein:
R¹ is an attachment group;
L₁ is a bridging spacer;
Lp is a peptide group comprising 1 to 6 amino acids;
A is a bond, -OC(=O)-*, 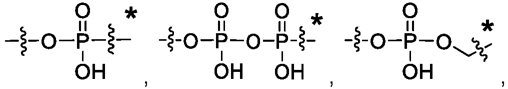 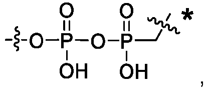 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D;
L₃ is a spacer moiety; and
R² is a hydrophilic moiety.

11. The antibody-drug conjugate of claim 10, wherein:
(1) L₁ comprises: 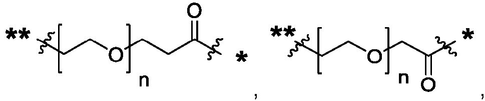 or
*-CH(OH)CH(OH)CH(OH)CH(OH)-**,
wherein each n is an integer from 1 to 12, wherein the * of L₁ indicates the point of direct or indirect attachment to Lp, and the ** of L₁ indicates the point of direct or indirect attachment to R¹;
(2) L₁ is 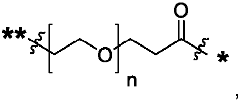 and n is an integer from 1 to 12 or n is 1 or n is 12, wherein the * of L₁ indicates the point of direct or indirect attachment to Lp, and the ** of L₁ indicates the point of direct or indirect attachment to R¹;
(3) L₁ is 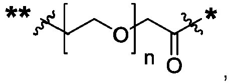 and n is an integer from 1 to 12, wherein the * of L₁ indicates the point of direct or indirect attachment to Lp, and the ** of L₁ indicates the point of direct or indirect attachment to R¹;
(4) L₁ comprises 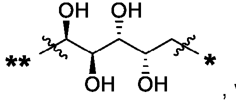 wherein the * of L₁ indicates the point of direct or indirect attachment to Lp, and the ** of L₁ indicates the point of direct or indirect attachment to R¹;
(5) L₁ is a bridging spacer comprising:
*-C(=O)(CH₂)ₘO(CH₂)ₘ-**; *-C(=O)((CH₂)ₘO)ₜ(CH₂)ₙ-**; *-C(=O)(CH₂)ₘ-**;
*-C(=O)NH((CH₂)ₘO)ₜ(CH₂)ₙ-**;
*-C(=O)O(CH₂)ₘSSC(R³)₂(CH₂)ₘC(=O)NR³(CH₂)ₘNR³C(=O)(CH₂)ₘ-**;
*-C(=O)O(CH₂)ₘC(=O)NH(CH₂)ₘ-**; *-C(=O)(CH₂)ₘNH(CH₂)ₘ-**;
*-C(=O)(CH₂)ₘNH(CH₂)ₙC(=O)-**; *-C(=O)(CH₂)ₘX₁(CH₂)ₘ-**;
*-C(=O)((CH₂)ₘO)ₜ(CH₂)ₙX₁(CH₂)ₙ-**; *-C(=O)(CH₂)ₘNHC(=O)(CH₂)ₙ-**;
*-C(=O)((CH₂)ₘO)ₜ(CH₂)ₙNHC(=O)(CH₂)ₙ-**;
*-C(=O)(CH₂)ₘNHC(=O)(CH₂)ₙX₁(CH₂)ₙ-**;
*-C(=O)((CH₂)ₘO)ₜ(CH₂)ₙNHC(=O)(CH₂)ₙX₁(CH₂)ₙ-**;
*-C(=O)((CH₂)ₘO)ₜ(CH₂)ₙC(=O)NH(CH₂)ₘ-**; *-C(=O)(CH₂)ₘC(R³)₂-**
or
*-C(=O)(CH₂)ₘC(=O)NH(CH₂)ₘ-**,
wherein the * of L₁ indicates the point of direct or indirect attachment to Lp, and the ** of L₁ indicates the point of direct or indirect attachment to R¹;
X₁ is 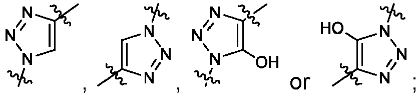 and
each m is independently selected from 1, 2, 3, 4, 5, 6, 7, 8, 9 and 10;
each n is independently selected from 1, 2, 3, 4, 5, 6, 7, 8, 9 and 10; and
each t is independently selected from 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30;
and each R³ is independently selected from H and C₁-C₆alkyl.

12. The antibody-drug conjugate of claim 10 or 11, wherein
(1) R² is a hydrophilic moiety comprising polyethylene glycol, polyalkylene glycol, a polyol, a polysarcosine, a sugar, an oligosaccharide, a polypeptide, or C₂-C₆ alkyl substituted with 1 to 3 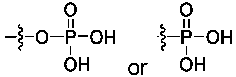 groups;
(2) R² is 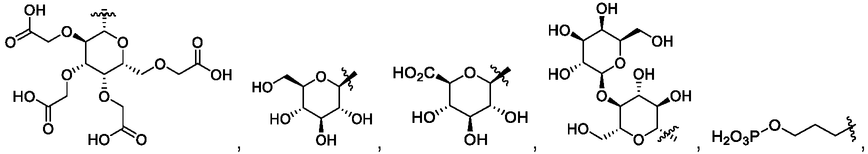 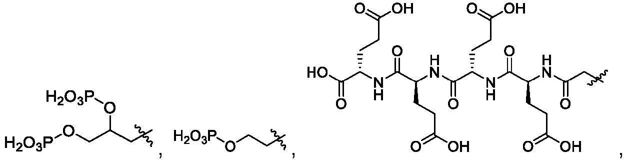 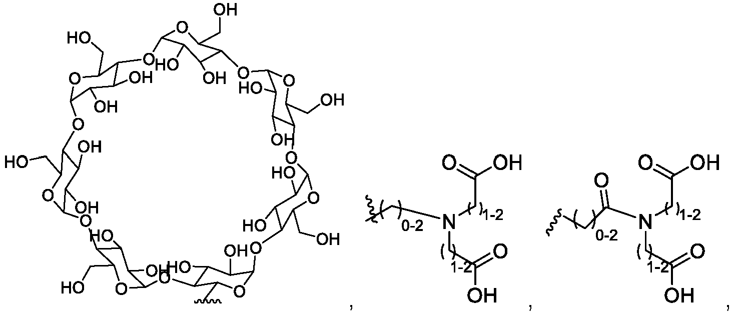 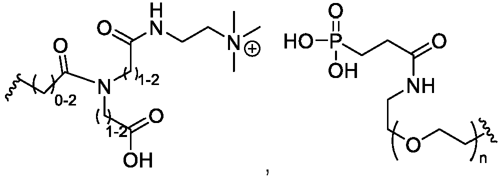 wherein n is an integer between 1 and 6, 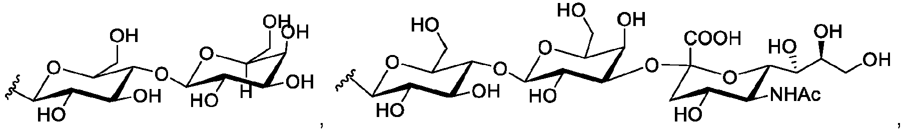 or 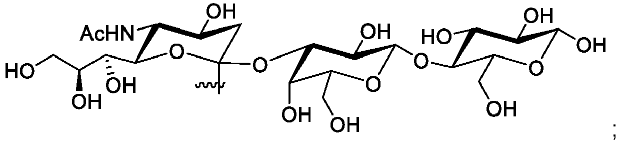
(3) the hydrophilic moiety represented by R² comprises:
(i) a polysarcosine, e.g., with the following moiety: 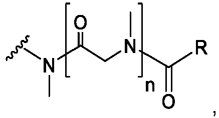 wherein n is an integer between 3 and 25; and R is H, -CH₃ or - CH₂CH₂C(=O)OH; or
(ii) a polyethylene glycol of formula: 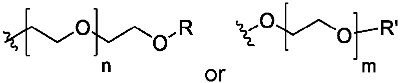 , wherein R is H, -CH₃, CH₂CH₂NHC(=O)ORₐ, -CH₂CH₂NHC(=O)Rₐ, or -CH₂CH₂C(=O)ORₐ, R' is OH, -OCH₃, -CH₂CH₂NHC(=O)ORₐ, -CH₂CH₂NHC(=O)Rₐ, or -OCH₂CH₂C(=O)ORₐ, in which Rₐ is H or C₁₋₄ alkyl optionally substituted with either OH or C₁₋₄ alkoxyl, and each of m and n is independently an integer between 2 and 25; or
(4) the hydrophilic moiety represented by R² comprises

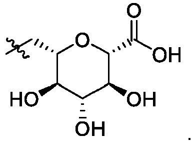

13. The antibody-drug conjugate of any one of claims 10 to 12, wherein:
(i) L₃ is a spacer moiety having the structure 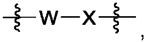 wherein:
W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, - CH₂N(X-R²)C(=O)-, -C(=O)NR^{b}-, -C(=O)NH-, -CH₂N R^{b} C(=O)-, -CH₂N R^{b} C(=O)NH-, - CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
X is a bond, triazolyl, or -CH₂-triazolyl-; or
(ii) L₃ is a spacer moiety having the structure 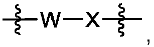 wherein:
W is -CH₂-, -CH₂O-, -CH₂N(R^{b})C(=O)O-, -NHC(=O)C(R^{b})₂NHC(=O)O-, -NHC(=O)C(R^{b})₂NH-, -NHC(=O)C(R^{b})₂NHC(=O)-, -CH₂N(X-R²)C(=O)O-, -C(=O)N(X-R²)-, -CH₂N(X-R²)C(=O)-, -C(=O)NR^{b}-, -C(=O)NH-, -CH₂NR^{b}C(=O)-, -CH₂NR^{b}C(=O)NH-, -CH₂NR^{b}C(=O)NR^{b}-, -NHC(=O)-, -NHC(=O)O-, -NHC(=O)NH-, -OC(=O)NH-, -S(O)₂NH-, -NHS(O)₂-, -C(=O)-, -C(=O)O- or -NH-, wherein each R^{b} is independently selected from H, C₁-C₆alkyl, and C₃-C₈ cycloalkyl; and
X is -CH₂-triazolyl-C₁₋₄ alkylene-OC(O)NHS(O)₂NH-, -C₄₋₆ cycloalkylene-OC(O)NHS(O)₂NH-, -(CH₂CH₂O)ₙ-C(O)NHS(O)₂NH-, -(CH₂CH₂O)ₙ-C(O)NHS(O)₂NH-(CH₂CH₂O)ₙ-, or -CH₂-triazolyl-C₁₋₄ alkylene-OC(O)NHS(O)₂NH-(CH₂CH₂O)ₙ-, wherein each n independently is 1, 2, or 3.

14. The antibody-drug conjugate of any one of claims 3 to 13, wherein
(1) the attachment group is formed by a reaction comprising at least one reactive group;
(2) the attachment group is formed by reacting:
a first reactive group that is attached to the linker, and
a second reactive group that is attached to the antibody or antigen-binding fragment or is an amino acid residue of the antibody or antigen-binding fragment, wherein optionally,
(i) at least one of the reactive groups comprises:
a thiol,
a maleimide,
a haloacetamide,
an azide,
an alkyne,
a cyclooctene,
a triaryl phosphine,
an oxanorbornadiene,
a cyclooctyne,
a diaryl tetrazine,
a monoaryl tetrazine,
a norbornene,
an aldehyde,
a hydroxylamine,
a hydrazine,
NH₂-NH-C(=O)-,
a ketone,
a vinyl sulfone,
an aziridine,
an amino acid residue, 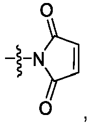 -ONH₂, -NH₂, 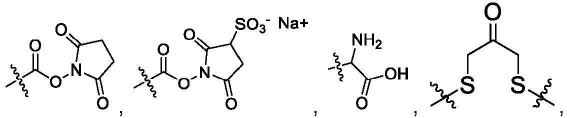 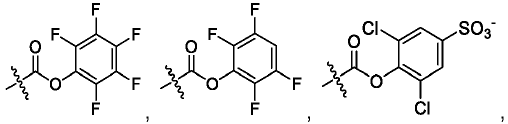 -N₃, 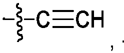 -SH, -SR³, -SSR⁴, -S(=O)₂(CH=CH₂), -(CH₂)₂S(=O)₂(CH=CH₂), -NHS(=O)₂(CH=CH₂), - NHC(=O)CH₂Br, -NHC(=O)CH₂I, 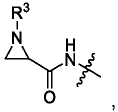 -C(O)NHNH₂, 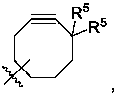 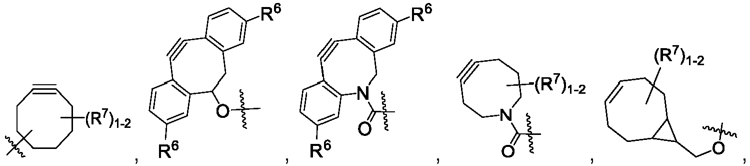 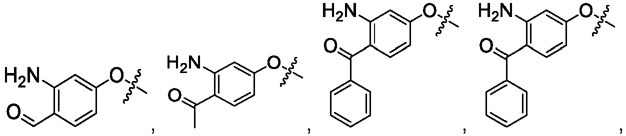 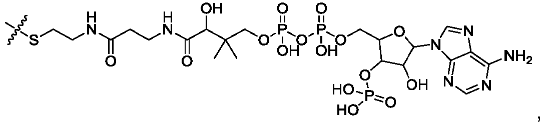 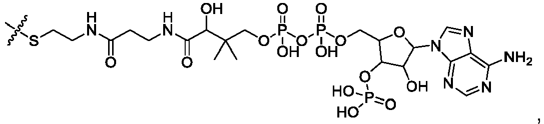 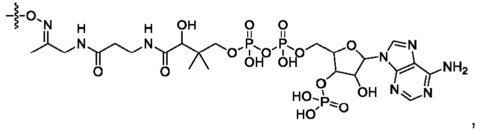 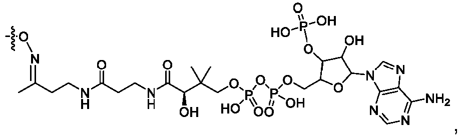 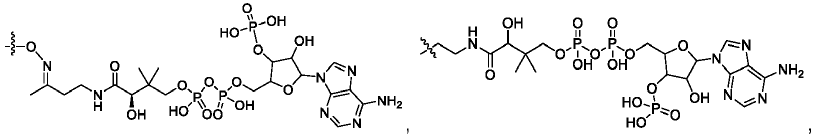 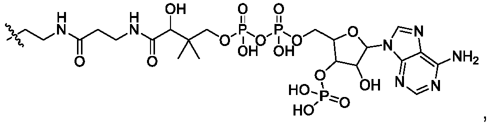 or 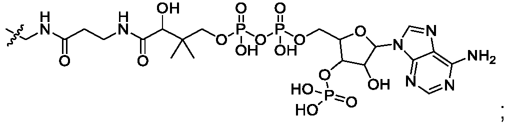 wherein:
each R³ is independently selected from H and C₁-C₆alkyl;
each R⁴ is 2-pyridyl or 4-pyridyl;
each R⁵ is independently selected from H, C₁-C₆alkyl, F, Cl, and -OH;
each R⁶ is independently selected from H, C₁-C₆alkyl, F, Cl, -NH₂, -OCH₃, - OCH₂CH₃, -N(CH₃)₂, -CN, -NO₂ and -OH;
each R⁷ is independently selected from H, C₁₋₆alkyl, fluoro, benzyloxy substituted with -C(=O)OH, benzyl substituted with -C(=O)OH, C₁₋₄alkoxy substituted with - C(=O)OH and C₁₋₄alkyl substituted with -C(=O)OH; and/or
(ii) the first reactive group and second reactive group comprise:
a thiol and a maleimide,
a thiol and a haloacetamide,
a thiol and a vinyl sulfone,
a thiol and an aziridine,
an azide and an alkyne,
an azide and a cyclooctyne,
an azide and a cyclooctene,
an azide and a triaryl phosphine,
an azide and an oxanorbornadiene,
a diaryl tetrazine and a cyclooctene,
a monoaryl tetrazine and a norbornene,
an aldehyde and a hydroxylamine,
an aldehyde and a hydrazine,
an aldehyde and NH₂-NH-C(=O)-,
a ketone and a hydroxylamine,
a ketone and a hydrazine,
a ketone and NH₂-NH-C(=O)-,
a hydroxylamine and 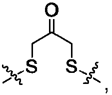
an amine and 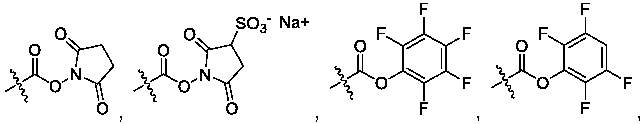 or 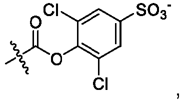 or
a CoA or CoA analogue and a serine residue; or
(3) the attachment group comprises a group selected from: 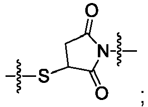 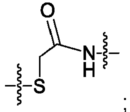 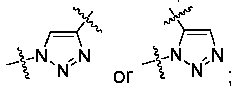 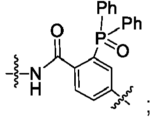 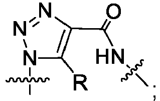 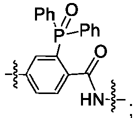 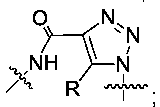 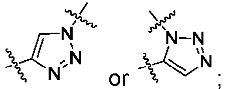 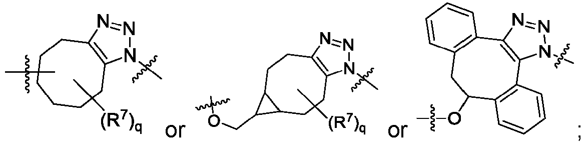 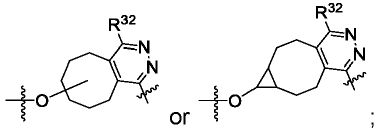 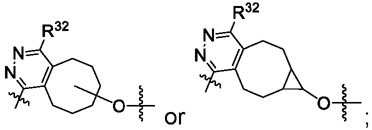 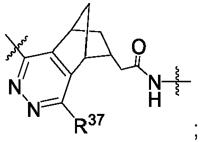 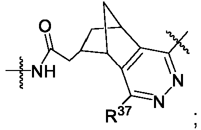 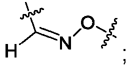 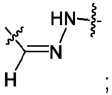 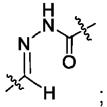 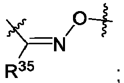 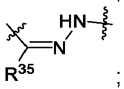 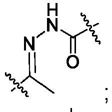 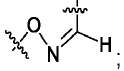 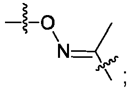 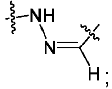 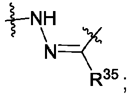 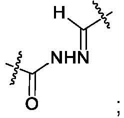 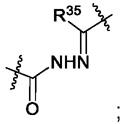 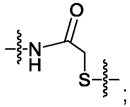 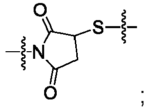 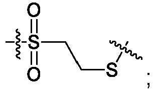 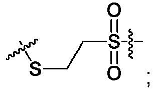 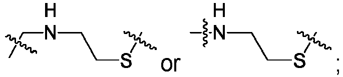 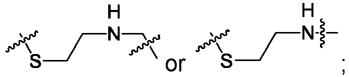 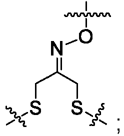 amide; 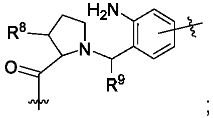 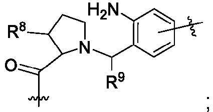 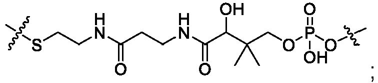 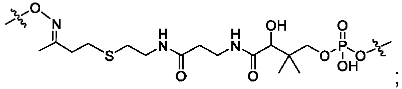 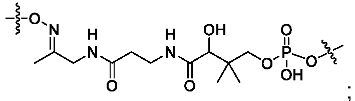 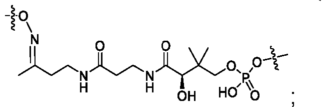 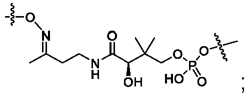 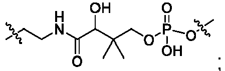 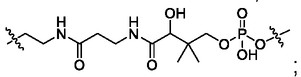 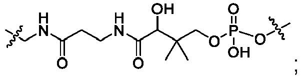 and
disulfide,
wherein:
R³² is H, C₁₋₄ alkyl, phenyl, pyrimidine or pyridine;
R³⁵ is H, C₁₋₆ alkyl, phenyl or C₁₋₄ alkyl substituted with 1 to 3 -OH groups;
each R⁷ is independently selected from H, C₁₋₆ alkyl, fluoro, benzyloxy substituted with - C(=O)OH, benzyl substituted with -C(=O)OH, C₁₋₄ alkoxy substituted with -C(=O)OH and C₁₋₄ alkyl substituted with -C(=O)OH;
R³⁷ is independently selected from H, phenyl and pyridine;
q is 0, 1, 2 or 3;
R⁸ is H or methyl; and
R⁹ is H, -CH₃ or phenyl.

15. The antibody-drug conjugate any one of claims 10 to 14, wherein
(1) the peptide group represented by Lp comprises 1 to 4 or 1 to 3 or 1 or 2 amino acid residues, optionally the amino acid residues are selected from L-glycine (Gly), L-valine (Val), L-citrulline (Cit), L-cysteic acid (sulfo-Ala), L-lysine (Lys), L-isoleucine (lle), L-phenylalanine (Phe), L-methionine (Met), L-asparagine (Asn), L-proline (Pro), L-alanine (Ala), L-leucine (Leu), L-tryptophan (Trp), and L-tyrosine (Tyr);
(2) the peptide group represented by Lp comprises Val-Cit, Phe-Lys, Val-Ala, Val-Lys, Leu-Cit, sulfo-Ala-Val, and/or sulfo-Ala-Val-Ala; or
(3) Lp is selected from:

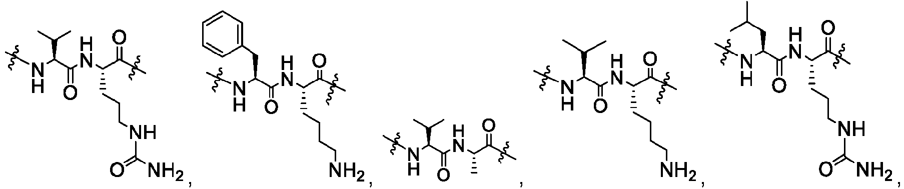

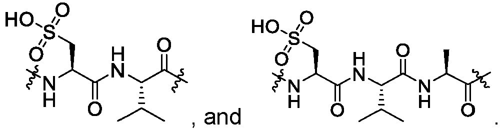

16. The antibody-drug conjugate of any one of claims 10 to 15, wherein:
-(L-D) comprises or is formed from a compound of formula:
(1) 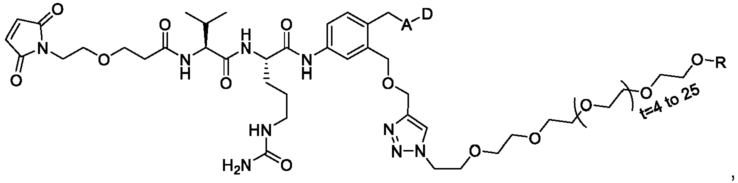 wherein:
R is H, -CH₃ or -CH₂CH₂C(=O)OH;
A is a bond, -OC(=O)-*, 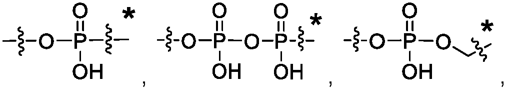 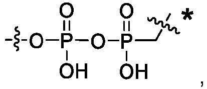 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(2) 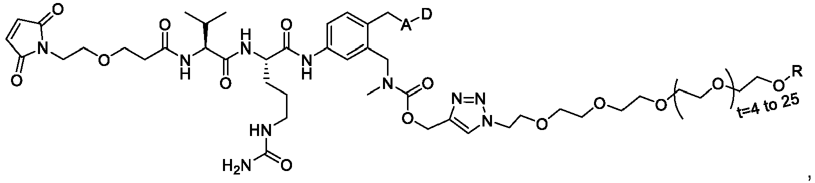 wherein:
R is H, -CH₃ or -CH₂CH₂C(=O)OH;
A is a bond, -OC(=O)-*, 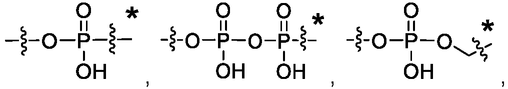 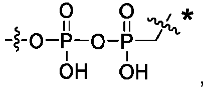 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(3) 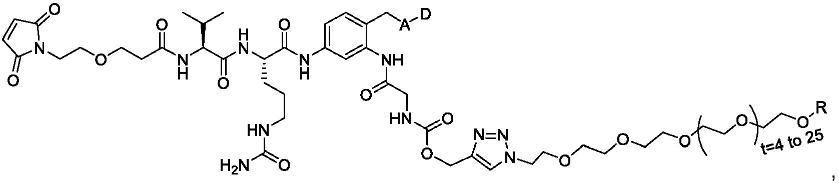 wherein:
R is H, -CH₃ or -CH₂CH₂C(=O)OH;
A is a bond, -OC(=O)-*, 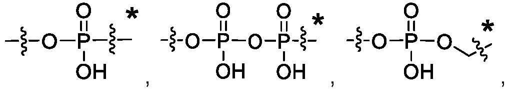 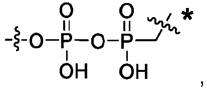 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(4) 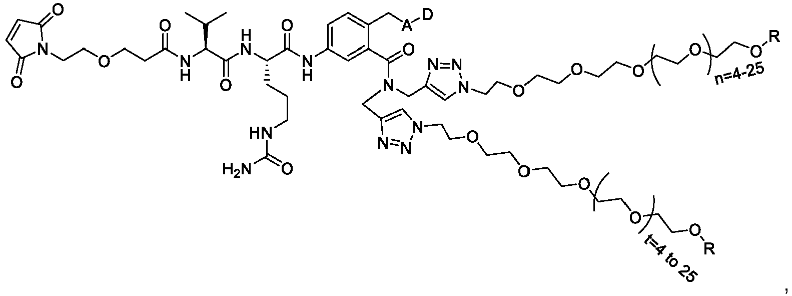 wherein:
each R is independently selected from H, -CH₃, and -CH₂CH₂C(=O)OH;
A is a bond, -OC(=O)-*, 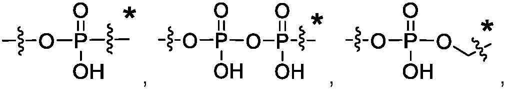 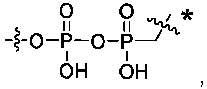 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(5) 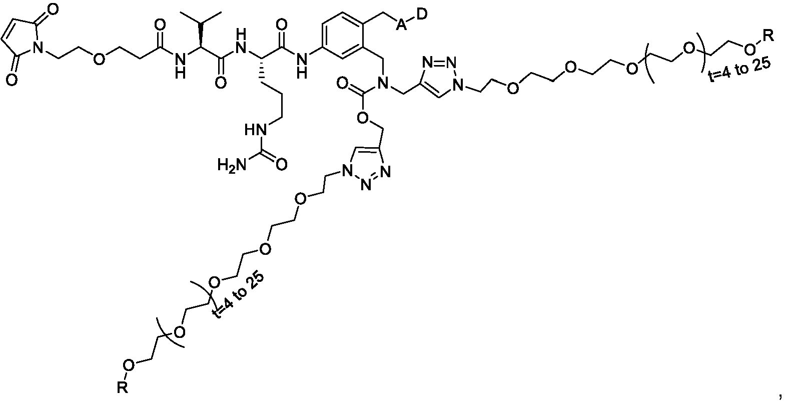 wherein:
each R is independently selected from H, -CH₃, and -CH₂CH₂C(=O)OH;
A is a bond, -OC(=O)-*, 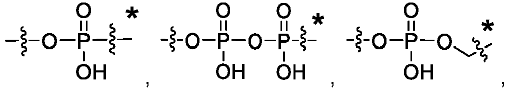 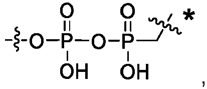 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(6) 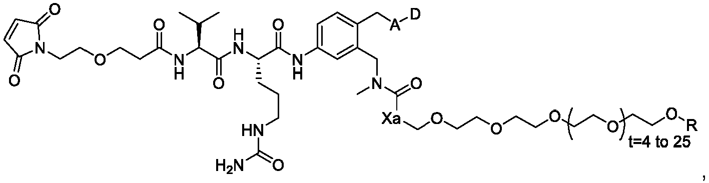 wherein:
Xa is -CH₂-, -OCH₂-, -NHCH₂- or -NRCH₂- and each R independently is H, -CH₃ or - CH₂CH₂C(=O)OH;
A is a bond, -OC(=O)-*, 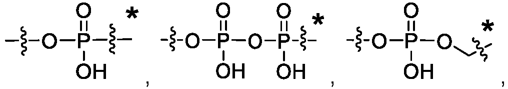 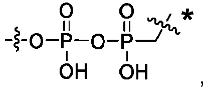 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(7) 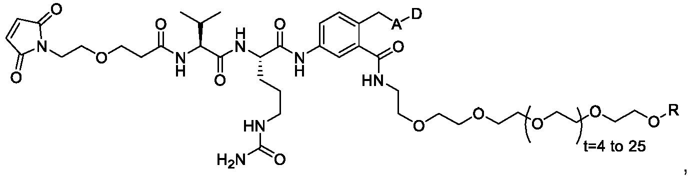 wherein:
R is H, -CH₃ or -CH₂CH₂C(=O)OH;
A is a bond, -OC(=O)-*, 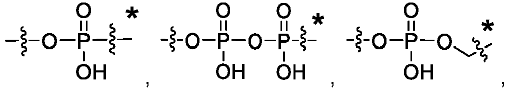 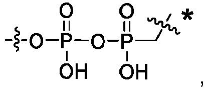 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(8) 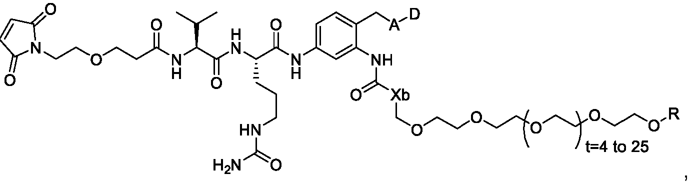 wherein:
Xb is -CH₂-, -OCH₂-, -NHCH₂- or -NRCH₂- and each R independently is H, -CH₃ or - CH₂CH₂C(=O)OH;
A is a bond, -OC(=O)-*, 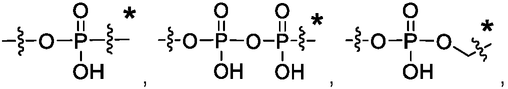 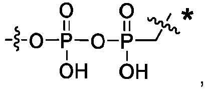 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(9) 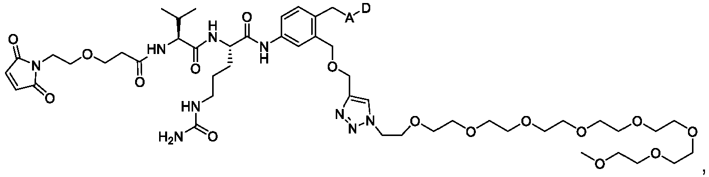 wherein:
A is a bond, -OC(=O)-*, 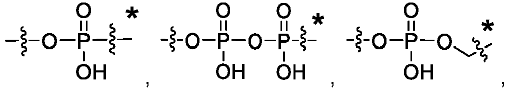 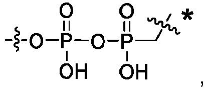 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(10) 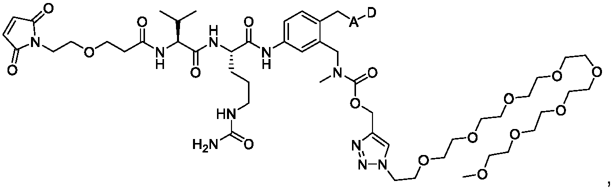 wherein:
A is a bond, -OC(=O)-*, 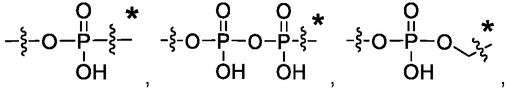 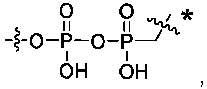 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(11) 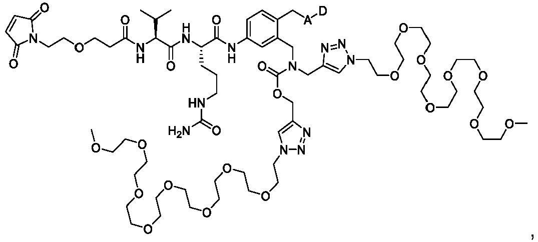 wherein:
A is a bond, -OC(=O)-*, 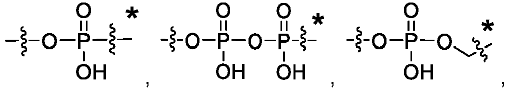 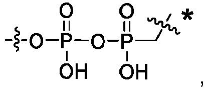 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(12) 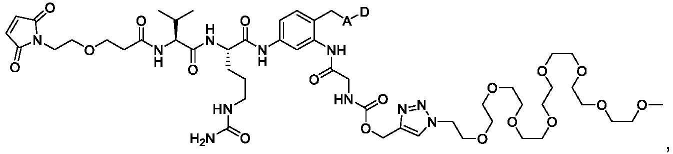 wherein:
A is a bond, -OC(=O)-*, 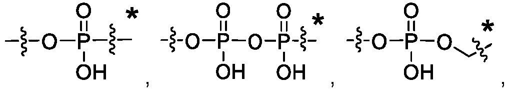 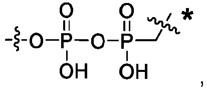 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(13) 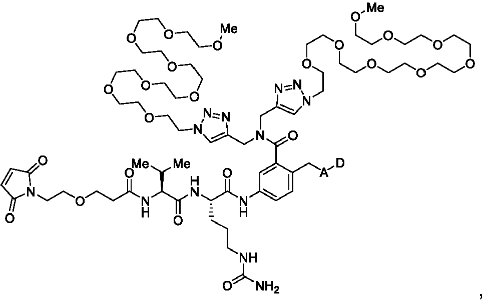 wherein:
A is a bond, -OC(=O)-*, 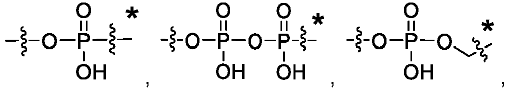 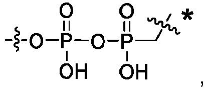 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(14) 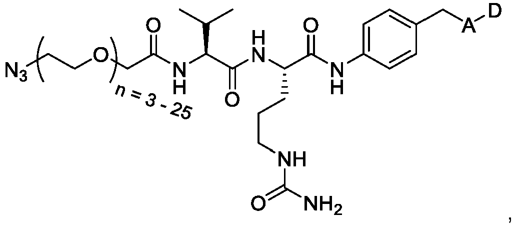 wherein:
A is a bond, -OC(=O)-*, 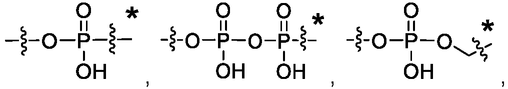 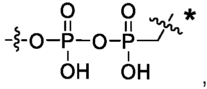 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1;
(15) 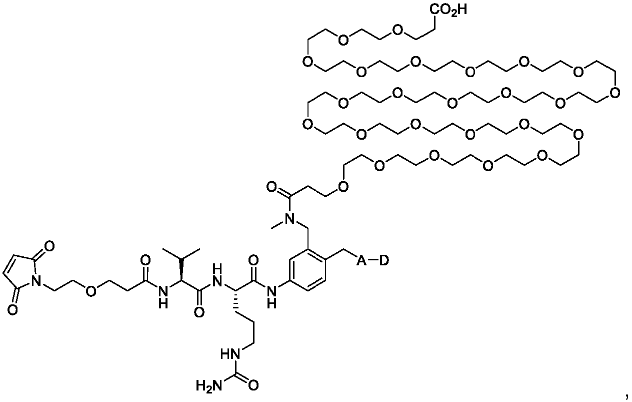 wherein:
A is a bond, -OC(=O)-*, 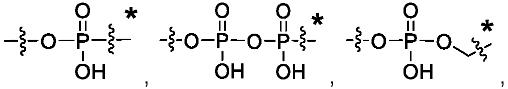 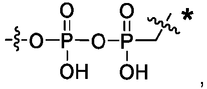 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1; or
(16) 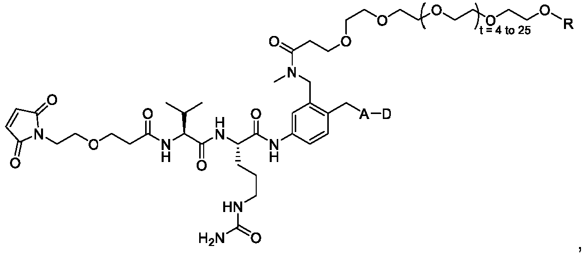 wherein:
each R independently is H, -CH₃ or -CH₂CH₂C(=O)OH;
A is a bond, -OC(=O)-*, 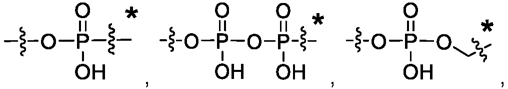 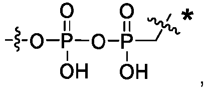 -OC(=O)N(CH₃)CH₂CH₂N(CH₃)C(=O)-* or -OC(=O)N(CH₃)C(R^{a})₂C(R^{a})₂N(CH₃)C(=O)-*,
wherein each R^{a} is independently selected from H, C₁-C₆ alkyl, and C₃-C₈ cycloalkyl and the * of A indicates the point of attachment to D; and
D is an Mcl-1 inhibitor as defined in claim 1.

17. The antibody-drug conjugate of any one of claims 10 to 16, wherein A is a bond and/or R is -CH₃.

18. The antibody-drug conjugate of any one of claims 1 to 17, wherein D comprises a compound of Formula (III): 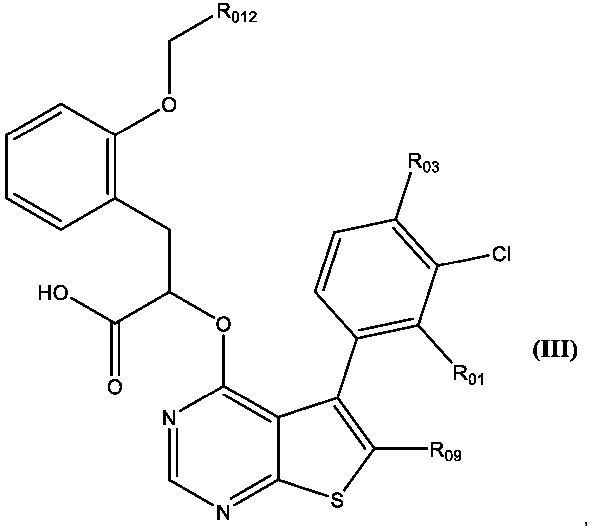
or the enantiomer, diastereoisomer, atropisomer, deuterated derivative, and/or pharmaceutically acceptable salt thereof;
wherein:
R₀₁ is a linear or branched (C₁-C₆)alkyl group,
R₀₃ is -O-(C₁-C₆)alkyl-NR₀₁₁R₀₁₁', or 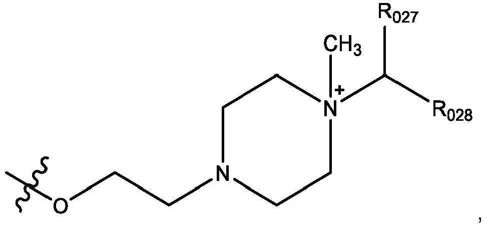
wherein R₀₁₁ and R₀₁₁' independently of one another are a hydrogen atom, an optionally substituted linear or branched (C₁-C₆)alkyl group, or -(C₀-C₆)alkyl-Cy₀₁;
or the pair (R₀₁₁, R₀₁₁') together with the nitrogen atom to which they are attached form an aromatic or non-aromatic ring containing 5 to 7 ring members, which optionally contains, in addition to the nitrogen atom, 1 to 3 heteroatoms selected from O, S and N, wherein the N atom may be substituted by 1 or 2 groups selected from a hydrogen atom or a linear or branched (C₁-C₆)alkyl group,
and wherein R₀₂₇ is a hydrogen atom and R₀₂₈ is a -(CH₂)ₚ₀-O-SO₂-O⁻ group or a -(CH₂)ₚ₀-SO₂-OR₀₃₀ group;
R₀₉ is a linear or branched (C₂-C₆)alkynyl group or -Cy₀₂,
R₀₁₂ is -Cy₀₅, -Cy₀₅-(C₀-C₆)alkyl-Cy₀₆, or -Cy₀₅-(C₀-C₆)alkyl-Cy₀₉,
Cy₀₁, Cy₀₂, Cy₀₅ and Cy₀₆ independently of one another, are a cycloalkyl group, a heterocycloalkyl group, an aryl group or a heteroaryl group, each of which is optionally substituted,
Cy₀₅ is 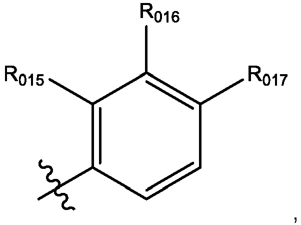
R₀₁₅, R₀₁₆, and R₀₁₇ are as defined for formula (II),
wherein one of the R₀₃, R₀₉, or R₀₁₂ groups is covalently attached to the linker.

19. The antibody-drug conjugate of claim 18, wherein
(1) R₀₁ is methyl or ethyl;
(2) R₀₃ is -O-CH₂-CH₂-NR₀₁₁R₀₁₁' in which R₀₁₁ and R₀₁₁' form, together with the nitrogen atom carrying them, a piperazinyl group which may be substituted by a group being a hydrogen atom or a linear or branched (C₁-C₆)alkyl group;
(3) R₀₃ comprises the formula: 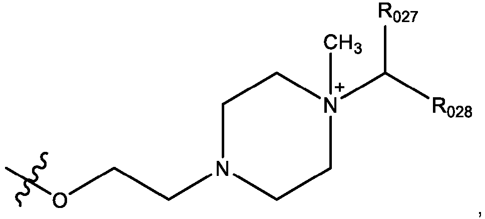 wherein R₀₂₇ is a hydrogen atom and R₀₂₈ is a -(CH₂)ₚ₀-SO₂-OR₀₃₀ group;
(4) R₀₃ comprises the formula: 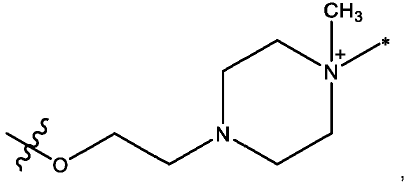 wherein -* is a bond to the linker;
(5) R₀₉ is Cy₀₂;
(6) Cy₀₂ is an optionally substituted aryl group;
(7) Cy₀₅ comprises a heteroaryl group selected from a pyrazolyl group and a pyrimidinyl group;
(8) Cy₀₅ is a pyrimidinyl group; or
(9) the L is attached to D by a covalent bond from L to R₀₃ of formula (II), or (III); and/or the L is attached to D by a covalent bond from L to R₀₉ of formula (II), or (III).

20. The antibody-drug conjugate of any one of claims 1 to 18, wherein:
(1) D comprises: 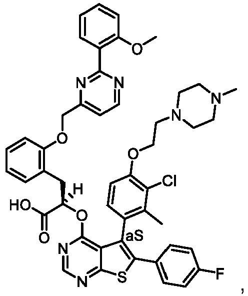 or an enantiomer, diastereoisomer, atropisomer, deuterated derivative, and/or pharmaceutically acceptable salt thereof;
(2) D comprises: 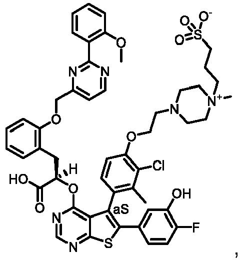 or an enantiomer, diastereoisomer, atropisomer, deuterated derivative, and/or pharmaceutically acceptable salt thereof;
(3) D comprises: 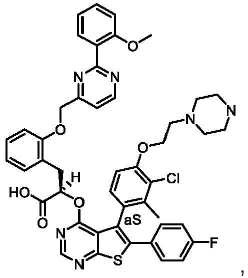 or an enantiomer, diastereoisomer, atropisomer, deuterated derivative, and/or pharmaceutically acceptable salt thereof;
(4) D comprises: 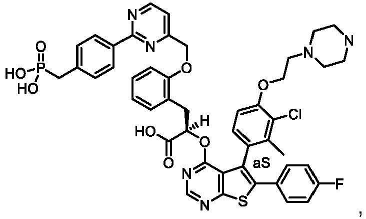 or an enantiomer, diastereoisomer, atropisomer, deuterated derivative, and/or pharmaceutically acceptable salt thereof;
(5) D comprises: 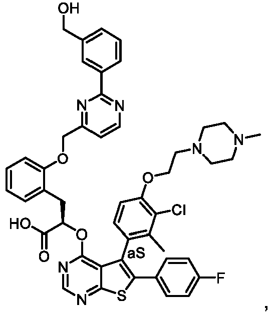 or an enantiomer, diastereoisomer, atropisomer, deuterated derivative, and/or pharmaceutically acceptable salt thereof;
(6) D comprises: 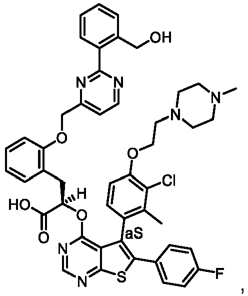 or an enantiomer, diastereoisomer, atropisomer, deuterated derivative, and/or pharmaceutically acceptable salt thereof;
(7) D comprises: 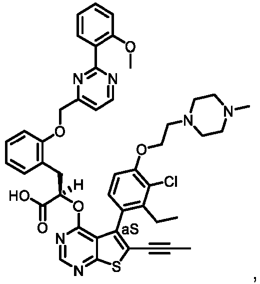 or an enantiomer, diastereoisomer, atropisomer, deuterated derivative, and/or pharmaceutically acceptable salt thereof;
(8) D comprises: 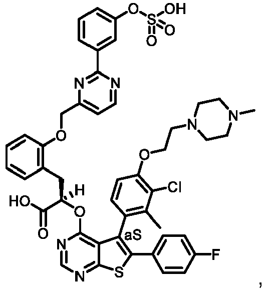 or an enantiomer, diastereoisomer, atropisomer, deuterated derivative, and/or pharmaceutically acceptable salt thereof;
(9) D comprises: 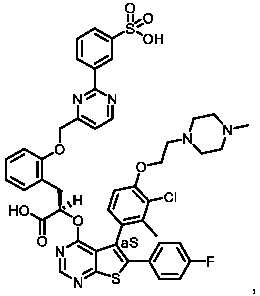 or an enantiomer, diastereoisomer, atropisomer, deuterated derivative, and/or pharmaceutically acceptable salt thereof;
(10) D comprises: 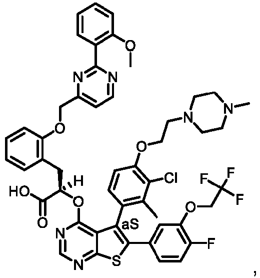 or an enantiomer, diastereoisomer, atropisomer, deuterated derivative, and/or pharmaceutically acceptable salt thereof;
(11) D comprises: 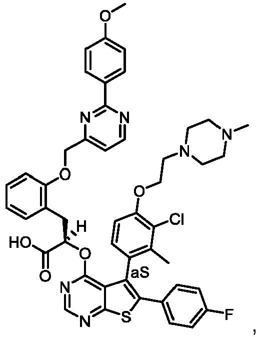 or an enantiomer, diastereoisomer, atropisomer, deuterated derivative, and/or pharmaceutically acceptable salt thereof;
(12) D comprises: 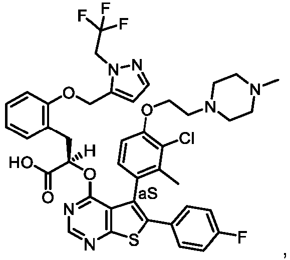 or an enantiomer, diastereoisomer, atropisomer, deuterated derivative, and/or pharmaceutically acceptable salt thereof;
(13) D comprises: 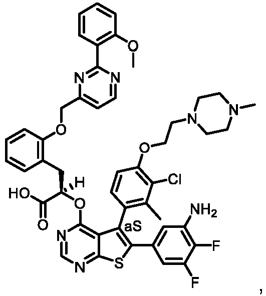 or an enantiomer, diastereoisomer, atropisomer, deuterated derivative, and/or pharmaceutically acceptable salt thereof; or
(14) D comprises: 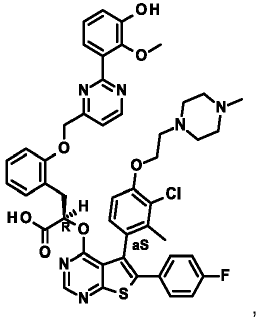 or an enantiomer, diastereoisomer, atropisomer, deuterated derivative, and/or pharmaceutically acceptable salt thereof.

21. The antibody-drug conjugate any one of claims 1 to 18, wherein -(L-D) is formed from a compound in Table A or an enantiomer, diastereoisomer, atropisomer, deuterated derivative, and/or pharmaceutically acceptable salt thereof.

22. The antibody-drug conjugate of any one of claims 1 to 21, wherein the antibody or antigen-binding fragment binds to a target antigen on the cancer cell; optionally wherein
(1)
(i) the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB;
(ii) the target antigen is BCMA, CD33, PCAD, HER2, CD38, CD46, CD48, or CD79b; or
(iii) the target antigen is BCMA, CD33, CD48, PCAD, or HER2;
(2) the antibody or antigen-binding fragment is an anti-BCMA antibody or antigen-binding fragment;
(3) the antibody or antigen-binding fragment comprises:
(a) three heavy chain complementarity determining regions (HCDRs) comprising amino acid sequences of SEQ ID NO:15 (HCDR1), SEQ ID NO:16 (HCDR2), and SEQ ID NO:17 (HCDR3); and three light chain complementarity determining regions (LCDRs) comprising amino acid sequences of SEQ ID NO:18 (LCDR1), SEQ ID NO:19 (LCDR2), and SEQ ID NO:20 (LCDR3); or
(b) the antibody or antigen-binding fragment comprises a heavy chain variable region comprising an amino acid sequence of SEQ ID NO:1, and a light chain variable region comprising an amino acid sequence of SEQ ID NO:2;
(4)
(a) the antibody or antigen-binding fragment comprises an IgG1 heavy chain constant domain or a modified IgG1 heavy chain constant domain, optionally the IgG1 heavy chain constant domain comprises a cysteine residue (C) at position 152 and position 375, or the IgG1 heavy chain constant domain comprises a cysteine residue (C) at position 156 and position 379; and/or
(b) the antibody or antigen-binding fragment comprises an Ig kappa light chain constant domain;
(5) the antibody or antigen-binding fragment is an anti-CD33 antibody or antigen-binding fragment;
(6)
(a) the antibody or antigen-binding fragment comprises three heavy chain complementarity determining regions (HCDRs) comprising amino acid sequences of SEQ ID NO:21 (HCDR1), SEQ ID NO:22 (HCDR2), and SEQ ID NO:23 (HCDR3); and three light chain complementarity determining regions (LCDRs) comprising amino acid sequences of SEQ ID NO:24 (LCDR1), SEQ ID NO:25 (LCDR2), and SEQ ID NO:26 (LCDR3); and/or
(b) the antibody or antigen-binding fragment comprises a heavy chain variable region comprising an amino acid sequence of SEQ ID NO:3, and a light chain variable region comprising an amino acid sequence of SEQ ID NO:4;
(7)
(a) the antibody or antigen-binding fragment comprises an IgG1 heavy chain constant domain or a modified IgG1 heavy chain constant domain, optionally the IgG1 heavy chain constant domain comprises a glutamine (Q) at position 297; and/or
(b) the antibody or antigen-binding fragment comprises an Ig kappa light chain constant domain;
(8) the antibody or antigen-binding fragment is an anti-PCAD antibody or antigen-binding fragment;
(9)
(a) the antibody or antigen-binding fragment comprises three heavy chain complementarity determining regions (HCDRs) comprising amino acid sequences of SEQ ID NO:33 (HCDR1), SEQ ID NO:34 (HCDR2), and SEQ ID NO:35 (HCDR3); and three light chain complementarity determining regions (LCDRs) comprising amino acid sequences of SEQ ID NO:36 (LCDR1), SEQ ID NO:37 (LCDR2), and SEQ ID NO:38 (LCDR3); and/or
(b) the antibody or antigen-binding fragment comprises a heavy chain variable region comprising an amino acid sequence of SEQ ID NO:7, and a light chain variable region comprising an amino acid sequence of SEQ ID NO:8;
(10) the antibody or antigen-binding fragment is an anti-HER2 antibody or antigen-binding fragment;
(11)
(a) the antibody or antigen-binding fragment comprises three heavy chain complementarity determining regions (HCDRs) comprising amino acid sequences of SEQ ID NO:39 (HCDR1), SEQ ID NO:40 (HCDR2), and SEQ ID NO:41 (HCDR3); and three light chain complementarity determining regions (LCDRs) comprising amino acid sequences of SEQ ID NO:42 (LCDR1), SEQ ID NO:43 (LCDR2), and SEQ ID NO:44 (LCDR3); and/or
(b) the antibody or antigen-binding fragment comprises a heavy chain variable region comprising an amino acid sequence of SEQ ID NO:9, and a light chain variable region comprising an amino acid sequence of SEQ ID NO:10;
(12)
(a) the antibody or antigen-binding fragment comprises an IgG1 heavy chain constant domain or a modified IgG1 heavy chain constant domain, optionally the IgG1 heavy chain constant domain comprises a glutamine (Q) at position 297 or the IgG1 heavy chain constant domain comprises a serine (S) at position 297; and/or
(b) the antibody or antigen-binding fragment comprises an Ig kappa light chain constant domain; or
(13) the antibody or antigen-binding fragment is an anti-CD38 antibody or antigen-binding fragment; an anti-CD46 antibody or antigen-binding fragment; an anti-CD48 antibody or antigen-binding fragment; or an anti-CD79b antibody or antigen-binding fragment.

23. A composition comprising multiple copies of the antibody-drug conjugate of any one of claims 1 to 22, wherein the average *p* of the antibody-drug conjugates in the composition is from about 2 to about 16, e.g., about 2 to about 8, e.g., about 2 to about 4.

24. A pharmaceutical composition comprising the antibody-drug conjugate of any one of claims 1 to 22 or the composition of claim 23, and a pharmaceutically acceptable carrier.

25. An antibody-drug conjugate of any one of claims 1 to 22, the composition of claim 23, or the pharmaceutical composition of claim 24 for use in treating a subject having or suspected of having a cancer, wherein
(1) the cancer expresses a target antigen; or
(2) the cancer is a tumor or a hematological cancer, optionally, the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer.

26. An antibody-drug conjugate of any one of claims 1 to 22, the composition of claim 23, or the pharmaceutical composition of claim 24 for use in reducing or inhibiting the growth of a tumor in a subject, wherein
(1) the tumor expresses a target antigen;
(2) the tumor is a breast cancer, gastric cancer, bladder cancer, brain cancer, cervical cancer, colorectal cancer, esophageal cancer, hepatocellular cancer, melanoma, oral cancer, ovarian cancer, non-small cell lung cancer, prostate cancer, small cell lung cancer, or spleen cancer; or
(3) administration of the antibody-drug conjugate, composition, or pharmaceutical composition reduces or inhibits the growth of the tumor by at least about 10%, at least about 20%, at least about 30%, at least about 40%, at least about 50%, at least about 60%, at least about 70%, at least about 80%, at least about 90%, at least about 95%, or at least about 99%.

27. An antibody-drug conjugate of any one of claims 1 to 22, the composition of claim 23, or the pharmaceutical composition of claim 24 for use in reducing or slowing the expansion of a cancer cell population in a subject, wherein
(1) the cancer cell population expresses a target antigen;
(2) the cancer cell population is from a tumor or a hematological cancer, optionally wherein the cancer cell population is from a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer; or
(3) administration of the antibody-drug conjugate, composition, or pharmaceutical composition reduces the cancer cell population or slows the expansion of the cancer cell population by at least about 10%, at least about 20%, at least about 30%, at least about 40%, at least about 50%, at least about 60%, at least about 70%, at least about 80%, at least about 90%, at least about 95%, or at least about 99%.

28. A method of determining whether a subject having or suspected of having a cancer will be responsive to treatment with the antibody-drug conjugate of any one of claims 1 to 22, the composition of claim 23, or the pharmaceutical composition of claim 24, comprising providing a biological sample from the subject; contacting the sample with the antibody-drug conjugate; and detecting binding of the antibody-drug conjugate to cancer cells in the sample; wherein
(1) the cancer cells in the sample express a target antigen;
(2) the cancer expresses a target antigen;
(3) the cancer is a tumor or a hematological cancer, optionally the cancer is a breast cancer, multiple myeloma, plasma cell myeloma, leukemia, lymphoma, gastric cancer, acute myeloid leukemia, bladder cancer, brain cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, prostate cancer, small cell lung cancer, or spleen cancer; or
(4) the sample is a tissue biopsy sample, a blood sample, or a bone marrow sample.

29. An antibody-drug conjugate of any one of claims 1 to 22, the composition of claim 23, or the pharmaceutical composition of claim 24 for use according to any one of claims 25 to 27, or the method of claim 28, wherein (i) the target antigen is BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, or GPNMB; (ii) the target antigen is BCMA, CD33, PCAD, HER2, CD38, CD46, CD48, or CD79b; or (iii) BCMA, CD33, CD48, PCAD, or HER2.

30. A method of producing the antibody-drug conjugate of any one of claims 1 to 22, comprising reacting an antibody or antigen-binding fragment with a cleavable linker joined to an MCL1 inhibitor under conditions that allow conjugation.

31. An antibody-drug conjugate of any one of claims 1 to 22, the composition of claim 23, or the pharmaceutical composition of claim 24 for use according to any one of claims 25 to 27, or the method of claim 28, further comprising administering to the subject in need thereof at least one additional therapeutic agent, preferably the one additional therapeutic agent is a Bcl-2 inhibitor, more preferably the one additional therapeutic agent is venetoclax, compound A1 or compound A2.

32. A compound having one of the following structures: 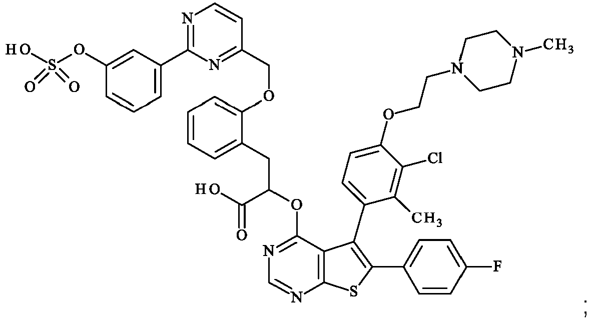 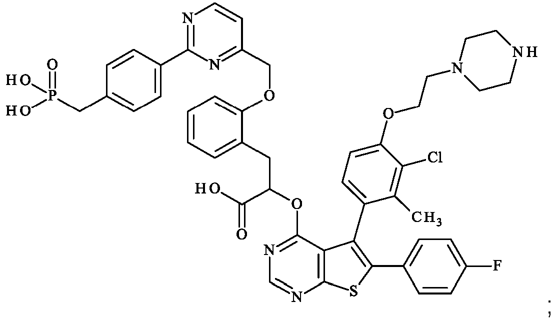 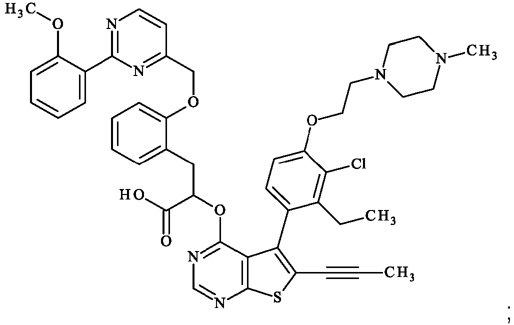 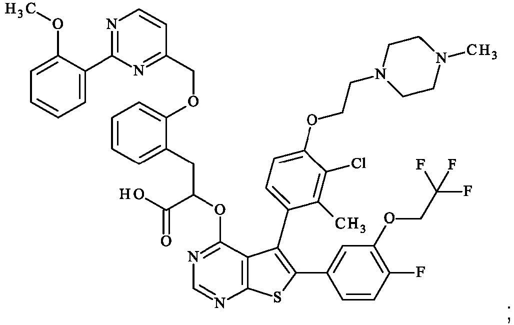 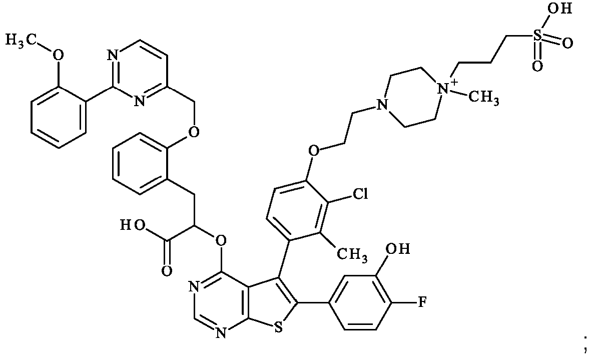 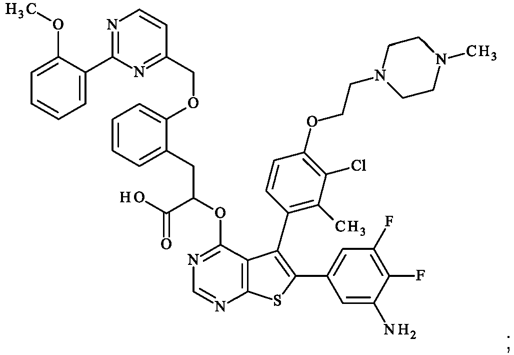 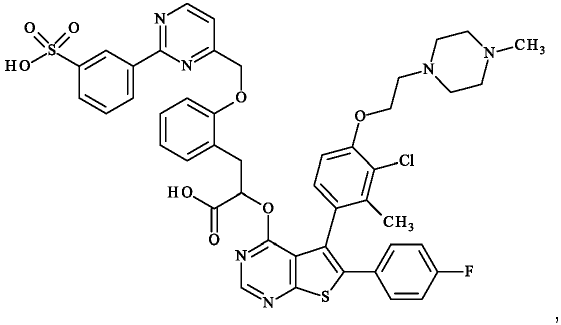 or 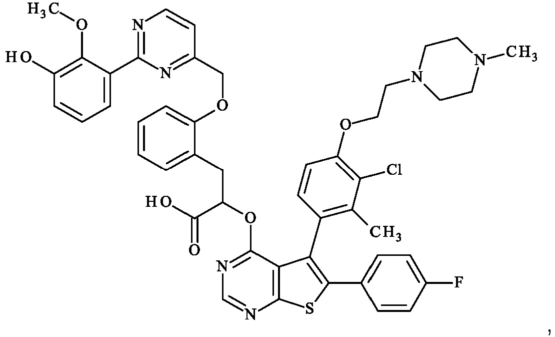 or a salt thereof (e.g., pharmaceutically acceptable salt thereof).

33. The compound of claim 32, wherein the compound is used as a synthesis intermediate for the preparation of an antibody-drug conjugate of Formula (1) according to claim 1.

34. The compound according to claim 32 or a salt thereof with a pharmaceutically acceptable acid or base, for use as a pro-apoptotic agent.

## Patentansprüche

1. Antikörper-Wirkstoff-Konjugat der Formel (1):
Ab-(L-D)*ₚ* (1)
wobei Ab ein Antikörper oder ein Antigenbindungsfragment davon ist;
D ein Mcl-1-Inhibitor ist, wobei D eine Verbindung der Formel (II) umfasst: 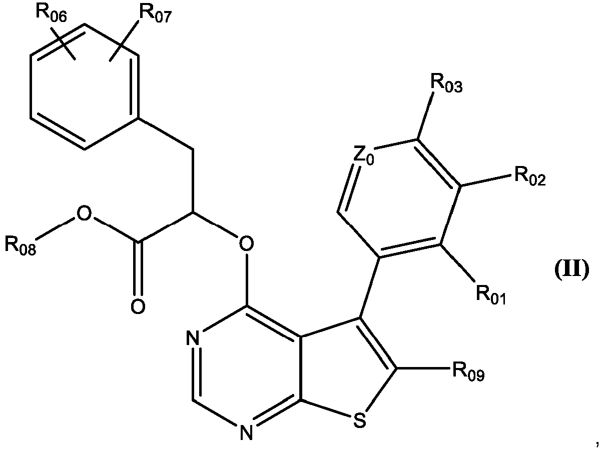
oder Enantiomer, Diastereoisomer, Atropisomer, deuteriertes Derivat und/oder pharmazeutisch annehmbares Salz davon;
wobei:
Z₀ ein Stickstoffatom oder eine C-R₀₄-Gruppe ist,
R₀₁ ein Halogenatom, eine lineare oder verzweigte (C₁-C₆)Alkylgruppe, eine lineare oder verzweigte (C₂-C₆)Alkenylgruppe, eine lineare oder verzweigte (C₂-C₆)Alkinylgruppe, eine lineare oder verzweigte (C₁-C₆)Haloalkylgruppe, eine Hydroxygruppe, eine lineare oder verzweigte (C₁-C₆)Alkoxygruppe, eine -S-(C₁-C₆)Alkylgruppe, eine Cyanogruppe, -Cy₀₈,-NR₀₁₁R₀₁₁' ist,
R₀₂, R₀₃ und R₀₄ unabhängig voneinander ein Wasserstoffatom, ein Halogenatom, eine lineare oder verzweigte (C₁-C₆)Alkylgruppe, eine lineare oder verzweigte (C₂-C₆)Alkenylgruppe, eine lineare oder verzweigte (C₂-C₆)Alkinylgruppe, ein lineares oder verzweigtes (C₁-C₆)Haloalkyl, eine Hydroxygruppe, eine lineare oder verzweigte (C₁-C₆)Alkoxygruppe, eine -S-(C₁-C₆)Alkylgruppe, eine Cyanogruppe, eine Nitrogruppe, -(C₀-C₆)Alkyl-NR₀₁₁R₀₁₁', -O-Cy₀₁,-(C₀-C₆)Alkyl-Cy₀₁, -(C₂-C₆)Alkenyl-Cy₀₁, -(C₂-C₆)Alkinyl-Cy₀₁, -O-(C₁-C₆)Alkyl-NR₀₁₁R₀₁₁',-O-(C₁-C₆)Alkyl-R₀₃₁, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)Alkyl-NR₀₁₁-C(O)-R₀₁₁', -SO₂-NR₀₁₁R₀₁₁' oder-SO₂-(C₁-C₆)Alkyl sind, oder das Paar (R₀₂, R₀₃) oder (R₀₃, R₀₄) zusammen mit den Kohlenstoffatomen, an die sie angeknüpft sind, einen aromatischen oder nichtaromatischen Ring bilden, der 5 bis 7 Ringelemente enthält, der optional 1 bis 3 Heteroatome ausgewählt aus O, S und N enthält, wobei der Ring optional substituiert ist durch eine Gruppe ausgewählt aus einem linearen oder verzweigten (C₁-C₆)Alkyl, -NR₀₁₃R₀₁₃', -(C₀-C₆)Alkyl-Cy₀₁ und Oxo,
R₀₆ und R₀₇ unabhängig voneinander ein Wasserstoffatom, ein Halogenatom, eine lineare oder verzweigte (C₁-C₆)Alkylgruppe, eine lineare oder verzweigte (C₂-C₆)Alkenylgruppe, eine lineare oder verzweigte (C₂-C₆)Alkinylgruppe, ein lineares oder verzweigtes (C₁-C₆)Haloalkyl, eine Hydroxygruppe, eine lineare oder verzweigte (C₁-C₆)Alkoxygruppe, eine -S-(C₁-C₆)Alkylgruppe, eine Cyanogruppe, eine Nitrogruppe, -(C₀-C₆)Alkyl-NR₀₁₁R₀₁₁', -O-Cy₀₁, -(C₀-C₆)Alkyl-Cy₀₁, -(C₂-C₆)Alkenyl-Cy₀₁, -(C₂-C₆)Alkinyl-Cy₀₁, -O-(C₁-C₆)Alkyl-R₀₁₂, -C(O)-OR₀₁₁, -O-C(O)-R₀₁₁, -C(O)-NR₀₁₁R₀₁₁', -NR₀₁₁-C(O)-R₀₁₁', -NR₀₁₁-C(O)-OR₀₁₁', -(C₁-C₆)Alkyl-NR₀₁₁-C(O)-R₀₁₁', -SO₂-NR₀₁₁R₀₁₁' oder -SO₂-(C₁-C₆)Alkyl sind, oder das Paar (R₀₆, R₀₇), wenn mit zwei benachbarten Kohlenstoffatomen kondensiert, zusammen mit den Kohlenstoffatomen, an die sie angeknüpft sind, einen aromatischen oder nichtaromatischen Ring bilden, der 5 bis 7 Ringelemente enthält, der optional 1 bis 3 Heteroatome ausgewählt aus O, S und N enthält, und wobei der resultierende Ring optional substituiert ist durch eine Gruppe ausgewählt aus einer linearen oder verzweigten (C₁-C₆)Alkylgruppe, -NR₀₁₃R₀₁₃', -(C₀-C₆)Alkyl-Cy₀₁ und einem Oxo,
R₀₈ ein Wasserstoffatom, eine lineare oder verzweigte (C₁-C₆)Alkylgruppe, eine Arylgruppe, eine Heteroarylgruppe, eine Aryl-(C₁-C₆)alkylgruppe oder eine Heteroaryl(C₁-C₆)alkylgruppe ist,
R₀₉ eine lineare oder verzweigte (C₁-C₆)Alkylgruppe, eine lineare oder verzweigte (C₂-C₆)Alkenylgruppe, eine lineare oder verzweigte (C₂-C₆)Alkinylgruppe, -Cy₀₂, -(C₁-C₆)Alkyl-Cy₀₂, -(C₂-C₆)Alkenyl-Cy₀₂, -(C₂-C₆)Alkinyl-Cy₀₂, -Cy₀₂-Cy₀₃, -(C₂-C₆)Alkinyl-O-Cy₀₂, -Cy₀₂-(C₀-C₆)Alkyl-O-(C₀-C₆)Alkyl-Cy₀₃, ein Halogenatom, eine Cyanogruppe, -C(O)-R₀₁₄, -C(O)-NR₀₁₄R₀₁₄' ist,
R₀₁₁ und R₀₁₁' unabhängig voneinander ein Wasserstoffatom, eine optional substituierte lineare oder verzweigte (C₁-C₆)Alkylgruppe oder -(C₀-C₆)Alkyl-Cy₀₁ sind, oder das Paar (R₀₁₁, R₀₁₁') zusammen mit dem Stickstoffatom, an das sie angeknüpft sind, einen aromatischen oder nichtaromatischen Ring bilden, der 5 bis 7 Ringelemente enthält, der optional zusätzlich zu dem Stickstoffatom 1 bis 3 Heteroatome ausgewählt aus O, S und N enthält, wobei das N-Atom optional substituiert ist durch eine lineare oder verzweigte (C₁-C₆)Alkylgruppe, und wobei eines oder mehrere der Kohlenstoffatome der linearen oder verzweigten (C₁-C₆)Alkylgruppe optional deuteriert ist,
R₀₁₂ -Cy₀₅, -Cy₀₅^{_}(C₀-C₆)Alkyl-Cy₀₆, -Cy₀₅-(C₀-C₆)Alkyl-O-(C₀-C₆)alkyl-Cy₀₆, -Cy₀₅-(C₀-C₆)Alkyl-NR₀₁₁-(C₀-C₆)alkyl-Cy₀₆, -Cy₀₅-Cy₀₆-O-(C₀-C₆)Alkyl-Cy₀₇, -Cy₀₅-(C₀-C₆)Alkyl-Cy₀₉,-NH-C(O)-NH-R₀₁₁, -C(O)-NR₀₁₁R₀₁₁', -NR₀₁₁R₀₁₁', -OR₀₁₁, -NR₀₁₁-C(O)-R₀₁₁', -O-(C₁-C₆)Alkyl-OR₀₁₁, -SO₂-R₀₁₁, oder -C(O)-OR₀₁₁ ist,
R₀₁₃, R₀₁₃', R₀₁₄ und R₀₁₄' unabhängig voneinander ein Wasserstoffatom oder eine optional substituierte lineare oder verzweigte (C₁-C₆)Alkylgruppe sind,
Cy₀₁, Cy₀₂, Cy₀₃, Cy₀₅, Cy₀₆, Cy₀₇, Cy₀₈ und Cy₀₁₀ unabhängig voneinander eine Cycloalkylgruppe, eine Heterocycloalkylgruppe, eine Arylgruppe oder eine Heteroarylgruppe sind, die jeweils optional substituiert ist,
Cy₀₉ wie folgt ist 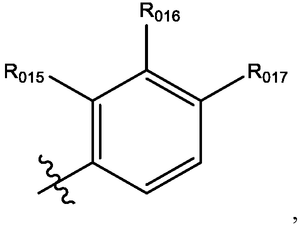
R₀₁₅ ein Wasserstoffatom; eine -(CH₂)ₚ₀-O-(CHR₀₁₈-CHR₀₁₉-O)_{q0}-R₀₂₀-Gruppe; eine lineare oder verzweigte (C₁-C₆)Alkoxy(C₁-C₆)alkylgruppe; eine -U₀-(CH₂)_{q0}-NR₀₂₁R₀₂₁'-Gruppe; oder eine -(CH₂)ᵣ₀-U₀-(CH₂)ₛ₀--Heterocycloalkylgruppe ist,
R₀₁₆ ein Wasserstoffatom; eine Hydroxygruppe; eine Hydroxy(C₁-C₆)alkylgruppe; eine-(CH₂)ᵣ₀-U₀-(CH₂)ₛ₀--Heterocycloalkylgruppe; eine (CH₂)ᵣ₀-U₀-V₀-O-P(O)(OR₀₂₀)₂-Gruppe; eine-O-P(O)(O⁻M⁺)₂-Gruppe; eine -O-S(O)₂OR₀₂₀-Gruppe; eine -S(O)₂OR₀₂₀-Gruppe; eine -(CH₂)ₚ₀-O-(CHR₀₁₈-CHR₀₁₉-O)_{q0}-R₀₂₀-Gruppe; eine -(CH₂)ₚ₀-O-C(O)-NR₀₂₂R₀₂₃-Gruppe; oder eine -U₀-(CH₂)_{q0}-NR₀₂₁R₀₂₁'-Gruppe ist,
R₀₁₇ ein Wasserstoffatom; eine -(CH2)ₚ₀-O-(CHR₀₁₈-CHR₀₁₉-O)_{q0}-R₀₂₀-Gruppe; eine-CH₂-P(O)(OR₀₂₀)₂-Gruppe, eine -O-P(O)(OR₀₂₀)₂-Gruppe; eine -O-P(O)(O⁻M⁺)₂-Gruppe; eine Hydroxygruppe; eine Hydroxy(C₁₋C₆)alkylgruppe; eine -(CH₂)ᵣ₀-U₀-(CH₂)ₛ₀-Heterocycloalkylgruppe; eine -U₀-(CH₂)_{q0}-NR₀₂₁R₀₂₁'-Gruppe; oder eine Aldonsäure ist,
M⁺ ein pharmazeutisch annehmbares einwertiges Kation ist,
U₀ eine Bindung oder ein Sauerstoffatom ist,
V₀ eine -(CH₂)ₛ₀--Gruppe oder eine -C(O)--Gruppe ist,
R₀₁₈ ein Wasserstoffatom oder eine (C₁-C₆)Alkoxy(C₁-C₆)alkylgruppe ist,
R₀₁₉ ein Wasserstoffatom oder eine Hydroxy(C₁-C₆)alkylgruppe ist,
R₀₂₀ ein Wasserstoffatom oder eine lineare oder verzweigte (C₁-C₆)Alkylgruppe ist,
R₀₂₁ und R₀₂₁' unabhängig voneinander ein Wasserstoffatom, eine lineare oder verzweigte (C₁-C₆)Alkylgruppe oder eine Hydroxy(C₁-C₆)alkylgruppe sind,
oder das Paar (R₀₂₁, R₀₂₁') zusammen mit dem Stickstoffatom, an das sie angeknüpft sind, einen aromatischen oder nichtaromatischen Ring bilden, der 5 bis 7 Ringelemente enthält,
R₀₂₂ eine (C₁-C₆)Alkoxy(C₁-C₆)alkylgruppe, eine -(CH₂)ₚ₀-NR₀₂₄R₀₂₄'-Gruppe oder eine -(CH₂)ₚ₀-O-(CHR₀₁₈-CHR₀₁₉-O)_{q0}-R₀₂₀-Gruppe ist,
R₀₂₃ ein Wasserstoffatom oder eine (C₁-C₆)Alkoxy(C₁-C₆)alkylgruppe ist,
oder das Paar (R₀₂₂, R₀₂₃) zusammen mit dem Stickstoffatom, an das sie angeknüpft sind, einen aromatischen oder nichtaromatischen Ring bilden, der 5 bis 18 Ringelemente enthält, der optional zusätzlich zu dem Stickstoffatom 1 bis 5 Heteroatome ausgewählt aus O, S und N enthält, wobei der resultierende Ring optional substituiert ist durch ein Wasserstoffatom, eine lineare oder verzweigte (C₁-C₆)Alkylgruppe oder eine Heterocycloalkylgruppe,
R₀₂₄ und R₀₂₄' unabhängig voneinander ein Wasserstoffatom oder eine lineare oder verzweigte (C₁-C₆)Alkylgruppe sind,
oder das Paar (R₀₂₄, R₀₂₄') zusammen mit dem Stickstoffatom, an das sie angeknüpft sind, einen aromatischen oder nichtaromatischen Ring bestehend aus 5 bis 7 Ringelementen bilden, der zusätzlich zu dem Stickstoffatom 1 bis 3 Heteroatome ausgewählt aus O, S und N enthalten kann, und wobei der resultierende Ring optional substituiert ist durch ein Wasserstoffatom oder eine lineare oder verzweigte (C₁-C₆)Alkylgruppe,
R₀₃₁ wie folgt ist 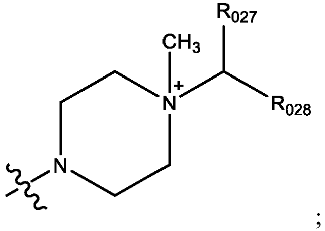
R₀₂₇ ein Wasserstoffatom oder eine lineare oder verzweigte (C₁-C₆)Alkylgruppe ist,
R₀₂₈ eine -O-P(O)(O⁻)(O⁻)-Gruppe, eine -O-P(O)(O⁻)(OR₀₃₀)-Gruppe, eine -O-P(O)(OR₀₃₀)(OR₀₃₀')-Gruppe, eine -(CH₂)ₚ₀-O-SO₂-O⁻-Gruppe, eine -(CH₂)ₚ₀-SO₂-O⁻-Gruppe, eine -(CH₂)ₚ₀-O-SO₂-OR₀₃₀-Gruppe, -Cy₀₁₀, eine -(CH₂)ₚ₀-SO₂-OR₀₃₀-Gruppe, eine -O-C(O)-R₀₂₉-Gruppe, eine -O-C(O)-OR₀₂₉-Gruppe oder eine -O-C(O)-NR₀₂₉R₀₂₉'-Gruppe ist;
R₀₂₉ und R₀₂₉' unabhängig voneinander ein Wasserstoffatom, eine lineare oder verzweigte (C₁-C₆)-Alkylgruppe oder eine lineare oder verzweigte Amino(C₁-C₆)alkylgruppe sind,
R₀₃₀ und R₀₃₀' unabhängig voneinander ein Wasserstoffatom, eine lineare oder verzweigte (C₁-C₆)Alkylgruppe oder eine Aryl(C₁-C₆)alkylgruppe sind,
p₀ eine ganze Zahl gleich 0, 1, 2 oder 3 ist,
q₀ eine ganze Zahl gleich 1, 2, 3 oder 4 ist,
r₀ und s₀ unabhängig eine ganze Zahl gleich 0 oder 1 sind;
wobei eine der R₀₃-, R₀₉- oder R₀₁₂-Gruppen kovalent an den Linker angeknüpft ist, L ein Linker ist, der kovalent Ab an D anknüpft; und
*p* eine ganze Zahl von 1 bis 16 ist.

2. Antikörper-Wirkstoff-Konjugat nach Anspruch 1, wobei *p* eine ganze Zahl von 1 bis 6 oder von 2 bis 4 ist, oder p 2 oder 4 ist; oder *p* bestimmt ist durch Flüssigchromatographie-Massenspektrometrie (LC-MS).

3. Antikörper-Wirkstoff-Konjugat nach Anspruch 1 oder 2, wobei L Folgendes umfasst:
eine Anknüpfungsgruppe;
zumindest eine überbrückende Spacer-Gruppe; und
zumindest eine spaltbare Gruppe, optional zumindest eine spaltbare Gruppe, umfassend eine Pyrophosphatgruppe und/oder eine selbstimmolative Gruppe.

4. Antikörper-Wirkstoff-Konjugat nach Anspruch 3, wobei -(L-D) von der Formel (A) ist: 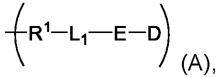 wobei:
R¹ eine Anknüpfungsgruppe ist;
L₁ eine überbrückende Spacer-Gruppe ist;
E eine spaltbare Gruppe ist.

5. Antikörper-Wirkstoff-Konjugat nach Anspruch 3 oder 4, wobei
(1) die spaltbare Gruppe eine Pyrophosphatgruppe umfasst oder die spaltbare Gruppe Folgendes umfasst 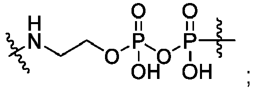
(2) die überbrückende Spacer-Gruppe Folgendes umfasst:
(i) eine Polyoxyethylen(PEG-)Gruppe;
(ii) eine PEG-Gruppe ausgewählt aus PEG1, PEG2, PEG3, PEG4, PEG5, PEG6, PEG7, PEG8, PEG9, PEG10, PEG11, PEG12, PEG13, PEG14 und PEG15;
(iii) eine -CO-CH₂-CH₂-PEG12--Gruppe;
(iv) eine Butanoyl-, Pentanoyl-, Hexanoyl-, Heptanoyl- oder Octanoylgruppe; oder
(v) eine Hexanoylgruppe;
(3)
(i) die Anknüpfungsgruppe aus zumindest einer reaktiven Gruppe ausgewählt aus einer Maleimidgruppe, Thiolgruppe, Cyclooctingruppe und einer Azidogruppe gebildet ist; wobei optional:
a) die Maleimidgruppe die folgende Struktur aufweist: 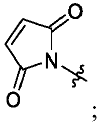
b) die Azidogruppe die folgende Struktur aufweist: -N=N⁺=N⁻;
c) die Cyclooctingruppe die folgende Struktur aufweist: 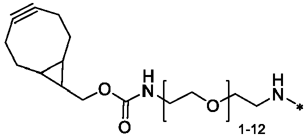 oder 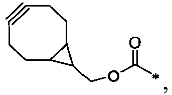 und wobei -* eine Bindung an den Antikörper oder das Antigenbindungsfragment ist; oder
d) die Cyclooctingruppe die folgende Struktur aufweist: 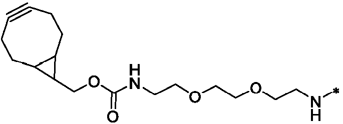 , und
wobei -* eine Bindung an den Antikörper oder das Antigenbindungsfragment ist; oder
(ii) die Anknüpfungsgruppe eine Formel aufweist, umfassend: 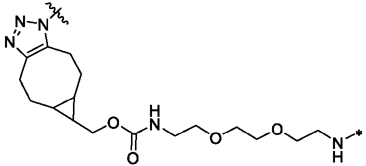 oder 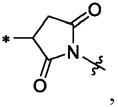 und
wobei -* eine Bindung an den Antikörper oder das Antigenbindungsfragment ist;
(4) der Antikörper oder das Antigenbindungsfragment mit dem Linker (L) durch eine Anknüpfungsgruppe ausgewählt aus Folgendem verknüpft ist: 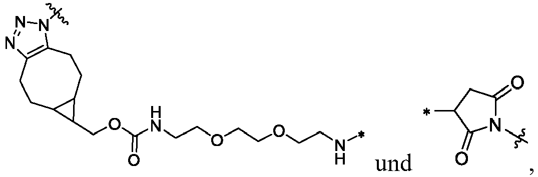 wobei -* eine Bindung an den Antikörper oder das Antigenbindungsfragment ist und wobei 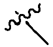 eine Bindung an die überbrückende Spacer-Gruppe ist;
(5) die überbrückende Spacer-Gruppe -CO-CH₂-CH₂-PEG12- ist;
(6) die überbrückende Spacer-Gruppe mit einer spaltbaren Gruppe verknüpft ist; optional die spaltbare Gruppe -Pyrophosphat-CH₂-CH₂-NH₂- ist; oder
(7) die spaltbare Gruppe mit dem Mcl-1-Inhibitor (D) verknüpft ist oder die spaltbare Gruppe mit dem Mcl-1-Inhibitor (D) durch eine Phenylpyrimidinylgruppe verknüpft ist.

6. Antikörper-Wirkstoff-Konjugat nach einem der Ansprüche 1 bis 3, wobei der Linker Folgendes umfasst:
eine Anknüpfungsgruppe,
zumindest eine überbrückende Spacer-Gruppe,
eine Peptidgruppe, und
zumindest eine spaltbare Gruppe.

7. Antikörper-Wirkstoff-Konjugat nach Anspruch 6, wobei -(L-D) von der Formel (B) ist: 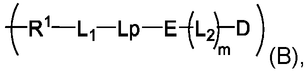 wobei:
R¹ eine Anknüpfungsgruppe ist;
L₁ ein überbrückender Spacer ist;
Lp eine Peptidgruppe umfassend 1 bis 6 Aminosäurereste ist oder Lp eine folgende Gruppe umfasst 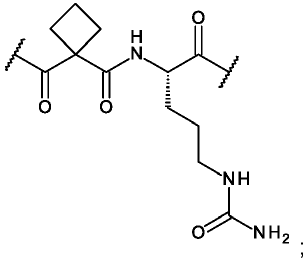
E eine spaltbare Gruppe ist
L₂ ein überbrückender Spacer ist;
m 0 oder 1 ist; und
D ein Mcl-1-Inhibitor wie definiert in Anspruch 1 ist.

8. Antikörper-Wirkstoff-Konjugat nach Anspruch 6 oder 7, wobei
(1)
(i) die Anknüpfungsgruppe aus zumindest einer reaktiven Gruppe umfassend eine Maleimidgruppe, Thiolgruppe, Cyclooctingruppe und/oder eine Azidogruppe gebildet ist, wobei optional:
a) die Maleimidgruppe die folgende Struktur aufweist: 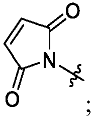
b) die Azidogruppe die folgende Struktur aufweist: -N=N⁺=N⁻;
c) die Cyclooctingruppe die folgende Struktur aufweist: 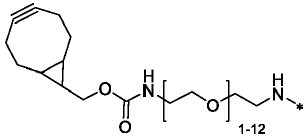 oder 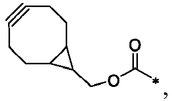 und wobei -* eine Bindung an den Antikörper oder das Antigenbindungsfragment ist; oder
d) die Cyclooctingruppe die folgende Struktur aufweist: 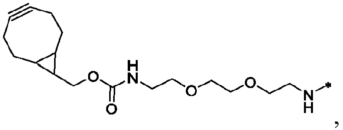 und
wobei -* eine Bindung an den Antikörper oder das Antigenbindungsfragment ist; oder
(ii) die Anknüpfungsgruppe eine Formel aufweist, umfassend: 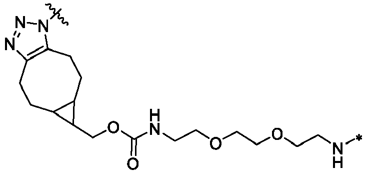 oder 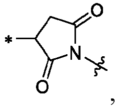 und
wobei -* eine Bindung an den Antikörper oder das Antigenbindungsfragment ist;
(2)
(i) zumindest ein überbrückender Spacer eine PEG-Gruppe umfasst, optional die PEG-Gruppe ausgewählt ist aus PEG1, PEG2, PEG3, PEG4, PEG5, PEG6, PEG7, PEG8, PEG9, PEG10, PEG11, PEG12, PEG13, PEG14 und PEG15; oder
(ii) zumindest ein überbrückender Spacer ausgewählt ist aus *-C(O)-CH₂-CH₂-PEG1-**, *-C(O)-CH₂- PEG₃-**, *-C(O)-CH₂-CH₂-PEG12**, *-NH-CH₂-CH₂-PEG1-**, einer Polyhydroxyalkylgruppe und *-C(O)-N(CH₃)-CH₂-CH₂-N(CH₃)-C(O)-**, wobei ** den Punkt der direkten oder indirekten Anknüpfung des zumindest einen überbrückenden Spacers an die Anknüpfungsgruppe angibt und * den Punkt der direkten oder indirekten Anknüpfung des zumindest einen überbrückenden Spacers an die Peptidgruppe angibt;
(3) L₁ ausgewählt ist aus *-C(O)-CH₂-CH₂-PEG1-**, *-C(O)-CH₂-PEG3-**, *-C(O)-CH₂-CH₂-PEG12**, *-NH-CH₂-CH₂-PEG1-** und einer Polyhydroxyalkylgruppe, wobei ** den Punkt der direkten oder indirekten Anknüpfung von L₁ an R¹ angibt und * den Punkt der direkten oder indirekten Anknüpfung von L₁ an Lp angibt;
(4) m 1 ist und L₂ -C(O)-N(CH₃)-CH₂-CH₂-N(CH₃)-C(O)- ist;
(5)
(i) die Peptidgruppe 1 bis 6, 1 bis 4, 1 bis 3 oder 1 bis 2 Aminosäurereste umfasst, optional die Aminosäurerest ausgewählt sind aus L-Glycin (Gly), L-Valin (Val), L-Citrullin (Cit), L-Cysteinsäure (sulfo-Ala), L-Lysin (Lys), L-Isoleucin (Ile), L-Phenylalanin (Phe), L-Methionin (Met), L-Asparagin (Asn), L-Prolin (Pro), L-Alanin (Ala), L-Leucin (Leu), L-Tryptophan (Trp) und L-Tyrosin (Tyr);
(ii) die Peptidgruppe Val-Cit, Val-Ala, Val-Lys und/oder sulfo-Ala-Val-Ala umfasst;
(iii) die Peptidgruppe ausgewählt ist aus: 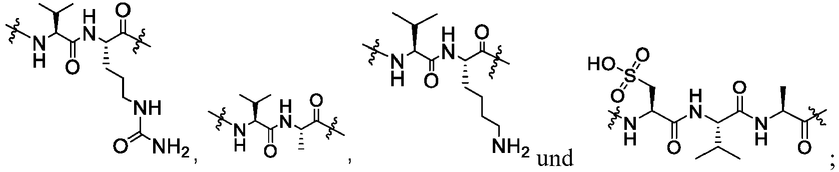
(6) (i) die spaltbare Gruppe ein Pyrophosphat und/oder eine selbstimmolative Gruppe umfasst; (ii) die spaltbare Gruppe eine selbstimmolative Gruppe umfasst; oder (iii) die spaltbare Gruppe eine selbstimmolative Gruppe umfasst, die para-Aminobenzylcarbamat, para-Aminobenzyl-ammonium, para-Amino-(sulfo)benzyl-ammonium, para-Amino-(sulfo)benzyl-carbamat, para-Amino- (alkoxy-PEG-alkyl) benzyl-carbamat, para-Amino-(polyhydroxycarboxytetrahydropyranyl)alkyl-benzyl-carbamat oder para-Amino-(polyhydroxycarboxytetrahydropyranyl)alkyl-benzyl-ammonium umfasst; oder
(7) m 0 oder 1 ist oder m 1 ist und der überbrückende Spacer Folgendes umfasst

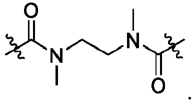

9. Antikörper-Wirkstoff-Konjugat nach Anspruch 7 oder 8, wobei
(1) -(L-D) gebildet ist aus einer Verbindung ausgewählt aus: 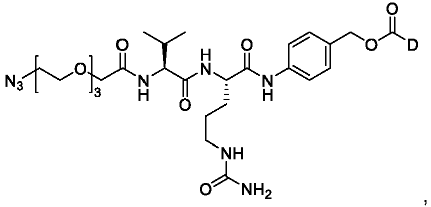 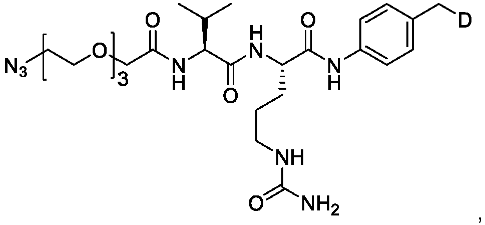 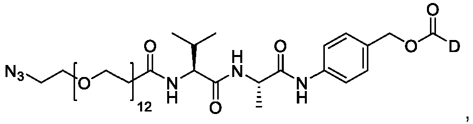 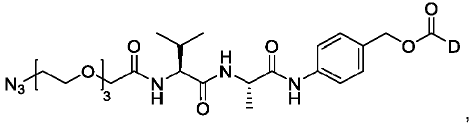 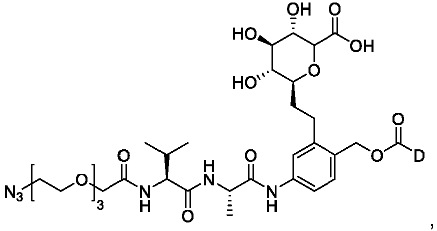 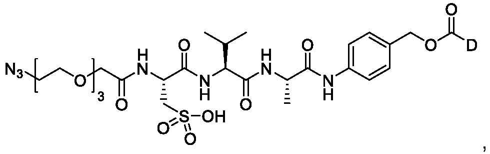 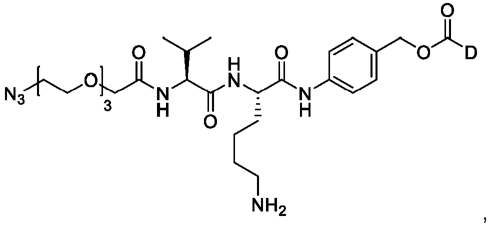 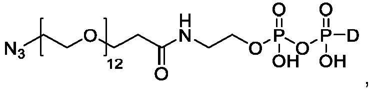 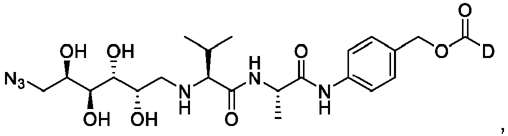 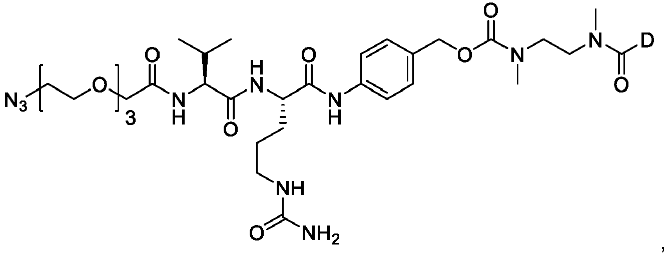 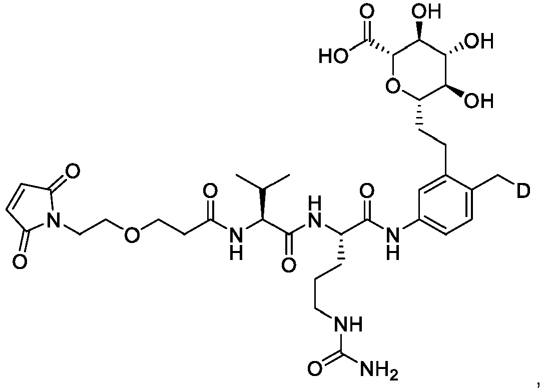 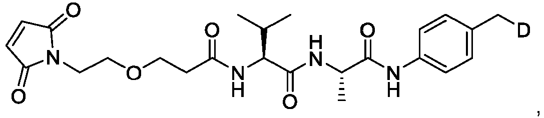 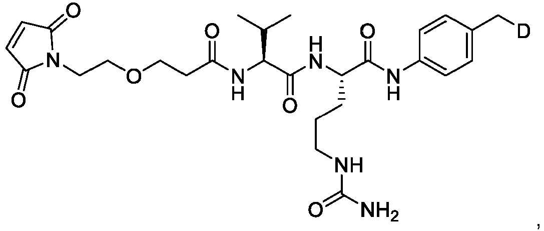 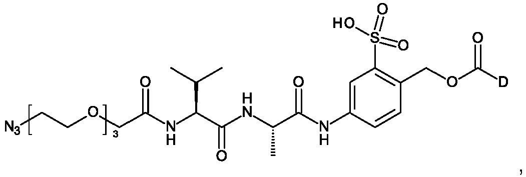 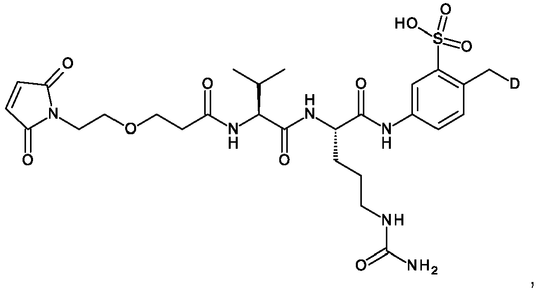 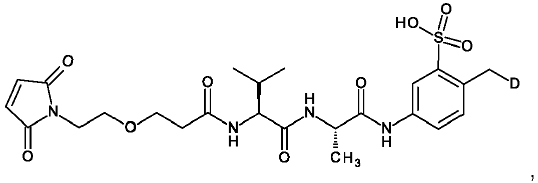 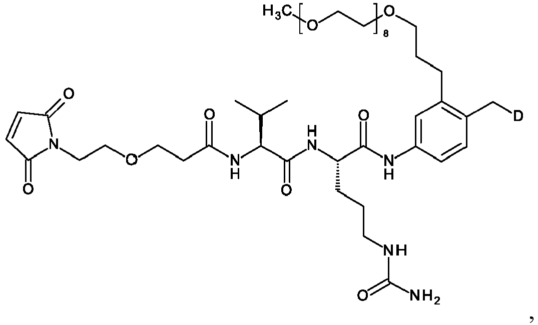 und 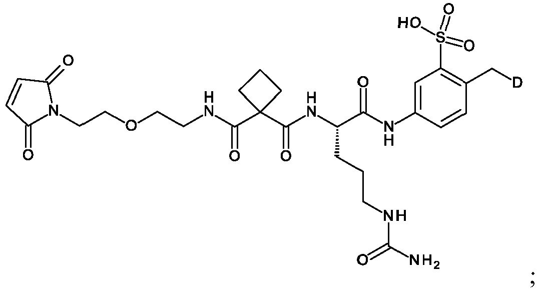 oder
(2) -(L-D) eine Formel ausgewählt aus Folgendem umfasst: 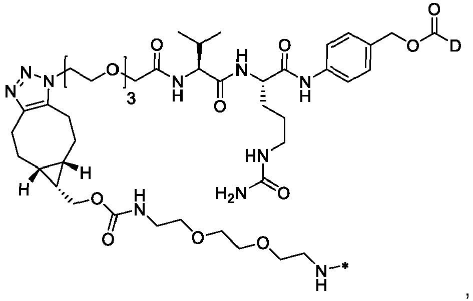 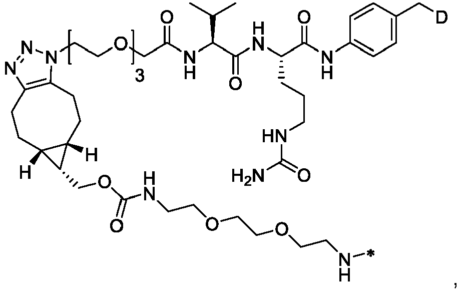 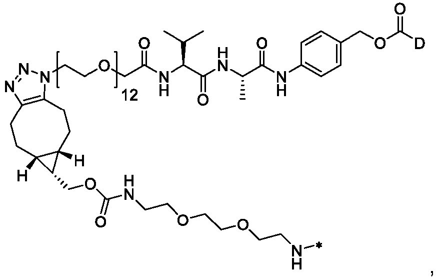 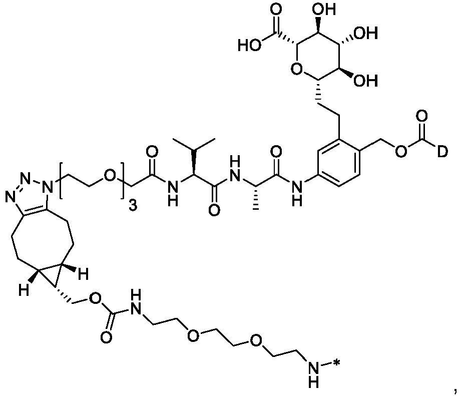 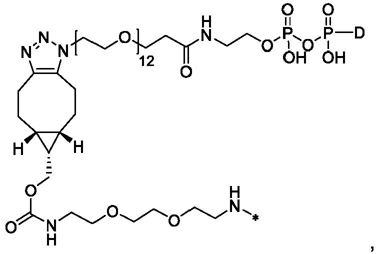 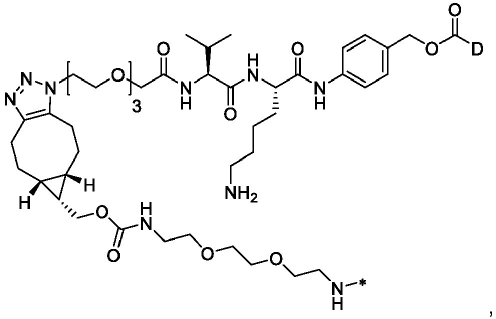 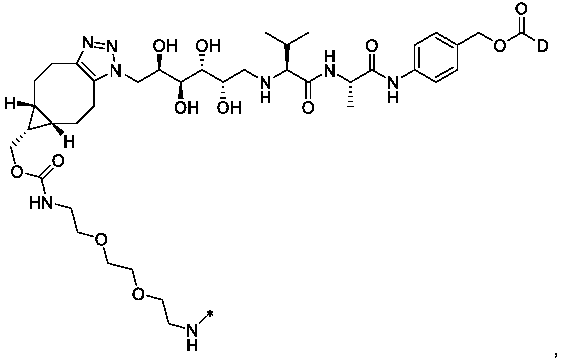 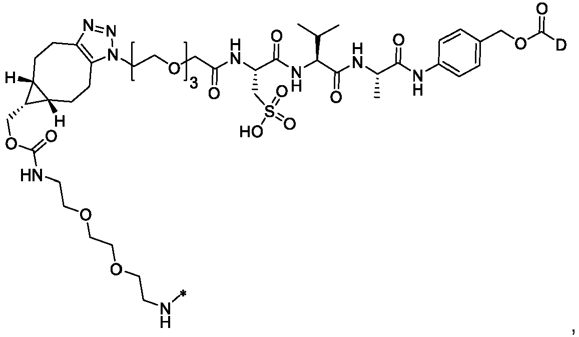 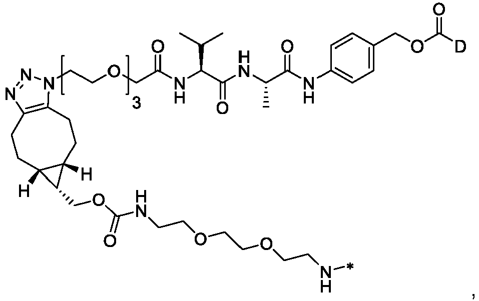 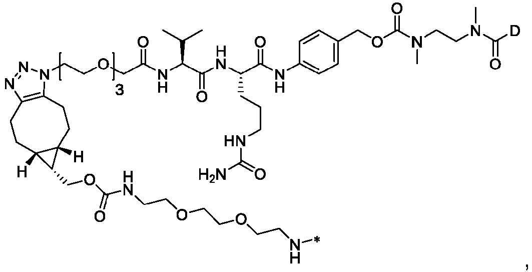 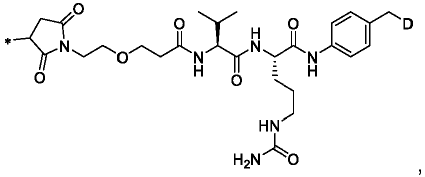 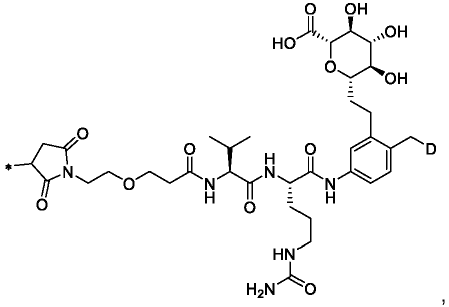 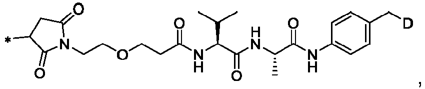 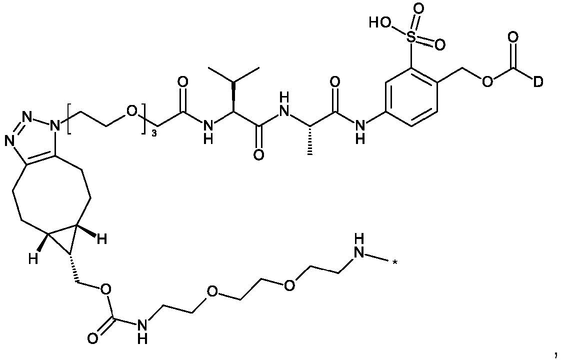 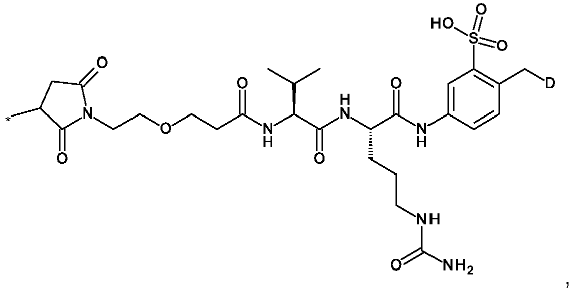 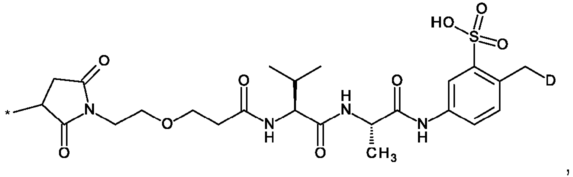 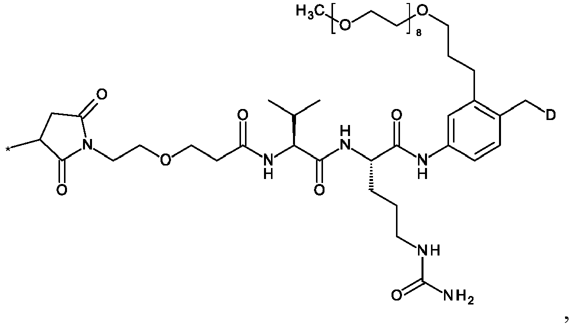 und 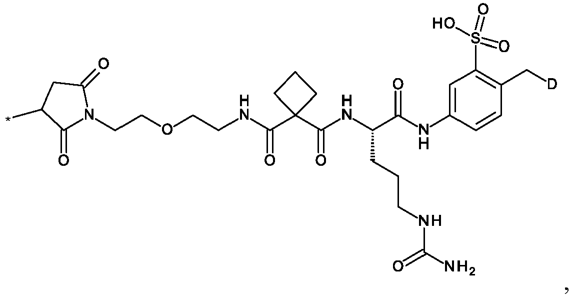 und
wobei -* eine Bindung an den Antikörper oder das Antigenbindungsfragment ist.

10. Antikörper-Wirkstoff-Konjugat nach Anspruch 1 oder 2, wobei
(1) -(L-D) von der Formel (C) ist: 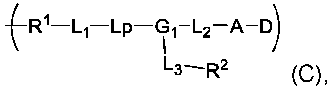 wobei:
R¹ eine Anknüpfungsgruppe ist;
L₁ ein überbrückender Spacer ist;
Lₚ eine Peptidgruppe ist, die 1 bis 6 Aminosäuren umfasst;
D ein Mcl-1-Inhibitor wie definiert in Anspruch 1 ist;
G₁-L₂-A ein selbstimmolativer Spacer ist;
L₂ eine Bindung, ein Methylen, ein Neopentylen oder ein C₂-C₃-Alkenylen ist;
A eine Bindung, -OC(=O)-*, 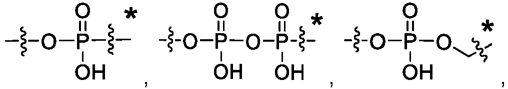 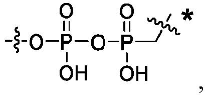 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt;
L₃ eine Spacer-Einheit ist; und
R² eine hydrophile Einheit ist; oder
(2) -(L-D) von Formel (D) ist:
wobei: 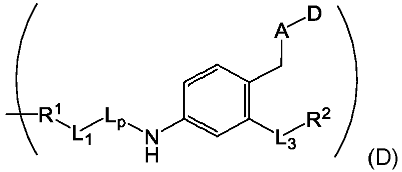
R¹ eine Anknüpfungsgruppe ist;
L₁ ein überbrückender Spacer ist;
Lp eine Peptidgruppe umfassend 1 bis 6 Aminosäuren ist;
A eine Bindung, -OC(=O)-*, 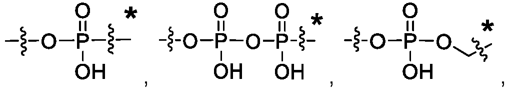 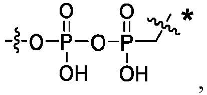 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt;
L₃ eine Spacer-Einheit ist; und
R² eine hydrophile Einheit ist.

11. Antikörper-Wirkstoff-Konjugat nach Anspruch 10, wobei:
(1) L₁ Folgendes umfasst: 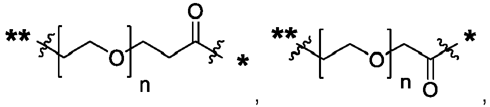 oder
*-CH(OH)CH(OH)CH(OH)CH(OH)-**,
wobei jedes n eine ganze Zahl von 1 bis 12 ist, wobei das * von L₁ den Punkt von direkter oder indirekter Anknüpfung an Lp angibt, und das ** von L₁ den Punkt von direkter oder indirekter Anknüpfung an R¹ angibt;
(2) L₁ 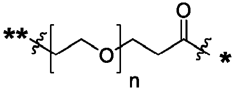 ist und n eine ganze Zahl von 1 bis 12 ist oder n 1 ist oder n 12 ist,
wobei das * von L₁ den Punkt von direkter oder indirekter Anknüpfung an Lp angibt und das ** von L₁ den Punkt von direkter oder indirekter Anknüpfung an R¹ angibt;
(3) L₁ 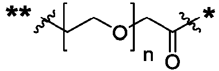 ist, und n eine ganze Zahl von 1 bis 12 ist, wobei das * von L₁ den Punkt von direkter oder indirekter Anknüpfung an Lp angibt und das ** von L₁ den Punkt von direkter oder indirekter Anknüpfung an R¹ angibt;
(4) L₁ 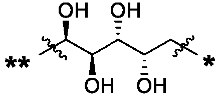 umfasst, wobei das * von L₁ den Punkt von direkter oder indirekter Anknüpfung an Lp angibt, und das ** von L₁ den Punkt von direkter oder indirekter Anknüpfung an R¹ angibt;
(5) L₁ ein überbrückender Spacer ist, umfassend:
*-C(=O)(CH₂)ₘO(CH₂)ₘ-**; *-C(=O)((CH₂)ₘO)ₜ(CH₂)ₙ-**; *-C(=O)(CH₂)ₘ-**;
*-C(=O)NH((CH₂)ₘO)ₜ(CH₂)ₙ-**;
*-C(=O)O(CH₂)ₘSSC(R³)₂(CH₂)ₘC(=O)NR³(CH₂)ₘNR³C(=O)(CH₂)ₘ-**;
*-C(=O)O(CH₂)ₘC(=O)NH(CH₂)ₘ-**; *-C(=O)(CH₂)ₘNH(CH₂)ₘ-**;
*-C(=O)(CH₂)ₘNH(CH₂)ₙC(=O)-**; *-C(=O)(CH₂)ₘX₁(CH₂)ₘ-**;
*-C(=O)((CH₂)ₘO)ₜ(CH₂)ₙX₁(CH₂)ₙ-**; *-C(=O)(CH₂)ₘNHC(=O)(CH₂)ₙ-**;
*-C(=O)((CH₂)ₘO)ₜ(CH₂)ₙNHC(=O)(CH₂)ₙ-**;
*-C(=O)(CH₂)ₘNHC(=O)(CH₂)ₙX₁(CH₂)ₙ-**;
*-C(=O)((CH₂)ₘO)ₜ(CH₂)ₙNHC(=O)(CH₂)ₙX₁(CH₂)ₙ-**;
*-C(=O)((CH₂)ₘO)ₜ(CH₂)ₙC(=O)NH(CH₂)ₘ-**; *-C(=O)(CH₂)ₘC(R³)₂-**
oder
*-C(=O)(CH₂)ₘC(=O)NH(CH₂)ₘ-**,
wobei das * von L₁ den Punkt von direkter oder indirekter Anknüpfung an Lp angibt und das ** von L₁ den Punkt von direkter oder indirekter Anknüpfung an R¹ angibt;
X₁ wie folgt ist 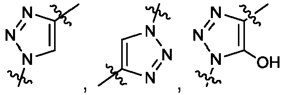 oder 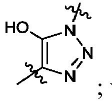 und
jedes m unabhängig ausgewählt ist aus 1, 2, 3, 4, 5, 6, 7, 8, 9 und 10;
jedes n unabhängig ausgewählt ist aus 1, 2, 3, 4, 5, 6, 7, 8, 9 und 10; und
jedes t unabhängig ausgewählt ist aus 1, 2, 3, 4, 5, 6, 7, 8, 9, 10, 11, 12, 13, 14, 15, 16, 17, 18, 19, 20, 21, 22, 23, 24, 25, 26, 27, 28, 29 und 30;
und jedes R³ unabhängig ausgewählt ist aus H und C₁-C₆-Alkyl.

12. Antikörper-Wirkstoff-Konjugat nach Anspruch 10 oder 11, wobei
(1) R² eine hydrophile Einheit ist, umfassend Polyethylenglykol, Polyalkylenglykol, ein Polyol, ein Polysarcosin, einen Zucker, ein Oligosaccharid, ein Polypeptid oder C₂-C₆-Alkyl substituiert mit 1 bis 3 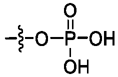 - oder 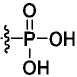 -Gruppen;
(2) R² wie folgt ist 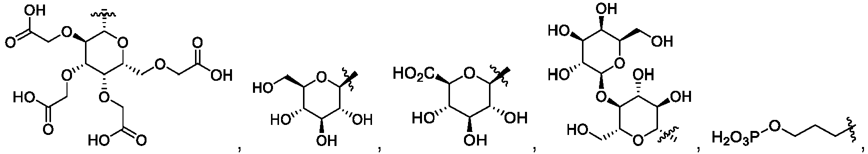 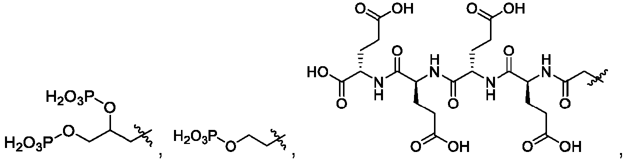 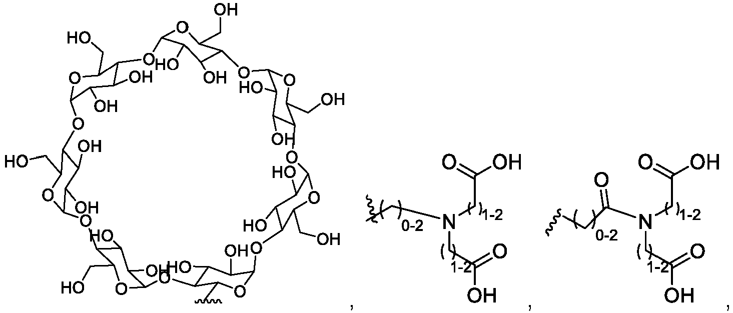 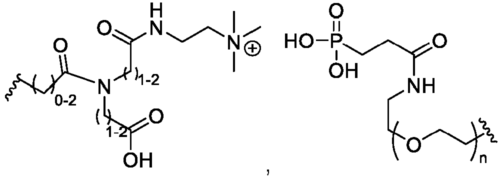 wobei n eine ganze Zahl zwischen 1 und 6 ist, 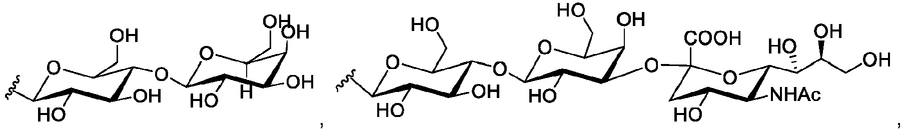 oder 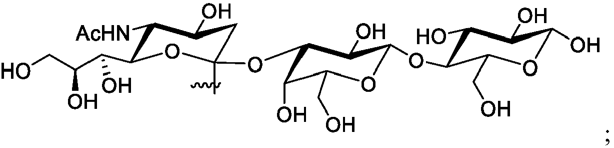
(3) die hydrophile Einheit dargestellt durch R² Folgendes umfasst:
(i) ein Polysarkosin, z. B. mit der folgenden Einheit: 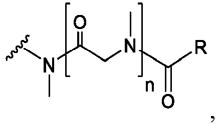 wobei n eine ganze Zahl zwischen 3 und 25 ist; und R H, -CH₃ oder -CH₂CH₂C(=O)OH ist; oder
(ii) ein Polyethylenglykol der Formel: 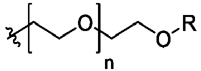 oder 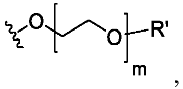 wobei R H, -CH₃, CH₂CH₂NHC(=O)ORₐ, -CH₂CH₂NHC(=O)Rₐ oder -CH₂CH₂C(=O)ORₐ ist, R' OH,-OCH₃, -CH₂CH₂NHC(=O)ORₐ, -CH₂CH₂NHC(=O)Rₐ oder -OCH₂CH₂C(=O)ORₐ ist, wobei Rₐ H oder C₁₋₄-Alkyl optional substituiert mit entweder OH oder C₁₋₄-Alkoxyl ist, und jedes von m und n unabhängig eine ganze Zahl zwischen 2 und 25 ist; oder
(4) die hydrophile Einheit dargestellt durch R² Folgendes umfasst

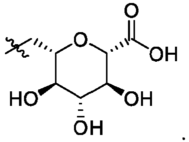

13. Antikörper-Wirkstoff-Konjugat nach einem der Ansprüche 10 bis 12, wobei:
(i) L₃ eine Spacer-Einheit mit der folgenden Struktur ist 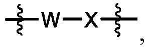 wobei:
W -CH₂-, -CH₂O-, -CH₂N(R^{b})C(=O)O-, -NHC(=O)C(R^{b})₂NHC(=O)O-,-NHC(=O)C(R^{b})₂NH-, -NHC(=O)C(R^{b})₂NHC(=O)-, -CH₂N(X-R²)C(=O)O-, -C(=O)N(X-R²)-,-CH₂N(X-R²)C(=O)-, -C(=O)NR^{b}-, -C(=O)NH-, -CH₂N R^{b} C(=O)-, -CH₂N R^{b} C(=O)NH-,-CH₂NR^{b}C(=O)NR^{b}-, -NHC(=O)-, -NHC(=O)O-, -NHC(=O)NH-, -OC(=O)NH-, -S(O)₂NH-,-NHS(O)₂-, -C(=O)-, -C(=O)O- oder -NH- ist, wobei jedes R^{b} unabhängig ausgewählt ist aus H, C₁-C₆-Alkyl und C₃-C₈-Cycloalkyl; und
X eine Bindung, Triazolyl oder -CH₂-Triazolyl- ist; oder
(ii) L₃ eine Spacer-Einheit mit der folgenden Struktur ist 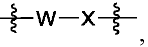 wobei:
W -CH₂-, -CH₂O-, -CH₂N(R^{b})C(=O)O-, -NHC(=O)C(R^{b})₂NHC(=O)O-,-NHC(=O)C(R^{b})₂NH-, -NHC(=O)C(R^{b})₂NHC(=O)-, -CH₂N(X-R²)C(=O)O-, -C(=O)N(X-R²)-,-CH₂N(X-R²)C(=O)-, -C(=O)NR^{b}-, -C(=O)NH-, -CH₂NR^{b}C(=O)-, -CH₂NR^{b}C(=O)NH-,-CH₂NR^{b}C(=O)NR^{b}-, -NHC(=O)-, -NHC(=O)O-, -NHC(=O)NH-, -OC(=O)NH-, -S(O)₂NH-,-NHS(O)₂-, -C(=O)-, -C(=O)O- oder -NH- ist, wobei jedes R^{b} unabhängig ausgewählt ist aus H, C₁-C₆-Alkyl und C₃-C₈-Cycloalkyl; und
X -CH₂-Triazolyl-C₁₋₄-alkylen-OC(O)NHS(O)₂NH-, -C₄₋₆-Cycloalkylen-OC(O)NHS(O)₂NH-, -(CH₂CH₂O)ₙ-C(O)NHS(O)₂NH-, -(CH₂CH₂O)ₙ-C(O)NHS(O)₂NH-(CH₂CH₂O)ₙ- oder -CH₂-Triazolyl-C₁₋₄-alkylen-OC(O)NHS(O)₂NH-(CH₂CH₂O)ₙ- ist, wobei jedes n unabhängig 1, 2 oder 3 ist.

14. Antikörper-Wirkstoff-Konjugat nach einem der Ansprüche 3 bis 13, wobei
(1) die Anknüpfungsgruppe durch eine Reaktion gebildet ist, die zumindest eine reaktive Gruppe umfasst;
(2) die Anknüpfungsgruppe durch Reagieren von Folgendem gebildet ist:
einer ersten reaktiven Gruppe, die an den Linker angeknüpft ist, und
einer zweiten reaktiven Gruppe, die an den Antikörper oder das Antigenbindungsfragment angeknüpft ist oder ein Aminosäurerest des Antikörpers oder des Antigenbindungsfragments ist, wobei optional (i) zumindest eine der reaktiven Gruppen Folgendes umfasst:
ein Thiol,
ein Maleimid,
ein Haloacetamid,
ein Azid,
ein Alkin,
ein Cycloocten,
ein Triarylphosphin,
ein Oxanorbornadien,
ein Cyclooctyn,
ein Diaryltetrazin,
ein Monoaryltetrazin,
ein Norbornen,
ein Aldehyd,
ein Hydroxylamin,
ein Hydrazin,
NH₂-NH-C(=O)-,
ein Keton,
ein Vinylsulfon,
ein Aziridin,
einen Aminosäurerest, 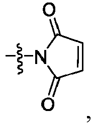 -ONH₂, -NH₂, 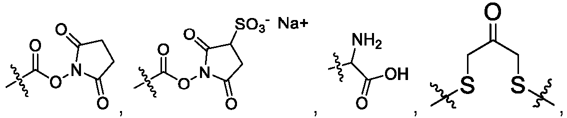 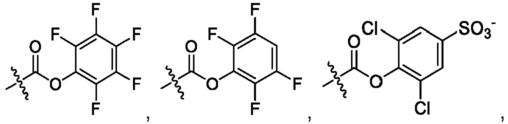 -N₃, 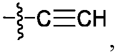 -SH, -SR³, -SSR⁴,-S(=O)₂(CH=CH₂), -(CH₂)₂S(=O)₂(CH=CH₂), -NHS(=O)₂(CH=CH₂), -NHC(=O)CH₂Br,-NHC(=O)CH₂I, 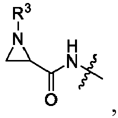 -C(O)NHNH₂, 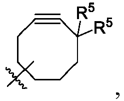 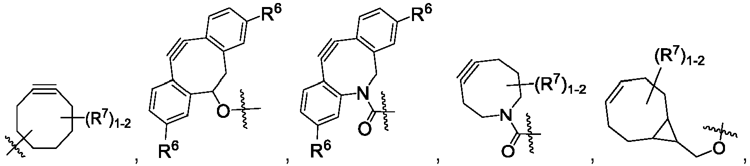 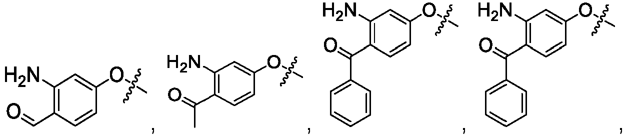 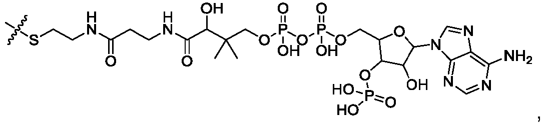 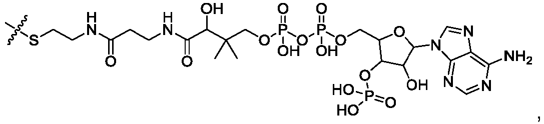 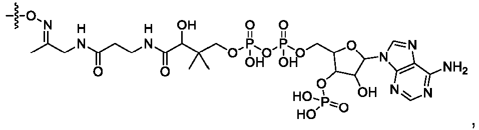 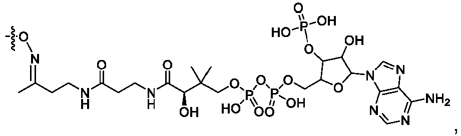 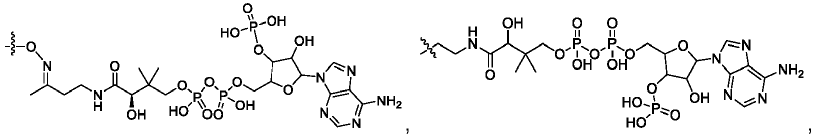 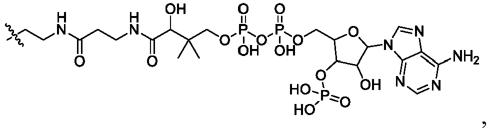 oder 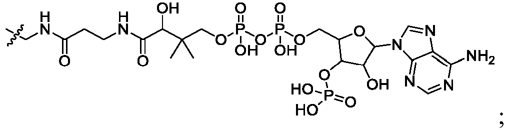
wobei:
jedes R³ unabhängig ausgewählt ist aus H und C₁-C₆-Alkyl;
jedes R⁴ 2-Pyridyl oder 4-Pyridyl ist;
jedes R⁵ unabhängig ausgewählt ist aus H, C₁-C₆-Alkyl, F, Cl und -OH;
jedes R⁶ unabhängig ausgewählt ist aus H, C₁₋C₆-Alkyl, F, Cl, -NH₂, -OCH₃, -OCH₂CH₃, -N(CH₃)₂, -CN, -NO₂ und -OH;
jedes R⁷ unabhängig ausgewählt ist aus H, C₁₋₆-Alkyl, Fluor, Benzyloxy substituiert mit-C(=O)OH, Benzyl substituiert mit -C(=O)OH, C₁₋₄-Alkoxy substituiert mit -C(=O)OH und C₁₋₄-Alkyl substituiert mit -C(=O)OH; und/oder
(ii) die erste reaktive Gruppe und die zweite reaktive Gruppe Folgendes umfassen:
ein Thiol und ein Maleimid,
ein Thiol und ein Haloacetamid,
ein Thiol und ein Vinylsulfon,
ein Thiol und ein Aziridin,
ein Azid und ein Alkin,
ein Azid und ein Cyclooctyn,
ein Azid und ein Cycloocten,
ein Azid und ein Triarylphosphin,
ein Azid und ein Oxanorbornadien,
ein Diaryltetrazin und ein Cycloocten,
ein Monoaryltetrazin und ein Norbornen,
ein Aldehyd und ein Hydroxylamin,
ein Aldehyd und ein Hydrazin,
ein Aldehyd und ein NH₂-NH-C(=O)-,
ein Keton und ein Hydroxylamin,
ein Keton und ein Hydrazin,
ein Keton und ein NH₂-NH-C(=O)-,
ein Hydroxylamin und 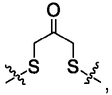
ein Amin und 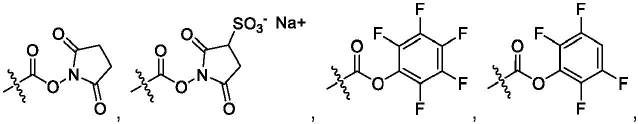 oder 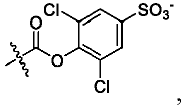 oder
ein CoA oder CoA-Analogon und einen Serinrest; oder
(3) die Anknüpfungsgruppe eine Gruppe ausgewählt aus Folgendem umfasst: 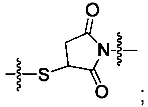 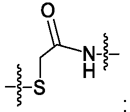 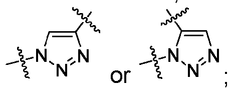 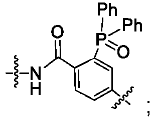 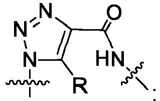 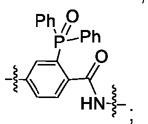 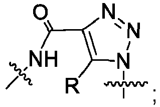 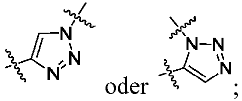 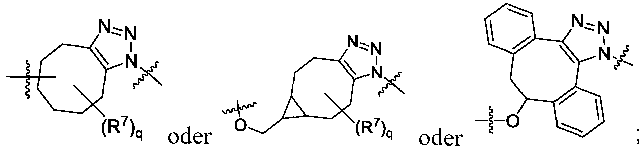 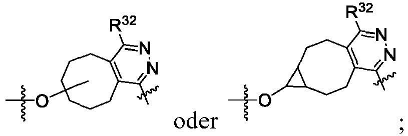 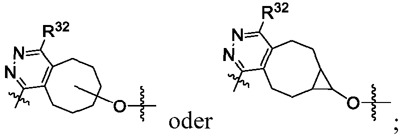 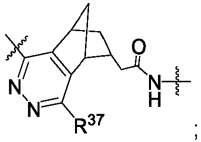 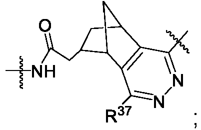 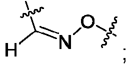 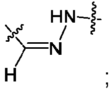 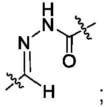 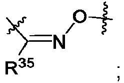 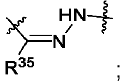 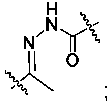 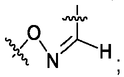 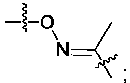 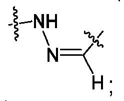 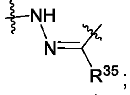 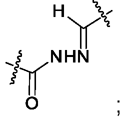 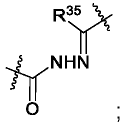 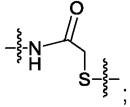 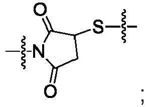 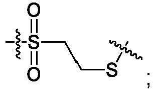 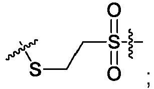 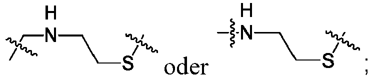 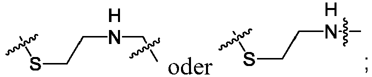 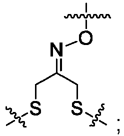 Amid; 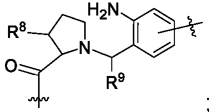 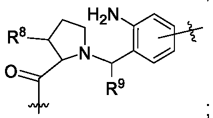 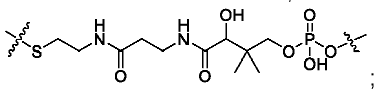 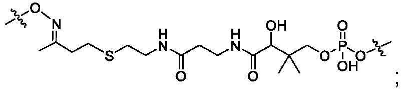 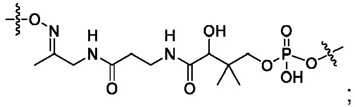 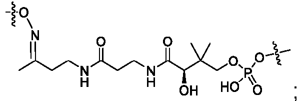 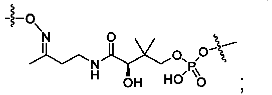 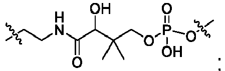 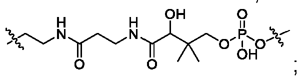 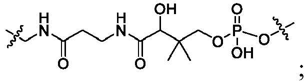 und Disulfid,
wobei:
R³² H, C₁₋₄-Alkyl, Phenyl, Pyrimidin oder Pyridin ist;
R³⁵ H, C₁₋₆-Alkyl, Phenyl oder C₁₋₄-Alkyl substituiert mit 1 bis 3 -OH-Gruppen ist;
jedes R⁷ unabhängig ausgewählt ist aus H, C₁₋₆-Alkyl, Fluor, Benzyloxy substituiert mit-C(=O)OH, Benzyl substituiert mit -C(=O)OH, C₁₋₄-Alkoxy substituiert mit -C(=O)OH und C₁₋₄-Alkyl substituiert mit -C(=O)OH;
R³⁷ unabhängig ausgewählt ist aus H, Phenyl und Pyridin;
q 0, 1, 2 oder 3 ist;
R⁸ H oder Methyl ist; und
R⁹ H, -CH₃ oder Phenyl ist.

15. Antikörper-Wirkstoff-Konjugat nach einem der Ansprüche 10 bis 14, wobei
(1) die Peptidgruppe dargestellt durch Lp 1 bis 4 oder 1 bis 3 oder 1 oder 2 Aminosäurerest umfasst, optional die Aminosäurerest ausgewählt sind aus L-Glycin (Gly), L-Valin (Val), L-Citrullin (Cit), L-Cysteinsäure (sulfo-Ala), L-Lysin (Lys), L-Isoleucin (Ile), L-Phenylalanin (Phe), L-Methionin (Met), L-Asparagin (Asn), L-Prolin (Pro), L-Alanin (Ala), L-Leucin (Leu), L-Tryptophan (Trp) und L-Tyrosin (Tyr);
(2) die Peptidgruppe dargestellt durch Lp Val-Cit, Phe-Lys, Val-Ala, Val-Lys, Leu-Cit, sulfo-Ala-Val und/oder sulfo-Ala-Val-Ala umfasst; oder
(3) Lp ausgewählt ist aus:

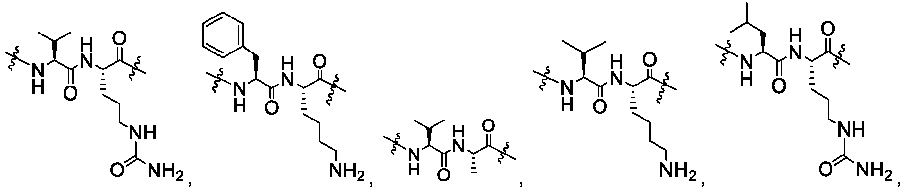

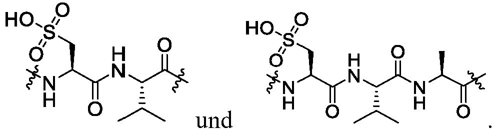

16. Antikörper-Wirkstoff-Konjugat nach einem der Ansprüche 10 bis 15, wobei:
-(L-D) eine Verbindung der folgenden Formel umfasst oder daraus gebildet ist:
(1) 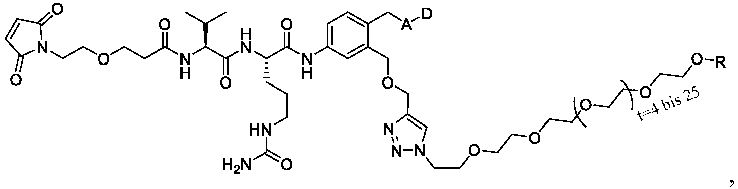 wobei:
R H, -CH₃ oder -CH₂CH₂C(=O)OH ist;
A eine Bindung, -OC(=O)-*, 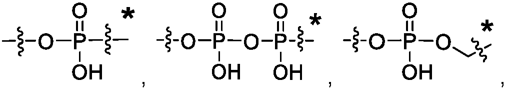 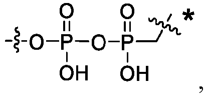 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(2) 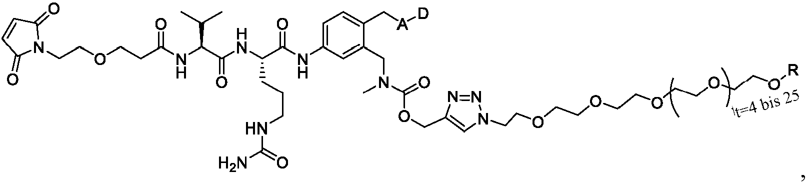 wobei:
R H, -CH₃ oder -CH₂CH₂C(=O)OH ist;
A eine Bindung, -OC(=O)-*, 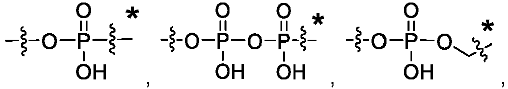 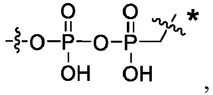 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(3) 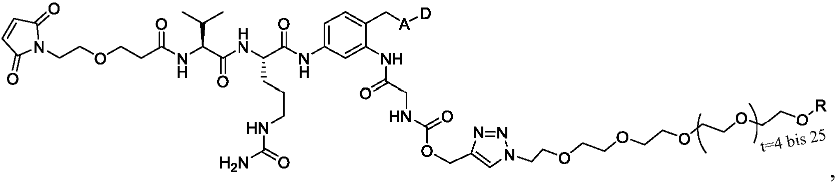 wobei:
R H, -CH₃ oder -CH₂CH₂C(=O)OH ist;
A eine Bindung, -OC(=O)-*, 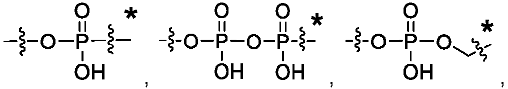 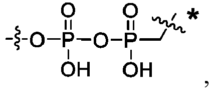 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(4) 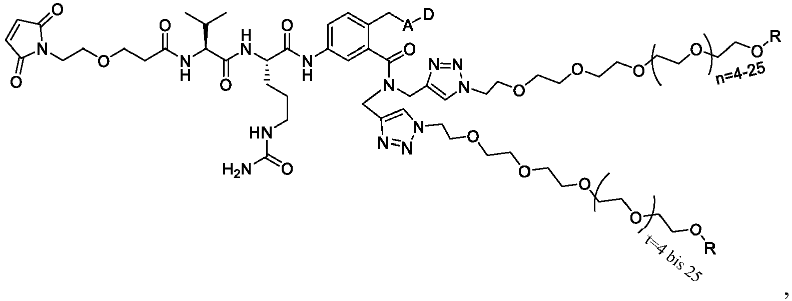 wobei:
jedes R unabhängig ausgewählt ist aus H, -CH₃ und -CH₂CH₂C(=O)OH;
A eine Bindung, -OC(=O)-*, 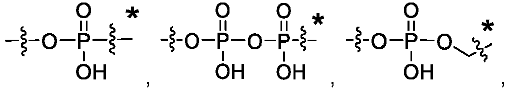 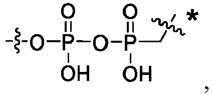 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und D ein Mcl-1-Inhibitor wie definiert in Anspruch 1 ist;
(5) 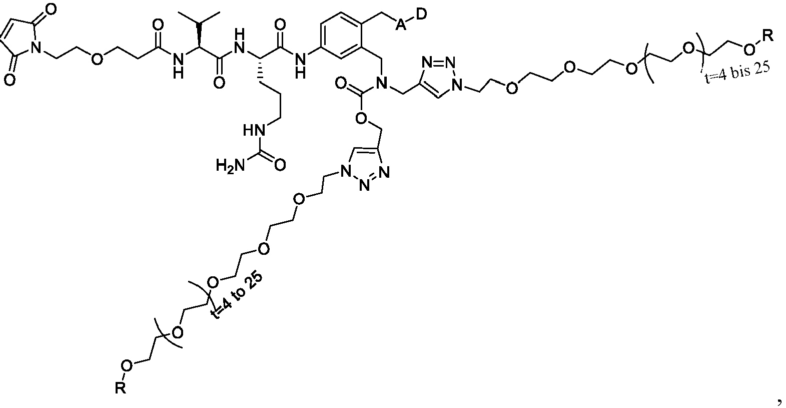 wobei:
jedes R unabhängig ausgewählt ist aus H, -CH₃ und -CH₂CH₂C(=O)OH;
A eine Bindung, -OC(=O)-*, 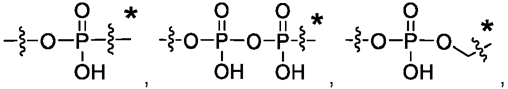 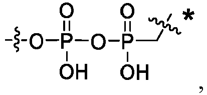 -OC(=O)N(CH₃)CH₂CH₂N(CH₃)C(=O)-* oder-OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(6) 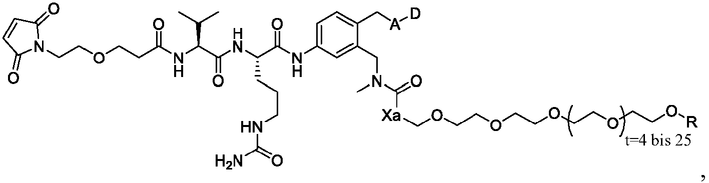 wobei:
Xa -CH₂-, -OCH₂-, -NHCH₂- oder-NRCH₂- ist und jedes R unabhängig H, -CH₃ oder-CH₂CH₂C(=O)OH ist;
A eine Bindung, -OC(=O)-*, 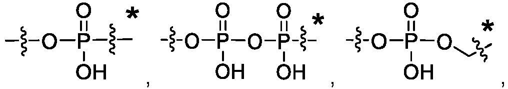 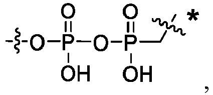 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(7) 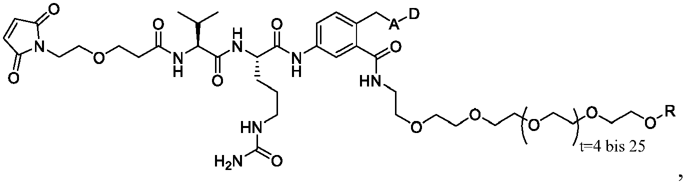 wobei:
R H, -CH₃ oder -CH₂CH₂C(=O)OH ist;
A eine Bindung, -OC(=O)-*, 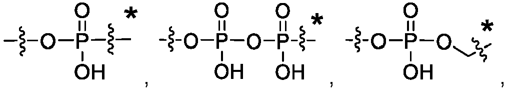 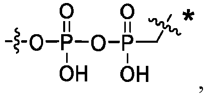 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(8) 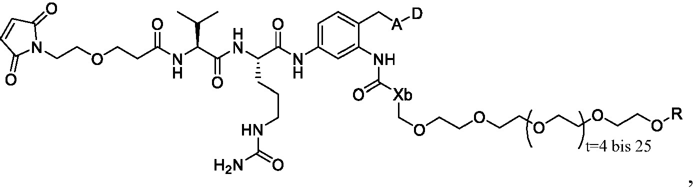 wobei:
Xb -CH₂-, -OCH₂-, -NHCH₂- oder-NRCH₂- ist und jedes R unabhängig H, -CH₃ oder-CH₂CH₂C(=O)OH ist;
A eine Bindung, -OC(=O)-*, 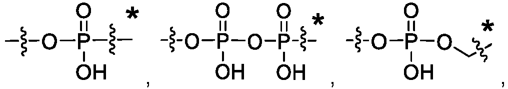 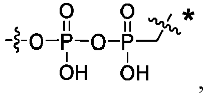 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(9) 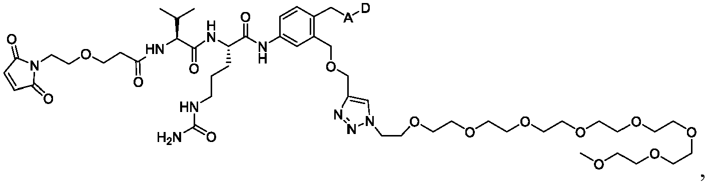 wobei:
A eine Bindung, -OC(=O)-*, 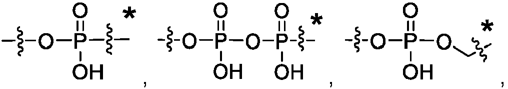 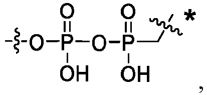 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(10) 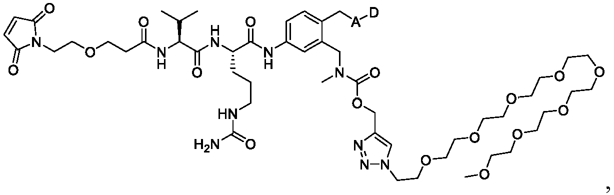 wobei:
A eine Bindung, -OC(=O)-*, 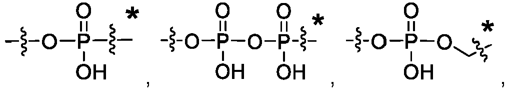 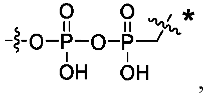 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(11) 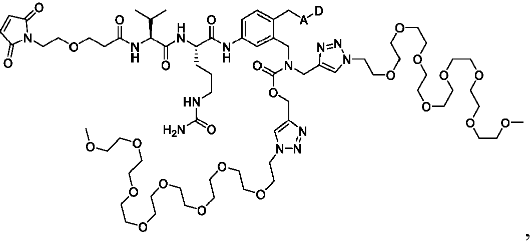 wobei:
A eine Bindung, -OC(=O)-*, 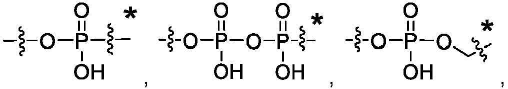 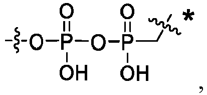 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(12) 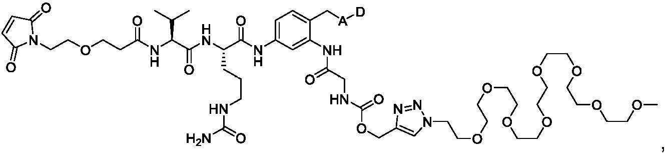 wobei:
A eine Bindung, -OC(=O)-*, 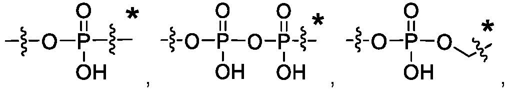 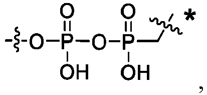 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(13) 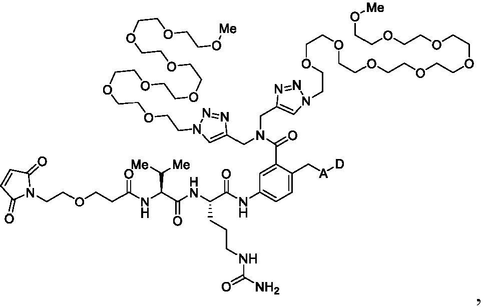 wobei:
A eine Bindung, -OC(=O)-*, 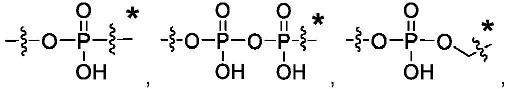 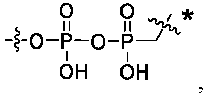 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(14) 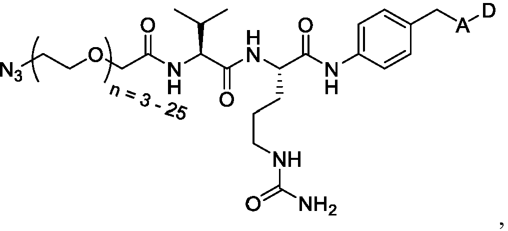 wobei:
A eine Bindung, -OC(=O)-*, 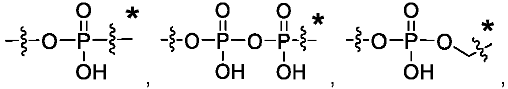 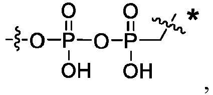 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist;
(15) 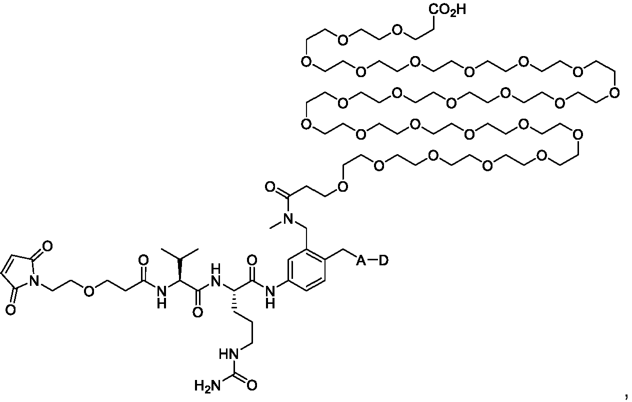 wobei:
A eine Bindung, -OC(=O)-*, 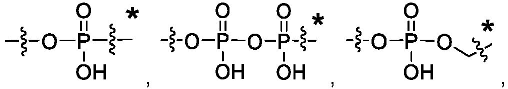 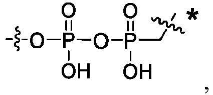 -OC(=O)N(CH₃)CH₂CH₂N(CH₃)C(=O)-* oder - OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist; oder
(16) 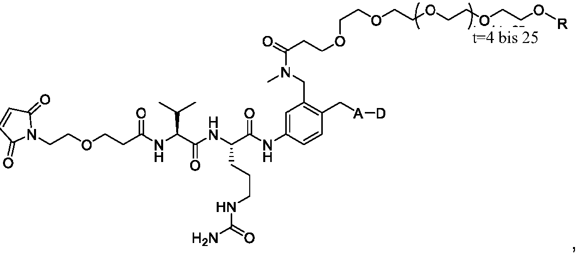 wobei:
jedes R unabhängig H, -CH₃ oder -CH₂CH₂C(=O)OH ist;
A eine Bindung, -OC(=O)-*, 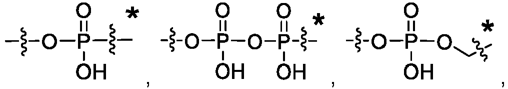 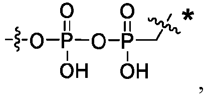 -OC(=O)N(CH₃)CH₂CH₂N(CH₃)C(=O)-* oder-OC(=O)N(CH₃)C(R^{a})₂C(R^{a})₂N(CH₃)C(=O)-* ist,
wobei jedes R^{a} unabhängig ausgewählt ist aus H, C₁-C₆-Alkyl und C₃-C₈-Cycloalkyl und das * von A den Anknüpfungspunkt an D angibt; und
D ein Mcl-1-Inhibitor wie definiert in Anspruch 1 ist.

17. Antikörper-Wirkstoff-Konjugat nach einem der Ansprüche 10 bis 16, wobei A eine Bindung ist und/oder R -CH₃ ist.

18. Antikörper-Wirkstoff-Konjugat nach einem der Ansprüche 1 bis 17, wobei D eine Verbindung der Formel (III) umfasst: 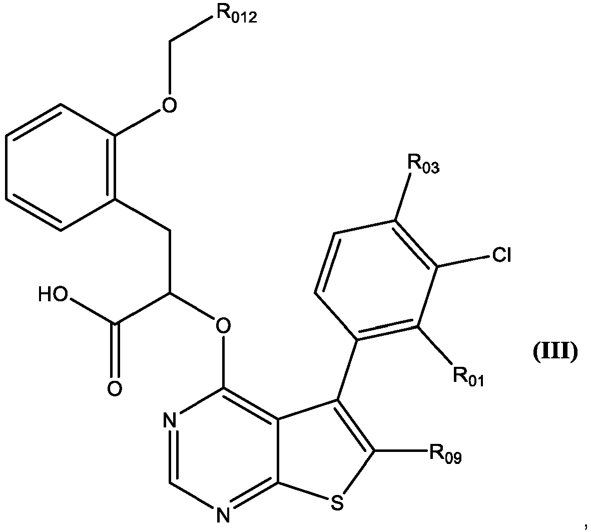
oder das Enantiomer, Diastereoisomer, Atropisomer, deuterierte Derivat und/oder pharmazeutisch annehmbare Salz davon;
wobei:
R₀₁ eine lineare oder verzweigte (C₁-C₆)Alkylgruppe ist,
R₀₃ -O-(C₁-C₆)Alkyl-NR₀₁₁ R₀₁₁' ist, oder 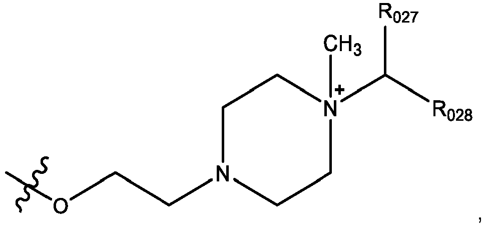
wobei R₀₁₁ und R₀₁₁' unabhängig voneinander ein Wasserstoffatom, eine optional substituierte lineare oder verzweigte (C₁-C₆)Alkylgruppe oder -(C₀-C₆)Alkyl-Cy₀₁ sind;
oder das Paar (R₀₁₁, R₀₁₁') zusammen mit dem Stickstoffatom, an das sie angeknüpft sind, einen aromatischen oder nichtaromatischen Ring bilden, der 5 bis 7 Ringelemente enthält, der optional zusätzlich zu dem Stickstoffatom 1 bis 3 Heteroatome ausgewählt aus O, S und N enthält, wobei das N-Atom substituiert sein kann durch 1 oder 2 Gruppen ausgewählt aus einem Wasserstoffatom oder einer linearen oder verzweigten (C₁-C₆)Alkylgruppe,
und wobei R₀₂₇ ein Wasserstoffatom ist und R₀₂₈ eine -(CH₂)ₚ₀-O-SO₂-O⁻-Gruppe oder eine -(CH₂)ₚ₀-SO₂-OR₀₃₀-Gruppe ist;
R₀₉ eine lineare oder verzweigte (C₂₋C₆)-Alkinylgruppe oder -Cy₀₂ ist,
R₀₁₂ -Cy₀₅, -Cy₀₅-(C₀₋C₆)Alkyl-Cy₀₆ oder -Cy₀₅-(C₀₋C₆)Alkyl-Cy₀₉ ist,
Cy₀₁, Cy₀₂, Cy₀₅ und Cy₀₆ unabhängig voneinander eine Cycloalkylgruppe, eine Heterocycloalkylgruppe, eine Arylgruppe oder eine Heteroarylgruppe sind, die jeweils optional substituiert ist,
Cy₀₉ wie folgt ist 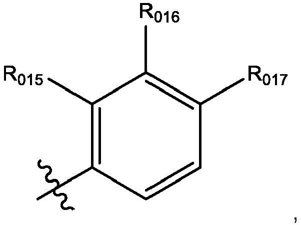
R₀₁₅, R₀₁₆ und R₀₁₇ wie für Formel (II) definiert sind,
wobei eine der R₀₃-, R₀₉- oder R₀₁₂-Gruppen kovalent an den Linker angeknüpft ist.

19. Antikörper-Wirkstoff-Konjugat nach Anspruch 18, wobei
(1) R₀₁ Methyl oder Ethyl ist;
(2) R₀₃ -O-CH₂-CH₂-NR₀₁₁R₀₁₁' ist, wobei R₀₁₁ und R₀₁₁' zusammen mit dem Stickstoffatom, das sie trägt, eine Piperazinylgruppe bilden, die substituiert sein kann durch eine Gruppe, die ein Wasserstoffatom oder eine lineare oder verzweigte (C₁-C₆)Alkylgruppe ist;
(3) R₀₃ die folgende Formel umfasst: 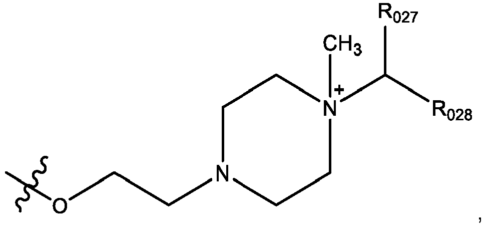 wobei R₀₂₇ ein Wasserstoffatom ist und R₀₂₈ eine -(CH₂)ₚ₀-SO₂-OR₀₃₀-Gruppe ist;
(4) R₀₃ die folgende Formel umfasst: 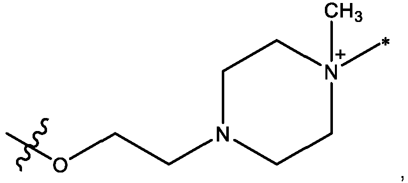 wobei -* eine Bindung an den Linker ist;
(5) R₀₉ Cy₀₂ ist;
(6) Cy₀₂ eine optional substituierte Arylgruppe ist;
(7) Cy₀₅ eine Heteroarylgruppe ausgewählt aus einer Pyrazolylgruppe und einer Pyrimidinylgruppe umfasst;
(8) Cy₀₅ eine Pyrimidinylgruppe ist; oder
(9) das L an D durch eine kovalente Bindung von L an R₀₃ der Formel (II) oder (III) angeknüpft ist; und/oder das L an D durch eine kovalente Bindung von L an R₀₉ der Formel (II) oder (III) angeknüpft ist.

20. Antikörper-Wirkstoff-Konjugat nach einem der Ansprüche 1 bis 18, wobei:
(1) D Folgendes umfasst: 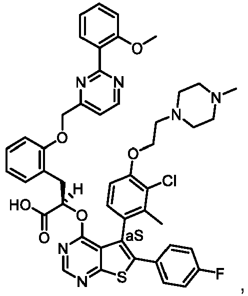 oder Enantiomer, Diastereoisomer, Atropisomer, deuteriertes Derivat und/oder pharmazeutisch annehmbares Salz davon;
(2) D Folgendes umfasst: 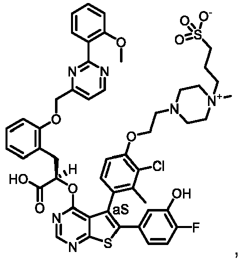 oder Enantiomer, Diastereoisomer, Atropisomer, deuteriertes Derivat und/oder pharmazeutisch annehmbares Salz davon;
(3) D Folgendes umfasst: 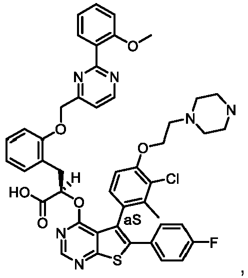 oder Enantiomer, Diastereoisomer, Atropisomer, deuteriertes Derivat und/oder pharmazeutisch annehmbares Salz davon;
(4) D Folgendes umfasst: 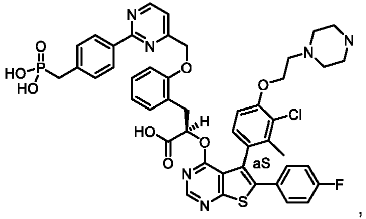 oder Enantiomer, Diastereoisomer, Atropisomer, deuteriertes Derivat und/oder pharmazeutisch annehmbares Salz davon;
(5) D Folgendes umfasst: 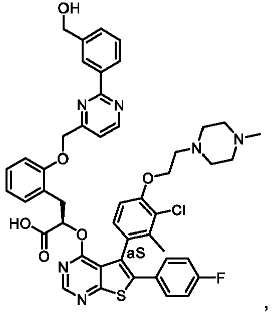 oder Enantiomer, Diastereoisomer, Atropisomer, deuteriertes Derivat und/oder pharmazeutisch annehmbares Salz davon;
(6) D Folgendes umfasst: 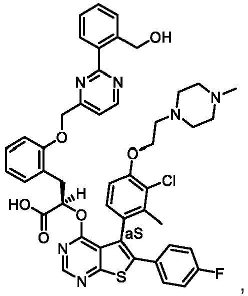 oder Enantiomer, Diastereoisomer, Atropisomer, deuteriertes Derivat und/oder pharmazeutisch annehmbares Salz davon;
(7) D Folgendes umfasst: 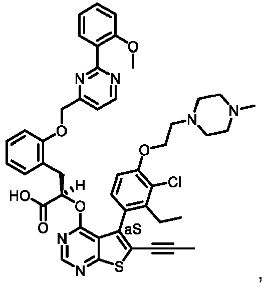 oder Enantiomer, Diastereoisomer, Atropisomer, deuteriertes Derivat und/oder pharmazeutisch annehmbares Salz davon;
(8) D Folgendes umfasst: 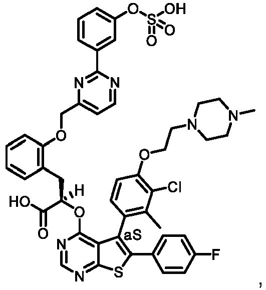 oder Enantiomer, Diastereoisomer, Atropisomer, deuteriertes Derivat und/oder pharmazeutisch annehmbares Salz davon;
(9) D Folgendes umfasst: 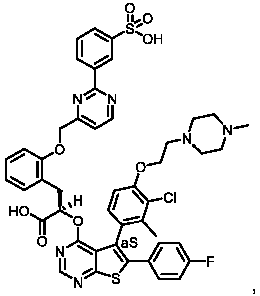 oder Enantiomer, Diastereoisomer, Atropisomer, deuteriertes Derivat und/oder pharmazeutisch annehmbares Salz davon;
(10) D Folgendes umfasst: 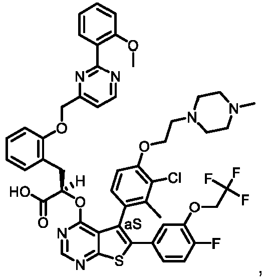 oder Enantiomer, Diastereoisomer, Atropisomer, deuteriertes Derivat und/oder pharmazeutisch annehmbares Salz davon;
(11) D Folgendes umfasst: 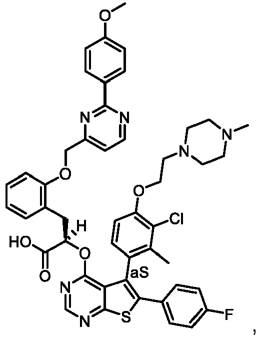 oder Enantiomer, Diastereoisomer, Atropisomer, deuteriertes Derivat und/oder pharmazeutisch annehmbares Salz davon;
(12) D Folgendes umfasst: 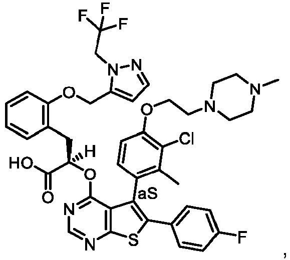 oder Enantiomer, Diastereoisomer, Atropisomer, deuteriertes Derivat und/oder pharmazeutisch annehmbares Salz davon;
(13) D Folgendes umfasst: 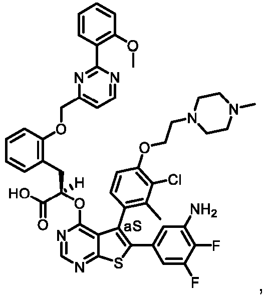 oder Enantiomer, Diastereoisomer, Atropisomer, deuteriertes Derivat und/oder pharmazeutisch annehmbares Salz davon; oder
(14) D Folgendes umfasst: 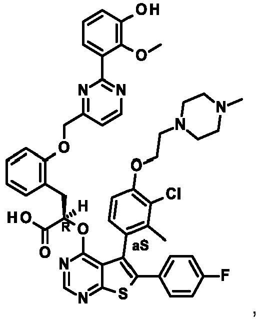 oder Enantiomer, Diastereoisomer, Atropisomer, deuteriertes Derivat und/oder pharmazeutisch annehmbares Salz davon.

21. Antikörper-Wirkstoff-Konjugat nach einem der Ansprüche 1 bis 18, wobei -(L-D) gebildet ist aus einer Verbindung in Tabelle A oder einem Enantiomer, Diastereoisomer, Atropisomer, deuterierten Derivat und/oder pharmazeutisch annehmbaren Salz davon.

22. Antikörper-Wirkstoff-Konjugat nach einem der Ansprüche 1 bis 21, wobei der Antikörper oder das Antigenbindungsfragment an ein Zielantigen auf der Krebszelle bindet; wobei optional
(1)
(i) das Zielantigen BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46 oder GPNMB ist;
(ii) das Zielantigen BCMA, CD33, PCAD, HER2, CD38, CD46, CD48 oder CD79b ist;
oder
(iii) das Zielantigen BCMA, CD33, CD48, PCAD oder HER2 ist;
(2) der Antikörper oder das Antigenbindungsfragment ein Anti-BCMA-Antikörper oder ein Antigenbindungsfragment ist;
(3) der Antikörper oder das Antigenbindungsfragment Folgendes umfasst:
(a) drei Schwerkettenkomplementaritätsbestimmungsregionen (HCDRs) umfassend Aminosäuresequenzen von SEQ ID NO:15 (HCDR1), SEQ ID NO:16 (HCDR2) und SEQ ID NO:17 (HCDR3); und drei Leichtkettenkomplementaritätsbestimmungsregionen (LCDRs) umfassend Aminosäuresequenzen von SEQ ID NO:18 (LCDR1), SEQ ID NO:19 (LCDR2) und SEQ ID NO:20 (LCDR3); oder
(b) der Antikörper oder das Antigenbindungsfragment eine variable Schwerkettenregion umfassend eine Aminosäuresequenz von SEQ ID NO:1 und eine variable Leichtkettenregion umfassend eine Aminosäuresequenz von SEQ ID NO:2 umfasst;
(4)
(a) der Antikörper oder das Antigenbindungsfragment eine konstante IgG1-Schwerkettendomäne oder eine modifizierte konstante IgG1-Schwerkettendomäne umfasst, optional die konstante IgG1-Schwerkettendomäne einen Cysteinrest (C) an Position 152 und Position 375 umfasst oder die konstante IgG1-Schwerkettendomäne einen Cysteinrest (C) an Position 156 und Position 379 umfasst;
und/oder
(b) der Antikörper oder das Antigenbindungsfragment eine konstante Ig-Kappa-Leichtkettendomäne umfasst;
(5) der Antikörper oder das Antigenbindungsfragment ein Anti-CD33-Antikörper oder - Antigenbindungsfragment ist;
(6)
(a) der Antikörper oder das Antigenbindungsfragment drei Schwerkettenkomplementaritätsbestimmungsregionen (HCDRs) umfassend Aminosäuresequenzen von SEQ ID NO:21 (HCDR1), SEQ ID NO:22 (HCDR2) und SEQ ID NO:23 (HCDR3); und drei Leichtkettenkomplementaritätsbestimmungsregionen (LCDRs) umfassend Aminosäuresequenzen von SEQ ID NO:24 (LCDR1), SEQ ID NO:25 (LCDR2) und SEQ ID NO:26 (LCDR3) umfasst; und/oder
(b) der Antikörper oder das Antigenbindungsfragment eine variable Schwerkettenregion umfassend eine Aminosäuresequenz von SEQ ID NO:3 und eine variable Leichtkettenregion umfassend eine Aminosäuresequenz von SEQ ID NO:4 umfasst;
(7)
(a) der Antikörper oder das Antigenbindungsfragment eine konstante IgG1-Schwerkettendomäne oder eine modifizierte konstante IgG1-Schwerkettendomäne umfasst, wobei optional die konstante IgG1-Schwerkettendomäne ein Glutamin (Q) an Position 297 umfasst; und/oder
(b) der Antikörper oder das Antigenbindungsfragment eine konstante Ig-Kappa-Leichtkettendomäne umfasst;
(8) der Antikörper oder das Antigenbindungsfragment ein Anti-PCAD-Antikörper oder - Antigenbindungsfragment ist;
(9)
(a) der Antikörper oder das Antigenbindungsfragment drei Schwerkettenkomplementaritätsbestimmungsregionen (HCDRs) umfassend Aminosäuresequenzen von SEQ ID NO:33 (HCDR1), SEQ ID NO:34 (HCDR2) und SEQ ID NO:35 (HCDR3); und drei Leichtkettenkomplementaritätsbestimmungsregionen (LCDRs) umfassend Aminosäuresequenzen von SEQ ID NO:36 (LCDR1), SEQ ID NO:37 (LCDR2) und SEQ ID NO:38 (LCDR3) umfasst; und/oder
(b) der Antikörper oder das Antigenbindungsfragment eine variable Schwerkettenregion umfassend eine Aminosäuresequenz von SEQ ID NO:7 und eine variable Leichtkettenregion umfassend eine Aminosäuresequenz von SEQ ID NO:8 umfasst;
(10) der Antikörper oder das Antigenbindungsfragment ein anti-HER2-Antikörper oder - Antigenbindungsfragment ist;
(11)
(a) der Antikörper oder das Antigenbindungsfragment drei Schwerkettenkomplementaritätsbestimmungsregionen (HCDRs) umfassend Aminosäuresequenzen von SEQ ID NO:39 (HCDR1), SEQ ID NO:40 (HCDR2) und SEQ ID NO:41 (HCDR3); und drei Leichtkettenkomplementaritätsbestimmungsregionen (LCDRs) umfassend Aminosäuresequenzen von SEQ ID NO:42 (LCDR1), SEQ ID NO:43 (LCDR2) und SEQ ID NO:44 (LCDR3) umfasst; und/oder
(b) der Antikörper oder das Antigenbindungsfragment eine variable Schwerkettenregion umfassend eine Aminosäuresequenz von SEQ ID NO:9 und eine variable Leichtkettenregion umfassend eine Aminosäuresequenz von SEQ ID NQ:10 umfasst;
(12)
(a) der Antikörper oder das Antigenbindungsfragment eine konstante IgG1-Schwerkettendomäne oder eine modifizierte konstante IgG1-Schwerkettendomäne umfasst, optional die konstante IgG1-Schwerkettendomäne ein Glutamin (Q) an Position 297 umfasst oder die konstante IgG1-Schwerkettendomäne ein Serin (S) an Position 297 umfasst; und/oder
(b) der Antikörper oder das Antigenbindungsfragment eine konstante Ig-Kappa-Leichtkettendomäne umfasst; oder
(13) der Antikörper oder das Antigenbindungsfragment ein Anti-CD38-Antikörper oder - Antigenbindungsfragment; ein Anti-CD46-Antikörper oder -Antigenbindungsfragment; ein Anti-CD48-Antikörper oder -Antigenbindungsfragment; oder ein Anti-CD79b-Antikörper oder - Antigenbindungsfragment ist.

23. Zusammensetzung, umfassend mehrere Kopien des Antikörper-Wirkstoff-Konjugats nach einem der Ansprüche 1 bis 22, wobei der Durchschnitt *p* der Antikörper-Wirkstoff-Konjugate in der Zusammensetzung von etwa 2 bis etwa 16, z. B. etwa 2 bis etwa 8, z. B. etwa 2 bis etwa 4 ist.

24. Pharmazeutische Zusammensetzung, umfassend das Antikörper-Wirkstoff-Konjugat nach einem der Ansprüche 1 bis 22 oder die Zusammensetzung nach Anspruch 23 und einen pharmazeutisch annehmbaren Träger.

25. Antikörper-Wirkstoff-Konjugat nach einem der Ansprüche 1 bis 22, Zusammensetzung nach Anspruch 23 oder pharmazeutische Zusammensetzung nach Anspruch 24 zur Verwendung beim Behandeln eines Subjekts mit oder unter Verdacht auf Krebs, wobei
(1) der Krebs ein Zielantigen exprimiert; oder
(2) der Krebs ein Tumor oder ein hämatologischer Krebs ist, optional der Krebs ein Brustkrebs, multiples Myelom, Plasmazellmyelom, Leukämie, Lymphom, Magenkrebs, akute myeloische Leukämie, Blasenkrebs, Hirnkrebs, Knochenmarkkrebs, Gebärmutterhalskrebs, chronische lymphatische Leukämie, Darmkrebs, Speiseröhrenkrebs, Leberzellkrebs, lymphoblastische Leukämie, follikuläres Lymphom, lymphatische Malignome mit T-Zell- oder B-Zell-Ursprung, Melanom, myelogene Leukämie, Myelom, Mundkrebs, Eierstockkrebs, nicht-kleinzelliger Lungenkrebs, chronische lymphatische Leukämie, Prostatakrebs, kleinzelliger Lungenkrebs oder Milzkrebs ist.

26. Antikörper-Wirkstoff-Konjugat nach einem der Ansprüche 1 bis 22, Zusammensetzung nach Anspruch 23 oder pharmazeutische Zusammensetzung nach Anspruch 24 zur Verwendung beim Reduzieren oder Inhibieren des Wachstums eines Tumors bei einem Subjekt, wobei
(1) der Tumor ein Zielantigen exprimiert;
(2) der Tumor ein Brustkrebs, Magenkrebs, Blasenkrebs, Hirnkrebs, Gebärmutterhalskrebs, Darmkrebs, Speiseröhrenkrebs, Leberzellkrebs, Melanom, Mundkrebs, Eierstockkrebs, nicht-kleinzelliger Lungenkrebs, Prostatakrebs, kleinzelliger Lungenkrebs oder Milzkrebs ist; oder
(3) Verabreichung des Antikörper-Wirkstoff-Konjugats, der Zusammensetzung oder der pharmazeutischen Zusammensetzung das Wachstum des Tumors um zumindest etwa 10 %, zumindest etwa 20 %, zumindest etwa 30 %, zumindest etwa 40 %, zumindest etwa 50 %, zumindest etwa 60 %, zumindest etwa 70 %, zumindest etwa 80 %, zumindest etwa 90 %, zumindest etwa 95 % oder zumindest etwa 99 % reduziert oder inhibiert.

27. Antikörper-Wirkstoff-Konjugat nach einem der Ansprüche 1 bis 22, Zusammensetzung nach Anspruch 23 oder pharmazeutische Zusammensetzung nach Anspruch 24 zur Verwendung beim Reduzieren oder Verlangsamen der Expansion einer Krebszellpopulation bei einem Subjekt, wobei
(1) die Krebszellenpopulation ein Zielantigen exprimiert;
(2) die Krebszellpopulation von einem Tumor oder einem hämatologischen Krebs ist, wobei optional die Krebszellpopulation von einem Brustkrebs, multiplem Myelom, Plasmazellmyelom, Leukämie, Lymphom, Magenkrebs, akuter myeloischer Leukämie, Blasenkrebs, Hirnkrebs, Knochenmarkkrebs, Gebärmutterhalskrebs, chronischer lymphatischer Leukämie, Darmkrebs, Speiseröhrenkrebs, Leberzellkrebs, lymphoblastischer Leukämie, follikulärem Lymphom, lymphatischen Malignomen mit T-Zell- oder B-Zell-Ursprung, Melanom, myelogener Leukämie, Myelom, Mundkrebs, Eierstockkrebs, nicht-kleinzelligem Lungenkrebs, chronischer lymphatischer Leukämie, Prostatakrebs, kleinzelligem Lungenkrebs oder Milzkrebs ist; oder
(3) Verabreichung des Antikörper-Wirkstoff-Konjugats, der Zusammensetzung oder der pharmazeutischen Zusammensetzung die Krebszellpopulation oder die Expansion der Krebszellpopulation um zumindest etwa 10 %, zumindest etwa 20 %, zumindest etwa 30 %, zumindest etwa 40 %, zumindest etwa 50 %, zumindest etwa 60 %, zumindest etwa 70 %, zumindest etwa 80 %, zumindest etwa 90 %, zumindest etwa 95 % oder zumindest etwa 99 % reduziert oder verlangsamt.

28. Verfahren zum Bestimmen, ob ein Subjekt mit oder unter Verdacht auf Krebs auf Behandlung mit dem Antikörper-Wirkstoff-Konjugat nach einem der Ansprüche 1 bis 22, der Zusammensetzung nach Anspruch 23 oder der pharmazeutischen Zusammensetzung nach Anspruch 24 anspricht, umfassend Bereitstellen einer biologischen Probe von dem Subjekt; Kontaktieren der Probe mit dem Antikörper-Wirkstoff-Konjugat; und Detektieren von Bindung des Antikörper-Wirkstoff-Konjugats an Krebszellen in der Probe; wobei
(1) die Krebszellen in der Probe ein Zielantigen exprimieren;
(2) der Krebs ein Zielantigen exprimiert;
(3) der Krebs ein Tumor oder ein hämatologischer Krebs ist, optional der Krebs ein Brustkrebs, multiples Myelom, Plasmazellmyelom, Leukämie, Lymphom, Magenkrebs, akute myeloische Leukämie, Blasenkrebs, Hirnkrebs, Knochenmarkkrebs, Gebärmutterhalskrebs, chronische lymphatische Leukämie, Darmkrebs, Speiseröhrenkrebs, Leberzellkrebs, lymphoblastische Leukämie, follikuläres Lymphom, lymphatische Malignome mit T-Zell- oder B-Zell-Ursprung, Melanom, myelogene Leukämie, Myelom, Mundkrebs, Eierstockkrebs, nicht-kleinzelliger Lungenkrebs, chronische lymphatische Leukämie, Prostatakrebs, kleinzelliger Lungenkrebs oder Milzkrebs ist; oder
(4) die Probe eine Gewebebiopsieprobe, eine Blutprobe oder eine Knochenmarkprobe ist.

29. Antikörper-Wirkstoff-Konjugat nach einem der Ansprüche 1 bis 22, Zusammensetzung nach Anspruch 23 oder pharmazeutische Zusammensetzung nach Anspruch 24 zur Verwendung nach einem der Ansprüche 25 bis 27 oder Verfahren nach Anspruch 28, wobei (i) das Zielantigen BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46 oder GPNMB ist; (ii) das Zielantigen BCMA, CD33, PCAD, HER2, CD38, CD46, CD48 oder CD79b; oder (iii) BCMA, CD33, CD48, PCAD oder HER2 ist.

30. Verfahren zum Produzieren des Antikörper-Wirkstoff-Konjugats nach einem der Ansprüche 1 bis 22, umfassend Reagieren eines Antikörpers oder Antigenbindungsfragments mit einem spaltbaren Linker, der mit einem MCL1-Inhibitor verknüpft ist, unter Bedingungen, die Konjugation ermöglichen.

31. Antikörper-Wirkstoff-Konjugat nach einem der Ansprüche 1 bis 22, Zusammensetzung nach Anspruch 23 oder pharmazeutische Zusammensetzung nach Anspruch 24 zur Verwendung nach einem der Ansprüche 25 bis 27 oder Verfahren nach Anspruch 28, ferner umfassend Verabreichen, an das Subjekt, das dessen bedarf, von zumindest einem zusätzlichen therapeutischen Mittel, wobei bevorzugt das eine zusätzliche therapeutische Mittel ein Bcl-28-Inhibitor ist, bevorzugter das eine zusätzliche therapeutische Mittel Venetoclax, Verbindung A1 oder Verbindung A2 ist.

32. Verbindung mit einer der folgenden Strukturen: 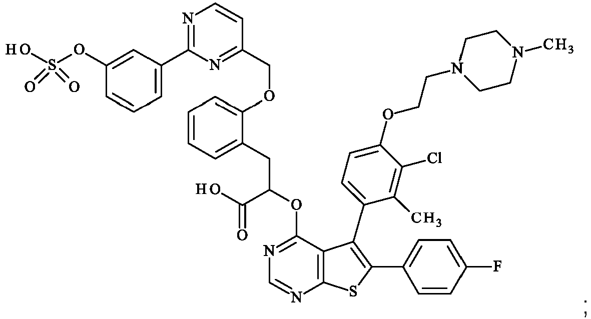 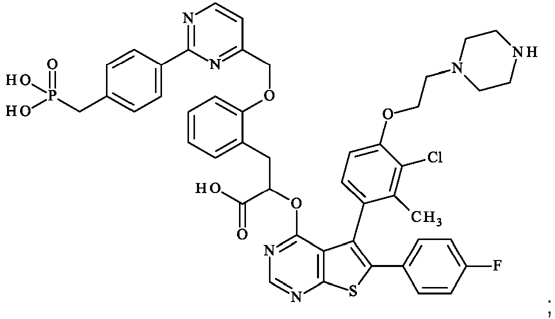 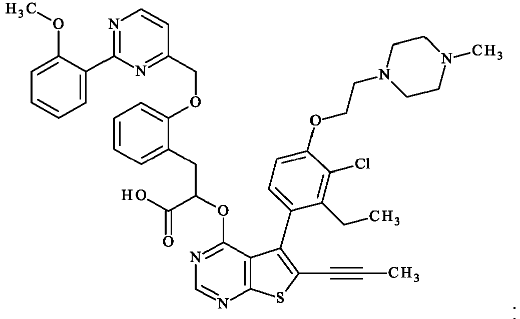 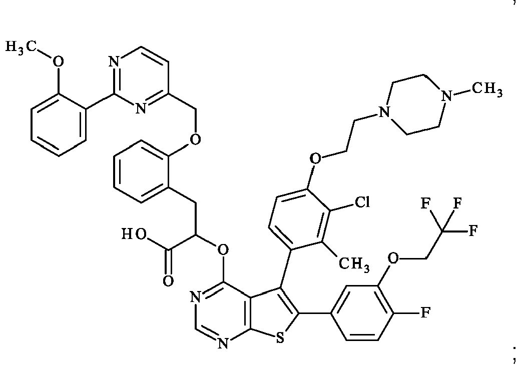 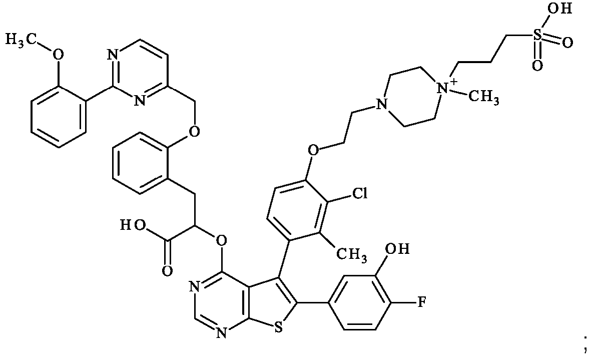 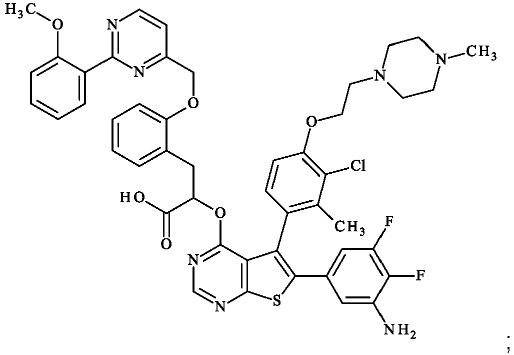 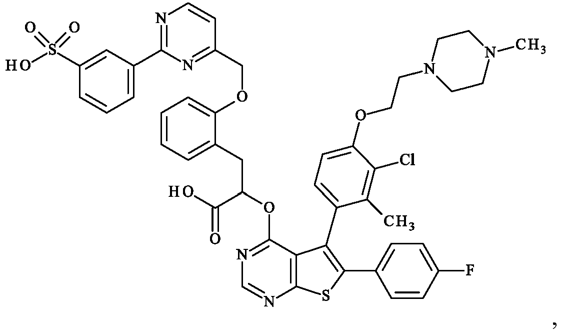 oder 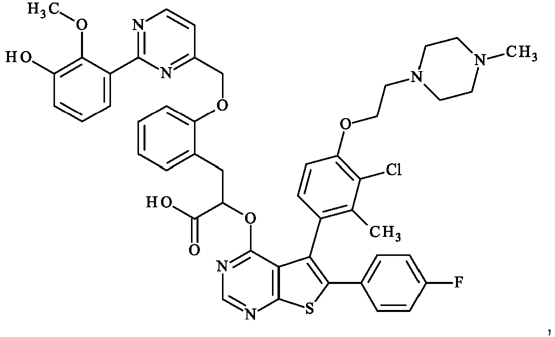 oder Salz davon (z. B. pharmazeutisch annehmbares Salz davon).

33. Verbindung nach Anspruch 32, wobei die Verbindung als Synthesezwischenprodukt für die Herstellung eines Antikörper-Wirkstoff-Konjugats der Formel (1) nach Anspruch 1 verwendet wird.

34. Verbindung nach Anspruch 32 oder Salz davon mit einer pharmazeutisch annehmbaren Säure oder Base zur Verwendung als pro-apoptotisches Mittel.

## Revendications

1. Conjugué anticorps-médicament de Formule (1) :
Ab-(L-D)ₚ (1)
où Ab est un anticorps ou un fragment de liaison à un antigène de celui-ci ;
D est un inhibiteur de Mcl-1, où D comprend un composé de Formule (II) : 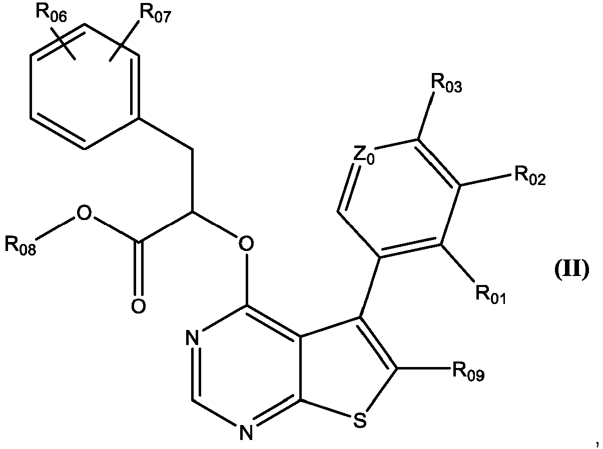
ou un énantiomère, un diastéréoisomère, un atropisomère, un dérivé deutéré et/ou un sel pharmaceutiquement acceptable de celui-ci ;
où :
Z₀ est un atome d'azote ou un groupe C-R₀₄,
R₀₁ est un atome d'halogène, un groupe alkyle en (C₁-C₆) linéaire ou ramifié, un groupe alcényle en (C₂-C₆) linéaire ou ramifié, un groupe alcynyle en (C₂-C₆) linéaire ou ramifié, un groupe halogénoalkyle en (C₁-C₆) linéaire ou ramifié, un groupe hydroxy, un groupe alcoxy en (C₁-C₆) linéaire ou ramifié, un groupe -S-alkyle en (C₁-C₆), un groupe cyano, -Cy₀₈, -NR₀₁₁R₀₁₁',
R₀₂, R₀₃ et R₀₄ sont, indépendamment l'un de l'autre, un atome d'hydrogène, un atome d'halogène, un groupe alkyle en (C₁-C₆) linéaire ou ramifié, un groupe alcényle en (C₂-C₆) linéaire ou ramifié, un groupe alcynyle en (C₂-C₆) linéaire ou ramifié, un halogénoalkyle en (C₁-C₆) linéaire ou ramifié, un groupe hydroxy, un groupe alcoxy en (C₁₋C₆) linéaire ou ramifié, un groupe -S-alkyle en (C₁-C₆), un groupe cyano, un groupe nitro, -alkyle en (C₀-C₆)-NR₀₁₁R₀₁₁', -O-Cy₀₁, - alkyle en (C₀-C₆)-Cy₀₁, -alcényle en (C₂-C₆)-Cy₀₁, -alcynyle en (C₂-C₆)-Cy₀₁, -O-alkyle en (C₁-C₆)-NR₀₁₁R₀₁₁', -O-alkyle en (C₁-C₆)-R₀₃₁, -C(O)-OR₀₁₁, -O-C(O)-R₀₁₁, -C(O)-NR₀₁₁R₀₁₁', - NR₀₁₁-C(O)-R₀₁₁', -NR₀₁₁-C(O)-OR₀₁₁', -alkyle en (C₁-C₆)-NR₀₁₁-C(O)-R₀₁₁', -SO₂-NR₀₁₁R₀₁₁' ou -SO₂-alkyle en (C₁-C₆), ou la paire (R₀₂, R₀₃) ou (R₀₃, R₀₄), conjointement avec les atomes de carbone auxquels elle est liée, forme un cycle aromatique ou non aromatique contenant 5 à 7 chaînons de cycle, qui contient éventuellement 1 à 3 hétéroatomes choisis parmi O, S et N, où le cycle est éventuellement substitué par un groupe choisi parmi un alkyle en (C₁-C₆) linéaire ou ramifié, -NR₀₁₃R₀₁₃', -alkyle en (C₀-C₆)-Cy₀₁ et un oxo,
R₀₆ et R₀₇ sont, indépendamment l'un de l'autre, un atome d'hydrogène, un atome d'halogène, un groupe alkyle en (C₁-C₆) linéaire ou ramifié, un groupe alcényle en (C₂-C₆) linéaire ou ramifié, un groupe alcynyle en (C₂-C₆) linéaire ou ramifié, un halogénoalkyle en (C₁-C₆) linéaire ou ramifié, un groupe hydroxy, un groupe alcoxy en (C₁-C₆) linéaire ou ramifié, un groupe -S-alkyle en (C₁-C₆), un groupe cyano, un groupe nitro, -alkyle en (C₀-C₆)-NR₀₁₁R₀₁₁', -O-Cy₀₁, - alkyle en (C₀-C₆)-Cy₀₁, -alcényle en (C₂-C₆)-Cy₀₁, -alcynyle en (C₂-C₆)-Cy₀₁, -O-alkyle en (C₁-C₆)-R₀₁₂, -C(O)-OR₀₁₁, -O-C(O)-R₀₁₁, -C(O)-NR₀₁₁R₀₁₁', -NR₀₁₁-C(O)-R_{011'}, -NR₀₁₁-C(O)-OR₀₁₁', -alkyle en (C₁-C₆)-NR₀₁₁-C(O)-R₀₁₁', -SO₂-NR₀₁₁R₀₁₁' ou -SO₂-alkyle en (C₁-C₆),
ou la paire (R₀₆, R₀₇), lorsqu'elle est fusionnée avec deux atomes de carbone adjacents, forme, conjointement avec les atomes de carbone auxquels elle est liée, un cycle aromatique ou non aromatique contenant 5 à 7 chaînons, qui contient éventuellement 1 à 3 hétéroatomes choisis parmi O, S et N, et où le cycle résultant est éventuellement substitué par un groupe choisi parmi un groupe alkyle en (C₁-C₆) linéaire ou ramifié, -NR₀₁₃R₀₁₃', -alkyle en (C₀-C₆)-Cy₀₁ et un oxo,
R₀₈ est un atome d'hydrogène, un groupe alkyle en (C₁-C₈) linéaire ou ramifié, un groupe aryle, un groupe hétéroaryle, un groupe aryle-alkyle en (C₁-C₆) ou un groupe hétéroaryle-alkyle en (C₁-C₆),
R₀₉ est un groupe alkyle en (C₁-C₆) linéaire ou ramifié, un groupe alcényle en (C₂-C₆) linéaire ou ramifié, un groupe alcynyle en (C₂-C₆) linéaire ou ramifié, -Cy₀₂, -alkyle en (C₁-C₆)-Cy₀₂, -alcényle en (C₂-C₆)-Cy₀₂, -alcynyle en (C₂-C₆)-Cy₀₂, -Cy₀₂-Cy₀₃, -alcynyle en (C₂-C₆)-O-Cy₀₂, -Cy₀₂-alkyle en (C₀-C₆)-O-alkyle en (C₀-C₆)-Cy₀₃, un atome d'halogène, un groupe cyano, - C(O)-R₀₁₄, -C(O)-NR₀₁₄R₀₁₄',
R₀₁₁ et R₀₁₁' sont, indépendamment l'un de l'autre, un atome d'hydrogène, un groupe alkyle en (C₁-C₆) linéaire ou ramifié éventuellement substitué ou -alkyle en (C₀-C₆)-Cy₀₁, ou la paire (R₀₁₁, R₀₁₁'), conjointement avec l'atome d'azote auquel elle est liée, forme un cycle aromatique ou non aromatique contenant 5 à 7 chaînons de cycle, qui contient éventuellement, en plus de l'atome d'azote, 1 à 3 hétéroatomes choisis parmi O, S et N, où l'atome N est éventuellement substitué par un groupe alkyle en (C₁-C₆) linéaire ou ramifié,
et où un ou plusieurs des atomes de carbone du groupe alkyle en (C₁-C₆) linéaire ou ramifié est éventuellement deutéré,
R₀₁₂ est -Cy₀₅, -Cy₀₅-alkyle en (C₀-C₆)-Cy₀₆, -Cy₀₅-alkyle en (C₀-C₆)-O-alkyle en (C₀-C₆)-Cy₀₆, -Cy₀₅-alkyle en (C₀-C₆)-NR₀₁₁-alkyle en (C₀-C₆)-Cy₀₆, -Cy₀₅-Cy₀₆-O-alkyle en (C₀-C₆)-Cy₀₇, -Cy₀₅-alkyle en (C₀-C₆)-Cy₀₉, -NH-C(O)-NH-R₀₁₁, -C(O)-NR₀₁₁R₀₁₁', -NR₀₁₁R₀₁₁', -OR₀₁₁, - NR₀₁₁-C(O)-R₀₁₁', -O-alkyle en (C₁-C₆)-OR₀₁₁, -SO₂-R₀₁₁ ou -C(O)-OR₀₁₁,
R₀₁₃, R₀₁₃', R₀₁₄ et R₀₁₄' sont, indépendamment les uns des autres, un atome d'hydrogène, ou un groupe alkyle en (C₁-C₆) linéaire ou ramifié éventuellement substitué,
Cy₀₁, Cy₀₂, Cy₀₃, Cy₀₅, Cy₀₆, Cy₀₇, Cy₀₈ et Cy₀₁₀ sont, indépendamment les uns des autres, un groupe cycloalkyle, un groupe hétérocycloalkyle, un groupe aryle ou un groupe hétéroaryle, dont chacun est éventuellement substitué,
Cy₀₉ est 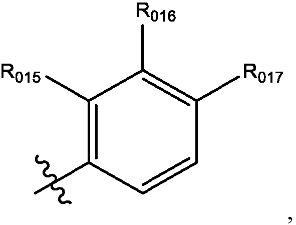
R₀₁₅ est un atome d'hydrogène ; un groupe -(CH₂)ₚ₀-O-(CHR₀₁₈-CHR₀₁₉-O)_{q0}-R₀₂₀ ; un groupe alcoxy en (C₁-C₆)-alkyle en (C₁₋C₆) linéaire ou ramifié ; un groupe -U₀-(CH₂)_{q0}-NR₀₂₁R₀₂₁' ; ou un groupe -(CH₂)ᵣ₀-U₀-(CH₂)ₛ₀-hétérocycloalkyle,
R₀₁₆ est un atome d'hydrogène ; un groupe hydroxy ; un groupe hydroxy-alkyle en (C₁-C₆) ; un groupe -(CH₂)ᵣ₀-U₀-(CH₂)ₛ₀-hétérocycloalkyle ; un groupe (CH₂)ᵣ₀-U₀-V₀-O-P(O)(OR₀₂₀)₂ ; un groupe -O-P(O)(O⁻M⁺)₂ ; un groupe -O-S(O)₂OR₀₂₀ ; un groupe -S(O)₂OR₀₂₀ ; un groupe -(CH₂)ₚ₀-O-(CHR₀₁₈-CHR₀₁₉-O)_{q0}-R₀₂₀ ; un groupe -(CH₂)ₚ₀-O-C(O)-NR₀₂₂R₀₂₃ ; ou un groupe -U₀-(CH₂)_{q0}-NR₀₂₁R₀₂₁',
R₀₁₇ est un atome d'hydrogène ; un groupe -(CH₂)ₚ₀-O-(CHR₀₁₈-CHR₀₁₉-O)_{q0}-R₀₂₀ ; un groupe -CH₂-P(O)(OR₀₂₀)₂, un groupe -O-P(O)(OR₀₂₀)₂ ; un groupe -O-P(O)(O⁻M⁺)₂ ; un groupe hydroxy ; un groupe hydroxy-alkyle en (C₁-C₆) ; un groupe -(CH₂)ᵣ₀-U₀-(CH₂)ₛ₀-hétérocycloalkyle ; un groupe -U₀-(CH₂)_{q0}-NR₀₂₁R₀₂₁' ; ou un acide aldonique,
M⁺ est un cation monovalent pharmaceutiquement acceptable,
U₀ est une liaison ou un atome d'oxygène,
V₀ est un groupe -(CH₂)ₛ₀- ou un groupe -C(O)-,
R₀₁₈ est un atome d'hydrogène ou un groupe alcoxy en (C₁-C₆)-alkyle en (C₁₋C₆),
R₀₁₉ est un atome d'hydrogène ou un groupe hydroxy-alkyle en (C₁-C₆),
R₀₂₀ est un atome d'hydrogène ou un groupe alkyle en (C₁-C₆) linéaire ou ramifié,
R₀₂₁ et R₀₂₁', sont indépendamment l'un de l'autre, un atome d'hydrogène, un groupe alkyle en (C₁-C₆) linéaire ou ramifié, ou un groupe hydroxy-alkyle en (C₁-C₆),
ou la paire (R₀₂₁, R₀₂₁'), conjointement avec l'atome d'azote auquel elle est liée, forme un cycle aromatique ou non aromatique contenant de 5 à 7 chaînons,
R₀₂₂ est un groupe alcoxy en (C₁-C₆)-alkyle en (C₁-C₆), un groupe -(CH₂)ₚ₀-NR₀₂₄R₀₂₄' ou un groupe -(CH₂)ₚ₀-O-(CHR₀₁₈-CHR₀₁₉-O)_{q0}-R₀₂₀,
R₀₂₃ est un atome d'hydrogène ou un groupe alcoxy en (C₁-C₆)-alkyle en (C₁-C₆),
ou la paire (R₀₂₂, R₀₂₃), conjointement avec l'atome d'azote auquel elle est liée, forme un cycle aromatique ou non aromatique contenant 5 à 18 chaînons de cycle, qui contient éventuellement, en plus de l'atome d'azote, 1 à 5 hétéroatomes choisis parmi O, S et N, où le cycle résultant est éventuellement substitué par un atome d'hydrogène, un groupe alkyle en (C₁-C₆) linéaire ou ramifié ou un groupe hétérocycloalkyle,
R₀₂₄ et R₀₂₄' sont, indépendamment l'un de l'autre, un atome d'hydrogène ou un groupe alkyle en (C₁-C₆) linéaire ou ramifié,
ou la paire (R₀₂₄, R₀₂₄'), conjointement avec l'atome d'azote auquel elle est liée, forme un cycle aromatique ou non aromatique composé de 5 à 7 chaînons de cycle, qui peut contenir, en plus de l'atome d'azote, 1 à 3 hétéroatomes choisis parmi O, S et N, et où le cycle résultant est éventuellement substitué par un atome d'hydrogène ou un groupe alkyle en (C₁-C₆) linéaire ou ramifié,
R₀₃₁ est 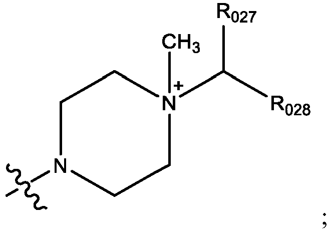
R₀₂₇ est un atome d'hydrogène ou un groupe alkyle en (C₁-C₆) linéaire ou ramifié,
R₀₂₈ est un groupe -O-P(O)(O⁻)(O⁻), un groupe -O-P(O)(O⁻)(OR₀₃₀), un groupe -O-P(O)(OR₀₃₀)(OR₀₃₀'), un groupe -(CH₂)ₚ₀-O-SO₂-O⁻, un groupe -(CH₂)ₚ₀-SO₂-O⁻, un groupe - (CH₂)ₚ₀-O-SO₂-OR₀₃₀, -Cy₀₁₀, un groupe -(CH₂)ₚ₀-SO₂-OR₀₃₀, un groupe -O-C(O)-R₀₂₉, un groupe -O-C(O)-OR₀₂₉ ou un groupe -O-C(O)-NR₀₂₉OR₀₂₉' ;
R₀₂₉ et R₀₂₉' sont, indépendamment l'un de l'autre, un atome d'hydrogène, un groupe alkyle en (C₁-C₆) linéaire ou ramifié ou un groupe amino-alkyle en (C₁-C₆) linéaire ou ramifié,
R₀₃₀ et R₀₃₀' sont, indépendamment l'un de l'autre, un atome d'hydrogène, un groupe alkyle en (C₁-C₆) linéaire ou ramifié ou un groupe aryle-alkyle en (C₁-C₆),
p₀ est un nombre entier égal à 0, 1, 2 ou 3,
q₀ est un nombre entier égal à 1, 2, 3 ou 4,
r₀ et s₀ sont indépendamment un nombre entier égal à 0 ou 1 ;
où l'un des groupes R₀₃, R₀₉ ou R₀₁₂ est lié de manière covalente au lieur, L est un lieur qui lie de manière covalente Ab à D ; et
*p* est un nombre entier de 1 à 16.

2. Conjugué anticorps-médicament de la revendication 1, dans lequel *p* est un nombre entier de 1 à 6 ou de 2 à 4, ou p vaut 2 ou 4 ; ou *p* est déterminé par chromatographie liquide-spectrométrie de masse (LC-MS).

3. Conjugué anticorps-médicament de l'une des revendications 1 ou 2, dans lequel L comprend :
un groupe de liaison ;
au moins un groupe espaceur pontant ; et
au moins un groupe clivable, éventuellement au moins un groupe clivable comprenant un groupe pyrophosphate et/ou un groupe auto-immolant.

4. Conjugué anticorps-médicament de la revendication 3, dans lequel -(L-D) répond à la formule (A) : 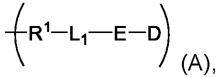 où :
R¹ est un groupe de liaison ;
L₁ est un groupe espaceur pontant ;
E est un groupe clivable.

5. Conjugué anticorps-médicament de l'une des revendications 3 ou 4, dans lequel
(1) le groupe clivable comprend un groupe pyrophosphate ou le groupe clivable comprend 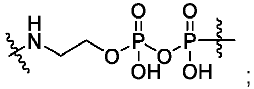
(2) le groupe espaceur pontant comprend :
(i) un groupe polyoxyéthylène (PEG) ;
(ii) un groupe PEG choisi parmi PEG1, PEG2, PEG3, PEG4, PEG5, PEG6, PEG7, PEG8, PEG9, PEG10, PEG11, PEG12, PEG13, PEG14 et PEG15 ;
(iii) un groupe -CO-CH₂-CH₂-PEG12- ;
(iv) un groupe butanoyle, pentanoyle, hexanoyle, heptanoyle ou octanoyle ; ou
(v) un groupe hexanoyle ;
(3)
(i) le groupe de liaison est formé à partir d'au moins un groupe réactif choisi parmi un groupe maléimide, un groupe thiol, un groupe cyclooctyne et un groupe azido ; éventuellement où :
a) le groupe maléimide présente la structure : 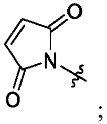
b) le groupe azido présente la structure : -N=N⁺=N⁻ ;
c) le groupe cyclooctyne présente la structure : 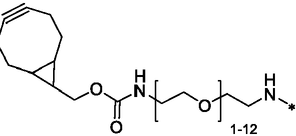 ou 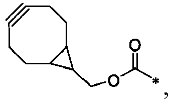 et où -* est une liaison à l'anticorps ou au fragment de liaison à l'antigène ; ou
d) le groupe cyclooctyne présente la structure : 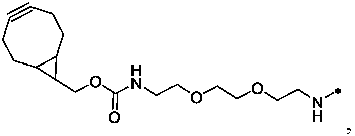 et
où -* est une liaison à l'anticorps ou au fragment de liaison à l'antigène ; ou
(ii) le groupe de liaison présente une formule comprenant : 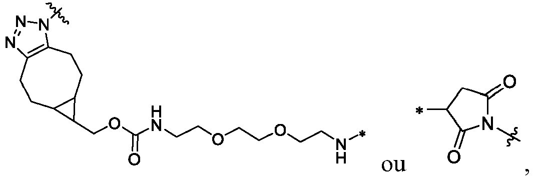 et
où -* est une liaison à l'anticorps ou au fragment de liaison à l'antigène ;
(4) l'anticorps ou le fragment de liaison à l'antigène est lié au lieur (L) par un groupe de liaison choisi parmi : 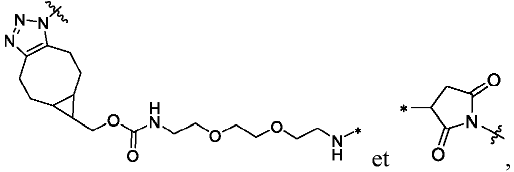 où -* est une liaison à l'anticorps ou au fragment de liaison à l'antigène, et où 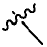 est une liaison au groupe espaceur pontant ;
(5) le groupe espaceur pontant est -CO-CH₂-CH₂-PEG12- ;
(6) le groupe espaceur pontant est lié à un groupe clivable ; éventuellement, le groupe clivable est -pyrophosphate-CH₂-CH₂-NH₂- ; ou
(7) le groupe clivable est lié à l'inhibiteur de Mcl-1 (D), ou le groupe clivable est lié à l'inhibiteur de Mcl-1 (D) par un groupe phényle-pyrimidinyle.

6. Conjugué anticorps-médicament de l'une quelconque des revendications 1 à 3, dans lequel le lieur comprend :
un groupe de liaison,
au moins un groupe espaceur pontant,
un groupe peptidique, et
au moins un groupe clivable.

7. Conjugué anticorps-médicament de la revendication 6, dans lequel -(L-D) répond à la formule (B) : 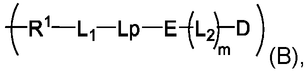 où :
R¹ est un groupe de liaison ;
L₁ est un espaceur pontant ;
Lp est un groupe peptidique comprenant 1 à 6 résidus d'acides aminés ou Lp comprend un groupe 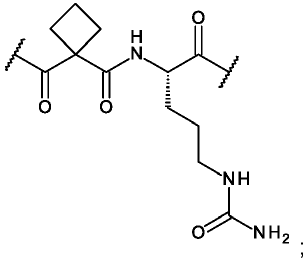
E est un groupe clivable
L₂ est un espaceur pontant ;
m vaut 0 ou 1 ; et
D est un inhibiteur de Mcl-1 tel que défini dans la revendication 1.

8. Conjugué anticorps-médicament de l'une des revendications 6 ou 7, dans lequel
(1)
(i) le groupe de liaison est formé à partir d'au moins un groupe réactif comprenant un groupe maléimide, un groupe thiol, un groupe cyclooctyne et/ou un groupe azido, éventuellement dans lequel :
a) le groupe maléimide présente la structure : 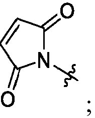
b) le groupe azido présente la structure : -N=N⁺=N⁻ ;
c) le groupe cyclooctyne présente la structure : 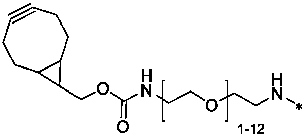 ou 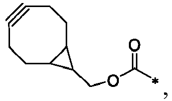 et où -* est une liaison à l'anticorps ou au fragment de liaison à l'antigène ; ou
d) le groupe cyclooctyne présente la structure : 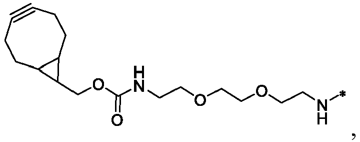 et
où -* est une liaison à l'anticorps ou au fragment de liaison à l'antigène ; ou
(ii) le groupe de liaison présente une formule comprenant : 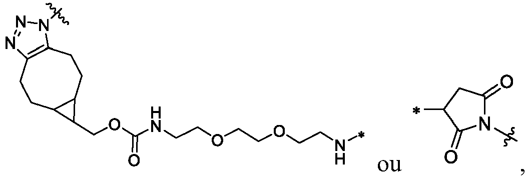 et
où -* est une liaison à l'anticorps ou au fragment de liaison à l'antigène ;
(2)
(i) au moins un espaceur pontant comprend un groupe PEG, éventuellement le groupe PEG est choisi parmi PEG1, PEG2, PEG3, PEG4, PEG5, PEG6, PEG7, PEG8, PEG9, PEG10, PEG11, PEG12, PEG13, PEG14 et PEG15 ; ou
(ii) au moins un espaceur pontant est choisi parmi *-C(O)-CH₂-CH₂-PEG1-**, *-C(O)-CH₂-PEG₃-**, *-C(O)-CH₂-CH₂-PEG12**, *-NH-CH₂-CH₂-PEG1-**, un groupe polyhydroxyalkyle et *-C(O)-N(CH₃)-CH₂-CH₂-N(CH₃)-C(O)-**, où ** indique le point de liaison directe ou indirecte de l'au moins un espaceur pontant au groupe de liaison et * indique le point de liaison directe ou indirecte de l'au moins un espaceur pontant au groupe peptidique ;
(3) L₁ est choisi parmi *-C(O)-CH₂-CH₂-PEG1-**, *-C(O)-CH₂-PEG3-**, *-C(O)-CH₂-CH₂-PEG12**, *-NH-CH₂-CH₂-PEG1-** et un groupe polyhydroxyalkyle, où ** indique le point de liaison directe ou indirecte de L₁ à R¹ et * indique le point de liaison directe ou indirecte de L₁ à Lp ;
(4) m vaut 1 et L₂ est -C(O)-N(CH₃)-CH₂-CH₂-N(CH₃)-C(O)- ;
(5)
(i) le groupe peptidique comprend 1 à 6, 1 à 4, 1 à 3 ou 1 à 2 résidus d'acides aminés, éventuellement, les résidus d'acides aminés sont choisis parmi la L-glycine (Gly), la L-valine (Val), la L-citrulline (Cit), l'acide L-cystéique (sulfo-Ala), la L-lysine (Lys), la L-isoleucine (Ile), la L-phénylalanine (Phe), la L-méthionine (Met), la L-asparagine (Asn), la L-proline (Pro), la L-alanine (Ala), la L-leucine (Leu), le L-tryptophane (Trp) et la L-tyrosine (Tyr) ;
(ii) le groupe peptidique comprend Val-Cit, Val-Ala, Val-Lys et/ou sulfo-Ala-Val-Ala ;
(iii) le groupe peptidique est choisi parmi : 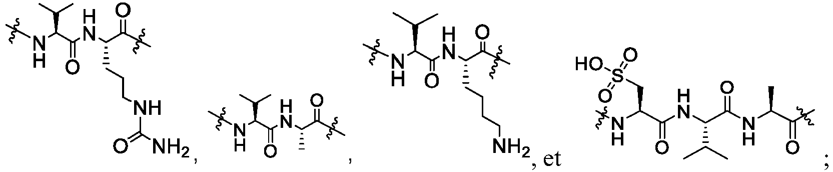
(6) (i) le groupe clivable comprend un pyrophosphate et/ou un groupe auto-immolant ; (ii) le groupe clivable comprend un groupe auto-immolant ; ou (iii) le groupe clivable comprend un groupe auto-immolant comprenant un para-aminobenzyle-carbamate, un para-aminobenzyle-ammonium, un para-amino-(sulfo)benzyle-ammonium, un para-amino-(sulfo)benzyle-carbamate, un para-amino-(alcoxy-PEG-alkyle)benzyle-carbamate, un para-amino-(polyhydroxycarboxytétrahydropyranyle)alkyle-benzyle-carbamate ou un para-amino-(polyhydroxycarboxytétrahydropyranyle)alkyle-benzyle-ammonium ; ou
(7) m vaut 0 ou 1 ou m vaut 1 et l'espaceur pontant comprend

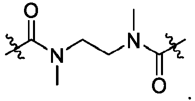

9. Conjugué anticorps-médicament de l'une des revendications 7 ou 8, dans lequel
(1) -(L-D) est formé à partir d'un composé choisi parmi : 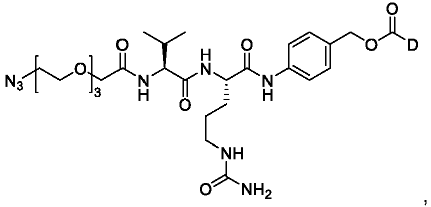 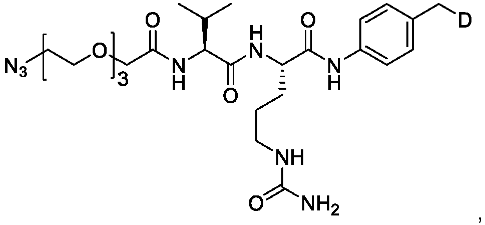 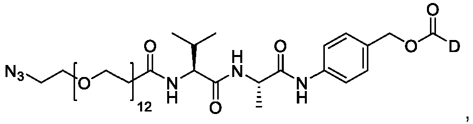 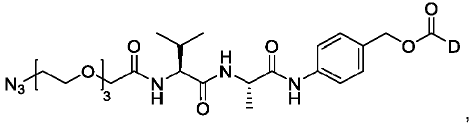 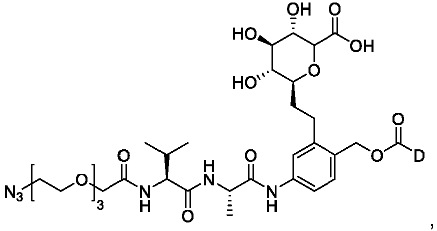 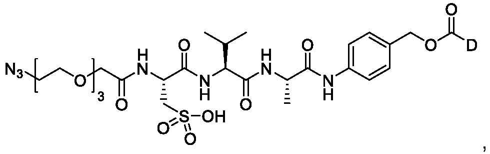 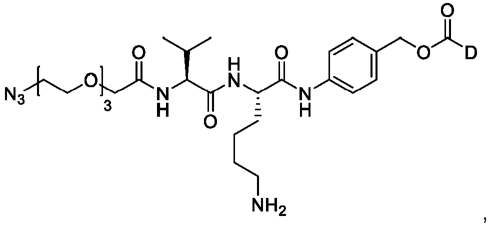 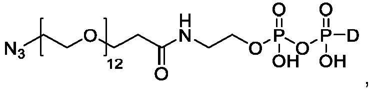 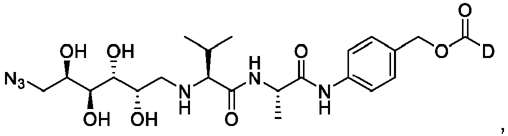 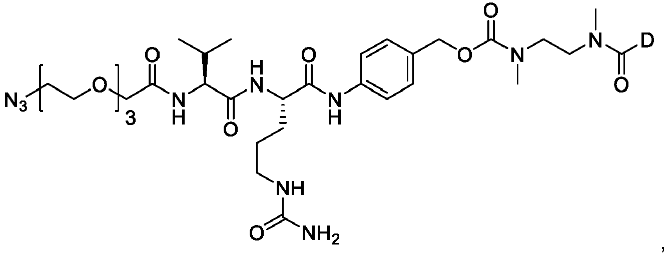 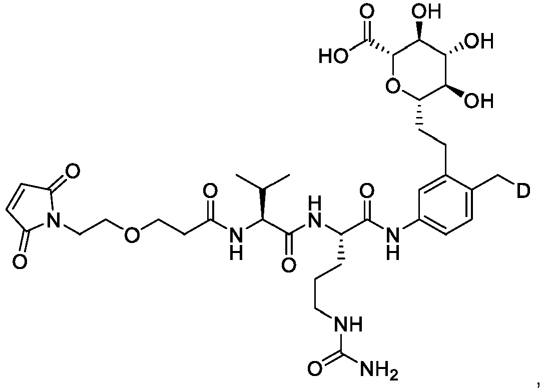 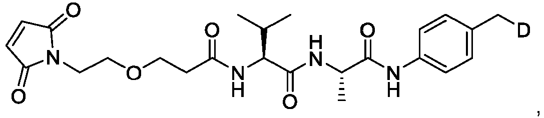 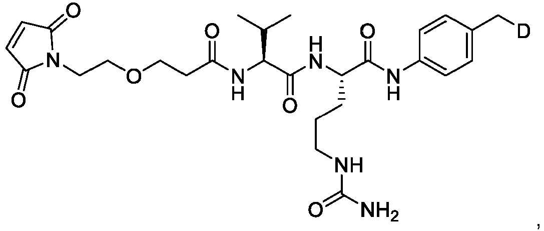 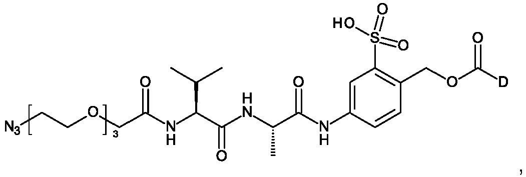 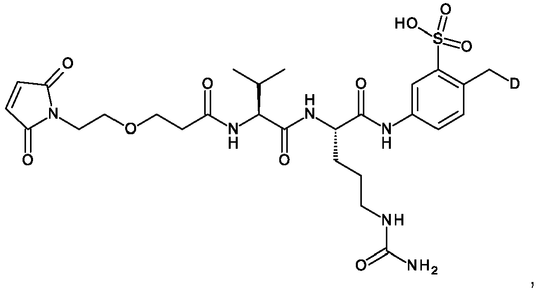 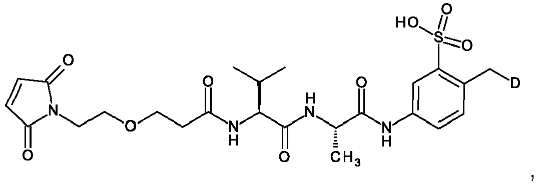 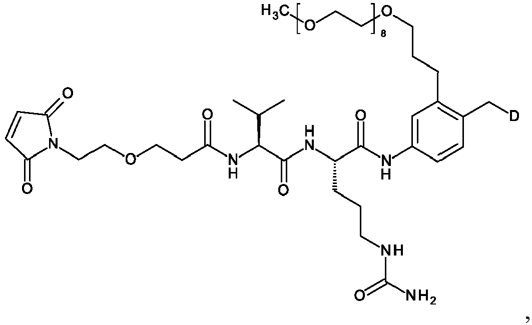 et 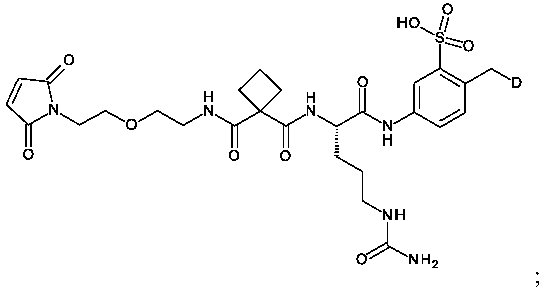 ou
(2) -(L-D) comprend une formule choisie parmi : 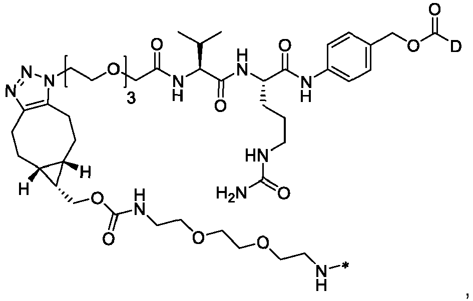 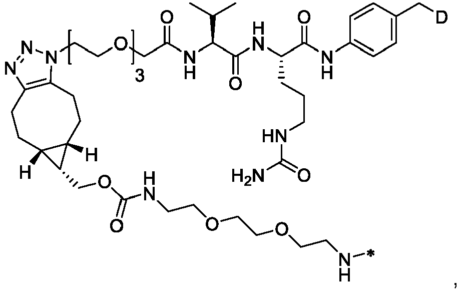 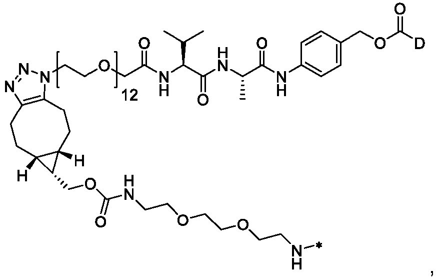 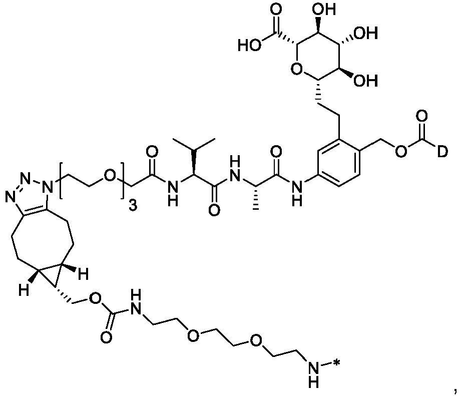 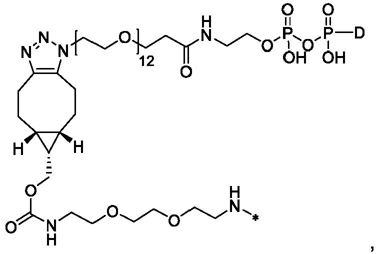 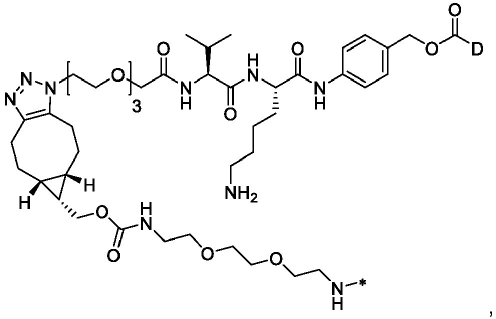 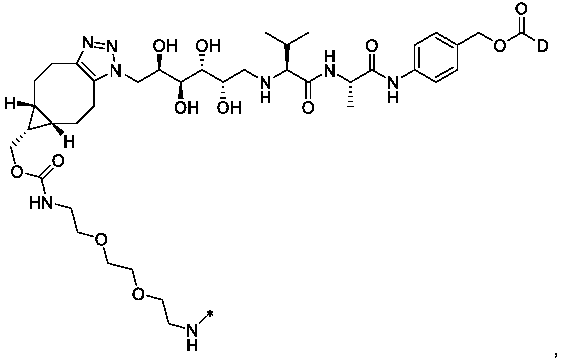 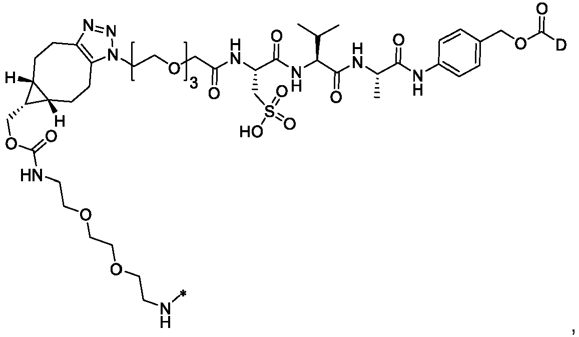 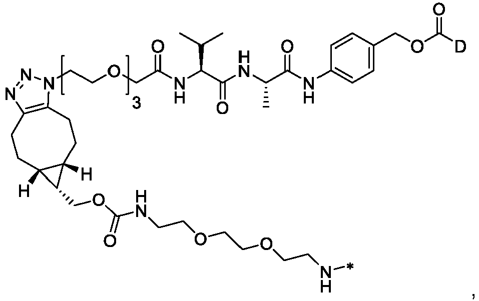 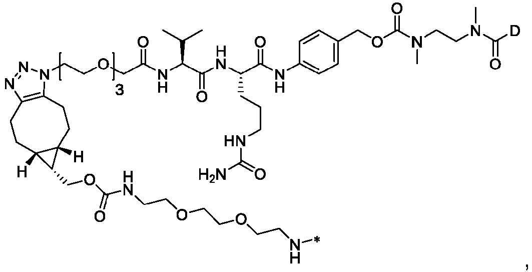 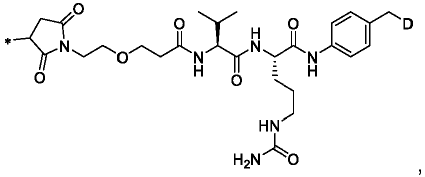 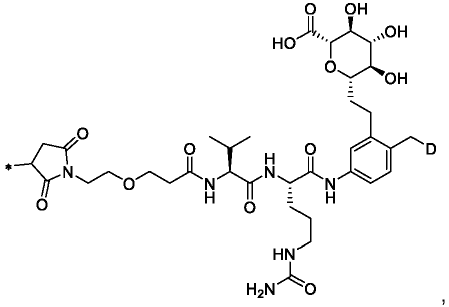 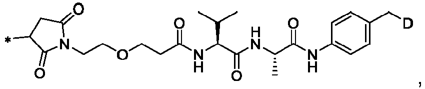 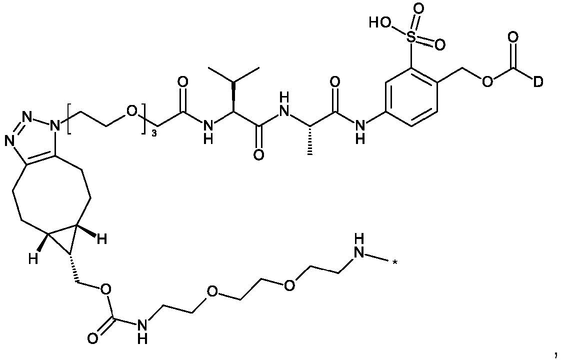 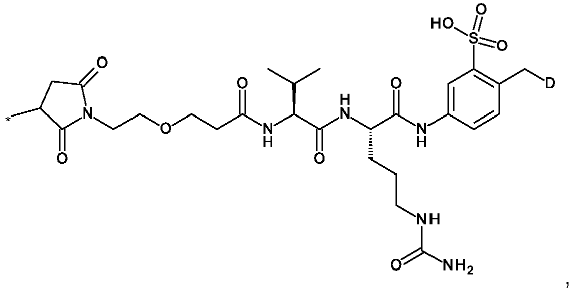 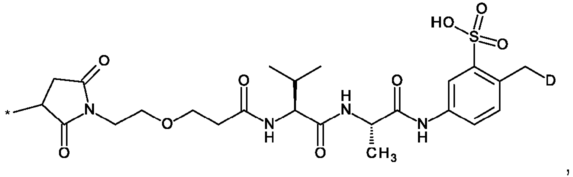 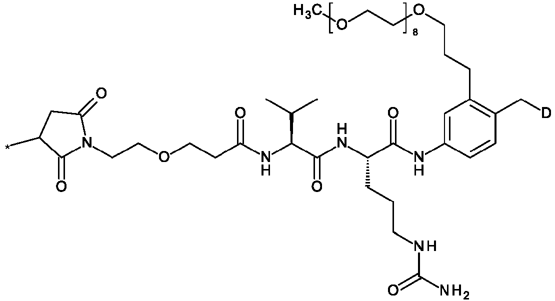 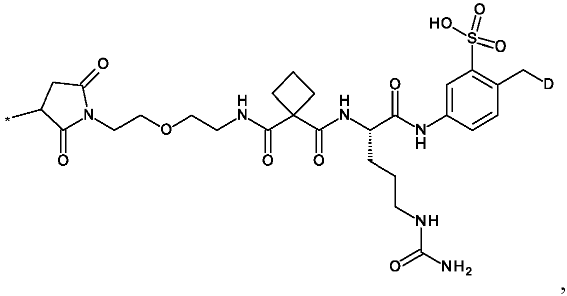 et
où -* est une liaison à l'anticorps ou au fragment de liaison à l'antigène.

10. Conjugué anticorps-médicament de l'une des revendications 1 ou 2, dans lequel
(1) -(L-D) répond à la formule (C) : 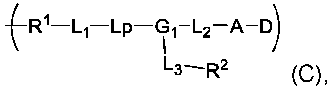 où :
R¹ est un groupe de liaison ;
L₁ est un espaceur pontant ;
Lₚ est un groupe peptidique comprenant 1 à 6 acides aminés ;
D est un inhibiteur de Mcl-1 tel que défini dans la revendication 1 ;
G₁-L₂-A est un espaceur auto-immolant ;
L₂ est une liaison, un méthylène, un néopentylène ou un alcénylène en C₂-C₃ ;
A est une liaison, -OC(=O)-*, 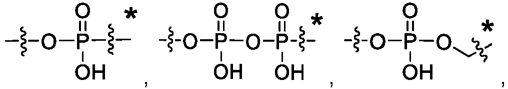 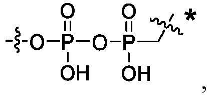 -OC(=O)N(CH₃)CH₂CH₂N(CH₃)C(=O)-* ou -OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ;
L₃ est un fragment espaceur ; et
R² est un fragment hydrophile ; ou
(2) -(L-D) répond à la Formule (D) : 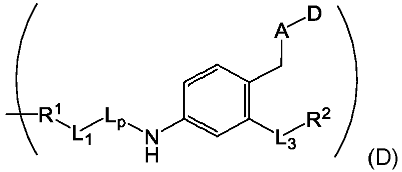 où :
R¹ est un groupe de liaison ;
L₁ est un espaceur pontant ;
Lp est un groupe peptidique comprenant 1 à 6 acides aminés ;
A est une liaison, -OC(=O)-*, 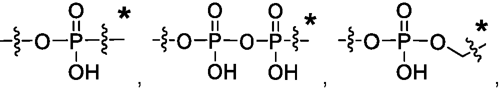 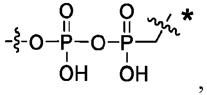 -OC(=O)N(CH₃)CH₂CH₂N(CH₃)C(=O)-* ou -OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ;
L₃ est un fragment espaceur ; et
R² est un fragment hydrophile.

11. Conjugué anticorps-médicament de la revendication 10, dans lequel :
(1) L₁ comprend : 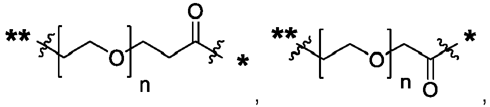 ou
*-CH(OH)CH(OH)CH(OH)CH(OH)-**,
où chaque n est un nombre entier de 1 à 12, où * de L₁ indique le point de liaison directe ou indirecte à Lp, et ** de L₁ indique le point de liaison directe ou indirecte à R¹ ;
(2) L₁ est 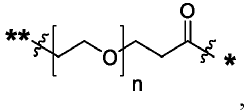 et n est un nombre entier de 1 à 12 ou n vaut 1 ou n vaut 12, où * de L₁ indique le point de liaison directe ou indirecte à Lp, et ** de L₁ indique le point de liaison directe ou indirecte à R¹ ;
(3) L₁ est 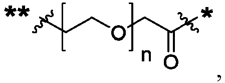 et n est un nombre entier de 1 à 12, où * de L₁ indique le point de liaison directe ou indirecte à Lp, et ** de L₁ indique le point de liaison directe ou indirecte à R¹ ;
(4) L₁ comprend 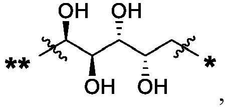 où * de L₁ indique le point de liaison directe ou indirecte à Lp, et ** de L₁ indique le point de liaison directe ou indirecte à R¹ ;
(5) L₁ est un espaceur pontant comprenant :
*-C(=O)(CH₂)ₘO(CH₂)ₘ-** ; *-C(=O)((CH₂)ₘO)ₜ(CH₂)ₙ-** ; *-C(=O)(CH₂)ₘ-** ;
*-C(=O)NH((CH₂)ₘO)ₜ(CH₂)ₙ-** ;
*-C(=O)O(CH₂)ₘSSC(R³)₂(CH₂)ₘC(=O)NR³(CH₂)ₘNR³C(=O)(CH₂)ₘ-** ;
*-C(=O)O(CH₂)ₘC(=O)NH(CH₂)ₘ-** ; *-C(=O)(CH₂)ₘNH(CH₂)ₘ-** ;
*-C(=O)(CH₂)ₘNH(CH₂)ₙC(=O)-** ; *-C(=O)(CH₂)ₘX₁(CH₂)ₘ-** ;
*-C(=O)((CH₂)ₘO)ₜ(CH₂)ₙX₁(CH₂)ₙ-** ; *-C(=O)(CH₂)ₘNHC(=O)(CH₂)ₙ-** ;
*-C(=O)((CH₂)ₘO)ₜ(CH₂)ₙNHC(=O)(CH₂)ₙ-** ;
*-C(=O)(CH₂)ₘNHC(=O)(CH₂)ₙX₁(CH₂)ₙ-** ;
*-C(=O)((CH₂)ₘO)ₜ(CH₂)ₙNHC(=O)(CH₂)ₙX₁(CH₂)ₙ-** ;
*-C(=O)((CH₂)ₘO)ₜ(CH₂)ₙC(=O)NH(CH₂)ₘ-** ; *-C(=O)(CH₂)ₘC(R³)₂-**
ou
*-C(=O)(CH₂)ₘC(=O)NH(CH₂)ₘ-**,
où * de L₁ indique le point de liaison directe ou indirecte à Lp, et ** de L₁ indique le point de liaison directe ou indirecte à R¹ ;
X₁ est 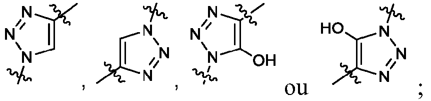 et
chaque m est indépendamment choisi parmi 1, 2, 3, 4, 5, 6, 7, 8, 9 et 10 ;
chaque n est indépendamment choisi parmi 1, 2, 3, 4, 5, 6, 7, 8, 9 et 10 ; et
chaque t est indépendamment choisi parmi 1, 2, 3, 4, 5, 6, 7, 8, 9, 10, 11, 12, 13, 14, 15, 16, 17, 18, 19, 20, 21, 22, 23, 24, 25, 26, 27, 28, 29 et 30 ;
et chaque R³ est indépendamment choisi parmi H et un alkyle en C₁-C₆.

12. Conjugué anticorps-médicament de l'une des revendications 10 ou 11, dans lequel
(1) R² est un fragment hydrophile comprenant un polyéthylène glycol, un polyalkylène glycol, un polyol, une polysarcosine, un sucre, un oligosaccharide, un polypeptide ou un alkyle en C₂-C₆ substitué par 1 à 3 groupes 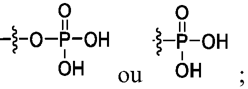
(2) R² est 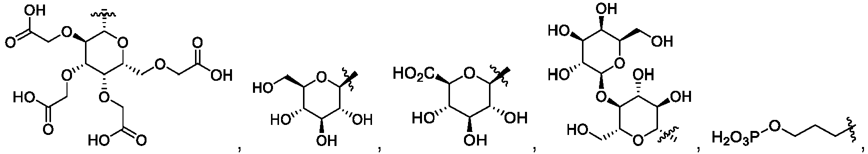 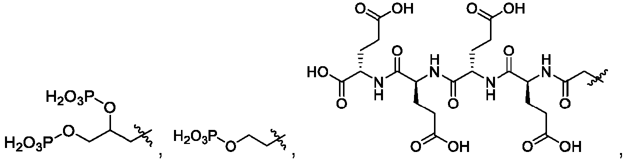 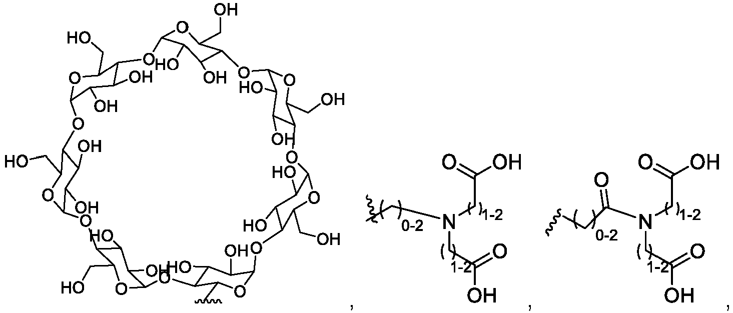 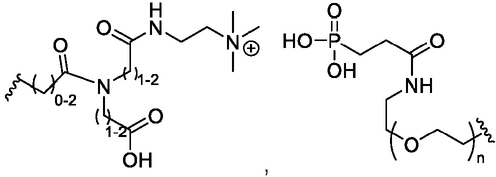 où n est un nombre entier compris entre 1 et 6, 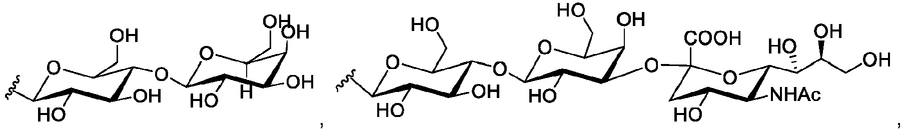 ou 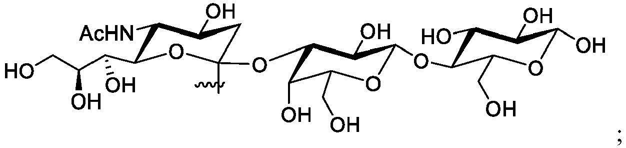
(3) le fragment hydrophile représenté par R² comprend :
(i) une polysarcosine, par exemple, avec le fragment suivant : 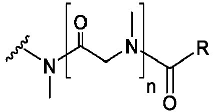 où n est un nombre entier compris entre 3 et 25 ; et R est H, -CH₃ ou -CH₂CH₂C(=O)OH ; ou
(ii) un polyéthylène glycol de formule : 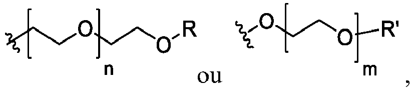 où R est H, -CH₃, CH₂CH₂NHC(=O)ORₐ, -CH₂CH₂NHC(=O)Rₐ ou -CH₂CH₂C(=O)ORₐ, R' est OH, - OCH₃, -CH₂CH₂NHC(=O)ORₐ, -CH₂CH₂NHC(=O)Rₐ ou -OCH₂CH₂C(=O)ORₐ, où Rₐ est H ou un alkyle en C₁-₄ éventuellement substitué par OH ou un alcoxyle en C₁-₄, et chacun de m et n est indépendamment un nombre entier compris entre 2 et 25 ; ou
(4) le fragment hydrophile représenté par R² comprend

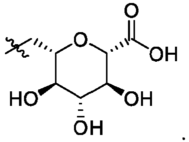

13. Conjugué anticorps-médicament de l'une quelconque des revendications 10 à 12, dans lequel :
(i) L₃ est un fragment espaceur présentant la structure 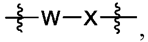 où :
W est -CH₂-, -CH₂O-, -CH₂N(R^{b})C(=O)O-, -NHC(=O)C(R^{b})₂NHC(=O)O-, - NHC(=O)C(R^{b})₂NH-, -NHC(=O)C(R^{b})₂NHC(=O)-, -CH₂N(X-R²)C(=O)O-, -C(=O)N(X-R²)-, - CH₂N(X-R²)C(=O)-, -C(=O)NR^{b}-, -C(=O)NH-, -CH₂NR^{b}C(=O)-, -CH₂NR^{b}C(=O)NH-, - CH₂NR^{b}C(=O)NR^{b}-, -NHC(=O)-, -NHC(=O)O-, -NHC(=O)NH-, -OC(=O)NH-, -S(O)₂NH-, - NHS(O)₂-, -C(=O)-, -C(=O)O - ou -NH-, où chaque R^{b} est indépendamment choisi parmi H, un alkyle en C₁-C₆ et un cycloalkyle en C₃-C₈ ; et
X est une liaison, un triazolyle ou -CH₂-triazolyle- ; ou
(ii) L₃ est un fragment espaceur présentant la structure 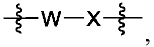 où :
W est -CH₂-, -CH₂O-, -CH₂N(R^{b})C(=O)O-, -NHC(=O)C(R^{b})₂NHC(=O)O-, - NHC(=O)C(R^{b})₂NH-, -NHC(=O)C(R^{b})₂NHC(=O)-, -CH₂N(X-R²)C(=O)O-, -C(=O)N(X-R²)-, - CH₂N(X-R²)C(=O)-, -C(=O)NR^{b}-, -C(=O)NH-, -CH₂NR^{b}C(=O)-, -CH₂NR^{b}C(=O)NH-, - CH₂NR^{b}C(=O)NR^{b}-, -NHC(=O)-, -NHC(=O)O-, -NHC(=O)NH-, -OC(=O)NH-, -S(O)₂NH-, - NHS(O)₂-, -C(=O)-, -C(=O)O- ou -NH-, où chaque R^{b} est indépendamment choisi parmi H, un alkyle en C₁-C₆ et un cycloalkyle en C₃-C₈ ; et
X est -CH₂-triazolyle-alkylène en C₁₋₄-OC(O)NHS(O)₂NH-, -cycloalkylène en C₄₋₆-OC(O)NHS(O)₂NH-, -(CH₂CH₂O)ₙ-C(O)NHS(O)₂NH-, -(CH₂CH₂O)ₙ-C(O)NHS(O)₂NH-(CH₂CH₂O)ₙ-, ou
-CH₂-triazolyle-alkylène en C₁₋₄-OC(O)NHS(O)₂NH-(CH₂CH₂O)ₙ-, où chaque n vaut indépendamment 1, 2 ou 3.

14. Conjugué anticorps-médicament de l'une quelconque des revendications 3 à 13, dans lequel
(1) le groupe de liaison est formé par une réaction comprenant au moins un groupe réactif ;
(2) le groupe de liaison est formé en faisant réagir :
un premier groupe réactif lié au lieur, et
un second groupe réactif lié à l'anticorps ou au fragment de liaison à l'antigène ou est un résidu d'acide aminé de l'anticorps ou du fragment de liaison à l'antigène, dans lequel éventuellement, (i) au moins l'un des groupes réactifs comprend :
un thiol,
un maléimide,
un halogénoacétamide,
un azide,
un alcyne,
un cyclooctène,
une triarylphosphine,
un oxanorbomadiène,
un cyclooctyne,
une diaryltétrazine,
une monoaryltétrazine,
un norbomène,
un aldéhyde,
une hydroxylamine,
une hydrazine,
NH₂-NH-C(=O)-,
une cétone,
une vinylsulfone,
une aziridine,
un résidu d'acide aminé, 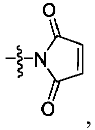 -ONH₂, -NH₂, 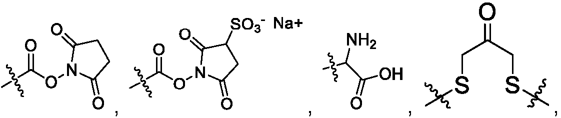 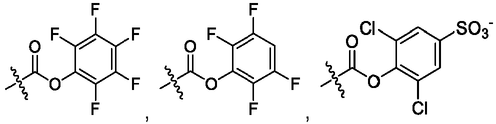 -N₃, 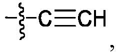 -SH, -SR³, -SSR⁴, - S(=O)₂(CH=CH₂), -(CH₂)₂S(=O)₂(CH=CH₂), -NHS(=O)₂(CH=CH₂), -NHC(=O)CH₂Br, - NHC(=O)CH₂I, 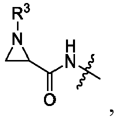 -C(O)NHNH₂, 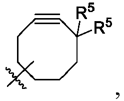 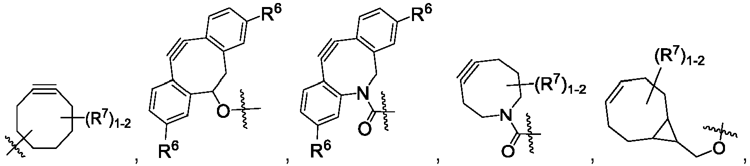 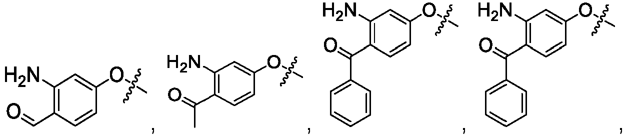 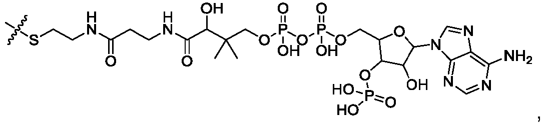 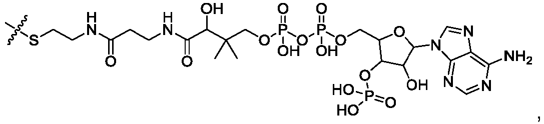 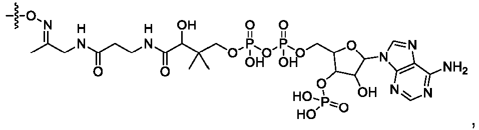 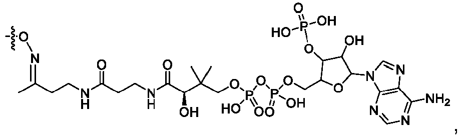 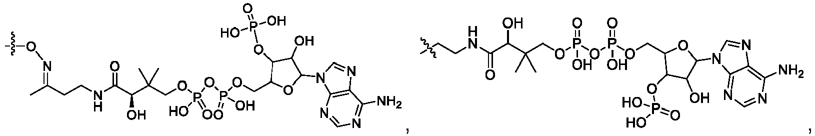 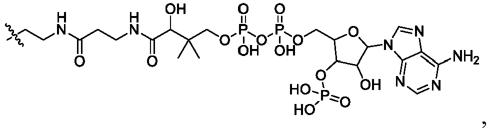 ou 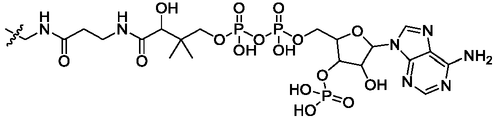
où :
chaque R³ est indépendamment choisi parmi H et un alkyle en C₁-C₆ ;
chaque R⁴ est le 2-pyridyle ou le 4-pyridyle ;
chaque R⁵ est indépendamment choisi parmi H, un alkyle en C₁-C₆, F, Cl et -OH ;
chaque R⁶ est indépendamment choisi parmi H, un alkyle en C₁-C₆, F, Cl, -NH₂, -OCH₃, - OCH₂CH₃, -N(CH₃)₂, -CN, -NO₂ et -OH ;
chaque R⁷ est indépendamment choisi parmi H, un alkyle en C₁₋₆, un fluoro, un benzyloxy substitué par -C(=O)OH, un benzyle substitué par -C(=O)OH, un alcoxy en C₁₋₄ substitué par - C(=O)OH et un alkyle en C₁₋₄ substitué par -C(=O)OH ; et/ou
(ii) le premier groupe réactif et le second groupe réactif comprennent :
un thiol et un maléimide,
un thiol et un halogénoacétamide,
un thiol et une vinylsulfone,
un thiol et une aziridine,
un azide et un alcyne,
un azide et un cyclooctyne,
un azide et un cyclooctène,
un azide et une triarylphosphine,
un azide et un oxanorbornadiène,
une diaryltétrazine et un cyclooctène,
une monoaryltétrazine et un norbornène,
un aldéhyde et une hydroxylamine,
un aldéhyde et une hydrazine,
un aldéhyde et NH₂-NH-C(=O)-,
une cétone et une hydroxylamine,
une cétone et une hydrazine,
une cétone et NH₂-NH-C(=O)-,
une hydroxylamine et 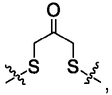
une amine et 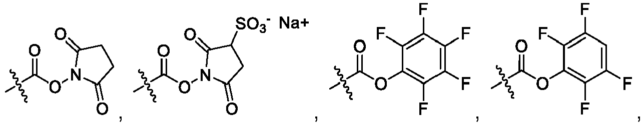 ou 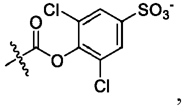 ou
une CoA ou un analogue de CoA et un résidu de sérine ; ou
(3) le groupe de liaison comprend un groupe choisi parmi : 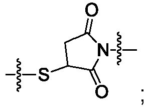 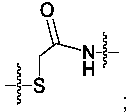 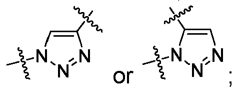 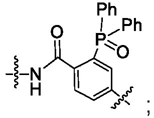 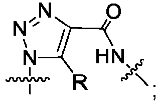 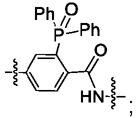 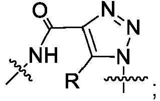 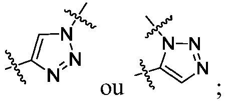 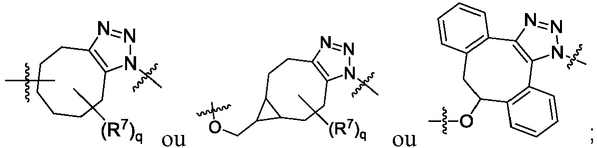 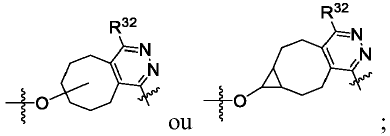 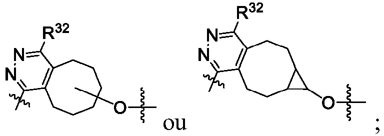 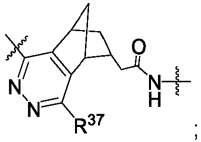 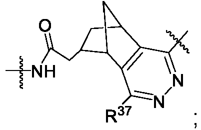 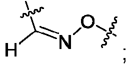 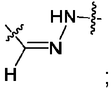 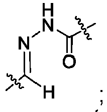 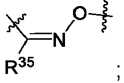 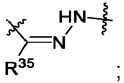 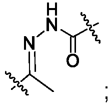 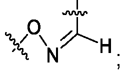 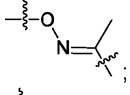 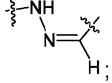 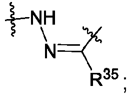 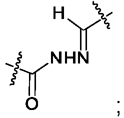 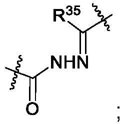 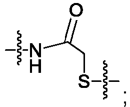 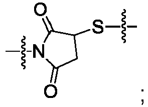 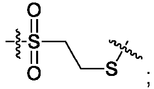 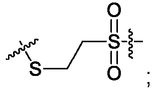 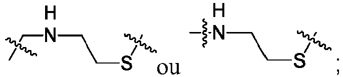 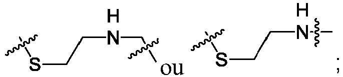 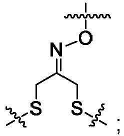 un amide ; 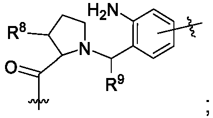 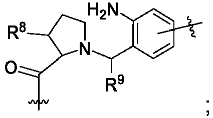 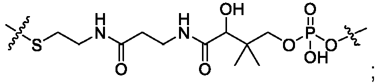 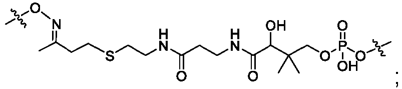 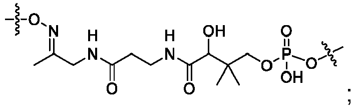 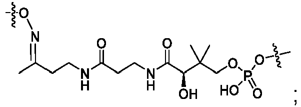 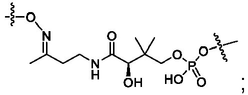 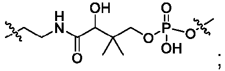 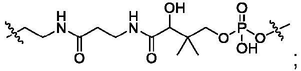 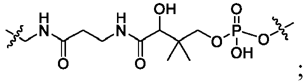 et
un disulfure,
où :
R³² est H, un alkyle en C₁₋₄, un phényle, une pyrimidine ou une pyridine ;
R³⁵ est H, un alkyle en C₁₋₆, un phényle ou un alkyle en C₁₋₄ substitué par 1 à 3 groupes - OH ;
chaque R⁷ est indépendamment choisi parmi H, un alkyle en C₁₋₆, un fluoro, un benzyloxy substitué par -C(=O)OH, un benzyle substitué par -C(=O)OH, un alcoxy en C₁₋₄ substitué par - C(=O)OH et un alkyle en C₁₋₄ substitué par -C(=O)OH ;
R³⁷ est indépendamment choisi parmi H, un phényle et une pyridine ;
q vaut 0, 1, 2 ou 3 ;
R⁸ est H ou un méthyle ; et
R⁹ est H, -CH₃ ou un phényle.

15. Conjugué anticorps-médicament de l'une quelconque des revendications 10 à 14, dans lequel
(1) le groupe peptidique représenté par Lp comprend 1 à 4 ou 1 à 3 ou 1 ou 2 résidus d'acides aminés, éventuellement les résidus d'acides aminés sont choisis parmi la L-glycine (Gly), la L-valine (Val), la L-citrulline (Cit), l'acide L-cystéique (sulfo-Ala), la L-lysine (Lys), la L-isoleucine (Ile), la L-phénylalanine (Phe), la L-méthionine (Met), la L-asparagine (Asn), la L-proline (Pro), la L-alanine (Ala), la L-leucine (Leu), le L-tryptophane (Trp) et la L-tyrosine (Tyr) ;
(2) le groupe peptidique représenté par Lp comprend Val-Cit, Phe-Lys, Val-Ala, Val-Lys, Leu-Cit, sulfo-Ala-Val et/ou sulfo-Ala-Val-Ala ; ou
(3) Lp est choisi parmi :

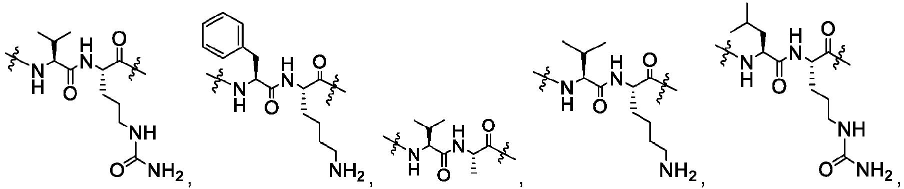

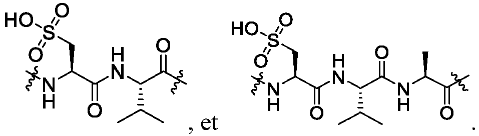

16. Conjugué anticorps-médicament de l'une quelconque des revendications 10 à 15, dans lequel :
-(L-D) comprend ou est formé à partir d'un composé de formule :
(1) 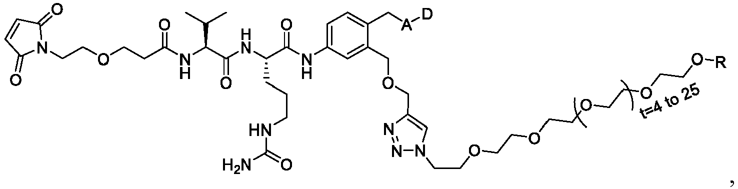 où :
R est H, -CH₃ ou -CH₂CH₂C(=O)OH ;
A est une liaison, -OC(=O)-*, 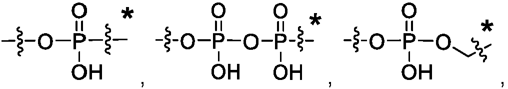 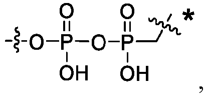 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(2) 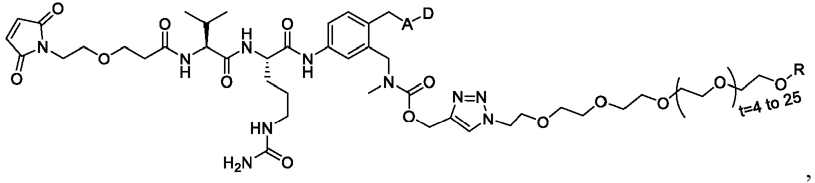 où :
R est H, -CH₃ ou -CH₂CH₂C(=O)OH ;
A est une liaison, -OC(=O)-*, 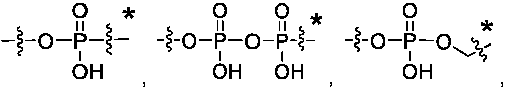 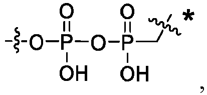 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(3) 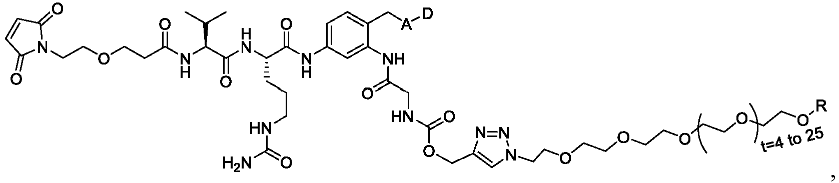 où :
R est H, -CH₃ ou -CH₂CH₂C(=O)OH ;
A est une liaison, -OC(=O)-*, 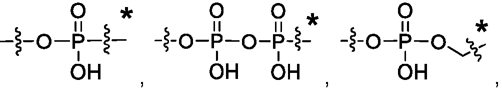 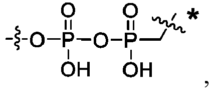 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(4) 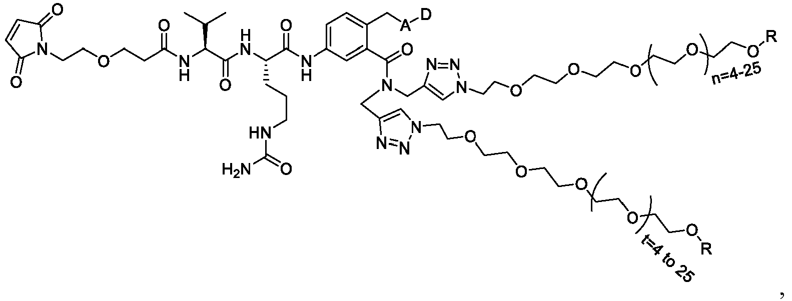 où :
chaque R est indépendamment choisi parmi H, -CH₃ et -CH₂CH₂C(=O)OH ;
A est une liaison, -OC(=O)-*, 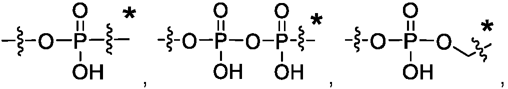 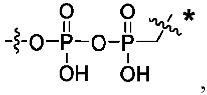 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(5) 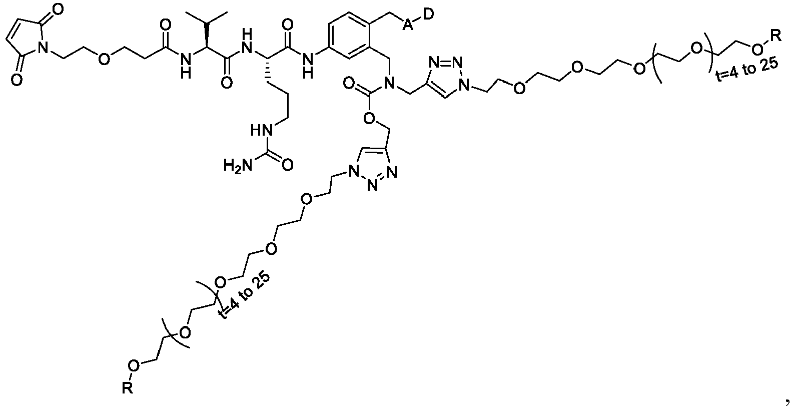 où :
chaque R est indépendamment choisi parmi H, -CH₃ et -CH₂CH₂C(=O)OH ;
A est une liaison, -OC(=O)-*, 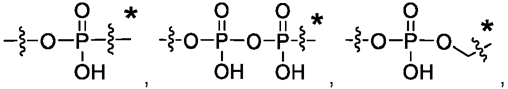 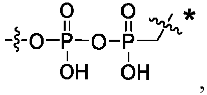 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(6) 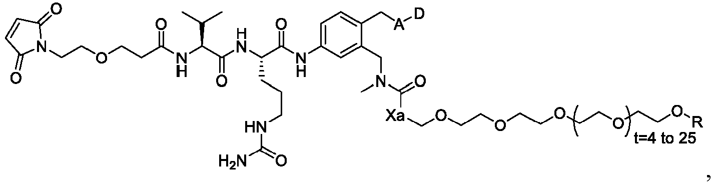 où :
Xa est -CH₂-, -OCH₂-, -NHCH₂- ou -NRCH₂- et chaque R est indépendamment H, -CH₃ ou -CH₂CH₂C(=O)OH ;
A est une liaison, -OC(=O)-*, 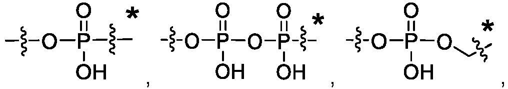 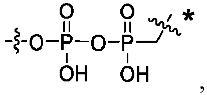 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(7) 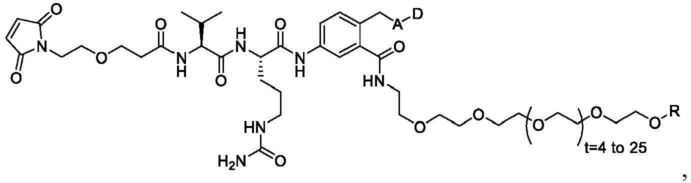 où :
R est H, -CH₃ ou -CH₂CH₂C(=O)OH ;
A est une liaison, -OC(=O)-*, 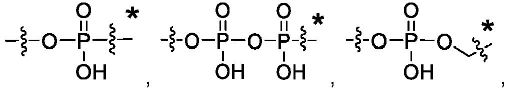 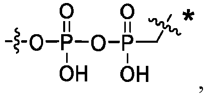 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(8) 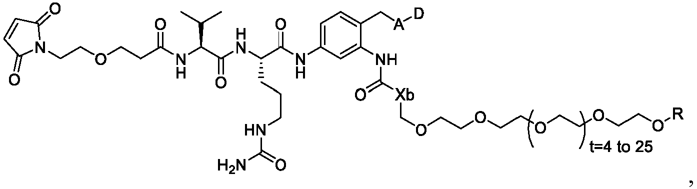 où :
Xb est -CH₂-, -OCH₂-, -NHCH₂- ou -NRCH₂- et chaque R est indépendamment H, -CH₃ ou -CH₂CH₂C(=O)OH ;
A est une liaison, -OC(=O)-*, 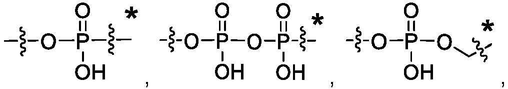 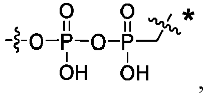 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(9) 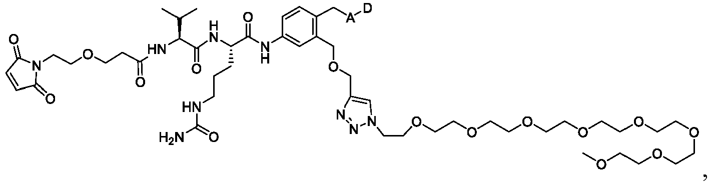 où :
A est une liaison, -OC(=O)-*, 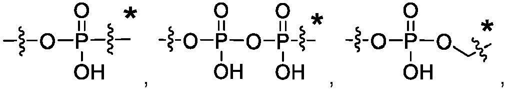 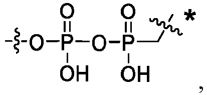 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(10) 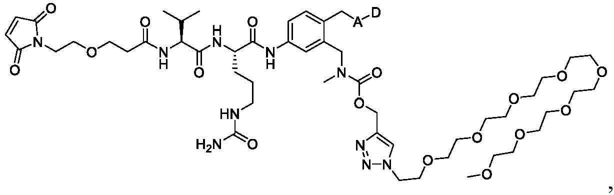 où :
A est une liaison, -OC(=O)-*, 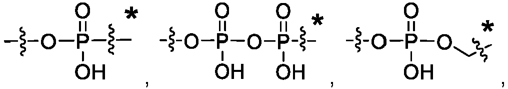 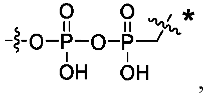 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(11) 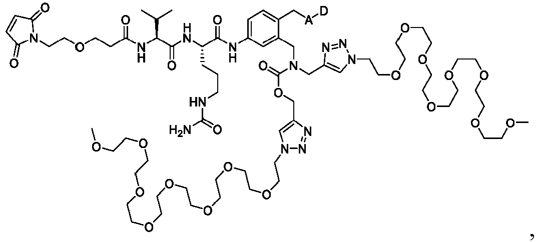 où :
A est une liaison, -OC(=O)-*, 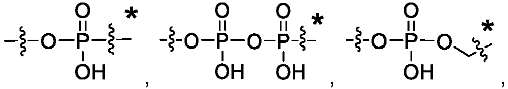 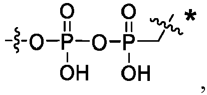 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(12) 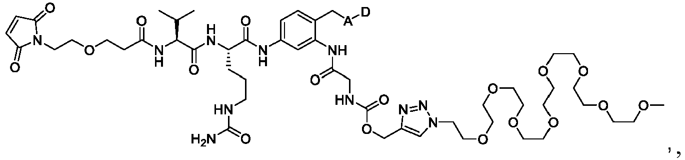 où :
A est une liaison, -OC(=O)-*, 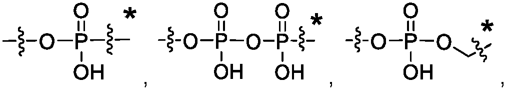 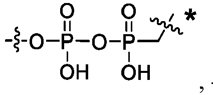 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(13) 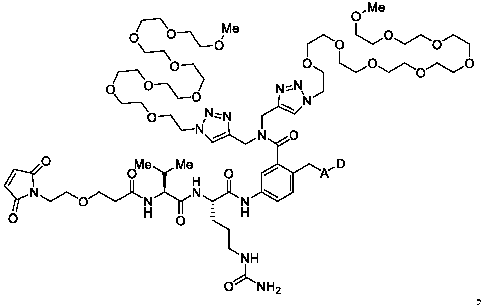 où :
A est une liaison, -OC(=O)-*, 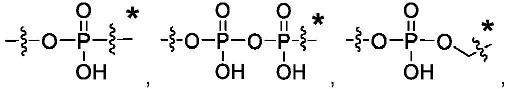 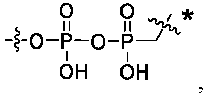 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(14) 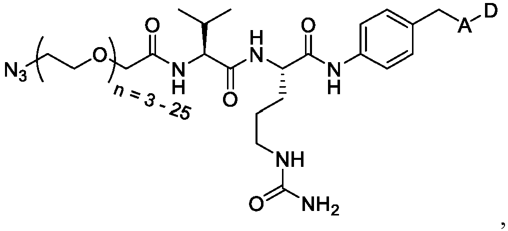 où :
A est une liaison, -OC(=O)-*, 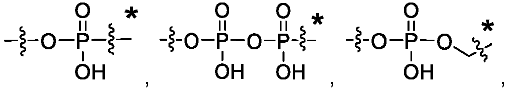 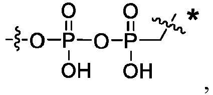 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ;
(15) 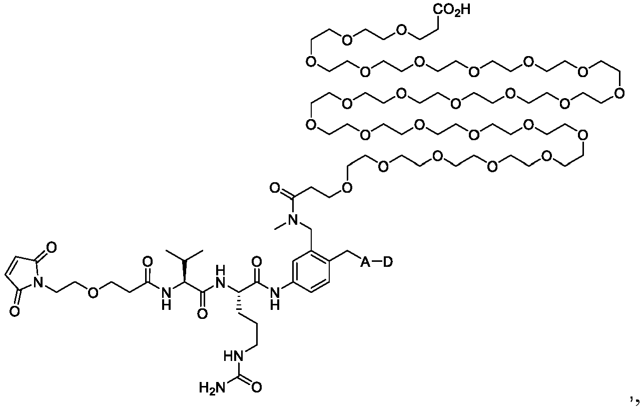 où :
A est une liaison, -OC(=O)-*, 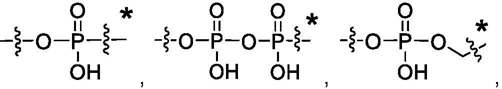 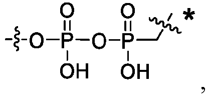 -OC(=O)N(CH₃)CH₂CH₂N(CH₃)C(=O)-* ou
-OC(=O)N(CH₃)C(R^{a})₂C(R^{a})₂N(CH₃)C(=O)-*_{,}
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1 ; ou
(16) 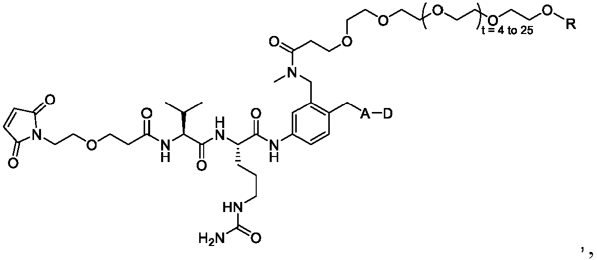 où :
chaque R est indépendamment H, -CH₃ ou -CH₂CH₂C(=O)OH ;
A est une liaison, -OC(=O)-*, 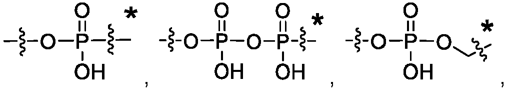 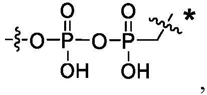 -OC(=O)N(CH₃)CH₂CH₂N(CH₃)C(=O)-* ou
-OC(=O)N(CH₃)C(R^{a})₂C(R^{a})₂N(CH₃)C(=O)-*,
où chaque R^{a} est indépendamment choisi parmi H, un alkyle en C₁-C₆ et un cycloalkyle en C₃-C₈ et * de A indique le point de liaison à D ; et
D est un inhibiteur de Mcl-1 tel que défini dans la revendication 1.

17. Conjugué anticorps-médicament de l'une quelconque des revendications 10 à 16, dans lequel A est une liaison et/ou R est -CH₃.

18. Conjugué anticorps-médicament de l'une quelconque des revendications 1 à 17, dans lequel D comprend un composé de Formule (III) : 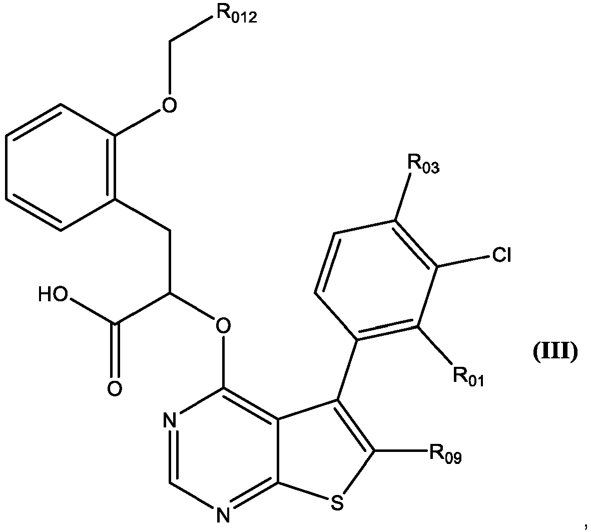
ou l'énantiomère, le diastéréoisomère, l'atropisomère, le dérivé deutéré et/ou le sel pharmaceutiquement acceptable de celui-ci ;
où :
R₀₁ est un groupe alkyle en (C₁-C₆) linéaire ou ramifié,
R₀₃ est -O-alkyle en (C₁-C₆)-NR₀₁₁R₀₁₁', ou 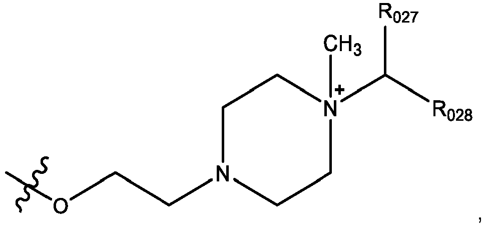
où R₀₁₁ et R₀₁₁' sont, indépendamment l'un de l'autre, un atome d'hydrogène, un groupe alkyle en (C₁-C₆) linéaire ou ramifié éventuellement substitué, ou -alkyle en (C₀-C₆)-Cy₀₁ ;
ou la paire (R₀₁₁, R₀₁₁'), conjointement avec l'atome d'azote auquel elle est liée, forme un cycle aromatique ou non aromatique contenant 5 à 7 chaînons de cycle, qui contient éventuellement, en plus de l'atome d'azote, 1 à 3 hétéroatomes choisis parmi O, S et N, où l'atome N peut être substitué par 1 ou 2 groupes choisis parmi un atome d'hydrogène ou un groupe alkyle en (C₁-C₆) linéaire ou ramifié,
et où R₀₂₇ est un atome d'hydrogène et R₀₂₈ est un groupe -(CH₂)ₚ₀-O-SO₂-O⁻ ou un groupe -(CH₂)ₚ₀-SO₂-OR₀₃₀ ;
R₀₉ est un groupe alcynyle en (C₂-C₆) linéaire ou ramifié ou -Cy₀₂,
R₀₁₂ est -Cy₀₅, -Cy₀₅-alkyle en (C₀-C₆)-Cy₀₆ ou-Cy₀₅-alkyle en (C₀-C₆)-Cy₀₉,
Cy₀₁, Cy₀₂, Cy₀₅ et Cy₀₆ sont, indépendamment les uns des autres, un groupe cycloalkyle, un groupe hétérocycloalkyle, un groupe aryle ou un groupe hétéroaryle, dont chacun est éventuellement substitué,
Cy₀₉ est 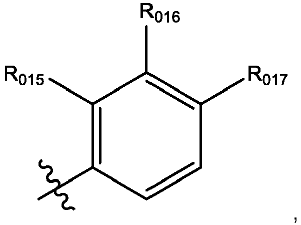
R₀₁₅, R₀₁₆ et R₀₁₇ sont tels que définis pour la formule (II),
où l'un des groupes R₀₃, R₀₉ ou R₀₁₂ est lié de manière covalente au lieur.

19. Conjugué anticorps-médicament de la revendication 18, dans lequel
(1) R₀₁ est un méthyle ou un éthyle ;
(2) R₀₃ est -O-CH₂-CH₂-NR₀₁₁R₀₁₁' où R₀₁₁ et R₀₁₁' forment, conjointement avec l'atome d'azote qui les porte, un groupe pipérazinyle qui peut être substitué par un groupe étant un atome d'hydrogène ou un groupe alkyle en (C₁-C₆) linéaire ou ramifié ;
(3) R₀₃ comprend la formule : 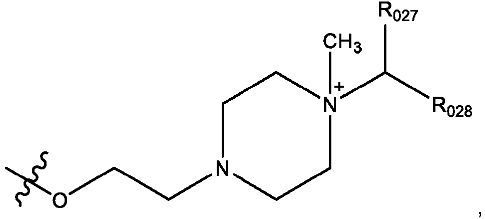 où R₀₂₇ est un atome d'hydrogène et R₀₂₈ est un groupe -(CH₂)ₚ₀-SO₂-OR₀₃₀ ;
(4) R₀₃ comprend la formule : 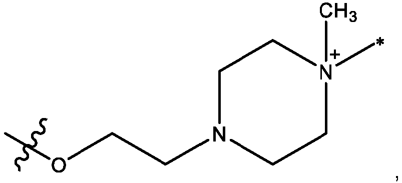 où -* est une liaison au lieur ;
(5) R₀₉ est Cy₀₂ ;
(6) Cy₀₂ est un groupe aryle éventuellement substitué ;
(7) Cy₀₅ comprend un groupe hétéroaryle choisi parmi un groupe pyrazolyle et un groupe pyrimidinyle ;
(8) Cy₀₅ est un groupe pyrimidinyle ; ou
(9) L est lié à D par une liaison covalente de L à R₀₃ de formule (II), ou (III) ; et/ou L est lié à D par une liaison covalente de L à R₀₉ de formule (II), ou (III).

20. Conjugué anticorps-médicament de l'une quelconque des revendications 1 à 18, dans lequel :
(1) D comprend : 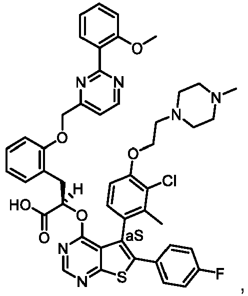 ou un énantiomère, un diastéréoisomère, un atropisomère, un dérivé deutéré et/ou un sel pharmaceutiquement acceptable de celui-ci ;
(2) D comprend : 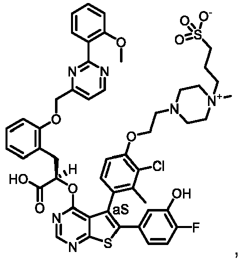 ou un énantiomère, un diastéréoisomère, un atropisomère, un dérivé deutéré et/ou un sel pharmaceutiquement acceptable de celui-ci ;
(3) D comprend : 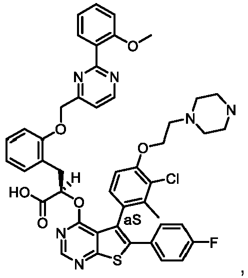 ou un énantiomère, un diastéréoisomère, un atropisomère, un dérivé deutéré et/ou un sel pharmaceutiquement acceptable de celui-ci ;
(4) D comprend : 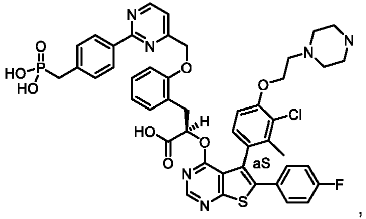 ou un énantiomère, un diastéréoisomère, un atropisomère, un dérivé deutéré et/ou un sel pharmaceutiquement acceptable de celui-ci ;
(5) D comprend : 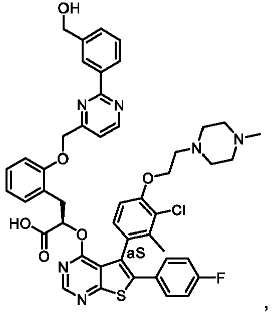 ou un énantiomère, un diastéréoisomère, un atropisomère, un dérivé deutéré et/ou un sel pharmaceutiquement acceptable de celui-ci ;
(6) D comprend : 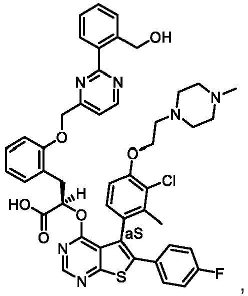 ou un énantiomère, un diastéréoisomère, un atropisomère, un dérivé deutéré et/ou un sel pharmaceutiquement acceptable de celui-ci ;
(7) D comprend : 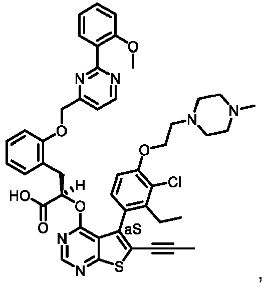 ou un énantiomère, un diastéréoisomère, un atropisomère, un dérivé deutéré et/ou un sel pharmaceutiquement acceptable de celui-ci ;
(8) D comprend : 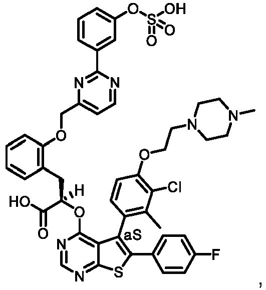 ou un énantiomère, un diastéréoisomère, un atropisomère, un dérivé deutéré et/ou un sel pharmaceutiquement acceptable de celui-ci ;
(9) D comprend : 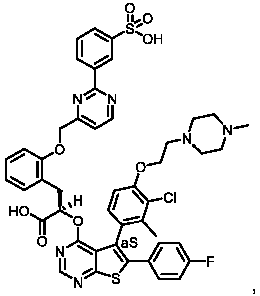 ou un énantiomère, un diastéréoisomère, un atropisomère, un dérivé deutéré et/ou un sel pharmaceutiquement acceptable de celui-ci ;
(10) D comprend : 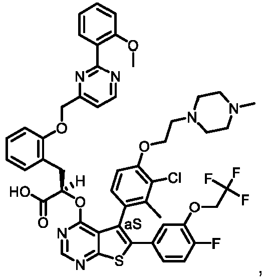 ou un énantiomère, un diastéréoisomère, un atropisomère, un dérivé deutéré et/ou un sel pharmaceutiquement acceptable de celui-ci ;
(11) D comprend : 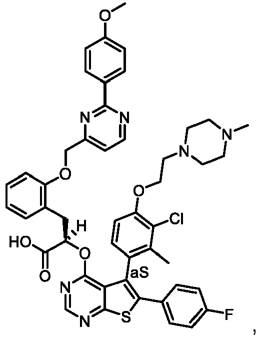 ou un énantiomère, un diastéréoisomère, un atropisomère, un dérivé deutéré et/ou un sel pharmaceutiquement acceptable de celui-ci ;
(12) D comprend : 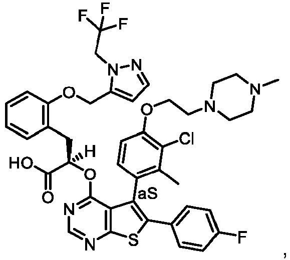 ou un énantiomère, un diastéréoisomère, un atropisomère, un dérivé deutéré et/ou un sel pharmaceutiquement acceptable de celui-ci ;
(13) D comprend : 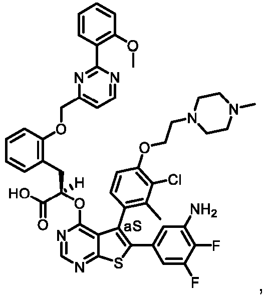 ou un énantiomère, un diastéréoisomère, un atropisomère, un dérivé deutéré et/ou un sel pharmaceutiquement acceptable de celui-ci ; ou
(14) D comprend : 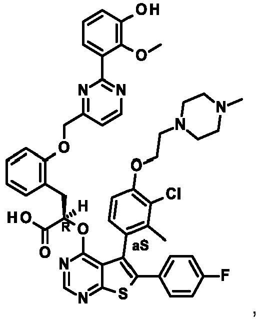 ou un énantiomère, un diastéréoisomère, un atropisomère, un dérivé deutéré et/ou un sel pharmaceutiquement acceptable de celui-ci.

21. Conjugué anticorps-médicament selon l'une quelconque des revendications 1 à 18, dans lequel -(L-D) est formé à partir d'un composé du Tableau A ou d'un énantiomère, diastéréoisomère, atropisomère, dérivé deutéré et/ou sel pharmaceutiquement acceptable de celui-ci.

22. Conjugué anticorps-médicament de l'une quelconque des revendications 1 à 21, dans lequel l'anticorps ou le fragment de liaison à l'antigène se lie à un antigène cible sur la cellule cancéreuse ; éventuellement dans lequel
(1)
(i) l'antigène cible est BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46 ou GPNMB ;
(ii) l'antigène cible est BCMA, CD33, PCAD, HER2, CD38, CD46, CD48 ou CD79b ; ou
(iii) l'antigène cible est BCMA, CD33, CD48, PCAD ou HER2 ;
(2) l'anticorps ou le fragment de liaison à l'antigène est un anticorps anti-BCMA ou un fragment de liaison à l'antigène ;
(3) l'anticorps ou le fragment de liaison à l'antigène comprend :
(a) trois régions déterminant la complémentarité de chaîne lourde (HCDR) comprenant des séquences d'acides aminés de SEQ ID N° : 15 (HCDR1), SEQ ID N° : 16 (HCDR2) et SEQ ID N° : 17 (HCDR3) ; et trois régions déterminant la complémentarité de chaîne légère (LCDR) comprenant des séquences d'acides aminés de SEQ ID N° : 18 (LCDR1), SEQ ID N° : 19 (LCDR2) et SEQ ID N° :20 (LCDR3) ; ou
(b) l'anticorps ou le fragment de liaison à l'antigène comprend une région variable de chaîne lourde comprenant une séquence d'acides aminés de SEQ ID N° : 1, et une région variable de chaîne légère comprenant une séquence d'acides aminés de SEQ ID N° : 2 ;
(4)
(a) l'anticorps ou le fragment de liaison à l'antigène comprend un domaine constant de chaîne lourde IgG1 ou un domaine constant de chaîne lourde IgG1 modifié, éventuellement le domaine constant de chaîne lourde IgG1 comprend un résidu cystéine (C) en position 152 et en position 375, ou le domaine constant de chaîne lourde IgG1 comprend un résidu cystéine (C) en position 156 et en position 379 ;
et/ou
(b) l'anticorps ou le fragment de liaison à l'antigène comprend un domaine constant de chaîne légère Ig kappa ;
(5) l'anticorps ou le fragment de liaison à l'antigène est un anticorps anti-CD33 ou un fragment de liaison à l'antigène ;
(6)
(a) l'anticorps ou le fragment de liaison à l'antigène comprend trois régions déterminant la complémentarité de chaîne lourde (HCDR) comprenant des séquences d'acides aminés de SEQ ID N° : 21 (HCDR1), SEQ ID N° : 22 (HCDR2) et SEQ ID N° : 23 (HCDR3) ; et trois régions déterminant la complémentarité de chaîne légère (LCDR) comprenant des séquences d'acides aminés de SEQ ID N° : 24 (LCDR1), SEQ ID N° : 25 (LCDR2) et SEQ ID N° : 26 (LCDR3) ; et/ou
(b) l'anticorps ou le fragment de liaison à l'antigène comprend une région variable de chaîne lourde comprenant une séquence d'acides aminés de SEQ ID N° : 3, et une région variable de chaîne légère comprenant une séquence d'acides aminés de SEQ ID N° : 4 ;
(7)
(a) l'anticorps ou le fragment de liaison à l'antigène comprend un domaine constant de chaîne lourde IgG1 ou un domaine constant de chaîne lourde IgG1 modifié, éventuellement le domaine constant de chaîne lourde IgG1 comprend une glutamine (Q) en position 297 ; et/ou
(b) l'anticorps ou le fragment de liaison à l'antigène comprend un domaine constant de chaîne légère Ig kappa ;
(8) l'anticorps ou le fragment de liaison à l'antigène est un anticorps anti-PCAD ou un fragment de liaison à l'antigène ;
(9)
(a) l'anticorps ou le fragment de liaison à l'antigène comprend trois régions déterminant la complémentarité de chaîne lourde (HCDR) comprenant des séquences d'acides aminés de SEQ ID N° : 33 (HCDR1), SEQ ID N° : 34 (HCDR2) et SEQ ID N° : 35 (HCDR3) ; et trois régions déterminant la complémentarité de chaîne légère (LCDR) comprenant des séquences d'acides aminés de SEQ ID N° : 36 (LCDR1), SEQ ID N° : 37 (LCDR2) et SEQ ID N° : 38 (LCDR3) ; et/ou
(b) l'anticorps ou le fragment de liaison à l'antigène comprend une région variable de chaîne lourde comprenant une séquence d'acides aminés de SEQ ID N° : 7, et une région variable de chaîne légère comprenant une séquence d'acides aminés de SEQ ID N° : 8 ;
(10) l'anticorps ou le fragment de liaison à l'antigène est un anticorps anti-HER2 ou un fragment de liaison à l'antigène ;
(11)
(a) l'anticorps ou le fragment de liaison à l'antigène comprend trois régions déterminant la complémentarité de chaîne lourde (HCDR) comprenant des séquences d'acides aminés de SEQ ID N° : 39 (HCDR1), SEQ ID N° : 40 (HCDR2) et SEQ ID N° : 41 (HCDR3) ; et trois régions déterminant la complémentarité de chaîne légère (LCDR) comprenant des séquences d'acides aminés de SEQ ID N° : 42 (LCDR1), SEQ ID N° : 43 (LCDR2) et SEQ ID N° : 44 (LCDR3) ; et/ou
(b) l'anticorps ou le fragment de liaison à l'antigène comprend une région variable de chaîne lourde comprenant une séquence d'acides aminés de SEQ ID N° : 9, et une région variable de chaîne légère comprenant une séquence d'acides aminés de SEQ ID N° :10 ;
(12)
(a) l'anticorps ou le fragment de liaison à l'antigène comprend un domaine constant de chaîne lourde IgG1 ou un domaine constant de chaîne lourde IgG1 modifié, éventuellement le domaine constant de chaîne lourde IgG1 comprend une glutamine (Q) en position 297 ou le domaine constant de chaîne lourde IgG1 comprend une sérine (S) en position 297 ; et/ou
(b) l'anticorps ou le fragment de liaison à l'antigène comprend un domaine constant de chaîne légère Ig kappa ; ou
(13) l'anticorps ou le fragment de liaison à l'antigène est un anticorps anti-CD38 ou un fragment de liaison à l'antigène ; un anticorps anti-CD46 ou un fragment de liaison à l'antigène ; un anticorps anti-CD48 ou un fragment de liaison à l'antigène ; ou un anticorps anti-CD79b ou un fragment de liaison à l'antigène.

23. Composition comprenant de multiples copies du conjugué anticorps-médicament de l'une quelconque des revendications 1 à 22, dans laquelle la valeur moyenne *p* des conjugués anticorps-médicament dans la composition est d'environ 2 à environ 16, par exemple d'environ 2 à environ 8, par exemple d'environ 2 à environ 4.

24. Composition pharmaceutique comprenant le conjugué anticorps-médicament de l'une quelconque des revendications 1 à 22 ou la composition de la revendication 23 et un support pharmaceutiquement acceptable.

25. Conjugué anticorps-médicament de l'une quelconque des revendications 1 à 22, composition de la revendication 23, ou composition pharmaceutique de la revendication 24 destiné(e) à être utilisé(e) dans le traitement d'un sujet atteint ou suspecté d'être atteint d'un cancer,
(1) le cancer exprime un antigène cible ; ou
(2) le cancer est une tumeur ou un cancer hématologique, éventuellement, le cancer est un cancer du sein, un myélome multiple, un myélome des cellules plasmatiques, une leucémie, un lymphome, un cancer gastrique, une leucémie myéloïde aiguë, un cancer de la vessie, un cancer du cerveau, un cancer de la moelle osseuse, un cancer du col de l'utérus, une leucémie lymphoïde chronique, un cancer colorectal, un cancer de l'œsophage, un cancer hépatocellulaire, une leucémie lymphoblastique, un lymphome folliculaire, des tumeurs malignes lymphoïdes d'origine lymphocytaire T ou B, un mélanome, une leucémie myéloïde, un myélome, un cancer de la cavité buccale, un cancer de l'ovaire, un cancer du poumon non à petites cellules, une leucémie lymphoïde chronique, un cancer de la prostate, un cancer du poumon à petites cellules ou un cancer de la rate.

26. Conjugué anticorps-médicament de l'une quelconque des revendications 1 à 22, composition de la revendication 23, ou composition pharmaceutique de la revendication 24 destiné(e) à être utilisé(e) dans la réduction ou l'inhibition de la croissance d'une tumeur chez un sujet, où
(1) la tumeur exprime un antigène cible ;
(2) la tumeur est un cancer du sein, un cancer gastrique, un cancer de la vessie, un cancer du cerveau, un cancer du col de l'utérus, un cancer colorectal, un cancer de l'œsophage, un cancer hépatocellulaire, un mélanome, un cancer de la cavité buccale, un cancer de l'ovaire, un cancer du poumon non à petites cellules, un cancer de la prostate, un cancer du poumon à petites cellules ou un cancer de la rate ; ou
(3) l'administration du conjugué anticorps-médicament, de la composition, ou de la composition pharmaceutique réduit ou inhibe la croissance de la tumeur d'au moins environ 10 %, d'au moins environ 20 %, d'au moins environ 30 %, d'au moins environ 40 %, d'au moins environ 50 %, d'au moins environ 60 %, d'au moins environ 70 %, d'au moins environ 80 %, d'au moins environ 90 %, d'au moins environ 95 %, ou d'au moins environ 99 %.

27. Conjugué anticorps-médicament de l'une quelconque des revendications 1 à 22, composition de la revendication 23, ou composition pharmaceutique de la revendication 24, destiné(e) à être utilisé(e) dans la réduction ou le ralentissement de l'expansion d'une population de cellules cancéreuses chez un sujet, où
(1) la population de cellules cancéreuses exprime un antigène cible ;
(2) la population de cellules cancéreuses provient d'une tumeur ou d'un cancer hématologique, où éventuellement la population de cellules cancéreuses provient d'un cancer du sein, d'un myélome multiple, d'un myélome des cellules plasmatiques, d'une leucémie, d'un lymphome, d'un cancer gastrique, d'une leucémie myéloïde aiguë, d'un cancer de la vessie, d'un cancer du cerveau, d'un cancer de la moelle osseuse, d'un cancer du col de l'utérus, d'une leucémie lymphoïde chronique, d'un cancer colorectal, d'un cancer de l'œsophage, d'un cancer hépatocellulaire, d'une leucémie lymphoblastique, d'un lymphome folliculaire, de tumeurs malignes lymphoïdes d'origine lymphocytaire T ou B, d'un mélanome, d'une leucémie myéloïde, d'un myélome, d'un cancer de la cavité buccale, d'un cancer de l'ovaire, d'un cancer du poumon non à petites cellules, d'une leucémie lymphoïde chronique, d'un cancer de la prostate, d'un cancer du poumon à petites cellules ou d'un cancer de la rate ; ou
(3) l'administration du conjugué anticorps-médicament, de la composition ou de la composition pharmaceutique réduit la population de cellules cancéreuses ou ralentit l'expansion de la population de cellules cancéreuses d'au moins environ 10 %, d'au moins environ 20 %, d'au moins environ 30 %, d'au moins environ 40 %, d'au moins environ 50 %, d'au moins environ 60 %, d'au moins environ 70 %, d'au moins environ 80 %, d'au moins environ 90 %, d'au moins environ 95 % ou d'au moins environ 99 %.

28. Procédé permettant de déterminer si un sujet atteint ou suspecté d'être atteint d'un cancer répondra à un traitement par le conjugué anticorps-médicament de l'une quelconque des revendications 1 à 22, la composition de la revendication 23 ou la composition pharmaceutique de la revendication 24, comprenant la fourniture d'un échantillon biologique du sujet ; la mise en contact de l'échantillon avec le conjugué anticorps-médicament ; et la détection de la liaison du conjugué anticorps-médicament à des cellules cancéreuses dans l'échantillon ; dans lequel
(1) les cellules cancéreuses dans l'échantillon expriment un antigène cible ;
(2) le cancer exprime un antigène cible ;
(3) le cancer est une tumeur ou un cancer hématologique, éventuellement, le cancer est un cancer du sein, un myélome multiple, un myélome des cellules plasmatiques, une leucémie, un lymphome, un cancer gastrique, une leucémie myéloïde aiguë, un cancer de la vessie, un cancer du cerveau, un cancer de la moelle osseuse, un cancer du col de l'utérus, une leucémie lymphoïde chronique, un cancer colorectal, un cancer de l'œsophage, un cancer hépatocellulaire, une leucémie lymphoblastique, un lymphome folliculaire, des tumeurs malignes lymphoïdes d'origine lymphocytaire T ou B, un mélanome, une leucémie myéloïde, un myélome, un cancer de la cavité buccale, un cancer de l'ovaire, un cancer du poumon non à petites cellules, une leucémie lymphoïde chronique, un cancer de la prostate, un cancer du poumon à petites cellules ou un cancer de la rate ; ou
(4) l'échantillon est un échantillon de biopsie tissulaire, un échantillon de sang ou un échantillon de moelle osseuse.

29. Conjugué anticorps-médicament de l'une quelconque des revendications 1 à 22, composition de la revendication 23, ou composition pharmaceutique de la revendication 24 destiné(e) à être utilisé(e) selon l'une quelconque des revendications 25 à 27, ou procédé de la revendication 28, où (i) l'antigène cible est BCMA, CD33, HER2, CD38, CD48, CD79b, PCAD, CD74, CD138, SLAMF7, CD123, CLL1, FLT3, CD7, CKIT, CD56, DLL3, DLK1, B7-H3, EGFR, CD71, EPCAM, FOLR1, ENPP3, MET, AXL, SLC34A2, Nectin4, TROP2, LIV1, CD46, ou GPNMB ; (ii) l'antigène cible est BCMA, CD33, PCAD, HER2, CD38, CD46, CD48 ou CD79b ; ou (iii) BCMA, CD33, CD48, PCAD ou HER2.

30. Procédé de production du conjugué anticorps-médicament de l'une quelconque des revendications 1 à 22, comprenant la réaction d'un anticorps ou d'un fragment de liaison à l'antigène avec un lieur clivable lié à un inhibiteur de MCL1 dans des conditions qui permettent la conjugaison.

31. Conjugué anticorps-médicament de l'une quelconque des revendications 1 à 22, composition de la revendication 23, ou composition pharmaceutique de la revendication 24, destiné(e) à être utilisé(e) selon l'une quelconque des revendications 25 à 27, ou procédé de la revendication 28, comprenant en outre l'administration au sujet en ayant besoin d'au moins un agent thérapeutique supplémentaire, de préférence l'agent thérapeutique supplémentaire est un inhibiteur de Bcl-2, idéalement l'agent thérapeutique supplémentaire est le vénétoclax, le composé A1 ou le composé A2.

32. Composé présentant l'une des structures suivantes : 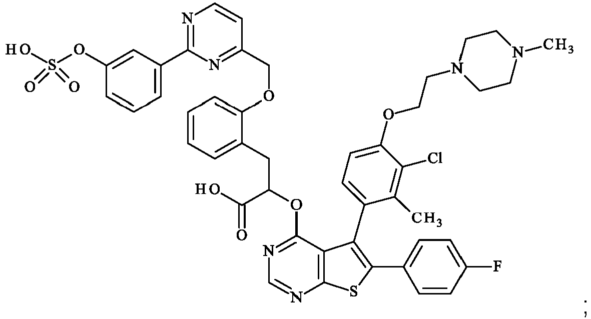 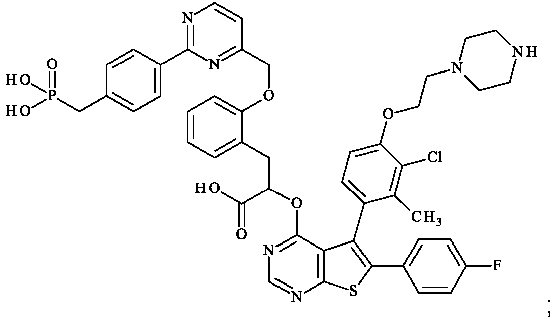 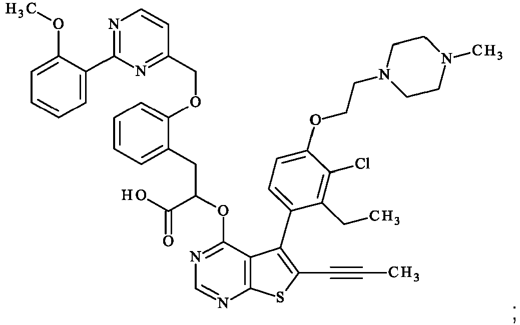 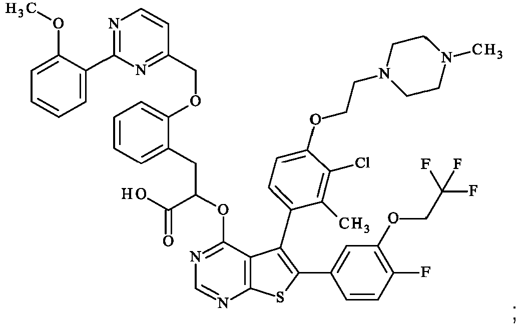 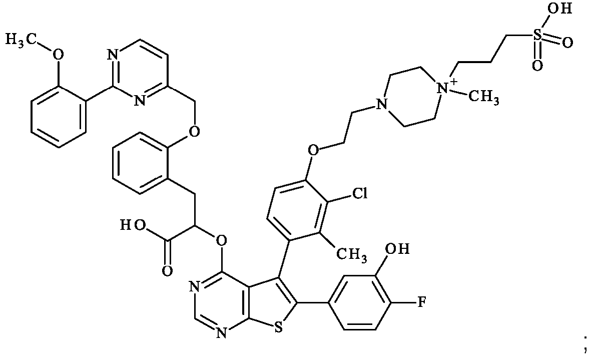 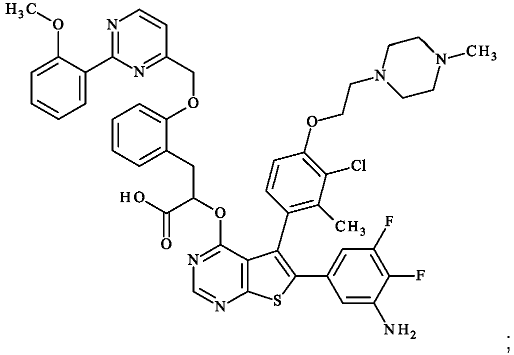 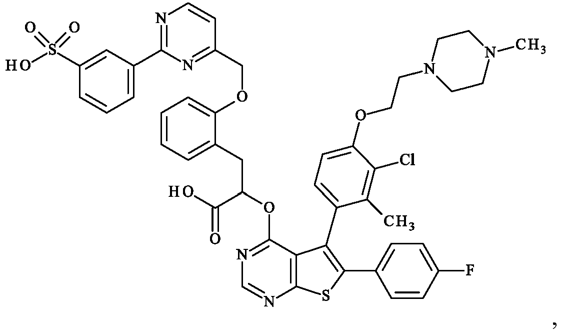 ou 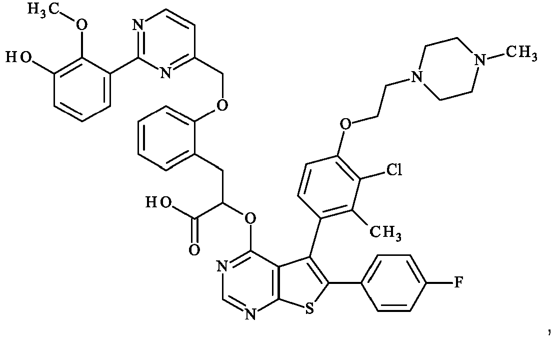 ou un sel de celui-ci (par exemple*,* un sel pharmaceutiquement acceptable de celui-ci).

33. Composé de la revendication 32, dans lequel le composé est utilisé comme intermédiaire de synthèse pour la préparation d'un conjugué anticorps-médicament de Formule (1) selon la revendication 1.

34. Composé selon la revendication 32 ou sel de celui-ci avec un acide ou une base pharmaceutiquement acceptable, destiné à être utilisé en tant qu'agent pro-apoptotique.
